# EUROPEAN PATENT SPECIFICATION

(11) **EP 3 377 473 B1**
(45) Date of publication and mention of the grant of the patent: **19.06.2024**
(21) Application number: 16840309.5
(22) Date of filing: 21.11.2016
(51) Int. Cl.: C07D 245/04, A61K 31/395, A61P 31/04

(54) **MACROCYCLIC BROAD SPECTRUM ANTIBIOTICS**
MAKROCYCLISCHE BREITSPEKTRUM-ANTIBIOTIKA
ANTIBIOTIQUES MACROCYCLIQUES À LARGE SPECTRE

(30) Priority: 20.11.2015 WO PCT/CN2015/095166; 08.11.2016 WO PCT/CN2016/105042
(43) Date of publication of application: 26.09.2018
(73) Proprietor: RQX Pharmaceuticals, Inc., La Jolla, CA 92037 (US); Genentech, Inc., South San Francisco, CA 94080 (US)
(72) Inventor: CHEN, Yongsheng, Shanghai 200131 (CN); SMITH, Peter Andrew, San Francisco, CA 94107 (US); ROBERTS, Tucker Curran, San Diego, CA 92103 (US); HIGUCHI, Robert I., Solana Beach, CA 92075 (US); PARASELLI, Prasuna, San Diego, CA 92130 (US); KOEHLER, Michael F. T., Palo Alto, CA 94306 (US); SCHWARZ, Jacob Bradley, San Ramon, CA 94582 (US); CRAWFORD, James John, San Francisco, CA 94131 (US); LY, Cuong Q., Burlingame, CA 94010 (US); HANAN, Emily J., Redwood City, CA 94061 (US); HU, Huiyong, Fremont, CA 94555 (US); YU, Zhiyong, Shanghai 200131 (CN)
(74) Representative: Mewburn Ellis LLP
(86) International application number: PCT/CN2016/106598
(87) International publication number: WO 2017/084630

(56) References cited:
- WO-A1-2013/138187
- WO-A1-2013/138187
- WO-A2-2012/166665
- WO-A2-2015/179441
- LIU JIAN ET AL: "Efforts toward broadening the spectrum of arylomycin antibiotic activity", BIOORGANIC & MEDICINAL CHEMISTRY LETTERS, vol. 23, no. 20, 14 August 2013 (2013-08-14), pages 5654-5659, XP028731070, ISSN: 0960-894X, DOI: 10.1016/J.BMCL.2013.08.026
- LIU JIAN ET AL: "Efforts toward broadening the spectrum of arylomycin antibiotic activity", BIOORGANIC & MEDICINAL CHEMISTRY LETTERS, ELSEVIER, AMSTERDAM NL, vol. 23, no. 20, 14 August 2013 (2013-08-14), pages 5654-5659, XP028731070, ISSN: 0960-894X, DOI: 10.1016/J.BMCL.2013.08.026

## Description

### BACKGROUND OF THE INVENTION

Antibiotic resistance is a serious and growing phenomenon in contemporary medicine and has emerged as a major public health concern of the 21st century. Therefore, novel classes of broad-spectrum antibiotics, especially those that target novel mechanisms of action, are needed to treat multidrug-resistant pathogens.
WO2013/138187A1 describes arylomycin analogs with broad spectrum bioactivity.
Liu, Jian et al (Bioorg. Med. Chem. Lett., 2013, 23(20), 5654-9) reports the synthesis and evaluation of several arylomycin derivatives and demonstrates that both C-terminal homologation with a glycyl aldehyde and addition of a positive charge to the macrocycle increase the activity and spectrum of the arylomycin scaffold.

### SUMMARY OF THE INVENTION

Described herein are novel macrocyclic compounds for the treatment of microbial infections, such as for the treatment of bacterial infections. In various embodiments, the present disclosure provides lipopeptide macrocyclic compounds for the treatment of bacterial infections. In various embodiments, the present disclosure provides classes and subclasses of chemical compounds structurally related to arylomycin for the treatment of bacterial infections. In various embodiments, the macrocyclic compounds act by inhibition of bacterial type 1 signal peptidase (SpsB), an essential protein in bacteria.

In one aspect the present invention provides a compound selected from: 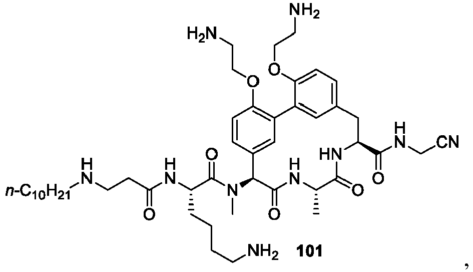 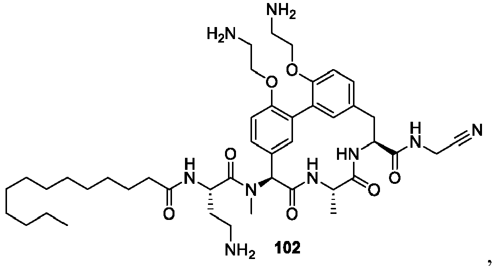 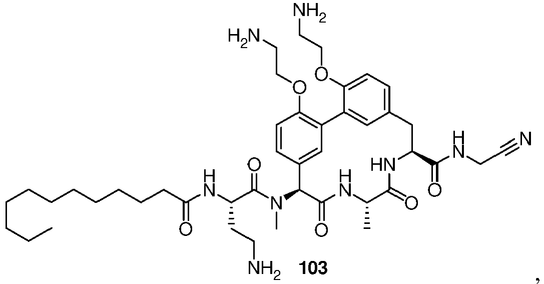 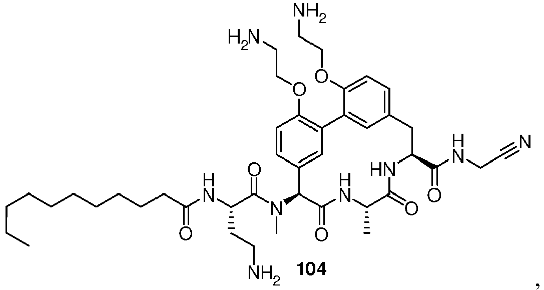 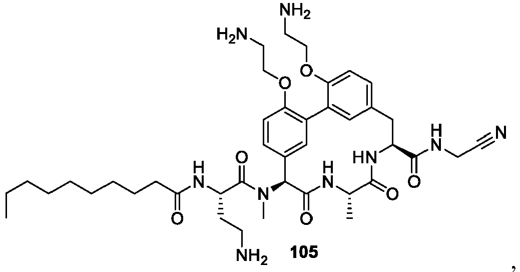 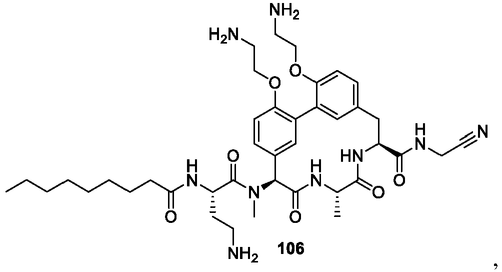 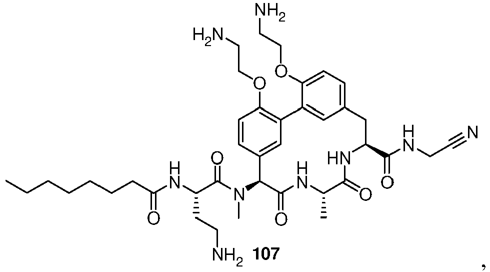 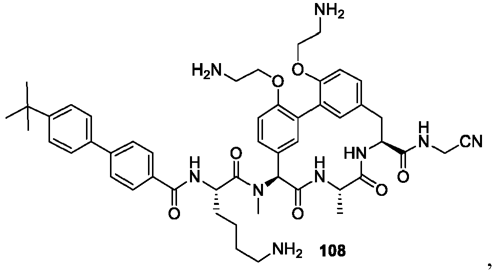 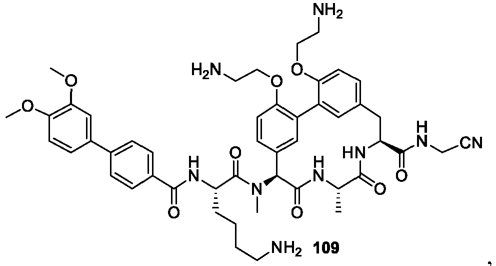 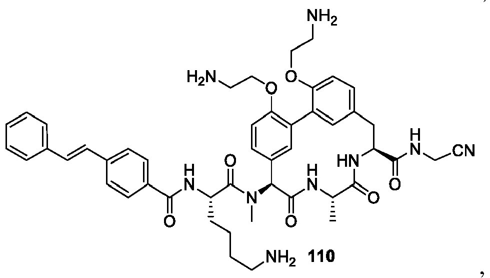 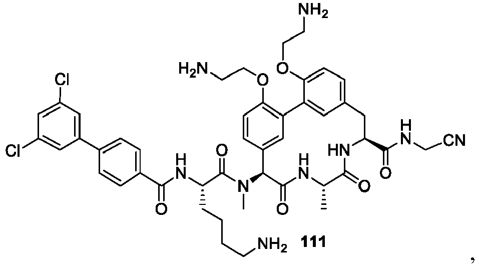 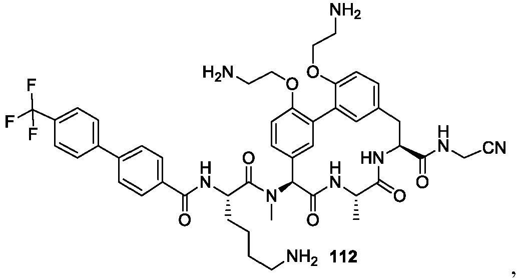 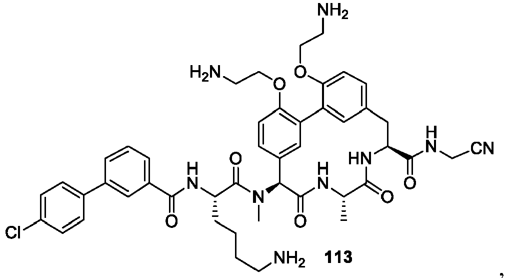 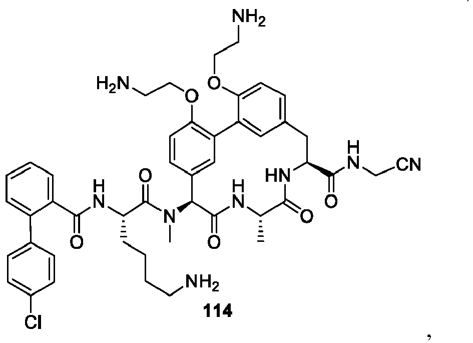 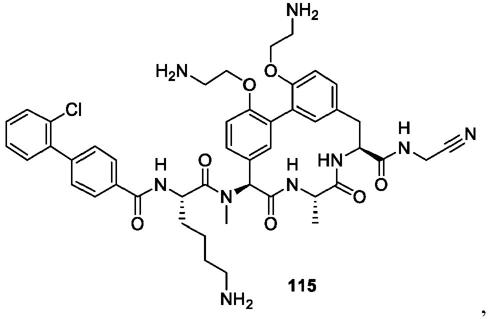 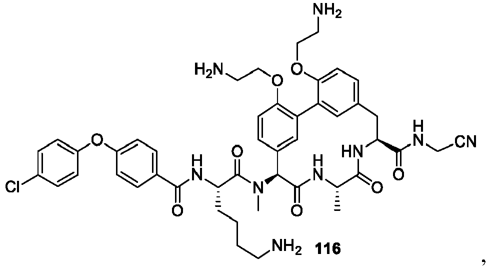 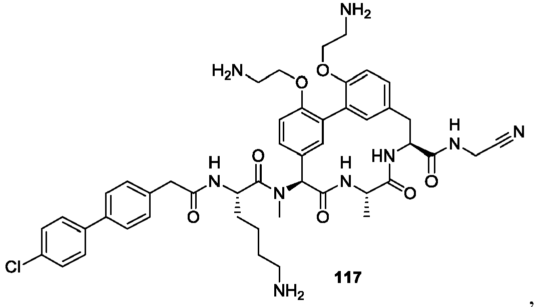 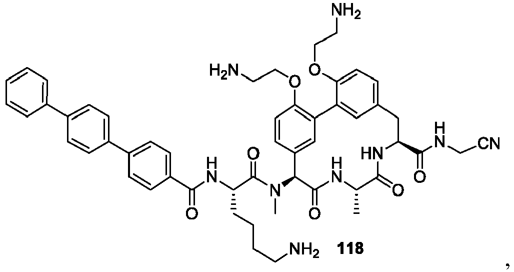 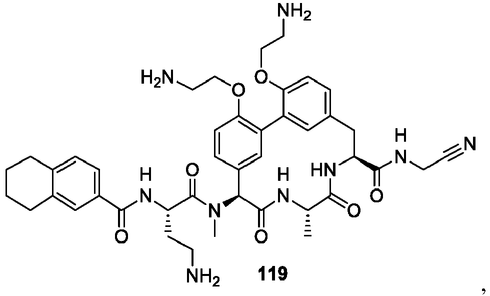 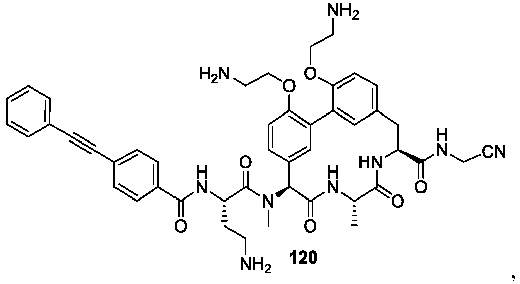 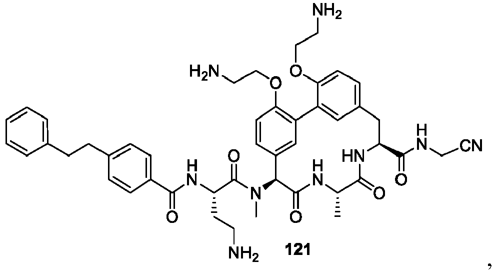 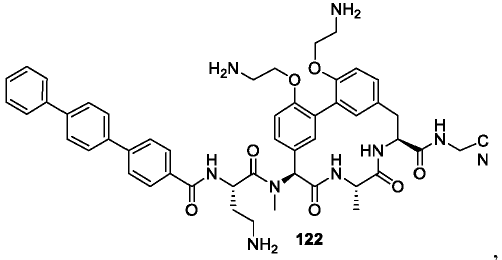 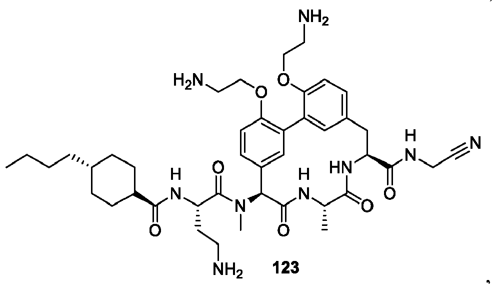 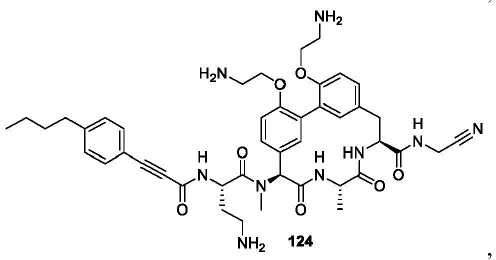 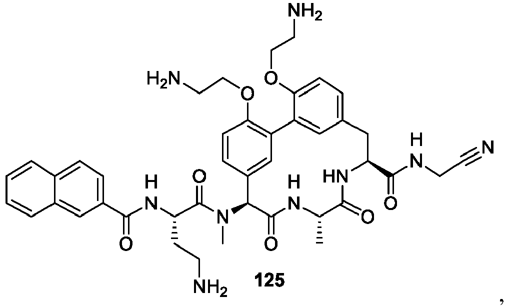 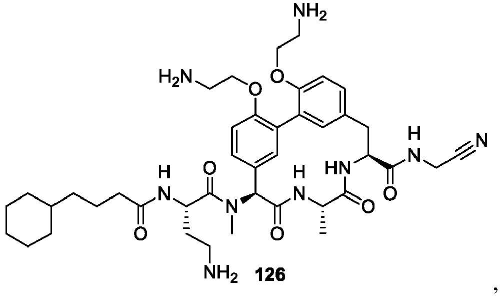 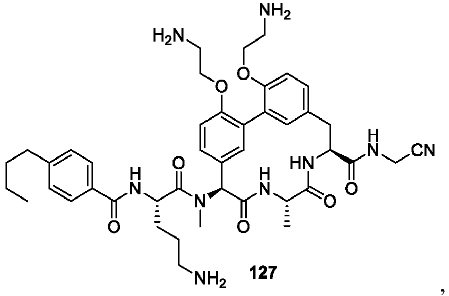 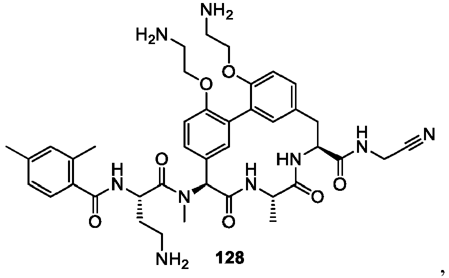 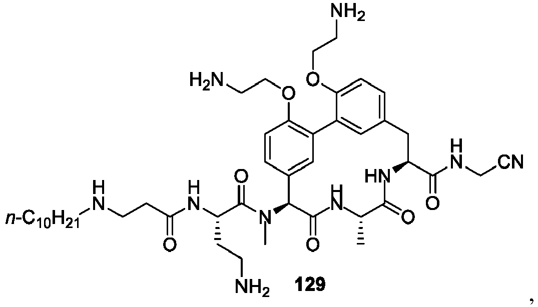 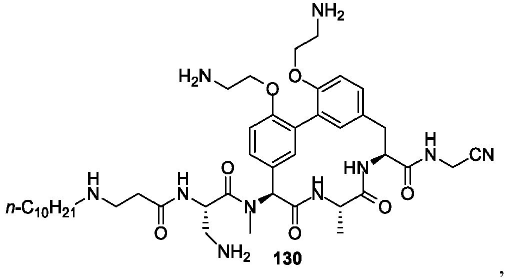 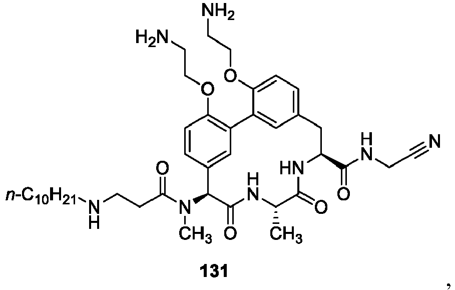 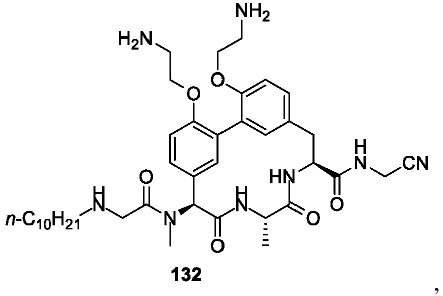 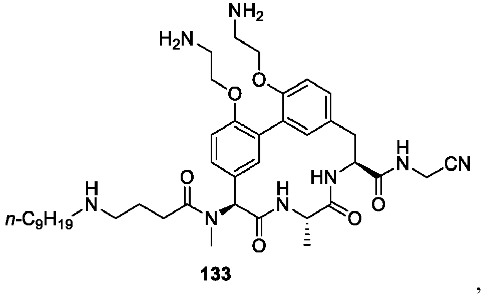 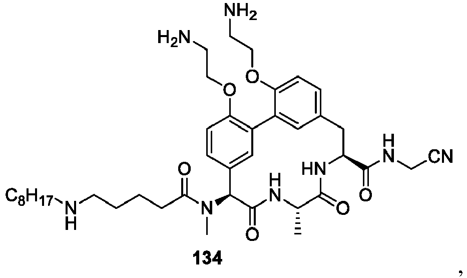 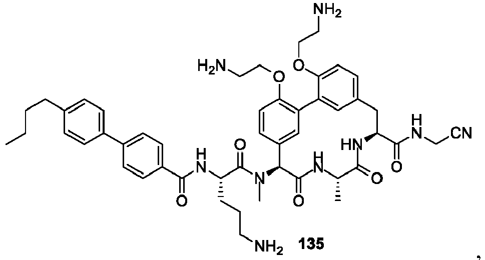 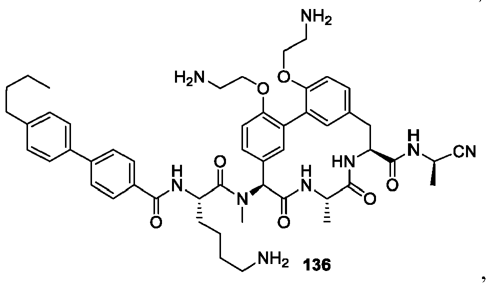 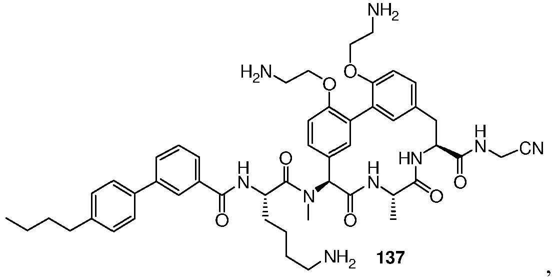 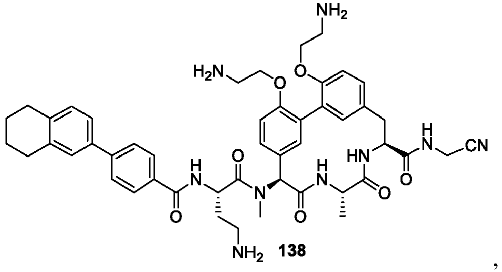 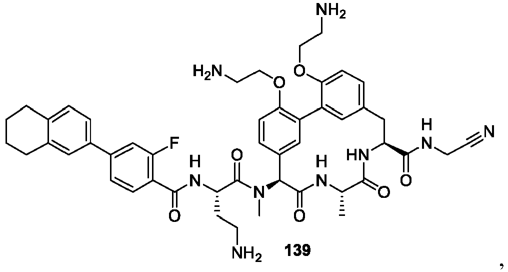 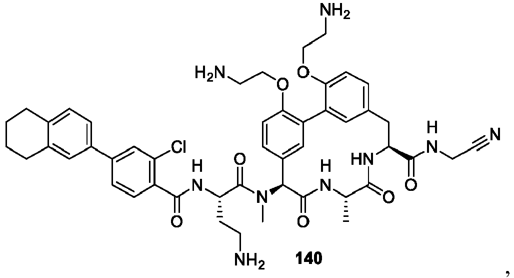 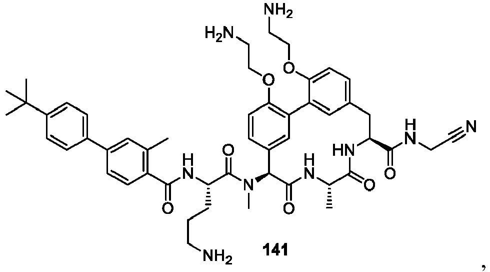 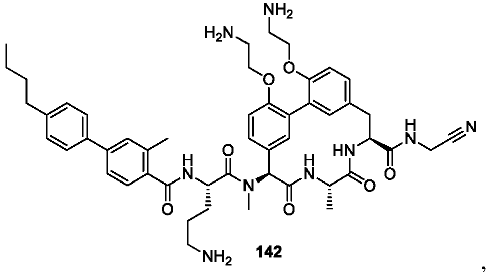 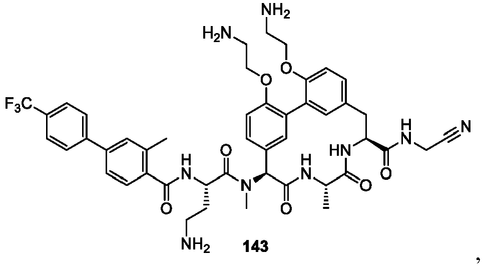 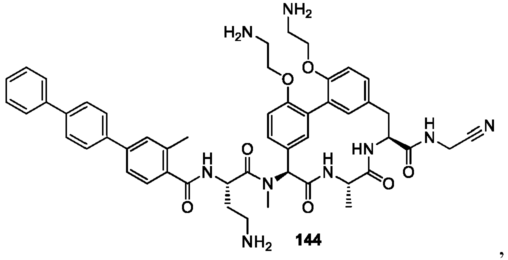 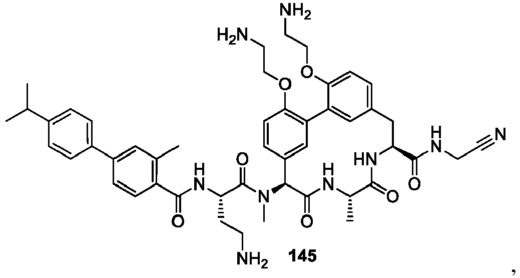 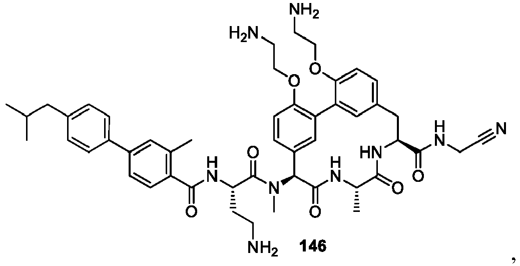 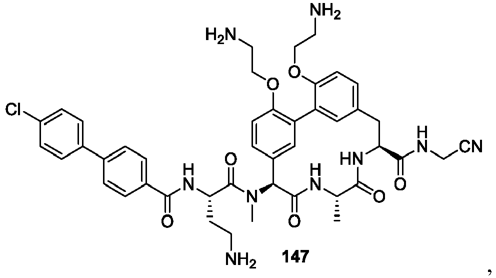 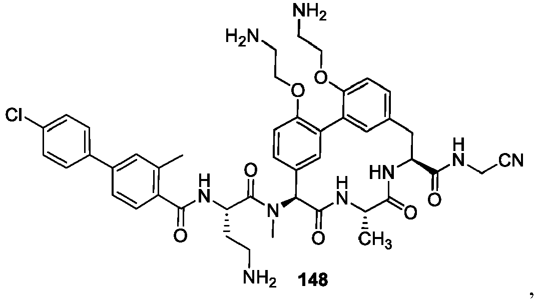 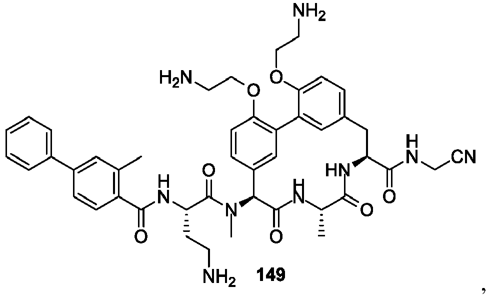 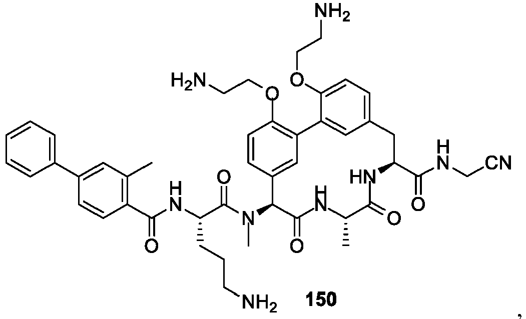 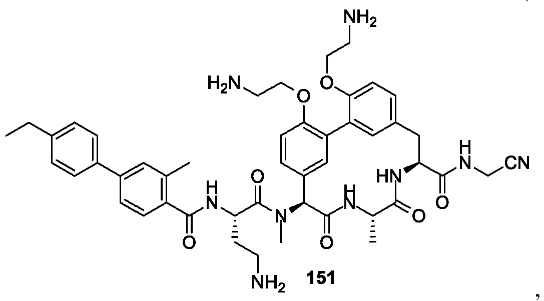 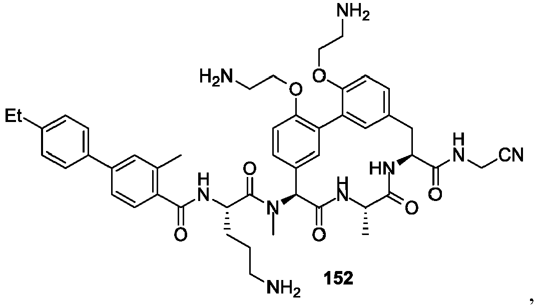 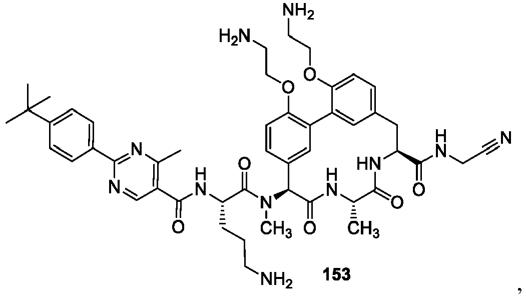 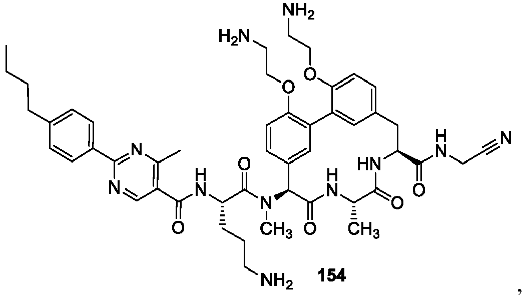 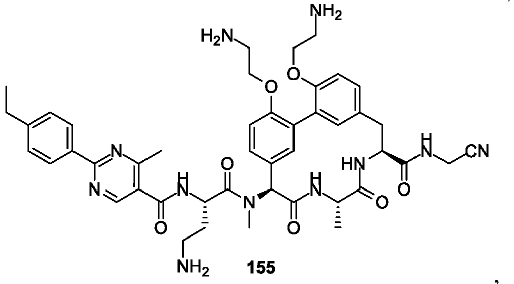 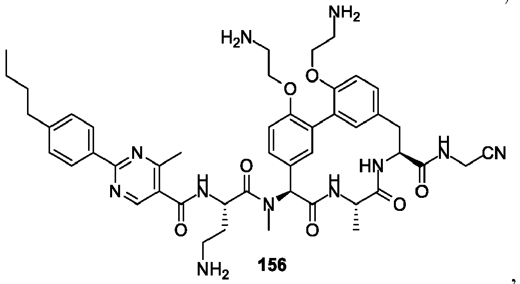 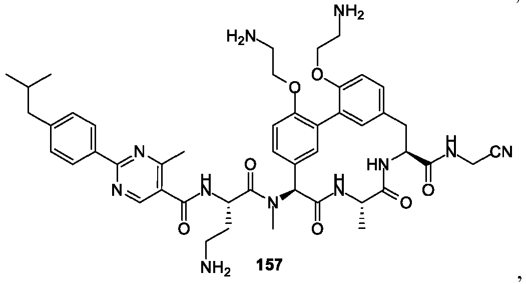 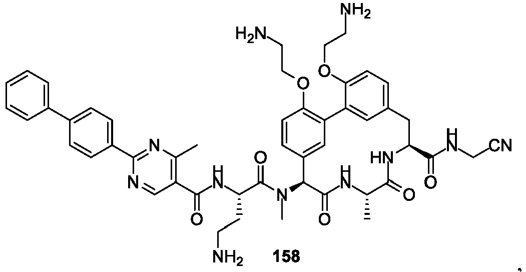 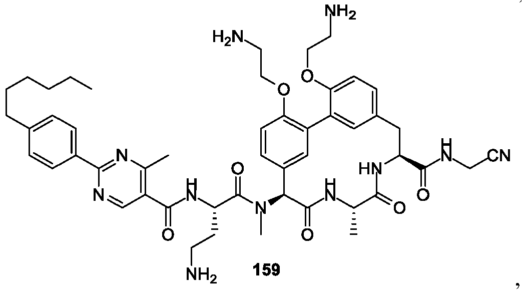 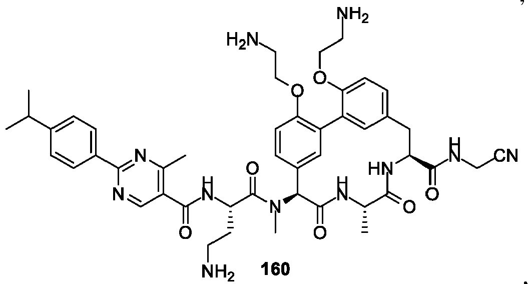 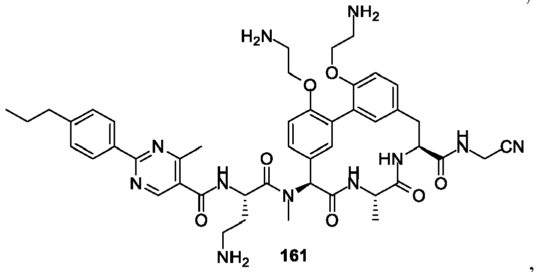 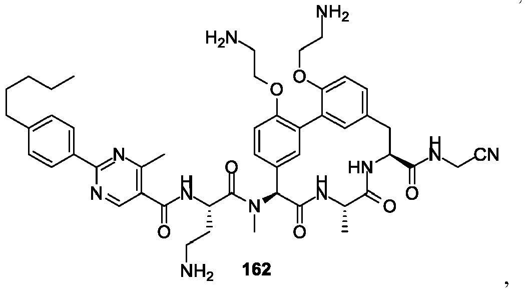 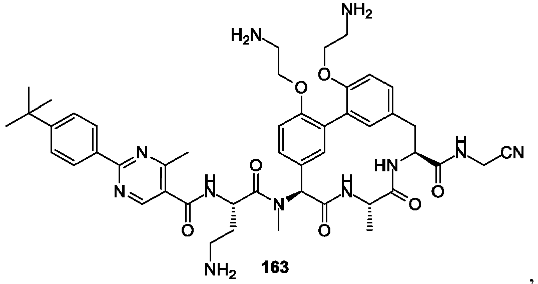 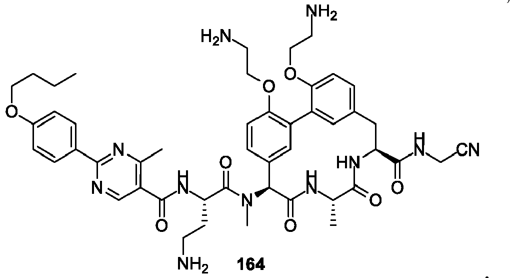 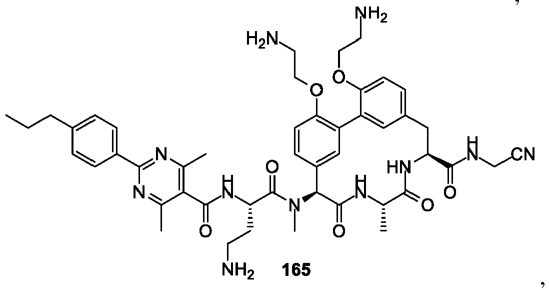 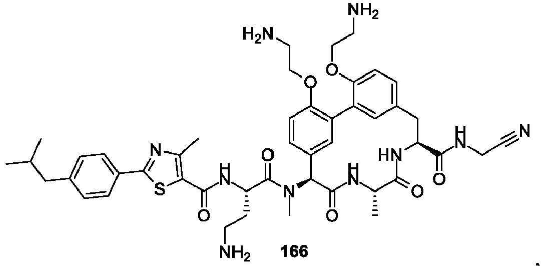 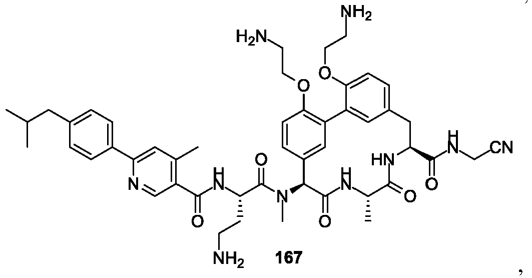 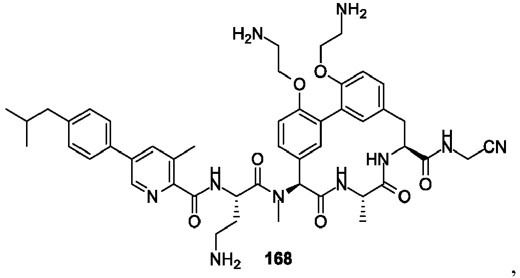 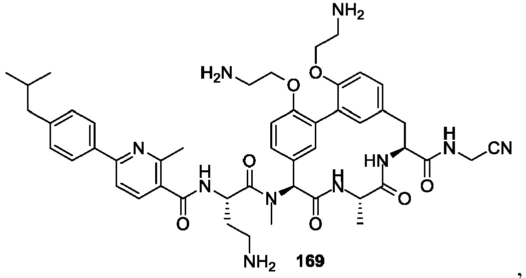 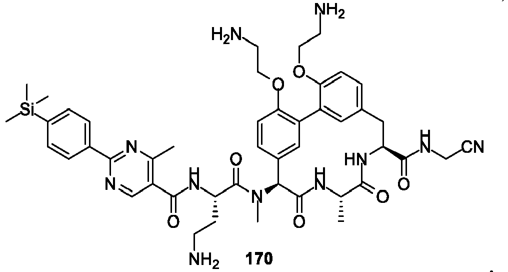 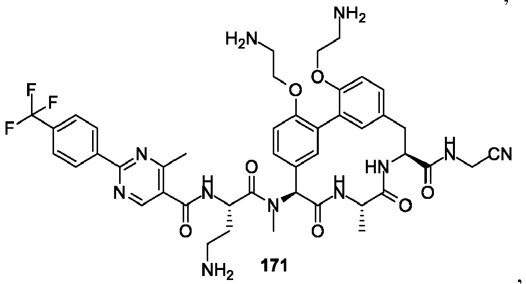 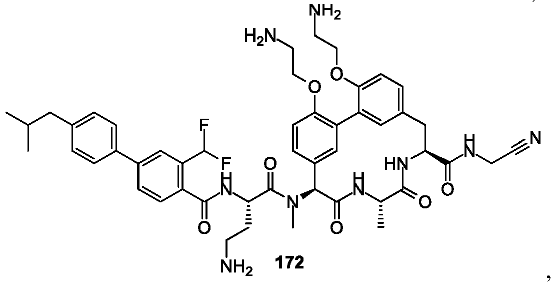 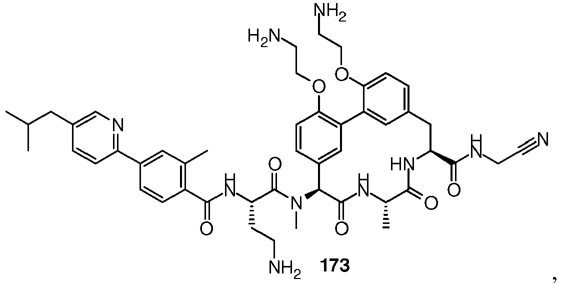 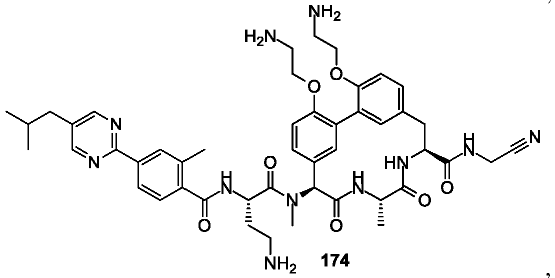 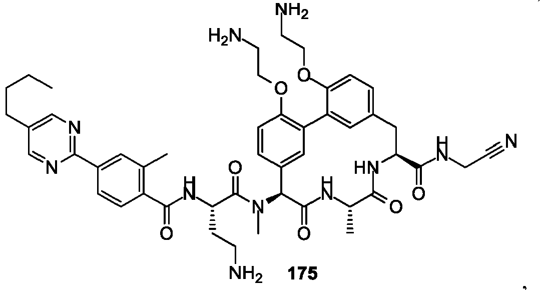 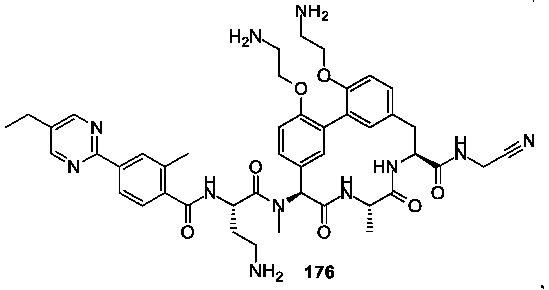 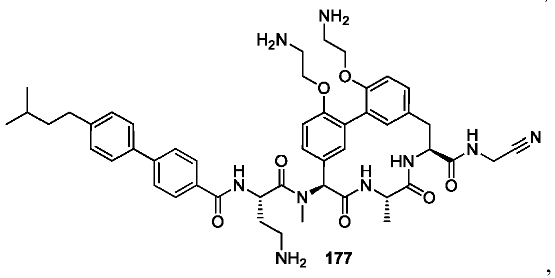 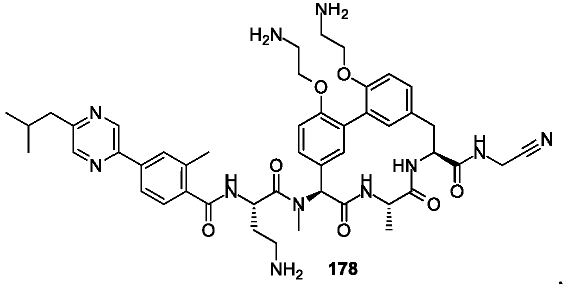 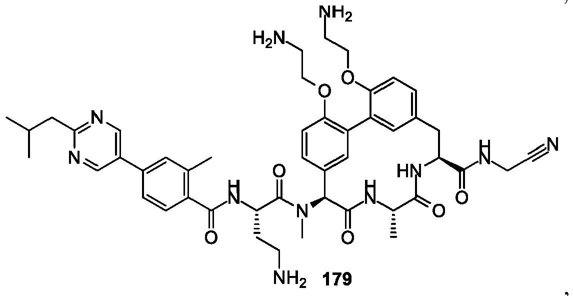 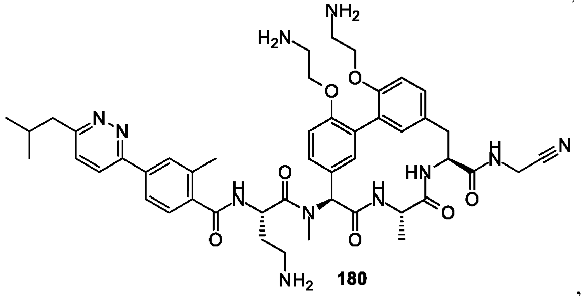 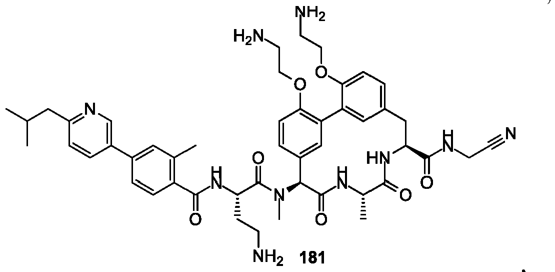 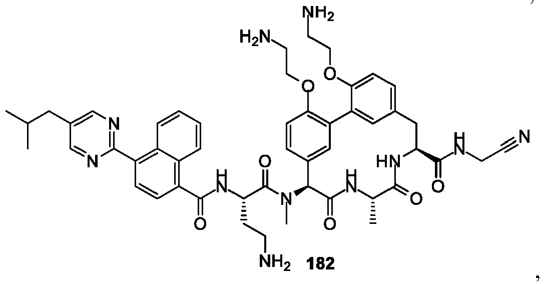 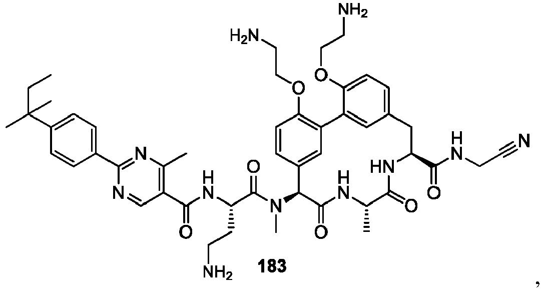 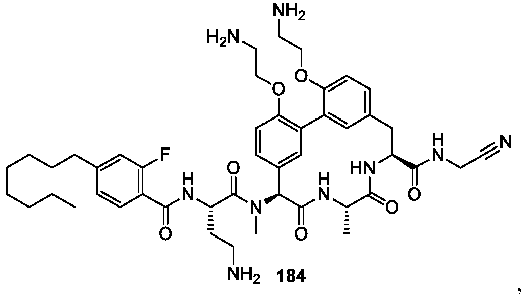 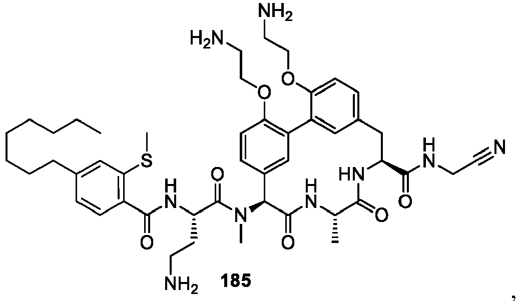 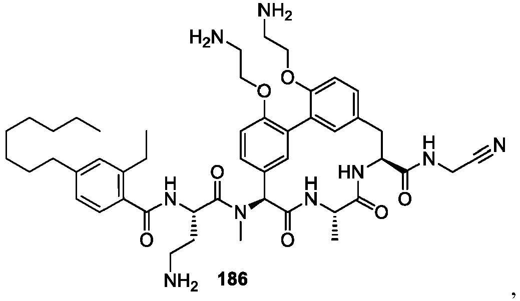 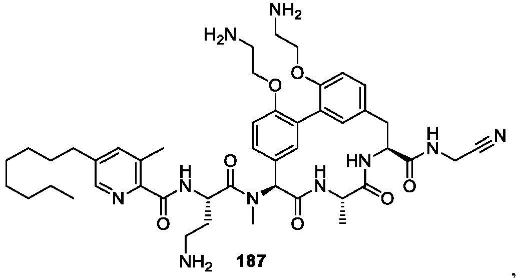 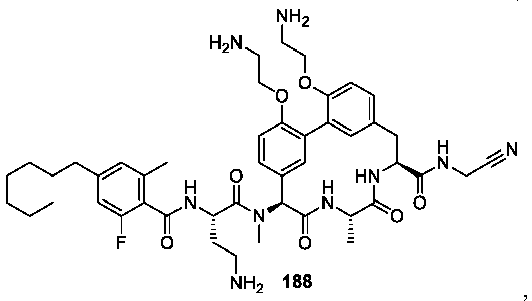 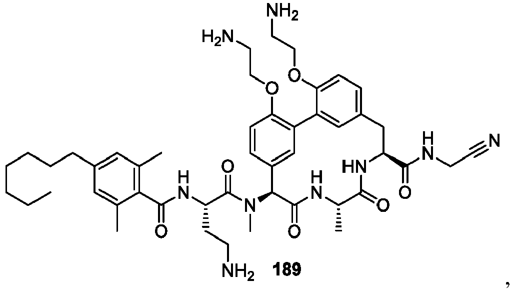 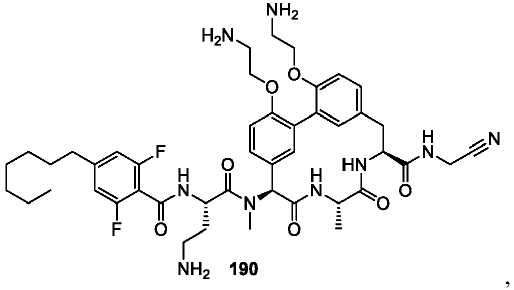 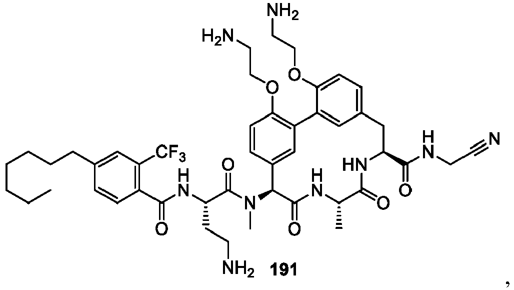 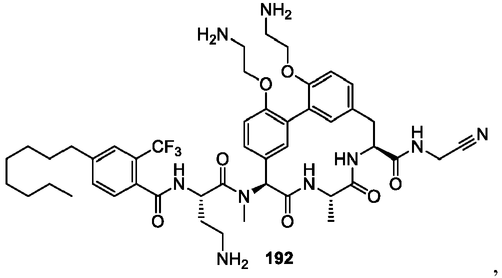 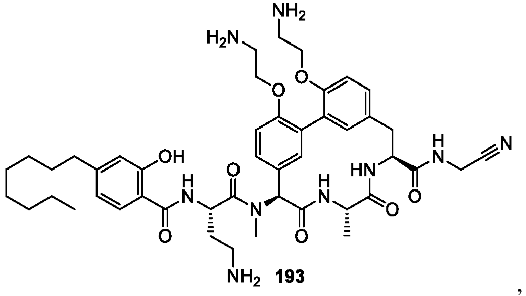 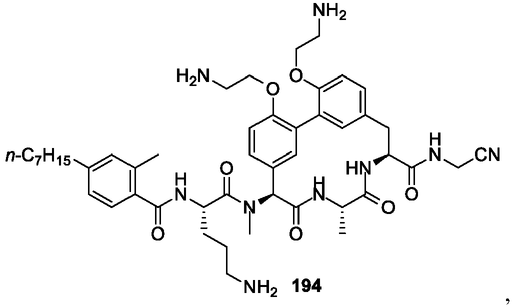 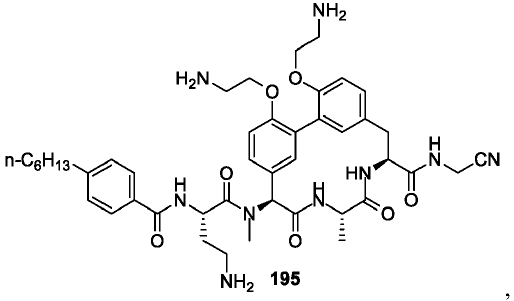 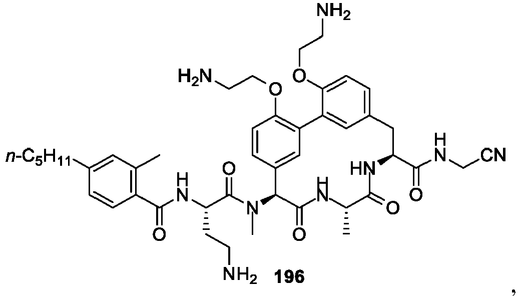 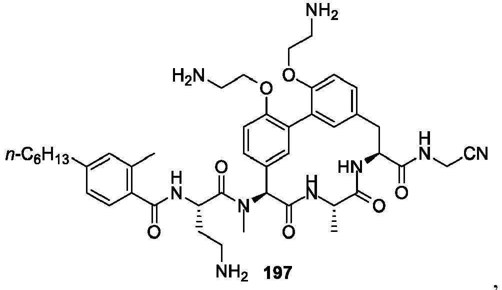 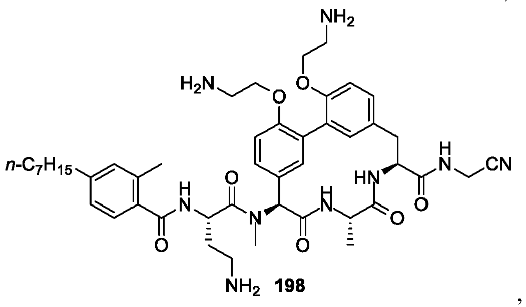 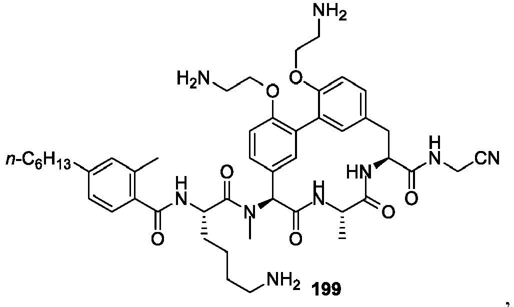 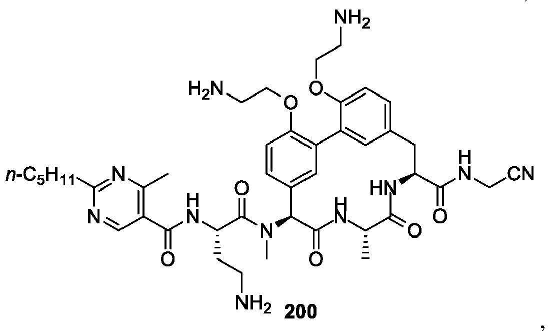 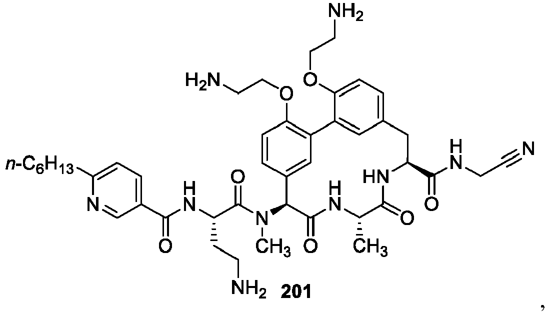 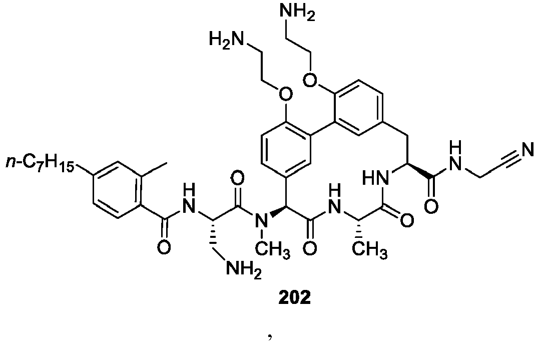 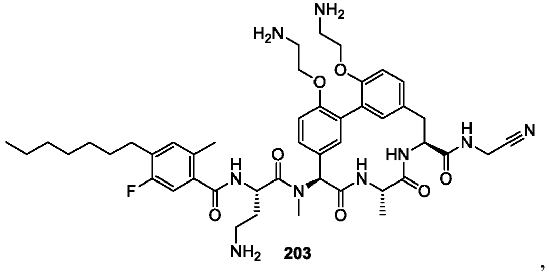 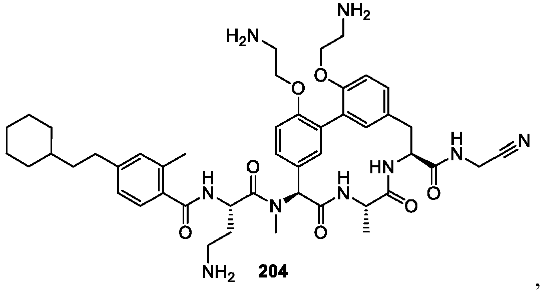 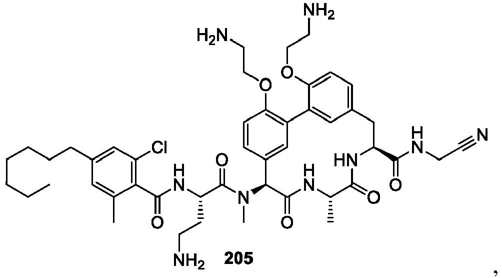 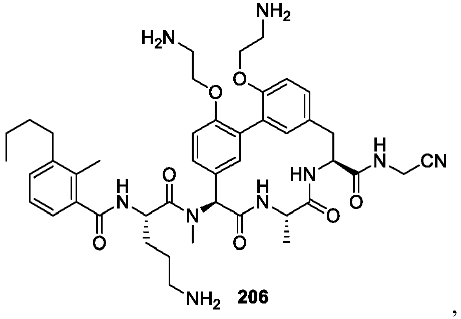 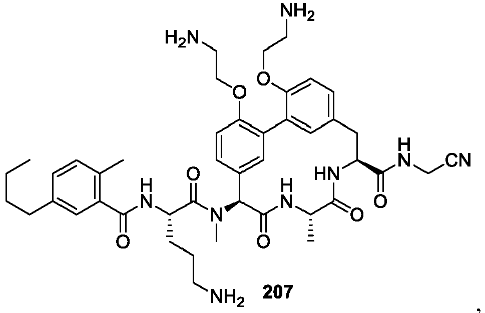 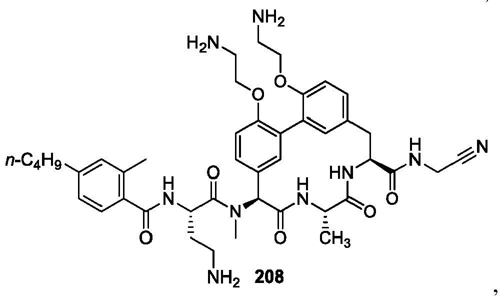 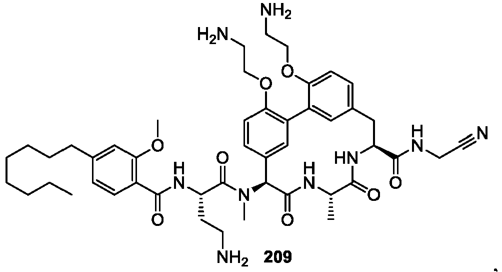 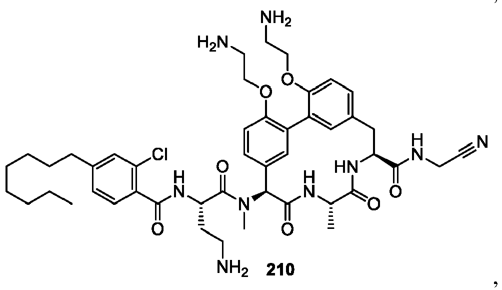 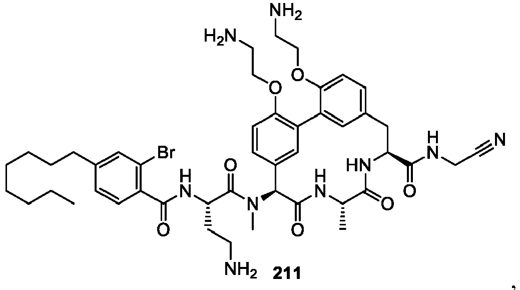 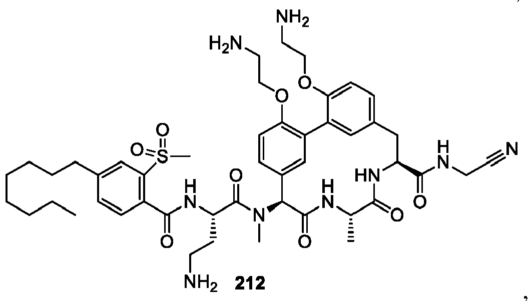 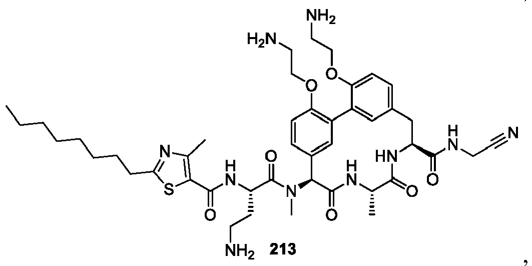 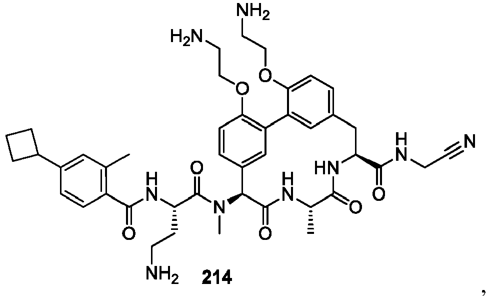 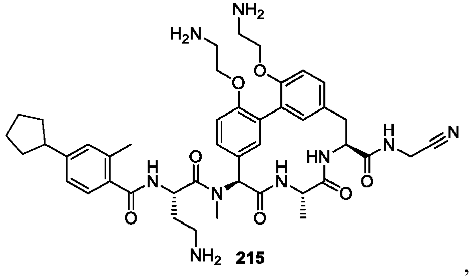 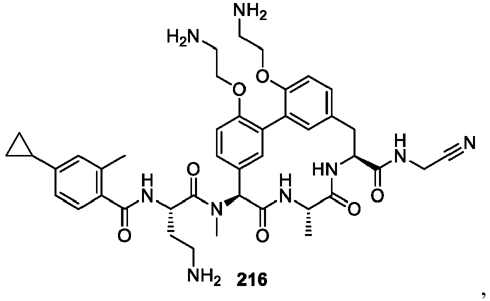 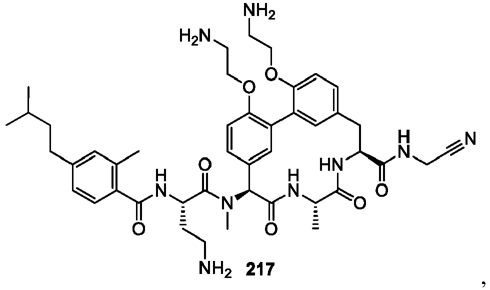 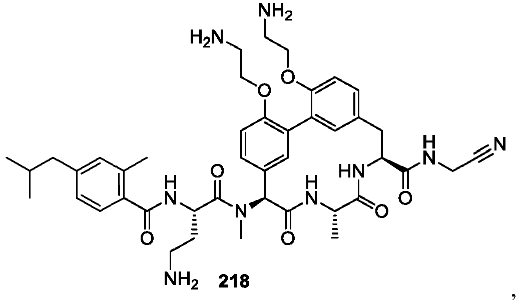 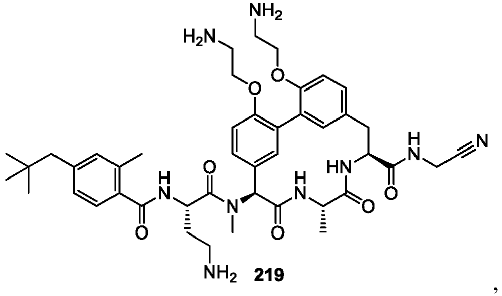 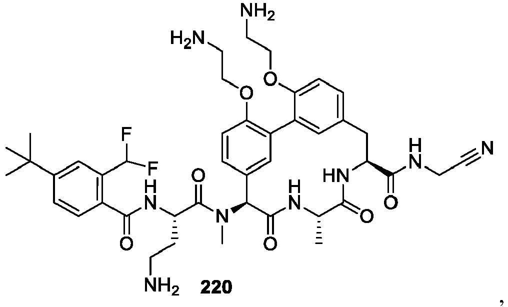 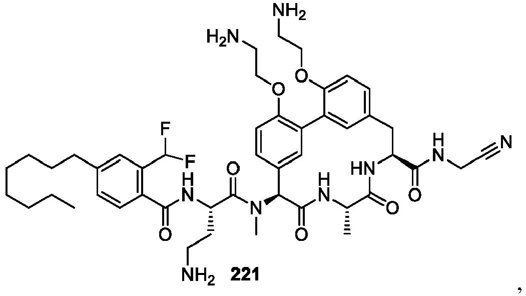 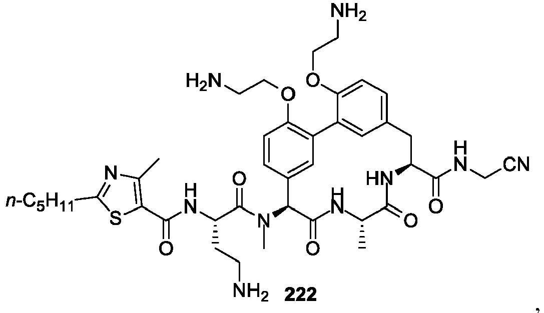 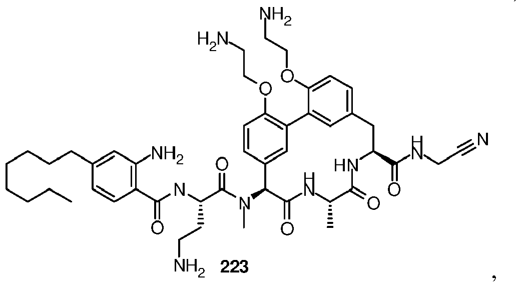 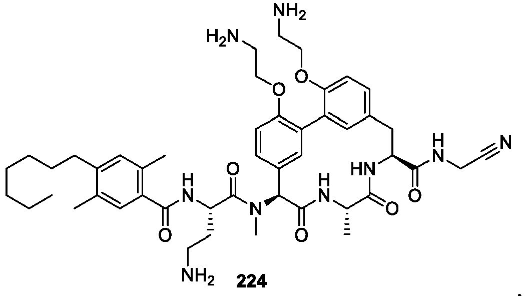 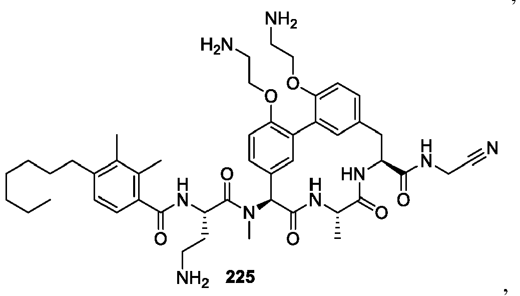 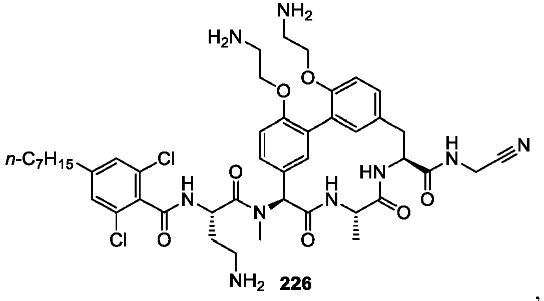 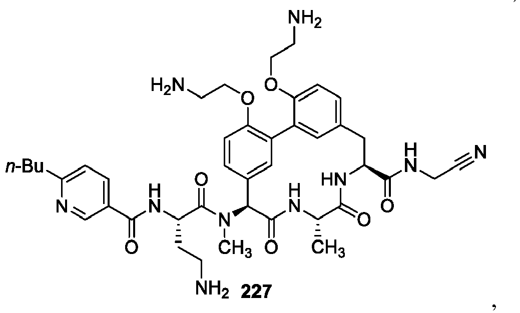 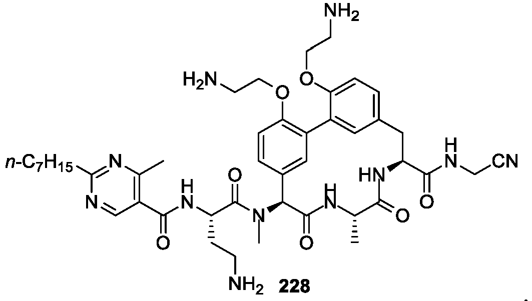 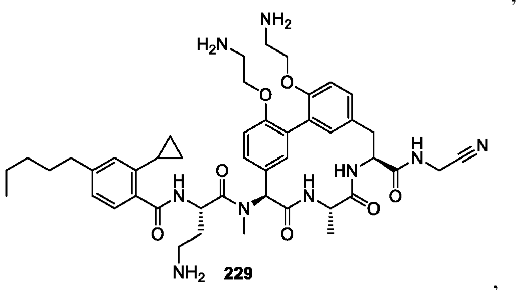 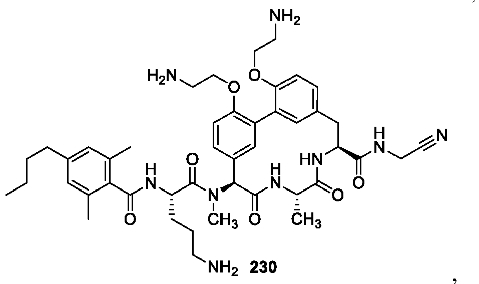 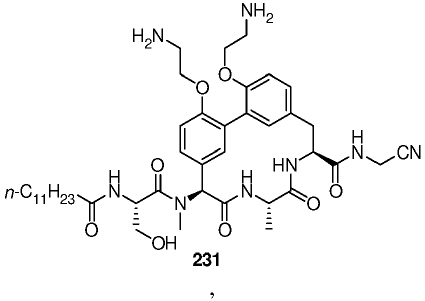 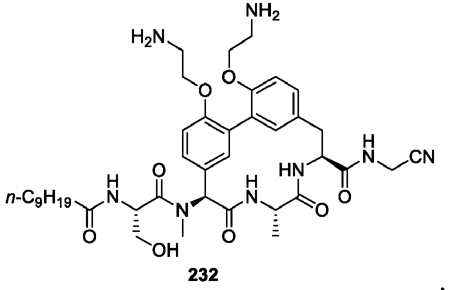 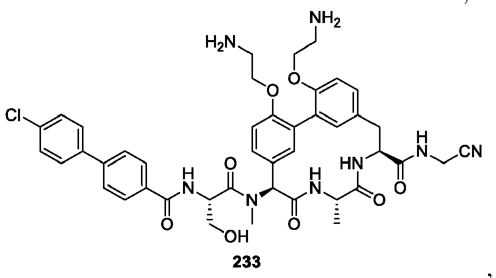 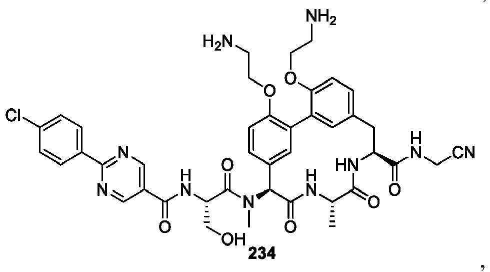 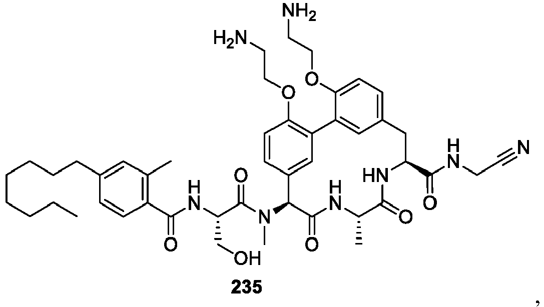 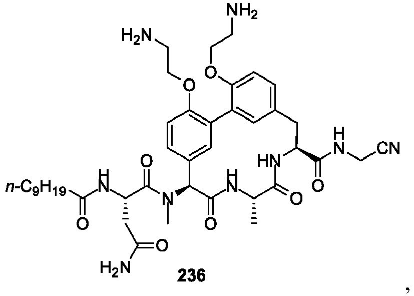 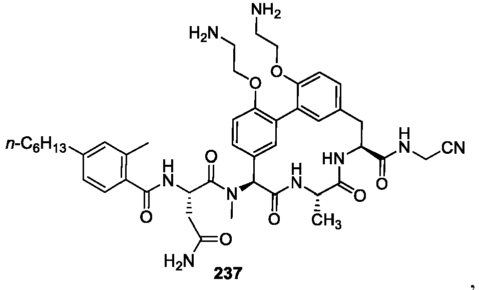 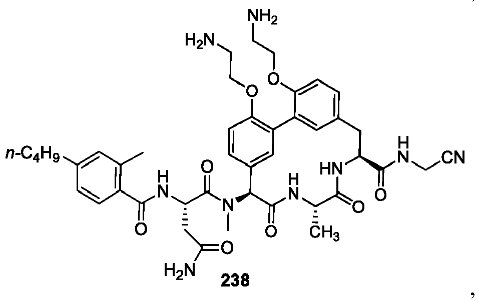 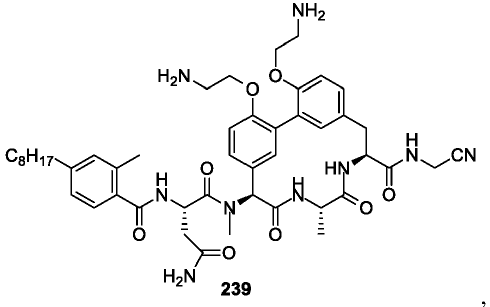 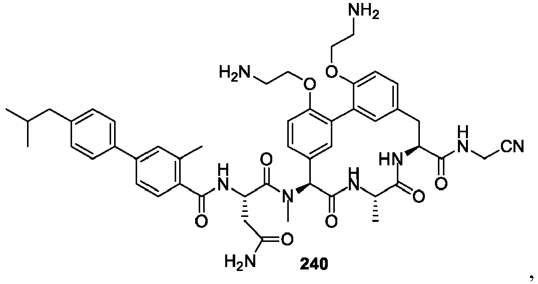 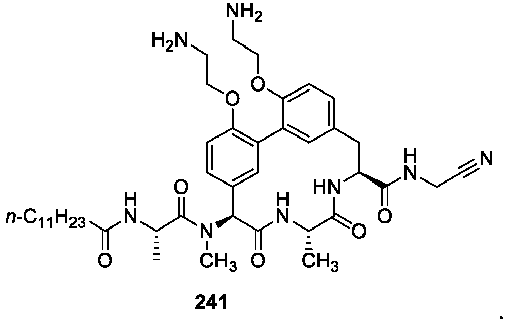 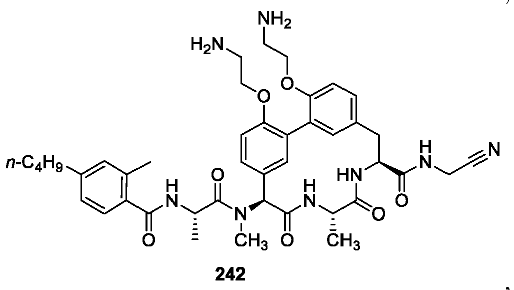 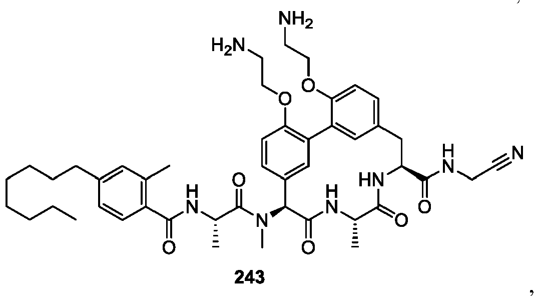 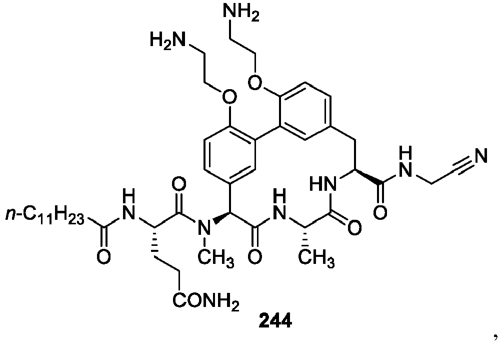 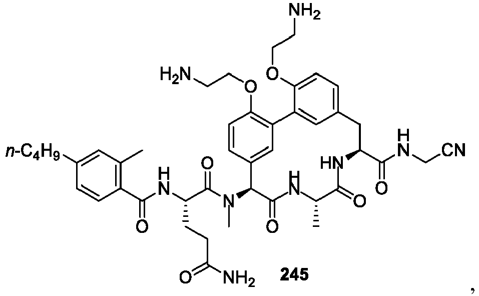 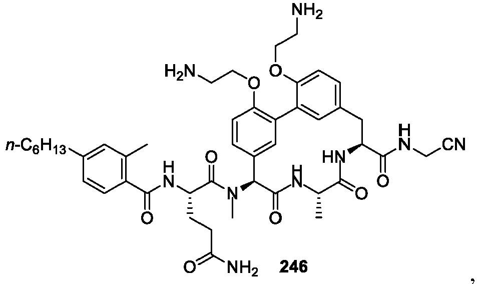 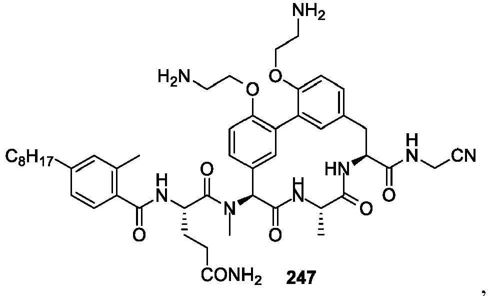 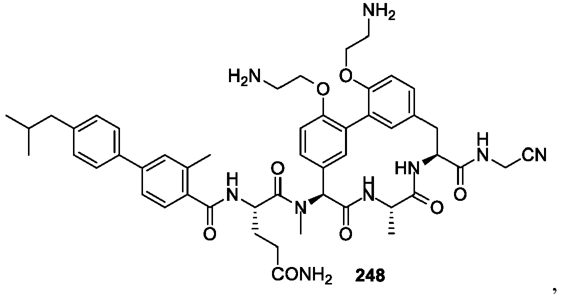 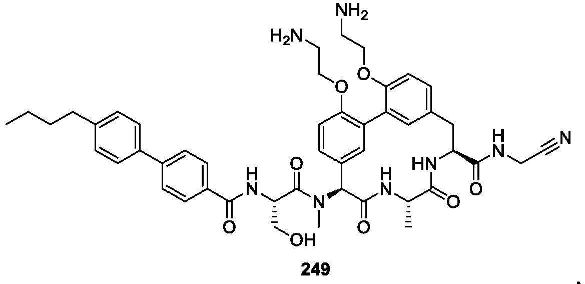 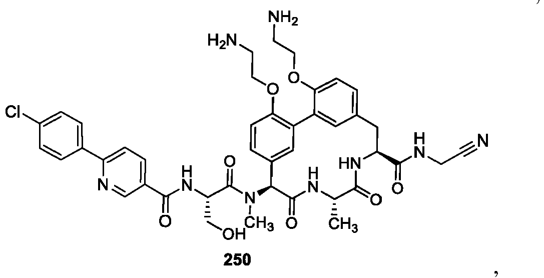 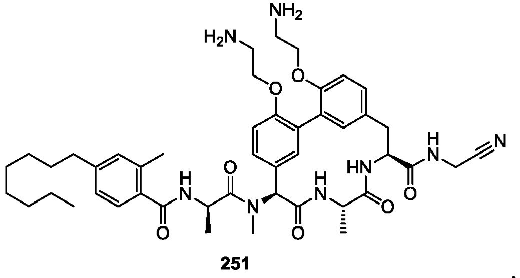 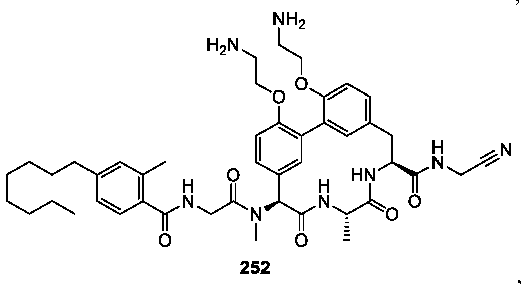 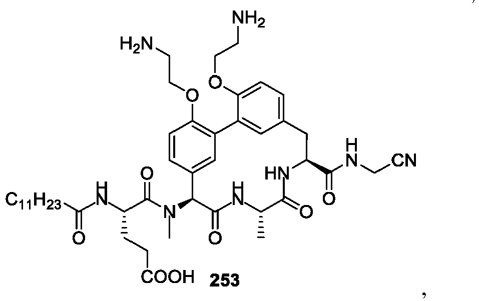 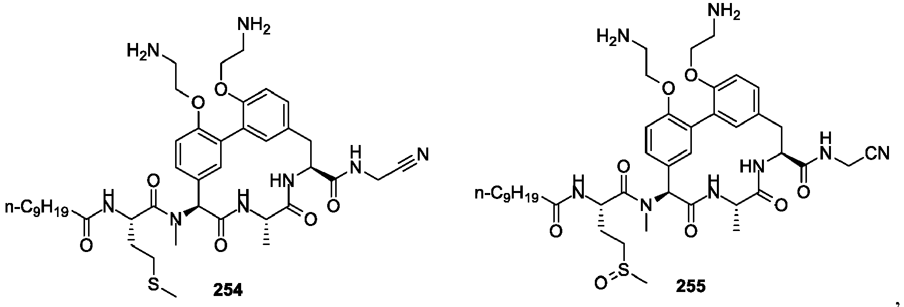 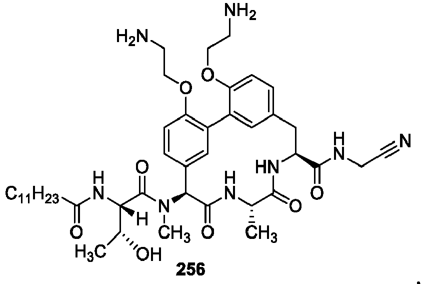 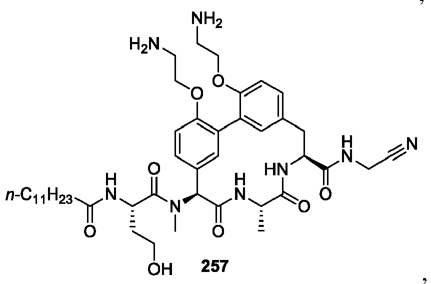 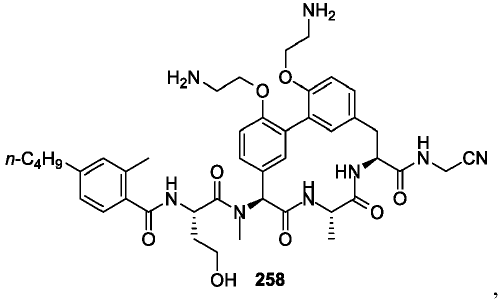 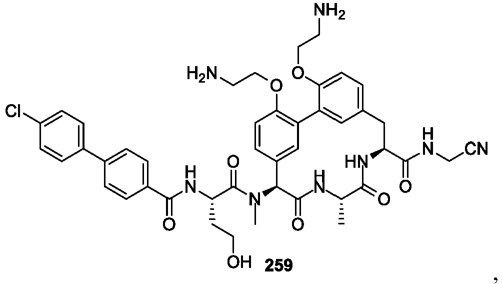 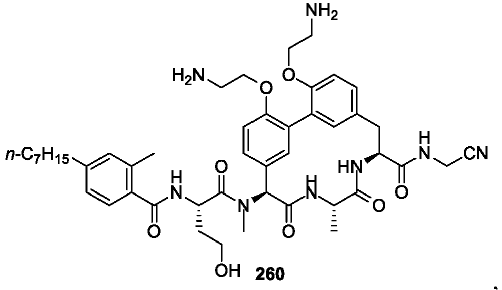 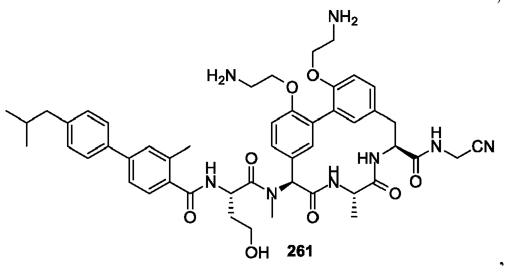 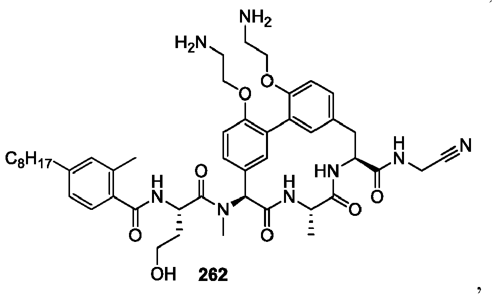 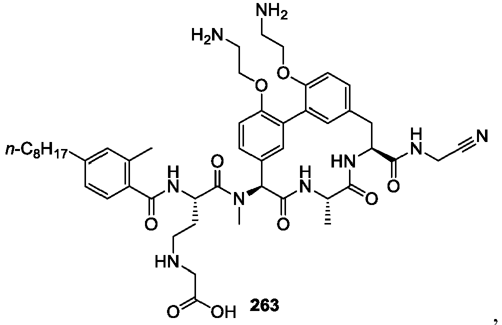 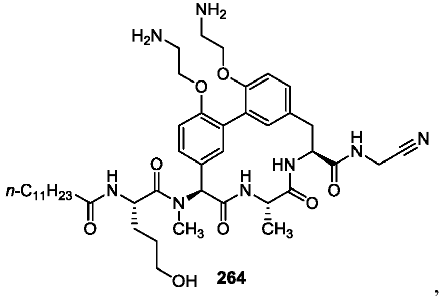 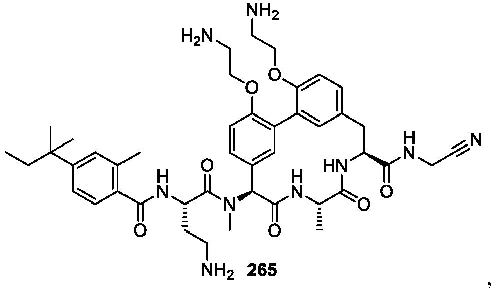 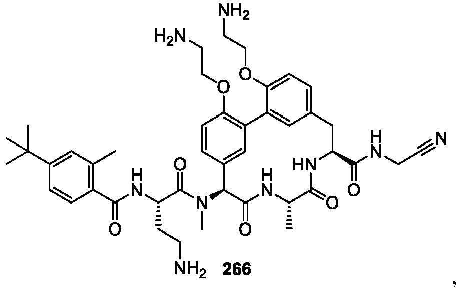 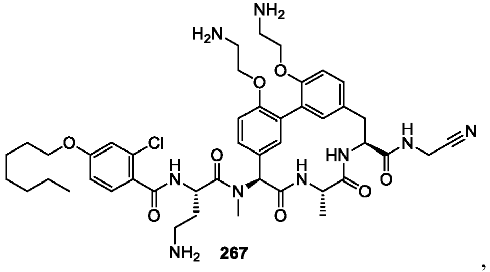 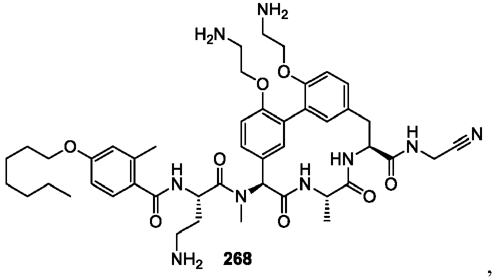 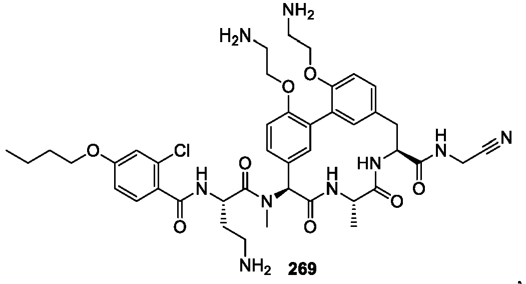 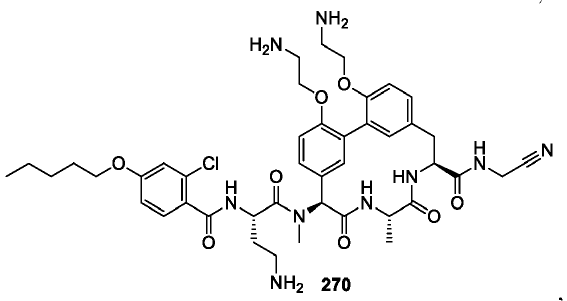 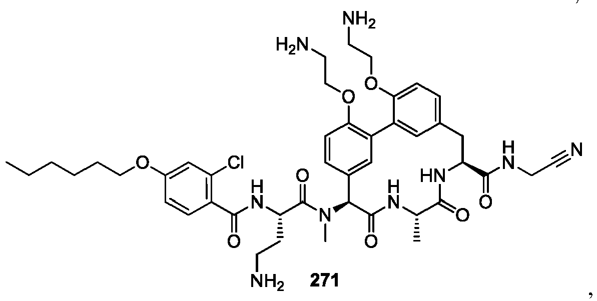 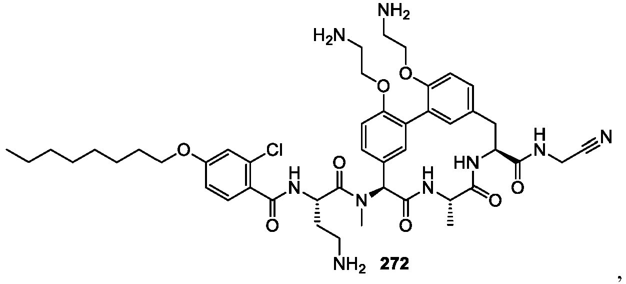 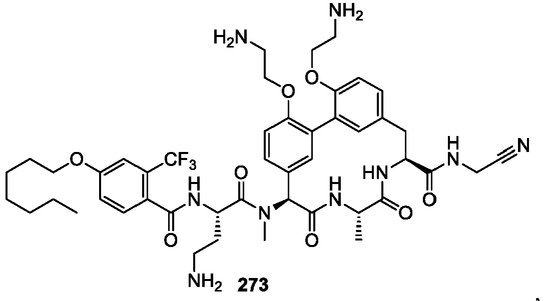 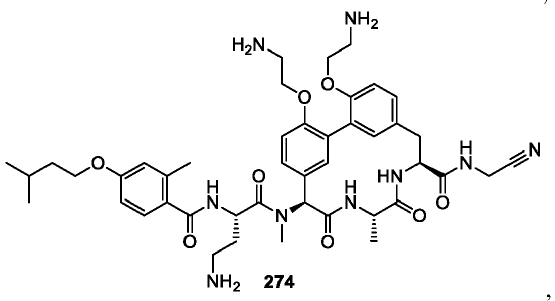 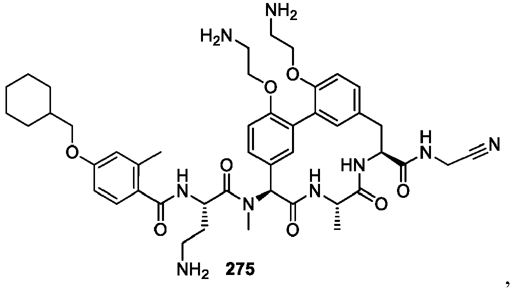 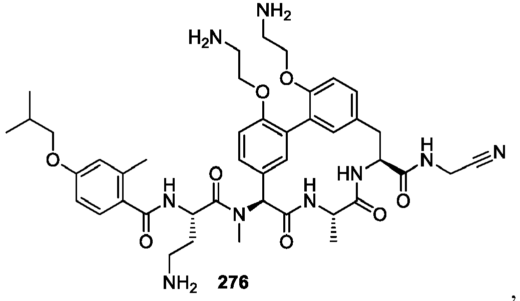 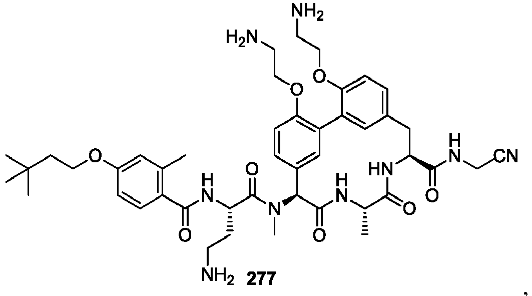 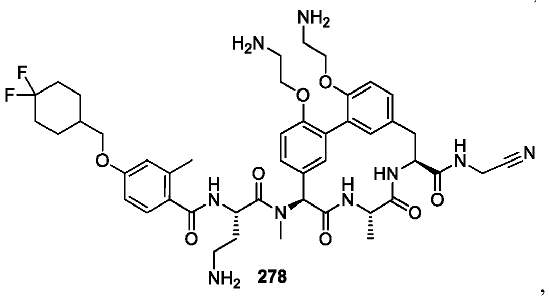 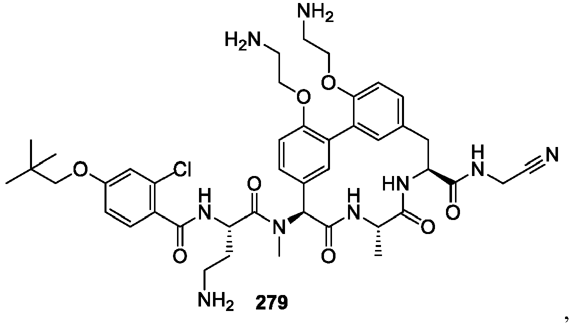 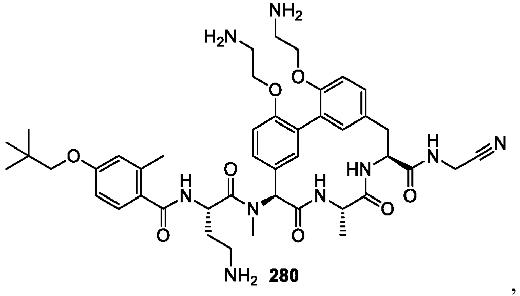 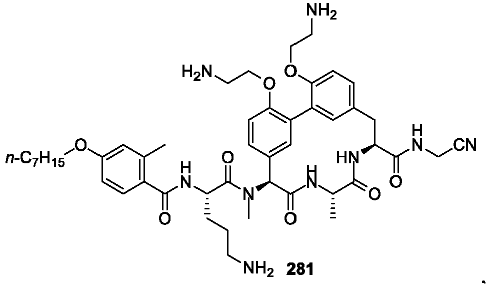 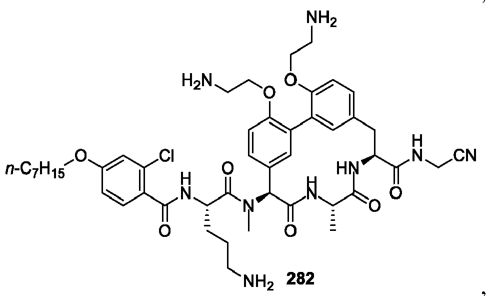 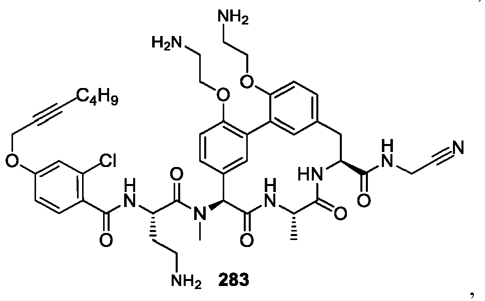 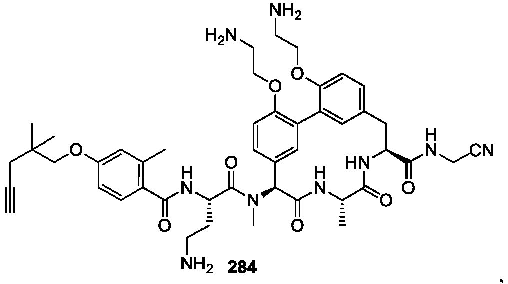 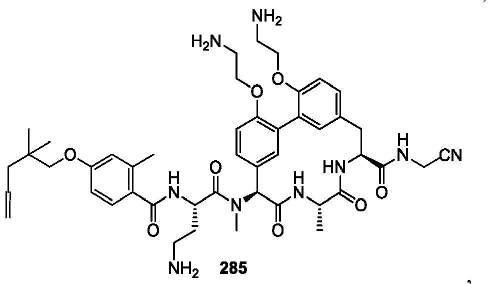 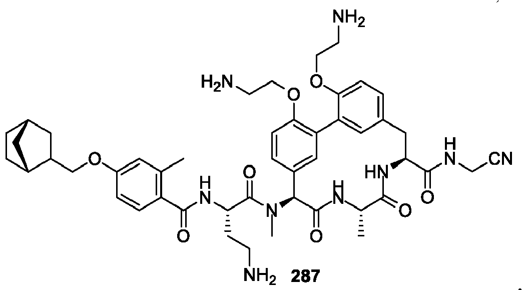 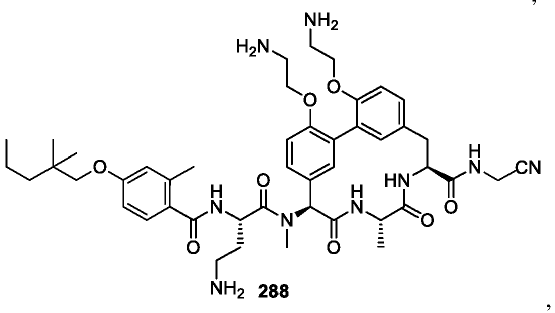 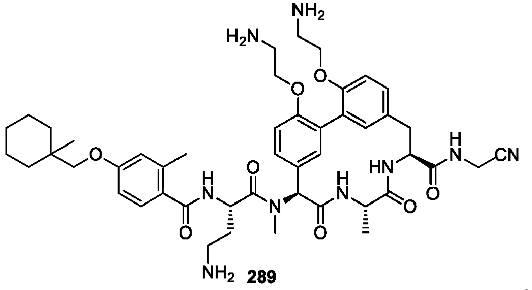 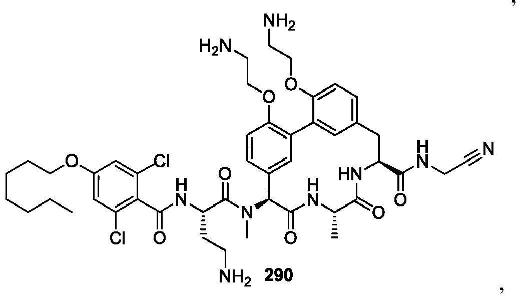 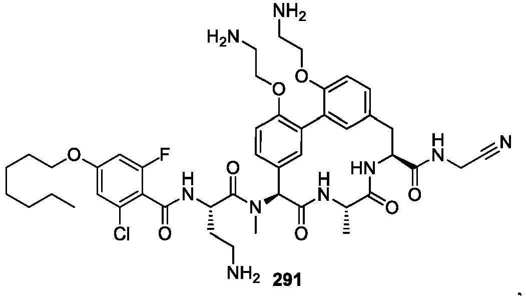 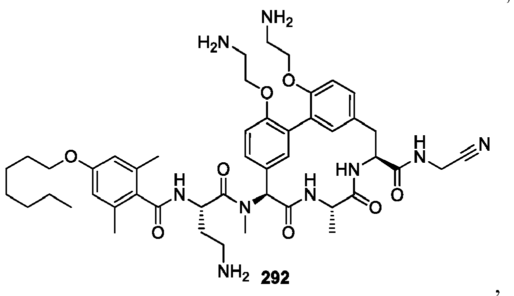 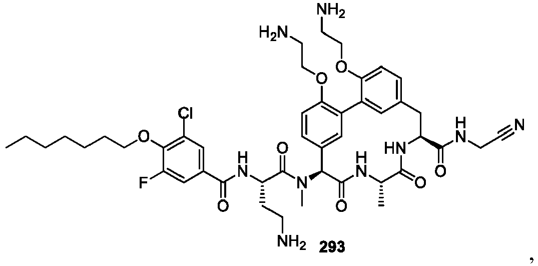 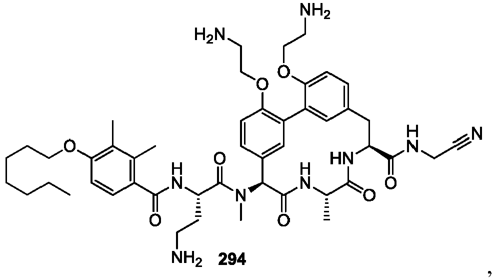 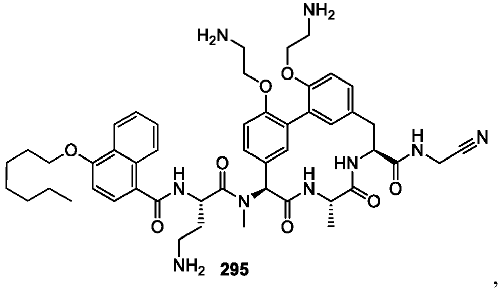 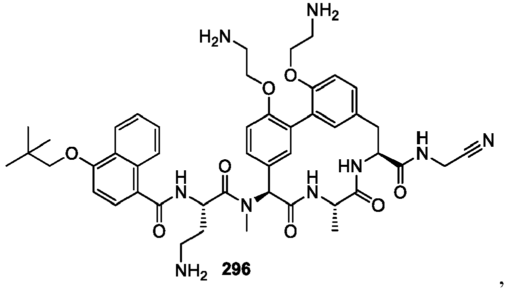 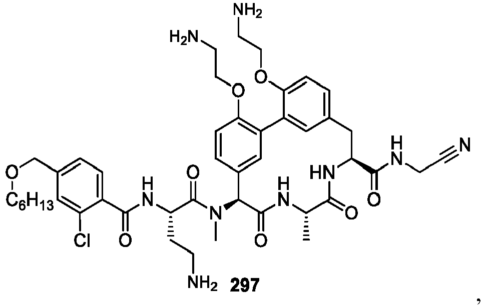 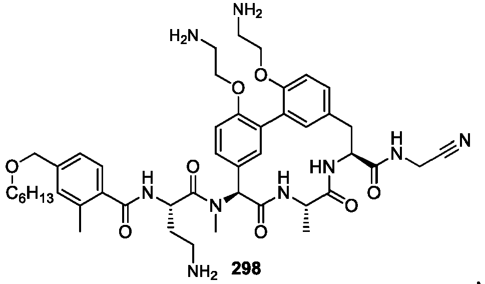 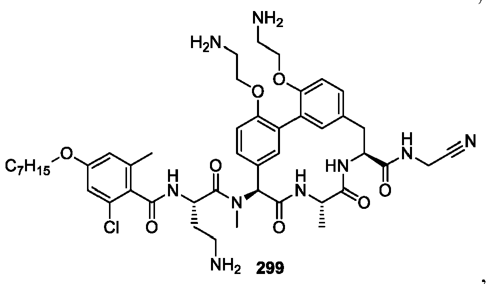 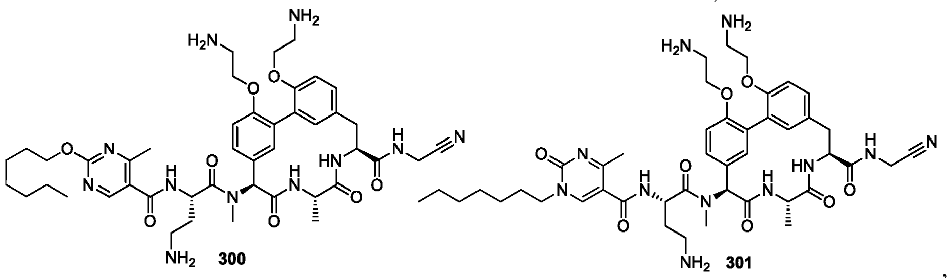 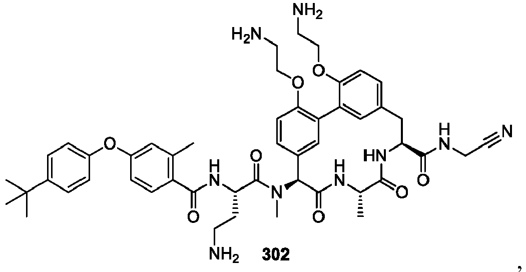 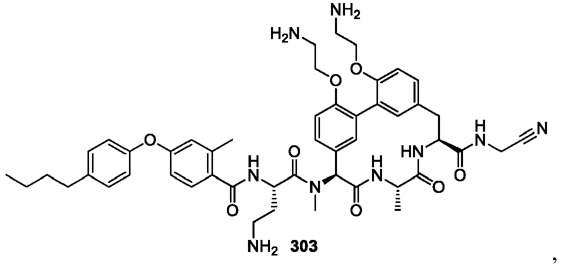 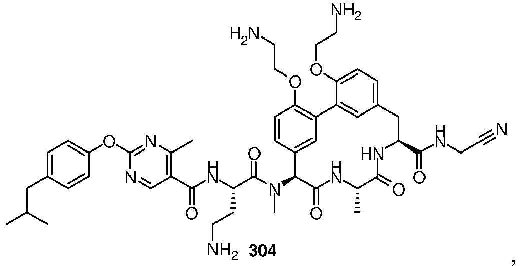 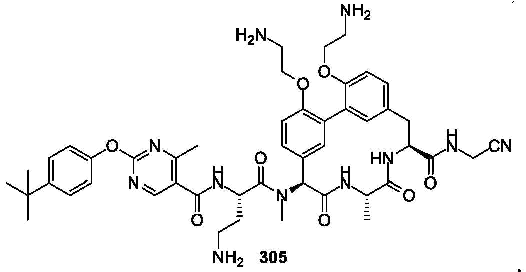 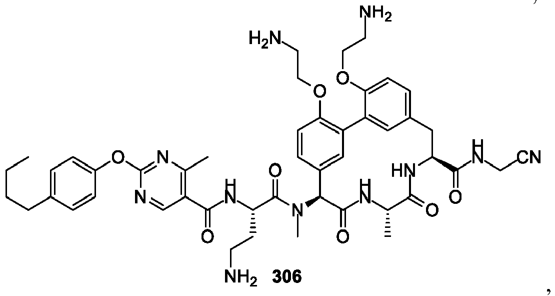 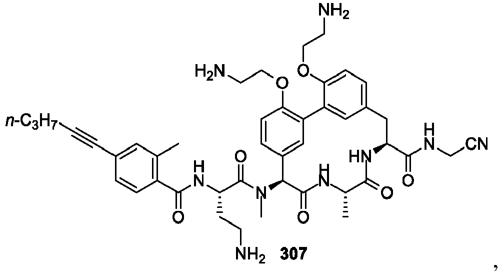 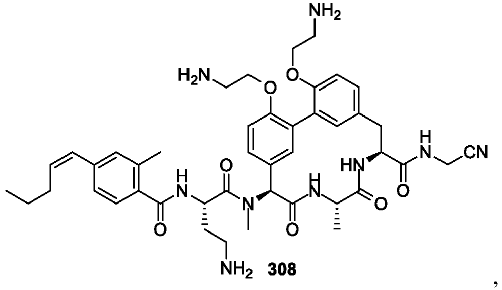 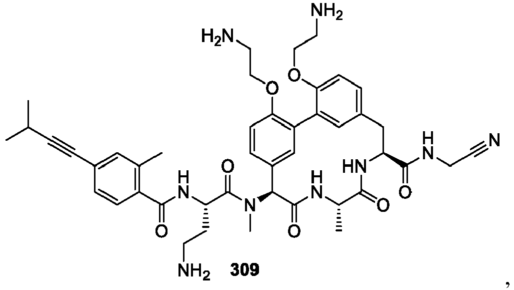 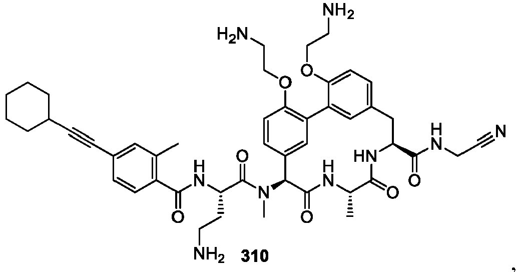 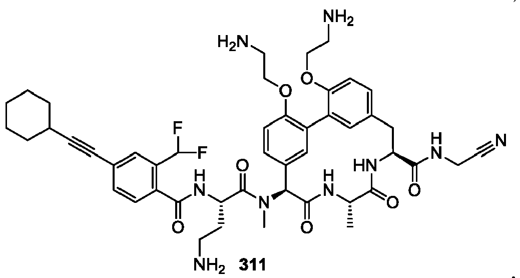 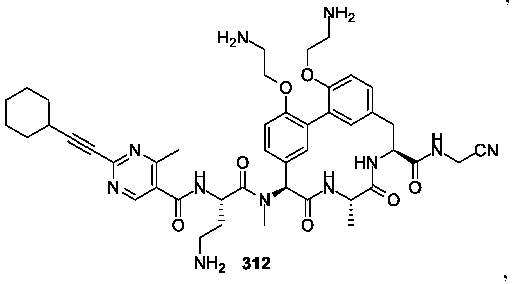 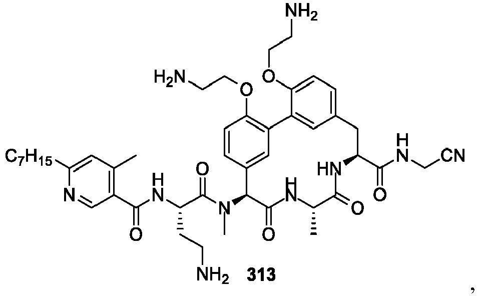 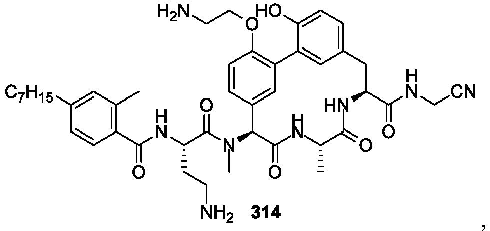 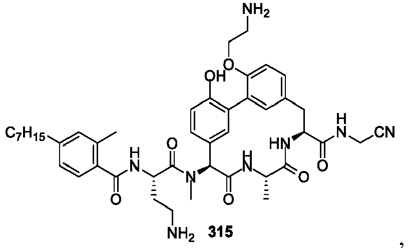 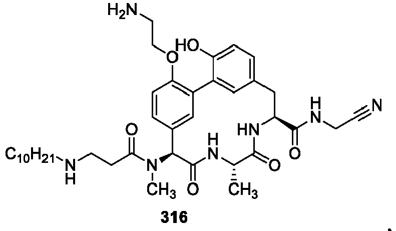 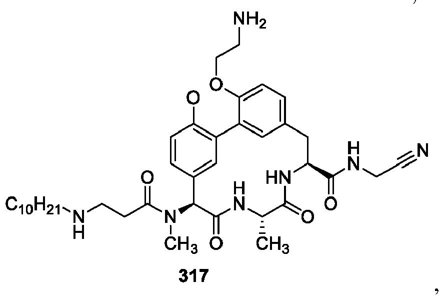 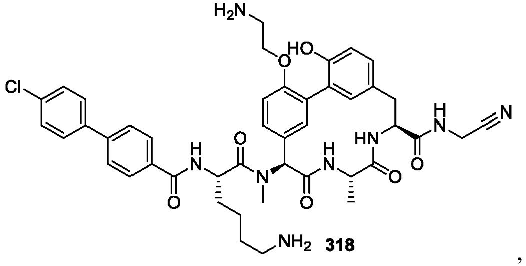 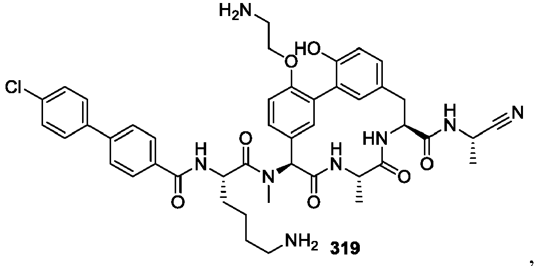 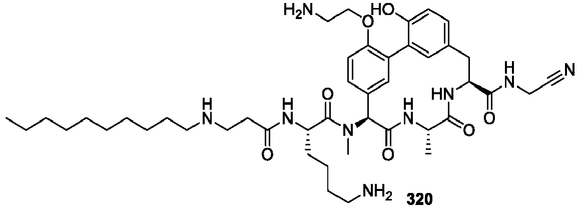 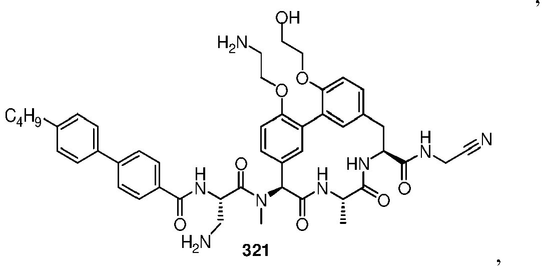 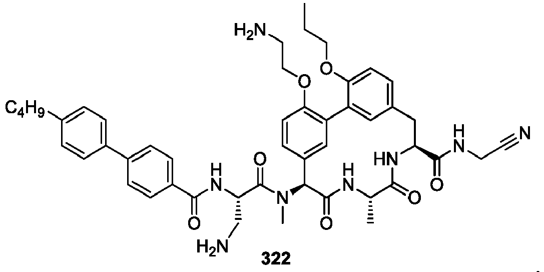 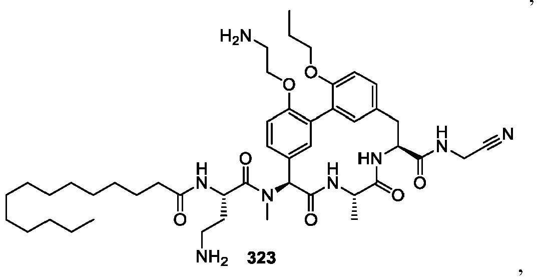 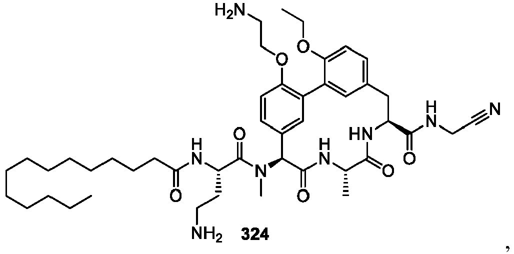 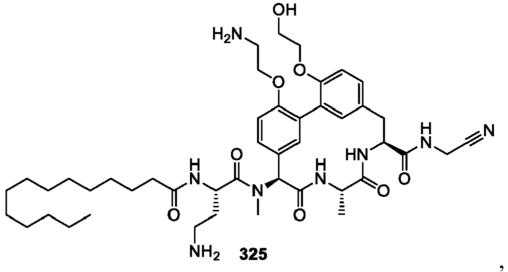 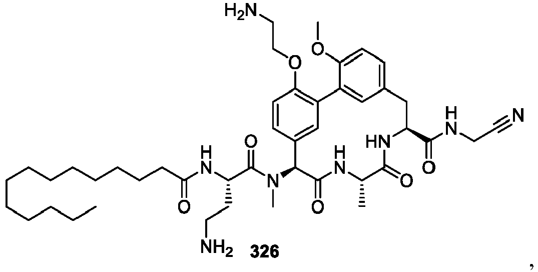 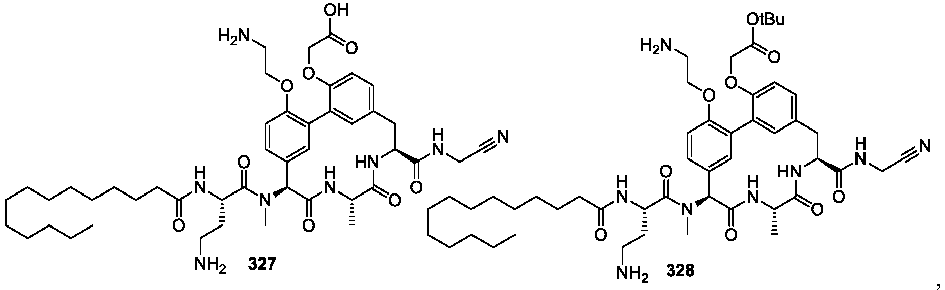 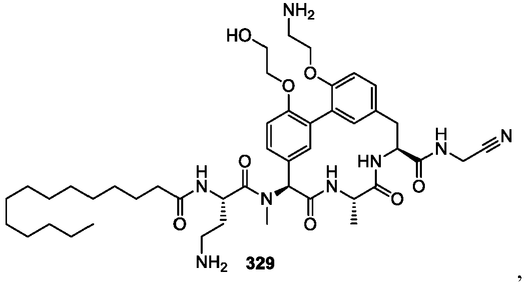 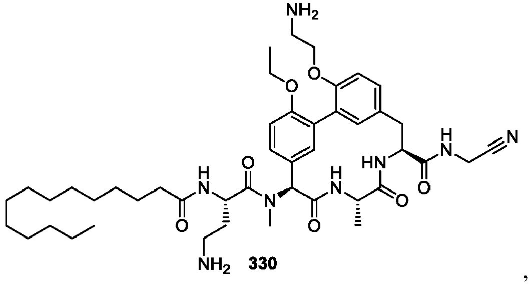 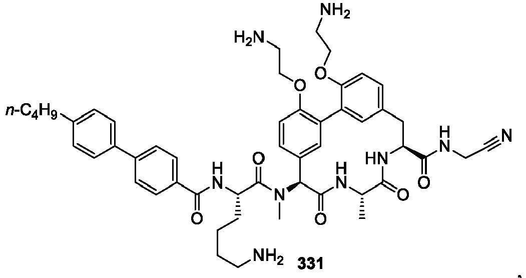 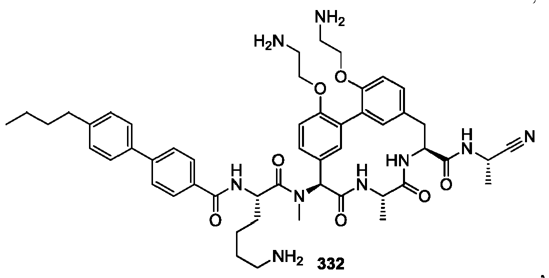 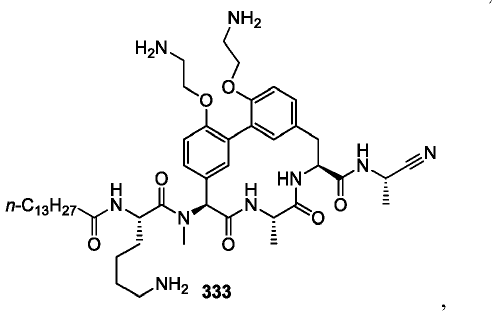 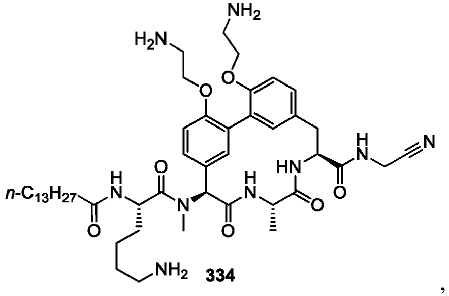 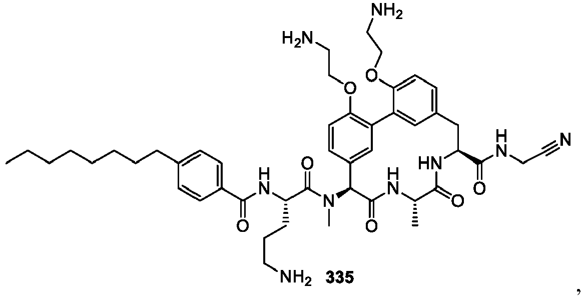 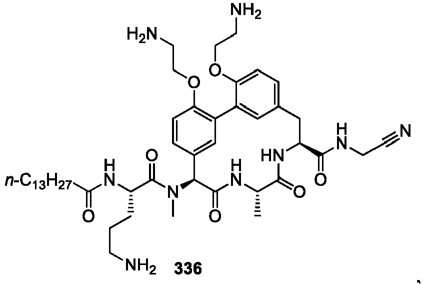 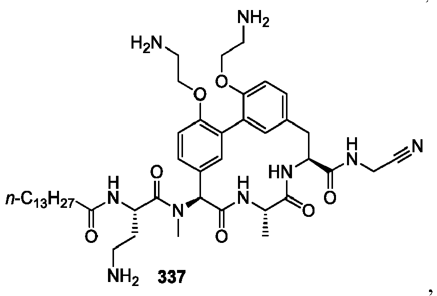 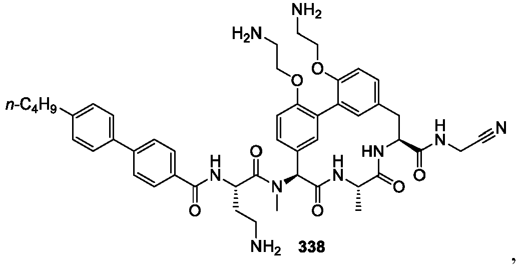 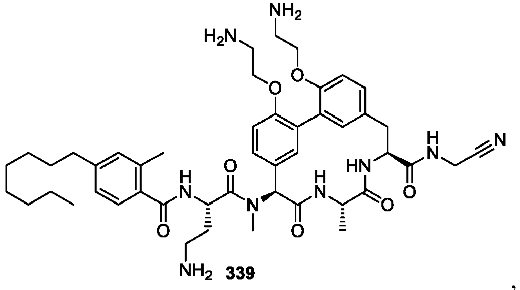 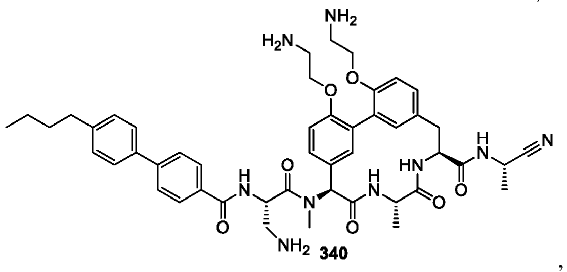 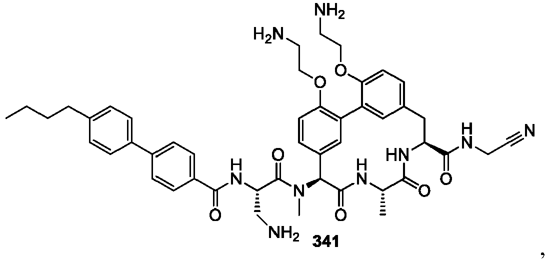 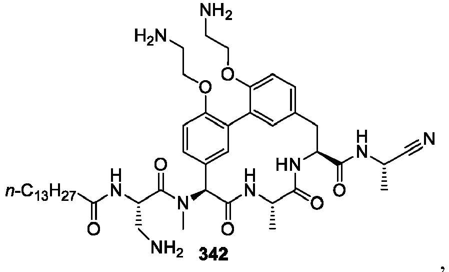 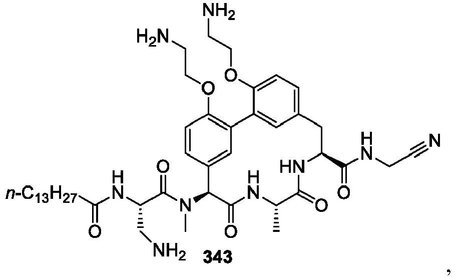 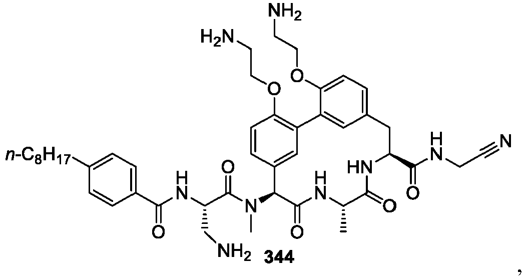 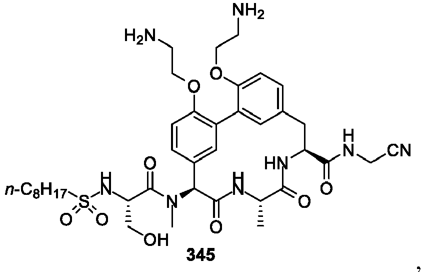 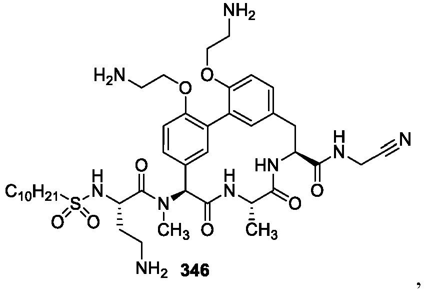 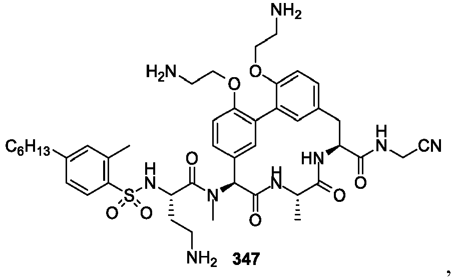 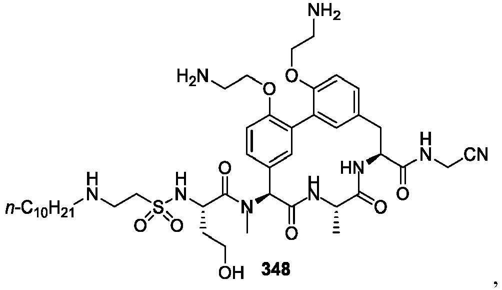 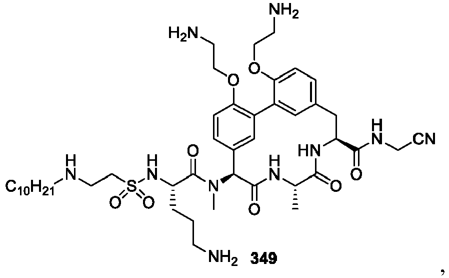 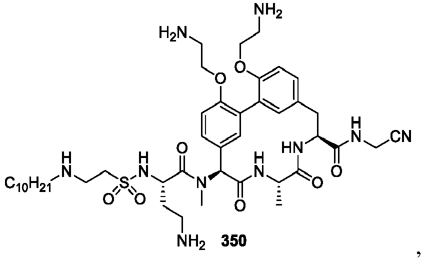 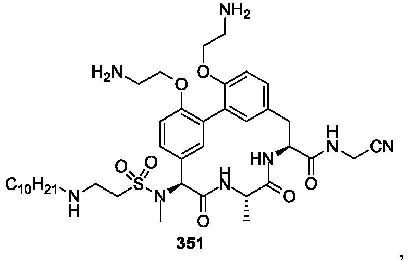 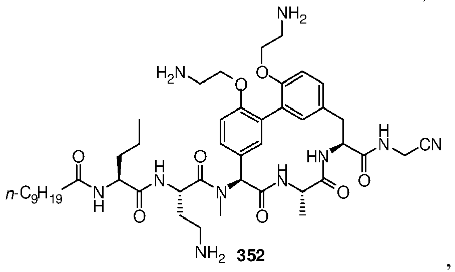 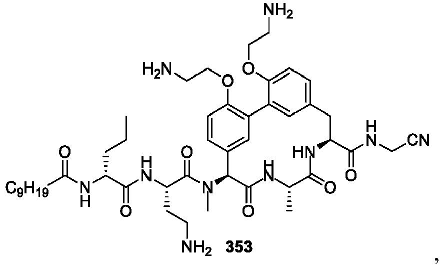 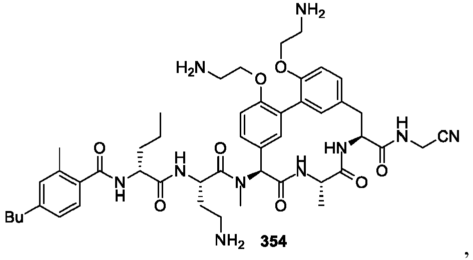 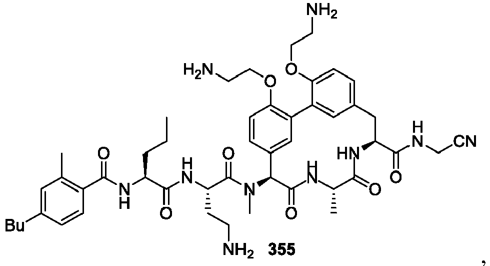 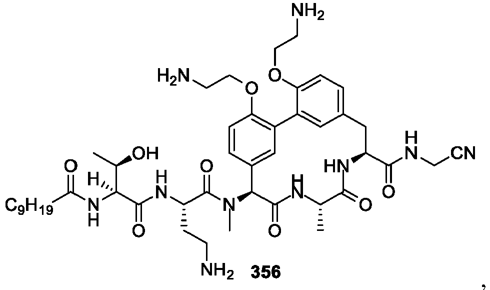 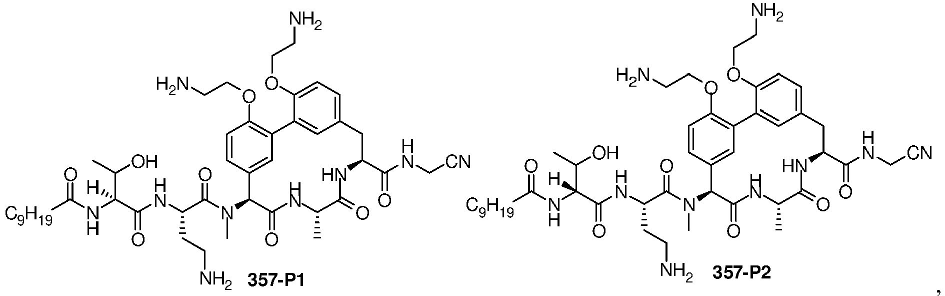 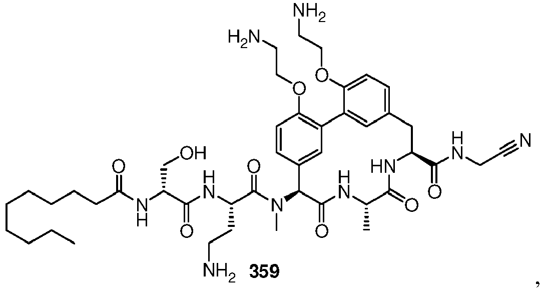 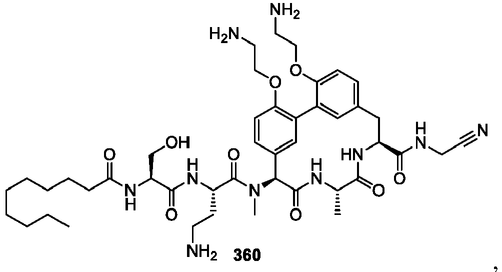 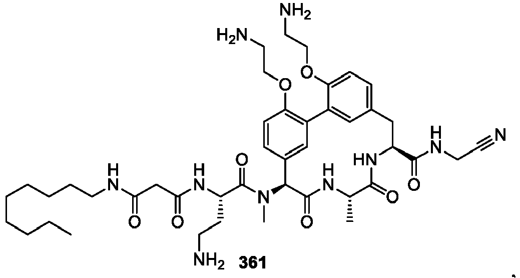 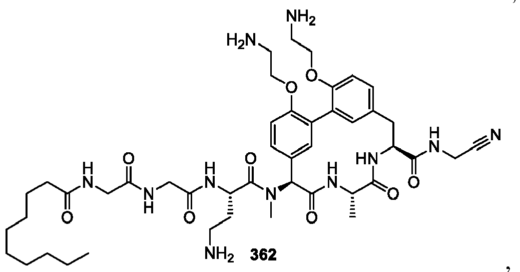 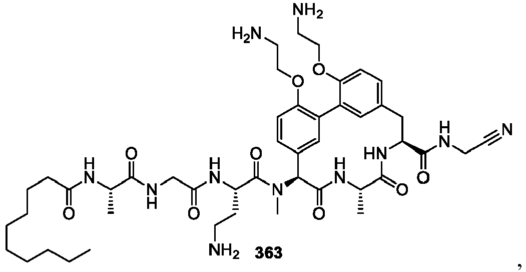 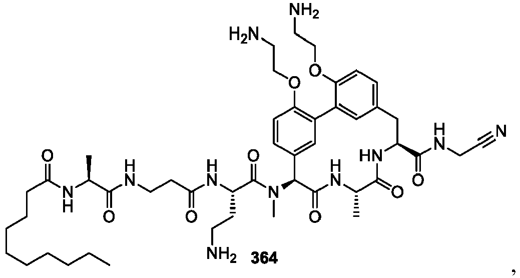 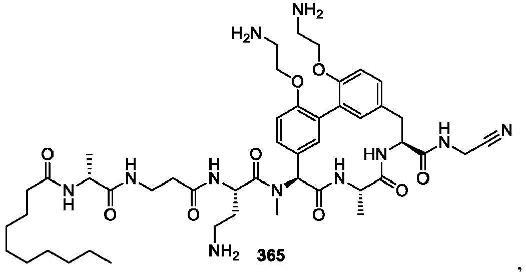 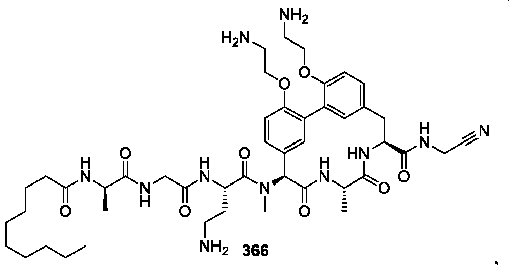 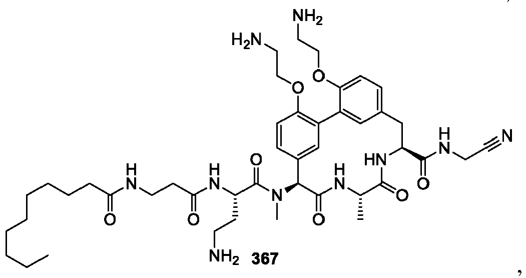 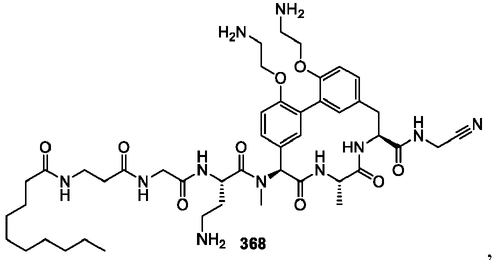 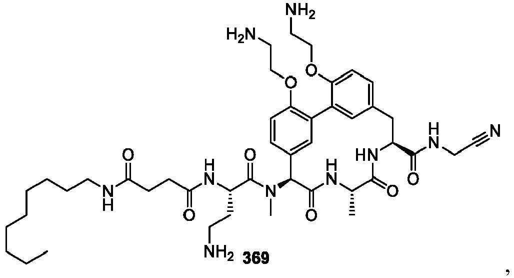 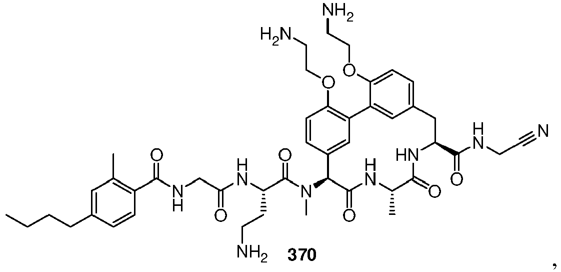 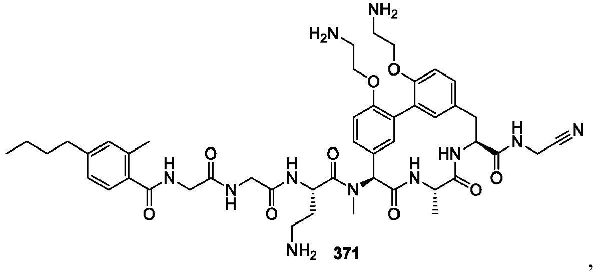 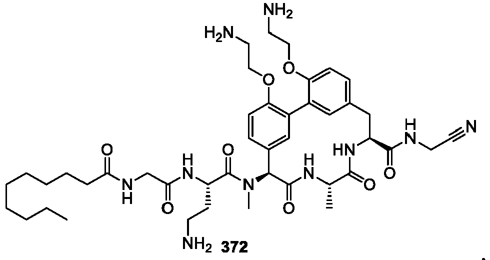 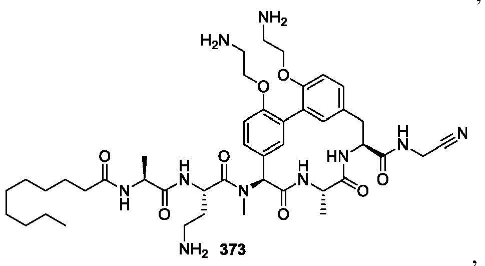 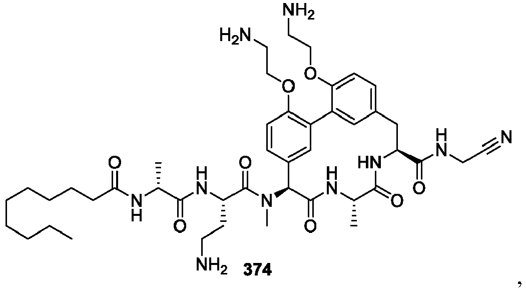 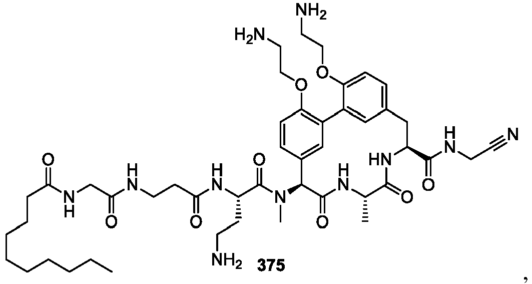 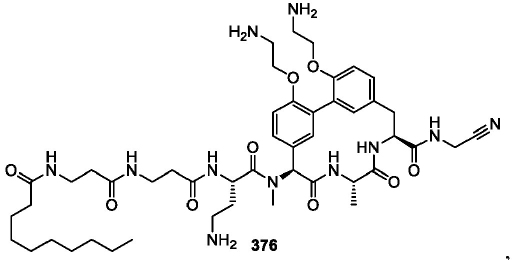 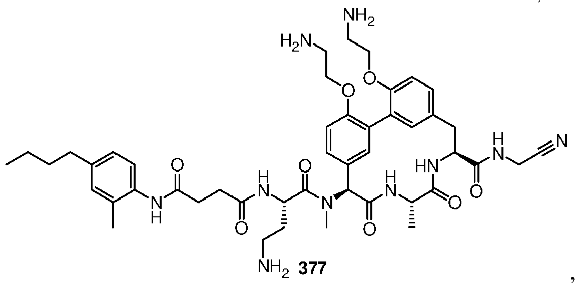 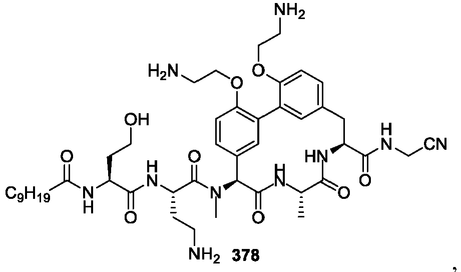 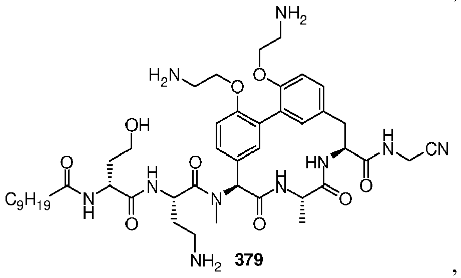 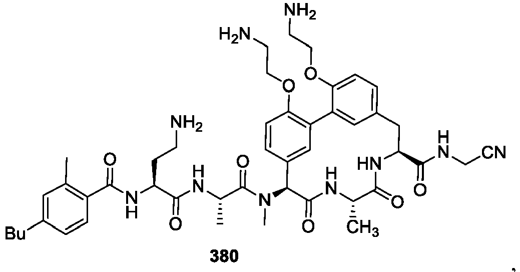 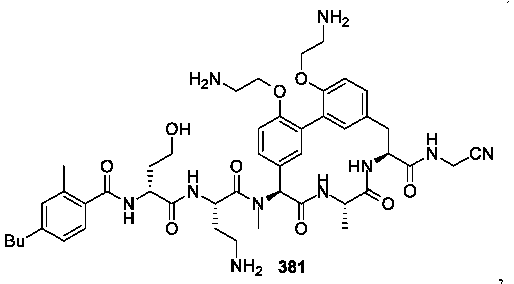 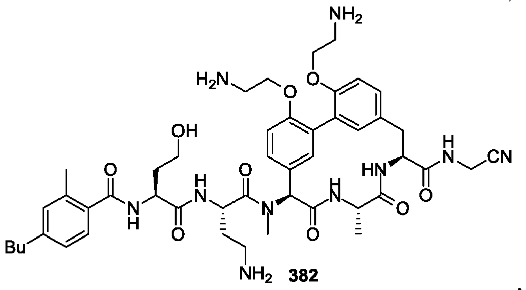 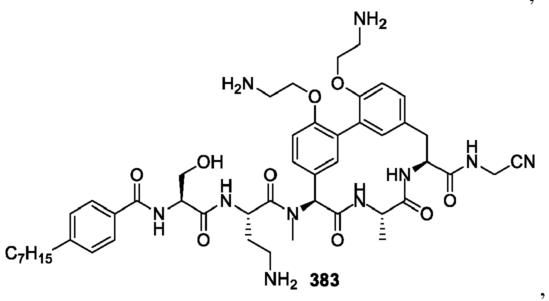 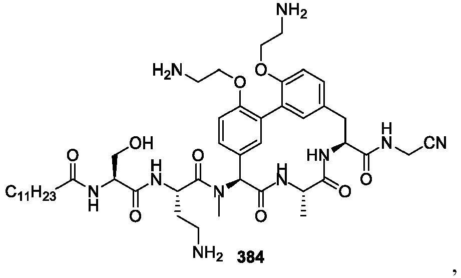 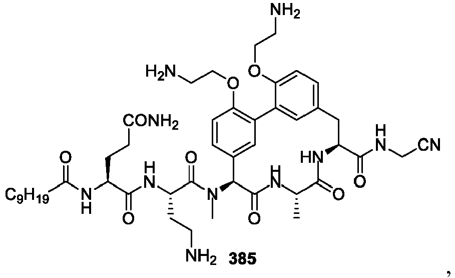 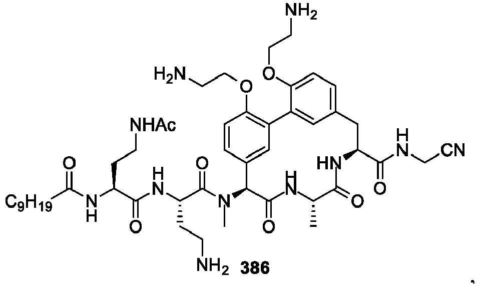 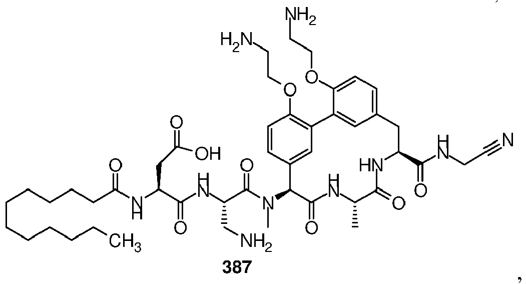 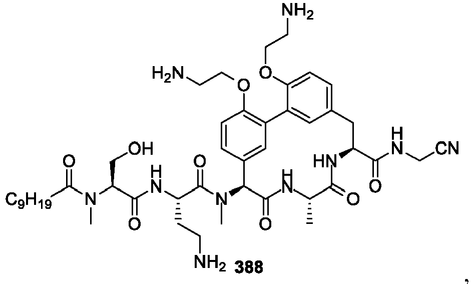 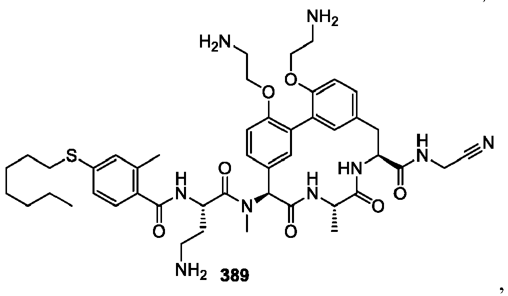 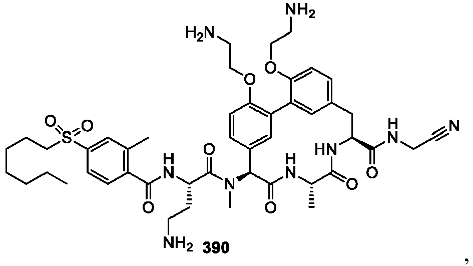 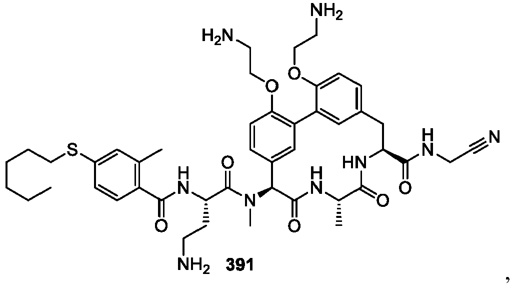 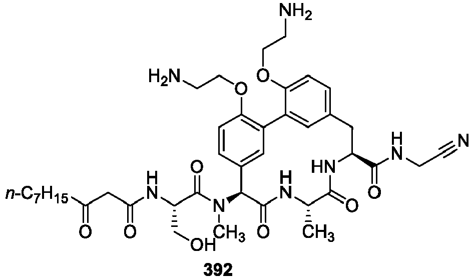 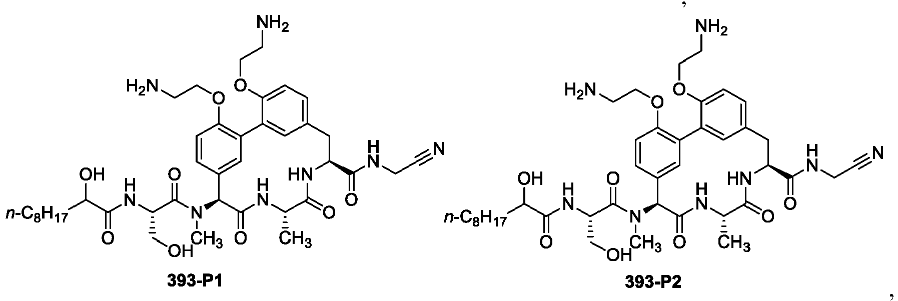 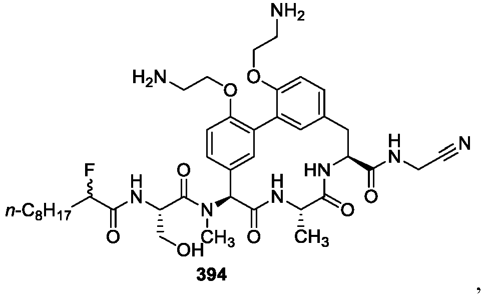 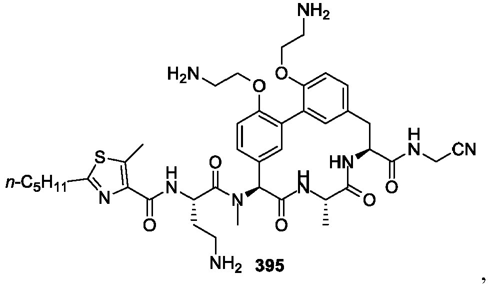 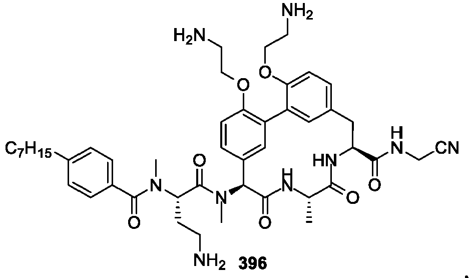 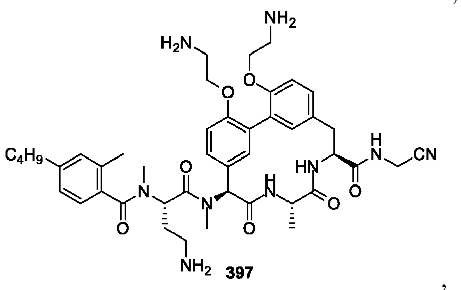 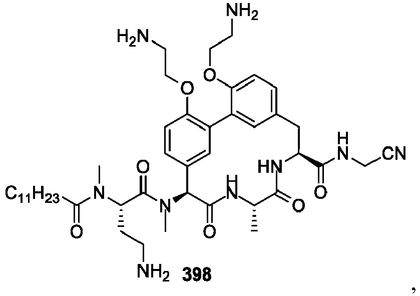 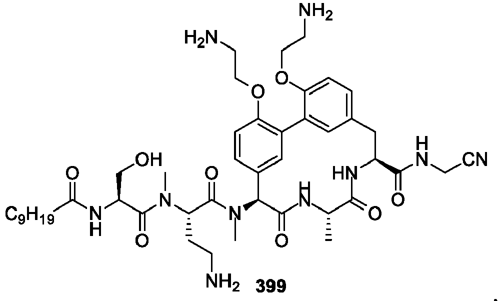 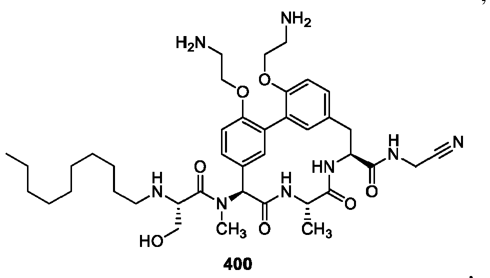 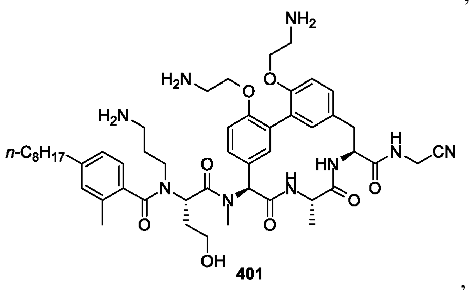 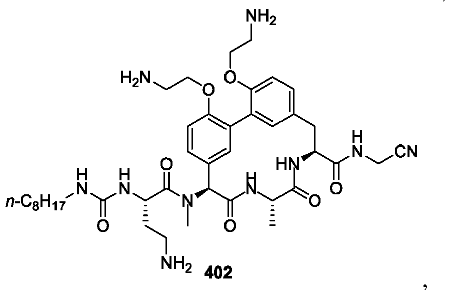 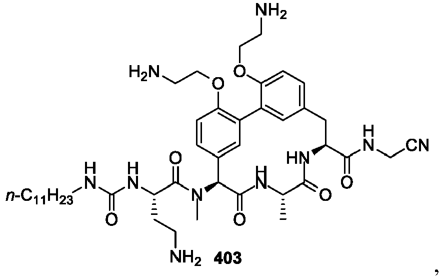 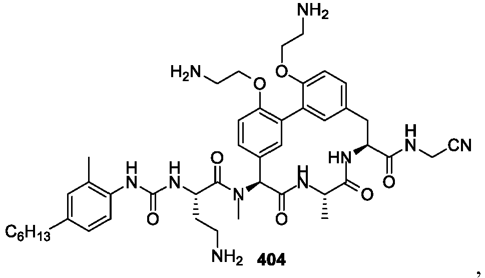 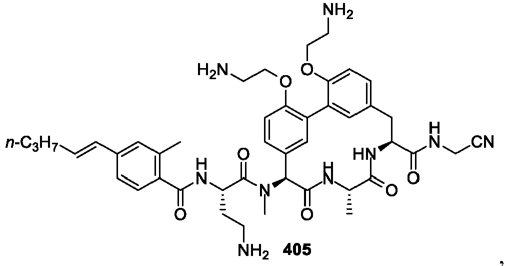 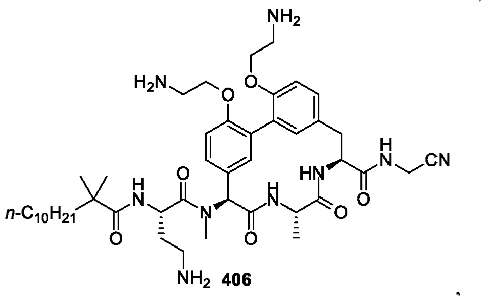 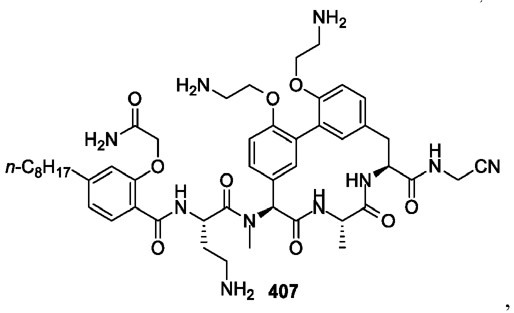 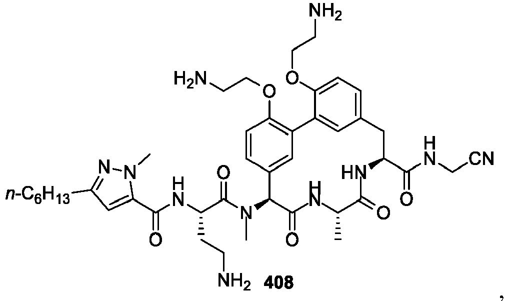 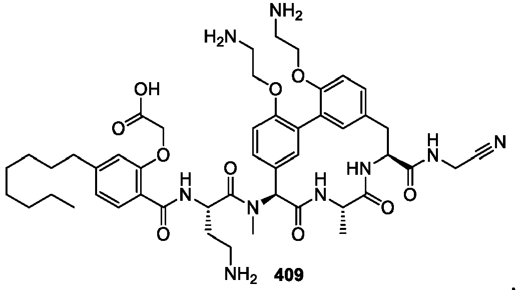 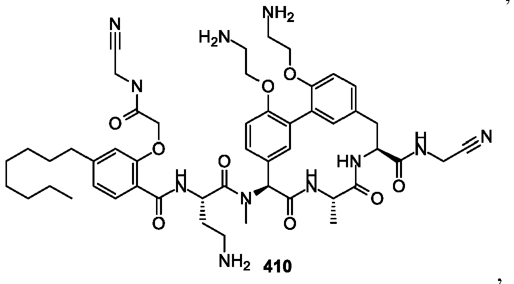 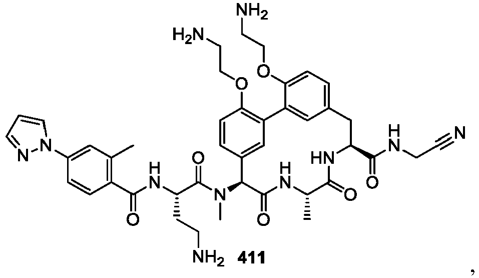 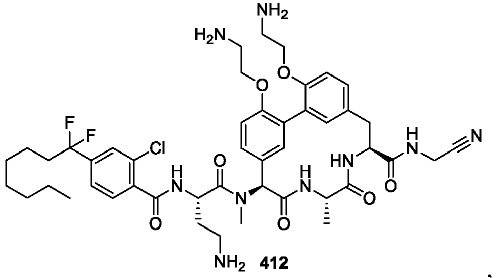 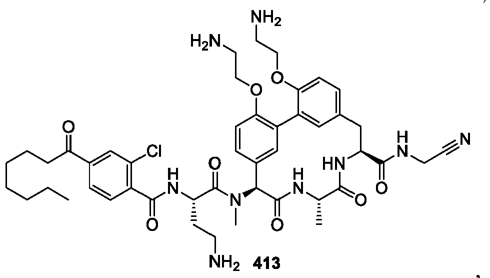 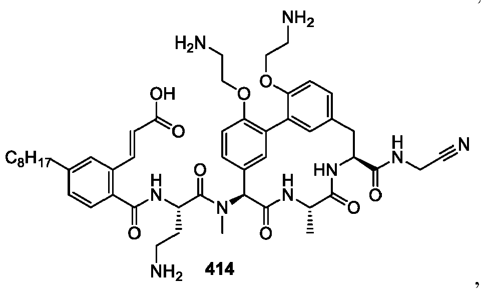 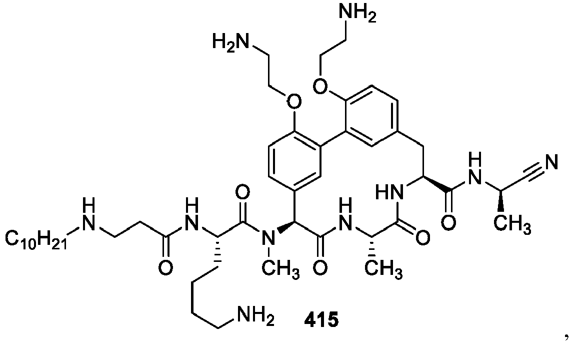 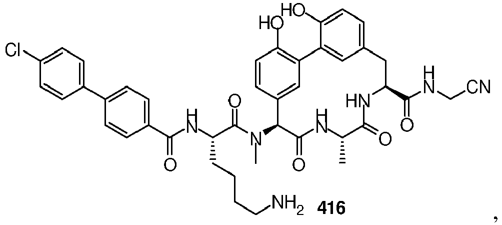 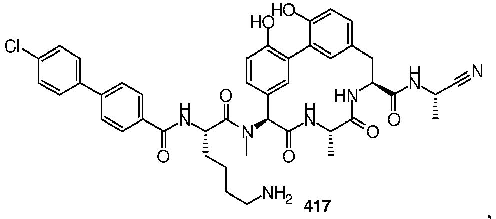 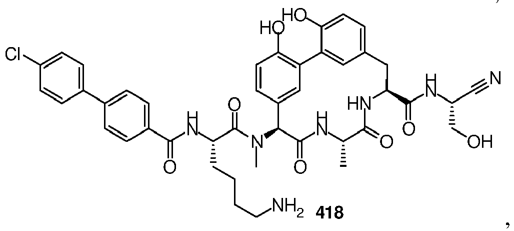 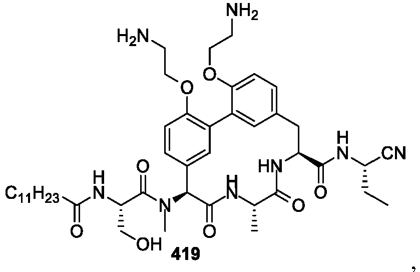 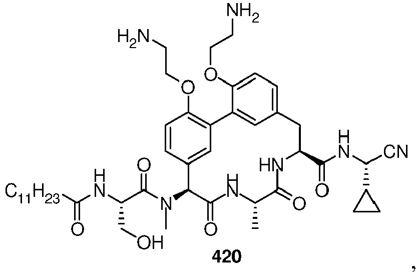 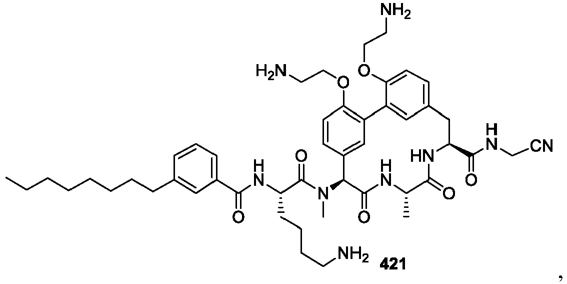 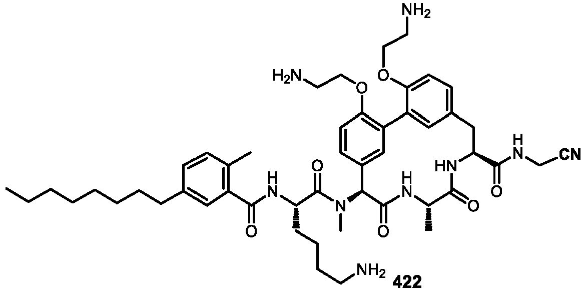 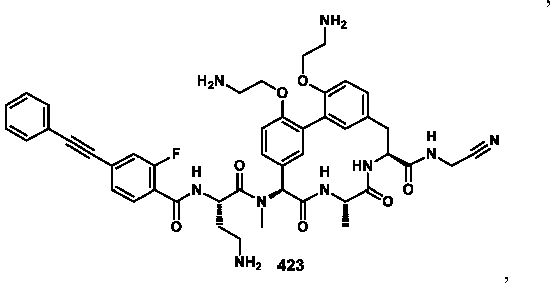 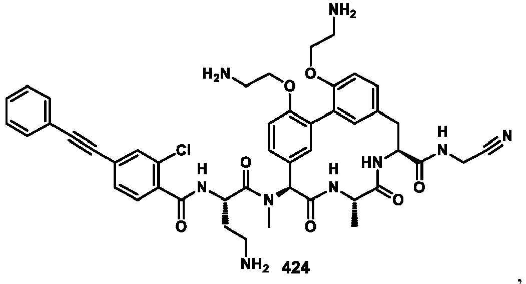 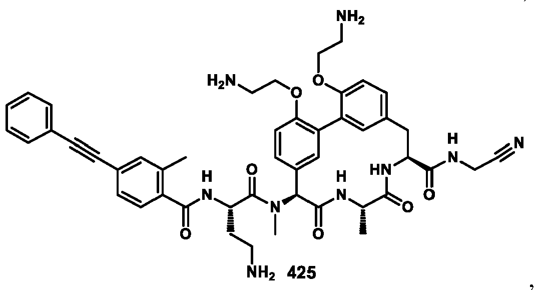 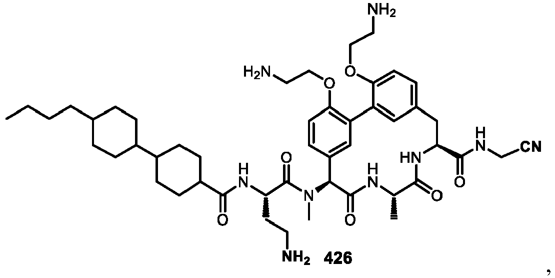 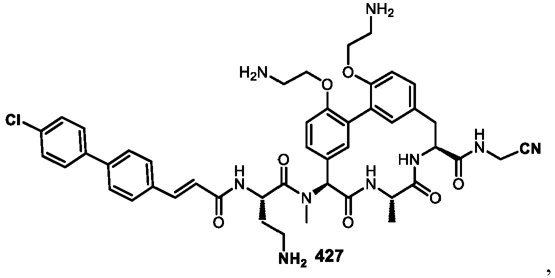 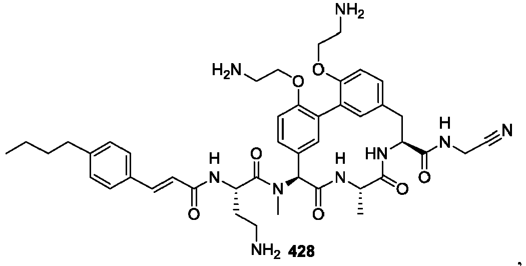 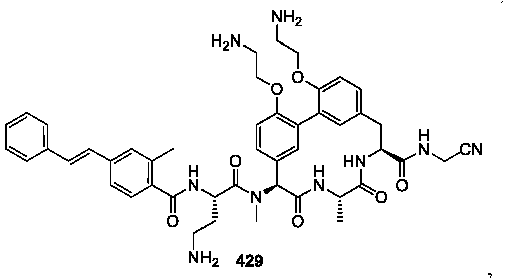 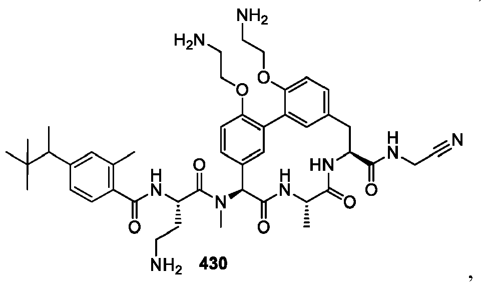 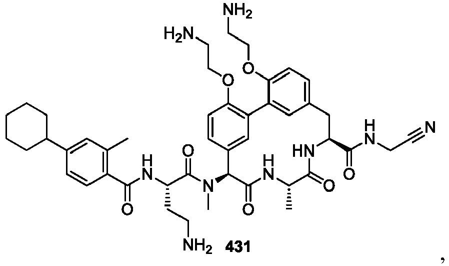 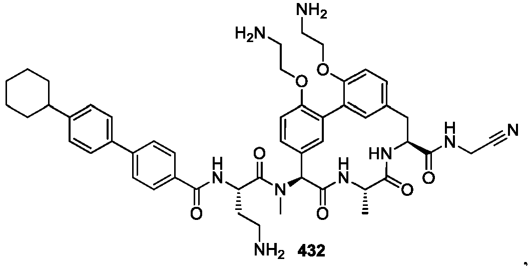 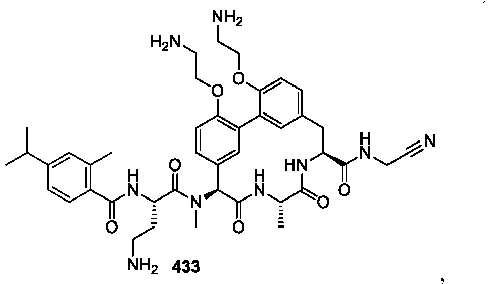 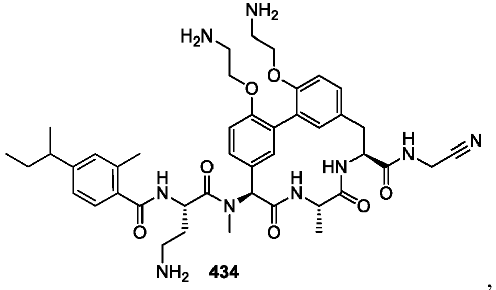 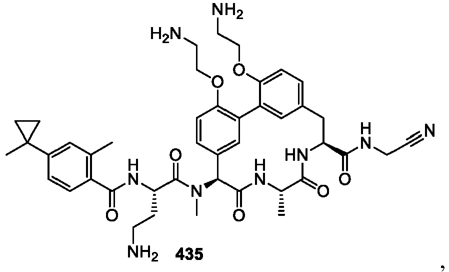 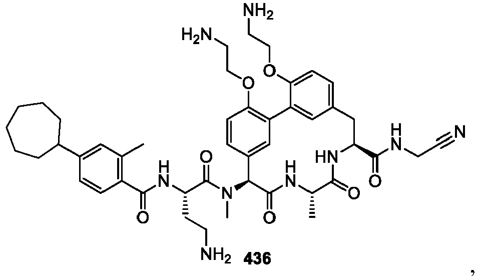 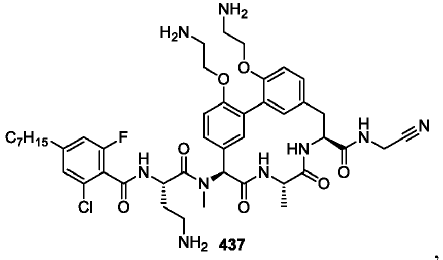 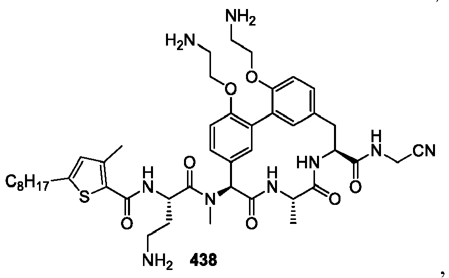 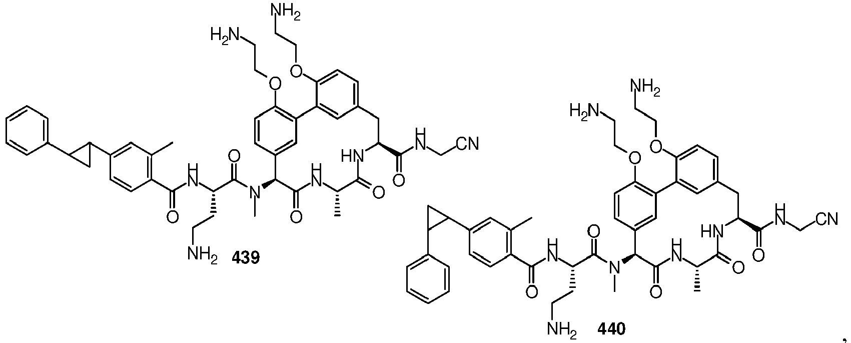 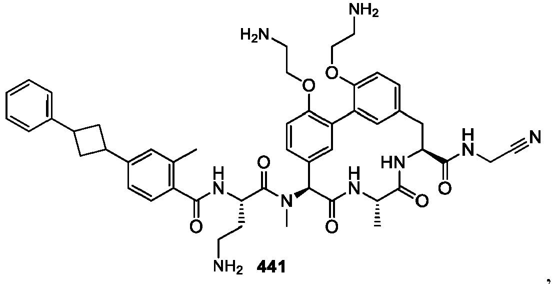 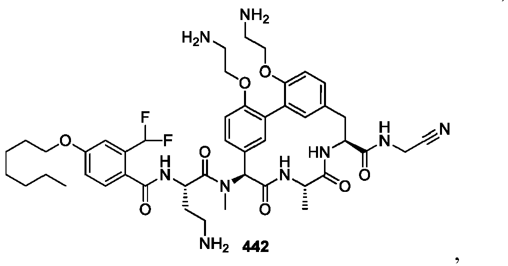 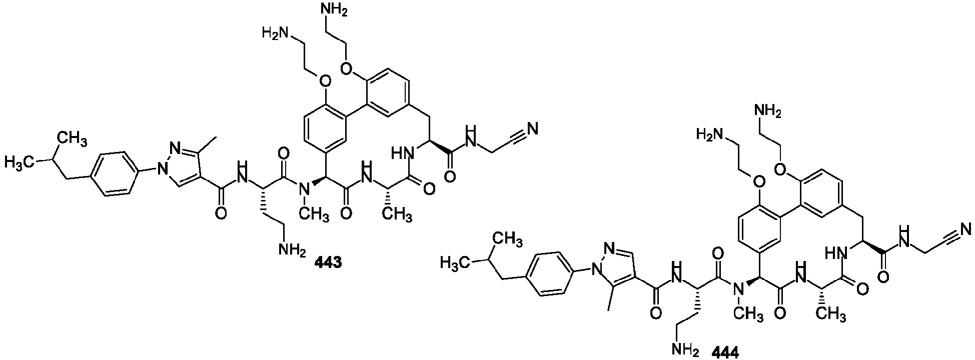 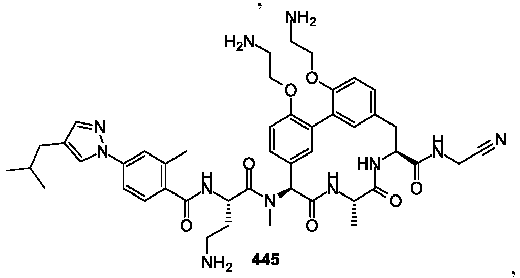 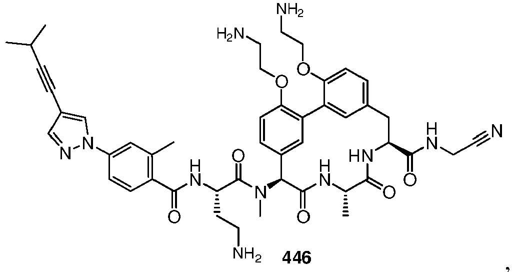 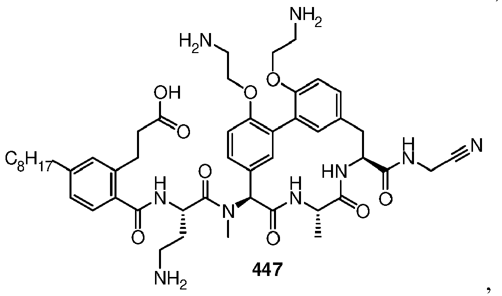 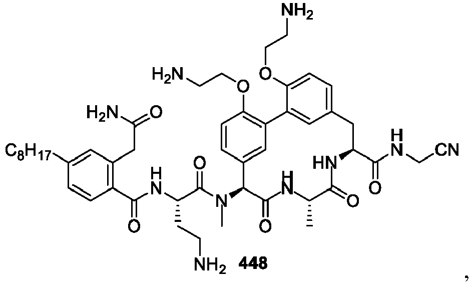 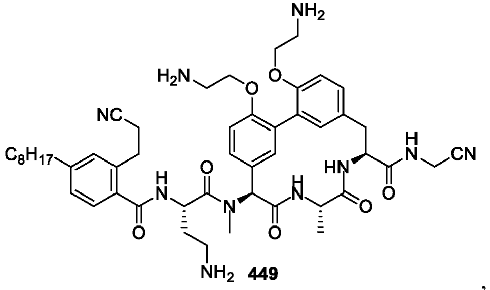 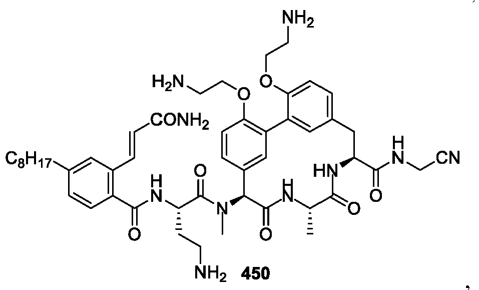 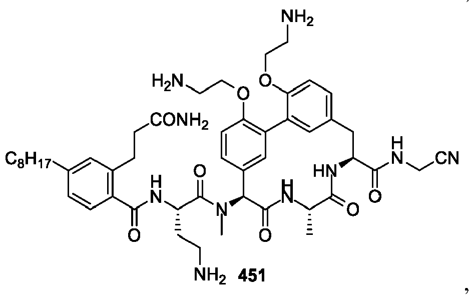 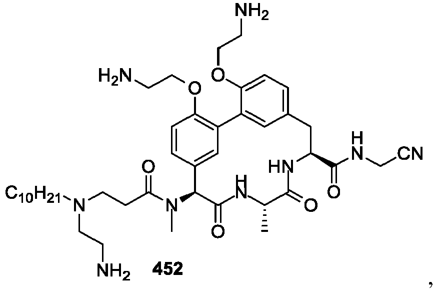 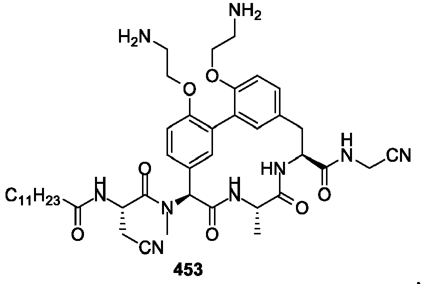 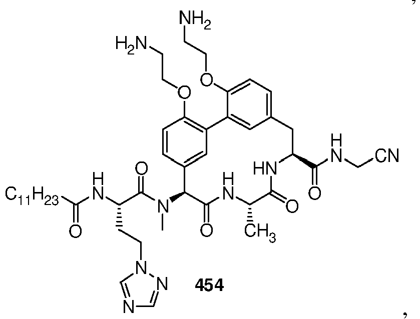 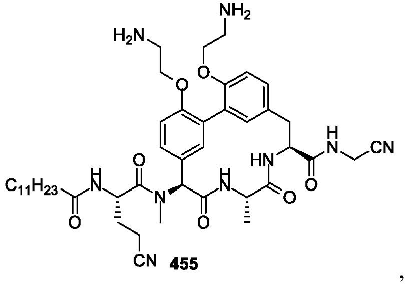 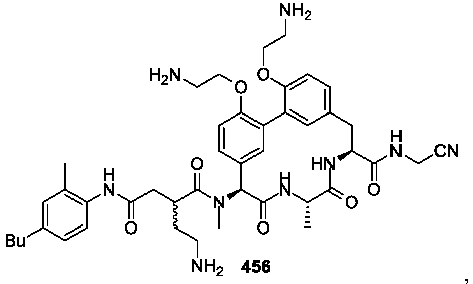 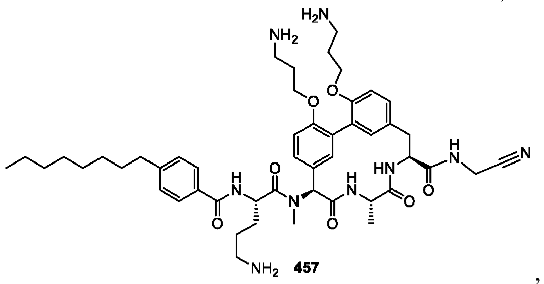 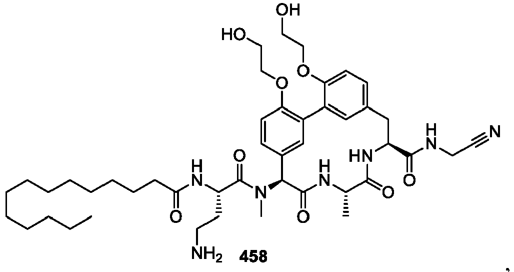 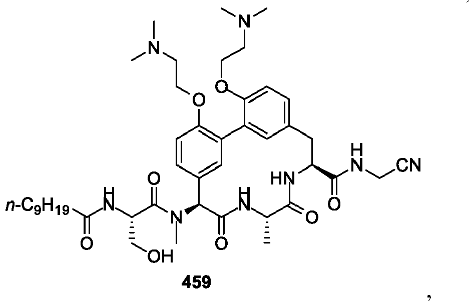 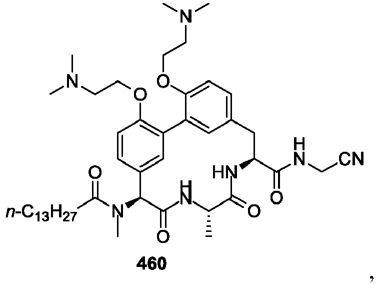 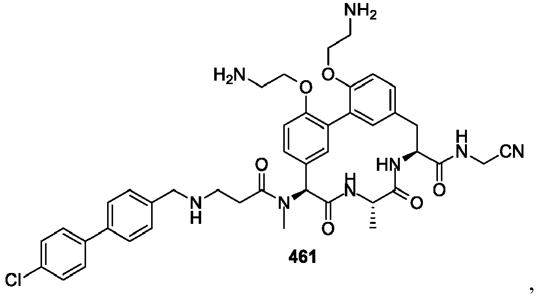 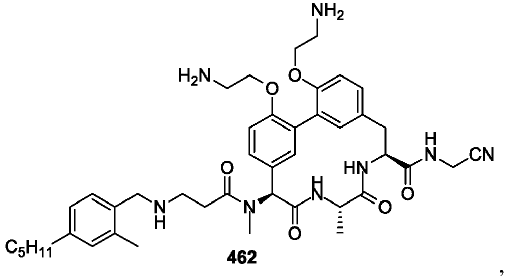 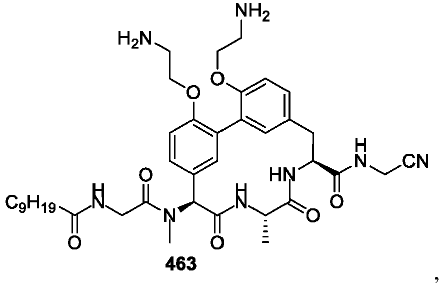 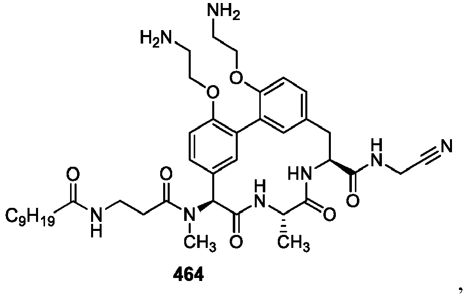 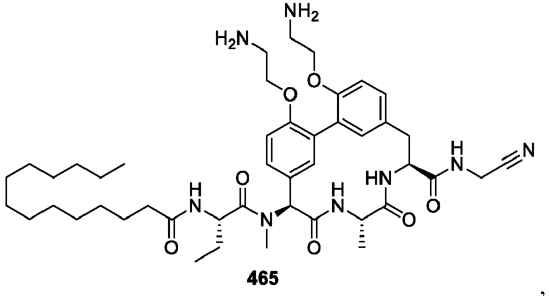 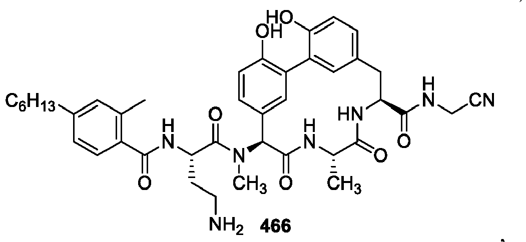 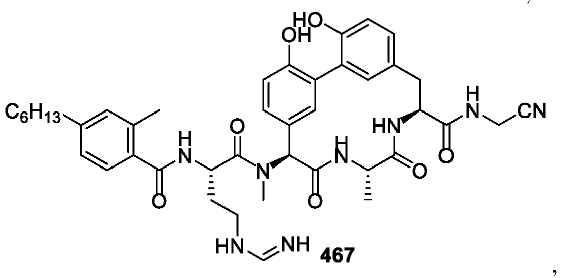 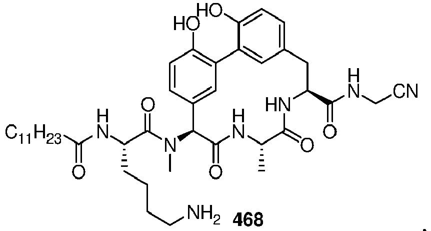 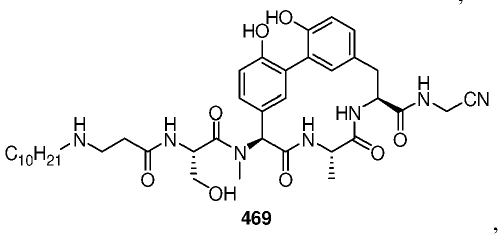 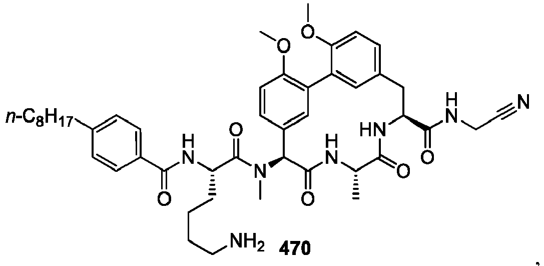 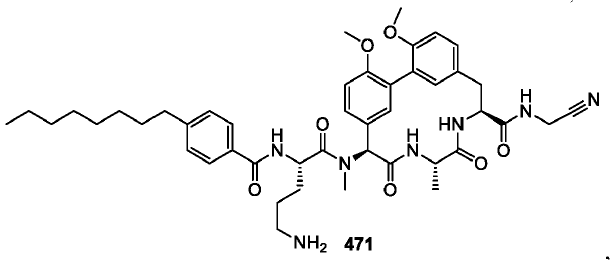 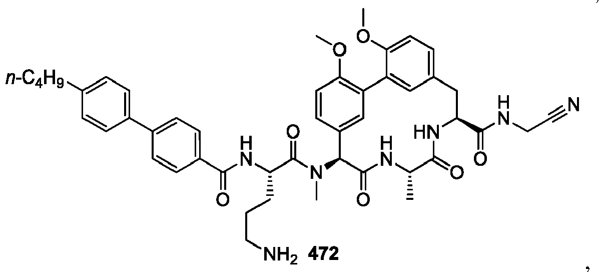 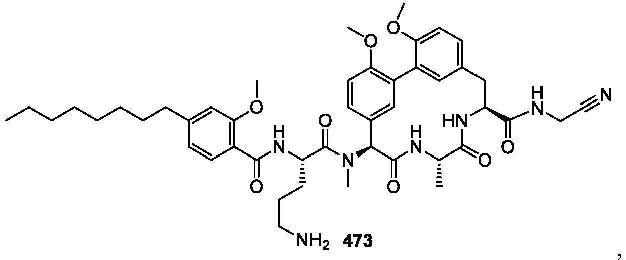 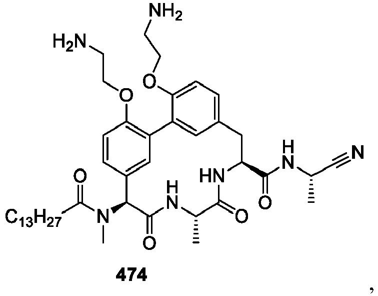 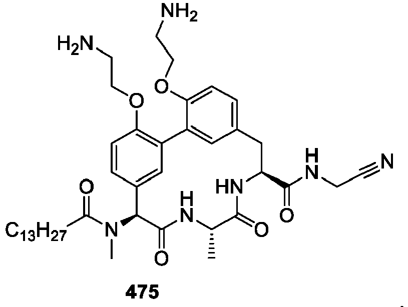 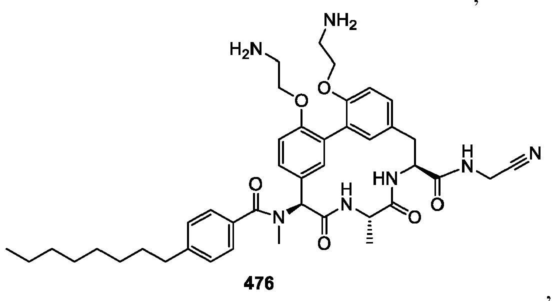 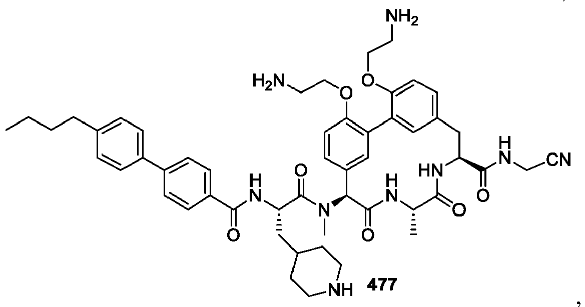 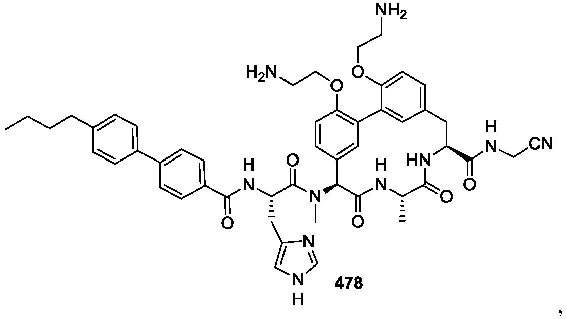 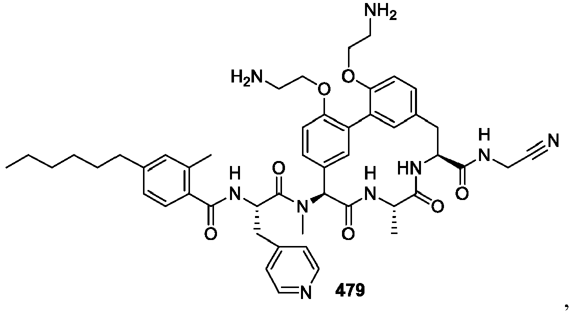 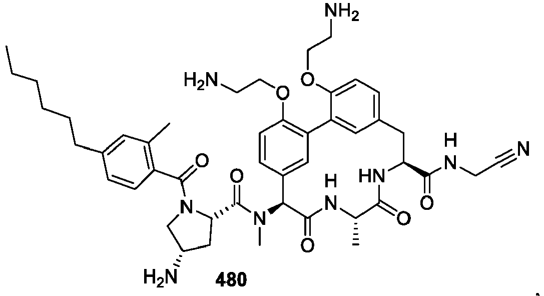 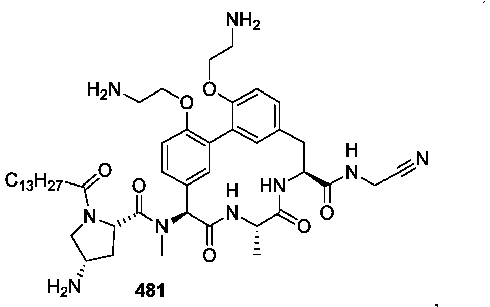 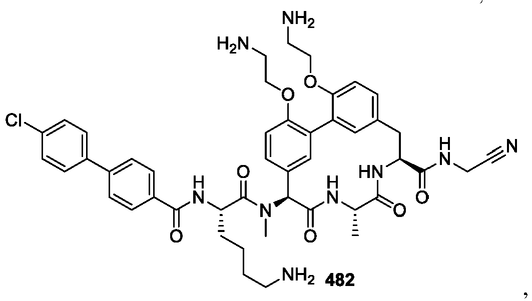 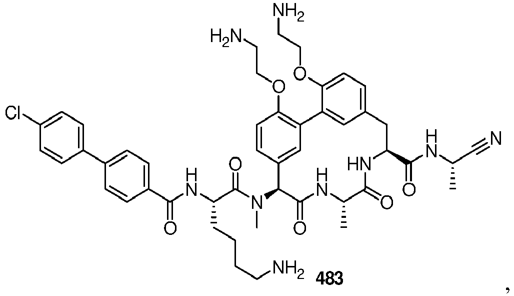 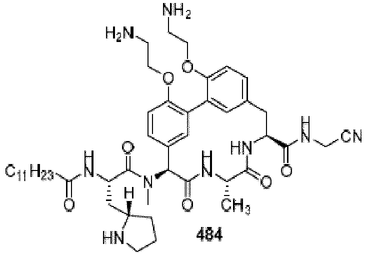 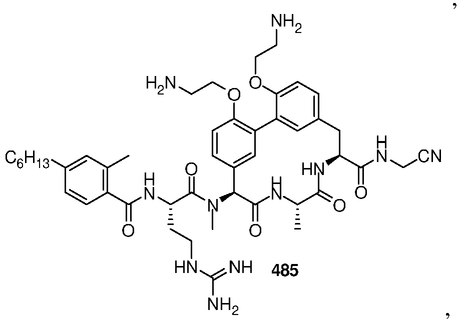 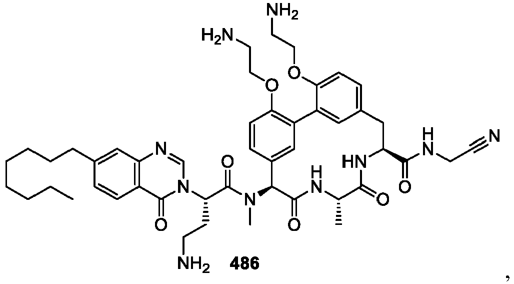 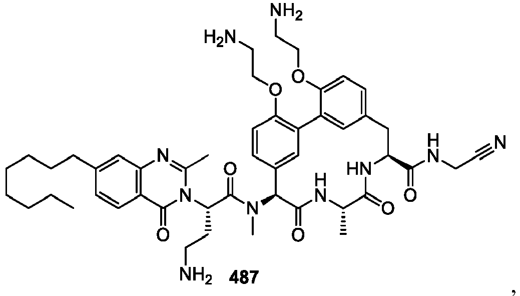 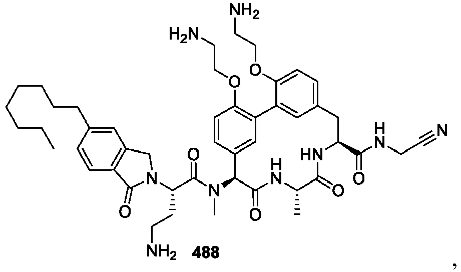 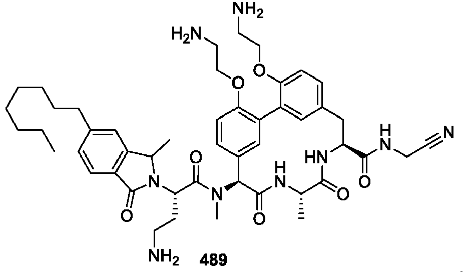 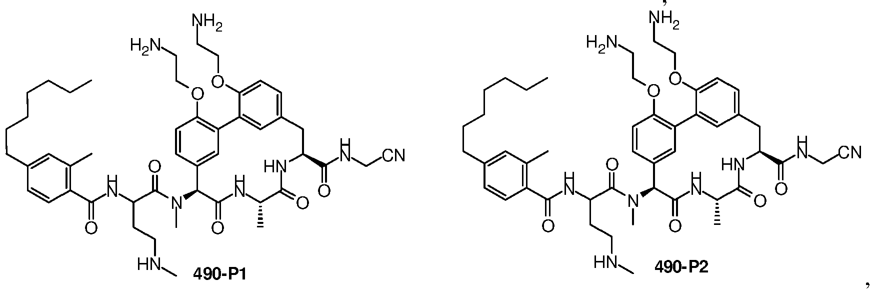 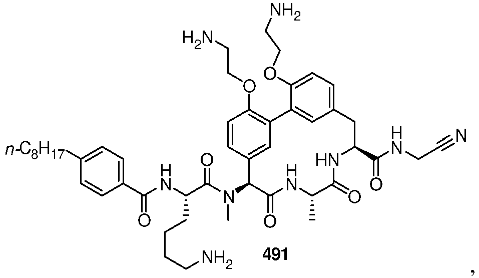 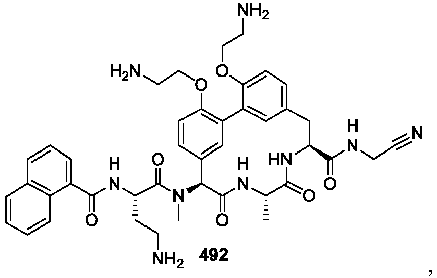 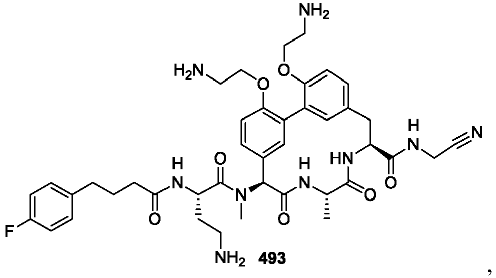 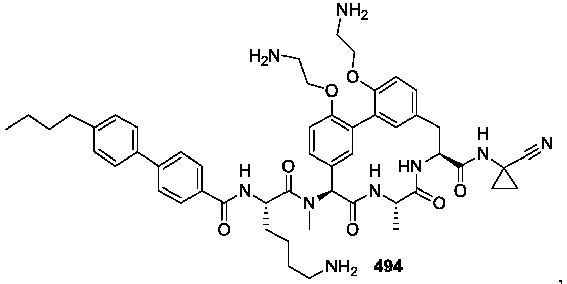 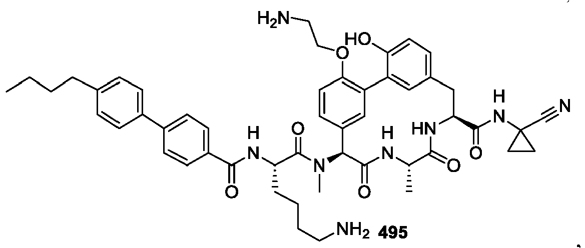 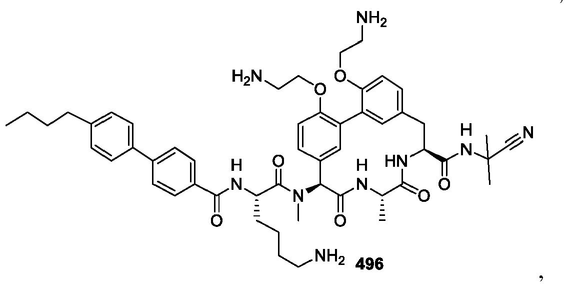 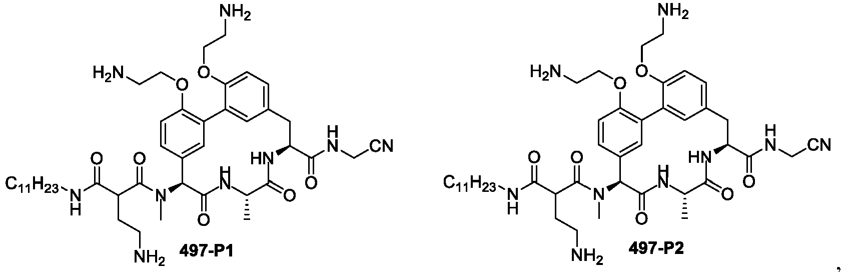 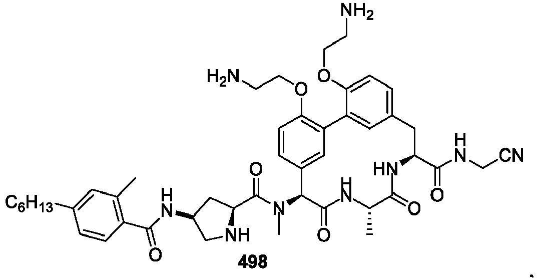 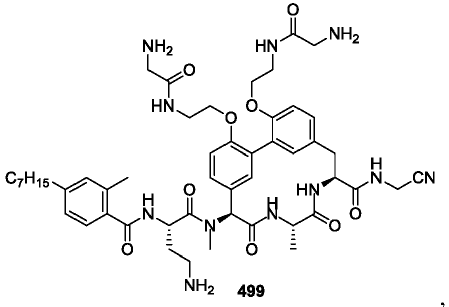 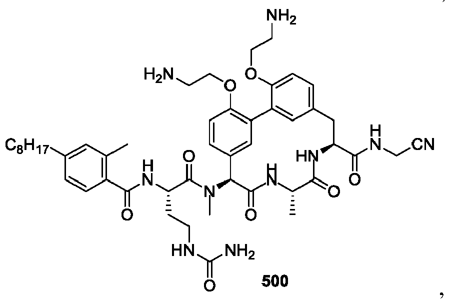 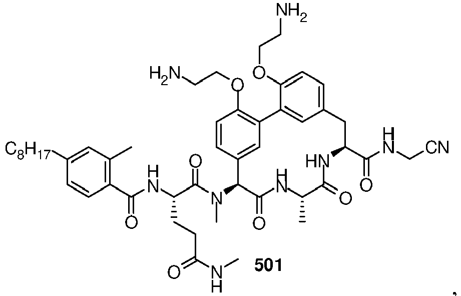 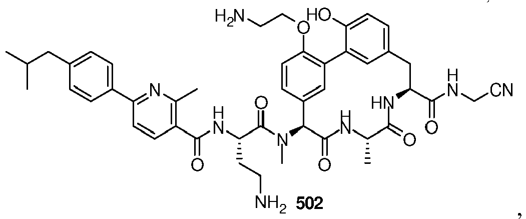 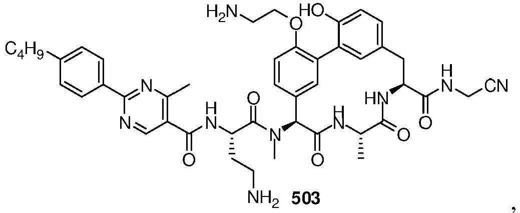 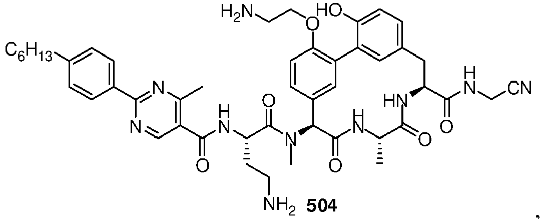 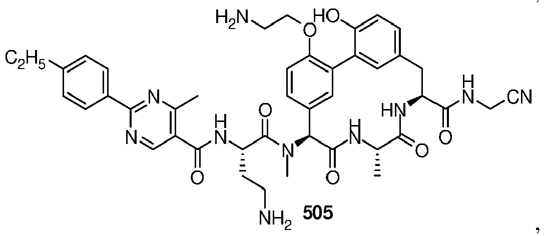 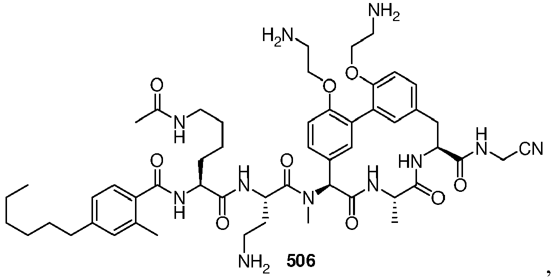 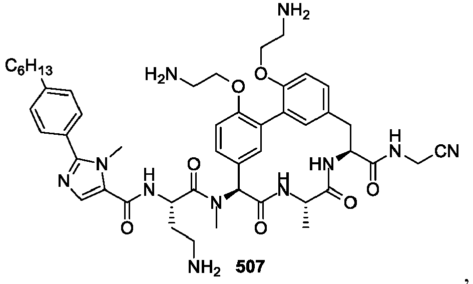 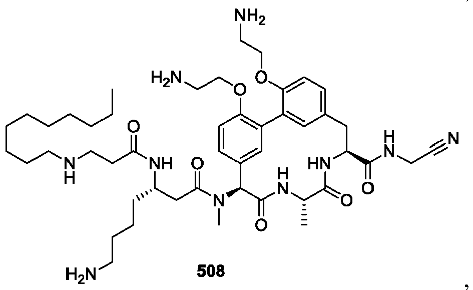 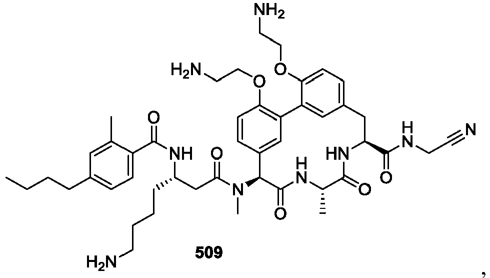 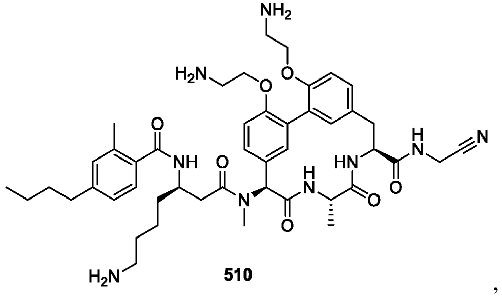 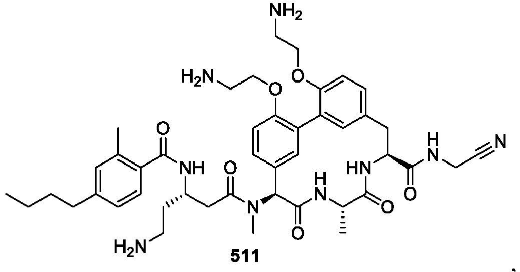 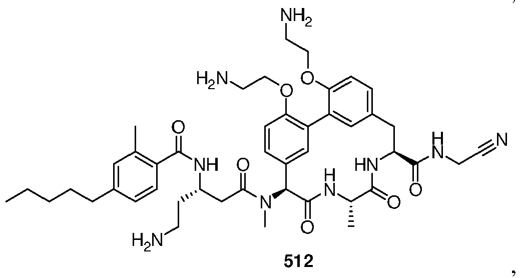 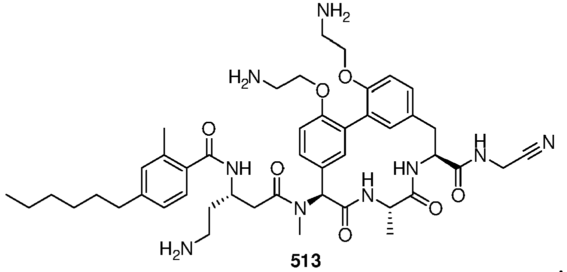 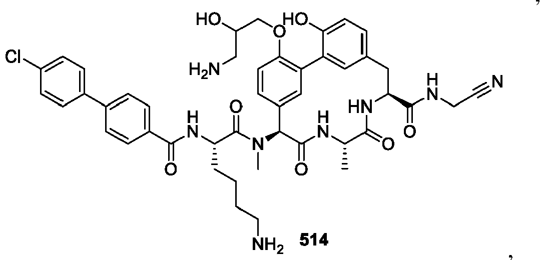 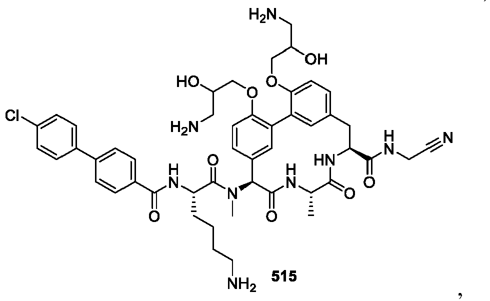 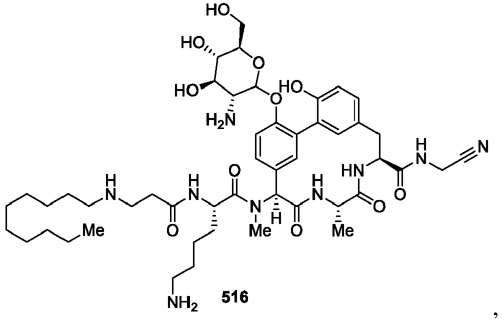 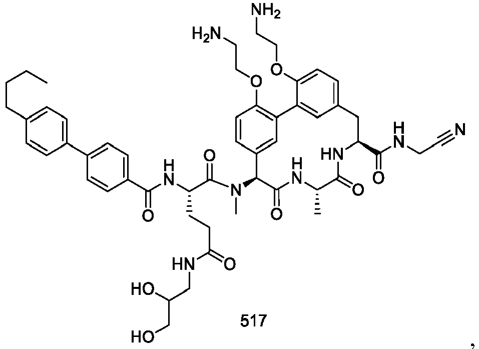 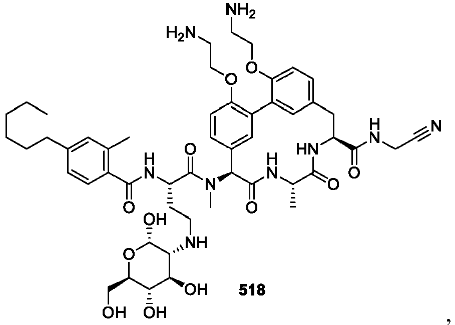 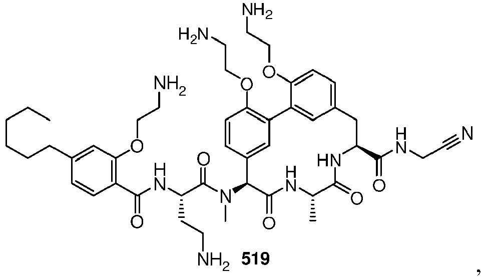 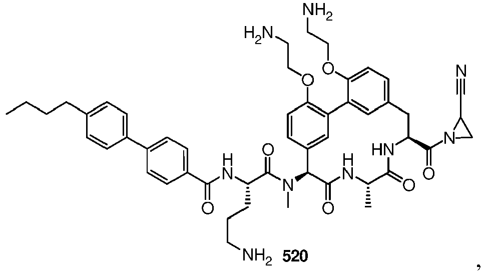 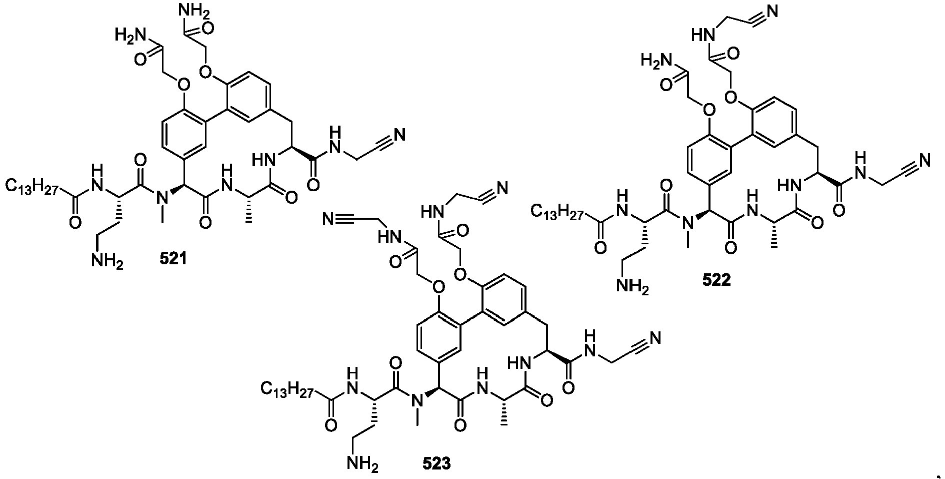 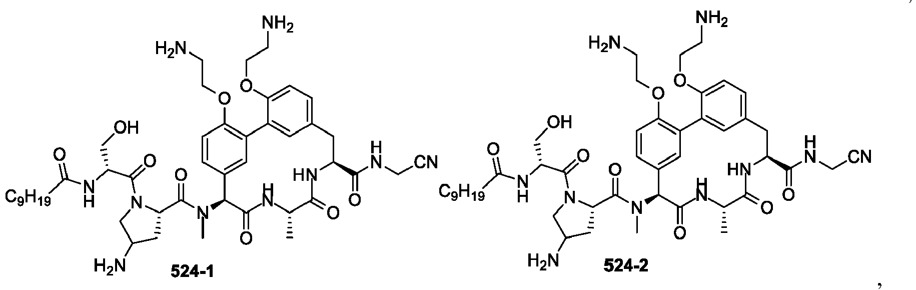 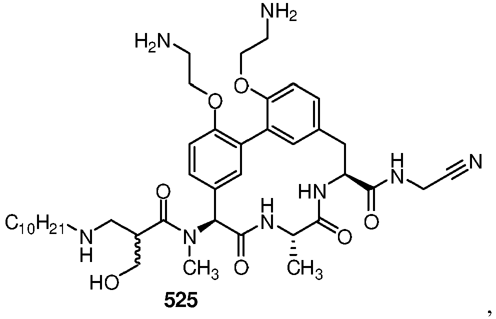 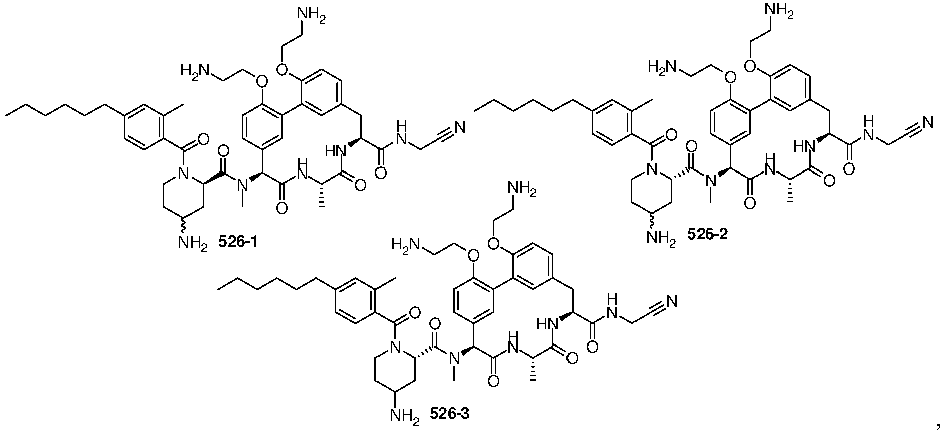 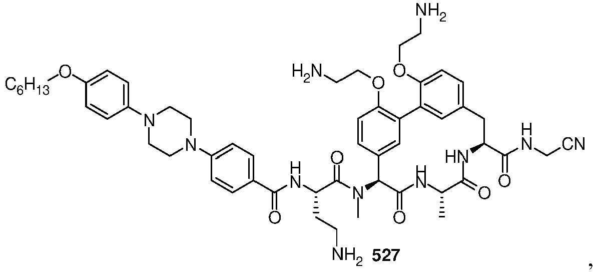 and 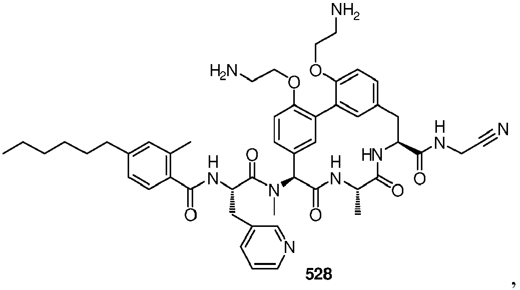 or a pharmaceutically acceptable salt or solvate thereof.

Also provided is a pharmaceutical composition comprising said compound, or a pharmaceutically acceptable salt or solvate thereof, and a pharmaceutically acceptable excipient.

Also provided is a compound as defined in the appended claims for use in a method of treatment of a bacterial infection in a mammal. In some embodiments, the bacterial infection is an infection involving: Pseudomonas aeruginosa, Pseudomonas fluorescens, Pseudomonas acidovorans, Pseudomonas alcaligenes, Pseudomonas putida, Stenotrophomonas maltophilia,

Burkholderia cepacia, Aeromonas hydrophilia, Escherichia coli, Citrobacter freundii, Salmonella typhimurium, Salmonella typhi, Salmonella paratyphi, Salmonella enteritidis, Shigella dysenteriae, Shigella flexneri, Shigella sonnei, Enterobacter cloacae, Enterobacter aerogenes, Klebsiella pneumoniae, Klebsiella oxytoca, Serratia marcescens, Francisella tularensis, Morganella morganii, Proteus mirabilis, Proteus vulgaris, Providencia alcalifaciens, Providencia rettgeri, Providencia stuartii, Acinetobacter baumannii, Acinetobacter calcoaceticus, Acinetobacter haemolyticus, Yersinia enterocolitica, Yersinia pestis, Yersinia pseudotuberculosis, Yersinia intermedia, Bordetella pertussis, Bordetella parapertussis, Bordetella bronchiseptica, Haemophilus influenzae, Haemophilus parainfluenzae, Haemophilus haemolyticus, Haemophilus parahaemolyticus, Haemophilus ducreyi, Pasteurella multocida, Pasteurella haemolytica, Branhamella catarrhalis, Helicobacter pylori, Campylobacter fetus, Campylobacter jejuni, Campylobacter coli, Borrelia burgdorferi, Vibrio cholerae, Vibrio parahaemolyticus, Legionella pneumophila, Listeria monocytogenes, Neisseria gonorrhoeae, Neisseria meningitidis, Kingella, Moraxella, Gardnerella vaginalis, Bacteroides fragilis, Bacteroides distasonis, Bacteroides 3452A homology group, Bacteroides vulgatus, Bacteroides ovalus, Bacteroides thetaiotaomicron, Bacteroides uniformis, Bacteroides eggerthii, Bacteroides splanchnicus, Clostridium difficile, Mycobacterium tuberculosis, Mycobacterium avium, Mycobacterium intracellulare, Mycobacterium leprae, Corynebacterium diphtheriae, Corynebacterium ulcerans, Streptococcus pneumoniae, Streptococcus agalactiae, Streptococcus pyogenes, Enterococcus faecalis, Enterococcus faecium, Staphylococcus aureus, Staphylococcus epidermidis, Staphylococcus saprophyticus, Staphylococcus intermedius, Staphylococcus hyicus subsp. hyicus, Staphylococcus haemolyticus, Staphylococcus hominis, or Staphylococcus saccharolyticus. In some embodiments, the bacterial infection is an infection involving a Gram-negative bacteria.

In some embodiments, the method comprises topical administration of the compound, salt, or solvate.

In some embodiments, the method further comprises administering a second therapeutic agent. In some embodiments, the second therapeutic agent is not an SpsB inhibitor. In some embodiments, the second therapeutic agent is an aminoglycoside antibiotic, fluoroquinolone antibiotic, β-lactam antibiotic, macrolide antibiotic, glycopeptide antibiotic, rifampicin, chloramphenicol, fluoramphenicol, colistin, mupirocin, bacitracin, daptomycin, or linezolid.

In some embodiments, the second therapeutic agent is a β-lactam antibiotic. In some embodiments the β-lactam antibiotic is selected from penicillins, monobactams, cephalosporins, cephamycins, and carbapenems. In some embodiments, the β-lactam antibiotic is selected from Azlocillin, Amoxicillin, Ampicillin, Doripenem, Meropenem, Biapenem, Cefamandole, Imipenem, Mezlocillin, Cefmetazole, Cefprozil, Piperacilfin/tazobactam, Carbenicillin, Cefaclor, Cephalothin, Ertapenem, Cefazolin, Cefepime, Cefonicid, Cefoxitin, Ceftazidime, Oxacillin, Cefdinir, Cefixime, Cefotaxime, Cefotetan, Cefpodoxime, Ceftizoxime, Ceftriaxone, Faropenem, Mecillinam, Methicillin, Moxalactam, Ticarcillin, Tomopenem, Ceftobiprole, Ceftaroline, Flomoxef, Cefiprome, and Cefozopran. In some embodiments, the method further comprises administering a β-lactamase inhibitor.

### BRIEF DESCRIPTION OF THE FIGURE

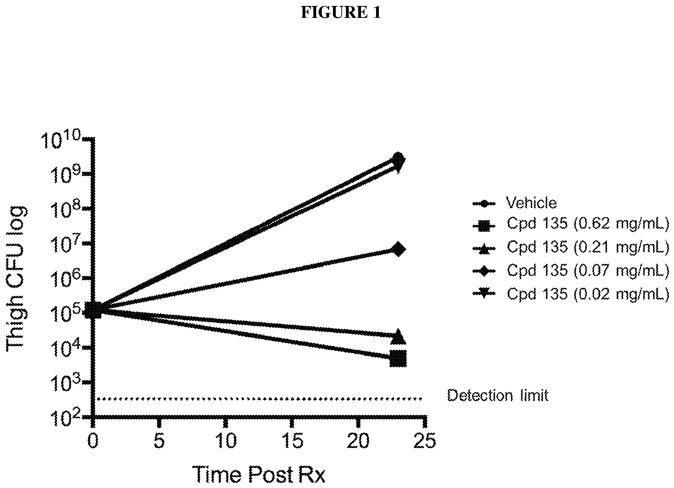

Figure 1 depicts the activity of Compound 135 in a neutropenic thigh infection model.

### DETAILED DESCRIPTION OF THE INVENTION

### Definitions

As used in the specification and the appended claims, the singular forms "a," "an" and "the" include plural referents unless the context clearly dictates otherwise.

The term "about" as used herein, when referring to a numerical value or range, allows for a degree of variability in the value or range, for example, within 10%, or within 5% of a stated value or of a stated limit of a range.

All percent compositions are given as weight-percentages, unless otherwise stated.

All average molecular weights of polymers are weight-average molecular weights, unless otherwise specified.

As used herein, "individual" (as in the subject of the treatment) means both mammals and non-mammals. Mammals include, for example, humans; non-human primates, e.g. apes and monkeys; and non-primates, e.g. dogs, cats, cattle, horses, sheep, and goats. Non-mammals include, for example, fish and birds.

The term "disease" or "disorder" or "malcondition" are used interchangeably, and are used to refer to diseases or conditions wherein a bacterial SPase plays a role in the biochemical mechanisms involved in the disease or malcondition such that a therapeutically beneficial effect can be achieved by acting on the enzyme. "Acting on" SPase can include binding to SPase and/or inhibiting the bioactivity of an SPase.

The expression "effective amount", when used to describe therapy to an individual suffering from a disorder, refers to the amount of a compound described herein that is effective to inhibit or otherwise act on SPase in the individual's tissues wherein SPase involved in the disorder is active, wherein such inhibition or other action occurs to an extent sufficient to produce a beneficial therapeutic effect.

"Substantially" as the term is used herein means completely or almost completely; for example, a composition that is "substantially free" of a component either has none of the component or contains such a trace amount that any relevant functional property of the composition is unaffected by the presence of the trace amount, or a compound is "substantially pure" is there are only negligible traces of impurities present.

"Treating" or "treatment" within the meaning herein refers to an alleviation of symptoms associated with a disorder or disease, or inhibition of further progression or worsening of those symptoms, or prevention or prophylaxis of the disease or disorder, or curing the disease or disorder. Similarly, as used herein, an "effective amount" or a "therapeutically effective amount" of a compound refers to an amount of the compound that alleviates, in whole or in part, symptoms associated with the disorder or condition, or halts or slows further progression or worsening of those symptoms, or prevents or provides prophylaxis for the disorder or condition. In particular, a "therapeutically effective amount" refers to an amount effective, at dosages and for periods of time necessary, to achieve the desired therapeutic result. A therapeutically effective amount is also one in which any toxic or detrimental effects of compounds described herein are outweighed by the therapeutically beneficial effects.

By "chemically feasible" is meant a bonding arrangement or a compound where the generally understood rules of organic structure are not violated; for example a structure within a definition of a claim that would contain in certain situations a pentavalent carbon atom that would not exist in nature would be understood to not be within the claim. The structures disclosed herein, in all of their embodiments are intended to include only "chemically feasible" structures, and any recited structures that are not chemically feasible, for example in a structure shown with variable atoms or groups, are not intended to be disclosed or claimed herein.

When a substituent is specified to be an atom or atoms of specified identity, "or a bond", a configuration is referred to when the substituent is "a bond" that the groups that are immediately adjacent to the specified substituent are directly connected to each other in a chemically feasible bonding configuration.

All chiral, diastereomeric, racemic forms of a structure are intended, unless a particular stereochemistry or isomeric form is specifically indicated. Compounds described herein can include enriched or resolved optical isomers at any or all asymmetric atoms as are apparent from the depictions, at any degree of enrichment. Both racemic and diastereomeric mixtures, as well as the individual optical isomers can be isolated or synthesized so as to be substantially free of their enantiomeric or diastereomeric partners, and these are all within the scope of the invention.

The inclusion of an isotopic form of one or more atoms in a molecule that is different from the naturally occurring isotopic distribution of the atom in nature is referred to as an "isotopically labeled form" of the molecule. All isotopic forms of atoms are included as options in the composition of any molecule, unless a specific isotopic form of an atom is indicated. For example, any hydrogen atom or set thereof in a molecule can be any of the isotopic forms of hydrogen, i.e., protium (¹H), deuterium (²H), or tritium (³H) in any combination. Similarly, any carbon atom or set thereof in a molecule can be any of the isotopic form of carbons, such as ¹¹C, ¹²C, ¹³C, or ¹⁴C, or any nitrogen atom or set thereof in a molecule can be any of the isotopic forms of nitrogen, such as ¹³N, ¹⁴N, or ¹⁵N. A molecule can include any combination of isotopic forms in the component atoms making up the molecule, the isotopic form of every atom forming the molecule being independently selected. In a multi-molecular sample of a compound, not every individual molecule necessarily has the same isotopic composition. For example, a sample of a compound can include molecules containing various different isotopic compositions, such as in a tritium or ¹⁴C radiolabeled sample where only some fraction of the set of molecules making up the macroscopic sample contains a radioactive atom. It is also understood that many elements that are not artificially isotopically enriched themselves are mixtures of naturally occurring isotopic forms, such as ¹⁴N and ¹⁵N, ³²S and ³⁴S, and so forth. A molecule as recited herein is defined as including isotopic forms of all its constituent elements at each position in the molecule. As is well known in the art, isotopically labeled compounds can be prepared by the usual methods of chemical synthesis, except substituting an isotopically labeled precursor molecule. The isotopes, radiolabeled or stable, can be obtained by any method known in the art, such as generation by neutron absorption of a precursor nuclide in a nuclear reactor, by cyclotron reactions, or by isotopic separation such as by mass spectrometry. The isotopic forms are incorporated into precursors as required for use in any particular synthetic route. For example, ¹⁴C and ³H can be prepared using neutrons generated in a nuclear reactor. Following nuclear transformation, ¹⁴C and ³H are incorporated into precursor molecules, followed by further elaboration as needed.

The term "amino protecting group" or "N-protected" as used herein refers to those groups intended to protect an amino group against undesirable reactions during synthetic procedures and which can later be removed to reveal the amine. Commonly used amino protecting groups are disclosed in Protective Groups in Organic Synthesis, Greene, T.W.; Wuts, P. G. M., John Wiley & Sons, New York, NY, (3rd Edition, 1999). Amino protecting groups include acyl groups such as formyl, acetyl, propionyl, pivaloyl, t-butylacetyl, 2-chloroacetyl, 2-bromoacetyl, trifluoroacetyl, trichloroacetyl, o-nitrophenoxyacetyl, α-chlorobutyryl, benzoyl, 4-chlorobenzoyl, 4-bromobenzoyl, 4-nitrobenzoyl, and the like; sulfonyl groups such as benzenesulfonyl, p-toluenesulfonyl and the like; alkoxy- or aryloxy-carbonyl groups (which form urethanes with the protected amine) such as benzyloxycarbonyl (Cbz), p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyloxycarbonyl, 2,4-dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydryloxycarbonyl, t-butyloxycarbonyl (Boc), diisopropylmethoxycarbonyl, isopropyloxycarbonyl, ethoxycarbonyl, methoxycarbonyl, allyloxycarbonyl (Alloc), 2,2,2-trichloroethoxycarbonyl, 2-trimethylsilylethyloxycarbonyl (Teoc), phenoxycarbonyl, 4-nitrophenoxycarbonyl, fluorenyl-9-methoxycarbonyl (Fmoc), cyclopentyloxycarbonyl, adamantyloxycarbonyl, cyclohexyloxycarbonyl, phenylthiocarbonyl and the like; aralkyl groups such as benzyl, triphenylmethyl, benzyloxymethyl and the like; and silyl groups such as trimethylsilyl and the like. Amine protecting groups also include cyclic amino protecting groups such as phthaloyl and dithiosuccinimidyl, which incorporate the amino nitrogen into a heterocycle. Typically, amino protecting groups include formyl, acetyl, benzoyl, pivaloyl, t-butylacetyl, phenylsulfonyl, Alloc, Teoc, benzyl, Fmoc, Boc and Cbz. It is well within the skill of the ordinary artisan to select and use the appropriate amino protecting group for the synthetic task at hand.

The term "hydroxyl protecting group" or "O-protected" as used herein refers to those groups intended to protect an OH group against undesirable reactions during synthetic procedures and which can later be removed to reveal the amine. Commonly used hydroxyl protecting groups are disclosed in Protective Groups in Organic Synthesis, Greene, T.W.; Wuts, P. G. M., John Wiley & Sons, New York, NY, (3rd Edition, 1999). Hydroxyl protecting groups include acyl groups such as formyl, acetyl, propionyl, pivaloyl, t-butylacetyl, 2-chloroacetyl, 2-bromoacetyl, trifluoroacetyl, trichloroacetyl, o-nitrophenoxyacetyl, α-chlorobutyryl, benzoyl, 4-chlorobenzoyl, 4-bromobenzoyl, 4-nitrobenzoyl, and the like; sulfonyl groups such as benzenesulfonyl, p-toluenesulfonyl and the like; acyloxy groups (which form urethanes with the protected amine) such as benzyloxycarbonyl (Cbz), p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyloxycarbonyl, 2,4-dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydryloxycarbonyl, t-butyloxycarbonyl (Boc), diisopropylmethoxycarbonyl, isopropyloxycarbonyl, ethoxycarbonyl, methoxycarbonyl, allyloxycarbonyl (Alloc), 2,2,2-trichloroethoxycarbonyl, 2-trimethylsilylethyloxycarbonyl (Teoc), phenoxycarbonyl, 4-nitrophenoxycarbonyl, fluorenyl-9-methoxycarbonyl (Fmoc), cyclopentyloxycarbonyl, adamantyloxycarbonyl, cyclohexyloxycarbonyl, phenylthiocarbonyl and the like; aralkyl groups such as benzyl, triphenylmethyl, benzyloxymethyl and the like; and silyl groups such as trimethylsilyl and the like. It is well within the skill of the ordinary artisan to select and use the appropriate hydroxyl protecting group for the synthetic task at hand.

In general, "substituted" refers to an organic group as defined herein in which one or more bonds to a hydrogen atom contained therein are replaced by one or more bonds to a non-hydrogen atom such as, but not limited to, a halogen (i.e., F, Cl, Br, and I); an oxygen atom in groups such as hydroxyl groups, alkoxy groups, aryloxy groups, aralkyloxy groups, oxo(carbonyl) groups, carboxyl groups including carboxylic acids, carboxylates, and carboxylate esters; a sulfur atom in groups such as thiol groups, alkyl and aryl sulfide groups, sulfoxide groups, sulfone groups, sulfonyl groups, and sulfonamide groups; a nitrogen atom in groups such as amines, hydroxylamines, nitriles, nitro groups, N-oxides, hydrazides, azides, and enamines; and other heteroatoms in various other groups. Non-limiting examples of substituents that can be bonded to a substituted carbon (or other) atom include F, Cl, Br, I, OR', OC(O)N(R')₂, CN, NO, NO₂, ONO₂, azido, CF₃, OCF₃, R', O (oxo), S (thiono), C(O), S(O), methylenedioxy, ethylenedioxy, N(R')₂, SR', SOR', SO₂R', SO₂N(R')₂, SO₃R', C(O)R', C(O)C(O)R', C(O)CH₂C(O)R', C(S)R', C(O)OR', OC(O)R', C(O)N(R')₂, OC(O)N(R')₂, C(S)N(R')₂, (CH₂)₀₋₂N(R')C(O)R', (CH₂)₀₋₂N(R')N(R')₂, N(R')N(R')C(O)R', N(R')N(R')C(O)OR', N(R')N(R')CON(R')₂, N(R')SO₂R', N(R')SO₂N(R')₂, N(R')C(O)OR', N(R')C(O)R', N(R')C(S)R', N(R')C(O)N(R')₂, N(R')C(S)N(R')₂, N(COR')COR', N(OR')R', C(=NH)N(R')₂, C(O)N(OR')R', or C(=NOR')R' wherein R' can be hydrogen or a carbon-based moiety, and wherein the carbon-based moiety can itself be further substituted.

When a substituent is monovalent, such as, for example, For Cl, it is bonded to the atom it is substituting by a single bond. When a substituent is more than monovalent, such as O, which is divalent, it can be bonded to the atom it is substituting by more than one bond, i.e., a divalent substituent is bonded by a double bond; for example, a C substituted with O forms a carbonyl group, C=O, which can also be written as "CO", "C(O)", or "C(=O)", wherein the C and the O are double bonded. When a carbon atom is substituted with a double-bonded oxygen (=O) group, the oxygen substituent is termed an "oxo" group. When a divalent substituent such as NR is double-bonded to a carbon atom, the resulting C(=NR) group is termed an "imino" group. When a divalent substituent such as S is double-bonded to a carbon atom, the results C(=S) group is termed a "thiocarbonyl" group.

Alternatively, a divalent substituent such as O, S, C(O), S(O), or S(O)₂ can be connected by two single bonds to two different carbon atoms. For example, O, a divalent substituent, can be bonded to each of two adjacent carbon atoms to provide an epoxide group, or the O can form a bridging ether group, termed an "oxy" group, between adjacent or non-adjacent carbon atoms, for example bridging the 1,4-carbons of a cyclohexyl group to form a [2.2.1]-oxabicyclo system. Further, any substituent can be bonded to a carbon or other atom by a linker, such as (CH₂)ₙ or (CR'₂)ₙ wherein n is 1, 2, 3, or more, and each R' is independently selected.

C(O) and S(O)₂ groups can be bound to one or two heteroatoms, such as nitrogen, rather than to a carbon atom. For example, when a C(O) group is bound to one carbon and one nitrogen atom, the resulting group is called an "amide" or "carboxamide." When a C(O) group is bound to two nitrogen atoms, the functional group is termed a urea. When a S(O)₂ group is bound to one carbon and one nitrogen atom, the resulting unit is termed a "sulfonamide." When a S(O)₂ group is bound to two nitrogen atoms, the resulting unit is termed a "sulfamate."

Substituted alkyl, alkenyl, alkynyl, cycloalkyl, and cycloalkenyl groups as well as other substituted groups also include groups in which one or more bonds to a hydrogen atom are replaced by one or more bonds, including double or triple bonds, to a carbon atom, or to a heteroatom such as, but not limited to, oxygen in carbonyl (oxo), carboxyl, ester, amide, imide, urethane, and urea groups; and nitrogen in imines, hydroxyimines, oximes, hydrazones, amidines, guanidines, and nitriles.

Substituted ring groups such as substituted cycloalkyl, aryl, heterocyclyl and heteroaryl groups also include rings and fused ring systems in which a bond to a hydrogen atom is replaced with a bond to a carbon atom. Therefore, substituted cycloalkyl, aryl, heterocyclyl and heteroaryl groups can also be substituted with alkyl, alkenyl, and alkynyl groups as defined herein.

By a "ring system" as the term is used herein is meant a moiety comprising one, two, three or more rings, which can be substituted with non-ring groups or with other ring systems, or both, which can be fully saturated, partially unsaturated, fully unsaturated, or aromatic, and when the ring system includes more than a single ring, the rings can be fused, bridging, or spirocyclic. By "spirocyclic" is meant the class of structures wherein two rings are fused at a single tetrahedral carbon atom, as is well known in the art.

As to any of the groups described herein, which contain one or more substituents, it is understood, of course, that such groups do not contain any substitution or substitution patterns which are sterically impractical and/or synthetically non-feasible. In addition, the compounds of this disclosed subject matter include all stereochemical isomers arising from the substitution of these compounds.

Selected substituents within the compounds described herein are present to a recursive degree. In this context, "recursive substituent" means that a substituent may recite another instance of itself or of another substituent that itself recites the first substituent. Because of the recursive nature of such substituents, theoretically, a large number may be present in any given claim. One of ordinary skill in the art of medicinal chemistry and organic chemistry understands that the total number of such substituents is reasonably limited by the desired properties of the compound intended. Such properties include, by of example and not limitation, physical properties such as molecular weight, solubility or log P, application properties such as activity against the intended target, and practical properties such as ease of synthesis.

Recursive substituents are an intended aspect of the disclosed subject matter. One of ordinary skill in the art of medicinal and organic chemistry understands the versatility of such substituents. To the degree that recursive substituents are present in a claim of the disclosed subject matter, the total number should be determined as set forth above.

Alkyl groups include straight chain and branched alkyl groups and cycloalkyl groups having from 1 to about 20 carbon atoms, and typically from 1 to 12 carbons or, in some embodiments, from 1 to 8 carbon atoms. Examples of straight chain alkyl groups include those with from 1 to 8 carbon atoms such as methyl, ethyl, n-propyl, n-butyl, n-pentyl, n-hexyl, n-heptyl, and n-octyl groups. Examples of branched alkyl groups include, but are not limited to, isopropyl, iso-butyl, sec-butyl, t-butyl, neopentyl, isopentyl, and 2,2-dimethylpropyl groups. As used herein, the term "alkyl" encompasses n-alkyl, isoalkyl, and anteisoalkyl groups as well as other branched chain forms of alkyl. Representative substituted alkyl groups can be substituted one or more times with any of the groups listed above, for example, amino, hydroxy, cyano, carboxy, nitro, thio, alkoxy, and halogen groups.

The term "alkylene" means a linear saturated divalent hydrocarbon radical of one to six carbon atoms or a branched saturated divalent hydrocarbon radical of one to six carbon atoms unless otherwise stated, such as methylene, ethylene, propylene, 1-methylpropylene, 2-methylpropylene, butylene, pentylene, and the like.

The term "carbonyl" means C=O.

The terms "carboxy" and "hydroxycarbonyl" mean COOH.

Cycloalkyl groups are cyclic alkyl groups such as, but not limited to, cyclopropyl, cyclobutyl, cyclopentyl, cyclohexyl, cycloheptyl, and cyclooctyl groups. In some embodiments, the cycloalkyl group can have 3 to about 8-12 ring members, whereas in other embodiments the number of ring carbon atoms range from 3 to 4, 5, 6, or 7. Cycloalkyl groups further include polycyclic cycloalkyl groups such as, but not limited to, norbornyl, adamantyl, bornyl, camphenyl, isocamphenyl, and carenyl groups, and fused rings such as, but not limited to, decalinyl, and the like. Cycloalkyl groups also include rings that are substituted with straight or branched chain alkyl groups as defined above. Representative substituted cycloalkyl groups can be mono-substituted or substituted more than once, such as, but not limited to, 2,2-, 2,3-, 2,4- 2,5- or 2,6-disubstituted cyclohexyl groups or mono-, di- or tri-substituted norbornyl or cycloheptyl groups, which can be substituted with, for example, amino, hydroxy, cyano, carboxy, nitro, thio, alkoxy, and halogen groups. The term "cycloalkenyl" alone or in combination denotes a cyclic alkenyl group.

The terms "carbocyclic," "carbocyclyl," and "carbocycle" denote a ring structure wherein the atoms of the ring are carbon, such as a cycloalkyl group or an aryl group. In some embodiments, the carbocycle has 3 to 8 ring members, whereas in other embodiments the number of ring carbon atoms is 4, 5, 6, or 7. Unless specifically indicated to the contrary, the carbocyclic ring can be substituted with as many as N-1 substituents wherein N is the size of the carbocyclic ring with, for example, alkyl, alkenyl, alkynyl, amino, aryl, hydroxy, cyano, carboxy, heteroaryl, heterocyclyl, nitro, thio, alkoxy, and halogen groups, or other groups as are listed above. A carbocyclyl ring can be a cycloalkyl ring, a cycloalkenyl ring, or an aryl ring. A carbocyclyl can be monocyclic or polycyclic, and if polycyclic each ring can independently be a cycloalkyl ring, a cycloalkenyl ring, or an aryl ring.

(Cycloalkyl)alkyl groups, also denoted cycloalkylalkyl, are alkyl groups as defined above in which a hydrogen or carbon bond of the alkyl group is replaced with a bond to a cycloalkyl group as defined above.

Alkenyl groups include straight and branched chain and cyclic alkyl groups as defined above, except that at least one double bond exists between two carbon atoms. Thus, alkenyl groups have from 2 to about 20 carbon atoms, and typically from 2 to 12 carbons or, in some embodiments, from 2 to 8 carbon atoms. Examples include, but are not limited to vinyl, -CH=CH(CH₃), -CH=C(CH₃)₂, -C(CH₃)=CH₂, -C(CH₃)=CH(CH₃), -C(CH₂CH₃)=CH₂, cyclohexenyl, cyclopentenyl, cyclohexadienyl, butadienyl, pentadienyl, and hexadienyl among others.

Cycloalkenyl groups include cycloalkyl groups having at least one double bond between 2 carbons. Thus for example, cycloalkenyl groups include but are not limited to cyclohexenyl, cyclopentenyl, and cyclohexadienyl groups. Cycloalkenyl groups can have from 3 to about 8-12 ring members, whereas in other embodiments the number of ring carbon atoms range from 3 to 5, 6, or 7. Cycloalkyl groups further include polycyclic cycloalkyl groups such as, but not limited to, norbornyl, adamantyl, bornyl, camphenyl, isocamphenyl, and carenyl groups, and fused rings such as, but not limited to, decalinyl, and the like, provided they include at least one double bond within a ring. Cycloalkenyl groups also include rings that are substituted with straight or branched chain alkyl groups as defined above.

(Cycloalkenyl)alkyl groups are alkyl groups as defined above in which a hydrogen or carbon bond of the alkyl group is replaced with a bond to a cycloalkenyl group as defined above.

Alkynyl groups include straight and branched chain alkyl groups, except that at least one triple bond exists between two carbon atoms. Thus, alkynyl groups have from 2 to about 20 carbon atoms, and typically from 2 to 12 carbons or, in some embodiments, from 2 to 8 carbon atoms. Examples include, but are not limited to -C≡CH, -C≡C(CH₃), - C≡C(CH₂CH₃), -CH₂C≡CH, -CH₂C≡C(CH₃), and -CH₂C≡C(CH₂CH₃) among others.

The term "heteroalkyl" by itself or in combination with another term means, unless otherwise stated, a stable straight or branched chain alkyl group consisting of the stated number of carbon atoms and one or two heteroatoms selected from the group consisting of O, N, and S, and wherein the nitrogen and sulfur atoms may be optionally oxidized and the nitrogen heteroatom may be optionally quaternized. The heteroatom(s) may be placed at any position of the heteroalkyl group, including between the rest of the heteroalkyl group and the fragment to which it is attached, as well as attached to the most distal carbon atom in the heteroalkyl group. Examples
include: -O-CH₂-CH₂-CH₃, -CH₂-CH₂CH₂-OH, -CH₂-CH₂-NH-CH₃, -CH₂-S-CH₂-CH₃, -CH₂CH₂ -S(=O)-CH₃, and -CH₂CH₂-O-CH₂CH₂-O-CH₃. Up to two heteroatoms may be consecutive, such as, for example, -CH₂-NH-OCH₃, or -CH₂-CH₂-S-S-CH₃.

A "heterocycloalkyl" ring is a cycloalkyl ring containing at least one heteroatom. A heterocycloalkyl ring can also be termed a "heterocyclyl," described below.

The term "heteroalkenyl" by itself or in combination with another term means, unless otherwise stated, a stable straight or branched chain monounsaturated or di-unsaturated hydrocarbon group consisting of the stated number of carbon atoms and one or two heteroatoms selected from the group consisting of O, N, and S, and wherein the nitrogen and sulfur atoms may optionally be oxidized and the nitrogen heteroatom may optionally be quaternized. Up to two heteroatoms may be placed consecutively. Examples
include -CH=CH-O-CH₃, -CH=CH-CH₂-OH, -CH₂-CH=N-OCH₃, -CH=CH-N(CH₃)-CH₃, -CH₂-CH=CH-CH₂-SH, and -CH=CH-O-CH₂CH₂-O-CH₃.

Aryl groups are cyclic aromatic hydrocarbons that do not contain heteroatoms in the ring. Thus aryl groups include, but are not limited to, phenyl, azulenyl, heptalenyl, biphenyl, indacenyl, fluorenyl, phenanthrenyl, triphenylenyl, pyrenyl, naphthacenyl, chrysenyl, biphenylenyl, anthracenyl, and naphthyl groups. In some embodiments, aryl groups contain about 6 to about 14 carbons in the ring portions of the groups. Aryl groups can be unsubstituted or substituted, as defined above. Representative substituted aryl groups can be mono-substituted or substituted more than once, such as, but not limited to, 2-, 3-, 4-, 5-, or 6-substituted phenyl or 2-8 substituted naphthyl groups, which can be substituted with carbon or non-carbon groups such as those listed above.

Aralkyl groups are alkyl groups as defined above in which a hydrogen or carbon bond of an alkyl group is replaced with a bond to an aryl group as defined above. Representative aralkyl groups include benzyl and phenylethyl groups and fused (cycloalkylaryl)alkyl groups such as 4-ethyl-indanyl. Aralkenyl group are alkenyl groups as defined above in which a hydrogen or carbon bond of an alkyl group is replaced with a bond to an aryl group as defined above.

Heterocyclyl groups or the term "heterocyclyl" includes aromatic and non-aromatic ring compounds containing 3 or more ring members, of which, one or more is a heteroatom such as, but not limited to, N, O, and S. Thus a heterocyclyl can be a heterocycloalkyl, or a heteroaryl, or if polycyclic, any combination thereof. In some embodiments, heterocyclyl groups include 3 to about 20 ring members, whereas other such groups have 3 to about 15 ring members. A heterocyclyl group designated as a C₂-heterocyclyl can be a 5-ring with two carbon atoms and three heteroatoms, a 6-ring with two carbon atoms and four heteroatoms and so forth. Likewise a C₄-heterocyclyl can be a 5-ring with one heteroatom, a 6-ring with two heteroatoms, and so forth. The number of carbon atoms plus the number of heteroatoms sums up to equal the total number of ring atoms. A heterocyclyl ring can also include one or more double bonds. A heteroaryl ring is an embodiment of a heterocyclyl group. The phrase "heterocyclyl group" includes fused ring species including those comprising fused aromatic and non-aromatic groups. For example, a dioxolanyl ring and a benzdioxolanyl ring system (methylenedioxyphenyl ring system) are both heterocyclyl groups within the meaning herein. The phrase also includes polycyclic ring systems containing a heteroatom such as, but not limited to, quinuclidyl. Heterocyclyl groups can be unsubstituted, or can be substituted as discussed above. Heterocyclyl groups include, but are not limited to, pyrrolidinyl, piperidinyl, piperazinyl, morpholinyl, pyrrolyl, pyrazolyl, triazolyl, tetrazolyl, oxazolyl, isoxazolyl, thiazolyl, pyridinyl, thiophenyl, benzothiophenyl, benzofuranyl, dihydrobenzofuranyl, indolyl, dihydroindolyl, azaindolyl, indazolyl, benzimidazolyl, azabenzimidazolyl, benzoxazolyl, benzothiazolyl, benzothiadiazolyl, imidazopyridinyl, isoxazolopyridinyl, thianaphthalenyl, purinyl, xanthinyl, adeninyl, guaninyl, quinolinyl, isoquinolinyl, tetrahydroquinolinyl, quinoxalinyl, and quinazolinyl groups. Representative substituted heterocyclyl groups can be mono-substituted or substituted more than once, such as, but not limited to, piperidinyl or quinolinyl groups, which are 2-, 3-, 4-, 5-, or 6-substituted, or disubstituted with groups such as those listed above.

Heteroaryl groups are aromatic ring compounds containing 5 or more ring members, of which, one or more is a heteroatom such as, but not limited to, N, O, and S; for instance, heteroaryl rings can have 5 to about 8-12 ring members. A heteroaryl group is a variety of a heterocyclyl group that possesses an aromatic electronic structure. A heteroaryl group designated as a C₂-heteroaryl can be a 5-ring with two carbon atoms and three heteroatoms, a 6-ring with two carbon atoms and four heteroatoms and so forth. Likewise a C₄-heteroaryl can be a 5-ring with one heteroatom, a 6-ring with two heteroatoms, and so forth. The number of carbon atoms plus the number of heteroatoms sums up to equal the total number of ring atoms. Heteroaryl groups include, but are not limited to, groups such as pyrrolyl, pyrazolyl, triazolyl, tetrazolyl, oxazolyl, isoxazolyl, thiazolyl, pyridinyl, thiophenyl, benzothiophenyl, benzofuranyl, indolyl, azaindolyl, indazolyl, benzimidazolyl, azabenzimidazolyl, benzoxazolyl, benzothiazolyl, benzothiadiazolyl, imidazopyridinyl, isoxazolopyridinyl, thianaphthalenyl, purinyl, xanthinyl, adeninyl, guaninyl, quinolinyl, isoquinolinyl, tetrahydroquinolinyl, quinoxalinyl, and quinazolinyl groups. Heteroaryl groups can be unsubstituted, or can be substituted with groups as is discussed above. Representative substituted heteroaryl groups can be substituted one or more times with groups such as those listed above.

Additional examples of aryl and heteroaryl groups include but are not limited to phenyl, biphenyl, indenyl, naphthyl (1-naphthyl, 2-naphthyl), N-hydroxytetrazolyl, N-hydroxytriazolyl, N-hydroxyimidazolyl, anthracenyl (1-anthracenyl, 2-anthracenyl, 3-anthracenyl), thiophenyl (2-thienyl, 3-thienyl), furyl (2-furyl, 3-furyl) , indolyl, oxadiazolyl, isoxazolyl, quinazolinyl, fluorenyl, xanthenyl, isoindanyl, benzhydryl, acridinyl, thiazolyl, pyrrolyl (2-pyrrolyl), pyrazolyl (3-pyrazolyl), imidazolyl (1-imidazolyl, 2-imidazolyl, 4-imidazolyl, 5-imidazolyl), triazolyl (1,2,3-triazol-1-yl, 1,2,3-triazol-2-yl 1,2,3-triazol-4-yl, 1,2,4-triazol-3-yl), oxazolyl (2-oxazolyl, 4-oxazolyl, 5-oxazolyl), thiazolyl (2-thiazolyl, 4-thiazolyl, 5-thiazolyl), pyridyl (2-pyridyl, 3-pyridyl, 4-pyridyl), pyrimidinyl (2-pyrimidinyl, 4-pyrimidinyl, 5-pyrimidinyl, 6-pyrimidinyl), pyrazinyl, pyridazinyl (3- pyridazinyl, 4-pyridazinyl, 5-pyridazinyl), quinolyl (2-quinolyl, 3-quinolyl, 4-quinolyl, 5-quinolyl, 6-quinolyl, 7-quinolyl, 8-quinolyl), isoquinolyl (1-isoquinolyl, 3-isoquinolyl, 4-isoquinolyl, 5-isoquinolyl, 6-isoquinolyl, 7-isoquinolyl, 8-isoquinolyl), benzo[b]furanyl (2-benzo[b]furanyl, 3-benzo[b]furanyl, 4-benzo[b]furanyl, 5-benzo[b]furanyl, 6-benzo[b]furanyl, 7-benzo[b]furanyl), 2,3-dihydro-benzo[b]furanyl (2-(2,3-dihydro-benzo[b]furanyl), 3-(2,3-dihydro-benzo[b]furanyl), 4-(2,3-dihydro-benzo[b]furanyl), 5-(2,3-dihydro-benzo[b]furanyl), 6-(2,3-dihydro-benzo[b]furanyl), 7-(2,3-dihydro-benzo[b]furanyl), benzo[b]thiophenyl (2-benzo[b]thiophenyl, 3-benzo[b]thiophenyl, 4-benzo[b]thiophenyl, 5-benzo[b]thiophenyl, 6-benzo[b]thiophenyl, 7-benzo[b]thiophenyl), 2,3-dihydro-benzo[b]thiophenyl, (2-(2,3-dihydro-benzo[b]thiophenyl), 3-(2,3-dihydro-benzo[b]thiophenyl), 4-(2,3-dihydro-benzo[b]thiophenyl), 5-(2,3-dihydro-benzo[b]thiophenyl), 6-(2,3-dihydro-benzo[b]thiophenyl), 7-(2,3-dihydro-benzo[b]thiophenyl), indolyl (1-indolyl, 2-indolyl, 3-indolyl, 4-indolyl, 5-indolyl, 6-indolyl, 7-indolyl), indazole (1-indazolyl, 3-indazolyl, 4-indazolyl, 5-indazolyl, 6-indazolyl, 7-indazolyl), benzimidazolyl (1-benzimidazolyl, 2-benzimidazolyl, 4-benzimidazolyl, 5-benzimidazolyl, 6-benzimidazolyl, 7-benzimidazolyl, 8-benzimidazolyl), benzoxazolyl (1-benzoxazolyl, 2-benzoxazolyl), benzothiazolyl (1-benzothiazolyl, 2-benzothiazolyl, 4-benzothiazolyl, 5-benzothiazolyl, 6-benzothiazolyl, 7-benzothiazolyl), carbazolyl (1-carbazolyl, 2-carbazolyl, 3-carbazolyl, 4-carbazolyl), 5H-dibenz[b,f]azepine (5H-dibenz[b,f]azepin-1-yl, 5H-dibenz[b,f]azepine-2-yl, 5H-dibenz[b,f]azepine-3-yl, 5H-dibenz[b,f]azepine-4-yl, 5H-dibenz[b,f]azepine-5-yl), 10,11-dihydro-5H-dibenz[b,f]azepine (10,11-dihydro-5H-dibenz[b,f]azepine-1-yl, 10,11-dihydro-5H-dibenz[b,f]azepine-2-yl, 10,11-dihydro-5H-dibenz[b,f]azepine-3-yl, 10,11-dihydro-5H-dibenz[b,f]azepine-4-yl, 10,11-dihydro-5H-dibenz[b,f]azepine-5-yl), and the like.

Heterocyclylalkyl groups are alkyl groups as defined above in which a hydrogen or carbon bond of an alkyl group as defined above is replaced with a bond to a heterocyclyl group as defined above. Representative heterocyclyl alkyl groups include, but are not limited to, furan-2-yl methyl, furan-3-yl methyl, pyridine-3-yl methyl, tetrahydrofuran-2-yl ethyl, and indol-2-yl propyl.

Heteroarylalkyl groups are alkyl groups as defined above in which a hydrogen or carbon bond of an alkyl group is replaced with a bond to a heteroaryl group as defined above.

The term "alkoxy" refers to an oxygen atom connected to an alkyl group, including a cycloalkyl group, as are defined above. Examples of linear alkoxy groups include but are not limited to methoxy, ethoxy, propoxy, butoxy, pentyloxy, hexyloxy, and the like. Examples of branched alkoxy include but are not limited to isopropoxy, sec-butoxy, tert-butoxy, isopentyloxy, isohexyloxy, and the like. Examples of cyclic alkoxy include but are not limited to cyclopropyloxy, cyclobutyloxy, cyclopentyloxy, cyclohexyloxy, and the like. An alkoxy group can include one to about 12-20 carbon atoms bonded to the oxygen atom, and can further include double or triple bonds, and can also include heteroatoms. For example, an allyloxy group is an alkoxy group within the meaning herein. A methoxyethoxy group is also an alkoxy group within the meaning herein, as is a methylenedioxy group in a context where two adjacent atoms of a structures are substituted therewith.

The term "thioalkoxy" refers to an alkyl group previously defined attached to the parent molecular moiety through a sulfur atom.

The term "glycosyloxyoxy" refers to a glycoside attached to the parent molecular moiety through an oxygen atom.

The term "alkoxycarbonyl" represents as ester group; i.e. an alkoxy group, attached to the parent molecular moiety through a carbonyl group such as methoxycarbonyl, ethoxycarbonyl, and the like.

The terms "halo" or "halogen" or "halide" by themselves or as part of another substituent mean, unless otherwise stated, a fluorine, chlorine, bromine, or iodine atom, preferably, fluorine, chlorine, or bromine.

A "haloalkyl" group includes mono-halo alkyl groups, poly-halo alkyl groups wherein all halo atoms can be the same or different, and per-halo alkyl groups, wherein all hydrogen atoms are replaced by halogen atoms, such as fluoro. Examples of haloalkyl include trifluoromethyl, 1,1-dichloroethyl, 1,2-dichloroethyl, 1,3-dibromo-3,3-difluoropropyl, perfluorobutyl, and the like.

A "haloalkoxy" group includes mono-halo alkoxy groups, poly-halo alkoxy groups wherein all halo atoms can be the same or different, and per-halo alkoxy groups, wherein all hydrogen atoms are replaced by halogen atoms, such as fluoro. Examples of haloalkoxy include trifluoromethoxy, 1,1-dichloroethoxy, 1,2-dichloroethoxy, 1,3-dibromo-3,3-difluoropropoxy, perfluorobutoxy, and the like.

The term "(Cₓ-C_{y})perfluoroalkyl," wherein x < y, means an alkyl group with a minimum of x carbon atoms and a maximum of y carbon atoms, wherein all hydrogen atoms are replaced by fluorine atoms. Preferred is -(C₁-C₆)perfluoroalkyl, more preferred is -(C₁-C₃)perfluoroalkyl, most preferred is -CF₃.

The term "(Cₓ-C_{y})perfluoroalkylene," wherein x < y, means an alkyl group with a minimum of x carbon atoms and a maximum of y carbon atoms, wherein all hydrogen atoms are replaced by fluorine atoms. Preferred is -(C₁-C₆)perfluoroalkylene, more preferred is -(C₁-C₃)perfluoroalkylene, most preferred is -CF₂-.

The terms "aryloxy" and "arylalkoxy" refer to, respectively, an aryl group bonded to an oxygen atom and an aralkyl group bonded to the oxygen atom at the alkyl moiety. Examples include but are not limited to phenoxy, naphthyloxy, and benzyloxy.

An "acyl" group as the term is used herein refers to a group containing a carbonyl moiety wherein the group is bonded via the carbonyl carbon atom. The carbonyl carbon atom is also bonded to another carbon atom, which can be part of an alkyl, aryl, aralkyl cycloalkyl, cycloalkylalkyl, heterocyclyl, heterocyclylalkyl, heteroaryl, heteroarylalkyl group or the like. In the special case wherein the carbonyl carbon atom is bonded to a hydrogen, the group is a "formyl" group, an acyl group as the term is defined herein. An acyl group can include 0 to about 12-20 additional carbon atoms bonded to the carbonyl group. An acyl group can include double or triple bonds within the meaning herein. An acryloyl group is an example of an acyl group. An acyl group can also include heteroatoms within the meaning here. A nicotinoyl group (pyridyl-3-carbonyl) group is an example of an acyl group within the meaning herein. Other examples include acetyl, benzoyl, phenylacetyl, pyridylacetyl, cinnamoyl, and acryloyl groups and the like. When the group containing the carbon atom that is bonded to the carbonyl carbon atom contains a halogen, the group is termed a "haloacyl" group. An example is a trifluoroacetyl group.

The term "amine" includes primary, secondary, and tertiary amines having, e.g., the formula N(group)₃ wherein each group can independently be H or non-H, such as alkyl, aryl, and the like. Amines include but are not limited to R-NH₂, for example, alkylamines, arylamines, alkylarylamines; R₂NH wherein each R is independently selected, such as dialkylamines, diarylamines, aralkylamines, heterocyclylamines and the like; and R₃N wherein each R is independently selected, such as trialkylamines, dialkylarylamines, alkyldiarylamines, triarylamines, and the like. The term "amine" also includes ammonium ions as used herein.

An "amino" group is a substituent of the form -NH₂, -NHR, -NR₂, -NR₃⁺, wherein each R is independently selected, and protonated forms of each, except for -NR₃⁺, which cannot be protonated. Accordingly, any compound substituted with an amino group can be viewed as an amine. An "amino group" within the meaning herein can be a primary, secondary, tertiary or quaternary amino group. An "alkylamino" group includes a monoalkylamino, dialkylamino, and trialkylamino group.

An "ammonium" ion includes the unsubstituted ammonium ion NH₄⁺, but unless otherwise specified, it also includes any protonated or quaternarized forms of amines. Thus, trimethylammonium hydrochloride and tetramethylammonium chloride are both ammonium ions, and amines, within the meaning herein.

The term "amide" (or "amido") includes C- and N-amide groups, i.e., -C(O)NR₂, and - NRC(O)R groups, respectively. Amide groups therefore include but are not limited to primary carboxamide groups (-C(O)NH₂) and formamide groups (-NHC(O)H). A "carboxamido" or "aminocarbonyl" group is a group of the formula C(O)NR₂, wherein R can be H, alkyl, aryl, etc.

The term "azido" refers to an N₃ group. An "azide" can be an organic azide or can be a salt of the azide (N₃⁻) anion. The term "nitro" refers to an NO₂ group bonded to an organic moiety. The term "nitroso" refers to an NO group bonded to an organic moiety. The term nitrate refers to an ONO₂ group bonded to an organic moiety or to a salt of the nitrate (NO₃⁻) anion.

The term "urethane" ("carbamoyl" or "carbamyl") includes N- and O-urethane groups, i.e., -NRC(O)OR and -OC(O)NR₂ groups, respectively.

The term "sulfonamide" (or "sulfonamido") includes S- and N-sulfonamide groups, i.e., -SO₂NR₂ and -NRSO₂R groups, respectively. Sulfonamide groups therefore include but are not limited to sulfamoyl groups (-SO₂NH₂). An organosulfur structure represented by the formula -S(O)(NR)- is understood to refer to a sulfoximine, wherein both the oxygen and the nitrogen atoms are bonded to the sulfur atom, which is also bonded to two carbon atoms.

The term "amidine" or "amidino" includes groups of the formula -C(NR)NR₂. Typically, an amidino group is -C(NH)NH₂.

The term "guanidine" or "guanidino" includes groups of the formula -NRC(NR)NR₂. Typically, a guanidino group is -NHC(NH)NH₂.

The term "ring derived from a sugar" refers to a compound that forms a ring by removing the hydrogen atoms from two hydroxyl groups of any sugar.

A "salt" as is well known in the art includes an organic compound such as a carboxylic acid, a sulfonic acid, or an amine, in ionic form, in combination with a counterion. For example, acids in their anionic form can form salts with cations such as metal cations, for example sodium, potassium, and the like; with ammonium salts such as NH₄⁺ or the cations of various amines, including tetraalkyl ammonium salts such as tetramethylammonium, or other cations such as trimethylsulfonium, and the like. A "pharmaceutically acceptable" or "pharmacologically acceptable" salt is a salt formed from an ion that has been approved for human consumption and is generally non-toxic, such as a chloride salt or a sodium salt. A "zwitterion" is an internal salt such as can be formed in a molecule that has at least two ionizable groups, one forming an anion and the other a cation, which serve to balance each other. For example, amino acids such as glycine can exist in a zwitterionic form. A "zwitterion" is a salt within the meaning herein. The compounds described herein may take the form of salts. The term "salts" embraces addition salts of free acids or free bases which are compounds described herein. Salts can be "pharmaceutically-acceptable salts." The term "pharmaceutically-acceptable salt" refers to salts which possess toxicity profiles within a range that affords utility in pharmaceutical applications. Pharmaceutically unacceptable salts may nonetheless possess properties such as high crystallinity, which have utility in the practice of the present disclosure, such as for example utility in process of synthesis, purification or formulation of compounds of the present disclosure.

Suitable pharmaceutically-acceptable acid addition salts may be prepared from an inorganic acid or from an organic acid. Examples of inorganic acids include hydrochloric, hydrobromic, hydriodic, nitric, carbonic, sulfuric, and phosphoric acids. Appropriate organic acids may be selected from aliphatic, cycloaliphatic, aromatic, araliphatic, heterocyclic, carboxylic and sulfonic classes of organic acids, examples of which include formic, acetic, propionic, succinic, glycolic, gluconic, lactic, malic, tartaric, citric, ascorbic, glucuronic, maleic, fumaric, pyruvic, aspartic, glutamic, benzoic, anthranilic, 4-hydroxybenzoic, phenylacetic, mandelic, embonic (pamoic), methanesulfonic, ethanesulfonic, benzenesulfonic, pantothenic, trifluoromethanesulfonic, 2-hydroxyethanesulfonic, p-toluenesulfonic, sulfanilic, cyclohexylaminosulfonic, stearic, alginic, β-hydroxybutyric, salicylic, galactaric and galacturonic acid. Examples of pharmaceutically unacceptable acid addition salts include, for example, perchlorates and tetrafluoroborates.

Suitable pharmaceutically acceptable base addition salts of compounds of the present disclosure include, for example, metallic salts including alkali metal, alkaline earth metal and transition metal salts such as, for example, calcium, magnesium, potassium, sodium and zinc salts. Pharmaceutically acceptable base addition salts also include organic salts made from basic amines such as, for example, *N,N'*-dibenzylethylenediamine, chloroprocaine, choline, diethanolamine, ethylenediamine, meglumine (N-methylglucamine) and procaine. Examples of pharmaceutically unacceptable base addition salts include lithium salts and cyanate salts. Although pharmaceutically unacceptable salts are not generally useful as medicaments, such salts may be useful, for example as intermediates in the synthesis of Formula (I), (I'), (Ia), (Ib), (Ic), (Id), (II), (II'), (IIa), (IIb), (IIc), (IId), or (IIe) compounds, for example in their purification by recrystallization. All of these salts may be prepared by conventional means from the corresponding compound according to Formula (I), (I'), (Ia), (Ib), (Ic), (Id), (II), (II'), (IIa), (IIb), (IIc), (IId), or (IIe) by reacting, for example, the appropriate acid or base with the compound according to Formula (I), (I'), (Ia), (Ib), (Ic), (Id), (II), (II'), (IIa), (IIb), (IIc), (IId), or (IIe). The term "pharmaceutically acceptable salts" refers to nontoxic inorganic or organic acid and/or base addition salts, see, for example, Lit et al., Salt Selection for Basic Drugs (1986), Int J. Pharm., 33, 201-217.

A "hydrate" is a compound that exists in a composition with water molecules. The composition can include water in stoichiometic quantities, such as a monohydrate or a dihydrate, or can include water in random amounts. As the term is used herein a "hydrate" refers to a solid form, i.e., a compound in water solution, while it may be hydrated, is not a hydrate as the term is used herein.

A "solvate" is a similar composition except that a solvent other that water replaces the water. For example, methanol or ethanol can form an "alcoholate", which can again be stoichiometic or non-stoichiometric. As the term is used herein a "solvate" refers to a solid form, i.e., a compound in solution in a solvent, while it may be solvated, is not a solvate as the term is used herein.

A "prodrug" as is well known in the art is a substance that can be administered to a patient where the substance is converted in vivo by the action of biochemicals within the patient's body, such as enzymes, to the active pharmaceutical ingredient. Examples of prodrugs include esters of carboxylic acid groups, which can be hydrolyzed by endogenous esterases as are found in the bloodstream of humans and other mammals. Further examples of prodrugs include boronate esters which can be hydrolyzed under physiological conditions to afford the corresponding boronic acid. Conventional procedures for the selection and preparation of suitable prodrug derivatives are described, for example, in "Design of Prodrugs", ed. H. Bundgaard, Elsevier, 1985.

In addition, where features or aspects of the present disclosure are described in terms of Markush groups, those skilled in the art will recognize that the presently described compounds is also thereby described in terms of any individual member or subgroup of members of the Markush group. For example, if X is described as selected from the group consisting of bromine, chlorine, and iodine, claims for X being bromine and claims for X being bromine and chlorine are fully described. Moreover, where features or aspects of the present disclosure are described in terms of Markush groups, those skilled in the art will recognize that the present disclosure is also thereby described in terms of any combination of individual members or subgroups of members of Markush groups. Thus, for example, if X is described as selected from the group consisting of bromine, chlorine, and iodine, and Y is described as selected from the group consisting of methyl, ethyl, and propyl, claims for X being bromine and Y being methyl are fully described.

If a value of a variable that is necessarily an integer, e.g., the number of carbon atoms in an alkyl group or the number of substituents on a ring, is described as a range, e.g., 0-4, what is meant is that the value can be any integer between 0 and 4 inclusive, i.e., 0, 1, 2, 3, or 4.

Compounds of the present invention are listed in the appended claims. Also generally disclosed and described herein, but encompassing compounds which are not explicitly claimed, are further compounds of general formulae (I), (I'), (Ia), (Ib), (Ic), (Id), (II), (II'), (IIa), (IIb), (IIc), (IId), or (IIe). The present disclosure further embraces isolated compounds according to Formula (I), (I'), (Ia), (Ib), (Ic), (Id), (II), (II'), (IIa), (IIb), (IIc), (IId), or (IIe). The expression "isolated compound" refers to a preparation of a compound of Formula (I), (I'), (Ia), (Ib), (Ic), (Id), (II), (II'), (Ila), (IIb), (IIc), (IId), or (IIe), or a mixture of compounds according to Formula (I), (I'), (Ia), (Ib), (Ic), (Id), (II), (II'), (Ila), (IIb), (IIc), (IId), or (IIe), wherein the isolated compound has been separated from the reagents used, and/or byproducts formed, in the synthesis of the compound or compounds. "Isolated" does not mean that the preparation is technically pure (homogeneous), but it is sufficiently pure to compound in a form in which it can be used therapeutically. Preferably an "isolated compound" refers to a preparation of a compound of Formula (I), (I'), (Ia), (Ib), (Ic), (Id), (II), (II'), (IIa), (IIb), (IIc), (IId), or (IIe) or a mixture of compounds according to Formula (I), (I'), (Ia), (Ib), (Ic), (Id), (II), (II'), (IIa), (IIb), (IIc), (IId), or (IIe), which contains the named compound or mixture of compounds according to Formula (I), (I'), (Ia), (Ib), (Ic), (Id), (II), (II'), (IIa), (IIb), (IIc), (IId), or (IIe) in an amount of at least 10 percent by weight of the total weight. Preferably the preparation contains the named compound or mixture of compounds in an amount of at least 50 percent by weight of the total weight; more preferably at least 80 percent by weight of the total weight; and most preferably at least 90 percent, at least 95 percent or at least 98 percent by weight of the total weight of the preparation.

The compounds described herein and intermediates may be isolated from their reaction mixtures and purified by standard techniques such as filtration, liquid-liquid extraction, solid phase extraction, distillation, recrystallization or chromatography, including flash column chromatography, or HPLC.

### Isomerism and Tautomerism in Compounds Described Herein

### Tautomerism

Within the present disclosure it is to be understood that a compound of Formula (I), (I'), (Ia), (Ib), (Ic), (Id), (II), (II'), (Ila), (IIb), (IIc), (IId), or (IIe) or a salt thereof may exhibit the phenomenon of tautomerism whereby two chemical compounds that are capable of facile interconversion by exchanging a hydrogen atom between two atoms, to either of which it forms a covalent bond. Since the tautomeric compounds exist in mobile equilibrium with each other they may be regarded as different isomeric forms of the same compound. It is to be understood that the formulae drawings within this specification can represent only one of the possible tautomeric forms. However, it is also to be understood that the present disclosure encompasses any tautomeric form, and is not to be limited merely to any one tautomeric form utilized within the formulae drawings. The formulae drawings within this specification can represent only one of the possible tautomeric forms and it is to be understood that the specification encompasses all possible tautomeric forms of the compounds drawn not just those forms which it has been convenient to show graphically herein. For example, tautomerism may be exhibited by a pyrazolyl group bonded as indicated by the wavy line. While both substituents would be termed a 4-pyrazolyl group, it is evident that a different nitrogen atom bears the hydrogen atom in each structure.

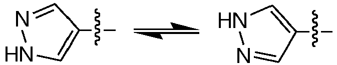

Such tautomerism can also occur with substituted pyrazoles such as 3-methyl, 5-methyl, or 3,5-dimethylpyrazoles, and the like. Another example of tautomerism is amido-imido (lactam-lactim when cyclic) tautomerism, such as is seen in heterocyclic compounds bearing a ring oxygen atom adjacent to a ring nitrogen atom. For example, the equilibrium: 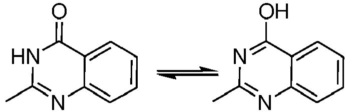 is an example of tautomerism. Accordingly, a structure depicted herein as one tautomer is intended to also include the other tautomer.

### Optical Isomerism

It will be understood that when compounds of the present disclosure contain one or more chiral centers, the compounds may exist in, and may be isolated as pure enantiomeric or diastereomeric forms or as racemic mixtures. The present disclosure therefore includes any possible enantiomers, diastereomers, racemates or mixtures thereof of the compounds described herein.

The isomers resulting from the presence of a chiral center comprise a pair of non-superimposable isomers that are called "enantiomers." Single enantiomers of a pure compound are optically active, *i.e.,* they are capable of rotating the plane of plane polarized light. Single enantiomers are designated according to the *Cahn-Ingold-Prelog* system. The priority of substituents is ranked based on atomic weights, a higher atomic weight, as determined by the systematic procedure, having a higher priority ranking. Once the priority ranking of the four groups is determined, the molecule is oriented so that the lowest ranking group is pointed away from the viewer. Then, if the descending rank order of the other groups proceeds clockwise, the molecule is designated (R) and if the descending rank of the other groups proceeds counterclockwise, the molecule is designated (*S*). In the example below, the *Cahn-Ingold-Prelog* ranking is A > B > C > D. The lowest ranking atom, D is oriented away from the viewer.

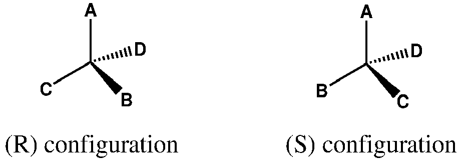

The present disclosure is meant to encompass diastereomers as well as their racemic and resolved, diastereomerically and enantiomerically pure forms and salts thereof. Diastereomeric pairs may be resolved by known separation techniques including normal and reverse phase chromatography, and crystallization.

"Isolated optical isomer" means a compound which has been substantially purified from the corresponding optical isomer(s) of the same formula. Preferably, the isolated isomer is at least about 80%, more preferably at least 90% pure, even more preferably at least 98% pure, most preferably at least about 99% pure, by weight.

Isolated optical isomers may be purified from racemic mixtures by well-known chiral separation techniques. According to one such method, a racemic mixture of a compound described herein, or a chiral intermediate thereof, is separated into 99% wt.% pure optical isomers by HPLC using a suitable chiral column, such as a member of the series of DAICEL^{®} CHIRALPAK^{®} family of columns (Daicel Chemical Industries, Ltd., Tokyo, Japan). The column is operated according to the manufacturer's instructions.

### Rotational Isomerism

It is understood that due to chemical properties (i.e., resonance lending some double bond character to the C-N bond) of restricted rotation about the amide bond linkage (as illustrated below) it is possible to observe separate rotamer species and even, under some circumstances, to isolate such species (see below). It is further understood that certain structural elements, including steric bulk or substituents on the amide nitrogen, may enhance the stability of a rotamer to the extent that a compound may be isolated as, and exist indefinitely, as a single stable rotamer. The present disclosure therefore includes any possible stable rotamers of formula (I) which are biologically active in the treatment of cancer or other proliferative disease states.

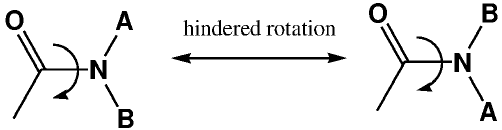

### Regioisomerism

In some embodiments, the compounds described herein have a particular spatial arrangement of substituents on the aromatic rings, which is related to the structure activity relationship demonstrated by the compound class. Often such substitution arrangement is denoted by a numbering system; however, numbering systems are often not consistent between different ring systems. In six-membered aromatic systems, the spatial arrangements are specified by the common nomenclature "para" for 1,4-substitution, "meta" for 1,3-substitution and "ortho" for 1,2-substitution as shown below.

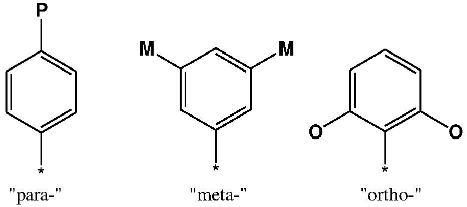

The compound or set of compounds described herein can be any one of any of the combinations and/or sub-combinations of the above-listed embodiments.

### Compounds

Compounds of the present invention are the compounds listed in the appended claims. Also generally disclosed and described herein, but encompassing compounds which are not explicitly claimed, are further compounds of general formulae (I), (I'), (Ia), (Ib), (Ic), (Id), (II), (II'), (Ila), (IIb), (IIc), (IId), or (IIe). Accordingly, generally described herein are compounds of Formula (I): 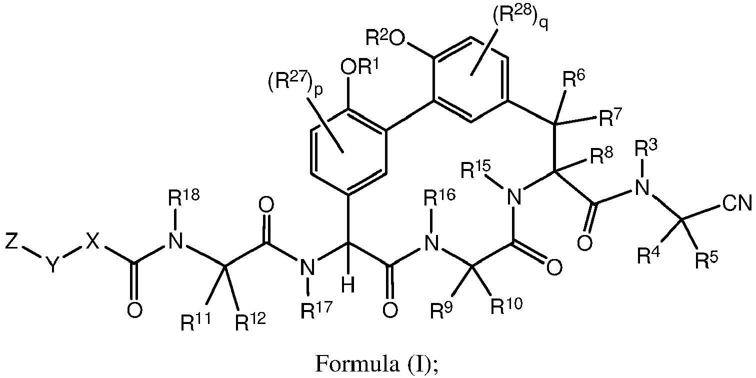 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, -(C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²², or -(C₁-C₆)alkyl-C(O)N(R²³)(C₁-C₆)alkyl, or optionally substituted heterocycloalkyl;
R³ is H, or -(C₁-C₆)alkyl;
R⁴ is H, -(C₁-C₆)alkyl, -(C₁-C₆)alkyl-OH, -(C₃-C₆)cycloalkyl , or -C(O)NH₂; or R³ and R⁴ are combined to form a heterocycloalkyl ring;
R⁵ is H, or -(C₁-C₆)alkyl; or R⁴ and R⁵ and the carbon atom to which they are attached form a cyclopropyl ring;
R⁶, R⁷, and R⁸ are each independently H, or -(C₁-C₆)alkyl;
R⁹ is H, -(C₁-C₆)alkyl, -(C₁-C₆)haloalkyl, or -(C₃-C₆)cycloalkyl;
R¹⁰ is H, or -(C₁-C₆)alkyl;
R¹¹ and R¹² are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-CN, -(C₁-C₆)alkyl-C(O)NR²⁵R²⁶, -(C₁-C₆)heteroalkyl-CO₂H, -(C₁-C₆)alkyl-S(O)-(C₁-C₆)alkyl, -(C₁-C₆)alkyl-N(H)CH=NH, -(C₁-C₆)alkyl-N(H)C(NH)NH₂, -(C₁-C₆)alkyl-heterocycloalkyl, optionally substituted -(C₁-C₆)alkyl-N(H)heterocycloalkyl, or -(C₁-C₆)alkyl-heteroaryl; or R¹¹ and R¹⁸ are combined to form an optionally substituted heterocycloalkyl ring, and R¹² is H;
R¹⁵, R¹⁶, R¹⁷, and R¹⁸ are each independently H, -(C₁-C₆)alkyl, -(C₃-C₆)cycloalkyl, -(C₁-C₆)alkyl-OR²³, -(C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, - N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, - SO₂(C₁-C₆)alkyl-, -C(O)NH(C₁-C₆)alkyl-, -(C₃-C₇)cycloalkyl-, optionally substituted - C(O)N(R²⁴)aryl-, optionally substituted -N(R²⁴)C(O)aryl-, optionally substituted -N(R²⁴)SO₂aryl- ,optionally substituted aryl, or optionally substituted heteroaryl;
Z is H, halogen, -NH₂, -CN, -CF₃, -CO₂H, -(C₁-C₁₂)alkyl,-(C₂-C₁₂)alkenyl, -(C₂-C₁₂)alkynyl, - C(O)NR²⁵R²⁶, -O-(C₁-C₁₂)alkyl, -N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, - C(O)(C₁-C₆)alkyl, -C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl; or R²⁵ and R²⁶ and the nitrogen atom to which they are attached form a heterocycloalkyl ring;
each R²⁷ is independently halogen, optionally substituted -(C₁-C₆)alkyl, or optionally substituted - (C₁-C₆)heteroalkyl;
each R²⁸ is independently halogen, optionally substituted -(C₁-C₆)alkyl, or optionally substituted - (C₁-C₆)heteroalkyl;
p is 0, 1, or 2; and
q is 0, 1, or 2;
or a pharmaceutically acceptable salt, solvate, or prodrug thereof.

Also generally described is a compound of Formula (I) wherein R⁶, R⁷, and R⁸ are H.

Also described is a compound of Formula (I) wherein R¹⁵ and R¹⁶ are H.

Also described is a compound of Formula (I) wherein R¹⁷ is -(C₁-C₆)alkyl. Also described is a compound of Formula (I) wherein R¹⁷ is -CH₃. Also described is a compound of Formula (I) wherein R¹⁷ is -CH₂CH₃. Also described is a compound of Formula (I) wherein R¹⁷ is -(C₃-C₆)cycloalkyl. Also described is a compound of Formula (I) wherein R¹⁷ is cyclopropyl. Also described is a compound of Formula (I) wherein R¹⁷ is -(C₁-C₆)alkyl-C(O)OR²³. Also described is a compound of Formula (I) wherein R¹⁷ is -CH₂CH₂OH. Also described is a compound of Formula (I) wherein R¹⁷ is -(C₁-C₆)alkyl-NR²¹R²², Also described is a compound of Formula (I) wherein R¹⁷ is -CH₂CH₂NH₂. Also described is a compound of Formula (I) wherein R¹⁷ is H.

Also described is a compound of Formula (I) wherein R¹⁸ is H.

Also described is a compound of Formula (I) wherein R³ is H.

Also described is a compound of Formula (I) wherein R⁵ is H.

Also described is a compound of Formula (I) wherein R⁴ is H. Also described is a compound of Formula (I) wherein R⁴ is -(C₁-C₆)alkyl. Also described is a compound of Formula (I) wherein R⁴ is -CH₃. Also described is a compound of Formula (I) wherein R⁴ is -CH₂CH₃. Also described is a compound of Formula (I) wherein R⁴ is -(C₁-C₆)alkyl-OH. Also described is a compound of Formula (I) wherein R⁴ is -CH₂OH. Also described t is a compound of Formula (I) wherein R⁴ is -(C₃-C₆)cycloalkyl. Also described is a compound of Formula (I) wherein R⁴ is cyclopropyl. Also described is a compound of Formula (I) wherein R⁴ is -C(O)NH₂.

Also described is a compound of Formula (I) wherein R³, R⁴, and R⁵ are H.

Also described is a compound of Formula (I) wherein R⁴ and R⁵ and the carbon atom to which they are attached form a cyclopropyl ring.

Also described is a compound of Formula (I) wherein R¹⁰ is H.

Also described is a compound of Formula (I) wherein R¹⁰ is H and R⁹ is -(C₁-C₆)alkyl. Also described is a compound of Formula (I) wherein R¹⁰ is H and R⁹ is -CH₃. Also described is a compound of Formula (I) wherein R¹⁰ is H and R⁹ is -CH₂CH₃. Also described is a compound of Formula (I) wherein R¹⁰ is H and R⁹ is -(C₁-C₆)haloalkyl. Also described is a compound of Formula (I) wherein R¹⁰ is H and R⁹ is -CH₂F. Also described is a compound of Formula (I) wherein R¹⁰ is H and R⁹ is -CHF₂. Also described is a compound of Formula (I) wherein R¹⁰ is H and R⁹ is -(C₃-C₆)cycloalkyl. Also described is a compound of Formula (I) wherein R¹⁰ is H and R⁹ is cyclopropyl. Also described is a compound of Formula (I) wherein R¹⁰ is H and R⁹ is H.

Also described is a compound of Formula (I) wherein R¹² is H.

Also described is a compound of Formula (I) wherein R¹² is H and R¹¹ is -(C₁-C₆)alkyl. Also described is a compound of Formula (I) wherein R¹² is H and R¹¹ is -CH₃. Also described is a compound of Formula (I) wherein R¹² is H and R¹¹ is -(C₁-C₆)alkyl-OR²³. Also described is a compound of Formula (I) wherein R¹² is H and R¹¹ is -CH₂OH. Also described is a compound of Formula (I) wherein R¹² is H and R¹¹ is -CH₂CH₂OH. Also described is a compound of Formula (I) wherein R¹² is H and R¹¹ is -(C₁-C₆)alkyl. Also described is a compound of Formula (I) wherein R¹² is H and R¹¹ is -(C₁-C₆)alkyl-NR²¹R²², Also described is a compound of Formula (I) wherein R¹² is H and R¹¹ is -(C₁-C₆)alkyl-NH₂. Also described is a compound of Formula (I) wherein R¹² is H and R¹¹ is -CH₂NH₂. Also described is a compound of Formula (I) wherein R¹² is H and R¹¹ is -CH₂CH₂NH₂. Also described is a compound of Formula (I) wherein R¹² is H and R¹¹ is -CH₂CH₂CH₂NH₂. Also described is a compound of Formula (I) wherein R¹² is H and R¹¹ is -CH₂CH₂CH₂CH₂NH₂. Also described is a compound of Formula (I) wherein R¹² is H and R¹¹ is -(C₁-C₆)alkyl-CN. Also described is a compound of Formula (I) wherein R¹² is H and R¹¹ is - CH₂CN. Also described is a compound of Formula (I) wherein R¹² is H and R¹¹ is -(C₁-C₆)alkyl-C(O)NR²⁵R²⁶, Also described is a compound of Formula (I) wherein R¹² is H and R¹¹ is - CH₂C(O)NH₂. Also described is a compound of Formula (I) wherein R¹² is H and R¹¹ is - CH₂CH₂C(O)NH₂. Also described is a compound of Formula (I) wherein R¹² is H and R¹¹ is - (C₁-C₆)alkyl-heteroaryl. Also described is a compound of Formula (I) wherein R¹² is H and R¹¹ is H.

Also described is a compound of Formula (I) wherein R¹¹ and R¹⁸ are combined to form an optionally substituted heterocycloalkyl ring and R¹² is H.

Also described is a compound of Formula (I) wherein p is 1 and R²⁷ is halogen. Also described is a compound of Formula (I) wherein p is 1 and R²⁷ is optionally substituted -(C₁-C₆)alkyl. Also described is a compound of Formula (I) wherein q is 0, p is 1 and R²⁷ is halogen. Also described is a compound of Formula (I) wherein q is 0, p is 1 and R²⁷ is optionally substituted -(C₁-C₆)alkyl. Also described is a compound of Formula (I) wherein q is 1 and R²⁸ is halogen. Also described is a compound of Formula (I) wherein q is 1 and R²⁸ is optionally substituted -(C₁-C₆)alkyl. Also described is a compound of Formula (I) wherein p is 0, q is 1 and R²⁸ is halogen. Also described is a compound of Formula (I) wherein p is 0, q is 1 and R²⁸ is optionally substituted -(C₁-C₆)alkyl.

Also described is a compound of Formula (I) wherein p is 0, and q is 0.

Also described is a compound of Formula (I) wherein R¹ and R² are each independently H, or -(C₁-C₆)alkyl-NR²¹R²², Also described is a compound of Formula (I) wherein R¹ and R² are each H. Also described is a compound of Formula (I) wherein R¹ and R² are each independently - (C₁-C₆)alkyl-NR²¹R²². Also described is a compound of Formula (I) wherein R¹ is H, and R² is - (C₁-C₆)alkyl-NR²¹R²². Also described is a compound of Formula (I) wherein R¹ is -(C₁-C₆)alkyl-NR²¹R²², and R² is H. Also described is a compound of Formula (I) wherein R¹ is H, and R² is - CH₂CH₂NH₂. Also described is a compound of Formula (I) wherein R¹ is -CH₂CH₂NH₂, and R² is H. Also described is a compound of Formula (I) wherein R¹ and R² are each -CH₂CH₂NH₂. Also described is a compound of Formula (I) wherein R¹ is -(C₁-C₆)alkyl-NR²¹R²² and R² is H. Also described is a compound of Formula (I) wherein R¹ is -CH₂CH₂NH₂ and R² is H. Also described is a compound of Formula (I) wherein R¹ is H and R² is -(C₁-C₆)alkyl-NR²¹R²². Also described is a compound of Formula (I) wherein R¹ is H and R² is -CH₂CH₂NH₂.

Also described is a compound of Formula (I) wherein X is optionally substituted aryl. Also described is a compound of Formula (I) wherein X is optionally substituted phenyl. Also described is a compound of Formula (I) wherein X is optionally substituted heteroaryl. Also described is a compound of Formula (I) wherein X is heteroaryl which is unsubstituted or substituted once or twice with -(C₁-C₆)alkyl. Also described is a compound of Formula (I) wherein X is heteroaryl which is unsubstituted or substituted once with -(C₁-C₆)alkyl. Also described is a compound of Formula (I) wherein X is optionally substituted pyridine or optionally substituted pyrimidine. Also described is a compound of Formula (I) wherein X is pyridine which is unsubstituted or substituted once or twice with -(C₁-C₆)alkyl. Also described is a compound of Formula (I) wherein X is pyridine which is unsubstituted or substituted once with - (C₁-C₆)alkyl. Also described is a compound of Formula (I) wherein X is pyridine which is unsubstituted or substituted once or twice with methyl. Also described is a compound of Formula (I) wherein X is pyrimidine which is unsubstituted or substituted once or twice with -(C₁-C₆)alkyl. Also described is a compound of Formula (I) wherein X is pyrimidine which is unsubstituted or substituted once with -(C₁-C₆)alkyl. Also described is a compound of Formula (I) wherein X is pyrimidine which is unsubstituted or substituted once or twice with methyl. Also described is a compound of Formula (I) wherein X is pyridine which is substituted once with methyl. Also described is a compound of Formula (I) wherein X is pyrimidine which is substituted once with methyl. Also described is a compound of Formula (I) wherein X is pyrimidine which is substituted twice with methyl. Also described is a compound of Formula (I) wherein X is optionally substituted -(C₁-C₆)alkyl-. Also described is a compound of Formula (I) wherein Y is optionally substituted aryl. Also described is a compound of Formula (I) wherein Y is optionally substituted phenyl. Also described is a compound of Formula (I) wherein Y is optionally substituted heteroaryl. Also described is a compound of Formula (I) wherein Y is optionally substituted -(C₁-C₆)alkyl-. Also described is a compound of Formula (I) wherein Y is -O-(C₁-C₆)alkyl-. Also described is a compound of Formula (I) wherein Y is -N(H)-(C₁-C₆)alkyl-. Also described is a compound of Formula (I) wherein Y is a bond. Also described is a compound of Formula (I) wherein Z is -(C₁-C₆)alkyl. Also described is a compound of Formula (I) wherein Z is optionally substituted aryl. Also described is a compound of Formula (I) wherein Z is optionally substituted phenyl. Also described is a compound of Formula (I) wherein Z is phenyl substituted once or twice with -(C₁-C₈)alkyl. Also described is a compound of Formula (I) wherein Z is phenyl substituted once with n-butyl, isobutyl, or tert-butyl. Also described is a compound of Formula (I) wherein Z is phenyl substituted once with n-butyl. Also described is a compound of Formula (I) wherein Z is phenyl substituted once with isobutyl. Also described is a compound of Formula (I) wherein Z is phenyl substituted once with tert-butyl. Also described is a compound of Formula (I) wherein Z is optionally substituted heteroaryl. Also described is a compound of Formula (I) wherein Z is optionally substituted -(C₃-C₇)cycloalkyl. Also described is a compound of Formula (I) wherein Z is halogen.

Also described is a compound of Formula (I) wherein -X-Y-Z is

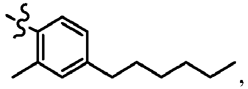

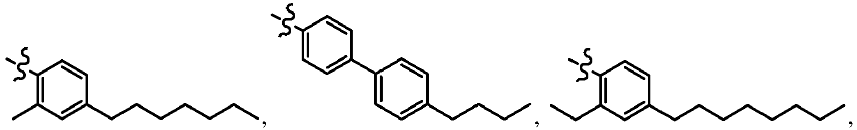

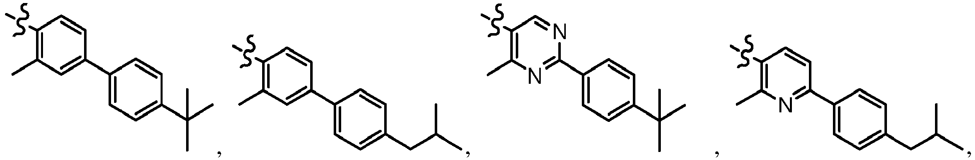

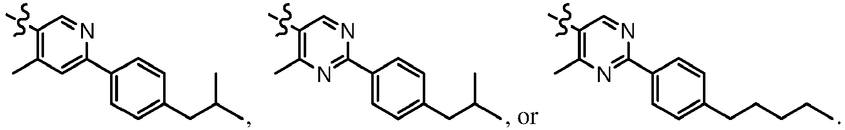

In one aspect described herein are compounds of Formula (I'): 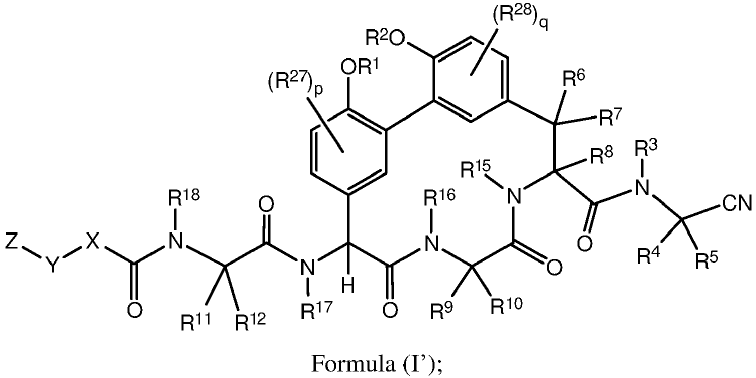 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, -(C₁-C₆)alkyl-NR²¹R²², -(C₁-C₆)alkyl-C(O)NR²⁵R²⁶, -(C₁-C₆)alkyl-N(R²³)C(O)(C₁-C₆)alky1NR²¹R²², -(C₁-C₆)alkyl-C(O)N(R²³)(C₁-C₆)alkyl, -(C₁-C₆)alkyl-C(O)N(R²³)(C₁-C₆)alkyl-heterocycloalkyl, (C₁-C₆)heteroalkyl or optionally substituted heterocycloalkyl;
R³ is H, or -(C₁-C₆)alkyl;
R⁴ is H, -(C₁-C₆)alkyl, -(C₁-C₆)alkyl-OH, -(C₃-C₆)cycloalkyl , or -C(O)NH₂; or R³ and R⁴ are combined to form a heterocycloalkyl ring;
R⁵ is H, or -(C₁-C₆)alkyl; or R⁴ and R⁵ and the carbon atom to which that are attached form a cyclopropyl ring;
R⁶, R⁷, and R⁸ are each independently H, fluoro, hydroxyl, amino, optionally substituted alkyl or heteroalkyl or -(C₁-C₆)alkyl;
R⁹ is H, -(C₁-C₆)alkyl, -(C₁-C₆)haloalkyl, or -(C₃-C₆)cycloalkyl;
R¹⁰ is H, -(C₁-C₆)alkyl, -(C₁-C₆)haloalkyl, or -(C₃-C₆)cycloalkyl;
or R⁹ and R¹⁰ are combined to form a heterocycloalkyl or cycloalkyl ring
R¹¹ and R¹² are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-CN, -(C₁-C₆)alkyl-C(O)NR²⁵R²⁶, -(C₁-C₆)heteroalkyl-CO₂H, -(C₁-C₆)alkyl-S(O)-(C₁-C₆)alkyl, -(C₁-C₆)alkyl-N(H)CH=NH, -(C₁-C₆)alkyl-C(NH₂)=NH, -(C₁-C₆)alkyl-N(H)C(NH)NH₂, -(C₁-C₆)alkyl-N(H)SO₂NR²⁵R²⁶, -(C₁-C₆)alkyl-N(H)-C(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl; or R¹¹ and R¹⁸ are combined to form an optionally substituted heterocycloalkyl ring, and R¹² is H;
R¹⁵, R¹⁶, R¹⁷, and R¹⁸ are each independently H, -(C₁-C₆)alkyl, -(C₃-C₆)cycloalkyl, -(C₁-C₆)alkyl-OR²³, -(C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, - N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, - SO₂(C₁-C₆)alkyl-, -C(O)NH(C₁-C₆)alkyl-, -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-,optionally substituted aryl, or optionally substituted heteroaryl;
Z is H, halogen, -NH₂, -CN, -CF₃, -CO₂H, -(C₁-C₁₂)alkyl,-(C₂-C₁₂)alkenyl, -(C₂-C₁₂)alkynyl, - C(O)NR²⁵R²⁶, -O-(C₁-C₁₂)alkyl, -N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, - C(O)(C₁-C₆)alkyl, -C(O)O(C₁-C₆)alkyl, -C(O)O(C₁-C₆)haloalkyl, -C(=NH)(C₁-C₆)alkyl, - C(=NH)N(R³¹)₂, -C(O)N(R³¹)₂, or -SO₂N(R³¹)₂; or R²¹ and R²² and the nitrogen atom to which that are attached form a heterocycloalkyl ring;
each R³¹ is independently H or -(C₁-C₆)alkyl; or two R³¹ and the nitrogen atom to which that are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl; or R²⁵ and R²⁶ and the nitrogen atom to which that are attached form a heterocycloalkyl ring;
each R²⁷ is independently halogen, -NR²³R²⁴, -NC(O)R²³, -NC(O)NR²³R²⁴), nitro, hydroxyl, optionally substituted -(C₁-C₆)alkyl, optionally substituted -(C₁-C₆)heteroalkyl, -(C₁-C₆)alkoxy, - C(O)(C₁-C₆)alkyl, or -S(O)₂(C₁-C₆)alkyl;
each R²⁸ is independently halogen, -NR²³R²⁴, -NC(O)R²³, -NC(O)NR²³R²⁴), nitro, hydroxyl, optionally substituted -(C₁-C₆)alkyl, optionally substituted -(C₁-C₆)heteroalkyl, -(C₁-C₆)alkoxy, - C(O)(C₁-C₆)alkyl, or -S(O)₂(C₁-C₆)alkyl;
p is 0, 1, or 2; and
q is 0, 1, or 2;
or a pharmaceutically acceptable salt, solvate, or prodrug thereof.

Also described is a compound of Formula (I') wherein R⁶, R⁷, and R⁸ are H.

Also described is a compound of Formula (I') wherein R¹⁵ and R¹⁶ are H.

Also described is a compound of Formula (I') wherein R¹⁷ is -(C₁-C₆)alkyl. Also described is a compound of Formula (I') wherein R¹⁷ is -CH₃. Also described is a compound of Formula (I') wherein R¹⁷ is -CH₂CH₃. Also described is a compound of Formula (I') wherein R¹⁷ is -(C₃-C₆)cycloalkyl. Also described is a compound of Formula (I') wherein R¹⁷ is cyclopropyl. Also described is a compound of Formula (I') wherein R¹⁷ is -(C₁-C₆)alkyl-C(O)OR²³. Also described is a compound of Formula (I') wherein R¹⁷ is -CH₂CH₂OH. Also described is a compound of Formula (I') wherein R¹⁷ is -(C₁-C₆)alkyl-NR²¹R²². Also described is a compound of Formula (I') wherein R¹⁷ is -CH₂CH₂NH₂. Also described is a compound of Formula (I') wherein R¹⁷ is H.

Also described is a compound of Formula (I') wherein R¹⁸ is H.

Also described is a compound of Formula (I') wherein R³ is H.

Also described is a compound of Formula (I') wherein R⁵ is H.

Also described is a compound of Formula (I') wherein R⁴ is H. Also described is a compound of Formula (I') wherein R⁴ is -(C₁-C₆)alkyl. Also described is a compound of Formula (I') wherein R⁴ is -CH₃. Also described is a compound of Formula (I') wherein R⁴ is -CH₂CH₃. Also described is a compound of Formula (I') wherein R⁴ is -(C₁-C₆)alkyl-OH. Also described is a compound of Formula (I') wherein R⁴ is -CH₂OH. Also described is a compound of Formula (I') wherein R⁴ is -(C₃-C₆)cycloalkyl. Also described is a compound of Formula (I') wherein R⁴ is cyclopropyl. Also described is a compound of Formula (I') wherein R⁴ is -C(O)NH₂.

Also described is a compound of Formula (I') wherein R³, R⁴, and R⁵ are H.

Also described is a compound of Formula (I') wherein R⁴ and R⁵ and the carbon atom to which they are attached form a cyclopropyl ring.

Also described is a compound of Formula (I') wherein R¹⁰ is H.

Also described is a compound of Formula (I') wherein R¹⁰ is H and R⁹ is -(C₁-C₆)alkyl. Also described is a compound of Formula (I') wherein R¹⁰ is H and R⁹ is -CH₃. Also described is a compound of Formula (I') wherein R¹⁰ is H and R⁹ is -CH₂CH₃. Also described is a compound of Formula (I') wherein R¹⁰ is H and R⁹ is -(C₁-C₆)haloalkyl. Also described is a compound of Formula (I') wherein R¹⁰ is H and R⁹ is -CH₂F. Also described is a compound of Formula (I') wherein R¹⁰ is H and R⁹ is -CHF₂. Also described is a compound of Formula (I') wherein R¹⁰ is H and R⁹ is -(C₃-C₆)cycloalkyl. Also described is a compound of Formula (I') wherein R¹⁰ is H and R⁹ is cyclopropyl. Also described is a compound of Formula (I') wherein R¹⁰ is H and R⁹ is H.

Also described is a compound of Formula (I') wherein R¹² is H.

Also described is a compound of Formula (I') wherein R¹² is H and R¹¹ is -(C₁-C₆)alkyl. Also described is a compound of Formula (I') wherein R¹² is H and R¹¹ is -CH₃. Also described is a compound of Formula (I') wherein R¹² is H and R¹¹ is -(C₁-C₆)alkyl-OR²³. Also described is a compound of Formula (I') wherein R¹² is H and R¹¹ is -CH₂OH. Also described is a compound of Formula (I') wherein R¹² is H and R¹¹ is -CH₂CH₂OH. Also described is a compound of Formula (I') wherein R¹² is H and R¹¹ is -(C₁-C₆)alkyl. Also described is a compound of Formula (I') wherein R¹² is H and R¹¹ is -(C₁-C₆)alkyl-NR²¹R²². Also described is a compound of Formula (I') wherein R¹² is H and R¹¹ is -(C₁-C₆)alkyl-NH₂. Also described is a compound of Formula (I') wherein R¹² is H and R¹¹ is -CH₂NH₂. Also described is a compound of Formula (I') wherein R¹² is H and R¹¹ is -CH₂CH₂NH₂. Also described is a compound of Formula (I') wherein R¹² is H and R¹¹ is -CH₂CH₂CH₂NH₂. Also described is a compound of Formula (I') wherein R¹² is H and R¹¹ is -CH₂CH₂CH₂CH₂NH₂. Also described is a compound of Formula (I') wherein R¹² is H and R¹¹ is -(C₁-C₆)alkyl-CN. Also described is a compound of Formula (I') wherein R¹² is H and R¹¹ is -CH₂CN. Also described is a compound of Formula (I') wherein R¹² is H and R¹¹ is -(C₁-C₆)alkyl-C(O)NR²⁵R²⁶. Also described is a compound of Formula (I') wherein R¹² is H and R¹¹ is -CH₂C(O)NH₂. Also described is a compound of Formula (I') wherein R¹² is H and R¹¹ is - CH₂CH₂C(O)NH₂. Also described is a compound of Formula (I') wherein R¹² is H and R¹¹ is - (C₁-C₆)alkyl-heteroaryl. Also described is a compound of Formula (I') wherein R¹² is H and R¹¹ is H.

Also described is a compound of Formula (I') wherein R¹¹ and R¹⁸ are combined to form an optionally substituted heterocycloalkyl ring and R¹² is H.

Also described is a compound of Formula (I') wherein p is 1 and R²⁷ is halogen. Also described is a compound of Formula (I') wherein p is 1 and R²⁷ is optionally substituted -(C₁-C₆)alkyl. Also described is a compound of Formula (I') wherein q is 0, p is 1 and R²⁷ is halogen. Also described is a compound of Formula (I') wherein q is 0, p is 1 and R²⁷ is optionally substituted -(C₁-C₆)alkyl. Also described is a compound of Formula (I') wherein q is 1 and R²⁸ is halogen. Also described is a compound of Formula (I') wherein q is 1 and R²⁸ is optionally substituted -(C₁-C₆)alkyl. Also described is a compound of Formula (I') wherein p is 0, q is 1 and R²⁸ is halogen. Also described is a compound of Formula (I') wherein p is 0, q is 1 and R²⁸ is optionally substituted -(C₁-C₆)alkyl.

Also described is a compound of Formula (I') wherein p is 0, and q is 0.

Also described is a compound of Formula (I') wherein R¹ and R² are each independently H, or -(C₁-C₆)alkyl-NR²¹R²², Also described is a compound of Formula (I') wherein R¹ and R² are each H. Also described is a compound of Formula (I') wherein R¹ and R² are each independently -(C₁-C₆)alkyl-NR²¹R²². Also described is a compound of Formula (I') wherein R¹ is H, and R² is -(C₁-C₆)alkyl-NR²¹R²². Also described is a compound of Formula (I') wherein R¹ is -(C₁-C₆)alkyl-NR²¹R²², and R² is H. Also described is a compound of Formula (I') wherein R¹ is H, and R² is -CH₂CH₂NH₂. Also described is a compound of Formula (I') wherein R¹ is - CH₂CH₂NH₂, and R² is H. Also described is a compound of Formula (I') wherein R¹ and R² are each -CH₂CH₂NH₂. Also described is a compound of Formula (I') wherein R¹ is -(C₁-C₆)alkyl-NR²¹R²² and R² is H. Also described is a compound of Formula (I') wherein R¹ is -CH₂CH₂NH₂ and R² is H. Also described is a compound of Formula (I') wherein R¹ is H and R² is -(C₁-C₆)alkyl-NR²¹R²², Also described is a compound of Formula (I') wherein R¹ is H and R² is - CH₂CH₂NH₂.

Also described is a compound of Formula (I') wherein X is optionally substituted aryl. Also described is a compound of Formula (I') wherein X is optionally substituted phenyl. Also described is a compound of Formula (I') wherein X is optionally substituted heteroaryl. Also described is a compound of Formula (I') wherein X is heteroaryl which is unsubstituted or substituted once or twice with -(C₁-C₆)alkyl. Also described is a compound of Formula (I') wherein X is heteroaryl which is unsubstituted or substituted once with -(C₁-C₆)alkyl. Also described is a compound of Formula (I') wherein X is optionally substituted pyridine or optionally substituted pyrimidine. Also described is a compound of Formula (I') wherein X is pyridine which is unsubstituted or substituted once or twice with -(C₁-C₆)alkyl. Also described is a compound of Formula (I') wherein X is pyridine which is unsubstituted or substituted once with -(C₁-C₆)alkyl. Also described is a compound of Formula (I') wherein X is pyridine which is unsubstituted or substituted once or twice with methyl. Also described is a compound of Formula (I') wherein X is pyrimidine which is unsubstituted or substituted once or twice with -(C₁-C₆)alkyl. Also described is a compound of Formula (I') wherein X is pyrimidine which is unsubstituted or substituted once with -(C₁-C₆)alkyl. Also described is a compound of Formula (I') wherein X is pyrimidine which is unsubstituted or substituted once or twice with methyl. Also described is a compound of Formula (I') wherein X is pyridine which is substituted once with methyl. Also described is a compound of Formula (I') wherein X is optionally substituted - (C₁-C₆)alkyl-. Also described is a compound of Formula (I') wherein Y is optionally substituted aryl. Also described is a compound of Formula (I') wherein Y is optionally substituted phenyl. Also described is a compound of Formula (I') wherein Y is optionally substituted heteroaryl. Also described is a compound of Formula (I') wherein Y is optionally substituted -(C₁-C₆)alkyl-. Also described is a compound of Formula (I') wherein Y is -O-(C₁-C₆)alkyl-. Also described is a compound of Formula (I') wherein Y is -N(H)-(C₁-C₆)alkyl-. Also described is a compound of Formula (I') wherein Y is a bond. Also described is a compound of Formula (I') wherein Z is - (C₁-C₆)alkyl. Also described is a compound of Formula (I') wherein Z is optionally substituted aryl. Also described is a compound of Formula (I') wherein Z is optionally substituted phenyl. Also described is a compound of Formula (I') wherein Z is phenyl substituted once or twice with -(C₁-C₈)alkyl. Also described is a compound of Formula (I') wherein Z is phenyl substituted once with n-butyl, isobutyl, or tert-butyl. Also described is a compound of Formula (I') wherein Z is phenyl substituted once with n-butyl. Also described is a compound of Formula (I') wherein Z is phenyl substituted once with isobutyl. Also described is a compound of Formula (I') wherein Z is phenyl substituted once with tert-butyl. Also described is a compound of Formula (I') wherein Z is optionally substituted heteroaryl. Also described is a compound of Formula (I') wherein Z is optionally substituted -(C₃-C₇)cycloalkyl. Also described is a compound of Formula (I') wherein Z is halogen.

Also described is a compound of Formula (I') wherein -X-Y-Z is

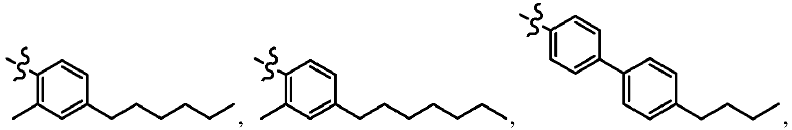

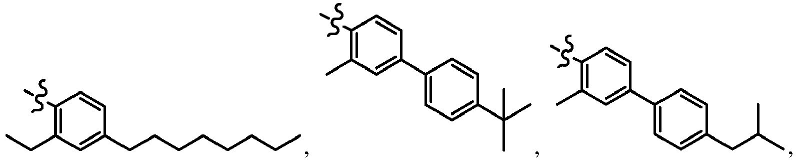

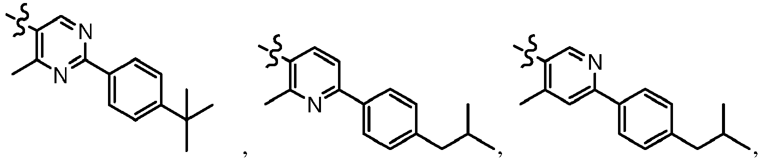

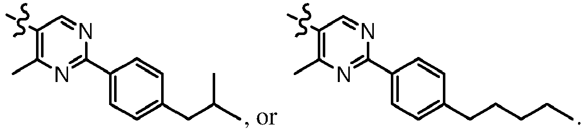

Also described is a compound of Formula (I) having the structure of Formula (Ia): 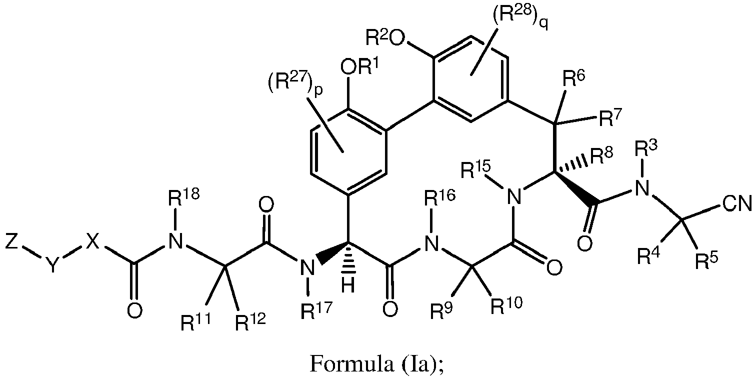 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, -(C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²², or -(C₁-C₆)alkyl-C(O)N(R²³)(C₁-C₆)alkyl, or optionally substituted heterocycloalkyl;
R³ is H, or -(C₁-C₆)alkyl;
R⁴ is H, -(C₁-C₆)alkyl, -(C₁-C₆)alkyl-OH, -(C₃-C₆)cycloalkyl , or -C(O)NH₂; or R³ and R⁴ are combined to form a heterocycloalkyl ring;
R⁵ is H, or -(C₁-C₆)alkyl; or R⁴ and R⁵ and the carbon atom to which they are attached form a cyclopropyl ring;
R⁶, R⁷, and R⁸ are each independently H, or -(C₁-C₆)alkyl;
R⁹ is H, -(C₁-C₆)alkyl, -(C₁-C₆)haloalkyl, or -(C₃-C₆)cycloalkyl;
R¹⁰ is H, or -(C₁-C₆)alkyl;
R¹¹ and R¹² are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-CN, -(C₁-C₆)alkyl-C(O)NR²⁵R²⁶, -(C₁-C₆)heteroalkyl-CO₂H, -(C₁-C₆)alkyl-S(O)-(C₁-C₆)alkyl, -(C₁-C₆)alkyl-N(H)CH=NH, -(C₁-C₆)alkyl-N(H)C(NH)NH₂, -(C₁-C₆)alkyl-heterocycloalkyl, optionally substituted -(C₁-C₆)alkyl-N(H)heterocycloalkyl, or -(C₁-C₆)alkyl-heteroaryl; or R¹¹ and R¹⁸ are combined to form an optionally substituted heterocycloalkyl ring, and R¹² is H;
R¹⁵, R¹⁶, R¹⁷, and R¹⁸ are each independently H, -(C₁-C₆)alkyl, -(C₃-C₆)cycloalkyl, -(C₁-C₆)alkyl-OR²³, -(C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, - N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, - SO₂(C₁-C₆)alkyl-, -C(O)NH(C₁-C₆)alkyl-, -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-,optionally substituted aryl, or optionally substituted heteroaryl;
Z is H, halogen, -NH₂, -CN, -CF₃, -CO₂H, -(C₁-C₁₂)alkyl,-(C₂-C₁₂)alkenyl, -(C₂-C₁₂)alkynyl, - C(O)NR²⁵R²⁶, -O-(C₁-C₁₂)alkyl, -N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, - C(O)(C₁-C₆)alkyl, -C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl; or R²⁵ and R²⁶ and the nitrogen atom to which they are attached form a heterocycloalkyl ring;
each R²⁷ is independently halogen, optionally substituted -(C₁-C₆)alkyl, or optionally substituted - (C₁-C₆)heteroalkyl;
each R²⁸ is independently halogen, optionally substituted -(C₁-C₆)alkyl, or optionally substituted - (C₁-C₆)heteroalkyl;
p is 0, 1, or 2; and
q is 0, 1, or 2;
or a pharmaceutically acceptable salt, solvate, or prodrug thereof.

Also described is a compound of Formula (Ia) wherein R⁶, R⁷, and R⁸ are H.

Also described is a compound of Formula (Ia) wherein R¹⁵ and R¹⁶ are H.

Also described is a compound of Formula (Ia) wherein R¹⁷ is -(C₁-C₆)alkyl. Also described is a compound of Formula (Ia) wherein R¹⁷ is -CH₃. Also described is a compound of Formula (Ia) wherein R¹⁷ is -CH₂CH₃. Also described is a compound of Formula (Ia) wherein R¹⁷ is -(C₃-C₆)cycloalkyl. Also described is a compound of Formula (Ia) wherein R¹⁷ is cyclopropyl. Also described is a compound of Formula (Ia) wherein R¹⁷ is -(C₁-C₆)alkyl-C(O)OR²³. Also described is a compound of Formula (Ia) wherein R¹⁷ is -CH₂CH₂OH. Also described is a compound of Formula (Ia) wherein R¹⁷ is -(C₁-C₆)alkyl-NR²¹R²². Also described is a compound of Formula (Ia) wherein R¹⁷ is -CH₂CH₂NH₂. Also described is a compound of Formula (Ia) wherein R¹⁷ is H.

Also described is a compound of Formula (Ia) wherein R¹⁸ is H.

Also described is a compound of Formula (Ia) wherein R³ is H.

Also described is a compound of Formula (Ia) wherein R⁵ is H.

Also described is a compound of Formula (Ia) wherein R⁴ is H. Also described is a compound of Formula (Ia) wherein R⁴ is -(C₁-C₆)alkyl. Also described is a compound of Formula (Ia) wherein R⁴ is -CH₃. Also described is a compound of Formula (Ia) wherein R⁴ is - CH₂CH₃. Also described is a compound of Formula (Ia) wherein R⁴ is -(C₁-C₆)alkyl-OH. Also described is a compound of Formula (Ia) wherein R⁴ is -CH₂OH. Also described is a compound of Formula (Ia) wherein R⁴ is -(C₃-C₆)cycloalkyl. Also described is a compound of Formula (Ia) wherein R⁴ is cyclopropyl. Also described is a compound of Formula (Ia) wherein R⁴ is - C(O)NH₂.

Also described is a compound of Formula (Ia) wherein R⁴ and R⁵ and the carbon atom to which they are attached form a cyclopropyl ring.

Also described is a compound of Formula (Ia) wherein R³, R⁴, and R⁵ are H.

Also described is a compound of Formula (Ia) wherein R¹⁰ is H.

Also described is a compound of Formula (Ia) wherein R¹⁰ is H and R⁹ is -(C₁-C₆)alkyl. Also described is a compound of Formula (Ia) wherein R¹⁰ is H and R⁹ is -CH₃. Also described is a compound of Formula (Ia) wherein R¹⁰ is H and R⁹ is -CH₂CH₃. Also described is a compound of Formula (Ia) wherein R¹⁰ is H and R⁹ is -(C₁-C₆)haloalkyl. Also described is a compound of Formula (Ia) wherein R¹⁰ is H and R⁹ is -CH₂F. Also described is a compound of Formula (Ia) wherein R¹⁰ is H and R⁹ is -CHF₂. Also described is a compound of Formula (Ia) wherein R¹⁰ is H and R⁹ is -(C₃-C₆)cycloalkyl. Also described is a compound of Formula (Ia) wherein R¹⁰ is H and R⁹ is cyclopropyl. Also described is a compound of Formula (Ia) wherein R¹⁰ is H and R⁹ is H.

Also described is a compound of Formula (Ia) wherein R¹² is H.

Also described is a compound of Formula (Ia) wherein R¹² is H and R¹¹ is -(C₁-C₆)alkyl. Also described is a compound of Formula (Ia) wherein R¹² is H and R¹¹ is -CH₃. Also described is a compound of Formula (Ia) wherein R¹² is H and R¹¹ is -(C₁-C₆)alkyl-OR²³. Also described is a compound of Formula (Ia) wherein R¹² is H and R¹¹ is -CH₂OH. Also described is a compound of Formula (Ia) wherein R¹² is H and R¹¹ is -CH₂CH₂OH. Also described is a compound of Formula (Ia) wherein R¹² is H and R¹¹ is -(C₁-C₆)alkyl. Also described is a compound of Formula (Ia) wherein R¹² is H and R¹¹ is -(C₁-C₆)alkyl-NR²¹R²². Also described is a compound of Formula (Ia) wherein R¹² is H and R¹¹ is -(C₁-C₆)alkyl-NH₂. Also described is a compound of Formula (Ia) wherein R¹² is H and R¹¹ is -CH₂NH₂. Also described is a compound of Formula (Ia) wherein R¹² is H and R¹¹ is -CH₂CH₂NH₂. Also described is a compound of Formula (Ia) wherein R¹² is H and R¹¹ is -CH₂CH₂CH₂NH₂. Also described is a compound of Formula (Ia) wherein R¹² is H and R¹¹ is -CH₂CH₂CH₂CH₂NH₂. Also described is a compound of Formula (Ia) wherein R¹² is H and R¹¹ is -(C₁-C₆)alkyl-CN. Also described is a compound of Formula (Ia) wherein R¹² is H and R¹¹ is -CH₂CN. Also described is a compound of Formula (Ia) wherein R¹² is H and R¹¹ is -(C₁-C₆)alkyl-C(O)NR²⁵R²⁶. Also described is a compound of Formula (Ia) wherein R¹² is H and R¹¹ is -CH₂C(O)NH₂. Also described is a compound of Formula (Ia) wherein R¹² is H and R¹¹ is -CH₂CH₂C(O)NH₂. Also described is a compound of Formula (Ia) wherein R¹² is H and R¹¹ is -(C₁-C₆)alkyl-heteroaryl. Also described is a compound of Formula (Ia) wherein R¹² is H and R¹¹ is H.

Also described is a compound of Formula (Ia) wherein R¹¹ and R¹⁸ are combined to form an optionally substituted heterocycloalkyl ring and R¹² is H.

Also described is a compound of Formula (Ia) wherein p is 1 and R²⁷ is halogen. Also described is a compound of Formula (Ia) wherein p is 1 and R²⁷ is optionally substituted -(C₁-C₆)alkyl. Also described is a compound of Formula (I) wherein q is 0, p is 1 and R²⁷ is halogen. Also described is a compound of Formula (I) wherein q is 0, p is 1 and R²⁷ is optionally substituted -(C₁-C₆)alkyl. Also described is a compound of Formula (Ia) wherein q is 1 and R²⁸ is halogen. Also described is a compound of Formula (Ia) wherein q is 1 and R²⁸ is optionally substituted -(C₁-C₆)alkyl. Also described is a compound of Formula (Ia) wherein p is 0, q is 1 and R²⁸ is halogen. Also described is a compound of Formula (Ia) wherein p is 0, q is 1 and R²⁸ is optionally substituted -(C₁-C₆)alkyl.

Also described is a compound of Formula (Ia) wherein p is 0, and q is 0.

Also described is a compound of Formula (Ia) wherein R¹ and R² are each independently H, or -(C₁-C₆)alkyl-NR²¹R²². Also described is a compound of Formula (Ia) wherein R¹ and R² are each H. Also described is a compound of Formula (Ia) wherein R¹ and R² are each independently -(C₁-C₆)alkyl-NR²¹R²². Also described is a compound of Formula (Ia) wherein R¹ is H, and R² is -(C₁-C₆)alkyl-NR²¹R²². Also described is a compound of Formula (Ia) wherein R¹ is -(C₁-C₆)alkyl-NR²¹R²². and R² is H. Also described is a compound of Formula (Ia) wherein R¹ is H, and R² is -CH₂CH₂NH₂. Also described is a compound of Formula (Ia) wherein R¹ is - CH₂CH₂NH₂, and R² is H. Also described is a compound of Formula (Ia) wherein R¹ and R² are each -CH₂CH₂NH₂. Also described is a compound of Formula (Ia) wherein R¹ is -(C₁-C₆)alkyl-NR²¹R²² and R² is H. Also described is a compound of Formula (Ia) wherein R¹ is -CH₂CH₂NH₂ and R² is H. Also described is a compound of Formula (Ia) wherein R¹ is H and R² is -(C₁-C₆)alkyl-NR²¹R²². Also described is a compound of Formula (Ia) wherein R¹ is H and R² is - CH₂CH₂NH₂.

Also described is a compound of Formula (Ia) wherein X is optionally substituted aryl. Also described is a compound of Formula (Ia) wherein X is optionally substituted phenyl. Also described is a compound of Formula (Ia) wherein X is optionally substituted heteroaryl. Also described is a compound of Formula (Ia) wherein X is heteroaryl which is unsubstituted or substituted once or twice with -(C₁-C₆)alkyl. Also described is a compound of Formula (Ia) wherein X is heteroaryl which is unsubstituted or substituted once with -(C₁-C₆)alkyl. Also described is a compound of Formula (Ia) wherein X is optionally substituted pyridine or optionally substituted pyrimidine. Also described is a compound of Formula (Ia) wherein X is pyridine which is unsubstituted or substituted once or twice with -(C₁-C₆)alkyl. Also described is a compound of Formula (Ia) wherein X is pyridine which is unsubstituted or substituted once with -(C₁-C₆)alkyl. Also described is a compound of Formula (Ia) wherein X is pyridine which is unsubstituted or substituted once or twice with methyl. Also described is a compound of Formula (Ia) wherein X is pyrimidine which is unsubstituted or substituted once or twice with -(C₁-C₆)alkyl. Also described is a compound of Formula (Ia) wherein X is pyrimidine which is unsubstituted or substituted once with -(C₁-C₆)alkyl. Also described is a compound of Formula (Ia) wherein X is pyrimidine which is unsubstituted or substituted once or twice with methyl. Also described is a compound of Formula (Ia) wherein X is pyridine which is substituted once with methyl. Also described is a compound of Formula (Ia) wherein X is pyrimidine which is substituted once with methyl. Also described is a compound of Formula (Ia) wherein X is pyrimidine which is substituted twice with methyl. Also described is a compound of Formula (Ia) wherein X is optionally substituted -(C₁-C₆)alkyl-. Also described is a compound of Formula (Ia) wherein Y is optionally substituted aryl. Also described is a compound of Formula (Ia) wherein Y is optionally substituted phenyl. Also described is a compound of Formula (Ia) wherein Y is optionally substituted heteroaryl. Also described is a compound of Formula (Ia) wherein Y is optionally substituted -(C₁-C₆)alkyl-. Also described is a compound of Formula (Ia) wherein Y is -O-(C₁-C₆)alkyl-. Also described is a compound of Formula (Ia) wherein Y is - N(H)-(C₁-C₆)alkyl-. Also described is a compound of Formula (Ia) wherein Y is a bond. Also described is a compound of Formula (Ia) wherein Z is -(C₁-C₆)alkyl. Also described is a compound of Formula (Ia) wherein Z is optionally substituted aryl. Also described is a compound of Formula (Ia) wherein Z is optionally substituted phenyl. Also described is a compound of Formula (Ia) wherein Z is phenyl substituted once or twice with -(C₁-C₈)alkyl. Also described is a compound of Formula (Ia) wherein Z is phenyl substituted once with n-butyl, isobutyl, or tert-butyl. Also described is a compound of Formula (Ia) wherein Z is phenyl substituted once with n-butyl. Also described is a compound of Formula (Ia) wherein Z is phenyl substituted once with isobutyl. Also described is a compound of Formula (Ia) wherein Z is phenyl substituted once with tert-butyl. Also described is a compound of Formula (Ia) wherein Z is optionally substituted heteroaryl. Also described is a compound of Formula (Ia) wherein Z is optionally substituted -(C₃-C₇)cycloalkyl. Also described is a compound of Formula (Ia) wherein Z is halogen.

Also described is a compound of Formula (Ia) wherein -X-Y-Z is

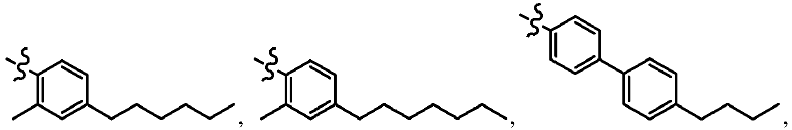

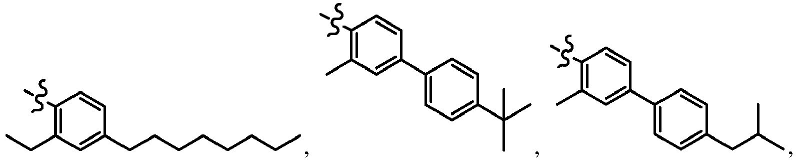

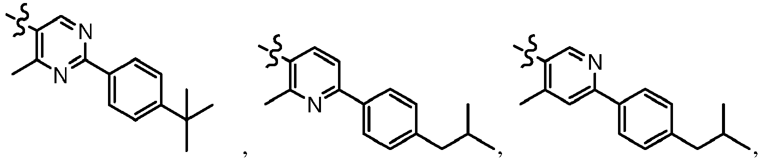

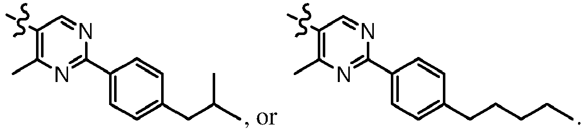

Also described is a compound of Formula (I) having the structure of Formula (Ib): 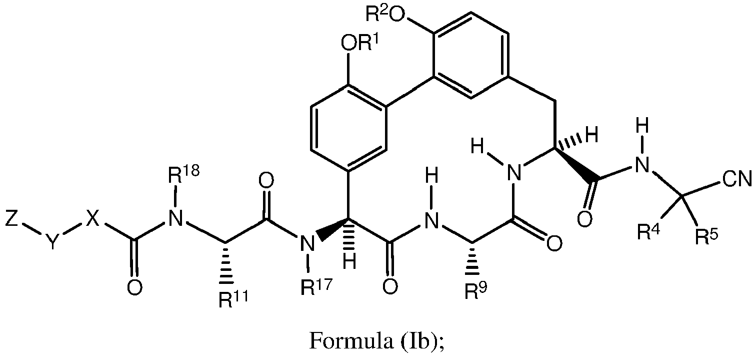 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, -(C₁-C₆)alkyl-NR²¹R²². -(C₁-C₆)alkyl-C(O)NR²⁵R²⁶, -(C₁-C₆)alkyl-N(R²³)C(O)(C₁-C₆)alkylNR²¹R²², or -(C₁-C₆)alkyl-C(O)N(R²³)(C₁-C₆)alkyl, or optionally substituted heterocycloalkyl;
R⁴ is H, -(C₁-C₆)alkyl, -(C₁-C₆)alkyl-OH, -(C₃-C₆)cycloalkyl , or -C(O)NH₂;
R⁵ is H, or -(C₁-C₆)alkyl; or R⁴ and R⁵ and the carbon atom to which they are attached form a cyclopropyl ring;
R⁹ is H, -(C₁-C₆)alkyl, -(C₁-C₆)haloalkyl, or -(C₃-C₆)cycloalkyl;
R¹¹ is H, -NH₂, -(C₁-C₆)alkyl, -(C₁-C₆)alkyl-OR²³, -(C₁-C₆)alkyl-SR²³, -(C₁-C₆)alkyl-C(O)OR²³, - (C₁-C₆)alkyl-NR²¹R²², -(C₁-C₆)alkyl-CN, -(C₁-C₆)alkyl-C(O)NR²⁵R²⁶, -(C₁-C₆)heteroalkyl-CO₂H, -(C₁-C₆)alkyl-S(O)-(C₁-C₆)alkyl, -(C₁-C₆)alkyl-N(H)CH=NH, -(C₁-C₆)alkyl-N(H)C(NH)NH₂, - (C₁-C₆)alkyl-heterocycloalkyl, optionally substituted -(C₁-C₆)alkyl-N(H)heterocycloalkyl, or - (C₁-C₆)alkyl-heteroaryl; or R¹¹ and R¹⁸ are combined to form an optionally substituted heterocycloalkyl ring;
R¹⁷ and R¹⁸ are each independently H, -(C₁-C₆)alkyl, -(C₃-C₆)cycloalkyl, -(C₁-C₆)alkyl-OR²³, - (C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, - N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, - SO₂(C₁-C₆)alkyl-, -C(O)NH(C₁-C₆)alkyl-, -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-,optionally substituted aryl, or optionally substituted heteroaryl;
Z is H, halogen, -NH₂, -CN, -CF₃, -CO₂H, -(C₁-C₁₂)alkyl,-(C₂-C₁₂)alkenyl, -(C₂-C₁₂)alkynyl, - C(O)NR²⁵R²⁶, -O-(C₁-C₁₂)alkyl, -N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, - C(O)(C₁-C₆)alkyl, -C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl; and
each R²⁵ and R²⁶ is independently H or optionally substituted -(C₁-C₆)alkyl; or R²⁵ and R²⁶ and the nitrogen atom to which they are attached form a heterocycloalkyl ring;
or a pharmaceutically acceptable salt, solvate, or prodrug thereof.

Also described is a compound of Formula (Ib) wherein R¹⁷ is -(C₁-C₆)alkyl. Also described is a compound of Formula (Ib) wherein R¹⁷ is -CH₃. Also described is a compound of Formula (Ib) wherein R¹⁷ is -CH₂CH₃. Also described is a compound of Formula (Ib) wherein R¹⁷ is -(C₃-C₆)cycloalkyl. Also described is a compound of Formula (Ib) wherein R¹⁷ is cyclopropyl. Also described is a compound of Formula (Ib) wherein R¹⁷ is -(C₁-C₆)alkyl-C(O)OR²³. Also described is a compound of Formula (Ib) wherein R¹⁷ is -CH₂CH₂OH. Also described is a compound of Formula (Ib) wherein R¹⁷ is -(C₁-C₆)alkyl-NR²¹R²². Also described is a compound of Formula (Ib) wherein R¹⁷ is -CH₂CH₂NH₂. Also described is a compound of Formula (Ib) wherein R¹⁷ is H.

Also described is a compound of Formula (Ib) wherein R¹⁸ is H.

Also described is a compound of Formula (Ib) wherein R⁵ is H.

Also described is a compound of Formula (Ib) wherein R⁴ is H. Also described is a compound of Formula (Ib) wherein R⁴ is -(C₁-C₆)alkyl. Also described is a compound of Formula (Ib) wherein R⁴ is -CH₃. Also described is a compound of Formula (Ib) wherein R⁴ is - CH₂CH₃. Also described is a compound of Formula (Ib) wherein R⁴ is -(C₁-C₆)alkyl-OH. Also described is a compound of Formula (Ib) wherein R⁴ is -CH₂OH. Also described is a compound of Formula (Ib) wherein R⁴ is -(C₃-C₆)cycloalkyl. Also described is a compound of Formula (Ib) wherein R⁴ is cyclopropyl. Also described is a compound of Formula (Ib) wherein R⁴ is - C(O)NH₂.

Also described is a compound of Formula (Ib) wherein R⁴ and R⁵ and the carbon atom to which they are attached form a cyclopropyl ring.

Also described is a compound of Formula (Ib) wherein R⁹ is -(C₁-C₆)alkyl. Also described is a compound of Formula (Ib) wherein R⁹ is -CH₃. Also described is a compound of Formula (Ib) wherein R⁹ is -CH₂CH₃. Also described is a compound of Formula (Ib) wherein R⁹ is -(C₁-C₆)haloalkyl. Also described is a compound of Formula (Ib) wherein R⁹ is -CH₂F. Also described is a compound of Formula (Ib) wherein R⁹ is -CHF₂. Also described is a compound of Formula (Ib) wherein R⁹ is -(C₃-C₆)cycloalkyl. Also described is a compound of Formula (Ib) wherein R⁹ is cyclopropyl. Also described is a compound of Formula (Ib) wherein R⁹ is H.

Also described is a compound of Formula (Ib) wherein R¹¹ is -(C₁-C₆)alkyl. Also described is a compound of Formula (Ib) wherein R¹¹ is -CH₃. Also described is a compound of Formula (Ib) wherein R¹¹ is -(C₁-C₆)alkyl-OR²³. Also described is a compound of Formula (Ib) wherein R¹¹ is -CH₂OH. Also described is a compound of Formula (Ib) wherein R¹¹ is - CH₂CH₂OH. Also described is a compound of Formula (Ib) wherein R¹¹ is -(C₁-C₆)alkyl. Also described is a compound of Formula (Ib) wherein R¹¹ is -(C₁-C₆)alkyl-NR²¹R²². Also described is a compound of Formula (Ib) wherein R¹¹ is -(C₁-C₆)alkyl-NH₂. Also described is a compound of Formula (Ib) wherein R¹¹ is -CH₂NH₂. Also described is a compound of Formula (Ib) wherein R¹¹ is -CH₂CH₂NH₂. Also described is a compound of Formula (Ib) wherein R¹¹ is - CH₂CH₂CH₂NH₂. Also described is a compound of Formula (Ib) wherein R¹¹ is - CH₂CH₂CH₂CH₂NH₂. Also described is a compound of Formula (Ib) wherein R¹¹ is -(C₁-C₆)alkyl-CN. Also described is a compound of Formula (Ib) wherein R¹¹ is -CH₂CN. Also described is a compound of Formula (Ib) wherein R¹¹ is -(C₁-C₆)alkyl-C(O)NR²⁵R²⁶. Also described is a compound of Formula (Ib) wherein R¹¹ is -CH₂C(O)NH₂. Also described is a compound of Formula (Ib) wherein R¹¹ is -CH₂CH₂C(O)NH₂. Also described is a compound of Formula (Ib) wherein R¹¹ is -(C₁-C₆)alkyl-heteroaryl. Also described is a compound of Formula (Ib) wherein R¹¹ is H.

Also described is a compound of Formula (Ib) wherein R¹¹ and R¹⁸ are combined to form an optionally substituted heterocycloalkyl ring.

Also described is a compound of Formula (Ib) wherein R¹ and R² are each independently H, or -(C₁-C₆)alkyl-NR²¹R²². Also described is a compound of Formula (Ib) wherein R¹ and R² are each H. Also described is a compound of Formula (Ib) wherein R¹ and R² are each independently -(C₁-C₆)alkyl-NR²¹R²². Also described is a compound of Formula (Ib) wherein R¹ is H, and R² is -(C₁-C₆)alkyl-NR²¹R²². Also described is a compound of Formula (Ib) wherein R¹ is -(C₁-C₆)alkyl-NR²¹R²². and R² is H. Also described is a compound of Formula (Ib) wherein R¹ is H, and R² is -CH₂CH₂NH₂. Also described is a compound of Formula (Ib) wherein R¹ is -CH₂CH₂NH₂, and R² is H. Also described is a compound of Formula (Ib) wherein R¹ and R² are each -CH₂CH₂NH₂. Also described is a compound of Formula (Ib) wherein R¹ is -(C₁-C₆)alkyl-NR²¹R²² and R² is H. Also described is a compound of Formula (Ib) wherein R¹ is - CH₂CH₂NH₂ and R² is H. Also described is a compound of Formula (Ib) wherein R¹ is H and R² is -(C₁-C₆)alkyl-NR²¹R²². Also described is a compound of Formula (Ib) wherein R¹ is H and R² is -CH₂CH₂NH₂.

Also described is a compound of Formula (Ib) wherein X is optionally substituted aryl. Also described is a compound of Formula (Ib) wherein X is optionally substituted phenyl. Also described is a compound of Formula (Ib) wherein X is optionally substituted heteroaryl. Also described is a compound of Formula (Ib) wherein X is heteroaryl which is unsubstituted or substituted once or twice with -(C₁-C₆)alkyl. Also described is a compound of Formula (Ib) wherein X is heteroaryl which is unsubstituted or substituted once with -(C₁-C₆)alkyl. Also described is a compound of Formula (Ib) wherein X is optionally substituted pyridine or optionally substituted pyrimidine. Also described is a compound of Formula (Ib) wherein X is pyridine which is unsubstituted or substituted once or twice with -(C₁-C₆)alkyl. Also described is a compound of Formula (Ib) wherein X is pyridine which is unsubstituted or substituted once with -(C₁-C₆)alkyl. Also described is a compound of Formula (Ib) wherein X is pyridine which is unsubstituted or substituted once or twice with methyl. Also described is a compound of Formula (Ib) wherein X is pyrimidine which is unsubstituted or substituted once or twice with -(C₁-C₆)alkyl. Also described is a compound of Formula (Ib) wherein X is pyrimidine which is unsubstituted or substituted once with -(C₁-C₆)alkyl. Also described is a compound of Formula (Ib) wherein X is pyrimidine which is unsubstituted or substituted once or twice with methyl. Also described is a compound of Formula (Ib) wherein X is pyridine which is substituted once with methyl. Also described is a compound of Formula (Ib) wherein X is pyrimidine which is substituted once with methyl. Also described is a compound of Formula (Ib) wherein X is pyrimidine which is substituted twice with methyl. Also described is a compound of Formula (Ib) wherein X is optionally substituted -(C₁-C₆)alkyl-. Also described is a compound of Formula (Ib) wherein Y is optionally substituted aryl. Also described is a compound of Formula (Ib) wherein Y is optionally substituted phenyl. Also described is a compound of Formula (Ib) wherein Y is optionally substituted heteroaryl. Also described is a compound of Formula (Ib) wherein Y is optionally substituted -(C₁-C₆)alkyl-. Also described is a compound of Formula (Ib) wherein Y is -O-(C₁-C₆)alkyl-. Also described is a compound of Formula (Ib) wherein Y is -N(H)-(C₁-C₆)alkyl-. Also described is a compound of Formula (Ib) wherein Y is a bond. Also described is a compound of Formula (Ib) wherein Z is -(C₁-C₆)alkyl. Also described is a compound of Formula (Ib) wherein Z is optionally substituted aryl. Also described is a compound of Formula (Ib) wherein Z is optionally substituted phenyl. Also described is a compound of Formula (Ib) wherein Z is phenyl substituted once or twice with -(C₁-C₈)alkyl. Also described is a compound of Formula (Ib) wherein Z is phenyl substituted once with n-butyl, isobutyl, or tert-butyl. Also described is a compound of Formula (Ib) wherein Z is phenyl substituted once with n-butyl. Also described is a compound of Formula (Ib) wherein Z is phenyl substituted once with isobutyl. Also described is a compound of Formula (Ib) wherein Z is phenyl substituted once with tert-butyl. Also described is a compound of Formula (Ib) wherein Z is optionally substituted heteroaryl. Also described is a compound of Formula (Ib) wherein Z is optionally substituted - (C₃-C₇)cycloalkyl. Also described is a compound of Formula (Ib) wherein Z is halogen.

Also described is a compound of Formula (Ib) wherein -X-Y-Z is

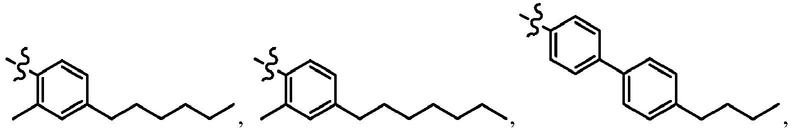

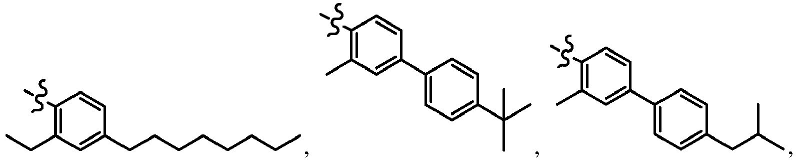

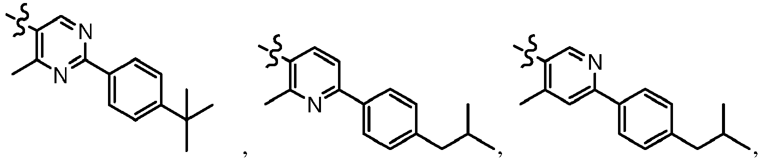

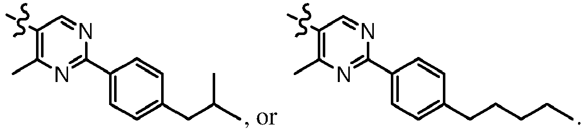

Also described is a compound of Formula (I) having the structure of Formula (Ic): 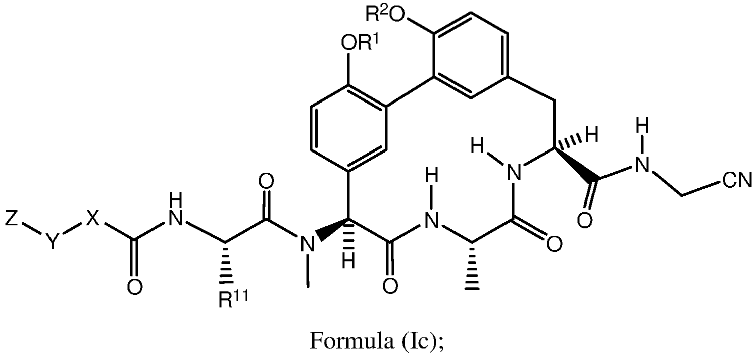 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, -(C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²², or -(C₁-C₆)alkyl-C(O)N(R²³)(C₁-C₆)alkyl, or optionally substituted heterocycloalkyl;
R¹¹ is H, -NH₂, -(C₁-C₆)alkyl, -(C₁-C₆)alkyl-OR²³, -(C₁-C₆)alkyl-SR²³, -(C₁-C₆)alkyl-C(O)OR²³, - (C₁-C₆)alkyl-NR²¹R²², -(C₁-C₆)alkyl-CN, -(C₁-C₆)alkyl-C(O)NR²⁵R²⁶, -(C₁-C₆)heteroalkyl-CO₂H, -(C₁-C₆)alkyl-S(O)-(C₁-C₆)alkyl, -(C₁-C₆)alkyl-N(H)CH=NH, -(C₁-C₆)alkyl-N(H)C(NH)NH₂, - (C₁-C₆)alkyl-heterocycloalkyl, optionally substituted -(C₁-C₆)alkyl-N(H)heterocycloalkyl, or - (C₁-C₆)alkyl-heteroaryl;
X is optionally substituted -(C₁-C₆)alkyl-, -(C₂-C₆)alkenyl-, -(C₂-C₆)alkynyl, -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, - N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, - SO₂(C₁-C₆)alkyl-, -C(O)NH(C₁-C₆)alkyl-, -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-,optionally substituted aryl, or optionally substituted heteroaryl;
Z is H, halogen, -NH₂, -CN, -CF₃, -CO₂H, -(C₁-C₁₂)alkyl,-(C₂-C₁₂)alkenyl, -(C₂-C₁₂)alkynyl, - C(O)NR²⁵R²⁶, -O-(C₁-C₁₂)alkyl, -N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, - C(O)(C₁-C₆)alkyl, -C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl; and
each R²⁵ and R²⁶ is independently H or optionally substituted -(C₁-C₆)alkyl; or R²⁵ and R²⁶ and the nitrogen atom to which they are attached form a heterocycloalkyl ring;
or a pharmaceutically acceptable salt, solvate, or prodrug thereof.

Also described is a compound of Formula (Ic) wherein R¹¹ is -(C₁-C₆)alkyl. Also described is a compound of Formula (Ic) wherein R¹¹ is -CH₃. Also described is a compound of Formula (Ic) wherein R¹¹ is -(C₁-C₆)alkyl-OR²³. Also described is a compound of Formula (Ic) wherein R¹¹ is -CH₂OH. Also described is a compound of Formula (Ic) wherein R¹¹ is - CH₂CH₂OH. Also described is a compound of Formula (Ic) wherein R¹¹ is -(C₁-C₆)alkyl. Also described is a compound of Formula (Ic) wherein R¹¹ is -(C₁-C₆)alkyl-NR²¹R²². Also described is a compound of Formula (Ic) wherein R¹¹ is -(C₁-C₆)alkyl-NH₂. Also described is a compound of Formula (Ic) wherein R¹¹ is -CH₂NH₂. Also described is a compound of Formula (Ic) wherein R¹¹ is -CH₂CH₂NH₂. Also described is a compound of Formula (Ic) wherein R¹¹ is - CH₂CH₂CH₂NH₂. Also described is a compound of Formula (Ic) wherein R¹¹ is - CH₂CH₂CH₂CH₂NH₂. Also described is a compound of Formula (Ic) wherein R¹¹ is -(C₁-C₆)alkyl-CN. Also described is a compound of Formula (Ic) wherein R¹¹ is -CH₂CN. Also described is a compound of Formula (Ic) wherein R¹¹ is -(C₁-C₆)alkyl-C(O)NR²⁵R²⁶, Also described is a compound of Formula (Ic) wherein R¹¹ is -CH₂C(O)NH₂. Also described is a compound of Formula (Ic) wherein R¹¹ is -CH₂CH₂C(O)NH₂. Also described is a compound of Formula (Ic) wherein R¹¹ is -(C₁-C₆)alkyl-heteroaryl. Also described is a compound of Formula (Ic) wherein R¹¹ is H.

Also described is a compound of Formula (Ic) wherein R¹ and R² are each independently H, or -(C₁-C₆)alkyl-NR²¹R²². Also described is a compound of Formula (Ic) wherein R¹ and R² are each H. Also described is a compound of Formula (Ic) wherein R¹ and R² are each independently -(C₁-C₆)alkyl-NR²¹R²². Also described is a compound of Formula (Ic) wherein R¹ is H, and R² is -(C₁-C₆)alkyl-NR²¹R²². Also described is a compound of Formula (Ic) wherein R¹ is -(C₁-C₆)alkyl-NR²¹R²². and R² is H. Also described is a compound of Formula (Ic) wherein R¹ is H, and R² is -CH₂CH₂NH₂. Also described is a compound of Formula (Ic) wherein R¹ is - CH₂CH₂NH₂, and R² is H. Also described is a compound of Formula (Ic) wherein R¹ and R² are each -CH₂CH₂NH₂. Also described is a compound of Formula (Ic) wherein R¹ is -(C₁-C₆)alkyl-NR²¹R²² and R² is H. Also described is a compound of Formula (Ic) wherein R¹ is -CH₂CH₂NH₂ and R² is H. Also described is a compound of Formula (Ic) wherein R¹ is H and R² is -(C₁-C₆)alkyl-NR²¹R²². Also described is a compound of Formula (Ic) wherein R¹ is H and R² is - CH₂CH₂NH₂.

Also described is a compound of Formula (Ic) wherein X is optionally substituted aryl. Also described is a compound of Formula (Ic) wherein X is optionally substituted phenyl. Also described is a compound of Formula (Ic) wherein X is optionally substituted heteroaryl. Also described is a compound of Formula (Ic) wherein X is heteroaryl which is unsubstituted or substituted once or twice with -(C₁-C₆)alkyl. Also described is a compound of Formula (Ic) wherein X is heteroaryl which is unsubstituted or substituted once with -(C₁-C₆)alkyl. Also described is a compound of Formula (Ic) wherein X is optionally substituted pyridine or optionally substituted pyrimidine. Also described is a compound of Formula (Ic) wherein X is pyridine which is unsubstituted or substituted once or twice with -(C₁-C₆)alkyl. Also described is a compound of Formula (Ic) wherein X is pyridine which is unsubstituted or substituted once with -(C₁-C₆)alkyl. Also described is a compound of Formula (Ic) wherein X is pyridine which is unsubstituted or substituted once or twice with methyl. Also described is a compound of Formula (Ic) wherein X is pyrimidine which is unsubstituted or substituted once or twice with -(C₁-C₆)alkyl. Also described is a compound of Formula (Ic) wherein X is pyrimidine which is unsubstituted or substituted once with -(C₁-C₆)alkyl. Also described is a compound of Formula (Ic) wherein X is pyrimidine which is unsubstituted or substituted once or twice with methyl. Also described is a compound of Formula (Ic) wherein X is pyridine which is substituted once with methyl. Also described is a compound of Formula (Ic) wherein X is pyrimidine which is substituted once with methyl. Also described is a compound of Formula (Ic) wherein X is pyrimidine which is substituted twice with methyl. Also described is a compound of Formula (Ic) wherein X is optionally substituted -(C₁-C₆)alkyl-. Also described is a compound of Formula (Ic) wherein Y is optionally substituted aryl. Also described is a compound of Formula (Ic) wherein Y is optionally substituted phenyl. Also described is a compound of Formula (Ic) wherein Y is optionally substituted heteroaryl. Also described is a compound of Formula (Ic) wherein Y is optionally substituted -(C₁-C₆)alkyl-. Also described is a compound of Formula (Ic) wherein Y is -O-(C₁-C₆)alkyl-. Also described is a compound of Formula (Ic) wherein Y is -N(H)-(C₁-C₆)alkyl-. Also described is a compound of Formula (Ic) wherein Y is a bond. Also described is a compound of Formula (Ic) wherein Z is -(C₁-C₆)alkyl. Also described is a compound of Formula (Ic) wherein Z is optionally substituted aryl. Also described is a compound of Formula (Ic) wherein Z is optionally substituted phenyl. Also described is a compound of Formula (Ic) wherein Z is phenyl substituted once or twice with -(C₁-C₈)alkyl. Also described is a compound of Formula (Ic) wherein Z is phenyl substituted once with n-butyl, isobutyl, or tert-butyl. Also described is a compound of Formula (Ic) wherein Z is phenyl substituted once with n-butyl. Also described is a compound of Formula (Ic) wherein Z is phenyl substituted once with isobutyl. Also described is a compound of Formula (Ic) wherein Z is phenyl substituted once with tert-butyl. Also described is a compound of Formula (Ic) wherein Z is optionally substituted heteroaryl. Also described is a compound of Formula (Ic) wherein Z is optionally substituted - (C₃-C₇)cycloalkyl. Also described is a compound of Formula (Ic) wherein Z is halogen.

Also described is a compound of Formula (Ic) wherein -X-Y-Z is

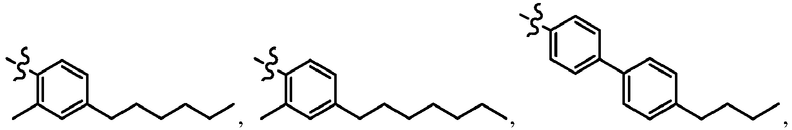

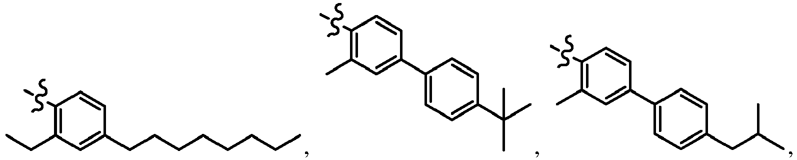

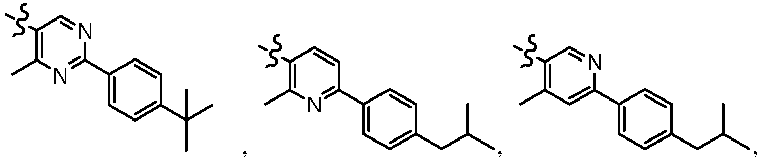

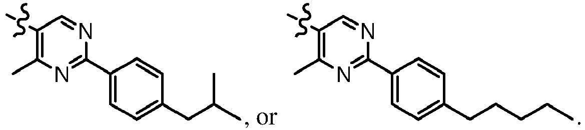

Also described is a compound of Formula (I) having the structure of Formula (Id): 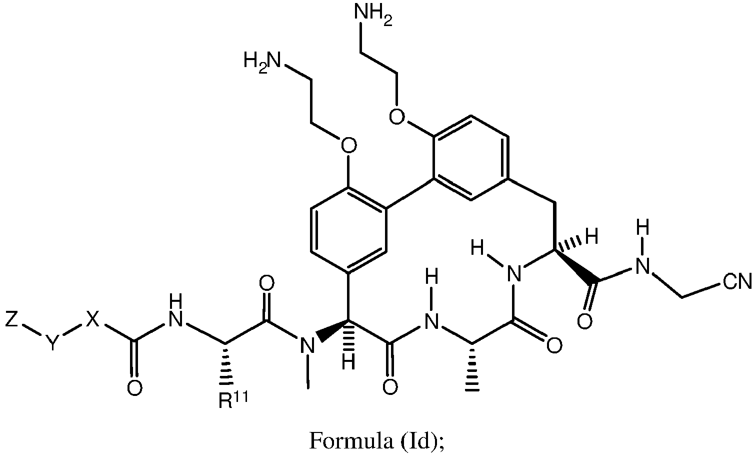 wherein:
R¹¹ is -CH₂NH₂, -CH₂CH₂NH₂, or -CH₂CH₂CH₂NH₂;
X is optionally substituted -(C₁-C₆)alkyl-, -(C₂-C₆)alkenyl-, -(C₂-C₆)alkynyl, -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, - N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, - SO₂(C₁-C₆)alkyl-, -C(O)NH(C₁-C₆)alkyl-, -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-,optionally substituted aryl, or optionally substituted heteroaryl;
Z is H, halogen, -NH₂, -CN, -CF₃, -CO₂H, -(C₁-C₁₂)alkyl,-(C₂-C₁₂)alkenyl, -(C₂-C₁₂)alkynyl, - C(O)NR²⁵R²⁶, -O-(C₁-C₁₂)alkyl, -N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-heterocycloalkyl, optionally substituted aryl, or optionally substituted heteroaryl;
each R²⁴ is independently H or -(C₁-C₆)alkyl; and
R²⁵ and R²⁶ is independently H or optionally substituted -(C₁-C₆)alkyl; or R²⁵ and R²⁶ and the nitrogen atom to which they are attached form a heterocycloalkyl ring;
or a pharmaceutically acceptable salt, solvate, or prodrug thereof.

Also described is a compound of Formula (Id) wherein R¹¹ is -CH₂NH₂. Also described is a compound of Formula (Id) wherein R¹¹ is -CH₂CH₂NH₂. Also described is a compound of Formula (Id) wherein R¹¹ is -CH₂CH₂CH₂NH₂.

Also described is a compound of Formula (Id) wherein X is optionally substituted aryl. Also described is a compound of Formula (Id) wherein X is optionally substituted phenyl. Also described is a compound of Formula (Id) wherein X is optionally substituted heteroaryl. Also described is a compound of Formula (Id) wherein X is heteroaryl which is unsubstituted or substituted once or twice with -(C₁-C₆)alkyl. Also described is a compound of Formula (Id) wherein X is heteroaryl which is unsubstituted or substituted once with -(C₁-C₆)alkyl. Also described is a compound of Formula (Id) wherein X is optionally substituted pyridine or optionally substituted pyrimidine. Also described is a compound of Formula (Id) wherein X is pyridine which is unsubstituted or substituted once or twice with -(C₁-C₆)alkyl. Also described is a compound of Formula (Id) wherein X is pyridine which is unsubstituted or substituted once with -(C₁-C₆)alkyl. Also described is a compound of Formula (Id) wherein X is pyridine which is unsubstituted or substituted once or twice with methyl. Also described is a compound of Formula (Id) wherein X is pyrimidine which is unsubstituted or substituted once or twice with -(C₁-C₆)alkyl. Also described is a compound of Formula (Id) wherein X is pyrimidine which is unsubstituted or substituted once with -(C₁-C₆)alkyl. Also described is a compound of Formula (Id) wherein X is pyrimidine which is unsubstituted or substituted once or twice with methyl. Also described is a compound of Formula (Id) wherein X is pyridine which is substituted once with methyl. Also described is a compound of Formula (Id) wherein X is pyrimidine which is substituted once with methyl. Also described is a compound of Formula (Id) wherein X is pyrimidine which is substituted twice with methyl. Also described is a compound of Formula (Id) wherein X is optionally substituted -(C₁-C₆)alkyl-. Also described is a compound of Formula (Id) wherein Y is optionally substituted aryl. Also described is a compound of Formula (Id) wherein Y is optionally substituted phenyl. Also described is a compound of Formula (Id) wherein Y is optionally substituted heteroaryl. Also described is a compound of Formula (Id) wherein Y is optionally substituted -(C₁-C₆)alkyl-. Also described is a compound of Formula (Id) wherein Y is -O-(C₁-C₆)alkyl-. Also described is a compound of Formula (Id) wherein Y is -N(H)-(C₁-C₆)alkyl-. Also described is a compound of Formula (Id) wherein Y is a bond. Also described is a compound of Formula (Id) wherein Z is -(C₁-C₆)alkyl. Also described is a compound of Formula (Id) wherein Z is optionally substituted aryl. Also described is a compound of Formula (Id) wherein Z is optionally substituted phenyl. Also described is a compound of Formula (Id) wherein Z is phenyl substituted once or twice with -(C₁-C₈)alkyl. Also described is a compound of Formula (Id) wherein Z is phenyl substituted once with n-butyl, isobutyl, or tert-butyl. Also described is a compound of Formula (Id) wherein Z is phenyl substituted once with n-butyl. Also described is a compound of Formula (Id) wherein Z is phenyl substituted once with isobutyl. Also described is a compound of Formula (Id) wherein Z is phenyl substituted once with tert-butyl. Also described is a compound of Formula (Id) wherein Z is optionally substituted heteroaryl. Also described is a compound of Formula (Id) wherein Z is optionally substituted - (C₃-C₇)cycloalkyl. Also described is a compound of Formula (Id) wherein Z is halogen.

Also described is a compound of Formula (Id) wherein -X-Y-Z is

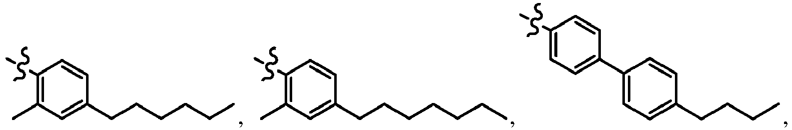

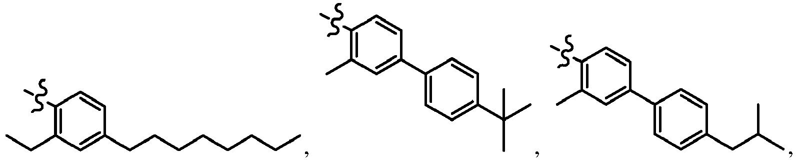

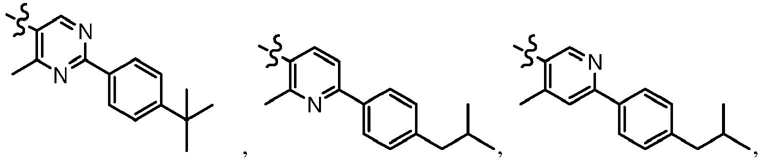

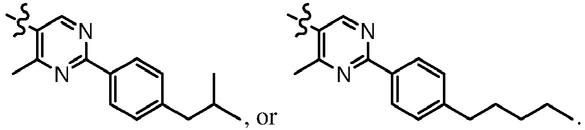

In another aspect described herein is a compound of Formula (II): 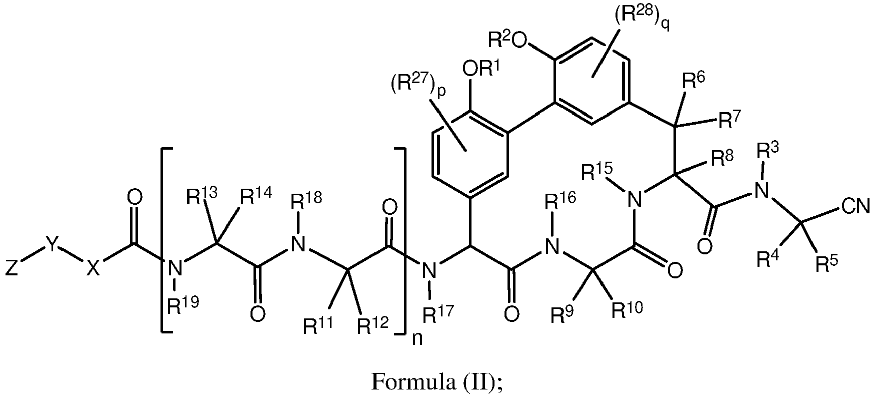 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, -(C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²², or -(C₁-C₆)alkyl-C(O)N(R²³)(C₁-C₆)alkyl, or optionally substituted heterocycloalkyl;
R³ is H, or -(C₁-C₆)alkyl;
R⁴ is H, -(C₁-C₆)alkyl, -(C₁-C₆)alkyl-OH, -(C₃-C₆)cycloalkyl , or -C(O)NH₂; or R³ and R⁴ are combined to form a heterocycloalkyl ring;
R⁵ is H, or -(C₁-C₆)alkyl; or R⁴ and R⁵ and the carbon atom to which they are attached form a cyclopropyl ring;
R⁶, R⁷, and R⁸ are each independently H, or -(C₁-C₆)alkyl;
R⁹ is H, -(C₁-C₆)alkyl, -(C₁-C₆)haloalkyl, or -(C₃-C₆)cycloalkyl;
R¹⁰ is H, or -(C₁-C₆)alkyl;
R¹¹ and R¹² are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-CN, -(C₁-C₆)alkyl-C(O)NR²⁵R²⁶, -(C₁-C₆)heteroalkyl-CO₂H, -(C₁-C₆)alkyl-S(O)-(C₁-C₆)alkyl, -(C₁-C₆)alkyl-N(H)CH=NH, -(C₁-C₆)alkyl-N(H)C(NH)NH₂, -(C₁-C₆)alkyl-heterocycloalkyl, optionally substituted -(C₁-C₆)alkyl-N(H)heterocycloalkyl, or -(C₁-C₆)alkyl-heteroaryl; or R¹¹ and R¹⁸ are combined to form an optionally substituted heterocycloalkyl ring, and R¹² is H;
R¹³ and R¹⁴ are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-CN, -(C₁-C₆)alkyl-C(O)NR²⁵R²⁶, -(C₁-C₆)alkyl-N(H)C(NH)NH₂, -(C₁-C₆)alkyl-heterocycloalkyl, or -(C₁-C₆)alkyl-heteroaryl; or R¹³ and R¹⁹ are combined to form an optionally substituted heterocycloalkyl ring, and R¹⁴ is H;
R¹⁵, R¹⁶, R¹⁷, R¹⁸, and R¹⁹ are each independently H, -(C₁-C₆)alkyl, -(C₃-C₆)cycloalkyl, -(C₁-C₆)alkyl-OR²³, -(C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, - N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, - SO₂(C₁-C₆)alkyl-, -C(O)NH(C₁-C₆)alkyl-, -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-,optionally substituted aryl, or optionally substituted heteroaryl;
Z is H, halogen, -NH₂, -CN, -CF₃, -(C₁-C₁₂)alkyl,-(C₂-C₁₂)alkenyl, -(C₂-C₁₂)alkynyl, - C(O)NR²⁵R²⁶, -O-(C₁-C₁₂)alkyl, -N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, - C(O)(C₁-C₆)alkyl, -C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl; or R²⁵ and R²⁶ and the nitrogen atom to which they are attached form a heterocycloalkyl ring;
each R²⁷ is independently halogen, optionally substituted -(C₁-C₆)alkyl, or optionally substituted - (C₁-C₆)heteroalkyl;
each R²⁸ is independently halogen, optionally substituted -(C₁-C₆)alkyl, or optionally substituted - (C₁-C₆)heteroalkyl;
n is 0 or 1;
p is 0, 1, or 2; and
q is 0, 1, or 2;
or a pharmaceutically acceptable salt, solvate, or prodrug thereof.

Also described is a compound of Formula (II) wherein n is 0. Also described is a compound of Formula (II) wherein n is 1.

Also described is a compound of Formula (II) wherein R⁶, R⁷, and R⁸ are H.

Also described is a compound of Formula (II) wherein R¹⁵ and R¹⁶ are H.

Also described is a compound of Formula (II) wherein R¹⁷ is -(C₁-C₆)alkyl. Also described is a compound of Formula (II) wherein R¹⁷ is -CH₃. Also described is a compound of Formula (II) wherein R¹⁷ is -CH₂CH₃. Also described is a compound of Formula (II) wherein R¹⁷ is -(C₃-C₆)cycloalkyl. Also described is a compound of Formula (II) wherein R¹⁷ is cyclopropyl. Also described is a compound of Formula (II) wherein R¹⁷ is -(C₁-C₆)alkyl-C(O)OR²³. Also described is a compound of Formula (II) wherein R¹⁷ is -CH₂CH₂OH. Also described is a compound of Formula (II) wherein R¹⁷ is -(C₁-C₆)alkyl-NR²¹R²². Also described is a compound of Formula (II) wherein R¹⁷ is -CH₂CH₂NH₂. Also described is a compound of Formula (II) wherein R¹⁷ is H.

Also described is a compound of Formula (II) wherein R¹⁸ is H.

Also described is a compound of Formula (II) wherein R¹⁹ is H.

Also described is a compound of Formula (II) wherein R³ is H.

Also described is a compound of Formula (II) wherein R⁵ is H.

Also described is a compound of Formula (II) wherein R⁴ is H. Also described is a compound of Formula (II) wherein R⁴ is -(C₁-C₆)alkyl. Also described is a compound of Formula (II) wherein R⁴ is -CH₃. Also described is a compound of Formula (II) wherein R⁴ is -CH₂CH₃. Also described is a compound of Formula (II) wherein R⁴ is -(C₁-C₆)alkyl-OH. Also described is a compound of Formula (II) wherein R⁴ is -CH₂OH. Also described is a compound of Formula (II) wherein R⁴ is -(C₃-C₆)cycloalkyl. Also described is a compound of Formula (II) wherein R⁴ is cyclopropyl. Also described is a compound of Formula (II) wherein R⁴ is -C(O)NH₂.

Also described is a compound of Formula (II) wherein R³, R⁴, and R⁵ are H.

Also described is a compound of Formula (II) wherein R⁴ and R⁵ and the carbon atom to which they are attached form a cyclopropyl ring.

Also described is a compound of Formula (II) wherein R¹⁰ is H.

Also described is a compound of Formula (II) wherein R¹⁰ is H and R⁹ is -(C₁-C₆)alkyl. Also described is a compound of Formula (II) wherein R¹⁰ is H and R⁹ is -CH₃. Also described is a compound of Formula (II) wherein R¹⁰ is H and R⁹ is -CH₂CH₃. Also described is a compound of Formula (II) wherein R¹⁰ is H and R⁹ is -(C₁-C₆)haloalkyl. Also described is a compound of Formula (II) wherein R¹⁰ is H and R⁹ is -CH₂F. Also described is a compound of Formula (II) wherein R¹⁰ is H and R⁹ is -CHF₂. Also described is a compound of Formula (II) wherein R¹⁰ is H and R⁹ is -(C₃-C₆)cycloalkyl. Also described is a compound of Formula (II) wherein R¹⁰ is H and R⁹ is cyclopropyl. Also described is a compound of Formula (II) wherein R¹⁰ is H and R⁹ is H.

Also described is a compound of Formula (II) wherein R¹² is H.

Also described is a compound of Formula (II) wherein R¹² is H and R¹¹ is -(C₁-C₆)alkyl. Also described is a compound of Formula (II) wherein R¹² is H and R¹¹ is -CH₃. Also described is a compound of Formula (II) wherein R¹² is H and R¹¹ is -(C₁-C₆)alkyl-OR²³. Also described is a compound of Formula (II) wherein R¹¹ is -CH₂OH. Also described is a compound of Formula (II) wherein R¹² is H and R¹¹ is -CH₂CH₂OH. Also described is a compound of Formula (II) wherein R¹² is H and R¹¹ is -(C₁-C₆)alkyl. Also described is a compound of Formula (II) wherein R¹² is H and R¹¹ is -(C₁-C₆)alkyl-NR²¹R²². Also described is a compound of Formula (II) wherein R¹² is H and R¹¹ is -(C₁-C₆)alkyl-NH₂. Also described is a compound of Formula (II) wherein R¹² is H and R¹¹ is -CH₂NH₂. Also described is a compound of Formula (II) wherein R¹² is H and R¹¹ is -CH₂CH₂NH₂. Also described is a compound of Formula (II) wherein R¹² is H and R¹¹ is - CH₂CH₂CH₂NH₂. Also described is a compound of Formula (II) wherein R¹² is H and R¹¹ is - CH₂CH₂CH₂CH₂NH₂. Also described is a compound of Formula (II) wherein R¹² is H and R¹¹ is -(C₁-C₆)alkyl-CN. Also described is a compound of Formula (II) wherein R¹² is H and R¹¹ is - CH₂CN. Also described is a compound of Formula (II) wherein R¹² is H and R¹¹ is -(C₁-C₆)alkyl-C(O)NR²⁵R²⁶, Also described is a compound of Formula (II) wherein R¹² is H and R¹¹ is - CH₂C(O)NH₂. Also described is a compound of Formula (II) wherein R¹² is H and R¹¹ is - CH₂CH₂C(O)NH₂. Also described is a compound of Formula (II) wherein R¹² is H and R¹¹ is - (C₁-C₆)alkyl-heteroaryl. Also described is a compound of Formula (II) wherein R¹² is H and R¹¹ is H.

Also described is a compound of Formula (II) wherein R¹¹ and R¹⁸ are combined to form an optionally substituted heterocycloalkyl ring and R¹² is H.

Also described is a compound of Formula (II) wherein R¹⁴ is H.

Also described is a compound of Formula (II) wherein R¹⁴ is H and R¹³ is -(C₁-C₆)alkyl. Also described is a compound of Formula (II) wherein R¹⁴ is H and R¹³ is -CH₃. Also described is a compound of Formula (II) wherein R¹⁴ is H and R¹³ is -(C₁-C₆)alkyl-OR²³. Also described is a compound of Formula (II) wherein R¹⁴ is H and R¹³ is -CH₂OH. Also described is a compound of Formula (II) wherein R¹⁴ is H and R¹³ is -CH₂CH₂OH. Also described is a compound of Formula (II) wherein R¹⁴ is H and R¹³ is -(C₁-C₆)alkyl. Also described is a compound of Formula (II) wherein R¹⁴ is H and R¹³ is -(C₁-C₆)alkyl-NR²¹R²². Also described is a compound of Formula (II) wherein R¹⁴ is H and R¹³ is -(C₁-C₆)alkyl-NH₂. Also described is a compound of Formula (II) wherein R¹⁴ is H and R¹³ is -CH₂NH₂. Also described is a compound of Formula (II) wherein R¹⁴ is H and R¹³ is -CH₂CH₂NH₂. Also described is a compound of Formula (II) wherein R¹⁴ is H and R¹³ is -CH₂CH₂CH₂NH₂. Also described is a compound of Formula (II) wherein R¹⁴ is H and R¹³ is -CH₂CH₂CH₂CH₂NH₂. Also described is a compound of Formula (II) wherein R¹⁴ is H and R¹³ is -(C₁-C₆)alkyl-CN. Also described is a compound of Formula (II) wherein R¹⁴ is H and R¹³ is -CH₂CN. Also described is a compound of Formula (II) wherein R¹⁴ is H and R¹³ is -(C₁-C₆)alkyl-C(O)NR²⁵R²⁶. Also described is a compound of Formula (II) wherein R¹⁴ is H and R¹³ is -CH₂C(O)NH₂. Also described is a compound of Formula (II) wherein R¹⁴ is H and R¹³ is - CH₂CH₂C(O)NH₂. Also described is a compound of Formula (II) wherein R¹⁴ is H and R¹³ is - (C₁-C₆)alkyl-heteroaryl. Also described is a compound of Formula (II) wherein R¹⁴ is H and R¹³ is H.

Also described is a compound of Formula (II) wherein R¹³ and R¹⁹ are combined to form an optionally substituted heterocycloalkyl ring and R¹⁴ is H.

Also described is a compound of Formula (II) wherein p is 1 and R²⁷ is halogen. Also described is a compound of Formula (II) wherein p is 1 and R²⁷ is optionally substituted -(C₁-C₆)alkyl. Also described is a compound of Formula (II) wherein q is 0, p is 1 and R²⁷ is halogen. Also described is a compound of Formula (II) wherein q is 0, p is 1 and R²⁷ is optionally substituted -(C₁-C₆)alkyl. Also described is a compound of Formula (II) wherein q is 1 and R²⁸ is halogen. Also described is a compound of Formula (II) wherein q is 1 and R²⁸ is optionally substituted -(C₁-C₆)alkyl. Also described is a compound of Formula (II) wherein p is 0, q is 1 and R²⁸ is halogen. Also described is a compound of Formula (II) wherein p is 0, q is 1 and R²⁸ is optionally substituted -(C₁-C₆)alkyl.

Also described is a compound of Formula (II) wherein p is 0, and q is 0.

Also described is a compound of Formula (II) wherein R¹ and R² are each independently H, or -(C₁-C₆)alkyl-NR²¹R²². Also described is a compound of Formula (II) wherein R¹ and R² are each H. Also described is a compound of Formula (II) wherein R¹ and R² are each independently -(C₁-C₆)alkyl-NR²¹R²². Also described is a compound of Formula (II) wherein R¹ is H, and R² is -(C₁-C₆)alkyl-NR²¹R²². Also described is a compound of Formula (II) wherein R¹ is -(C₁-C₆)alkyl-NR²¹R²², and R² is H. Also described is a compound of Formula (II) wherein R¹ is H, and R² is -CH₂CH₂NH₂. Also described is a compound of Formula (II) wherein R¹ is - CH₂CH₂NH₂, and R² is H. Also described is a compound of Formula (II) wherein R¹ and R² are each -CH₂CH₂NH₂.

Also described is a compound of Formula (II) wherein X is optionally substituted aryl. Also described is a compound of Formula (II) wherein X is optionally substituted phenyl. Also described is a compound of Formula (II) wherein X is optionally substituted heteroaryl. Also described is a compound of Formula (II) wherein X is optionally substituted pyridine or optionally substituted pyrimidine. Also described is a compound of Formula (II) wherein X is optionally substituted -(C₁-C₆)alkyl-. Also described is a compound of Formula (II) wherein Y is optionally substituted aryl. Also described is a compound of Formula (II) wherein Y is optionally substituted phenyl. Also described is a compound of Formula (II) wherein Y is optionally substituted heteroaryl. Also described is a compound of Formula (II) wherein Y is optionally substituted -(C₁-C₆)alkyl-. Also described is a compound of Formula (II) wherein Y is -O-(C₁-C₆)alkyl-. Also described is a compound of Formula (II) wherein Y is -N(H)-(C₁-C₆)alkyl-. Also described is a compound of Formula (II) wherein Y is a bond. Also described is a compound of Formula (II) wherein Z is -(C₁-C₆)alkyl. Also described is a compound of Formula (II) wherein Z is optionally substituted aryl. Also described is a compound of Formula (II) wherein Z is optionally substituted phenyl. Also described is a compound of Formula (II) wherein Z is optionally substituted heteroaryl. Also described is a compound of Formula (II) wherein Z is optionally substituted -(C₃-C₇)cycloalkyl. Also described is a compound of Formula (II) wherein Z is halogen.

Also described is a compound of Formula (II) wherein -X-Y-Z is

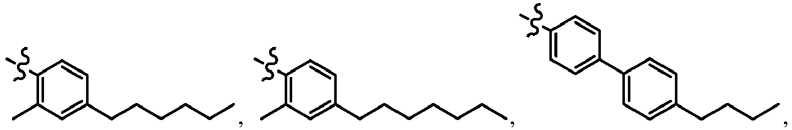

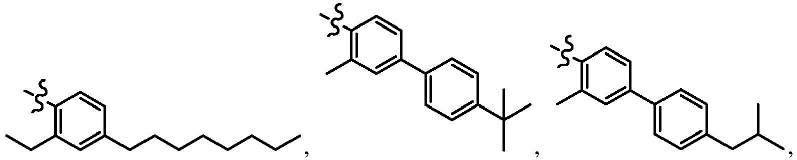

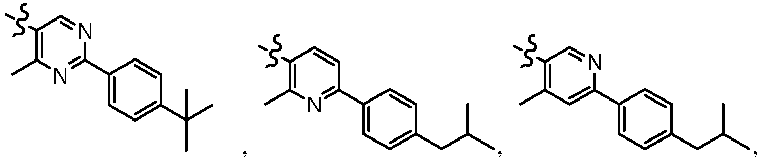

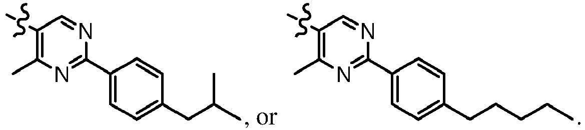

In another aspect described herein is a compound of Formula (II'): 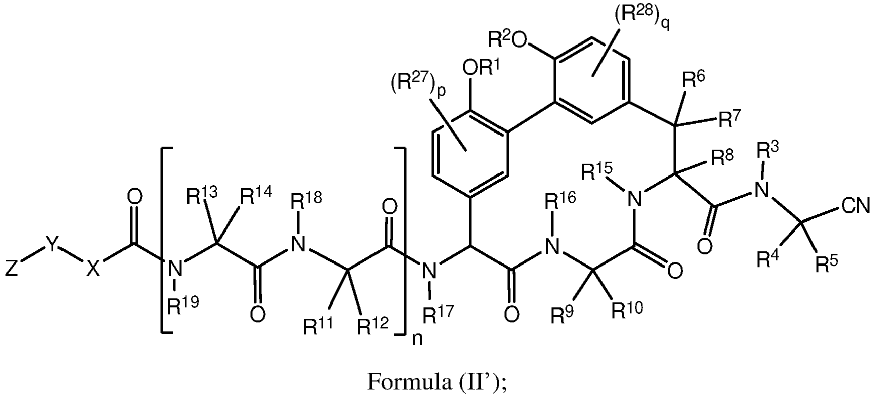 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, -(C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²², -(C₁-C₆)alkyl-C(O)N(R²³)(C₁-C₆)alkyl, -(C₁-C₆)alkyl-C(O)N(R²³)(C₁-C₆)alkyl-heterocycloalkyl, optionally substituted (C₁-C₆)heteroalkyl or optionally substituted heterocycloalkyl;
R³ is H, or -(C₁-C₆)alkyl;
R⁴ is H, -(C₁-C₆)alkyl, -(C₁-C₆)alkyl-OH, -(C₃-C₆)cycloalkyl , or -C(O)NH₂; or R³ and R⁴ are combined to form a heterocycloalkyl ring;
R⁵ is H, or -(C₁-C₆)alkyl; or R⁴ and R⁵ and the carbon atom to which that are attached form a cyclopropyl ring;
R⁶, R⁷, and R⁸ are each independently H, or -(C₁-C₆)alkyl;
R⁹ is H, -(C₁-C₆)alkyl, -(C₁-C₆)haloalkyl, or -(C₃-C₆)cycloalkyl;
R¹⁰ is H, or -(C₁-C₆)alkyl;
R¹¹ and R¹² are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-CN, -(C₁-C₆)alkyl-C(O)NR²⁵R²⁶, -(C₁-C₆)heteroalkyl-CO₂H, -(C₁-C₆)alkyl-S(O)-(C₁-C₆)alkyl, -(C₁-C₆)alkyl-N(H)CH=NH, -(C₁-C₆)alkyl-C(NH₂)=NH, -(C₁-C₆)alkyl-N(H)C(NH)NH₂, -(C₁-C₆)alkyl-N(H)SO₂NR²⁵R²⁶, -(C₁-C₆)alkyl-N(H)-C(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl; or R¹¹ and R¹⁸ are combined to form an optionally substituted heterocycloalkyl ring, and R¹² is H;
R¹³ and R¹⁴ are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-CN, -(C₁-C₆)alkyl-C(O)NR²⁵R²⁶, -(C₁-C₆)alkyl-N(H)C(NH)NH₂, -(C₁-C₆)alkyl-heterocycloalkyl, or -(C₁-C₆)alkyl-heteroaryl; or R¹³ and R¹⁹ are combined to form an optionally substituted heterocycloalkyl ring, and R¹⁴ is H;
R¹⁵, R¹⁶, R¹⁷, R¹⁸, and R¹⁹ are each independently H, -(C₁-C₆)alkyl, -(C₃-C₆)cycloalkyl, -(C₁-C₆)alkyl-OR²³, -(C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, - N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, - SO₂(C₁-C₆)alkyl-, -C(O)NH(C₁-C₆)alkyl-, -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-,optionally substituted aryl, or optionally substituted heteroaryl;
Z is H, halogen, -NH₂, -CN, -CF₃, -(C₁-C₁₂)alkyl,-(C₂-C₁₂)alkenyl, -(C₂-C₁₂)alkynyl, - C(O)NR²⁵R²⁶, -O-(C₁-C₁₂)alkyl, -N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, - C(O)(C₁-C₆)alkyl, -C(O)N(R³¹)₂, or -SO₂N(R³¹)₂; or R²¹ and R²² and the nitrogen atom to which that are attached form a heterocycloalkyl ring;
each R³¹ is independently H or -(C₁-C₆)alkyl; or two R³¹ and the nitrogen atom to which that are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl; or R²⁵ and R²⁶ and the nitrogen atom to which that are attached form a heterocycloalkyl ring;
each R²⁷ is independently halogen, -NR²³R²⁴, -NC(O)R²³, -NC(O)NR²³R²⁴), nitro, hydroxyl, optionally substituted -(C₁-C₆)alkyl, optionally substituted -(C₁-C₆)heteroalkyl, -(C₁-C₆)alkoxy, - C(O)(C₁-C₆)alkyl, or -S(O)₂(C₁-C₆)alkyl;
each R²⁸ is independently halogen, -NR²³R²⁴, -NC(O)R²³, -NC(O)NR²³R²⁴), nitro, hydroxyl, optionally substituted -(C₁-C₆)alkyl, optionally substituted -(C₁-C₆)heteroalkyl, -(C₁-C₆)alkoxy, - C(O)(C₁-C₆)alkyl, or -S(O)₂(C₁-C₆)alkyl;
n is 0 or 1;
p is 0, 1, or 2; and
q is 0, 1, or 2;
or a pharmaceutically acceptable salt, solvate, or prodrug thereof.

Also described is a compound of Formula (II') wherein n is 0. Also described is a compound of Formula (II') wherein n is 1.

Also described is a compound of Formula (II') wherein R⁶, R⁷, and R⁸ are H.

Also described is a compound of Formula (II') wherein R¹⁵ and R¹⁶ are H.

Also described is a compound of Formula (II') wherein R¹⁷ is -(C₁-C₆)alkyl. Also described is a compound of Formula (II') wherein R¹⁷ is -CH₃. Also described is a compound of Formula (II') wherein R¹⁷ is -CH₂CH₃. Also described is a compound of Formula (II') wherein R¹⁷ is -(C₃-C₆)cycloalkyl. Also described is a compound of Formula (II') wherein R¹⁷ is cyclopropyl. Also described is a compound of Formula (II') wherein R¹⁷ is -(C₁-C₆)alkyl-C(O)OR²³. Also described is a compound of Formula (II') wherein R¹⁷ is -CH₂CH₂OH. Also described is a compound of Formula (II') wherein R¹⁷ is -(C₁-C₆)alkyl-NR²¹R²². Also described is a compound of Formula (II') wherein R¹⁷ is -CH₂CH₂NH₂. Also described is a compound of Formula (II') wherein R¹⁷ is H.

Also described is a compound of Formula (II') wherein R¹⁸ is H.

Also described is a compound of Formula (II') wherein R¹⁹ is H.

Also described is a compound of Formula (II') wherein R³ is H.

Also described is a compound of Formula (II') wherein R⁵ is H.

Also described is a compound of Formula (II') wherein R⁴ is H. Also described is a compound of Formula (II') wherein R⁴ is -(C₁-C₆)alkyl. Also described is a compound of Formula (II') wherein R⁴ is -CH₃. Also described is a compound of Formula (II') wherein R⁴ is - CH₂CH₃. Also described is a compound of Formula (II') wherein R⁴ is -(C₁-C₆)alkyl-OH. Also described is a compound of Formula (II') wherein R⁴ is -CH₂OH. Also described is a compound of Formula (II') wherein R⁴ is -(C₃-C₆)cycloalkyl. Also described is a compound of Formula (II') wherein R⁴ is cyclopropyl. Also described is a compound of Formula (II') wherein R⁴ is - C(O)NH₂.

Also described is a compound of Formula (II') wherein R³, R⁴, and R⁵ are H.

Also described is a compound of Formula (II') wherein R⁴ and R⁵ and the carbon atom to which they are attached form a cyclopropyl ring.

Also described is a compound of Formula (II') wherein R¹⁰ is H.

Also described is a compound of Formula (II') wherein R¹⁰ is H and R⁹ is -(C₁-C₆)alkyl. Also described is a compound of Formula (II') wherein R¹⁰ is H and R⁹ is -CH₃. Also described is a compound of Formula (II') wherein R¹⁰ is H and R⁹ is -CH₂CH₃. Also described is a compound of Formula (II') wherein R¹⁰ is H and R⁹ is -(C₁-C₆)haloalkyl. Also described is a compound of Formula (II') wherein R¹⁰ is H and R⁹ is -CH₂F. Also described is a compound of Formula (II') wherein R¹⁰ is H and R⁹ is -CHF₂. Also described is a compound of Formula (II') wherein R¹⁰ is H and R⁹ is -(C₃-C₆)cycloalkyl. Also described is a compound of Formula (II') wherein R¹⁰ is H and R⁹ is cyclopropyl. Also described is a compound of Formula (II') wherein R¹⁰ is H and R⁹ is H.

Also described is a compound of Formula (II') wherein R¹² is H.

Also described is a compound of Formula (II') wherein R¹² is H and R¹¹ is -(C₁-C₆)alkyl. Also described is a compound of Formula (II') wherein R¹² is H and R¹¹ is -CH₃. Also described is a compound of Formula (II') wherein R¹² is H and R¹¹ is -(C₁-C₆)alkyl-OR²³. Also described is a compound of Formula (II') wherein R¹¹ is -CH₂OH. Also described is a compound of Formula (II') wherein R¹² is H and R¹¹ is -CH₂CH₂OH. Also described is a compound of Formula (II') wherein R¹² is H and R¹¹ is -(C₁-C₆)alkyl. Also described is a compound of Formula (II') wherein R¹² is H and R¹¹ is -(C₁-C₆)alkyl-NR²¹R²². Also described is a compound of Formula (II') wherein R¹² is H and R¹¹ is -(C₁-C₆)alkyl-NH₂. Also described is a compound of Formula (II') wherein R¹² is H and R¹¹ is -CH₂NH₂. Also described is a compound of Formula (II') wherein R¹² is H and R¹¹ is -CH₂CH₂NH₂. Also described is a compound of Formula (II') wherein R¹² is H and R¹¹ is -CH₂CH₂CH₂NH₂. Also described is a compound of Formula (II') wherein R¹² is H and R¹¹ is -CH₂CH₂CH₂CH₂NH₂. Also described is a compound of Formula (II') wherein R¹² is H and R¹¹ is -(C₁-C₆)alkyl-CN. Also described is a compound of Formula (II') wherein R¹² is H and R¹¹ is -CH₂CN. Also described is a compound of Formula (II') wherein R¹² is H and R¹¹ is -(C₁-C₆)alkyl-C(O)NR²⁵R²⁶. Also described is a compound of Formula (II') wherein R¹² is H and R¹¹ is -CH₂C(O)NH₂. Also described is a compound of Formula (II') wherein R¹² is H and R¹¹ is -CH₂CH₂C(O)NH₂. Also described is a compound of Formula (II') wherein R¹² is H and R¹¹ is -(C₁-C₆)alkyl-heteroaryl. Also described is a compound of Formula (II') wherein R¹² is H and R¹¹ is H.

Also described is a compound of Formula (II') wherein R¹¹ and R¹⁸ are combined to form an optionally substituted heterocycloalkyl ring and R¹² is H.

Also described is a compound of Formula (II') wherein R¹⁴ is H.

Also described is a compound of Formula (II') wherein R¹⁴ is H and R¹³ is -(C₁-C₆)alkyl. Also described is a compound of Formula (II') wherein R¹⁴ is H and R¹³ is -CH₃. Also described is a compound of Formula (II') wherein R¹⁴ is H and R¹³ is -(C₁-C₆)alkyl-OR²³. Also described is a compound of Formula (II') wherein R¹⁴ is H and R¹³ is -CH₂OH. Also described is a compound of Formula (II') wherein R¹⁴ is H and R¹³ is -CH₂CH₂OH. Also described is a compound of Formula (II') wherein R¹⁴ is H and R¹³ is -(C₁-C₆)alkyl. Also described is a compound of Formula (II') wherein R¹⁴ is H and R¹³ is -(C₁-C₆)alkyl-NR²¹R²². Also described is a compound of Formula (II') wherein R¹⁴ is H and R¹³ is -(C₁-C₆)alkyl-NH₂. Also described is a compound of Formula (II') wherein R¹⁴ is H and R¹³ is -CH₂NH₂. Also described is a compound of Formula (II') wherein R¹⁴ is H and R¹³ is -CH₂CH₂NH₂. Also described is a compound of Formula (II') wherein R¹⁴ is H and R¹³ is -CH₂CH₂CH₂NH₂. Also described is a compound of Formula (II') wherein R¹⁴ is H and R¹³ is -CH₂CH₂CH₂CH₂NH₂. Also described is a compound of Formula (II') wherein R¹⁴ is H and R¹³ is -(C₁-C₆)alkyl-CN. Also described is a compound of Formula (II') wherein R¹⁴ is H and R¹³ is -CH₂CN. Also described is a compound of Formula (II') wherein R¹⁴ is H and R¹³ is -(C₁-C₆)alkyl-C(O)NR²⁵R²⁶. Also described is a compound of Formula (II') wherein R¹⁴ is H and R¹³ is -CH₂C(O)NH₂. Also described is a compound of Formula (II') wherein R¹⁴ is H and R¹³ is -CH₂CH₂C(O)NH₂. Also described is a compound of Formula (II') wherein R¹⁴ is H and R¹³ is -(C₁-C₆)alkyl-heteroaryl. Also described is a compound of Formula (II') wherein R¹⁴ is H and R¹³ is H.

Also described is a compound of Formula (II') wherein R¹³ and R¹⁹ are combined to form an optionally substituted heterocycloalkyl ring and R¹⁴ is H.

Also described is a compound of Formula (II') wherein p is 1 and R²⁷ is halogen. Also described is a compound of Formula (II') wherein p is 1 and R²⁷ is optionally substituted -(C₁-C₆)alkyl. Also described is a compound of Formula (II') wherein q is 0, p is 1 and R²⁷ is halogen. Also described is a compound of Formula (II') wherein q is 0, p is 1 and R²⁷ is optionally substituted -(C₁-C₆)alkyl. Also described is a compound of Formula (II') wherein q is 1 and R²⁸ is halogen. Also described is a compound of Formula (II') wherein q is 1 and R²⁸ is optionally substituted -(C₁-C₆)alkyl. Also described is a compound of Formula (II') wherein p is 0, q is 1 and R²⁸ is halogen. Also described is a compound of Formula (II') wherein p is 0, q is 1 and R²⁸ is optionally substituted -(C₁-C₆)alkyl.

Also described is a compound of Formula (II') wherein p is 0, and q is 0.

Also described is a compound of Formula (II') wherein R¹ and R² are each independently H, or -(C₁-C₆)alkyl-NR²¹R²². Also described is a compound of Formula (II') wherein R¹ and R² are each H. Also described is a compound of Formula (II') wherein R¹ and R² are each independently -(C₁-C₆)alkyl-NR²¹R²². Also described is a compound of Formula (II') wherein R¹ is H, and R² is -(C₁-C₆)alkyl-NR²¹R²². Also described is a compound of Formula (II') wherein R¹ is -(C₁-C₆)alkyl-NR²¹R²², and R² is H. Also described is a compound of Formula (II') wherein R¹ is H, and R² is -CH₂CH₂NH₂. Also described is a compound of Formula (II') wherein R¹ is -CH₂CH₂NH₂, and R² is H. Also described is a compound of Formula (II') wherein R¹ and R² are each -CH₂CH₂NH₂.

Also described is a compound of Formula (II') wherein X is optionally substituted aryl. Also described is a compound of Formula (II') wherein X is optionally substituted phenyl. Also described is a compound of Formula (II') wherein X is optionally substituted heteroaryl. Also described is a compound of Formula (II') wherein X is optionally substituted pyridine or optionally substituted pyrimidine. Also described is a compound of Formula (II') wherein X is optionally substituted -(C₁-C₆)alkyl-. Also described is a compound of Formula (II') wherein Y is optionally substituted aryl. Also described is a compound of Formula (II') wherein Y is optionally substituted phenyl. Also described is a compound of Formula (II') wherein Y is optionally substituted heteroaryl. Also described is a compound of Formula (II') wherein Y is optionally substituted -(C₁-C₆)alkyl-. Also described is a compound of Formula (II') wherein Y is -O-(C₁-C₆)alkyl-. Also described is a compound of Formula (II') wherein Y is -N(H)-(C₁-C₆)alkyl-. Also described is a compound of Formula (II') wherein Y is a bond. Also described is a compound of Formula (II') wherein Z is -(C₁-C₆)alkyl. Also described is a compound of Formula (II') wherein Z is optionally substituted aryl. Also described is a compound of Formula (II') wherein Z is optionally substituted phenyl. Also described is a compound of Formula (II') wherein Z is optionally substituted heteroaryl. Also described is a compound of Formula (II') wherein Z is optionally substituted -(C₃-C₇)cycloalkyl. Also described is a compound of Formula (II') wherein Z is halogen.

Also described is a compound of Formula (II') wherein -X-Y-Z is

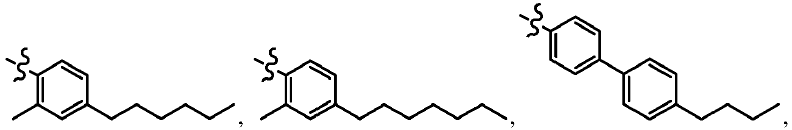

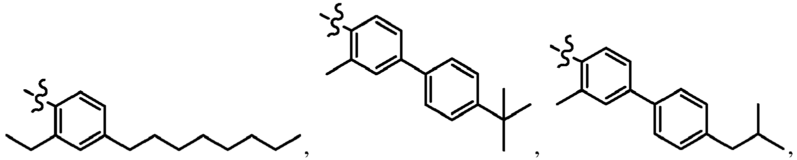

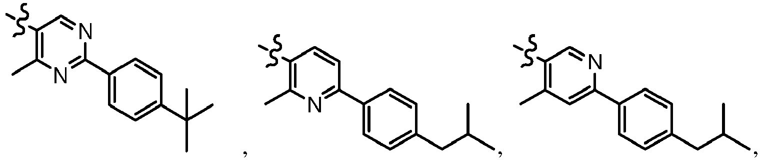

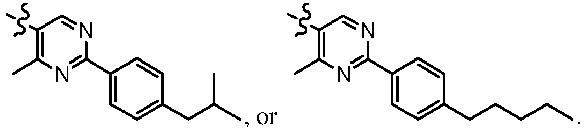

Also described herein is a compound of Formula (II) having the structure of Formula (IIa): 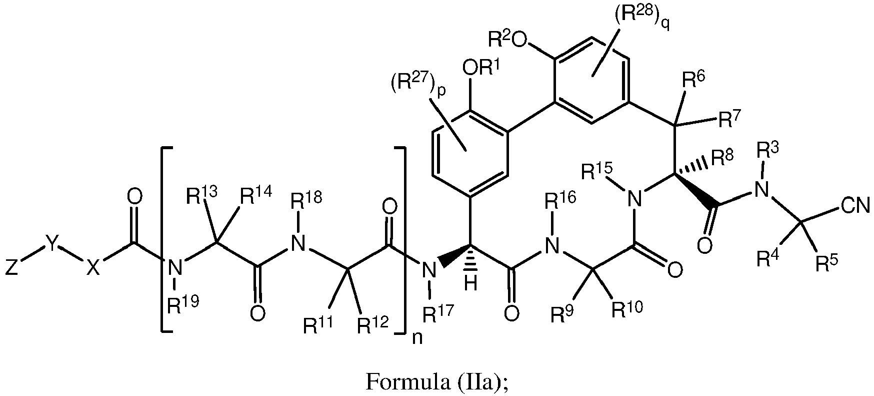 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, -(C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²², or -(C₁-C₆)alkyl-C(O)N(R²³)(C₁-C₆)alkyl, or optionally substituted heterocycloalkyl;
R³ is H, or -(C₁-C₆)alkyl;
R⁴ is H, -(C₁-C₆)alkyl, -(C₁-C₆)alkyl-OH, -(C₃-C₆)cycloalkyl , or -C(O)NH₂; or R³ and R⁴ are combined to form a heterocycloalkyl ring;
R⁵ is H, or -(C₁-C₆)alkyl; or R⁴ and R⁵ and the carbon atom to which they are attached form a cyclopropyl ring;
R⁶, R⁷, and R⁸ are each independently H, or -(C₁-C₆)alkyl;
R⁹ is H, -(C₁-C₆)alkyl, -(C₁-C₆)haloalkyl, or -(C₃-C₆)cycloalkyl;
R¹⁰ is H, or -(C₁-C₆)alkyl;
R¹¹ and R¹² are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-CN, -(C₁-C₆)alkyl-C(O)NR²⁵R²⁶, -(C₁-C₆)heteroalkyl-CO₂H, -(C₁-C₆)alkyl-S(O)-(C₁-C₆)alkyl, -(C₁-C₆)alkyl-N(H)CH=NH, -(C₁-C₆)alkyl-N(H)C(NH)NH₂, -(C₁-C₆)alkyl-heterocycloalkyl, optionally substituted -(C₁-C₆)alkyl-N(H)heterocycloalkyl, or -(C₁-C₆)alkyl-heteroaryl; or R¹¹ and R¹⁸ are combined to form an optionally substituted heterocycloalkyl ring, and R¹² is H;
R¹³ and R¹⁴ are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-CN, -(C₁-C₆)alkyl-C(O)NR²⁵R²⁶, -(C₁-C₆)alkyl-N(H)C(NH)NH₂, -(C₁-C₆)alkyl-heterocycloalkyl, or -(C₁-C₆)alkyl-heteroaryl; or R¹³ and R¹⁹ are combined to form an optionally substituted heterocycloalkyl ring, and R¹⁴ is H;
R¹⁵, R¹⁶, R¹⁷, R¹⁸, and R¹⁹ are each independently H, -(C₁-C₆)alkyl, -(C₃-C₆)cycloalkyl, -(C₁-C₆)alkyl-OR²³, -(C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, - N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, - SO₂(C₁-C₆)alkyl-, -C(O)NH(C₁-C₆)alkyl-, -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-,optionally substituted aryl, or optionally substituted heteroaryl;
Z is H, halogen, -NH₂, -CN, -CF₃, -(C₁-C₁₂)alkyl,-(C₂-C₁₂)alkenyl, -(C₂-C₁₂)alkynyl, - C(O)NR²⁵R²⁶, -O-(C₁-C₁₂)alkyl, -N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, - C(O)(C₁-C₆)alkyl, -C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl; or R²⁵ and R²⁶ and the nitrogen atom to which they are attached form a heterocycloalkyl ring;
each R²⁷ is independently halogen, optionally substituted -(C₁-C₆)alkyl, or optionally substituted - (C₁-C₆)heteroalkyl;
each R²⁸ is independently halogen, optionally substituted -(C₁-C₆)alkyl, or optionally substituted - (C₁-C₆)heteroalkyl;
n is 0 or 1;
p is 0, 1, or 2; and
q is 0, 1, or 2;
or a pharmaceutically acceptable salt, solvate, or prodrug thereof.

Also described is a compound of Formula (IIa) wherein n is 0. Also described is a compound of Formula (IIa) wherein n is 1.

Also described is a compound of Formula (IIa) wherein R⁶, R⁷, and R⁸ are H.

Also described is a compound of Formula (IIa) wherein R¹⁵ and R¹⁶ are H.

Also described is a compound of Formula (IIa) wherein R¹⁷ is -(C₁-C₆)alkyl. Also described is a compound of Formula (IIa) wherein R¹⁷ is -CH₃. Also described is a compound of Formula (IIa) wherein R¹⁷ is -CH₂CH₃. Also described is a compound of Formula (IIa) wherein R¹⁷ is -(C₃-C₆)cycloalkyl. Also described is a compound of Formula (IIa) wherein R¹⁷ is cyclopropyl. Also described is a compound of Formula (IIa) wherein R¹⁷ is -(C₁-C₆)alkyl-C(O)OR²³. Also described is a compound of Formula (IIa) wherein R¹⁷ is -CH₂CH₂OH. Also described is a compound of Formula (IIa) wherein R¹⁷ is -(C₁-C₆)alkyl-NR²¹R²². Also described is a compound of Formula (IIa) wherein R¹⁷ is -CH₂CH₂NH₂. Also described is a compound of Formula (IIa) wherein R¹⁷ is H.

Also described is a compound of Formula (IIa) wherein R¹⁸ is H.

Also described is a compound of Formula (IIa) wherein R¹⁹ is H.

Also described is a compound of Formula (IIa) wherein R³ is H.

Also described is a compound of Formula (IIa) wherein R⁵ is H.

Also described is a compound of Formula (IIa) wherein R⁴ is H. Also described is a compound of Formula (IIa) wherein R⁴ is -(C₁-C₆)alkyl. Also described is a compound of Formula (IIa) wherein R⁴ is -CH₃. Also described is a compound of Formula (IIa) wherein R⁴ is - CH₂CH₃. Also described is a compound of Formula (IIa) wherein R⁴ is -(C₁-C₆)alkyl-OH. Also described is a compound of Formula (IIa) wherein R⁴ is -CH₂OH. Also described is a compound of Formula (IIa) wherein R⁴ is -(C₃-C₆)cycloalkyl. Also described is a compound of Formula (IIa) wherein R⁴ is cyclopropyl. Also described is a compound of Formula (IIa) wherein R⁴ is - C(O)NH₂.

Also described is a compound of Formula (IIa) wherein R³, R⁴, and R⁵ are H.

Also described is a compound of Formula (IIa) wherein R⁴ and R⁵ and the carbon atom to which they are attached form a cyclopropyl ring.

Also described is a compound of Formula (IIa) wherein R¹⁰ is H.

Also described is a compound of Formula (IIa) wherein R¹⁰ is H and R⁹ is -(C₁-C₆)alkyl. Also described is a compound of Formula (IIa) wherein R¹⁰ is H and R⁹ is -CH₃. Also described is a compound of Formula (IIa) wherein R¹⁰ is H and R⁹ is -CH₂CH₃. Also described is a compound of Formula (IIa) wherein R¹⁰ is H and R⁹ is -(C₁-C₆)haloalkyl. Also described is a compound of Formula (IIa) wherein R¹⁰ is H and R⁹ is -CH₂F. Also described is a compound of Formula (IIa) wherein R¹⁰ is H and R⁹ is -CHF₂. Also described is a compound of Formula (IIa) wherein R¹⁰ is H and R⁹ is -(C₃-C₆)cycloalkyl. Also described is a compound of Formula (IIa) wherein R¹⁰ is H and R⁹ is cyclopropyl. Also described is a compound of Formula (IIa) wherein R¹⁰ is H and R⁹ is H.

Also described is a compound of Formula (IIa) wherein R¹² is H.

Also described is a compound of Formula (IIa) wherein R¹² is H and R¹¹ is -(C₁-C₆)alkyl. Also described is a compound of Formula (IIa) wherein R¹² is H and R¹¹ is -CH₃. Also described is a compound of Formula (IIa) wherein R¹² is H and R¹¹ is -(C₁-C₆)alkyl-OR²³. Also described is a compound of Formula (IIa) wherein R¹¹ is -CH₂OH. Also described is a compound of Formula (IIa) wherein R¹² is H and R¹¹ is -CH₂CH₂OH. Also described is a compound of Formula (IIa) wherein R¹² is H and R¹¹ is -(C₁-C₆)alkyl. Also described is a compound of Formula (IIa) wherein R¹² is H and R¹¹ is -(C₁-C₆)alkyl-NR²¹R²². Also described is a compound of Formula (IIa) wherein R¹² is H and R¹¹ is -(C₁-C₆)alkyl-NH₂. Also described is a compound of Formula (IIa) wherein R¹² is H and R¹¹ is -CH₂NH₂. Also described is a compound of Formula (IIa) wherein R¹² is H and R¹¹ is -CH₂CH₂NH₂. Also described is a compound of Formula (IIa) wherein R¹² is H and R¹¹ is -CH₂CH₂CH₂NH₂. Also described is a compound of Formula (IIa) wherein R¹² is H and R¹¹ is -CH₂CH₂CH₂CH₂NH₂. Also described is a compound of Formula (IIa) wherein R¹² is H and R¹¹ is -(C₁-C₆)alkyl-CN. Also described is a compound of Formula (IIa) wherein R¹² is H and R¹¹ is -CH₂CN. Also described is a compound of Formula (IIa) wherein R¹² is H and R¹¹ is -(C₁-C₆)alkyl-C(O)NR²⁵R²⁶. Also described is a compound of Formula (IIa) wherein R¹² is H and R¹¹ is -CH₂C(O)NH₂. Also described is a compound of Formula (IIa) wherein R¹² is H and R¹¹ is -CH₂CH₂C(O)NH₂. Also described is a compound of Formula (IIa) wherein R¹² is H and R¹¹ is -(C₁-C₆)alkyl-heteroaryl. Also described is a compound of Formula (IIa) wherein R¹² is H and R¹¹ is H.

Also described is a compound of Formula (IIa) wherein R¹¹ and R¹⁸ are combined to form an optionally substituted heterocycloalkyl ring and R¹² is H.

Also described is a compound of Formula (IIa) wherein R¹⁴ is H.

Also described is a compound of Formula (IIa) wherein R¹⁴ is H and R¹³ is -(C₁-C₆)alkyl. Also described is a compound of Formula (IIa) wherein R¹⁴ is H and R¹³ is -CH₃. Also described is a compound of Formula (IIa) wherein R¹⁴ is H and R¹³ is -(C₁-C₆)alkyl-OR²³. Also described is a compound of Formula (IIa) wherein R¹⁴ is H and R¹³ is -CH₂OH. Also described is a compound of Formula (IIa) wherein R¹⁴ is H and R¹³ is -CH₂CH₂OH. Also described is a compound of Formula (IIa) wherein R¹⁴ is H and R¹³ is -(C₁-C₆)alkyl. Also described is a compound of Formula (IIa) wherein R¹⁴ is H and R¹³ is -(C₁-C₆)alkyl-NR²¹R²². Also described is a compound of Formula (IIa) wherein R¹⁴ is H and R¹³ is -(C₁-C₆)alkyl-NH₂. Also described is a compound of Formula (IIa) wherein R¹⁴ is H and R¹³ is -CH₂NH₂. Also described is a compound of Formula (IIa) wherein R¹⁴ is H and R¹³ is -CH₂CH₂NH₂. Also described is a compound of Formula (IIa) wherein R¹⁴ is H and R¹³ is -CH₂CH₂CH₂NH₂. Also described is a compound of Formula (IIa) wherein R¹⁴ is H and R¹³ is -CH₂CH₂CH₂CH₂NH₂. Also described is a compound of Formula (IIa) wherein R¹⁴ is H and R¹³ is -(C₁-C₆)alkyl-CN. Also described is a compound of Formula (IIa) wherein R¹⁴ is H and R¹³ is -CH₂CN. Also described is a compound of Formula (IIa) wherein R¹⁴ is H and R¹³ is -(C₁-C₆)alkyl-C(O)NR²⁵R²⁶. Also described is a compound of Formula (IIa) wherein R¹⁴ is H and R¹³ is -CH₂C(O)NH₂. Also described is a compound of Formula (IIa) wherein R¹⁴ is H and R¹³ is -CH₂CH₂C(O)NH₂. Also described is a compound of Formula (IIa) wherein R¹⁴ is H and R¹³ is -(C₁-C₆)alkyl-heteroaryl. Also described is a compound of Formula (IIa) wherein R¹⁴ is H and R¹³ is H.

Also described is a compound of Formula (IIa) wherein R¹³ and R¹⁹ are combined to form an optionally substituted heterocycloalkyl ring and R¹⁴ is H.

Also described is a compound of Formula (IIa) wherein p is 1 and R²⁷ is halogen. Also described is a compound of Formula (IIa) wherein p is 1 and R²⁷ is optionally substituted -(C₁-C₆)alkyl. Also described is a compound of Formula (IIa) wherein q is 0, p is 1 and R²⁷ is halogen. Also described is a compound of Formula (IIa) wherein q is 0, p is 1 and R²⁷ is optionally substituted -(C₁-C₆)alkyl. Also described is a compound of Formula (IIa) wherein q is 1 and R²⁸ is halogen. Also described is a compound of Formula (IIa) wherein q is 1 and R²⁸ is optionally substituted -(C₁-C₆)alkyl. Also described is a compound of Formula (IIa) wherein p is 0, q is 1 and R²⁸ is halogen. Also described is a compound of Formula (IIa) wherein p is 0, q is 1 and R²⁸ is optionally substituted -(C₁-C₆)alkyl.

Also described is a compound of Formula (IIa) wherein p is 0, and q is 0.

Also described is a compound of Formula (IIa) wherein R¹ and R² are each independently H, or -(C₁-C₆)alkyl-NR²¹R²². Also described is a compound of Formula (IIa) wherein R¹ and R² are each H. Also described is a compound of Formula (IIa) wherein R¹ and R² are each independently -(C₁-C₆)alkyl-NR²¹R²². Also described is a compound of Formula (IIa) wherein R¹ is H, and R² is -(C₁-C₆)alkyl-NR²¹R²². Also described is a compound of Formula (IIa) wherein R¹ is -(C₁-C₆)alkyl-NR²¹R²², and R² is H. Also described is a compound of Formula (IIa) wherein R¹ is H, and R² is -CH₂CH₂NH₂. Also described is a compound of Formula (IIa) wherein R¹ is -CH₂CH₂NH₂, and R² is H. Also described is a compound of Formula (IIa) wherein R¹ and R² are each -CH₂CH₂NH₂.

Also described is a compound of Formula (IIa) wherein X is optionally substituted aryl. Also described is a compound of Formula (IIa) wherein X is optionally substituted phenyl. Also described is a compound of Formula (IIa) wherein X is optionally substituted heteroaryl. Also described is a compound of Formula (IIa) wherein X is optionally substituted pyridine or optionally substituted pyrimidine. Also described is a compound of Formula (IIa) wherein X is optionally substituted -(C₁-C₆)alkyl-. Also described is a compound of Formula (IIa) wherein Y is optionally substituted aryl. Also described is a compound of Formula (IIa) wherein Y is optionally substituted phenyl. Also described is a compound of Formula (IIa) wherein Y is optionally substituted heteroaryl. Also described is a compound of Formula (IIa) wherein Y is optionally substituted -(C₁-C₆)alkyl-. Also described is a compound of Formula (IIa) wherein Y is -O-(C₁-C₆)alkyl-. Also described is a compound of Formula (IIa) wherein Y is -N(H)-(C₁-C₆)alkyl-. Also described is a compound of Formula (IIa) wherein Y is a bond. Also described is a compound of Formula (IIa) wherein Z is -(C₁-C₆)alkyl. Also described is a compound of Formula (IIa) wherein Z is optionally substituted aryl. Also described is a compound of Formula (IIa) wherein Z is optionally substituted phenyl. Also described is a compound of Formula (IIa) wherein Z is optionally substituted heteroaryl. Also described is a compound of Formula (IIa) wherein Z is optionally substituted -(C₃-C₇)cycloalkyl. Also described is a compound of Formula (IIa) wherein Z is halogen.

Also described is a compound of Formula (IIa) wherein -X-Y-Z is

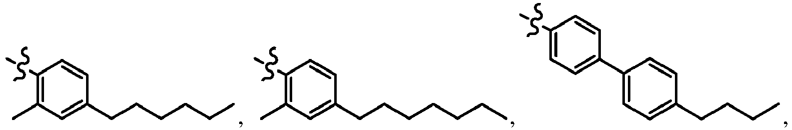

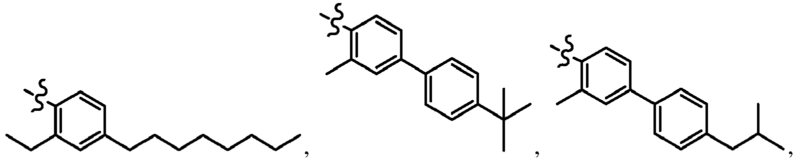

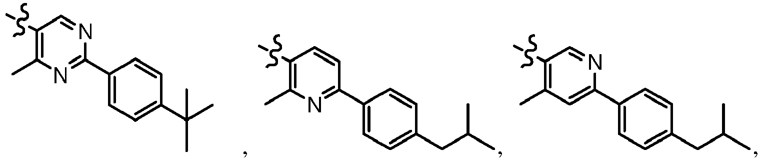

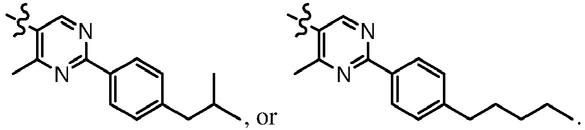

Also described herein is a compound of Formula (II) having the structure of Formula (IIb): 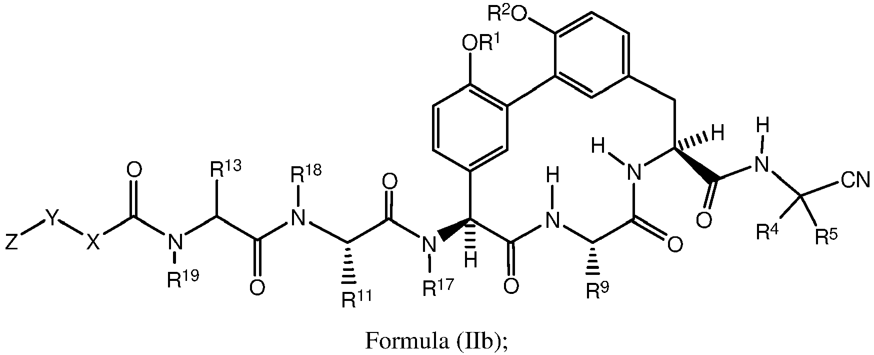 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, -(C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²², or -(C₁-C₆)alkyl-C(O)N(R²³)(C₁-C₆)alkyl, or optionally substituted heterocycloalkyl;
R⁴ is H, -(C₁-C₆)alkyl, -(C₁-C₆)alkyl-OH, -(C₃-C₆)cycloalkyl , or -C(O)NH₂;
R⁵ is H, or -(C₁-C₆)alkyl; or R⁴ and R⁵ and the carbon atom to which they are attached form a cyclopropyl ring;
R⁹ is H, -(C₁-C₆)alkyl, -(C₁-C₆)haloalkyl, or -(C₃-C₆)cycloalkyl;
R¹¹ is H, -NH₂, -(C₁-C₆)alkyl, -(C₁-C₆)alkyl-OR²³, -(C₁-C₆)alkyl-SR²³, -(C₁-C₆)alkyl-C(O)OR²³, - (C₁-C₆)alkyl-NR²¹R²², -(C₁-C₆)alkyl-CN, -(C₁-C₆)alkyl-C(O)NR²⁵R²⁶, -(C₁-C₆)heteroalkyl-CO₂H, -(C₁-C₆)alkyl-S(O)-(C₁-C₆)alkyl, -(C₁-C₆)alkyl-N(H)CH=NH, -(C₁-C₆)alkyl-N(H)C(NH)NH₂, - (C₁-C₆)alkyl-heterocycloalkyl, optionally substituted -(C₁-C₆)alkyl-N(H)heterocycloalkyl, or - (C₁-C₆)alkyl-heteroaryl; or R¹¹ and R¹⁸ are combined to form an optionally substituted heterocycloalkyl ring;
R¹³ is H, -NH₂, -(C₁-C₆)alkyl, -(C₁-C₆)alkyl-OR²³, -(C₁-C₆)alkyl-SR²³, -(C₁-C₆)alkyl-C(O)OR²³, - (C₁-C₆)alkyl-NR²¹R²², -(C₁-C₆)alkyl-CN, -(C₁-C₆)alkyl-C(O)NR²⁵R²⁶, -(C₁-C₆)alkyl-N(H)C(NH)NH₂, -(C₁-C₆)alkyl-heterocycloalkyl, or -(C₁-C₆)alkyl-heteroaryl; or R¹³ and R¹⁹ are combined to form an optionally substituted heterocycloalkyl ring;
R¹⁷, R¹⁸, and R¹⁹ are each independently H, -(C₁-C₆)alkyl, -(C₃-C₆)cycloalkyl, -(C₁-C₆)alkyl-OR²³, -(C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, - N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, - SO₂(C₁-C₆)alkyl-, -C(O)NH(C₁-C₆)alkyl-, -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-,optionally substituted aryl, or optionally substituted heteroaryl;
Z is H, halogen, -NH₂, -CN, -CF₃, -(C₁-C₁₂)alkyl,-(C₂-C₁₂)alkenyl, -(C₂-C₁₂)alkynyl, - C(O)NR²⁵R²⁶, -O-(C₁-C₁₂)alkyl, -N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, - C(O)(C₁-C₆)alkyl, -C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl; and
each R²⁵ and R²⁶ is independently H or optionally substituted -(C₁-C₆)alkyl; or R²⁵ and R²⁶ and the nitrogen atom to which they are attached form a heterocycloalkyl ring;
or a pharmaceutically acceptable salt, solvate, or prodrug thereof.

Also described is a compound of Formula (IIb) wherein R¹⁷ is -(C₁-C₆)alkyl. Also described is a compound of Formula (IIb) wherein R¹⁷ is -CH₃. Also described is a compound of Formula (IIb) wherein R¹⁷ is -CH₂CH₃. Also described is a compound of Formula (IIb) wherein R¹⁷ is -(C₃-C₆)cycloalkyl. Also described is a compound of Formula (IIb) wherein R¹⁷ is cyclopropyl. Also described is a compound of Formula (IIb) wherein R¹⁷ is -(C₁-C₆)alkyl-C(O)OR²³. Also described is a compound of Formula (IIb) wherein R¹⁷ is -CH₂CH₂OH. Also described is a compound of Formula (IIb) wherein R¹⁷ is -(C₁-C₆)alkyl-NR²¹R²². Also described is a compound of Formula (IIb) wherein R¹⁷ is -CH₂CH₂NH₂. Also described is a compound of Formula (IIb) wherein R¹⁷ is H.

Also described is a compound of Formula (IIb) wherein R¹⁸ is H.

Also described is a compound of Formula (IIb) wherein R¹⁹ is H.

Also described is a compound of Formula (IIb) wherein R⁵ is H.

Also described is a compound of Formula (IIb) wherein R⁴ is H. Also described is a compound of Formula (IIb) wherein R⁴ is -(C₁-C₆)alkyl. Also described is a compound of Formula (IIb) wherein R⁴ is -CH₃. Also described is a compound of Formula (IIb) wherein R⁴ is - CH₂CH₃. Also described is a compound of Formula (IIb) wherein R⁴ is -(C₁-C₆)alkyl-OH. Also described is a compound of Formula (IIb) wherein R⁴ is -CH₂OH. Also described is a compound of Formula (IIb) wherein R⁴ is -(C₃-C₆)cycloalkyl. Also described is a compound of Formula (IIb) wherein R⁴ is cyclopropyl. Also described is a compound of Formula (IIb) wherein R⁴ is - C(O)NH₂.

Also described is a compound of Formula (IIb) wherein R⁴ and R⁵ are H.

Also described is a compound of Formula (IIb) wherein R⁴ and R⁵ and the carbon atom to which they are attached form a cyclopropyl ring.

Also described is a compound of Formula (IIb) wherein R⁹ is -(C₁-C₆)alkyl. Also described is a compound of Formula (IIb) wherein R⁹ is -CH₃. Also described is a compound of Formula (IIb) wherein R⁹ is -CH₂CH₃. Also described is a compound of Formula (IIb) wherein R⁹ is -(C₁-C₆)haloalkyl. Also described is a compound of Formula (IIb) wherein R⁹ is -CH₂F. Also described is a compound of Formula (IIb) wherein R⁹ is -CHF₂. Also described is a compound of Formula (IIb) wherein R⁹ is -(C₃-C₆)cycloalkyl. Also described is a compound of Formula (IIb) wherein R⁹ is cyclopropyl. Also described is a compound of Formula (IIb) wherein R⁹ is H.

Also described is a compound of Formula (IIb) wherein R¹¹ is -(C₁-C₆)alkyl. Also described is a compound of Formula (IIb) wherein R¹¹ is -CH₃. Also described is a compound of Formula (IIb) wherein R¹¹ is -(C₁-C₆)alkyl-OR²³. Also described is a compound of Formula (IIb) wherein R¹¹ is -CH₂OH. Also described is a compound of Formula (IIb) wherein R¹¹ is - CH₂CH₂OH. Also described is a compound of Formula (IIb) wherein R¹¹ is -(C₁-C₆)alkyl. Also described is a compound of Formula (IIb) wherein R¹¹ is -(C₁-C₆)alkyl-NR²¹R²². Also described is a compound of Formula (IIb) wherein R¹¹ is -(C₁-C₆)alkyl-NH₂. Also described is a compound of Formula (IIb) wherein R¹¹ is -CH₂NH₂. Also described is a compound of Formula (IIb) wherein R¹¹ is -CH₂CH₂NH₂. Also described is a compound of Formula (IIb) wherein R¹¹ is - CH₂CH₂CH₂NH₂. Also described is a compound of Formula (IIb) wherein R¹¹ is - CH₂CH₂CH₂CH₂NH₂. Also described is a compound of Formula (IIb) wherein R¹¹ is -(C₁-C₆)alkyl-CN. Also described is a compound of Formula (IIb) wherein R¹¹ is -CH₂CN. Also described is a compound of Formula (IIb) wherein R¹¹ is -(C₁-C₆)alkyl-C(O)NR²⁵R²⁶. Also described is a compound of Formula (IIb) wherein R¹¹ is -CH₂C(O)NH₂. Also described is a compound of Formula (IIb) wherein R¹¹ is -CH₂CH₂C(O)NH₂. Also described is a compound of Formula (IIb) wherein R¹¹ is -(C₁-C₆)alkyl-heteroaryl. Also described is a compound of Formula (IIb) wherein R¹¹ is H.

Also described is a compound of Formula (IIb) wherein R¹¹ and R¹⁸ are combined to form an optionally substituted heterocycloalkyl ring and R¹² is H.

Also described is a compound of Formula (IIb) wherein R¹³ is -(C₁-C₆)alkyl. Also described is a compound of Formula (IIb) wherein R¹³ is -CH₃. Also described is a compound of Formula (IIb) wherein R¹³ is -(C₁-C₆)alkyl-OR²³. Also described is a compound of Formula (IIb) wherein R¹³ is -CH₂OH. Also described is a compound of Formula (IIb) wherein R¹³ is - CH₂CH₂OH. Also described is a compound of Formula (IIb) wherein R¹³ is -(C₁-C₆)alkyl. Also described is a compound of Formula (IIb) wherein R¹³ is -(C₁-C₆)alkyl-NR²¹R²², Also described is a compound of Formula (IIb) wherein R¹³ is -(C₁-C₆)alkyl-NH₂. Also described is a compound of Formula (IIb) wherein R¹³ is -CH₂NH₂. Also described is a compound of Formula (IIb) wherein R¹³ is -CH₂CH₂NH₂. Also described is a compound of Formula (IIb) wherein R¹³ is - CH₂CH₂CH₂NH₂. Also described is a compound of Formula (IIb) wherein R¹³ is - CH₂CH₂CH₂CH₂NH₂. Also described is a compound of Formula (IIb) wherein R¹³ is -(C₁-C₆)alkyl-CN. Also described is a compound of Formula (IIb) wherein R¹³ is -CH₂CN. Also described is a compound of Formula (IIb) wherein R¹³ is -(C₁-C₆)alkyl-C(O)NR²⁵R²⁶. Also described is a compound of Formula (IIb) wherein R¹³ is -CH₂C(O)NH₂. Also described is a compound of Formula (IIb) wherein R¹³ is -CH₂CH₂C(O)NH₂. Also described is a compound of Formula (IIb) wherein R¹³ is -(C₁-C₆)alkyl-heteroaryl. Also described is a compound of Formula (IIb) wherein R¹³ is H.

Also described is a compound of Formula (IIb) wherein R¹³ and R¹⁹ are combined to form an optionally substituted heterocycloalkyl ring and R¹⁴ is H.

Also described is a compound of Formula (IIb) wherein R¹ and R² are each independently H, or -(C₁-C₆)alkyl-NR²¹R²², Also described is a compound of Formula (IIb) wherein R¹ and R² are each H. Also described is a compound of Formula (IIb) wherein R¹ and R² are each independently -(C₁-C₆)alkyl-NR²¹R²². Also described is a compound of Formula (IIb) wherein R¹ is H, and R² is -(C₁-C₆)alkyl-NR²¹R²², Also described is a compound of Formula (IIb) wherein R¹ is -(C₁-C₆)alkyl-NR²¹R²², and R² is H. Also described is a compound of Formula (IIb) wherein R¹ is H, and R² is -CH₂CH₂NH₂. Also described is a compound of Formula (IIb) wherein R¹ is -CH₂CH₂NH₂, and R² is H. Also described is a compound of Formula (IIb) wherein R¹ and R² are each -CH₂CH₂NH₂.

Also described is a compound of Formula (IIb) wherein X is optionally substituted aryl. Also described is a compound of Formula (IIb) wherein X is optionally substituted phenyl. Also described is a compound of Formula (IIb) wherein X is optionally substituted heteroaryl. Also described is a compound of Formula (IIb) wherein X is optionally substituted pyridine or optionally substituted pyrimidine. Also described is a compound of Formula (IIb) wherein X is optionally substituted -(C₁-C₆)alkyl-. Also described is a compound of Formula (IIb) wherein Y is optionally substituted aryl. Also described is a compound of Formula (IIb) wherein Y is optionally substituted phenyl. Also described is a compound of Formula (IIb) wherein Y is optionally substituted heteroaryl. Also described is a compound of Formula (IIb) wherein Y is optionally substituted -(C₁-C₆)alkyl-. Also described is a compound of Formula (IIb) wherein Y is -O-(C₁-C₆)alkyl-. Also described is a compound of Formula (IIb) wherein Y is -N(H)-(C₁-C₆)alkyl-. Also described is a compound of Formula (IIb) wherein Y is a bond. Also described is a compound of Formula (IIb) wherein Z is -(C₁-C₆)alkyl. Also described is a compound of Formula (IIb) wherein Z is optionally substituted aryl. Also described is a compound of Formula (IIb) wherein Z is optionally substituted phenyl. Also described is a compound of Formula (IIb) wherein Z is optionally substituted heteroaryl. Also described is a compound of Formula (IIb) wherein Z is optionally substituted -(C₃-C₇)cycloalkyl. Also described is a compound of Formula (IIb) wherein Z is halogen.

Also described is a compound of Formula (IIb) wherein -X-Y-Z is

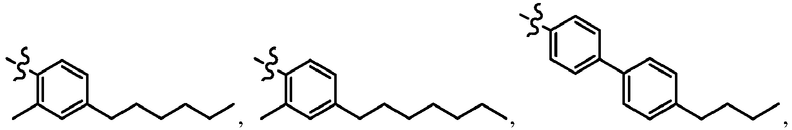

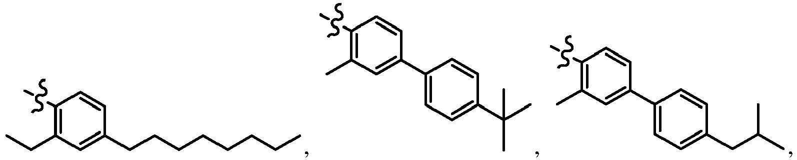

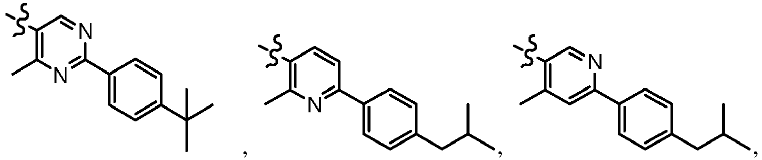

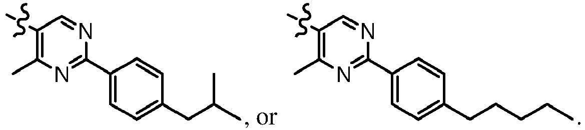

Also described herein is a compound of Formula (II) having the structure of Formula (IIc): 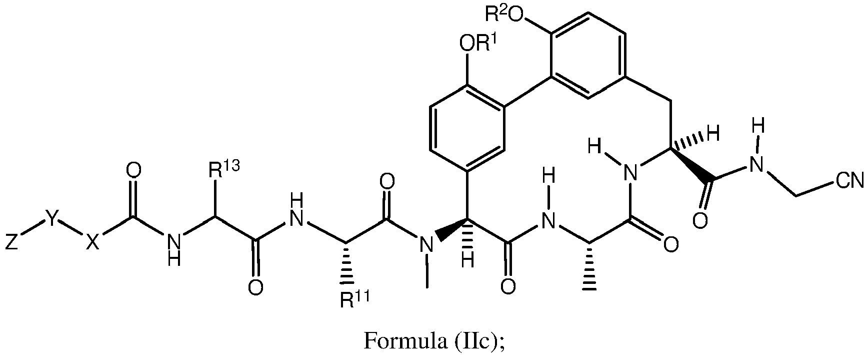 wherein:
R¹ and R² are each independently H or -CH₂CH₂NH₂;
R¹¹ is H, -NH₂, -(C₁-C₆)alkyl, -(C₁-C₆)alkyl-OR²³, -(C₁-C₆)alkyl-SR²³, -(C₁-C₆)alkyl-C(O)OR²³, - (C₁-C₆)alkyl-NR²¹R²², -(C₁-C₆)alkyl-CN, -(C₁-C₆)alkyl-C(O)NR²⁵R²⁶, -(C₁-C₆)heteroalkyl-CO₂H, -(C₁-C₆)alkyl-S(O)-(C₁-C₆)alkyl, -(C₁-C₆)alkyl-N(H)CH=NH, -(C₁-C₆)alkyl-N(H)C(NH)NH₂, - (C₁-C₆)alkyl-heterocycloalkyl, optionally substituted -(C₁-C₆)alkyl-N(H)heterocycloalkyl, or - (C₁-C₆)alkyl-heteroaryl;
R¹³ is H, -NH₂, -(C₁-C₆)alkyl, -(C₁-C₆)alkyl-OR²³, -(C₁-C₆)alkyl-SR²³, -(C₁-C₆)alkyl-C(O)OR²³, - (C₁-C₆)alkyl-NR²¹R²², -(C₁-C₆)alkyl-CN, -(C₁-C₆)alkyl-C(O)NR²⁵R²⁶, -(C₁-C₆)alkyl-N(H)C(NH)NH₂, -(C₁-C₆)alkyl-heterocycloalkyl, or -(C₁-C₆)alkyl-heteroaryl;
X is optionally substituted -(C₁-C₆)alkyl-, -(C₂-C₆)alkenyl-, -(C₂-C₆)alkynyl, -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, - N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, - SO₂(C₁-C₆)alkyl-, -C(O)NH(C₁-C₆)alkyl-, -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-,optionally substituted aryl, or optionally substituted heteroaryl;
Z is H, halogen, -NH₂, -CN, -CF₃, -(C₁-C₁₂)alkyl,-(C₂-C₁₂)alkenyl, -(C₂-C₁₂)alkynyl, - C(O)NR²⁵R²⁶, -O-(C₁-C₁₂)alkyl, -N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, - C(O)(C₁-C₆)alkyl, -C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl; and
each R²⁵ and R²⁶ is independently H or optionally substituted -(C₁-C₆)alkyl; or R²⁵ and R²⁶ and the nitrogen atom to which they are attached form a heterocycloalkyl ring;
or a pharmaceutically acceptable salt, solvate, or prodrug thereof.

Also described is a compound of Formula (IIc) wherein R¹¹ is -(C₁-C₆)alkyl. Also described is a compound of Formula (IIc) wherein R¹¹ is -CH₃. Also described is a compound of Formula (IIc) wherein R¹¹ is -(C₁-C₆)alkyl-OR²³. Also described is a compound of Formula (IIc) wherein R¹¹ is -CH₂OH. Also described is a compound of Formula (IIc) wherein R¹¹ is - CH₂CH₂OH. Also described is a compound of Formula (IIc) wherein R¹¹ is -(C₁-C₆)alkyl. Also described is a compound of Formula (IIc) wherein R¹¹ is -(C₁-C₆)alkyl-NR²¹R²². Also described is a compound of Formula (IIc) wherein R¹¹ is -(C₁-C₆)alkyl-NH₂. Also described is a compound of Formula (IIc) wherein R¹¹ is -CH₂NH₂. Also described is a compound of Formula (IIc) wherein R¹¹ is -CH₂CH₂NH₂. Also described is a compound of Formula (IIc) wherein R¹¹ is - CH₂CH₂CH₂NH₂. Also described is a compound of Formula (IIc) wherein R¹¹ is - CH₂CH₂CH₂CH₂NH₂. Also described is a compound of Formula (IIc) wherein R¹¹ is -(C₁-C₆)alkyl-CN. Also described is a compound of Formula (IIc) wherein R¹¹ is -CH₂CN. Also described is a compound of Formula (IIc) wherein R¹¹ is -(C₁-C₆)alkyl-C(O)NR²⁵R²⁶. Also described is a compound of Formula (IIc) wherein R¹¹ is -CH₂C(O)NH₂. Also described is a compound of Formula (IIc) wherein R¹¹ is -CH₂CH₂C(O)NH₂. Also described is a compound of Formula (IIc) wherein R¹¹ is -(C₁-C₆)alkyl-heteroaryl. Also described is a compound of Formula (IIc) wherein R¹¹ is H.

Also described is a compound of Formula (IIc) wherein R¹³ is -(C₁-C₆)alkyl. Also described is a compound of Formula (IIc) wherein R¹³ is -CH₃. Also described is a compound of Formula (IIc) wherein R¹³ is -(C₁-C₆)alkyl-OR²³. Also described is a compound of Formula (IIc) wherein R¹³ is -CH₂OH. Also described is a compound of Formula (IIc) wherein R¹³ is - CH₂CH₂OH. Also described is a compound of Formula (IIc) wherein R¹³ is -(C₁-C₆)alkyl. Also described is a compound of Formula (IIc) wherein R¹³ is -(C₁-C₆)alkyl-NR²¹R²². Also described is a compound of Formula (IIc) wherein R¹³ is -(C₁-C₆)alkyl-NH₂. Also described is a compound of Formula (IIc) wherein R¹³ is -CH₂NH₂. Also described is a compound of Formula (IIc) wherein R¹³ is -CH₂CH₂NH₂. Also described is a compound of Formula (IIc) wherein R¹³ is - CH₂CH₂CH₂NH₂. Also described is a compound of Formula (IIc) wherein R¹³ is - CH₂CH₂CH₂CH₂NH₂. Also described is a compound of Formula (IIc) wherein R¹³ is -(C₁-C₆)alkyl-CN. Also described is a compound of Formula (IIc) wherein R¹³ is -CH₂CN. Also described is a compound of Formula (IIc) wherein R¹³ is -(C₁-C₆)alkyl-C(O)NR²⁵R²⁶. Also described is a compound of Formula (IIc) wherein R¹³ is -CH₂C(O)NH₂. Also described is a compound of Formula (IIc) wherein R¹³ is -CH₂CH₂C(O)NH₂. Also described is a compound of Formula (IIc) wherein R¹³ is -(C₁-C₆)alkyl-heteroaryl. Also described is a compound of Formula (IIc) wherein R¹³ is H.

Also described is a compound of Formula (IIc) wherein R¹ and R² are each H. Also described is a compound of Formula (IIc) wherein R¹ is H, and R² is -CH₂CH₂NH₂. Also described is a compound of Formula (IIc) wherein R¹ is -CH₂CH₂NH₂, and R² is H. Also described is a compound of Formula (IIc) wherein R¹ and R² are each -CH₂CH₂NH₂.

Also described is a compound of Formula (IIc) wherein X is optionally substituted aryl. Also described is a compound of Formula (IIc) wherein X is optionally substituted phenyl. Also described is a compound of Formula (IIc) wherein X is optionally substituted heteroaryl. Also described is a compound of Formula (IIc) wherein X is optionally substituted pyridine or optionally substituted pyrimidine. Also described is a compound of Formula (IIc) wherein X is optionally substituted -(C₁-C₆)alkyl-. Also described is a compound of Formula (IIc) wherein Y is optionally substituted aryl. Also described is a compound of Formula (IIc) wherein Y is optionally substituted phenyl. Also described is a compound of Formula (IIc) wherein Y is optionally substituted heteroaryl. Also described is a compound of Formula (IIc) wherein Y is optionally substituted -(C₁-C₆)alkyl-. Also described is a compound of Formula (IIc) wherein Y is -O-(C₁-C₆)alkyl-. Also described is a compound of Formula (IIc) wherein Y is -N(H)-(C₁-C₆)alkyl-. Also described is a compound of Formula (IIc) wherein Y is a bond. Also described is a compound of Formula (IIc) wherein Z is -(C₁-C₆)alkyl. Also described is a compound of Formula (IIc) wherein Z is optionally substituted aryl. Also described is a compound of Formula (IIc) wherein Z is optionally substituted phenyl. Also described is a compound of Formula (IIc) wherein Z is optionally substituted heteroaryl. Also described is a compound of Formula (IIc) wherein Z is optionally substituted -(C₃-C₇)cycloalkyl. Also described is a compound of Formula (IIc) wherein Z is halogen.

Also described is a compound of Formula (IIc) wherein -X-Y-Z is

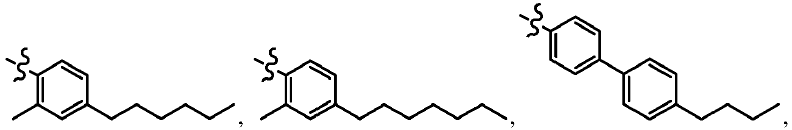

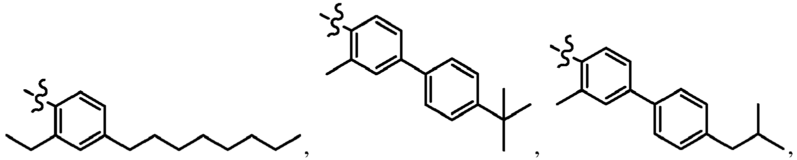

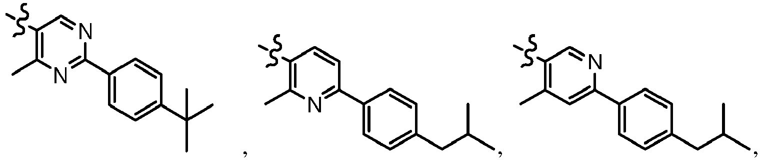

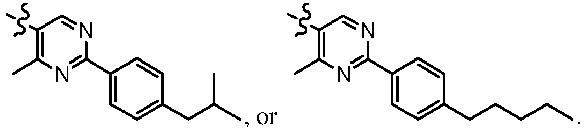

Also described herein are compounds of Formula (II) having the structure of Formula (IId): 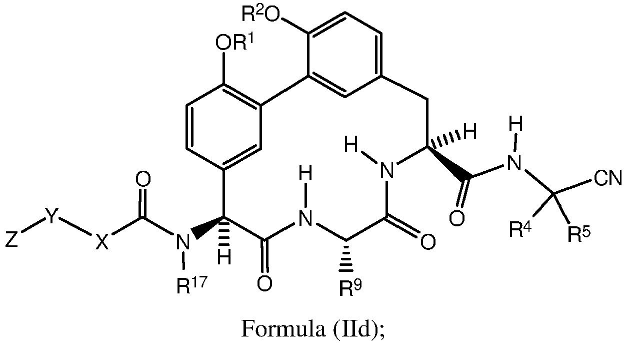 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, -(C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²², or -(C₁-C₆)alkyl-C(O)N(R²³)(C₁-C₆)alkyl, or optionally substituted heterocycloalkyl;
R⁴ is H, -(C₁-C₆)alkyl, -(C₁-C₆)alkyl-OH, -(C₃-C₆)cycloalkyl , or -C(O)NH₂;
R⁵ is H, or -(C₁-C₆)alkyl; or R⁴ and R⁵ and the carbon atom to which they are attached form a cyclopropyl ring;
R⁹ is H, -(C₁-C₆)alkyl, -(C₁-C₆)haloalkyl, or -(C₃-C₆)cycloalkyl;
R¹⁷ is H, -(C₁-C₆)alkyl, -(C₃-C₆)cycloalkyl, -(C₁-C₆)alkyl-OR²³, -(C₁-C₆)alkyl-C(O)OR²³, or - (C₁-C₆)alkyl-NR²¹R²²;
X is optionally substituted -(C₁-C₆)alkyl-, -(C₂-C₆)alkenyl-, -(C₂-C₆)alkynyl, -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, - N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, - SO₂(C₁-C₆)alkyl-, -C(O)NH(C₁-C₆)alkyl-, -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-,optionally substituted aryl, or optionally substituted heteroaryl;
Z is H, halogen, -NH₂, -CN, -CF₃, -(C₁-C₁₂)alkyl,-(C₂-C₁₂)alkenyl, -(C₂-C₁₂)alkynyl, - C(O)NR²⁵R²⁶, -O-(C₁-C₁₂)alkyl, -N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, - C(O)(C₁-C₆)alkyl, -C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl; and
each R²⁵ and R²⁶ is independently H or optionally substituted -(C₁-C₆)alkyl; or R²⁵ and R²⁶ and the nitrogen atom to which they are attached form a heterocycloalkyl ring;
or a pharmaceutically acceptable salt, solvate, or prodrug thereof.

Also described is a compound of Formula (IId) wherein R¹⁷ is -(C₁-C₆)alkyl. Also described is a compound of Formula (IId) wherein R¹⁷ is -CH₃. Also described is a compound of Formula (IId) wherein R¹⁷ is -CH₂CH₃. Also described is a compound of Formula (IId) wherein R¹⁷ is -(C₃-C₆)cycloalkyl. Also described is a compound of Formula (IId) wherein R¹⁷ is cyclopropyl. Also described is a compound of Formula (IId) wherein R¹⁷ is -(C₁-C₆)alkyl-C(O)OR²³. Also described is a compound of Formula (IId) wherein R¹⁷ is -CH₂CH₂OH. Also described is a compound of Formula (IId) wherein R¹⁷ is -(C₁-C₆)alkyl-NR²¹R²². Also described is a compound of Formula (IId) wherein R¹⁷ is -CH₂CH₂NH₂. Also described is a compound of Formula (IId) wherein R¹⁷ is H.

Also described is a compound of Formula (IId) wherein R⁵ is H.

Also described is a compound of Formula (IId) wherein R⁴ is H. Also described is a compound of Formula (IId) wherein R⁴ is -(C₁-C₆)alkyl. Also described is a compound of Formula (IId) wherein R⁴ is -CH₃. Also described is a compound of Formula (IId) wherein R⁴ is - CH₂CH₃. Also described is a compound of Formula (IId) wherein R⁴ is -(C₁-C₆)alkyl-OH. Also described is a compound of Formula (IId) wherein R⁴ is -CH₂OH. Also described is a compound of Formula (IId) wherein R⁴ is -(C₃-C₆)cycloalkyl. Also described is a compound of Formula (IId) wherein R⁴ is cyclopropyl. Also described is a compound of Formula (IId) wherein R⁴ is - C(O)NH₂.

Also described is a compound of Formula (IId) wherein R⁴ and R⁵ are H.

Also described is a compound of Formula (IId) wherein R⁴ and R⁵ and the carbon atom to which they are attached form a cyclopropyl ring.

Also described is a compound of Formula (IId) wherein R⁹ is -(C₁-C₆)alkyl. Also described is a compound of Formula (IId) wherein R⁹ is -CH₃. Also described is a compound of Formula (IId) wherein R⁹ is -CH₂CH₃. Also described is a compound of Formula (IId) wherein R⁹ is -(C₁-C₆)haloalkyl. Also described is a compound of Formula (IId) wherein R⁹ is -CH₂F. Also described is a compound of Formula (IId) wherein R⁹ is -CHF₂. Also described is a compound of Formula (IId) wherein R⁹ is -(C₃-C₆)cycloalkyl. Also described is a compound of Formula (IId) wherein R⁹ is cyclopropyl. Also described is a compound of Formula (IId) wherein R⁹ is H.

Also described is a compound of Formula (IId) wherein R¹ and R² are each independently H, or -(C₁-C₆)alkyl-NR²¹R²², Also described is a compound of Formula (IId) wherein R¹ and R² are each H. Also described is a compound of Formula (IId) wherein R¹ and R² are each independently -(C₁-C₆)alkyl-NR²¹R²². Also described is a compound of Formula (IId) wherein R¹ is H, and R² is -(C₁-C₆)alkyl-NR²¹R²², Also described is a compound of Formula (IId) wherein R¹ is -(C₁-C₆)alkyl-NR²¹R²², and R² is H. Also described is a compound of Formula (IId) wherein R¹ is H, and R² is -CH₂CH₂NH₂. Also described is a compound of Formula (IId) wherein R¹ is -CH₂CH₂NH₂, and R² is H. Also described is a compound of Formula (IId) wherein R¹ and R² are each -CH₂CH₂NH₂.

Also described is a compound of Formula (IId) wherein X is optionally substituted aryl. Also described is a compound of Formula (IId) wherein X is optionally substituted phenyl. Also described is a compound of Formula (IId) wherein X is optionally substituted heteroaryl. Also described is a compound of Formula (IId) wherein X is optionally substituted pyridine or optionally substituted pyrimidine. Also described is a compound of Formula (IId) wherein X is optionally substituted -(C₁-C₆)alkyl-. Also described is a compound of Formula (IId) wherein Y is optionally substituted aryl. Also described is a compound of Formula (IId) wherein Y is optionally substituted phenyl. Also described is a compound of Formula (IId) wherein Y is optionally substituted heteroaryl. Also described is a compound of Formula (IId) wherein Y is optionally substituted -(C₁-C₆)alkyl-. Also described is a compound of Formula (IId) wherein Y is -O-(C₁-C₆)alkyl-. Also described is a compound of Formula (IId) wherein Y is -N(H)-(C₁-C₆)alkyl-. Also described is a compound of Formula (IId) wherein Y is a bond. Also described is a compound of Formula (IId) wherein Z is -(C₁-C₆)alkyl. Also described is a compound of Formula (IId) wherein Z is optionally substituted aryl. Also described is a compound of Formula (IId) wherein Z is optionally substituted phenyl. Also described is a compound of Formula (IId) wherein Z is optionally substituted heteroaryl. Also described is a compound of Formula (IId) wherein Z is optionally substituted -(C₃-C₇)cycloalkyl. Also described is a compound of Formula (IId) wherein Z is halogen.

Also described is a compound of Formula (IId) wherein -X-Y-Z is

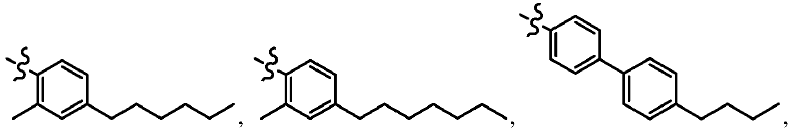

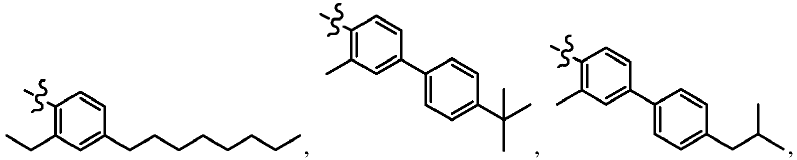

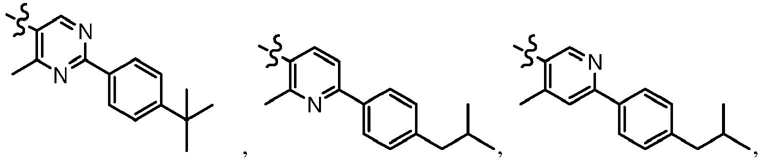

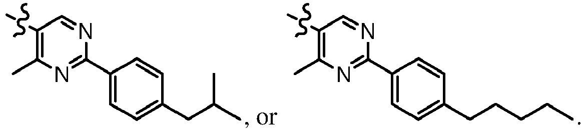

Also described herein are compounds of Formula (II) having the structure of Formula (IIe): 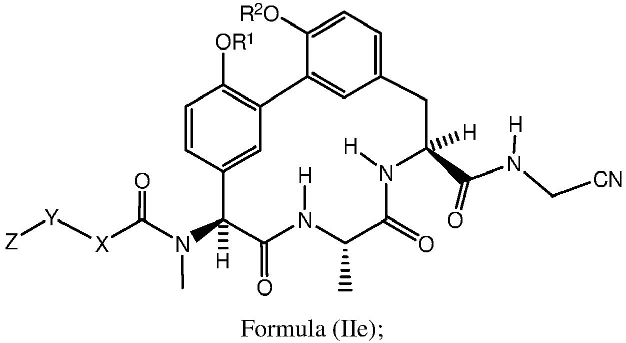 wherein:
R¹ and R² are each independently H or -CH₂CH₂NH₂;
X is optionally substituted -(C₁-C₆)alkyl-, -(C₂-C₆)alkenyl-, -(C₂-C₆)alkynyl, -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, - N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, - SO₂(C₁-C₆)alkyl-, -C(O)NH(C₁-C₆)alkyl-, -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-,optionally substituted aryl, or optionally substituted heteroaryl;
Z is H, halogen, -NH₂, -CN, -CF₃, -(C₁-C₁₂)alkyl,-(C₂-C₁₂)alkenyl, -(C₂-C₁₂)alkynyl, - C(O)NR²⁵R²⁶, -O-(C₁-C₁₂)alkyl, -N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-heterocycloalkyl, optionally substituted aryl, or optionally substituted heteroaryl;
each R²⁴ is independently H or -(C₁-C₆)alkyl; and
R²⁵ and R²⁶ is independently H or optionally substituted -(C₁-C₆)alkyl; or R²⁵ and R²⁶ and the nitrogen atom to which they are attached form a heterocycloalkyl ring;
or a pharmaceutically acceptable salt, solvate, or prodrug thereof.

Also described is a compound of Formula (IIe) wherein R¹ and R² are each H. Also described is a compound of Formula (IIe) wherein R¹ is H, and R² is -CH₂CH₂NH₂. Also described is a compound of Formula (IIe) wherein R¹ is -CH₂CH₂NH₂, and R² is H. Also described is a compound of Formula (IIe) wherein R¹ and R² are each -CH₂CH₂NH₂.

Also described is a compound of Formula (IIe) wherein X is optionally substituted aryl. Also described is a compound of Formula (IIe) wherein X is optionally substituted phenyl. Also described is a compound of Formula (IIe) wherein X is optionally substituted heteroaryl. Also described is a compound of Formula (IIe) wherein X is optionally substituted pyridine or optionally substituted pyrimidine. Also described is a compound of Formula (IIe) wherein X is optionally substituted -(C₁-C₆)alkyl-. Also described is a compound of Formula (IIe) wherein Y is optionally substituted aryl. Also described is a compound of Formula (IIe) wherein Y is optionally substituted phenyl. Also described is a compound of Formula (IIe) wherein Y is optionally substituted heteroaryl. Also described is a compound of Formula (IIe) wherein Y is optionally substituted -(C₁-C₆)alkyl-. Also described is a compound of Formula (IIe) wherein Y is -O-(C₁-C₆)alkyl-. Also described is a compound of Formula (IIe) wherein Y is -N(H)-(C₁-C₆)alkyl-. Also described is a compound of Formula (IIe) wherein Y is a bond. Also described is a compound of Formula (IIe) wherein Z is -(C₁-C₆)alkyl. Also described is a compound of Formula (IIe) wherein Z is optionally substituted aryl. Also described is a compound of Formula (IIe) wherein Z is optionally substituted phenyl. Also described is a compound of Formula (IIe) wherein Z is optionally substituted heteroaryl. Also described is a compound of Formula (IIe) wherein Z is optionally substituted -(C₃-C₇)cycloalkyl. Also described is a compound of Formula (IIe) wherein Z is halogen.

Also described is a compound of Formula (IIe) wherein -X-Y-Z is

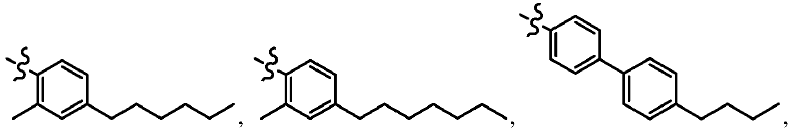

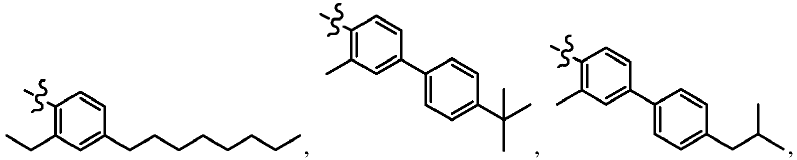

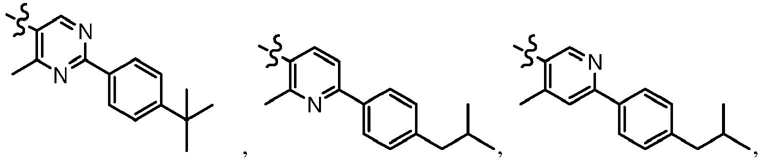

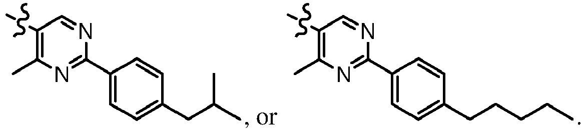

Also described herein are hydrates or metabolites comprising any of the aforementioned compounds.

Also described are pharmaceutical compositions comprising any of the aforementioned compounds together with a pharmaceutically acceptable excipient.

Also described herein is the use of a compound described herein in the manufacture of a medicament for treatment of a bacterial infection in a patient.

Also described herein are methods of treating a mammal in need of such treatment comprising administering to the mammal an antibacterial effective amount of any of the aforementioned compounds at a frequency and for a duration sufficient to provide a beneficial effect to the mammal. For example, the mammal has a bacteria-related infection that is resistant to treatment with arylomycin A2. For example, the causative bacteria species of the bacteria infection is an infection involving Pseudomonas aeruginosa, Pseudomonas fluorescens, Pseudomonas acidovorans, Pseudomonas alcaligenes, Pseudomonas putida, Stenotrophomonas maltophilia, Burkholderia cepacia, Aeromonas hydrophilia, Escherichia coli, Citrobacter freundii, Salmonella typhimurium, Salmonella typhi, Salmonella paratyphi, Salmonella enteritidis, Shigella dysenteriae, Shigella flexneri, Shigella sonnei, Enterobacter cloacae, Enterobacter aerogenes, Klebsiella pneumoniae, Klebsiella oxytoca, Serratia marcescens, Francisella tularensis, Morganella morganii, Proteus mirabilis, Proteus vulgaris, Providencia alcalifaciens, Providencia rettgeri, Providencia stuartii, Acinetobacter baumannii, Acinetobacter calcoaceticus, Acinetobacter haemolyticus, Yersinia enterocolitica, Yersinia pestis, Yersinia pseudotuberculosis, Yersinia intermedia, Bordetella pertussis, Bordetella parapertussis, Bordetella bronchiseptica, Haemophilus influenzae, Haemophilus parainfluenzae, Haemophilus haemolyticus, Haemophilus parahaemolyticus, Haemophilus ducreyi, Pasteurella multocida, Pasteurella haemolytica, Branhamella catarrhalis, Helicobacter pylori, Campylobacter fetus, Campylobacter jejuni, Campylobacter coli, Borrelia burgdorferi, Vibrio cholerae, Vibrio parahaemolyticus, Legionella pneumophila, Listeria monocytogenes, Neisseria gonorrhoeae, Neisseria meningitidis, Kingella, Moraxella, Gardnerella vaginalis, Bacteroides fragilis, Bacteroides distasonis, Bacteroides 3452A homology group, Bacteroides vulgatus, Bacteroides ovalus, Bacteroides thetaiotaomicron, Bacteroides uniformis, Bacteroides eggerthii, Bacteroides splanchnicus, Clostridium difficile, Mycobacterium tuberculosis, Mycobacterium avium, Mycobacterium intracellulare, Mycobacterium leprae, Corynebacterium diphtheriae, Corynebacterium ulcerans, Streptococcus pneumoniae, Streptococcus agalactiae, Streptococcus pyogenes, Enterococcus faecalis, Enterococcus faecium, Staphylococcus aureus, Staphylococcus epidermidis, Staphylococcus saprophyticus, Staphylococcus intermedius, Staphylococcus hyicus subsp. hyicus, Staphylococcus haemolyticus, Staphylococcus hominis, or Staphylococcus saccharolyticus. For example the bacterial infection is an infection involving a Gram-negative bacteria. For example, the bacterial infection is an infection involving a Gram-positive bacteria.

Also described herein are methods of treating a mammal in need of such treatment comprising administering to the mammal a second therapeutic agent to any of the aforementioned methods of treatment. For example, the second therapeutic agent is a not an SpsB inhibitor. For example, the second therapeutic agent is an aminoglycoside antibiotic, fluoroquinolone antibiotic, β-lactam antibiotic, macrolide antibiotic, glycopeptide antibiotic, rifampicin, chloramphenicol, fluoramphenicol, colistin, mupirocin, bacitracin, daptomycin, or linezolid.

Also described herein is a method for treating a bacterial infection in a patient, preferably a human, where the treatment includes administering a therapeutically or pharmacologically effective amount of a combination of 1) a β-lactam antibiotic; and 2) a compound of Formula (I), (I'), (Ia), (Ib), (Ic), (Id), (II), (II'), (IIa), (IIb), (IIc), (IId), or (IIe), or a pharmaceutically acceptable salt thereof; and 3) a pharmaceutically acceptable carrier. Where a β-lactam antibiotic is used in combination with a compound of Formula (I), (I'), (Ia), (Ib), (Ic), (Id), (II), (II'), (IIa), (IIb), (IIc), (IId), or (IIe), the β-lactam antibiotic may be a carbapenem, cephalosporin, cephamycin, monobactam or penicillin. Exemplary carbapenem antibiotics useful in the methods described herein include ertapenem, imipenem, biapenem, and meropenem. Exemplary cephalosporin antibiotics useful in the methods described herein include, ceftobiprole, ceftaroline, Cefiprome, Cefozopran, cefepime, Cefotaxime, and ceftriazone. Exemplary penicillin antibiotics useful in the methods described herein include ampicillin, amoxacillin, piperacillin, oxacillin, cloxacillin, methicillin, and nafcillin. The β-lactam may be administered with a β-lactamase inhibitor. The carbapenem may be administered with a DHP inhibitor, e.g., cilastatin.

Where a compound of Formula (I), (I'), (Ia), (Ib), (Ic), (Id), (II), (II'), (IIa), (IIb), (IIc), (IId), or (IIe) and a β-lactam antibiotic are used in combination, the β-lactam antibiotic and compound of Formula (I), (I'), (Ia), (Ib), (Ic), (Id), (II), (II'), (Ila), (IIb), (IIc), (IId), or (IIe) can be administered sequentially or concurrently. Preferably, the β-lactam antibiotic and compound of Formula (I), (I'), (Ia), (Ib), (Ic), (Id), (II), (II'), (IIa), (IIb), (IIc), (IId), or (IIe) are administered together. When administered concurrently, the β-lactam antibiotic and compound of Formula (I), (I'), (Ia), (Ib), (Ic), (Id), (II), (II'), (Ila), (IIb), (IIc), (IId), or (IIe) may be administered in the same formulation or in separate formulations. When administered sequentially, either the β-lactam or compound of Formula (I), (I'), (Ia), (Ib), (Ic), (Id), (II), (II'), (IIa), (lib), (IIc), (IId), or (IIe) may be administered first. After administration of the first compound, the other compound is administered, for example, within from 1 to 60 minutes, e.g., within 1, 2, 3, 4, 5, 10, 15, 30, or 60 minutes. When a β-lactamase inhibitor is used, it may be administered separately, or in a formulation with the compound of Formula (I), (I'), (Ia), (Ib), (Ic), (Id), (II), (II'), (Ila), (IIb), (IIc), (IId), or (IIe) and/or β-lactam antibiotic. When a DHP inhibitor is used to improve the stability of a carbapenem, it may be administered separately, or in a formulation with the compound of Formula (I), (I'), (Ia), (Ib), (Ic), (Id), (II), (II'), (IIa), (IIb), (IIc), (IId), or (IIe) and/or carbapenem.

Further described herein are pharmaceutical compositions comprising a compound of Formula (I), (I'), (Ia), (Ib), (Ic), (Id), (II), (II'), (IIa), (IIb), (IIc), (IId), or (IIe), a pharmaceutically acceptable carrier, and optionally a β-lactam antibiotic. Where a combination is used, the β-lactam antibiotic and the compound of Formula (I), (I'), (Ia), (Ib), (Ic), (Id), (II), (II'), (IIa), (IIb), (IIc), (IId), or (IIe), are present in such amounts that their combination constitutes a therapeutically effective amount. Due to the potentiating effects of the compound of Formula (I), (I'), (Ia), (Ib), (Ic), (Id), (II), (II'), (IIa), (IIb), (IIc), (IId), or (IIe), the amount of β-lactam antibiotic present in a combination may be less that of a β-lactam antibiotic used alone. Certain compositions described herein further comprise a β-lactamase antibiotic.

Where the β-lactam antibiotic is a carbapenem, a pharmaceutical composition comprising a carbapenem antibiotic, a DHP inhibitor, a compound of Formula (I), (I'), (Ia), (Ib), (Ic), (Id), (II), (II'), (IIa), (IIb), (IIc), (IId), or (IIe), and a pharmaceutically acceptable carrier may be described. Where the β-lactara antibiotic is a carbepenem, the carbapenem antibiotic is preferably selected from the group consisting of ertapenem, imipenem, and meropenem.

Also described herein is a compound of Formula (I), (I'), (Ia), (Ib), (Ic), (Id), (II), (II'), (IIa), (IIb), (IIc), (IId), or (IIe) for use in treating a bacterial infection. Also described herein is a compound of Formula (I), (I'), (Ia), (Ib), (Ic), (Id), (II), (II'), (IIa), (IIb), (IIc), (IId), or (IIe), in combination with one or more additional therapeutical agents including a β-lactam antibiotic, for use in treating a bacterial infection. Also described herein is a compound of Formula (I), (I'), (Ia), (Ib), (Ic), (Id), (II), (II'), (Ila), (IIb), (IIc), (IId), or (IIe) for use as a medicament for treating a bacterial infection. Also described is a compound of Formula (I), (I'), (Ia), (Ib), (Ic), (Id), (II), (II'), (IIa), (IIb), (IIc), (IId), or (IIe), in combination with one or more additional therapeutical agents including a β-lactam antibiotic, for use as a medicament for treating a bacterial infection. Also described is a compound of Formula (I), (I'), (Ia), (Ib), (Ic), (Id), (II), (II'), (IIa), (IIb), (IIc), (IId), or (IIe) for use in the preparation of a medicament for treating a bacterial infection. Also described is a compound of Formula (I), (I'), (Ia), (Ib), (Ic), (Id), (II), (II'), (IIa), (IIb), (IIc), (IId), or (IIe), in combination with one or more additional therapeutical agents including a β-lactam antibiotic, for use in the preparation of a medicament for treating a bacterial infection.

As described herein, a compound of Formula (I), (I'), (Ia), (Ib), (Ic), (Id), (II), (II'), (IIa), (IIb), (IIc), (IId), or (IIe) can enhance the activity of a β-lactam antibacterial agent by inducing susceptibility to the antibacterial agent in a drug- resistant strain such as MRSA. In some embodiments, a compound of Formula (I), (I'), (Ia), (Ib), (Ic), (Id), (II), (II'), (Ila), (IIb), (IIc), (IId), or (IIe) can enhance the activity of a β-lactam antibacterial agent by reducing the dosage of the antibacterial agent need for a therapeutic effect in a drug-sensitive strain. For example, if a compound of Formula (I), (I'), (Ia), (Ib), (Ic), (Id), (II), (II'), (IIa), (IIb), (IIc), (IId), or (IIe) reduces the Minimum Inhibitory Concentration (MIC) of an antibacterial agent (where the MIC is the minimum concentration of antibacterial agent which will completely inhibit growth) in a susceptible strain, then such treatment may be advantageous to enable a reduction in the amount of antibacterial agent administered (could reduce side effects of an antibiotic), or to decrease the frequency of administration. Compounds of Formula (I), (I'), (Ia), (Ib), (Ic), (Id), (II), (II'), (IIa), (IIb), (IIc), (IId), or (IIe) can enhance the activity of an antibacterial agent such as a carbapenem to prevent the emergence of a resistant sub-population in a heterogeneous bacterial population with a resistant sub-population.

Potentiators can be used to enhance the activity of antibacterial agents whose clinical efficacy has been limited by the increasing prevalence of resistant strains. As described herein, a compound of Formula (I), (I'), (Ia), (Ib), (Ic), (Id), (II), (II'), (IIa), (IIb), (IIc), (IId), or (IIe) may be used as a potentiator wherein a compound of Formula (I), (I'), (Ia), (Ib), (Ic), (Id), (II), (II'), (IIa), (IIb), (IIc), (IId), or (IIe) can be administered together with a β-lactam antibiotic (either concurrently or sequentially) to allow effective treatment of an infection involving a resistant bacterium, or to reduce the amount of antibacterial agent necessary to treat an infection.

Also described is a compound described herein which displays antibiotic activity useful in the treatment of bacterial infections, such as by way of example only, various strains of S. aureus, S. pneumoniae, E. faecalis, E. faecium, B. subtilis and E. coli including species that are resistant to many known antibiotics such as methicillin-resistant S. aureus (MRSA), vancomycin-resistant Enterococcus sp. (VRE), multidrug-resistant E. faecium, macrolide-resistant S. aureus and S. epidermidis, and linezolide-resistant S. aureus and E. faecium.

### Methicillin-Resistant Staphylococcus aureus

Staphylococcus aureus (S. aureus), a spherical bacterium, is the most common cause of staph infections. S. aureus has been known to cause a range of illnesses from minor skin infections, such as pimples, impetigo, boils, cellulitis folliculitis, furuncles, carbuncles, scalded skin syndrome, abscesses, to life-threatening diseases such as pneumonia, meningitis, osteomyelitis endocarditis, toxic shock syndrome, and septicemia. Further, S. aureus is one of the most common causes of nosocomial infections, often causing postsurgical wound infections.

Methicillin was introduced in the late 1950s to treat infections caused by penicillin-resistant S. aureus. It has been reported previously that *S. aureus* isolates had acquired resistance to methicillin (methicillin-resistant *S*. *aureus,* MRSA). The methicillin resistance gene (*mecA*) encodes a methicillin-resistant penicillin-binding protein that is not present in susceptible strains. *mecA* is carried on a mobile genetic element, the staphylococcal cassette chromosome *mec* (*SCCmec*), of which four forms have been described that differ in size and genetic composition. The methicillin-resistant penicillin-binding protein allows for resistance to β-lactam antibiotics and obviates their clinical use during MRSA infections.

Also described is a method for treating a subject having a resistant bacterium comprising administering to the subject a compound of Formula (I), (I'), (Ia), (Ib), (Ic), (Id), (II), (II'), (IIa), (IIb), (IIc), (IId), or (IIe) or a pharmaceutically acceptable salt, ester, solvate, alkylated quaternary ammonium salt, stereoisomer, tautomer or prodrug thereof. In one embodiment, the bacterium is a Gram-positive bacteria. For example, the Gram-positive bacterium is S. aureus. For example, the S. aureus is resistant or refractory to a beta-lactam antibiotic. For example, the beta-lactam antibiotic belongs to the class of penicillins. In a further embodiment, the beta-lactam antibiotic is methicillin. For example, the subject has a methicillin-resistant S. aureus bacteria. For example the beta-lactam antibiotic is flucloxacillin. Also described herein is a method for treating a subject having a dicloxacillin-resistant bacteria comprising administering to the subject a compound of Formula (I), (I'), (Ia), (Ib), (Ic), (Id), (II), (II'), (IIa), (IIb), (IIc), (IId), or (IIe) or a pharmaceutically acceptable salt, ester, solvate, alkylated quaternary ammonium salt, stereoisomer, tautomer or prodrug thereof wherein the subject is refractory to dicloxacillin. Also disclosed herein is a method for treating a subject having a methicillin-resistant bacteria comprising administering a compound of Formula (I), (I'), (Ia), (Ib), (Ic), (Id), (II), (II'), (IIa), (IIb), (IIc), (IId), or (IIe) or a pharmaceutically acceptable salt, ester, solvate, alkylated quaternary ammonium salt, stereoisomer, tautomer or prodrug thereof wherein the subject has been determined to have a methicillin-resistant bacteria. For example the subject is screened for methicillin-resistant bacteria. For example, the subject screening is performed through a nasal culture. For example the methicillin-resistant bacteria is detected by swabbing the nostril(s) of the subject and isolating the bacteria. In another embodiment, Real-time PCR and/or Quantitative PCR is employed to determine whether the subject has a methicillin-resistant bacteria.

Also described is a method for treating a subject having a first-generation cephalosporin-resistant bacteria comprising administering a compound of Formula (I), (I'), (Ia), (Ib), (Ic), (Id), (II), (II'), (IIa), (IIb), (IIc), (IId), or (IIe) or a pharmaceutically acceptable salt, ester, solvate, alkylated quaternary ammonium salt, stereoisomer, tautomer or prodrug thereof wherein the subject is refractory to a first-generation cephalosporin. For example, the bacteria is resistant to a first-generation cephalosporin. For example, the bacteria is resistant to cefacetrile. For example, the bacteria is resistant to cefadroxil. For example, the bacteria is resistant to cefalexin. For example, the bacteria is resistant to cefaloglycin. For example, the bacteria is resistant to cefalonium. For example, the bacteria is resistant to cefaloridine. For example, the bacteria is resistant to cefalotin. For example, the bacteria is resistant to cefapirin. For example, the bacteria is resistant to cefatrizine. For example, the bacteria is resistant to cefazaflur. For example, the bacteria is resistant to cefazedone. For example, the bacteria is resistant to cefazolin. For example, the bacteria is resistant to cefradine. For example, the bacteria is resistant to cefroxadine. For example, the bacteria is resistant to ceftezole.

Also described herein is a method for treating a subject having a second-generation cephalosporin-resistant bacteria comprising administering a compound of Formula (I), (I'), (Ia), (Ib), (Ic), (Id), (II), (II'), (IIa), (IIb), (IIc), (IId), or (IIe) or a pharmaceutically acceptable salt, ester, solvate, alkylated quaternary ammonium salt, stereoisomer, tautomer or prodrug thereof wherein the subject is refractory to a second-generation cephalosporin. For example, the bacteria is resistant to a second-generation cephalosporin. For example, the bacteria is resistant to cefaclor. For example, the bacteria is resistant to cefonicid. For example, the bacteria is resistant to cefprozil. For example, the bacteria is resistant to cefuroxime. For example, the bacteria is resistant to cefuzonam. For example, the bacteria is resistant to cefmetazole. For example, the bacteria is resistant to cefotetan. For example, the bacteria is resistant to cefoxitin.

Also described is a method for treating a subject having a third-generation cephalosporin-resistant bacteria comprising administering a compound of Formula (I), (I'), (Ia), (Ib), (Ic), (Id), (II), (II'), (IIa), (IIb), (IIc), (IId), or (IIe) or a pharmaceutically acceptable salt, ester, solvate, alkylated quaternary ammonium salt, stereoisomer, tautomer or prodrug thereof wherein the subject is refractory to a third-generation cephalosporin. For example, the bacteria is resistant to a third-generation cephalosporin. In a further embodiment, the bacteria is resistant to cefcapene. For example, the bacteria is resistant to cefdaloxime. For example, the bacteria is resistant to cefdinir. For example, the bacteria is resistant to cefditoren. For example, the bacteria is resistant to cefixime. For example, the bacteria is resistant to cefmenoxime. For example, the bacteria is resistant to cefodizime. For example, the bacteria is resistant to cefotaxime. For example, the bacteria is resistant to cefpimizole. For example, the bacteria is resistant to cefpodoxime. For example, the bacteria is resistant to cefteram. For example, the bacteria is resistant to ceftibuten. For example, the bacteria is resistant to ceftiofur. For example, the bacteria is resistant to ceftiolene. For example, the bacteria is resistant to ceftizoxime. For example, the bacteria is resistant to ceftriaxone. For example the bacteria is resistant to cefoperazone. For example, the bacteria is resistant to ceftazidime.

Also described is a method for treating a subject having a fourth-generation cephalosporin-resistant bacteria comprising administering a compound of Formula (I), (I'), (Ia), (Ib), (Ic), (Id), (II), (II'), (IIa), (IIb), (IIc), (IId), or (IIe) or a pharmaceutically acceptable salt, ester, solvate, alkylated quaternary ammonium salt, stereoisomer, tautomer or prodrug thereof wherein the subject is refractory to a fourth-generation cephalosporin. For example, the bacteria is resistant to a fourth-generation cephalosporin. For example, the bacteria is resistant to cefclidine. For example, the bacteria is resistant to cefepime. For example, the bacteria is resistant to cefluprenam. For example, the bacteria is resistant to cefoselis. For example, the bacteria is resistant to cefozopran. For example, the bacteria is resistant to cefpirome. For example, the bacteria is refractory to cefquinome.

Also described is a method for treating a subject having a carbapenem-resistant bacteria comprising administering a compound of Formula (I), (I'), (Ia), (Ib), (Ic), (Id), (II), (II'), (Ila), (IIb), (IIc), (IId), or (IIe) or a pharmaceutically acceptable salt, ester, solvate, alkylated quaternary ammonium salt, stereoisomer, tautomer or prodrug thereof wherein the subject is refractory to a carbapenem. For example, the bacteria is resistant to a carbapenem. For example, the bacteria is resistant to imipenem. For example, the bacteria is resistant to meropenem. For example, the bacteria is resistant to ertapenem. For example, the bacteria is resistant to faropenem. For example, the bacteria is resistant to doripenem. For example, the bacteria is resistant to panipenem. For example, the bacteria is resistant to biapenem,

### Vancomycin-Intermediate and Vancomycin-Resistant Staphylococcus aureus

Vancomycin-intermediate Staphylococcus aureus and vancomycin-resistant staphylococcus aureus are specific types of antimicrobial-resistant Staph bacteria that are refractory to vancomycin treatment. S. aureus isolates for which vancomycin MICs are 4-8 µg/mL are classified as vancomycin-intermediate and isolates for which vancomycin MICs are ≥16 µg/mL are classified as vancomycin-resistant (Clinical and Laboratory Standards Institute/NCCLS. Performance Standards for Antimicrobial Susceptibility Testing. Sixteenth informational supplement. M100-S16. Wayne, PA: CLSI, 2006).

As used herein, the term "minimum inhibitory concentration" (MIC) refers to the lowest concentration of an antibiotic that is needed to inhibit growth of a bacterial isolate in vitro. A common method for determining the MIC of an antibiotic is to prepare several tubes containing serial dilutions of the antibiotic, that are then inoculated with the bacterial isolate of interest. The MIC of an antibiotic is determined from the tube with the lowest concentration that shows no turbidity (no growth).

Also described herein is a method of treating a subject having a bacterial infection comprising administering to the subject a compound of Formula (I), (I'), (Ia), (Ib), (Ic), (Id), (II), (II'), (IIa), (IIb), (IIc), (IId), or (IIe) or a pharmaceutically acceptable salt, ester, solvate, alkylated quaternary ammonium salt, stereoisomer, tautomer or prodrug thereof wherein the bacterial infection comprises a vancomycin-intermediate Staphylococcus aureus bacterium. For example, the vancomycin-intermediate Staphylococcus aureus bacterium has a MIC of between about 4 to about 8 µg/mL. For example, the vancomycin-intermediate Staphylococcus aureus bacterium has a MIC of about 4 µg/mL. For example, the vancomycin-intermediate Staphylococcus aureus bacterium has a MIC of about 5 µg/mL. For example, the vancomycin-intermediate Staphylococcus aureus bacterium has a MIC of about 6 µg/mL. For example, the vancomycin-intermediate Staphylococcus aureus bacterium has a MIC of about 7 µg/mL. For example, the vancomycin-intermediate Staphylococcus aureus bacterium has a MIC of about 8 µg/mL.

Also described herein is a method of treating a subject having a bacterial infection comprising administering to the subject a compound of Formula (I), (I'), (Ia), (Ib), (Ic), (Id), (II), (II'), (IIa), (IIb), (IIc), (IId), or (IIe) or a pharmaceutically acceptable salt, ester, solvate, alkylated quaternary ammonium salt, stereoisomer, tautomer or prodrug thereof wherein the bacterial infection comprises a vancomycin-resistant Staphylococcus aureus bacterium. For example, the vancomycin-resistant Staphylococcus aureus bacterium has a MIC of between about 16 µg/mL. For example, the vancomycin-resistant Staphylococcus aureus bacterium has a MIC of about ≥ 16 µg/mL. For example, the vancomycin-resistant Staphylococcus aureus bacterium has a MIC of about 20 µg/mL. For example, the vancomycin-resistant Staphylococcus aureus bacterium has a MIC of about 25 µg/mL.

For example, conditions treated by the compounds described herein include, but are not limited to, endocarditis, osteomyelitis, neningitis, skin and skin structure infections, genitourinary tract infections, abscesses, and necrotizing infections. For example, the compounds disclosed herein are used to treat conditions, such as, but not limited to, diabetic foot infections, decubitus ulcers, burn infections, animal or human bite wound infections, synergistic-necrotizing gangrene, necrotizing fascilitis, intra-abdominal infection associated with breeching of the intestinal barrier, pelvic infection associated with breeching of the intestinal barrier, aspiration pneumonia, and post-operative wound infections. In another embodiment, the conditions listed herein are caused by, contain, or result in the presence of VISA and/or VRSA.

### Vancomycin-Resistant Enterococci

Enterococci are bacteria that are normally present in the human intestines and in the female genital tract and are often found in the environment. These bacteria sometimes cause infections. In some cases, enterococci have become resistant to vancomycin (also known as vancomycin-resistant enterococci or VRE.) Common forms of resistance to vancomycin occur in enterococcal strains that involve the acquisition of a set of genes encoding proteins that direct peptidoglycan precursors to incorporate D-Ala-D-Lac instead of D-Ala-D-Ala. The six different types of vancomycin resistance shown by enterococcus are: Van-A, Van-B, Van-C, Van-D, VanE and Van-F. In some cases, Van-A VRE is resistant to both vancomycin and teicoplanin, while in other cases, Van-B VRE is resistant to vancomycin but sensitive to teicoplanin; in further cases Van-C is partly resistant to vancomycin, and sensitive to teicoplanin.

Also described herein is a method of treating a subject having a vancomycin-resistant enterococci comprising administering to the subject a compound of Formula (I), (I'), (Ia), (Ib), (Ic), (Id), (II), (II'), (Ila), (IIb), (IIc), (IId), or (IIe) or a pharmaceutically acceptable salt, ester, solvate, alkylated quaternary ammonium salt, stereoisomer, tautomer or prodrug thereof wherein the enterococci has developed resistance to vancomycin. For example, the subject has been previously treated with vancomycin for a sustained period of time. For example, the subject has been hospitalized. For example, the subject has a weakened immune system such as patients in Intensive Care Units or in cancer or transplant wards. For example, the subject has undergone surgical procedures such as, for example, abdominal or chest surgery. For example, the subject has been colonized with VRE. For example, the subject has a medical device such that an infection has developed. For example, the medical device is a urinary catheter or central intravenous (IV) catheter.

Also described herein is a method of treating a subject having a vancomycin-resistant enterococci comprising administering to the subject a compound of Formula (I), (I'), (Ia), (Ib), (Ic), (Id), (II), (II'), (IIa), (IIb), (IIc), (IId), or (IIe) or a pharmaceutically acceptable salt, ester, solvate, alkylated quaternary ammonium salt, stereoisomer, tautomer or prodrug thereof wherein the enterococcus has Van-A resistance.

Also described herein is a method of treating a subject having a vancomycin-resistant enterococci comprising administering to the subject a compound of Formula (I), (I'), (Ia), (Ib), (Ic), (Id), (II), (II'), (IIa), (IIb), (IIc), (IId), or (IIe) or a pharmaceutically acceptable salt, ester, solvate, alkylated quaternary ammonium salt, stereoisomer, tautomer or prodrug thereof wherein the enterococcus has Van-B resistance.

Also described herein is a method of treating a subject having a vancomycin-resistant enterococci comprising administering to the subject a compound of Formula (I), (I'), (Ia), (Ib), (Ic), (Id), (II), (II'), (IIa), (IIb), (IIc), (IId), or (IIe) or a pharmaceutically acceptable salt, ester, solvate, alkylated quaternary ammonium salt, stereoisomer, tautomer or prodrug thereof wherein the enterococcus has Van-C resistance.

### Administration and Pharmaceutical Composition

Pharmaceutical compositions described herein comprise a therapeutically effective amount of a compound described herein (i.e., a compound of the invention, or a compound of any of Formula (I), (I'), (Ia), (Ib), (Ic), (Id), (II), (II'), (Ila), (IIb), (IIc), (IId), or (IIe) as generally described herein) formulated together with one or more pharmaceutically acceptable carriers. As used herein, the term "pharmaceutically acceptable carrier" means a non-toxic, inert solid, semisolid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator. The pharmaceutical compositions described herein can be administered to humans and other animals orally, rectally, parenterally, intracisternally, intravaginally, intraperitoneally, topically (as by powders, ointments, or drops), bucally, or as an oral or nasal spray, or a liquid aerosol or dry powder formulation for inhalation.

Liquid dosage forms for oral administration include pharmaceutically acceptable emulsions, microemulsions, solutions, suspensions, syrups and elixirs. In addition to the active compounds, the liquid dosage forms optionally contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions are optionally formulated according to the known art using suitable dispersing or wetting agents and suspending agents. The sterile injectable preparation is optionally a sterile injectable solution, suspension or emulsion in a nontoxic parenterally acceptable diluent or solvent, for example, as a solution in 1,3-butanediol. Among the acceptable vehicles and solvents that are optionally employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This is optionally accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is optionally accomplished by dissolving or suspending the drug in an oil vehicle. Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are optionally prepared by entrapping the drug in liposomes or microemulsions which are compatible with body tissues.

Compositions for rectal or vaginal administration are preferably suppositories which can be prepared by mixing the compound described herein (i.e., a compound of the invention, or a compound of any of Formula (I), (I'), (Ia), (Ib), (Ic), (Id), (II), (II'), (IIa), (IIb), (IIc), (IId), or (IIe) as generally described herein) with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, acetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form optionally comprise buffering agents.

Solid compositions of a similar type are optionally employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings known in the pharmaceutical formulating art. They optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Solid compositions of a similar type are optionally employed as fillers in soft and hard-filled gelatin capsules using such excipients as lactose or milk sugar as well as high molecular weight polyethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings known in the pharmaceutical formulating art. In such solid dosage forms the active compound is optionally admixed with at least one inert diluent such as sucrose, lactose or starch. Such dosage forms optionally comprise, as is normal practice, additional substances other than inert diluents, e.g., tableting lubricants and other tableting aids such a magnesium stearate and microcrystalline cellulose. In the case of capsules, tablets and pills, the dosage forms optionally comprise buffering agents. They optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Dosage forms for topical or transdermal administration of a compound described herein include ointments, pastes, creams, lotions, gels, powders, solutions, sprays, inhalants or patches. The active component is admixed under sterile conditions with a pharmaceutically acceptable carrier and any needed preservatives or buffers as are optionally required. Ophthalmic formulations, ear drops, and the like are also contemplated.

The ointments, pastes, creams and gels may contain, in addition to an active compound described herein, excipients such as animal and vegetable fats, oils, waxes, paraffins, starch, tragacanth, cellulose derivatives, polyethylene glycols, silicones, bentonites, silicic acid, talc and zinc oxide, or mixtures thereof.

Compositions described herein are optionally formulated for delivery as a liquid aerosol or inhalable dry powder. Liquid aerosol formulations are optionally nebulized predominantly into particle sizes that can be delivered to the terminal and respiratory bronchioles where bacteria reside in patients with bronchial infections, such as chronic bronchitis and pneumonia. Pathogenic bacteria are commonly present throughout airways down to bronchi, bronchioli and lung parenchema, particularly in terminal and respiratory bronchioles. During exacerbation of infection, bacteria can also be present in alveoli. Liquid aerosol and inhalable dry powder formulations are preferably delivered throughout the endobronchial tree to the terminal bronchioles and eventually to the parenchymal tissue.

Aerosolized formulations described herein are optionally delivered using an aerosol forming device, such as a jet, vibrating porous plate or ultrasonic nebulizer, preferably selected to allow the formation of aerosol particles having with a mass medium average diameter predominantly between 1 to 5 µ. Further, the formulation preferably has balanced osmolarity ionic strength and chloride concentration, and the smallest aerosolizable volume able to deliver effective dose of the compounds described herein compound described herein (i.e., a compound of the invention, or a compound of any of Formula (I), (I'), (Ia), (Ib), (Ic), (Id), (II), (II'), (Ila), (IIb), (IIc), (IId), or (IIe) as generally described herein) to the site of the infection. Additionally, the aerosolized formulation preferably does not impair negatively the functionality of the airways and does not cause undesirable side effects.

Aerosolization devices suitable for administration of aerosol formulations described herein include, for example, jet, vibrating porous plate, ultrasonic nebulizers and energized dry powder inhalers, that are able to nebulize the formulation into aerosol particle size predominantly in the size range from 1-5 µ. Predominantly in this application means that at least 70% but preferably more than 90% of all generated aerosol particles are within 1-5 µ range. A jet nebulizer works by air pressure to break a liquid solution into aerosol droplets. Vibrating porous plate nebulizers work by using a sonic vacuum produced by a rapidly vibrating porous plate to extrude a solvent droplet through a porous plate. An ultrasonic nebulizer works by a piezoelectric crystal that shears a liquid into small aerosol droplets. A variety of suitable devices are available, including, for example, AeroNebTM and AeroDoseTM vibrating porous plate nebulizers (AeroGen, Inc., Sunnyvale, California), Sidestream^{®} nebulizers (Medic-Aid Ltd., West Sussex, England), Pari LC^{®} and Pari LC Star^{®} jet nebulizers (Pari Respiratory Equipment, Inc., Richmond, Virginia), and AerosonicTM (DeVilbiss Medizinische Produkte (Deutschland) GmbH, Heiden, Germany) and UltraAire^{®} (Omron Healthcare, Inc., Vernon Hills, Illinois) ultrasonic nebulizers.

Compounds described herein (i.e., a compound of the invention, or a compound of any of Formula (I), (I'), (Ia), (Ib), (Ic), (Id), (II), (II'), (IIa), (IIb), (IIc), (IId), or (IIe) as generally described) may be formulated for use as topical powders and sprays that contain, in addition to the compounds described herein, excipients such as lactose, talc, silicic acid, aluminum hydroxide, calcium silicates and polyamide powder, or mixtures of these substances. Sprays optionally contain customary propellants such as chlorofluorohydrocarbons.

Transdermal patches have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

According to the methods of treatment described herein, bacterial infections are treated or prevented in a patient such as a human or lower mammal by administering to the patient a therapeutically effective amount of a compound described herein, in such amounts and for such time as is necessary to achieve the desired result. By a "therapeutically effective amount" of a compound described herein is meant a sufficient amount of the compound to treat bacterial infections, at a reasonable benefit/risk ratio applicable to any medical treatment. It will be understood, however, that the total daily usage of the compounds and compositions described herein will be decided by the attending physician within the scope of sound medical judgment. The specific therapeutically effective dose level for any particular patient will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors known in the medical arts.

The total daily dose of the compounds described herein compound described herein (i.e., a compound of the invention, or a compound of any of Formula (I), (I'), (Ia), (Ib), (Ic), (Id), (II), (II'), (IIa), (IIb), (IIc), (IId), or (IIe) as generally described) administered to a human or other mammal in single or in divided doses can be in amounts, for example, from 0.01 to 50 mg/kg body weight or more usually from 0.1 to 25 mg/kg body weight. Single dose compositions may contain such amounts or submultiples thereof to make up the daily dose. In general, treatment regimens described herein comprise administration to a patient in need of such treatment from about 10 mg to about 2000 mg of the compound(s) described herein per day in single or multiple doses.

### Examples

Compounds disclosed herein are made by the methods depicted in the reaction schemes shown below. Procedures are provided herein that, in combination with the knowledge of the synthetic organic chemist of ordinary skill in the art, are in some embodiments used to prepare the full range of compounds as disclosed and claimed herein.

The starting materials and reagents used in preparing these compounds are either available from commercial suppliers such as Aldrich Chemical Co., (Milwaukee, Wis.), Bachem (Torrance, Calif.), or Sigma (St. Louis, Mo.) or are prepared by methods known to those skilled in the art following procedures set forth in references such as Fieser and Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991), March's Advanced Organic Chemistry, (John Wiley and Sons, 4th Edition) and Larock's Comprehensive Organic Transformations (VCH Publishers Inc., 1989). These schemes are merely illustrative of some methods by which the compounds disclosed herein are in some embodiments synthesized, and various modifications to these schemes can be made and will be suggested to one skilled in the art having referred to this disclosure. The starting materials and the intermediates, and the final products of the reaction may be isolated and purified if desired using conventional techniques, including but not limited to filtration, distillation, crystallization, chromatography and the like. Such materials may be characterized using conventional means, including physical constants and spectral data. Compounds are typically isolated as formic acid salts by reverse phase HPLC using AcCN/H₂0 with formic acid as an additive. In some instances, purifications are conducted without formic acid, and the compounds are isolated as the free base.

The methods of LCMS analysis are as follows:
LCMS (Method 5-95 AB, ESI): ESI, 5% AcCN/H₂O, 0.7 min; to 95% AcCN/H₂O, 0.4 min; 1.5 mL/min, Merck RP-18e, 2 x 25 mm.
LCMS (Method 10-80AB, 2 min, ESI): ESI, 10% AcCN/H₂O (0.04% TFA), 0.9 min to 80% AcCN/H₂O (0.04% TFA), then held for 0.6 min; 1.2 mL/min, Xtimate C18, 3 µm, 2.1 × 30 mm).
LCMS (Method 10-80AB, 7 min, ESI): ESI, 10% AcCN/H₂O (0.04% TFA), 6 min to 80% AcCN/H₂O (0.04% TFA), then held for 0.9 min; 0.8 mL/min, Xtimate C18, 3 µm, 2.1 × 30 mm).
Some abbreviations used herein are as follows:
DIPEA: diisopropylethylamine
DMAP: 4-dimethylaminopyridine
DMF: dimethylformamide
DCM: dichloromethane
TFA: trifluoroacetic acid
EDC: 1-ethyl-3-(3-dimethylaminopropyl)carbodiimide
HATU: *O*-(7-azabenzotriazol-1-yl)-*N,N,N',N*'-tetramethyluronium hexafluorophosphate
HCTU: *O*-(6-Chlorobenzotriazol-1-yl)-*N*,*N*,*N'*,*N'*-tetramethyluronium hexafluorophosphate
HOBt: hydroxybenzotriazole
pyBOP: (Benzotriazol-1-yloxy)tripyrrolidinophosphonium hexafluorophosphate
DMDO: 3,3-Dimethyldioxirane
DMP: Dess-Martin periodinane
THF: tetrahydrofuran
MeOH: methanol
EtOAc: ethyl acetate
Trt resin: 2-Chlorotrityl chloride resin
Rink amide resin: Rink amide (aminomethyl)polystyrene
Boc: t-butoxycarbonyl
CBz: benzyloxycarbonyl
Fmoc: [(9*H*-fluoren-9-yl)methoxy]carbonyl
Teoc: Trimethylsilylethoxycarbonyl
CDI: 1,1'-Carbonyldiimidazole
HFIP: 1,1,1,3,3,3-hexafluoropropan-2-ol
TLC: thin-layer chromatography

### Example 1: Synthesis of (S)-methyl 2-amino-3-(4-methoxy-3-(4,4,5,5-tetramethyl-1,3,2-dioxaborolan-2-yl)phenyl)propanoate

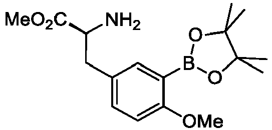

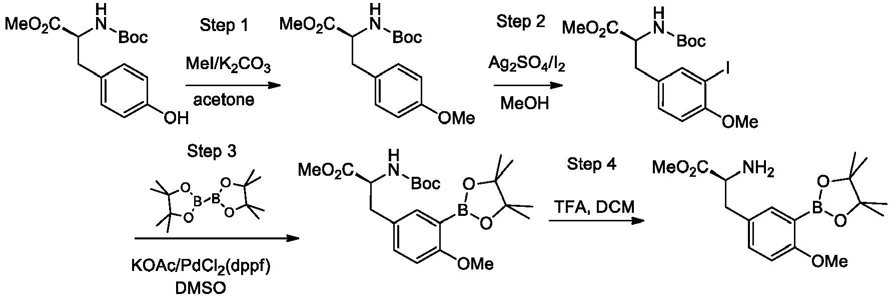

Step 1: To a solution of (S)-methyl 2-((tert-butoxycarbonyl)amino)-3-(4-hydroxyphenyl)propanoate (100 g, 0.323 mol) in acetone (2.0 L) was added K₂CO₃ (37 g, 0.34 mol). After the addition, MeI (32 mL, 0.97 mol) was added dropwise, and the reaction mixture was stirred at room temperature for 72 h and monitored by TLC. The reaction had not yet gone to completion, so NaOH (0.1 eq) was added to the reaction mixture. And after 2 h, the reaction was completed. The solid was filtered and the solvent was removed. The residue was taken up in ethyl acetate and washed with H₂O, extracted with ethyl acetate (300 mL x 3). The combined organic layers were washed with brine, dried over Na₂SO₄ and concentrated to give (S)-methyl 2-((tert-butoxycarbonyl)amino)-3-(4-methoxyphenyl)propanoate (100 g, 95.4%).

Step 2: To a solution of (S)-methyl 2-((tert-butoxycarbonyl)amino)-3-(4-methoxyphenyl)propanoate (80 g, 40 g each x 2, run in two separate batches, 259 mmol overall) in methanol (1.5 L in each of the two flasks) was added sequentially Ag₂SO₄ (85 g, 272 mmol, ½-added to each flask) and I₂ (72 g, 283 mmol, ½-added to each flask). The reaction mixture was stirred at room temperature for 2 h. The reaction was monitored by LCMS. When all (S)-methyl 2-((tert-butoxycarbonyl)amino)-3-(4-methoxyphenyl)propanoate had been consumed, then a solution of 10% (w/w) sodium thiosulfate was added until the reaction turned pale yellow. The solid was filtered and most of the methanol was evaporated by rotary evaporation. Water and ethyl acetate were added to each batch. The aqueous layer was extracted with ethyl acetate (3 x 200 mL). The combined organic layers were washed with brine, dried over sodium sulfate and concentrated. The crude material was combined for the two batches and they were purified together by flash column chromatography on silica gel (25% then 35% then 40% ethyl acetate in hexanes) to give (S)-methyl 2-((tert-butoxycarbonyl)amino)-3-(3-iodo-4-methoxyphenyl)propanoate (97 g, 89%).

Step 3: (S)-Methyl 2-((tert-butoxycarbonyl)amino)-3-(3-iodo-4-methoxyphenyl)propanoate (92 g, 46 g each run in two separate batches, 211 mmol) was dissolved in anhydrous DMSO (1.5 L, Yz-added for each batch) under argon and to the solution was added bis(pinacolato) diboron (80.5 g, 317 mmol, ½-added for each batch) and KOAc (103 g, 1.05 mol, ½-added for each batch). This mixture was degassed with argon for twenty minutes, then Pd(dppf)Cl₂ (4.6 g, 6 mmol, ½-added for each batch) was added. The mixture was degassed with argon five times, then kept under argon and heated to 80°C for 3 h. TLC showed that the reaction was complete, and the reaction mixture was cooled to room temperature and filtered. The reaction mixture was dissolved in EA and washed with H₂O. The aqueous layer was extracted ethyl acetate (3 x 200 mL). The combined organic layers were dried over sodium sulfate, filtered and concentrated to give the crude product. The batches were then combined and purified together by flash column chromatography on silica gel (3% ethyl acetate in hexanes, then 20% to 25% ethyl acetate in hexanes to give (S)-methyl 2-((tert-butoxycarbonyl)amino)-3-(4-methoxy-3-(4,4,5,5-tetramethyl-1,3,2-dioxaborolan-2-yl)phenyl)propanoate (70 g, 76%).

Step 4: (S)-Methyl 2-((tert-butoxycarbonyl)amino)-3-(4-methoxy-3-(4,4,5,5-tetramethyl-1,3,2-dioxaborolan-2-yl)phenyl)propanoate (22 g, 50.6 mmol) was dissolved in dichloromethane (150 mL) and treated with trifluoroacetic acid (50 mL). The reaction mixture was stirred at room temperature and the reaction was monitored by HPLC. When all of the starting material had been consumed, the solvents were evaporated, DCM was added and Na₂CO₃ was added to neutralize the TFA. The mixture was filtered, and the solution was concentrated. DCM was added to the concentrated oil, and the mixture was cooled at 0 °C for 1hr, whereupon the solid precipitates that formed were filtered. The filtrate was concentrated to give (S)-methyl 2-amino-3-(4-methoxy-3-(4,4,5,5-tetramethyl-1,3,2-dioxaborolan-2-yl)phenyl)propanoate. The material was used without further purification.

### Example 2: Synthesis of (S)-2-((tert-butoxycarbonyl)amino)-2-(4-hydroxyphenyl)acetic acid

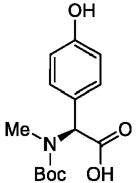

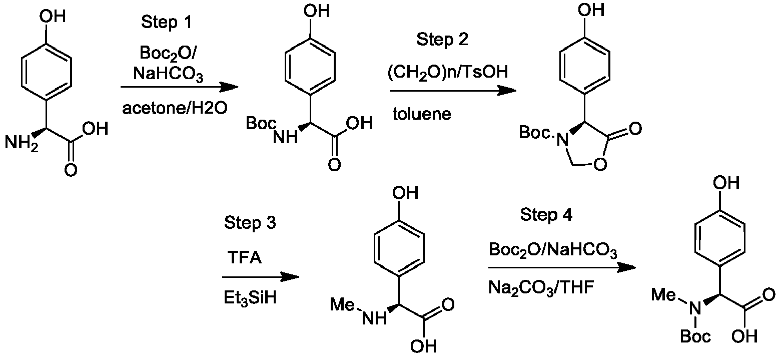

Step 1: To a stirred mixture of (S)-2-amino-2-(4-hydroxyphenyl)acetic acid (100 g, 0.6 mol, 1 eq) in a mixture of acetone (400 mL) and water (400 mL) was added di-tert-butyl dicarbonate (130.5 g, 0.6 mol, 1 eq) and NaHCO₃ (75.4 g, 0.9 mol, 1.5 eq). The mixture was allowed to stir at 25°C overnight. After HPLC showed the reaction was complete, the mixture was acidified with 5% citric acid (pH ~ 3). The mixture was filtered and the filter cake was washed with water, then dried to give (S)-2-((tert-butoxycarbonyl)amino)-2-(4-hydroxyphenyl)acetic acid (140 g, 87.5%). The crude product was used directly without further purification.

Step 2: To a solution of (S)-2-((tert-butoxycarbonyl)amino)-2-(4-hydroxyphenyl)acetic acid (45 g, 0.17 mol) in dry benzene (500 mL) was added paraformaldehyde (75.6 g, 0.84 mol, 5 eq) and *p*-toluenesulfonic acid (1.6 g, 8.5 mmol, 0.05 eq). A Dean-Stark apparatus with an attached condenser was then fit to the top of the flask and the mixture was heated at approximately 120°C until LC-MS showed the reaction was complete. The reaction was then cooled and the benzene was evaporated. The residue was taken up in ethyl acetate, washed with saturated NaHCO₃ (2 × 150 mL), then dried over sodium sulfate, and filtered. The solvent was removed to give (S)-tert-butyl 4-(4-hydroxyphenyl)-5-oxooxazolidine-3-carboxylate (36 g, 76.5%).

Step 3: (S)-tert-Butyl 4-(4-hydroxyphenyl)-5-oxooxazolidine-3-carboxylate (36 g, 0.13 mol, 1 eq) was dissolved in trifluoroacetic acid (75 mL) at 0°C then treated with triethylsilane (80 mL, 4 eq). The mixture was stirred at room temperature overnight. After LC-MS showed the reaction was complete, TFA was then evaporated to afford (S)-2-(4-hydroxyphenyl)-2-(methylamino)acetic acid, which was used without further purification.

Step 4: The resultant (S)-2-(4-hydroxyphenyl)-2-(methylamino)acetic acid was dissolved in water (85 mL), and to this solution was added solid NaHCO₃ until the pH reached 7. The solution was cooled to 0 °C, then Na₂CO₃ was added until pH reached 9. A solution of di-tert-butyldicarbonate (28.3 g, 1.0 eq) in THF (75 mL) was added to the mixture. The mixture was allowed to warm to room temperature then stirred overnight. After HPLC showed the reaction was complete, THF was then evaporated. The aqueous solution was extracted 2x with hexanes and then acidified with citric acid to pH ~3-4. The acidified solution was then extracted with ethyl acetate (200 mL × 3). The combined organic layers were washed with brine, dried over sodium sulfate, filtered and concentrated to give (S)-2-((tert-butoxycarbonyl)(methyl)amino)-2-(4-hydroxyphenyl)acetic acid (35 g, 97% via 2 steps).

### Example 3: Synthesis of Compound 101-B

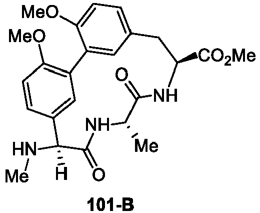

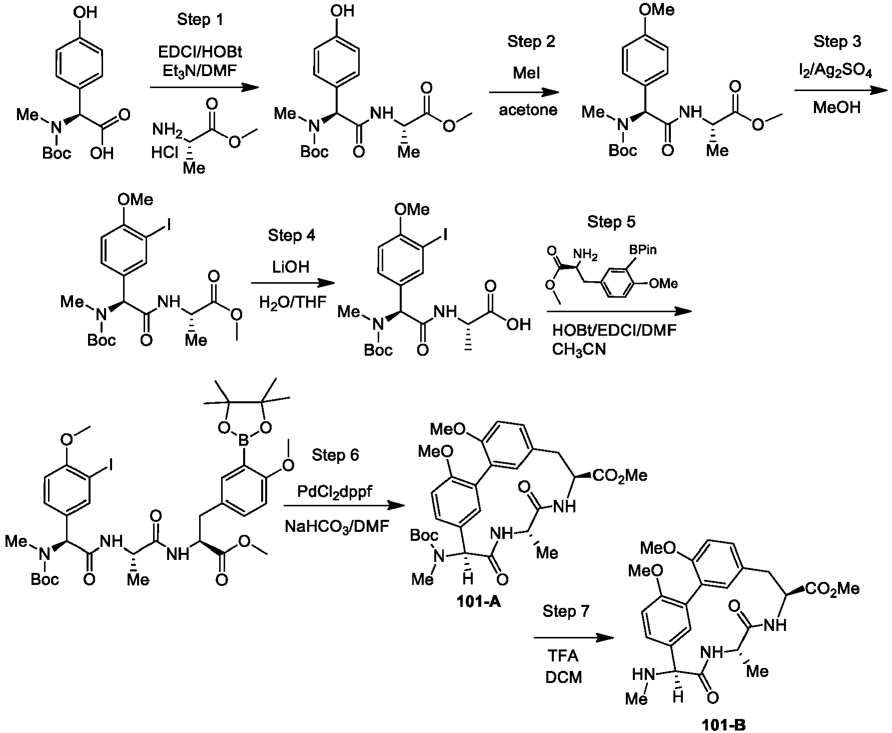

Step 1: To a solution of (S)-2-((tert-butoxycarbonyl)(methyl)amino)-2-(4-hydroxyphenyl) acetic acid (35 g, 0.12 mol) in DMF (300 mL) was added triethylamine (18.4 mL, 0.14 mol, 1.1 eq), HOBt (16.2 g, 0.12 mol, 1 eq), Ala-OMe HCl (19.5 g, 0.14 mol, 1.1 eq) and EDC (26.7 g, 0.14 mol, 1.1 eq) and the reaction was stirred overnight. After LC-MS showed the reaction was complete, water and EtOAc were added. The aqueous layer was extracted with EtOAc (3×150 mL), and the combined organic layers were washed with 5% citric acid (pH - 3), saturated NaHCO₃ (aq), water and brine. The combined organic layers were then dried over sodium sulfate, filtered and concentrated to give (S)-methyl 2-((S)-2-((tert-butoxycarbonyl)(methyl)amino)-2-(4-hydroxyphenyl)acetamido)propanoate (30 g, 65.8%) as a white foam. The crude product was taken on to the next step directly without further purification.

Step 2: To a solution of (S)-methyl 2-((S)-2-((tert-butoxycarbonyl)(methyl)amino)-2-(4-hydroxyphenyl)acetamido)propanoate (30 g, 82 mmol) in acetone (400 mL) was added K₂CO₃ (56.6 g, 0.41 mol, 5 eq) and iodomethane (20.8 mL, 0.41 mol, 5 eq) and the reaction was stirred at reflux overnight. After LC-MS showed the reaction was complete, the reaction was then cooled to room temperature and the mixture was filtered. The filtrate was concentrated and the residue was taken up in water and ethyl acetate. The aqueous phase was extracted with EtOAc (3 × 150 mL). The combined organic layers were dried over sodium sulfate, filtered and concentrated to give (S)-methyl 2-((S)-2-((tert-butoxycarbonyl)(methyl)amino)-2-(4-methoxyphenyl)acetamido)propanoate (28 g, 90%), as a white foam.

Step 3: To a solution of (S)-methyl 2-((S)-2-((tert-butoxycarbonyl)(methyl)amino)-2-(4-methoxyphenyl)acetamido)propanoate (85 g, 0.22 mol, 1 eq) in methanol (1000 mL) was added sequentially Ag₂SO₄ (72.6 g, 0.23 mol, 1.05 eq) and I₂ (59.6 g, 1.05 eq). After LC-MS showed the reaction was complete, a solution of 10% (w/w) sodium thiosulfate was added until the reaction turned pale yellow. Most of the methanol was evaporated by rotary evaporation and then water and ethyl acetate were added. The aqueous layer was extracted with ethyl acetate (3 × 300 mL). The combined organic layers were washed with brine, dried over sodium sulfate and concentrated to give (S)-methyl 2-((S)-2-((tert-butoxycarbonyl)(methyl)amino)-2-(3-iodo-4-methoxyphenyl)acetamido)propanoate (100 g, 88.5%).

Step 4: To (S)-methyl 2-((S)-2-((tert-butoxycarbonyl)(methyl)amino)-2-(3-iodo-4-methoxyphenyl)acetamido)propanoate (25 g, 49.4 mmol, 1 eq) in THF (300 mL) was added 0.2 M LiOH (500 mL, 98.8 mmol, 2 eq). The solution was stirred until TLC showed all starting material had been consumed. 5% citric acid (pH - 3) was added to pH - 3 and then the THF was evaporated by rotary evaporation. The aqueous layer was extracted with EtOAc (3 × 100 mL). The combined organic layers were washed with brine, dried over sodium sulfate, filtered and concentrated to give (S)-2-((S)-2-((tert-butoxycarbonyl)(methyl)amino)-2-(3-iodo-4-methoxyphenyl)acetamido)propanoic acid (23 g, 94.6%), which was used directly without further purification.

Step 5: To a solution of (S)-methyl 2-amino-3-(4-methoxy-3-(4,4,5,5-tetramethyl-1,3,2-dioxaborolan-2-yl)phenyl)propanoate (6.5 g, 19.4 mmol, 1 eq) and (S)-2-((S)-2-((tert-butoxycarbonyl)(methyl)amino)-2-(3-iodo-4-methoxyphenyl)acetamido)propanoic acid (10 g, 20.3 mmol, 1.05 eq) in acetonitrile:DMF (2.2:1, 168 mL) was added HOBt (6.5 g, 48.5 mmol, 2.5 eq) and EDC (8.1 g, 42.7 mmol, 2.2 eq). The reaction was stirred at room temperature overnight. After LC-MS showed the reaction was complete, diluted citric acid (pH- 3) was added and the aqueous was extracted with EtOAc (3 × 150 mL). The combined organic layers were then washed with saturated NaHCO₃ solution, brine and dried over sodium sulfate. The mixture was filtered and the filtrate was concentrated to give the crude product (6S,9S,12S)-methyl 6-(3-iodo-4-methoxyphenyl)-12-(4-methoxy-3-(4,4,5,5-tetramethyl-1,3,2-dioxaborolan-2-yl)benzyl)-2,2,5,9-tetramethyl-4,7,10-trioxo-3-oxa-5,8,11-triazatridecan-13-oate, which was used directly without further purification.

Step 6: (6S,9S,12S)-Methyl 6-(3-iodo-4-methoxyphenyl)-12-(4-methoxy-3-(4,4,5,5-tetramethyl-1,3,2-dioxaborolan-2-yl)benzyl)-2,2,5,9-tetramethyl-4,7,10-trioxo-3-oxa-5, 8,11-triazatridecan-13-oate (16 g, 19.4 mmol, 1 eq) and NaHCO₃ (16.3 g, 0.19 mol) were sealed in a flask with a condenser and put under an atmosphere of argon. DMF (600 mL) in a round bottle flask was purged several times via cycling with vacuum and Ar. PdCl₂(dppf) (3.3 g, 4.5 mmol) was then added to the DMF. The DMF solution was then degassed with Ar for 15 minutes. The solution of PdCl₂(dppf) dissolved in DMF was then transferred via syringe to the flask containing the substrate and NaHCO₃. The resulting mixture was submitted to several more cycles of vacuum and Ar then heated to 120°C overnight. After LCMS showed the reaction was completed, DMF was evaporated under vacuum. The crude material was subjected to abbreviated column chromatography (40% EA in PE) to remove most of the Pd species and then purified by prep HPLC to give Compound 101-A (2.1 g, 19.5% over two steps).

Step 7: To a stirred solution of Compound 101-A (2.1 g, 3.78 mmol) in DCM (25 mL) was added TFA (2 mL). The reaction was monitored via TLC and when starting material was consumed, the solvent was evaporated under vacuum. The residue was then dissolved in EtOAc and the organic layer was washed with saturated NaHCO₃ (10 mL), dried over sodium sulfate and concentrated to give Compound 101-B (1.7 g, 98.8%). MS (ESI) *m*/*z* 456.2 (M + H)⁺.

### Example 4: Synthesis of Compound 101-G

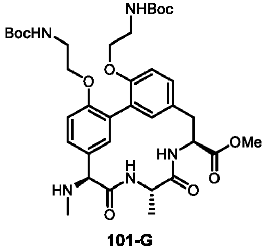

Step 1: The removal of the methoxy protecting groups is described and is referred to as General Method 1. To a solution of Compound 101-B (5.0 g, 11.0 mmol) in EtSH (116 mL, 1.61 mol), AlBr₃ (165 mL, 165 mmol) was added slowly at 0°C under N₂. The mixture was stirred for 18 h. The volatiles were removed under reduced pressure and the residue was quenched by water (50 mL), which was further washed by DCM (20 mL × 3). The aqueous layer was purified by prep-HPLC (acetonitrile 1-20% / 0.1% TFA in water) to give Compound 101-C (4.5g, 99.2% yield) as a white solid.

Step 2: To a solution of Compound 101-C (4.7 g, 8.9 mmol) in 1,4-dioxane/H₂O (9:1, 165 mL) was added 1 N NaOH dropwise until pH~11. A solution of Cbz-OSu (6.66 g, 26.7 mmol) dissolved in 1,4-dioxane (50 mL) was then added. After stirring for 1 h, NaOH (1.07 g, 26.7 mmol) was then added to the reaction followed by MeOH (60 mL). This resulting mixture was allowed to stir for 20 mins. To the reaction was then added dilute citric acid (10% v/v, 50 mL), the aqueous layer was extracted with EtOAc (3 × 150 mL) and the combined organic layers were washed with brine (3 × 100 mL), dried over Na₂SO₄ and concentrated to give the crude product. The residue was diluted with DCM (50 mL) and the suspension was filtered to give desired compound (3.2 g). The DCM phase was concentrated and the residue was purified by silica gel column (eluting 10-20% methanol in EtOAc) to give the desired compound (1.0 g). The combined batches gave Compound 101-D (4.2 g, 86.1% yield) as a white solid.

Step 3: To Compound 101-D (4.3 g, 7.85 mmol) was added a solution of 1.25M HCl in MeOH (128 mL) and the reaction was stirred at 0°C. The volatiles were removed to afford Compound 101E (4.15 g, 94.1% yield) as a white solid, which was used directly in the next step.

Step 4: The bis-alkylation of phenol groups is described and is referred to as General Method 2. To a solution of Compound 101-E (3.9 g, 6.94 mmol) and K₂CO₃ (14.4 g, 104 mmol) in DMF (50 mL) was added tert-butyl 2-bromoethylcarbamate (15.6 g, 69.5 mmol) at 0°C. The mixture was stirred at room temperature for 48 h. The mixture was filtered and the filtrate was diluted with EtOAc (500 mL). The EtOAc layer was washed with brine (2 × 400 mL), dried over Na₂SO₄, concentrated and purified by chromatography on silica (solvent gradient: 0-60% EtOAc in petroleum ether) to afford Compound 101-F (4.8 g, 81.5% yield) as a white solid.

Step 5: The hydrogenation of Cbz protecting groups is described and is referred to as General Method 3. To a solution of Compound 101-F (4.8 g, 5.7 mmol) in MeOH (100 mL), 10% Pd/C (1.26 g, 1.18 mmol) on carbon was added at room temperature. The reaction mixture was stirred for 1 h at the same temperature under hydrogen atmosphere (15 psi). The filtrate was then concentrated to afford Compound 101-G (4.0 g, 99% yield) as a white solid.

### Example 5: Synthesis of Compounds 101-1, 101-J, 101-K, and 101-L

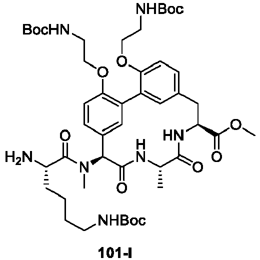

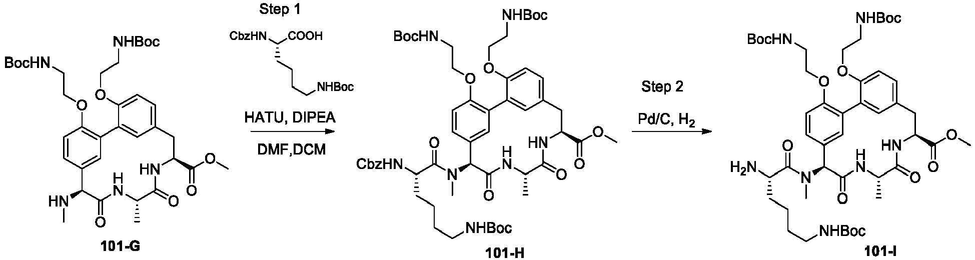

Step 1: The coupling of a Cbz-protected amino acid to an amine is described and is referred to as General Method 4. To a solution of Compound 101-G (3.5 g, 4.9 mmol) and (S)-2-(((benzyloxy)carbonyl)amino)-6-((tert-butoxycarbonyl)amino)hexanoic acid (2.4 g, 6.4 mmol) in DCM (30 mL) at 0 °C, HATU (3.7 g, 9.8 mmol) and DIPEA (1.9 g, 14.7 mmol) was added. The resulting mixture was allowed to gradually warm up to room temperature and stirred for 2 h. The reaction mixture was diluted with DCM (100 mL), which was washed with brine (100 mL × 3). The organic layer was dried over Na₂SO₄, concentrated and the residue was purified by silica column chromatography to afford Compound 101-H (5.3 g, 99% yield) as a white solid.

Step 2: The hydrogenation step was performed using General Method 3 (Example 4) using Compound 101-H (1.5 g, 1.4 mmol) to afford Compound 101-I (1.2 g, 93% yield) as a white solid. LCMS (Method 5-95 AB, ESI): t_{R} = 0.711, [M + H]⁺ = 942.6.

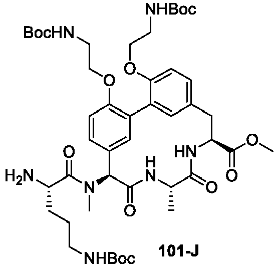

Compound 101-J was prepared from Compound 101-G and (S)-2-(((benzyloxy)carbonyl)amino)-5-((tert-butoxycarbonyl)amino)pentanoic acid using the conditions in Example 5. LCMS (Method 5-95 AB, ESI): t_{R} = 0.841, [M + H]⁺ = 928.4.

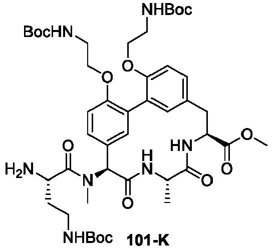

Compound 101-K was prepared from Compound 101G and (S)-2-(((benzyloxy)carbonyl)amino)-4-((tert-butoxycarbonyl)amino)butanoic acid using the conditions in Example 5. LCMS (Method 5-95 AB, ESI): t_{R} = 0.838, [M + H]⁺ = 914.5.

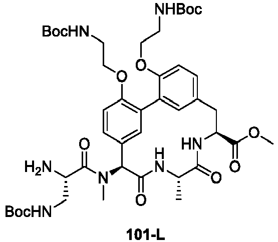

Compound 101-L was prepared from Compound 101G and (S)-2-(((benzyloxy)carbonyl)amino)-3-((tert-butoxycarbonyl)amino)propanoic acid using the conditions in Example 5. LCMS (Method 5-95 AB, ESI): t_{R} = 0.833, [M + H]⁺ = 900.5.

### Example 6: Synthesis of 3-((tert-butoxycarbonyl)(decyl)amino)propanoic acid

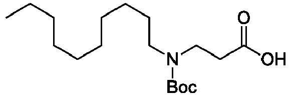

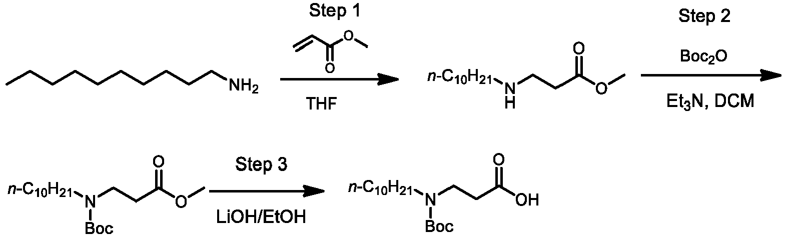

To a solution of methyl acrylate (2.2 g, 26 mmol) in THF (20 mL) was added a solution of decan-1-amine (6 g, 38 mmol) in THF (20 mL) at 0°C. The reaction mixture was stirred at 30°C for 48 h. The resulting solution was concentrated to obtain methyl 3-(decylamino)propanoate (6.4 g).

Step 2: To a solution of crude methyl 3-(decylamino)propanoate (6.4 g, 15 mmol) and Et₃N (4 g, 40 mmol) in DCM (30 mL) was added dropwise a solution of Boc₂O (5.7 g, 26 mmol) in DCM (20 mL) at 0°C. The reaction mixture was then allowed to warm to 30°C gradually and stirred for 18 h. After the reaction was completed, H₂O (50 mL) was added and the resulting aqueous layer was further extracted with DCM (50 mL*2). The combined organic layers were concentrated and the residue was purified by silica gel column (PE/EtOAc=50/1~20/1) to give methyl 3-((tert-butoxycarbonyl)(decyl)amino)propanoate (6.5 g, 73%) as a colorless oil.

Step 3: To a solution of methyl 3-((tert-butoxycarbonyl)(decyl)amino)propanoate (8.2 g, 23.9 mmol, crude) in EtOH (40 mL) was added a solution of LiOH (1.15 g, 48 mmol) in H₂O (20 mL) at 0°C. The reaction mixture was then allowed to warm to 30°C gradually and stirred for 18 h. After the reaction was complete, EtOH was removed under reduced pressure. The remaining aqueous solution was then adjusted to pH=2~3 with 6 N HCl, followed by the extraction with EtOAc (50 mL *3). The combined EtOAc layers were dried over Na₂SO₄, and concentrated to give 3-((tert-butoxycarbonyl)(decyl)amino)propanoic acid (7 g, 88.6%) as a colorless oil. ¹H NMR (400 MHz, CDCl₃) δ 3.47-3.43 (t, *J*=6.8Hz, 2H), 3.19-3.15 (t, *J*=7.2Hz, 2H), 2.61 (brs, 2H), 1.51-1.39 (m, 11H), 1.24-1.22 (m, 14H), 0.88-0.84 (t, *J*=6.8Hz, 3H).

### Example 7: Synthesis of Compound 101

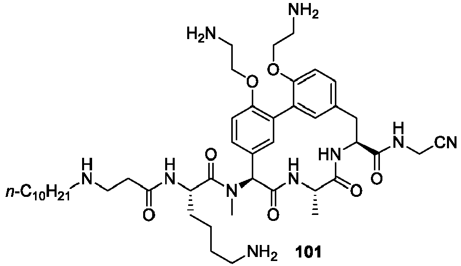

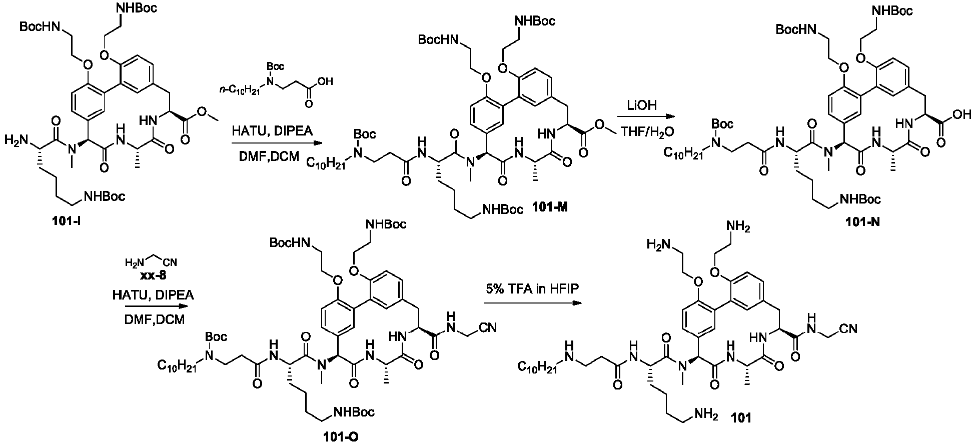

Step 1: General Method 4 (Example 5) was applied to Compound 101-I (1.0 g, 1.27 mmol) and 3-((tert-butoxycarbonyl)(decyl)amino)propanoic acid (504 mg, 1.53 mmol) to afford Compound 101-M (1.3 g, 82% yield) as a white solid.

Step 2: The lithium hydroxide hydrolysis of an ester to an acid is described and is referred to as General Method 5. To a solution of Compound 101-M (1.3 g, 1.04 mmol) in THF/H₂O (40 mL, 1:1) was added LiOH monohydrate (87 mg, 2.07 mmol) at 0°C. The mixture was allowed to gradually warm up to room temperature and stirred for 1 h. Most THF was removed under reduced pressure and the resulting mixture was adjusted pH=5 with saturated citric acid, which was further extracted by EtOAc (30 mL × 3). The combined organic layers were washed with brine (100 mL), dried over Na₂SO₄ and concentrated to give Compound 101-N (1.1 g, 86% yield) as a white solid.

Steps 3 and 4: The coupling of an aminonitrile to a carboxylic acid and subsequent Boc-deprotection is described and is referred to as General Method 6. To a solution of Compound 101-N (180 mg, 0.15 mmol), aminoacetonitrile hydrochloride (31 mg, 0.33 mmol) and DIPEA (38 mg, 0.29 mmol) in DCM/DMF (3 mL, 2:1) at 0°C, HATU (56 mg, 0.15 mmol) was added while stirring. The resulting mixture was stirred at room temperature for 1h. Most DCM was removed under reduced pressure and the residue was poured into water (10 mL), which was extracted with EtOAc (20 mL × 3). The combined organic layers were washed with brine (50 mL), dried over Na₂SO₄, concentrated to the residue, which was purified by flash chromatography column to afford Compound 101-O (140 mg, 76%) as a white solid.

Compound 101-O (130 mg, 0.10 mmol) was added to 5% TFA in HFIP (6.5 mL) and the mixture was stirred at room temperature for 2 h. Volatiles were removed under reduced pressure and the resulting crude was re-dissolved with DMF (5mL), which was neutralized with solid NaHCO₃. The filtrate was then purified by HPLC to afford Compound 101 (54 mg, 60% yield) as a white solid. LCMS (Method 5-95 AB, ESI): t_{R} = 0.710, M+H⁺ = 877.6; ¹HNMR (400 MHz, MeOH-*d*4) δ 8.51 (brs, 2H, HCOOH), 7.28-7.25 (m, 2H), 7.20 (d, *J*=8 Hz, 1H), 7.18 (d, *J*=8 Hz, 1H), 6.90 (brs, 1H), 6.84 (brs, 1H), 6.37 (s, 1H), 4.82-4.79 (m, 3H), 4.28-4.20 (m, 4H), 4.21 (s, 2H), 3.33-3.26 (m, 2H), 3.26-3.16 (m, 5H), 3.16-3.12 (m, 1H), 3.11-2.95 (m, 2H), 2.95-2.91 (m, 2H), 2.90 (s, 3H), 2.73-2.66 (m, 2H), 1.75-1.65 (m, 6H), 1.64-1.51 (m, 1H), 1.50-1.16 (m, 18H), 0.92 (t, *J*=6.8 Hz, 3H).

### Example 8: Synthesis of Compound 102

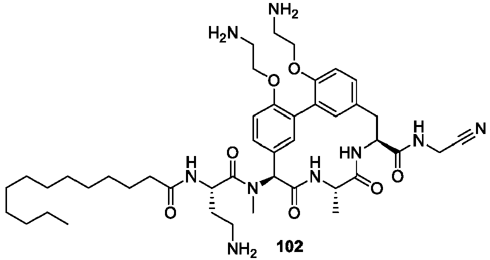

Compound 102 (formic acid salt) was prepared as a white solid utilizing the methods in Example 7 (Compound 101). LCMS (Method 5-95 AB, ESI): t_{R} = 0.766 min, [M + H]⁺ = 834.4.

### Example 9: Synthesis of Compound 103

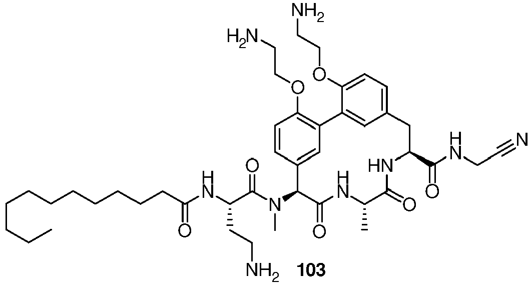

Compound 103 (formic acid salt) was prepared as a white solid utilizing the methods in Example 7 (Compound 101). LCMS (Method 5-95 AB, ESI): t_{R} = 0.747 min, [M + H]⁺ = 820.4.

### Example 10: Synthesis of Compound 104

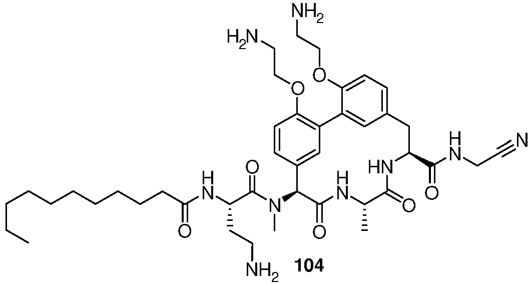

Compound 104 (formic acid salt) was prepared as a white solid utilizing the methods in Example 7 (Compound 101). LCMS (Method 5-95 AB, ESI): t_{R} = 0.725 min, [M + H]⁺ = 806.5.

### Example 11: Synthesis of Compound 105

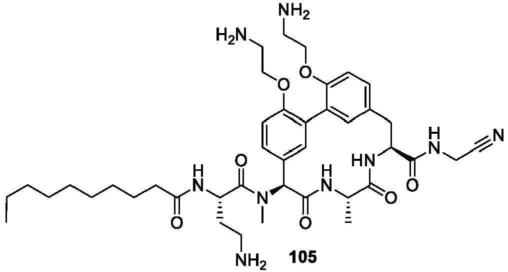

Compound 105 (formic acid salt) was prepared in 50% yield as a white solid utilizing the methods in Example 7 (Compound 101). LCMS (Method 5-95 AB, ESI): t_{R} = 0.709 min, [M + H]⁺ = 792.3.

### Example 12: Synthesis of Compound 106

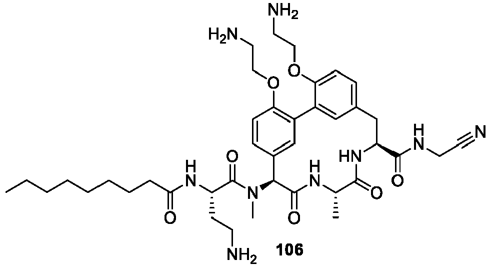

Compound 106 (formic acid salt) was prepared in 43% yield as a white solid utilizing the methods in Example 7 (Compound 101). LCMS (Method 5-95 AB, ESI): t_{R} = 0.681 min, [M + H]⁺ = 778.3.

### Example 13: Synthesis of Compound 107

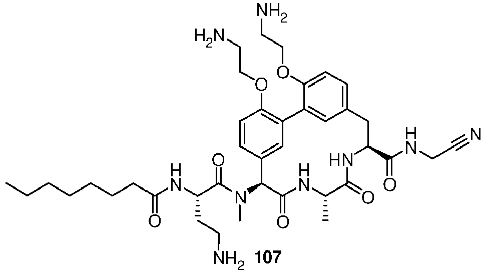

Compound 107 (formic acid salt) was prepared as a white solid utilizing the methods in Example 7 (Compound 101). LCMS (Method 5-95 AB, ESI): t_{R} = 0.652 min, [M + H]⁺ = 764.4.

### Example 14: Synthesis of Compound 108

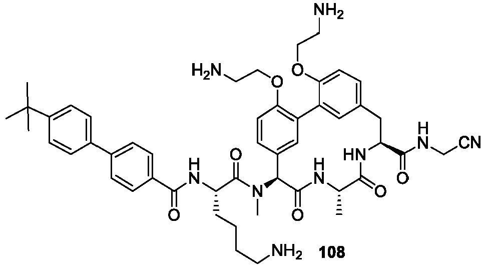

Compound 108 (formic acid salt) was prepared utilizing the methods in Example 7 (Compound 101). LC-MS: m/z = 902 [M + H]⁺; ¹H NMR (500 MHz, DMSO-*d*₆) δ 9.21 (d, *J* = 7.7 Hz, 1H), 9.00 (d, *J* = 5.1 Hz, 1H), 8.80 (dd, *J* = 23.6, 7.8 Hz, 1H), 8.39 (d, *J* = 7.9 Hz, 1H), 8.01 (dd, *J* = 15.0, 8.4 Hz, 2H), 7.78 (d, *J* = 8.4 Hz, 2H), 7.68 (d, *J* = 8.3 Hz, 2H), 7.52 (d, *J* = 8.4 Hz, 2H), 7.21 (d, *J* = 8.4 Hz, 1H), 7.15 (s, 2H), 7.04 (dd, *J* = 21.3, 7.0 Hz, 1H), 6.72 (s, 2H), 6.35 (s, 1H), 4.84 (dd, *J* = 13.5, 7.8 Hz, 1H), 4.79 - 4.74 (m, 1H), 4.69 (dd, *J* = 15.3, 8.2 Hz, 1H), 4.48 (d, *J* = 6.8 Hz, 1H), 4.17 (d, *J* = 5.6 Hz, 2H), 4.11 - 4.02 (m, 4H), 3.19 (d, *J* = 16.0 Hz, 1H), 3.01 (d, *J* = 17.3 Hz, 1H), 2.98 - 2.87 (m, 5H), 2.80 (s, 3H), 2.74 (dd, *J* = 18.2, 7.5 Hz, 2H), 1.94 - 1.72 (m, 2H), 1.68 - 1.55 (m, 2H), 1.53 - 1.37 (m, 2H), 1.33 (d, *J* = 3.7 Hz, 9H), 1.20 (d, *J* = 6.6 Hz, 3H).

### Example 15: Synthesis of Compound 109

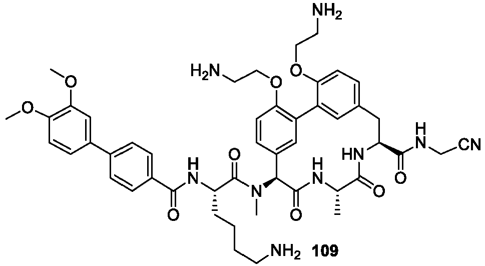

Compound 109 (formic acid salt) was prepared utilizing the methods in Example 7 (Compound 101). LC-MS: m/z = 906 [M + H]⁺; ¹H NMR (400 MHz, DMSO-*d*₆) δ 8.97 (d, *J* = 8.0 Hz, 1H), 8.76 - 8.68 (m, 2H), 8.37 (d, *J* = 9.0 Hz, 1H), 8.16 (s, 1H), 8.03 - 7.97 (m, 2H), 7.82 - 7.76 (m, 2H), 7.33 - 7.27 (m, 2H), 7.25 - 7.19 (m, 1H), 7.16 (s, 1H), 7.07 (d, *J* = 8.7 Hz, 2H), 6.73 (d, *J* = 2.1 Hz, 2H), 6.37 (s, 1H), 4.86 (q, *J=* 7.3 Hz, 1H), 4.80 - 4.67 (m, 2H), 4.18 (d, *J* = 5.9 Hz, 2H), 4.10 (dq, *J* = 12.3, 5.9, 5.1 Hz, 4H), 3.84 (d, *J* = 23.8 Hz, 6H), 3.17 (s, 1H), 3.01 (dt, *J* = 10.6, 5.6 Hz, 4H), 2.80 (s, 3H), 2.77 (d, *J* = 1.4 Hz, 2H), 1.78 (d, *J* = 7.8 Hz, 2H), 1.65 - 1.53 (m, 2H), 1.51 - 1.38 (m, 2H), 1.21 (d, *J* = 6.7 Hz, 3H).

### Example 16: Synthesis of Compound 110

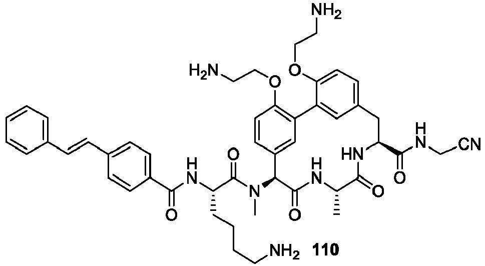

Compound 110 (formic acid salt) was prepared utilizing the methods in Example 7 (Compound 101). LC-MS: m/z = 872 [M + H]⁺; ¹H NMR (400 MHz, DMSO-*d*₆) δ 8.95 (d, *J* = 8.0 Hz, 1H), 8.70 (t, *J* = 6.5 Hz, 2H), 8.35 (d, *J* = 9.0 Hz, 1H), 7.95 (d, *J* = 8.2 Hz, 2H), 7.75 - 7.70 (m, 2H), 7.68 - 7.62 (m, 2H), 7.45 - 7.38 (m, 3H), 7.36 - 7.30 (m, 2H), 7.25 - 7.21 (m, 1 7.16 (d, *J* = 1.3 Hz, 2H), 7.08 (d, *J* = 8.5 Hz, 1H), 6.72 (s, 2H), 6.37 (s, 1H), 4.84 (d, *J* = 7.2 Hz, 1H), 4.72 (dd, *J* = 17.1, 9.2 Hz, 2H), 4.19 (d, *J* = 5.9 Hz, 2H), 4.17 - 4.06 (m, 4H), 3.18 (s, 1H), 3.05 (dt, *J* = 10.9, 5.9 Hz, 5H), 2.80 (s, 3H), 2.77 (s, 2H), 1.77 (d, *J* = 7.7 Hz, 2H), 1.59 (d, *J* = 7.3 Hz, 2H), 1.48 (s, 2H), 1.21 (d, *J* = 6.7 Hz, 3H).

### Example 17: Synthesis of Compound 111

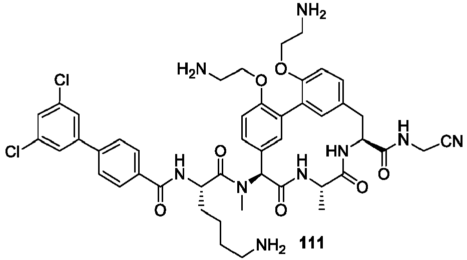

Compound 111 (formic acid salt) was prepared utilizing the methods in Example 7 (Compound 101). LC-MS: m/z = 914 [M + H]⁺; ¹H NMR (400 MHz, DMSO-*d*₆) δ 8.96 (d, *J=* 8.0 Hz, 1H), 8.83 (d, *J* = 7.8 Hz, 1H), 8.70 (t, *J* = 5.6 Hz, 1H), 8.37 (d, *J* = 9.0 Hz, 1H), 8.07 - 8.01 (m, 2H), 7.94 - 7.88 (m, 2H), 7.83 (dd, *J* = 5.6, 1.9 Hz, 2H), 7.68 (t, *J* = 1.8 Hz, 1H), 7.22 (d, *J* = 8.8 Hz, 1H), 7.15 (d, *J* = 1.3 Hz, 2H), 7.07 (d, *J* = 8.5 Hz, 1H), 6.73 (d, *J* = 1.9 Hz, 2H), 6.37 (s, 1H), 4.86 (d, *J* = 7.3 Hz, 1H), 4.72 (dd, *J* = 16.4, 9.0 Hz, 2H), 4.18 (d, *J* = 5.9 Hz, 2H), 4.15 - 4.06 (m, 4H), 3.17 (s, 1H), 3.01 (q, *J* = 5.3 Hz, 5H), 2.80 (s, 3H), 2.79 (d, *J* = 2.2 Hz, 2H), 1.83 - 1.74 (m, 2H), 1.64 - 1.55 (m, 2H), 1.46 (s, 2H), 1.20 (d, *J* = 6.7 Hz, 3H).

### Example 18: Synthesis of Compound 112

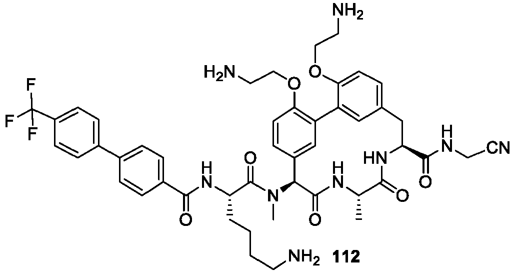

Compound 112 (formic acid salt) was prepared utilizing the methods in Example 7 (Compound 101). LC-MS: m/z = 914 [M + H]⁺; ¹H NMR (400 MHz, DMSO-*d*₆) δ 8.97 (d, *J* = 8.0 Hz, 1H), 8.82 (d, *J* = 7.8 Hz, 1H), 8.70 (t, *J* = 5.5 Hz, 1H), 8.38 (d, *J* = 9.0 Hz, 1H), 8.10 - 8.05 (m, 2H), 7.98 (d, *J* = 8.2 Hz, 2H), 7.88 (dd, *J* = 8.4, 5.5 Hz, 4H), 7.22 (dd, *J* = 8.5, 2.3 Hz, 1H), 7.15 (d, *J* = 1.4 Hz, 2H), 7.07 (d, *J* = 8.5 Hz, 1H), 6.73 (s, 2H), 6.37 (s, 1H), 4.90 - 4.82 (m, 1H), 4.80 - 4.66 (m, 2H), 4.18 (d, *J* = 5.8 Hz, 2H), 4.12 - 4.05 (m, 4H), 3.17 (s, 1H), 2.99 (dt, *J* = 9.7, 5.3 Hz, 5H), 2.81 (s, 3H), 2.81 - 2.77 (m, 2H), 1.79 (q, *J* = 7.5 Hz, 2H), 1.65 - 1.54 (m, 2H), 1.51 - 1.38 (m, 2H), 1.20 (d, *J* = 6.7 Hz, 3H).

### Example 19: Synthesis of Compound 113

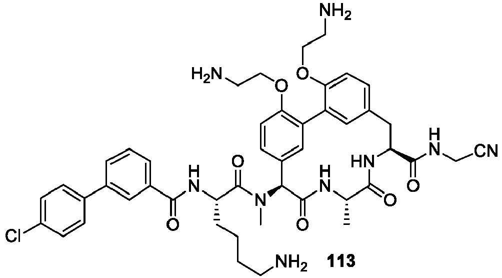

Compound 113 (formic acid salt) was prepared utilizing the methods in Example 7 (Compound 101). LC-MS: m/z = 880 [M + H]⁺; ¹H NMR (400 MHz, DMSO-*d*₆) δ 8.98 (d, *J* = 7.7 Hz, 1H), 8.92 (d, *J=* 7.9 Hz, 1H), 8.73 (t, *J* = 5.8 Hz, 1H), 8.39 (d, *J* = 9.0 Hz, 1H), 8.21 (t, *J* = 1.9 Hz, 1H), 7.93 -7.80 (m, 4H), 7.64 - 7.57 (m, 3H), 7.19 (ddd, *J* = 15.7, 8.7, 2.5 Hz, 2H), 7.04 (dd, *J* = 12.0, 8.5 Hz, 2H), 6.72 (t, *J* = 2.3 Hz, 2H), 6.36 (s, 1H), 4.93 - 4.85 (m, 1H), 4.79 - 4.68 (m, 2H), 4.18 (d, *J* = 5.7 Hz, 2H), 4.02 (dd, *J* = 11.1, 5.5 Hz, 4H), 3.19 (d, *J* = 18.3 Hz, 1H), 3.01 - 2.88 (m, 5H), 2.78 (s, 5H), 1.77 (d, *J* = 16.9 Hz, 2H), 1.59 (q, *J* = 7.4 Hz, 2H), 1.46 (s, 2H), 1.20 (d, *J* = 6.8 Hz, 3H).

### Example 20: Synthesis of Compound 114

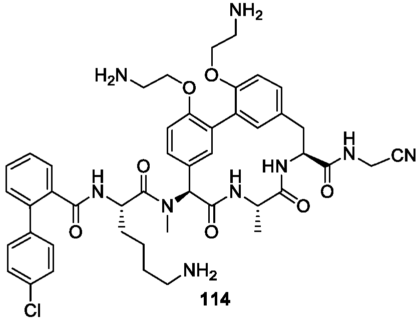

Compound 114 (formic acid salt) was prepared utilizing the methods in Example 7 (Compound 101). LC-MS: m/z = 880 [M + H]⁺; ¹H NMR (400 MHz, DMSO-*d*₆) δ 8.97 (d, *J=* 8.0 Hz, 1H), 8.79 (d, *J* = 7.5 Hz, 1H), 8.71 (t, *J* = 5.7 Hz, 1H), 8.36 (d, *J* = 9.0 Hz, 1H), 7.56 (ddd, *J* = 7.6, 6.2, 2.7 Hz, 1H), 7.50 - 7.42 (m, 7H), 7.23 (dd, *J* = 8.6, 2.3 Hz, 1H), 7.10 - 7.02 (m, 2H), 6.99 (dd, *J* = 8.7, 2.4 Hz, 1H), 6.76 - 6.69 (m, 2H), 6.47 (s, 1H), 4.81 - 4.68 (m, 2H), 4.61 - 4.54 (m, 1H), 4.19 (d, *J* = 5.9 Hz, 2H), 4.17 - 4.08 (m, 4H), 3.19 (s, 1H), 3.12 - 3.00 (m, 5H), 2.78 (d, *J* = 8.5 Hz, 2H), 2.74 (s, 3H), 1.67 - 1.49 (m, 4H), 1.38 - 1.28 (m, 2H), 1.21 (d, *J* = 6.7 Hz, 3H).

### Example 21: Synthesis of Compound 115

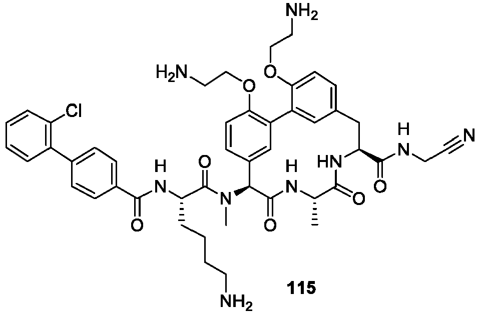

Compound 115 (formic acid salt) was prepared utilizing the methods in Example 7 (Compound 101). LC-MS: m/z = 880 [M + H]⁺; ¹H NMR (400 MHz, DMSO-*d*₆) δ 8.96 (d, *J* = 8.0 Hz, 1H), 8.81 (d, *J* = 7.9 Hz, 1H), 8.70 (t, *J* = 5.6 Hz, 1H), 8.35 (d, *J* = 8.9 Hz, 1H), 8.04 - 7.98 (m, 2H), 7.64 - 7.59 (m, 1H), 7.59 - 7.54 (m, 2H), 7.49 - 7.42 (m, 3H), 7.26 - 7.20 (m, 1H), 7.16 (d, *J* = 1.3 Hz, 2H), 7.08 (d, *J* = 8.6 Hz, 1H), 6.73 (d, *J* = 3.3 Hz, 2H), 6.39 (s, 1H), 4.90 - 4.83 (m, 1H), 4.80 - 4.67 (m, 2H), 4.19 (d, *J* = 6.0 Hz, 2H), 4.13 (dd, *J* = 10.5, 5.2 Hz, 4H), 3.18 (s, 1H), 3.06 (dq, *J* = 9.7, 5.4 Hz, 5H), 2.82 (s, 3H), 2.80 (s, 2H), 1.78 (d, *J* = 7.6 Hz, 2H), 1.59 (d, *J* = 7.1 Hz, 2H), 1.50 (s, 2H), 1.21 (d, *J* = 6.8 Hz, 3H).

### Example 22: Synthesis of Compound 116

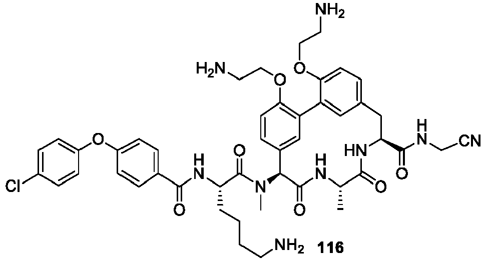

Compound 116 (formic acid salt) was prepared utilizing the methods in Example 7 (Compound 101). LC-MS: m/z = 896 [M + H]⁺; ¹H NMR (400 MHz, DMSO-*d*₆) δ 8.99 (d, *J* = 7.9 Hz, 1H), 8.74 (t, *J* = 5.6 Hz, 1H), 8.66 (d, *J* = 7.7 Hz, 1H), 8.36 (d, *J* = 8.9 Hz, 1H), 8.01 - 7.95 (m, 2H), 7.53 - 7.46 (m, 2H), 7.25 - 7.19 (m, 1H), 7.16 - 7.04 (m, 7H), 6.76 - 6.69 (m, 2H), 6.35 (s, 1H), 4.83 (t, *J* = 7.2 Hz, 1H), 4.79 - 4.67 (m, 2H), 4.18 (d, *J* = 5.9 Hz, 2H), 4.09 (dd, *J* = 10.9, 5.6 Hz, 4H), 3.19 (d, *J* = 16.6 Hz, 1H), 2.98 (dt, *J* = 11.0, 5.4 Hz, 5H), 2.79 (s, 3H), 2.78 (s, 2H), 1.75 (q, *J* = 7.6 Hz, 2H), 1.58 (dq, *J* = 15.2, 7.4 Hz, 2H), 1.44 (dq, *J* = 15.0, 7.3 Hz, 2H), 1.20 (d, *J* = 6.7 Hz, 3H).

### Example 23: Synthesis of Compound 117

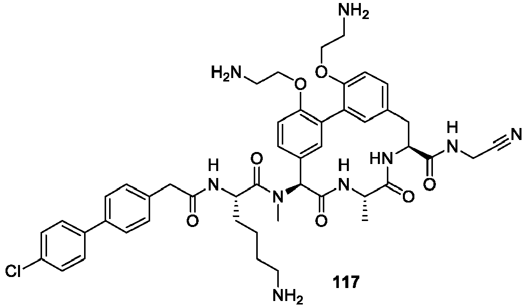

Compound 117 (formic acid salt) was prepared utilizing the methods in Example 7 (Compound 101). LC-MS: m/z = 894 [M + H]⁺.

### Example 24: Synthesis of Compound 118

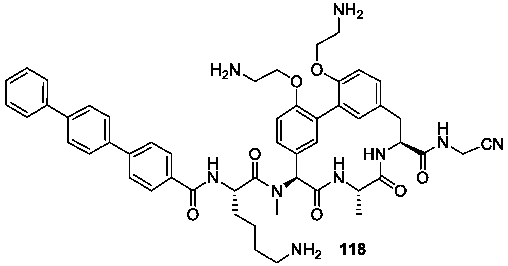

Compound 118 (formic acid salt) was prepared utilizing the methods in Example 7 (Compound 101). LC-MS: m/z = 922 [M + H]⁺.

### Example 25: Synthesis of Compound 119

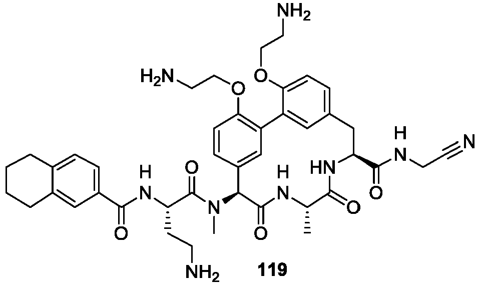

Compound 119 (formic acid salt) was prepared utilizing the methods in Example 7 (Compound 101). LC-MS: m/z = 796 [M + H]⁺.

### Example 26: Synthesis of Compound 120

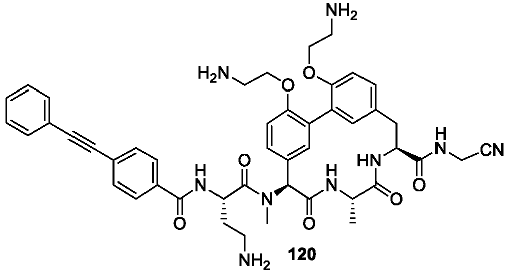

Compound 120 (formic acid salt) was prepared utilizing the methods in Example 7 (Compound 101). LC-MS: m/z = 842 [M + H]⁺; ¹H NMR (400 MHz, DMSO-*d*₆) δ 9.07 (d, *J* = 7.8 Hz, 1H), 9.01 (d, *J* = 7.3 Hz, 1H), 8.82 (t, *J* = 5.6 Hz, 1H), 8.38 (d, *J* = 8.9 Hz, 1H), 7.99 (d, *J* = 8.5 Hz, 2H), 7.69 (d, *J* = 8.4 Hz, 2H), 7.63 - 7.57 (m, 2H), 7.49 - 7.44 (m, 3H), 7.22 - 7.08 (m, 3H), 7.04 (d, *J* = 8.5 Hz, 1H), 6.72 (d, *J* = 2.5 Hz, 2H), 6.30 (s, 1H), 4.96 (s, 1H), 4.74 (dt, *J* = 15.5, 8.9 Hz, 2H), 4.17 (d, *J* = 5.8 Hz, 2H), 4.00 (dd, *J* = 11.9, 6.4 Hz, 4H), 3.18 (d, *J* = 16.0 Hz, 1H), 3.05 - 2.96 (m, 1H), 2.88 (d, *J* = 8.9 Hz, 6H), 2.77 (s, 3H), 2.01 (s, 2H), 1.19 (d, *J* = 6.7 Hz, 3H).

### Example 27: Synthesis of Compound 121

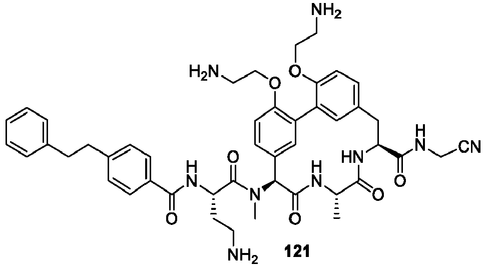

Compound 121 (formic acid salt) was prepared utilizing the methods in Example 7 (Compound 101). LC-MS: m/z = 846 [M + H]⁺.

### Example 28: Synthesis of Compound 122

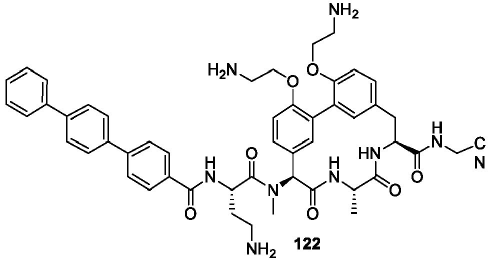

Compound 122 (formic acid salt) was prepared utilizing the methods in Example 7 (Compound 101). LC-MS: m/z = 894 [M + H]⁺; ¹H NMR (400 MHz, DMSO-*d*₆) δ 8.98 (dd, *J* = 7.6, 4.4 Hz, 2H), 8.70 (t, *J* = 5.6 Hz, 1H), 8.42 (d, *J* = 8.8 Hz, 1H), 8.05 (d, *J* = 8.5 Hz, 2H), 7.91 - 7.79 (m, 5H), 7.77 - 7.73 (m, 2H), 7.54 - 7.49 (m, 2H), 7.44 - 7.38 (m, 2H), 7.22 (dt, *J* = 8.6, 2.4 Hz, 1H), 7.16 (d, *J* = 8.6 Hz, 2H), 7.07 (d, *J* = 8.5 Hz, 1H), 6.73 (t, *J* = 2.3 Hz, 2H), 6.33 (s, 1H), 5.02 (q, *J* = 7.3 Hz, 1H), 4.79 - 4.70 (m, 2H), 4.18 (d, *J* = 5.9 Hz, 2H), 4.13 - 4.05 (m, 4H), 3.20 (d, *J* = 18.2 Hz, 1H), 3.06 - 2.92 (m, 9H), 2.77 (s, 3H), 2.07 (dd, *J* = 20.0, 12.5 Hz, 2H), 1.21 (d, *J* = 6.7 Hz, 3H).

### Example 29: Synthesis of Compound 123

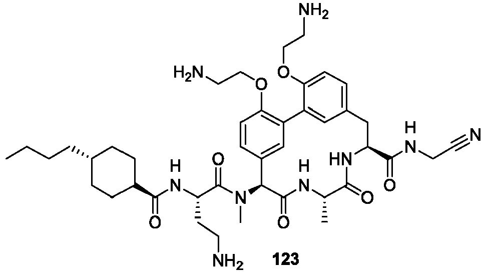

Compound 123 (formic acid salt) was prepared utilizing the methods in Example 7 (Compound 101). LC-MS: m/z = 804 [M + H]⁺.

### Example 30: Synthesis of Compound 124

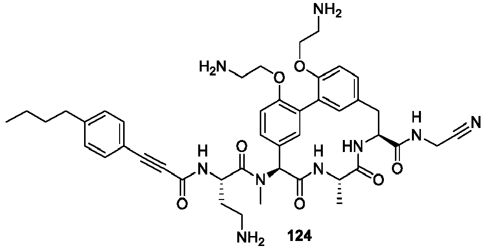

Compound 124 (formic acid salt) was prepared utilizing the methods in Example 7 (Compound 101). LC-MS: m/z = 822 [M + H]⁺; ¹H NMR (400 MHz, DMSO-*d*₆) δ 9.28 (d, *J* = 7.5 Hz, 1H), 8.96 (d, *J* = 7.9 Hz, 1H), 8.70 (t, *J* = 5.7 Hz, 1H), 8.39 (d, *J* = 9.0 Hz, 1H), 7.51 (d, *J* = 8.2 Hz, 2H), 7.34 - 7.29 (m, 2H), 7.22 (dd, *J* = 8.7, 2.3 Hz, 1H), 7.18 - 7.12 (m, 2H), 7.08 (d, J = 8.5 Hz, 1H), 6.72 (d, J = 2.4 Hz, 2H), 6.34 (s, 1H), 4.83 (s, 1H), 4.73 (td, *J* = 15.9, 14.1, 9.1 Hz, 2H), 4.18 (d, *J* = 5.8 Hz, 2H), 4.16 - 4.06 (m, 4H), 3.19 (d, *J* = 16.2 Hz, 1H), 3.01 (dt, *J* = 10.3, 5.4 Hz, 5H), 2.88 (dd, *J* = 10.0, 6.0 Hz, 2H), 2.77 (s, 3H), 2.67 - 2.59 (m, 2H), 2.05 - 1.88 (m, 2H), 1.61 - 1.53 (m, 2H), 1.35 - 1.26 (m, 2H), 1.20 (d, *J* = 6.7 Hz, 3H), 0.90 (t, *J* = 7.3 Hz, 3H).

### Example 31: Synthesis of Compound 125

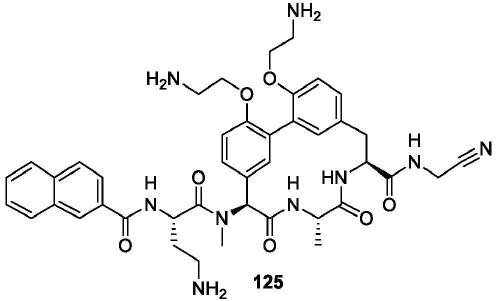

Compound 125 (formic acid salt) was prepared utilizing the methods in Example 7 (Compound 101). LC-MS: m/z = 792 [M + H]⁺; ¹H NMR (400 MHz, DMSO-*d*₆) δ 9.08 (d, *J* = 7.8 Hz, 1H), 8.98 (d, *J* = 7.8 Hz, 1H), 8.71 (t, *J* = 5.6 Hz, 1H), 8.42 (d, *J* = 9.0 Hz, 1H), 8.08 - 7.95 (m, 4H), 7.64 (ddt, *J* = 10.3, 5.2, 3.7 Hz, 3H), 7.23 (t, *J* = 9.0 Hz, 1H), 7.15 (s, 2H), 7.06 (d, *J* = 8.8 Hz, 1H), 6.73 (d, *J* = 4.0 Hz, 2H), 6.33 (d, *J* = 0.9 Hz, 1H), 5.04 (t, *J* = 7.1 Hz, 1H), 4.80 - 4.70 (m, 2H), 4.18 (d, *J* = 5.9 Hz, 2H), 4.08 (s, 4H), 3.19 (d, *J* = 16.4 Hz, 1H), 2.98 (d, *J* = 16.3 Hz, 5H), 2.78 (s, 3H), 2.68 - 2.64 (m, 2H), 2.17 - 2.01 (m, 2H), 1.21 (d, *J=* 6.7 Hz, 3H).

### Example 32: Synthesis of Compound 126

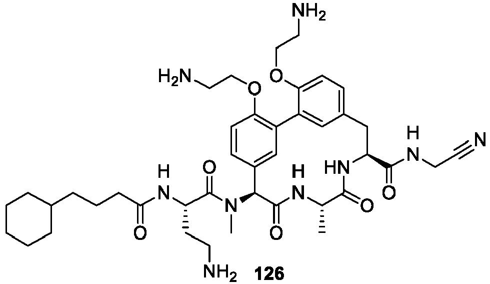

Compound 126 (formic acid salt) was prepared utilizing the methods in Example 7 (Compound 101). LC-MS: m/z = 790 [M + H]⁺.

### Example 33: Synthesis of Compound 127

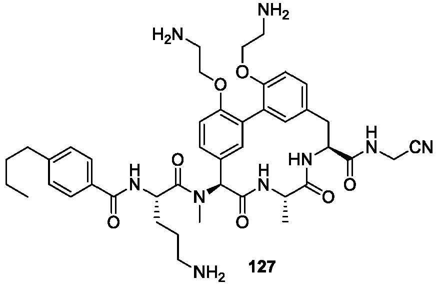

Compound 127 (formic acid salt) was prepared as a white solid utilizing the methods in Example 7 (Compound 101) from Compound 101-J and 4-butylbenzoic acid. LCMS (Method 5-95 AB, ESI): t_{R} = 0.545, [M + H]⁺ = 812.2.

### Example 34: Synthesis of Compound 128

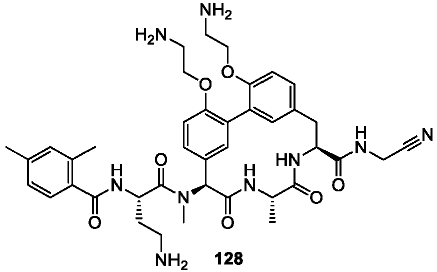

Compound 128 (formic acid salt) was prepared as a white solid utilizing the methods in Example 7 (Compound 101). LCMS (Method 0-60AB): t_{R} = 0.850 minI 2 min, [M+H]⁺ = 770.5.

### Example 35: Synthesis of Compound 129

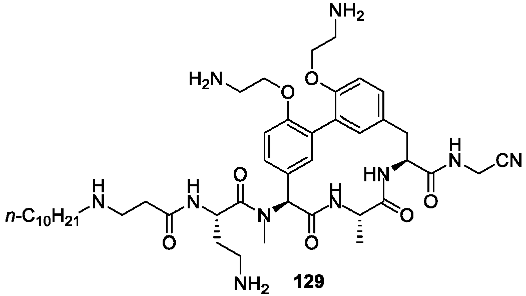

Compound 129 (formic acid salt) was prepared as a white solid utilizing the methods in Example 7 (Compound 101) from 3-((tert-butoxycarbonyl)(decyl)amino)propanoic acid. LCMS (Method 5-95 AB, ESI): t_{R} = 0.557, [M + H]⁺ = 849.5; ¹H NMR (400 MHz, MeOH-*d*₄) δ 8.49 (brs, 2H, HCOOH), 7.29-7.24 (m, 2H), 7.17 (d, *J*=8 Hz, 1H), 7.10 (d, *J*=8 Hz, 1H), 6.88 (brs, 1H), 6.81 (brs, 1H), 6.29 (s, 1H), 4.91-4.76 (m, 3H), 4.27-4.15 (m, 4H), 4.19 (s, 2H), 3.36-3.23 (m, 6H), 3.17-3.01 (m, 6H), 2.86 (s, 3H), 2.76-2.71 (m, 2H), 2.20-2.10 (m, 1H), 2.05-1.95 (m, 1H), 1.71 (brs, 2H), 1.38-1.31 (m, 17H), 0.90 (t, *J*=6.4 Hz, 3H).

### Example 36: Synthesis of Compound 130

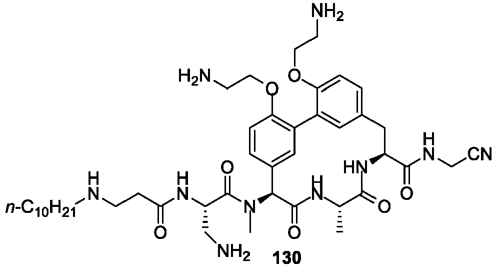

Compound 130 (formic acid salt) was prepared as a white solid utilizing the methods in Example 7 (Compound 101). LCMS (Method 5-95 AB, ESI): t_{R} = 0.699, [M + H]⁺ = 835.6; ¹H NMR (400 MHz, MeOH-*d*₄) δ 8.45 (brs, 1H), 7.34-7.20 (m, 2H), 7.16 (d, *J*=8.5 Hz, 1H), 7.10 (d, *J*=8.5 Hz, 1H), 6.88 (brs, 1H), 6.80 (brs, 1H), 6.22 (s, 1H), 5.21-5.13 (m, 1H), 4.86-4.79 (m, 2H), 4.30-4.17 (m, 4H), 4.19 (s, 2H), 3.41-3.32 (m, 3H), 3.29-3.22 (m, 5H), 3.22-3.09 (m, 2H), 3.06-2.99 (m, 2H), 2.80 (s, 3H), 1.77-1.65 (m, 2H), 1.46 - 1.23 (m, 19H), 0.90 (t, *J*=6.8 Hz, 3H).

### Example 37: Synthesis of Compound 131

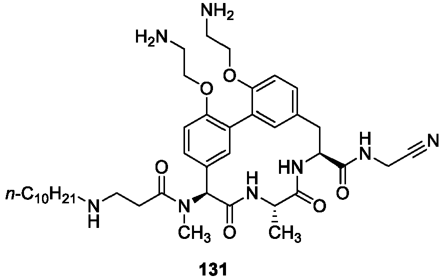

Compound 131 (formic acid salt) was prepared as a white solid utilizing the methods in Example 7 (Compound 101) from 3-((tert-butoxycarbonyl)(decyl)amino)propanoic acid and Compound 101-G. LCMS (Method 5-95 AB, ESI): t_{R} = 0.585, [M + H]⁺ = 749.3; ¹H NMR (400 MHz, MeOH-*d*₄) δ 8.47 (brs, 2H, HCOOH),7.29-7.23(m, 2H), 7.17 (d, *J*=8.0 Hz, 1H), 7.09 (d, *J*=8.0 Hz, 1H), 6.87 (brs, 1H), 6.80 (brs, 1H), 6.31 (s, 1H), 4.81-4.76 (m, 2H), 4.26-4.18 (m, 4H), 4.21 (s, 2H), 3.34-3.28 (m, 2H), 3.25-3.15 (m, 4H), 3.15-3.10 (m, 2H), 3.07-3.03 (m, 2H), 2.94-2.90 (m, 2H), 2.78 (s, 3H), 1.74-1.70 (m, 2H), 1.39-1.32 (m, 17H), 0.90 (t, *J*=6.4 Hz, 3H).

### Example 38: Synthesis of Compound 132

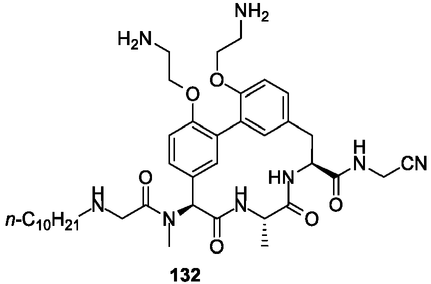

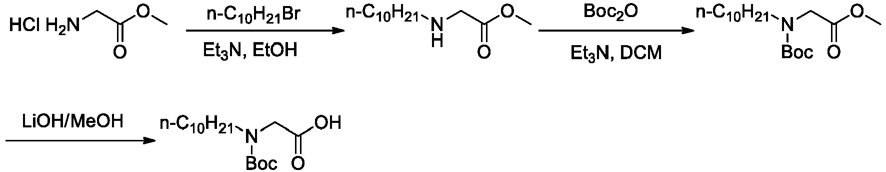

To a solution of decan-1-amine (10.5 g, 66.8 mmol) in anhydrous dichloromethane (250 mL) was added triethylamine (13.5 g, 133.5 mmol) at 0oC and the reaction mixture was stirred at 0°C for 30 min. Methyl bromoacetate (10.2 g, 66.8 mmol) was then added dropwise at 0°C, and the reaction mixture was stirred at room temperature for 14h. The solution containing methyl 2-(decylamino)acetate was used directly for the next step.

The N-Boc formation and LiOH ester hydrolysis was performed in a manner similar to Example 6 to afford 1.1 g of 2-((tert-butoxycarbonyl)(decyl)amino)acetic acid. ¹H NMR (400 MHz, CDCl₃) δ 3.96 (s, 1H), 3.89 (s, 1H), 3.25-3.23 (m, 2H), 1.50-1.41 (m, 11H), 1.25 (m, 14H), 0.88-0.85 (t, J=6.8Hz, 3H).

Compound 132 (formic acid salt) was prepared as a white solid utilizing the methods in Example 37 (Compound 131) from 2-((tert-butoxycarbonyl)(decyl)amino)acetic acid and Compound 101-G. LCMS (Method 5-95 AB, ESI): t_{R} = 0.627, [M + H]⁺ = 735.2.

### Example 39: Synthesis of Compound 133

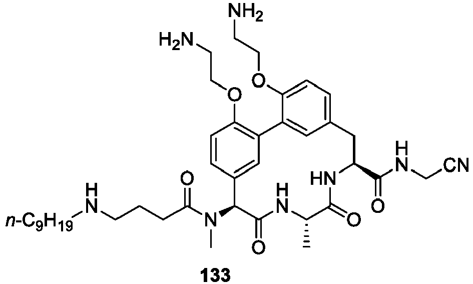

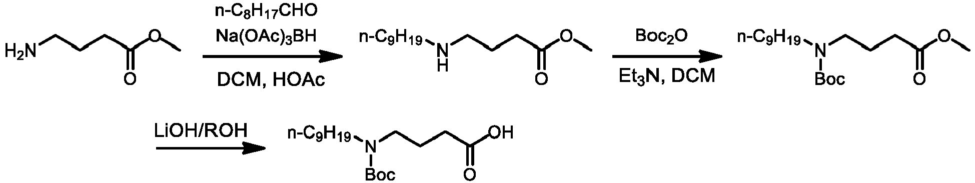

To a solution of nonanal (600 mg, 4.22 mmol) in DCM (25 mL) at 0°C was added methyl 4-aminobutanoate (988 mg, 8.44 mmol) and HOAc (1 mL), followed by the addition of NaBH₃CN (398 mg, 2 mmol). The mixture was stirred at 15°C for 12h. After the reaction was complete, H₂O (20 mL) was added and the aqueous layer was extracted by DCM (30 mL*2). The combined organic layers were concentrated to obtain methyl 4-(nonylamino) butanoate. LCMS (Method 5-95 AB, ESI): t_{R} = 0.770, [M + H]⁺ = 244.0

The N-Boc formation and LiOH ester hydrolysis was performed in a manner similar to Example 6 to afford 0.46 g of 4-((tert-butoxycarbonyl)(nonyl)amino)butanoic acid. ELSD-LC/MS 352.3 [M+Na⁺].

Compound 133 (formic acid salt) was prepared as a white solid utilizing the methods in Example 37 (Compound 131). LCMS (Method 5-95 AB, ESI): t_{R} = 0.717, [M + H]⁺ = 749.5; ¹H NMR (400 MHz, MeOH-*d*₄) δ 8.48 (brs, 3H, HCOOH), 7.29-7.20 (m, 2H), 7.16 (d, *J*=8.4 Hz, 1H), 7.09 (d, *J*=8.4 Hz, 1H), 6.91(brs, 1H), 6.77 (brs, 1H), 6.30 (s, 1H), 4.82-4.74 (m, 2H), 4.29-4.12 (m, 4H), 4.19 (s, 2H), 3.38-3.33 (m, 1H), 3.24-3.05 (m, 7H), 3.04-2.95 (m, 3H), 2.78 (s, 3H), 2.73-2.57 (m, 3H), 2.07-1.94 (m, 2H), 1.74-1.63 (m, 2H), 1.50-1.23 (m, 15H), 0.90 (t, J=6.6 Hz, 3H).

### Example 40: Synthesis of Compound 134

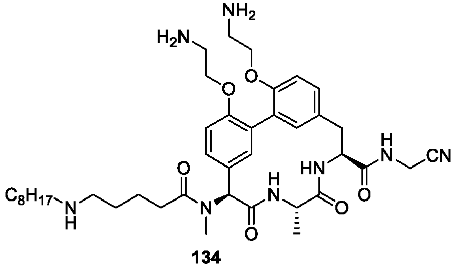

Compound 134 (formic acid salt) was prepared as a white solid utilizing the methods in Example 39 (Compound 133). LCMS (Method 5-95 AB, ESI): t_{R} = 0.562, M+H⁺ = 749.3.

### Example 41: Synthesis of Compound 135

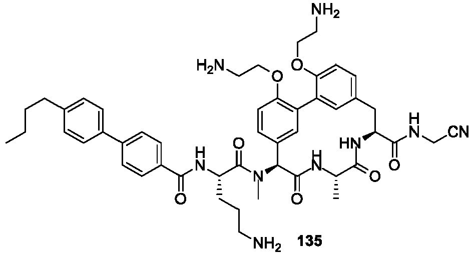

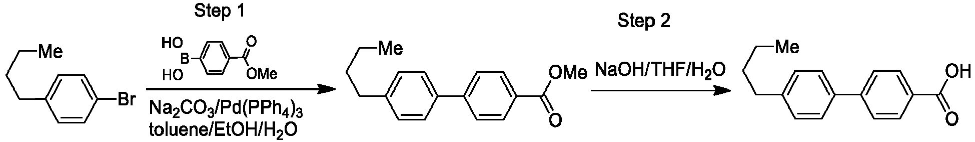

Step 1: A solution of 1-bromo-4-*n*-butylbenzene (100 g, 0.472 mol), 4-(methoxycarbonyl)benzeneboronic acid (82.0 g, 0.456 mol), 2 M Na₂CO₃ (150 g, 1.42 mol) in toluene/EtOH (900 mL/300 mL) was degassed with N₂ three times, then Pd(PPh₃)₄ (27.2 g, 23.6 mmol) was added. The resulting mixture was degassed with N₂ three times and then heated to reflux for 5h. After TLC showed the reaction was complete, toluene and EtOH was removed under vacuum. The residue was extracted with EA (3x). The combined organic layers were washed with brine and dried with Na₂SO₄. The solvent was removed to give the crude product. The crude product was purified by column chromatography on silica gel eluted with PE: EA (150:1). The solvent was removed to give methyl 4'-butyl-[1,1'-biphenyl]-4-carboxylate (105 g, 86.0%) as a white solid.

Step 2: A mixture of methyl 4'-butyl-[1,1'-biphenyl]-4-carboxylate (89.0 g, 0.332 mol), NaOH (26.6 g, 0.664 mol) in THF/H₂O (500 mL/100 mL) was heated to reflux overnight. After TLC showed the reaction was complete, THF was removed. The residue was adjusted pH=3~4 with 2 N HCl solution. The resulting mixture was filtered and the cake was washed with water, and dried to give 4'-butyl-[1,1'-biphenyl]-4-carboxylic acid (60.0 g, 71.1%) as a white solid.

Compound 135 (formic acid salt) was prepared as a white solid utilizing the methods in Example 7 (Compound 101) from Compound 101-J and 4'-butyl-[1,1'-biphenyl]-4-carboxylic acid. LCMS (Method 5-95 AB, ESI): t_{R} = 0.757, [M + H]⁺ = 888.4; ¹H NMR (400 MHz, MeOH-*d*₄) δ 8.47 (brs, 1H, HCOOH), 7.91 (d, *J*=8 Hz, 2H), 7.71 (d, *J*=8.0 Hz, 2H), 7.53 (d, *J*=8 Hz, 2H), 7.36-7.20 (m, 4H), 7.25-7.18 (m, 2H), 6.90 (brs, 1H), 6.81 (brs, 1H), 6.50 (s, 1H), 5.10-5.05 (m, 1H), 4.85-4.72 (m, 2H), 4.32-4.20 (m, 4H), 4.21 (s, 2H), 3.35-3.10 (m, 6H), 3.08-2.95 (m, 2H), 2.92 (s, 3H), 2.70-2.60 (m, 2H), 2.05-1.75 (m, 4H), 1.70-1.55 (m, 2H), 1.45-1.30 (m, 2H), 1.37 (d, J=7.2 Hz, 3H), 0.95 (t, J=6.8 Hz, 3H).

### Example 42: Synthesis of Compound 136

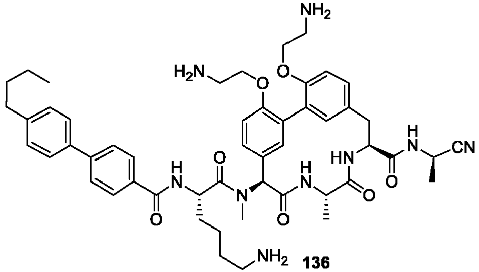

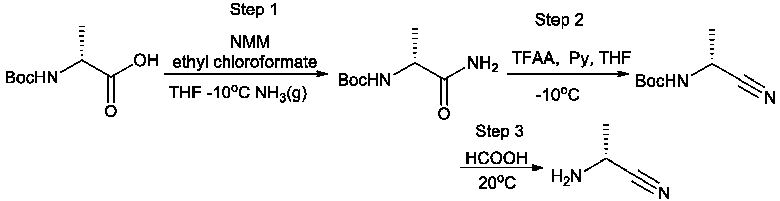

Step 1: To a stirred solution of (R)-2-((tert-butoxycarbonyl)amino)propanoic acid (10.0 g, 52.8 mmol), NMM (5.88 g, 58.1 mmol) in THF (50 mL) was added ethyl chloroformate (8.86 g, 81.6 mmol) dropwise at -10°C and the mixture was stirred at the same temperature for 20 mins, followed by the addition of 10N ammonia in THF (50 mL, 500 mmol) slowly. The resulting mixture was stirred at -10°C for another 2 h. The volatiles were removed and the residue was taken up with EtOAc (100 mL), which was washed with 1N KHSO₄ solution and brine (50 mL each). The organic layer was dried over Na₂SO₄ and concentrated to give (R)-tert-butyl (1-amino-1-oxopropan-2-yl)carbamate (6.5 g, 65.3% yield) as a white solid.

Step 2: To a solution of (R)-tert-butyl (1-amino-1-oxopropan-2-yl)carbamate (3.5 g, 18.6 mmol) and pyridine (4.4 g, 55.8 mmol) in anhydrous THF (50 mL) was added TFAA (5.9 g, 27.9 mmol) dropwise at -10°C and the mixture was stirred at the same temperature for 2 h. The volatiles were removed and the residue was taken up with EtOAc (100 mL), which was washed with 1N KHSO₄ solution and brine (50 mL each). The organic layer was dried over Na₂SO₄ and concentrated to crude product, which was further re-crystallized using 100 mL (20% EtOAc in petroleum ether) to afford (R)-tert-butyl (1-cyanoethyl)carbamate (1.5 g, 47.4% yield) as a white solid. ¹H NMR (400 MHz, CDCl₃) δ 4.82 (brs, 1H), 4.61 (brs, 1H), 1.53 (d, *J*=7.6 Hz, 3H), 1.45 (s, 9H).

Step 3: (R)-Tert-butyl (1-cyanoethyl)carbamate (100 mg, 0.59 mmol) was added in portions into HCOOH (1.0 mL) at 0°C and the mixture was allowed to warm to 20°C while stirring and stirred at the same temperature for 3 h. The volatiles were removed to afford (R)-2-aminopropanenitrile (65 mg, 95.3% yield) as a white solid, which was used directly in the next step.

Compound 136 (formic acid salt) was prepared utilizing the methods in Example 41 (Compound 135) except (R)-2-aminopropanenitrile hydrochloride is used in the coupling step. LC-MS: m/z = 880 [M + H]⁺; ¹H NMR (400 MHz, DMSO-*d*₆) δ 8.91 (d, *J* = 7.9 Hz, 1H), 8.82 (d, *J* = 7.5 Hz, 1H), 8.74 (d, *J* = 7.9 Hz, 1H), 8.37 (d, *J* = 8.9 Hz, 1H), 8.01 (d, *J* = 8.4 Hz, 2H), 7.80 - 7.75 (m, 2H), 7.69 - 7.63 (m, 2H), 7.33 (d, *J* = 8.1 Hz, 2H), 7.19 (d, *J* = 8.6 Hz, 1H), 7.15 (s, 2H), 7.09 - 7.02 (m, 1H), 6.74 (s, 2H), 6.37 (s, 1H), 4.84 (dd, *J* = 16.0, 7.4 Hz, 2H), 4.72 (d, *J* = 12.6 Hz, 2H), 4.05 (s, 4H), 2.94 (s, 5H), 2.80 (s, 3H), 2.78 (d, *J* = 7.9 Hz, 2H), 2.65 - 2.62 (m, 2H), 1.78 (s, 2H), 1.60 (t, *J* = 7.6 Hz, 4H), 1.44 (d, *J* = 7.2 Hz, 5H), 1.38 - 1.31 (m, 2H), 1.20 (d, *J* = 6.7 Hz, 3H), 0.96 - 0.88 (m, 3H).

### Example 43: Synthesis of Compound 137

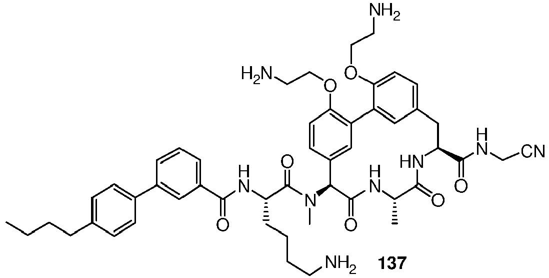

Compound 137 (formic acid salt) was prepared utilizing the methods in Example 41 (Compound 135). LC-MS: m/z = 903 [M + H]⁺; ¹H NMR (400 MHz, DMSO-*d*₆) δ 9.00 (d, *J* = 7.8 Hz, 1H), 8.89 (d, *J* = 8.0 Hz, 1H), 8.74 (t, *J* = 5.7 Hz, 1H), 8.39 (d, *J* = 9.0 Hz, 1H), 8.20 (t, *J* = 1.8 Hz, 1H), 7.90 - 7.81 (m, 2H), 7.72 - 7.66 (m, 2H), 7.57 (t, *J* = 7.8 Hz, 1H), 7.35 (d, *J* = 8.2 Hz, 2H), 7.18 (ddd, *J* = 16.6, 8.6, 2.5 Hz, 2H), 7.03 (t, *J* = 8.7 Hz, 2H), 6.72 (d, *J* = 2.6 Hz, 2H), 6.35 (s, 1H), 4.88 (q, *J* = 7.5, 7.0 Hz, 1H), 4.79 - 4.68 (m, 2H), 4.18 (d, *J* = 5.8 Hz, 2H), 3.99 (dt, *J* = 10.6, 5.5 Hz, 4H), 3.18 (d, *J* = 17.8 Hz, 1H), 3.02 (d, *J* = 12.1 Hz, 1H), 2.89 (q, *J* = 5.6, 4.3 Hz, 4H), 2.78 (s, 3H), 2.76 (s, 2H), 2.68 - 2.62 (m, 2H), 1.77 (d, *J* = 13.1 Hz, 2H), 1.65 - 1.55 (m, 4H), 1.45 (s, 4H), 1.40 - 1.29 (m, 2H), 1.20 (d, *J* = 6.7 Hz, 3H), 0.92 (t, *J* = 7.3 Hz, 3H).

### Example 44: Synthesis of Compound 138

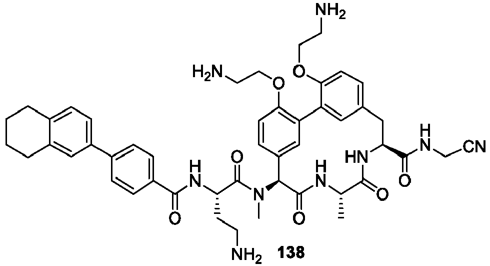

Compound 138 (formic acid salt) was prepared utilizing the methods in Example 41 (Compound 135). LC-MS: m/z = 872 [M + H]⁺; ¹H NMR (500 MHz, DMSO-*d*₆) δ 9.09 (t, *J* = 7.7 Hz, 1H), 8.98 (d, *J* = 7.6 Hz, 1H), 8.89 - 8.79 (m, 1H), 8.44 (d, *J* = 9.0 Hz, 1H), 8.01 (d, *J* = 8.4 Hz, 2H), 7.77 (d, *J* = 8.4 Hz, 2H), 7.47 - 7.42 (m, 2H), 7.24 - 7.12 (m, 5H), 7.07 (d, *J* = 8.6 Hz, 1H), 6.75 - 6.71 (m, 2H), 6.31 (s, 1H), 5.02 - 4.94 (m, 1H), 4.79 - 4.68 (m, 2H), 4.18 (d, *J* = 6.5 Hz, 2H), 4.08 (dq, *J* = 26.7, 10.5, 7.8 Hz, 4H), 3.19 (d, *J* = 15.8 Hz, 1H), 3.08 - 2.88 (m, 7H), 2.83 - 2.79 (m, 2H), 2.76 (s, 5H), 2.14 - 1.98 (m, 2H), 1.79 - 1.70 (m, 4H), 1.21 (d, *J* = 6.7 Hz, 3H).

### Example 45: Synthesis of Compound 139

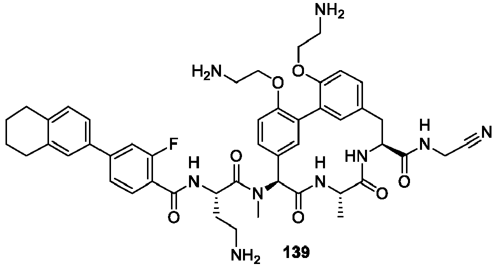

Compound 139 (formic acid salt) was prepared utilizing the methods in Example 41 (Compound 135). LC-MS: m/z = 890 [M + H]⁺.

### Example 46: Synthesis of Compound 140

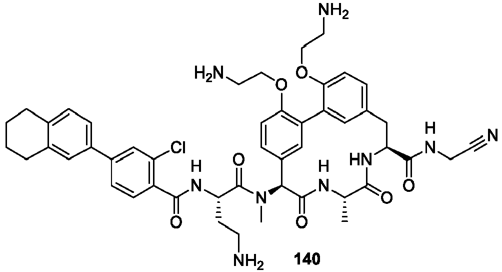

Compound 140 (formic acid salt) was prepared utilizing the methods in Example 41 (Compound 135). LC-MS: m/z = 906 [M + H]⁺; ¹H NMR (400 MHz, DMSO-*d*₆) δ 9.00 (t, *J* = 8.7 Hz, 1H), 8.74 (t, *J* = 5.6 Hz, 1H), 8.39 (d, *J* = 9.0 Hz, 1H), 7.77 (d, *J* = 1.7 Hz, 1H), 7.70 (dd, *J* = 8.0, 1.8 Hz, 2H), 7.50 (d, *J* = 8.0 Hz, 2H), 7.44 (d, *J* = 6.6 Hz, 2H), 7.24 - 7.11 (m, 2H), 7.06 (d, *J* = 8.6 Hz, 2H), 6.77 - 6.71 (m, 2H), 6.32 (s, 1H), 4.99 (d, *J* = 7.5 Hz, 1H), 4.80 - 4.68 (m, 2H), 4.18 (d, *J* = 5.8 Hz, 2H), 4.05 (dt, *J* = 11.2, 5.5 Hz, 4H), 3.19 (d, *J* = 15.8 Hz, 1H), 3.05 - 3.00 (m, 1H), 2.92 (t, *J* = 7.7 Hz, 4H), 2.83 (s, 3H), 2.81 (s, 2H), 2.76 (s, 4H), 2.09 - 1.93 (m, 2H), 1.77 (p, *J* = 3.4 Hz, 4H), 1.20 (d, *J* = 6.8 Hz, 3H).

### Example 47: Synthesis of Compound 141

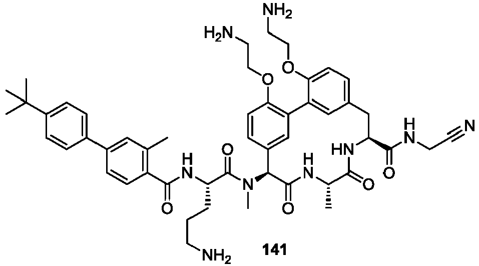

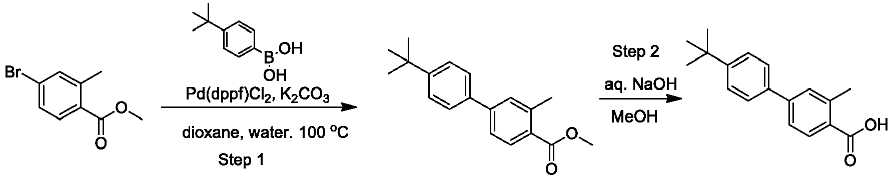

The synthesis of a biaryl tail via Pd-catalyzed Suzuki coupling of an aryl boronic acid and an aryl halide, followed by base hydrolysis of the ester is described and is referred to as General Method 7.

Step 1: To a solution of 4-t-butylbenzeneboronic acid (151.6 mg, 0.85 mmol) in 1,4-dioxane (5 mL) and water (1 mL) were added potassium carbonate (181.0 mg, 1.31 mmol), 1,1'-bis(diphenylphosphino)ferrocene palladium dichloride (47.9 mg, 0.07 mmol), and methyl 4-bromo-2-methylbenzoate (150.0 mg, 0.65 mmol). The reaction mixture was stirred at 100 °C for 2 h under N₂ and concentrated. The residue was taken up in EtOAc (20 mL) and washed with water (20 mL × 2) and brine (20 mL). The organic layer was dried over MgSO₄ and concentrated. The residue was purified by flash column chromatography (5% EtOAc in petroleum ether, Rf = 0.7) to afford methyl 4-(4-tert-butylphenyl)-2-methyl-benzoate (120 mg, 64.9% yield) as a colorless oil. LCMS (5-95AB_1.5min): t_{R} = 0.972 min, [M + H]⁺ = 281.9.

Step 2: Methyl 4-(4-tert-butylphenyl)-2-methyl-benzoate (120.0 mg, 0.430 mmol) was hydrolyzed using General Method NaOH to give 4-(4-tert-butylphenyl)-2-methyl-benzoic acid (100 mg, 0.3726 mmol, 87.7% yield) as a white solid.

Compound 141 (formic acid salt) was prepared as a white solid utilizing the methods in Example 7 (Compound 101). LCMS (Method 5-95 AB, ESI): t_{R} = 0.633 min, [M + H]⁺ = 902.4.

### Example 48: Synthesis of Compound 142

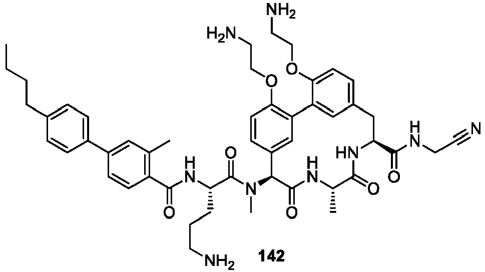

Compound 142 (formic acid salt) was prepared as a white solid utilizing the methods in Example 47 (Compound 141) from 4-(4-butylphenyl)-2-methylbenzoic acid. LCMS (Method 5-95 AB, ESI): t_{R} = 0.643 min, [M + H]⁺ = 902.4.

### Example 49: Synthesis of Compound 143

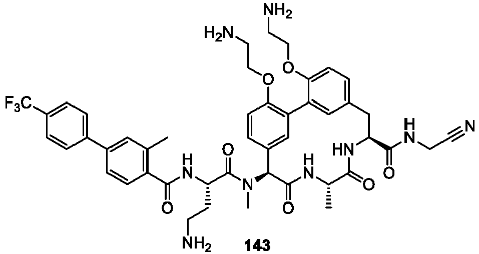

Compound 143 (formic acid salt) was prepared as a white solid utilizing the methods in Example 47 (Compound 141) from 4-(4-trifluoromethylphenyl)-2-methylbenzoic acid. LCMS (Method 5-95 AB, ESI): t_{R} = 0.588 min, [M + H]⁺ = 900.3.

### Example 50: Synthesis of Compound 144

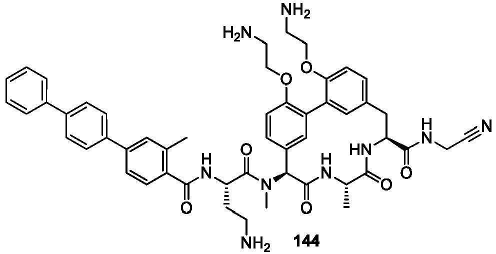

Compound 144 (formic acid salt) was prepared as a white solid utilizing the methods in Example 47 (Compound 141) from 3-methyl-[1,1':4',1"-terphenyl]-4-carboxylic acid. LCMS (Method 5-95 AB, ESI): t_{R} = 0.615 min, [M + H]⁺ = 908.4.

### Example 51: Synthesis of Compound 145

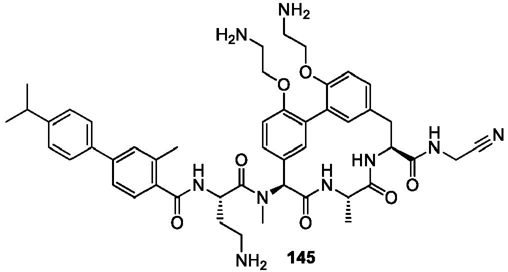

Compound 145 (formic acid salt) was prepared as a white solid utilizing the methods in Example 47 (Compound 141). LCMS (Method 5-95 AB, ESI): t_{R} = 0.632 min, [M + H]⁺ = 874.6.

### Example 52: Synthesis of Compound 146

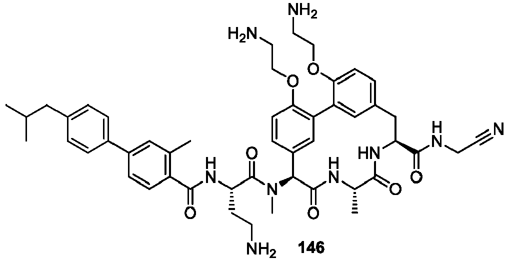

Compound 146 (formic acid salt) was prepared as a white solid utilizing the methods in Example 47 (Compound 141) from 4-(4-isobutylphenyl)-2-methylbenzoic acid. LCMS (Method 5-95 AB, ESI): t_{R} = 0.653 min, [M + H]⁺ = 888.5. ¹H NMR (400 MHz, CD₃OD): δ 7.56-7.50 (m, 4H), 7.48-7.44 (m, 1H), 7.35-7.31 (m, 1H), 7.26-7.17 (m, 4H), 7.10-7.08 (m, 1H), 6.92-6.90 (m, 1H), 6.79 (br s, 1H), 6.39 (s, 1H), 5.19-5.15 (m, 1H), 4.81-4.79 (m, 3H), 4.30-4.15 (m, 5H), 3.36-3.33 (m, 1H), 3.25-3.05 (m, 7H), 2.95 (s, 3H), 2.53 (d, J = 7.6 Hz, 2H), 2.49 (s, 3H), 2.35-2.20 (m, 1H), 2.20-2.05 (m, 1H), 1.95-1.80 (m, 1H), 1.36 (d, J = 7.2 Hz, 3H), 0.94 (d, J = 6.4 Hz, 6H).

### Example 53: Synthesis of Compound 147

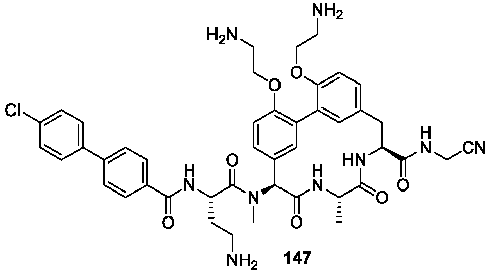

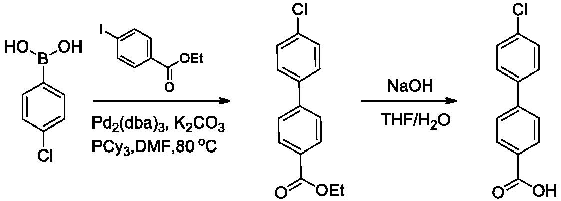

An oven-dried three neck flask (500 mL) was charged with (4-chlorophenyl)boronic acid (12 g, 74.7 mmol), ethyl 4-iodobenzoate (14.1 g 51.2 mmol), Pd₂(dba)₃ (4.68 g, 5.12 mmol), PCy₃ (1.43 g, 5.12 mmol) and K₂CO₃ (21.21 g, 153.5 mmol). DMF (100 mL) was added and the reaction mixture was purged with N₂. The mixture was stirred at 80 °C for 12 h. The reaction mixture was cooled to room temperature and filtered to remove K₂CO₃. The solvent was removed and the brown residue was purified by column (1% to 5% EtOAc in petroleum ether) to give ethyl 4'-chloro-[1,1'-biphenyl]-4-carboxylate (9.52 g, 71.4%).

To a solution of ethyl 4'-chloro-[1,1'-biphenyl]-4-carboxylate (9.52 g, 36.6 mmol) in a mixture of THF (150 mL) and H₂O (20 mL) was added NaOH (4N, 5.86 g, 146 mmol). After the mixture was stirred at 70°C for 10 h, the organic solvent was removed under reduced pressure, and pH was adjusted to 3 with 4M HCl. The product was collected by filtration, washed with water and dried to give 4'-chloro-[1,1'-biphenyl]-4-carboxylic acid (8.5 g, 100%). ¹H NMR (400 MHz, CDCl₃) δ 8.10 (d, *J* = 8.4 Hz, 2 H), 7.70 (d, *J* = 8 Hz, 2 H), 7.65 (d, *J* = 8.8 Hz, 2 H), 7.47 (d, *J* = 8.8 Hz, 2 H).

Compound 147 (formic acid salt) was prepared as a white solid utilizing the methods in Example 47 (Compound 141) from 4'-chloro-[1,1'-biphenyl]-4-carboxylic acid. LCMS (Method 5-95 AB, ESI): t_{R} = 0.710, [M + H]⁺ = 852.4; ¹H NMR (400 MHz, MeOH-*d*₄) δ 8.48 (brs, 2H, HCOOH), 7.97 (d, *J*=8.4 Hz, 2H), 7.77 (d, *J*=8.4 Hz, 2H), 7.68 (d, *J*=8.4 Hz, 2H), 7.50 (d, *J*=8.4 Hz, 2H), 7.32 (d, *J*=8.0 Hz, 1H), 7.24 (d, *J*=8.0 Hz, 1H), 7.16 (d, *J*=8.0 Hz, 1H), 7.09 (d, *J*=8.0 Hz, 1H), 6.89 (brs, 1H), 6.82 (brs, 1H), 6.34 (s, 1H), 5.22-5.14 (m, 1H), 4.83-4.79 (m, 2H), 4.32-4.14 (m, 4H), 4.20 (s, 2H), 3.37-3.33 (m, 2H), 3.27-2.95 (m, 7H), 2.89 (s, 3H), 2.37-2.30 (m, 1H), 2.22-2.14 (m, 1H), 1.36 (d, *J*=6.4 Hz, 3H).

### Example 54: Synthesis of Compound 148

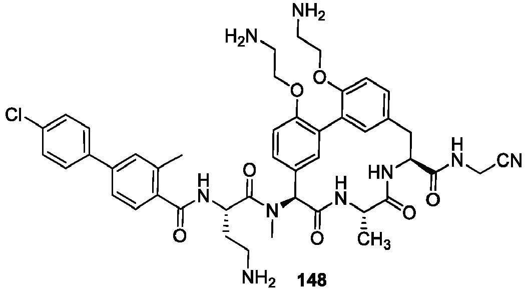

Compound 148 (formic acid salt) was prepared as a white solid utilizing the methods in Example 47 (Compound 141) from 4'-chloro-3-methyl-[1,1'-biphenyl]-4-carboxylic acid. LCMS (Method 5-95 AB, ESI): t_{R} = 0.560, [M + H]⁺ = 866.2.

### Example 55: Synthesis of Compound 149

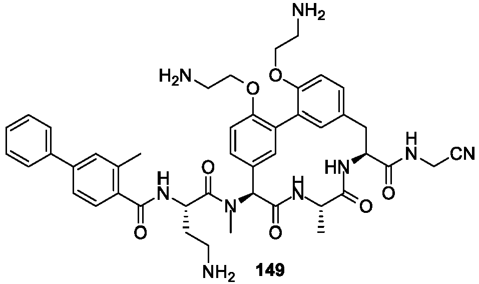

Compound 149 (free base) was prepared as a white solid utilizing the methods in Example 47 (Compound 141). LCMS (Method 5-95 AB, ESI): t_{R} = 0.661, [M + H]⁺ = 832.5.

### Example 56: Synthesis of Compound 150

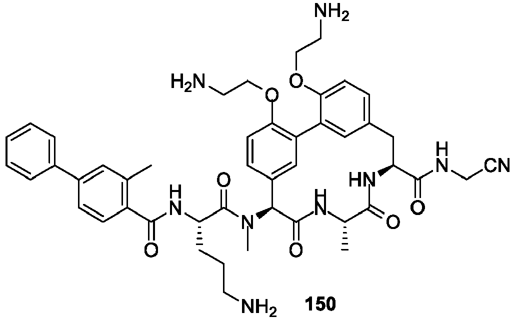

Compound 150 (free base) was prepared as a white solid utilizing the methods in Example 47 (Compound 141) from 3-methyl-[1,1'-biphenyl]-4-carboxylic acid. LCMS (Method 5-95 AB, ESI): t_{R} = 0.649, [M + H]⁺ = 846.4.

### Example 57: Synthesis of Compound 151

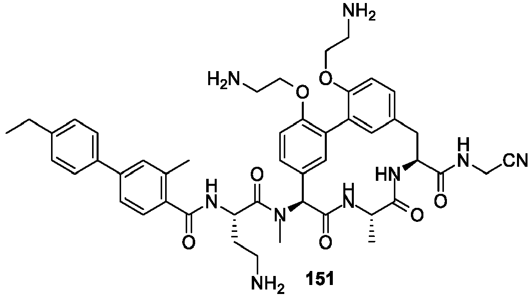

Compound 151 (free base) was prepared as a white solid utilizing the methods in Example 47 (Compound 141). LCMS (Method 5-95 AB, ESI): t_{R} = 0.735, [M + H]⁺ = 860.5; ¹H NMR (400 MHz, MeOH-*d*₄) δ 7.45-7.38 (m, 2H), 7.36-7.16 (m, 7H), 7.06-6.98 (m, 2H), 6.84 (brs, 1H), 6.71 (brs, 1H), 6.50 (s, 1H), 5.20-5.13 (m, 1H), 4.78-4.71 (m, 1H), 4.54-4.46 (m, 1H), 4.23-3.97 (m, 4H), 4.21 (s, 2H), 3.10-2.80 (m, 5H), 3.00 (s, 3H), 2.67 (q, J=6.8 Hz, 2H), 2.33 (s, 3H), 2.16-2.06 (m, 1H), 2.06-1.96 (m, 1H), 1.36 (d, J=6.8 Hz, 3H), 1.26 (t, J=6.8 Hz, 3H). Example 58: Synthesis of Compound 152

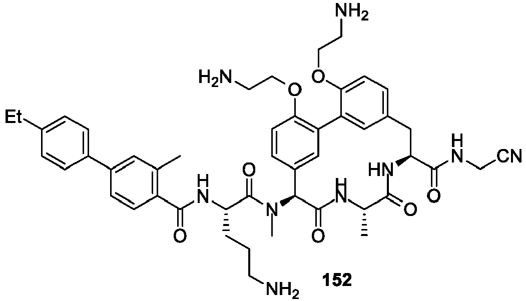

Compound 152 (formic acid salt) was prepared as a white solid utilizing the methods in Example 47 (Compound 141) from 4'-ethyl-3-methyl-[1,1'-biphenyl]-4-carboxylic acid. LCMS (Method 5-95 AB, ESI): t_{R} = 0.698, [M + H]⁺ = 874.2; ¹H NMR (400 MHz, MeOH-*d*₄) δ 8.50 (brs, 1H, HCOOH), 7.50-7.44 (m, 5H), 7.35-7.33 (m, 1H), 7.29 (d, *J*=8 Hz, 2H), 7.22 (d, *J*=8 Hz, 2H), 7.12 (d, *J*=8 Hz, 1H), 6.92 (brs, 1H), 6.74-6.58 (m, 2H), 5.11-5.08 (m, 1H), 4.85-4.81 (m, 2H), 4.31-4.21 (m, 6H), 3.28-3.24 (m, 5H), 3.15-3.01 (m, 3H), 3.04 (s, 3H), 2.71 (q, *J*=7.2 Hz, 2H), 2.46 (s, 3H), 2.08-1.70 (m, 4H), 1.39 (d, *J*=7.2 Hz, 3H), 1.29 (t, *J*=7.2 Hz, 3H).

### Example 59: Synthesis of Compound 153

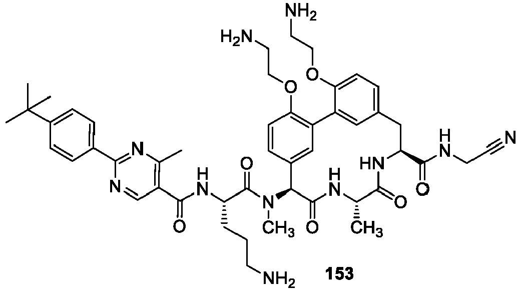

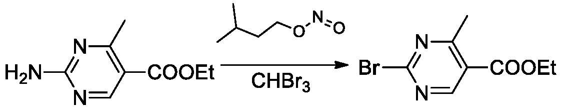

To a solution of ethyl 2-amino-4-methylpyrimidine-5-carboxylate (4.0 g, 22 mmol) in CHBr₃ (66 mL) was added isopentyl nitrite (44 mL) and the mixture was stirred at 85°C for 4 h. The volatiles were removed and the residue was taken up by EtOAc (100 mL), which was washed by brine (100 mL × 2). The organic layer was dried over Na₂SO₄, concentrated and the residue was purified by silica gel flash column to give ethyl 2-bromo-4-methylpyrimidine-5-carboxylate (3.0 g, 55.5% yield) as a white solid. ¹H NMR (400 MHz, CDCl₃): δ 8.93 (s, 1H), 4.41 (q, *J* = 7.2 Hz, 2H), 2.82 (s, 3H), 1.41 (t, *J* = 7.0 Hz, 3H).

Compound 153 (formic acid salt) was prepared as a white solid utilizing the methods in Example 47 (Compound 141) from ethyl 2-bromo-4-methylpyrimidine-5-carboxylate. LCMS (Method 5-95 AB, ESI): t_{R} = 0.736 min, [M+H]⁺ = 904.7.

### Example 60: Synthesis of Compound 154

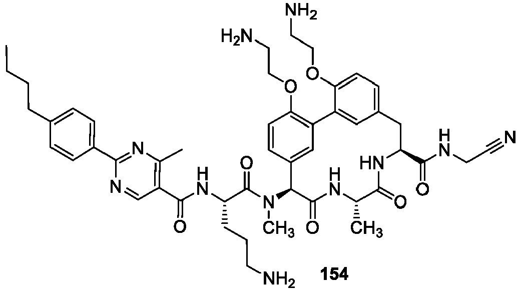

Compound 154 (formic acid salt) was prepared as a white solid utilizing the methods in Example 47 (Compound 141). LCMS (Method 5-95 AB, ESI): t_{R} = 0.661 min, [M+H]⁺ = 905.6.

### Example 61: Synthesis of Compound 155

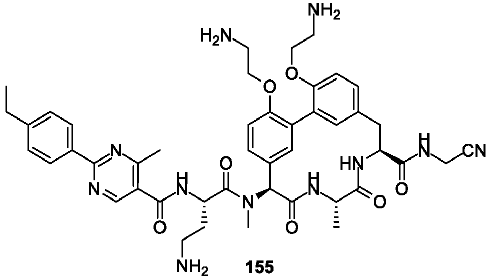

Compound 155 (formic acid salt) was prepared as a white solid utilizing the methods in Example 47 (Compound 141). LCMS (Method 5-95 AB, ESI): t_{R} = 0.681 min, [M+H]⁺ = 862.4.

### Example 62: Synthesis of Compound 156

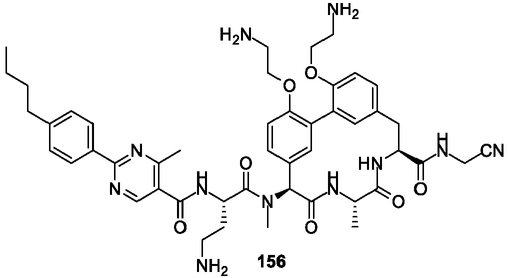

Compound 156 (formic acid salt) was prepared as a white solid utilizing the methods in Example 47 (Compound 141). LCMS (Method 5-95 AB, ESI): t_{R} = 0.617 min, [M+H]⁺ = 890.4. ¹H NMR (400 MHz, CD₃OD): δ 8.77 (s, 1H), 8.53 (s, 1H), 8.32 (d, J = 8.4 Hz, 2H), 7.32 (d, J = 8.0 Hz, 2H), 7.19 (d, J = 8.4 Hz, 2H), 7.07 (d, J = 8.4 Hz, 1H), 6.89 (s, 1H), 6.74 (s, 1H), 6.45 (s, 1H), 5.25-5.15 (m, 1H), 4.85-4.75 (m, 2H), 4.30-4.10 (m, 6H), 3.35 (s, 2H), 3.30-3.20 (m, 1H), 3.20-3.05 (m, 5H), 2.96 (s, 3H), 2.80-2.60 (m, 5H), 2.35-2.20 (m, 1H), 2.20-2.0 (m, 1H), 1.75-1.60 (m, 2H), 1.50-1.25 (m, 5H), 0.98 (t, J = 7.2 Hz, 3H).

### Example 63: Synthesis of Compound 157

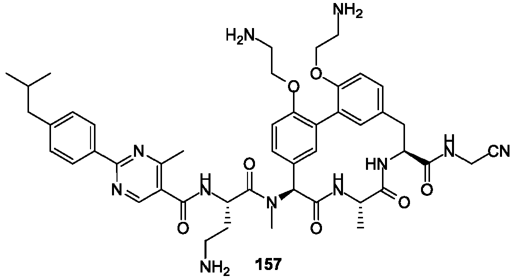

Compound 157 (formic acid salt) was prepared as a white solid utilizing the methods in Example 47 (Compound 141). LCMS (Method 5-95 AB, ESI): t_{R} = 0.607 min, [M+H]⁺ = 890.5. ¹H NMR (400 MHz, CD₃OD): δ 8.70 (s, 1H), 8.10 (d, J = 7.6 Hz, 2H), 7.30-7.05 (m, 4H), 7.00-6.80 (m, 3H), 6.74 (s, 1H), 6.28 (s, 1H), 5.25-5.10 (m, 1H), 4.80-4.65 (m, 2H), 4.50-4.35 (m, 1H), 4.30-4.05 (m, 4H), 4.05-3.90 (m, 1H), 3.10-2.75 (m, 11H), 2.60-2.40 (m, 5H), 2.15-2.05 (m, 1H), 2.05-1.85 (m, 2H), 1.34 (d, J = 6.8 Hz, 3H), 0.97 (d, J = 6.0 Hz, 6H).

### Example 64: Synthesis of Compound 158

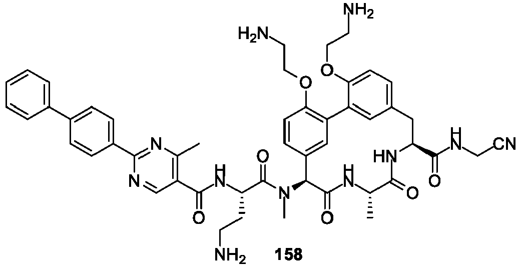

Compound 158 (formic acid salt) was prepared as a white solid utilizing the methods in Example 47 (Compound 141). LCMS (Method 5-95 AB, ESI): t_{R} = 0.722 min, [M+H]⁺ = 910.4.

### Example 65: Synthesis of Compound 159

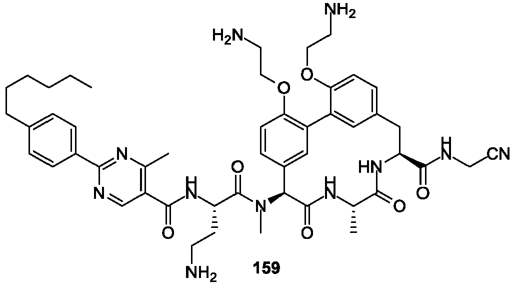

Compound 159 (formic acid salt) was prepared as a white solid utilizing the methods in Example 47 (Compound 141). LCMS (Method 5-95 AB, ESI): t_{R} = 0.770 min, [M+Na]⁺ = 940.0.

### Example 66: Synthesis of Compound 160

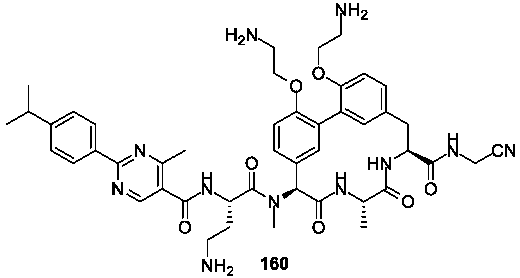

Compound 160 (formic acid salt) was prepared as a white solid utilizing the methods in Example 47 (Compound 141). LCMS (Method 5-95 AB, ESI): t_{R} = 0.710 min, [M+H]⁺ = 876.8.

### Example 67: Synthesis of Compound 161

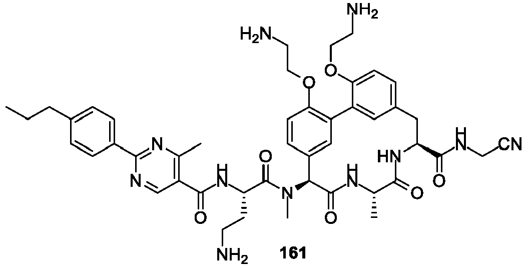

Compound 161 (formic acid salt) was prepared as a white solid utilizing the methods in Example 47 (Compound 141). LCMS (Method 5-95 AB, ESI): t_{R} = 0.587 min, [M+H]⁺ = 876.6.

### Example 68: Synthesis of Compound 162

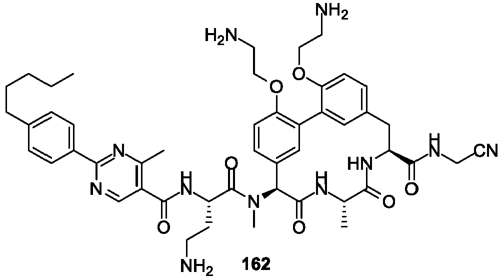

Compound 162 (formic acid salt) was prepared as a white solid utilizing the methods in Example 47 (Compound 141). LCMS (Method 5-95 AB, ESI): t_{R} = 0.757 min, [M+H]⁺ = 904.6. ¹H NMR (400 MHz, CD₃OD): δ 8.78 (s, 1H), 8.50 (br s, 2H), 8.35 (d, *J* = 8.4 Hz, 2H), 7.35-7.25 (m, 3H), 7.23 - 7.18 (m, 2H), 7.08 (d, *J* = 8.4 Hz, 1H), 6.90 (s, 1H), 6.79 (s, 1H), 6.40 (s, 1H), 5.19 - 5.16 (m, 1H), 4.80-4.70 (m, 2H), 4.21 - 4.18 (m, 6H), 3.15 - 3.13 (m, 7H), 2.95 (s, 3H), 2.72 - 2.68 (m, 6H), 2.32 - 2.14 (m, 2H), 1.70 - 1.66 (m, 2H), 1.38 - 1.35 (m, 7H), 0.92 (t, *J* = 6.8 Hz, 3H).

### Example 69: Synthesis of Compound 163

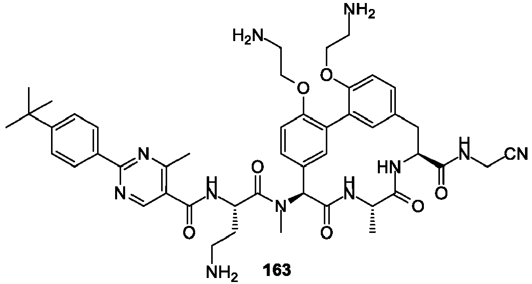

Compound 163 (formic acid salt) was prepared as a white solid utilizing the methods in Example 47 (Compound 141). LCMS (Method 5-95 AB, ESI): t_{R} = 0.711 min, [M+H]⁺ = 890.5. ¹H NMR (400 MHz, CD₃OD): δ 8.78 (s, 1H), 8.48 (br s, 2H), 8.36 (d, *J* = 8.4 Hz, 2H), 7.55 (d, *J* = 8.4 Hz, 2H), 7.32 (d, *J* = 8.8 Hz, 1H), 7.25-7.15 (m, 2H), 7.08 (d, *J* = 8.8 Hz, 1H), 6.90 (s, 1H), 6.79 (s, 1H), 6.39 (s, 1H), 5.19 - 5.16 (m, 1H), 4.80-4.70 (m, 2H), 4.25 - 4.10 (m, 6H), 3.20 - 3.10 (m, 8H), 2.95 (s, 3H), 2.75 - 2.65 (m, 4H), 2.32 - 2.14 (m, 1H), 1.40 - 1.30 (m, 12H).

### Example 70: Synthesis of Compound 164

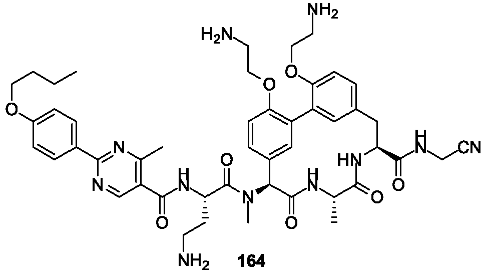

Compound 164 (formic acid salt) was prepared as a white solid utilizing the methods in Example 47 (Compound 141). LCMS (Method 5-95 AB, ESI): t_{R} = 0.727 min, [M+H]⁺ = 906.5. ¹H NMR (400 MHz, CD₃OD): 8.74 (s, 1H), 8.50 (br s, 2H), 8.36 (d, J = 8.8 Hz, 2H), 7.32 (d, J = 8.4 Hz, 1H), 7.20 (d, *J* = 8.4 Hz, 2H), 7.09 (d, *J* = 8.4 Hz, 1H), 7.02 (d, *J* = 8.8 Hz, 2H), 6.89 (s, 1H), 6.75 (s, 1H), 6.44 (s, 1H), 5.25 - 5.15 (m, 1H), 4.85-4.75 (m, 2H), 4.30 - 4.15 (m, 6H), 4.08 (d, *J* = 6.4 Hz, 2H), 3.25 - 3.10 (m, 8H), 2.96 (s, 3H), 2.67 (s, 3H), 2.35 - 2.25 (m, 1H), 2.25-2.10 (m, 1H), 1.85-1.75 (m, 2H), 1.60-1.50 (m, 2H), 1.36 (d, *J* = 6.8 Hz, 3H), 1.02 (t, *J* = 7.4 Hz, 3H).

### Example 71: Synthesis of Compound 165

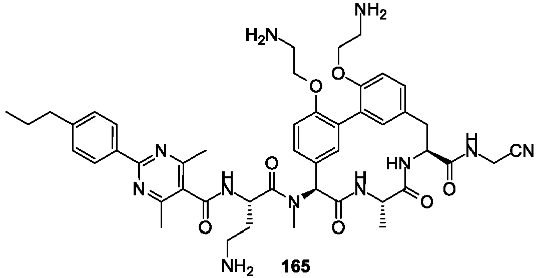

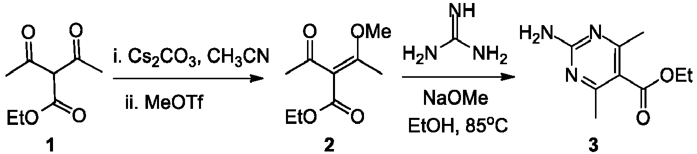

A mixture of ethyl diacetoacetate (4.0 g, 23.23 mmol) and Cs₂CO₃ (15.2 g, 46.46 mmol) in acetonitrile (50 mL) was stirred at 25°C for 30 min and cooled to 0°C. MeOTf (3.81 g, 23.23 mmol) was added dropwise and the mixture was stirred at 25°C for 1.5 h. The mixture was diluted with ethyl ether and filtered, and the filtrate was concentrated. The residue was taken up in ethyl ether (100 mL), washed with 2N NaOH (10 mL × 2), dried over Na₂SO₄ and concentrated to give crude ethyl (E)-2-acetyl-3-methoxy-but-2-enoate (2.7 g, 62.4% yield) as a yellow oil, which was used directly in the next step.

A mixture of ethyl (E)-2-acetyl-3-methoxy-but-2-enoate (2.7 g, 14.5 mmol), guanidine hydrochloride (3463.0 mg, 36.25 mmol) and NaOMe (1566.6 mg, 29 mmol) in ethanol (20 mL) was stirred at 80°C for 12 h, and cooled to 0°C and filtered. The filtrate was concentrated under reduced pressure. The residue was taken up in EtOAc (100 mL), washed with brine (50 mL × 3), dried over Na₂SO₄ and concentrated. The residue was purified by silica gel column (10% EtOAc in petroleum ether, Rf = 0.45) to give ethyl 2-amino-4,6-dimethyl-pyrimidine-5-carboxylate (820 mg, 29% yield) as a white solid. ¹H NMR (400 MHz, DMSO-d6): δ 6.96 (s, 2H), 4.26 (q, J = 7.2 Hz, 2H), 2.30 (s, 6H), 1.29 (t, J = 7.4 Hz, 3H).

Compound 165 (formic acid salt) was prepared as a white solid utilizing the methods in Example 59 (Compound 153). LCMS (Method 5-95 AB, ESI): t_{R} = 0.707 min, [M+H]⁺ = 890.4. ¹H NMR (400 MHz, CD₃OD): 8.50 (br s, 2H), 8.30-8.20 (m, 2H), 7.30-7.28 (m, 3H), 7.19 (d, *J* = 8.4 Hz, 2H), 7.08 (d, *J* = 8.4 Hz, 1H), 6.89 (s, 1H), 6.74 (s, 1H), 6.46 (s, 1H), 5.25 - 5.20 (m, 1H), 4.80-4.70 (m, 2H), 4.30 - 4.10 (m, 6H), 3.25 - 3.10 (m, 8H), 3.00 (s, 3H), 2.67 (t, *J* = 7.4 Hz, 2H), 2.53 (s, 6H), 2.35 - 2.20 (m, 1H), 2.20-2.05 (m, 1H), 1.73-1.67 (m, 2H), 1.35 (d, *J* = 6.8 Hz, 3H), 0.98 (t, *J* = 7.2 Hz, 3H).

### Example 72: Synthesis of Compound 166

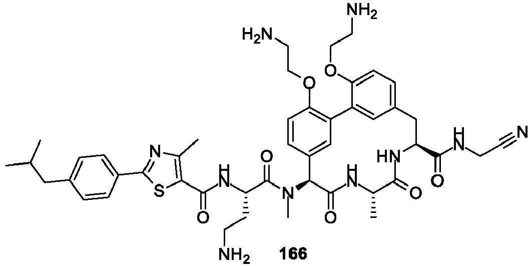

Compound 166 (formic acid salt) was prepared as a white solid utilizing the methods in Example 47 (Compound 141). LCMS (Method 5-95 AB, ESI): t_{R} = 0.613 min, [M+H]⁺ = 895.5.

### Example 73: Synthesis of Compound 167

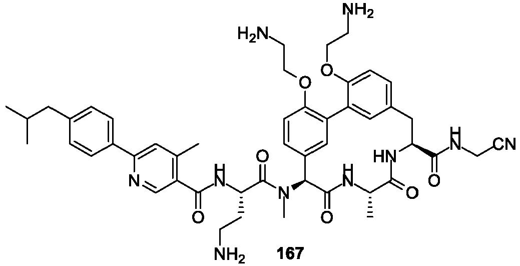

Compound 167 (formic acid salt) was prepared as a white solid utilizing the methods in Example 47 (Compound 141). LCMS (Method 5-95 AB, ESI): t_{R} = 0.604 min, [M+H]⁺ = 889.9.

### Example 74: Synthesis of Compound 168

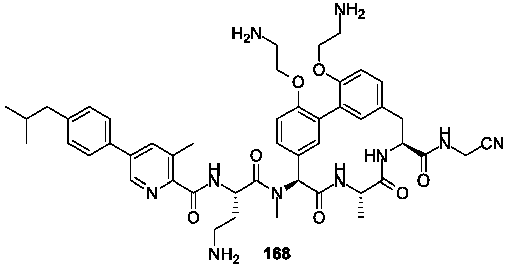

Compound 168 (formic acid salt) was prepared as a white solid utilizing the methods in Example 47 (Compound 141). LCMS (Method 5-95 AB, ESI): t_{R} = 0.745 min, [M+H]⁺ = 889.4.

### Example 75: Synthesis of Compound 169

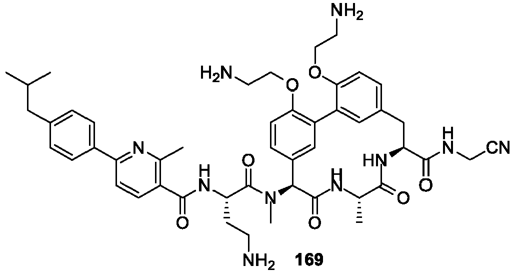

Compound 169 (formic acid salt) was prepared as a white solid utilizing the methods in Example 47 (Compound 141). LCMS (Method 5-95 AB, ESI): t_{R} = 0.644min, [M+H]⁺ = 889.5. ¹H NMR (400 MHz, CD₃OD): δ 7.88 (d, J = 7.6 Hz, 2H), 7.83 (d, J = 8.0 Hz, 1H), 7.72 (d, J = 8.0 Hz, 1H), 7.35 - 7.25 (m, 3H), 7.25 - 7.15 (m, 2H), 7.10 (d, J = 8.4 Hz, 1H), 6.90 (s, 1H), 6.76 (s, 1H), 6.44 (s, 1H), 5.20 - 5.10 (m, 1H), 4.85 - 4.75 (m, 2H), 4.30 - 4.15 (m, 6H), 3.25 - 3.05 (m, 8H), 2.96 (s, 3H), 2.67 (s, 3H), 2.56 (d, J = 7.2 Hz, 2H), 2.35 - 2.20 (m, 1H), 2.20 - 2.10 (m, 1H), 2.00 - 1.85 (m, 1H), 1.36 (d, J = 6.4 Hz, 3H), 0.95 (d, J = 6.4 Hz, 6H).

### Example 76: Synthesis of Compound 170

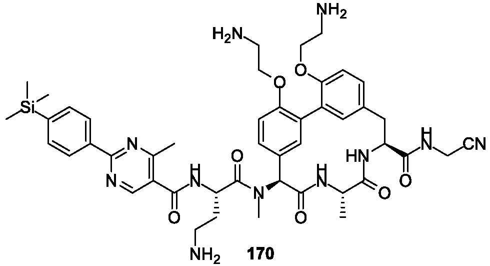

Compound 170 (formic acid salt) was prepared as a white solid utilizing the methods in Example 47 (Compound 141). LCMS (Method 5-95 AB, ESI): t_{R} = 0.737 min, [M+H]⁺ = 906.5. ¹H NMR (400 MHz, CD₃OD): δ 8.82 (s, 1H), 8.50 (br s, 2H, *FA*), 8.43 (d, J = 8.0 Hz, 2H), 7.69 (d, J = 8.0 Hz, 2H), 7.34-7.32 (m, 1H), 7.24 (d, J = 8.4 Hz, 1H), 7.19 (d, J = 8.8 Hz, 1H), 7.09 (d, J = 8.4 Hz, 1H), 6.92 (br s, 1H), 6.82 (br s, 1H), 6.39 (s, 1H), 5.18 - 5.17 (m, 1H), 4.82 - 4.79 (m, 2H), 4.25-4.15 (m, 6H), 3.50-3.30 (m, 3H), 3.15 - 3.12 (m, 5H), 2.96 (s, 3H), 2.72 (s, 3H), 2.40-2.10 (m, 2H), 1.36 (d, J = 6.8 Hz, 3H), 0.32 (s, 9H).

### Example 77: Synthesis of Compound 171

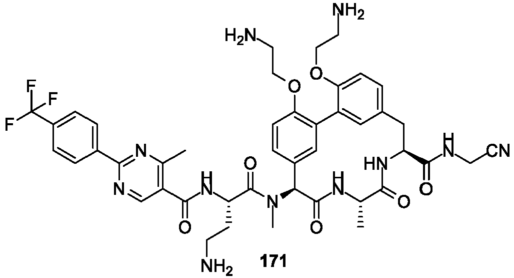

Compound 171 (formic acid salt) was prepared as a white solid utilizing the methods in Example 47 (Compound 141). LCMS (Method 5-95 AB, ESI): t_{R} = 0.697 min, [M+H]⁺ = 902.7.

### Example 78: Synthesis of Compound 172

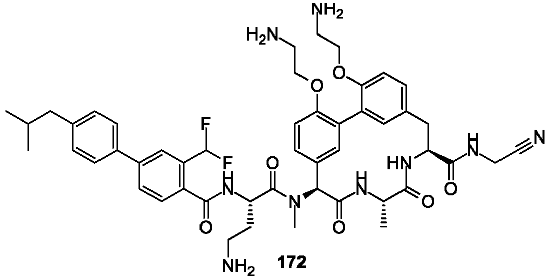

Compound 172 (formic acid salt) was prepared as a white solid utilizing the methods in Example 47 (Compound 141). LCMS (Method 5-95 AB, ESI): t_{R} = 0.769 min, [M+H]⁺ = 924.5.

### Example 79: Synthesis of Compound 173

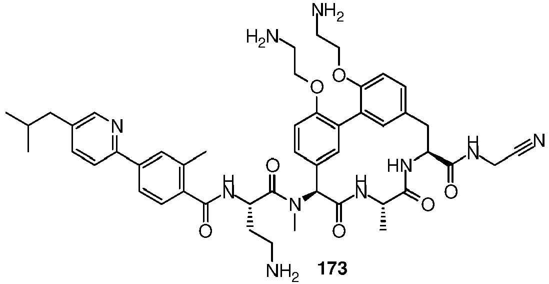

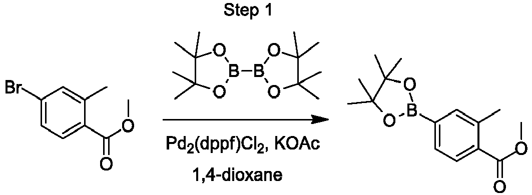

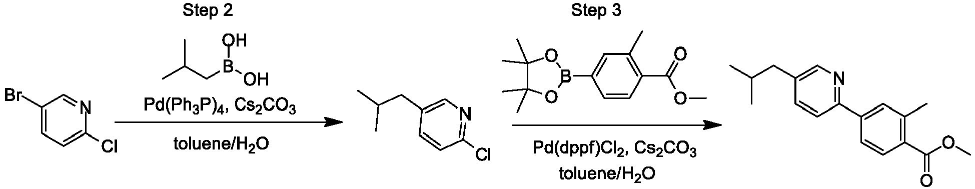

Step 1: A mixture of potassium acetate (649.2 mg, 6.55 mmol), 1,1'-bis(diphenylphosphino)ferrocene palladium dichloride (159.7 mg, 0.22 mmol), bis(pinacolato)diboron (1108.5 mg, 4.37 mmol) and methyl 4-bromo-2-methylbenzoate (500.0 mg, 2.18 mmol) in 1,4-dioxane (20 mL) was stirred at 110 °C for 4 h under N₂ protection, and evaporated to dryness. The residue was taken up in EtOAc (30 mL), washed with water (20 mL × 2) and brine (20 mL), dried over MgSO₄ and concentrated. The residue was purified by flash column chromatography (10% ethyl acetate in petroleum ether, Rf = 0.6) to afford methyl 2-methyl-4-(4,4,5,5-tetramethyl-1,3,2-dioxaborolan-2-yl)benzoate (400 mg, 66.4% yield) as a yellow oil.

Step 2: To a solution of 5-bromo-2-chloropyridine (200.0 mg, 1.04 mmol) in toluene (10 mL) and water (2 mL) were added cesium carbonate (1693.1 mg, 5.2 mmol), isobutylboronic acid (158.9 mg, 1.56 mmol) and tetrakis(triphenylphosphine)palladium(0) (120.1 mg, 0.10 mmol). The reaction mixture was stirred at 110 °C for 16 h and filtered. The filtrate was diluted with H₂O (20 mL) and extracted with EtOAc (40 mL × 2). The combined organic layers were washed with water (80 mL × 3) and brine (80 mL), dried over Na₂SO₄ and concentrated. The residue was purified by prep-TLC (7.5% EtOAc in petroleum ether) to obtain 2-chloro-5-isobutylpyridine (110 mg, 62.4% yield) as a yellow oil. LCMS (Method 5-95 AB, ESI): t_{R} = 0.872 min, [M+H]⁺ = 169.8.

Step 3: A mixture of 2-chloro-5-isobutylpyridine (50.0 mg, 0.29 mmol), 1,1'-bis(diphenylphosphino)ferrocene palladium dichloride (21.6 mg, 0.03 mmol), methyl 2-methyl-4-(4,4,5,5-tetramethyl-1,3,2-dioxaborolan-2-yl)benzoate (122.1 mg, 0.44 mmol) and cesium carbonate (288.1 mg, 0.88 mmol) in toluene (5 mL) and water (1 mL) was stirred at 110 °C for 16 h under nitrogen. The mixture was diluted with H₂O (10 mL) and extracted with EtOAc (10 mL × 2). The combined organic layers were washed with water (20 mL × 4) and brine (20 mL), dried over Na₂SO₄ and concentrated. The residue was purified by prep-TLC (7.5% EtOAc in petroleum) to obtain methyl 4-(5-isobutylpyridin-2-yl)-2-methylbenzoate (74 mg, 88.6% yield) as a yellow oil. LCMS (Method 5-95 AB, ESI): t_{R} = 0.816 min, [M+H]⁺ = 283.9.

Compound 173 (formic acid salt) was prepared as a white solid utilizing the methods in Example 47 (Compound 141). LCMS (Method 5-95 AB, ESI): t_{R} = 0.639 min, [M+H]⁺ = 889.5.

### Example 80: Synthesis of Compound 174

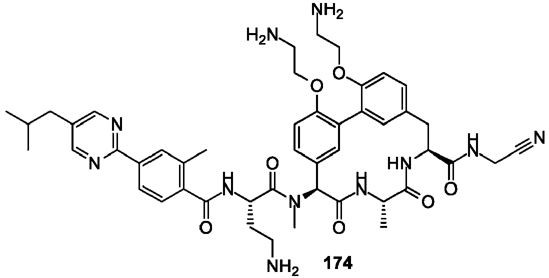

Compound 174 (formic acid salt) was prepared as a white solid utilizing the methods in Example 79 (Compound 173) from 5-bromo-2-chloropyrimidine. LCMS (Method 5-95 AB, ESI): t_{R} = 0.553 min, [M+H]⁺ = 890.9.

### Example 81: Synthesis of Compound 175

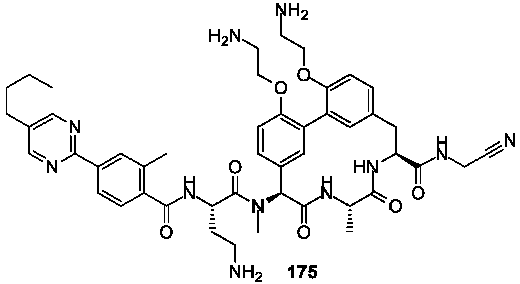

Compound 175 (formic acid salt) was prepared as a white solid utilizing the methods in Example 79 (Compound 173) from 5-bromo-2-chloropyrimidine. LCMS (Method 5-95 AB, ESI): t_{R} = 0.699 min, [M+H]⁺ = 890.5.

### Example 82: Synthesis of Compound 176

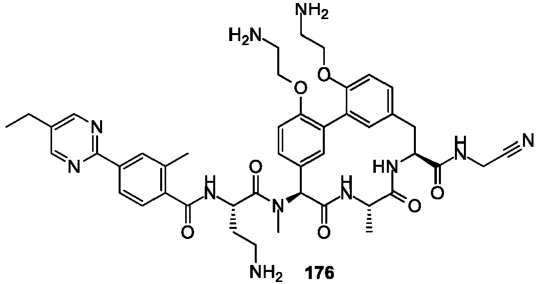

Compound 176 (formic acid salt) was prepared as a white solid utilizing the methods in Example 79 (Compound 173). LCMS (Method 5-95 AB, ESI): t_{R} = 0.626 min, [M+H]⁺ = 862.5.

### Example 83: Synthesis of Compound 177

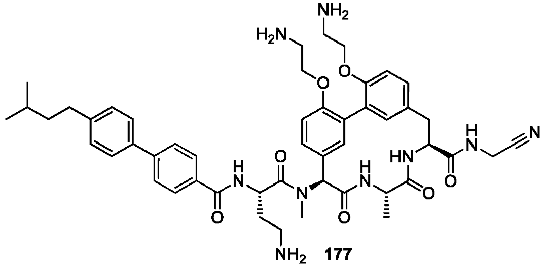

Compound 177 (formic acid salt) was prepared as a white solid utilizing the methods in Example 79 (Compound 173) from (4-(methoxycarbonyl)phenyl)boronic acid, 1-bromo-4-iodobenzene, and isopentylboronic acid. LCMS (Method 5-95 AB, ESI): t_{R} = 0.782 min, [M+H]⁺ = 888.5.

### Example 84: Synthesis of Compound 178

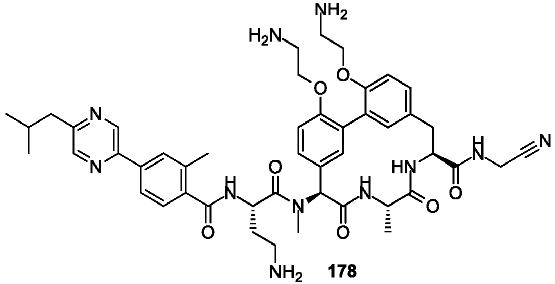

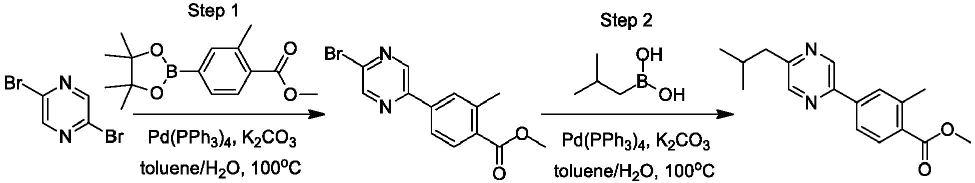

To a solution of 2,5-dibromopyrazine (200.0 mg, 0.84 mmol) in toluene (5 mL) and water (1 mL) were added potassium carbonate (348.6 mg, 2.52 mmol), methyl 2-methyl-4-(4,4,5,5-tetramethyl-1,3,2-dioxaborolan-2-yl)benzoate (232.2 mg, 0.84 mmol) and tetrakis(triphenylphosphine)palladium(0) (97.2 mg, 0.08 mmol). The reaction mixture was stirred at 80 °C for 16 h and filtered. The filtrate was diluted with H₂O (20 mL) and extracted with EtOAc (40 mL × 2). The combined organic layers were washed with water (80 mL × 3) and brine (80 mL), dried over Na₂SO₄ and concentrated. The residue was purified by prep-TLC (7.5% EtOAc in petroleum ether) to obtain methyl 4-(5-bromopyrazin-2-yl)-2-methylbenzoate (150 mg, 58.1% yield) as a white solid. ¹H NMR (400 MHz, CDCl₃): δ 8.81 (s, 1H), 8.75 (d, J = 1.2 Hz, 1H), 8.04 (d, J = 8.4 Hz, 1H), 7.88 (s, 1H), 7.85 (d, J = 8.4 Hz, 1H), 3.93 (s, 3H), 2.70 (s, 3H).

To a solution of isobutylboronic acid (99.6 mg, 0.98 mmol) in toluene (3 mL) and water (0.3 mL) were added tetrakis(triphenylphosphine)palladium(0) (56.4 mg, 0.05 mmol), potassium carbonate (202.5 mg, 1.47 mmol) and methyl 4-(5-bromopyrazin-2-yl)-2-methylbenzoate (150.0 mg, 0.49 mmol). The reaction mixture was stirred at 100 °C for 16 h and filtered. The filtrate was diluted with H₂O (20 mL) and extracted with EtOAc (40 mL × 2). The combined organic layers were washed with water (80 mL × 3) and brine (80 mL), dried over Na₂SO₄ and concentrated. The residue was purified by prep-TLC (9.5% EtOAc in petroleum ether, Rf = 0.4) to obtain methyl 4-(5-isobutylpyrazin-2-yl)-2-methyl-benzoate (52 mg, 37.4% yield) as a yellow oil. LCMS (Method 5-95 AB, ESI): t_{R} = 0.956 min, [M+H]⁺ = 284.9.

Compound 178 (formic acid salt) was prepared as a white solid utilizing the methods in Example 47 (Compound 141). LCMS (Method 5-95 AB, ESI): t_{R} = 0.679 min, [M+H]⁺ = 890.5.

### Example 85: Synthesis of Compound 179

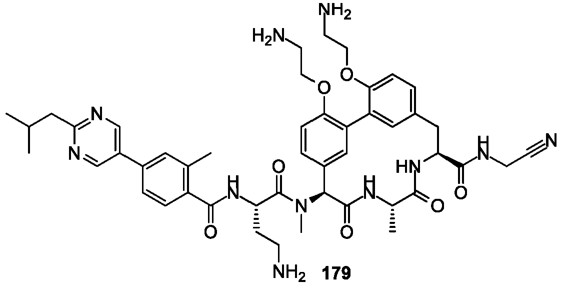

Compound 179 (formic acid salt) was prepared as a white solid utilizing the methods in Example 84 (Compound 178) from 5-bromo-2-chloropyrimidine. LCMS (Method 5-95 AB, ESI): t_{R} = 0.657 min, [M+H]⁺ = 890.2.

### Example 86: Synthesis of Compound 180

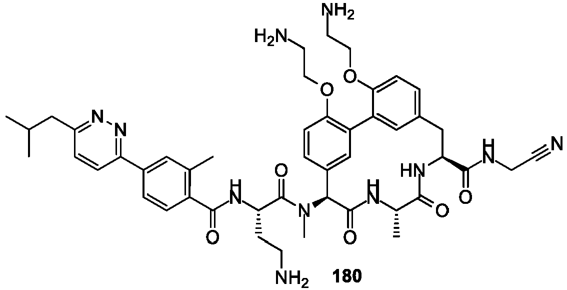

Compound 180 (formic acid salt) was prepared as a white solid utilizing the methods in Example 84 (Compound 178) from 3,6-dibromopyridazine. LCMS (Method 5-95 AB, ESI): t_{R} = 0.633 min, [M+H]⁺ = 890.4.

### Example 87: Synthesis of Compound 181

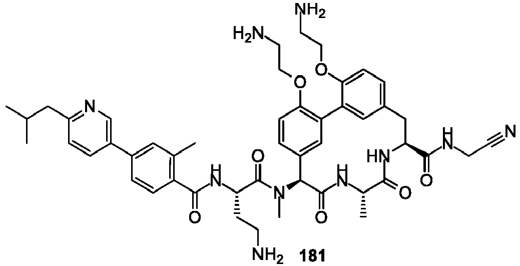

Compound 181 (formic acid salt) was prepared as a white solid utilizing the methods in Example 84 (Compound 178). LCMS (Method 5-95 AB, ESI): t_{R} = 0.503 min, [M+H]⁺ = 889.4.

### Example 88: Synthesis of Compound 182

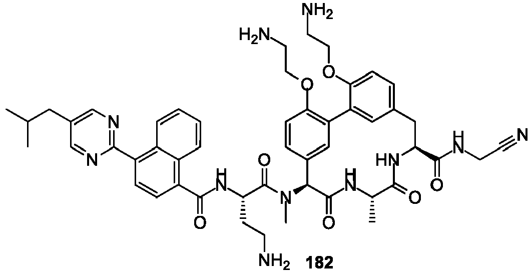

Compound 182 (formic acid salt) was prepared as a white solid utilizing the methods in Example 79 (Compound 173) from 2-chloro-5-isobutylpyrimidine and methyl 4-(4,4,5,5-tetramethyl-1,3,2-dioxaborolan-2-yl)-1-naphthoate. LCMS (Method 5-95 AB, ESI): t_{R} = 0.596 min, [M+H]⁺ = 926.3.

### Example 89: Synthesis of Compound 183

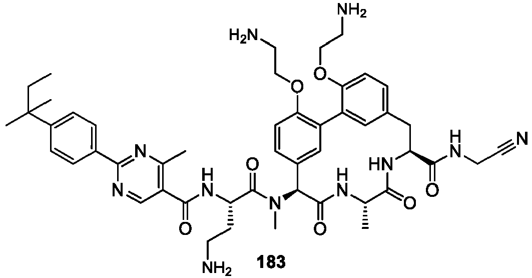

Compound 183 (formic acid salt) was prepared as a white solid utilizing the methods in Example 47 (Compound 141) from 4,4,5,5-tetramethyl-2-(4-(tert-pentyl)phenyl)-1,3,2-dioxaborolane. LCMS (Method 5-95 AB, ESI): t_{R} = 0.743 min, [M+H]⁺ = 905.2. ¹H NMR (400 MHz, CD₃OD): δ 8.79 (s, 1H), 8.52 (br s, 3H), 8.38 (d, J = 8.4 Hz, 2H), 7.50 (d, J = 8.8 Hz, 2H), 7.33 - 7.30 (m, 1H), 7.22 (d, J = 8.8 Hz, 1H), 7.18 (d, J = 8.8 Hz, 1H), 7.08 (d, J = 8.4 Hz, 1H), 6.90 (br s, 1H), 6.80 (br s, 1H), 6.39 (s, 1H), 5.19 - 5.17 (m, 1H), 4.82 - 4.75 (m, 1H), 4.20-4.13 (m, 7H), 3.15 - 3.05 (m, 8H), 2.95 (s, 3H), 2.70 (s, 3H), 2.30-2.10 (m, 2H), 1.74 (q, J = 7.3 Hz, 2H), 1.36 (s, 6H), 1.30-1.20 (m, 3H), 0.71 (t, J = 7.6 Hz, 3H).

### Example 90: Synthesis of Compound 184

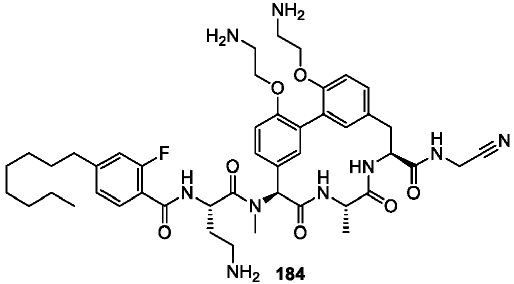

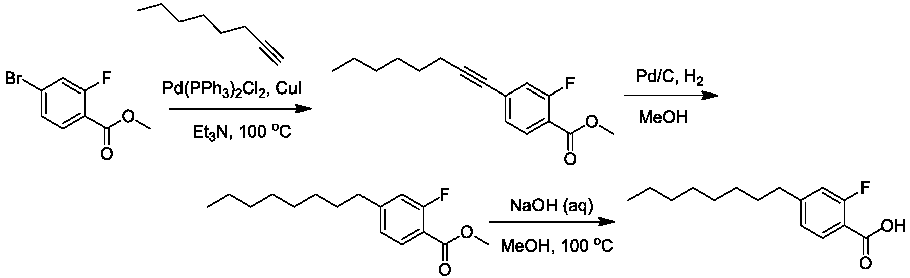

The Sonogashiro coupling of an acetylene to an aromatic halide, followed by reduction of the alkyne and hydrolysis of the ester is described and is referred to as General Method 8. A mixture of methyl 4-bromo-2-fluorobenzoate (500.0 mg, 2.15 mmol), oct-1-yne (702.9 mg, 6.44 mmol), bis(triphenylphosphine)palladium(II)dichloride (75.3 mg, 0.11 mmol) and copper(I) iodide (20.4 mg, 0.11 mmol) in triethylamine (9.83 mL, 70.9 mmol) was stirred at 100 °C for 2 h under nitrogen atmosphere. LCMS (5-95AB/1.5min): t_{R} = 1.006 min, [M + H]⁺ 262.9 showed 60% of DP. The reaction was quenched with water (15 mL) and extracted with dichloromethane (3 × 25 mL). The combined organic extracts were washed with brine (2 × 25 mL), dried over anhydrous Na₂SO₄ and filtered. The filtrate was concentrated and the residue was purified by column chromatography on silica gel (eluting with 5% ethyl acetate in petroleum ether, Rf = 0.5) to afford methyl 2-fluoro-4-(oct-1-yn-1-yl)benzoate (550 mg, 97.7% yield) as a brown solid. LCMS (5-95AB_1.5 min): t_{R} = 1.006 min, [M + H]⁺ 262.9.

To a solution of methyl 2-fluoro-4-(oct-1-yn-1-yl)benzoate (550.0 mg, 2.1 mmol) in methanol (25 mL) was added 10% Palladium on carbon (111.56 mg, 0.10 mmol). The mixture was stirred at 30 °C under hydrogen (40 psi) for 16h. The reaction was filtered over a pad of Celite and concentrated. The residue was purified by column chromatography on silica gel (eluting with petroleum ether / ethyl acetate from 100:1 to 10:1) to afford methyl 2-fluoro-4-octylbenzoate (500 mg, 89.5% yield) as a yellow solid. LCMS (5-95AB_1.5min): t_{R} = 1.033 min, [M + H]⁺ 266.

To a solution of methyl 2-fluoro-4-octylbenzoate (500.0 mg, 1.88 mmol) in methanol (5 mL) was added NaOH (1000.0 mg, 25 mmol) in water (5 mL). The mixture was stirred at 100 °C for 2h, cooled to RT and hydrochloric acid (1.0 M) was added until pH = 3-4. The mixture was extracted with ethyl acetate (3 × 30 mL). The combined organic extracts were washed with brine (2 × 30 mL), dried over sodium sulfate and filtered. The filtrate was concentrated to give 2-fluoro-4-octylbenzoic acid (450 mg, 95% yield) as a yellow solid. ¹H NMR (400 MHz, CDCl₃): δ 7.94 (t, *J* = 8.0 Hz, 1H), 7.06 (d, *J* = 8.4 Hz, 1H), 6.99 (d, *J* = 12.0 Hz, 1H), 2.66 (t, *J* = 7.4 Hz, 2H), 1.65 - 1.62 (m, 2H), 1.31 - 1.28 (m, 10H), 0.89 (t, *J* = 6.8 Hz, 3H).

Compound 184 (formic acid salt) was prepared as a white solid utilizing the methods in Example 7 (Compound 101) from 2-fluoro-4-octylbenzoic acid. LCMS (Method 5-95 AB, ESI): t_{R} = 0.775 min, [M + H]⁺ = 872.4.

### Example 91: Synthesis of Compound 185

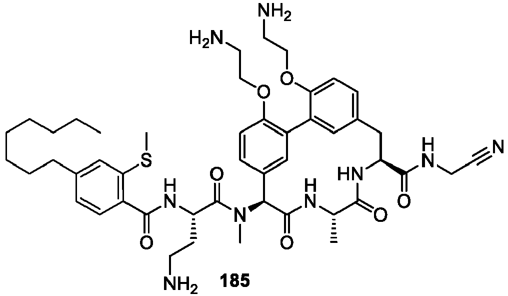

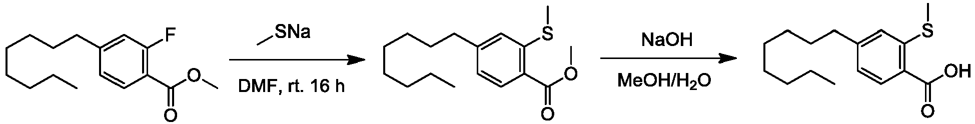

To a solution of sodium thiomethoxide (45.3 mg, 0.65 mmol) in N,N-dimethylformamide (3 mL) was added methyl 2-fluoro-4-octylbenzoate (86.0 mg, 0.32 mmol) and the mixture was stirred at 20 °C for 16 h. The mixture was diluted with water (5 mL) and extracted with EtOAc (10 mL × 3). The combined organic layers were dried over sodium sulfate, filtered and concentrated. The residue was purified by prep-TLC (10% EtOAc in petroleum ether, Rf = 0.7) to give methyl 2-(methylthio)-4-octylbenzoate (30 mg, 31.6% yield) as a yellow oil. ¹H NMR (400 MHz, CDCl₃): δ 7.90 (d, J = 8.0 Hz, 1H), 7.03 (s, 1H), 6.95 (d, J = 8.0 Hz, 1H), 3.88 (s, 3H), 2.62 (t, J = 7.6 Hz, 2H), 2.45 (s, 3H), 1.69 - 1.56 (m, 2H), 1.27 (m, 10H), 0.86 (t, J = 6.6 Hz, 3H).

Methyl 2-(methylthio)-4-octylbenzoate (30.0 mg, 0.10 mmol) was hydrolyzed with LiOH according to General Method 5 (Example 5) to give crude 2-(methylthio)-4-octylbenzoic acid (22 mg, 77% yield) as a yellow solid.

Compound 185 (formic acid salt) was prepared as a white solid utilizing the methods in Example 7 (Compound 101) from 2-(methylthio)-4-octylbenzoic acid. LCMS (Method 5-95 AB, ESI): t_{R} = 0.676 min, [M + H]⁺ = 900.4.

### Example 92: Synthesis of Compound 186

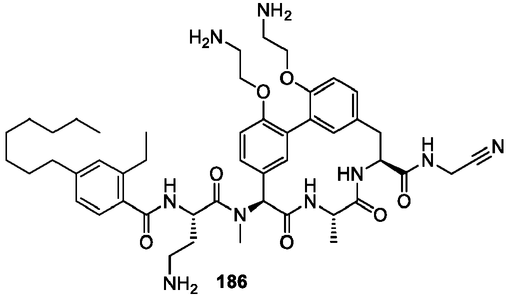

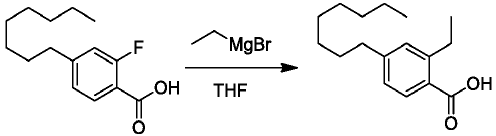

To a solution of 2-fluoro-4-octylbenzoic acid (120.0 mg, 0.48 mmol) in tetrahydrofuran (3 mL) was added ethylmagnesium bromide (0.55 mL, 1.66 mmol) in Et₂O (3 M) dropwise at 0 °C The mixture was stirred at 15 °C for 16 h. The reaction was quenched with water (2 mL) and extracted with EtOAc (20 mL × 3). The combined organic layers were washed with saturated brine (15 mL × 3), dried over Na₂SO₄ and concentrated. The residue was purified by prep-TLC (20% ethyl acetate in petroleum ether, Rf = 0.5) to give 2-ethyl-4-octylbenzoic acid (70 mg, 56.1% yield) as a white solid.

Compound 186 (formic acid salt) was prepared as a white solid utilizing the methods in Example 7 (Compound 101) from 2-ethyl-4-octylbenzoic acid. LCMS (Method 5-95 AB, ESI): t_{R} = 0.787 min, [M + H]⁺ = 882.5.

### Example 93: Synthesis of Compound 187

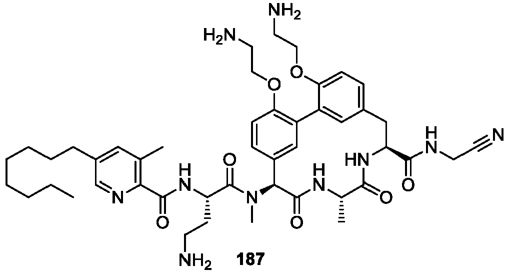

3-Methyl-5-octylpicolinic acid was prepared using the methods from Example 90 (Compound 184). LCMS (Method 5-95 AB, ESI): t_{R} = 0.730 min, [M + H]⁺ = 250.0.

Compound 187 (formic acid salt) was prepared as a white solid utilizing the methods in Example 90 (Compound 184) from 3-methyl-5-octylpicolinic acid. LCMS (Method 5-95 AB, ESI): t_{R} = 0.658 min, [M + H]⁺ = 869.6.

### Example 94: Synthesis of Compound 188

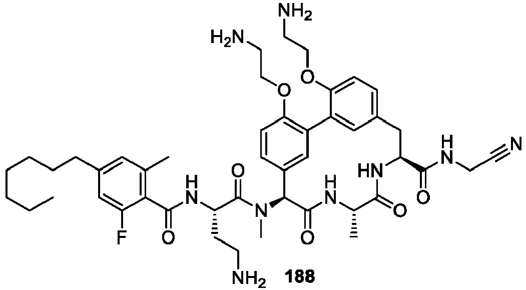

Compound 188 (formic acid salt) was prepared as a white solid utilizing the methods in Example 90 (Compound 184). LCMS (Method 5-95 AB, ESI): t_{R} = 0.614 min, [M + H]⁺ = 872.7.

### Example 95: Synthesis of Compound 189

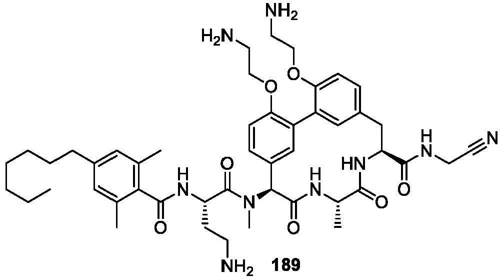

4-Heptyl-2,6-dimethylbenzoic acid a colorless oil, was prepared using the methods from Example 90 (Compound 184). ¹H NMR (400 MHz, CDCl₃) δ 6.76 (s, 2H), 2.44 (t, J = 7.6 Hz, 2H), 2.27 (s, 6H), 1.55-1.45 (m, 2H), 1.30-1.15 (m, 8H), 0.816 (t, J = 5.8 Hz, 3H).

Compound 189 (formic acid salt) was prepared as a white solid utilizing the methods in Example 90 (Compound 184) from 4-heptyl-2,6-dimethylbenzoic acid. LCMS (Method 5-95 AB, ESI): t_{R} = 0.776 min, [M + H]⁺ = 869.4.

### Example 96: Synthesis of Compound 190

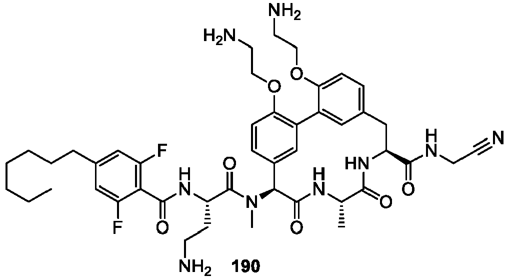

2,6-Difluoro-4-heptylbenzoic acid, a yellow solid, was prepared using the methods from Example 90 (Compound 184). LCMS (Method 5-95 AB, ESI): t_{R} = 0.873 min, [M + H]⁺ = 257.9.

Compound 190 (formic acid salt) was prepared as a white solid utilizing the methods in Example 90 (Compound 184) from 2,6-difluoro-4-heptylbenzoic acid. LCMS (Method 5-95 AB, ESI): t_{R} = 0.636 min, [M + H]⁺ = 876.4.

### Example 97: Synthesis of Compound 191

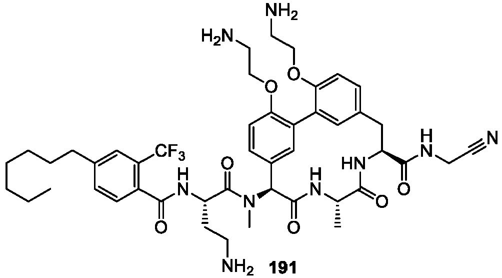

4-Heptyl-2-(trifluoromethyl)benzoic acid, a brown solid, was prepared using the methods from Example 90 (Compound 184). ¹H NMR (400 MHz, CD₃OD): δ 7.73 (d, J = 8.0 Hz, 1H), 7.61 (s, 1H), 7.53 (d, J = 8.0 Hz, 1H), 3.89 (s, 3H), 2.74 (t, J = 7.8 Hz, 2H), 1.70 - 1.60 (m, 2H), 1.40 - 1.20 (m, 8H), 0.90 (t, J = 6.8 Hz, 3H).

Compound 191 (formic acid salt) was prepared as a white solid utilizing the methods in Example 90 (Compound 184) from 4-heptyl-2-(trifluoromethyl)benzoic acid. LCMS (Method 5-95 AB, ESI): t_{R} = 0.640 min, [M + H]⁺ = 908.7.

### Example 98: Synthesis of Compound 192

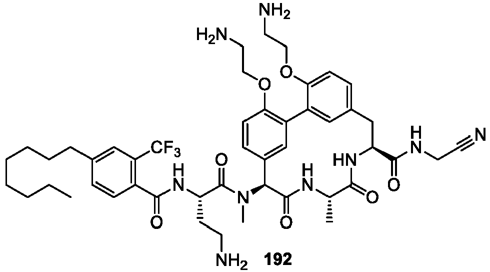

4-Octyl-2-(trifluoromethyl)benzoic acid, a yellow solid, was prepared using the methods from Example 90 (Compound 184). ¹H NMR (400 MHz, CD₃OD): δ 7.76 (d, J = 8.0 Hz, 1H), 7.59 (s, 1H), 7.51 (d, J = 7.2 Hz, 1H), 2.74 (t, J = 7.6 Hz, 2H), 1.70 - 1.60 (m, 2H), 1.40 - 1.20 (m, 10H), 0.90 (t, J = 7.0 Hz, 3H).

Compound 192 (formic acid salt) was prepared as a white solid utilizing the methods in Example 90 (Compound 184). LCMS (Method 5-95 AB, ESI): t_{R} = 0.664 min, [M + H]⁺ = 922.4.

### Example 99: Synthesis of Compound 193

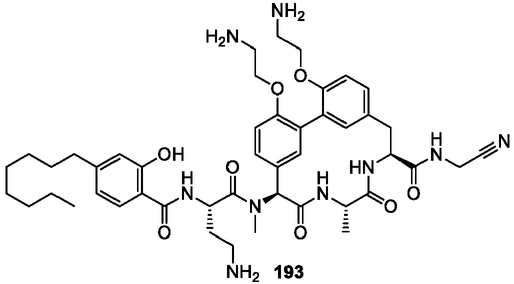

Compound 193 (formic acid salt) was prepared as a white solid utilizing the methods in Example 90 (Compound 184). LCMS (Method 5-95 AB, ESI): t_{R} = 0.637 min, [M + H]⁺ = 871.3.

### Example 100: Synthesis of Compound 194

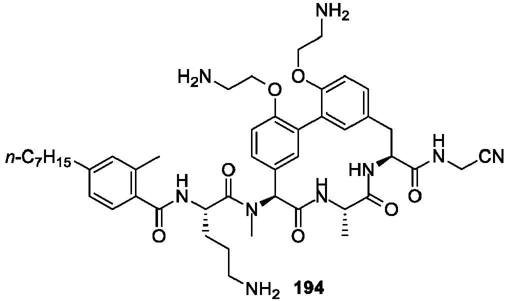

Compound 194 (formic acid salt) was prepared as a white solid (42.7 mg) utilizing the methods in Example 90 (Compound 184). LCMS (Method 5-95 AB, ESI): t_{R} = 0.633, [M + H]⁺ = 868.5; ¹H NMR (400 MHz, MeOH-*d*₄) δ 8.51 (brs, 2H, HCOOH), 7.36-7.27 (m, 2H), 7.24 (d, *J*=8.0 Hz, 1H), 7.17 (d, *J*=8.0 Hz, 1H), 7.13-7.06 (m, 3H), 6.90 (brs, 1H), 6.83 (brs, 1H), 6.42 (s, 1H), 5.04-5.00 (m, 1H), 4.81-4.77 (m, 2H), 4.27-4.15 (m, 4H), 4.20 (s, 2H), 3.36-3.30 (m, 1H), 3.22-3.10 (m, 5H), 3.05-2.93 (m, 2H), 2.99 (s, 3H), 2.61 (t, *J*=7.6 Hz, 2H), 2.45 (s, 3H), 2.03-1.95 (m, 2H), 1.90-1.80 (m, 3H), 1.65-1.58 (m, 2H), 1.40-1.25 (m, 10H), 0.90 (t, *J*=6.4 Hz, 3H).

### Example 101: Synthesis of Compound 195

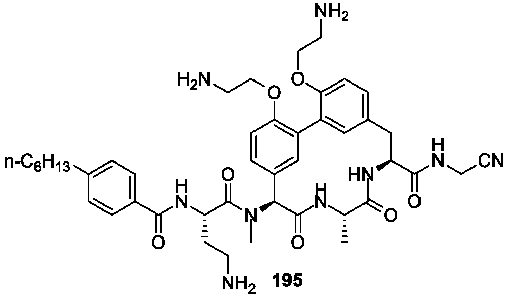

Compound 195 (formic acid salt) was prepared as a white solid utilizing the methods in Example 90 (Compound 184). LCMS (Method 5-95 AB, ESI): t_{R} = 0.590, [M + H]⁺ = 826.4.

### Example 102: Synthesis of Compound 196

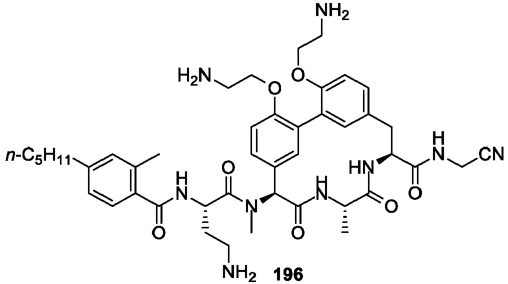

Compound 196 (formic acid salt) was prepared as a white solid utilizing the methods in Example 90 (Compound 184). LCMS (Method 5-95 AB, ESI): t_{R} = 0.704, [M + H]⁺ = 826.5.

### Example 103: Synthesis of Compound 197

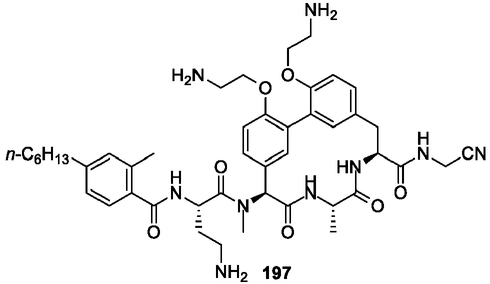

Compound 197 (formic acid salt) was prepared as a white solid utilizing the methods in Example 90 (Compound 184). LCMS (Method 5-95 AB, ESI): t_{R} = 0.584, [M + H]⁺ = 840.5.

### Example 104: Synthesis of Compound 198

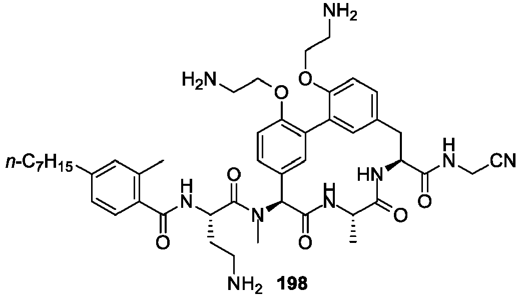

Compound 198 (formic acid salt) was prepared as a white solid utilizing the methods in Example 90 (Compound 184). LCMS (Method 5-95 AB, ESI): t_{R} = 0.753, [M + H]⁺ = 854.4; ¹H NMR (400 MHz, MeOH-*d*₄) δ 8.49 (brs, 1H), 7.34 (d, *J*=8.0 Hz, 2H), 7.26 (d, *J*=8.0 Hz, 1H), 7.13 (brs, 1H), 7.11 (d, *J*=8.0 Hz, 2H), 6.92 (brs, 1H), 6.83 (brs, 1H), 6.35 (s, 1H), 5.17-5.14 (m, 2H), 4.83-4.81 (m, 1H), 4.30-4.20 (m, 4H), 4.22 (s, 2H), 3.32-3.13 (m, 8H), 2.95 (s, 3H), 2.64 (t, *J*=7.2 Hz, 2H), 2.43 (s, 3H), 2.40-2.25 (m, 1H), 2.15-2.02 (m, 1H), 1.64 (brs, 2H), 1.40-1.20 (m, 11H), 0.92 (t, *J*=6.8 Hz, 3H).

### Example 105: Synthesis of Compound 199

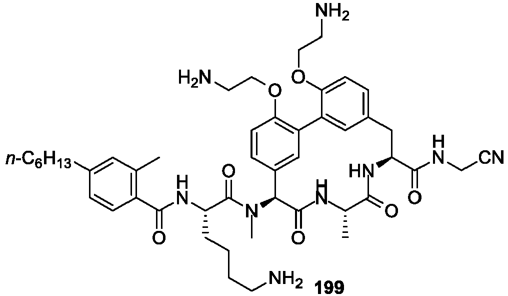

Compound 199 was prepared as a white solid utilizing the methods in Example 90 (Compound 184). LCMS (Method 5-95 AB, ESI): t_{R} = 0.596, M+H⁺ = 868.4.

### Example 106: Synthesis of Compound 200

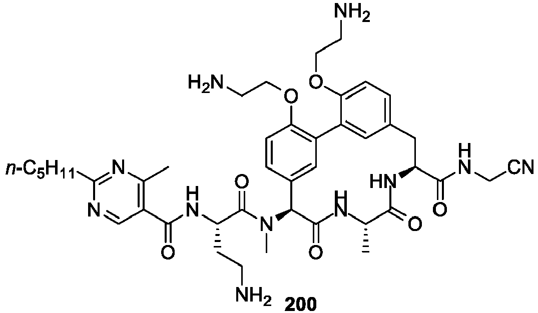

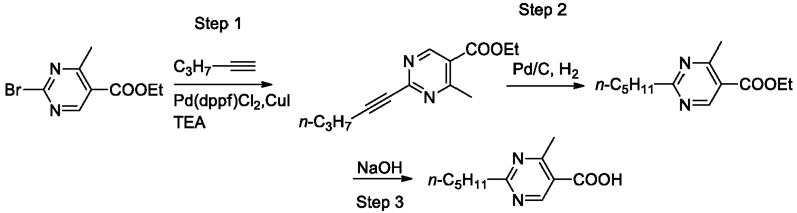

Steps 1-3: Starting from ethyl 2-bromo-4-methylpyrimidine-5-carboxylate, the standard procedure of Sonogashiro coupling, hydrogenation and ester hydrolysis (General Method Sonogashiro) afforded 4-methyl-2-pentylpyrimidine-5-carboxylic acid (105 mg) as a yellow solid. ¹H NMR (400 MHz, DMSO-*d*6) δ 8.97 (s, 1 H), 2.85 (d, *J*=7.6 Hz, 2H), 2.69 (s, 3H), 1.79-1.72 (m, 2H), 1.32-1.28 (m, 4H), 0.89-0.83 (t, *J*=6.8 Hz, 3H).

Compound 200 (formic acid salt) was prepared as a white solid utilizing the methods in Example 90 (Compound 184). LCMS (Method 5-95 AB, ESI): t_{R} = 0.492, [M + H]⁺ = 828.1; ¹H NMR (400 MHz, MeOH-*d*₄) δ 8.70 (s, 1H), 8.50 (brs, 2H, HCOOH), 7.33 (d, *J*=8.0, 1H), 7.26 (d, *J*=8.0, 1H), 7.20 (d, *J*=8.4, 1H), 7.11(d, *J*=8.4, 1H), 6.92 (brs, 1H), 6.84 (brs, 1H), 6.39 (s, 1H), 5.19-5.15 (m, 1H), 4.80-4.70 (m, 2H),4.27-4.18 (m, 4H), 4.22 (s, 2H), 3.33-3.12 (m, 9H), 2.96 (s, 3H), 2.95 (t, *J*=8.0 Hz, 2H), 2.65 (s, 3H), 2.29-2.19 (m, 1H), 2.17-2.05 (m, 1H), 1.86-1.78 (m, 2H), 1.40-1.25 (m, 7H), 0.94 (t, *J*=6.4 Hz, 3H).

### Example 107: Synthesis of Compound 201

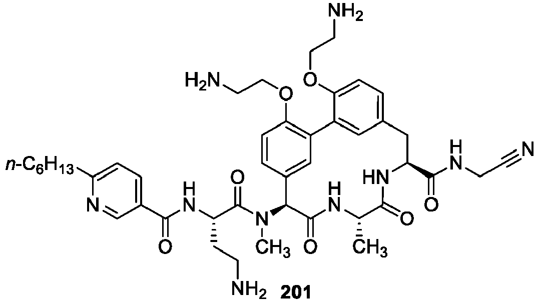

Compound 201 (formic acid salt) was prepared as a white solid utilizing the methods in Example 90 (Compound 184) from 6-hexylnicotinic acid. LCMS (Method 5-95 AB, ESI): t_{R} = 0.537, [M + H]⁺ = 827.3; ¹H NMR (400 MHz, MeOH-*d*₄) δ 8.92 (d, *J*=2 Hz, 1H), 8.48 (brs, 2H, HCOOH), 8.21 (d, *J*=8.0 Hz, 1H), 7.46 (d, *J*=8.0 Hz, 1H), 7.32 (d, *J*=8.0 Hz, 1H), 7.25 (d, *J*=8.0 Hz, 1H), 7.17 (d, *J*=8.0 Hz, 1H), 7.09 (d, *J*=8.0Hz, 1H), 6.88 (brs, 1H), 6.81 (brs, 1H), 6.34 (s, 1H), 5.18-5.14 (m, 2H), 4.8-4.77 (m, 1H), 4.26-4.10 (m, 4H), 4.20 (s, 2H), 3.37-3.33 (m, 1H), 3.22-3.17 (m, 5H), 3.14-3.10 (m, 3H), 2.89-2.85 (m, 4H), 2.33-2.20 (m, 1H), 2.20-2.05 (m, 1H), 1.76-1.60 (m, 3H), 1.40-1.20 (m, 8H), 0.91 (t, *J*=6.6 Hz, 3H).

### Example 108: Synthesis of Compound 202

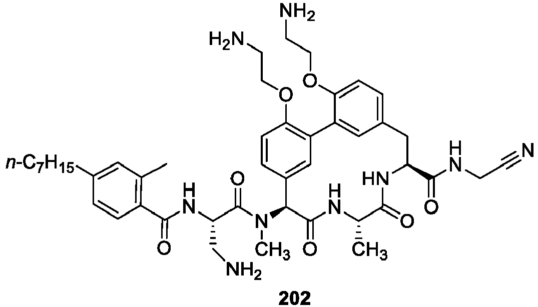

Compound 202 (formic acid salt) was prepared as a white solid utilizing the methods in Example 90 (Compound 184). LCMS (Method 5-95 AB, ESI): t_{R} = 0.749 min, [M+H]⁺ = 840.5.

### Example 109: Synthesis of Compound 203

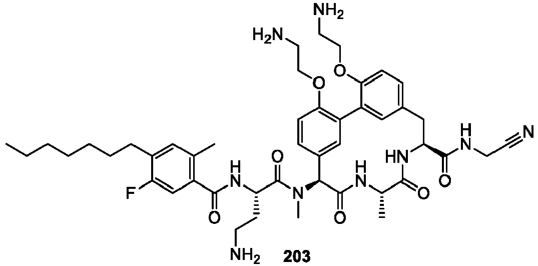

Compound 203 (formic acid salt) was prepared as a white solid utilizing the methods in Example 90 (Compound 184). LCMS (Method 5-95 AB, ESI): t_{R} = 0.641 min, [M+H]⁺ = 872.4.

### Example 110: Synthesis of Compound 204

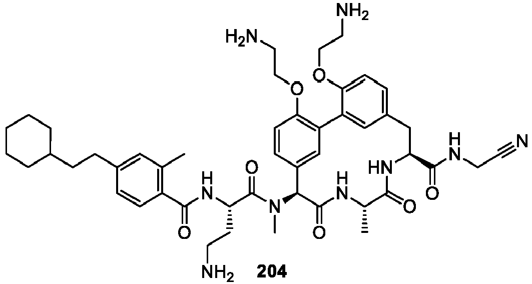

Compound 204 (formic acid salt) was prepared as a white solid utilizing the methods in Example 90 (Compound 184). LCMS (Method 5-95 AB, ESI): t_{R} = 0.636 min, [M+H]⁺ = 866.6.

### Example 111: Synthesis of Compound 205

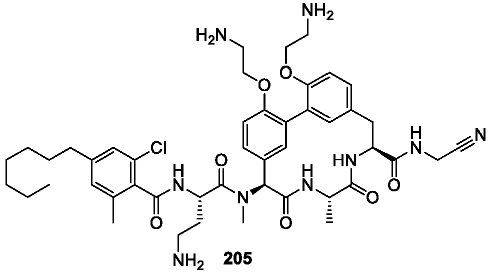

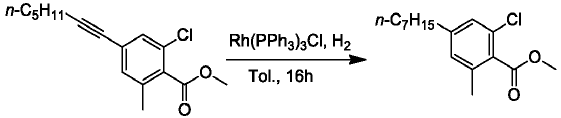

Compound 205 (formic acid salt) was prepared utilizing the methods in Example 90 (Compound 184) except the alkyne reduction was performed as follows. To a solution of methyl 2-chloro-4-(hept-1-yn-1-yl)-6-methylbenzoate (250.0 mg, 0.90 mmol) in toluene (10 mL) was added chlorotris(triphenylphosphine)rhodium(I) (124.5 mg, 0.13 mmol). The mixture was stirred under 15 psi hydrogen pressure at 60 °C for 16 h, filtered and evaporated. The residue was purified by chromatography on silica (0-5% EtOAc in petroleum ether) to afford methyl 2-chloro-4-heptyl-6-methylbenzoate (200 mg, 78.9% yield) as a yellow solid. ¹H NMR (400 MHz, CD₃OD): δ 7.10 (s, 1H), 7.03 (s, 1H), 3.90 (s, 3H), 2.58 (t, J = 7.8 Hz, 2H), 2.27 (s, 3H), 1.65 - 1.55 (m, 2H), 1.35 - 1.25 (m, 8H), 0.90 (t, J = 7.0 Hz, 3H).

Data for Compound 205: LCMS (Method 5-95 AB, ESI): t_{R} = 0.640 min, [M+H]⁺ = 888.5.

### Example 112: Synthesis of Compound 206

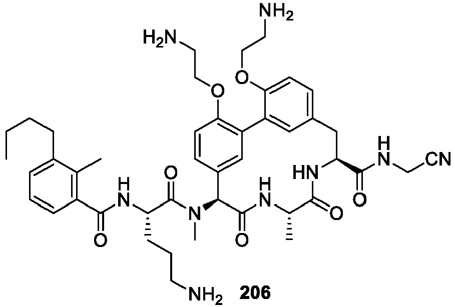

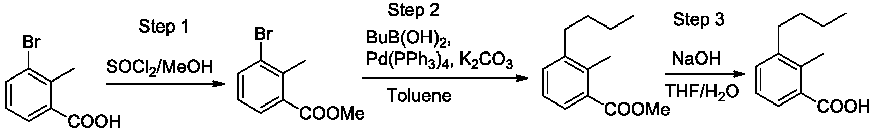

Step 1: To a mixture of 3-bromo-2-methylbenzoic acid (5.0 g, 23 mmol) in MeOH (80 mL) was added SOCl₂ (11.0 g, 93 mmol) at 20°C. The mixture was stirred for 1.5h at 70°C. The volatiles were removed and the residue was taken up by EtOAc (100 mL), which was washed sequentially with saturated NaHCO₃ and brine (each 100 mL). The EtOAc layer was dried over Na₂SO₄, concentrated and the residue was purified by flash column chromatography to give methyl 3-bromo-2-methylbenzoate (5.3g, 99% yield) as a red solid.

Step 2: A solution of methyl 3-bromo-2-methylbenzoate (500 mg, 2.2 mmol), n-butyl boronic acid (890 mg, 8.7 mmol), Pd(PPh₃)₄ (252 mg, 0.22 mmol) and K₂CO₃ (905 mg, 6.6 mmol) in toluene (20 mL) was stirred at 100°C for 4 h. After filtration, the filtrate was washed with brine (20 mL x 3), dried over Na₂SO₄ and concentrated. The residue was purified by HPLC to give methyl 3-butyl-2-methylbenzoate (120 mg, 27% yield) as colorless oil. LCMS (Method 5-95 AB, ESI): t_{R} = 0.871, [M + H]⁺ = 206.9.

Step 3: The ester hydrolysis method with NaOH (General Method NaOH) previously described (Example 47) was applied to methyl 3-butyl-2-methylbenzoate (120 mg, 0.58 mmol) to afford 3-butyl-2-methylbenzoic acid (110 mg, 98% yield) as a white solid.

Compound 206 (formic acid salt) was prepared as a white solid utilizing the methods in Example 7 (Compound 101) from 3-butyl-2-methylbenzoic acid. LCMS (Method 5-95 AB, ESI): t_{R} = 0.541, [M + H]⁺ = 826.3.

### Example 113: Synthesis of Compound 207

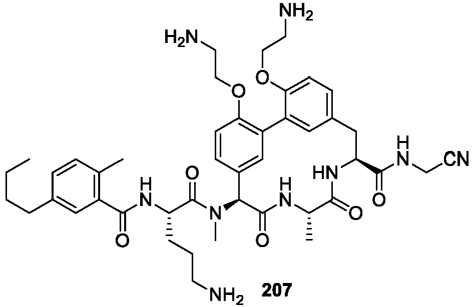

Compound 207 (formic acid salt) was prepared as a white solid utilizing the methods in Example 112 (Compound 206) from 5-butyl-2-methylbenzoic acid. LCMS (Method 5-95 AB, ESI): t_{R} = 0.554, [M + H]⁺ = 826.2.

### Example 114: Synthesis of Compound 208

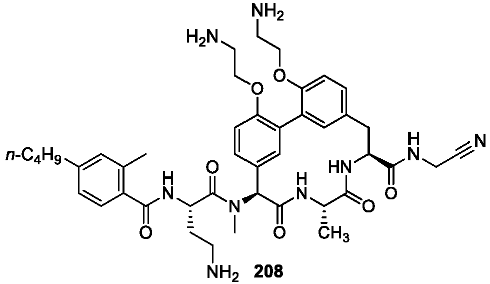

Compound 208 (formic acid salt) was prepared as a white solid utilizing the methods in Example 112 (Compound 206). LCMS (Method 5-95 AB, ESI): t_{R} = 0.546, [M + H]⁺ = 812.3.

### Example 115: Synthesis of Compound 209

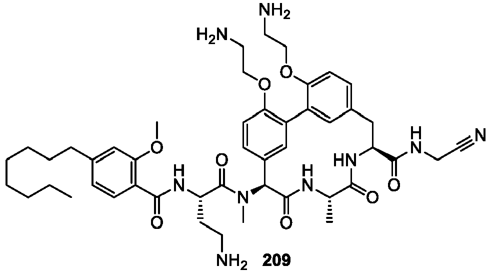

Compound 209 (formic acid salt) was prepared as a white solid utilizing the methods in Example 112 (Compound 206) from 2-methoxy-4-octylbenzoic acid. LCMS (Method 5-95 AB, ESI): t_{R} = 0.782 min, [M/2+H]⁺ = 442.9.

### Example 116: Synthesis of Compound 210

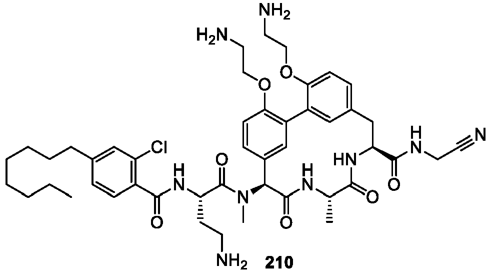

Compound 210 (formic acid salt) was prepared as a white solid utilizing the methods in Example 112 (Compound 206). LCMS (Method 5-95 AB, ESI): t_{R} = 0.772 min, [M/2+H]⁺ = 444.8.

### Example 117: Synthesis of Compound 211

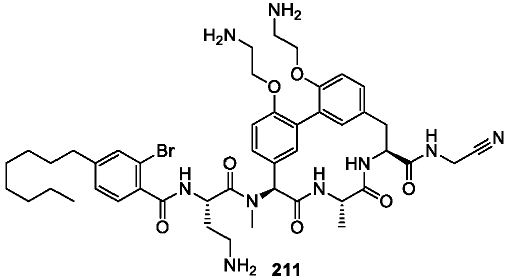

Following similar procedures as described for Compound 206 (Example 112), 2-bromo-4-octylbenzoic acid (180 mg, 0.5747 mmol, 94% yield) was obtained as a white solid from 2-bromo-4-iodobenzoic acid. ¹H NMR (400 MHz, CD₃OD): δ 7.74 (d, J = 8.0 Hz, 1H), 7.52 (s, 1H), 7.30 - 7.20 (m, 1H), 2.64 (t, J = 7.8 Hz, 2H), 1.65 - 1.55 (m, 2H), 1.35 - 1.20 (m, 10H), 0.90 (t, J = 6.8 Hz, 3H).

Compound 211 (formic acid salt) was prepared as a white solid utilizing the methods in Example 112 (Compound 206) from 2-bromo-4-octylbenzoic acid. LCMS (Method 5-95 AB, ESI): t_{R} = 0.626 min, [M + H]⁺ = 932.3.

### Example 118: Synthesis of Compound 212

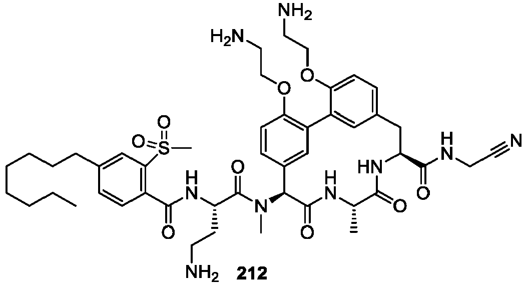

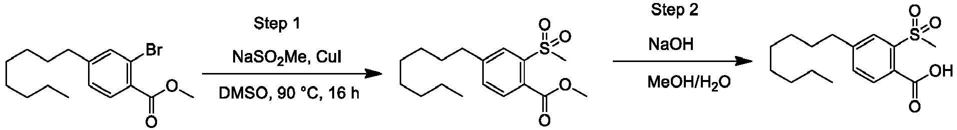

Step 1: To a solution of methyl 2-bromo-4-octylbenzoate (100.0 mg, 0.31 mmol) in dimethyl sulfoxide (2 mL) were added copper(I) iodide (174.6 mg, 0.92 mmol) and sodium methanesulfinate (93.6 mg, 0.92 mmol). The mixture was stirred at 100 °C for 16 h under N₂, diluted with water (10 mL) and extracted with EtOAc (10 mL x 3). The combined organic layers were dried over anhydrous sodium sulfate and concentrated in vacuo. The residue was purified by prep-TLC (20% EtOAc in petroleum ether, Rf = 0.3) to afford methyl 2-methylsulfonyl-4-octylbenzoate (80 mg, 80.2% yield) as a white solid. ¹H NMR (400 MHz, CDCl₃):δ7.95 (s, 1H), 7.65 (d, J = 7.6 Hz, 1H), 7.48 (d, J = 7.2 Hz, 1H), 3.97 (s, 3H), 3.37 (s, 3H), 2.72 (t, J = 7.8 Hz, 2H), 1.70-1.60 (m, 2H), 1.40-1.20 (m, 10H), 0.89 (t, J = 6.4 Hz, 3H).

Step 2: 2-(Methylsulfonyl)-4-octylbenzoic acid was prepared from methyl 2-(methylsulfonyl)-4-octylbenzoate utilizing General Method NaOH.

Compound 212 (formic acid salt) was prepared as a white solid utilizing the methods in Example 112 (Compound 206) from 2-(methylsulfonyl)-4-octylbenzoic acid. LCMS (Method 5-95 AB, ESI): t_{R} = 0.759 min, [M + H]⁺ = 932.5.

### Example 119: Synthesis of Compound 213

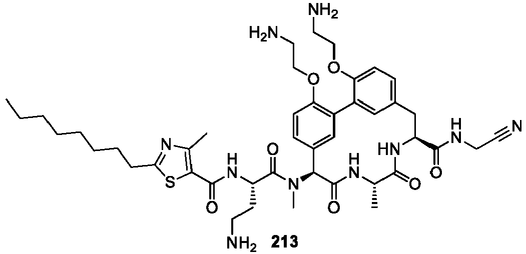

4-Methyl-2-octylthiazole-5-carboxylic acid, a white solid, was prepared using the methods in Example 112 (Compound 206) from ethyl 2-bromo-4-methylthiazole-5-carboxylate. ¹H NMR (400 MHz, CDCl₃): δ 3.10 - 2.90 (m, 2H), 2.74 (s, 3H), 1.90 - 1.70 (m, 2H), 1.46 - 1.24 (m, 10H), 0.89 (t, 6.6 Hz, 3H).

Compound 213 (formic acid salt) was prepared as a white solid utilizing the methods in Example 112 (Compound 206). LCMS (Method 5-95 AB, ESI): t_{R} = 0.775 min, [M + H]⁺ = 875.6.

### Example 120: Synthesis of Compound 214

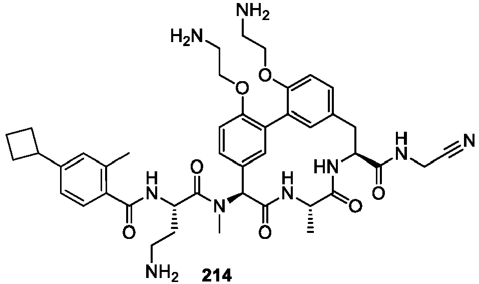

4-Cyclobutyl-2-methylbenzoic acid, a white solid, was prepared using the methods in Example 112 (Compound 206). ¹HNMR (400 MHz, CDCl₃): δ 8.01 (d, *J* = 8.0 Hz, 1H), 7.14 - 7.11 (m, 2H), 3.57 (p, *J* = 8.8 Hz, 1H), 2.65 (s, 3H), 2.41 -2.37 (m, 2H), 2.20 - 2.17 (m, 2H), 2.16 -2.07 (m, 1H), 1.95 - 1.84 (m, 1H).

Compound 214 (formic acid salt) was prepared as a white solid utilizing the methods in Example 112 (Compound 206) from 4-cyclobutyl-2-methylbenzoic acid. LCMS (Method 5-95 AB, ESI): t_{R} = 0.682 min, [M + H]⁺ = 810.7.

### Example 121: Synthesis of Compound 215

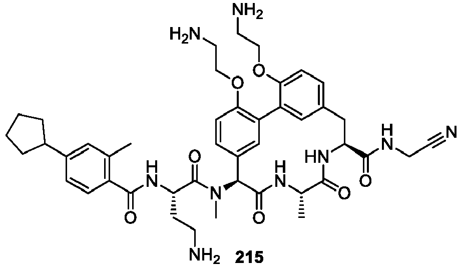

Compound 215 (formic acid salt) was prepared as a white solid utilizing the methods in Example 112 (Compound 206) from 4-cyclopentyl-2-methylbenzoic acid. LCMS (Method 5-95 AB, ESI): t_{R} = 0.550 min, [M + H]⁺ = 824.3.

### Example 122: Synthesis of Compound 216

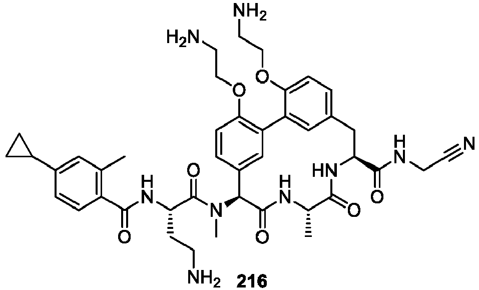

Compound 216 (formic acid salt) was prepared as a white solid utilizing the methods in Example 112 (Compound 206) from 4-cyclopropyl-2-methylbenzoic acid. LCMS (Method 5-95 AB, ESI): t_{R} = 0.479 min, [M + H]⁺ = 797.2.

### Example 123: Synthesis of Compound 217

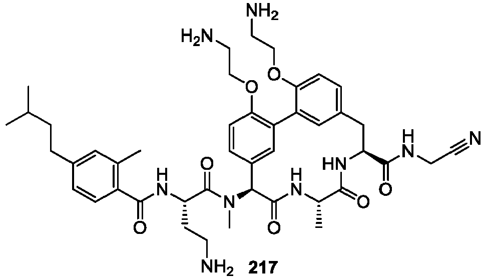

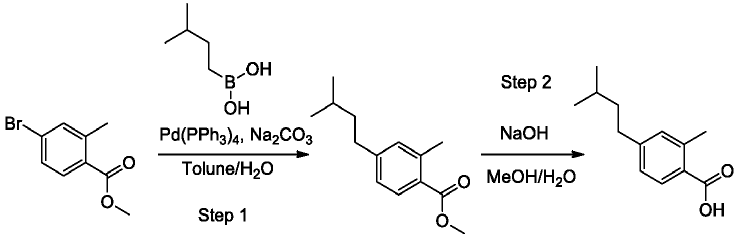

Step 1: To a solution of tetrakis(triphenylphosphine)palladium(0) (151.3 mg, 0.130 mmol) in toluene (10 mL) and water (2 mL) were added 3-methyl-1-butylboronic acid (759.4 mg, 6.55 mmol), methyl4-bromo-2-methylbenzoate (300 mg, 1.31 mmol) and sodium carbonate (694 mg, 6.55 mmol). The reaction mixture was stirred at 110 °C for 16h. The mixture was filtered and the filtrate was diluted with H₂O (30 mL) and extracted with EtOAc (30 mL x 2). The organic layers were combined and washed with water (60 mL x 2) and brine (30 mL). The organic layers were separated, dried over Na₂SO₄ and concentrated. The residue was purified by preparative TLC (5% EtOAc in petroleum ether) to obtain methyl 4-isopentyl-2-methylbenzoate (250 mg, 1.1348 mmol, 86.6% yield) as a colorless oil.

Step 2: Methyl 4-isopentyl-2-methylbenzoate (250 mg, 1.13 mmol) was hydrolyzed as previously described (General Method NaOH) to give crude 4-isopentyl-2-methylbenzoic acid (200 mg, 0.970 mmol, 85.4% yield) as a yellow solid.

Compound 217 (formic acid salt) was prepared as a white solid utilizing the methods in Example 7 (Compound 101). LCMS (Method 5-95 AB, ESI): t_{R} = 0.732 min, [M + H]⁺ = 826.5.

### Example 124: Synthesis of Compound 218

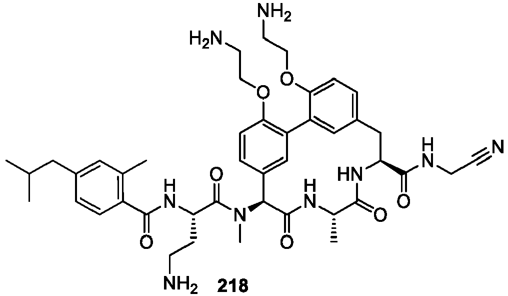

4-Isobutyl-2-methylbenzoic acid a white solid, was prepared using the methods from Example 112 (Compound 206). ¹H NMR (400 MHz, CDCl₃): δ 8.00 (d, J = 8.0 Hz, 1H), 7.14 - 6.94 (m, 2H), 2.65 (s, 3H), 2.50 (d, J = 6.8 Hz, 2H), 1.95-1.80 (m, 1H), 0.92 (d, J = 6.5 Hz, 6H).

Compound 218 (formic acid salt) was prepared as a white solid utilizing the methods in Example 112 (Compound 206). LCMS (Method 5-95 AB, ESI): t_{R} = 0.701 min, [M + H]⁺ = 812.4.

### Example 125: Synthesis of Compound 219

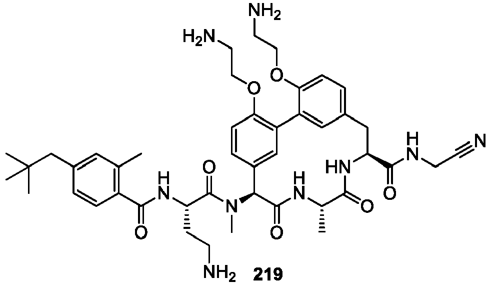

2-Methyl-4-neopentylbenzoic acid, a white solid, was prepared using the methods from Example 112 (Compound 206). LCMS (Method 5-95 AB, ESI): t_{R} = 0.807 min, [M + H]⁺ = 206.8.

Compound 219 (formic acid salt) was prepared as a white solid utilizing the methods in Example 112 (Compound 206) from 2-methyl-4-neopentylbenzoic acid. LCMS (Method 5-95 AB, ESI): t_{R} = 0.573 min, [M + H]⁺ = 826.3.

### Example 126: Synthesis of Compound 220

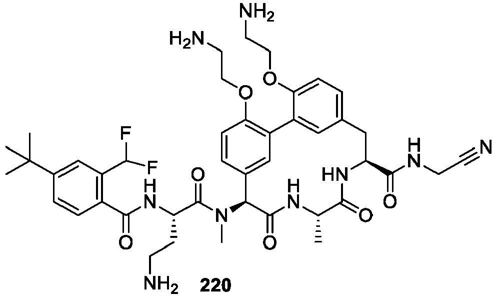

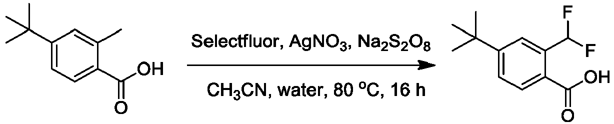

To a degassed mixture of 4-tert-butyl-2-methyl-benzoic acid (192.0 mg, 1 mmol), sodium persulfate (1.19 g, 4.99 mmol) and Selectfluor (1.77 g, 4.99 mmol) in acetonitrile (4 mL) and water (4 mL) in dry-ice acetone bath was added silvernitrate (17.0 mg, 0.10 mmol). The mixture was degassed by three freeze-pump-thaw cycles and heated at 80 °C for 16 h. Water (10 mL) was added and the mixture was extracted with EtOAc (15 mL x 2). The combined organic layers were concentrated and the residue was purified by prep-TLC (petroleum ether/EtOAc/HOAc 2/1/0.01, Rf = 0.3) to give 4-(tert-butyl)-2-(difluoromethyl)benzoic acid (75 mg, 32.9% yield) as a white solid.

Compound 220 (formic acid salt) was prepared as a white solid utilizing the methods in Example 7 (Compound 101). LCMS (Method 5-95 AB, ESI): t_{R} = 0.593 min, [M + H]⁺ = 848.3.

### Example 127: Synthesis of Compound 221

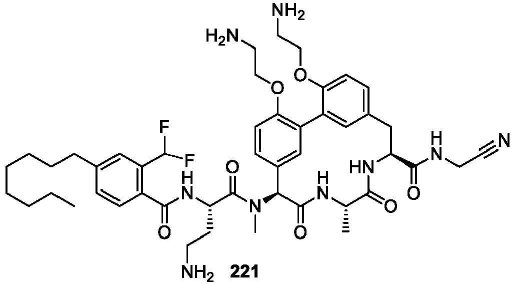

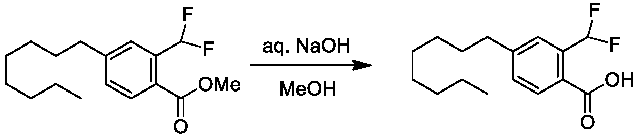

Methyl 2-(difluoromethyl)-4-octylbenzoate, a colorless oil, was prepared following similar procedures as described in Example 126 (Compound 220) and Example 112 (Compound 206). ¹H NMR (400 MHz, CDCl₃) δ 7.94 (d, J = 7.6 Hz, 1H), 7.61 (s, 1H), 7.52 (t, J = 55.8 Hz, 1H), 7.32 (d, J = 8.0 Hz, 1H), 3.90 (s, 3H), 2.68 (t, J = 7.8 Hz, 2H), 1.64 - 1.58 (m, 2H), 1.40 - 1.10 (m, 10H), 0.86 (t, J = 6.6 Hz, 3H). Methyl 2-(difluoromethyl)-4-octylbenzoate was hydrolyzed as previously described (General Method NaOH) to give 2-(difluoromethyl)-4-octylbenzoic acid.

Compound 221 (formic acid salt) was prepared as a white solid utilizing the methods in Example 126 (Compound 220) and Example 112 (Compound 206). LCMS (Method 5-95 AB, ESI): t_{R} = 0.780 min, [M + H]⁺ = 904.8.

### Example 128 Synthesis of Compound 222

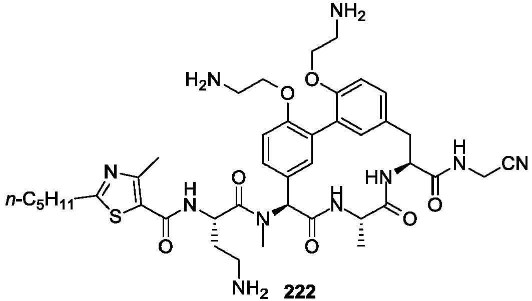

Compound 222 (formic acid salt) was prepared as a white solid utilizing the methods in Example 112 (Compound 206) from 4-methyl-2-pentylthiazole-5-carboxylic acid. LCMS (Method 5-95 AB, ESI): t_{R} = 0.643, [M + H]⁺ = 833.5; ¹H NMR (400 MHz, MeOH-*d*₄) δ 8.49 (brs, 2H, HCOOH), 7.33 (d, *J*=8.0 Hz, 1H), 7.27 (d, *J*=8.0 Hz, 1H), 7.19 (d, *J*=8.0 Hz, 1H), 7.11 (d, *J*=8.0 Hz, 1H), 6.91 (brs, 1H), 6.84 (brs, 1H), 6.34 (s, 1H), 5.12-5.09 (m, 1H), 4.82-4.81 (m, 2H), 4.28-4.20 (m, 4H), 4.21 (s, 2H), 3.35-3.25 (m, 1H), 3.22-3.09 (m, 7H), 3.03 (t, *J*=7.2 Hz, 2H), 2.90 (s, 3H), 2.64 (s, 3H), 2.31-2.25 (m, 1H), 2.15-2.11 (m, 1H), 1.84-1.80 (m, 2H), 1.42-1.37 (m, 7H), 0.98 (t, *J*=6.8 Hz, 3H).

### Example 129: Synthesis of Compound 223

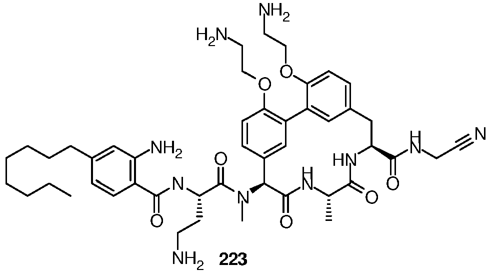

Compound 223 (formic acid salt) was prepared as a white solid utilizing the methods in Example 112 (Compound 206). LCMS (Method 5-95 AB, ESI): t_{R} = 0.760 min, [M+H]⁺ = 869.6.

### Example 130: Synthesis of Compound 224

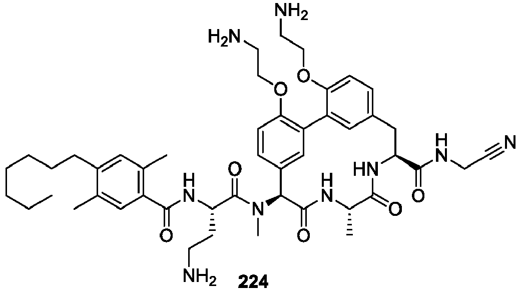

Compound 224 (formic acid salt) was prepared as a white solid utilizing the methods in Example 112 (Compound 206). LCMS (Method 5-95 AB, ESI): t_{R} = 0.758 min, [M+H]⁺ = 868.4.

### Example 131: Synthesis of Compound 225

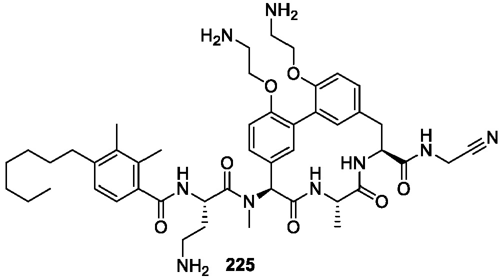

Compound 225 (formic acid salt) was prepared as a white solid utilizing the methods in Example 112 (Compound 206). LCMS (Method 5-95 AB, ESI): t_{R} = 0.753 min, [M+H]⁺ = 869.5.

### Example 132: Synthesis of Compound 226

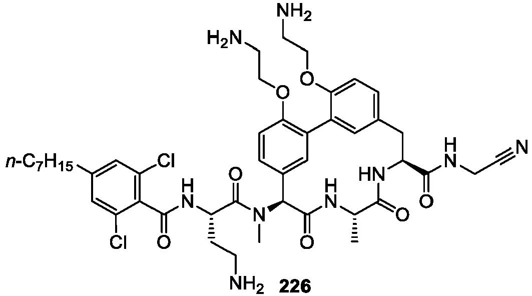

Compound 226 (formic acid salt) was prepared as a white solid utilizing the methods in Example 112 (Compound 206). LCMS (Method 5-95 AB, ESI): t_{R} = 0.753 min, [M+H]⁺ = 908.4.

### Example 133: Synthesis of Compound 227

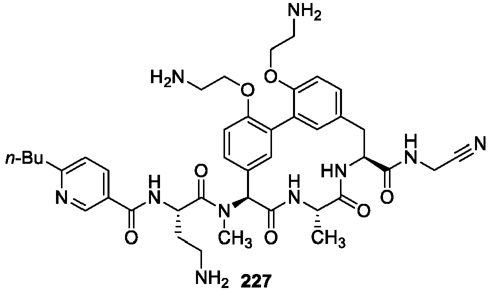

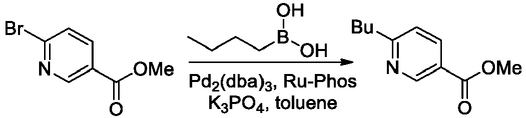

A solution of methyl 6-bromonicotinate (500 mg, 2.3 mmol), n-butyl boronic acid (708 mg, 6.9 mmol), Pd₂(dba)₃ (212 mg, 0.23 mmol), RuPhos (108 mg, 0.23 mmol) and K₃PO₄ (1.47 g, 6.9 mmol) was stirred for under N₂ at 100 °C overnight. The volatiles were removed and the resulting residue was purified by silica gel flash column to afford methyl 6-butylnicotinate (330 mg, 73.8% yield) as colorless oil. LCMS (Method 5-95 AB, ESI): t_{R} = 0.733, [M + H]⁺ = 194.0.

Compound 227 (formic acid salt) was prepared as a white solid utilizing the methods in Example 112 (Compound 206) from methyl 6-butylnicotinate. LCMS (0-60 AB, 2 min, ESI): t_{R} = 0.828, [M + H]⁺ = 799.5.

### Example 134: Synthesis of Compound 228

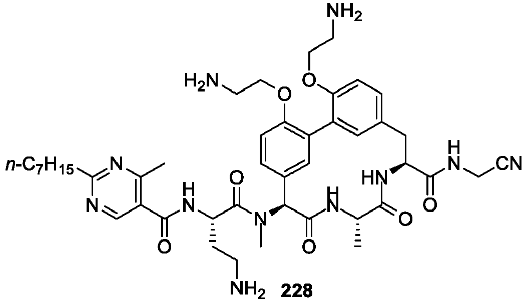

Compound 228 (formic acid salt) was prepared as a white solid utilizing the methods in Example 133 (Compound 227). LCMS (Method 5-95 AB, ESI): t_{R} = 0.566 min, [M+H]⁺ = 856.6.

### Example 135: Synthesis of Compound 229

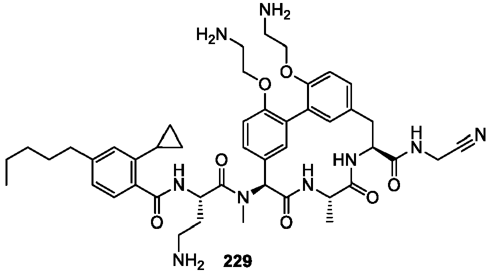

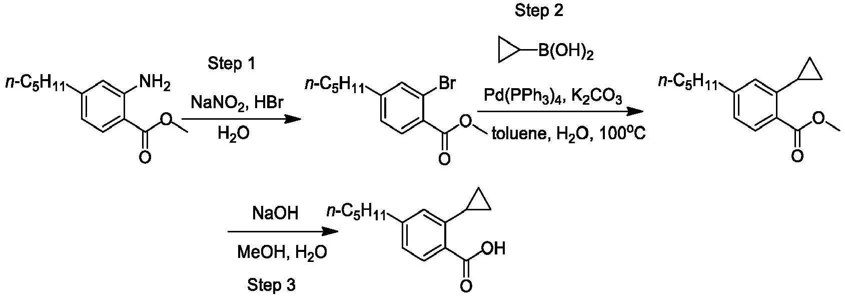

Methyl 2-amino-4-pentylbenzoate, a yellow oil, was prepared using the methods from Example 90 (Compound 184) from 2-amino-4-bromobenzoic acid. ¹H NMR (400 MHz, CDCl₃) δ 7.76 (d, J = 8.8 Hz, 1H), 6.56 - 6.43 (m, 2H), 5.66 (br s, 2H), 3.86 (s, 3H), 2.51 (t, J = 7.8 Hz, 2H), 1.62 - 1.57 (m, 2H), 1.33 - 1.30 (m, 4H), 0.89 (t, J = 6.6 Hz, 3H).

Step 1: To a solution of hydrobromic acid (1.08 mL, 37.08 mmol) in water (4 mL) was added methyl 2-amino-4-pentylbenzoate (400.0 mg, 1.8075 mmol). The solution was cooled to 0 °C and sodium nitrite (249.4 mg, 3.62 mmol) in water (0.1 mL) was added dropwise. The mixture was stirred at 0 °C until no gas emission, then heated at 80 °C for 16 h, and extracted with dichloromethane (40 mL x 2). The combined organic layers were washed with saturated NaHCO₃ (40 mL) and brine (40 mL), dried over anhydrous sodium sulfate, and concentrated in vacuo. The residue was purified by prep-TLC (10% EtOAc in petroleum ether, Rf = 0.6) to give methyl 2-bromo-4-pentylbenzoate (90 mg, 17.5% yield) as a colorless oil. ¹H NMR (400 MHz, CDCl₃) δ 7.74 (d, J = 8.0 Hz, 1H), 7.49 (s, 1H), 7.16 (d, J = 8.0 Hz, 1H), 3.92 (s, 3H), 2.61 (t, J = 7.8 Hz, 2H), 1.67 - 1.59 (m, 2H), 1.36 - 1.27 (m, 4H), 0.90 (t, J = 7.0 Hz, 3H).

Steps 2 and 3: Following similar procedures as described in Example 120 (Compound 214), 2-cyclopropyl-4-pentylbenzoic acid (40 mg, 94% yield) was obtained as a white solid. ¹H NMR (400 MHz, CDCl₃) δ 7.90 (d, J = 8.0 Hz, 1H), 7.05 (d, J = 8.0 Hz, 1H), 6.83 (s, 1H), 2.60 (t, J = 7.6 Hz, 2H), 1.63 - 1.57 (m, 3H), 1.35 - 1.29 (m, 4H), 1.06 - 1.00 (m, 2H), 0.90 (t, J = 6.6 Hz, 3H), 0.75 - 0.69 (m, 2H).

Compound 229 (formic acid salt) was prepared as a white solid utilizing the methods in Example 7 (Compound 101). LCMS (Method 5-95 AB, ESI): t_{R} = 0.733 min, [M + H]⁺ = 852.5.

### Example 136: Synthesis of Compound 230

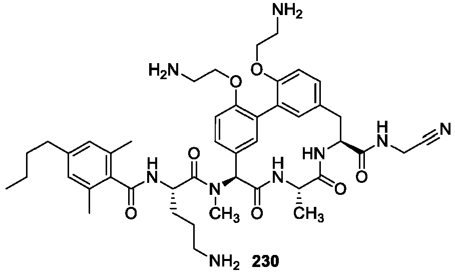

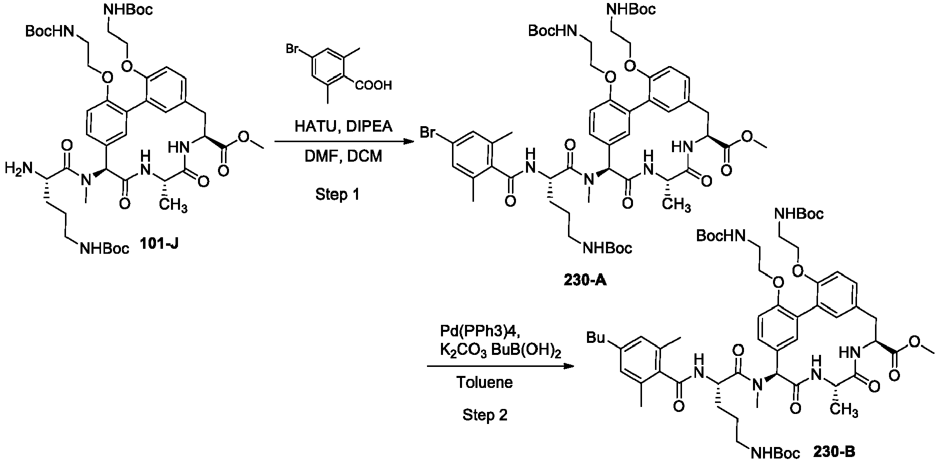

Step 1: General Method 4 (Example 5) was applied to Compound 101-J (200 mg, 0.22 mmol) and 4-bromo-2,6-dimethylbenzoic acid (99 mg, 0.44 mmol) to afford Compound 230-A (186 mg, 76% yields) as a white solid.

Step 2: General Method Alkyl Boronic Acid was applied to Compound 230-A (186 mg, 0.16 mmol) and n-butyl boronic acid (67 mg, 0.65 mmol) to afford Compound 230-B (160 mg, 88% yield) as a pale-yellow solid.

Compound 230 (formic acid salt) was prepared as a white solid utilizing the methods in Example 7 (Compound 101) from Compound 230-B. LCMS (Method 5-95 AB, ESI): t_{R} = 0.556, [M + H]⁺ = 840.4. ¹H NMR (400 MHz, MeOH-*d*₄) δ 8.48 (brs, 2H, HCOOH), 7.28-7.22 (m, 2H), 7.19 (d, *J*=8.0 Hz, 1H), 7.11 (d, *J*=8.0 Hz, 1H), 6.89 (brs, 2H), 6.86 (brs, 1H), 6.73 (brs, 1H), 6.53 (s, 1H), 5.10-5.08 (m, 1H), 4.89-4.78 (m, 2H), 4.29-4.23 (m, 4H), 4.22-4.20 (m, 2H), 3.35-3.33 (m, 1H), 3.28-3.20 (m, 4H), 3.15-3.08 (m, 2H), 3.02 (s, 3H), 2.98 (t, J=7.2 Hz, 2H), 2.53-2.46 (m, 2H), 2.26 (s, 6H), 1.99-1.94 (m, 1H), 1.90-1.78 (m, 3H), 1.57-1.49 (m, 2H), 1.37-1.27 (m, 2H), 1.36 (d, *J*=7.2 Hz, 3H), 0.92 (t, *J*=6.8 Hz, 3H).

### Example 137: Synthesis of Compound 231

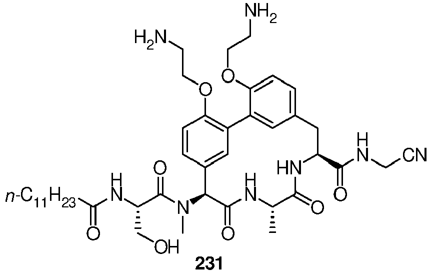

Compound 231 (formic acid salt) was prepared as a white solid utilizing the methods in Example 7 (Compound 101) from (S)-2-(((benzyloxy)carbonyl)amino)-3-(tert-butoxy)propanoic acid and dodecanoic acid. LCMS (Method 5-95 AB, ESI): t_{R} = 0.653, [M + H]⁺ = 807.3. LCMS (Method 5-95 AB, ESI): t_{R} = 0.653, [M + H]⁺ = 807.3; ¹HNMR (400 MHz, MeOH-*d*4) δ 8.48 (brs, 2H, HCOOH), 7.28-7.23 (m, 2H), 7.15 (d, *J*=8.0 Hz, 1H), 7.09 (d, *J*=8.0 Hz, 1H), 6.87 (brs, 1H), 6.81 (brs, 1H), 6.35 (s, 1H), 5.00-4.75 (m, 3H), 4.25-4.15 (m, 4H), 4.19 (s, 2H), 3.89-3.84 (m, 1H), 3.72-3.67 (m, 1H), 3.40-3.25 (m, 1H), 3.18-3.09 (m, 5H), 2.92 (s, 3H), 2.30-2.26 (m, 2H), 1.65-1.52 (m, 2H), 1.40-1.30 (m, 19H), 0.90 (t, *J*=6.4 Hz, 3H).

### Example 138: Synthesis of Compound 232

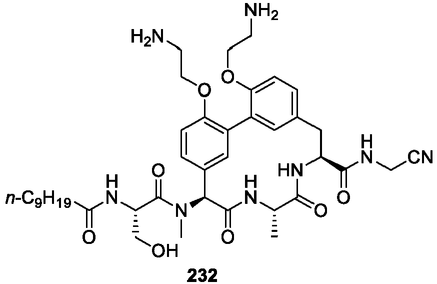

Compound 232 (formic acid salt) was prepared as a white solid using the methods in Example 137 (Compound 231) from decanoic acid. LCMS (Method 5-95 AB, ESI): t_{R} = 0.747, [M + H]⁺ = 779.4.

### Example 139: Synthesis of Compound 233

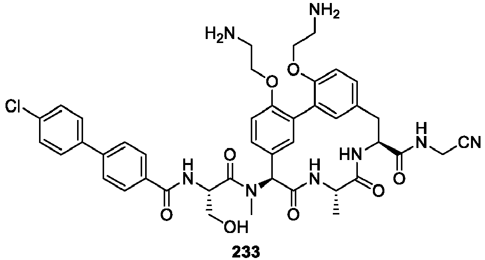

Compound 233 (formic acid salt) was prepared as a white solid (17.3 mg) utilizing the methods in Example 137 (Compound 231) from 4'-chloro-[1,1'-biphenyl]-4-carboxylic acid. LCMS (Method 5-95 AB, ESI): t_{R} = 0.616, [M + H]⁺ = 839.2. ¹H NMR (400 MHz, MeOH-d4) δ 8.49 (brs, 2H, HCOOH), 7.96 (d, *J* = 8.4 Hz, 2H), 7.72 (d, J=8.4 Hz, 4H), 7.47 (d, *J* = 8.4 Hz, 2H), 7.35-7.20 (m, 2H), 7.17-7.06 (m, 2H), 6.89 (s, 1H), 6.79 (s, 1H), 6.48 (s, 1H), 5.20-5.10 (m, 1H), 4.80-4.76 (m, 2H), 4.32-4.13 (m, 4H), 4.17 (s, 2H), 4.06-3.98 (m, 1H), 3.94-3.86 (m, 1H), 3.37-3.33 (m, 1H), 3.26-3.08 (m, 5H), 3.00 (s, 3H), 1.36 (d, J=4.8 Hz, 3H).

### Example 140: Synthesis of Compound 234

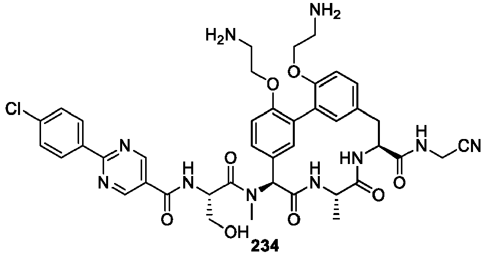

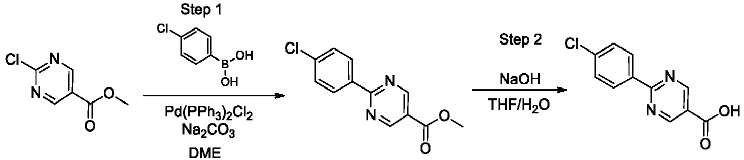

Step 1: A mixture of methyl 2-chloropyrimidine-5-carboxylate (2.0 g, 12.9 mmol) and (4-chlorophenyl)boronic acid (2.0 g, 12.9 mmol), Pd(PPh₃)₂Cl₂ (752 mg, 1.07 mmol) and Na₂CO₃ (1.1 g, 10.7 mmol) in DME (50 mL) was stirred at 80 °C for 24 h. Water (250 mL) was added into the reaction, which was extracted with EtOAc (100 mL x 3). The combined organic layers were combined, concentrated and the residue was purified by chromatography on silica to give methyl 2-(4-chlorophenyl)pyrimidine-5-carboxylate (250 mg, 8.9% yield) as a white solid. (Typical Suzuki coupling condition)

Step 2: To a solution of THF (15 mL) and H₂O (4 mL) was added methyl 2-(4-chlorophenyl)pyrimidine-5-carboxylate (250 mg, 1.0 mmol) and NaOH (103 mg, 2.6 mmol). The mixture was stirred at 80°C overnight. The mixture was concentrated and the residue was adjusted to pH~5 with 2N HCl solution. The resulting precipitate was collected, which was washed with water (20 mL) to give 2-(4-chlorophenyl)pyrimidine-5-carboxylic acid (200 mg, 85% yield) as a white solid. LCMS (Method 5-95 AB, ESI): t_{R} = 0.839, [M + H]⁺ = 234.7

### (Typical ester hydrolysis procedure, NaOH/THF/H₂O)

Compound 234 (formic acid salt) was prepared as a white solid utilizing the methods in Example 137 (Compound 231) from 2-(4-chlorophenyl)pyrimidine-5-carboxylic acid. LCMS (Method 5-95 AB, ESI): t_{R} = 0.698, [M + H]⁺ = 841.5.

### Example 141: Synthesis of Compound 235

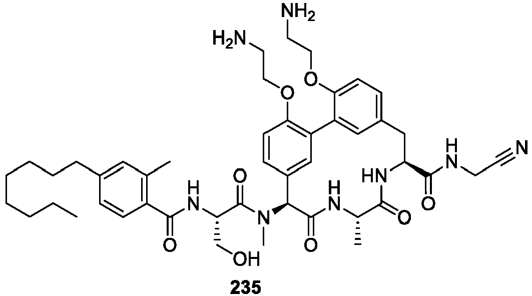

Compound 235 (formic acid salt) was prepared as a white solid utilizing the methods in Example 137 (Compound 231) except (S)-2-(((benzyloxy)carbonyl)amino)-3-(tert-butoxy)propanoic acid is used in the amino acid coupling step. LCMS (Method 5-95 AB, ESI): t_{R} = 0.805 min, [M + H]⁺ = 856.5.

### Example 142: Synthesis of Compound 236

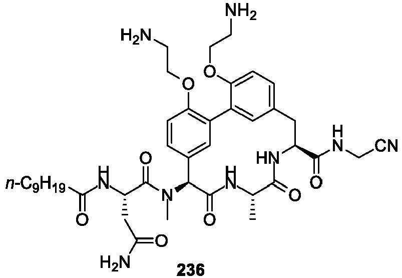

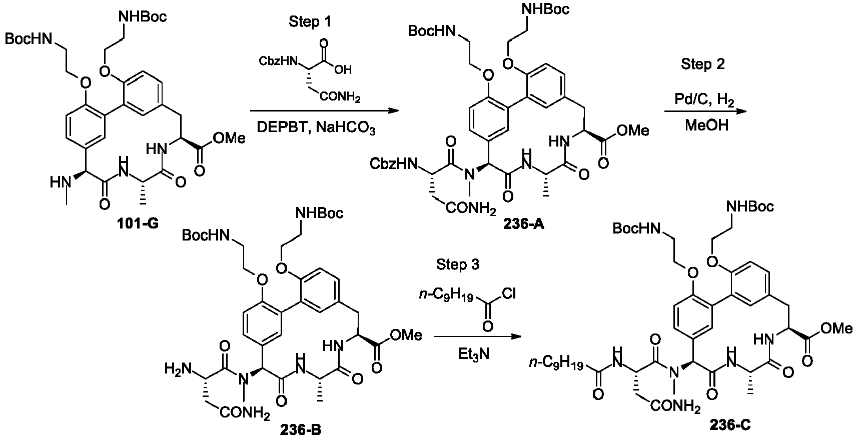

Step 1: To a solution of (S)-4-amino-2-(((benzyloxy)carbonyl)amino)-4-oxobutanoic acid (75 mg, 0.28 mmol) in THF (15 mL) at 0°C, DEPBT (252 mg, 0.84 mmol) and NaHCO₃ (71 mg, 0.84 mmol) was added. The mixture and stirred at the same temperature for 10 min, followed by the addition of Compound 101-G (100 mg, 0.14 mmol). The resulting mixture was stirred at room temperature for 4 days. The volatiles were removed and the resulting residue was taken up in EtOAc (50 mL), which was washed with brine (50 mL x 2). The EtOAc layer was dried over Na₂SO₄, concentrated and the residue was purified by flash column chromatography to afford Compound 236-A (95mg, 71% yield) as a white solid.

Step 2: General Method Hydrogenation (Pd/C, H₂) was applied to Compound 236-A (95 mg, 0.10 mmol) to afford Compound 236-B (84 mg, 99% yield) as a white solid.

Step 3: The coupling of an amine to an acid chloride is described and is referred to as General Method Acid Chloride. To a solution of Compound 236-B (84 mg, 0.10 mmol) in DCM (5 mL) was added decanoyl chloride (23 mg, 0.12 mmol) and Et₃N (20 mg, 0.20 mmol) at 0°C. The mixture was stirred at room temperature for 1h. The reaction was adjusted pH to 5 with saturated citric acid solution, which was extracted with DCM (10 mL x 3). The combined organic layers were washed with brine (20 mL). The DCM layer was dried over Na₂SO₄, concentrated and the residue was purified by prep-TLC to give Compound 236-C (87 mg, 90% yield) as a white solid.

Compound 236 (formic acid salt) was prepared as a white solid utilizing the methods in Example 7 (Compound 101) from Compound 236-C. LCMS (Method 5-95 AB, ESI): t_{R} = 0.896, [M + H]⁺ = 806.8. ¹H NMR (400 MHz, MeOH-d4) δ 7.28-7.22 (m, 2H), 7.14-7.06 (m, 2H), 6.87 (brs, 1H), 6.81 (brs, 1H), 6.33 (s, 1H), 5.22-5.18 (m, 1H), 4.80-4.70 (m, 2H), 4.21 (brs, 2H), 4.12-4.09 (m, 4H), 3.19-3.05 (m, 2H), 3.00 (brs, 4H), 2.92 (s, 3H), 2.76-2.65 (m, 1H), 2.61-2.50 (m, 1H), 2.26-2.15 (m, 2H), 1.64 (brs, 2H), 1.36-1.33 (m, 15H), 0.92 (t, *J*=7.2 Hz, 3H).

### Example 143: Synthesis of Compound 237

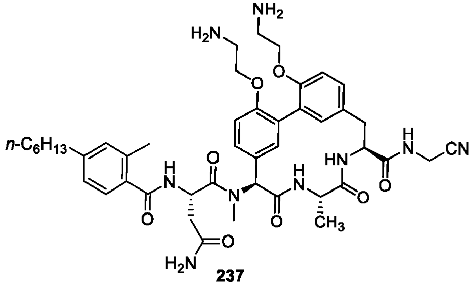

Compound 237 (formic acid salt) was prepared as a white solid utilizing the methods in Example 142 (Compound 236) from Compound 236-B. LCMS (Method 5-95 AB, ESI): t_{R} = 0.631, [M + H]⁺ = 854.3. ¹H NMR (400 MHz, MeOH-d4) δ 8.51 (brs, 2H, HCOOH), 7.35-7.28 (m, 2H), 7.25 (d, *J*=8.0 Hz, 1H), 7.16 (d, *J*=8.0 Hz, 1H), 7.12-7.03 (m, 3H), 6.90 (brs, 1H), 6.80 (brs, 1H), 6.38 (s, 1H), 5.38-5.33 (m, 1H), 4.81-4.74 (m, 2H), 4.25-4.12 (m, 4H), 4.19 (s, 2H), 3.20-3.06 (m, 4H), 2.99 (s, 3H), 2.83-2.76 (m, 2H), 2.73-2.64 (m, 2H), 2.63-2.57 (m, 2H), 2.39 (s, 3H), 1.66-1.54 (m, 2H), 1.40-1.24 (m, 9H), 0.90 (t, *J*=5.2 Hz, 3H).

### Example 144: Synthesis of Compound 238

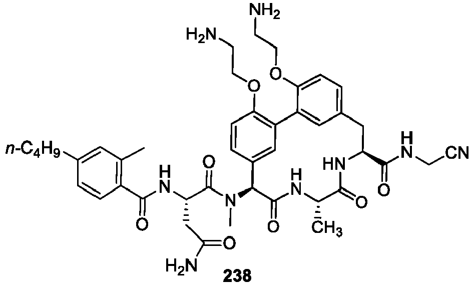

Compound 238 (formic acid salt) was prepared as a white solid utilizing the methods in Example 142 (Compound 236) from Compound 236-B. LCMS (Method 5-95 AB, ESI): t_{R} = 0.587, [M + H]⁺ = 826.4.

### Example 145: Synthesis of Compound 239

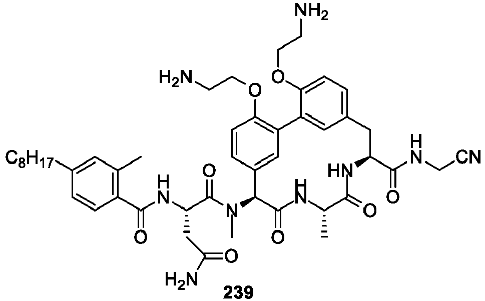

Compound 239 (free base) was prepared as a white solid utilizing the methods in Example 142 (Compound 236). LCMS (Method 5-95 AB, ESI): t_{R} = 0.813, [M + H]⁺ = 882.7.

### Example 146: Synthesis of Compound 240

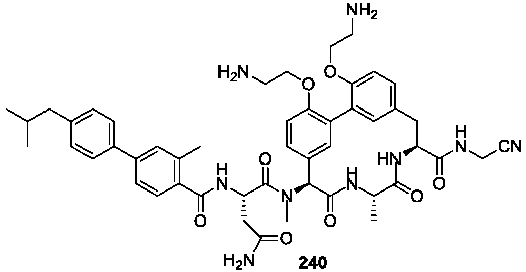

Compound 240 (free base) was prepared as a white solid utilizing the methods in Example 142 (Compound 236). LCMS (Method 5-95 AB, ESI): t_{R} = 0.782, [M + H]⁺ = 902.5.

### Example 147: Compound 241

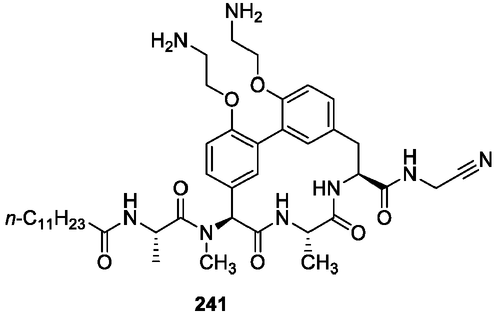

Compound 241 (formic acid salt) was prepared as a white solid utilizing the methods in Example 7 (Compound 101) from Compound 101-G and (S)-2-(((benzyloxy)carbonyl)amino)propanoic acid. LCMS (Method 5-95 AB, ESI): t_{R} = 0.679, [M + H]⁺ = 791.3. ¹H NMR (400 MHz, MeOH-*d*₄) δ 8.47 (brs, 2H, HCOOH), 7.30 (d, *J*=8.0 Hz, 1H), 7.24 (d, *J*=8.0 Hz, 1H), 7.16 (d, *J*=8.4 Hz, 1H), 7.09 (d, *J*=8.4 Hz, 1H), 6.89 (brs, 1H), 6.80 (brs, 1H), 6.35 (s, 1H), 4.79-4.72 (m, 3H), 4.30-4.15 (m, 4H), 4.19 (s, 2H), 3.40-3.34 (m, 1H), 3.24-3.09 (m, 5H), 2.88 (s, 3H), 2.25 (t, *J*=7.6 Hz, 2H), 1.69-1.53 (m, 3H), 1.50-1.22 (m, 21H), 0.90 (t, *J*=6.6 Hz, 3H).

### Example 148: Synthesis of Compound 242

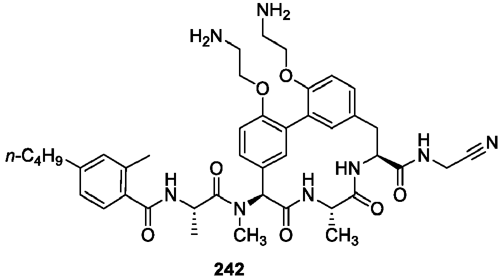

Compound 242 (formic acid salt) was prepared as a white solid utilizing the methods in Example 147 (Compound 241). LCMS (Method 5-95 AB, ESI): t_{R} = 0.616, [M + H]⁺ = 783.3.

### Example 149: Synthesis of Compound 243

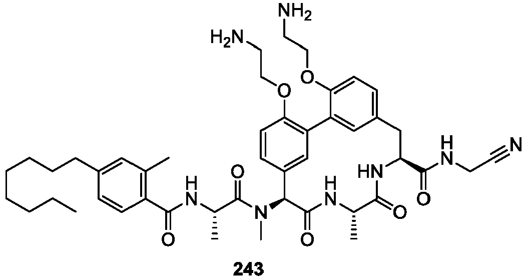

Compound 243 (formic acid salt) was prepared in 29% yield as a white solid utilizing the methods in Example 7 (Compound 101) from (S)-2-(((benzyloxy)carbonyl)amino)propanoic acid. LCMS (Method 5-95 AB, ESI): t_{R} = 0.707 min, [M + H]⁺ = 839.3.

### Example 150: Synthesis of Compound 244

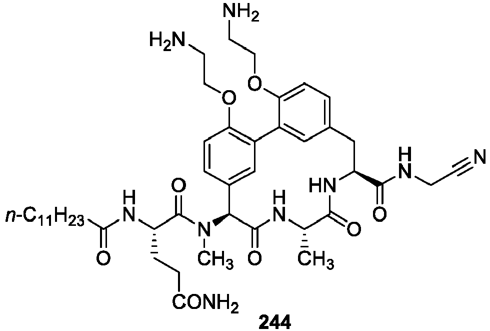

Compound 244 (formic acid salt) was prepared as a white solid utilizing the methods used in Example 7 (Compound 101) from Compound 101-G and (S)-5-amino-2-(((benzyloxy)carbonyl)amino)-5-oxopentanoic acid. LCMS (Method 5-95 AB, ESI): t_{R} = 0.782, [M + H]⁺ = 848.6. ¹H NMR (400 MHz, MeOH-*d*₄) δ 8.53 (brs, 2H, HCOOH), 7.31-7.26 (m, 2H), 7.18 (d, *J*=8.0 Hz, 1H), 7.12 (d, *J*=8.0 Hz, 1H), 6.90 (brs, 1H), 6.84 (brs, 1H), 6.36 (s, 1H), 5.00-4.76 (m, 3H), 4.30-4.20 (m, 4H), 4.21 (s, 2H), 3.40-3.22 (m, 5H), 3.20-3.10 (m, 1H), 2.94 (s, 3H), 2.39-2.36 (m, 2H), 2.29-2.27 (m, 2H), 2.20-2.05 (m, 1H), 1.93-1.82 (m, 1H), 1.70-1.54 (m, 2H), 1.38-1.33 (m, 19H), 0.92 (t, *J*=7.2 Hz, 3H).

### Example 151: Synthesis of Compound 245

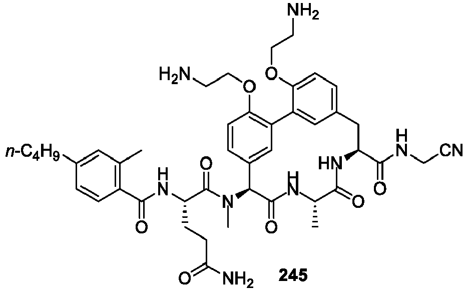

Compound 245 (formic acid salt) was prepared as a white solid utilizing the same methods used in Example 150 (Compound 244). LCMS (Method 5-95 AB, ESI): t_{R} = 0.732, [M + H]⁺ = 840.5.

### Example 152: Synthesis of Compound 246

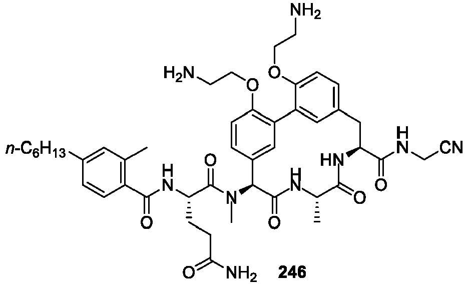

Compound 246 (formic acid salt) was prepared as a white solid utilizing the methods in Example 150 (Compound 244). LCMS (Method 5-95 AB, ESI): t_{R} = 0.637, [M + H]⁺ = 868.9.

### Example 153: Synthesis of Compound 247

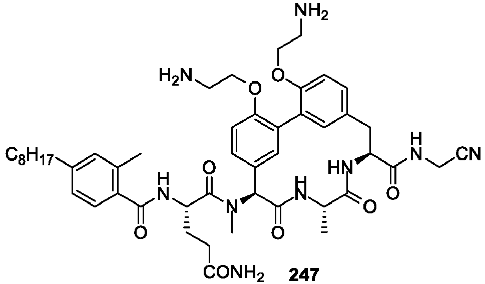

Compound 247 (formic acid salt) was prepared as a white solid utilizing the methods in Example 150 (Compound 244). LCMS (Method 5-95 AB, ESI): t_{R} = 0.835, [M + H]⁺ = 896.6.

### Example 154: Synthesis of Compound 248

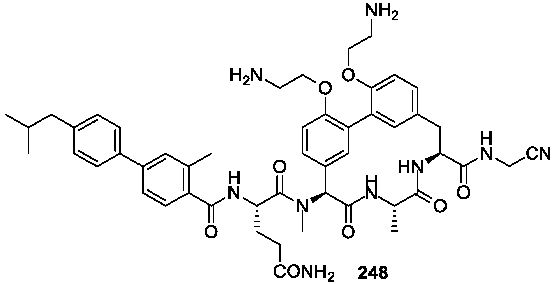

Compound 248 (formic acid salt) was prepared as a white solid utilizing the methods in Example 150 (Compound 244). LCMS (Method 5-95 AB, ESI): t_{R} = 0.786, [M + H]⁺ = 916.5.

### Example 155: Synthesis of Compound 249

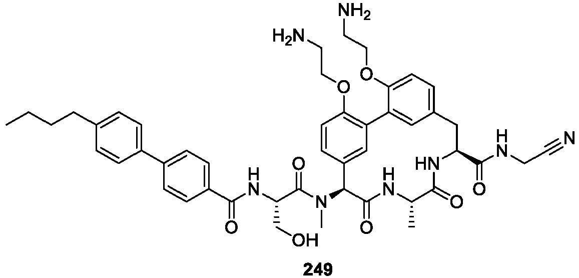

Compound 249 (formic acid salt) was prepared as a white solid utilizing the methods in Example 7 (Compound 101) except (S)-2-(((benzyloxy)carbonyl)amino)-3-hydroxypropanoic acid is used in the amino acid coupling step. LCMS (Method 5-95 AB, ESI): t_{R} = 0.659 min, [M + H]⁺ = 861.8.

### Example 156: Synthesis of Compound 250

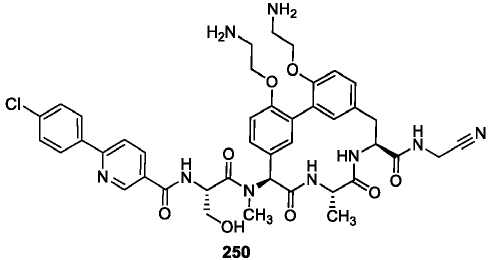

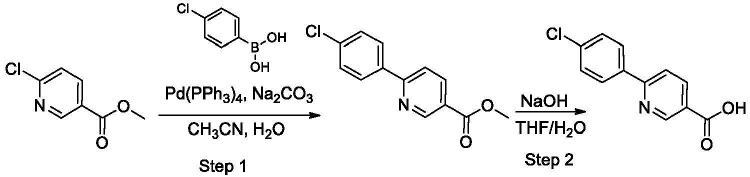

Step 1: A solution of methyl 6-chloronicotinate (4.0 g, 23.3 mmol), Pd(PPh₃)₄ (32.7 g, 46.6 mmol), Na₂CO₃ (7.4 g, 69.9 mmol), (4-chlorophenyl)boronic acid (7.3 g, 46.6 mmol) in ACN/H₂O (70 mL, v/v = 5:2) was stirred at 90°C for 16 h under N₂. After filtration, the volatiles were removed and the residue was taken up by EtOAc (90 mL), which was washed with brine (90 mL x 2). The organic layer was dried over Na₂SO₄, concentrated and the residue was purified on silica gel flash column to obtain the product of methyl 6-(4-chlorophenyl)nicotinate (2.7 g, 46.8% yield) as a white solid. ¹H NMR (400 MHz, CDCl₃) δ 9.25 (d, *J*=2.0 Hz, 1H), 8.35 (dd, *J*=8.4, 2.0 Hz, 1H), 8.00 (d, *J*=8.4 Hz, 2H), 7.78 (d, *J*=8.4 Hz, 1H), 7.46 (d, *J*=8.4 Hz, 2H), 3.96 (s, 3H).

Step 2: Typical ester hydrolysis (NaOH/THF) procedure was applied to methyl 6-(4-chlorophenyl)nicotinate (700 mg, 2.83 mmol) to afford 6-(4-chlorophenyl)nicotinic acid (470 mg, 72% yield) as a white solid.

Compound 250 (formic acid salt) was prepared as a white solid utilizing the same methods used in the preparation of Compound 231 (Example 137) except 6-(4-chlorophenyl)nicotinic acid was used in the coupling step. LCMS (Method 5-95 AB, ESI): t_{R} = 0.640, [M + H]⁺ = 840.0.

### Example 157 Synthesis of Compound 251

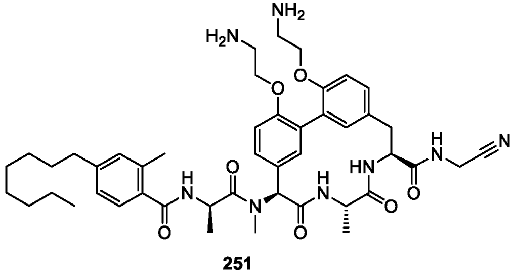

Compound 251 (formic acid salt) was prepared as a white solid utilizing the methods in Example 7 (Compound 101) from (R)-2-(((benzyloxy)carbonyl)amino)propanoic acid. LCMS (Method 5-95 AB, ESI): t_{R} = 0.851 min, [M + H]⁺ = 839.5.

### Example 158: Synthesis of Compound 252

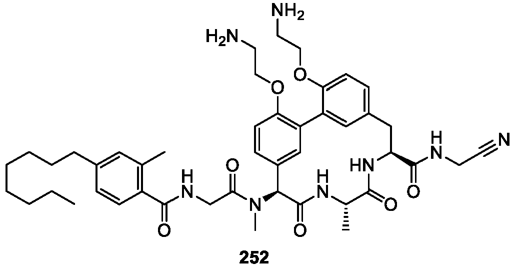

Compound 252 (formic acid salt) was prepared as a white solid utilizing the methods in Example 7 (Compound 101) from 2-(((benzyloxy)carbonyl)amino)acetic acid. LCMS (Method 5-95 AB, ESI): t_{R} = 0.723 min, [M + H]⁺ = 825.2.

### Example 159: Synthesis of Compound 253

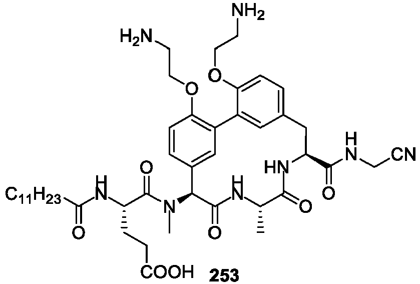

Compound 253 (formic acid salt) was prepared as a white solid utilizing the methods in Example 7 (Compound 101) from (S)-2-(((benzyloxy)carbonyl)amino)-5-(tert-butoxy)-5-oxopentanoic acid. LCMS (Method 5-95 AB, ESI): t_{R} = 0.799, [M + H]⁺ = 849.4

### Example 160: Synthesis of Compound 254 and Compound 255

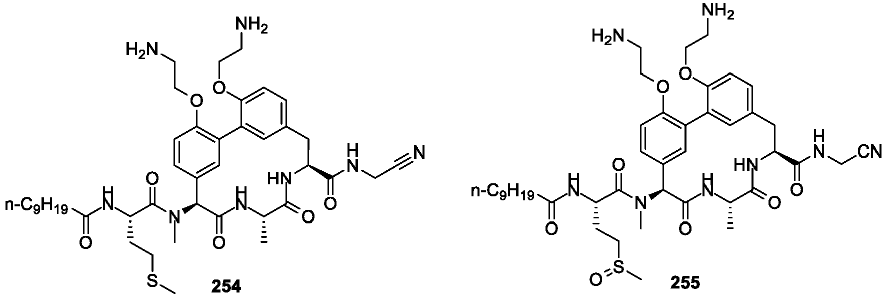

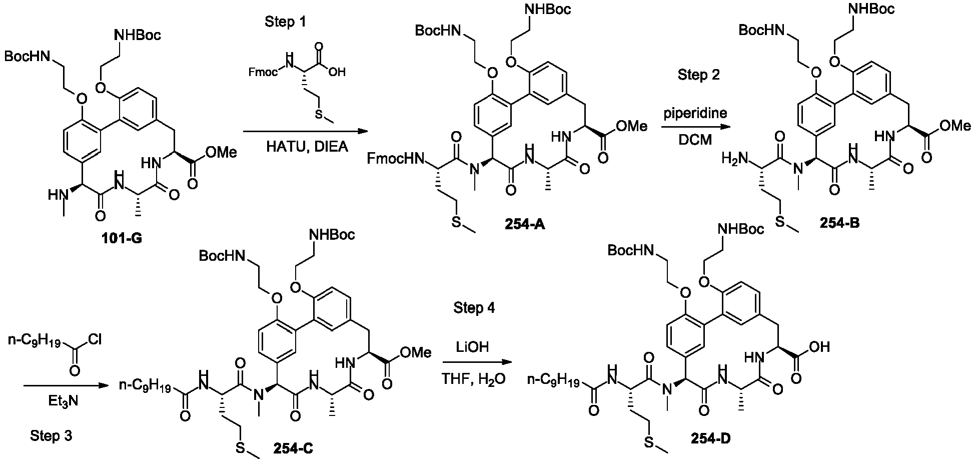

Step 1: Compound 254-A was prepared according to General Method 4 (Example 5) from Compound 101-G (104 mg, 0.28 mmol) and Fmoc-L-methionine to afford Compound 254-A (100 mg, 66.9%) as a light yellow solid after silica column chromatography.

Step 2: Compound 254-A was subject to the standard Fmoc-deprotection conditions. To a solution of Compound 254-A (100 mg, 0.18 mmol) in DCM (4 mL) was added piperidine (1.0 mL) at 0°C and the mixture was stirred at room temperature under N₂ for 2 h. The volatiles were concentrated and the residue was re-dissolved in EtOAc (30 mL), which was washed by brine (2 x 30 mL). The organic layer was dried over MgSO₄, concentrated and purified by prep-TLC (eluting 3% MeOH in DCM) to give Compound 254-B (50 mg, 63.1% yield) as a yellow solid.

Steps 3 and 4:Compound 254-C was prepared as previously described from decanoyl chloride using triethylamine as a base in DCM followed by LiOH ester hydrolysis.

Compound 254 (formic acid salt) was prepared as a white solid (16 mg) utilizing the methods in Example 137 (Compound 231). Data for Compound 254: LCMS (Method 5-95 AB, ESI): t_{R} = 0.663, [M + H]⁺ = 823.2.

Compound 255 (2 mg, white solid) was isolated as a by-product which occurs from oxidation of the sulfide during the preparation of Compound 254. Data for Compound 255: LCMS (Method 5-95 AB, ESI): t_{R} = 0.653, [M + H]⁺ = 839.2.

### Example 161: Synthesis of Compound 256

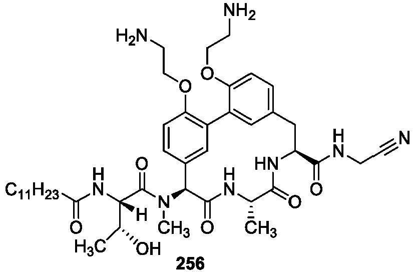

Compound 256 (formic acid salt) was prepared as a white solid utilizing the methods in Example 160 (Compound 254) from (2S,3R)-2-((((9H-fluoren-9-yl)methoxy)carbonyl)amino)-3-(tert-butoxy)butanoic acid. LCMS (Method 5-95 AB, ESI): t_{R} = 0.659, [M + H]⁺ = 821.3.

### Example 162: Synthesis of Compound 257

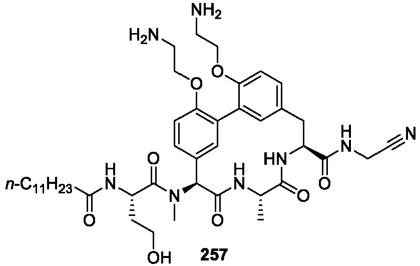

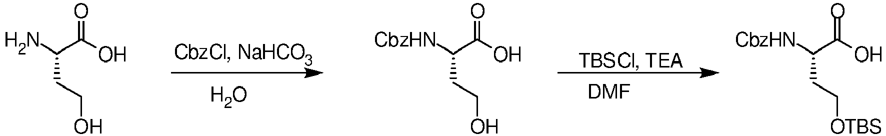

To a mixture of benzyl chloroformate (930.84 mg, 5.46 mmol) and sodium bicarbonate (705.25 mg, 8.39 mmol) in water (10 mL) was added (2S)-2-amino-4-hydroxy-butanoic acid (500.0 mg, 4.2 mmol), and stirred at 15 °C for 3 hours under nitrogen. The reaction mixture was washed with ethyl acetate (20 mL x 3), acidified to pH 4 using 2N HCl (about 20 mL) at 0°C, and extracted with ethyl acetate (30 mL x 3). The combined organic layers were dried over sodium sulfate and concentrated to afford (2S)-2-(benzyloxycarbonylamino)-4-hydroxy-butanoic acid (450 mg, 1.7769 mmol, 42.3% yield) as a colorless oil. It was used in the next step without further purification.

To a mixture of (2S)-2-(benzyloxycarbonylamino)-4-hydroxy-butanoic acid (450.0 mg, 1.78 mmol) and triethylamine (395.57 mg, 3.91 mmol) in N,N-dimethylformamide (8 mL) was added tert-butyldimethylchlorosilane (401.72 mg, 2.67 mmol) at 0°C and stirred at 15 °C for 1 hour. The reaction mixture was diluted with water (30 mL) and sodium carbonate (5 g) was added. The resulting mixture was washed with ethyl acetate (15 mL x 3). The aqueous phase was acidified to pH 4 using 2 N HCl (about 20 mL) at 0°C and extracted with ethyl acetate (30 mL x 3). The combined organic layers were dried over sodium sulfate and concentrated to afford the (S)-2-(((benzyloxy)carbonyl)amino)-4-((tert-butyldimethylsilyl)oxy)butanoic acid (450 mg, 1.2245 mmol, 68.9% yield) as a colorless oil. It was used in the next step without further purification. LCMS (Method 5-95 AB, ESI): t_{R} = 0.833 min, [M+Na]⁺ = 389.9.

Compound 257 (formic acid salt) was prepared as a white solid utilizing the methods in Example 7 (Compound 101) from Compound 101-G and (S)-2-(((benzyloxy)carbonyl)amino)-4-hydroxybutanoic acid. LCMS (Method 5-95 AB, ESI): t_{R} = 0.657, [M + H]⁺ = 821.3.

### Example 163: Synthesis of Compound 258

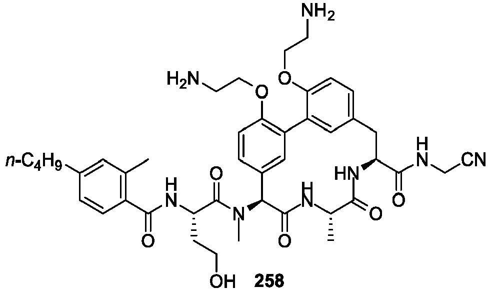

Compound 258 (formic acid salt) was prepared as a white solid (10 mg) utilizing the methods in Example 162 (Compound 257). LCMS (Method 5-95 AB, ESI): t_{R} = 0.585, [M + H]⁺ = 813.3. ¹H NMR (400 MHz, MeOH-d4) δ 8.48 (brs, 2H, HCOOH), 7.37-7.35 (m, 2H), 7.27 (d, *J*=8.0 Hz, 1H), 7.19 (d, J=8.0 Hz, 1H), 7.15-7.05 (m, 3H), 6.92 (brs, 1H), 6.85 (brs, 1H), 6.46 (s, 1H), 5.17-5.14 (m, 1H), 4.80-4.70 (m, 2H), 4.27-4.20 (m, 4H), 4.21 (s, 2H), 3.78-3.70 (m, 2H), 3.25-3.23 (m, 4H), 3.16-3.12 (m, 1H), 3.02 (s, 3H), 2.66-2.62 (m, 3H), 2.45 (s, 3H), 2.15-2.00 (m, 1H), 2.00-1.90 (m, 1H), 1.64-1.50 (m, 2H), 1.42-1.30 (m, 5H), 0.96 (t, *J*=7.2 Hz, 3H).

### Example 164: Synthesis of Compound 259

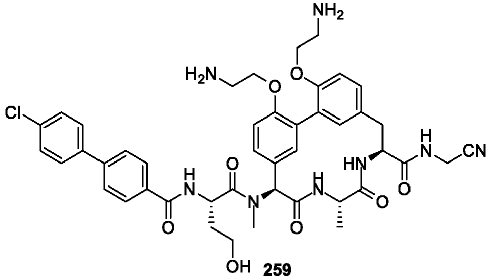

Compound 259 (formic acid salt) was prepared as a white solid utilizing the methods in Example 162 (Compound 257). LCMS (Method 5-95 AB, ESI): t_{R} = 0.598, [M + H]⁺ = 853.5.

### Example 165: Synthesis of Compound 260

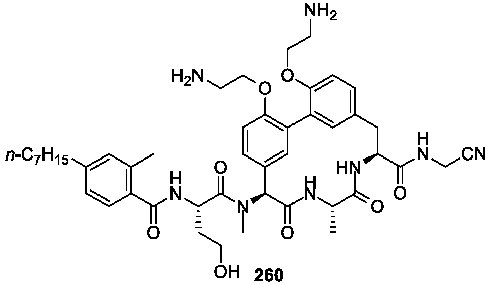

Compound 260 (free base) was prepared as a white solid utilizing the methods in Example 162 (Compound 257). LCMS (Method 5-95 AB, ESI): t_{R} = 0.810, [M + H]⁺ = 855.5; ¹H NMR (400 MHz, MeOH-*d*4) δ 7.31-7.22 (m, 2H), 7.22 (d, *J*=7.5 Hz, 1H), 7.14 (d, *J*=7.5 Hz, 1H), 7.07-7.04 (m, 3H), 6.84 (brs, 1H), 6.65-6.58 (m, 2H), 5.20-5.18 (m, 1H), 4.80-4.70 (m, 2H), 4.21 (s, 2H), 4.16-4.01(m, 4H), 3.74 (t, *J*=5.6 Hz, 2H), 3.32-3.28 (m, 1H), 3.15-2.91 (m, 5H), 3.02 (s, 3H), 2.61-2.52 (m, 2H), 2.36 (s, 3H), 2.16-2.05 (m, 1H), 2.00-1.90 (m, 1H), 1.57 (brs, 2H), 1.40-1.20 (m, 11H), 0.92 (t, *J*=6.4 Hz, 3H).

### Example 166: Synthesis of Compound 261

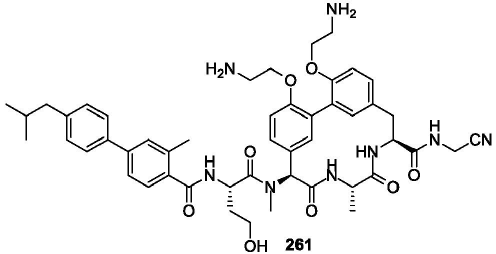

Compound 261 (formic acid salt) was prepared as a white solid utilizing the methods in Example 162 (Compound 257). LCMS (Method 5-95 AB, ESI): t_{R} = 0.794, [M + H]⁺ = 889.6.

### Example 167: Synthesis of Compound 262

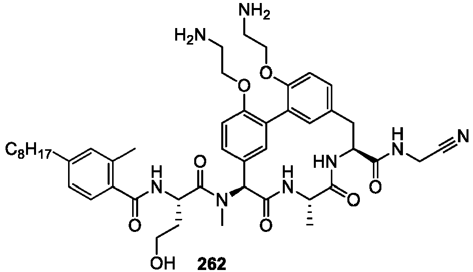

Compound 262 (formic acid salt) was prepared as a white solid utilizing the methods in Example 7 (Compound 101). LCMS (Method 5-95 AB, ESI): t_{R} = 0.684 min, [M + H]⁺ = 869.4.

### Example 168: Synthesis of Compound 263

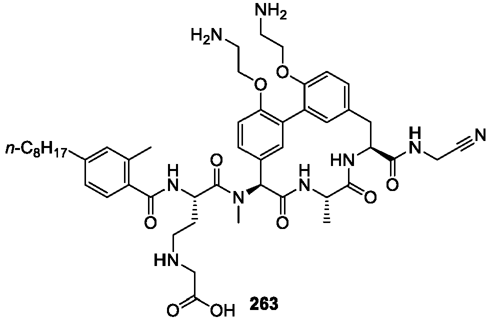

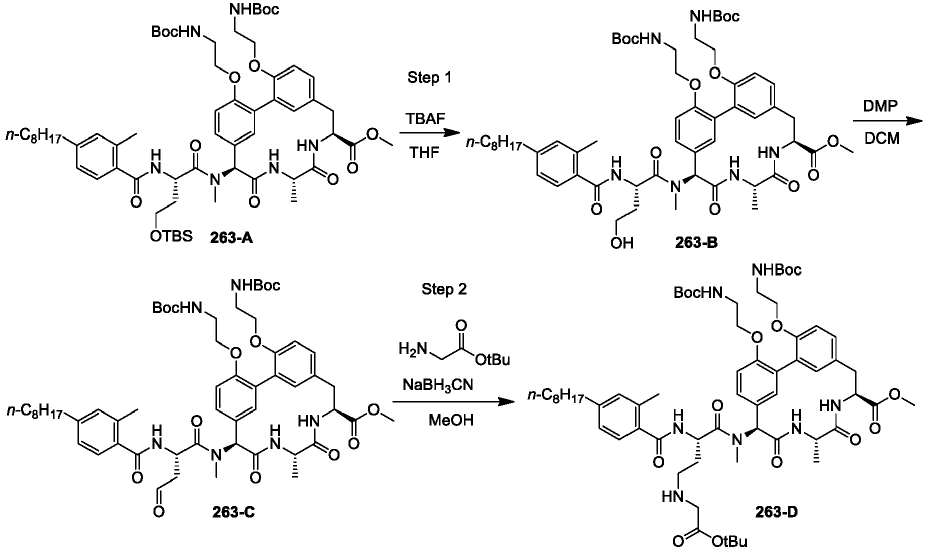

Compound 263-A is an intermediate in the preparation of Compound 262 (Example 167). To a solution of Compound 263-A (360.0 mg, 0.31 mmol) in tetrahydrofuran (8 mL) was added tetrabutylammonium fluoride (2M; 2.0 mL, 4 mmol) in tetrahydrofuran. The solution was stirred at 20 °C for 2 h and evaporated to dryness. The residue was taken up in EtOAc (20 mL), washed with water (20 mL x 2) and brine (20 mL), dried over MgSO₄ and concentrated. The residue was purified by flash column chromatography (5% methanol in DCM, Rf = 0.5) to afford Compound 263-B (320 mg, 98.6% yield) as a white solid. LCMS (Method 5-95 AB, ESI): t_{R} = 1.122 min, [M + H]⁺ = 1045.4.

To a solution of Compound 263-B (170.0 mg, 0.16 mmol) in dichloromethane (10 mL) was added Dess-Martin periodinane (138.0 mg, 0.33 mmol). The mixture was stirred at 20 °C for 2 h, quenched with saturate NaHCO₃ solution and extracted with EtOAc (20 mL). The organic layer was washed with water (20 mL x 2) and brine (20 mL), dried over MgSO₄ and concentrated to afford crude Compound 263-C (160 mg, 94.3% yield) as a white solid. LCMS (Method 5-95 AB, ESI): t_{R} = 0.974 min, [M + H]⁺ = 1043.5.

To a solution of Compound 263-C (400.0 mg, 0.38 mmol) and tert-butyl 2-aminoacetate hydrochloride (128.5 mg, 0.77 mmol) in methanol (20 mL) was added sodium cyanoborohydride (48.2 mg, 0.77 mmol). The reaction was stirred at 20 °C for 2 h and evaporated to dryness. The residue was taken up in EtOAc (10 mL), washed with water (10 mL x 2) and brine (10 mL), dried over MgSO₄ and concentrated. The residue was purified by flash column chromatography (5% methanol in DCM, Rf = 0.5) to afford Compound 263-D (300 mg, 67.5% yield) as a white solid. LCMS (Method 5-95 AB, ESI): t_{R} = 1.043 min, [M + H]⁺ = 1159.3.

Compound 263 (formic acid salt) was prepared as a white solid utilizing the methods in Example 7 (Compound 101) from Compound 263-D. LCMS (Method 5-95 AB, ESI): t_{R} = 0.642 min, [M + H]⁺ = 927.4.

### Example 169: Synthesis of Compound 264

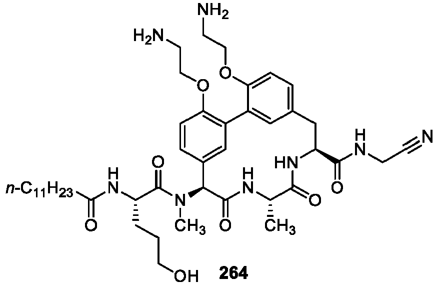

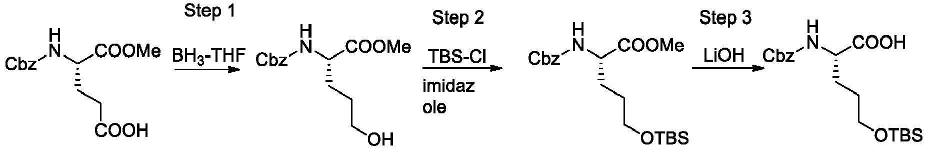

Step 1: To a solution of (S)-4-(((benzyloxy)carbonyl)amino)-5-methoxy-5-oxopentanoic acid (1.0 g, 3.4 mmol) in THF (3.5mL) at 0°C was added 1M BH₃/THF (6.7 mL, 6.7 mmol) and the mixture was stirred at the same temperature for 1 h. The reaction was quenched by addition of 1M NaHSO₄ (10 mL), followed by the removal of most THF under reduced pressure. The resulting mixture was diluted with water (20 mL), which was extracted by EtOAc (30 mL x 3). The combined organic layers were washed with brine (90 mL), dried over Na₂SO₄, concentrated and the residue was purified by silica gel flash column to afford (S)-methyl 2-(((benzyloxy)carbonyl)amino)-5-hydroxypentanoate (550 mg, 60% yield) as colorless oil. LCMS (Method 5-95 AB, ESI): t_{R} = 0.729, M+Na⁺ = 303.9

Steps 2 and 3: Typical TBS protection and ester hydrolysis (LiOH) procedure was followed to afford (S)-2-(((benzyloxy)carbonyl)amino)-5-((tert-butyldimethylsilyl)oxy)pentanoic acid (640 mg) as a colorless oil.

Compound 264 (formic acid salt) was prepared as a white solid utilizing the methods in Example 162 (Compound 257). LCMS (Method 5-95 AB, ESI): t_{R} = 0.658, [M + H]⁺ = 835.6; ¹H NMR (400 MHz, MeOH-*d*₄) δ 8.49 (brs, 2H, HCOOH), 7.35-7.25 (m, 2H), 7.17 (d, *J*=8.0 Hz, 1H), 7.12 (d, *J*=8.0 Hz, 1H), 6.92 (brs, 1H), 6.80 (brs, 1H), 6.41 (s, 1H), 4.95-4.88 (m, 1H), 4.82-4.70 (m, 2H), 4.40-4.22 (m, 4H), 4.20 (s, 2H), 3.65-3.58(m, 2H), 3.48-3.35 (m, 1H), 3.31-3.22 (m, 4H), 3.20-3.10 (m, 1H), 2.93 (s, 3H), 2.32-2.20 (m, 2H), 2.00-1.90 (m, 1H), 1.80-1.60 (m, 5H), 1.50-1.20 (m, 20H), 0.92 (t, J=6.8 Hz, 3H).

### Example 170: Synthesis of Compound 265

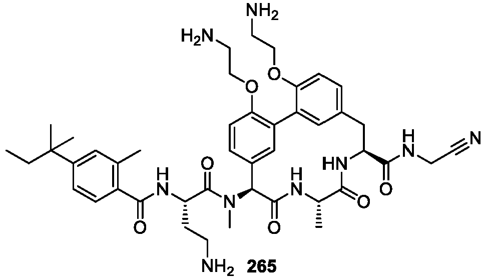

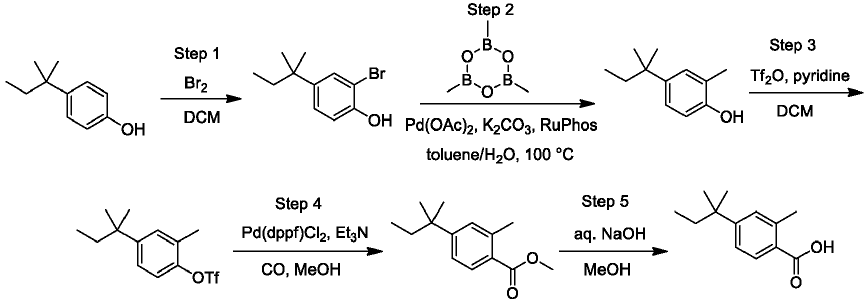

Step 1: To a solution of 4-tert-amylphenol (3.0 g, 18.27 mmol) in dichloromethane (20 mL) was added dropwise bromine (0.94 mL, 18.27 mmol) at 0 °C and stirred for 30 min. Then the mixture was allowed to warm to 20 °C and stirred for 16 h. The reaction mixture was diluted with DCM (20 mL), washed with water (50 mL x 3), dried over anhydrous sodium sulfate, and concentrated in vacuo. The residue was purified on silica gel column eluted with ethyl acetate/petroleum ether (1:10) to afford 2-bromo-4-(1,1-dimethylpropyl)phenol (2.6 g, 58.5% yield) as a white solid. ¹H NMR (400 MHz, CDCl₃): δ 7.37 (d, J = 2.0 Hz, 1H), 7.16 (dd, J = 8.4, 2.4 Hz, 1H), 6.94 (d, J = 8.4 Hz, 1H), 5.35 (s, 1H), 1.58 (q, J = 7.5 Hz, 2H), 1.23 (s, 6H), 0.66 (t, J = 7.4 Hz, 3H).

Step 2: A mixture of 2-bromo-4-(1,1-dimethylpropyl)phenol (500 mg, 2.06 mmol), palladium(II) acetate (46.2 mg, 0.21 mmol), RuPhos (192.0 mg, 0.41 mmol) and potassium carbonate (710.6 mg, 5.14 mmol) in toluene (10 mL) and water (1 mL) was treated with trimethylboroxine (516.3 mg, 4.11 mmol) and heated at 80 °C for 16 hours under nitrogen. The reaction mixture was diluted with water (10 mL) and extracted with ethyl acetate (20 mL x 3). The combined organic layers were dried over sodium sulfate and concentrated. The residue was purified by Prep-TLC (10% ethyl acetate in petroleum ether) to afford 4-(1,1-dimethylpropyl)-2-methyl-phenol (220 mg, 60% yield) as a colorless oil. ¹H NMR (400 MHz, CDCl₃): δ 7.05 - 6.95 (m, 2H), 6.69 (d, J = 8.4 Hz, 1H), 4.51 (s, 1H), 2.24 (s, 3H), 1.61 - 1.56 (m, 2H), 1.23 (s, 7H), 0.66 (t, J = 7.4 Hz, 3H).

Step 3: A solution of 4-(1,1-dimethylpropyl)-2-methyl-phenol (220.0 mg, 1.23 mmol) and pyridine (292.9 mg, 3.7 mmol) in dichloromethane (5 mL) was treated with trifluoromethanesulfonic anhydride (417.8 mg, 1.48 mmol) at 0 °C. The resulting mixture was stirred at 15°C for 1 hour under nitrogen. The reaction mixture was adjusted to pH = 5 using 2N HCl and extracted with dichloromethane (20 mL x 3). The combined organic layers were dried over sodium sulfate and filtered. The filtrate was concentrated to afford [4-(1,1-dimethylpropyl)-2-methyl-phenyl] trifluoromethanesulfonate (360 mg, 94% yield) as a brown oil, which was used in the next step without purification.

Step 4: The carbonylation of an aryl triflate is described and is referred to as General Method Carbonylation. This method can also be used with aryl bromides and iodides.

A solution of [4-(1,1-dimethylpropyl)-2-methyl-phenyl] trifluoromethanesulfonate (240 mg, 0.770 mmol) and triethylamine (234.8 mg, 2.32 mmol) in methanol (10 mL) was treated with 1,1'-bis(diphenylphosphino)ferrocene palladium dichloride (56.6 mg, 0.08 mmol). The resulting mixture was stirred at 60 °C for 16 hours under CO (35 psi). The reaction mixture was concentrated and the residue was purified with silica gel column (3% ethyl acetate in petroleum) to afford methyl 4-(1,1-dimethylpropyl)-2-methyl-benzoate (40 mg, 23.5% yield) as a colorless oil.

Step 5: 4-(1,1-Dimethylpropyl)-2-methyl-benzoate (40.0 mg, 0.18 mmol) was hydrolyzed as previously described (General Method NaOH) to give crude 4-(1,1-dimethylpropyl)-2-methyl-benzoic acid (30 mg, 80.1% yield) as a white solid.

Compound 265 (formic acid salt) was prepared as a white solid utilizing the methods in Example 7 (Compound 101). LCMS (Method 5-95 AB, ESI): t_{R} = 0.602 min, [M + H]⁺ = 826.2.

### Example 171: Synthesis of Compound 266

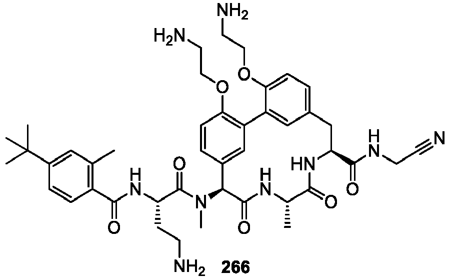

Compound 266 (formic acid salt) was prepared as a white solid utilizing the methods in Example 170 (Compound 265) starting from 4-(tert-butyl)-2-methylphenol. LCMS (Method 5-95 AB, ESI): t_{R} = 0.562 min, [M + H]⁺ = 812.5.

### Example 172: Synthesis of Compound 267

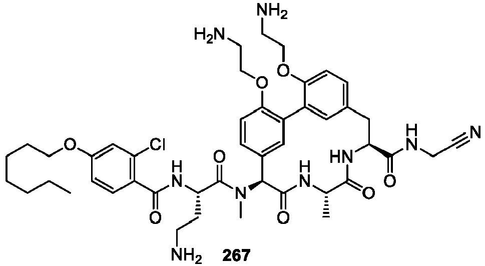

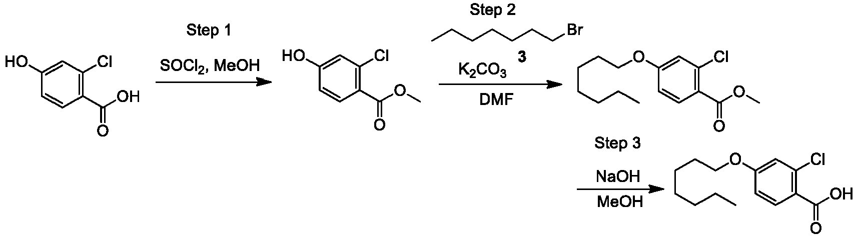

Step 1: To a solution of 2-chloro-4-hydroxybenzoic acid (200.0 mg, 1.16 mmol) in methanol (5 mL) was added thionyl chloride (413.6 mg, 3.48 mmol) dropwise at 0 °C. The reaction mixture was stirred at 70°C for 2 h and concentrated. The residue was diluted with water (15 mL) and extracted with EtOAc (15 mL x 2). The organic layers were combined and washed with water (30 mL x 2) and brine (20 mL). The organic layers were separated, dried over Na₂SO₄ and concentrated to obtain methyl 2-chloro-4-hydroxy-benzoate (200 mg, 92.5% yield) as a yellow solid which was used directly without further purification.

Step 2: To a solution of methyl 2-chloro-4-hydroxy-benzoate (100.0 mg, 0.54 mmol) in N,N-dimethylformamide (3 mL) was added 1-bromoheptane (2879.6 mg, 16.08 mmol) and potassium carbonate (2222.1 mg, 16.08 mmol). The mixture was stirred at 20 °C for 4 h, diluted with water (20 mL) and extracted with EtOAc (20 mL x 2). The organic layers were combined and washed with water (40 mL x 4) and brine (20 mL). The organic layers were separated, dried over Na₂SO₄ and concentrated. The residue was purified by preparative TLC (5% EtOAc in petroleum ether) to obtain methyl 2-chloro-4-heptoxy-benzoate (152 mg, 99.6% yield) as a yellow oil. For more sterically hindered alkyl halides, elevated temperatures are used.

Step 3: Methyl 2-chloro-4-heptoxy-benzoate (152.0 mg, 0.53 mmol) was hydrolyzed previously described (General Method NaOH) to give crude 2-chloro-4-heptoxy-benzoic acid (110 mg, 76.1% yield) as a yellow solid.

Compound 267 (formic acid salt) was prepared as a white solid utilizing the methods in Example 7 (Compound 101). LCMS (Method 5-95 AB, ESI): t_{R} = 0.647 min, [M + H]⁺ = 890.4.

### Example 173: Synthesis of Compound 268

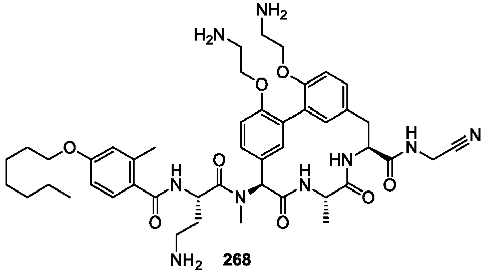

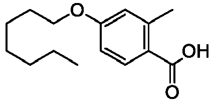

Following similar procedures as described in Example 172 (Compound 267), 4-(heptyloxy)-2-methylbenzoic acid (605 mg, 96.1% yield) was obtained as a yellow solid from 4-hydroxy-2-methylbenzoic acid. ¹H NMR (400 MHz, CDCl₃) δ 8.09 - 8.04 (m, 1H), 6.79 - 6.73 (m, 2H), 4.01 (t, J = 6.4 Hz, 2H), 2.64 (s, 3H), 1.84 - 1.73 (m, 2H), 1.49 - 1.27 (m, 8H), 0.90 (t, J = 7.0 Hz, 3H).

Compound 268 (formic acid salt) was prepared as a white solid utilizing the methods in Example 172 (Compound 267). LCMS (Method 5-95 AB, ESI): t_{R} = 0.608 min, [M + H]⁺ = 870.4. ¹H NMR (400 MHz, MeOH-*d*₄) δ 8.46 (brs, 2H), 7.38 (d, *J*=8.0 Hz, 1H), 7.32 (dd, *J*=8.4, 1.8 Hz, 1H), 7.25 (d, *J*=8.0 Hz, 1H), 7.18 (d, *J*=8.0 Hz, 1H), 7.10 (d, *J*=8.0 Hz, 1H), 6.90 (d, *J*=1.8 Hz, 1H), 6.85 - 6.77 (m, 3H), 6.35 (s, 1H), 5.14-5.10 (m, 1H), 4.82-4.76 (m, 2H), 4.36-4.18 (m, 4H), 4.20 (s, 2H), 3.99 (t, *J*=6.4 Hz, 2H), 3.35-3.20 (m, 5H), 3.17-3.08 (m, 3H), 2.91 (s, 3H), 2.40 (s, 3H), 2.30-2.24 (m, 1H), 2.20-2.05 (m, 1H), 1.81-1.74 (m, 2H), 1.53-1.31 (m, 11H), 0.92 (t, *J*=6.6 Hz, 3H).

### Example 174: Synthesis of Compound 269

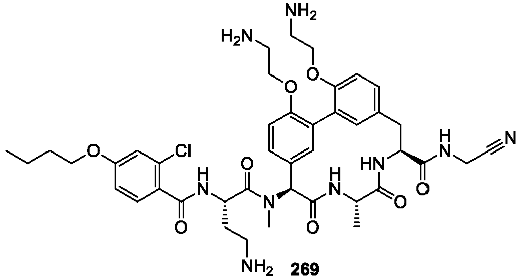

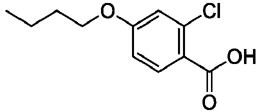

Following similar procedures as described in Example 172, 4-butoxy-2-chlorobenzoic acid (160 mg, 0.70 mmol) was obtained as a white solid from 2-chloro-4-hydroxybenzoic acid. ¹H NMR (400 MHz, CD₃OD): δ 7.90 (d, J = 9.2 Hz, 1H), 7.02 (d, J = 2.0 Hz, 1H), 6.91 (dd, J = 9.0, 2.6 Hz, 1H), 4.04 (t, J = 6.4 Hz, 2H), 1.80 - 1.70 (m, 2H), 1.55 - 1.45 (m, 2H), 0.99 (t, J = 7.6 Hz, 3H).

Compound 269 (formic acid salt) was prepared as a white solid utilizing the methods in Example 172 (Compound 267). LCMS (Method 5-95 AB, ESI): t_{R} = 0.574 min, [M + H]⁺ = 848.5.

### Example 175: Synthesis of Compound 270

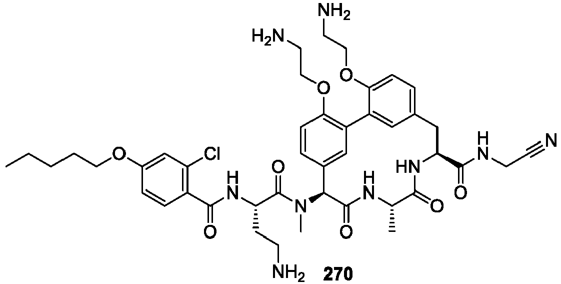

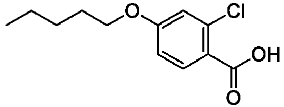

Following similar procedures as described in Example 172, 2-chloro-4-(pentyloxy)benzoic acid (160 mg, 0.6593 mmol, 94% yield) was obtained as a yellow solid. ¹H NMR (400 MHz, CD₃OD): δ 7.90 (d, J = 8.8 Hz, 1H), 7.02 (d, J = 2.8 Hz, 1H), 6.92 (dd, J = 8.8, 2.4 Hz, 1H), 4.04 (t, J = 6.4 Hz, 2H), 1.85 - 1.75 (m, 2H), 1.50 - 1.35 (m, 4H), 0.96 (t, J = 7.2 Hz, 3H).

Compound 270 (formic acid salt) was prepared as a white solid utilizing the methods in Example 172 (Compound 267). LCMS (Method 5-95 AB, ESI): t_{R} = 0.591 min, [M + H]⁺ = 862.6.

### Example 176: Synthesis of Compound 271

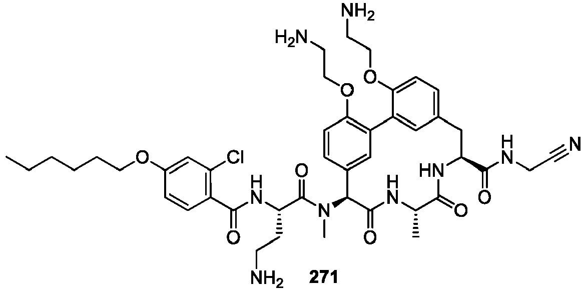

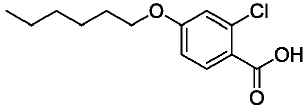

Following similar procedures as described in Example 172, 2-chloro-4-(hexyloxy)benzoic acid (670 mg, 2.61 mmol, 88.3% yield) was obtained as a white solid. LCMS (Method 5-95 AB, ESI): t_{R} = 0.926 min, [M + H]⁺ = 256.9.

Compound 271 (formic acid salt) was prepared as a white solid utilizing the methods in Example 172 (Compound 267). LCMS (Method 5-95 AB, ESI): t_{R} = 0.735 min, [M + H]⁺ = 876.6.

### Example 177: Synthesis of Compound 272

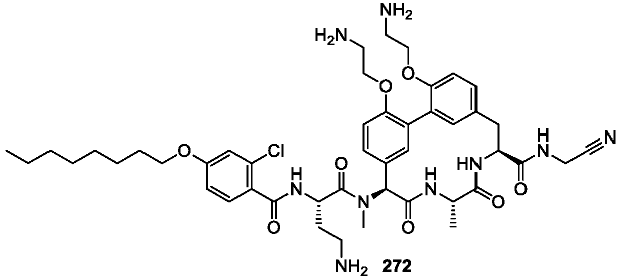

Compound 272 (formic acid salt) was prepared as a white solid utilizing the methods in Example 172 (Compound 267) from 2-chloro-4-(octyloxy)benzoic acid. LCMS (Method 5-95 AB, ESI): t_{R} = 0.649 min, [M + H]⁺ = 904.6.

### Example 178: Synthesis of Compound 273

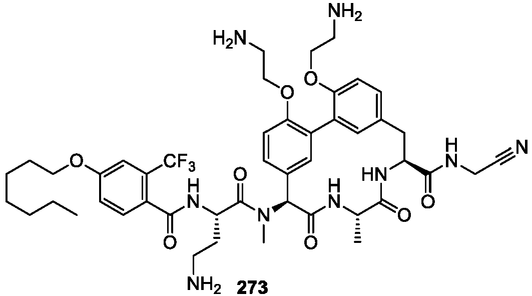

Compound 273 (formic acid salt) was prepared as a white solid utilizing the methods in Example 172 (Compound 267) from 4-(heptyloxy)-2-(trifluoromethyl)benzoic acid. LCMS (Method 5-95 AB, ESI): t_{R} = 0.634 min, [M + H]⁺ = 924.4.

### Example 179: Synthesis of Compound 274

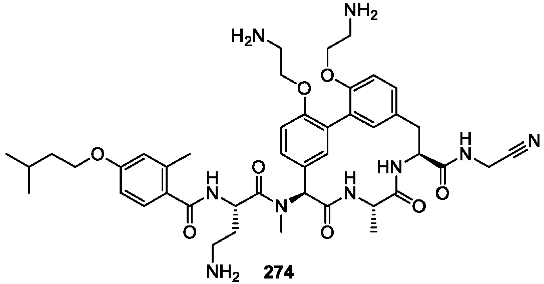

Compound 274 (formic acid salt) was prepared as a white solid utilizing the methods in Example 172 (Compound 267). LCMS (Method 5-95 AB, ESI): t_{R} = 0.582 min, [M+H]⁺ = 842.3.

### Example 180: Synthesis of Compound 275

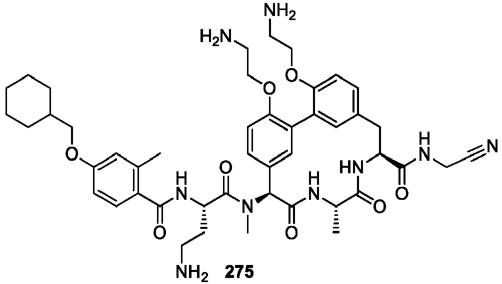

Compound 275 (formic acid salt) was prepared utilizing the methods in Example 172 (Compound 267). LCMS (Method 5-95 AB, ESI): t_{R} = 0.601 min, [M+H]⁺ = 868.5.

### Example 181: Synthesis of Compound 276

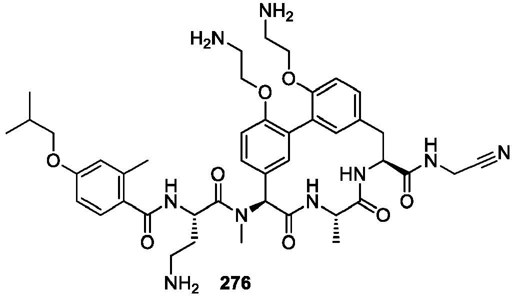

Compound 276 (formic acid salt) was prepared utilizing the methods in Example 172 (Compound 267). LCMS (Method 5-95 AB, ESI): t_{R} = 0.679 min, [M+H]⁺ = 828.5.

### Example 182: Synthesis of Compound 277

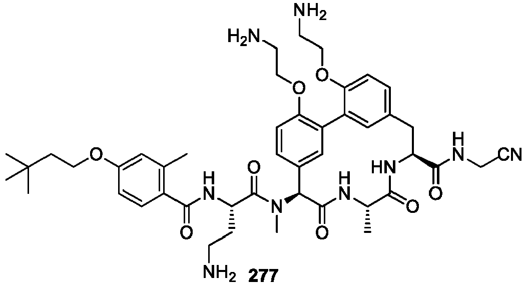

Compound 277 (formic acid salt) was prepared utilizing the methods in Example 172 (Compound 267). LCMS (Method 5-95 AB, ESI): t_{R} = 0.613 min, [M+Na]⁺ = 878.4.

### Example 183: Synthesis of Compound 278

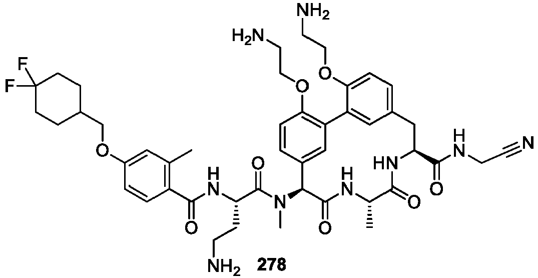

Compound 278 (formic acid salt) was prepared utilizing the methods in Example 172 (Compound 267). LCMS (Method 5-95 AB, ESI): t_{R} = 0.706 min, [M+H]⁺ = 904.5.

### Example 184: Synthesis of Compound 279

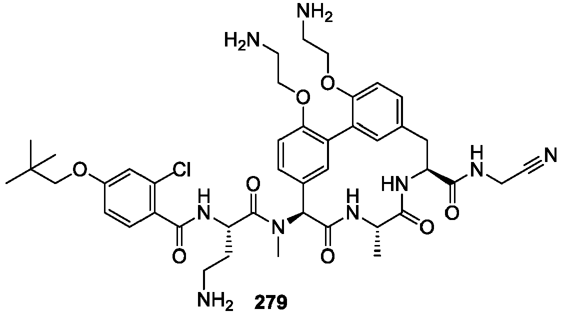

Compound 279 (formic acid salt) was prepared utilizing the methods in Example 172 (Compound 267). LCMS (Method 5-95 AB, ESI): t_{R} = 0.713 min, [M+H]⁺ = 862.4.

### Example 185: Synthesis of Compound 280

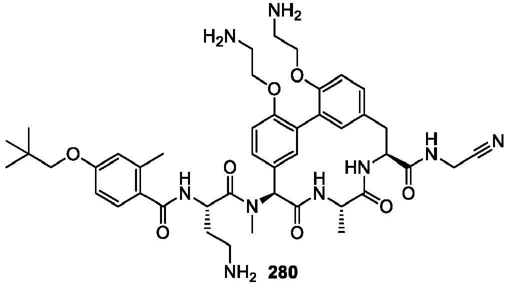

Compound 280 (formic acid salt) was prepared as a white solid utilizing the methods in Example 172 (Compound 267) from 2-methyl-4-(neopentyloxy)benzoic acid. LCMS (Method 5-95 AB, ESI): t_{R} = 0.575 min, [M/2+H]⁺ = 421.9.

### Example 186: Synthesis of Compound 281

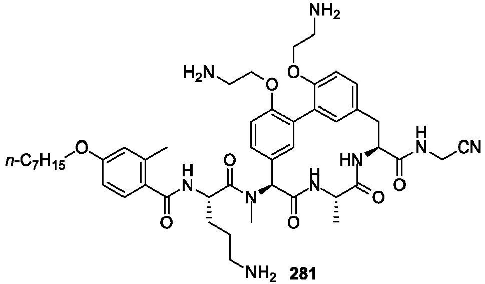

Compound 281 (formic acid salt) was prepared as a white solid (38 mg) utilizing the methods in Example 172 (Compound 267) from 4-(heptyloxy)-2-methylbenzoic acid. LCMS (Method 5-95 AB, ESI): t_{R} = 0.755, [M + H]⁺ = 884.5.

### Example 187: Synthesis of Compound 282

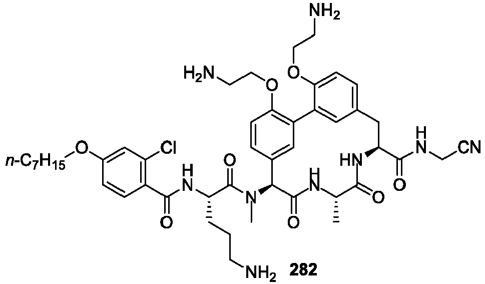

Compound 282 (formic acid salt) was prepared as a white solid (87 mg) utilizing the methods in Example 172 (Compound 267) from 2-chloro-4-(heptyloxy)benzoic acid. LCMS (Method 5-95 AB, ESI): t_{R} = 0.760, [M + H]⁺ = 904.9; ¹H NMR (400 MHz, MeOH-*d*₄) δ 8.45 (brs, 1H, HCOOH), 7.46 (d, *J*=8.0 Hz, 1H), 7.32-7.24 (m, 2H), 7.18 (d, *J*=8 Hz, 1H), 7.11 (d, *J*=8 Hz, 1H), 7.02 (brs, 1H), 6.94 (d, *J*=8.0 Hz, 1H), 6.89 (brs, 1H), 6.81 (brs, 1H), 6.41 (s, 1H), 5.07-5.03 (m, 1H), 4.81-4.75 (m, 2H), 4.29-4.17 (m, 4H), 4.20 (s, 2H), 4.01 (t, *J*=4.8 Hz, 2H), 3.38-3.34 (m, 1H), 3.28-3.24 (m, 4H), 3.15-3.05 (m, 1H), 3.01-2.94 (m, 1H), 2.96 (s, 3H), 2.02-1.97 (m, 1H), 1.90-1.75 (m, 5H), 1.52-1.30 (m, 11H), 0.92 (t, *J*=6.8 Hz, 3H).

### Example 188: Synthesis of Compound 283

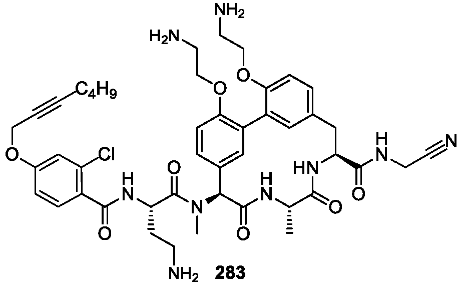

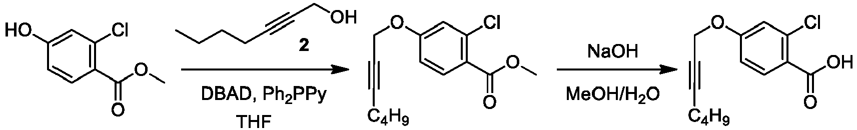

To a stirred solution of methyl 2-chloro-4-hydroxy-benzoate (200.0 mg, 1.07 mmol), hept-2-yn-1-ol (120.23 mg, 1.07 mmol), and diphenyl-2-pyridylphosphine (338.63 mg, 1.29 mmol) in dry tetrahydrofuran (10 mL) at 0 °C under a nitrogen atmosphere was added dropwise a solution of di-t-butyl azodicarboxylate (296.17 mg, 1.29 mmol) in THF (2 mL) over a period of 5 min. The reaction was stirred for 1 h and the solvent was evaporated under reduced pressure. The resulting oil was diluted with water (30 mL) and extracted with EtOAc (25mL x 3). The combined organic layers were washed with 1N HCl (10 mL x 3) and brine (20 mL x 3), dried over Na₂SO₄ and concentrated. The residue was purified by prep-TLC (10% EtOAc in petroleum ether, Rf = 0.1) to give methyl 2-chloro-4-hept-2-ynoxy-benzoate (220 mg, 0.7836 mmol, 73.1% yield) as a colorless oil. HNMR (400 MHZ, CDCl₃): 7.86 (d, J = 8.8 Hz, 1H), 7.04 (d, J = 2.8 Hz, 1H), 6.89-6.87 (m, 1H), 4.69 (s, 2H), 3.88 (s, 3H), 2.20 (t, J = 6.8 Hz, 2H), 1.49-1.43 (m, 2H), 1.39-1.35 (m, 2H), 0.87 (t, J = 7.2 Hz, 3H).

Compound 283 (formic acid salt) was prepared utilizing the methods in Example 7 (Compound 101). LCMS (Method 5-95 AB, ESI): t_{R} = 0.712 min, [M+H]⁺ = 886.4.

### Example 189: Synthesis of Compound 284

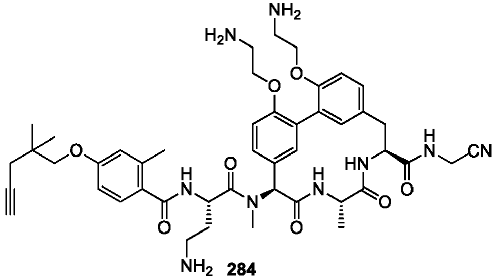

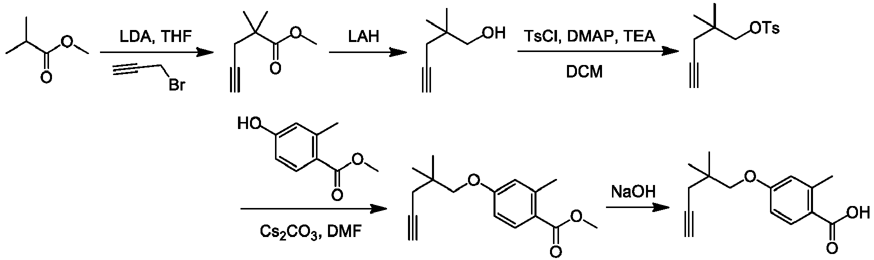

To a solution of methyl isobutyrate (2000.0 mg, 19.58 mmol) in tetrahydrofuran (50 mL) cooled at -78 °C under N₂ protection was added lithium diisopropylamide (11.75 mL, 23.5 mmol) and stirred at 0 °C for 3 h. Propargyl bromide (2562.5 mg, 21.54 mmol) was added. After being stirred at 20 °C for 2 h, the reaction was quenched with saturated NH₄Cl solution (10 mL) and extracted with EtOAc (20 mL). The organic layer was washed with water (20 mL x 2) and brine (20 mL), dried over MgSO₄ and concentrated. The residue was purified by flash column chromatography (10% EtOAc in petroleum ether) to give methyl 2,2-dimethylpent-4-ynoate (500 mg, 3.5668 mmol, 18.2% yield) as a yellow oil.

To a solution of methyl 2,2-dimethylpent-4-ynoate (4000.0 mg, 28.54 mmol) in tetrahydrofuran (5 mL) cooled at 0 °C was added lithium aluminum hydride (1600.0 mg, 42.8 mmol) in portions, and stirred at 0 °C for 3 h. The reaction was quenched sequentially with water (1 mL), 10% NaOH solution (1 mL), and water (1 mL). The mixture was filtered and concentrated to afford 2,2-dimethylpent-4-yn-1-ol (3000 mg, 26.745 mmol, 93.7% yield) as a yellow oil. ¹H NMR (400 MHz, CDCl₃) δ 3.44 (s, 2H), 2.18 (d, J = 2.4 Hz, 2H), 2.01 (t, J = 2.6 Hz, 1H), 0.99 (s, 6H).

A mixture of 2,2-dimethylpent-4-yn-1-ol (500.0 mg, 4.46 mmol), 4-dimethylaminopyridine (54.5 mg, 0.45 mmol), triethylamine (2255.3 mg, 22.29 mmol) and p-toluenesulfonyl chloride (1699.7 mg, 8.92 mmol) in dichloromethane (20 mL) was stirred at 35 °C for 16 h and concentrated to dryness. The residue was taken up in EtOAc (20 mL), washed with water (20 mL x 2) and brine (10 mL), dried over MgSO₄ and concentrated. The residue was purified by flash column chromatography (10% EtOAc in petroleum ether) to afford 2,2-dimethylpent-4-ynyl 4-methylbenzenesulfonate (900 mg, 3.38 mmol, 75.8% yield) as a yellow oil.

A mixture of methyl 4-hydroxy-2-methyl-benzoate (100.0 mg, 0.60 mmol), cesium carbonate (588.23 mg, 1.81 mmol) and 2,2-dimethylpent-4-ynyl 4-methylbenzenesulfonate (320.6 mg, 1.2 mmol) in N,N-dimethylformamide (10 mL) was stirred at 120 °C for 16 h. The reaction was quenched with water (20 mL) and extracted with EtOAc (20 mL). The organic layer was washed with water (20 mL x 2) and brine (20 mL), dried over MgSO₄ and concentrated. The residue was purified by flash column chromatography (10% EtOAc in petroleum ether) to afford methyl 4-(2,2-dimethylpent-4-ynoxy)-2-methyl-benzoate (150 mg, 95.7% yield) as a yellow oil. Ester hydrolysis with NaOH (General Method NaOH) afforded 4-((2,2-dimethylpent-4-yn-1-yl)oxy)-2-methylbenzoic acid.

Compound 284 (formic acid salt) was prepared utilizing the methods in Example 7 (Compound 101). LCMS (Method 5-95 AB, ESI): t_{R} = 0.600 min, [M+H]⁺ = 867.5.

### Example 190: Synthesis of Compound 285

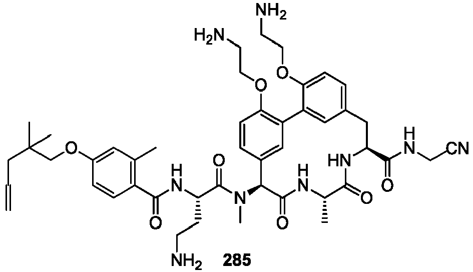

Compound 285 (formic acid salt) was prepared as a white solid utilizing the methods in Example 189 (Compound 284) except allyl bromide was used in the initial alkylation step with methyl isobutyrate. LCMS (Method 5-95 AB, ESI): t_{R} = 0.599 min, [M+H]⁺ = 868.5.

### Example 192: Synthesis of Compound 287

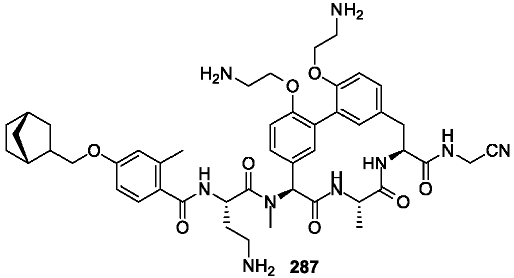

Compound 287 (formic acid salt) was prepared as a white solid utilizing the methods in Example 189 (Compound 284) starting from (1R,4S)-bicyclo[2.2.1]heptan-2-ylmethanol.

LCMS (Method 5-95 AB, ESI): RT = 0.723, [M+H]⁺ = 880.5.

### Example 193: Synthesis of Compound 288

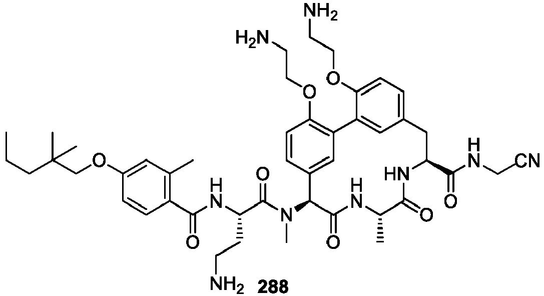

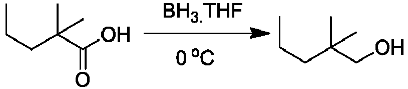

To 2,2-dimethylpentanoic acid (500 mg, 3.84 mmol) was added 1M borane (19.2 mL, 19.2 mmol) in tetrahydrofuran at 0°C, and stirred for 16 h under nitrogen. The reaction was quenched by MeOH at 0°C and concentrated to obtain crude 2,2-dimethylpentan-1-ol (540 mg, 4.6472 mmol) as a yellow oil. ¹H NMR (400 MHz, CDCl₃): δ 3.31 (s, 2H), 1.35-1.15 (m, 4H), 0.90 (t, J = 6.8 Hz, 3H), 0.86 (s, 6H).

Compound 288 (formic acid salt) was prepared as a white solid utilizing the methods in Example 189 (Compound 284). LCMS (Method 5-95 AB, ESI): t_{R} = 0.749 min, [M+Na]⁺ = 892.8.

### Example 194: Synthesis of Compound 289

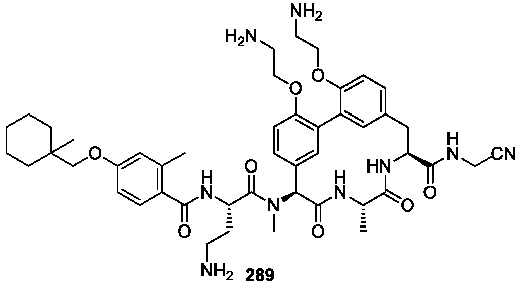

Compound 289 (formic acid salt) was prepared as a white solid utilizing the methods in Example 193 (Compound 288)starting from 1-methylcyclohexanecarboxylic acid. LCMS (Method 5-95 AB, ESI): t_{R} = 0.636 min, [M+Na]⁺ = 904.5.

### Example 195: Synthesis of Compound 290

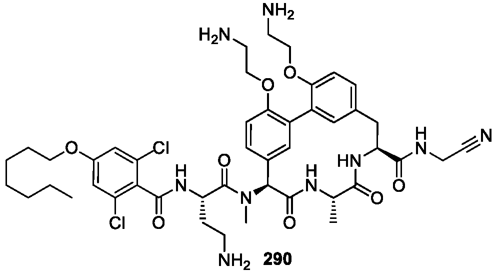

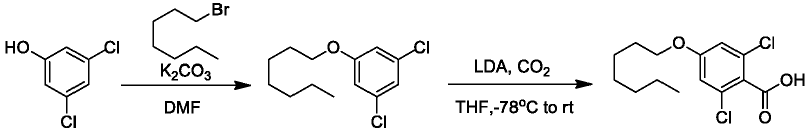

Step 1: 1,3-Dichloro-5-heptoxy-benzene was prepared using the methods in Example 172 (Compound 267).

Step 2: To a solution of 1,3-dichloro-5-heptoxy-benzene (200.0 mg, 0.77 mmol) in tetrahydrofuran (10 mL) was added lithium diisopropylamide (0.37 mL, 0.92 mmol) and stirred at -78°C for 1 h. CO₂ was bubbled and stirred at -78 °C for 2 h. The reaction was quenched with water (40 mL) and extracted with EtOAc (30 mL × 3). The organic layers were washed with water (50 mL × 2) and brine (50 mL), dried over MgSO₄ and concentrated. The residue was purified by prep-TLC (20% EtOAc in petroleum ether, Rf = 0.5) to obtain 2,6-dichloro-4-heptoxy-benzoic acid (150 mg, 64.2% yield) as a white solid. ¹H NMR (400 MHz, CDCl₃): δ 6.72 (s, 2H), 3.83 (t, J = 6.4 Hz, 2H), 1.69 - 1.64 (m, 2H), 1.35 - 1.19 (m, 8H), 0.82 (t, J = 6.4 Hz, 3H).

Compound 290 (formic acid salt) was prepared utilizing the methods in Example 7 (Compound 101). LCMS (Method 5-95 AB, ESI): t_{R} = 0.754 min, [M+H]⁺ = 924.5.

### Example 196: Synthesis of Compound 291

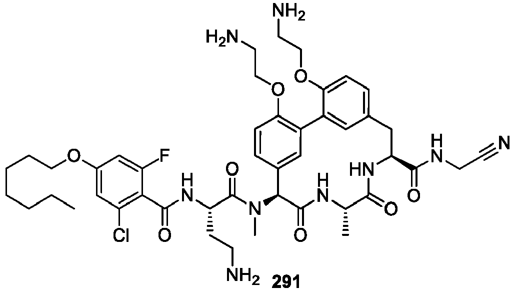

Compound 291 (formic acid salt) was prepared utilizing the methods in Example 195 (Compound 290). LCMS (Method 5-95 AB, ESI): t_{R} = 0.756 min, [M+H]⁺ = 909.6.

### Example 197: Synthesis of Compound 292

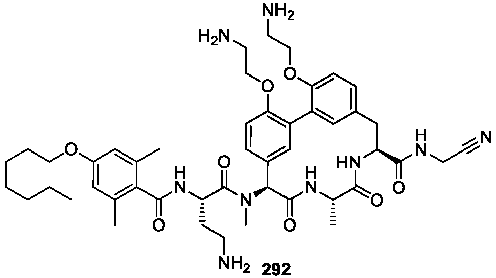

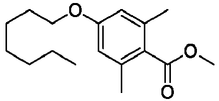

Methyl 4-(heptyloxy)-2,6-dimethylbenzoate was prepared by the carbonylation conditions from Example 170 (Compound 265) from 2-bromo-5-(heptyloxy)-1,3-dimethylbenzene.

Compound 292 (formic acid salt) was prepared utilizing the methods in Example 170 (Compound 265). LCMS (Method 5-95 AB, ESI): t_{R} = 0.752 min, [M+H]⁺ = 884.5.

### Example 198: Synthesis of Compound 293

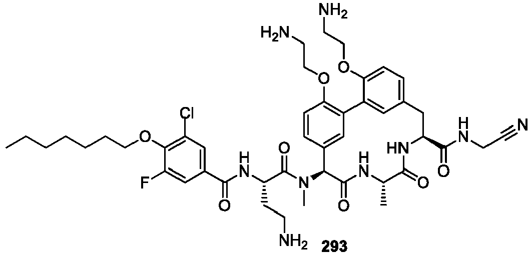

Compound 293 (formic acid salt) was prepared utilizing the methods in Example 197 (Compound 292). LCMS (Method 5-95 AB, ESI): t_{R} = 0.631 min, [M+H]⁺ = 908.5.

### Example 199: Synthesis of Compound 294

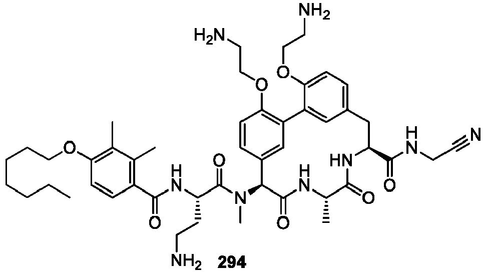

Compound 294 (formic acid salt) was prepared utilizing the methods in Example 197 (Compound 292). LCMS (Method 5-95 AB, ESI): t_{R} = 0.761 min, [M+H]⁺ = 884.4.

### Example 200: Synthesis of Compound 295

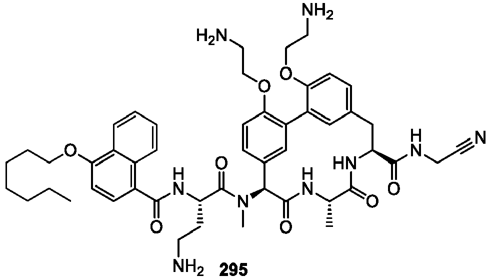

Compound 295 (formic acid salt) was prepared utilizing the methods in Example 197 (Compound 292). LCMS (Method 5-95 AB, ESI): t_{R} = 0.635 min, [M+H]⁺ = 906.4.

### Example 201: Synthesis of Compound 296

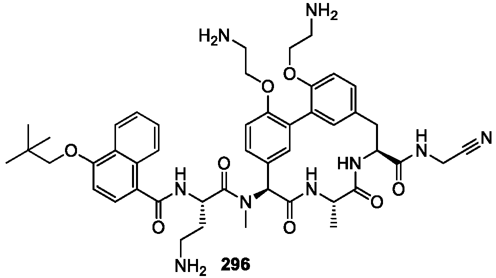

Compound 296 (formic acid salt) was prepared utilizing the methods in Example 197 (Compound 292). LCMS (Method 5-95 AB, ESI): t_{R} = 0.738 min, [M + H]⁺ = 878.4.

### Example 202: Synthesis of Compound 297

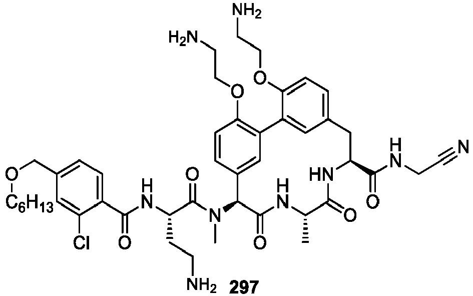

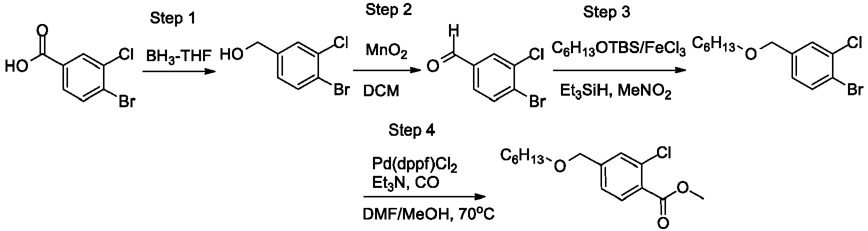

Step 1: Borane-THF complex (31.85 mL, 31.85 mmol) was added to 4-bromo-3-chlorobenzoic acid (1.5 g, 6.37 mmol) at 0 °C, and stirred at 0 °C for 10 h. The reaction was quenched by methanol (50 mL) and concentrated under reduced pressure to give (4-bromo-3-chlorophenyl)methanol (1.3 g, 5.8696 mmol, 92.1% yield) as a light yellow oil.

Step 2: To a solution of (4-bromo-3-chlorophenyl)methanol (1.3 g, 5.87 mmol) in 1,2-dichloroethane (20 mL) at 25 °C was added manganese dioxide (5.1 g, 58.7 mmol), and stirred at 25 °C for 18 h. The mixture was filtered and the filtrate was concentrated under reduced pressure. The residue was purified by silica gel column (5-10% EtOAc in petroleum ether) to give 4-bromo-3-chlorobenzaldehyde (600 mg, 46.6% yield) as a light yellow oil.

Step 3: To a suspension of anhydrous ferric chloride (14.2 mg, 0.09 mmol) and 4-bromo-3-chlorobenzaldehyde (462.4 mg, 2.11 mmol) in nitromethane (10 mL) were successively added tert-butyl(hexyloxy)dimethylsilane (380.0 mg, 1.76 mmol) and triethylsilane (449.1 mg, 3.86 mmol) at 0 °C under nitrogen. After stirring at room temperature for 30 min, the reaction was quenched with phosphate buffer till pH = 7 and extracted with DCM (45 mL × 3). The combined organic layers were washed with brine (15 mL × 3), dried over Na₂SO₄, filtered and concentrated. The residue was purified by prep-TLC (5% EtOAc in petroleum ether) to give 1-bromo-2-chloro-4-((hexyloxy)methyl)benzene (450 mg, 83.9% yield) as a colorless oil.

Step 4: Following the methoxycarbonylation procedure (General Method Carbonylation), 1-bromo-2-chloro-4-((hexyloxy)methyl)benzene (200.0 mg, 0.65 mmol) was converted to methyl 2-chloro-4-((hexyloxy)methyl)benzoate (125 mg, 67.1% yield) as a colorless oil. ¹H NMR (400 MHz, CDCl₃): δ 7.80 (d, *J* = 8.0 Hz, 1H), 7.42 (s, 1H), 7.25 (d, *J* = 8.0 Hz, 1H), 4.49 (s, 2H), 3.91 (s, 3H), 3.46 (t, *J* = 6.6 Hz, 2H), 1.64 - 1.61 (m, 2H), 1.37 - 1.29 (m, 6H), 0.88 (t, *J* = 6.6 Hz, 3H).

Compound 297 (formic acid salt) was prepared utilizing the methods in Example 7 (Compound 101). LCMS (Method 5-95 AB, ESI): t_{R} = 0.719 min, [M+H]⁺ = 890.9.

### Example 203: Synthesis of Compound 298

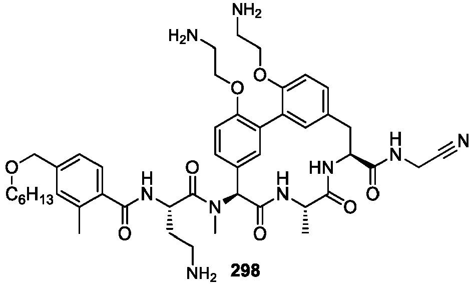

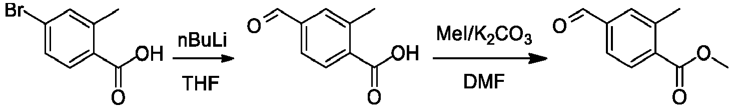

To a solution of 4-bromo-2-methylbenzoic acid (3.0 g, 13.95 mmol) in tetrahydrofuran (30 mL) at -78°C was added 2M nBuLi (13.95 mL, 27.9 mmol) in hexanes and stirred at -78 °C for 3 h. DMF (2.0 g, 27.9 mmol) was added and the mixture was stirred at -78 °C for 1.5 h. The reaction was quenched with 1N HCl (20 mL) and extracted with EtOAc (200 mL x 4). The combined organic layers were dried over Na₂SO₄ and concentrated. The resulting yellow solid was washed with petroleum ether to give crude 4-formyl-2-methyl-benzoic acid (900 mg, 5.4825 mmol, 39.3% yield), which was used directly in the next step.

To a solution of 4-formyl-2-methyl-benzoic acid (0.9 g, 5.48 mmol) in N,N-dimethylformamide (5 mL) at 20°C were added K₂CO₃ (2269.8 mg, 16.45 mmol) and iodomethane (2.84 g, 20.01 mmol) and stirred for 10 h. The mixture was diluted with 1N HCl (15 mL) and extracted with EtOAc (30 mL × 3). The combined organic layers were washed with brine (30 mL × 3), dried over Na₂SO₄ and concentrated. The residue was purified by silica gel column (5% EtOAc in petroleum ether) to give methyl 4-formyl-2-methyl-benzoate (900 mg, 5.0511 mmol, 92.1% yield) as a yellow oil. ¹H NMR (400 MHz, CDCl₃): δ 10.03 (s, 1H), 8.01 (d, J = 8.4 Hz, 1H), 7.74-7.72 (m, 2H), 3.92 (s, 3H), 2.65 (s, 3H).

Compound 298 (formic acid salt) was prepared utilizing the methods in Example 202 (Compound 297). LCMS (Method 5-95 AB, ESI): t_{R} = 0.726 min, [M+H]⁺ = 870.5.

### Example 204: Synthesis of Compound 299

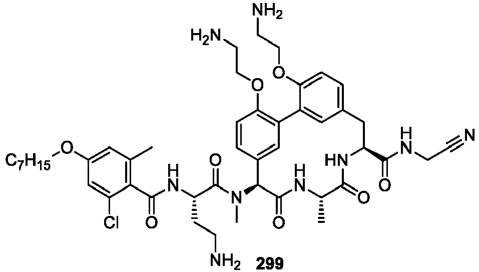

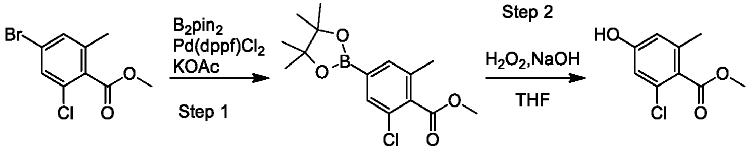

Step 1: To a solution of methyl 4-bromo-2-chloro-6-methylbenzoate (300 mg, 1.14 mmol) and bis(pinacolato)diboron (361.4 mg, 1.42 mmol) in dimethyl sulfoxide (5 mL) were added 1,1'-bis(diphenylphosphino)ferrocene palladium dichloride (83.3 mg, 0.11 mmol) and potassium acetate (290.5 mg, 2.96 mmol), and stirred at 100 °C for 16 h under N₂. The reaction mixture was diluted with water (5 mL) and extracted with EtOAc (10 mL × 3). The combined organic layers were washed with brine, dried over Na₂SO₄, filtered and concentrated. The residue was purified by prep-TLC (9% EtOAc in petroleum ether, Rf = 0.5) to give methyl 2-chloro-6-methyl-4-(4,4,5,5-tetramethyl-1,3,2-dioxaborolan-2-yl)benzoate (260 mg, 73.5% yield). ¹H NMR (400 MHz, CDCl₃): δ 7.63 (s, 1H), 7.51 (s, 1H), 3.92 (s, 3H), 2.30 (s, 3H), 1.32 (s, 12H).

Step 2: To a solution of methyl 2-chloro-6-methyl-4-(4,4,5,5-tetramethyl-1,3,2-dioxaborolan-2-yl)benzoate (260.0 mg, 0.84 mmol) in tetrahydrofuran (3 mL) were added hydrogen peroxide (187.1 mg, 1.93 mmol) and 1M aq. sodium hydroxide (0.84 mL, 0.84 mmol), and stirred at 28 °C for 16 h. The mixture was diluted with water (5 mL) and extracted with EtOAc (10 mL × 3). The combined organic layers were dried over Na₂SO₄, filtered and concentrated to give crude methyl 2-chloro-4-hydroxy-6-methylbenzoate (150 mg, 89.3% yield) as a yellow solid. LCMS (Method 5-95 AB, ESI): t_{R} = 0.654 min, [M+H]⁺ = 200.9.

Compound 299 (formic acid salt) was prepared as a white solid utilizing the methods in Example 172 (Compound 267). LCMS (Method 5-95 AB, ESI): t_{R} = 0.756 min, [M+H]⁺ = 904.5.

### Example 205: Synthesis of Compound 300 and 301

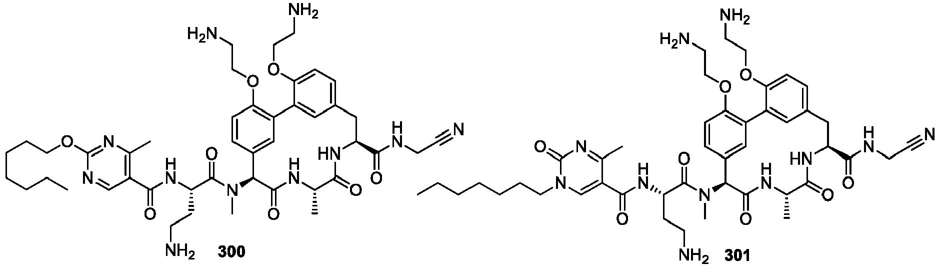

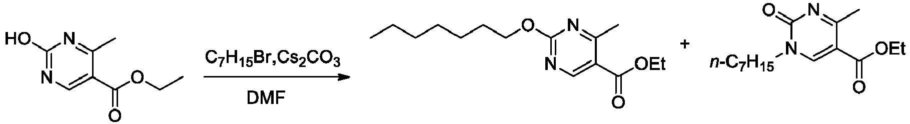

To a solution of ethyl 2-hydroxy-4-methylpyrimidine-5-carboxylate (200.0 mg, 1.1 mmol) in N,N-dimethylformamide (5 mL) were added cesium carbonate (1073.1 mg, 3.29 mmol) and 1-bromoheptane (636.03 mg, 3.29 mmol). The mixture was stirred at 20 °C for 16 h and concentrated to dryness. The residue was taken up in EtOAc (50 mL), washed with water (50 mL x 2) and brine (50 mL), dried over MgSO₄ and concentrated. The residue was purified by prep-TLC to afford ethyl 2-(heptyloxy)-4-methylpyrimidine-5-carboxylate (55 mg, 0.1962 mmol, 17.9% yield; 5% EtOAc in petroleum ether, Rf = 0.3) and ethyl 1-heptyl-4-methyl-2-oxo-1,2-dihydropyrimidine-5-carboxylate (130 mg, 0.4637 mmol, 42.2% yield; 50% EtOAc in petroleum ether, Rf = 0.3), both as a white solid.

Compound 299 (formic acid salt) was prepared as a white solid utilizing the methods in Example 7 (Compound 101) from ethyl 2-(heptyloxy)-4-methylpyrimidine-5-carboxylate. LCMS (Method 5-95 AB, ESI): t_{R} = 0.722 min, [M+H]⁺ = 872.4.

Compound 300 (formic acid salt) was prepared as a white solid utilizing the methods in Example 7 (Compound 101) from ethyl 1-heptyl-4-methyl-2-oxo-1,2-dihydropyrimidine-5-carboxylate. LCMS (Method 5-95 AB, ESI): t_{R} = 0.646 min, [M+H]⁺ = 872.6.

### Example 206: Synthesis of Compound 302

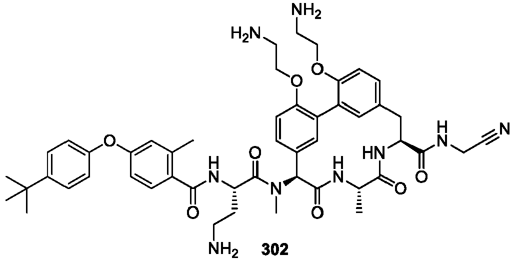

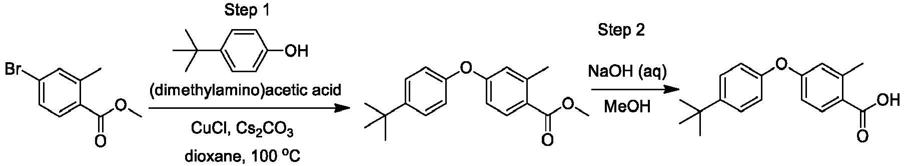

Step 1: A degassed mixture of 4-tert-butylphenol (275.4 mg, 1.83 mmol), methyl 4-bromo-2-methylbenzoate (350.0 mg, 1.53 mmol), (dimethylamino)acetic acid (86.7 mg, 0.84 mmol), cuprous chloride (75.6 mg, 0.76 mmol) and cesium carbonate (995.7 mg, 3.06 mmol) in 1,4-dioxane (10 mL) was heated at 105 °C for 16 h under nitrogen. After cooling down, the reaction was diluted with EtOAc (15 mL) and filtered. The filtrate was concentrated and the residue was purified by prep-TLC (2% EtOAc in petroleum ether, Rf = 0.5) to give methyl 4-(4-(tert-butyl)phenoxy)-2-methylbenzoate (240 mg, 52.6% yield) as a colorless oil.

Step 2: Methyl 4-(4-(tert-butyl)phenoxy)-2-methylbenzoate (240.0 mg, 0.80 mmol) was hydrolyzed as previously described (General Method NaOH) to give 4-(4-(tert-butyl)phenoxy)-2-methylbenzoic acid (210 mg, 91.8% yield) as a white solid. ¹H NMR (400 MHz, CDCl₃): δ 8.06 (d, *J* = 8.4 Hz, 1H), 7.41 (d, *J* = 8.8 Hz, 2H), 7.01 (d, *J* = 8.4 Hz, 2H), 6.89 - 6.78 (m, 2H), 2.64 (s, 3H), 1.36 (s, 9H).

Compound 302 (formic acid salt) was prepared as a white solid utilizing the methods in Example 7 (Compound 101). LCMS (Method 5-95 AB, ESI): t_{R} = 0.777 min, [M + H]⁺ = 904.6.

### Example 207: Synthesis of Compound 303

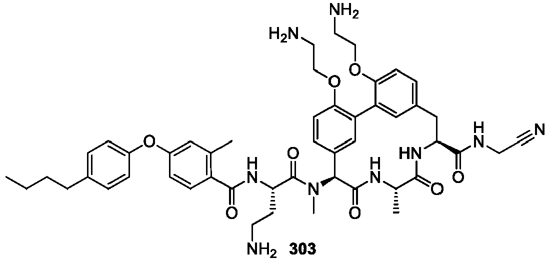

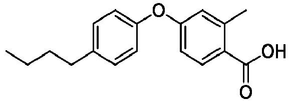

Following similar procedures as described in Example 206, 4-(4-butylphenoxy)-2-methylbenzoic acid (180 mg, 94.4% yield) was obtained as a white solid. ¹H NMR (400 MHz, CDCl₃): δ 8.06 (d, J = 8.8 Hz, 1H), 7.20 (d, J = 8.4 Hz, 2H), 6.99 (d, J = 8.4 Hz, 2H), 6.86 - 6.77 (m, 2H), 2.67 - 2.58 (m, 5H), 1.67-1.59 (m, 2H), 1.42-1.36 (m, 2H), 0.96 (t, J = 7.2 Hz, 3H).

Compound 303 (formic acid salt) was prepared as a white solid utilizing the methods in Example 7 (Compound 101). LCMS (Method 5-95 AB, ESI): t_{R} = 0.756 min, [M + H]⁺ = 904.4.

### Example 208: Synthesis of Compound 304

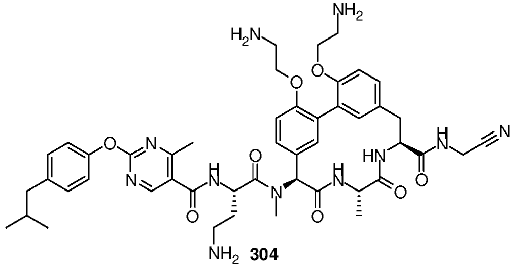

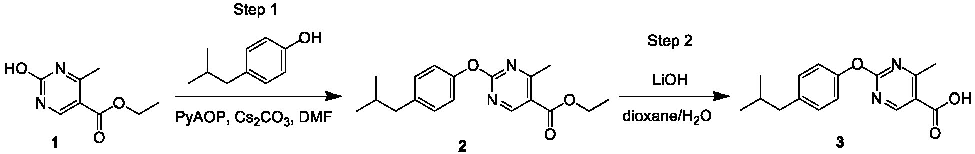

A mixture of ethyl 2-hydroxy-4-methyl-pyrimidine-5-carboxylate (50.0 mg, 0.27 mmol), PyAOP (485.5 mg, 1.1 mmol) and Cs₂CO₃ (179 mg, 0.55 mmol) in N,N-dimethylformamide (5 mL) was stirred at 25 °C for 1 h, then 4-isobutylphenol (164.9 mg, 1.1 mmol) and Cs₂CO₃ (179 mg, 0.55 mmol) were added. The reaction was stirred in the microwave at 75 °C for 1 h, diluted with water (20 mL) and extracted with EtOAc (25 mL × 3). The combined organic layers were washed with brine (25 mL × 3), dried over Na₂SO₄ and concentrated. The residue was purified by prep-TLC (10% EtOAc in petroleum ether) to give ethyl 2-(4-isobutylphenoxy)-4-methylpyrimidine-5-carboxylate (110 mg, 63.7% yield).

Step 2: Ethyl 2-(4-isobutylphenoxy)-4-methylpyrimidine-5-carboxylate (110.0 mg, 0.35 mmol) was hydrolyzed with LiOH (General Method LiOH) to afford 2-(4-isobutylphenoxy)-4-methylpyrimidine-5-carboxylic acid (60 mg, 59.9% yield) as a white solid. ¹H NMR (400 MHz, CDCl₃): δ 9.07 (s, 1H), 7.20 (d, *J* = 8.8 Hz, 2H), 7.11 (d, *J* = 8.4 Hz, 2H), 2.82 (s, 3H), 2.50 (d, *J* = 7.2 Hz, 2H), 1.95-1.80 (m, 1H), 0.94 (d, *J* = 6.4 Hz, 6H).

Compound 304 (formic acid salt) was prepared as a white solid utilizing the methods in Example 7 (Compound 101). LCMS (Method 5-95 AB, ESI): t_{R} = 0.722 min, [M+H]⁺ = 906.4.

### Example 209: Synthesis of Compound 305

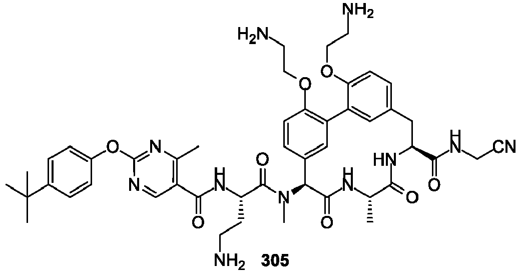

Compound 305 (formic acid salt) was prepared as a white solid utilizing the methods in Example 208 (Compound 304). LCMS (Method 5-95 AB, ESI): t_{R} = 0.706 min, [M+H]⁺ = 906.5.

### Example 210: Synthesis of Compound 306

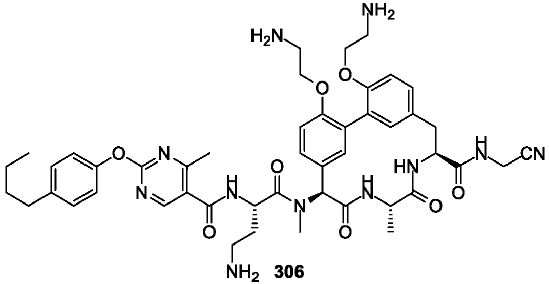

Compound 306 (formic acid salt) was prepared as a white solid utilizing the methods in Example 208 (Compound 304). LCMS (Method 5-95 AB, ESI): t_{R} = 0.718 min, [M+H]⁺ = 906.7.

### Example 211: Synthesis of Compound 307

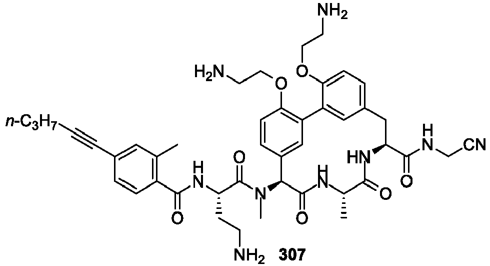

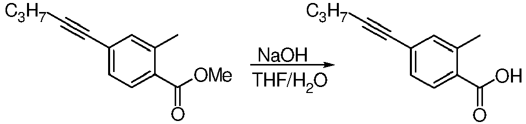

The preparation of alkynyl aromatic compounds is accomplished by Sonogashiro coupling and base hydrolysis. Methyl 2-methyl-4-(pent-1-yn-1-yl)benzoate is an intermediate in the preparation of Compound 197 (Example 103).
Ester hydrolysis with NaOH according to Example 47 afforded 2-methyl-4-(pent-1-yn-1-yl)benzoic acid.

Compound 307 (formic acid salt) was prepared as a white solid utilizing the methods in Example 7 (Compound 101). LCMS (5-95 AB, ESI): t_{R} = 0.686, [M + H]⁺ = 822.4.

### Example 212: Synthesis of Compound 308

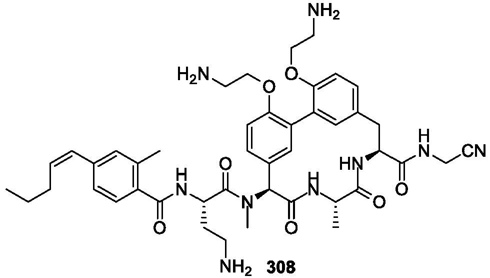

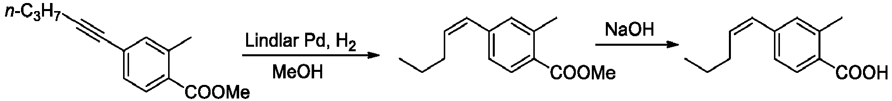

Step 1: To a solution of methyl 2-methyl-4-(pent-1-yn-1-yl)benzoate (Example 103) (300 mg, 1.4 mmol) in MeOH (50 mL) was added Lindlar Pd catalyst (30 mg, 0.46 mmol) and the mixture was stirred at room temperature under H₂ (15 psi) for 1 h. The filtrate was concentrated to afford (Z)-methyl 2-methyl-4-(pent-1-en-1-yl)benzoate (200 mg, 65% yield) as colorless oil. LCMS (5-95 AB, 2 min, ESI): t_{R} = 1.464, [M + H]⁺ = 219.2.

Step 2: Ester hydrolysis procedure with NaOH (Example 47) was applied to (Z)-methyl 2-methyl-4-(pent-1-en-1-yl)benzoate (100 mg, 0.45 mmol) to afford (Z)-2-methyl-4-(pent-1-en-1-yl)benzoic acid (80 mg, 85.4% yield) as a white solid.

Compound 308 (formic acid salt) was prepared as a white solid utilizing the methods in Example 7 (Compound 101). LCMS (5-95 AB, ESI): t_{R} = 0.573, [M + H]⁺ = 824.4; ¹H NMR (400 MHz, MeOH-*d*₄) δ 8.49 (brs, 2H, HCOOH), 7.39 (d, J=8.0 Hz, 1H), 7.32 (d, J=8.0 Hz, 1H), 7.24 (d, J=8.0 Hz, 1H), 7.18 (brs, 1H), 7.16 (d, J=8.0 Hz, 2H), 7.09 (d, J=8.0 Hz, 1H), 6.90 (brs, 1H), 6.82 (brs, 1H), 6.43 (d, 7=12.0 Hz, 1H), 6.33 (s, 1H), 5.80-5.70 (m, 1H), 5.12-5.16 (m, 1H), 4.82-4.78 (m, 2H), 4.36-4.16 (m, 4H), 4.19 (s, 2H), 3.37-3.10 (m, 8H), 2.93 (s, 3H), 2.43 (s, 3H), 2.33-2.15 (m, 3H), 2.12-2.00 (m, 1H), 1.52-1.46 (m, 2H), 1.36 (d, J=6.8 Hz, 3H), 0.94 (t, *J*=7.2 Hz, 3H).

### Example 213: Synthesis of Compound 309

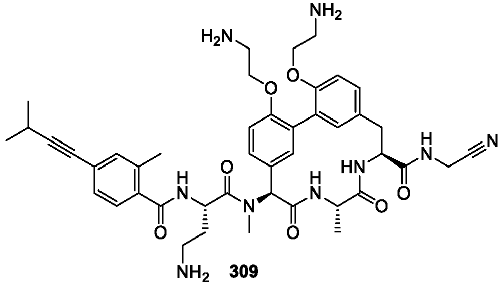

Compound 309 (formic acid salt) was prepared as a white solid utilizing the methods in Example 211 (Compound 307). LCMS (Method 5-95 AB, ESI): t_{R} = 0.567 min, [M+H]⁺ = 822.7.

### Example 214: Synthesis of Compound 310

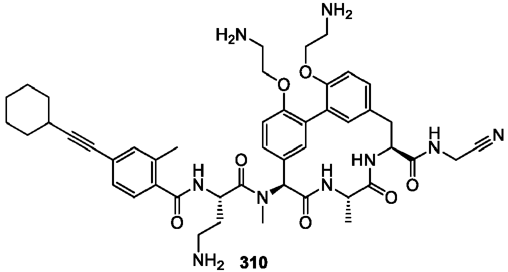

Compound 310 (formic acid salt) was prepared as a white solid utilizing the methods in Example 211 (Compound 307). LCMS (Method 5-95 AB, ESI): t_{R} = 0.743 min, [M+H]⁺ = 862.5.

### Example 215: Synthesis of Compound 311

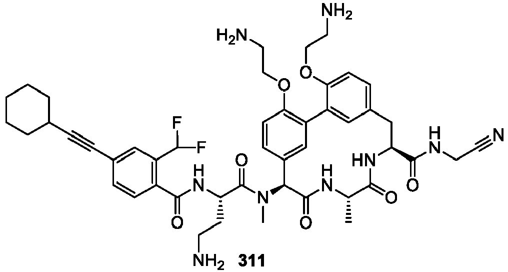

Compound 311 (formic acid salt) was prepared as a white solid utilizing the methods in Example 211 (Compound 307). LCMS (Method 5-95 AB, ESI): t_{R} = 0.743 min, [M+H]⁺ = 898.7.

### Example 216: Synthesis of Compound 312

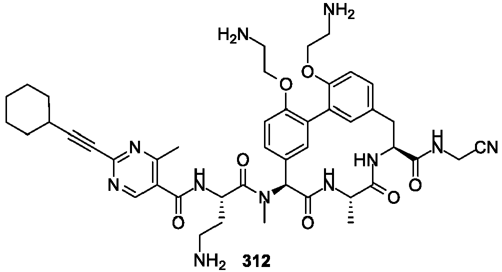

Compound 312 (formic acid salt) was prepared as a white solid utilizing the methods in Example 211 (Compound 307). LCMS (Method 5-95 AB, ESI): RT = 0.662 min, [M+Na]⁺ = 887.7.

### Example 217: Synthesis of Compound 313

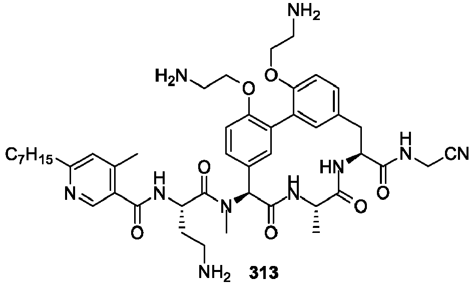

Compound 313 (formic acid salt) was prepared as a white solid utilizing the methods in Example 133 (Compound 227). LCMS (Method 5-95 AB, ESI): RT = 0.611 min, [M+Na]⁺ = 878.4.

### Example 218: Synthesis of Compound 314

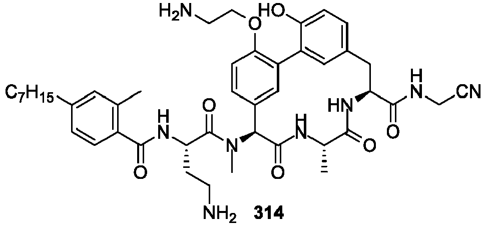

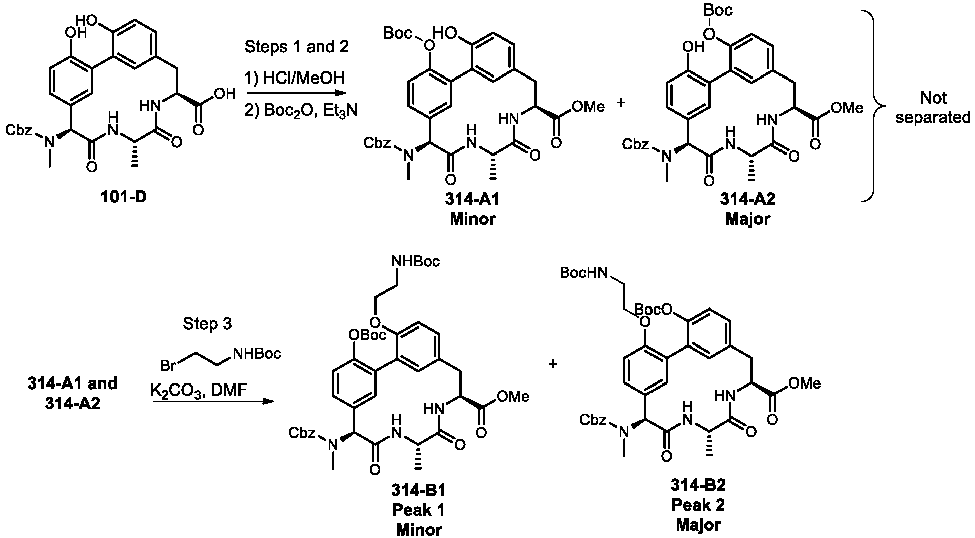

Step 1: Compound 101-D (2.0 g, 3.65 mmol) was added to a solution of 1.25N HCl/MeOH (150 mL) and the mixture was stirred at 0°C for 4 h. The volatiles were removed to give the crude as a white solid.

Step 2: The above crude was dissolved in DCM (5 mL) and the mixture was added Boc₂O (0.93 g, 4.27 mmol) and TEA (1.08 g, 10.7 mmol). The resulting mixture was stirred for at room temperature for 16 h. The volatiles were removed and the residue was purified by silica gel flash column to obtain Compound 314-A1 and 314-A2 as a mixture of regioisomers (1.8 g, 76.4% yield) as a white solid. LCMS (5-95 AB, ESI): t_{R} = 0.880, [M + H]⁺ = 684.6.
To a mixture of Compound 314-A1 and 314-A2 (1.8 g, 2.72 mmol) and t-butyl (2-bromoethyl)carbamate (3.0 g, 13.6 mmol) in DMF (5 mL) was added K₂CO₃ (3.8 g, 27.2 mmol) and the reaction mixture was stirred at room temperature for 3 h. The reaction mixture was added with DCM (50 mL), which was washed with 2N HCl, saturated NaHCO₃ and brine (20 mL each). The organic layer was then dried over Na₂SO₄, concentrated and the residue was purified on silica gel flash column to afford the mixture of regioisomers, which was further purified by SFC (OD, 250mm × 30mm, 5um) to afford Compound 314-B1(80 mg, 3.6% yield) and Compound 314-B2 (1.6 g, 73.2% yield) as a white solid.

Compound 314 (free base) was prepared utilizing methods similar to those from Example 5 and Example 7 from Compound 314-B2. LCMS (5-95 AB, ESI): t_{R} = 0.782, [M + H]⁺ = 811.5; ¹H NMR (400 MHz, MeOH-*d*₄) δ 7.28-7.15 (m, 2H), 7.10 (d, J=8.0 Hz, 1H), 7.09-7.01 (m, 3H), 6.92 (brs, 1H), 6.83 (d, J=8.0 Hz, 1H), 6.61 (brs, 1H), 6.55 (s, 1H), 5.12-5.09 (m, 1H), 4.81-4.78 (m, 1H), 4.68-4.66 (m, 1H), 4.19 (s, 2H), 4.18-4.10 (m, 2H), 3.24-3.20 (m, 1H), 3.14-3.06 (m, 1H), 3.02-2.96 (m, 2H), 2.98 (s, 3H), 2.87-2.80 (m, 2H), 2.53-2.48 (m, 1H), 2.33 (s, 3H), 2.10-1.90 (m, 1H), 1.57-1.54 (m, 2H), 1.36-1.29 (m, 12H), 0.91 (t, J=7.2 Hz, 3H).

### Example 219: Synthesis of Compound 315

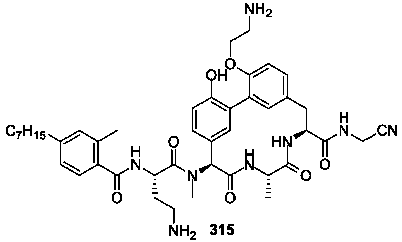

Compound 315 (free base) was prepared utilizing methods similar to those from Example 5 and Example 7 from Compound 314-B1. LCMS (5-95 AB, ESI): t_{R} = 0.788, [M + H]⁺ = 811.4.

### Example 220: Synthesis of Compound 316

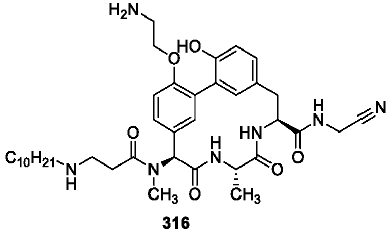

Compound 316 (formic acid salt) was prepared as a white solid utilizing the methods in Example 37 (Compound 131) from Compound 314-B2. LCMS (5-95 AB, ESI): t_{R} = 0.794, [M + H]⁺ = 706.3; ¹H NMR (400 MHz, MeOH-*d*₄) δ 8.53 (brs, 1H), 7.28 (d, J=8.0 Hz, 1H), 7.15-7.11 (m, 2H), 6.94 (brs, 1H), 6.90 (d, J=8.0 Hz, 1H), 6.78 (brs, 1H), 6.36 (s, 1H), 4.83-4.87 (m, 3H), 4.32-4.36 (m, 2H), 4.31 (s, 2H), 3.33-3.26 (m, 2H), 3.16-3.05 (m, 4H), 2.93 (brs, 2H), 2.76 (s, 3H), 1.66-1.80 (m, 2H), 1.35-1.42 (m, 19H), 0.92 (t, J=6.4 Hz, 3H).

### Example 221: Synthesis of Compound 317

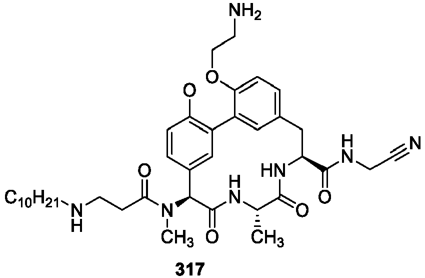

Compound 317 (formic acid salt) was prepared as a white solid utilizing the methods in Example 37 (Compound 131) from Compound 314-B1. LCMS (5-95 AB, ESI): t_{R} = 0.640, [M + H]⁺ = 706.3

### Example 222: Synthesis of Compound 318

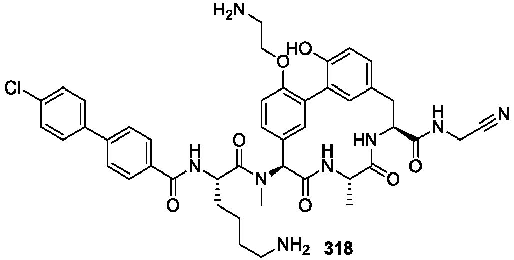

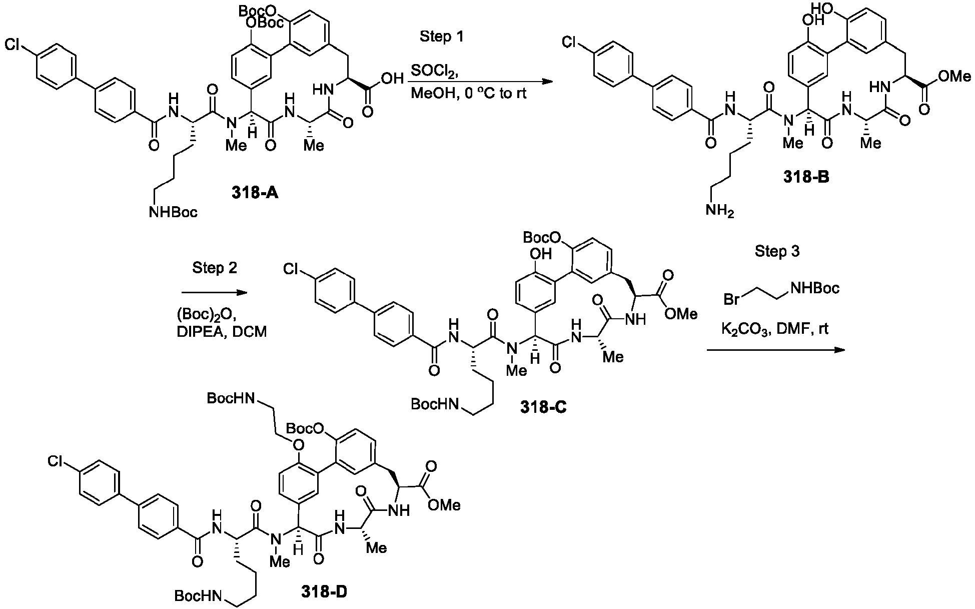

Step 1: To a stirred solution of Compound 318-A (211 mg, 0.2 mmol) in dry MeOH (5mL) at 0°C, was slowly added SOCl₂ (0.2 mL) dropwise. The reaction mixture was stirred at rt overnight. The solvent was removed under reduced pressure and the residue was dried under high vacuum and used directly in the next reaction to afford Compound 318-B. LCMS: MS (ESI) for C₄₁H₄₄ClN₅O₈: m/z 770.1 (M + H)⁺.

Step 2: To Compound 318-B (0.3 mmol) was dissolved in CH₂Cl₂ (8 mL) and Et₃N (140 µL, 1.0 mmol, 5eq), to this stirred solution was added (Boc)₂O (106 µL, 0.46 mmol, 2.3 eq). The reaction mixture was stirred at rt overnight. After the reaction was complete, brine solution was added and the mixture was extracted with ethyl acetate. The combined organic layers washed with brine, dried over anhydrous Na₂SO₄, filtered and solvent was removed under reduced pressure. The residue was purified by flash chromatography (DCM - 5% DCM-MeOH) to afford 152 mg (78%, over 2 steps) of Compound 318-C as a white solid. MS (ESI) for (C₅₁H₆₀ClN₅O₁₂): m/z 970.2 (M + H)⁺.

Step 3: To a stirred solution of Compound 318-C (145 mg, 0.15 mmol) in dry DMF (5 mL) was added K₂CO₃ (42 mg, 0.3 mmol, 2 eq) followed by tert-butyl (2-bromoethyl)carbamate (100 mg, 0.45 mmol). The reaction mixture was stirred at rt overnight. After completion of the reaction, crushed ice was added and the resultant white cloudy mixture was extracted with EtOAc. The combined organic layers were washed with brine and dried over anhydrous Na₂SO₄, filtered and the solvent was removed under vacuum. The residue was purified by flash chromatography (DCM - 5% DCM-MeOH) to afford 118 mg (71%) of the Compound 318-D as a white solid. MS (ESI) for (C₅₈H₇₃ClN₆O₁₄): m/z 1113.4 (M + H)⁺.

Compound 318 (formic acid salt) was prepared as a white solid utilizing the methods in Example 235 (Compound 331) from Compound 318-D. LCMS (ESI): [M + H]⁺ = 837.4.

### Example 223: Synthesis of Compound 319

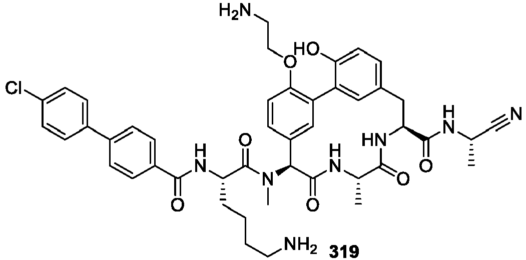

(S)-2-Aminopropanenitrile was prepared in a manner similar to (R)-2-aminopropanenitrile (Example 42 except that (S)-2-((tert-butoxycarbonyl)amino)propanoic acid is used as the starting material.

Compound 319 (formic acid salt) was prepared as a white solid utilizing the methods in Example 235 (Compound 331) from (S)-2-aminopropanenitrile. LCMS (ESI): [M + H]⁺ = 851.4.

### Example 224: Synthesis of Compound 320

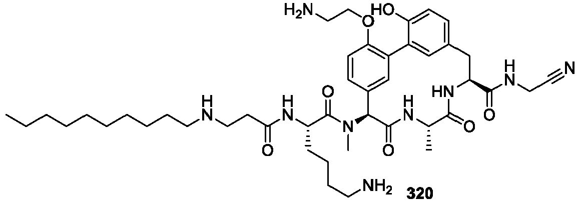

Compound 320 (formic acid salt) was prepared as a white solid utilizing the methods in Example 222. LCMS (ESI): [M + H]⁺ = 834.6.

### Example 225: Synthesis of Compound 321

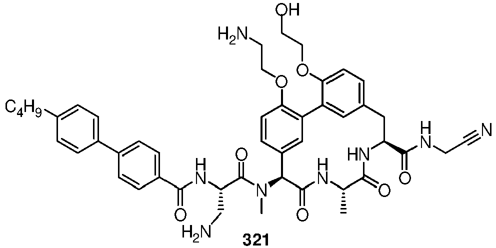

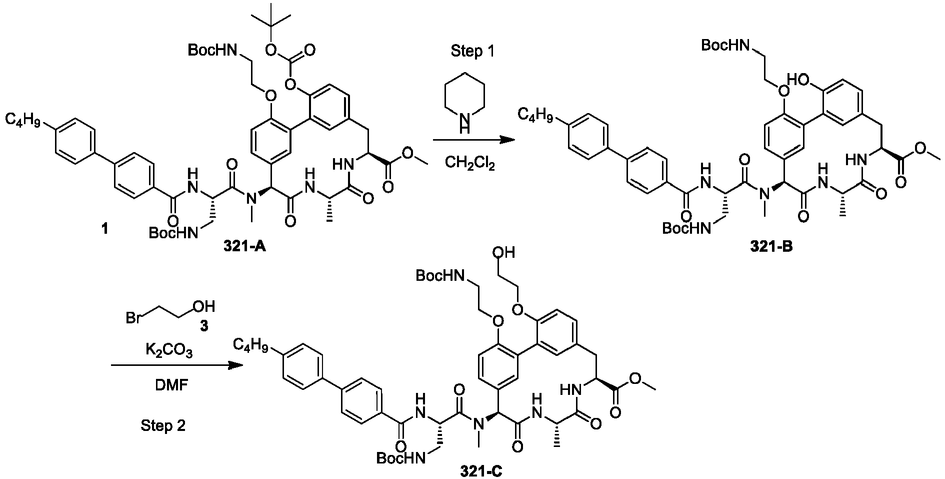

Compound 321-A was prepared utilizing the methods in Example 218.

A solution of 321-A (410.0 mg, 0.38 mmol) and piperidine (1 mL, 11.74 mmol) in dichloromethane (4 mL) was stirred at 25 °C for 18 h. The reaction was diluted with DCM (50 mL) and acidified by 10% aq. citric acid. The organic layer was washed with water (20 mL x 2) and brine (20 mL), dried over anhydrous sodium sulfate, and concentrated in vacuo to give 321-B (300 mg, 0.3021 mmol, 80.5% yield) as a white solid. LCMS (Method 5-95 AB, ESI): t_{R} = 1.062 min, [M+H]⁺ = 993.6.

A mixture of 321-B (50.0 mg, 0.05 mmol), 2-bromoethanol (188.7 mg, 1.51 mmol) and potassium carbonate (208.7 mg, 1.51 mmol) in N,N-dimethylformamide (1.5 mL) was stirred at 25 °C for 48 h. The mixture was poured into ice-water (10 mL) and extracted with EtOAc (20 mL x 2). The combined organic layers were washed with water (15 mL x 2) and brine (20 mL), dried over MgSO₄ and concentrated. The residue was purified by prep-TLC (10% methanol in DCM, Rf = 0.3) to afford 321-C (30 mg, 57.5% yield) as a white solid. LCMS (Method 5-95 AB, ESI): t_{R} = 1.055 min, [M+H]⁺ = 1038.2.

Compound 321 (formic acid salt) was prepared as a white solid utilizing methods similar to those from Example 7 using Compound 321-C. LCMS (Method 5-95 AB, ESI): t_{R} = 0.787 min, [M+H]⁺ = 861.7.

### Example 226: Synthesis of Compound 322

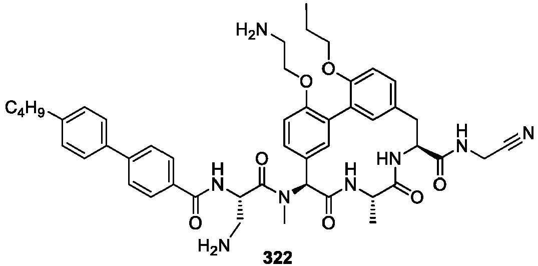

Compound 322 (formic acid salt) was prepared as a white solid utilizing the methods in Example 225 (Compound 321). LCMS (Method 5-95 AB, ESI): t_{R} = 0.804 min, [M+H]⁺ = 859.8.

### Example 227: Synthesis of Compound 323

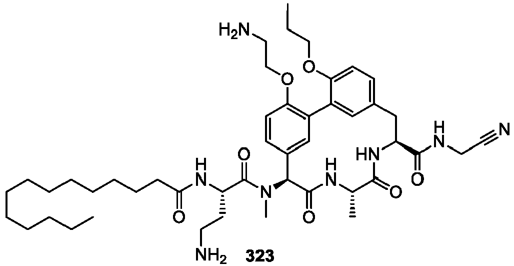

Compound 323 (formic acid salt) was prepared as a white solid utilizing the methods in Example 225 (Compound 321). LCMS (Method 5-95 AB, ESI): t_{R} = 0.823 min, [M+H]⁺ = 847.5.

### Example 228: Synthesis of Compound 324

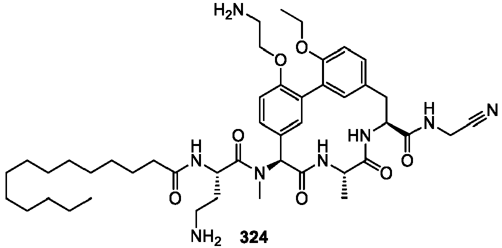

Compound 324 (formic acid salt) was prepared as a white solid utilizing the methods in Example 225 (Compound 321). LCMS (Method 5-95 AB, ESI): t_{R} = 0.823 min, [M + H]⁺ = 834.7.

### Example 229: Synthesis of Compound 325

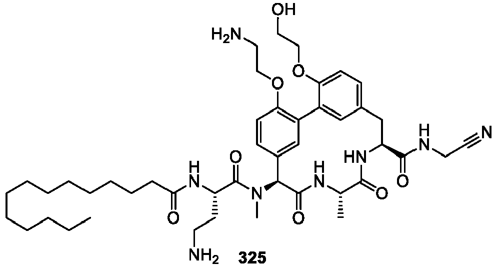

Compound 325 (formic acid salt) was prepared as a white solid utilizing the methods in Example 225 (Compound 321). LCMS (Method 5-95 AB, ESI): t_{R} = 0.816 min, [M + H]⁺ = 849.4.

### Example 230: Synthesis of Compound 326

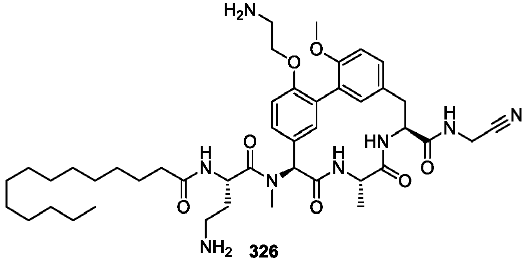

Compound 326 (formic acid salt) was prepared as a white solid utilizing the methods in Example 225 (Compound 321). LCMS (Method 5-95 AB, ESI): t_{R} = 0.692 min, [M + H]⁺ = 819.5.

### Example 231: Synthesis of Compound 327 and Compound 328

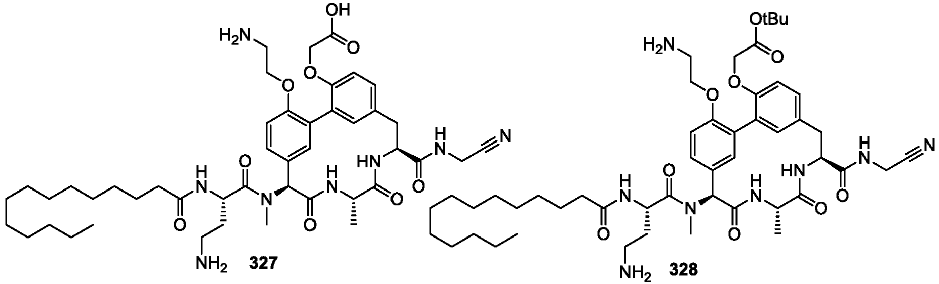

Compound 327 (formic acid salt) was prepared as a white solid utilizing the methods in Example 225 (Compound 321). LCMS (Method 5-95 AB, ESI): t_{R} = 0.822 min, [M + H]⁺ = 863.4.

Compound 328 (formic acid salt) was isolated as a white solid as a result of incomplete deprotection of the Boc-intermediates during the preparation of Compound 327. LCMS (Method 5-95 AB, ESI): t_{R} = 0.717 min, [M+H]⁺ = 919.4.

### Example 232: Synthesis of Compound 329

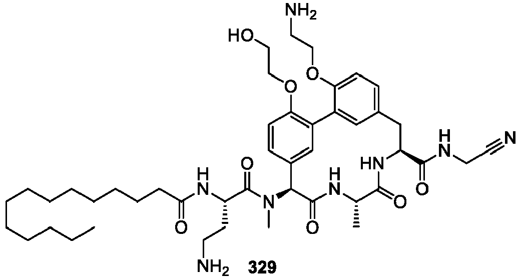

Compound 329 (formic acid salt) was prepared as a white solid utilizing the methods in Example 225 (Compound 321) except that the first alkylation of the phenol is performed with (2-bromoethoxy)(tert-butyl)dimethylsilane and the second alkylation is performed with tert-butyl (2-bromoethyl)carbamate. LCMS (Method 5-95 AB, ESI): t_{R} = 0.825 min, [M + H]⁺ = 849.9.

### Example 233: Synthesis of Compound 330

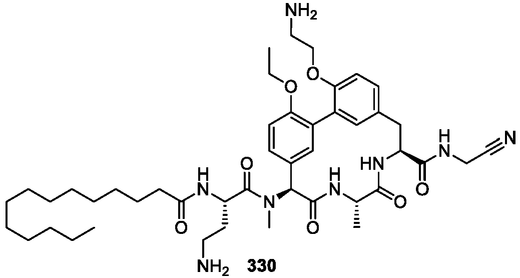

Compound 330 (formic acid salt) was prepared as a white solid utilizing the methods in Example 225 (Compound 321) except that the first alkylation of the phenol is performed with bromoethane and the second alkylation is performed with tert-butyl (2-bromoethyl)carbamate. LCMS (Method 5-95 AB, ESI): t_{R} = 0.839 minI 1.5 min, [M + H]⁺ = 833.6.

### Example 234: Synthesis of Compound 331-G

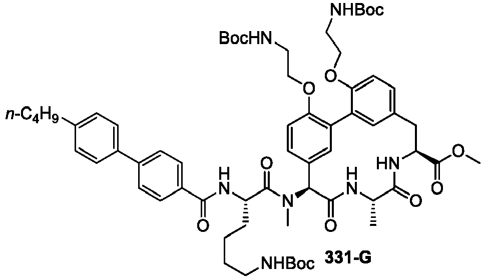

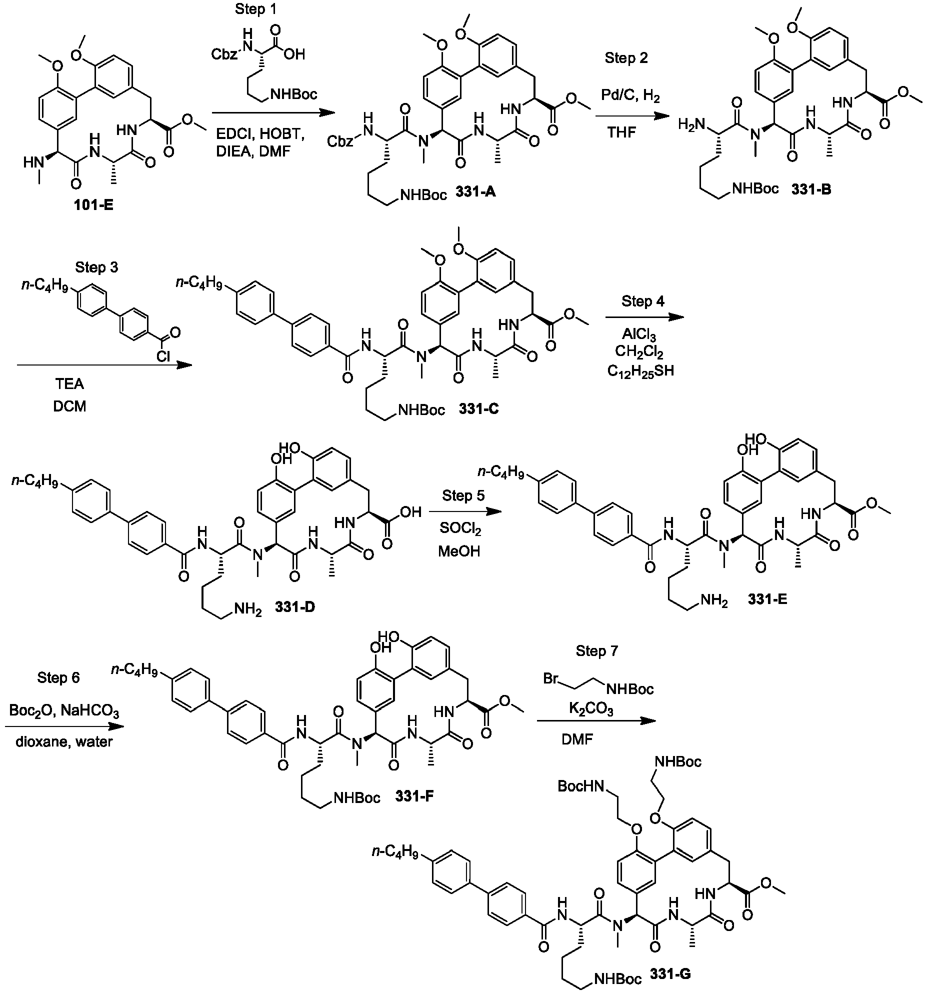

Step 1: To a solution of Compound 101-E (800 mg, 1.76 mmol) in DMF (15 mL) was added (S)-2-(((benzyloxy)carbonyl)amino)-6-((tert-butoxycarbonyl)amino)hexanoic acid (735 mg, 1.93 mmol), 3-[(E)-ethylazo]-N,N-dimethyl-propan-1-amine hydrochloride (946.77 mg, 5.27 mmol), 1-hydroxybenzotriazole (711.94 mg, 5.27 mmol), and N,N-diisopropylethylamine (681 mg, 5.27 mmol). The mixture was stirred at 30 °C for 16 h. TLC showed the start material was consumed (50% ethyl acetate in petroleum ether, Rf = 0.5). The mixture was poured into water (30 mL). The precipitate was filtered, washed with water, re-dissolved in methanol, and concentrated to give Compound 331-A (1200 mg, 1.45 mmol, 83.5% yield) as a yellow solid.

Step 2: To a solution of Compound 331-A (1200 mg, 1.47 mmol) in methanol (15 mL) was added Pd/C (200.0 mg, 1.47 mmol), and the mixture was stirred at 30 °C under hydrogen (50 psi) for 16 h. The catalyst was filtered off and the filtrate was concentrated to give Compound 331-B (900 mg, 1.12 mmol, 81.6% yield) as a white solid. LCMS (5-95AB_1.5min_1500): t_{R} = 0.782 min, [M + H]⁺ 684.4.

Step 3: The coupling of an acid chloride to an amine is General Method Acid Chloride and is described for this example. A mixture of 4-(4-butylphenyl)benzoic acid (200 mg, 0.79 mmol) in thionyl chloride (5.0 mL) was stirred at 60 °C for 16 h. The solution was concentrated and dissolved in dichloromethane (2 mL). To the solution of Compound 331-B (500 mg, 0.73 mmol) and triethylamine (74 mg, 0.73 mmol) in dichloromethane (15 mL) was added the above solution of 4-(4-butylphenyl)benzoyl chloride in dichloromethane. The reaction mixture was stirred at 25 °C for 3 h. LCMS showed that all of start material was consumed completely. TLC (10% dichloromethane in methanol, Rf = 0.4). The reaction was concentrated to dryness and the residue was purified by flash column chromatography (eluted with 5% dichloromethane in methanol). The desired fractions were concentrated to afford Compound 331-C (650 mg, 0.71 mmol, 96.6% yield) as a white solid. LCMS (5-95AB/1.5 min): t_{R} = 0.951 min, [M + H]⁺ 921.4. Alternatively, this coupling reaction can be performed using 4'-butyl-[1,1'-biphenyl]-4-carboxylic acid using General Method HATU conditions in Example 4.

Step 4: A mixture of aluminium chloride (2.8 g, 21.19 mmol) and 1-dodecanethiol (4.3 g, 21.19 mmol) in dichloromethane (12 mL) was stirred at 26 °C for 5 min, and then cooled to 0 °C. Then Compound 331-C (650 mg, 0.71 mmol) was added slowly. The solution was stirred at 26 °C for 2 h. LCMS showed that all of start material was consumed completely. The solution was quenched by 1N hydrochloride acid, and filtered. The filter cake was dried to afford crude Compound 331-D as a white solid. LCMS (5-95AB/1.5 min): t_{R} = 0.828 min, [M + H]⁺ =778.4.

Step 5: A solution of Compound 331-D (500 mg, 0.64 mmol) and thionyl chloride (229 mg, 1.93 mmol) in methanol (10 mL) was stirred at 60 °C for 1 h. LCMS showed that all of start material was consumed completely. The solution was concentrated to afford Compound 331-E (500 mg, 0.63 mmol, 98.2% yield) as a yellow solid. LCMS (5-95AB/1.5 min): t_{R} = 0.856 min, [M + H]⁺ = 792.8.

Step 6: To the solution of Compound 331-E (500 mg, 0.63 mmol) and sodium bicarbonate (10.6 mg, 0.13 mmol) in 1,4-dioxane (6 mL) and water (2 mL) was added di-tert-butyl dicarbonate (138 mg, 0.63 mmol). LCMS showed that all of start material was consumed completely. TLC (5% dichloromethane in methanol, Rf = 0.2). The reaction was concentrated to dryness and the residue was taken up in ethyl acetate (50 mL). It was washed with water (20 mL x 2) and brine (10 mL), dried (sodium sulfate) and concentrated. The crude was purified by flash column chromatography (eluted with 5% dichloromethane in methanol). The desired fractions were concentrated in vacuo afford Compound 331-F (500 mg, 0.56 mmol, 88.8% yield) as a white solid. LCMS (5-95AB/1.5 min): t_{R} = 1.048 min, [M + H]⁺ = 892.4.

Step 7: A mixture of Compound 331-F (500 mg, 0.56 mmol), tert-butyl 2-bromoethylcarbamate (1.25 g, 5.61 mmol) and potassium carbonate (2.32 g, 16.82 mmol) in N,N-dimethylformamide (20 mL) was stirred at 26 °C for 96 h. LCMS showed that all of start material was consumed completely. The reaction was quenched with ice-water (5 mL), and the mixture was taken up in ethyl acetate (20 mL). The organic layer was washed with water (20 mL x 2) and brine (10 mL), dried (sodium sulfate) and concentrated. The crude was purified by flash column chromatography (eluted with ethyl acetate). The desired fractions were concentrated to afford Compound 331-G (450 mg, 0.38 mmol, 68.1% yield) as a colorless oil. LCMS (5-95AB/1.5min): t_{R} = 0.995 min, [M + H]⁺ 1179.0.

### Example 235: Synthesis of Compound 331

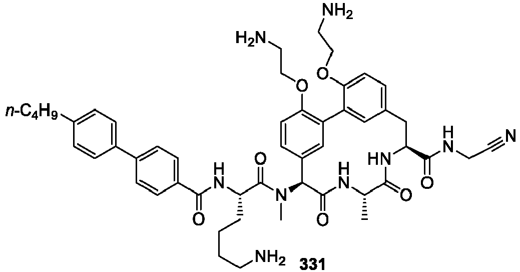

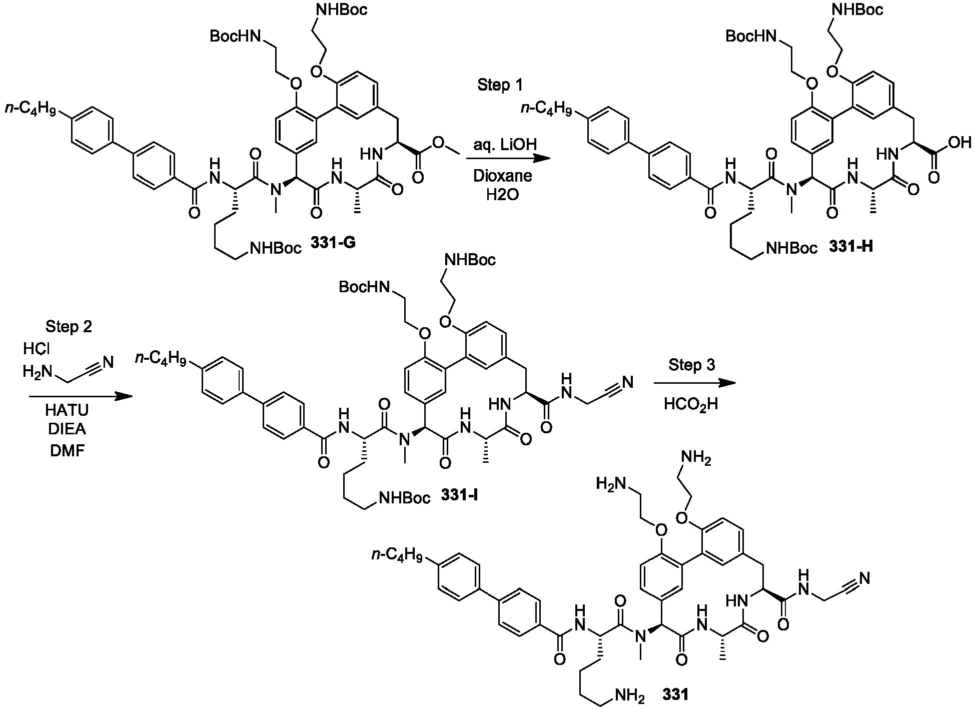

Step 1: A mixture of Compound 331-G (80 mg, 0.07 mmol) and aqueous lithium hydroxide hydrate (0.41 mL, 0.2 mmol, 0.5 M) in 1, 4-dioxane (2 mL) and water (1 mL) was stirred at 26 °C for 2 h. The solution was quenched with 5% aqueous potassium bisulfate solution to pH = 6, and the mixture was taken up in ethyl acetate (20 mL). The organics were washed with water (20 mL x 2) and brine (10 mL), dried (sodium sulfate) and concentrated to afford Compound 331-H (50 mg, 0.043 mmol, 63.2% yield) as a white solid. LCMS (5-95AB/1.5min): t_{R} = 1.103 min, [M + H]⁺ 1164.9.

Step 2: Starting from Compound 331-H (50.0 mg, 0.04 mmol) and 2-aminoacetonitrile hydrochloride (39.7 mg, 0.43 mmol), General Method HATU (Example 5) was used to prepare Compound 331-I (30 mg, 58.2% yield) as a white solid. LCMS (5-95AB_1.5min): t_{R} = 1.103 min, [M + H]⁺ 1204.1.

Step 3: The deprotection of an N-Boc group using neat formic acid is described and is referred to as General Method FA. A mixture of Compound 331-I (30.0 mg, 0.025 mmol) in formic acid was stirred at 15 °C for 1 hour, then the mixture was lyophilized to a solid. Preparative HPLC (acetonitrile/water with 0.22% formic acid) afforded Compound 331 (3.7 mg, 16 %) as a white solid. ¹H NMR (400 MHz, CD₃OD): δ 8.49 (s, 3H), 7.94 (d, J = 8.4 Hz, 2H), 7.74 (d, J = 8.0 Hz, 2H), 7.57 (d, J = 7.6 Hz, 2H), 7.32-7.11 (m, 5H), 6.90-6.82 (m, 2H), 6.45 (s, 1H), 5.06-4.95 (m, 1H), 4.80-4.79 (m, 2H), 4.29-4.15 (m, 5H), 3.18-3.10 (m, 5H), 2.99-2.85 (m, 5H), 2.71-2.64 (m, 3H), 2.01-1.92 (m, 2H), 1.82-1.45 (m, 6H), 1.43-1.38 (m, 6H), 0.97 (t, J = 7.2 Hz, 3H). LCMS (Method 5-95 AB, ESI): t_{R} = 0.753 min, [M + H]⁺ = 903.1.

### Example 236: Synthesis of Compound 332

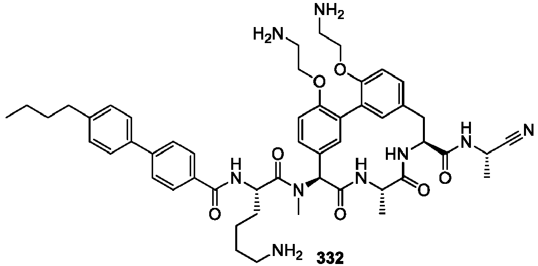

Compound 332 (formic acid salt) was prepared as a white solid utilizing the methods from Example 235 (Compound 331), from Compound 331-H and (S)-2-aminopropanenitrile formic acid salt. LCMS (Method 5-95 AB, ESI): t_{R} = 0.770 min, [M + H]⁺ = 916.7.

### Example 237: Synthesis of Compound 333

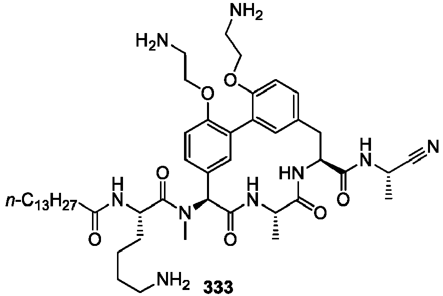

Compound 333 (formic acid salt) was prepared as a white solid utilizing the methods from Example 236 (Compound 332) using tetradecanoic acid. LCMS (Method 10-80AB): t_{R} = 2.785 min/ 7 min, [M + H]⁺ = 891.8.

### Example 238: Synthesis of Compound 334

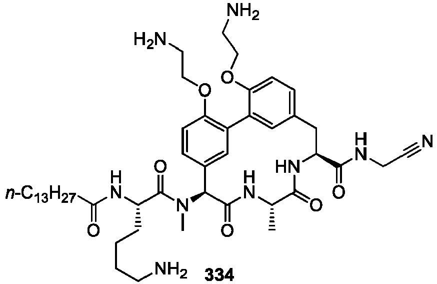

Compound 334 (formic acid salt) was prepared as a white solid utilizing the methods from the preparation of Compound 331 (Example 235). LCMS (Method 5-95 AB, ESI): t_{R} = 0.769 min, [M + H]⁺ = 876.5.

### Example 239: Synthesis of Compound 335

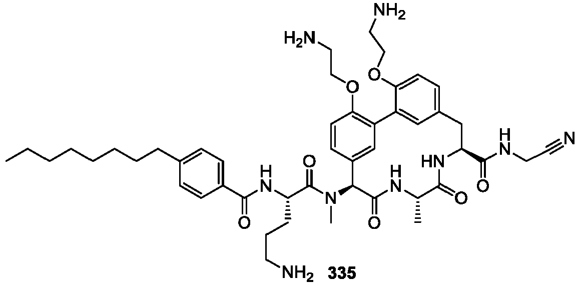

Compound 335 (formic acid salt) was prepared as a white solid utilizing the methods in Example 235 (Compound 331). LCMS (Method 5-95 AB, ESI): t_{R} = 0.753 min, [M + H]⁺ = 868.6.

### Example 240: Synthesis of Compound 336

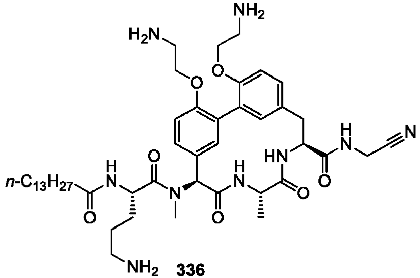

Compound 336 (formic acid salt) was prepared as a white solid utilizing the methods in Example 235 (Compound 331). LCMS (Method 5-95 AB, ESI): t_{R} = 0.783 min, [M + H]⁺ = 862.9.

### Example 241: Synthesis of Compound 337

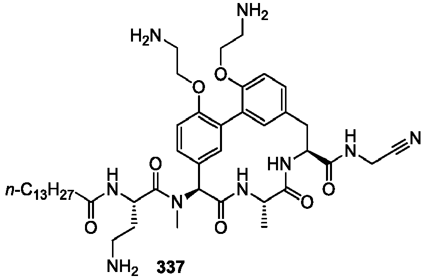

Compound 337 (formic acid salt) was prepared in as a white solid utilizing the methods in Example 235 (Compound 331). LCMS (Method 5-95 AB, ESI): t_{R} = 0.771 min, [M + H]⁺ = 848.7.

### Example 242: Synthesis of Compound 338

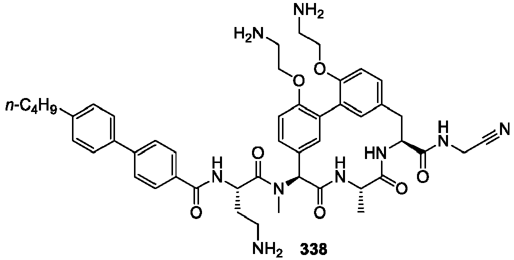

Compound 338 (formic acid salt) was prepared as a white solid utilizing the methods in Example 235 (Compound 331). LCMS (Method 5-95 AB, ESI): t_{R} = 0.746 min, [M + H]⁺ = 874.7.

### Example 243: Synthesis of Compound 339

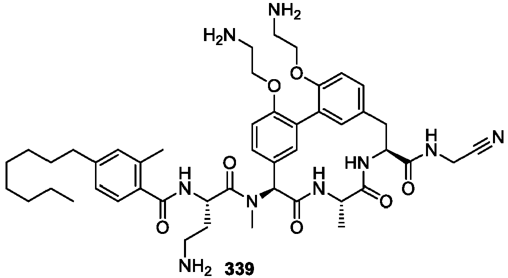

Compound 339 (formic acid salt) was prepared as a white solid utilizing the methods in Example 235 (Compound 331) from 2-methyl-4-octylbenzoic acid. LCMS (Method 5-95 AB, ESI): t_{R} = 0.778 min, [M+Na]⁺ = 890.5.

### Example 244: Synthesis of Compound 340

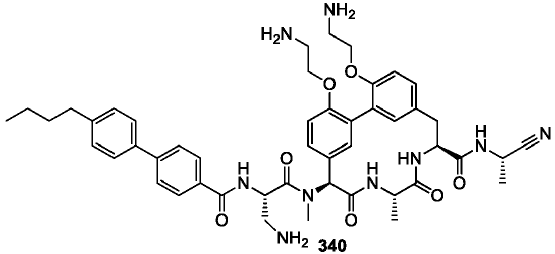

Compound 340 (formic acid salt) was prepared as a white solid utilizing the methods from the preparation of Example 236 (Compound 332) LCMS (Method 10-80AB): t_{R} = 0.885 min/ 2 min, [M + Na]⁺ = 896.3.

### Example 245: Synthesis of Compound 341

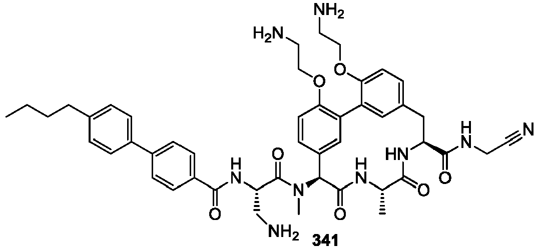

Compound 341 (formic acid salt) was prepared as a white solid utilizing the methods in Example 235 (Compound 331). LCMS (Method 5-95 AB, ESI): t_{R} = 0.733 min, [M + H]⁺ = 860.8.

### Example 246: Synthesis of Compound 342

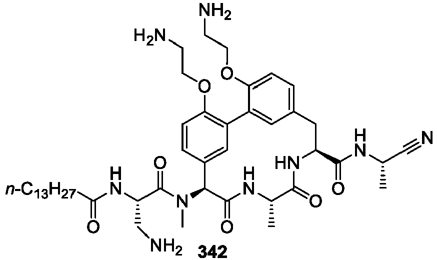

Compound 342 (formic acid salt) was prepared as a white solid utilizing the methods in Example 236 (Compound 332). LCMS (Method 5-95 AB, ESI): t_{R} = 0.813 min, [M + H]⁺ = 848.4.

### Example 247: Synthesis of Compound 343

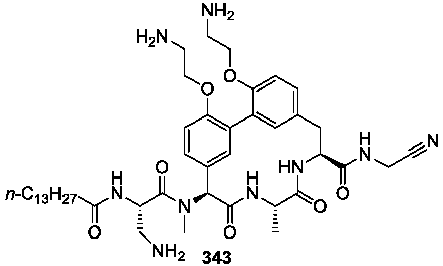

Compound 343 (formic acid salt) was prepared as a white solid utilizing the methods in Example 235 (Compound 331). LCMS (Method 5-95 AB, ESI): t_{R} = 0.802 min, [M + H]⁺ = 834.5.

### Example 248: Synthesis of Compound 344

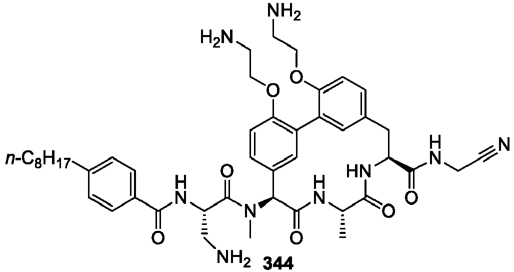

Compound 344 (formic acid salt) was prepared as a white solid utilizing the methods in Example 235 (Compound 331). LCMS (Method 5-95 AB, ESI): t_{R} = 0.754 min, [M + H]⁺ = 840.8.

### Example 249: Synthesis of Compound 345

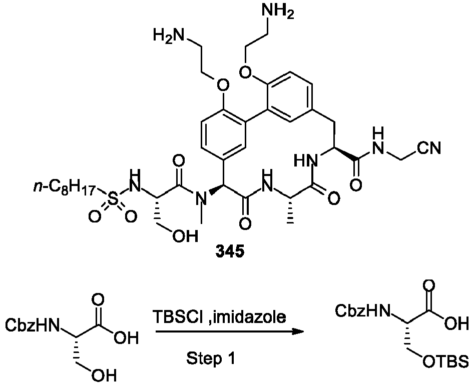

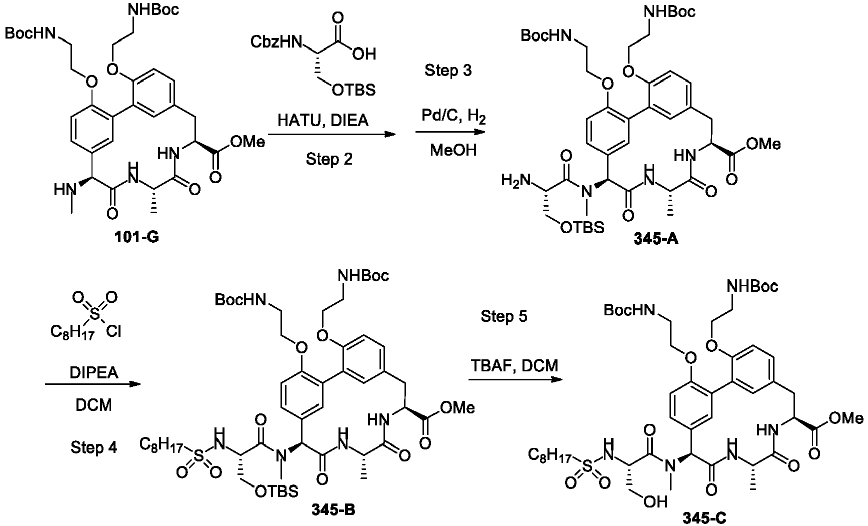

Step 1: To a mixture of (S)-2-(((benzyloxy)carbonyl)amino)-3-hydroxypropanoic acid (10.0 g, 42 mmol) and imidazole (5.7 g, 84 mmol) in DMF (500 mL), TBSCl (6.3 g, 42 mmol) was added at 0°C. The reaction mixture was warmed to room temperature slowly and stirred for another 16 hr. The volatiles were removed and the residue was taken up in EtOAc (1000 mL) and the EtOAc layer was washed sequentially with 1N HCl, saturated NaHCO₃ and brine (500 mL each). The EtOAc layer was dried over Na₂SO₄, concentrated and the resulting residue was purified by flash column chromatography to afford (S)-2-(((benzyloxy)carbonyl)amino)-3-((tertbutyldimethylsilyl)oxy)propanoic acid (7.0 g, 48% yield) as colorless oil.

Step 2: Typical HATU coupling condition was applied to Compound 101-G (Example 4) (100 mg, 0.14 mmol) and (S)-2-(((benzyloxy)carbonyl)amino)-3-((tertbutyldimethylsilyl)oxy)propanoic acid (99 mg, 0.28 mmol) to afford an off-white solid (120 mg, 82% yield).

Step 3: Typical hydrogenation condition (Pd/C, H₂) was applied to (120 mg, 0.11 mmol) to afford Compound 345-A (100 mg, 96% yield) as a white solid.

Step 4: To a solution of Compound 345-A (100 mg, 0.11 mmol) and octane-1-sulfonyl chloride compound (93 mg, 0.44 mmol) in DCM (5 mL) was added DIPEA (71 mg, 0.55 mmol) at 0°C. The resulting mixture was stirred at 0°C for 1 h. The volatiles were removed and the resulting residue was taken up in EtOAc (50 mL), which was washed with brine (50 mL x 2). The EtOAc layer was dried over MgSO₄, concentrated and the residue was purified by flash column chromatography to afford Compound 345-B (98 mg, 83% yield) as a white solid.

Step 5: To a solution of Compound 345-B (98 mg, 0.09 mmol) in DCM (5mL) was added TBAF hydrate (47 mg, 0.18 mmol). The resulting mixture was stirred for 1 h. The volatiles were removed and the residue was purified by flash column chromatography to afford Compound 345-C (80 mg, 92% yield) as a white solid. LCMS (5-95 AB, ESI): t_{R} = 0.857, [M + H]⁺ = 1000.3.

Compound 345 was prepared as a white solid utilizing the methods for Compound 101 (Example 7) from Compound 345-C. LCMS (5-95AB_30 min, ESI): t_{R} = 14.41, [M + H]⁺ = 801.3. ¹H NMR (400 MHz, MeOH-*d*4) δ 8.51 (brs, 2H, HCOOH), 7.31-7.26 (m, 2H), 7.19 (d, *J*=8.0 Hz, 1H), 7.12 (d, J=8.0 Hz, 1H), 6.91 (d, *J*=8.0 Hz, 1H), 6.83 (d, J=8.0 Hz, 1H), 6.45 (s, 0.6H), 6.23 (s, 0.4H), 4.80-4.33 (m, 2H), 4.28-4.20 (m, 4H), 4.21 (s, 2H), 3.86-3.80 (m, 1H), 3.78-3.70 (m, 1H), 3.30-3.00 (m, 6H), 2.94 (s, 3H), 2.75 (brs, 2H), 1.86-1.70 (m, 2H), 1.50-1.25 (m, 13H), 0.91 (t, 7=6.8 Hz, 3H).

### Example 250: Synthesis of Compound 346

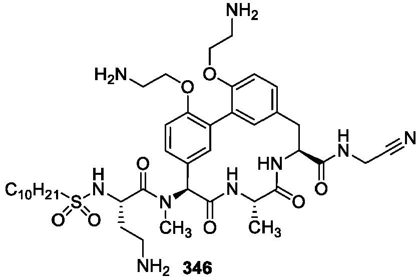

Compound 346 (formic acid salt) was prepared as a white solid utilizing the methods in Example 249 (Compound 345) from Compound 101-K (Example 5). LCMS (5-95 AB, ESI): t_{R} = 0.765, [M + H]⁺ = 842.6; ¹H NMR (400 MHz, MeOH-*d*₄) δ 8.49 (brs, 2H, HCOOH), 7.27-7.23 (m, 2H), 7.17 (d, J=8.0 Hz, 1H), 7.09 (d, J=8.0 Hz, 1H), 6.89 (brs, 1H), 6.82 (brs, 1H), 6.34 (s, 1H), 4.80-4.75 (m, 2H), 4.60-4.56 (m, 1H), 4.39-4.13 (m, 4H), 4.19 (s, 2H), 3.23-3.03 (m, 8H), 2.87 (s, 3H), 2.80-2.70 (m, 1H), 2.67-2.65 (m, 1H), 2.13-1.90 (m, 2H), 1.85-1.77 (m, 2H), 1.54-1.23 (m, 17H), 0.90 (t, *J*=6.0 Hz, 3H).

### Example 251: Synthesis of Compound 347

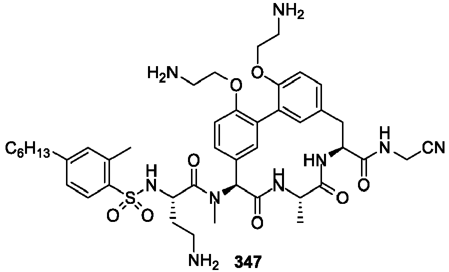

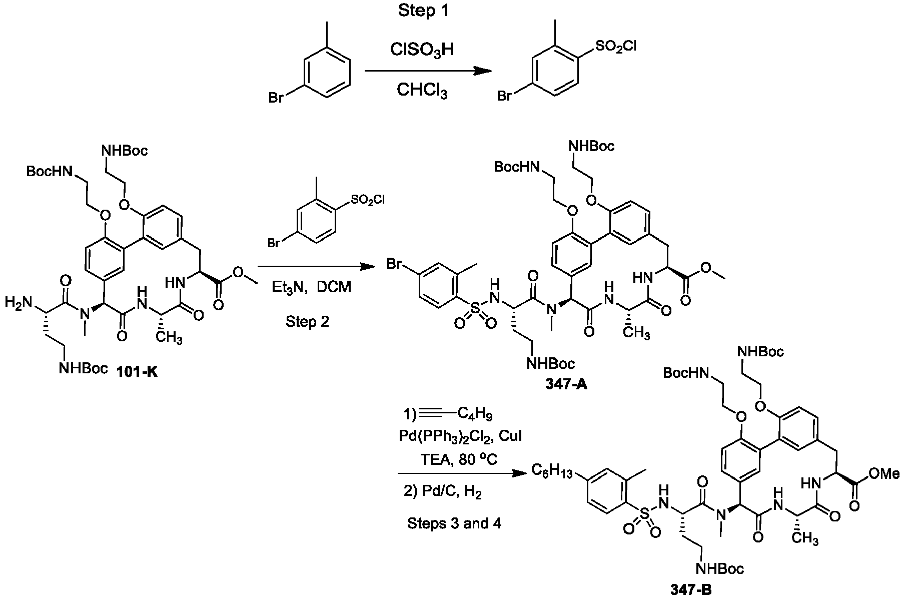

Step 1: To a solution of 1-bromo-3-methylbenzene (500 mg, 2.9 mmol) in CHCl₃ (10 mL) was added chlorosulfonic acid (1.2 mL) slowly at 0°C and the reaction was stirred at the same temperature for 4 h. The mixture was poured into crushed ice (50 mL), which was extracted by CHCl₃ (30 mL × 3). The combined organic layers were washed with brine (60 mL × 2), dried over Na₂SO₄ and concentrated to afford 4-bromo-2-methylbenzene-1-sulfonyl chloride (600 mg, 76% yield) as a white solid. ¹H NMR (400 MHz, CDCl₃) δ 7.91 (d, J=8.4 Hz, 1H), 7.58 (d, J=1.6 Hz, 1H), 7.55 (dd, 7=8.4, 1.6 Hz, 1H), 2.75 (s, 3H).

Step 2: The sulfonamide formation procedure from Example 249 (Compound 345) was applied to Compound 101-K (200 mg, 0.22 mmol) to afford Compound 347-A (160 mg, 63.7% yield) as a white solid.

Steps 3 and 4: Sonogashira coupling and reduction of Compound 347-A (General Method Sonogashiro) afforded Compound 347-B.

Compound 347 (formic acid salt) was prepared as a white solid utilizing the methods for Compound 101 (Example 7). LCMS (5-95 AB, ESI): t_{R} = 0.731, [M + H]⁺ = 876.6; ¹H NMR (400 MHz, MeOH-*d*₄) δ 8.47 (brs, 2H, HCOOH), 7.85 (d, J=8.0 Hz, 1H), 7.29 (brs, 1H), 7.25-7.16 (m, 2H), 7.14-7.04 (m, 2H), 6.83-6.74 (m, 3H), 6.00 (s, 1H), 4.76-4.66 (m, 2H), 4.36-4.30 (m, 1H), 4.29-4.15 (m, 4H), 4.18 (s, 2H), 3.27-2.96 (m, 8H), 2.76-2.72 (m, 2H), 2.67 (s, 3H), 2.61 (s, 3H), 2.13-1.85 (m, 2H), 1.75-1.62 (m, 2H), 1.46-1.24 (m, 9H), 0.90 (t, J=6.8 Hz, 3H).

### Example 252: Synthesis of Compound 348

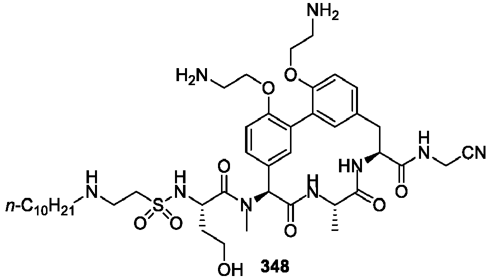

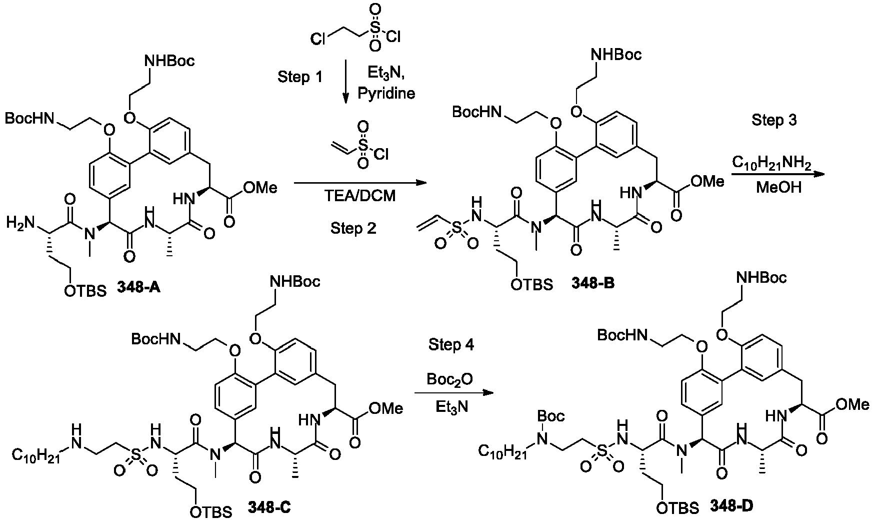

Step 1: To a solution of 2-chloroethanesulfonyl chloride (0.64 mL, 6.1 mmol) in DCM (10 mL) was added pyridine (0.97 g, 12.2 mmol) at -78°C and the resulting mixture was stirred at the same temperature for 20 min. The reaction was warmed to room temperature while stirring and stirred for another 20 min at the same temperature.

Step 2: To a solution of Compound 348-A (290 mg, 0.31 mmol) and Et₃N (474 mg, 4.7 mmol) in DCM (20 mL) was added the above solution at 0°C. The resulting mixture was warmed to room temperature and stirred for 1 h at the same temperature. DCM (50 mL) was then added and the mixture was washed with saturated citric acid, saturated NaHCO₃ and brine (50 mL each). The organic layer was dried over anhydrous Na₂SO₄, concentrated and the residue was purified by pre-TLC to afford 348-B (210 mg, 66% yield) as a yellow solid. LCMS (5-95 AB, ESI): RT = 1.056, M+Na⁺ = 1041.5.

Step 3: To a solution of 348-B (0.21 g, 0.206 mmol) in MeOH (2mL) was added 1-aminodecane (0.31 g, 2 mmol) at 0° C and the mixture was warmed and stirred at room temperature for 16 h. The volatiles were removed and the residue was purified by flash column chromatography to afford Compound 348-C (180 mg, zz % yield). LCMS (5-95 AB, ESI): t_{R} = 1.051, [M + H]⁺ = 1176.6.

Step 4: Typical Boc protection condition (Boc₂O, 1.5 equiv, Et₃N) was applied to Compound 348-C (180 mg, 0.15 mmol) to afford 348-D (170 mg, 87% yield) as a white solid.

Compound 348 (formic acid salt) was prepared as a white solid utilizing the methods in Example 7 (Compound 101). LCMS (5-95 AB, ESI): t_{R} = 0.748, [M + H]⁺ = 887.0. ¹H NMR (400 MHz, MeOH-*d*4) δ 8.50 (brs, 2H, HCOOH), 7.33-7.11 (m, 3H), 7.12 (d, J=8.5 Hz, 1H), 6.92 (d, J=8.5 Hz, 1H), 6.84 (d, J=8.5 Hz, 1H), 6.40 (s, 0.7H), 5.98 (s, 0.3H), 4.80-4.54 (m, 3H), 4.29-4.20 (m, 4H), 4.21 (s, 2H), 3.75-3.65 (m, 2H), 3.54-3.42 (m, 4H), 3.26-3.03 (m, 7H), 2.90 (s, 3H), 2.73(brs, 1H), 2.08-1.94 (m, 2H), 1.80-1.71 (m, 3H), 1.42-1.32 (m, 18H), 0.91 (t, J=4.0 Hz, 3H).

### Example 253: Synthesis of Compound 349

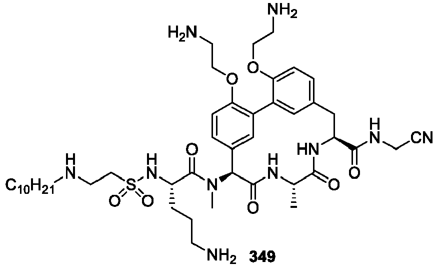

Compound 349 (free base) was prepared as a white solid utilizing the methods in Example 348. LCMS (5-95 AB, ESI): t_{R} = 0.583, [M + H]⁺ = 899.5; ¹H NMR (400 MHz, MeOH-*d*₄) δ 7.27-7.17 (m, 2H), 7.10 (d, J=8.4 Hz, 1H), 7.02 (d, J=8.4 Hz, 1H), 6.85-6.75 (m, 2H), 6.38 (s, 1H), 4.78-4.73 (m, 2H), 4.42 (brs, 1H), 4.20 (s, 2H), 4.12-3.95 (m, 4H), 3.26-3.20 (m, 2H), 3.16-3.09 (m, 2H), 3.09-2.96 (m, 3H), 2.94-2.84 (m, 2H), 2.89 (s, 3H), 2.84-2.67 (m, 3H), 2.65-2.56 (m, 2H), 1.94-1.63 (m, 6H), 1.58-1.45 (m, 3H), 1.43-1.23 (m, 14H), 0.90 (t, J=6.4 Hz, 3H).

### Example 254: Synthesis of Compound 350

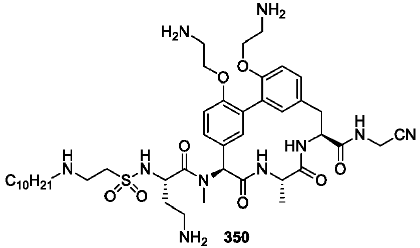

Compound 350 (formic acid salt) was prepared as a white solid utilizing the methods in Example 252 (Compound 348). LCMS (5-95 AB, ESI): t_{R} = 0.572, [M + H]⁺ = 885.7.

### Example 255: Synthesis of Compound 351

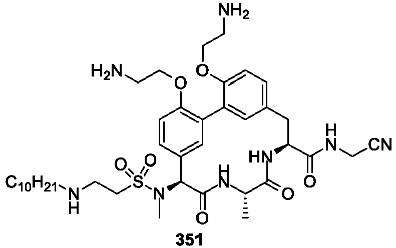

Compound 351 (formic acid salt) was prepared as a white solid utilizing the methods in Example 252 (Compound 348) from Compound 101-G. LCMS (5-95 AB, ESI): t_{R} = 0.747, [M + H]⁺ = 785.3.

### Example 256: Synthesis of Compound 352

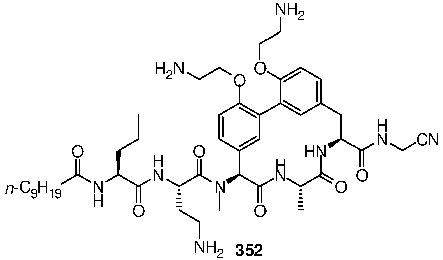

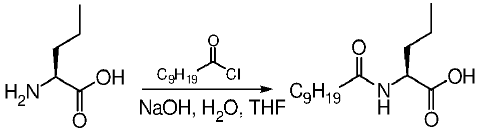

To a stirred solution of decanoyl chloride (500 mg, 2.6 mmol) in THF (5 mL) was added (S)-2-aminopentanoic acid (461 mg, 3.9 mmol) and 2N NaOH (5.0 mL) at 0°C and the resulting mixture was stirred at 0°C for 1 h. The pH of the mixture was adjusted to pH=2 using 1N HCl, which was extracted with EtOAc (20 mL x 3). The combined organic layers were washed with brine (50 mL x 2), dried over Na₂SO₄ and concentrated to afford (S)-2-decanamidopentanoic acid (630 mg, 88.5% yield) as a white solid, which was used directly in the next step. LCMS (5-95 AB, ESI): t_{R} = 0.904, [M + H]⁺ = 272.0.

Compound 352 (formic acid salt) was prepared as a white solid utilizing the methods in Example 7 (Compound 101). LCMS (5-95 AB, ESI): t_{R} = 0.620, [M + H]⁺ = 891.4; ¹H NMR (400 MHz, MeOH-*d*₄) δ 8.50 (brs, 3H, HCOOH), 7.24-7.16 (m, 2H), 7.14 (d, J=8.0 Hz, 1H), 7.08 (d, J=8.0 Hz, 1H), 6.87 (brs, 1H), 6.81 (brs, 1H), 6.26 (s, 1H), 5.00-4.97 (m, 1H), 4.85-4.77 (m, 3H), 4.23-4.15 (m, 4H), 4.19 (s, 2H), 3.34-3.33 (m, 1H), 3.17-3.03 (m, 7H), 2.82 (s, 3H), 2.28-2.19 (m, 5H), 2.05-1.90 (m, 2H), 1.67-1.62 (m, 5H), 1.35-1.30 (m, 14H), 0.98 (t, J=7.6 Hz, 3H), 0.91 (t, J=7.6 Hz, 3H).

### Example 257: Synthesis of Compound 353

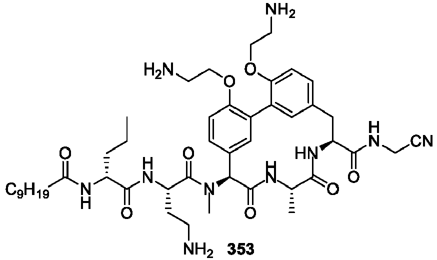

Compound 353 (formic acid salt) was prepared as a white solid utilizing the methods in Example 256 (Compound 352). LCMS (5-95 AB, ESI): t_{R} = 0.733, [M + H]⁺ = 891.6.

### Example 258: Synthesis of Compound 354

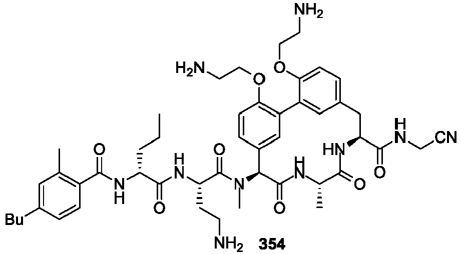

Compound 354 (formic acid salt) was prepared as a white solid utilizing the methods in Example 256 (Compound 352). LCMS (5-95 AB, ESI): t_{R} = 0.730, [M + H]⁺ = 911.4.

### Example 259: Synthesis of Compound 355

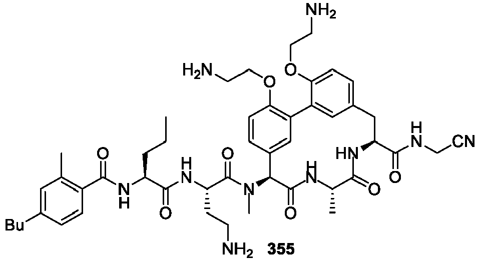

Compound 355 (formic acid salt) was prepared as a white solid utilizing the methods in Example 256 (Compound 352). LCMS (5-95 AB, ESI): t_{R} = 0.732, [M + H]⁺ = 911.5.

### Example 260: Synthesis of Compound 356

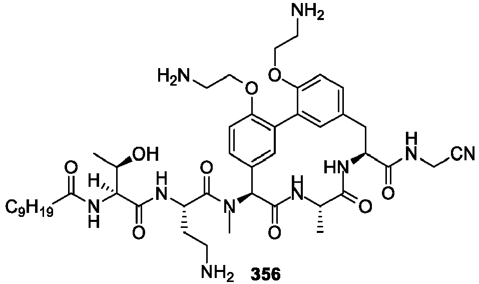

Compound 356 (formic acid salt) was prepared as a white solid utilizing the methods in Example 256 (Compound 352) from (2S,3R)-3-(tert-butoxy)-2-decanamidobutanoic acid. LCMS (5-95 AB, ESI): t_{R} = 0.720, [M + H]⁺ = 893.5.

### Example 261: Synthesis of Compound 357-P1 and Compound 357-P2

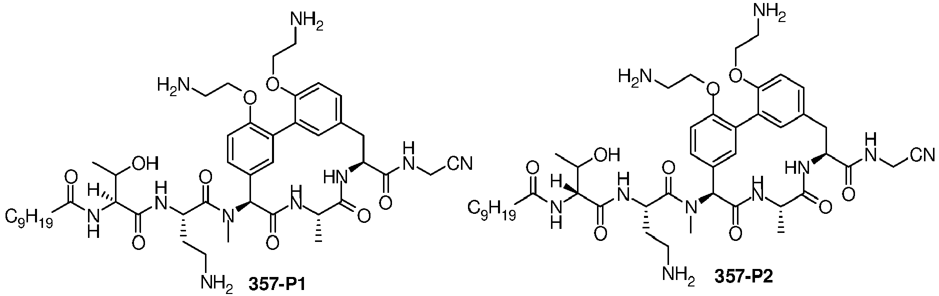

Compound 357-P1 and Compound 357-P2 (formic acid salts) were prepared as white solids utilizing the methods in Example 256 (Compound 352) from (D,L)-(erythro)-2-decanamido-3-hydroxybutanoic acid. The compounds are derived from racemic-allo threonine and the stereochemical assignment at these positions is arbitrary. They are distinguished by the elution from reverse phase HPLC with Compound 357-P1 as peak 1 and Compound 357-P2 as peak 2.

Data for Compound 357-P1: LCMS (5-95 AB, ESI): t_{R} = 0.574, [M + H]⁺ = 893.5.

Data for Compound 357-P2: LCMS (5-95 AB, ESI): t_{R} = 0.709, [M + H]⁺ = 893.7; ¹H NMR (400 MHz, MeOH-*d*₄) δ 7.21-7.18 (m, 2H), 7.08 (d, J=8.0 Hz, 1H), 7.01 (d, J=8.0 Hz, 1H), 6.82 (brs, 1H), 6.77 (brs, 1H), 6.31 (s, 1H), 4.83-4.75 (m, 2H), 4.37-4.35 (m, 1H), 4.32-4.28 (m, 1H), 4.19 (s, 2H), 4.10-3.95 (m, 5H), 3.35-3.15 (m, 1H), 3.16-3.13 (m, 2H), 2.90-2.75 (m, 7H), 2.34-2.32 (m, 3H), 2.13-2.08 (m, 2H), 1.92-1.88 (m, 2H), 1.65-1.62 (m, 3H), 1.33-1.19 (m, 16 H), 0.91 (t, *J*=6.6 Hz, 3H).

### Example 263: Synthesis of Compound 359

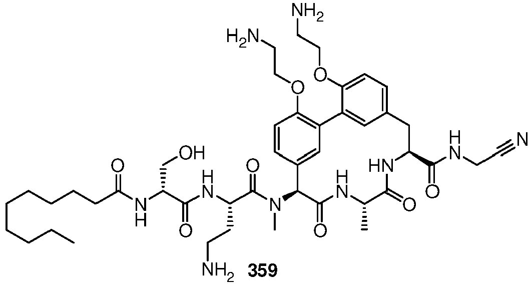

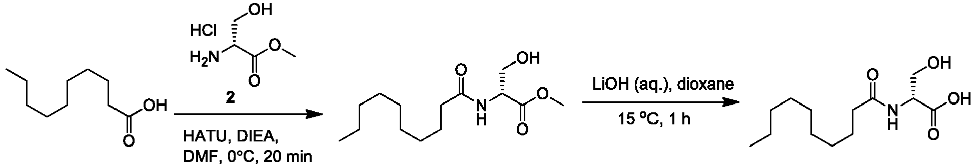

Step 1: Decanoic acid and (R)-methyl 2-amino-3-hydroxypropanoate hydrochloride was subjected to General Method HATU to afford (R)-methyl 2-decanamido-3-hydroxypropanoate.

Step 2: (R)-Methyl 2-decanamido-3-hydroxypropanoate was subjected to General Method LiOH to afford (R)-2-decanamido-3-hydroxypropanoic acid.

Compound 359 (formic acid salt) was prepared as a white solid utilizing the methods in Example 7 (Compound 101) from (R)-2-decanamido-3-hydroxypropanoic acid. LCMS (Method 5-95 AB, ESI): t_{R} = 0.576 min, [M/2+H]⁺ = 440.3.

### Example 264: Synthesis of Compound 360

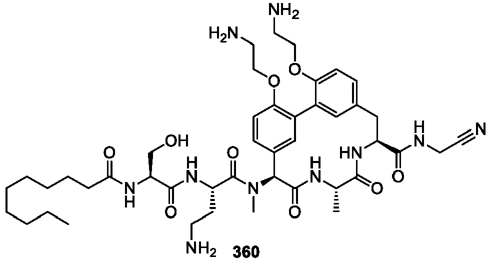

Compound 360 (formic acid salt) was prepared as a white solid utilizing the methods in Example 263 (Compound 359) from (S)-2-decanamido-3-hydroxypropanoic acid. LCMS (Method 5-95 AB, ESI): t_{R} = 0.709 min, [M + H]⁺ = 879.7.

### Example 265: Synthesis of Compound 361

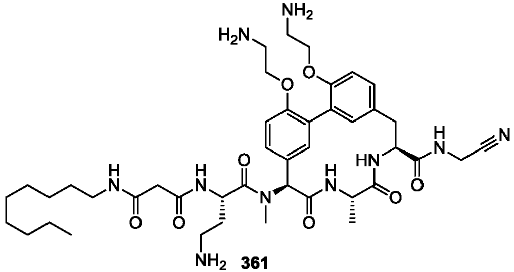

Compound 361 (formic acid salt) was prepared as a white solid utilizing the methods in Example 263 (Compound 359) from 3-(nonylamino)-3-oxopropanoic acid. LCMS (Method 5-95 AB, ESI): t_{R} = 0.718 min, [M + H]⁺ = 850.6.

### Example 266: Synthesis of Compound 362

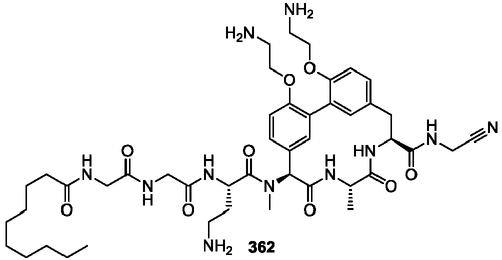

Compound 362 (formic acid salt) was prepared as a white solid utilizing the methods in Example 263 (Compound 359) from 2-(2-decanamidoacetamido)acetic acid. LCMS (Method 5-95 AB, ESI): t_{R} = 0.719 min, [M + H]⁺ = 906.5.

### Example 267: Synthesis of Compound 363

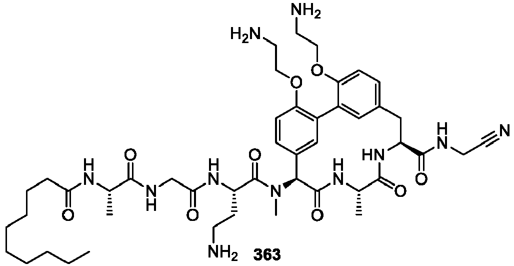

Compound 363 (formic acid salt) was prepared as a white solid utilizing the methods in Example 263 (Compound 359) from (S)-2-(2-decanamidopropanamido)acetic acid. LCMS (Method 5-95 AB, ESI): t_{R} = 0.718 min, [M + H]⁺ = 920.6.

### Example 268: Synthesis of Compound 364

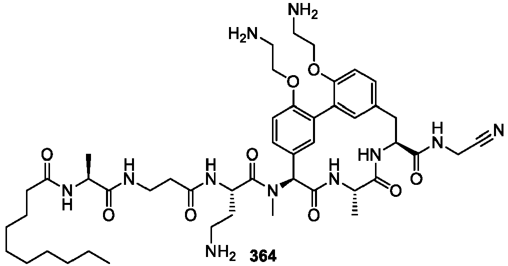

Compound 364 (formic acid salt) was prepared as a white solid utilizing the methods in Example 263 (Compound 359) from (S)-3-(2-decanamidopropanamido)propanoic acid. LCMS (Method 5-95 AB, ESI): t_{R} = 0.726 min, [M + H]⁺ = 934.7.

### Example 269: Synthesis of Compound 365

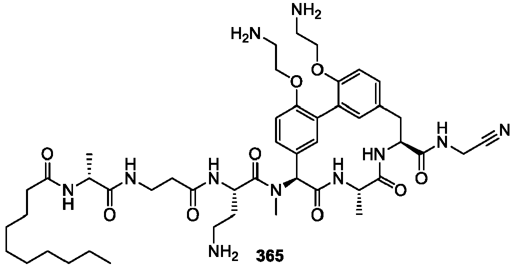

Compound 365 (formic acid salt) was prepared as a white solid utilizing the methods in Example 263 (Compound 359) from (R)-3-(2-decanamidopropanamido)propanoic acid. LCMS (Method 5-95 AB, ESI): t_{R} = 0.729 min, [M + H]⁺ = 934.5.

### Example 270: Synthesis of Compound 366

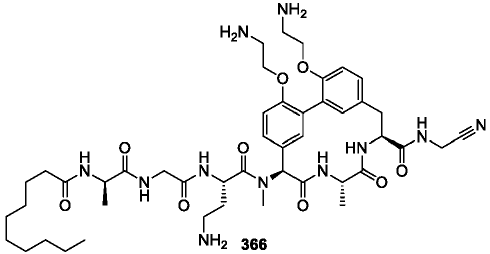

Compound 366 (formic acid salt) was prepared as a white solid utilizing the methods in Example 263 (Compound 359) from (R)-2-(2-decanamidopropanamido)acetic acid. LCMS (Method 5-95 AB, ESI): t_{R} = 0.609 min, [M + H]⁺ = 920.5.

### Example 271: Synthesis of Compound 367

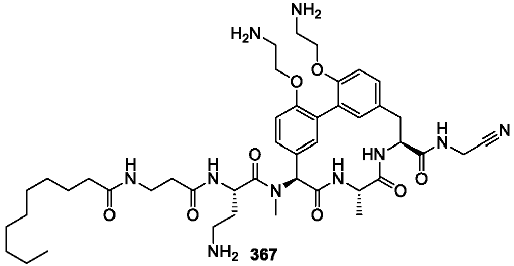

Compound 367 (formic acid salt) was prepared as a white solid utilizing the methods in Example 263 (Compound 359) from 3-decanamidopropanoic acid. LCMS (Method 5-95 AB, ESI): t_{R} = 0.578 min, [M + H]⁺ = 863.3.

### Example 272: Synthesis of Compound 368

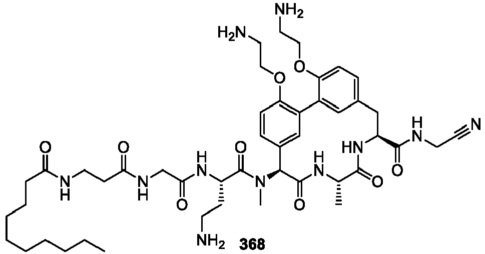

Compound 368 (formic acid salt) was prepared as a white solid utilizing the methods described previously from 2-(3-decanamidopropanamido)acetic acid. LCMS (Method 5-95 AB, ESI): t_{R} = 0.573 min, [M + H]⁺ = 920.4.

### Example 273: Synthesis of Compound 369

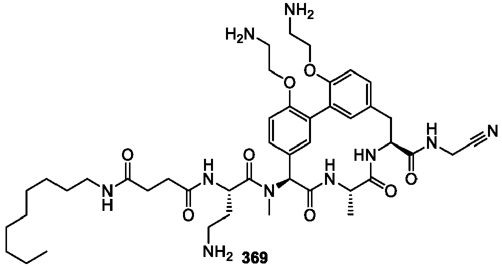

Compound 369 (formic acid salt) was prepared as a white solid utilizing the methods in Example 263 (Compound 359) from 4-(nonylamino)-4-oxobutanoic acid. LCMS (Method 5-95 AB, ESI): t_{R} = 0.713 min, [M + H]⁺ = 863.5.

### Example 274: Synthesis of Compound 370

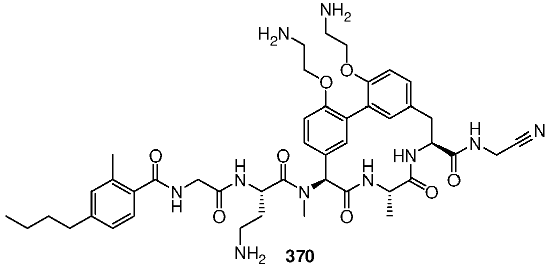

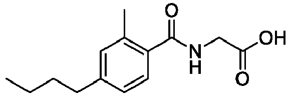

2-(4-Butyl-2-methylbenzamido)acetic acid, a white solid, was prepared from methods previously described. ¹H NMR (400 MHz, CD₃OD): δ 7.35 (d, J = 7.6 Hz, 1H), 7.10 - 7.00 (m, 2H), 4.05 (s, 2H), 2.60 (t, J = 7.8 Hz, 2H), 2.41 (s, 3H), 1.65 - 1.55 (m, 2H), 1.40 - 1.30 (m, 2H), 0.93 (t, J = 7.2 Hz, 3H).

Compound 370 (formic acid salt) was prepared as a white solid utilizing the methods in Example 7 (Compound 101). LCMS (Method 5-95 AB, ESI): t_{R} = 0.593 min, [M + H]⁺ = 870.3.

### Example 275: Synthesis of Compound 371

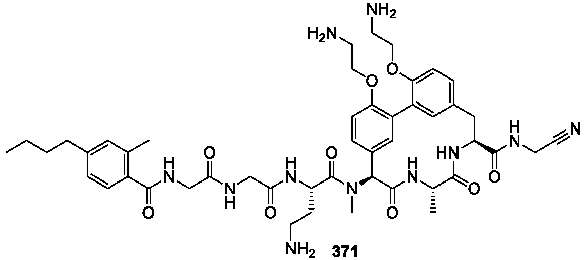

Compound 371 (formic acid salt) was prepared as a white solid utilizing the methods previously described from 2-(2-(4-butyl-2-methylbenzamido)acetamido)acetic acid. LCMS (Method 5-95 AB, ESI): t_{R} = 0.599 min, [M + H]⁺ = 926.3.

### Example 276: Synthesis of Compound 372

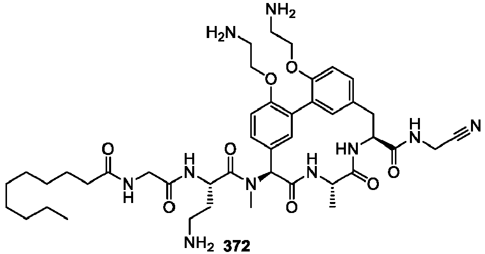

Compound 372 (formic acid salt) was prepared as a white solid utilizing the methods in Example 263 (Compound 359) from 2-decanamidoacetic acid. LCMS (Method 5-95 AB, ESI): t_{R} = 0.717 min, [M + H]⁺ = 849.5.

### Example 277: Synthesis of Compound 373

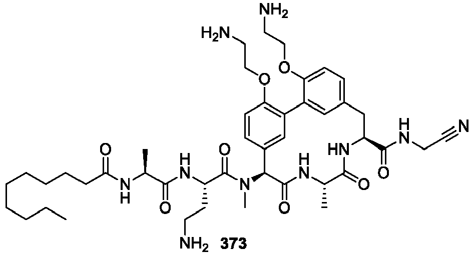

Compound 373 (formic acid salt) was prepared as a white solid utilizing the methods in Example 263 (Compound 359) from (S)-2-decanamidopropanoic acid. LCMS (Method 5-95 AB, ESI): t_{R} = 0.722 min, [M + H]⁺ = 863.6.

### Example 278: Synthesis of Compound 374

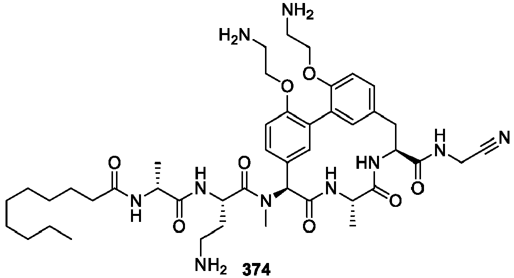

Compound 374 (formic acid salt) was prepared in as a white solid utilizing the methods in Example 263 (Compound 359) from (R)-2-decanamidopropanoic acid. LCMS (Method 5-95 AB, ESI): t_{R} = 0.718 min, [M + H]⁺ = 863.6.

### Example 279: Synthesis of Compound 375

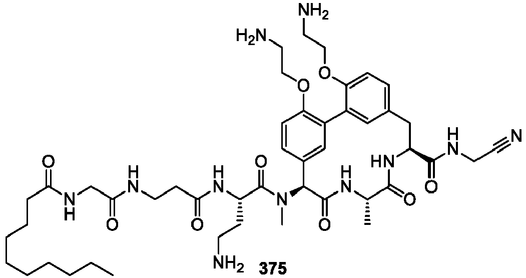

Compound 375 (formic acid salt) was prepared as a white solid utilizing the methods previously described from 3-(2-decanamidoacetamido)propanoic acid. LCMS (Method 5-95 AB, ESI): t_{R} = 0.588 min, [M+Na]⁺ = 943.1.

### Example 280: Synthesis of Compound 376

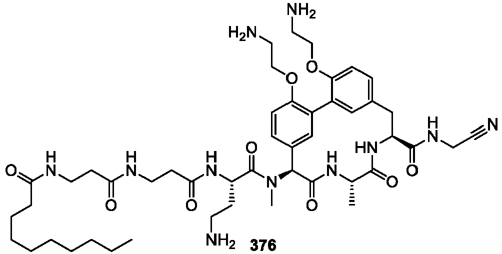

Compound 376 (formic acid salt) was prepared as a white solid utilizing the methods previously described from 3-(3-decanamidopropanamido)propanoic acid. LCMS (Method 5-95 AB, ESI): t_{R} = 0.584 min, [M + Na]⁺ = 956.6.

### Example 281: Synthesis of Compound 377

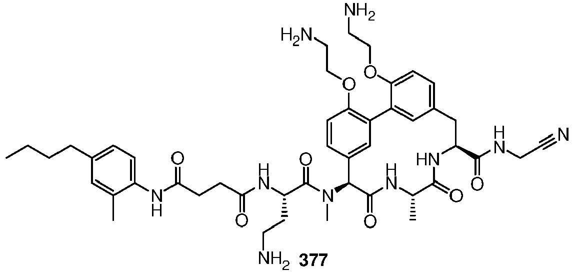

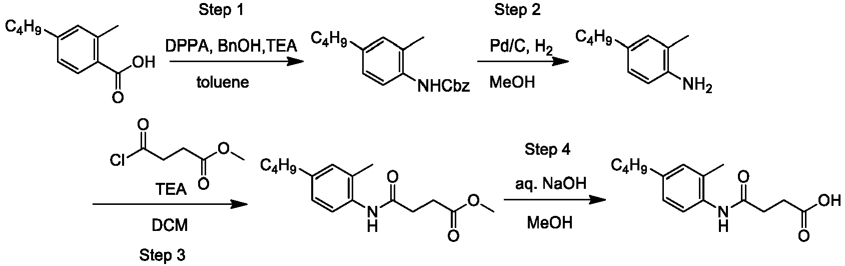

Step 1: A mixture of 4-butyl-2-methylbenzoic acid (150.0 mg, 0.78 mmol), diphenylphosphoryl azide (300 mg, 1.09 mmol), benzyl alcohol (252 mg, 2.34 mmol), and triethylamine (220 mg, 2.19 mmol) in toluene (10 mL) was stirred at 100°C for 12 h. The reaction mixture was diluted with water (20 mL) and extracted with EtOAc (30 mL x 3). The combined organic layers were washed with water (20 mL x 2) and brine (20 mL), dried over MgSO₄ and concentrated. The residue was purified by chromatography on silica gel (10% EtOAc in petroleum ether, Rf = 0.5) to obtain benzyl N-(4-butyl-2-methyl-phenyl)carbamate (100 mg, 0.34 mmol, 64.6% yield) as a white solid. ¹H NMR (400 MHz, DMSO-d6) δ 8.87 (s, 1H), 7.40 - 7.35 (m, 5H), 7.21 (d, J = 8.0 Hz, 1H), 6.99 - 6.95 (m, 2H), 5.12 (s, 2H), 2.50 (t, J = 7.6 Hz, 1H), 2.16 (s, 3H), 1.55 - 1.48 (m, 2H), 1.31 - 1.26 (m, 2H), 0.89 (t, J = 7.6 Hz, 1H).

Step 2: To a solution of benzyl N-(4-butyl-2-methyl-phenyl)carbamate (150 mg, 0.50 mmol) in methanol (10 mL) was added 10% palladium (53.68 mg, 0.05 mmol) on carbon. The reaction mixture was stirred at 15°C for 2 h under H₂ at 15 psi. The mixture was filtered and concentrated to obtain 4-butyl-2-methyl-aniline (80 mg, 0.49 mmol, 97.1% yield) as a white solid.

Step 3: To a mixture of 4-butyl-2-methylaniline (70 mg, 0.43 mmol) and triethylamine (0.18 mL, 1.29 mmol) in dichloromethane (10 mL) was added methyl 4-chloro-4-oxobutyrate (64.56 mg, 0.43 mmol). The reaction mixture was stirred at 20°C for 12 h. The mixture was concentrated to obtain methyl 4-(4-butyl-2-methyl-anilino)-4-oxo-butanoate (100 mg, 0.36 mmol, 84.1% yield) as a white solid. LCMS (5-95AB_1.5min): t_{R} = 0.919 min, [M + H]⁺ 278.0.

Step 4: Methyl 4-(4-butyl-2-methyl-anilino)-4-oxo-butanoate (90 mg, 0.32 mmol) was hydrolyzed as usual to obtain 4-(4-butyl-2-methyl-anilino)-4-oxo-butanoic acid (80 mg, 0.30 mmol, 93.6% yield) as a white solid.

Compound 377 (formic acid salt) was prepared as a white solid utilizing the methods in Example 7 (Compound 101). LCMS (Method 5-95 AB, ESI): t_{R} = 0.707 min, [M + H]⁺ = 883.7.

### Example 282: Synthesis of Compound 378

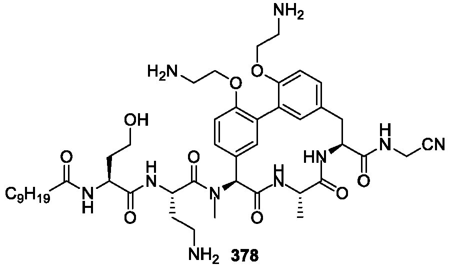

Compound 378 (formic acid salt) was prepared as a white solid utilizing the same methods used in the preparation of Compound 101 (Example 7) except an additional peptide coupling and deprotection step is added to the sequence, starting from (S)-2-(((benzyloxy)carbonyl)amino)-4-((tert-butyldimethylsilyl)oxy)butanoic acid. LCMS (5-95 AB, ESI): t_{R} = 0.715, [M + H]⁺ = 893.7; ¹H NMR (400 MHz, MeOH-*d*₄) δ 8.50 (brs, 2H, HCOOH), 7.33-7.24 (m, 2H), 7.19 (d, J=8.4 Hz, 1H), 7.11 (d, J=8.4 Hz, 1H), 6.90 (brs, 1H), 6.83 (brs, 1H), 6.29 (s, 1H), 5.03-4.76 (m, 3H), 4.40-4.38 (m, 1H), 4.32-4.18 (m, 4H), 4.21 (s, 2H), 3.74-3.62 (m, 2H), 3.32-2.95 (m, 7H), 2.84 (s, 3H), 2.34-2.14 (m, 3H), 2.13-1.82 (m, 3H), 1.66-1.63 (m, 2H), 1.39-1.27 (m, 16H), 0.92 (t, 7=6.4, 3H).

### Example 283: Synthesis of Compound 379

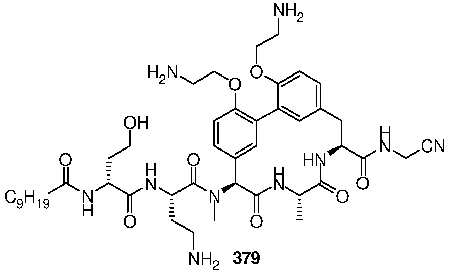

Compound 379 (formic acid salt) was prepared as a white solid utilizing the methods in Example 282 (Compound 378) from (R)-2-(((benzyloxy)carbonyl)amino)-4-((tertbutyldimethylsilyl)oxy)butanoic acid. LCMS (5-95 AB, ESI): t_{R} = 0.712, [M + H]⁺ = 893.6.

### Example 284: Synthesis of Compound 380

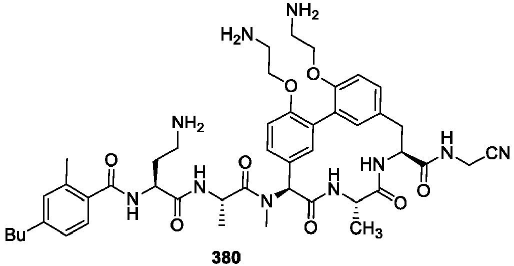

Compound 380 (formic acid salt) was prepared as a white solid utilizing the same methods used in the preparation of Compound 378 (Example 282). LCMS (5-95 AB, ESI): t_{R} = 0.713, [M + H]⁺ = 883.5.

### Example 285: Synthesis of Compound 381

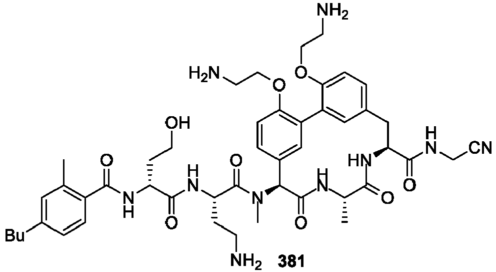

Compound 381 (formic acid salt) was prepared as a white solid utilizing the methods in Example 282 (Compound 378) from (R)-2-(((benzyloxy)carbonyl)amino)-4-((tertbutyldimethylsilyl)oxy)butanoic acid. LCMS (5-95 AB, ESI): t_{R} = 0.694, [M + H]⁺ = 913.7.

### Example 286: Synthesis of Compound 382

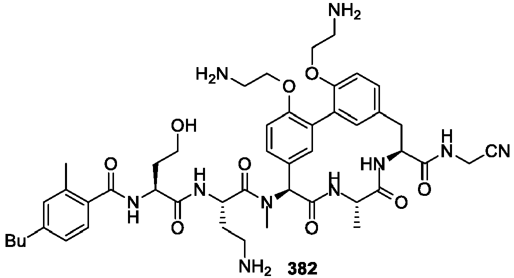

Compound 382 (formic acid salt) was prepared as a white solid utilizing the methods in Example 282 (Compound 378) from (S)-2-(((benzyloxy)carbonyl)amino)-4-((tertbutyldimethylsilyl)oxy)butanoic acid. LCMS (5-95 AB, ESI): t_{R} = 0.689, [M + H]⁺ = 913.9.

### Example 287: Synthesis of Compound 383

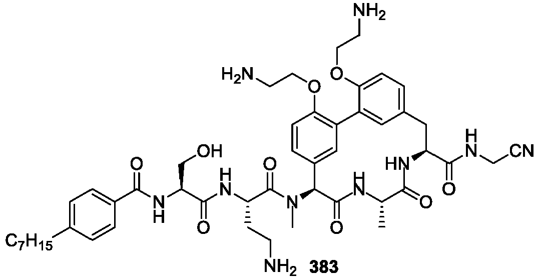

Compound 383 (formic acid salt) was prepared as a white solid utilizing the methods in Example 282 (Compound 378) from Cbz-Ser(O-t-Bu)-OH. LCMS (5-95 AB, ESI): t_{R} = 0.741, [M + H]⁺ = 927.8

### Example 288: Synthesis of Compound 384

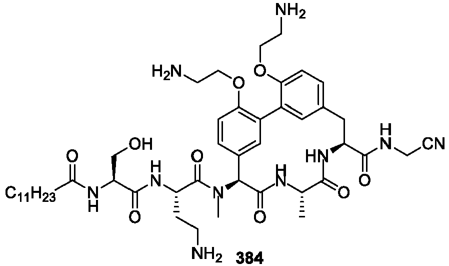

Compound 384 (formic acid salt) was prepared as a white solid utilizing the methods in Example 282 (Compound 378). LCMS (5-95 AB, ESI): t_{R} = 0.751, [M + H]⁺ = 907.6.

### Example 289: Synthesis of Compound 385

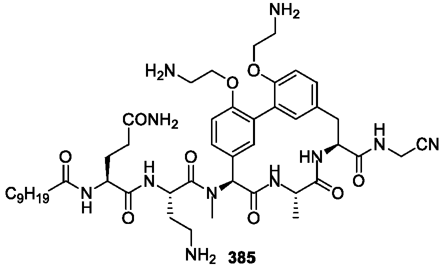

Compound 385 (free base) was prepared as a white solid utilizing the methods in Example 263 (Compound 359) from (S)-5-amino-2-(((benzyloxy)carbonyl)amino)-5-oxopentanoic acid. LCMS (5-95 AB, ESI): t_{R} = 0.573, [M + H]⁺ = 921.0.

### Example 290: Synthesis of Compound 386

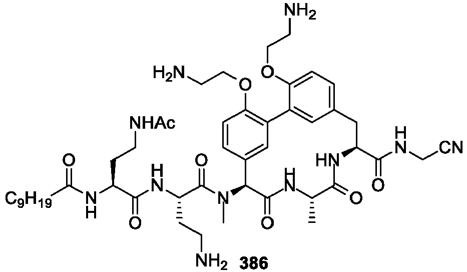

Compound 386 (formic acid salt) was prepared as a white solid utilizing the methods in Example 263 (Compound 359) from (S)-4-acetamido-2-(((benzyloxy)carbonyl)amino)butanoic acid. LCMS (5-95 AB, ESI): t_{R} = 0.716, [M + H]⁺ = 934.7.

### Example 291: Synthesis of Compound 387

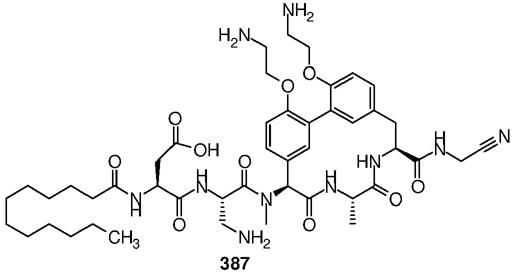

Compound 387 (formic acid salt) was prepared as a white solid utilizing the methods in Example 282 (Compound 378). LCMS (Method 5-95 AB, ESI): t_{R} = 0.757 min, [M+H]⁺ = 921.6.

### Example 292: Synthesis of Compound 388

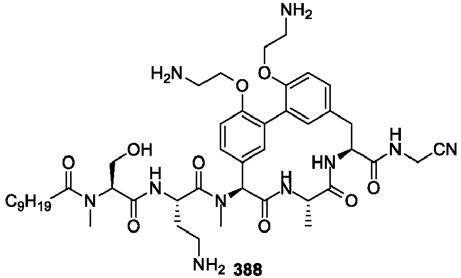

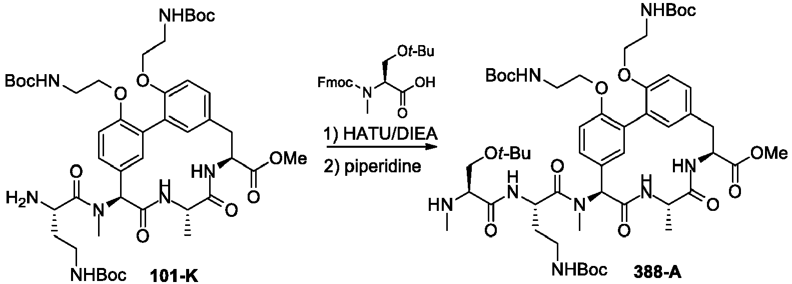

Starting from Compound 101-K, typical amide coupling (HATU) with (S)-2-((((9H-fluoren-9-yl)methoxy)carbonyl)(methyl)amino)-3-(tert-butoxy)propanoic acid and Fmoc removal (piperidine) procedure was followed to afford Compound 388-A (110 mg) as a white solid.

Compound 388 (formic acid salt) was prepared as a white solid utilizing the methods in Example 7 (Compound 101) from Compound 388-A. LCMS (5-95 AB, ESI): t_{R} = 0.708, [M + H]⁺ = 893.5.

### Example 293: Synthesis of Compound 389

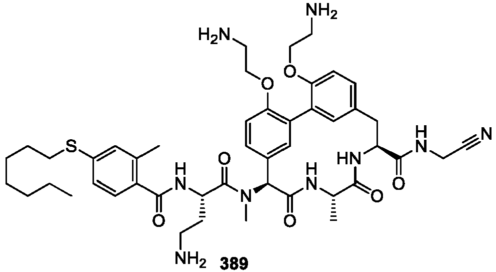

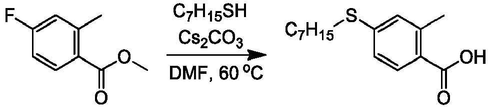

To a solution of methyl 4-fluoro-2-methylbenzoate (200.0 mg, 1.19 mmol) in N, N-dimethylformamide (10 mL) were added 1-heptanethiol (0.91 mL, 5.95 mmol) and cesium carbonate (1937.6 mg, 5.95 mmol), and stirred at 60°C for 16 h. The reaction was quenched by 5% aq. KHSO₄ till pH = 6, diluted with H₂O (20 mL) and extracted with EtOAc (20 mL x 2). The combined organic layers were washed with water (40 mL x 2) and brine (20 mL), dried over Na₂SO₄ and concentrated. The residue was triturated with petroleum ether. The resulting white solid was filtered, washed with petroleum ether and dried to obtain 4-(heptylthio)-2-methylbenzoic acid (198 mg, 62.5% yield). ¹H NMR (400 MHz, CD₃OD): δ 7.84 (d, *J*= 8.8 Hz, 1H), 7.15-7.05 (m, 2H), 3.00 (t, *J*= 7.2 Hz, 2H), 2.55 (s, 3H), 1.75-1.60 (m, 2H), 1.50-1.35 (m, 2H), 1.35-1.20 (m, 6H), 0.90 (t, *J* = 7.0 Hz, 3H).

Compound 389 (formic acid salt) was prepared as a white solid utilizing the methods in Example 7 (Compound 101). LCMS (Method 5-95 AB, ESI): RT = 0.649 min, [M+Na]⁺ = 908.5.

### Example 294: Synthesis of Compound 390

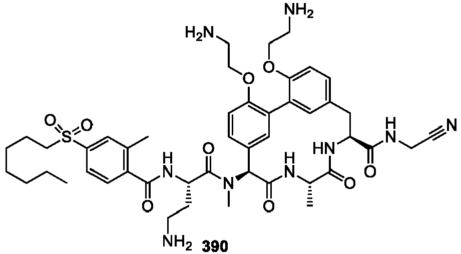

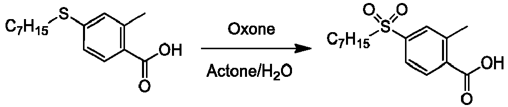

To a solution of 4-heptylsulfanyl-2-methyl-benzoic acid (50.0 mg, 0.19 mmol) in acetone (3 mL) was added potassium peroxymonosulfate (230.8 mg, 0.38 mmol) in water (1 mL). The mixture was stirred at 0°C for 2 h, quenched by saturated aqueous Na₂SO₃ solution and extracted with EtOAc (20 mL x 2). The combined organic layers were washed with water (40 mL x 2) and brine (20 mL), dried over Na₂SO₄ and concentrated to give 4-heptylsulfonyl-2-methylbenzoic acid (50 mg, 89.3% yield) as a white solid which was used directly.

Compound 390 (formic acid salt) was prepared as a white solid utilizing the methods in Example 7 (Compound 101). LCMS (Method 5-95 AB, ESI): t_{R} = 0.590 min, [M+H]⁺ = 918.4.

### Example 295: Synthesis of Compound 391

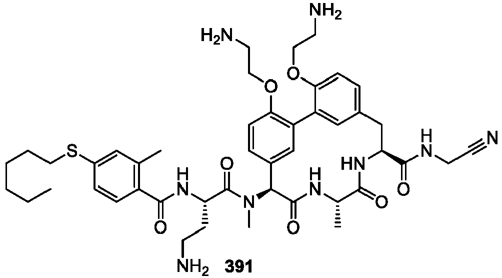

Compound 391 (formic acid salt) was prepared as a white solid utilizing the methods in Example 293 (Compound 389). LCMS (Method 5-95 AB, ESI): t_{R} = 0.637 min, [M+Na]⁺ = 894.4.

### Example 296: Synthesis of Compound 392

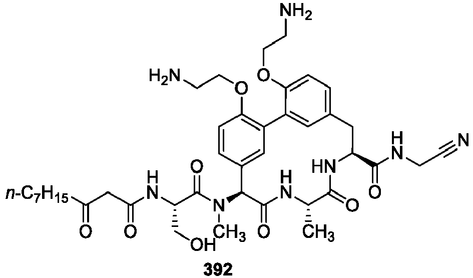

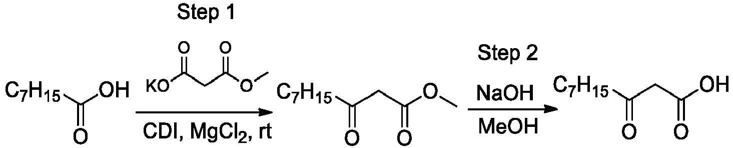

Step 1: To a solution of octanoic acid (5.0 g, 34.7 mmol) in THF (100 mL) was added potassium 3-methoxy-3-oxopropanoate (6.2 g, 38.1 mmol) and the mixture was stirred at room temperature for 2 h, followed by the addition of MgCl₂ (3.3 g, 34.7 mmol) and CDI (5.7 g, 36.4 mmol). The resulting mixture was heated to 60°C for 3 h. After cooling to room temperature, the filtrate was concentrated and the residue was taken up by EtOAc (150 mL), which was washed with brine (150 mL x 2). The organic layer was dried over Na₂SO₄, concentrated and the residue was purified by silica gel flash column to give methyl 3-oxodecanoate (4.4 g, 63.4% yield) as yellow oil.

Step 2: The General Method NaOH (NaOH ester hydrolysis) procedure was applied to methyl 3-oxodecanoate (200 mg, 1.0 mmol) to give 3-oxodecanoic acid (150 mg, 81% yield) as a white solid.

Compound 392 (formic acid salt) was prepared as a white solid utilizing the same methods used in the preparation of Compound 231 (Example 137) except 3-oxodecanoic acid was used in the coupling step. LCMS (5-95 AB, ESI): t_{R} = 0.574, [M + H]⁺ = 793.2.

### Example 297: Synthesis of Compound 393-P1 and Compound 393-P2

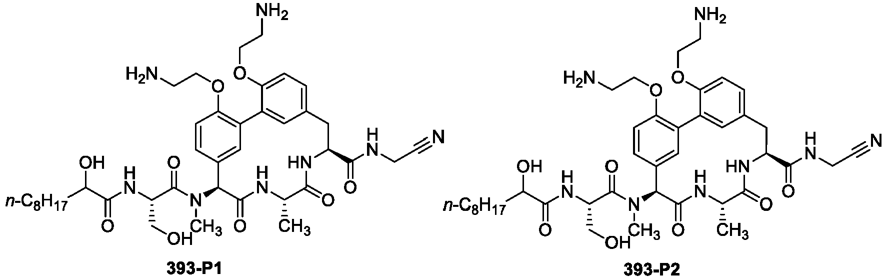

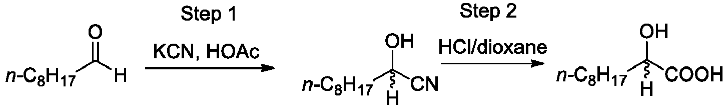

Step 1: To a mixture of KCN (2.59 g, 39.7 mmol) in EtOAc/MeOH (80 mL, v/v=1:1) was added sequentially with HOAc (2.39 g, 39.7 mmol) and nonanal (5.0 g, 35.2 mmol) at 0°C and the reaction was stirred at room temperature for 16 h. The volatiles were removed and the residue was poured into H₂O (50 mL), which was extracted with EtOAc (40 mL x 3). The combined organic layers were dried over Na₂SO₄, concentrated and the residue was purified by silica gel flash column to give (±)-2-hydroxydecanenitrile (5.8 g, 97.5% yield) as colorless oil.

Step 2: The acidic hydrolysis of a nitrile to an acid is described and is referred to as General Method Nitrile Hydrolysis. To a solution of (±)-2-hydroxydecanenitrile (2.0 g, 11.8 mmol) in 1,4-dioxane (10 mL) was added with 36% HCl solution (10 mL) and the mixture was heated to 100°C while stirring and stirred at the same temperature for 12 h. The volatiles were removed to afford (±)-2-hydroxydecanoic acid (1.6 g, 60.3% yield) as a white solid, which was used directly for the next step.

Compound 393-P1 (formic acid salt) and Compound 393-P2 were prepared as white solids utilizing the same methods used in the preparation of Compound 231 (Example 137) except (±)-2-hydroxydecanoic acid was used in the coupling step. The diastereomers were separated on prep-HPLC. Data for Compound 393-P1: LCMS (5-95 AB, ESI): t_{R} = 0.727, [M + H]⁺ = 795.4. Data for Compound 393-P2: LCMS (5-95 AB, ESI): t_{R} = 0.730, [M + H]⁺ = 795.4.

### Example 298: Synthesis of Compound 394

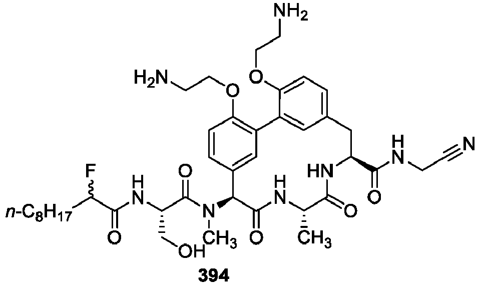

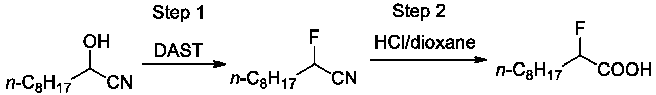

Step 1: To a solution of (±)-2-hydroxydecanenitrile (3.0 g, 17.7 mmol) in DCM (20 mL) was added DAST (6.4 g, 39.9 mmol) dropwise at -78°C within 0.5 h. The reaction was warmed up gradually to the room temperature and stirred at the same temperature for 36 h. The volatiles were removed and the residue was taken up by EtOAc (100 mL), which was washed with brine (100 mL x 2). The organic layer was dried with Na₂SO₄, concentrated and the residue was purified by silica gel flash column to afford (±)-2-fluorodecanenitrile (1.5 g, 49.4% yield) as colorless oil.

Step 2: (±)-2-Fluorodecanenitrile (490 mg, 2.9 mmol) was subject to General Method Nitrile hydrolysis to afford (±)-2-fluorodecanoic acid (450 mg, 83% yields) as a light yellow solid.

Compound 394-P1 (formic acid salt) and Compound 394-P2 were prepared as white solids utilizing the same methods used in the preparation of Compound 231 (Example 137) except (±)-2-fluorodecanoic acid was used in the coupling step. The diastereomers were separated on prep-HPLC. Data for Compound 394-P1: LCMS (5-95 AB, ESI): t_{R} = 0.760, [M + H]⁺ = 797.6. Data for Compound 394-P2: LCMS (5-95 AB, ESI): t_{R} = 0.773, [M + H]⁺ = 797.4.

### Example 299: Synthesis of Compound 395

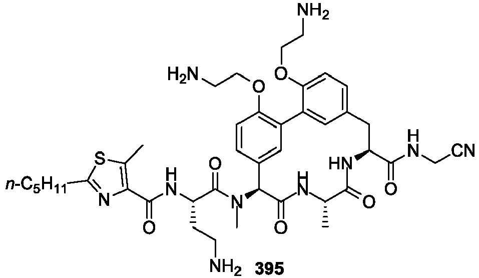

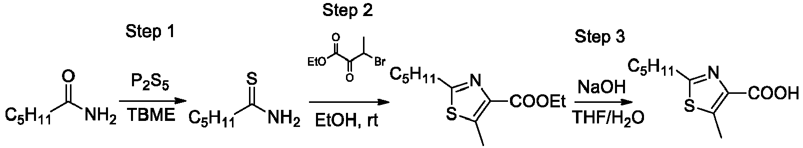

Step 1: To a solution of hexanamide (500 mg, 4.4 mmol) in TBME (40 mL) was added phosphorous penta-sulfide (241 mg, 1.1 mmol) at room temperature and the mixture was stirred at the same temperature for 18 h. The volatiles were removed and the residue was purified on silica gel flash column to afford hexanethioamide (420 mg, 73.7% yield) as a pale yellow solid.

Step 2: To a solution of hexanethioamide (420 mg, 3.2 mmol) in EtOH (6 mL) was added ethyl 3-bromo-2-oxobutanoate (669 mg, 3.2 mmol) dropwise under N₂ at 0°C and the mixture was gradually warmed up and stirred at room temperature for 18 h. The volatiles were removed and the residue was taken up by EtOAc (50 mL), which was washed with brine (50 mL). The organic layer was dried over Na₂SO₄, concentrated and the residue was purified on silica gel flash column to afford ethyl 5-methyl-2-pentylthiazole-4-carboxylate (390 mg, 50.5% yield) as pale green oil.

Step 3: The NaOH ester hydrolysis procedure (General Method NaOH) was applied to ethyl 5-methyl-2-pentylthiazole-4-carboxylate (390 mg, 1.62 mmol) to afford 5-methyl-2-pentylthiazole-4-carboxylic acid (250 mg, 72.5% yield) as a pale yellow solid.

Compound 395 (formic acid salt) was prepared as a white solid utilizing the methods in Example 7 (Compound 101). LCMS (5-95 AB, ESI): t_{R} = 0.568, [M + H]⁺ = 833.3.

### Example 300: Synthesis of Compound 396

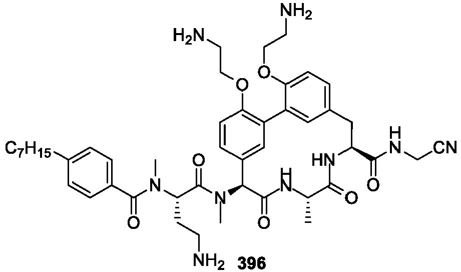

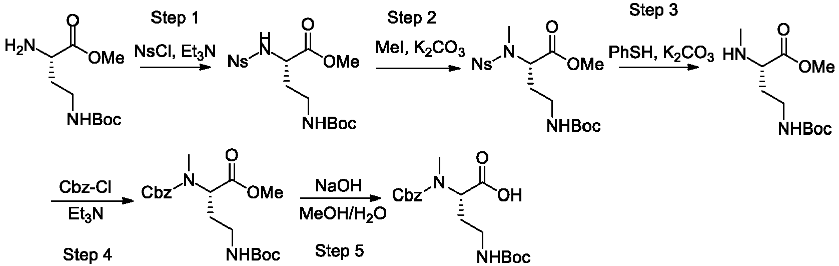

Step 1: To a solution of (S)-methyl 2-amino-4-((tert-butoxycarbonyl)amino)butanoate (2.8 g, 12.0 mmol), 2-nitrobenzenesulfonyl chloride (4.0 g, 18.0 mmol) in DCM (40 mL) was added Et₃N (3.7 g, 36.0 mmol) at 0°C and the mixture was stirred at the same temperature for 2h. The reaction mixture with then washed with 1 N HCl (40 mL), saturated NaHCO₃ solution (40 mL) and brine (40 mL). The organic layer was dried over Na₂SO₄, concentrated and the residue was purified by silica gel flash column to give (S)-methyl 4-((tert-butoxycarbonyl)amino)-2-(2-nitrophenylsulfonamido)butanoate (4.5 g, 89.4% yield) as pale-green oil.

Step 2: To a solution of (S)-methyl 4-((tert-butoxycarbonyl)amino)-2-(2-nitrophenylsulfonamido)butanoate (2.3 g, 5.5 mmol), K₂CO₃ (3.8 g, 27.5 mmol) in DMF (10 mL) was added methyl iodide (3.9 g, 27.5 mmol) and the mixture was stirred for at room temperature for 1.5 h. The reaction was added with EtOAc (100 mL) and the filtration was washed with brine (100 mL x 3). The organic layer was dried over Na₂SO₄, concentrated to give (S)-methyl 4-((tert-butoxycarbonyl)amino)-2-(N-methyl-2-nitrophenylsulfonamido)butanoate (2.3 g, 96.7% yield) as a pale-yellow oil.

Step 3: To a solution of (S)-methyl 4-((tert-butoxycarbonyl)amino)-2-(N-methyl-2-nitrophenylsulfonamido)butanoate (2.2 g, 5.1 mmol), K₂CO₃ (4.2 g, 30.6 mmol) in DMF (15 mL) was added thiophenol (6.3 g, 57.1 mmol) and the mixture was stirred at room temperature for 3 h. TLC(50% ethyl acetate in petroleum ether, Rf=0.1) showed the start material was consumed. The reaction was added with EtOAc (100 mL) and the filtration was washed with brine (100 mL x 3). The organic layer was dried over Na₂SO₄, concentrated to give (S)-methyl 4-((tert-butoxycarbonyl)amino)-2-(methylamino)butanoate (830 mg, 66.1% yield) as yellow oil. ¹H NMR (400 MHz, CDCl₃) δ 5.15-5.21 (m, 1H), 3.73 (s, 3H), 3.17-3.32 (m, 3H), 2.35 (s, 3H), 1.84-1.89 (m, 1H), 1.66-1.73 (m, 1H), 1.43 (s, 9H)

Step 4: To a solution of (S)-methyl 4-((tert-butoxycarbonyl)amino)-2-(methylamino)butanoate (730 mg, 2.96 mmol) and Et₃N (750 mg, 7.41 mmol) in DCM (50 mL) was added benzyl chloroformate (758 mg, 4.45 mmol) dropwise at 0°C and the mixture was warmed up to temperature while stirring and stirred at the same temperature for 2 h. The mixture was washed 1N HCl (30 mL) and brine (30 mL x 2) sequentially. The organic layer was dried over Na₂SO₄, concentrated and the residue was purified by silica gel flash column to give (S)-methyl 2-(((benzyloxy)carbonyl)(methyl)amino)-4-((tert-butoxycarbonyl)amino)butanoate (1.0 g, 88.7% yield) as a pale-yellow oil.

Step 5: Typical ester hydrolysis (NaOH/MeOH/H₂O) was applied to (S)-methyl 2-(((benzyloxy)carbonyl)(methyl)amino)-4-((tert-butoxycarbonyl)amino)butanoate (1.0 g, 2.63 mmol) to afford (S)-2-(((benzyloxy)carbonyl)(methyl)amino)-4-((tert-butoxycarbonyl)amino)butanoic acid (800 mg, 83% yield) as pale-yellow oil.

Compound 396 (6.0 mg) as a white solid utilizing the methods in Example 7 (Compound 101). LCMS (5-95 AB, ESI): t_{R} = 0.739, [M + H]⁺ = 854.6; ¹H NMR (400 MHz, MeOH-*d*₄) δ 8.49 (brs, 2H), 7.35-7.22 (m, 6H), 7.19 (d, *J*=8.4 Hz, 1H), 7.09 (d, *J*=8.4 Hz, 1H), 6.90 (brs, 1H), 6.81 (brs, 1H), 6.24 (s, 1H), 5.57 (s, 2H), 4.77-4.75 (m, 1H), 4.26-4.17 (m, 4H), 4.20 (s, 2H), 3.30-3.20 (m, 1H), 3.18-3.14 (m, 4H), 3.08-3.03 (m, 2H), 3.00 (s, 3H), 2.82 (s, 3H), 2.72-2.64 (m, 3H), 2.40-2.25 (m, 1H), 2.20-2.16 (m, 1H), 1.64 (s, 3H), 1.39-1.25 (m, 10H), 0.90 (t, *J*=6.6 Hz, 3H).

### Example 301: Synthesis of Compound 397

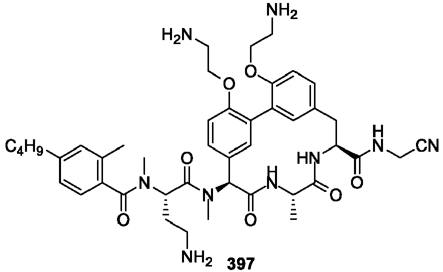

Compound 397 (formic acid salt) was prepared as a white solid utilizing the methods in Example 300. LCMS (5-95 AB, ESI): t_{R} = 0.688, [M + H]⁺ = 826.5.

### Example 302: Synthesis of Compound 398

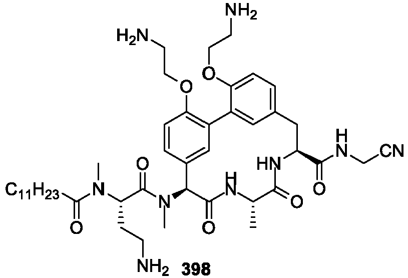

Compound 398 (formic acid salt) was prepared as a white solid utilizing the methods in Example 300. LCMS (5-95 AB, ESI): t_{R} = 0.753, [M + H]⁺ = 834.6.

### Example 303: Synthesis of Compound 399

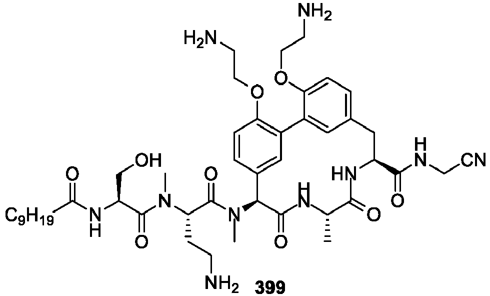

Compound 399 (formic acid salt) was prepared as a white solid utilizing the methods in Example 300. LCMS (5-95 AB, ESI): t_{R} = 0.585, [M + H]⁺ = 893.6

### Example 304: Synthesis of Compound 400

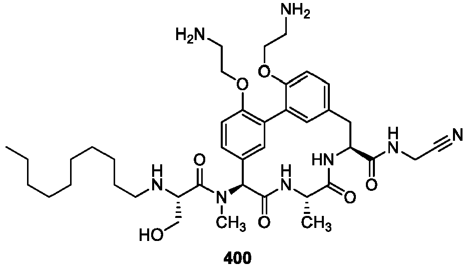

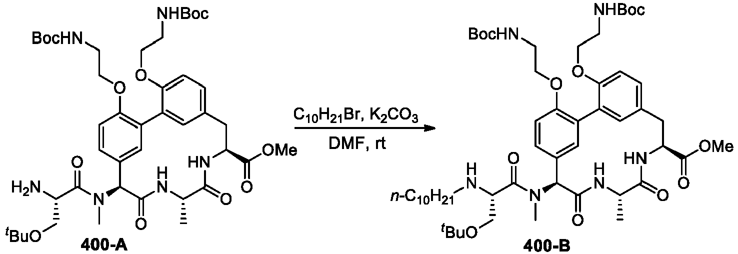

Compound 400-A is an intermediate in the preparation of Compound 231 (Example 137).

To a stirred solution of Compound 400-A (150 mg, 0.16 mmol) in DMF (5 mL) was added 1-bromodecane (697 mg, 3.15 mmol) and K₂CO₃ (435 mg, 3.15 mmol) and the mixture was stirred at room temperature for 2 d. The reaction mixture was then added with EtOAc (50 mL), which was washed with brine (50 mL x 2). The organic layer was dried over Na₂SO₄, concentrated and the residue was purified by prep-TLC to afford Compound 400-B (70 mg, 44.6% yield) as a white solid. LCMS (5-95 AB, ESI): t_{R} = 0.851, [M + H]⁺ = 997.5

Compound 400 (formic acid salt) was prepared as a white solid utilizing the same methods used in the preparation of Compound 101 (Example 7) starting from Compound 400-B. LCMS (5-95 AB, ESI): t_{R} = 0.597, [M + H]⁺ = 765.3; ¹H NMR (400 MHz, MeOH-*d*4) δ 7.31-7.28 (m, 2H), 7.20 (d, *J*=8.0 Hz, 1H), 7.12 (d, *J*=8.0 Hz, 1H), 6.91 (brs, 1H), 6.82 (brs, 1H), 6.39 (s, 1H), 4.80-4.75 (m, 1H), 4.27-4.15 (m, 6H), 4.21 (s, 2H), 4.05-3.95 (m, 1H), 3.85-3.77 (m, 1H), 3.25-3.16 (m, 7H), 2.93-2.80 (m, 1H), 2.89 (s, 3H), 1.73 (brs, 2H), 1.41-1.20 (m, 17H), 0.93 (t, *J*=6.0 Hz, 3H). LCMS (5-95 AB, ESI): tR = 0.597, [M + H]+ = 765.3; ¹H NMR (400 MHz, MeOH-d4) δ 7.31-7.28 (m, 2H), 7.20 (d, J=8.0 Hz, 1H), 7.12 (d, J=8.0 Hz, 1H), 6.91 (brs, 1H), 6.82 (brs, 1H), 6.39 (s, 1H), 4.80-4.75 (m, 1H), 4.27-4.15 (m, 6H), 4.21 (s, 2H), 4.05-3.95 (m, 1H), 3.85-3.77 (m, 1H), 3.25-3.16 (m, 7H), 2.93-2.80 (m, 1H), 2.89 (s, 3H), 1.73 (brs, 2H), 1.41-1.20 (m, 17H), 0.93 (t, J=6.0 Hz, 3H).

### Example 305: Synthesis of Compound 401

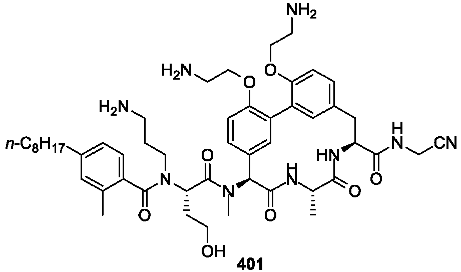

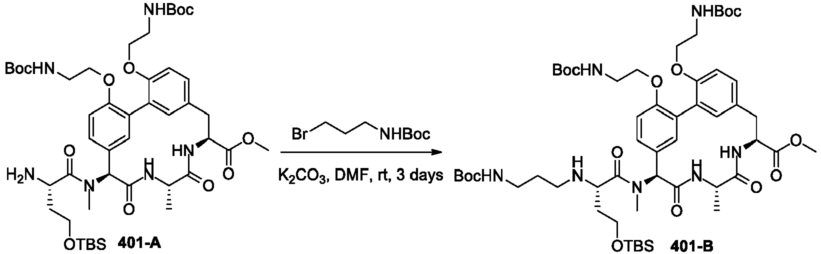

Compound 401-A is an intermediate in the synthesis of Compound 401 using the methods previously described.

To a stirred solution of Compound 401-A (100 mg, 0.11 mmol) in DMF (3 mL) was added tert-butyl (3-bromopropyl)carbamate (34 mg, 0.16 mmol), K₂CO₃ (37 mg, 0.27 mmol). The mixture was then stirred at room temperature for 72 h. The reaction mixture was added with EtOAc (30 mL), which was washed with brine (30 mL x 2). The organic layer was dried over MgSO₄, concentrated and the residue was purified by prep-TLC to give Compound 401-B (30 mg, 25.7% yield) as a white solid. LCMS (5-95 AB, ESI): t_{R} = 0.833, [M + H]⁺ = 1087.7

Compound 401 (formic acid salt) was prepared as a white solid utilizing the same methods used in the preparation of Compound 257 (Example 162). LCMS (5-95 AB, ESI): t_{R} = 0.737, [M + H]⁺ = 927.1.

### Example 306: Synthesis of Compound 402

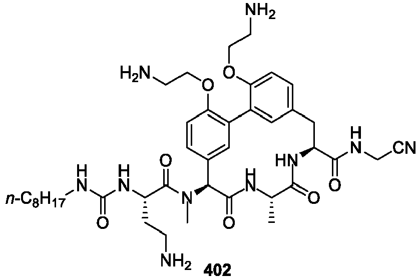

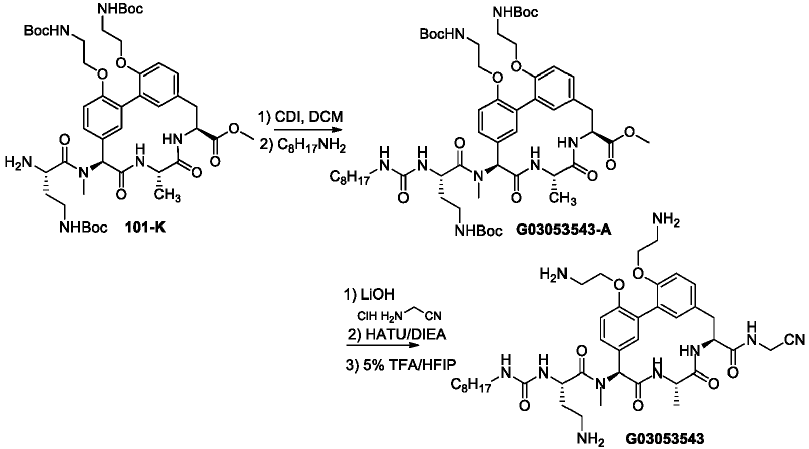

To a solution of Compound 101-K (100 mg, 0.20 mmol) in DCM (2 mL) was added CDI (20 mg, 0.20 mmol) at room temperature and the mixture was stirred overnight. To the mixture was then added n-C₈H₁₇NH₂ (42 mg, 0.33 mmol) at the same temperature and the resulting mixture was stirred for another 20 h at room temperature. The reaction mixture was diluted with DCM (50 mL), and then washed sequentially with saturated citric acid, saturated NaHCO₃ and brine (30 mL each). The organic layer was dried over Na₂SO₄, concentrated and the residue was purified on Pre-TLC to obtain Compound 402-A (85 mg, 72.9% yield) as a white solid. LCMS (5-95 AB, ESI): t_{R} = 1.072, [M + H]⁺ = 1070.0.

Starting from Compound 402-A, Compound 402 (formic acid salt) was prepared as a white solid utilizing the methods in Example 7 (Compound 101). LCMS (5-95 AB, ESI): t_{R} = 0.751, [M + H]⁺ = 793.9; ¹H NMR (400 MHz, MeOH-*d*₄) δ 8.50 (brs, 2H, HCOOH), 7.38-7.25 (m, 2H), 7.20-7.11 (m, 2H), 6.90 (brs, 1H), 6.83 (brs, 1H), 6.31 (s, 1H), 5.00-4.79 (m, 3H), 4.30-4.20 (m, 4H), 4.21 (s, 2H), 3.30-2.88 (m, 10H), 2.87 (s, 3H), 2.20-2.10 (m, 1H), 2.00-1.85 (m, 1H), 1.55-1.45 (m, 2H), 1.40-1.30 (m, 13H), 0.93 (t, *J*=6.8 Hz, 3H).

### Example 307: Synthesis of Compound 403

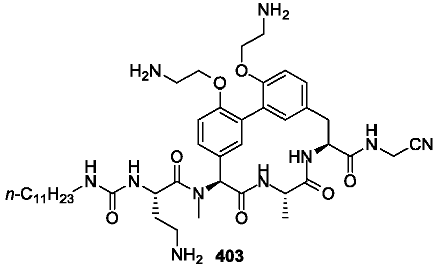

Compound 403 (formic acid salt) was prepared as a white solid utilizing the methods in Example 306 (Compound 402). LCMS (5-95 AB, ESI): t_{R} = 0.763, [M + H]⁺ = 835.5.

### Example 308: Synthesis of Compound 404

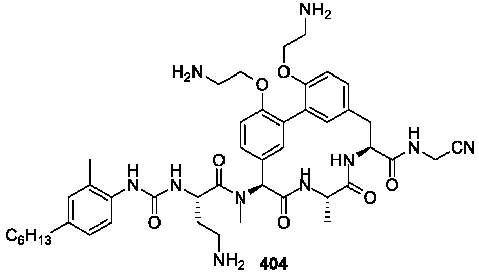

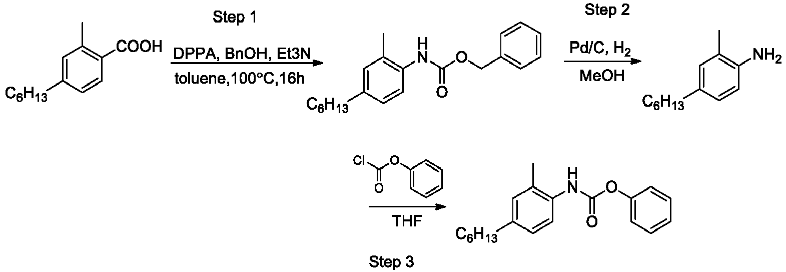

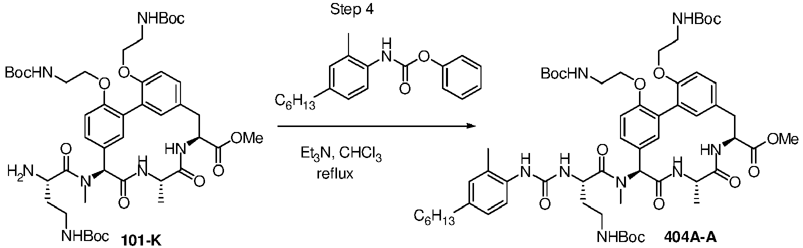

Step 1: A solution of 4-hexyl-2-methylbenzoic acid (300 mg, 1.4 mmol), diphenylphosphoryl azide (450 mg, 1.6 mmol), benzyl alcohol (442 mg, 4.1 mmol) and Et₃N (386 mg, 3.8 mmol) in toluene (10 mL) was stirred at 100 °C for 16 h. The reaction mixture was diluted with water (30 mL), which was extracted by EtOAc (30 mL x 2). The combined organic layers were washed with brine (50 mL x 2), dried over MgSO₄, concentrated and the residue was purified by silica gel flash column to give benzyl (4-hexyl-2-methylphenyl)carbamate (270 mg, 60.9% yield) as a white solid. LCMS (5-95 AB, ESI): t_{R} = 1.099, [M + H]⁺ = 326.0.

Step 2: The standard hydrogenation (Pd/C) procedure (General Method Hydrogenation) was applied to benzyl (4-hexyl-2-methylphenyl)carbamate (270 mg, 0.83 mmol) to afford 4-hexyl-2-methylaniline (125 mg, 79% yield) as a white solid.

Step 3: To a solution of 4-hexyl-2-methylaniline (30 mg, 0.16 mmol) in THF (1 mL) was added phenyl chloroformate (27 mg, 0.17 mmol) in portions and the mixture was stirred at room temperature for 1 h. The reaction was quenched with water (5 mL), the mixture was extracted by EtOAc (5 mL x 3). The combined organic layers were dried over Na₂SO₄, concentrated and the residue was purified by silica gel flash column to afford phenyl (4-hexyl-2-methylphenyl)carbamate (40 mg, 81.9% yield) as colorless oil. LCMS (5-95 AB, ESI): t_{R} = 0.960, [M + H]⁺ = 311.9

Step 4: To a solution of Compound 101-K (80 mg, 0.09 mmol) in CHCl₃ (3 mL) was added Et₃N (27 mg, 0.26 mmol) and phenyl (4-hexyl-2-methylphenyl)carbamate (35 mg, 0.11 mmol) and the mixture was heated to 80°C while stirring and stirred at the same temperature for 3 h. The volatiles were removed and the residue was purified by prep-TLC to give Compound 404A-A (90 mg, 90.9% yield) as an off-white solid. LCMS (5-95 AB, ESI): t_{R} = 0.983, [M + H]⁺ = 1132.5.

Starting from Compound 404A-A, Compound 404 (formic acid salt) was prepared as a white solid utilizing the methods in Example 7 (Compound 101). LCMS (5-95 AB, ESI): t_{R} = 0.627, [M + H]⁺ = 855.5; ¹H NMR (400 MHz, MeOH-*d*₄) δ 8.46 (brs, 2H, HCOOH), 7.35-7.27 (m, 2H), 7.24 (d, *J*=8.0 Hz, 1H), 7.15 (d, *J*=8.0 Hz, 1H), 7.09 (d, *J*=8.0 Hz, 1H), 7.03 (brs, 1H), 6.98 (d, *J*=8.0 Hz, 1H), 6.90 (brs, 1H), 6.81(brs, 1H), 6.31(s, 1H), 4.80-4.76 (m, 3H), 4.28-4.17 (m, 4H), 4.19 (s, 2H), 3.27-3.20 (m, 4H), 3.18-3.04 (m, 4H), 2.88 (s, 3H), 2.55 (t, *J*=7.2 Hz, 2H), 2.26-2.15 (m, 1H), 2.24 (s, 3H), 2.03-1.95 (m, 1H), 1.63-1.55 (m, 2H), 1.37-1.26 (m, 9H), 0.89 (t, *J*=6.4 Hz, 3H).

### Example 309: Synthesis of Compound 405

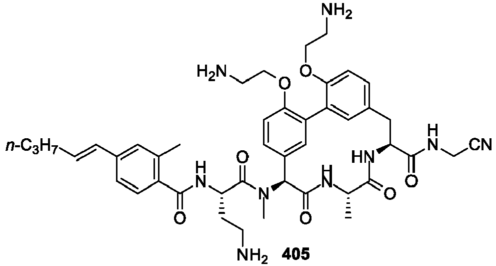

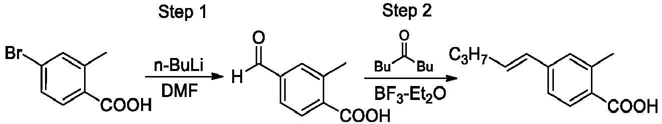

Step 1: To a solution of 4-bromo-2-methylbenzoic acid (10.7 g, 50 mmol) in THF (250 mL) at -78°C was added 2M n-BuLi in hexane (6.0 mL) dropwise and the mixture was stirred at the same temperature for 3 h. DMF (3.85 mL, 50 mmol) was then added to the mixture at -78°C and the resulting mixture was warmed up to room temperature slowly while stirring and stirred at the same temperature for 1.5 h. The reaction was quenched with 1N HCl (200 mL), which was extracted by EtOAc (200 mL x 3). The combined organic layers were dried over Na₂SO₄ and concentrated to give the crude, which was triturated with petroleum ether to afford 4-formyl-2-methylbenzoic acid (3.0 g, 36.7% yield) as an off-white solid.

Step 2: To a solution of 4-formyl-2-methylbenzoic acid (762 mg, 4.6 mmol), nonan-5-one (600 mg, 4.2 mmol) in DCM (10 mL) was added boron trifluoride diethyl etherate (1.07 mL, 8.44 mmol) and the mixture was stirred at 45°C for 16 h. DCM (30 mL) was added to the reaction mixture, which was then washed with brine (50 mL x 2). The organic layer was dried over Na₂SO₄, concentrated and the residue was purified by HPLC to afford (E)-2-methyl-4-(pent-1-en-1-yl)benzoic acid (400 mg, 46.4% yield) as an off-white solid. LCMS (10-80 AB 2 min, ESI): t_{R} = 1.221, [M + H]⁺ = 205.2.

Compound 405 (free base) was prepared as a white solid utilizing the methods in Example 7 (Compound 101). LCMS (5-95 AB, ESI): t_{R} = 0.700, [M + H]⁺ = 824.5.

### Example 310: Synthesis of Compound 406

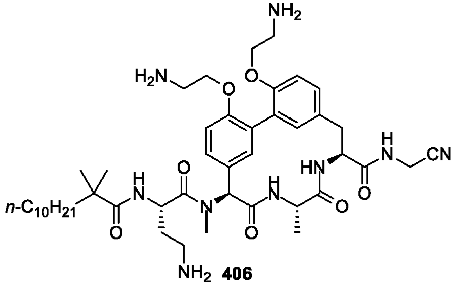

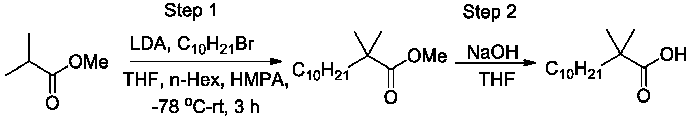

To a solution of methyl isobutyrate (1.0 g, 9.8 mmol) in THF (15 mL) was added 2M LDA in THF (5.9 mL, 11.8 mmol) dropwise at -78°C and the mixture was stirred for at the same temperature for 0.5 h. 1-bromodecane (2.6 g, 11.8 mmol) and HMPA (2.1 g, 11.8 mmol) was then added at -78°C and the resulting mixture was slowly warmed up to room temperature while stirring and stirred for another 3 h at room temperature. The reaction was quenched with saturated NH₄Cl solution (30 mL), which extracted with EtOAc (30 mL x 3). The combined organic layers were dried over Na₂SO₄, concentrated and the residue was purified by silica gel flash column to give methyl 2,2-dimethyldodecanoate (1.7 mg, 71.6% yield) as colorless oil. ¹H NMR (400 MHz, CDCl₃) δ 3.64 (s, 3H), 1.50-1.46 (m, 2H), 1.30-1.23 (m, 16H), 1.14 (s, 6H), 0.86 (t, *J*= 6.4 Hz, 3H).

Step 2: Ester hydrolysis procedure with NaOH (General Method NaOH) was applied to methyl 2,2-dimethyldodecanoate (500 mg, 0.45 mmol) to afford 2,2-dimethyldodecanoic acid (330 mg, 70.1% yield) as a light yellow solid.

Compound 406 (formic acid salt) was prepared as a white solid utilizing the methods in Example 7 (Compound 101). LCMS (5-95 AB, ESI): t_{R} = 0.609, [M + H]⁺ = 848.5

### Example 311: Synthesis of Compound 407

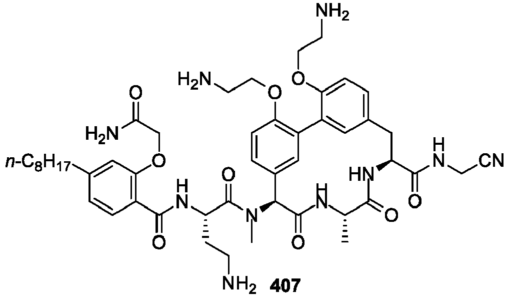

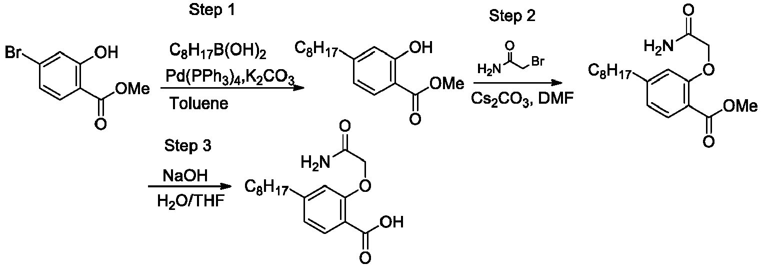

Step 1: A solution of methyl 4-bromo-2-hydroxybenzoate (600 mg, 2.8 mmol) and octylboronic acid (874 mg, 5.6 mmol), Pd(PPh₃)₄ (320 mg, 0.28 mmol) and K₂CO₃ (1.15 g, 8.3 mmol) in toluene (16 mL) was stirred at 100°C under N₂ for 16 h. The volatiles were removed and the residue was purified by silica gel flash column to give methyl 2-hydroxy-4-octylbenzoate (706 mg, 96.6% yield) as colorless oil.

Step 2: To a solution of methyl 2-hydroxy-4-octylbenzoate (300 mg, 1.13 mmol) in DMF (8 mL), 2-bromo acetamide (188 mg, 1.36 mmol) was added Cs₂CO₃ (740 mg, 2.27 mmol) and the mixture was stirred at room temperature for 16 h. The reaction mixture was diluted with EtOAc (50 mL), which was washed by brine (50 mL x 2). The organic layer was dried over Na₂SO₄, concentrated and the residue was purified by silica gel flash column to obtain methyl 2-(2-amino-2-oxoethoxy)-4-octylbenzoate (172 mg, 47.2% yield) as a white solid. LCMS (5-95 AB, ESI): t_{R} = 1.025, [M + H]⁺ = 322.0

Step 3: Typical ester hydrolysis (NaOH/THF) was applied to methyl 2-(2-amino-2-oxoethoxy)-4-octylbenzoate (70 mg, 0.22 mmol) to afford 2-(2-amino-2-oxoethoxy)-4-octylbenzoic acid (68 mg, 99% yield) as a white solid.

The methods in Example 7 (Compound 101) were followed to afford the Compound 407 (21.4 mg) as a white solid. LCMS (5-95 AB, ESI): t_{R} = 0.756, [M + H]⁺ = 927.5; ¹H NMR (400 MHz, MeOH-*d*₄) δ 7.81 (d, *J*=8.0 Hz, 1H), 7.32 (d, *J*=8.0 Hz, 1H), 7.23 (d, *J*=8.0 Hz, 1H), 7.15 (d, *J*=8.0 Hz, 1H), 7.09 (d, *J*=8.0 Hz, 1H), 6.97 (d, *J*=8.0 Hz, 1H), 6.93 (brs, 1H), 6.88 (brs, 1H), 6.81 (brs, 1H), 6.34 (s, 1H), 4.90-4.75 (m, 5H), 4.23-4.18 (m, 4H), 4.19 (s, 2H), 3.35-3.30 (m, 4H), 3.20-3.13 (m, 4H), 2.90 (s, 3H), 2.71-2.65 (m, 2H), 2.28-2.15 (m, 2H), 1.70-1.55 (m, 2H), 1.40-1.20 (m, 13H), 0.89 (t, *J*=6.0 Hz, 3H).

### Example 312: Synthesis of Compound 408

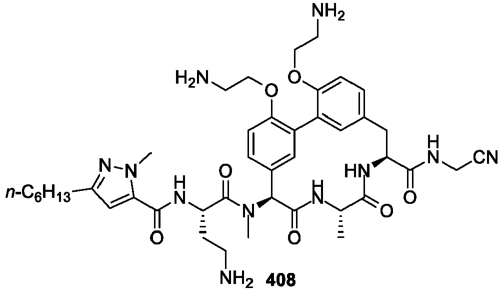

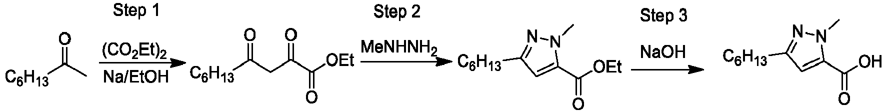

Step 1: Na (179 mg, 7.8 mmol) was added to EtOH (10 mL) while stirring in portions and the mixture was stirred at room temperature for 2 h, followed by the sequential addition of diethyl oxalate (1.14 g, 7.8 mmol) and octan-2-one (1.0 g, 7.8 mmol). The resulting mixture was stirred at room temperature for 16 h. The reaction mixture was added with EtOAc (50 mL), which was washed with brine (50 mL x 2). The organic layer was dried over Na₂SO₄, concentrated and the residue was purified on silica gel flash column to afford ethyl 2,4-dioxodecanoate as a light yellow oil (900 mg).

Step 2: A solution of ethyl 2,4-dioxodecanoate (700 mg, 3.1 mmol) and methyl hydrazine (155 mg, 3.4 mmol) in EtOH (10 mL) was stirred at 80°C for 3 h. The volatiles were removed and the residue was taken up in EtOAc (50 mL), which was washed with brine (30 mL x 2). The organic layer was dried over MgSO₄, concentrated and the residue was purified by silica gel flash column to afford ethyl 3-hexyl-1-methyl-1H-pyrazole-5-carboxylate (280 mg, 38.3% yield) as a yellow oil. ¹H NMR (400 MHz, CDCl₃) δ 6.61 (s, 1H), 4.31 (q, *J*=7.2 Hz, 2H), 4.12 (s, 3H), 2.61-2.54 (m, 2H), 1.66-1.56 (m, 2H), 1.34 (t, *J*=7.2 Hz, 3H), 1.33-1.25 (m, 6H), 0.89 (t, *J*=6.8 Hz, 3H).

Step 3: Typical ester hydrolysis (NaOH/THF) procedure was applied to ethyl 3-hexyl-1-methyl-1H-pyrazole-5-carboxylate (260, 1.1 mmol) to afford 3-hexyl-1-methyl-1H-pyrazole-5-carboxylic acid (220 mg, 96% yield) as a white solid.

The methods in Example 7 (Compound 101) were followed to afford Compound 408 (9.0 mg) as a white solid. LCMS (5-95 AB, ESI): t_{R} = 0.686, [M + H]⁺ = 830.5.

### Example 313: Synthesis of Compound 409

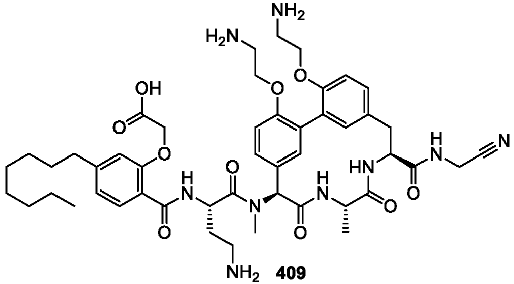

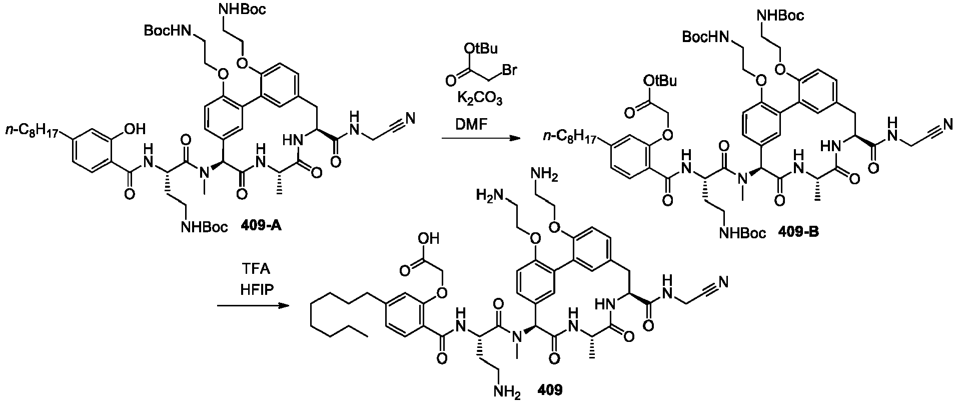

Compound 409-A is prepared during the synthesis of Compound 193 (Example 99) utilizing the methods in Example 112 (Compound 206). LCMS (Method 5-95AB): t_{R} = 1.017 min, [M + H]⁺ = 1171.0.

A mixture of Compound 409-A (30.0 mg, 0.03 mmol), potassium carbonate (10.6 mg, 0.08 mmol) and tert-butyl bromoacetate (15.0 mg, 0.08 mmol) in N,N-dimethylformamide (3 mL) was stirred at 15 °C for 2 h. The reaction was poured into water (3 mL) and extracted with EtOAc (10 mL). The organic extract was washed with water (10 mL x 2) and brine (10 mL), dried over MgSO₄ and concentrated. The residue was purified by prep-TLC (5% methanol in DCM, Rf = 0.4) to afford Compound 409-B (20 mg, 60.7% yield) as a white solid. LCMS (Method 5-95AB): t_{R} = 1.035 min, [M + H]⁺ = 1285.2.

Compound 409 (formic acid salt) was prepared as a white solid utilizing the methods in Example 7 (Compound 101). LCMS (Method 5-95AB): t_{R} = 0.627 min, [M + H]⁺ = 929.0.

### Example 314: Synthesis of Compound 410

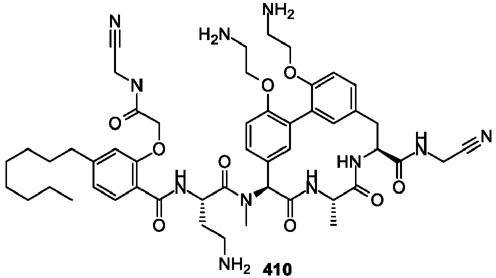

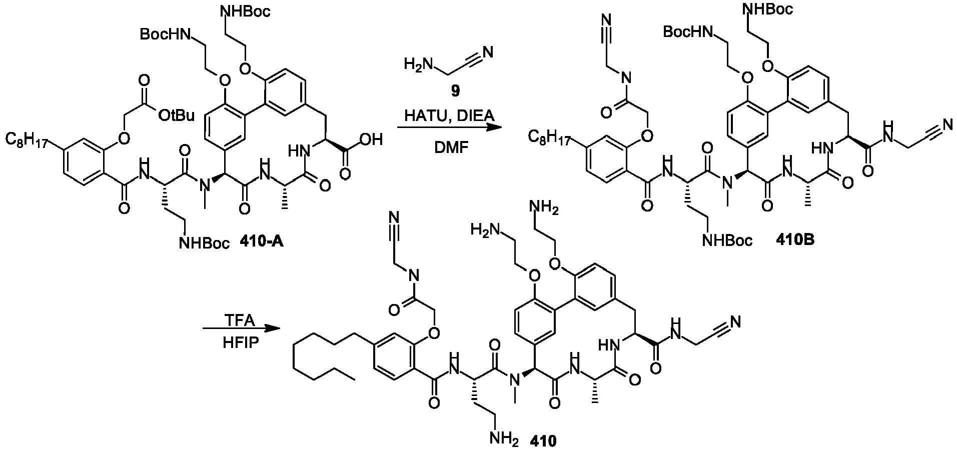

Compound 410-A is prepared using the previously described methods from 2-(2-(tert-butoxy)-2-oxoethoxy)-4-octylbenzoic acid. During coupling to the carboxylic acid utilizing General Method 4 (Example 5), bis-addition of the nitrile occurred to form Compound 410-B.

Compound 410 (formic acid salt) was prepared as a white solid from Compound 410-B utilizing the TFA hydrolysis conditions in Example 7. LCMS (Method 5-95AB): t_{R} = 0.617 min, [M + H]⁺ = 966.3.

### Example 315: Synthesis of Compound 411

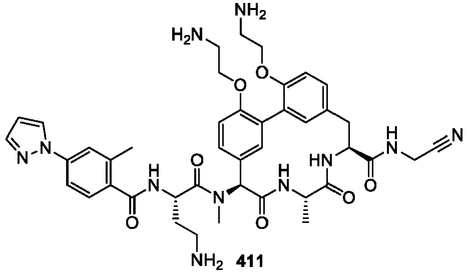

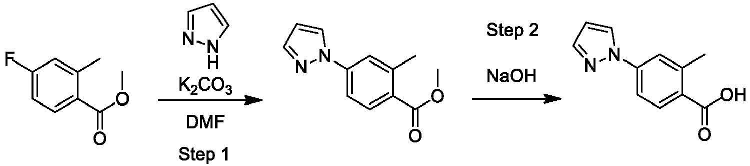

Step 1: A mixture of pyrazole (100 mg, 1.47 mmol), methyl 4-fluoro-2-methylbenzoate (123.51 mg, 0.73 mmol) and potassium carbonate (609.0 mg, 4.41 mmol) in N,N-dimethylformamide (5 mL) was stirred at 60 °C for 16 h. The reaction was poured into water (5 mL) and extracted with EtOAc (20 mL). The organic extract was washed with water (20 mL x 2) and brine (10 mL), dried over MgSO₄ and concentrated. The residue was purified by prep-TLC (33% ethyl acetate in petroleum ether, Rf = 0.3) to afford methyl 2-methyl-4-(1H-pyrazol-1-yl)benzoate (50 mg, 15.7% yield) as a white solid. LCMS (Method 5-95AB): t_{R} = 0.732 min, [M + H]⁺ = 216.9.

Step 2: Methyl 2-methyl-4-(1H-pyrazol-1-yl)benzoate was hydrolyzed as previously described (General Method NaOH) to give 2-methyl-4-(1H-pyrazol-1-yl)benzoic acid.

Compound 315 (formic acid salt) was prepared as a white solid utilizing the methods in Example 7 (Compound 101). LCMS (Method 0-60AB): t_{R} = 0.830 minI 2 min, [M + H]⁺ = 822.3.

### Example 316: Synthesis of Compound 412

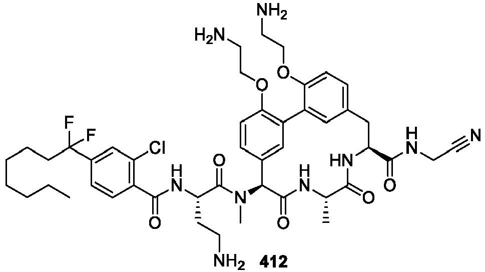

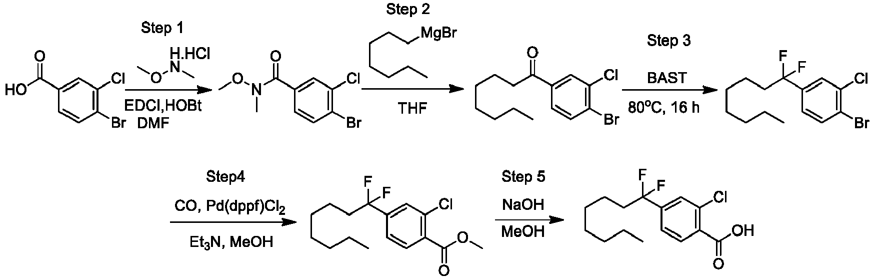

Step 1: A mixture of 1-(3-dimethylaminoprpyl)-3-ethylcarbodiimide hydrochloride (1.15 g, 6.01 mmol), N,N-diisopropylethylamine (2.48 mL, 15.03 mmol), 1-hydroxybenzotriazole (0.81 g, 6.01 mmol), 4-bromo-3-chlorobenzoic acid (1.18 g, 5.01 mmol) and N,O-dimethylhydroxylamine hydrochloride (0.98 g, 10.02 mmol) in N,N-dimethylformamide (10 mL) was stirred at 20 °C for 16 h. The reaction was quenched with water (20 mL), and extracted with EtOAc (25 mL x 2). The combined organic layers were concentrated and purified by column (30% EtOAc in petroleum ether, Rf = 0.3) to give 4-bromo-3-chloro-N-methoxy-N-methyl-benzamide (1.38 g, 98.9% yield) as a yellow oil.

Step 2: To a solution of heptylmagnesium bromide (2.37 mL, 2.37 mmol) in tetrahydrofuran (3 mL) was added 4-bromo-3-chloro-N-methoxy-N-methyl-benzamide (300.0 mg, 1.08 mmol) under N₂ at 0 °C and stirred at room temperature for 16 h. The reaction mixture was quenched with aq. NH₄Cl (1 mL) and extracted with EtOAc (10 mL x 3). The combined organic layers were dried over anhydrous sodium sulfate, and concentrated in vacuo. The residue was purified on silica gel column eluted with ethyl acetate/petroleum ether (1:10) to afford 1-(4-bromo-3-chloro-phenyl)octan-1-one (310 mg, 90.6% yield) as a colorless oil. ¹H NMR (400 MHz, CDCl₃): δ 8.00 (d, J = 1.6 Hz, 1H), 7.75 - 7.60 (m, 2H), 2.90 (t, J = 7.6 Hz, 2H), 1.70 (quint, J = 7.3 Hz, 2H), 1.45 - 1.20 (m, 8H), 0.87 (t, J = 7.0 Hz, 3H).

Step 3: A mixture of 1-(4-bromo-3-chloro-phenyl)octan-1-one (150.0 mg, 0.47 mmol) in deoxo-fluor(R) (2.14 mL, 11.81 mmol) was stirred at 80 °C for 16 h. The mixture was quenched with aq. Na₂S₂O₃ (3 mL) and aq. NaHCO₃ (3 mL) at 0°C and extracted with petroleum ether (10 mL x 3). The combined organic layers were washed with brine, dried over Na₂SO₄, filtered and concentrated. The residue was purified by prep-TLC (5% EtOAc in petroleum ether, Rf = 0.5) to afford 1-bromo-2-chloro-4-(1,1-difluorooctyl)benzene (54 mg, 33.7% yield) as a colorless oil.

Step 4: A solution of 1-bromo-2-chloro-4-(1,1-difluorooctyl)benzene (50.0 mg, 0.15 mmol) and triethylamine (44.7 mg, 0.44 mmol) in methanol (10 mL) was treated with 1,1'-bis(diphenylphosphino)ferrocene palladium dichloride (10.8 mg, 0.01 mmol). The resulting mixture was stirred at 80 °C for 16 hours under CO (45 psi). The reaction mixture was concentrated and the residue was purified by silica gel column (10% ethyl acetate in petroleum) to afford methyl 2-chloro-4-(1,1-difluorooctyl)benzoate (30 mg, 63.9% yield) as a colorless oil.

Step 5: Methyl 2-chloro-4-(1,1-difluorooctyl)benzoate (30.0 mg, 0.09 mmol) was hydrolyzed as previously described (General Method NaOH) to give crude 2-chloro-4-(1,1-difluorooctyl)benzoic acid (25 mg, 87.2% yield) as a white solid.

Compound 412 (formic acid salt) was prepared as a white solid utilizing the methods in Example 7 (Compound 101). LCMS (Method 5-95AB): t_{R} = 0.664 min, [M+Na]⁺ = 946.4.

### Example 317: Synthesis of Compound 413

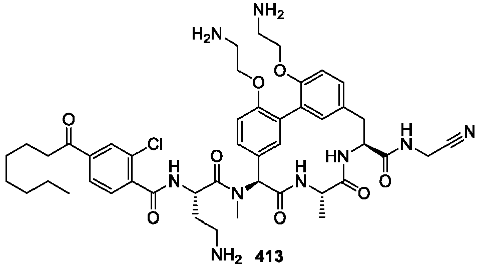

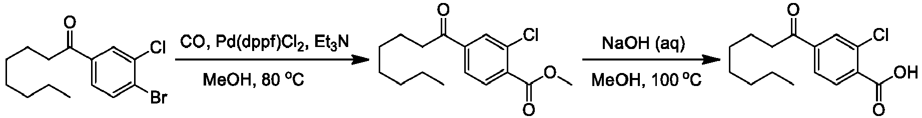

1-(4-Bromo-3-chloro-phenyl)octan-1-one has been described in Example 316. Using the methods in Example 316 (carbonylation followed by NaOH ester hydrolysis with General Method NaOH), 2-chloro-4-octanoylbenzoic acid was synthesized.

Compound 413 (formic acid salt) was prepared as a white solid utilizing the methods in Example 7 (Compound 101). LCMS (Method 5-95AB): t_{R} = 0.745 min, [M+H]⁺ = 902.7.

### Example 318: Synthesis of Compound 414

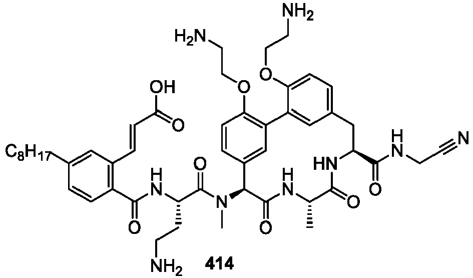

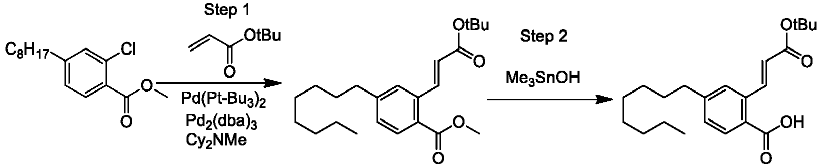

Step 1: To a solution of methyl 2-chloro-4-octyl-benzoate (350.0 mg, 1.24 mmol) in 1,4-dioxane (2 mL) were added t-butyl acrylate (174.5 mg, 1.36 mmol), bis(tri-t-butylphosphine)palladium(0) (9.5 mg, 0.02 mmol), tris(dibenzylideneacetone)dipalladium(0) (34.0 mg, 0.04 mmol) and N,N-dicyclohexylmethylamine (265.9 mg, 1.36 mmol). The mixture was stirred at 25 °C for 16 h under N₂ and filtered. The filtrate was diluted with water (10 mL) and extracted with EtOAc (10 mL x 3). The combined organic layers were dried over Na₂SO₄, filtered and concentrated. The residue was purified by prep-TLC (10% EtOAc in petroleum ether, Rf = 0.7) to afford methyl 2-[(E)-3-tert-butoxy-3-oxo-prop-1-enyl]-4-octyl-benzoate (290 mg, 62.6% yield) as a yellow oil. ¹H NMR (400 MHz, CDCl₃): δ 8.35 (d, J = 16.0 Hz, 1H), 7.86 (d, J = 8.0 Hz, 1H), 7.39 (s, 1H), 7.22 (d, J = 8.0 Hz, 1H), 6.23 (d, J = 15.9 Hz, 1H), 3.91 (s, 3H), 2.64 (t, J = 7.8 Hz, 2H), 1.65-1.55 (m, 2H), 1.54 (s, 9H), 1.33 - 1.22 (m, 10H), 0.88 (t, J = 6.6 Hz, 3H).

Step 2: To a solution of methyl 2-[(E)-3-tert-butoxy-3-oxo-prop-1-enyl]-4-octyl-benzoate (290.0 mg, 0.77 mmol) in 1,2-dichloroethane (2 mL) was added trimethyltin hydroxide (1400.2 mg, 7.74 mmol) and the mixture was stirred at 80 °C for 16 h. LCMS (5-95AB/1.5min): t_{R} = 1.158 min, [M+Na]+383.1 showed 36% of DP and 51% of SM. The mixture was diluted with 0.1N KHSO₄ (5 mL) and extracted with EtOAc (10 mL x 3). The combined organic layers were washed with brine, dried over Na₂SO₄, filtered and concentrated. The residue was purified by prep-TLC (50% EtOAc in petroleum ether, Rf = 0.2) to afford 2-[(E)-3-tert-butoxy-3-oxo-prop-1-enyl]-4-octyl-benzoic acid (80 mg, 28.7% yield) as a yellow solid.

Compound 414 (formic acid salt) was prepared utilizing the methods previously described. LCMS (Method 5-95AB): t_{R} = 0.760 min, [M+H]⁺ = 924.5.

### Example 319: Synthesis of Compound 415

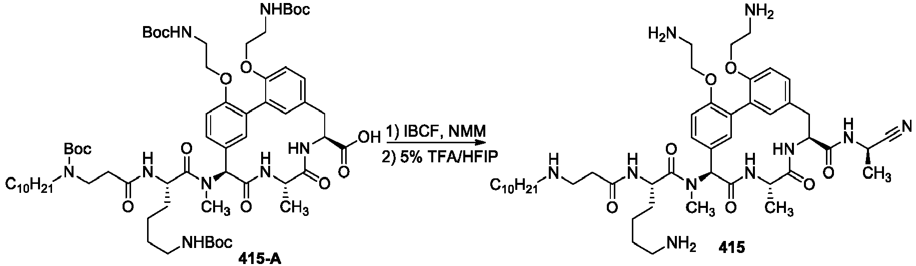

Compound 415-A is prepared using methods previously described.

Compound 415-A (57.6 mg, 0.05 mmol), was dissolved in anhydrous THF (2 mL) and cooled the reaction mixture to 0 °C in ice bath. Isobutyl chloroformate (10 µL, 0.075 mmol) followed by N-methyl morpholine (16 µL, 0.15 mmol) was added under N₂ atm. The reaction mixture was stirred for 30 min. After the starting material was consumed (monitored by TLC) (R)-2-aminopropanenitrile (7 mg, 0.1 mmol) in THF (1.0 mL) was added and the reaction mixture was stirred for 4h. After completion of the reaction (monitored by LCMS) the reaction mixture was quenched with saturated NH₄Cl solution (1.0 mL). The reaction mixture was diluted with brine (2 mL) and extracted with EtOAc and the combined organic layers washed with brine. The organic layer was dried over anhydrous Na₂SO₄, filtered and the solvent was removed under vacuum.

The residue was subjected for global Boc de-protection (TFA/HFIP) procedure (Example 7) was followed to afford the title compound (5.0 mg) as a white solid. LCMS (Method 5-95 AB, ESI): t_{R} = 0.718, [M + H]⁺ = 891.6; ¹H NMR (400 MHz, MeOH-*d*₄) δ 8.52 (brs, 4H), 7.27-7.22 (m, 2H), 7.17 (d, *J*=8.0 Hz, 1H), 7.09 (d, *J*=8.0 Hz, 1H), 6.87 (brs, 1H), 6.83(brs, 1H), 6.34 (s, 1H), 4.80-4.76 (m, 3H), 4.27-4.22 (m, 5H), 3.27-3.23 (m, 6H), 3.16-3.12 (m, 2H), 3.02 (t, *J*=7.6 Hz, 2H), 2.94 (t, J=7.6 Hz, 2H), 2.88 (s, 2H), 2.74 (brs, 2H), 1.87(brs, 2H) 1.71(m, 6H), 1.54 (d, *J*=8.0 Hz, 3H), 1.38-1.31(m, 18H), 0.91-0.88(m, 3H).

### Example 320: Synthesis of Compound 416

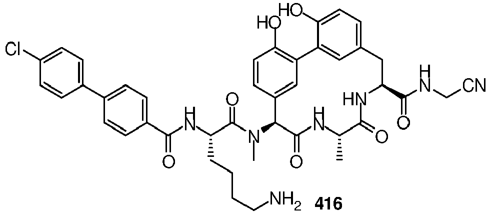

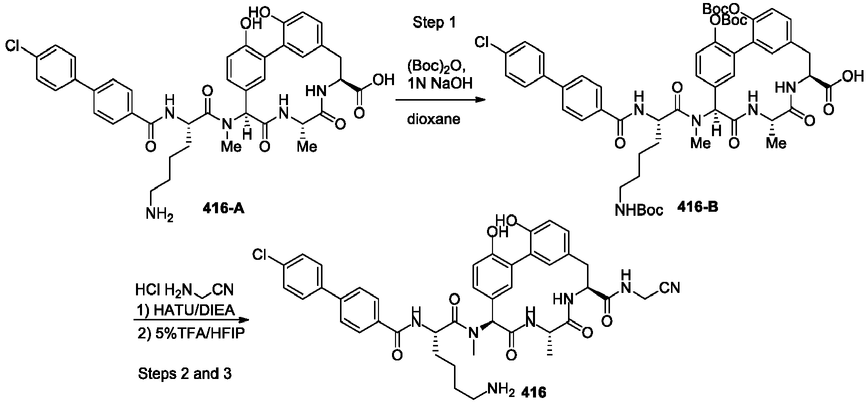

Step 1: To a solution of Compound 416-A (500 mg, 0.5 mmol) in dioxane (20 mL) was added 1M NaOH (10 mL, 10 mmol) and (Boc)₂O (1.2 mL, 5 mmol). The reaction mixture was stirred at room temperature overnight. The dioxane was removed under reduced pressure and the mixture was acidified with 1M HCl. The resultant white pasty material was dried to afford Compound 416-B (507 mg, 96%). MS (ESI) for (C₅₅H₆₆ClN₅O₁₄): *m*/*z* 1056 (M + H)⁺.

Steps 2 and 3: 416 (formic acid salt) was prepared utilizing the methods previously described. LCMS (5-95 AB, ESI): t_{R} = 0.658, [M + H]⁺ = 794.0; ¹H NMR (400 MHz, MeOH-*d*₄) δ 8.50 (brs, 1H), 7.75 (d, *J*= 8.0 Hz, 2H), 7.60 (d, *J*=8.0 Hz, 2H), 7.47 (d, *J*=8.0 Hz, 2H), 7.39 (d, *J*=8.4 Hz, 2H), 7.19 (d, *J*=8.0 Hz, 1 H), 6.94 (d, *J*=8.0 Hz, 1 H), 6.91 (brs, 1H), 6.90 (brs, 1H), 6.82 (d, *J*=8.0 Hz, 1H), 6.72 (brs, 2H), 4.95-4.91 (m, 1H), 4.79-4.72 (m, 1H), 4.58-4.50 (m, 1H), 4.18 (s, 2H), 3.10-2.80 (m, 4H), 2.94 (s, 3 H), 2.00-1.90 (m, 2H), 1.65-1.45 (m, 4H), 1.37 (d, *J*=6.8 Hz, 3 H).

### Example 321: Synthesis of Compound 417

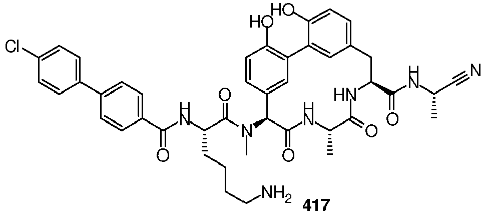

Compound 417 (formic acid salt) was prepared utilizing the methods previously described from (S)-2-aminopropanenitrile. LCMS (5-95 AB, ESI): t_{R} = 0.790, [M + H]⁺ = 808.7.

### Example 322: Synthesis of Compound 418

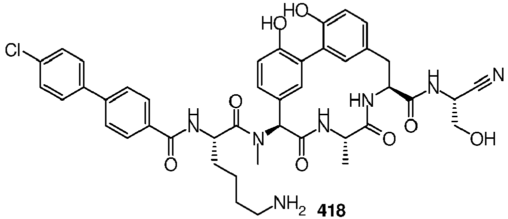

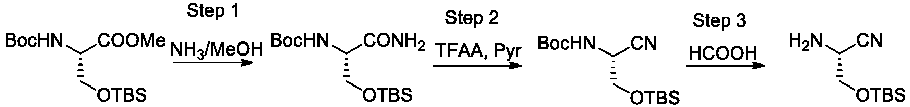

Step 1: (S)-methyl2-((tert-butoxycarbonyl)amino)-3-((tertbutyldimethylsilyl)oxy)propanoate (1.0 g, 3 mmol) was added to a solution saturated NH₃ in MeOH (50 mL) and the mixture was stirred at the sealed flask for 18 h. The volatiles were removed to give (S)-tert-butyl (1-amino-3-((tert-butyldimethylsilyl)oxy)-1-oxopropan-2-yl)carbamate (950 mg, 99.5% yield) as a white solid. ¹H NMR (400 MHz, DMSO-*d*6) δ 7.35 (brs, 1H), 7.16 (brs, 1H), 6.46 (d, *J*=8.4 Hz, 1H), 4.02-4.06 (m, 1H), 3.76-3.63 (m, 2H), 1.42(s, 9H), 0.88(s, 9H), 0.06 (s, 6H).

Steps 2 and 3: (S)-tert-Butyl (1-amino-3-((tert-butyldimethylsilyl)oxy)-1-oxopropan-2-yl)carbamate was subject to the same dehydration and hydrolysis conditions as in Example 42 (Compound 136).

Compound 418 (formic acid salt) was prepared utilizing the methods previously described. LCMS (5-95 AB, ESI): t_{R} = 0.793, [M + H]⁺ = 824.5; ¹H NMR (400 MHz, MeOH-*d*₄) δ 8.51(brs, 1H), 7.87 (d, *J*=8.0 Hz, 2H), 7.69 (d, *J*=8.0 Hz, 2H), 7.59 (d, *J*=8.0 Hz, 2H), 7.45 (d, *J*=8.0 Hz, 2H), 7.12 (d, *J*=8.0 Hz, 1H), 7.05 (d, *J*=8.0 Hz, 1H), 6.99-6.94 (m, 2H), 6.90-6.80 (m, 2H), 6.57 (s, 1H), 5.05-5.01 (m, 1H), 4.85-4.79 (m, 2H), 4.70-4.55 (m, 1H), 3.80-3.70 (m, 2H), 3.09-2.90 (m, 2H), 2.95 (m, 3H), 2.00-1.93 (m, 3H), 1.75-1.58 (m, 5H), 1.36 (d, *J*=6.8 Hz, 3H).

### Example 323: Synthesis of Compound 419

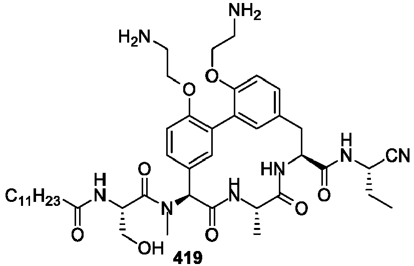

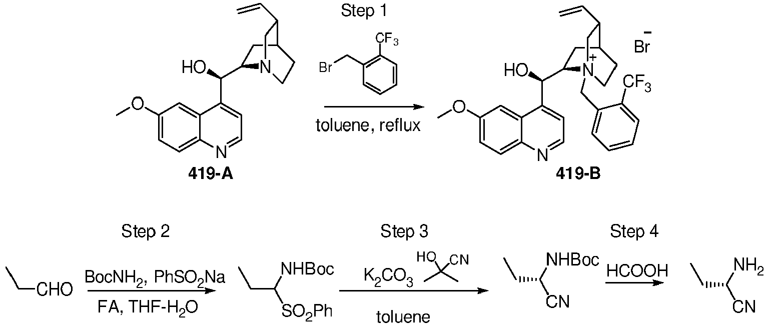

Step 1: A mixture of Compound 419-A (2.6 g, 8.0 mmol) and 1-(bromomethyl)-2-(trifluoromethyl)benzene (2.5 g, 10.4 mmol) in toluene (24 mL) was stirred at 100°C for 14h. After cooling to room temperature, the precipitated solid was collected, washed with pentane, which was further purified by HPLC to afford Compound 419-B (1.2 g, 27% yield) as a yellow solid.

To a solution of *t*-butyl carbamate (4.0 g, 34 mmol) in THF (14 mL) was added sodium benzenesulfinate (5.6 g, 34 mmol), propionaldehyde (2.18 g, 37.6 mmol) and formic acid (9.5 g, 205 mmol) sequentially and the mixture was stirred at room temperature for 18 h. The resulting precipitate was filtered, which was crystallized under Hex/EtOAc=4:1 to give tert-butyl (1-(phenylsulfonyl)propyl)carbamate (5.0 g, 48.9% yield) as a white solid. LCMS (5-95 AB, ESI): RT = 0.816, M+Na⁺ = 321.9

To a solution of tert-butyl (1-(phenylsulfonyl)propyl)carbamate (3.0 g, 10.0 mmol) in toluene (100 mL) was added Compound **419-B** (565 mg, 1.0 mmol) and acetone cyanohydrin (1.7 g, 20.0 mmol). After the above solution was cooled to -20°C, K₂CO₃ (6.9 g, 50.0 mmol) in H₂O (7.0 mL) was added in one portion and the resulting mixture was then vigorously stirred at the same temperature for 20 min, followed by gradually warming up to room temperature while stirring and further stirring for 48 h. The mixture was quenched by 5% NaHCO₃ solution (60 mL), which was extracted with EtOAc (100 mL x 2). The combined organic layers were washed with brine (100 mL x 3), dried over Na₂SO₄, concentrated and the residue was purified by silica gel column to afford (S)-tert-butyl (1-cyanopropyl)carbamate (1.4 g, 76% yield) as a light yellow solid. ¹H NMR (CDCl₃, 400 MHz) δ 4.87 (brs, 1H), 4.50 (brs, 1H), 1.86-1.74 (m, 2H), 1.44 (s, 9H), 1.08 (t, *J*=7.6 Hz, 3H).

Starting from (S)-tert-butyl (1-cyanopropyl)carbamate, the Boc removal (FA) procedure using methods previously described followed to afford (S)-2-aminobutanenitrile (70 mg, 67% yield) as yellow oil.

Compound 419 (formic acid salt) was prepared utilizing the methods previously described. LCMS (5-95 AB, ESI): RT = 0.802, M+H⁺ = 835.5.

### Example 324: Synthesis of Compound 420

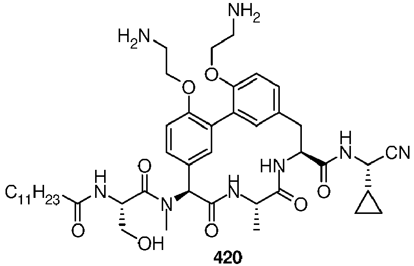

Compound 420 (formic acid salt) was prepared utilizing the methods in Example 323 (Compound 419). LCMS (5-95 AB, ESI): RT = 0.799, M+H⁺ = 847.5.

### Example 325: Synthesis of Compound 421

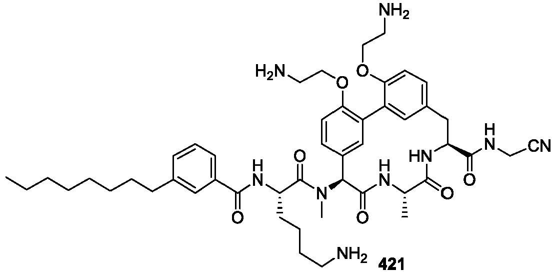

Compound 421 (formic acid salt) was prepared utilizing the methods in Example 90 (Compound 184). LC-MS: m/z = 882 [M + H]⁺.

### Example 326: Synthesis of Compound 422

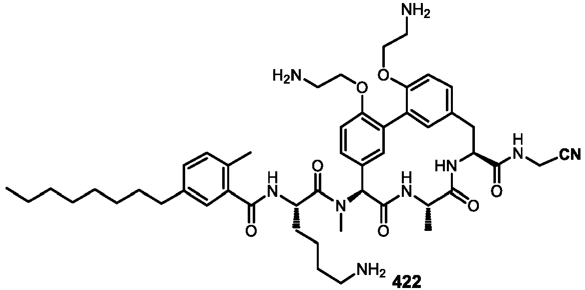

Compound 422 (formic acid salt) was prepared utilizing the methods in Example 90 (Compound 184). LC-MS: m/z = 896 [M + H]⁺.

### Example 327: Synthesis of Compound 423

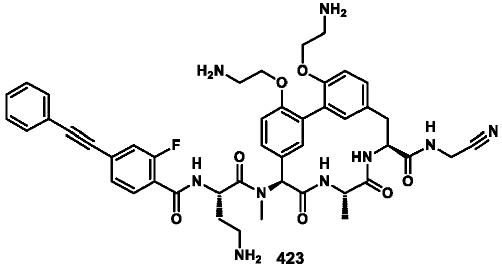

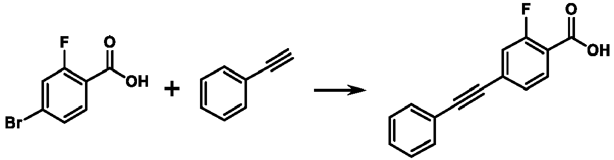

A mixture of 4-bromo-2-fluoro-benzoic acid (250 mg, 1.14 mmol), bis(triphenylphosphine)palladium(II) dichloride (120.18 mg, 0.17 mmol), and cuprous iodide (32.61 mg, 0.17 mmol) in TEA (8 mL) was degassed with N₂. Ethynylbenzene (349.74 mg, 3.42 mmol) was added, capped in a glass vial, heated in oil bath at 60°C overnight. It was diluted with sat NH₄Cl, washed with iPrOAc (2x). The aqueous layer was acidified with 1N HCl, extracted with iPrOAc (2 x 30 mL), dried over MgSO₄, filtered, concentrated in vacuo, dried under high vacuum to give 271 mg (99%) of 2-fluoro-4-(2-phenylethynyl)benzoic acid as brown solid. LC-MS: m/z = 241 [M + H]⁺.

Compound 423 (formic acid salt) was prepared utilizing the methods in Example 7 (Compound 101). LC-MS: m/z = 860 [M + H]⁺. ¹H NMR (400 MHz, DMSO-*d*₆) δ 8.97 (d, *J*= 7.8 Hz, 1H), 8.89 (d, *J*= 7.4 Hz, 1H), 8.71 (t, *J*= 5.6 Hz, 1H), 8.40 (d, *J*= 9.0 Hz, 1H), 7.68 (t, *J* = 7.8 Hz, 1H), 7.62 - 7.57 (m, 2H), 7.53 - 7.45 (m, 5H), 7.23 (dd, *J* = 8.6, 2.2 Hz, 1H), 7.19 - 7.15 (m, 2H), 7.08 (d, *J* = 8.5 Hz, 1H), 6.73 (t, *J* = 2.9 Hz, 2H), 6.32 (s, 1H), 5.03 - 4.96 (m, 1H), 4.79 - 4.69 (m, 2H), 4.19 (d, *J*= 5.8 Hz, 2H), 4.15 - 4.06 (m, 4H), 3.20 (d, *J*= 15.8 Hz, 1H), 3.03 (dd, *J*= 9.6, 4.8 Hz, 5H), 2.93 (t, *J*= 8.0 Hz, 2H), 2.80 (s, 3H), 2.08 (d, *J*= 5.7 Hz, 1H), 2.02 - 1.94 (m, 1H), 1.21 (d, *J*= 6.7 Hz, 3H).

### Example 328: Synthesis of Compound 424

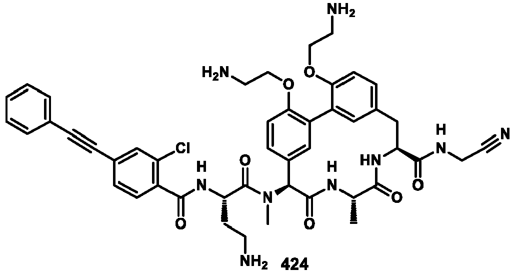

Compound 424 (formic acid salt) was prepared utilizing the methods in Example 327 (Compound 423). ¹H NMR (400 MHz, DMSO-*d*₆) δ 9.10 (d, *J*= 7.9 Hz, 1H), 8.97 (d, *J*= 7.8 Hz, 1H), 8.70 (t, *J* = 5.6 Hz, 1H), 8.39 (d, *J* = 9.0 Hz, 1H), 7.75 (d, *J* = 1.5 Hz, 1H), 7.64 - 7.58 (m, 3H), 7.51 - 7.46 (m, 4H), 7.23 (dd, *J* = 8.8, 2.3 Hz, 1H), 7.16 (d, *J* = 1.7 Hz, 2H), 7.08 (d, *J* = 8.5 Hz, 1H), 6.76 - 6.72 (m, 2H), 6.33 (s, 1H), 4.99 (q, *J* = 7.4 Hz, 1H), 4.79 - 4.68 (m, 2H), 4.18 (d, *J* = 5.9 Hz, 2H), 4.15 - 4.06 (m, 4H), 3.20 (d, *J* = 16.8 Hz, 1H), 3.00 (h, *J* = 11.8, 9.8 Hz, 9H), 2.83 (s, 3H), 2.12 - 1.94 (m, 2H), 1.20 (d, *J* = 6.7 Hz, 3H).

### Example 329: Synthesis of Compound 425

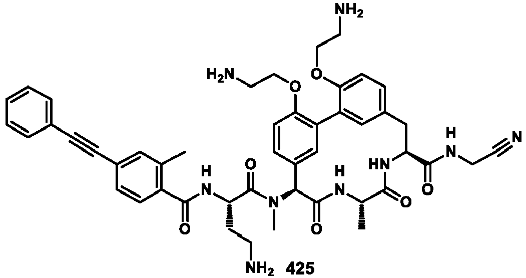

Compound 425 (formic acid salt) was prepared utilizing the methods in Example 327 (Compound 423). ¹H NMR (400 MHz, DMSO-*d*₆) δ 8.98 (d, *J* = 7.8 Hz, 1H), 8.85 (d, *J* = 7.6 Hz, 1H), 8.71 (t, *J* = 5.6 Hz, 1H), 8.39 (d, *J* = 9.2 Hz, 1H), 7.60 - 7.55 (m, 2H), 7.51 - 7.38 (m, 6H), 7.22 (dd, *J* = 8.6, 2.3 Hz, 1H), 7.17 (d, *J* = 2.0 Hz, 2H), 7.08 (d, *J* = 8.5 Hz, 1H), 6.74 (dd, *J* = 7.0, 2.1 Hz, 2H), 6.35 (s, 1H), 4.95 (q, *J* = 7.7 Hz, 1H), 4.79 - 4.68 (m, 2H), 4.18 (d, *J* = 5.7 Hz, 2H), 4.09 (dd, *J* = 11.4, 5.7 Hz, 4H), 3.19 (d, *J* = 16.7 Hz, 1H), 3.05 - 2.88 (m, 9H), 2.83 (s, 3H), 2.38 (s, 3H), 2.10 - 1.93 (m, 2H), 1.20 (d, *J* = 6.7 Hz, 3H).

### Example 330: Synthesis of Compound 426

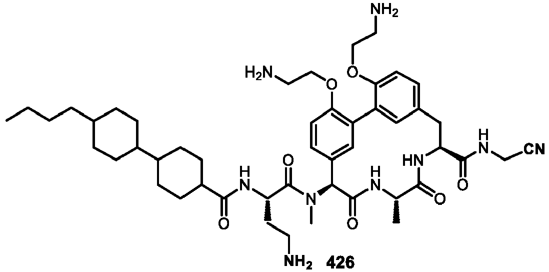

Compound 426 (formic acid salt) was prepared utilizing the methods previously described. LC-MS: m/z = 886 [M + H]⁺.

### Example 331: Synthesis of Compound 427

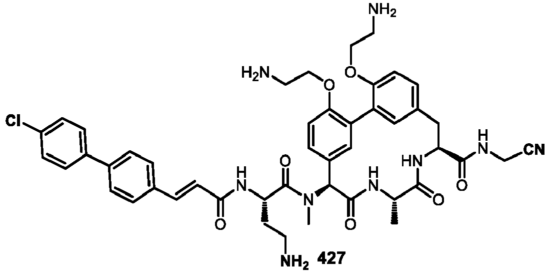

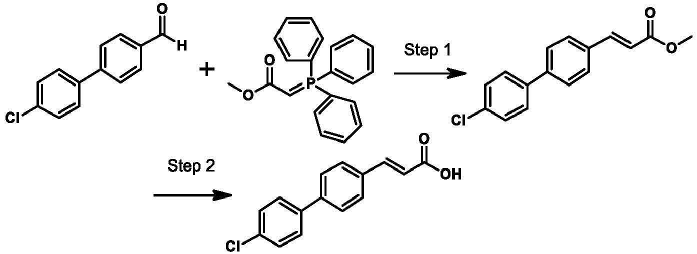

Step 1: A mixture of 4-(4-chlorophenyl)benzaldehyde (1.0 g, 4.6 mmol) and methyl 2-(triphenyl-λ₅-phosphanylidene)acetate (2.0 g, 6.0 mmol) in acetonitrile (12 mL) was stirred at room temperature overnight. It was purified with ISCO, 24 g column, eluded with 0-30% iPrOAc/heptane to give 1.18 g (94%) of methyl (E)-3-(4'-chloro-[1,1'-biphenyl]-4-yl)acrylate as white solid. LC-MS: m/z = 273 [M + H]⁺.

Step 2: A mixture of methyl (E)-3-[4-(4-chlorophenyl)phenyl]prop-2-enoate (1.18 g, 4.33 mmol) and LiOH (327 mg, 13.0 mmol) in 1,4-dioxane (16 mL) and water (4 mL) was stirred at room temperature overnight. It was concentrated in vacuo, suspended in water, acidified with 1N HCl to pH-5. The white precipitate was collected by filtration, washed with water, dried to give 1.11 g (99%) of (E)-3-(4'-chloro-[1,1'-biphenyl]-4-yl)acrylic acid as off white solid. LC-MS: m/z = 259 [M + H]⁺.

Compound 427 (formic acid salt) was prepared utilizing the methods in Example 7 (Compound 101). LC-MS: m/z = 878 [M + H]⁺.

### Example 332: Synthesis of Compound 428

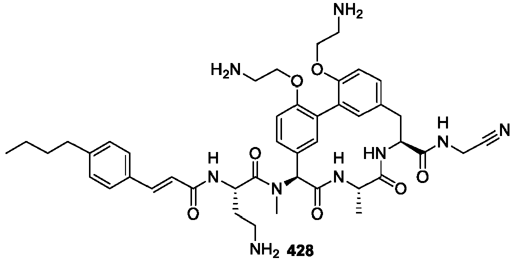

Compound 428 (formic acid salt) was prepared utilizing the methods in Example 331 (Compound 427). LC-MS: m/z = 824 [M + H]⁺.

### Example 333 Synthesis of Compound 429

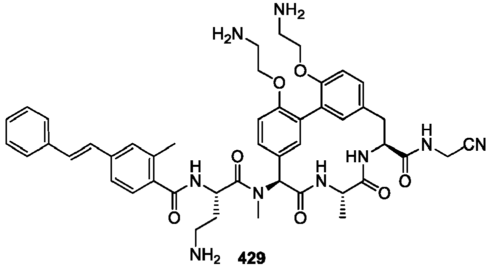

Compound 429 (formic acid salt) was prepared utilizing the methods in Example 47 (Compound 141) except (E)-styrylboronic acid is used in the Suzuki coupling. LCMS (Method 5-95 AB, ESI): t_{R} = 0.705 min, [M+H]⁺ = 858.9.

### Example 334: Synthesis of Compound 430

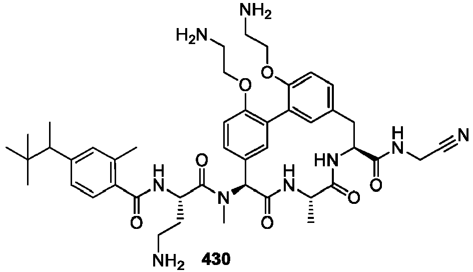

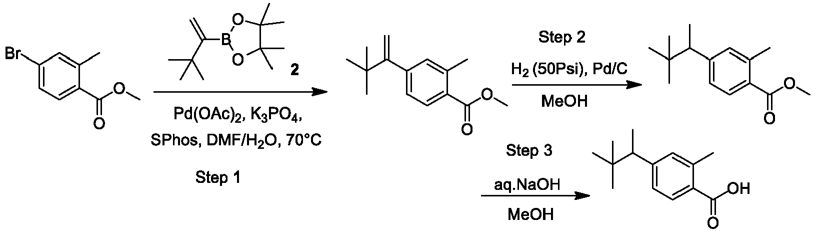

Step 1: To a mixture of palladium(II) acetate (29.4 mg, 0.13 mmol), 2-(3,3-dimethylbut-1-en-2-yl)-4,4,5,5-tetramethyl-1,3,2-dioxaborolane (328.6 mg, 1.57 mmol), potassium phosphate, tribasic (695.0 mg, 3.27 mmol) and SPhos (107.5 mg, 0.26 mmol) in DMF (3 mL) and water (0.3 mL) was added methyl 4-bromo-2-methylbenzoate (300 mg, 1.31 mmol). The resulting mixture was heated at 70 °C for 16 hours under nitrogen. The reaction mixture was diluted with water (10 mL) and extracted with ethyl acetate (20 mL x 3). The combined organic layers were dried over sodium sulfate and filtered. The filtrate was concentrated. The residue was purified by prep-TLC (10% ethyl acetate in petroleum ether) to afford methyl 4-(3,3-dimethylbut-1-en-2-yl)-2-methylbenzoate (246 mg, 80.9% yield) as a colorless oil.

Step 2: A mixture of methyl 4-(3,3-dimethylbut-1-en-2-yl)-2-methylbenzoate (246 mg, 1.06 mmol) and 10% palladium (56.3 mg, 0.05 mmol) on carbon in methanol (15 mL) was stirred at 15 °C under H₂ (50 psi) for 16 h. The catalyst was filtered off and the solvent was evaporated to give methyl 4-(3,3-dimethylbutan-2-yl)-2-methylbenzoate (220 mg, 88.7% yield) as a white solid.

Step 3: Methyl 4-(3,3-dimethylbutan-2-yl)-2-methylbenzoate (220.0 mg, 0.94 mmol) was hydrolyzed according to General Method NaOH to afford 4-(3,3-dimethylbutan-2-yl)-2-methylbenzoic acid (190 mg, 91.9% yield) as a white solid. ¹H NMR (400 MHz, CD₃OD): δ 7.90 (d, J = 8.0 Hz, 1H), 7.06 - 6.93 (m, 2H), 2.57 (s, 3H), 2.51 (q, J = 7.6 Hz, 1H), 1.19 (d, J = 7.2 Hz, 3H), 0.81 (s, 9H).

Compound 430 (formic acid salt) was prepared as a white solid utilizing the methods in Example 7 (Compound 101). LCMS (Method 5-95AB): t_{R} = 0.721 min, [M + H]⁺ = 840.5.

### Example 335: Synthesis of Compound 431

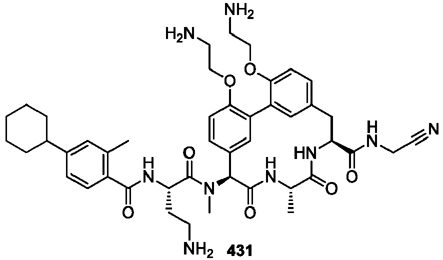

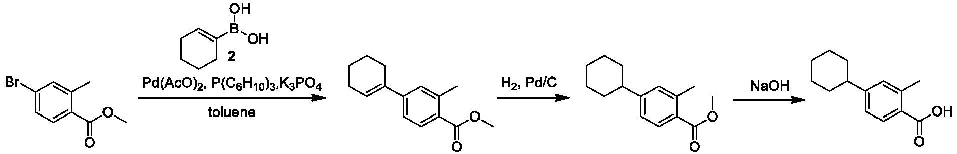

A mixture of methyl 4-bromo-2-methylbenzoate (50.0 mg, 0.22 mmol), 1,1'-bis(diphenylphosphino)ferrocene palladium dichloride (16.0 mg, 0.02 mmol), potassium carbonate (60.3 mg, 0.44 mmol) and 1-cyclohexen-1-yl boronic acid (41.2 mg, 0.33 mmol) in toluene (5 mL) was stirred at 120 °C for 2 h under N₂ and evaporated to dryness. The residue was taken up in EtOAc (20 mL), washed with water (20 mL x 2) and brine (20 mL), dried over MgSO₄ and concentrated. The residue was purified by flash column chromatography (5% EtOAc in petroleum ether, Rf = 0.7) to afford methyl 4-(cyclohexen-1-yl)-2-methylbenzoate (40 mg, 79.6% yield) as a colorless oil. ¹H NMR (400 MHz, CDCl₃) δ 7.88 (d, J = 8.8 Hz, 1H), 7.28 - 7.23 (m, 2H), 6.23-6.21 (m, 1H), 3.88 (s, 3H), 2.61 (s, 3H), 2.41 - 2.39 (m, 2H), 2.23-2.21 (m, 2H), 1.80-1.77 (m, 2H), 1.68-1.65 (m, 2H).

4-Cyclohexyl-2-methylbenzoic acid was prepared utilizing General Methods Hydrogenation and NaOH from methyl 4-(cyclohexen-1-yl)-2-methylbenzoate.

Compound 431 (formic acid salt) was prepared as a white solid utilizing the methods in Example 7 (Compound 101) from 4-cyclohexyl-2-methylbenzoic acid. LCMS (Method 5-95AB): t_{R} = 0.565 min, [M + H]⁺ = 838.3.

### Example 336: Synthesis of Compound 432

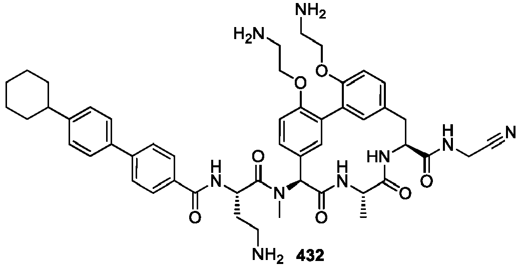

Compound 432 (formic acid salt) was prepared as a white solid utilizing the methods smilar to Example 47 (Compound 141) from methyl 4'-bromo-[1,1'-biphenyl]-4-carboxylate and cyclohex-1-en-1-ylboronic acid. LCMS (Method 5-95AB): t_{R} = 0.783 min, [M+H]⁺ = 900.5.

### Example 337: Synthesis of Compound 433

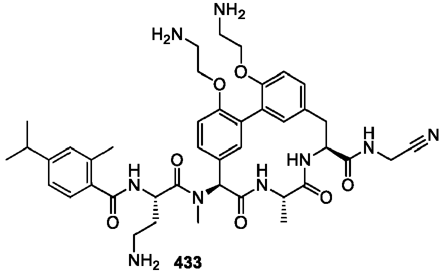

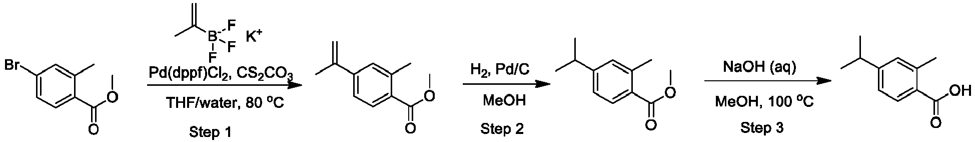

Step 1: A mixture of methyl 4-bromo-2-methylbenzoate (2.29 g, 10.0 mmol), cesium carbonate (6.51 g, 20.0 mmol), potassium isoprpenyltrifluoroborate (2.96 g, 20.0 mmol) and 1,1'-bis(diphenylphosphino)ferrocene palladium dichloride (0.73 g, 1 mmol) in tetrahydrofuran (100 mL) and water (5 mL) was stirred at 80 °C for 2 h. After cooling down, the reaction was evaporated to dryness. The residue was taken up in EtOAc (30 mL), washed with water (20 mL x 2) and brine (20 mL), dried over Na₂SO₄ and concentrated. The crude was purified by flash column chromatography (1% EtOAc in petroleum ether) to give methyl 2-methyl-4-(prop-1-en-2-yl)benzoate (1.8 g, 94.6% yield).

Steps 2 and 3: Methyl 2-methyl-4-(prop-1-en-2-yl)benzoate was hydrogenated and hydrolyzed as described previously to afford 4-isopropyl-2-methylbenzoic acid (340 mg, 96.5% yield) as a yellow solid. ¹H NMR (400 MHz, CDCl₃): δ 8.02 (d, *J* = 8.0 Hz, 1H), 7.20 - 7.10 (m, 2H), 3.00 - 2.85 (m, 1H), 2.66 (s, 3H), 1.28 (d, *J* = 7.2 Hz, 6H).

Compound 433 (formic acid salt) was prepared as a white solid utilizing the methods in Example 7 (Compound 101). LCMS (Method 5-95AB): t_{R} = 0.657 min, [M + H]⁺ = 798.5.

### Example 338: Synthesis of Compound 434

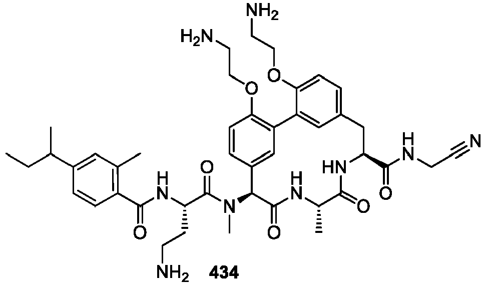

Compound 434 (formic acid salt) was prepared as a white solid utilizing the same methods in Example 433. LCMS (Method 5-95AB): t_{R} = 0.694 min, [M + H]⁺ = 812.5.

### Example 339: Synthesis of Compound 435

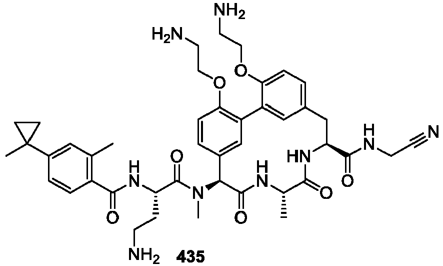

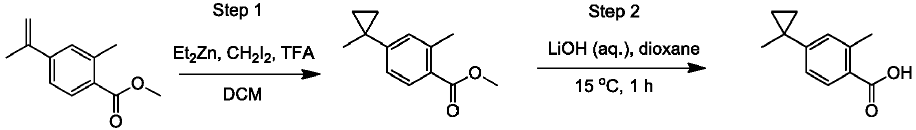

To a solution of methyl 2-methyl-4-(prop-1-en-2-yl)benzoate (1.0 g, 5.26 mmol) in dichloromethane (50 mL) was added diethylzinc (1 M in toluene; 157.7 mL, 157.7 mmol) at 0 °C, followed by a solution of diiodomethane (6.35 mL, 78.85 mmol) in dichloromethane (10 mL). The reaction was allowed to warm to 20 °C and stirred for 16 h, quenched with aq. NH₄Cl and extracted with EtOAc (50 mL x 3). The combined organic layers were washed with brine (50 mL) and dried over Na₂SO₄ and concentrated. The residue was purified by flash column chromatography (1% EtOAc in petroleum ether, Rf = 0.7) as a mixture of DP and STM, which was separated by prep-HPLC (acetonitrile 67-77% / 0.1% TFA in water) to give methyl 2-methyl-4-(1-methylcyclopropyl)benzoate (80 mg, 7.5% yield) as a colorless oil.

Methyl 2-methyl-4-(1-methylcyclopropyl)benzoate (80.0 mg, 0.39 mmol) was hydrolyzed as usual (General Method NaOH) to afford 2-methyl-4-(1-methylcyclopropyl)benzoic acid (70 mg, 93.9% yield) as a white solid. ¹H NMR (400 MHz, CD₃OD): δ 7.83 (d, J = 8.0 Hz, 1H), 7.15 - 7.10 (m, 2H), 2.56 (s, 3H), 1.42 (s, 3H), 0.95 - 0.85 (m, 2H), 0.85 - 0.75 (m, 2H).

Compound 435 (formic acid salt) was prepared as a white solid utilizing the methods in Example 7 (Compound 101). LCMS (Method 5-95AB): t_{R} = 0.557 min, [M + H]⁺ = 810.3.

### Example 340: Synthesis of Compound 436

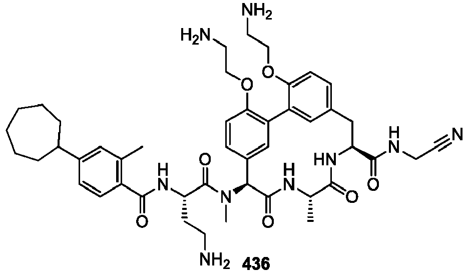

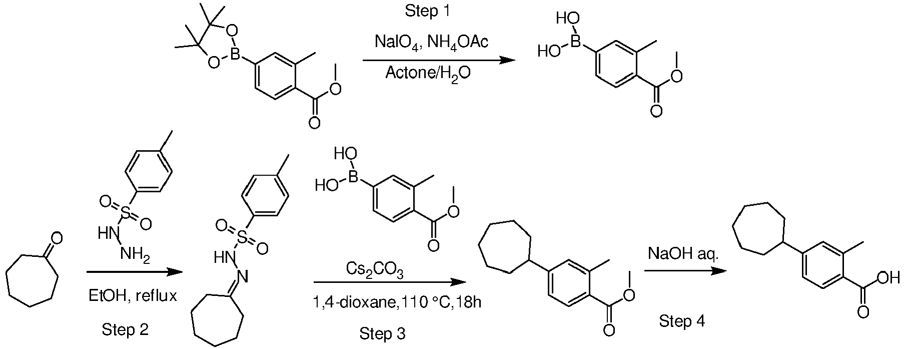

Step1: A mixture of methyl 2-methyl-4-(4,4,5,5-tetramethyl-1,3,2-dioxaborolan-2-yl)benzoate (400.0 mg, 1.45 mmol) , sodium periodate (309.8 mg, 1.45 mmol) and ammonium acetate (800.0 mg, 10.38 mmol) in acetone (8 mL) and water (4 mL) was stirred at 20 °C for 16 h, and evaporated to dryness. The residue was taken up in EtOAc (20 mL), washed with water (20 mL x 2) and brine (20 mL), dried over MgSO₄ and concentrated. The residue was purified by flash column chromatography (33% ethyl acetate in petroleum ether) to afford (4-methoxycarbonyl-3-methyl-phenyl)boronic acid (200 mg, 71.2% yield) as a white solid.

Step 2: A solution cycloheptanone (2000.0 mg, 17.83 mmol) and 4-methylbenzenesulfonohydrazide (3320 mg, 17.83 mmol) in ethanol (10 mL) was stirred at 60 °C for 16 h. The reaction was diluted with additional ethanol (10 mL) and filtered. The filtrate was concentrated to afford N-(cycloheptylideneamino)-4-methyl-benzenesulfonamide (4000 mg, 80% yield) as a white solid.

Step 3: A mixture of cesium carbonate (313.8 mg, 0.96 mmol), (4-methoxycarbonyl-3-methyl-phenyl)boronic acid (186.8 mg, 0.96 mmol) and N-(cycloheptylideneamino)-4-methyl-benzenesulfonamide (180.0 mg, 0.64 mmol) in 1,4-dioxane (5 mL) was stirred at 110 °C for 16 h under N₂ protection, and evaporated to dryness. The residue was taken up in EtOAc (30 mL), washed with water (30 mL x 2) and brine (30 mL), dried over MgSO₄ and concentrated. The residue was purified by prep-TLC (10% EtOAc in petroleum ether, Rf = 0.6) to afford methyl 4-cycloheptyl-2-methylbenzoate (80 mg, 50.6% yield) as a colorless oil. LCMS (Method 5-95 AB, ESI): t_{R} = 0.976 min, [M + H]⁺ = 246.9.

Step 4: Methyl 4-cycloheptyl-2-methylbenzoate was hydrolyzed as previously described (General Method NaOH) to give 4-cycloheptyl-2-methylbenzoic acid.

Compound 436 (formic acid salt) was prepared as a white solid utilizing the methods in Example 7 (Compound 101). LCMS (Method 5-95 AB, ESI): t_{R} = 0.654 min, [M + H]⁺ = 852.4.

### Example 341: Synthesis of Compound 437

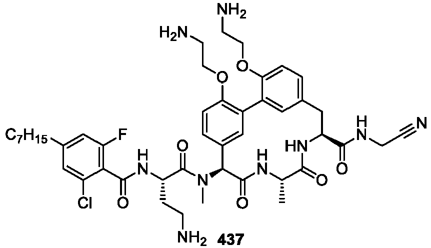

Compound 437 (formic acid salt) was prepared utilizing the methods in Example 340 (Compound 436) and Example 195 (Compound 290). LCMS (Method 5-95 AB, ESI): t_{R} = 0.625 min, [M+H]⁺ = 892.4.

### Example 342A: Synthesis of Compound 438

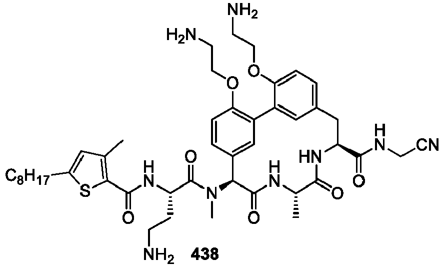

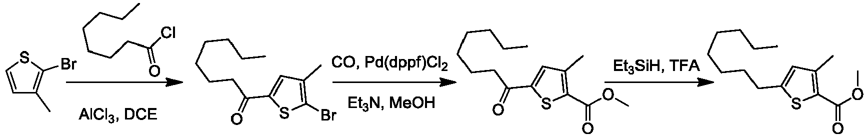

Step 1: To a mixture of 2-bromo-3-methyl-thiophene (226.7 mg, 1.28 mmol) and aluminum chloride (375.6 mg, 2.82 mmol) in 1,2-dichloroethane (3 mL) was added octanoyl chloride (229.1 mg, 1.41 mmol) and stirred at 20 °C for 12 h. LCMS (5-95AB/1.5min): RT =0.991 min, [M+H]⁺302.9 showed 45% of DP. The mixture was poured onto ice and extracted with EtOAc (30 mL). The organic layer was washed with brine, dried over Na₂SO₄ and concentrated. The residue was purified by prep-TLC (5% EtOAc in petroleum ether, Rf = 0.5) to give 1-(5-bromo-4-methyl-2-thienyl)octan-1-one (160 mg, 41.2% yield) as a yellow oil. ¹H NMR (400 MHz, CDCl₃): δ 7.60 (s, 1H), 2.86 (t, J = 7.4 Hz, 2H), 2.22 (s, 3H), 1.67 (quint, J = 7.3 Hz, 2H), 1.43 - 1.22 (m, 8H), 0.90 (t, J = 7.0 Hz, 3H).

Step 2: A solution of 1-(5-bromo-4-methyl-2-thienyl)octan-1-one (160.0 mg, 0.53 mmol) and triethylamine (0.22 mL, 1.58 mmol) in methanol (10 mL) was treated with 1,1'-bis(diphenylphosphino)ferrocene palladium dichloride (38.6 mg, 0.05 mmol). The resulting mixture was stirred at 80 °C for 10 h under CO (45 psi) and concentrated. The residue was purified by prep-TLC (7% ethyl acetate in petroleum) to afford methyl 3-methyl-5-octanoyl-thiophene-2-carboxylate (120 mg, 80.5% yield) as a colorless oil.

Step 3: To a solution of methyl 3-methyl-5-octanoyl-thiophene-2-carboxylate (50.0 mg, 0.18 mmol) in 2,2,2-trifluoroacetic acid (1 mL) was added triethylsilane (82.4 mg, 0.71 mmol) dropwise. The mixture was stirred at 20 °C for 16 h and concentrated. The residue was purified by prep-TLC (5% EtOAc in petroleum ether, Rf = 0.6) to afford methyl 3-methyl-5-octyl-thiophene-2-carboxylate (38 mg, 80% yield) as a yellow oil. LCMS (Method 5-95 AB, ESI): RT = 1.180 min, [M+H]⁺ = 269.1.

Compound 438 (formic acid salt) was prepared utilizing the methods in Example 7 (Compound 101). LCMS (Method 5-95 AB, ESI): t_{R} = 0.785 min, [M+H]⁺ = 874.4.

### Example 342B: Synthesis of Compound 439 and Compound 440

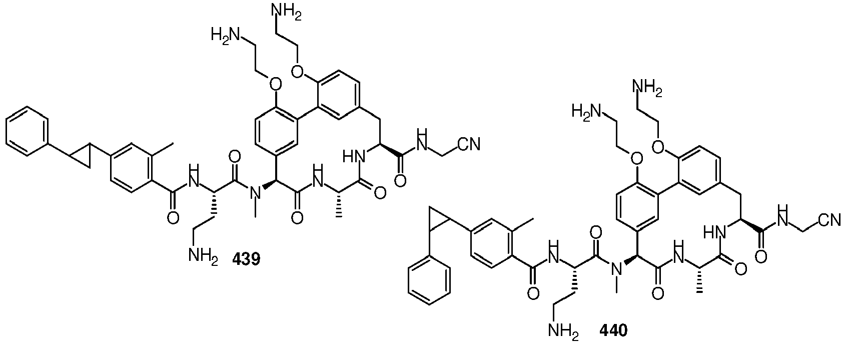

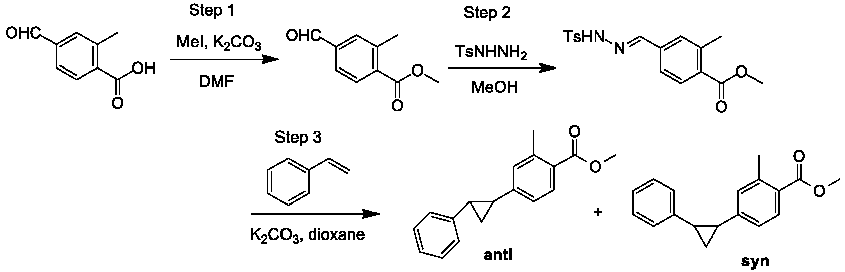

Step 1: To a solution of 4-formyl-2-methylbenzoic acid (450.0 mg, 2.74 mmol) in N,N-dimethylformamide (5 mL) at 30 °C were added K₂CO₃ (1.13 g, 8.22 mmol) and iodomethane (1.3 mL, 20.85 mmol) and stirred for 10 h. The reaction was diluted with 1N HCl (15 mL) and extracted with EtOAc (50 mL x 3). The combined organic layers were washed with brine (30 mL x 3), dried over Na₂SO₄ and concentrated. The residue was purified by silica gel column (5% EtOAc in petroleum) to give methyl 4-formyl-2-methylbenzoate (400 mg, 81.9% yield) as a yellow oil.

Step 2: To a solution of tosyl hydrazide (418.1 mg, 2.24 mmol) in methanol (2 mL) was added methyl 4-formyl-2-methylbenzoate (400.0 mg, 2.24 mmol) dropwise at 60 °C and stirred at 60 °C for 2 h. The solution was concentrated to give crude methyl 2-methyl-4-((2-tosylhydrazono)methyl)benzoate (580.0 mg) as a white solid, which was used directly in the next step.

Step 3: To a solution of methyl 2-methyl-4-((2-tosylhydrazono)methyl)benzoate (580.0 mg, 1.67 mmol) in dioxane (4 mL) were added styrene (348.8 mg, 3.35 mmol) and potassium carbonate (347.1 mg, 2.51 mmol). The reaction was quenched with water (10 mL) and extracted with EtOAc (20 mL x 3). The combined organic layers were washed with water (20 mL) and brine (20 mL), dried over MgSO₄ and concentrated. The residue was purified first by prep-TLC (8% ethyl acetate in petroleum ether), then by reverse phase chromatography (acetonitrile 70-100/0.225% formic acid in water) to afford (±)-anti-methyl 2-methyl-4-(2-phenylcyclopropyl)benzoate (91 mg, 20.4% yield) and (±)-syn-methyl 2-methyl-4-(2-phenylcyclopropyl)benzoate (40 mg, 9% yield), both as a colorless oil.

Compound 439 (formic acid salt) was prepared utilizing the methods previously described. LCMS (Method 5-95 AB, ESI): t_{R} = 0.715 min, [M+H]⁺ = 872.9.

Compound 440 (formic acid salt) was prepared utilizing the methods previously described. LCMS (Method 5-95 AB, ESI): t_{R} = 0.708 min, [M+H]⁺ = 872.6.

### Example 343A: Synthesis of Compound 441

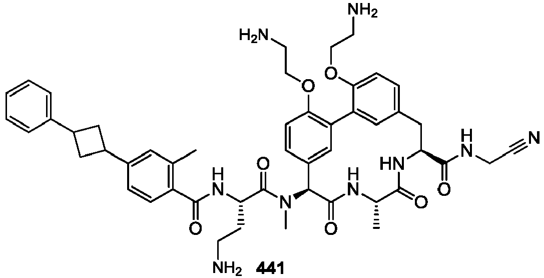

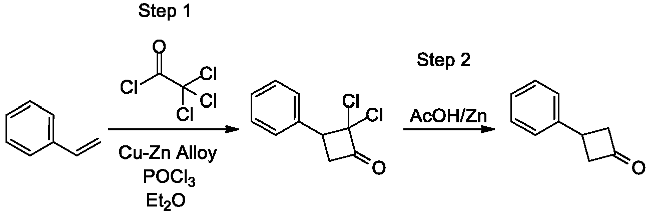

Step 1: A solution of trichloroacetyl chloride (9091.5 mg, 50 mmol) and phosphorus oxychloride (5.47 g, 35.67 mmol) in ether (25 mL) was added dropwise to a solution of styrene (2603.8 mg, 25 mmol) and zinc-copper couple (4.9 g, 75 mmol) in ether (50 mL). The resulting solution was stirred at 50°C for 2 h then at room temperature overnight. The resulting mixture was filtered over Celite and washed with ether (60 mL). To the filtrate was added hexane (200 mL) and gently stirred at room temperature for 1 h. The suspension was filtered and the filtrate was concentrated under reduced pressure to give crude 2,2-dichloro-3-phenyl-cyclobutanone (4.2 g, 78.1% yield) as a yellow oil. ¹H NMR (400 MHz, CDCl₃): δ 7.46-7.39 (m, 3H), 7.38-7.30 (m, 2H), 4.25 (t, J = 10.2 Hz, 1H), 3.73 (dd, J = 17.6, 10.4 Hz, 1H), 3.55 (dd, J = 17.6, 10.0 Hz, 1H).

Step 2: To a solution of 2,2-dichloro-3-phenyl-cyclobutanone (4.2 g, 19.53 mmol) in AcOH (50 mL, 19.53 mmol) was added zinc dust (5.11 g, 78.11 mmol) and stirred at 100 °C for 12 h. The mixture was diluted with water (100 mL) and extracted with petroleum ether (50 mL x4). The combined organic layers were washed with sat. NaHCO₃ (30 mL x 4) and brine (30 mL x 3), dried over Na₂SO₄ and concentrated. The residue was purified by silica gel column (5% EtOAc in petroleum ether) to give 3-phenylcyclobutanone (1.1 g, 38.5% yield) as a yellow oil. ¹H NMR (400 MHz, CDCl₃): δ 7.38-7.25 (m, 5H), 3.70-3.68 (m, 1H), 3.53-3.46 (m, 2H), 3.28-3.22 (m, 2H).

Compound 441 (formic acid salt) was prepared utilizing the methods in Example 340 (Compound 436). LCMS (Method 5-95 AB, ESI): t_{R} = 0.719 min, [M+H]⁺ = 886.5.

### Example 343B: Synthesis of Compound 442

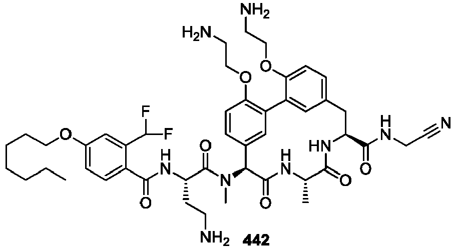

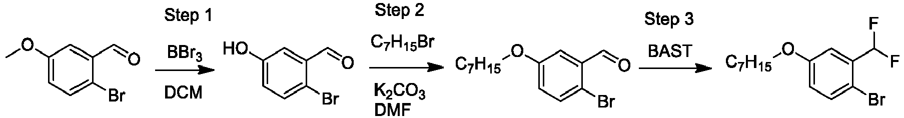

Step 1: To a mixture of 2-bromo-5-methoxybenzaldehyde (2000.0 mg, 9.3 mmol) in dichloromethane (10 mL) was added boron tribromide (2M in DCM; 4.65 mL, 9.3 mmol) slowly at 0 °C. The reaction was warmed up to 25 °C and stirred for 3 h. The reaction was quenched with water (10 mL) at 0°C and extracted with EtOAc (50 mL). The organic layer was washed with water (50 mL x 2) and brine (50 mL), dried over MgSO₄ and concentrated. The residue was purified by flash column chromatography (petroleum ether) to afford 2-bromo-5-hydroxybenzaldehyde (1.7 g, 90.9% yield) as a colorless oil.

Step 2: To a solution of 2-bromo-5-hydroxybenzaldehyde (100.0 mg, 0.50 mmol) in N,N-dimethylformamide (3 mL) were added 1-bromoheptane (267.3 mg, 1.49 mmol) and potassium carbonate (206.3 mg, 1.49 mmol). The mixture was stirred at 20 °C for 16 h and concentrated to dryness. The residue was taken up in EtOAc (50 mL), washed with water (50 mL x 2) and brine (50 mL), dried over MgSO₄ and concentrated. The residue was purified by prep-TLC (10% EtOAc in petroleum ether, Rf = 0.3) to afford 2-bromo-5-(heptyloxy)benzaldehyde (100 mg, 67.2% yield) as a yellow oil. ¹H NMR (400 MHz, CDCl₃): δ 10.32 (s, 1H), 7.52 (d, *J* = 9.2 Hz, 1H), 7.41 (d, *J* = 2.8 Hz, 1H), 7.03 (dd, *J* = 8.8, 3.2 Hz, 1H), 3.99 (t, *J* = 6.6 Hz, 2H), 1.85 - 1.75 (m, 2H), 1.50 - 1.20 (m, 8H), 0.90 (t, *J=* 6.8 Hz, 3H).

Step 3: A mixture of 2-bromo-5-(heptyloxy)benzaldehyde (100.0 mg, 0.33 mmol) in deoxo-fluor (2.0 mL, 1 mmol) was stirred at 80 °C for 16 h and concentrated to dryness. The residue was taken up in EtOAc (50 mL), washed with water (50 mL x 2) and brine (50 mL), dried over MgSO₄ and concentrated. The residue was purified by prep-TLC (5% EtOAc in petroleum ether, Rf = 0.3) to afford 1-bromo-2-(difluoromethyl)-4-(heptyloxy)benzene (80 mg, 74.5% yield) as a yellow oil. ¹H NMR (400 MHz, CDCl₃): δ 7.44 (d, *J* = 8.8 Hz, 1H), 7.15 (d, *J* = 3.2 Hz, 1H), 7.00-6.70 (m, 2H), 3.94 (t, *J*= 6.6 Hz, 2H), 1.80 - 1.70 (m, 2H), 1.50 - 1.20 (m, 8H), 0.88 (t, *J* = 6.6 Hz, 3H).

Compound 442 (formic acid salt) was prepared utilizing the methods in Example 170 (Compound 265). LCMS (Method 5-95 AB, ESI): t_{R} = 0.781 min, [M+H]⁺ = 906.4.

### Example 344: Synthesis of Compound 443 and Compound 444

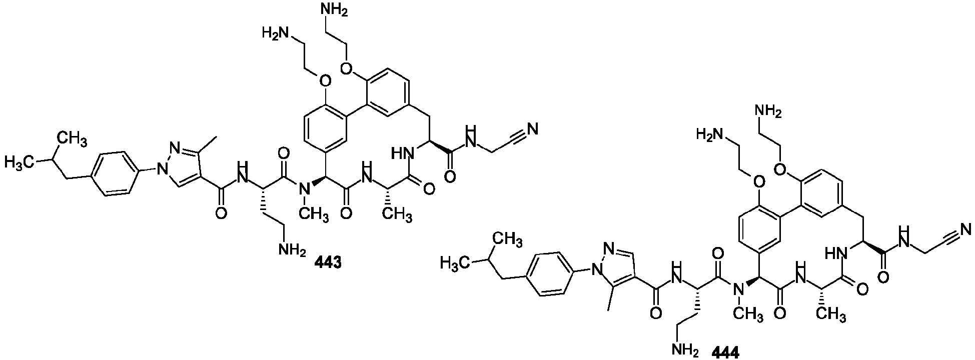

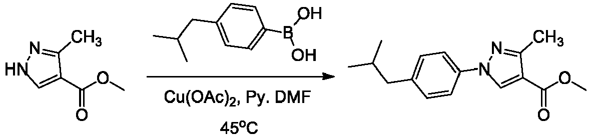

A mixture of methyl 3-methyl-1H-pyrazole-4-carboxylate (250.0 mg, 1.78 mmol), 4-isobutylphenyl boronic acid (412.9 mg, 2.32 mmol), pyridine (282.2 mg, 3.57 mmol) and copper(II) acetate (486.0 mg, 2.68 mmol) in N,N-dimethylformamide (6 mL) was stirred at 25°C for 14 h. The reaction was diluted with EtOAc (120 mL), washed with brine (50 mL x 3), dried over Na₂SO₄ and concentrated. The residue was purified by prep-TLC (20% EtOAc in petroleum ether, Rf = 0.45) to give methyl 1-(4-isobutylphenyl)-3-methyl-1H-pyrazole-4-carboxylate (350 mg, 72% yield) as a white solid. LCMS (Method 5-95 AB, ESI): t_{R} = 0.996 min, [M+H]⁺ = 272.9.

Compound 443 and Compound 444 (formic acid salt) was prepared utilizing the methods in Example 5 and Example 7. Prior to TFA deprotection, the isomers were separated on preparative TLC. Data for Compound 443: LCMS (Method 5-95 AB, ESI): t_{R} = 0.726 min, [M+H]⁺ = 878.5. Data for Compound 444: LCMS (Method 5-95 AB, ESI): t_{R} = 0.720 min, [M+H]⁺ = 878.5.

### Example 345: Synthesis of Compound 445

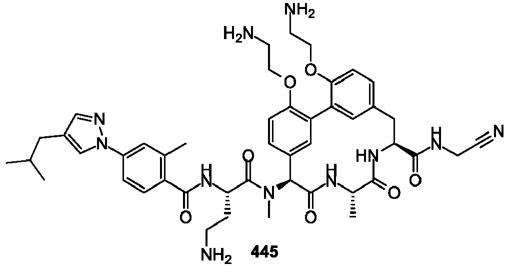

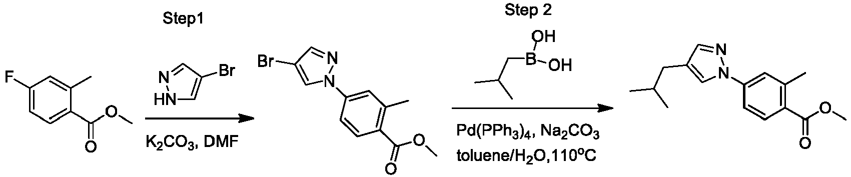

Step 1: A mixture of methyl 4-fluoro-2-methylbenzoate (300.0 mg, 1.78 mmol), 4-bromo-1H-pyrazole (393.3 mg, 2.68 mmol) and potassium carbonate (739.7 mg, 5.35 mmol) in N,N-dimethylformamide (20 mL) was stirred at 70°C for 12 h. The reaction mixture was diluted with water (100 mL) and extracted with EtOAc (100 mL x 3). The combined organic layers were washed with water (50 mL x 2) and brine (50 mL), dried over MgSO₄ and concentrated. The residue was purified by prep-TLC (20% EtOAc in petroleum ether, Rf = 0.5) to obtain methyl 4-(4-bromopyrazol-1-yl)-2-methyl-benzoate (200 mg, 38% yield) as a white solid. ¹H NMR (400 MHz, DMSO-d6): δ 8.87 (s, 1H), 7.94 - 7.91 (m, 2H), 7.81 (s, 1H), 7.76 - 7.74 (m, 1H), 3.80 (s, 3H), 2.56 (s, 3H).

Step 2: A mixture of methyl 4-(4-bromopyrazol-1-yl)-2-methyl-benzoate (200.0 mg, 0.68 mmol), isobutyl boronic acid (345.41 mg, 3.39 mmol), sodium carbonate (359.1 mg, 3.39 mmol) and tetrakis(triphenylphosphine)palladium(0) (783.1 mg, 0.68 mmol) in toluene (5 mL) and water (1 mL) was stirred at 110 °C for 12 h and filtered. The filtrate was concentrated and purified by prep-TLC (30% EtOAc in petroleum, Rf = 0.7) to obtain methyl 4-(4-isobutylpyrazol-1-yl)-2-methyl-benzoate (40 mg, 21.7% yield) as a white solid. LCMS (Method 5-95 AB, ESI): t_{R} = 1.010 min, [M+H]⁺ = 272.9.

Compound 445 (formic acid salt) was prepared utilizing the methods previously described. LCMS (Method 5-95 AB, ESI): t_{R} = 0.585 min, [M+H]⁺ = 878.5.

### Example 346: Synthesis of Compound 446

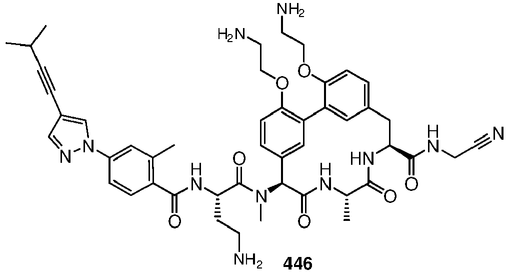

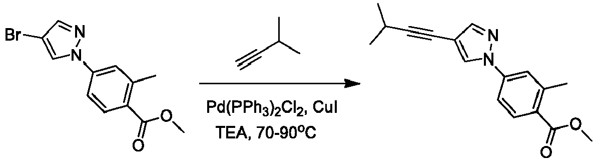

A mixture of methyl 4-(4-bromopyrazol-1-yl)-2-methyl-benzoate (150.0 mg, 0.51 mmol), bis(triphenylphosphine)palladium(II) dichloride (17.9 mg, 0.03 mmol), 3-methyl-1-butyne (103.9 mg, 1.52 mmol) and copper(I) iodide (4.9 mg, 0.03 mmol) in triethylamine (4.19 mL, 30.03 mmol) was stirred at 100 °C for 18 h under nitrogen. The reaction was quenched with water (10 mL) and extracted with dichloromethane (10 mL x 3). The combined organic extracts were washed with brine (10 mL x 2), dried over anhydrous Na₂SO₄ and concentrated. The residue was purified by prep-HPLC to afford methyl 2-methyl-4-[4-(3-methylbut-1-ynyl)pyrazol-1-yl]benzoate (35 mg, 24.4% yield) as a white solid. LCMS (Method 5-95 AB, ESI): t_{R} = 1.014 min, [M+H]⁺ = 283.2.

Compound 446 (formic acid salt) was prepared utilizing the methods previously described. LCMS (Method 5-95 AB, ESI): t_{R} = 0.709 min, [M+H]⁺ = 888.6.

### Example 347: Synthesis of Compound 447

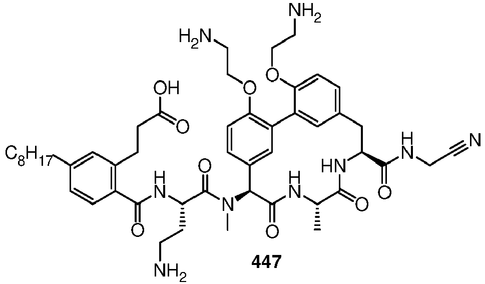

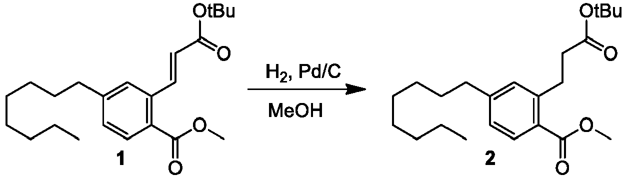

Compound 447 (formic acid salt) was prepared utilizing the methods in Example 318 (Compound 414) except methyl 2-[(E)-3-tert-butoxy-3-oxo-prop-1-enyl]-4-octyl-benzoate is subject to the standard hydrogenation conditions (Example 4) to afford methyl 2-(3-(tert-butoxy)-3-oxopropyl)-4-octylbenzoate. LCMS (Method 5-95 AB, ESI): RT = 0.768 min, [M+H]⁺ = 926.6.

### Example 348: Synthesis of Compound 448

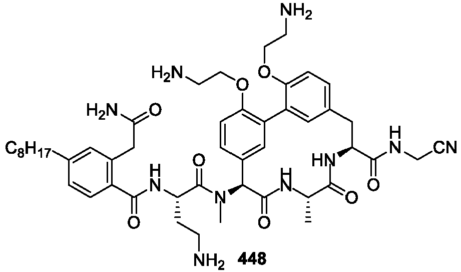

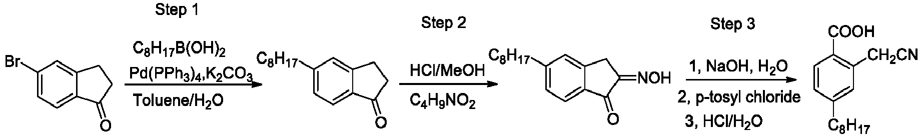

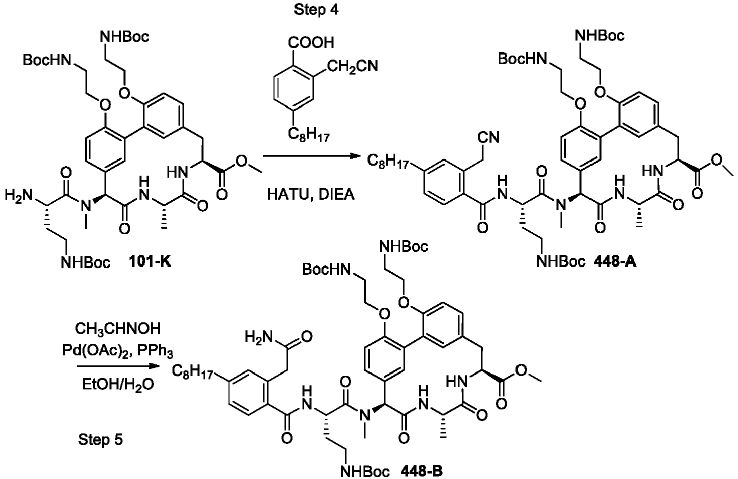

Step 1: A solution of 5-bromo-2,3-dihydro-1H-inden-1-one (1.0 g, 4.7 mmol), Pd(PPh₃)₄ (274 mg, 0.24 mmol), K₂CO₃ (1.3 g, 9.5 mmol) and octylboronic acid (1.1 g, 7.1 mmol) in toluene (15 mL) and H₂O (1mL) was stirred under N₂ at 100°C for 16 h. The reaction mixture was added with EtOAc (100 mL), which was washed by brine (100 mL). The organic layer dried over Na₂SO₄, concentrated and the residue was purified by silica gel flash column to give 5-octyl-2,3-dihydro-1H-inden-1-one (1.1 g, 95% yield) as yellow oil.

Step 2: To a solution of 5-octyl-2,3-dihydro-1H-inden-1-one (1.1 g, 4.5 mmol) in Et₂O (15 mL) was added 4M HCl/MeOH (5 mL), followed by the addition of a solution of 1-nitrobutane (511 mg, 5.0 mmol) in Et₂O (15 mL), and the mixture was stirred at room temperature for 2 h. The resulting precipitate was then collected, washed with Et₂O, and dried to give 2-(hydroxyimino)-5-octyl-2,3-dihydro-1H-inden-1-one (800 mg, 65% yield) as a yellow solid. LCMS (Method 5-95 AB, ESI): t_{R} = 0.920, [M + H]⁺ = 274.0

Step 3: A solution of 2-(hydroxyimino)-5-octyl-2,3-dihydro-1H-inden-1-one (800 mg, 2.9 mmol) and TsOH (725 mg, 3.8 mmol) in H₂O (10 mL) was heated to 50°C, followed by the addition of NaOH (468 mg, 11.7 mmol). The resulting mixture was heated further to 80°C for another 15min. After cooling to room temperature, the precipitate was removed from the mixture and the filtrate was acidified with concentrated HCl. The resulting precipitate was collected and purified by silica gel flash column to give 2-(cyanomethyl)-4-octylbenzoic acid (700 mg, 87.5% yield) as a yellow solid.

Step 4: Typical amide coupling (HATU/DIEA) procedure was applied to Compound 101-K (100 mg, 0.11 mmol) and 2-(cyanomethyl)-4-octylbenzoic acid (39 mg, 0.14 mmol) utilizing the methods previously described to afforded Compound 449-A (120 mg, 94% yield) as a white solid.

Step 5: To a solution of Compound 449-A (120 mg, 0.10 mmol) in EtOH/H₂O (3.0 mL, v/v=4/1) was added acetaldoxime (24 mg, 0.41 mmol), catalytic amount of Ph₃P (5.4 mg) and Pd(OAc)₂ (0.5 mg) and the mixture was stirred under N₂ at 80°C for 3h. H₂O (15 mL) was added to the reaction and the mixture was extracted with EtOAc (15 mL x 3). The combined organic layers was dried with Na₂SO₄ and concentrated to give Compound 449-B (110 mg, 90.3% yield) as a yellow solid, which was used directly in the next step. LCMS (Method 5-95 AB, ESI): t_{R} = 0.964, [M + H]⁺ = 1187.6.

Compound 448 (formic acid salt) was prepared utilizing the methods previously described. LCMS (Method 5-95 AB, ESI): t_{R} = 0.630, [M + H]⁺ = 911.6; ¹H NMR (400 MHz, MeOH-*d*₄) δ 8.47 (brs, 2H, HCOOH), 7.50 (d, *J*=8.0 Hz, 1H), 7.35 (d, *J*=8.0 Hz, 1H), 7.28-7.18 (m, 4H), 7.11 (d, *J*=8.0 Hz, 1H), 6.91 (brs, 1H), 6.83 (brs, 1H), 6.37(s, 1H), 5.11-5.08 (m, 1H), 4.81-4.75 (m, 2H), 4.28-4.19 (m, 4H), 4.22 (s, 2H), 3.87-3.77 (m, 2H), 3.37-3.35(m, 1H), 3.27-3.10 (m, 7H), 2.94 (s, 3H), 2.69-2.65 (m, 2H), 2.29-2.24 (m, 1H), 2.18-2.12 (m, 1H), 1.67-1.63 (m, 2H), 1.43-1.23 (m, 13H), 0.89 (t, *J*=6.8 Hz, 3H).

### Example 349: Synthesis of Compound 449

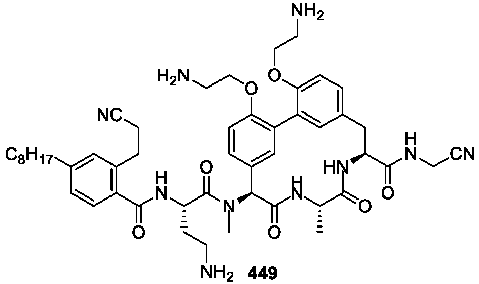

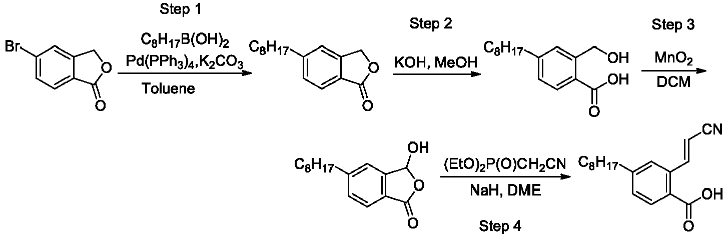

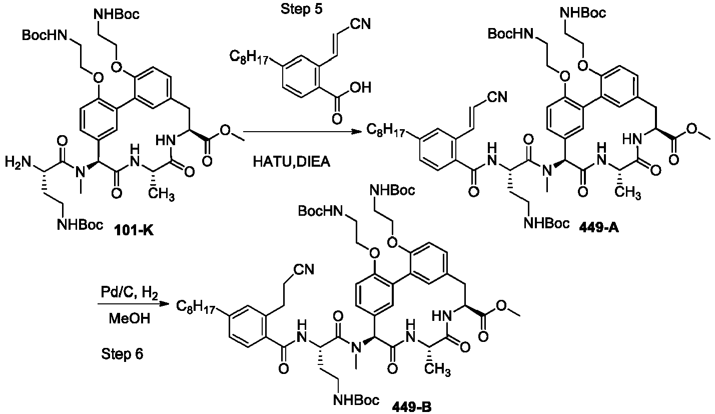

Step 1: A solution of 5-bromoisobenzofuran-1(3H)-one (2.0 g, 9.4 mmol), K₂CO₃ (3.9 g, 28.2 mmol), Pd(PPh₃)₄ (1.1 g, 0.94 mmol) and octylboronic acid (3.0 g, 18.8 mmol) in toluene (40 mL) was stirred under N₂ at 100°C for 24 h. The reaction mixture was added with EtOAc (100 mL), which was washed by brine (100 mL). The organic layer dried over Na₂SO₄, concentrated and the residue was purified by silica gel flash column to give 5-octylisobenzofuran-1(3H)-one (1.26 g, 54% yield) as colorless oil.

Step 2: To a solution of 5-octylisobenzofuran-1(3H)-one (1.26 g, 5.1 mmol) in MeOH (22 mL) was added KOH (430 mg, 7.7mmol) at room temperature and the mixture reaction was heated to 70°C while stirring and stirred at the same temperature for 6 h. The volatiles were removed and the residue was re-dissolved with water (20 mL), which was then acidified to pH=4 by 1M KHSO₄ solution. The resulting precipitate was collected, washed with water and dried in vacuo to afford 2-(hydroxymethyl)-4-octylbenzoic acid (852 mg, 63.1% yield) as a white solid. LCMS (Method 5-95 AB, ESI): t_{R} = 0.977, M+Na⁺ = 287.9

Step 3: To a solution of 2-(hydroxymethyl)-4-octylbenzoic acid (752 mg, 3.0 mmol) in DCM (18 mL) was added MnO₂ (2.6 g, 30 mmol) and the mixture was stirred at room temperature for 5 h. The filtrate was then concentrated to obtain 3-hydroxy-5-octylisobenzofuran-1(3H)-one (565 mg, 71.7% yield) as yellow oil, which was used directly in the next step. LCMS (Method 5-95 AB, ESI): t_{R} = 0.982, [M + H]⁺ = 262.9

Step 4: To a solution of 3-hydroxy-5-octylisobenzofuran-1(3H)-one (565 mg, 2.15 mmol) and diethyl-cyanomethyl phosphonate (382 mg, 2.15 mmol) in DME (11 mL) was added 60% NaH in oil (172 mg, 4.30 mmol) at 0°C and the mixture was stirred at the same temperature for 1 h. The reaction mixture was quenched with H₂O (30 mL), which was extracted by EtOAc (30 mL x 2). The combined organic layers were dried over Na₂SO₄, concentrated and the residue was purified by HPLC to obtain (E)-2-(2-cyanovinyl)-4-octylbenzoic acid (60 mg, 9.8% yield) as a white solid. ¹H NMR (400 MHz, CDCl₃) δ 8.38 (d, *J*=16 Hz, 1H), 8.05 (d, *J*=8 Hz, 1H), 7.34-7.31(m, 2H), 5.77 (d, *J*=16 Hz, 1H), 2.67 (t, *J*=8.0 Hz, 2H), 1.69-1.66 (m, 2H), 1.30-1.26 (m, 10H), 0.87 (t, *J*=6.8 Hz, 3H).

Typical amide coupling (HATU) procedure was applied to Compound 101-K (150 mg, 0.16 mmol) and (E)-2-(2-cyanovinyl)-4-octylbenzoic acid (56 mg, 0.20 mmol) to afford Compound 449-A (152 mg, 78% yield) as a white solid.

A solution of Compound 449-A (122 mg, 0.10 mmol), 10% Pd/C (22 mg, 0.02 mmol) in MeOH (12 mL) was stirred under H₂ at room temperature. LCMS was used to monitor the reaction to ensure no over-reduction of nitrile. After LCMS indicated the completion of the reaction, the filtrate was concentrated to give Compound 449-B (116 mg, 94.9% yield) as a white solid. LCMS (Method 5-95 AB, ESI): t_{R} = 1.139, [M + H]⁺ = 1183.8.

Compound 449 (formic acid salt) was prepared utilizing the methods previously described. LCMS (Method 5-95 AB, ESI): t_{R} = 0.769, [M + H]⁺ = 907.5

### Example 350: Synthesis of Compound 450

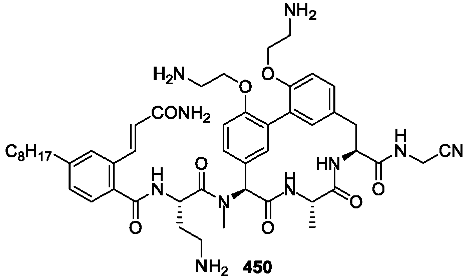

Compound 450 (free base) was prepared utilizing similar methods in Example 348 (Compound 448). LCMS (Method 5-95 AB, ESI): t_{R} = 0.764, [M + H]⁺ = 923.5.

### Example 351: Synthesis of Compound 451

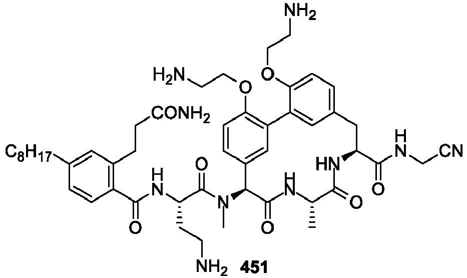

Compound 451 (free base) was prepared utilizing similar methods in Example 348 (Compound 448). LCMS (Method 5-95 AB, ESI): t_{R} = 0.776, [M + H]⁺ = 925.8.

### Example 352: Synthesis of Compound 452

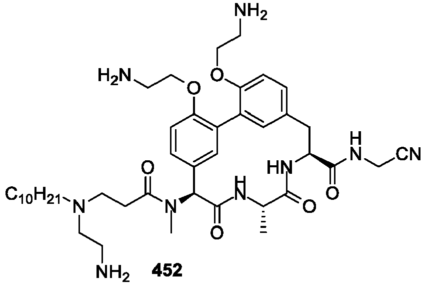

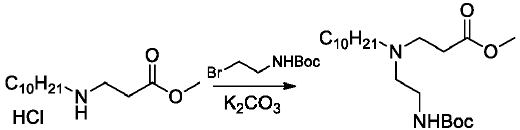

To a solution of methyl 3-(decylamino)propanoate (380 mg, 1.36 mmol) in DMF (3 mL) was added K₂CO₃ (560 mg, 4.07 mmol) and t-butyl 2-bromoethylcarbamate (300 mg, 1.36 mmol) at 0°C and the mixture was stirred at room temperature for 48 h. The reaction was added with H₂O (30 mL), which was extracted with EtOAc (30 mL x 2). The combined organic layers were dried over Na₂SO₄, concentrated and the residue was purified by silica gel flash column to give methyl 3-((2-((tert-butoxycarbonyl)amino)ethyl)(decyl)amino)propanoate (166 mg, 31.6% yield) as colorless oil. LCMS (Method 5-95 AB, ESI): t_{R} = 0.874, [M + H]⁺ = 387.3.

Compound 452 (formic acid salt) was prepared utilizing the methods previously described. LCMS (Method 5-95 AB, ESI): t_{R} = 0.700, [M + H]⁺ = 792.5.

### Example 353: Synthesis of Compound 453

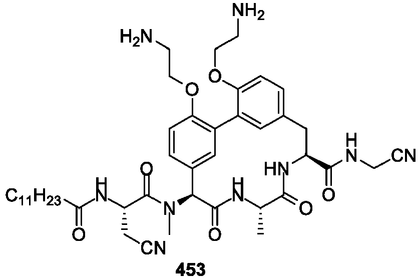

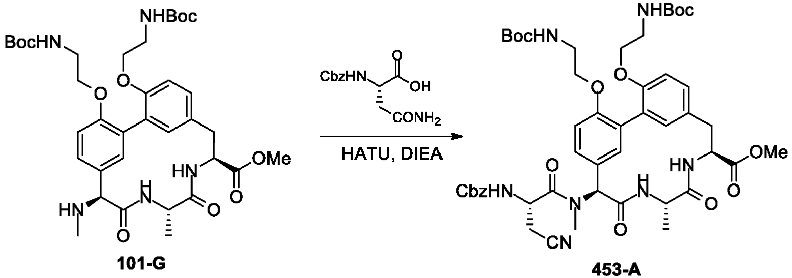

To a mixture of Compound 101-G (300 mg, 0.42 mmol), Cbz-Asn-OH (224 mg, 0.84 mmol), HATU (319 mg, 0.84 mmol), MgSO₄ (252 mg, 2.1 mmol) in DMF/DCM (20 mL, v/v=1:3) was added DIEA (217 mg, 1.68 mmol) at 0 °C and the mixture was warmed to room temperature while stirring and stirred for 2 h. The volatiles were removed and the residue was taken up by EtOAc (100 mL), which was washed by brine (50 mL x 2). The organic layer was dried over Na₂SO₄, concentrated and the residue was purified by prep-TLC to afford Compound 453-A (100 mg, 25.2% yield) as a white solid. During the coupling, dehydration of the amide occurs.

Compound 453 (free base) was prepared utilizing the methods previously described from Compound 453-A. LCMS (Method 5-95 AB, ESI): t_{R} = 0.686, [M + H]⁺ = 816.2; ¹H NMR (400 MHz, MeOH-*d*₄) δ 7.27-7.24 (m, 2H), 7.14 (d, *J*=8.0 Hz, 1H), 7.09 (d, *J*=8.0 Hz, 1H), 6.87 (brs, 1H), 6.79 (brs, 1H), 6.21(s, 1H), 5.18-5.15 (m, 1H), 4.83-4.77 (m, 2H), 4.21-4.17 (m, 4H), 4.19 (s, 2H), 3.16-3.13 (m, 5H), 2.93-2.90 (m, 2H), 2.81 (s, 3H), 2.28-2.25 (m, 3H), 1.65-1.61 (m, 3H), 1.33-1.29 (m, 18H), 0.90 (t, *J*=6.4 Hz, 3H).

### Example 354: Synthesis of Compound 454

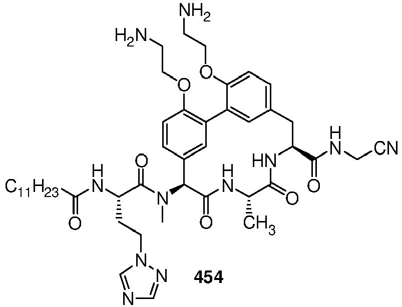

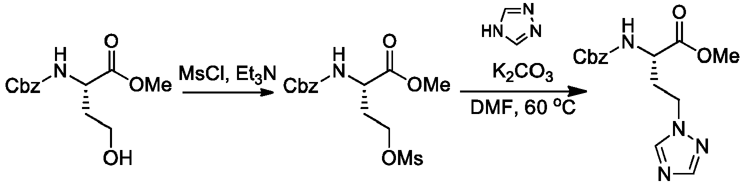

To a solution of (S)-methyl 2-(((benzyloxy)carbonyl)amino)-4-hydroxybutanoate (900 mg, 3.4 mmol) and Et₃N (0.94 mL, 6.8 mmol) in DCM (10 mL) was added MsCl (0.39 mL, 5.1 mmol) at 0°C and the reaction was warmed up to room temperature while stirring and stirred at the same temperature for 3 h. The pH of the reaction mixture was adjusted to pH=5 with saturated citric acid solution, which was extracted with DCM (30 mL x 2). The combined organic layers were dried over Na₂SO₄, concentrated and the residue was purified by HPLC to afford (S)-methyl 2-(((benzyloxy)carbonyl)amino)-4-((methylsulfonyl)oxy)butanoate (500 mg, 43% yield) as a white solid. LCMS (Method 5-95 AB, ESI): t_{R} = 0.780, [M + H]⁺ = 345.9.

A solution of (S)-methyl 2-(((benzyloxy)carbonyl)amino)-4-((methylsulfonyl)oxy)butanoate (200 mg, 0.58 mmol), K₂CO₃ (160 mg, 1.16 mmol), 1,2,4-triazole (80.0 mg, 1.16 mmol) in DMF (2 mL) was stirred at 60°C for 16 h. Water (10 mL) was added to the reaction, which was then extracted with EtOAc (20 mL x 3). The combined organic layers were washed with brine (50 mL), dried over MgSO₄, concentrated and the residue was purified by prep-TLC to afford (S)-methyl 2-(((benzyloxy)carbonyl)amino)-4-(1H-1,2,4-triazol-1-yl)butanoate (100 mg, 54.2% yield). LCMS (Method 5-95 AB, ESI): t_{R} = 0.728, [M + H]⁺ = 318.9.

Compound 454 (formic acid salt) was prepared utilizing the methods previously described. LCMS (Method 5-95 AB, ESI): t_{R} = 0.672, [M + H]⁺ = 872.4; ¹H NMR (400 MHz, MeOH-*d*₄) δ 8.51 (s, 1H), 8.49 (brs, 2H, HCOOH), 7.97 (s, 1H), 7.25-7.22 (m, 2H), 7.17 (d, *J*=8.0 Hz, 1H), 7.07 (d, *J*=8.0 Hz, 1H), 6.86 (brs, 1H), 6.78 (brs, 1H), 6.29 (s, 1H), 4.80-4.74 (m, 3H), 4.35 (t, *J*=6.4 Hz, 2H), 4.18 (brs, 6H), 3.16-3.13 (m, 5H), 2.71 (s, 3H), 2.40-2.30 (m, 2H), 2.26 (t, *J*=6.0 Hz, 2H), 2.25-2.15 (m, 2H), 1.68-1.64 (m, 3H), 1.34-1.28 (m, 17H), 0.89 (t, *J*=6.8 Hz, 3H).

### Example 355: Synthesis of Compound 455

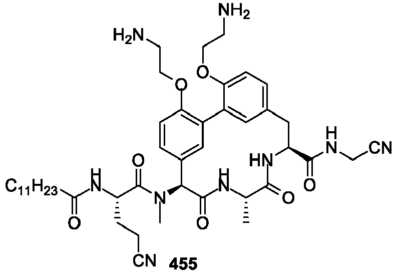

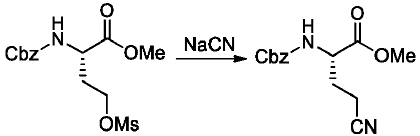

To a solution of (S)-methyl 2-(((benzyloxy)carbonyl)amino)-4-hydroxybutanoate (400 mg, 1.16 mmol) in DMF (5 mL) was added NaCN (62 mg, 1.27 mmol) at room temperature and the reaction mixture was warmed to 75 °C while stirring and stirred at the same temperature for 2 h. The volatiles were removed and the residue was taken by EtOAc (40 mL), which was washed by brine (40 mL). The organic layer was dried over Na₂SO₄, concentrated and the residue was purified by Prep-TLC to give (S)-methyl 2-(((benzyloxy)carbonyl)amino)-4-cyanobutanoate (210 mg, 65.6% yield) as colorless oil. LCMS (Method 5-95 AB, ESI): t_{R} = 0.779, M+Na⁺ = 298.9.

Compound 455 (formic acid salt) was prepared utilizing the methods previously described. LCMS (Method 5-95 AB, ESI): t_{R} = 0.814, [M + H]⁺ = 830.4.

### Example 356: Synthesis of Compound 456

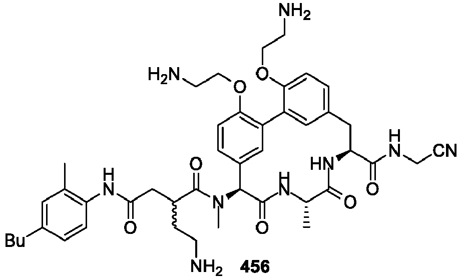

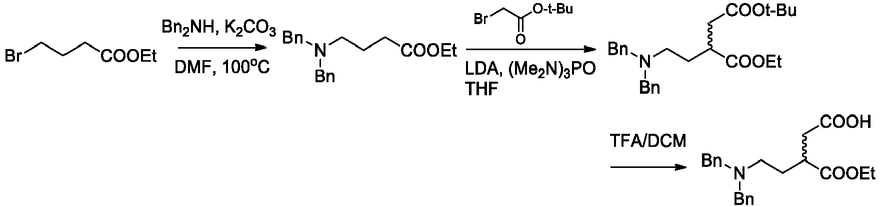

**Step** 1: To a mixture of Bn₂NH (1.0 g, 5.1 mmol) and ethyl 4-bromobutanoate (1.0 g, 5.1 mmol) in DMF (5 mL) was added K₂CO₃ (3.5 g, 25.6 mmol) at room temperature and the mixture was stirred at 100 °C for 16 h. The reaction mixture was added EtOAc (50 mL), which was washed with brine (50 mL x 3). The organic layer was dried over Na₂SO₄, concentrated and the residue was purified on silica gel flash column to obtain ethyl 4-(dibenzylamino)butanoate (520 mg, 32.6% yield) as pale oil.

Step 2: To a solution of ethyl 4-(dibenzylamino)butanoate (4.0 g, 12.8 mmol) in THF (40 mL) was added 2 N LDA in THF (6.4 mL, 12.8 mmol) dropwise at -78°C and the reaction mixture was stirred at the same temperature for 0.5 h, followed by the addition of a solution of tert-butyl 2-bromoacetate (3.0 g, 15.4 mmol) and HMPA (2.3 g, 12.8 mmol) in THF (40 mL). The resulting mixture was stirred for 3 h at -78°C and the reaction was warmed up to room temperature while stirring and stirred for another 3 h. The reaction mixture was added with EtOAc (200 mL), which was washed with saturated NH₄Cl and brine (each 200 mL). The organic layer was dried over Na₂SO₄, concentrated and the residue was purified on silica gel flash column to obtain 4-tert-butyl 1-ethyl 2-(2-(dibenzylamino)ethyl)succinate (800 mg, 14.6% yield) as pale oil. ¹H NMR (400 MHz, CDCl₃) δ 7.36-7.21 (m, 10H), 4.05 (q, *J*=7.0 Hz, 2H), 3.61 (d, *J*=16 Hz, 2H), 3.47 (d, *J*=16 Hz, 2H), 2.92-2.86 (m, 1H), 2.46-2.39 (m, 3H), 2.17-2.11 (m, 1H), 1.98-1.90 (m, 2H). 1.42 (s, 9H), 1.17 (t, *J*=7.2 Hz, 3H).

Step 3: Standard acidic hydrolysis of the t-butyl ester with TFA in DCM afforded 5-(dibenzylamino)-3-(ethoxycarbonyl)pentanoic acid.

Compound 456 (formic acid salt) was prepared utilizing the methods previously described as a mixture of diastereomers. LCMS (Method 5-95 AB, ESI): t_{R} = 0.719, [M + H]⁺ = 826.5; ¹H NMR (400 MHz, MeOH-*d*₄) δ 7.39-7.21 (m, 3H), 7.14-7.03 (m, 4H), 6.89 (brs, 1H), 6.83 (brs, 1H), 6.34 (s, 1H), 4.25-4.19 (m, 4H), 4.21 (s, 2H), 3.21-2.97 (m, 9H), 2.84 (s, 3H), 2.74-2.70 (m, 1H), 2.63-2.59 (m, 2H), 2.23 (s, 3H), 2.10-1.98 (m, 2H), 1.65-1.57 (m, 2H), 1.63-1.35 (m, 6H), 1.22-1.17 (m, 1H), 0.96 (t, *J*=6.8 Hz, 3H).

### Example 357: Synthesis of Compound 457

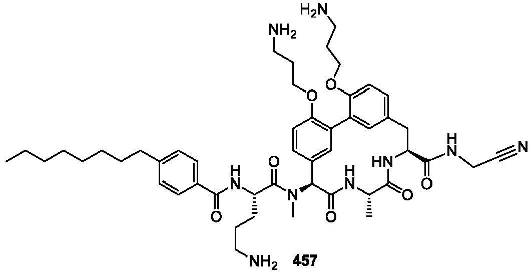

Compound 457 (formic acid salt) was prepared utilizing the methods previously described using tert-butyl (3-bromopropyl)carbamate in the phenol alkylation step. LCMS (Method 5-95AB): t_{R} = 0.772 minI 1.5 min, [M+H]⁺ = 896.6.

### Example 358: Synthesis of Compound 458

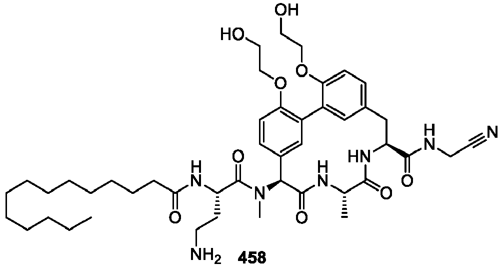

Compound 458 (formic acid salt) was prepared utilizing the methods in Example 7 (Compound 101) using (2-bromoethoxy)(tert-butyl)dimethylsilane in the phenol alkylation step. LCMS (Method 5-95AB): t_{R} = 0.723 minI 1.5 min, [M + H]⁺ = 850.8.

### Example 359: Synthesis of Compound 459

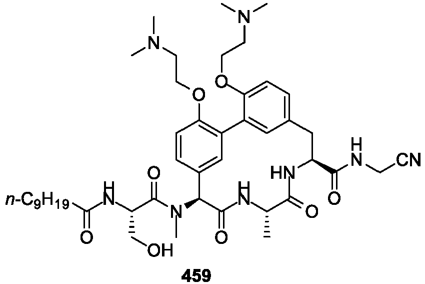

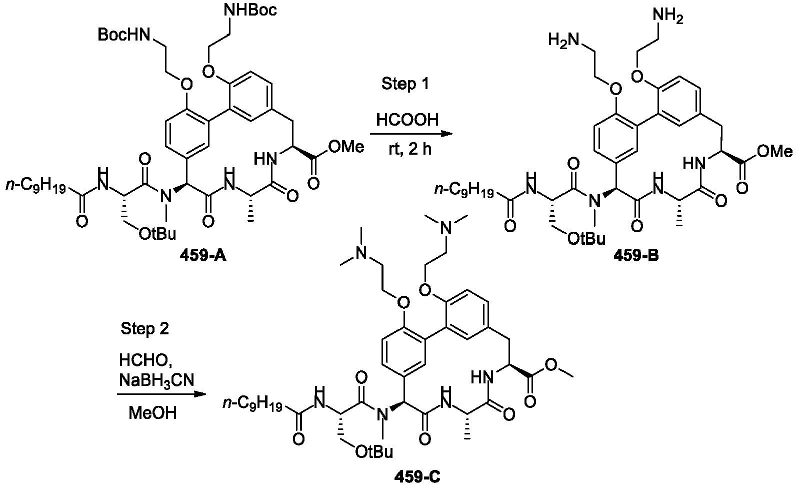

Compound 459-A was prepared utilizing utilizing the methods in Example 7.

Step 1: Compound 459-A (70 mg, 0.07 mmol) was treated according to General Method Formic Acid to afford Compound 459-B (40 mg, 67.4% yield) as a white solid. LCMS (5-95 AB, ESI): t_{R} = 0.678, [M + H]⁺ = 811.2.

Step 2: To a stirred solution of Compound 459-B (40 mg, 0.05 mmol) and HCHO (28 mg, 0.93 mmol) in MeOH (1 mL) was added Et₃N (14 mg, 0.14 mmol) and NaBH₃CN (59 mg, 0.93 mmol) and the mixture was stirred at room temperature for 1 h. The volatiles were removed and the residue was purified by prep-TLC to give Compound 459-C (20 mg, 49.4% yield) as a light yellow solid. LCMS (5-95 AB, ESI): t_{R} = 0.806, [M + H]⁺ = 867.9.

Starting from Compound 459-C, Compound 459 (formic acid salt) was prepared as a white solid utilizing the methods in Example 7 (Compound 101). LCMS (5-95 AB, ESI): t_{R} = 0.639, [M + H]⁺ = 835.3.

### Example 360: Synthesis of Compound 460

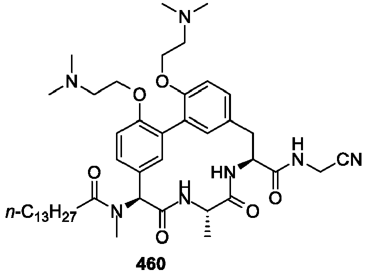

Compound 460 (formic acid salt) was prepared utilizing methods in Example 359 (Compound 459). LCMS (5-95 AB, ESI): t_{R} = 0.728, [M + H]⁺ = 804.4.

### Example 361: Synthesis of Compound 461

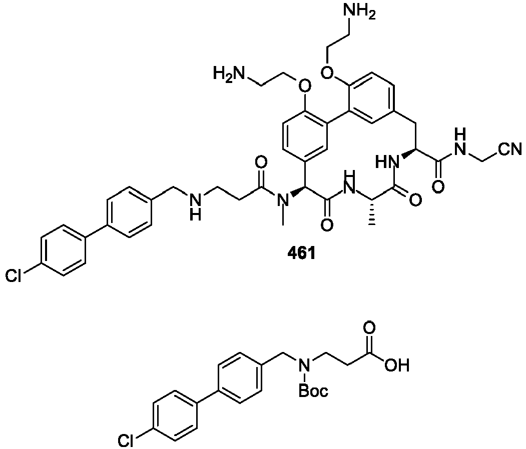

3-((Tert-butoxycarbonyl)((4'-chloro-[1,1'-biphenyl]-4-yl)methyl)amino)propanoic acid was prepared in a manner similar to 3-((tert-butoxycarbonyl)(decyl)amino)propanoic acid (Example 6). LCMS (5-95 AB, ESI): t_{R} = 1.015, [M+Na]⁺ = 412.1.

Compound 461 (formic acid salt) was prepared utilizing methods previously described. LCMS (5-95 AB, ESI): t_{R} = 0.586, [M + H]⁺ = 809.3.

### Example 362: Synthesis of Compound 462

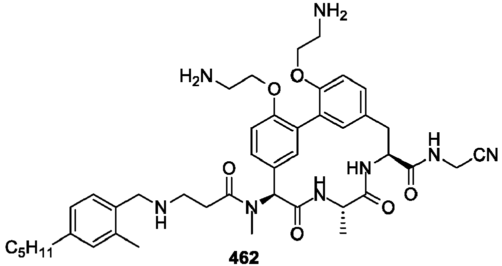

Compound 462 (formic acid salt) was prepared utilizing methods previously described. LCMS (5-95 AB, ESI): t_{R} = 0.747, [M + H]⁺ = 783.8.

### Example 363: Synthesis of Compound 463

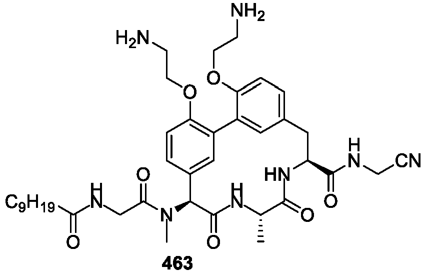

Compound 463 (formic acid salt) was prepared utilizing methods previously described from 2-decanamidoacetic acid. LCMS (5-95 AB, ESI): t_{R} = 0.601, [M + H]⁺ = 749.5.

### Example 364: Synthesis of Compound 464

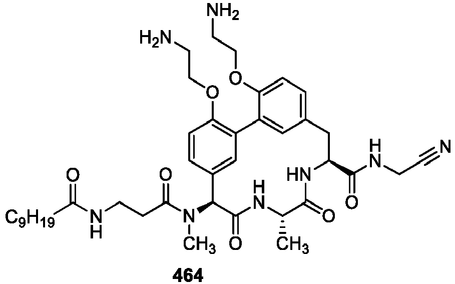

Compound 464 (formic acid salt) was prepared utilizing methods previously described from 3-decanamidopropanoic acid. LCMS (5-95 AB, ESI): t_{R} = 0.619, [M + H]⁺ = 763.3.

### Example 365: Synthesis of Compound 465

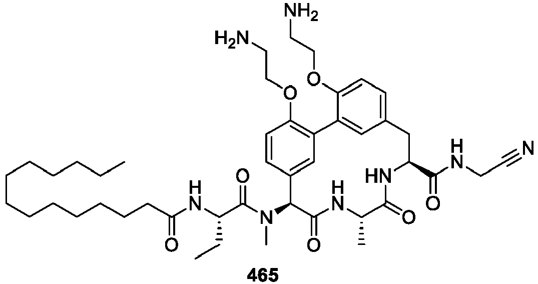

Compound 465 (formic acid salt) was prepared as a white solid utilizing methods previously described from (S)-2-(((benzyloxy)carbonyl)amino)butanoic acid. LCMS (Method 5-95AB): t_{R} = 0.864 minI 1.5 min, [M + H]⁺ = 833.4.

### Example 366: Synthesis of Compound 466

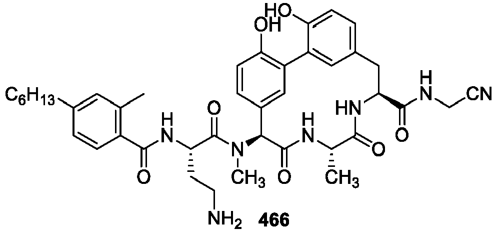

Compound 466 (formic acid salt) was prepared utilizing methods previously described. LCMS (5-95 AB, ESI): t_{R} = 0.835, [M + H]⁺ = 754.5

### Example 367: Synthesis of Compound 467

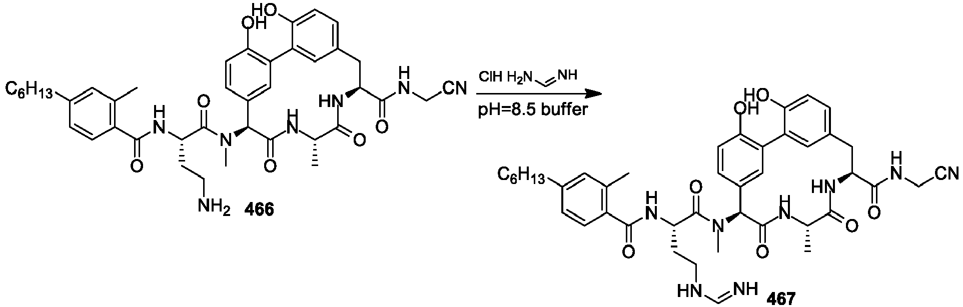

To a stirred solution of Compound 466 (20 mg, 0.03 mmol) in pH=7 buffer containing 1N NaH₂PO₃ and 1N Na₂HPO₃ was added 1N NaOH solution dropwise until pH=8.5, followed by the addition of imido-formamide hydrochloride (43 mg, 0.53 mmol). The resulting mixture was stirred at 0°C for 2 h. MeOH (2 mL) was added to the reaction mixture. After filtration, the filtrate concentrated and the residue was purified by HPLC to afford Compound 467 (1.6 mg, 7.7% yield) as a white solid. LCMS (5-95 AB, ESI): t_{R} = 0.838, [M + H]⁺ = 781.4; ¹H NMR (400 MHz, MeOH-*d*₄) δ 8.55 (brs, 1H), 7.86 (s, 1H), 7.36-7.29 (m, 1H), 7.13-6.93 (m, 6H), 6.92-6.86 (m, 1H), 6.85-6.79 (m, 1H), 6.40 (s, 1H), 5.16-5.08 (m, 1H), 4.79-4.74 (m, 2H), 4.19 (s, 2H), 3.52-3.41 (m, 2H), 3.18-3.00 (m, 2H), 2.92 (s, 3H), 2.62-2.50 (m, 2H), 2.38 (s, 3H), 2.29-2.07 (m, 2H), 1.65-1.50 (m, 2H), 1.40-1.21 (m, 9H), 0.90 (t, *J*=6.8 Hz, 3H).

### Example 368: Synthesis of Compound 468

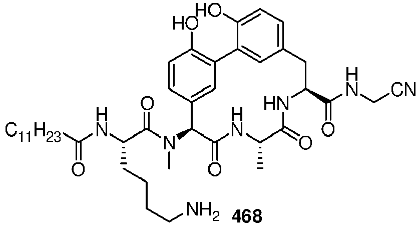

Compound 468 (formic acid salt) was prepared utilizing methods previously described. LCMS (5-95 AB, ESI): t_{R} = 0.842, [M + H]⁺ = 762.4.

### Example 369: Synthesis of Compound 469

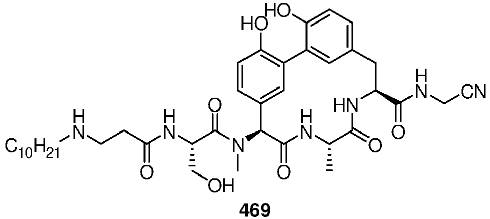

Compound 469 (formic acid salt) was prepared utilizing methods previously described. LCMS (5-95 AB, ESI): t_{R} = 0.671, [M + H]⁺ = 750.2.

### Example 370 : Synthesis of Compound 470

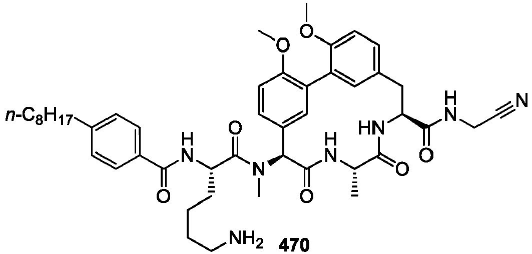

Compound 470 (formic acid salt) was prepared as a white solid utilizing methods previously described. LCMS (Method 5-95AB): t_{R} = 0.905 minI 1.5 min, [M+H]⁺ = 825.0.

### Example 371: Synthesis of Compound 471

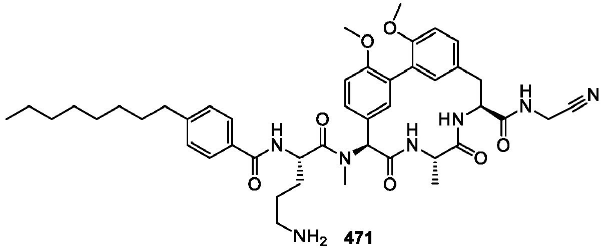

Compound 471 (formic acid salt) was prepared as a white solid utilizing the methods previously described. LCMS (Method 5-95AB): t_{R} = 0.905 minI 1.5 min, [M+H]⁺ = 810.5.

### Example 372: Synthesis of Compound 472

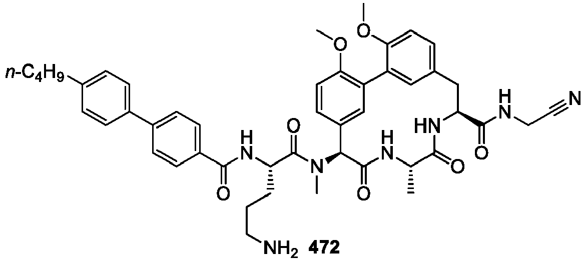

Compound 472 (formic acid salt) was prepared as a white solid utilizing the methods previously described. LCMS (Method 5-95AB): t_{R} = 0.876 minI 1.5 min, [M+H]⁺ = 831.6.

### Example 373: Synthesis of Compound 473

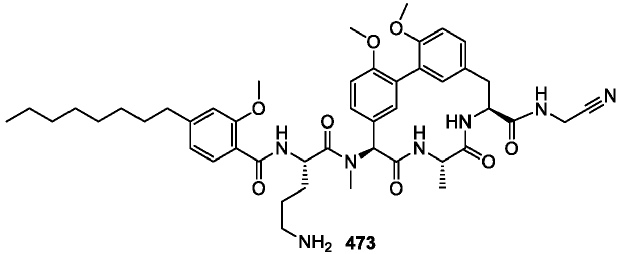

Compound 473 (formic acid salt) was prepared as a white solid utilizing the methods previously described. LCMS (Method 5-95AB): t_{R} = 0.895 minI 1.5 min, [M+H]⁺ = 840.6.

### Example 374: Synthesis of Compound 474

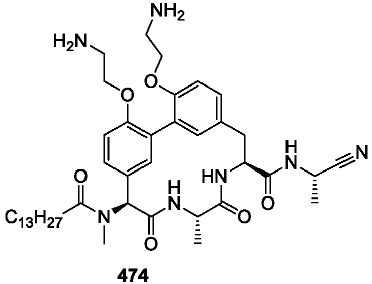

Compound 474 (formic acid salt) was prepared utilizing methods previously described. LCMS (Method 5-95AB): t_{R} = 0.838 minI 1.5 min, [M+H]⁺ = 762.5.

### Example 375: Synthesis of Compound 475

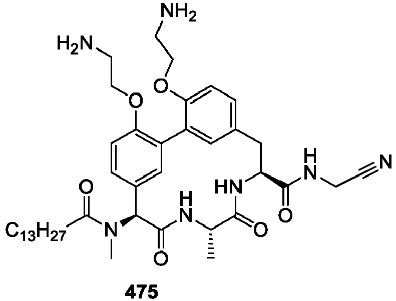

Compound 475 (formic acid salt) was prepared utilizing methods previously described. LCMS (Method 5-95AB): t_{R} = 0.848 min/ 1.5 min, [M+H]⁺ = 748.5.

### Example 376: Synthesis of Compound 476

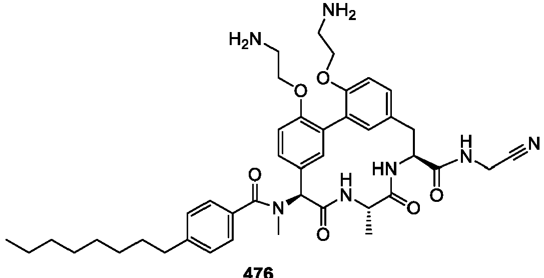

Compound 476 (formic acid salt) was prepared utilizing methods previously described. LCMS (Method 5-95AB): t_{R} = 0.821 minI 1.5 min, [M+H]⁺ = 754.5.

### Example 377: Synthesis of Compound 477

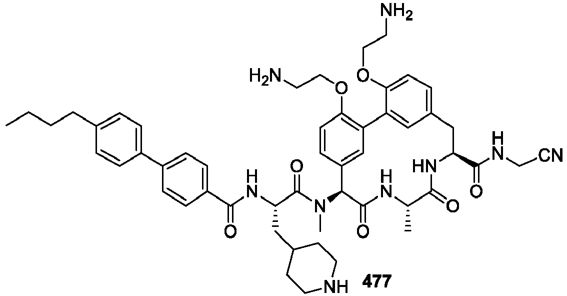

Compound 477 (formic acid salt) was prepared utilizing methods previously described from (S)-2-(((benzyloxy)carbonyl)amino)-3-(1-(tert-butoxycarbonyl)piperidin-4-yl)propanoic acid. LC-MS: m/z = 928 [M + H]⁺.

### Example 378: Synthesis of Compound

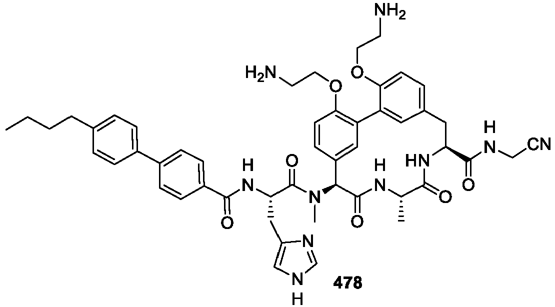

Compound 478 (formic acid salt) was prepared utilizing methods previously described from (S)-2-((((9H-fluoren-9-yl)methoxy)carbonyl)amino)-3-(1-trityl-1H-imidazol-4-yl)propanoic acid. LC-MS: m/z = 911 [M + H]⁺.

### Example 379: Synthesis of Compound 479

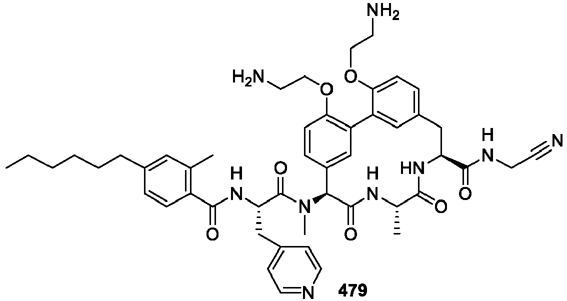

Compound 479 (formic acid salt) was prepared utilizing methods previously described from (S)-2-((((9H-fluoren-9-yl)methoxy)carbonyl)amino)-3-(pyridin-4-yl)propanoic acid. LC-MS: m/z = 888 [M + H]⁺.

### Example 380: Synthesis of Compound 480

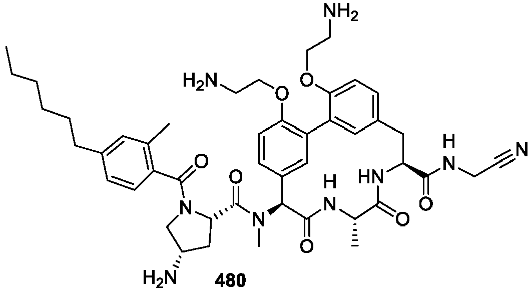

Compound 480 (formic acid salt) was prepared utilizing methods previously described from (2S,4S)-1-((benzyloxy)carbonyl)-4-((tert-butoxycarbonyl)amino)pyrrolidine-2-carboxylic acid. LC-MS: m/z = 852 [M + H]⁺.

### Example 381: Synthesis of Compound

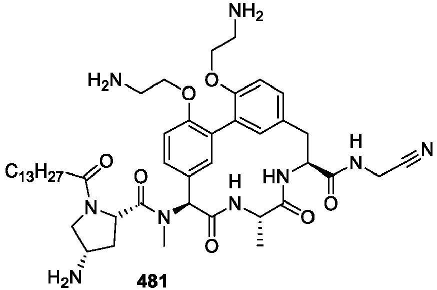

Compound 481 (formic acid salt) was prepared utilizing methods previously described from (2S,4S)-1-((benzyloxy)carbonyl)-4-((tert-butoxycarbonyl)amino)pyrrolidine-2-carboxylic acid. LC-MS: m/z = 860 [M + H]⁺.

### Example 382: Synthesis of Compound 482

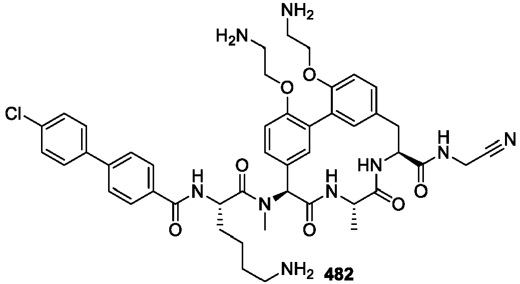

Compound 482 (formic acid salt) was prepared utilizing methods previously described. LCMS (ESI): [M + H]⁺ = 880.4.

### Example 383: Synthesis of Compound 483

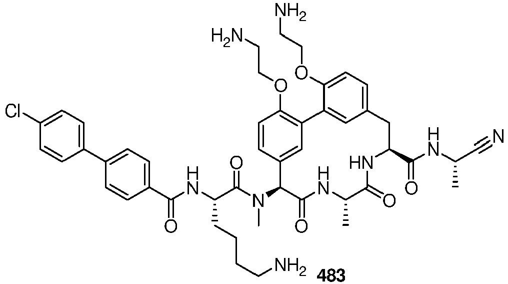

Compound 483 (formic acid salt) was prepared utilizing methods previously described. LCMS (ESI): [M + H]⁺ = 894.4.

### Example 384: Synthesis of Compound 484

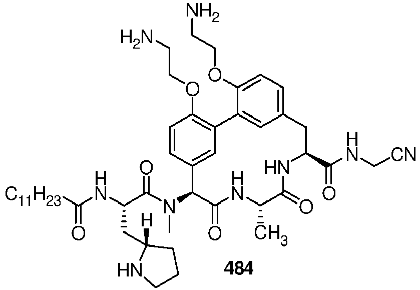

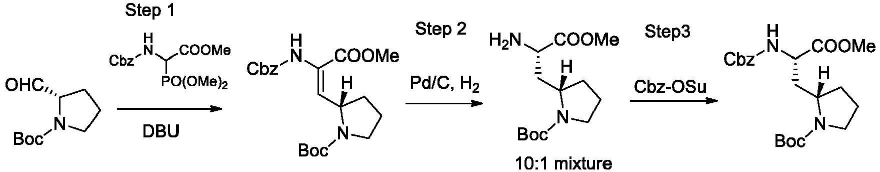

A solution of methyl 2-(((benzyloxy)carbonyl)amino)-2-(dimethoxyphosphoryl)acetate (1.0 g, 3.0 mmol) and DBU (0.35mL) in DCM (5 mL) was stirred at 0°C for 20 min, followed by the addition of a solution of (S)-tert-butyl 2-formylpyrrolidine-1-carboxylate (500 mg, 2.5 mmol) in DCM (5 mL). The resulting mixture was then warmed up to room temperature while stirring and stirred for 20 h at the same temperature. The volatiles were removed and the residue was purified by silica gel flash column to give (S,E)-tert-butyl 2-(2-(((benzyloxy)carbonyl)amino)-3-methoxy-3-oxoprop-1-en-1-yl)pyrrolidine-1-carboxylate (700 mg, 69% yield) as colorless oil. LCMS (Method 5-95 AB, ESI): t_{R} = 0.801, M+Na⁺ = 427.0

A solution of (S,E)-tert-butyl 2-(2-(((benzyloxy)carbonyl)amino)-3-methoxy-3-oxoprop-1-en-1-yl)pyrrolidine-1-carboxylate (500 mg, 1.24 mmol) and 10% Pd/C (132 mg, 0.12 mmol) in MeOH (10 mL) was stirred at room temperature for 2 h under H₂ (15 psi). The filtrate was concentrated and the residue was purified by HPLC (10mM NH₄HCO₃-ACN, 30%-50%) to give (S)-tert-butyl 2-((S)-2-(((benzyloxy)carbonyl)amino)-3-methoxy-3-oxopropyl)pyrrolidine-1-carboxylate (200 mg, 60% yield) as a white solid. Optical purity was confirmed by SFC. LCMS (Method 5-95 AB, ESI): t_{R} = 0.639, [M + H]⁺ = 272.9

To a solution of (S)-tert-butyl 2-((S)-2-(((benzyloxy)carbonyl)amino)-3-methoxy-3-oxopropyl)pyrrolidine-1-carboxylate (900 mg, 3.3 mmol) in DCM (10 mL) was added Et3N (501 mg, 5.0 mmol), followed by the addition of a solution of Cbz-OSu (820 mg, 3.3 mmol) in DCM (25 mL). The resulting mixture was stirred at room temperature for 16 h. The volatiles were removed and the residue was purified by silica-gel flash column to give (S)-tert-butyl 2-((S)-2-(((benzyloxy)carbonyl)amino)-3-methoxy-3-oxopropyl)pyrrolidine-1-carboxylate (900 mg, 67% yield) as colorless oil.

Compound 484 (formic acid salt) was prepared utilizing methods previously described. LCMS (Method 5-95 AB, ESI): t_{R} = 0.780, [M + H]⁺ = 860.4; ¹H NMR (400 MHz, MeOH-*d*₄) δ 8.50 (brs, 3H, HCOOH), 7.33-7.23 (m, 2H), 7.18 (d, *J*=8.4 Hz, 1H), 7.11 (d, *J*=8.4 Hz, 1H), 6.93 (brs, 1H), 6.79 (brs, 1H), 6.28 (s, 1H), 4.35-4.14 (m, 4H), 4.21 (s, 2H), 3.63-3.48 (m, 1H), 3.26-3.04 (m, 5H), 2.85 (s, 3H), 2.40-1.94 (m, 8H), 1.86-1.56 (m, 4H), 1.47-1.19 (m, 20H), 0.92 (t, *J*=6.4 Hz, 3H).

### Example 385: Synthesis of Compound 485

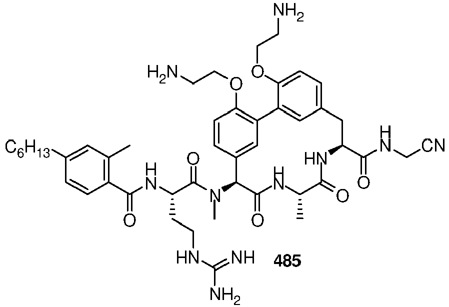

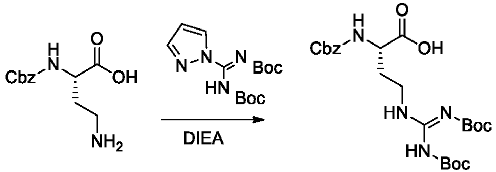

To a stirred solution of (S)-4-amino-2-(((benzyloxy)carbonyl)amino)butanoic acid (200 mg, 0.8 mmol) in DMF (5 mL) was added tert-butyl (((tert-butoxycarbonyl)amino)(1H-pyrazol-1-yl)methylene)carbamate (492 mg, 1.6 mmol) and DIEA (205 mg, 1.6 mmol) and the mixture was stirred at room temperature for 3 h. The volatiles were removed and the residue was purified by silica gel flash column to afford (S)-2-(((benzyloxy)carbonyl)amino)-4-(2,3-bis(tert-butoxycarbonyl)guanidino)butanoic acid (220 mg, 56.1% yield) as a white solid. LCMS (Method 5-95 AB, ESI): t_{R} = 0.894, [M + H]⁺ = 495.1

Compound 485 (formic acid salt) was prepared utilizing methods previously described. LCMS (Method 5-95 AB, ESI): t_{R} = 0.757, [M + H]⁺ = 882.4.

### Example 386: Synthesis of Compound 486

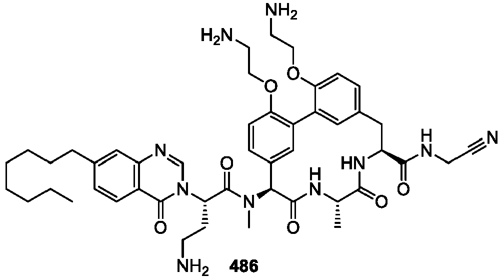

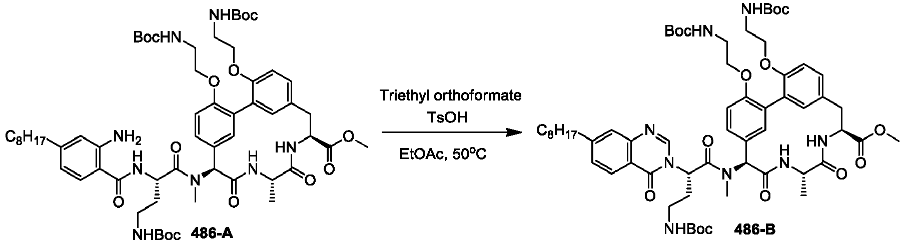

Compound 486-A is an intermediate in the preparation of Compound 223 (Example 129).

A mixture of Compound 486-A (100.0 mg, 0.09 mmol), triethylorthoformate (14.2 mg, 0.10 mmol), and p-toluenesulfonic acid (1.5 mg, 0.01 mmol) in ethyl acetate (5 mL) was stirred at 50°C for 2 h. The mixture was concentrated and purified by prep-TLC (7% methanol in dichloromethane, Rf = 0.5) to obtain Compound 486-A (70 mg, 69.4% yield) as a white solid. LCMS (Method 5-95 AB, ESI): t_{R} = 1.139 min, [M + H]⁺ = 1156.0.

Compound 486 (formic acid salt) was prepared as a white solid utilizing the methods in Example 7 (Compound 101). LCMS (Method 5-95 AB, ESI): t_{R} = 0.759 min, [M + H]⁺ = 879.9.

### Example 387: Synthesis of Compound 487

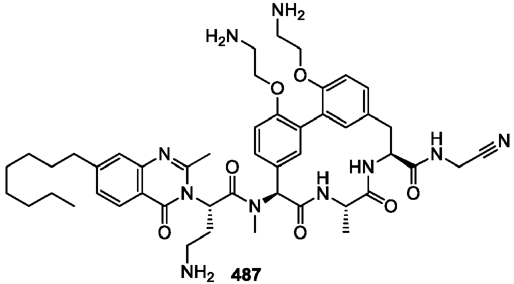

Compound 487 (formic acid salt) was prepared as a white solid utilizing the methods in Example 386 (Compound 486) except that triethyl orthoacetate is used in the cyclization step. LCMS (Method 5-95 AB, ESI): t_{R} = 0.760 min, [M + H]⁺ = 893.5.

### Example 388: Synthesis of Compound 488

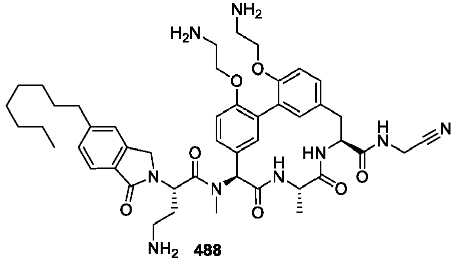

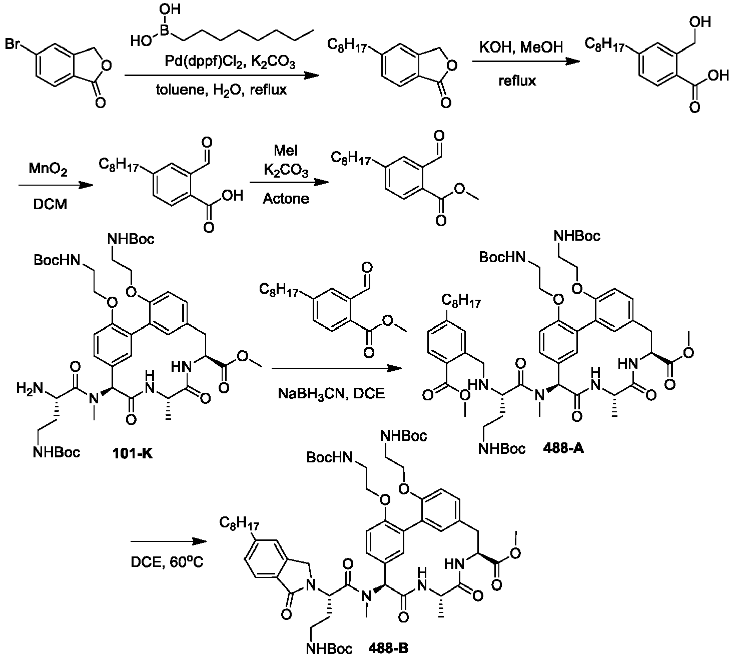

To a solution of 5-octyl-3H-isobenzofuran-1-one (200.0 mg, 0.81 mmol) in methanol (8.5 mL) and water (1.5 mL) was added potassium hydroxide (68.33 mg, 1.22 mmol). The mixture was stirred at 80 °C for 3 h and concentrated. The residue was taken up in EtOAc (20 mL), washed with water (20 mL x 2) and brine (20 mL), dried over MgSO₄ and concentrated to give crude 2-(hydroxymethyl)-4-octyl-benzoic acid (200 mg, 93.2% yield) as a white solid.

A mixture of 2-(hydroxymethyl)-4-octyl-benzoic acid (189.9 mg, 0.72 mmol), manganese dioxide (187.4 mg, 2.16 mmol) in dichloromethane (5 mL) was stirred at 20 °C for 16 h and filtered. The filtrate was concentrated and purified by flash column chromatography (5% methanol in DCM, Rf = 0.4) to afford 2-formyl-4-octyl-benzoic acid (150 mg, 79.6% yield) as a white solid.

A mixture of 2-formyl-4-octyl-benzoic acid (100.0 mg, 0.38 mmol), iodomethane (108.2 mg, 0.76 mmol) and potassium carbonate (158.1 mg, 1.14 mmol) in N,N-dimethylformamide (3 mL) was stirred at 20 °C for 16 h. The reaction was diluted with EtOAc (20 mL), washed with water (20 mL x 2) and brine (20 mL), dried over MgSO₄ and concentrated. The crude was purified by flash column chromatography (10% ethyl acetate in petroleum ether, Rf = 0.5) to afford methyl 2-formyl-4-octyl-benzoate (100 mg, 94.9% yield) as a colorless oil. ¹H NMR (400 MHz, CDC1₃): δ 10.65 (s, 1H), 7.91 (d, J = 8.0 Hz, 1H), 7.75 (s, 1H) ,7.46-7.43 (m, 1H), 3.97 (s, 3H), 2.70 (t, J = 7.6 Hz, 2H), 1.66-1.60 (m, 2H), 1.30-1.24 (m, 10H), 0.88 (t, J = 6.8 Hz, 3H).

**To** a solution of Compound 101-K (100.0 mg, 0.11 mmol), methyl 2-formyl-4-octyl-benzoate (60.47 mg, 0.22 mmol) and acetic acid (0.30 mL) in methanol (10 mL) was added sodium cyanoborohydride (8.3 mg, 0.13 mmol). The solution was stirred at 20 °C for 6 h and concentrated. The residue was taken up in EtOAc (20 mL), washed with water (20 mL x 2) and brine (20 mL), dried over MgSO₄ and concentrated. The crude was purified by prep-TLC (66% EtOAc in petroleum ether, Rf = 0.4) to afford Compound 488-A (100 mg, 77.8% yield) as a white solid. LCMS (Method 5-95 AB, ESI): t_{R} = 0.888 min, [M + H]⁺ = 1174.6.

A solution of Compound 488-A (100.0 mg, 0.09 mmol) in 1,2-dichloroethane (5 mL) was stirred at 60 °C for 16 h and concentrated to afford crude Compound 488-B (99 mg) as a white solid. LCMS (Method 5-95 AB, ESI): t_{R} = 1.015 min, [M + H]⁺ = 1164.9.

Compound 488 (formic acid salt) was prepared utilizing methods previously described. LCMS (Method 5-95 AB, ESI): t_{R} = 0.633 min, [M + H]⁺ = 866.5.

### Example 389: Synthesis of Compound 489

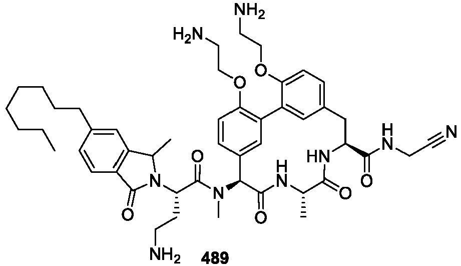

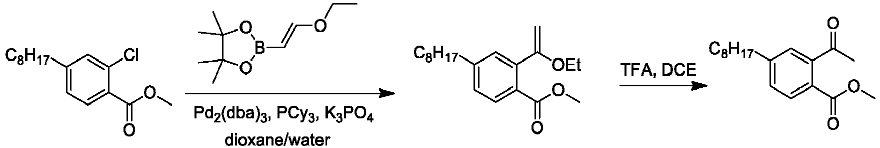

Step 1: A mixture of methyl 2-chloro-4-octyl-benzoate (160.0 mg, 0.57 mmol), tricyclohexylphosphine (47.6 mg, 0.17 mmol), tris(dibenzylideneacetone)dipalladium(0) (51.8 mg, 0.06 mmol), (E)-1-ethoxyethene-2-boronic acid pinacol ester (123.3 mg, 0.62 mmol) and potassium phosphate tribasic (240.2 mg, 1.13 mmol) in 1,4-dioxane (4 mL) was stirred at 100 °C for 16 h and concentrated. The residue was taken up in EtOAc (20 mL), washed with water (20 mL x 2) and brine (20 mL), dried over MgSO₄ and concentrated. The crude was purified by flash column chromatography (20% ethyl acetate in petroleum ether, Rf = 0.7) to afford presumably methyl 2-(1-ethoxyvinyl)-4-octylbenzoate (150 mg, 83.3% yield) as a yellow oil, instead of expected regioisomer.

Step 2: To a solution of methyl 2-(1-ethoxyvinyl)-4-octylbenzoate (150.0 mg, 0.47 mmol) in dichloromethane (5 mL) was added trifluoroacetic acid (0.21 mL, 2.83 mmol), stirred at 20 °C for 2 h and concentrated. The residue was taken up in EtOAc (20 mL), washed with water (10 mL x 2) and brine (10 mL), dried over MgSO₄ and concentrated to give crude methyl 2-acetyl-4-octylbenzoate (130 mg, 95% yield) as a yellow oil.

Compound 489 (formic acid salt) was prepared utilizing the methods in Example 388 (Compound 488). LCMS (Method 5-95 AB, ESI): t_{R} = 0.676 min, [M+Na]⁺ = 902.5.

### Example 390: Synthesis of Compound 490-P1 and Compound 490-P2

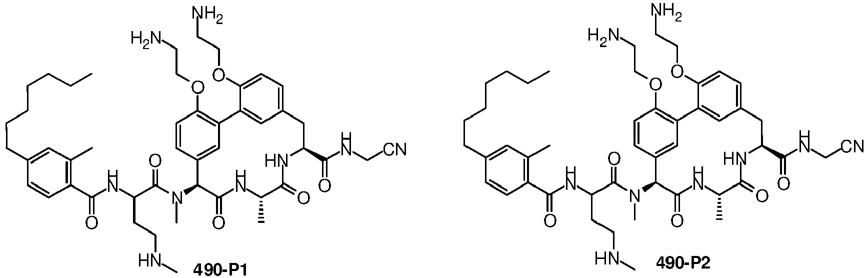

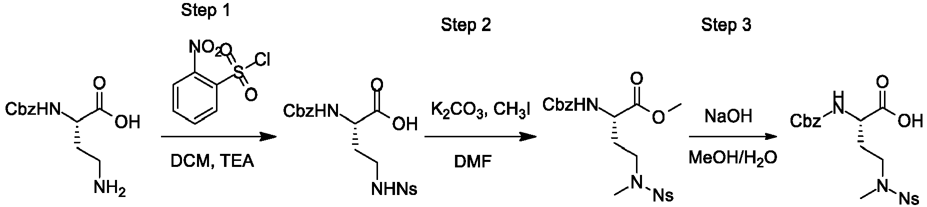

Step 1: To a solution of (S)-4-amino-2-(((benzyloxy)carbonyl)amino)butanoic acid (2000.0 mg, 7.93 mmol) in N,N-dimethylformamide (50 mL) were added 2-nitrobenzenesulfonylchloride (0.46 mL, 23.78 mmol) and triethylamine (4.42 mL, 31.71 mmol) dropwise. The reaction mixture was stirred at 25 °C for 12 h and filtered. To the filtrate was added water (50 mL) and the resulting precipitate was collected to obtain (S)-2-(((benzyloxy)carbonyl)amino)-4-(2-nitrophenylsulfonamido)butanoic acid (2000 mg, 4.5723 mmol, 57.7% yield) as a white solid. LCMS (Method 5-95 AB, ESI): RT = 0.790 min, [M + H]⁺ = 437.0.

Step 2: To a solution of (S)-2-(((benzyloxy)carbonyl)amino)-4-(2-nitrophenylsulfonamido)butanoic acid (800.0 mg, 1.83 mmol) in N,N-dimethylformamide (5 mL) were added iodomethane (4.59 mL, 73.43 mmol) and potassium carbonate (758.3 mg, 5.49 mmol). The mixture was stirred at 25°C for 14 h, diluted with H₂O (20 mL) and extracted with EtOAc (35 mL x 3). The combined organic layers were washed with water (30 mL x4) and brine (40 mL), dried over Na₂SO₄ and concentrated. The residue was purified by flash column chromatography (30% EtOAc in petroleum ether, Rf = 0.3) to obtain (S)-methyl 2-(((benzyloxy)carbonyl)amino)-4-(N-methyl-2-nitrophenylsulfonamido)butanoate (610 mg, 71.7% yield) as a yellow oil. LCMS (Method 5-95 AB, ESI): RT = 0.813 min, [M + H]⁺ = 466.1.

Steps 3 and 4: Standard NaOH hydrolysis of the ester afforded (S)-2-(((benzyloxy)carbonyl)amino)-4-(N-methyl-2-nitrophenylsulfonamido)butanoic acid was followed by standard HATU coupling (Example 5) afforded Compound 390-A.

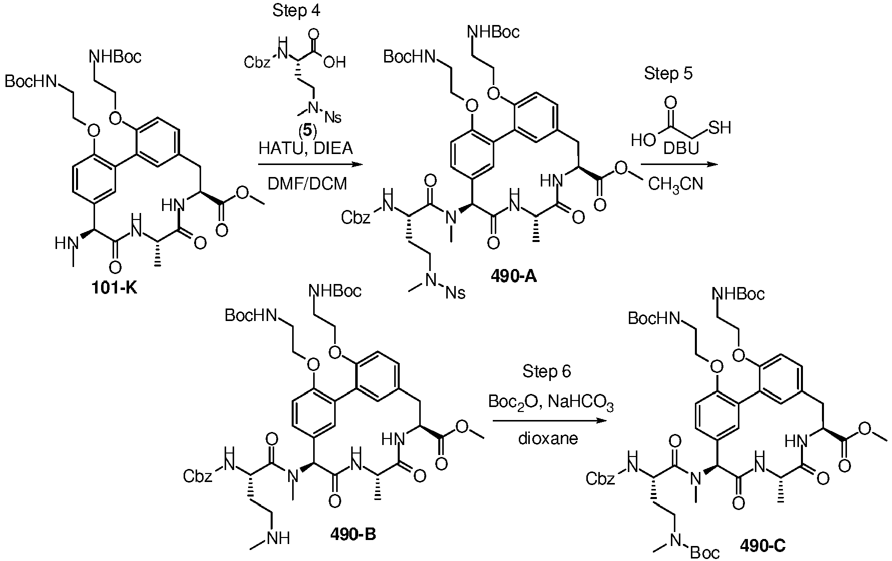

Step 5: To a solution of Compound 490-A (220.0 mg, 0.19 mmol) in acetonitrile (3 mL) were added thioglycolic acid (0.09 mL, 1.27 mmol) and DBU (0.29 mL, 1.94 mmol). The reaction mixture was stirred at 25 °C for 4 h, diluted with H₂O (20 mL) and extracted with EtOAc (20 mL x 2). The combined organic layers were washed with saturated aq. NaHCO₃ (40 mL x 2), water (40 mL x 2) and brine (40 mL), dried over Na₂SO₄ and concentrated. The residue was purified by preparative TLC (10% methanol in dichloromethane, Rf = 0.4) to obtain Compound 490-B (170 mg, 92.1% yield) as yellow solid. LCMS (Method 5-95 AB, ESI): RT = 0.720 min, [M + H]⁺ = 962.5.

Step 6: Standard Boc-conditions afforded Compound 490-C.

Compound 490-P1 and Compound 490-P2 (formic acid salt) was prepared utilizing methods previously described and were isolated as pure diastereomers by reverse phase HPLC. Data for Compound 490-P1: LCMS (Method 5-95 AB, ESI): RT = 0.642 min, [M + H]⁺ = 868.6. Data for Comound 490-P2: LCMS (Method 5-95 AB, ESI): RT = 0.658 min, [M + H]⁺ = 868.6.

### Example 391: Synthesis of Compound 491

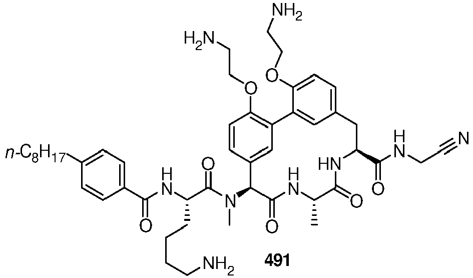

Compound **491** (formic acid salt) was prepared as a white solid utilizing methods previously described using octylbenzoic acid. LCMS (Method 5-95AB): t_{R} = 0.756 minI 1.5 min, [M + H]⁺ = 882.5.

### Example 392: Synthesis of Compound 492

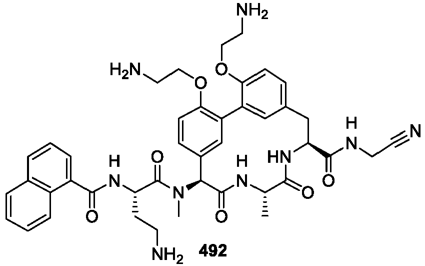

Compound 492 (formic acid salt) was prepared utilizing methods previously described . LC-MS: m/z = 792 (M+H).

### Example 393: Synthesis of Compound 493

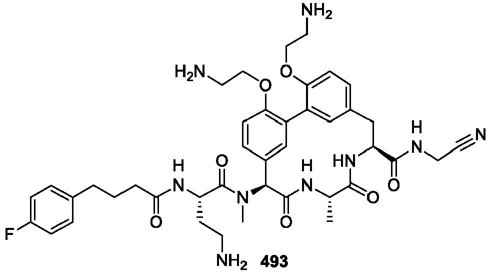

Compound 493 (formic acid salt) was prepared utilizing methods previously described . LC-MS: m/z = 802 (M+H).

### Example 394: Synthesis of Compound 494

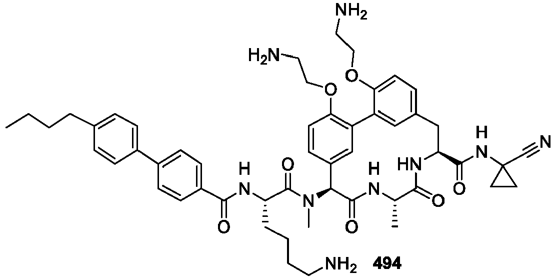

Compound 494 (formic acid salt) was prepared utilizing methods previously described from 1-aminocyclopropanecarbonitrile hydrochloride. LC-MS: m/z = 928 (M+H).

### Example 395: Synthesis of Compound 495

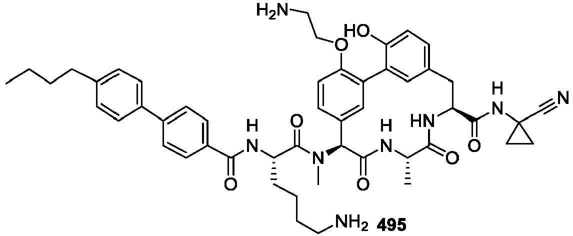

Compound 495 (formic acid salt) was prepared utilizing methods previously described from 1-aminocyclopropanecarbonitrile hydrochloride. LC-MS: m/z = 885 (M+H).

### Example 396: Synthesis of Compound 496

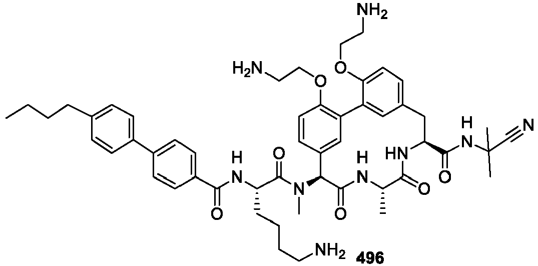

Compound 496 (formic acid salt) was prepared utilizing methods previously described from 2-amino-2-methyl-propanenitrile. LC-MS: m/z = 930 (M+H).

### Example 397: Synthesis of Compound 497-P1 and Compound 497-P2

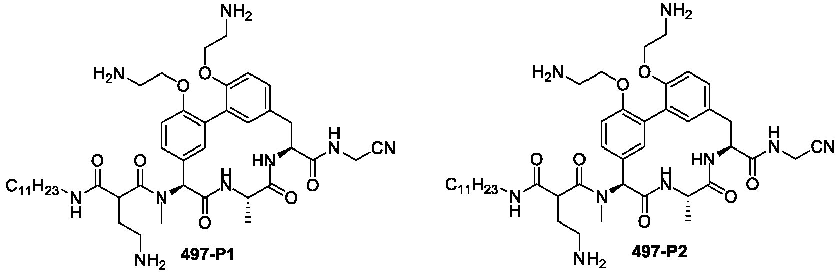

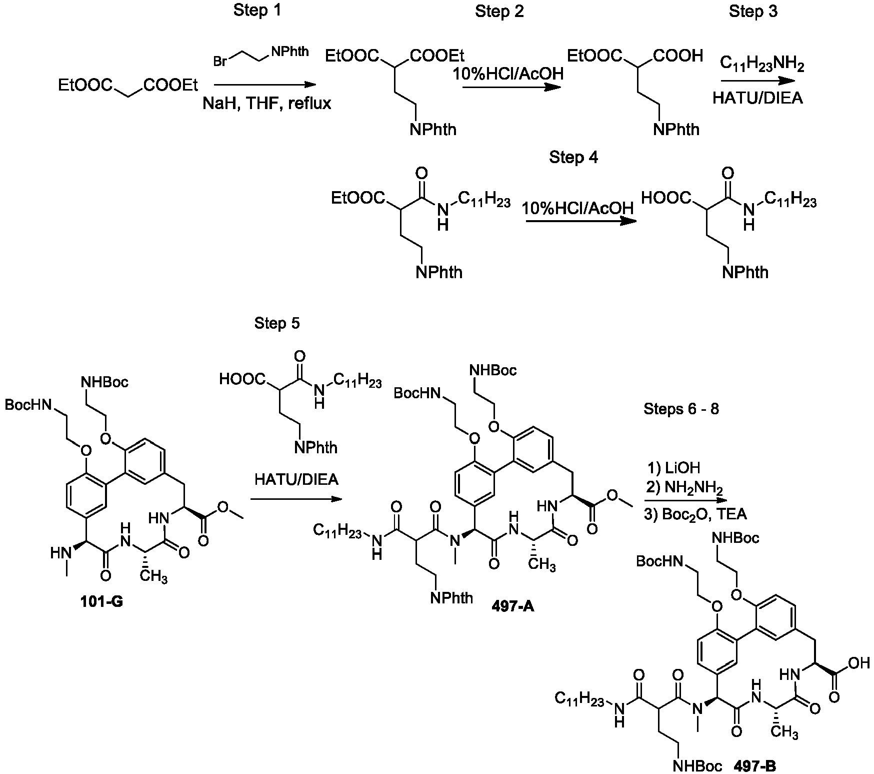

To a stirred solution of 60% NaH (1.0 g, 25 mmol) in THF (30 mL) was added 2-(2-bromoethyl)isoindoline-1,3-dione (5.6 g, 25 mmol) and the mixture was stirred at 0°C for 0.5 h, followed by the addition of diethyl malonate (4.0 g, 25 mmol). The resulting mixture was then stirred at room temperature for additional 16 h. The reaction mixture was diluted with EtOAc (100 mL), which was washed with brine (100 mL x 3). The organic layer was dried over Na₂SO₄, concentrated and the residue was purified on silica gel flash column to afford compound diethyl 2-(2-(1,3-dioxoisoindolin-2-yl)ethyl)malonate (1.2 g, 15.8% yield) as light yellow oil.

To a stirred solution of diethyl 2-(2-(1,3-dioxoisoindolin-2-yl)ethyl)malonate (1.0 g, 3.0 mmol) in HOAc (9.0 mL) was added 10% HCl solution (3.0 mL) and the mixture was heated to 90°C while stirring and stirred for 2 h. The volatiles were removed and the residue was taken up in EtOAc (50 mL), which was washed with brine (30 mL x 2). The organic layer was dried over MgSO4, concentrated and the residue was purified by HPLC to afford 4-(1,3-dioxoisoindolin-2-yl)-2-(ethoxycarbonyl)butanoic acid (250 mg, 27.3% yield) as yellow oil. LCMS (Method 5-95 AB, ESI): t_{R} = 0.941, [M + H]⁺ = 306.1.

Typical amide coupling (HATU, Example 5)) and ester hydrolysis with HOAc/HCl procedure was applied to afford 4-(1,3-dioxoisoindolin-2-yl)-2-(undecylcarbamoyl)butanoic acid (150 mg) as a white solid. LCMS (Method 5-95 AB, ESI): t_{R} = 1.327, [M + H]⁺ = 431.3

Typical amide coupling (HATU, Example 5)) procedure was applied to Compound 101 - G (150 mg, 0.21 mmol) and 4-(1,3-dioxoisoindolin-2-yl)-2-(undecylcarbamoyl)butanoic acid (90 mg, 0.21 mmol) to afford Compound 497-A (220 mg, 93% yield) as a white solid.

Typical ester hydrolysis (LiOH/THF) procedure was applied to Compound 497-A (150 mg, 0.13 mmol) to yield the crude product. A solution of the product, hydrazine monohydrate (27 mg, 0.53 mmol) in MeOH (2 mL) was stirred at 80 °C for 16 h. The reaction mixture was diluted with DCM (30 mL), which was washed with brine (30 mL x 2). The organic layer was dried over Na₂SO₄, concentrated to give a residue. And the typical Boc addition procedure was applied to the residue to afford Compound 497-B (60 mg) as a white solid. LCMS (Method 5-95 AB, ESI): t_{R} = 1.104, [M + H]⁺ = 1082.7.

Compound 497-P1 (4.4 mg) and Compound 497-P2 (1.9 mg) (formic acid salt) was prepared utilizing methods previously described and were isolated as separated diastereomers. Data for Compound 497-P1 and Compound 497-P2: LCMS (Method 5-95 AB, ESI): t_{R} = 0.766, [M + H]⁺ = 821.0 (both are identical under these HPLC conditions).

### Example 398: Synthesis of Compound 498

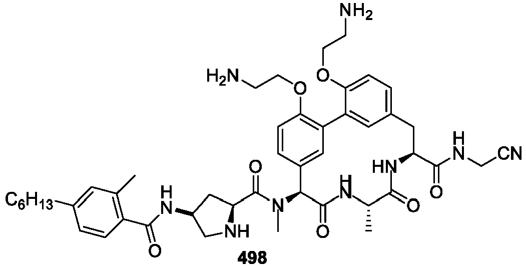

Compound 498 (free base) was prepared utilizing methods previously described . LCMS (Method 5-95 AB, ESI): RT = 0.759, [M + H]⁺ = 852.5.

### Example 399: Synthesis of Compound 499

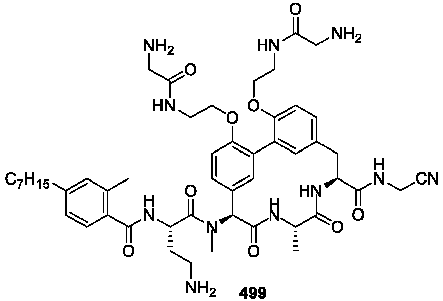

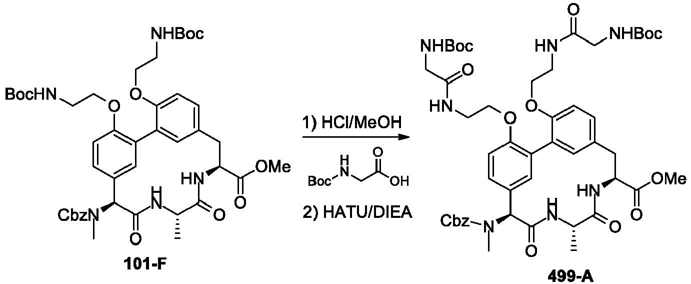

Starting from Compound 101-F (Example 4), typical procedure including Boc removal (HCl/MeOH), amide coupling with 2-((tert-butoxycarbonyl)amino)acetic acid (HATU/DIEA), affords Compound 499-A.

Compound 499 (formic acid salt) was prepared utilizing methods previously described. LCMS (Method 5-95 AB, ESI): RT = 0.751, [M + H]⁺ = 969.7; ¹H NMR (400 MHz, MeOH-*d*₄) δ 8.52 (brs, 3H), 7.34 (d, *J*=8.0 Hz, 2H), 7.28 (d, *J*=8.0 Hz, 1H), 7.21 (d, 7=8.0 Hz, 1H), 7.12-7.04 (m, 4H), 6.85 (brs, 1H), 6.73 (brs, 1H), 6.39 (s, 1H), 5.17-5.13(m, 1H), 4.84-4.79 (m, 2H), 4.22 (s, 2H), 4.20-4.05 (m, 6H), 3.58-3.43(m, 8H), 3.17-3.10 (m, 3H), 2.97 (s, 3H), 2.62 (t, *J*=6.8 Hz, 2H), 2.41 (s, 3H), 2.33-2.24 (m, 1H), 2.19-2.10 (m, 1H), 1.68-1.51(m, 2H), 1.40-1.25 (m, 11H), 0.92 (t, *J*=6.8 Hz, 3H).

### Example 400: Synthesis of Compound 500

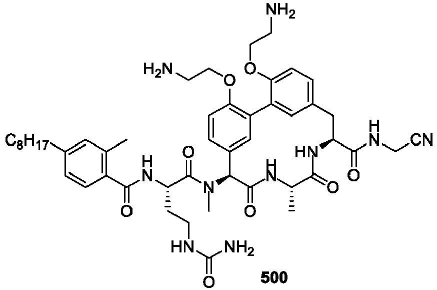

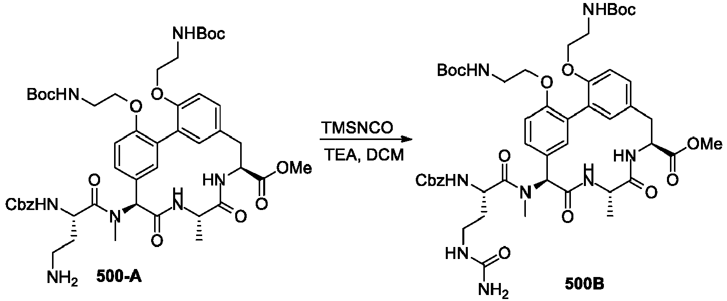

Compound 500-A is made utilizing the methods in Example 5 except (S)-4-((((9H-fluoren-9-yl)methoxy)carbonyl)amino)-2-(((benzyloxy)carbonyl)amino)butanoic acid is used in the coupling followed by Fmoc deprotection with piperidine.

To a solution of Compound 500-A (120 mg, 0.13 mmol) and Et₃N (53 µL, 0.38 mmol) in DCM (5 mL) was added trimethylsilyl isocyanate (44 mg, 0.38 mmol) at 0°C. The resulting mixture was warmed up to room temperature while stirring and stirred at the same temperature for 2 h. The volatiles were removed and the residue was purified by prep-TLC to afford Compound 500-B (90 mg, 72% yield) as a white solid. LCMS (Method 5-95 AB, ESI): RT = 0.759, [M + H]⁺ = 991.7.

Starting from Compound 500-B, typical hydrogenation, amide coupling (HATU/DIEA) and global Boc removal (TFA/HFIP) procedure (Examples 5 and 7) was followed to afford Compound 500 as a white solid. LCMS (Method 5-95 AB, ESI): RT = 0.803, [M + H]⁺ = 911.6; ¹H NMR (400 MHz, MeOH-*d*₄) δ 8.51 (brs, 2H), 7.42-7.31 (m, 2H), 7.26 (d, *J*=8.4 Hz, 1H), 7.19 (d, *J*=8.4 Hz, 1H), 7.14-7.03 (m, 3H), 6.90 (brs, 1H), 6.81 (brs, 1H), 6.46 (s, 1H), 5.02-4.98 (m, 1H), 4.81-4.76 (m, 2H), 4.33-4.22 (m, 4H), 4.21 (s, 2H), 3.43-3.35 (m, 2H), 3.28-3.14 (m, 6H), 2.98 (s, 3H), 2.61 (t, *J*=7.6 Hz, 2H), 2.43 (s, 3H), 2.17-2.03 (m, 1H), 1.97-1.84 (m, 1H), 1.62 (brs, 2H), 1.39 - 1.26 (m, 13H), 0.92 (t, *J*=6.8 Hz, 3H).

### Example 401: Synthesis of Compound 501

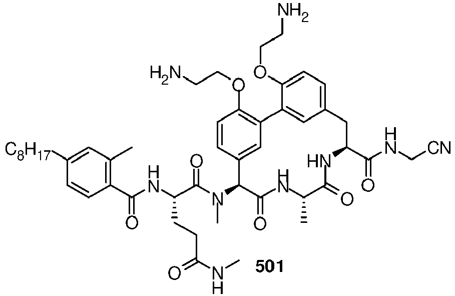

Compound 501 (formic acid salt) was prepared utilizing the methods previously described. LCMS (Method 5-95 AB, ESI): RT = 0.805, [M + H]⁺ = 910.5

### Example 402: Synthesis of Compound 502

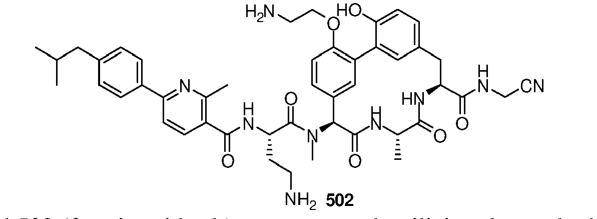

Compound 502 (formic acid salt) was prepared utilizing the methods previously described. LCMS (Method 5-95AB): t_{R} = 0.729, [M + H]⁺ = 846.5.

### Example 403: Synthesis of Compound 503

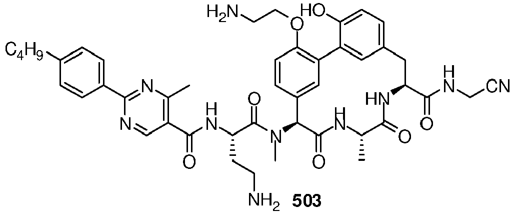

Compound 503 (formic acid salt) was prepared utilizing the methods previously described. LCMS (Method 5-95AB): t_{R} = 0.756, [M + H]⁺ = 847.6

### Example 404: Synthesis of Compound 504

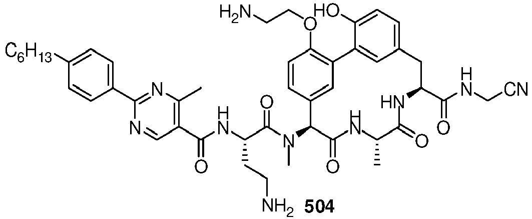

Compound 504 (formic acid salt) was prepared utilizing the methods previously described. LCMS (Method 5-95AB): t_{R} = 0.793, [M + H]⁺ = 875.6.

### Example 405: Synthesis of Compound 505

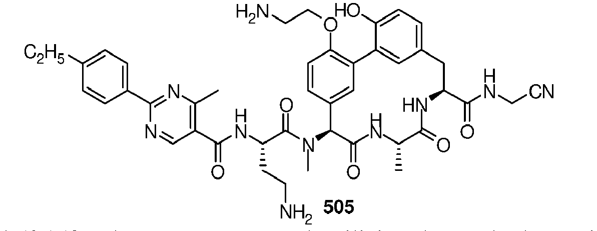

Compound 505 (free base) was prepared utilizing the methods previously described. LCMS (Method 5-95AB): t_{R} = 0.719, [M + H]⁺ = 819.4.

### Example 406: Synthesis of Compound 506

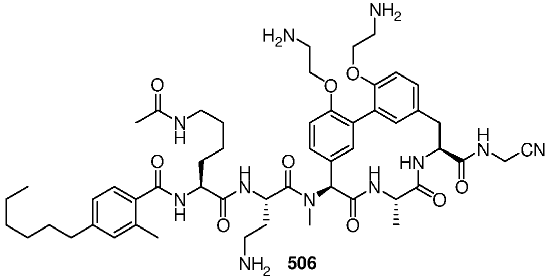

Compound 506 (free base) was prepared utilizing the methods previously described . MS (ESI) for (C₅₃H₇₅N₁₁O₉): *m*/*z* 1010.7 [M + H]⁺. HPLC t_{R} 2.73 min (10% AcCN/H₂O - 90% AcCN/H₂O, 3.0 min, 1.0 mL/min Kinetix C18, 4.8 x 50 mm).

### Example 407: Synthesis of Compound 507

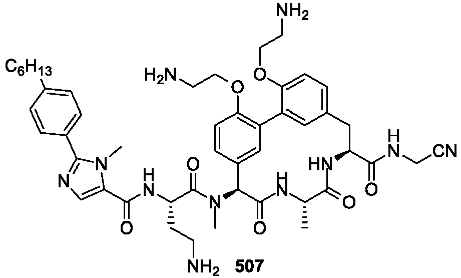

Compound 507 (formic acid salt) was prepared utilizing the methods previously described. LCMS (Method 5-95AB): t_{R} = 0.722, [M + H]⁺ = 906.7.

### Example 408: Synthesis of Compound 508

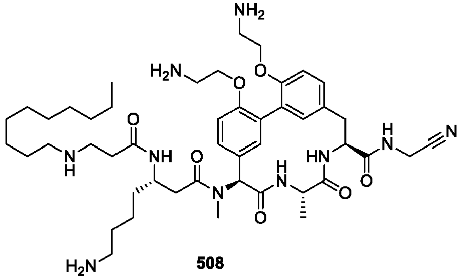

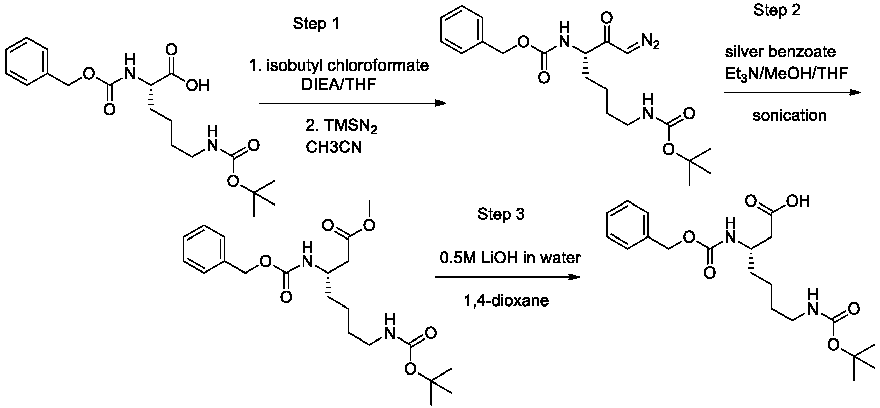

Step 1: Synthesis of (S)-3-(((benzyloxy)carbonyl)amino)-7-((tert-butoxycarbonyl)amino)heptanoic acid. Isobutyl chloroformate (0.81 mL, 6.2 mmol) in anhydrous THF (5 mL) was added to a solution of (2S)-2-(benzyloxycarbonylamino)-6-(tert-butoxycarbonylamino)hexanoic acid (2282 mg, 6.0 mmol) and DIEA (0.68 mL, 6.2 mmol) in THF (30 mL) at -15°C, and the mixture was stirred for 30 minutes at -5°C. Acetonitrile (20 mL) and (trimethylsilyl)diazomethane (2.0 mol/L) in hexane (6.0 mL) were added to the reaction mixture and the mixture was stirred at room temperature overnight (18 hours).

Step 2: Diethyl ether (80 mL) was added and the mixture was extracted with 10 % aq. citric acid, sat. aq. NaHCO₃ and sat. aq. NaCl (60 mL each). The organic layer was dried over MgSO₄ and the solvents evaporated to give crude tert-butyl N-[(5S)-5-(benzyloxycarbonylamino)-7-diazo-6-oxo-heptyl]carbamate (2427 mg, 100.0%).

tert-butyl N-[(5S)-5-(benzyloxycarbonylamino)-7-diazo-6-oxo-heptyl]carbamate (2427 mg, 6.0 mmol) was suspended in THF (30 mL) and methanol (30 mL) and a solution of silver benzoate (274.8 mg, 1.2 mmol) in triethylamine (8.36 mL, 60.0 mmol) was gradually added while the mixture was sonicated in an ultrasound bath. The reaction was completed in 30 minutes at room temperature. Methanol and THF were evaporated and the residue was dissolved in iPrOAc (60 mL), extracted with sat. aq. NaHCO₃, 10 % aq. citric acid and sat. aq. NaCl (40 mL each) and dried over MgSO₄. Evaporation of the solvent followed by silica gel column chromatography (0 to 100% EtOAc in heptane) to afford methyl (3S)-3-(benzyloxycarbonylamino)-7-(tert-butoxycarbonylamino)heptanoate (1434.2 mg, 58.5%).

Methyl (3S)-3-(benzyloxycarbonylamino)-7-(tert-butoxycarbonylamino)heptanoate (1.4341g, 3.511 mmol) was dissolved in 1,4-dioxane (20 mL), and lithium hydroxide (10 mmol) was added at 0°C. The reaction mixture was stirred at room temperature for 1 hour. The reaction mixture was cooled to 0°C and acidified with 1.0 M HCl. The resultant white precipitate was extracted with iPrOAc, dried with MgSO₄, filtered, and concentrated to afford (3S)-3-(benzyloxycarbonylamino)-7-(tert-butoxycarbonylamino)heptanoic acid (1.405 g, 100%), which was used without further purification.

Compound 508 (formic acid salt) was prepared utilizing the methods in Example 7 (Compound 101) using (S)-3-(((benzyloxy)carbonyl)amino)-7-((tert-butoxycarbonyl)amino)heptanoic acid. MS+ 892.6. ¹H NMR (400 MHz, Methanol-*d*₄) δ 7.42 - 7.21 (m, 3H), 7.21 - 7.02 (m, 4H), 6.92 - 6.78 (m, 2H), 4.57 (d, *J* = 7.4 Hz, 1H), 4.32 - 4.17 (m, 6H), 3.31 - 3.19 (m, 27H), 3.19 - 3.01 (m, 3H), 2.98 - 2.86 (m, 1H), 2.85 (s, 2H), 2.79 (dd, *J* = 16.1, 5.8 Hz, 1H), 2.61 (t, *J* = 7.6 Hz, 2H), 2.40 (s, 3H), 2.08 (dtd, *J* = 16.5, 8.2, 3.9 Hz, 1H), 1.93 (dtd, *J* = 13.7, 9.1, 8.3, 3.7 Hz, 1H), 1.65 - 1.52 (m, 2H), 1.35 (p, *J* = 7.5 Hz, 5H), 0.93 (td, *J* = 7.3, 2.9 Hz, 3H).

### Example 409: Synthesis of Compound 509

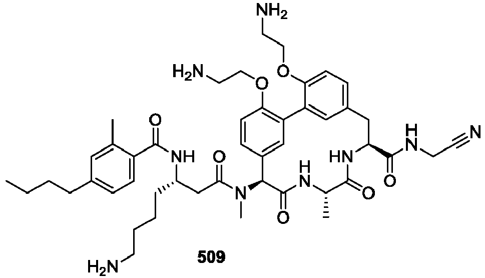

Compound 509 (formic acid salt) was prepared utilizing the methods in Example 7 (Compound 101) using (S)-3-(((benzyloxy)carbonyl)amino)-7-((tert-butoxycarbonyl)amino)heptanoic acid. MS+ 855.5 ¹H NMR (400 MHz, Methanol-*d*₄) δ 7.36 - 7.24 (m, 3H), 7.24 - 7.10 (m, 3H), 7.10 - 7.04 (m, 2H), 6.95 - 6.80 (m, 2H), 4.81 (q, *J* = 6.8 Hz, 4H), 4.54 (h, *J* = 5.8, 5.1 Hz, 1H), 4.37 - 4.19 (m, 6H), 3.41 - 3.22 (m, 9H), 3.15 (dd, *J* = 17.3, 11.2 Hz, 1H), 3.07 - 2.92 (m, 3H), 2.90 (s, 2H), 2.79 (dd, *J* = 6.8, 3.3 Hz, 2H), 2.63 (t, *J* = 7.6 Hz, 2H), 2.42 (s, 3H), 1.88 - 1.71 (m, 3H), 1.71 - 1.51 (m, 5H), 1.44 - 1.29 (m, 5H), 0.95 (t, *J* = 7.4 Hz, 3H).

### Example 410: Synthesis of Compound 510

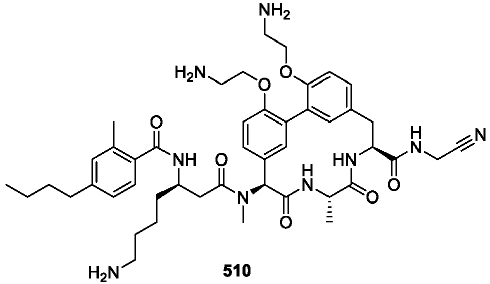

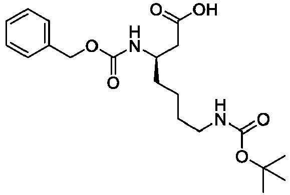

The synthesis of (R)-3-(((benzyloxy)carbonyl)amino)-7-((tert-butoxycarbonyl)amino)heptanoic acid: Starting with N²-((benzyloxy)carbonyl)-N⁶-(tert-butoxycarbonyl)-D-lysine , (R)-3-(((benzyloxy)carbonyl)amino)-7-((tert-butoxycarbonyl)amino)heptanoic acid was made following the procedures in Example 408 (63% overall yield over the three steps).

Compound 510 (formic acid salt) was prepared utilizing the methods in Example 7 (Compound 101) using (R)-3-(((benzyloxy)carbonyl)amino)-7-((tert-butoxycarbonyl)amino)heptanoic acid. MS+ 855.5 ¹H NMR (500 MHz, Methanol-*d*₄) δ 7.30 (d, *J* = 7.7 Hz, 1H), 7.28 - 7.24 (m, 2H), 7.16 (d, *J* = 8.6 Hz, 1H), 7.15 - 7.10 (m, 2H), 7.08 (d, *J*= 8.0 Hz, 1H), 6.91 (d, *J* = 2.4 Hz, 1H), 6.84 (d, *J* = 2.2 Hz, 1H), 6.35 (s, 1H), 4.88 (dd, *J* = 11.3, 3.0 Hz, 1H), 4.80 (q, *J* = 7.0 Hz, 1H), 4.52 (dq, *J* = 10.5, 6.4 Hz, 1H), 4.28 (dtd, *J* = 19.7, 11.0, 5.2 Hz, 4H), 4.22 (d, *J* = 2.3 Hz, 2H), 3.37 (d, *J* = 2.8 Hz, 1H), 3.29 (ddd, *J* = 11.0, 7.3, 4.9 Hz, 4H), 3.15 (dd, *J* = 17.3, 11.3 Hz, 1H), 3.04 - 2.93 (m, 2H), 2.90 - 2.85 (m, 1H), 2.85 (s, 3H), 2.76 - 2.69 (m, 1H), 2.62 (t, *J* = 7.6 Hz, 2H), 2.42 (s, 3H), 1.88 - 1.65 (m, 4H), 1.65 - 1.51 (m, 4H), 1.42 - 1.29 (m, 5H), 0.96 (t, *J* = 7.4 Hz, 3H).

### Example 411: Synthesis of Compound 511

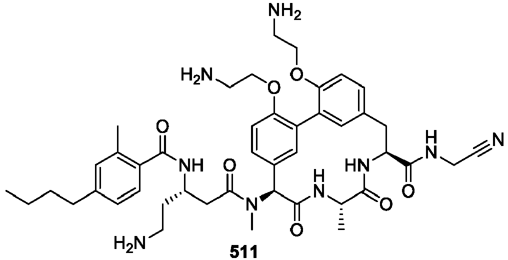

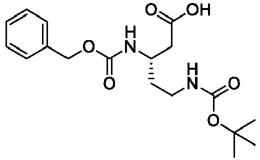

The synthesis of (S)-3-(((benzyloxy)carbonyl)amino)-5-((tert-butoxycarbonyl)amino)pentanoic acid: Starting with (S)-2-(((benzyloxy)carbonyl)amino)-4-((tert-butoxycarbonyl)amino)butanoic acid, (S)-3-(((benzyloxy)carbonyl)amino)-5-((tert-butoxycarbonyl)amino)pentanoic acid was made following the procedures in Example 408 (58% overall yield for the three steps).

Compound 511 (formic acid salt) was prepared utilizing the methods in Example 7 (Compound 101) using (S)-3-(((benzyloxy)carbonyl)amino)-5-((tert-butoxycarbonyl)amino)pentanoic acid. MS+ 827.5

### Example 412: Synthesis of Compound 512

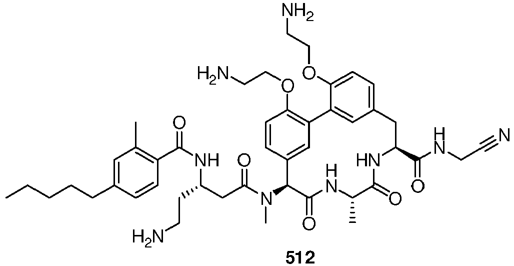

Compound 512 (formic acid salt) was prepared utilizing the methods in Example 7 (Compound 101) using (S)-3-(((benzyloxy)carbonyl)amino)-5-((tert-butoxycarbonyl)amino)pentanoic acid. MS+ 840.5.

### Example 413: Synthesis of Compound 513

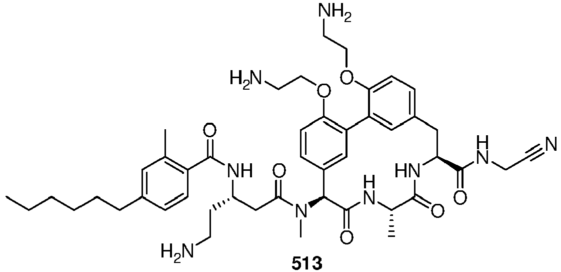

Compound 513 (formic acid salt) was prepared utilizing the methods in Example 7 (Compound 101) using (S)-3-(((benzyloxy)carbonyl)amino)-5-((tert-butoxycarbonyl)amino)pentanoic acid. MS+ 854.5.

### Example 414: Synthesis of Compound 514

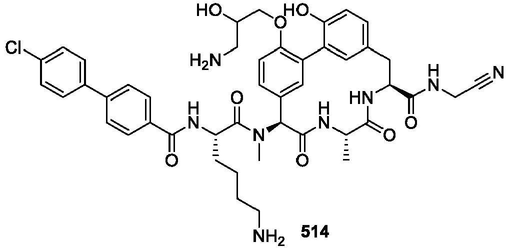

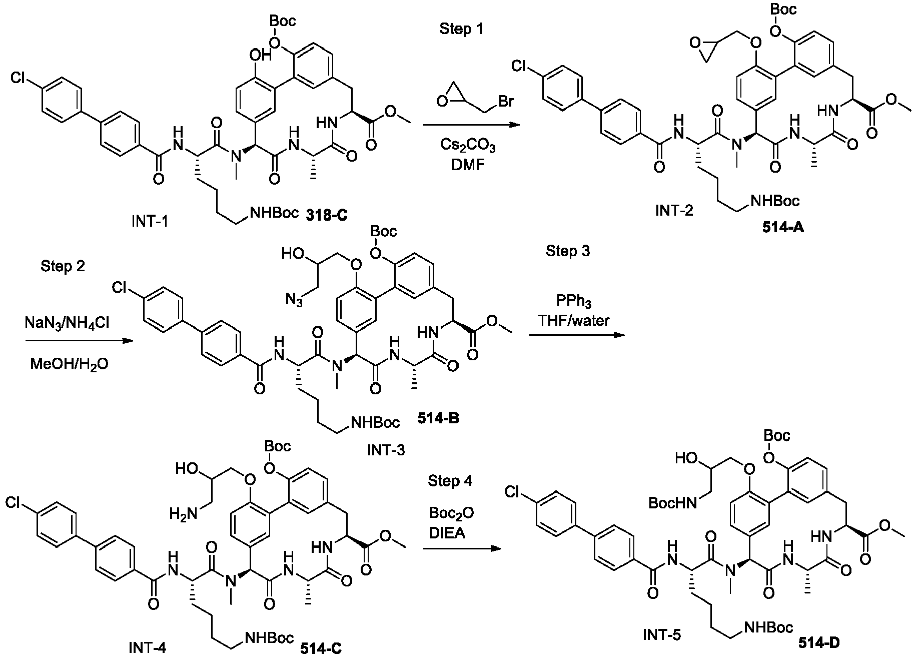

Step 1: To a mixture of Compound 318-C (Example 222) (485.2 mg, 0.50 mmol) and cesium carbonate (325.8 mg, 1.0 mmol) in N,N-dimethylformamide (10 mL) was added epibromohydrin (0.05 mL, 0.60 mmol). The reaction was stirred at room temperature over the weekend. The reaction mixture was poured into ice water and extracted 3 times with iPrOAc. The organic layers were washed with brine, dried (MgSO₄), and concentrated and the residue was purified on silica eluted with 0 to 3.5% MeOH in DCM to afford Compound 514-A (513.3 mg, 83%).

Step 2: To a mixture of Compound 514-A (319 mg, 0.31073 mmol) in methanol (10 mL) and water (1 mL) was added ammonium chloride (41.6 mg, 0.77684 mmol), followed by addition of sodium azide (102.0 mg, 1.5537 mmol). The reaction mixture was heated at 65 °C for 6 hours. The reaction mixture was concentrated and the residue was portioned between iPrOAc and water. The organic layer was dried and concentrated to afford crude Compound 514-B (321 mg, 96.582%), which was used without further purification.

Step 3: A mixture of Compound 514-B (428.5 mg, 0.40 mmol) and triphenylphosphine (111.4 mg, 0.42 mmol) in THF (10 mL) and water (2 mL) was stirred at room temperature over the weekend. The reaction mixture was concentrated, and the residue was partitioned between iPrOAc and brine. The organic layer was dried (MgSO₄) and the solvent removed under reduced pressure to give crude Compound 514-C (418.1 mg, 100.0%).

Step 4: Compound 514-C (418.1 mg, 0.40 mmol) was dissolved in dichloromethane (12 mL) and N,N-diisopropylethylamine (0.14 mL, 0.80 mmol) was added, followed by addition of di-tert-butyl dicarbonate (180.3 mg, 0.80 mmol). The reaction mixture was stirred at room temperature for 18 hours. The reaction mixture was concentrated and the residue was purified on silica eluted with 0 to 5% MeOH in DCM to afford Compound 514-D (188 mg, 41.03% for 2 steps).

Compound 514 (a mixture of diastereomers) was prepared as a white solid utilizing the methods in Example 7 (Compound 101) from Compound 514-D. MS+ 867.4 ¹H NMR (400 MHz, Methanol-*d*₄) δ 8.02 - 7.89 (m, 2H), 7.80 - 7.70 (m, 2H), 7.70 - 7.59 (m, 2H), 7.51 - 7.43 (m, 2H), 7.34 - 7.25 (m, 1H), 7.18 - 7.01 (m, 2H), 6.88 - 6.78 (m, 1H), 6.76 - 6.63 (m, 1H), 6.54 - 6.46 (m, 1H), 5.04 (dd, *J* = 8.7, 5.4 Hz, 1H), 4.19 (d, *J* = 1.0 Hz, 2H), 4.18 - 4.05 (m, 3H), 3.28 - 3.12 (m, 3H), 3.12 - 2.90 (m, 7H), 2.08 - 1.88 (m, 2H), 1.74 (q, *J* = 6.9, 6.0 Hz, 2H), 1.61 (dd, *J* = 13.1, 6.4 Hz, 2H), 1.37 (d, *J* = 6.8 Hz, 3H).

### Example 415: Synthesis of Compound 515

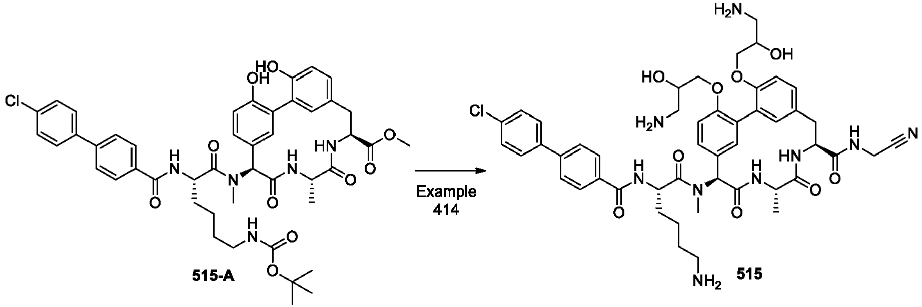

Compound 515 (mixture of diastereomers) was prepared as a white solid utilizing the methods in Example 414 from Compound 515-A. MS+ 942.5 ¹H NMR (400 MHz, Methanol-*d*₄) δ 8.02 - 7.93 (m, 2H), 7.76 (dd, *J* = 8.7, 2.8 Hz, 2H), 7.72 - 7.64 (m, 2H), 7.53 - 7.45 (m, 2H), 7.34 - 7.26 (m, 1H), 7.21 (d, *J* = 8.5 Hz, 1H), 7.07 (ddd, *J* = 33.2, 8.4, 3.6 Hz, 2H), 6.85 (q, *J* = 3.5, 3.0 Hz, 1H), 6.77 (s, 1H), 6.42 (d, *J* = 3.8 Hz, 1H), 5.06 (dd, *J* = 8.5, 5.4 Hz, 1H), 4.77 (q, *J* = 6.4 Hz, 2H), 4.23 - 4.08 (m, 5H), 4.08 - 3.96 (m, 3H), 3.31 (dp, *J* = 3.3, 1.4 Hz, 25H), 3.18 - 3.00 (m, 4H), 3.00 - 2.84 (m, 7H), 2.09 - 1.84 (m, 2H), 1.74 (d, *J* = 7.4 Hz, 2H), 1.36 (d, *J* = 6.8 Hz, 3H).

### Example 416: Synthesis of Compound 516

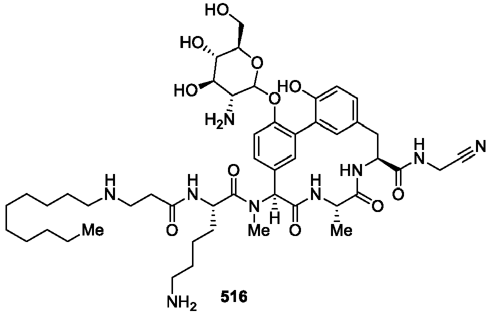

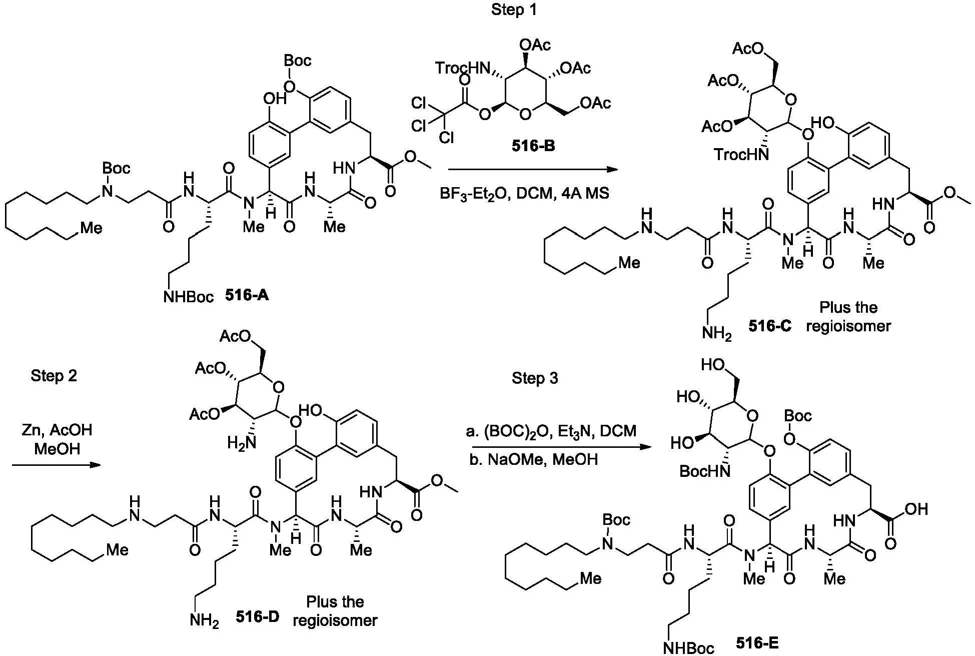

Compound 516-A is an intermediate in the preparation of Compound 320 and prepared according to the methods in Example 222.

Compound 516-B was synthesized from *β*-D-glucosamine using the procedures described in the literature. (WO2012/135049 and Organic Letters, 16(14), 3772-3775; 2014): MS (ESI) for (C₁₅H₂₀Cl₃NO₁₀): m/z 344.3 (M-277). HPLC: (two peaks with same mass; tR 3.21 min (30%) and 3.54 min (70%) (10% AcCN/H₂O - 90% AcCN/H₂O (with 0.05% TFA), 3.0 min, 1.0 mL/min Kinetix C18, 4.8 x 50 mm). ¹H NMR (400 MHz, CDCl₃) δ 8.80 (s, 1H), 6.42 (d, J = 3.0 Hz, 1H), 5.35 (t, J = 8.0 Hz, 1H), 5.25 (t, J = 8.0 Hz, 1H), 5.18 (d, J = 8.0 Hz, 1H), 4.71 (ABq, J = 10.0, 3.0 Hz, 1H), 4.29-4.26 (m, 2H), 2.08 (s, 3H), 2.05 (s, 3H), 2.03 (s, 3H).

Step 1: To a mixture of Compound 516-A (106 mg, 0.1 mmol), Compound 516-B (311 mg, 0.5 mmol) and 4 Å MS (flame dried) in dry DCM (5.0 mL) was added BF₃-Et₂O (125 µL, 0.3 mmol) dropwise at 0°C. Stirring was continued for an additional 6 h at the same temperature, and then the reaction was left in the refrigerator for overnight. The reaction mixture was diluted with DCM and filtered through celite and filtrate concentrated under reduced pressure to afford regioisomeric mixture of Compound 516-C. MS (ESI): (C₅₆H₈₁Cl₃N₇O₁₇), m/z 1228.9 [M + H]⁺; HPLC: tR 3.31 min, (10% AcCN/H₂O - 90% AcCN/H₂O (with 0.05%TFA), 3.0 min, 1.0 mL/min Kinetix C18, 4.8 x 50 mm).

Step 2: The resultant mixture of Compound 516-C was dissolved in 1:1 MeOH-AcOH (5.0 mL) and cooled to 0 °C, then Zn dust (130 mg, 20 eq) was added and the reaction mixture was stirred at rt for 3h. The mixture was filtered through celite bed and thoroughly washed the celite bed with MeOH. The filtrate was concentrated under reduced pressure. The residue was purified by preparative HPLC (10% AcCN/H₂O - 60% AcCN/H₂O (with 0.05% HCO₂H) to afford Compound 516-D as a mixture of regioisomers. LCMS (ESI): (C₅₃H₈₀N₇O₁₅): m/z 1055.3 (M+2H); HPLC: t_{R} 2.99 min (10% AcCN/H₂O - 90% AcCN/H₂O (with 0.05%TFA), 3.0 min, 1.0 mL/min Kinetix C18, 4.8 x 50 mm).

Step 3: Compound 516-D (22 mg, 0.2 mmol) was subjected to Boc protection using the conditions for Example 218 (Compound 314) then the resultant residue was dissolved in MeOH. Cooled the mixture to 0 °C and a solution of NaOMe (20 eq, 25 wt% in MeOH) was added. The reaction mixture was stirred at rt overnight, quenched with ice and continued stirring for another 1h. Methanol was removed, cooled the mixture in ice bath and acidified with 1M HCl. The sticky material was collected and dried under high vacuum to afford Compound 516-E.

Compound 516 (formic acid salt) was prepared as a white solid utilizing the methods in Example 7 (Compound 101) from Compound 516-E. MS (ESI): (C₄₈H₇₃N₉O₁₁): m/z 952.6 [M + H]⁺; HPLC: t_{R} 2.70 min (10% AcCN/H₂O - 90% AcCN/H₂O (with 0.05%TFA), 3.0 min, 1.0 mL/min Kinetix C18, 4.8 x 50 mm).

### Example 417: Synthesis of Compound 517

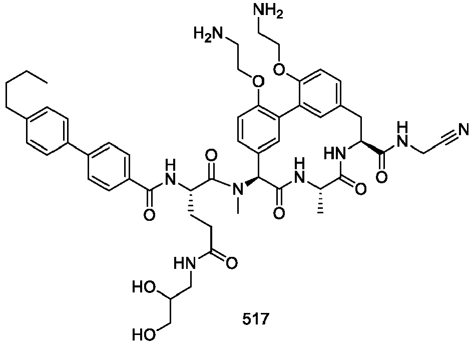

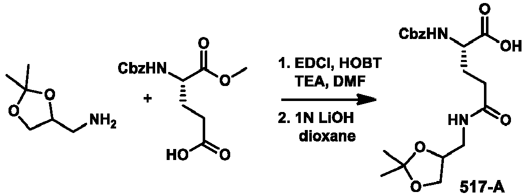

Compound 517-A was prepared by using (2,2-dimethyl-1,3-dioxolan-4-yl)methanamine and (S)-4-(((benzyloxy)carbonyl)amino)-5-methoxy-5-oxopentanoic acid followed by the general procedures EDCI-HOBT coupling (Example 234) and LiOH hydrolysis (Example 7). MS (ESI): (C₁₉H₂₇N₂O₇), m/z 395.3 [M + H]⁺; HPLC: tR 2.88 min, (10% AcCN/H₂O - 90% AcCN/H₂O (with 0.05%TFA), 3.0 min, 1.0 mL/min Kinetix C18, 4.8 x 50 mm).

Compound 517 (formic acid salt) was prepared as a white solid using 101-G and Compound 517-A by utilizing the methods in Example 5 and 7 (Compound 101-I and 101). MS (ESI): (C₅₂H₆₆N₉O₁₀): m/z 976.4 [M + H]⁺; HPLC: tR 2.68 min (10% AcCN/H₂O - 90% AcCN/H₂O (with 0.05% TFA), 3.0 min, 1.0 mL/min Kinetix C18, 4.8 x 50 mm).

### Example 418: Synthesis of Compound 518

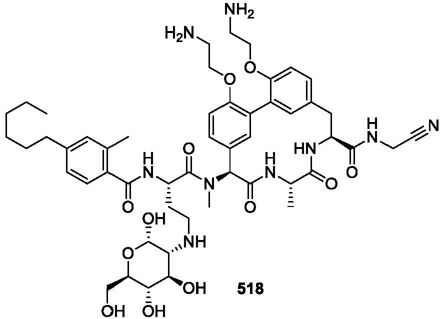

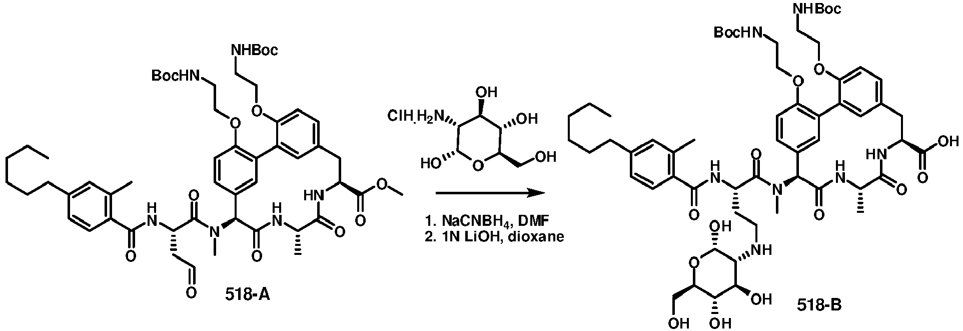

Compound 518-A was prepared according to the methods in Example 168.

Compound 518-B was prepared using the procedure as described for Example 168 (Compound 263). MS (ESI): (C₅₉H₈₆N₇O₁₇), m/z 1164.3 [M + H]⁺; HPLC: tR 3.23 min, (50% AcCN/H₂O - 90% AcCN/H₂O (with 0.05%TFA), 3.0 min, 1.0 mL/min Kinetix C18, 4.8 x 50 mm).

Compound 518 (formic acid salt) was prepared as a white solid utilizing the methods in Example 7 (Compound 101) from Compound 518-B. MS (ESI): (C₅₁H₇₂N₉O₁₂: m/z 1002.6 [M + H]⁺; HPLC: tR 2.68 min (10% AcCN/H₂O - 90% AcCN/H₂O (with 0.05%TFA), 3.0 min, 1.0 mL/min Kinetix C18, 4.8 x 50 mm).

### Example 419: Synthesis of Compound 519

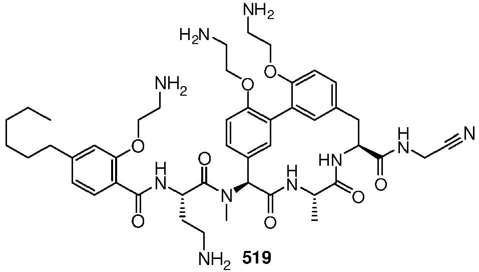

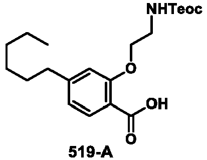

Compound 519-A was prepared by using the alkylation procedure described for Example 311 (Compound 407). MS (ESI): (C₂₂H₃₇NO₅Si), m/z 432.3 [M + Na]⁺; HPLC: tR 2.27 min, (75% AcCN/H₂O - 90% AcCN/H₂O (with 0.05%TFA), 3.0 min, 1.0 mL/min Kinetix C18, 4.8 x 50 mm).

The methods in Example 7 (Compound 101) were followed to afford Compound 519 (21.4 mg) as a white solid. MS (ESI): (C₆ₗH₈₈N₁₀O₁₄), m/z 1185.9 [M + H]⁺; HPLC: tR 2.87 min, (50% A_{C}CN/H₂O - 90% AcCN/H₂O (with 0.05%TFA), 3.0 min, 1.0 mL/min Kinetix C18, 4.8 x 50 mm). The residue was subjected for global deprotection following the general method (Boc - deprotection). MS (ESI): (C₄₆H₆₄N₁₀O₈), m/z 885.6 [M + H]⁺; HPLC: tR 2.54 min, (10% AcCN/H₂O - 90% AcCN/H₂O (with 0.05%TFA), 3.0 min, 1.0 mL/min Kinetix C18, 4.8 x 50 mm). ¹H NMR (400 MHz, CD₃OD) δ 7.87-7.86 (d, J=6.4 Hz, 1H), 7.32-7.30 (d, J= 8 Hz, 1H), 7.26 -7.24 (d, J=8 Hz, 1H), 7.18-7.16 (d, J=8 Hz, 1H), 7.11-7.09 (d, J=8Hz, 1H), 7.04 (s, 1H), 7.01-6.99 (d, J=8 Hz, 2H), 6.89 (s, 1H), 6.82 (s, 1H), 6.41 (s, 1H), 5.27-5.24 (dd, J=3.2, 7.6 Hz, 1H), 4.90-4.88 (dd, J=2, 7.6 Hz, 1H), 4.85-4.78 (dd, J=4, 7.6 Hz, 1H), 4.23-4.18 (m, 6H), 4.20 (s, 2H), 3. 60-3.58 (m, 1H), 3.52-3.50 (m, 1H), 3.18-3.15 (m, 6H), 3.08-3.07 (m, 2H), 2.94 (s, 8H), 0.92-0.89 (t, J=112Hz, 3H).

### Example 420: Synthesis of Compound 520

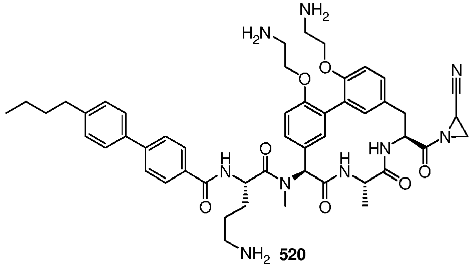

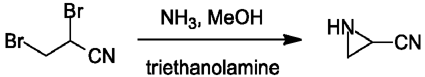

NH₃ (gas) was bubbled into methanol (20 mL) at -30 °C for 20 minutes. To the solution was slowly added 2,3-dibromopropionitrile (5.3 g, 24.9 mmol) at 5 °C. After 20 minutes, triethanolamine (7429.0 mg, 49.8 mmol) was slowly added at 10 °C and stirred at 14 °C for 40 minutes. The reaction mixture was refluxed for 4 h, cooled to 5 °C and kept at 5 °C for another 16 h. The mixture was filtered and washed with methanol (2.5 mL). The filtrate was dried under vacuum. To the residue was added 2 M H₂SO₄ (1.25 mL) at 0 °C, followed by aq. sodium sulfite (5.5 g in 100 mL of water) till pH = 6, and extracted with EtOAc (10 mL x 5). The combined organic layers were concentrated and purified by TLC (25% EtOAc in petroleum ether, Rf = 0.2) to afford aziridine-2-carbonitrile (800 mg, 23.6% yield) as a colorless oil (impurity by HNMR).

Compound 520 was prepared utilizing the methods in Example 7 (Compound 101) from aziridine-2-carbonitrile. LCMS (Method 5-95AB): t_{R} = 0.761 minI 1.5 min, [M + H]⁺ = 901.5.

### Example 421: Synthesis of Compound 521, Compound 522, and Compound 523

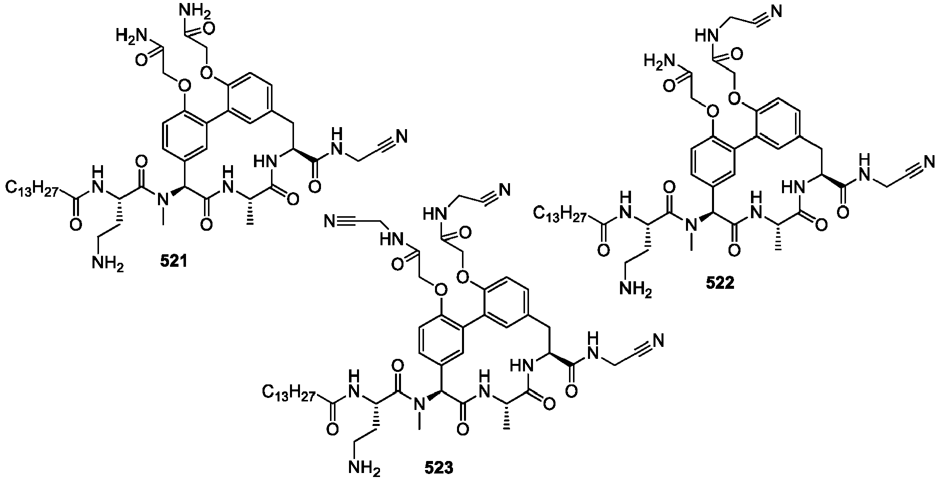

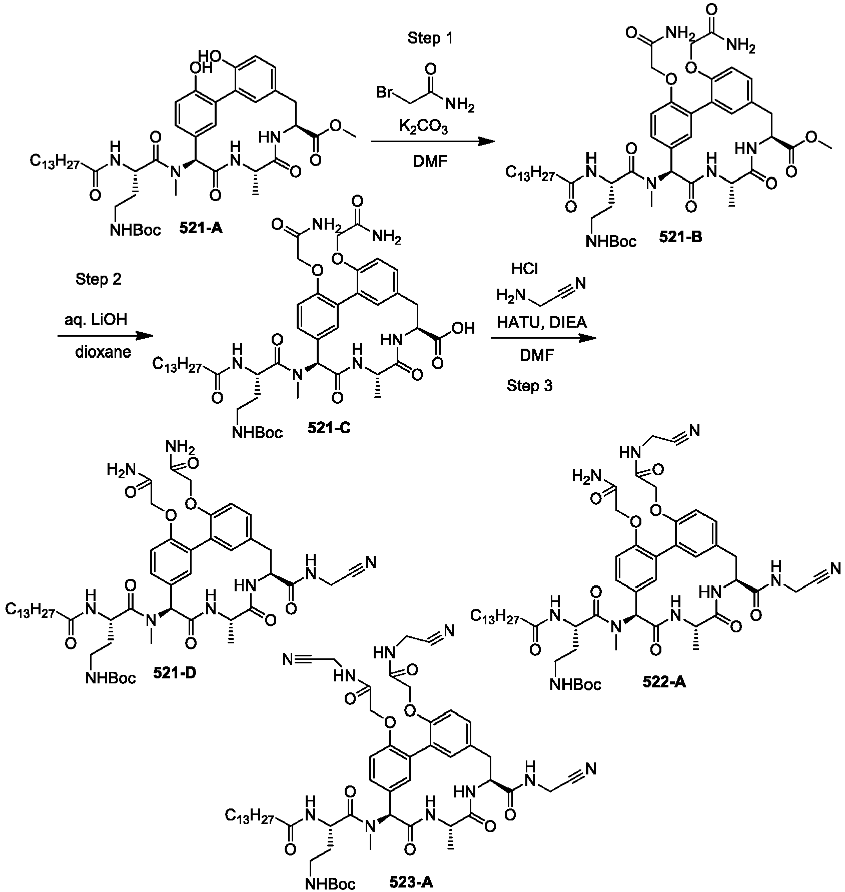

Steps 1 and 2: Compound 521-A was prepared utilizing methods previously described.. Phenol alkylation with 2-bromoacetamide according to General Method 2 (Example 4) and LiOH hydrolysis (Example 7) afforded Compound 521-C.

Step 3: The coupling of Compound 521-C (150.0 mg, 0.16 mmol) with 2-aminoacetonitrile (General Method 6 (Example 7) (74.0 mg, 0.80 mmol) afforded desired Compound 521-D (30 mg), together with two byproducts, Compound 522-A (60 mg) and Compound 523-A (30 mg).

Compound 521 was prepared utilizing hydrolysis methods previously described (Example 7). LCMS (Method 5-95 AB, ESI): t_{R} = 0.844 min, [M + H]⁺ = 877.6.

Compound 522 was prepared utilizing hydrolysis methods previously described (Example 7). LCMS (Method 5-95 AB, ESI): t_{R} = 0.861 min, [M + H]⁺ = 916.7.

Compound 523 was prepared utilizing hydrolysis methods previously described (Example 7). LCMS (Method 5-95 AB, ESI): t_{R} = 0.874 min, [M + H]⁺ = 956.6.

### Example 422: Synthesis of Compound 524-1 and Compound 524-2

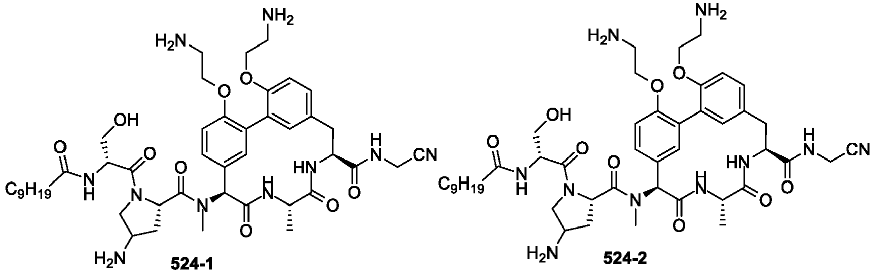

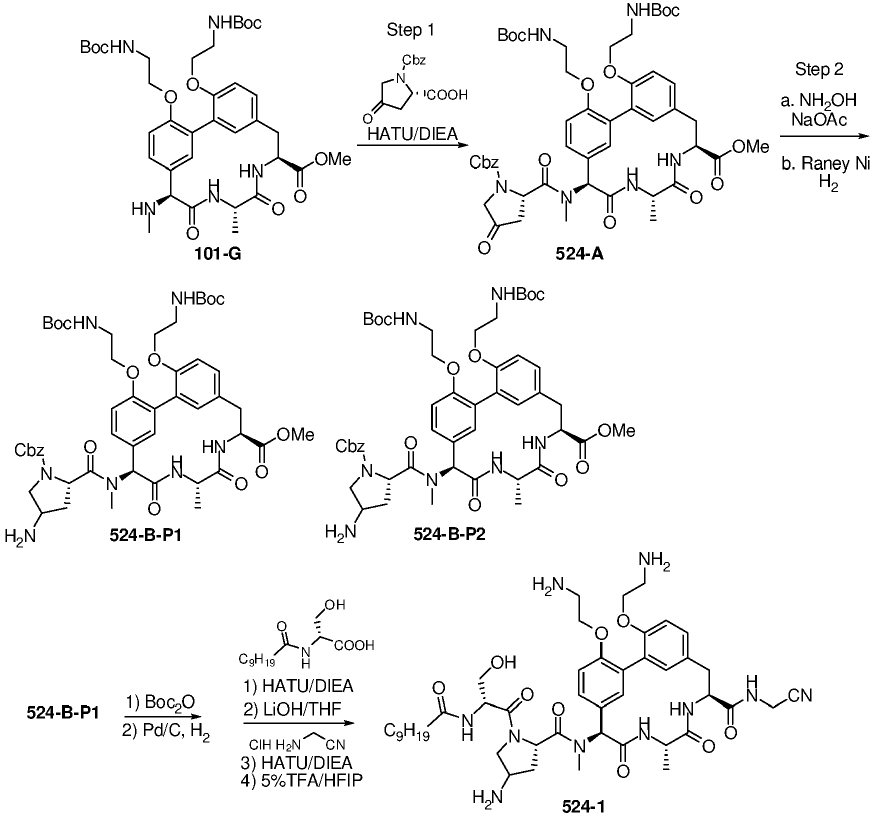

Step 1: Starting from Compound 101-G (1 g, 1.40 mmol), typical amide coupling (HATU) procedure was followed to afford Compound 524-A (1.25 g, 93% yield) as a white solid.

Step 2: To a solution of Compound 524-A (1.25 g, 1.3 mmol) in EtOH (26 mL) was added HONH₂·HCl (725 mg, 10.4 mmol) and Et₃N (1.06 g, 10.4 mmol) and the mixture was stirred at room temperature for 2 h. The volatiles were removed and the residue was added with H₂O (50 mL), which was extracted with DCM (60 mL). The organic layer was dried over Na₂SO₄, concentrated to afford crude Compound 524-B, which was used directly in the next step. The above residue was then dissolved in MeOH (26 mL), which was added Raney Ni (15 mg, 0.26 mmol). The resulting mixture was stirred at room temperature for 16 h under H₂ (15 psi). The filtrate was concentrated and the residue was purified on silica gel column eluted (1% methyl alcohol in dichloromethane) to obtain a mixture of diastereomers, which were further purified by pre-HPLC to obtain Compound 524-B-P1 (200 mg, 16% yield) as a white solid and Compound 524-B-P2 (180 mg, 14% yield) as a white solid.

Step 3: Starting from Compound 524-B-P1, utilizing methods previously described, Compound 524-1 (11.0 mg) was prepared as a white solid from (R)-2-decanamido-3-hydroxypropanoic acid. LCMS (Method 5-95 AB, ESI): t_{R} = 0.713, [M + H]⁺ = 891.8; ¹H NMR (400 MHz, MeOH-*d*₄) δ 8.50 (brs, 2H), 7.43 (brs, 1H), 7.36 (d, *J*=8.4 Hz, 1H), 7.24 (d, *J*=8.4 Hz, 1H), 7.17 (d, *J*=8.0 Hz, 1H), 7.09 (d, *J*=8.0 Hz, 1H), 6.87 (brs, 1H), 6.81 (brs, 1H), 6.27 (s, 1H), 5.05-5.00 (m, 1H), 4.85-4.75 (m, 3H), 4.26-4.20 (m, 4H), 4.18 (s, 2H), 4.06-3.76 (m, 2H), 3.76-3.72 (m, 2H), 3.38-3.13 (m, 5H), 3.35 (s, 3H), 2.91-2.85 (m, 2H), 2.30-2.25 (m, 2H), 1.65-1.48 (m, 2H), 1.35-1.20 (m, 17H), 0.89 (t, *J*=7.2 Hz, 3H).

Starting from Compound 524-B-P2, Compound 524-2 was prepared utilizing methods previously described from (R)-2-decanamido-3-hydroxypropanoic acid. Data for Compound 524-2: LCMS (Method 5-95 AB, ESI): t_{R} = 0.711, [M + H]⁺ = 891.5.

### Example 423: Synthesis of Compound 525

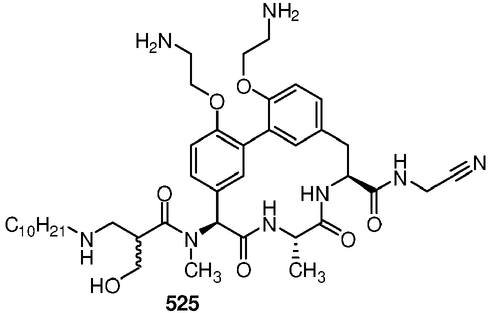

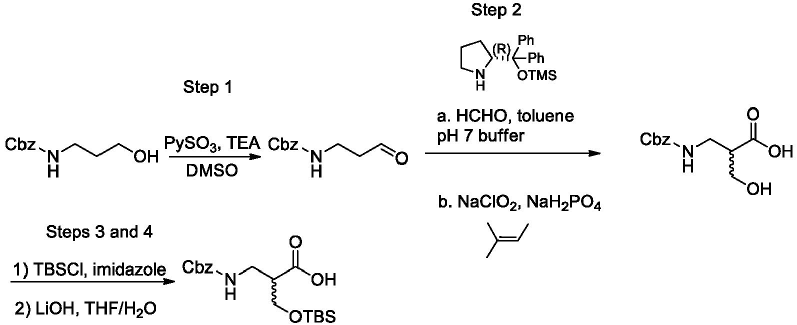

Step 1: To a solution of benzyl (3-hydroxypropyl)carbamate (1.0 g, 4.8 mmol) in DMSO (5 mL) was added Et₃N (2.4 g, 24.0 mmol) and sulfur trioxide-pyridine complex (2.3 g, 14.4 mmol) at 0°C and the mixture was stirred at the same temperature for 1 h. The mixture was then diluted with DCM (20 mL), which was washed with 10% CuSO₄ solution, saturated citric acid solution and brine (30 mL each). The organic layer was dried over Na₂SO₄, concentrated and the residue was purified by silica-gel column to give benzyl (3-oxopropyl)carbamate (200 mg, 20.2% yield) as a white solid.

Step 2: To a vigorously stirred solution of formaldehyde (235 mg, 2.9 mmol) in toluene (2.0 mL) was added 0.2 M phosphate buffer (pH=7, 2.0 mL), (R)-2-(diphenyl((trimethylsilyl)oxy)methyl)pyrrolidine (94 mg, 0.29 mmol) and benzyl (3-oxopropyl)carbamate (200 mg, 0.97 mmol) sequentially and the resulting mixture was stirred at room temperature for 12 h. The toluene layer was separated and concentrated in vacuo (caution: keep the water bath temperature < 40 °C while evaporating). The residue was then dissolved with t-BuOH (5.0 mL), where 2-methyl-2-butene (677 mg, 9.6 mmol) and a solution of NaClO₂ (349 mg, 3.9 mmol) and NaH₂PO₄ (463 mg, 3.9 mmol) in H₂O (2 mL) was added. The mixture was stirred at room temperature for 5 h. Volatiles were removed and the residue was dissolved in EtOAc (20 mL), which was washed with 1% HCl and brine (20 mL each). The organic layer was dried over Na₂SO₄, concentrated and the residue was purified of by prep-TLC to provide 3-(((benzyloxy)carbonyl)amino)-2-(hydroxymethyl)propanoic acid (53 mg, 21.7% yield) as light yellow oil.

Steps 3 and 4: Starting from 3-(((benzyloxy)carbonyl)amino)-2-(hydroxymethyl)propanoic acid (153 mg), standard TBS protection and ester hydrolysis (LiOH, THF/H₂O) procedure was followed to afford 3-(((benzyloxy)carbonyl)amino)-2-(((tertbutyldimethylsilyl)oxy)methyl)propanoic acid (155 mg) as colorless oil.

Compound 525 (formic acid salt) was prepared utilizing methods previously described. LCMS (5-95 AB, ESI): t_{R} = 0.755, [M + H]⁺ = 779.5.

### Example 424: Synthesis of Compound 526-1, Compound 526-2, Compound 526-3

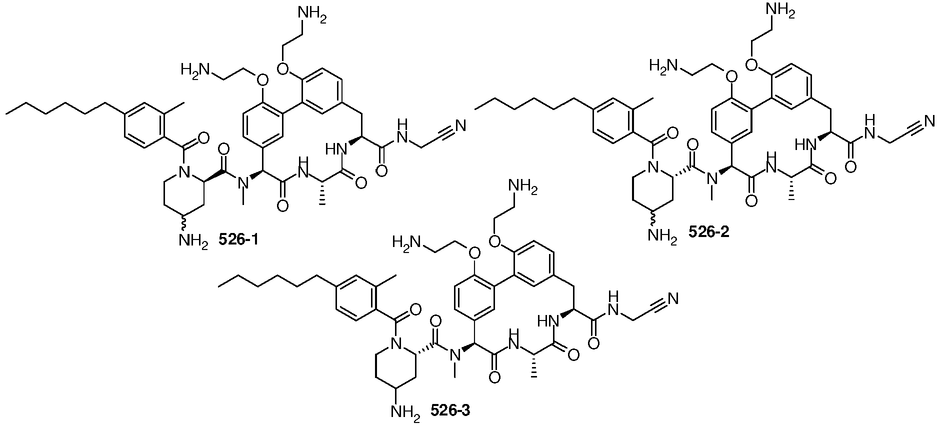

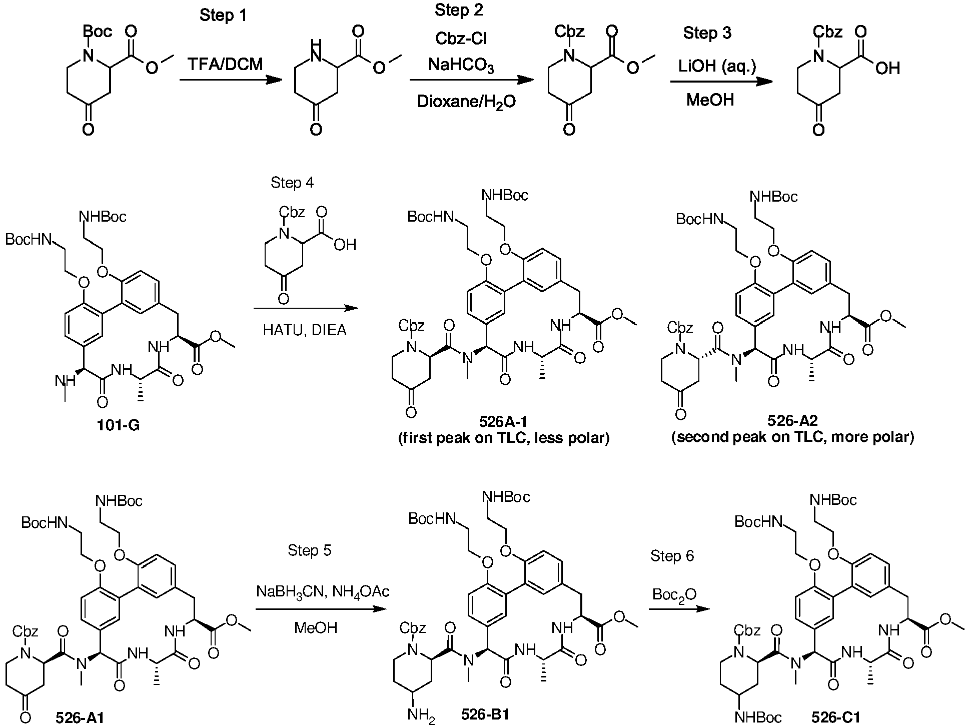

Steps 1-3: 1-((Benzyloxy)carbonyl)-4-oxopiperidine-2-carboxylic acid was prepared using methods previously described (Boc deprotection, Cbz protection, and ester hydrolysis) from 1-tert-butyl 2-methyl 4-oxopiperidine-1,2-dicarboxylate.

Step 4: Treatment of Compound 101-G with 1-((benzyloxy)carbonyl)-4-oxopiperidine-2-carboxylic acid under standard HATU conditions (Example 5) afforded Compound 526-A1 (peak 1) and Compound 526-A2 (peak 2) after silica gel chromatography.

Step 5: To a solution of Compound 526-A1 (225.0 mg, 0.23 mmol) and 4A MS (200.0 mg, 0.23 mmol) in methanol (4 mL) were added ammonium acetate (196.1 mg, 2.54 mmol) and sodium cyanoborohydride (14.5 mg, 0.23 mmol), and stirred at 25 °C for 48 h. The reaction was evaporated and purified by prep-TLC (10% MeOH in DCM, Rf = 0.3) to give Compound 526-B1 (80 mg, 35.5% yield) as a white solid. LCMS (Method 5-95 AB, ESI): t_{R} = 0.850 min, [M + Na]⁺ = 997.0.

Step 6: Standard Boc protection of Compound 526-B1 afforded Compound 527-C1.

Compound 526-1 (formic acid salt) was prepared utilizing methods previously described from Compound 526-C1. LCMS (Method 5-95 AB, ESI): t_{R} = 0.606 min, [M/2+H]⁺ = 433.7.

Compound 526-2 (formic acid salt) was prepared utilizing methods previously described from Compound 526-C1 and was isolated as the second peak. LCMS (Method 5-95 AB, ESI): t_{R} = 0.622 min, [M + H]⁺ = 866.6.

Starting from Compound 526-A2, the more polar intermediate, Compound 526-3 was prepared utilizing methods previously described. LCMS (Method 5-95 AB, ESI): t_{R} = 0.616 min, [M + Na]⁺ = 889.0.

### Example 425: Synthesis of Compound 527

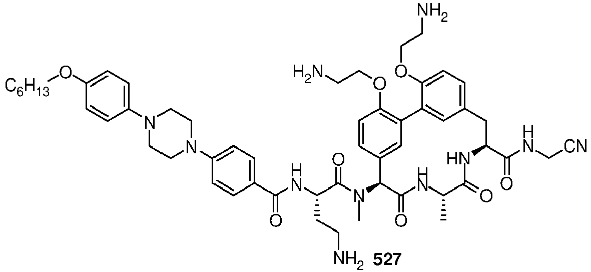

Compound 528 (formic acid salt) was prepared utilizing the methods previously described. LCMS (Method 5-95AB): t_{R} = 0.754, [M + H]⁺ = 1002.2.

### Example 426: Synthesis of Compound 528

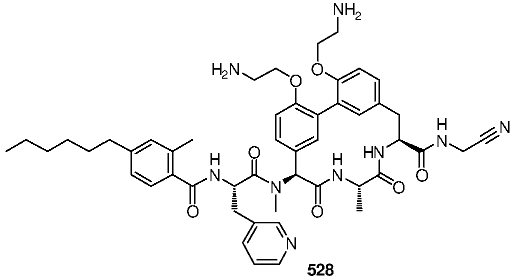

Compound 529 (formic acid salt) was prepared as a white solid utilizing methods previously described. LC-MS: m/z = 888 [M + H]⁺.

### Biological Assays

### Example 427: Determination of Minimum Inhibitory Concentration

In vitro antimicrobial activity of each compound was determined by measuring minimal inhibitor concentrations (MICs) using the broth micro-dilution technique as approved by the Clinical and Laboratory Standards Institute (CLSI) (Methods for Dilution Antimicrobial Susceptibility Tests for Bacteria that Grow Aerobically; Approved Standard - Eighth Edition. CLSI document M07-A8. Wayne, PA: Clinical and Laboratory Standards; 2009). Antibacterial activity was measure against three strains of bacteria: a Methicillin Resistant *Staphylococcus aureus* strain USA 300, NRS384 (**S**. *aureus*); a strain of *Escherichia coli* MC4100 harboring the IMP4213 (*E*. *coli* IMP), which results in increased outer-membrane permeability (B Martin and Silhavy T. Imp/OstA is required for cell envelope biogenesis in *Escherichia coli.* (2002) Molecular Microbiology, 45(5), 1289-1302), and *Escherichia coli* ATCC 25922 (*E*. *coli),* a clinically relevant Gram-negative strain. Cells were inoculated onto plates of Trypyticase Soy Agar or Luria Agar respectively and grown at 35°C for 20 hours. Inocula suspensions were prepared by scraping cells into 1 mL of testing media (cation adjusted Mueller Hinton Broth supplemented with 0.002% v/v Tween-80) and diluting to a final OD₆₀₀ₙₘ of 0.01.

Test compounds were prepared in DMSO at a concentration of 10 mg/mL. The compounds were tested under several different dilution formats and the data are reported in Table 1. In protocol 1, the compound stocks were diluted into testing media at a concentration of 64 µg/ml and serial 2-fold dilutions were made in the same media, in 96-well U bottom microtiter dishes, for a total of 10 compound concentrations. In protocol 2, the compound stocks were diluted into testing media at a concentration of 4 µg/mL and serial 2-fold dilutions were made in the same media, in 96-well U bottom microtiter dishes, for a total of 10 compound concentrations. In protocol 3, compound stocks were diluted into testing media at a concentration of 0.5 µg/mL, with serial 2-fold dilutions conducted as described above. In protocol 4, compound stocks were diluted into testing media at a concentration of 0.13 µg/mL, with serial 2-fold dilutions conducted as described above. Inocula suspensions were added to the 2-fold serial dilutions of test compounds to a final density of OD OD₆₀₀ₙₘ of 0.0005 and incubated at 35°C for 22 hours. After incubation the plates were examined visually and the lowest concentration of test compound that completely prevented bacterial growth were recorded as the MICs. The results are listed in Table 1.

**Table 1**

| Compound | MIC (µg/mL) S. aureus | MIC (µg/mL) *E. coli* IMP | MIC (µg/mL) *E. coli* | Compound | MIC (µg/mL) S. aureus | MIC (µg/mL) *E. coli* IMP | MIC (µg/mL) *E. coli* |
|---|---|---|---|---|---|---|---|
| 101 | 0.094 | 1.7 | 48 | 326 | 0.19 | 0.016 | 1 |
| 102 | 0.023 | 0.0078 | 0.25 | 327 | 0.25 | 0.0078 | 2 |
| 103 | 0.023 | 0.0078 | 1 | 328 | 1 | 0.25 | 4 |
| 104 | 0.047 | 0.023 | 4 | 329 | 0.063 | 0.0078 | 0.5 |
| 105 | 0.13 | 0.031 | 8 | 330 | 0.25 | 0.063 | 1 |
| 106 | 0.38 | 0.13 | 24 | 331 | 0.063 | 0.023 | 0.69 |
| 107 | 1 | 0.38 | 32 | 332 | 0.13 | < 0.063 | 3 |
| 108 | 0.063 | 0.047 | 0.5 | 333 | < 0.063 | < 0.063 | 1 |
| 109 | 2 | 1 | > 64 | 334 | < 0.063 | < 0.063 | 0.5 |
| 110 | 0.19 | 0.063 | 8 | 335 | 0.031 | 0.016 | 0.25 |
| 111 | 0.13 | 0.13 | 8 | 336 | 0.063 | 0.023 | 0.25 |
| 112 | 0.13 | 0.13 | 8 | 337 | 0.031 | 0.016 | 0.25 |
| 113 | 0.19 | 0.13 | 32 | 338 | 0.063 | 0.023 | 0.19 |
| 114 | 2 | 2 | > 64 | 339 | 0.047 | 0.018 | 0.13 |
| 115 | 0.25 | 0.25 | 64 | 340 | | < 0.063 | 0.75 |
| 116 | 0.13 | 0.063 | 12 | 341 | 0.13 | 0.031 | 0.5 |
| 117 | 0.13 | 0.5 | > 64 | 342 | 0.19 | 0.063 | 0.5 |
| 118 | 0.063 | 0.031 | 0.75 | 343 | 0.094 | 0.063 | 0.25 |
| 119 | 1 | 0.25 | 16 | 344 | 0.094 | 0.036 | 0.38 |
| 120 | 0.094 | 0.016 | 0.44 | 345 | 0.5 | 2 | > 64 |
| 121 | 0.13 | 0.023 | 2 | 346 | 0.031 | 0.063 | 8 |
| 122 | 0.063 | 0.031 | 0.25 | 347 | 0.063 | 0.063 | 8 |
| 123 | 0.19 | 0.0078 | 0.5 | 348 | 0.055 | 1 | > 64 |
| 124 | 0.13 | 0.031 | 2 | 349 | 0.016 | 0.13 | 8 |
| 125 | 2 | 0.25 | 16 | 350 | 0.016 | 0.063 | 4 |
| 126 | 0.5 | 0.25 | 32 | 351 | 1 | > 4.0 | > 64 |
| 127 | 0.5 | 0.063 | 4 | 352 | 0.047 | 0.0078 | 1.5 |
| 128 | 4 | 0.5 | 16 | 353 | 0.047 | 0.016 | 2 |
| 129 | 0.023 | 0.094 | 4 | 354 | 0.25 | 0.063 | 8 |
| 130 | 0.031 | 0.5 | 24 | 355 | 0.13 | 0.031 | 4 |
| 131 | 0.047 | 1.8 | 64 | 356 | 0.094 | 0.13 | 12 |
| 132 | 0.25 | 3 | > 64 | 357-P1 | 0.5 | 0.5 | 64 |
| 133 | 0.25 | > 4.0 | > 64 | 357-P2 | 0.094 | 0.094 | 12 |
| 134 | 1.5 | > 4.0 | > 64 | 359 | 0.063 | 0.13 | 16 |
| 135 | 0.052 | 0.023 | 0.22 | 360 | 0.22 | 0.31 | 24 |
| 136 | 2 | 0.38 | 8 | 361 | 0.13 | 0.13 | 16 |
| 137 | 0.063 | 0.063 | 4 | 362 | 0.25 | 0.5 | 32 |
| 138 | 0.063 | 0.055 | 0.25 | 363 | 0.25 | 0.5 | 64 |
| 139 | 0.047 | 0.012 | 0.25 | 364 | 0.38 | 0.75 | 48 |
| 140 | 0.031 | 0.0078 | 0.13 | 365 | 0.38 | 0.5 | 64 |
| 141 | 0.031 | 0.023 | 0.22 | 366 | 0.25 | 0.38 | 48 |
| 142 | 0.039 | 0.042 | 0.16 | 367 | 0.094 | 0.13 | 12 |
| 143 | 0.047 | 0.018 | 0.44 | 368 | 0.25 | 1 | 64 |
| 144 | 0.031 | 0.053 | 0.16 | 369 | 0.063 | 0.063 | 4 |
| 145 | 0.047 | 0.012 | 0.16 | 370 | 0.19 | 0.13 | 16 |
| 146 | 0.047 | 0.0078 | < 0.063 | 371 | 0.25 | 0.5 | 32 |
| 147 | 0.094 | 0.023 | 1.5 | 372 | 0.13 | 0.094 | 8 |
| 148 | 0.055 | 0.0098 | 0.88 | 373 | 0.19 | 0.063 | 4 |
| 149 | 0.19 | 0.023 | 3 | 374 | 0.19 | 0.13 | 16 |
| 150 | 0.25 | 0.063 | 4 | 375 | 0.25 | 0.75 | 64 |
| 151 | 0.031 | 0.012 | 0.31 | 376 | 0.5 | 2.3 | > 64 |
| 152 | 0.031 | 0.014 | 0.63 | 377 | 0.13 | 0.13 | 8 |
| 153 | 0.023 | 0.0059 | 0.19 | 378 | 0.13 | 0.25 | 24 |
| 154 | 0.012 | 0.0059 | 0.25 | 379 | 0.25 | 0.25 | 32 |
| 155 | 0.13 | 0.0098 | 0.44 | 380 | > 4.0 | 4 | 64 |
| 156 | 0.016 | 0.0044 | 0.063 | 381 | 0.5 | 0.5 | 64 |
| 157 | 0.023 | 0.0034 | 0.098 | 382 | 0.5 | 0.5 | 64 |
| 158 | 0.031 | 0.002 | 0.11 | 383 | 0.018 | 0.023 | 4 |
| 159 | 0.063 | < 0.0039 | 0.13 | 384 | 0.012 | 0.031 | 3 |
| 160 | 0.047 | 0.0039 | 0.13 | 385 | 0.25 | 0.5 | 32 |
| 161 | 0.031 | 0.003 | 0.13 | 386 | 0.38 | 0.5 | 64 |
| 162 | 0.023 | 0.0025 | 0.063 | 387 | 0.25 | 0.38 | 64 |
| 163 | 0.031 | 0.002 | < 0.063 | 388 | 0.25 | 0.25 | 48 |
| 164 | 0.047 | 0.0039 | 0.13 | 389 | 0.063 | 0.031 | 0.25 |
| 165 | 0.047 | 0.0039 | 0.19 | 390 | 0.13 | 0.031 | 3 |
| 166 | 0.063 | 0.016 | 0.5 | 391 | 0.047 | 0.016 | 0.5 |
| 167 | 0.023 | 0.0049 | 0.14 | 392 | 4 | 2 | > 64 |
| 168 | 0.063 | 0.031 | 0.75 | 393-P1 | 2 | 1 | >64 |
| 169 | 0.023 | 0.0078 | 0.13 | 393-P2 | 1 | 2 | >64 |
| 170 | 0.073 | 0.0085 | 0.13 | 394-P1 | 1 | 0.25 | >64 |
| 171 | 0.094 | 0.0078 | 0.5 | 394-P2 | 0.5 | 0.38 | 64 |
| 172 | 0.094 | 0.016 | 0.19 | 395 | 1 | 1 | > 64 |
| 173 | 0.094 | 0.016 | 0.31 | 396 | 0.094 | 0.094 | 16 |
| 174 | 0.17 | 0.026 | 0.83 | 397 | 3 | 0.38 | 64 |
| 175 | 0.13 | 0.0078 | 0.5 | 398 | 0.13 | 0.13 | 16 |
| 176 | 1 | 0.13 | 2 | 399 | 0.25 | 0.19 | 16 |
| 177 | 0.094 | 0.016 | 0.19 | 400 | 0.25 | 2 | > 64 |
| 178 | 0.25 | 0.039 | 1.5 | 401 | 2 | 4 | > 64 |
| 179 | 0.047 | 0.0078 | 0.19 | 402 | 0.38 | 0.19 | 32 |
| 180 | 1 | 0.38 | 8 | 403 | 0.029 | 0.023 | 1.3 |
| 181 | 0.13 | 0.031 | 2 | 404 | 0.063 | 0.047 | 2 |
| 182 | 0.5 | 0.13 | 12 | 405 | 0.047 | 0.016 | 0.63 |
| 183 | 0.063 | 0.0059 | 0.13 | 406 | 0.063 | 0.031 | 2 |
| 184 | 0.094 | 0.012 | 0.25 | 407 | 0.047 | 0.047 | 4 |
| 185 | 0.19 | 0.094 | 0.75 | 408 | 0.5 | 0.25 | 24 |
| 186 | 0.063 | 0.016 | 0.19 | 409 | 0.25 | < 0.0039 | 0.5 |
| 187 | 0.063 | 0.047 | 2 | 410 | 0.13 | 0.13 | 6 |
| 188 | 0.047 | 0.018 | 0.22 | 411 | 2 | 0.5 | 16 |
| 189 | 0.031 | 0.023 | 0.19 | 412 | 0.031 | 0.012 | 0.13 |
| 190 | 0.063 | 0.02 | 0.31 | 413 | 0.031 | 0.0078 | 0.25 |
| 191 | 0.031 | 0.016 | 0.75 | 414 | 0.5 | 0.25 | 64 |
| 192 | 0.063 | 0.012 | 0.25 | 415 | > 4.0 | 4 | 64 |
| 193 | 0.063 | 0.047 | 0.5 | 416 | 0.5 | 2 | > 64 |
| 194 | 0.016 | 0.0078 | 0.25 | 417 | 13 | 4 | > 64 |
| 195 | 0.063 | 0.0078 | 0.5 | 418 | 4 | > 4.0 | > 64 |
| 196 | 0.094 | 0.013 | 0.67 | 419 | 4 | 0.75 | 32 |
| 197 | 0.078 | 0.011 | 0.31 | 420 | 4 | 1 | 24 |
| 198 | 0.063 | 0.018 | 0.24 | 421 | 0.063 | 0.063 | 2 |
| 199 | 0.031 | 0.031 | 3 | 422 | 0.063 | 0.031 | 1.5 |
| 200 | 1 | 0.13 | 4 | 423 | 0.063 | 0.014 | 0.44 |
| 201 | 0.19 | 0.031 | 2 | 424 | 0.063 | 0.016 | 0.56 |
| 202 | 0.031 | 0.016 | 0.25 | 425 | 0.047 | 0.018 | 0.63 |
| 203 | 0.031 | 0.012 | 0.13 | 426 | 0.063 | < 0.0039 | 0.19 |
| 204 | 0.031 | 0.0078 | 0.19 | 427 | 0.063 | 0.047 | 1 |
| 205 | 0.063 | 0.016 | 0.25 | 428 | 0.094 | 0.023 | 1 |
| 206 | 0.38 | 0.094 | 16 | 429 | 0.063 | 0.0078 | 0.75 |
| 207 | 1.5 | 0.063 | 16 | 430 | 0.063 | 0.031 | 1.3 |
| 208 | 0.25 | 0.029 | 1.3 | 431 | 0.063 | 0.023 | 0.88 |
| 209 | 0.19 | 0.094 | 2 | 432 | 0.094 | 0.016 | 0.13 |
| 210 | 0.094 | 0.029 | 0.11 | 433 | 0.5 | 0.094 | 4 |
| 211 | 0.063 | 0.055 | 0.11 | 434 | 0.25 | 0.047 | 2 |
| 212 | 0.031 | 0.047 | 2 | 435 | 0.5 | 0.063 | 4 |
| 213 | 0.047 | 0.023 | 2 | 436 | 0.047 | 0.0078 | 0.38 |
| 214 | 0.25 | 0.031 | 3 | 437 | 0.063 | 0.0078 | 0.25 |
| 215 | 0.19 | 0.039 | 1.8 | 438 | 0.063 | 0.031 | 0.38 |
| 216 | 1 | 0.25 | 8 | 439 | 0.047 | 0.0078 | 0.5 |
| 217 | 0.094 | 0.016 | 0.5 | 440 | 0.19 | 0.063 | 8 |
| 218 | 0.25 | 0.031 | 2 | 441 | 0.063 | 0.016 | 1 |
| 219 | 0.19 | 0.023 | 1.5 | 442 | 0.063 | 0.0078 | 0.19 |
| 220 | 0.25 | 0.047 | 2.5 | 443 | 0.094 | 0.047 | 4 |
| 221 | 0.063 | 0.023 | 0.19 | 444 | 0.094 | 0.031 | 3 |
| 222 | 1 | 0.25 | 32 | 445 | 0.094 | 0.016 | 1 |
| 223 | 0.031 | 0.016 | 0.25 | 446 | 0.063 | 0.012 | 0.5 |
| 224 | 0.13 | 0.031 | 0.75 | 447 | 0.13 | 0.012 | 2 |
| 225 | 0.047 | 0.012 | 0.25 | 448 | 0.023 | 0.016 | 0.75 |
| 226 | 0.094 | 0.012 | 0.38 | 449 | 0.094 | 0.063 | 1 |
| 227 | 1.5 | 0.38 | 16 | 450 | 0.13 | 0.063 | 16 |
| 228 | 0.13 | 0.016 | 1 | 451 | 0.023 | 0.016 | 2 |
| 229 | 0.047 | 0.016 | 1.5 | 452 | 0.19 | 2 | 64 |
| 230 | 0.38 | 0.031 | 4 | 453 | 0.13 | 0.13 | 16 |
| 231 | 0.13 | 0.063 | 8 | 454 | 0.13 | 0.13 | 32 |
| 232 | 1.5 | 0.25 | > 64 | 455 | 0.5 | 0.5 | 32 |
| 233 | 0.5 | 0.13 | 32 | 456 | 0.25 | 0.5 | 64 |
| 234 | 1 | 0.25 | 32 | 457 | 0.047 | 0.0078 | 0.25 |
| 235 | 0.094 | 0.012 | 1 | 458 | 0.25 | 0.0078 | 16 |
| 236 | 1 | 1.5 | > 64 | 459 | 4 | 1.5 | > 64 |
| 237 | 0.13 | 0.094 | 16 | 460 | 2 | 1.5 | 16 |
| 238 | 1 | 0.5 | >64 | 461 | 0.063 | 4 | > 64 |
| 239 | 0.047 | 0.094 | 3 | 462 | 0.094 | 4 | 64 |
| 240 | 0.19 | 0.094 | 1.5 | 463 | 0.5 | 0.5 | > 64 |
| 241 | 0.13 | 0.063 | 6 | 464 | 1 | > 4.0 | > 64 |
| 242 | 1 | 0.5 | >64 | 465 | 0.094 | 0.19 | 4 |
| 243 | 0.094 | 0.031 | 1.5 | 466 | 0.25 | 0.063 | 64 |
| 244 | 0.13 | 0.19 | 48 | 467 | 1 | 1 | > 64 |
| 245 | 1 | 0.38 | >64 | 468 | 0.19 | 0.5 | 64 |
| 246 | 0.13 | 0.063 | 16 | 469 | 4 | > 4.0 | > 64 |
| 247 | 0.094 | 0.094 | 2 | 470 | 3 | 0.5 | > 64 |
| 248 | 0.19 | 0.094 | 1 | 471 | NT | < 0.063 | 48 |
| 249 | 0.13 | 0.012 | 0.69 | 472 | 1 | 0.19 | > 64 |
| 250 | 1 | 0.25 | 64 | 473 | 1.5 | 0.25 | > 64 |
| 251 | 4 | 4 | 8 | 474 | 2 | 0.19 | 4 |
| 252 | 0.25 | 0.023 | 2 | 475 | 1 | 0.5 | 6 |
| 253 | 2 | 2 | > 64 | 476 | 2 | 8 | 32 |
| 254 | 0.5 | 0.5 | 48 | 477 | 0.063 | 0.063 | 4 |
| 255 | 2 | 3 | > 64 | 478 | 0.19 | 0.19 | 6 |
| 256 | 0.19 | 0.094 | 16 | 479 | 0.25 | 0.5 | 32 |
| 257 | 0.13 | 0.063 | 12 | 480 | 0.19 | 0.012 | 0.5 |
| 258 | 1 | 0.19 | 64 | 481 | 0.047 | 0.0078 | 0.25 |
| 259 | 0.5 | 0.25 | 64 | 482 | 0.13 | 0.13 | 16 |
| 260 | 0.094 | 0.031 | 2 | 483 | 2 | 0.5 | 48 |
| 261 | 0.13 | 0.047 | 0.5 | 484 | 0.063 | 0.063 | 8 |
| 262 | 0.063 | 0.02 | 1 | 485 | 0.063 | 0.031 | 2 |
| 263 | 0.13 | 0.023 | 2 | 486 | 0.094 | 0.13 | 3 |
| 264 | 0.13 | 0.094 | 16 | 487 | 0.5 | 0.25 | 8 |
| 265 | 0.13 | 0.043 | 2.5 | 488 | 0.094 | 0.13 | 4 |
| 266 | 0.5 | 0.083 | 3.5 | 489 | 0.19 | 0.25 | 8 |
| 267 | 0.027 | 0.012 | 0.13 | 490-P1 | 0.073 | 0.068 | 0.75 |
| 268 | 0.035 | < 0.0039 | 0.22 | 490-P2 | > 0.50 | > 0.13 | 8 |
| 269 | 0.25 | 0.023 | 1 | 491 | NT | < 0.063 | 0.75 |
| 270 | 0.094 | 0.016 | 0.63 | 492 | 2 | 0.5 | 24 |
| 271 | 0.047 | 0.016 | 0.31 | 492 | 3 | 2 | > 64 |
| 272 | 0.047 | 0.0078 | 0.13 | 493 | 3 | 2 | >64 |
| 273 | 0.031 | 0.016 | 0.5 | 494 | 1 | 0.5 | 16 |
| 274 | 0.13 | 0.02 | 1.3 | 495 | 3 | 1 | 16 |
| 275 | 0.063 | 0.014 | 0.38 | 496 | 3 | 0.5 | 16 |
| 276 | 0.58 | 0.031 | 2 | 497-P1 | 0.063 | 0.016 | 2 |
| 277 | 0.063 | 0.012 | 1 | 497-P2 | 1 | 0.38 | 24 |
| 278 | 0.5 | 0.13 | 4 | 498 | 0.5 | 1 | 16 |
| 279 | 0.13 | 0.02 | 0.5 | 499 | 0.063 | 0.023 | 0.5 |
| 280 | 0.13 | 0.027 | 0.63 | 500 | 0.19 | 0.13 | 3 |
| 281 | 0.023 | 0.0078 | 0.25 | 501 | 0.38 | > 0.13 | 16 |
| 282 | 0.016 | 0.0078 | 0.25 | 502 | 0.063 | 0.003 | 0.25 |
| 283 | 0.063 | 0.012 | 1 | 503 | 0.031 | 0.002 | 0.13 |
| 284 | 0.13 | 0.012 | 1 | 504 | 0.094 | 0.016 | 0.25 |
| 285 | 0.063 | 0.0078 | 0.75 | 505 | 0.25 | 0.016 | 2 |
| 287 | 0.094 | 0.0078 | 0.38 | 506 | NT | NT | NT |
| 288 | 0.063 | 0.0078 | 0.25 | 507 | 0.031 | 0.016 | 0.75 |
| 289 | 0.031 | 0.0078 | 0.25 | 508 | 0.75 | > 4.0 | > 64 |
| 290 | 0.063 | 0.012 | 0.38 | 509 | 3 | > 4.0 | > 64 |
| 291 | 0.063 | 0.0078 | 0.19 | 510 | 1.5 | > 4.0 | > 64 |
| 292 | 0.047 | 0.0078 | 0.38 | 511 | 1.5 | 1 | > 64 |
| 293 | 0.063 | < 0.0039 | 0.38 | 512 | 0.38 | 0.5 | 64 |
| 294 | 0.063 | 0.0078 | 0.25 | 513 | 0.19 | 0.13 | 16 |
| 295 | 0.031 | < 0.0039 | 0.13 | 514 | 0.25 | 0.19 | 32 |
| 296 | 0.047 | 0.031 | 2 | 515 | 0.094 | 0.063 | 4 |
| 297 | 0.063 | 0.0078 | 1 | 516 | 2 | > 4.0 | > 64 |
| 298 | 0.063 | 0.0078 | 1 | 517 | 0.38 | 0.19 | 6 |
| 299 | 0.094 | 0.016 | 0.5 | 518 | 0.38 | 0.063 | 4 |
| 300 | 0.094 | 0.0078 | 0.5 | 519 | 0.13 | 0.25 | 16 |
| 301 | 4 | 4 | > 64 | 520 | 0.38 | 0.19 | 6 |
| 302 | 0.031 | 0.016 | 0.25 | 521 | 0.25 | 0.016 | 12 |
| 303 | 0.047 | 0.012 | 0.19 | 522 | 0.5 | 0.047 | 24 |
| 304 | 0.047 | 0.016 | 1 | 523 | 1 | 0.094 | > 64 |
| 305 | 0.063 | 0.016 | 1 | 524-1 | 0.22 | 0.19 | 16 |
| 306 | 0.031 | 0.0078 | 1 | 524-2 | 0.5 | > 0.13 | >4 |
| 307 | 0.25 | 0.031 | 0.88 | 525 | 1.5 | > 4.0 | > 64 |
| 308 | 0.063 | 0.016 | 1 | 526-1 | 0.19 | 0.13 | 16 |
| 309 | 0.19 | 0.02 | 1.1 | 526-2 | > 0.5 | > 0.13 | 64 |
| 310 | 0.047 | 0.0098 | 0.13 | 526-3 | 0.063 | 0.031 | 3 |
| 311 | 0.047 | 0.012 | 0.25 | 527 | 0.063 | 0.063 | 4 |
| 312 | 0.25 | 0.023 | 0.5 | 528 | 0.19 | 0.38 | 32 |
| 313 | 0.031 | 0.0078 | 0.5 | | | | |
| 314 | 0.078 | 0.023 | 0.44 | | | | |
| 315 | 0.078 | 0.014 | 0.38 | | | | |
| 316 | 0.13 | 2 | > 64 | | | | |
| 317 | 0.094 | 1 | 64 | | | | |
| 318 | NT | NT | NT | | | | |
| 319 | 2 | 0.75 | > 64 | | | | |
| 320 | 0.13 | 2 | > 64 | | | | |
| 321 | 0.38 | 0.016 | 1 | | | | |
| 322 | 1.5 | 0.13 | 4 | | | | |
| 323 | 0.75 | 0.094 | 1.5 | | | | |
| 324 | 0.25 | 0.031 | 1 | | | | |
| 325 | 0.13 | < 0.0039 | 0.5 | | | | |

| | | | | | | | |
|---|---|---|---|---|---|---|---|
| NT = not tested | | | | | | | |

### Example 428: Whole-cell SpsB Biochemical Screening Assay

A kinetic fluorogenic enzyme activity assay was used to assess inhibition of SpsB (*Staphylococcus aureus* signal peptidase) activity and IC₅₀s were determined. This assay uses a suspension of *Staphylococcus aureus* cells as a source of SpsB instead of recombinant SpsB protein.

Cell preparation: Luria broth (LB) was inoculated with *S*. *aureus* (USA300 background, overexpressing SpsB) and shaken at 37°C until an OD₆₀₀ₙₘ of 1.5-2.0 was reached (~4 hr). The culture was then diluted to an OD₆₀₀ₙₘ of 1.0 with LB, aliquoted and centrifuged at 10,000x g for 2 mins. The supernatant was removed and the pellet was resuspended in phosphate buffer (1× PBS, 12.5 mg/L MgCl₂, 25 mg/L CaCl₂, 0.1% Tween-80) to an OD₆₀₀ₙₘ of 0.5, then centrifuged again at 10,000x g for 2 mins. The supernatant was removed and the pellets were frozen at -20°C.

Test compounds were prepared in DMSO at a concentration of 10 mg/mL. These compound stocks were diluted into DMSO to a concentration of 25 µg/mL and serial 3-fold dilutions were made in DMSO, for a total of 11 compound concentrations. 20 nL of each compound solution was pre-spotted into a white 384-well plate (50 µL/well polypropylene, Nunc) using acoustic fluid transfer (Echo).

Frozen S. aureus pellets were resuspended in assay buffer (1× PBS, 12.5 mg/L MgCl2, 25 mg/L CaCl2, 0.1% Tween-80) to an OD600nm of 0.05, then mixed 1:1 (v/v) with 20 µM substrate ((Dabcyl)βAla-KPAKAAE(Edans)) in assay buffer, and this solution was added (20 µL/well) to the 384-well plate that had been pre-spotted with compound. Fluorescence intensity was then immediately read kinetically for 30 minutes with 2 minute read intervals to monitor cleavage of the internally quenched peptide substrate (excitation wavelength = 340 nm, emission wavelength = 490nm, Molecular Devices Spectramax M5). Reaction rate (slope) was plotted against inhibitor concentration to derive the IC₅₀. The results are listed in Table 2.

**Table 2**

| Compound | SpsB IC50 (nM) | Compound | SpsB IC50 (nM) | Compound | SpsB IC50 (nM) | Compound | SpsB IC50 (nM) |
|---|---|---|---|---|---|---|---|
| 101 | 0.72 | 226 | 0.88 | 352 | 0.8 | 475 | 18 |
| 102 | 0.52 | 227 | 5.5 | 353 | 0.82 | 476 | 86 |
| 103 | 0.57 | 228 | NT | 354 | 1.8 | 477 | 0.68 |
| 104 | 0.5 | 229 | 0.92 | 355 | 1.1 | 478 | 1.5 |
| 105 | 0.82 | 230 | 1.3 | 356 | NT | 479 | NT |
| 106 | 1.7 | 231 | 0.57 | 357-P1 | NT | 480 | 0.92 |
| 107 | 4 | 232 | 2.4 | 357-P2 | 1.1 | 481 | 1.2 |
| 108 | 1.2 | 233 | 4 | 359 | NT | 482 | 1.6 |
| 109 | 7.5 | 234 | 3.9 | 360 | 1.2 | 483 | 6.8 |
| 110 | 0.96 | 235 | 0.98 | 361 | NT | 484 | 1 |
| 111 | 1 | 236 | 1.5 | 362 | NT | 485 | NT |
| 112 | 0.77 | 237 | 1.1 | 363 | NT | 486 | NT |
| 113 | 0.51 | 238 | NT | 364 | NT | 487 | NT |
| 114 | 6.1 | 239 | 0.74 | 365 | NT | 488 | NT |
| 115 | NT | 240 | NT | 366 | 0.64 | 489 | NT |
| 116 | 1.3 | 241 | 1.4 | 367 | 0.88 | 490-P1 | NT |
| 117 | 2 | 242 | NT | 368 | NT | 490-P2 | NT |
| 118 | 1.4 | 243 | 1.3 | 369 | 0.47 | 491 | <0.5 |
| 119 | NT | 244 | 1 | 370 | 1.1 | 492 | NT |
| 120 | 1 | 245 | 6.3 | 371 | 1.3 | 492 | NT |
| 121 | 1.2 | 246 | 1.1 | 372 | 0.47 | 493 | NT |
| 122 | 1.6 | 247 | NT | 373 | 0.88 | 494 | 2.1 |
| 123 | 1.2 | 248 | NT | 374 | NT | 495 | 20 |
| 124 | 1.6 | 249 | 1.2 | 375 | NT | 496 | 17 |
| 125 | NT | 250 | 7 | 376 | NT | 497-P1 | NT |
| 126 | NT | 251 | NT | 377 | 1 | 497-P2 | NT |
| 127 | 5.9 | 252 | NT | 378 | 0.87 | 498 | NT |
| 128 | NT | 253 | 12 | 379 | 0.89 | 499 | NT |
| 129 | 0.67 | 254 | 1.6 | 380 | NT | 500 | NT |
| 130 | NT | 255 | NT | 381 | 2.9 | 501 | NT |
| 131 | 0.74 | 256 | 0.8 | 382 | NT | 502 | NT |
| 132 | 0.71 | 257 | 0.92 | 383 | 0.88 | 503 | NT |
| 133 | 1.1 | 258 | 2.8 | 384 | 1.2 | 504 | NT |
| 134 | 3.5 | 259 | NT | 385 | NT | 505 | NT |
| 135 | 0.54 | 260 | 1.1 | 386 | NT | 506 | NT |
| 136 | 10 | 261 | 4.6 | 387 | NT | 507 | NT |
| 137 | 1.1 | 262 | 0.9 | 388 | NT | 508 | NT |
| 138 | 0.93 | 263 | 1.1 | 389 | 0.81 | 509 | NT |
| 139 | 0.94 | 264 | 2 | 390 | 1.1 | 510 | NT |
| 140 | 0.99 | 265 | 0.89 | 391 | 0.99 | 511 | NT |
| 141 | 0.75 | 266 | 2.1 | 392 | NT | 512 | NT |
| 142 | 0.72 | 267 | 0.63 | 393-P1 | 5.9 | 513 | NT |
| 143 | 0.57 | 268 | 0.86 | 393-P2 | 4.7 | 514 | 2 |
| 144 | 0.91 | 269 | 1.2 | 394-P1 | 3.9 | 515 | NT |
| 145 | 0.59 | 270 | 0.89 | 394-P2 | 2.7 | 516 | NT |
| 146 | 0.9 | 271 | 1.1 | 395 | NT | 517 | NT |
| 147 | 0.69 | 272 | 0.7 | 396 | NT | 518 | NT |
| 148 | 0.75 | 273 | 0.6 | 397 | NT | 519 | NT |
| 149 | 0.8 | 274 | 0.79 | 398 | NT | 520 | 0.73 |
| 150 | 0.82 | 275 | 0.55 | 399 | NT | 521 | 2.3 |
| 151 | 0.59 | 276 | 1.8 | 400 | 5 | 522 | 3.1 |
| 152 | NT | 277 | 1.1 | 401 | NT | 523 | 5.6 |
| 153 | NT | 278 | NT | 402 | NT | 524-1 | NT |
| 154 | NT | 279 | NT | 403 | <0.5 | 524-2 | NT |
| 155 | 2.5 | 280 | 2 | 404 | 0.88 | 525 | NT |
| 156 | NT | 281 | 0.75 | 405 | 0.68 | 526-1 | NT |
| 157 | 0.79 | 282 | 0.71 | 406 | 1.2 | 526-2 | NT |
| 158 | NT | 283 | 0.87 | 407 | 1.1 | 526-3 | NT |
| 159 | 0.82 | 284 | 0.82 | 408 | NT | 527 | NT |
| 160 | 0.66 | 285 | NT | 409 | NT | 528 | NT |
| 161 | NT | 287 | NT | 410 | NT | | |
| 162 | 0.72 | 288 | 1.1 | 411 | NT | | |
| 163 | 0.71 | 289 | 0.93 | 412 | 0.6 | | |
| 164 | 0.62 | 290 | 1.2 | 413 | 0.88 | | |
| 165 | 0.61 | 291 | 1.3 | 414 | NT | | |
| 166 | 0.78 | 292 | 1.2 | 415 | 14 | | |
| 167 | NT | 293 | 1.5 | 416 | NT | | |
| 168 | 0.8 | 294 | NT | 417 | NT | | |
| 169 | NT | 295 | 0.69 | 418 | NT | | |
| 170 | 0.72 | 296 | NT | 419 | NT | | |
| 171 | 0.72 | 297 | NT | 420 | 8.4 | | |
| 172 | 1 | 298 | 0.82 | 421 | 1.6 | | |
| 173 | NT | 299 | 1.1 | 422 | 1.1 | | |
| 174 | 0.91 | 300 | NT | 423 | 0.85 | | |
| 175 | 0.76 | 301 | NT | 424 | 0.75 | | |
| 176 | 2.6 | 302 | 0.79 | 425 | 1.1 | | |
| 177 | 1.1 | 303 | 0.93 | 426 | 1.2 | | |
| 178 | 0.9 | 304 | 0.68 | 427 | 1.2 | | |
| 179 | 1.8 | 305 | 0.73 | 428 | 1.2 | | |
| 180 | NT | 306 | 0.69 | 429 | 0.81 | | |
| 181 | NT | 307 | 3.5 | 430 | 1 | | |
| 182 | 3.2 | 308 | 0.71 | 431 | 0.77 | | |
| 183 | 0.62 | 309 | 1 | 432 | 1.4 | | |
| 184 | 0.89 | 310 | 0.99 | 433 | NT | | |
| 185 | 0.91 | 311 | 0.64 | 434 | 1.5 | | |
| 186 | 0.56 | 312 | NT | 435 | NT | | |
| 187 | 0.74 | 313 | 0.68 | 436 | 0.78 | | |
| 188 | 0.67 | 314 | NT | 437 | 0.9 | | |
| 189 | NT | 315 | NT | 438 | 0.78 | | |
| 190 | 0.7 | 316 | 0.84 | 439 | NT | | |
| 191 | 0.62 | 317 | 0.87 | 440 | 0.81 | | |
| 192 | 0.89 | 318 | 0.8 | 441 | NT | | |
| 193 | 0.93 | 319 | 6.3 | 442 | 2.9 | | |
| 194 | 0.83 | 320 | 0.97 | 443 | 0.9 | | |
| 195 | 1.4 | 321 | 1.8 | 444 | NT | | |
| 196 | 1.1 | 322 | 8.4 | 445 | 1 | | |
| 197 | <0.5 | 323 | 2.8 | 446 | NT | | |
| 198 | 0.72 | 324 | 0.82 | 447 | NT | | |
| 199 | 0.73 | 325 | 0.77 | 448 | 0.93 | | |
| 200 | 2.8 | 326 | 0.98 | 449 | NT | | |
| 201 | 1.4 | 327 | NT | 450 | 0.66 | | |
| 202 | 0.66 | 328 | NT | 451 | NT | | |
| 203 | 0.72 | 329 | 0.71 | 452 | NT | | |
| 204 | 1.2 | 330 | 1 | 453 | 2.3 | | |
| 205 | 0.85 | 331 | 0.34 | 454 | 1.8 | | |
| 206 | NT | 332 | 0.56 | 455 | NT | | |
| 207 | NT | 333 | 0.8 | 456 | NT | | |
| 208 | 0.99 | 334 | 1.3 | 457 | 0.64 | | |
| 209 | 0.92 | 335 | 0.56 | 458 | NT | | |
| 210 | 0.8 | 336 | <0.5 | 459 | NT | | |
| 211 | NT | 337 | <0.5 | 460 | 14 | | |
| 212 | 0.62 | 338 | <0.5 | 461 | 0.74 | | |
| 213 | 0.64 | 339 | <0.5 | 462 | 0.81 | | |
| 214 | 0.98 | 340 | 0.69 | 463 | 3.2 | | |
| 215 | 0.97 | 341 | 1.1 | 464 | 6 | | |
| 216 | 3.3 | 342 | 1.3 | 465 | 1.6 | | |
| 217 | 0.88 | 343 | 0.73 | 466 | NT | | |
| 218 | 0.88 | 344 | 1 | 467 | NT | | |
| 219 | 1.9 | 345 | 2.2 | 468 | 3.4 | | |
| 220 | 1.5 | 346 | 0.7 | 469 | 5 | | |
| 221 | 1.3 | 347 | NT | 470 | 24 | | |
| 222 | NT | 348 | 0.8 | 471 | 10 | | |
| 223 | 1.5 | 349 | NT | 472 | 9.9 | | |
| 224 | 1.3 | 350 | 1 | 473 | 39 | | |
| 225 | NT | 351 | NT | 474 | NT | | |

| | | | | | | | |
|---|---|---|---|---|---|---|---|
| NT = not tested | | | | | | | |

### Example 429: Activity in a neutropenic thigh infection model

The ability of a compound to inhibit an infection of a bacterial pathogen can be measured using a murine neutropenic thigh infection model. The reduction of bacterial burden is a measure of antibacterial activity *in vivo.*

Jugular vein cannulated CD-1 mice were subjected to induced neutropenia (<100 cells/mm³) by injecting 150 mg/kg and 100 mg/kg cyclophosphamide at day -5 and day -2 respectively. At day -1, saline was infused at 20 µL/hour for 12 hours using Harvard Apparatus PHD 2000 Infusion pumps. At day 0, mice were infected in the thigh muscle with 1×10⁵ CFU/ 50 µL of *Escherichia coli* strain ATCC 25922. There were four test groups and one vehicle group that begin dosing at 1 hour post infection:
Group 1 - vehicle control (3% HP-beta-cyclodextrin in PBS)
Group 2 - Compound 135 group dosed at a concentration of 0.62 mg/mL solution, infused at 80µL/hour for 23 hours, with a target steady-state concentration (Css) of 13 µg/mL.
Group 3 - Compound 135 group dosed at a concentration of 0.21 mg/mL solution, infused at 80 µL/hour for 23 hours to achieve a steady state concentration (Css) of 3.4 ug/mL.
Group 4 - Compound 135 group dosed at 0.07 mg/mL solution (Css 1.2 ug/mL) infused at 80 µL/hour for 23 hours.
Group 5 - Compound 135 group dosed at 0.02 mg/mL solution (Css 0.31 ug/mL) infused at 80 µL/hour for 23 hours.

At 24 hours post infection, bacterial burden in the thigh muscle was determined by plating the tissue homogenate in serial dilutions on blood agar plates. As depicted in Figure 1, Compound 135 exhibits dose-dependent reduction in bacterial burden demonstrating its in vivo activity.

### Example 430: Clinical Trial of the Safety and Efficacy of Compounds of Formula (I), (I'), (Ia), (Ib), (Ic), (Id), (II), (II'), (IIa), (IIb), (IIc), (IId), or (IIe) in Patients with C. Difficile-Associated Diarrhea

**Purpose:** This study aims to determine the safety and efficacy of compounds presented herein for the treatment of symptoms of C. difficile-associated diarrhea and lowering the risk of repeat episodes of diarrhea. The compounds are evaluated in comparison to current standard antibiotic treatment, so all patients will receive active medication. All study-related care is provided including doctor visits, physical exams, laboratory tests and study medication. Total length of participation is approximately 10 weeks.

**Patients:** Eligible subjects will be men and women 18 years and older.

### Criteria:

### Inclusion Criteria:

Be at least 18 years old;
Have active mild to moderate C. difficile- Associated Diarrhea (CDAD);
Be able to tolerate oral medication;
Not be pregnant or breast-feeding; and
Sign and date an informed consent form.

**Study Design:** This is a randomized, double-blind, active control study of the efficacy, safety, and tolerability of a compound of Formula (I), (I'), (Ia), (Ib), (Ic), (Id), (II), (II'), (IIa), (IIb), (IIc), (IId), or (IIe) in patients with C. difficile-associated diarrhea.

### Example 431: Clinical Trial Comparing a Compound of Formula (I), (I'), (Ia), (Ib), (Ic), (Id), (II), (II'), (IIa), (IIb), (IIc), (IId), or (IIe) with Vancomycin for the Treatment of MRSA Osteomyleitis

**Purpose:** This study aims to determine the efficacy of compounds presented herein as compared to vancomycin for the treatment of methicillin-resistant Staphylococcus aureus (MRSA) osteomyelitis.

**Patients:** Eligible subjects will be men and women 18 years and older.

### Criteria:

### Inclusion Criteria:

Culture-proven MRSA, obtained in operating room or sterile biopsy procedure from bone site. The infection and sampling site is either within the bone or a deep soft-tissue site that is contiguous with bone; OR radiographic abnormality consistent with osteomyelitis in conjunction with a positive blood culture for MRSA;
Surgical debridement of infection site, as needed;
Subject is capable of providing written informed consent; and
Subject capable of receiving outpatient parenteral therapy for 12 weeks.

### Exclusion Criteria:

Hypersensitivity to a compound of Formula (I), (I'), (Ia), (Ib), (Ic), (Id), (II), (II'), (IIa), (IIb), (IIc), (IId), or (IIe) or vancomycin;
S. aureus resistant to a compound of Formula (I), (I'), (Ia), (Ib), (Ic), (Id), (II), (II'), (IIa), (IIb), (IIc), (IId), or (IIe) or vancomycin;
Osteomyelitis that develops directly from a chronic, open wound;
Polymicrobial culture (the only exception is if coagulase-negative staphylococcus is present in the culture and the clinical assessment is that it is a contaminant);
Subject has a positive pregnancy test at study enrollment;
Baseline renal or hepatic insufficiency that would preclude administration of study drugs;
Active injection drug use without safe conditions to administer intravenous antibiotics for 3 months; and
Anticipated use of antibiotics for greater than 14 days for an infection other than osteomyelitis.

**Study Design:** This is a randomized, open-label, active control, efficacy trial comparing vancomycin with a compound of Formula (I), (I'), (Ia), (Ib), (Ic), (Id), (II), (II'), (IIa), (IIb), (IIc), (IId), or (IIe) for the treatment of MRSA Osteomyelitis.

### Example 432: Clinical Trial Evaluating a Compound of Formula (I), (I'), (Ia), (Ib), (Ic), (Id), (II), (II'), (IIa), (IIb), (IIc), (IId), or (IIe) in Selected Serious Infections Caused by Vancomycin-Resistant Enterococcus (VRE)

**Purpose:** This study aims to determine the safety and efficacy of a compound of Formula (I), (I'), (Ia), (Ib), (Ic), (Id), (II), (II'), (IIa), (IIb), (IIc), (IId), or (IIe) in the treatment of selected serious infections caused by VRE.

**Patients:** Eligible subjects will be men and women 18 years and older.

### Criteria:

### Inclusion Criteria:

Isolation of one of the following multi-antibiotic resistant bacteria: vancomycin-resistant Enterococcus faecium, vancomycin-resistant Enterococcus faecalis alone or as part of a polymicrobial infection; and
Have a confirmed diagnosis of a serious infection (eg, bacteremia [unless due to an excluded infection], complicated intra-abdominal infection, complicated skin and skin structure infection, or pneumonia) requiring administration of intravenous (IV) antibiotic therapy.

### Exclusion Criteria:

Subjects with any concomitant condition or taking any concomitant medication that, in the opinion of the investigator, could preclude an evaluation of a response or make it unlikely that the contemplated course of therapy or follow-up assessment will be completed or that will substantially increase the risk associated with the subject's participation in this study.
Anticipated length of antibiotic therapy less than 7 days.

**Study Design:** This is a randomized, double-blind, safety and efficacy study of a compound of Formula (I), (I'), (Ia), (Ib), (Ic), (Id), (II), (II'), (IIa), (IIb), (IIc), (IId), or (IIe) in the treatment of selected serious infections caused by VRE.

### Pharmaceutical Compositions

### Parenteral Composition

To prepare a parenteral pharmaceutical composition suitable for administration by injection, 100 mg of a compound of Formula (I), (I'), (Ia), (Ib), (Ic), (Id), (II), (II'), (Ila), (IIb), (IIc), (IId), or (IIe) is dissolved in DMSO and then mixed with 10 mL of 0.9% sterile saline. The mixture is incorporated into a dosage unit form suitable for administration by injection.

In another embodiment, the following ingredients are mixed to form an injectable formulation:

| Ingredient | Amount |
|---|---|
| Compound of Formula (I), (I'), (Ia), (Ib), (Ic), (Id), (II), (II'), (IIa), (IIb), (IIc), (IId), or (IIe) | 1.2 g |
| sodium acetate buffer solution (0.4 M) | 2.0 mL |
| HCl (1 N) or NaOH (1 M) | q.s. to suitable pH |
| water (distilled, sterile) | q.s.to 20 mL |

All of the above ingredients, except water, are combined and stirred and if necessary, with slight heating if necessary. A sufficient quantity of water is then added.

### Oral Composition

To prepare a pharmaceutical composition for oral delivery, 100 mg of a compound of Formula (I), (I'), (Ia), (Ib), (Ic), (Id), (II), (II'), (IIa), (IIb), (IIc), (IId), or (IIe) is mixed with 750 mg of starch. The mixture is incorporated into an oral dosage unit, such as a hard gelatin capsule, which is suitable for oral administration.

In another embodiment, the following ingredients are mixed intimately and pressed into single scored tablets.

| Ingredient | Quantity per tablet, mg |
|---|---|
| compound of Formula (I), (I'), (Ia), (Ib), (Ic), (Id), (II), (II'), (IIa), (IIb), (IIc), (IId), or (IIe) | 200 |
| Cornstarch | 50 |
| croscarmellose sodium | 25 |
| Lactose | 120 |
| magnesium stearate | 5 |

In yet another embodiment, the following ingredients are mixed intimately and loaded into a hard-shell gelatin capsule.

| Ingredient | Quantity per tablet, mg |
|---|---|
| compound of Formula (I), (I'), (Ia), (Ib), (Ic), (Id), (II), (II'), (IIa), (IIb), (IIc), (IId), or (IIe) | 200 |
| lactose, spray-dried | 148 |
| magnesium stearate | 2 |

In yet another embodiment, the following ingredients are mixed to form a solution/suspension for oral administration:

| Ingredient | **Amount** |
|---|---|
| Compound of Formula (I), (I'), (Ia), (Ib), (Ic), (Id), (II), (II'), (IIa), (IIb), (IIc), (IId), or (IIe) | 1 g |
| | 0.1 g |
| Anhydrous Sodium Carbonate | |
| Ethanol (200 proof), USP | 10 mL |
| Purified Water, USP | 90 mL |
| Aspartame | 0.003g |

### Topical Gel Composition

To prepare a pharmaceutical topical gel composition, 100 mg of a compound of Formula (I), (I'), (Ia), (Ib), (Ic), (Id), (II), (II'), (IIa), (lib), (IIc), (IId), or (IIe) is mixed with 1.75 g of hydroxypropyl cellulose, 10 mL of propylene glycol, 10 mL of isopropyl myristate and 100 mL of purified alcohol USP. The resulting gel mixture is then incorporated into containers, such as tubes, which are suitable for topical administration.

## Claims

1. A compound selected from: 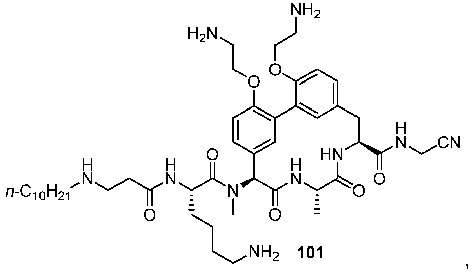 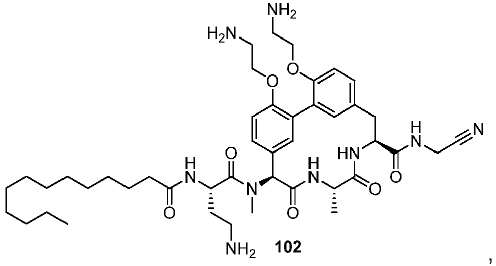 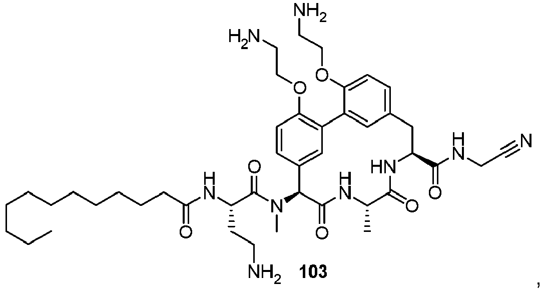 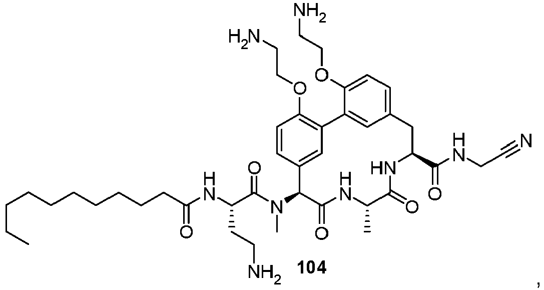 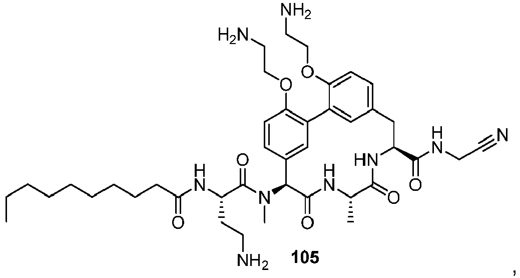 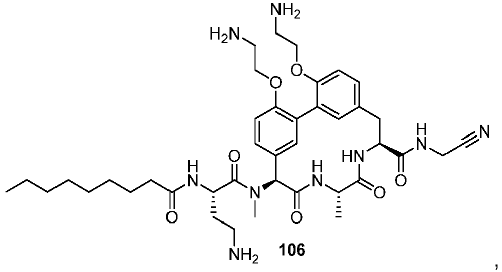 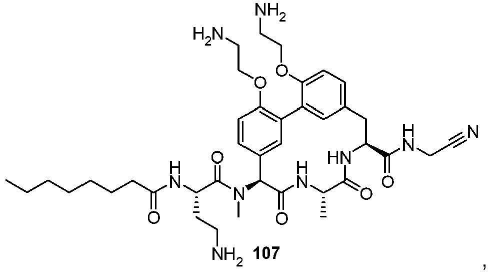 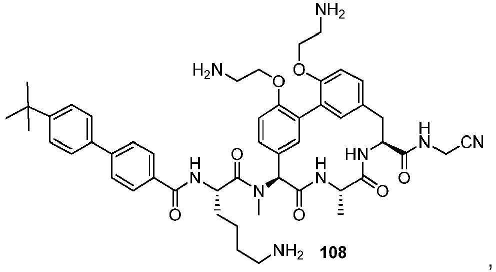 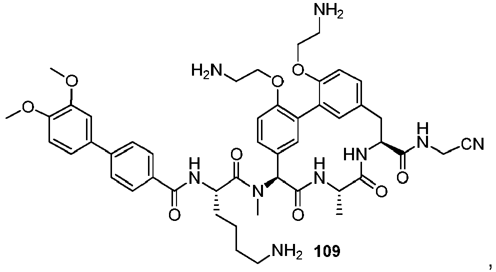 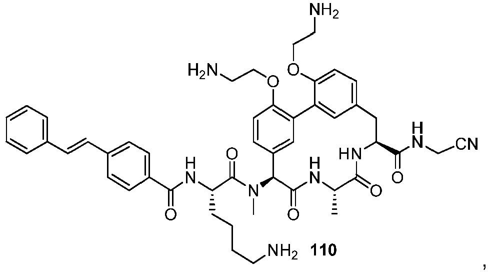 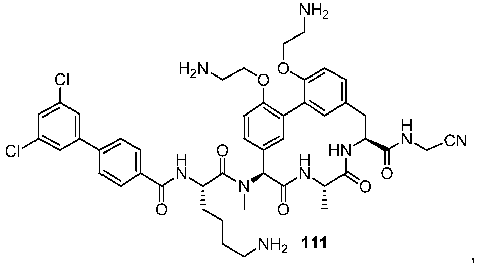 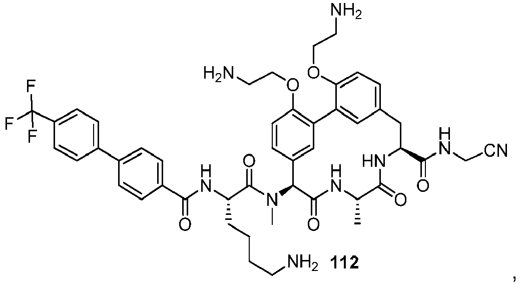 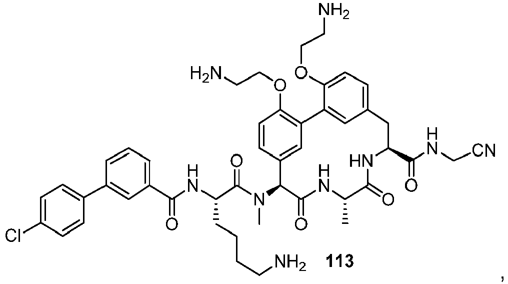 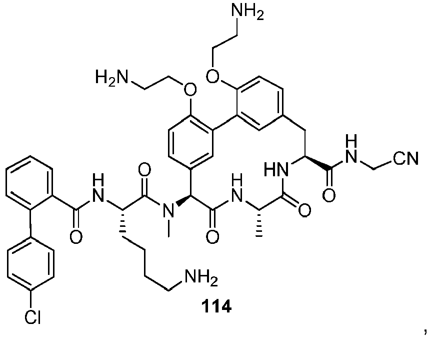 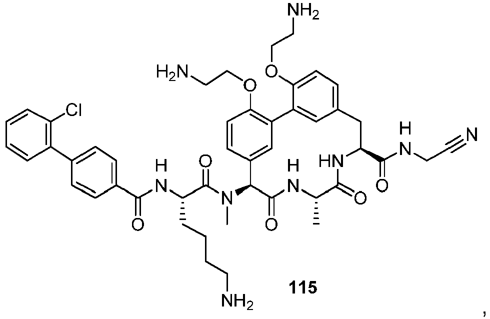 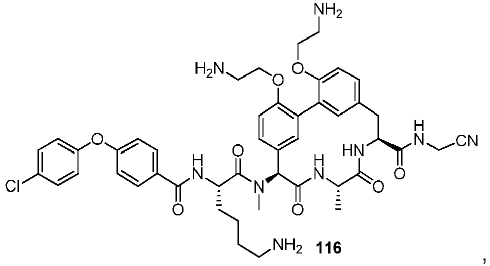 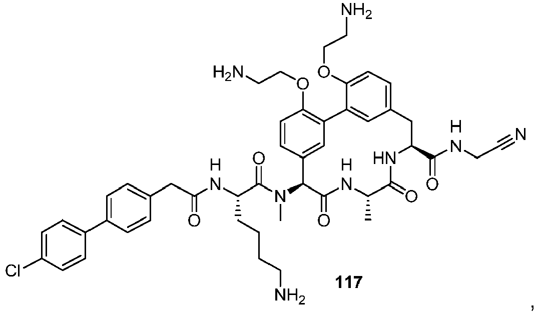 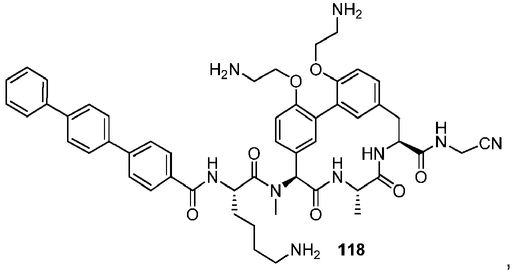 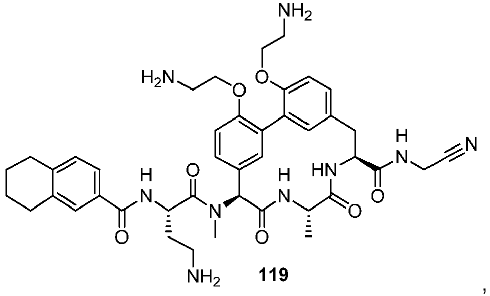 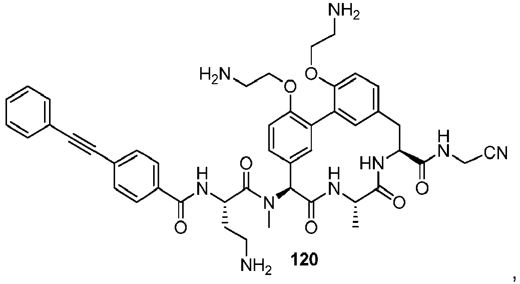 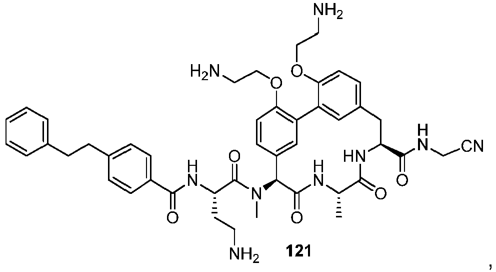 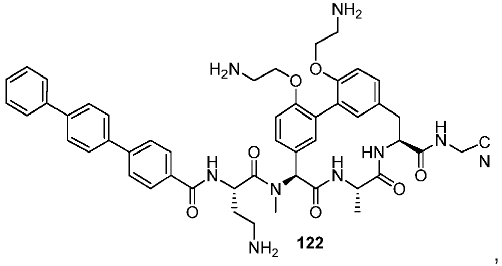 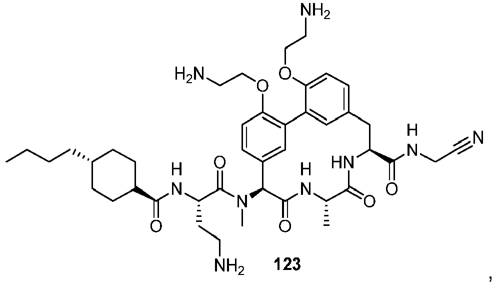 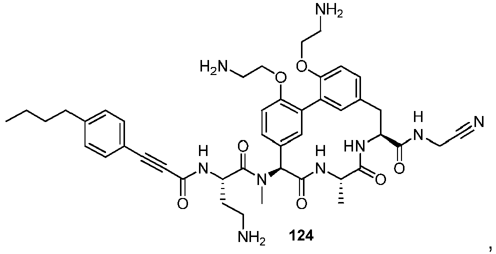 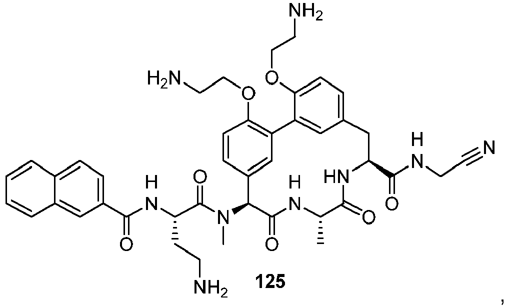 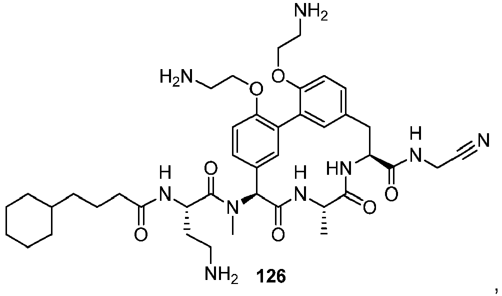 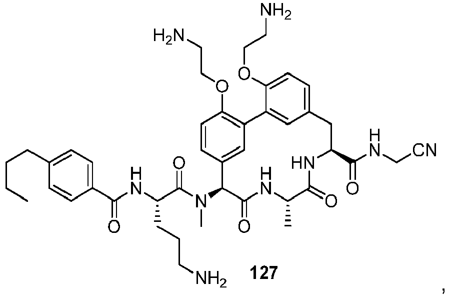 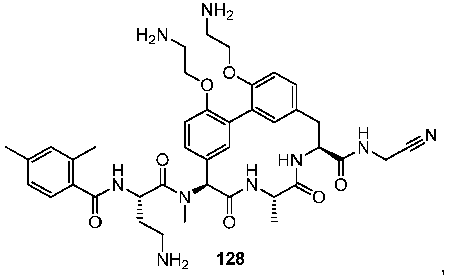 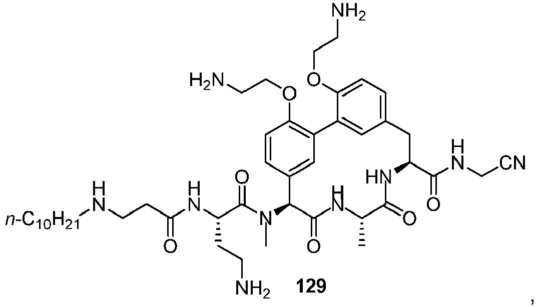 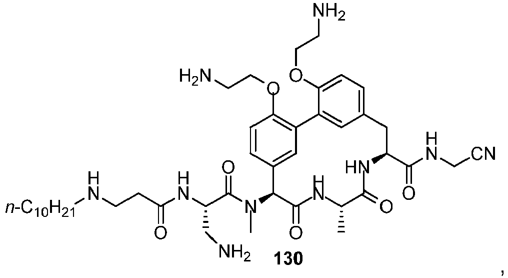 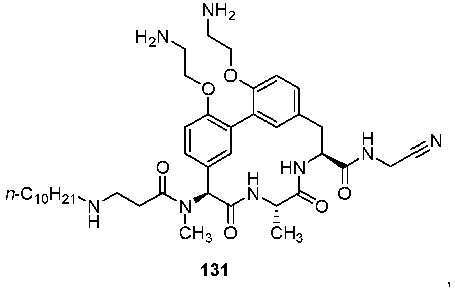 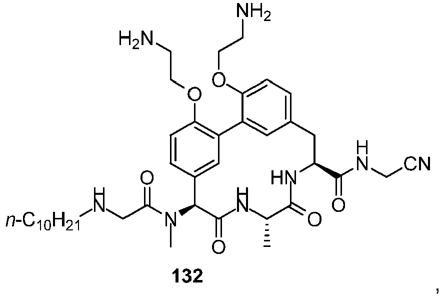 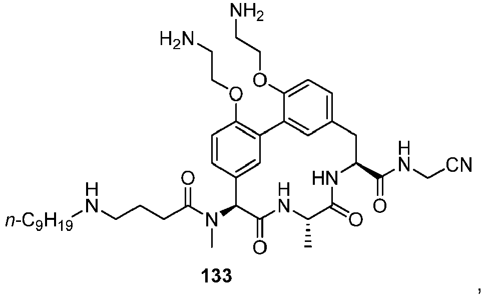 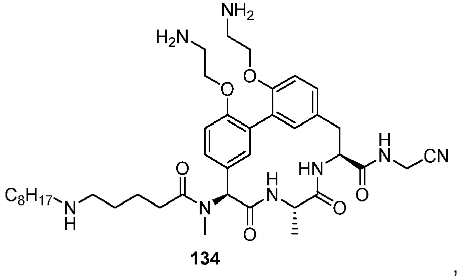 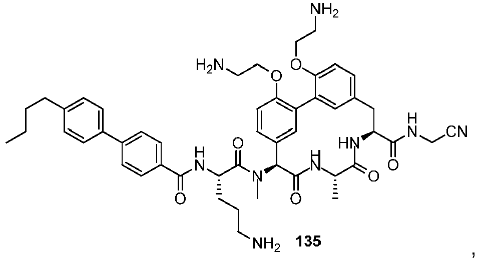 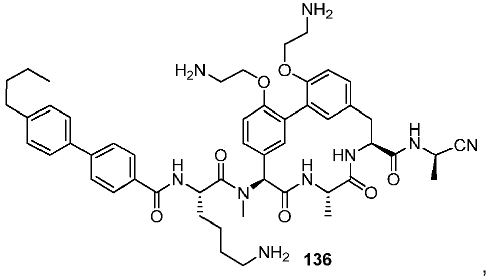 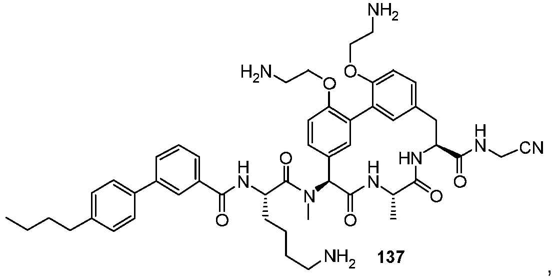 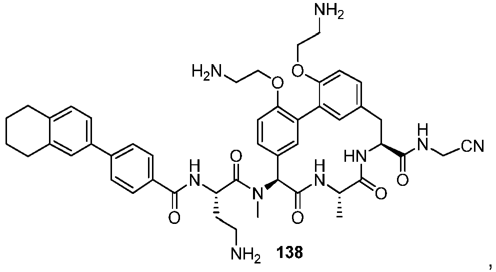 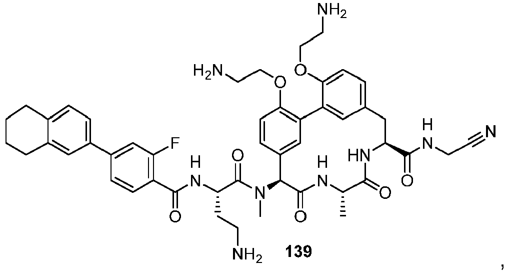 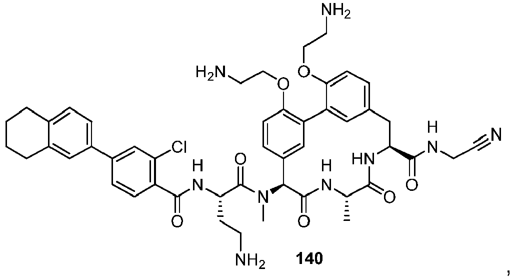 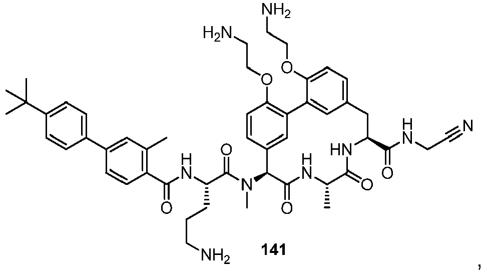 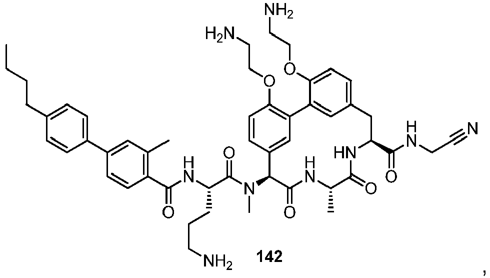 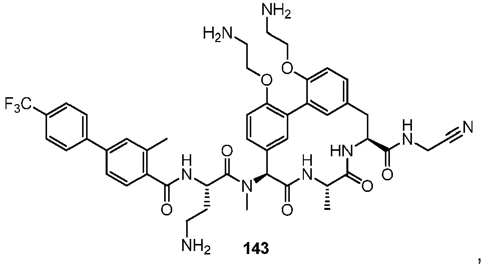 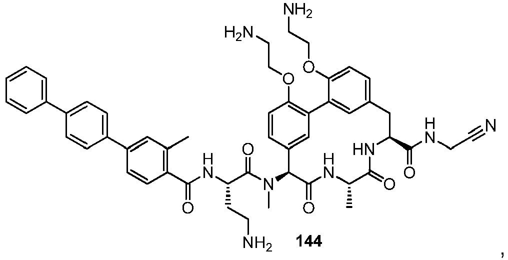 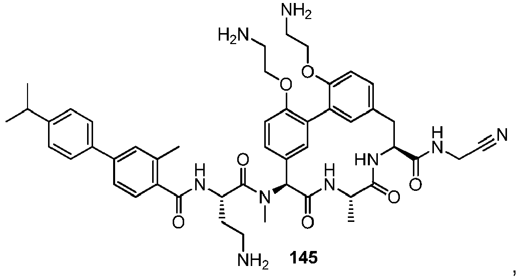 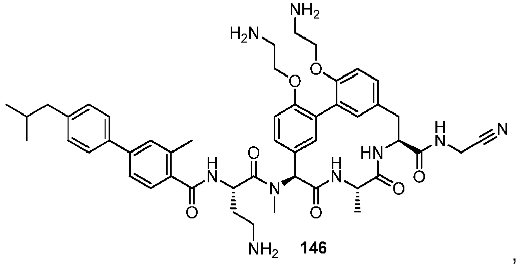 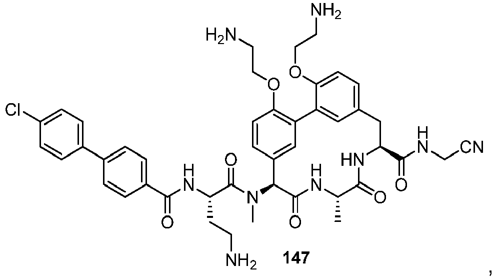 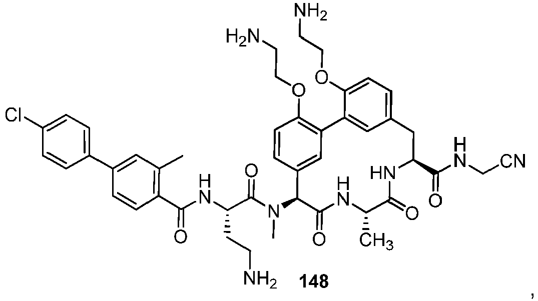 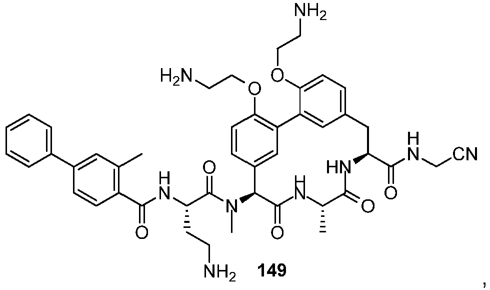 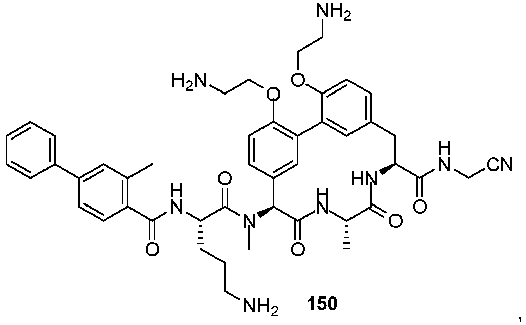 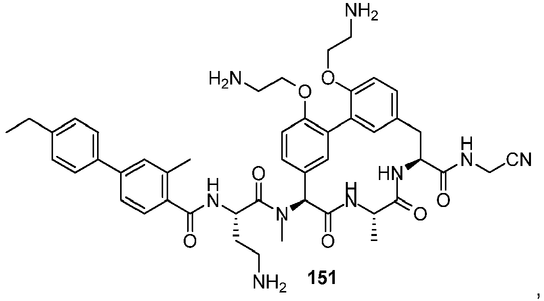 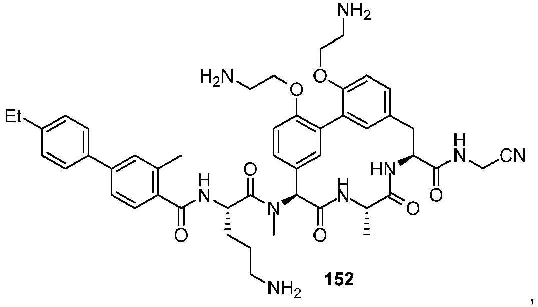 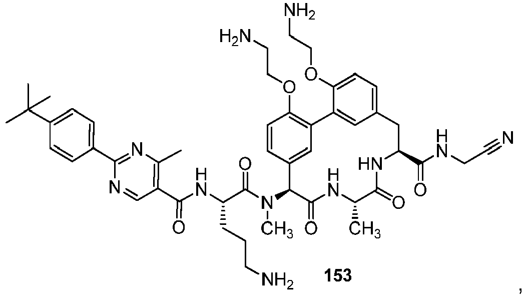 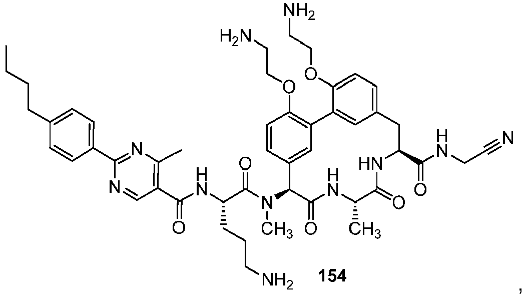 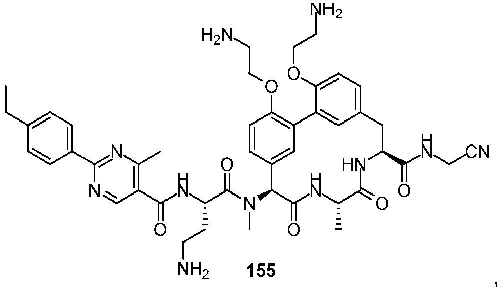 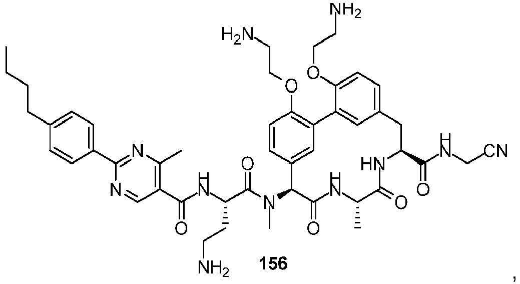 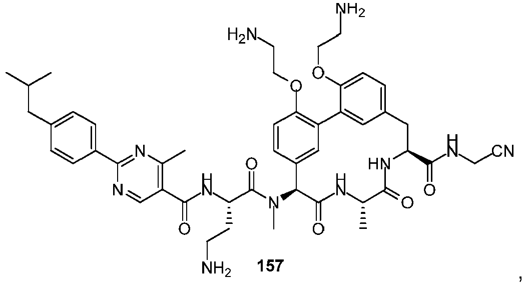 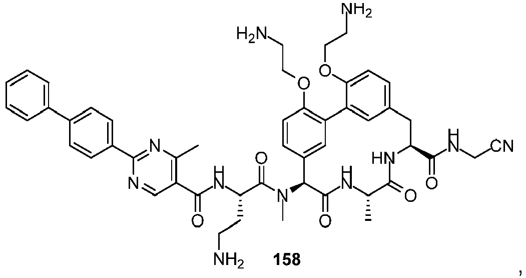 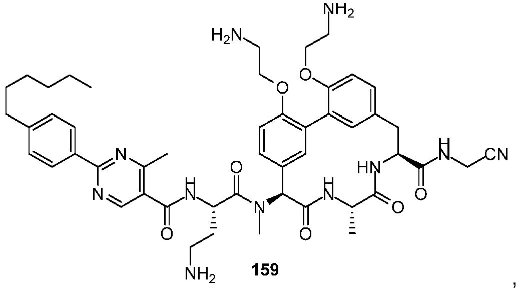 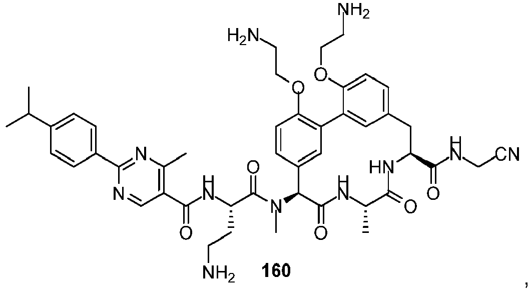 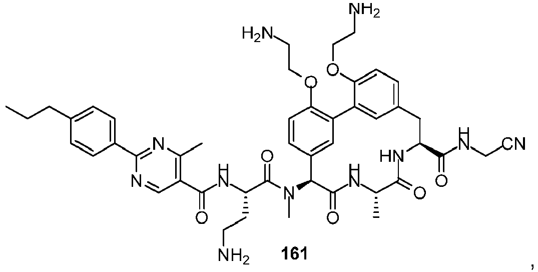 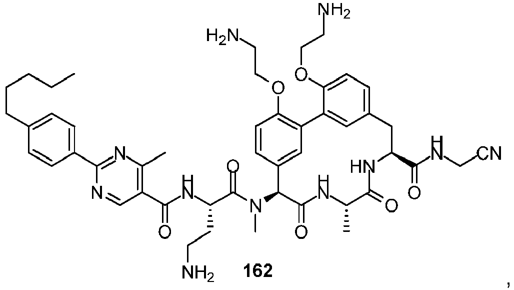 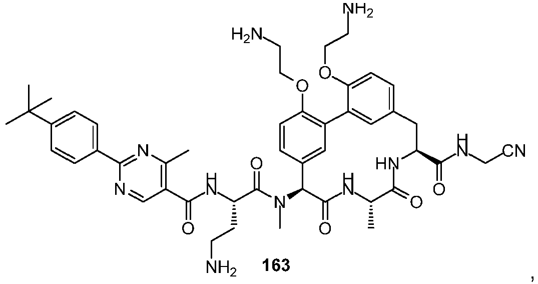 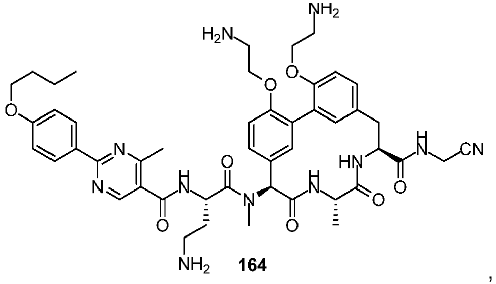 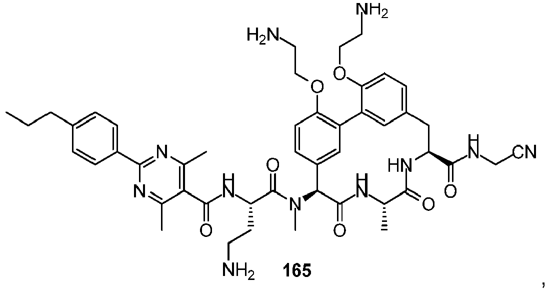 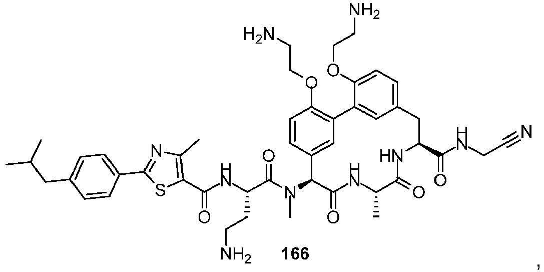 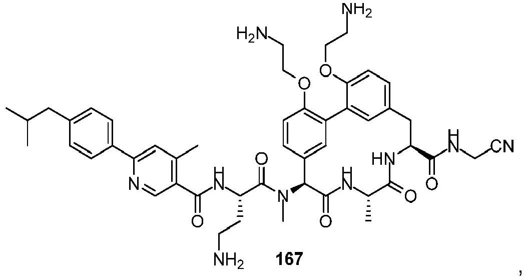 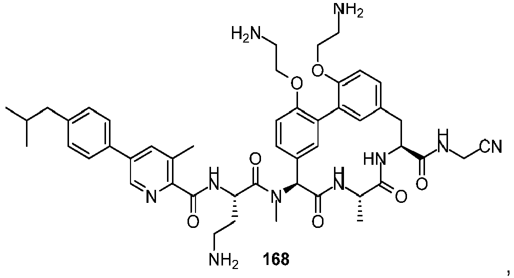 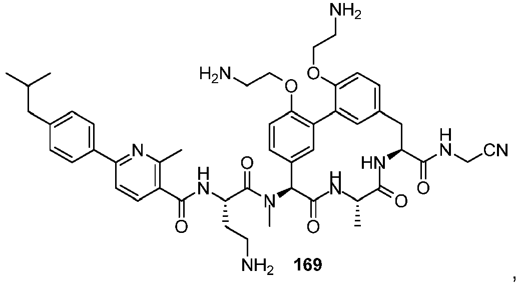 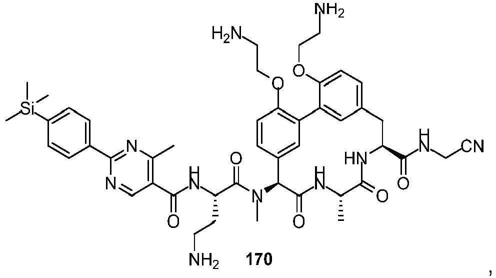 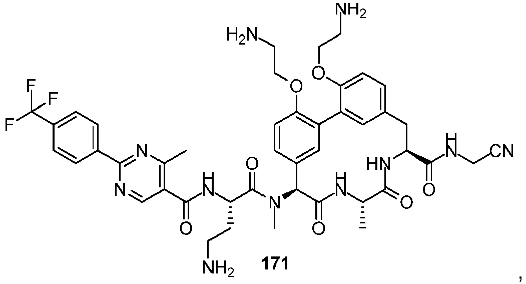 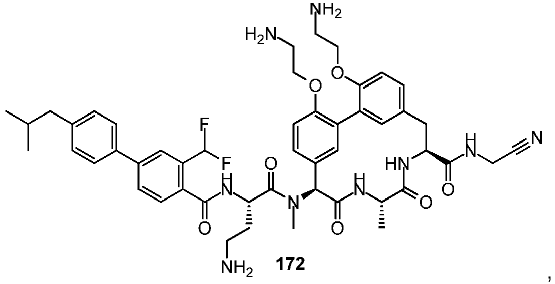 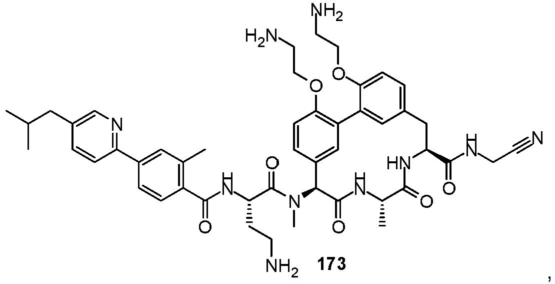 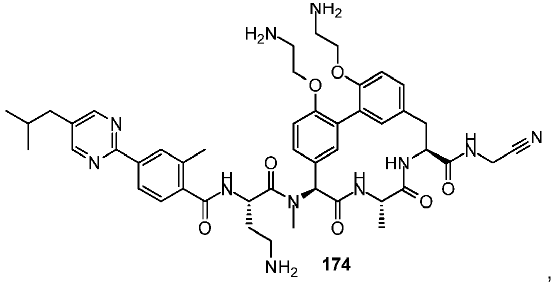 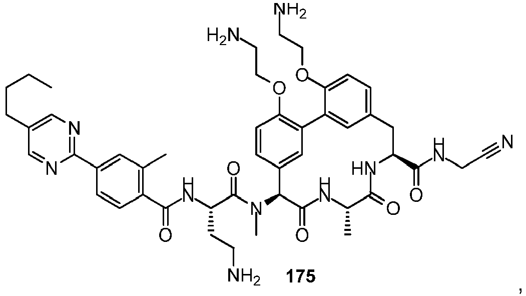 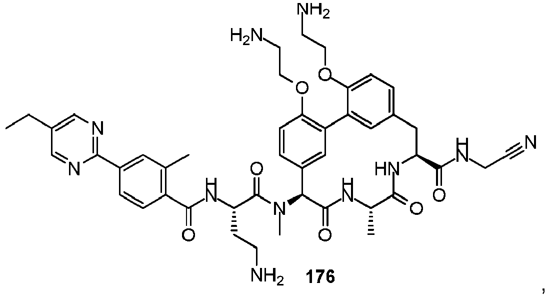 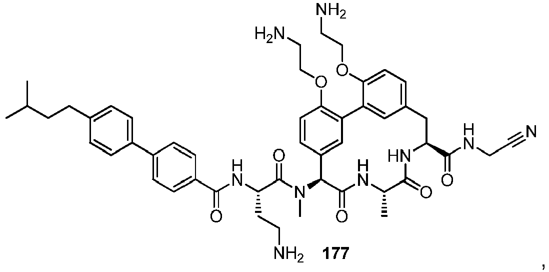 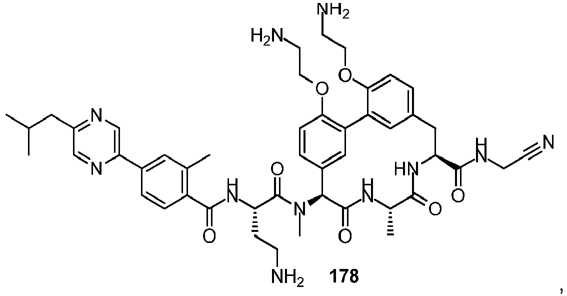 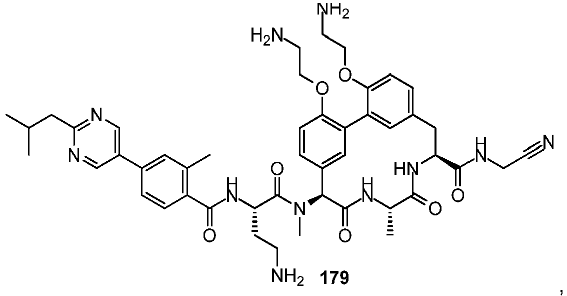 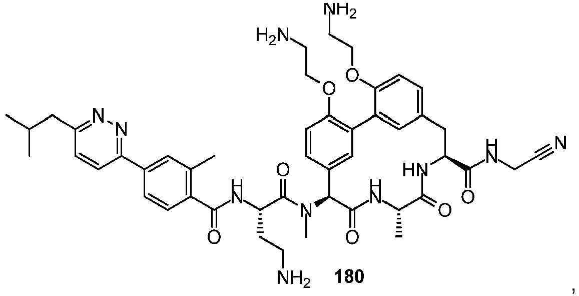 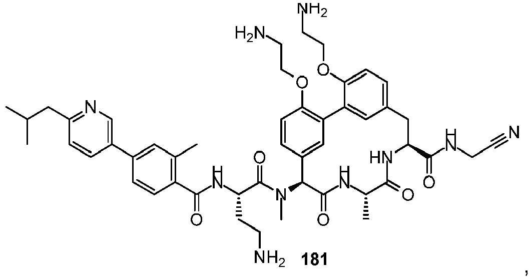 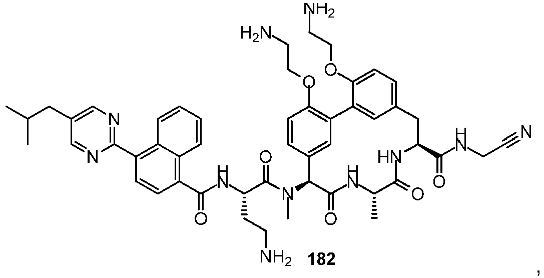 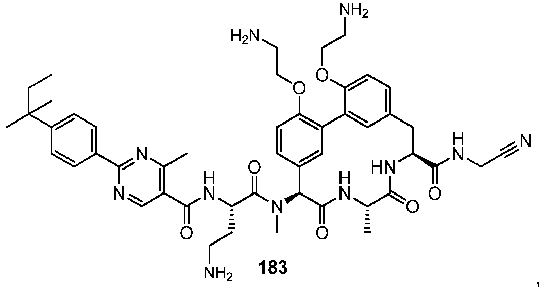 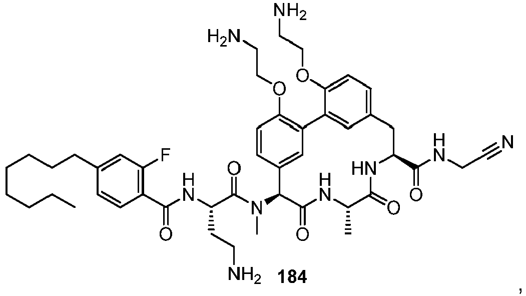 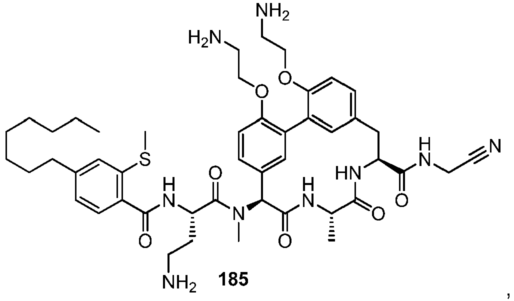 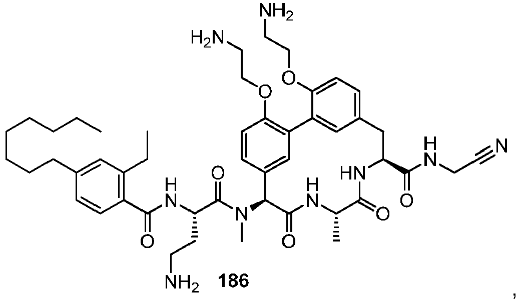 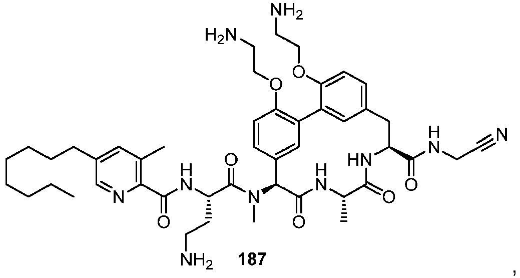 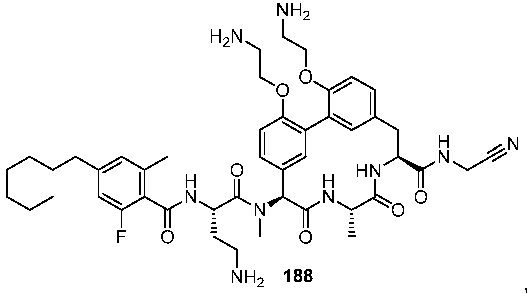 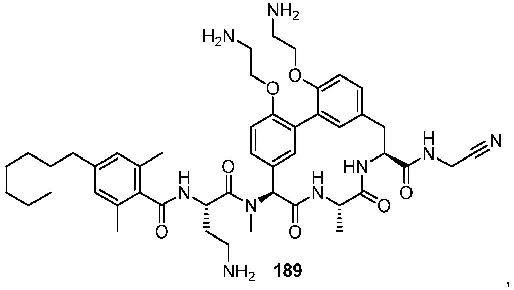 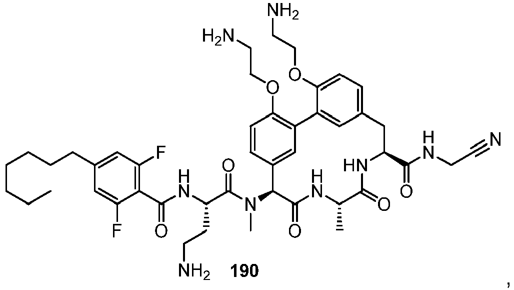 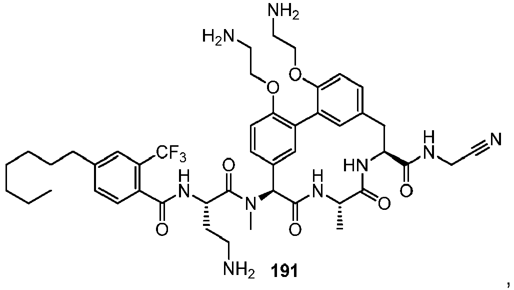 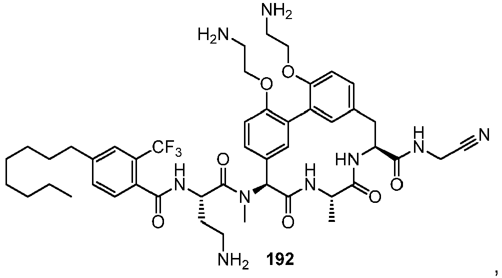 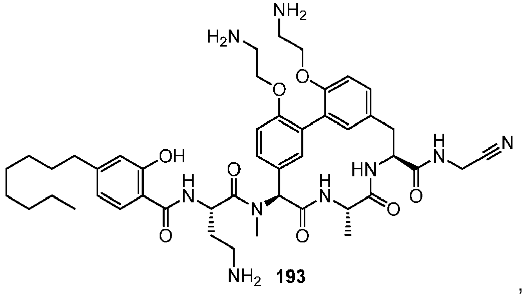 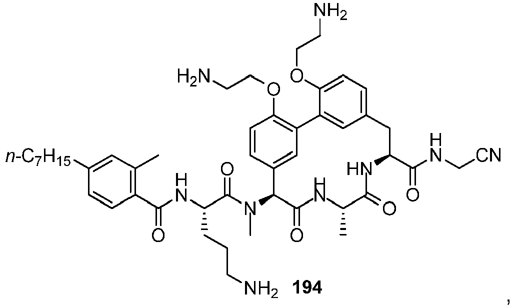 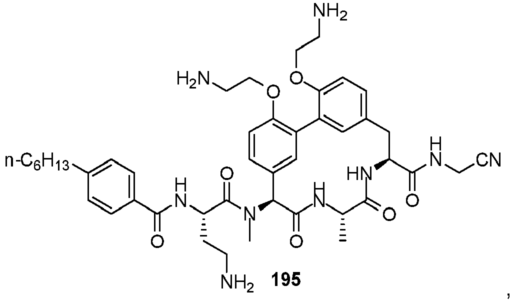 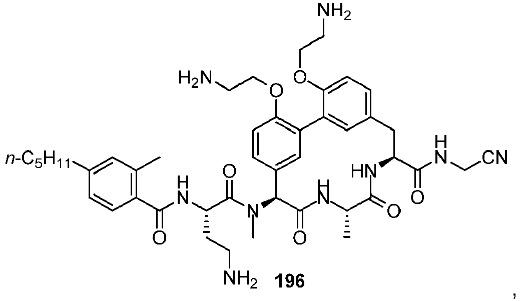 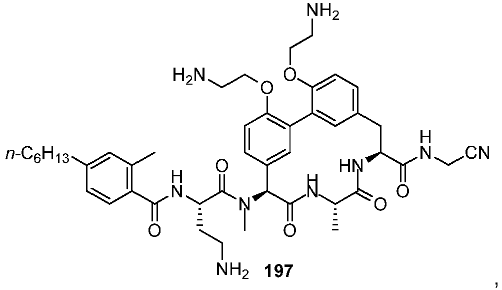 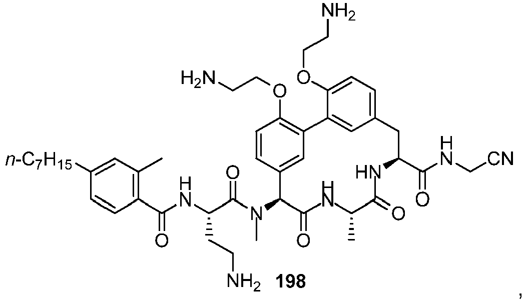 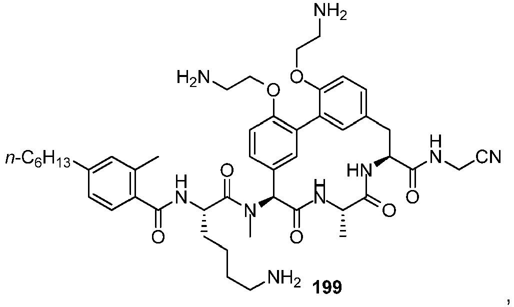 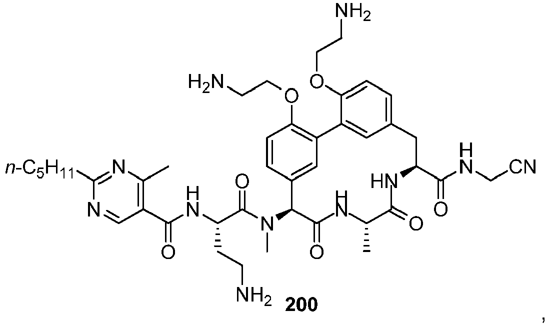 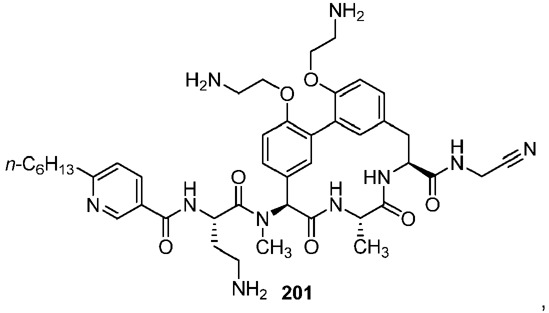 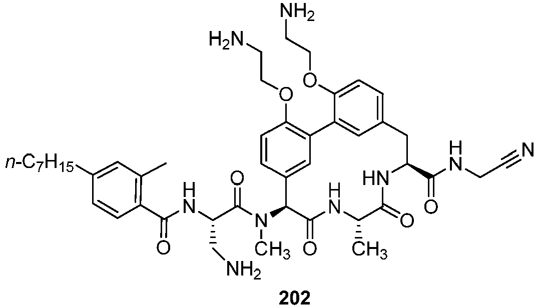 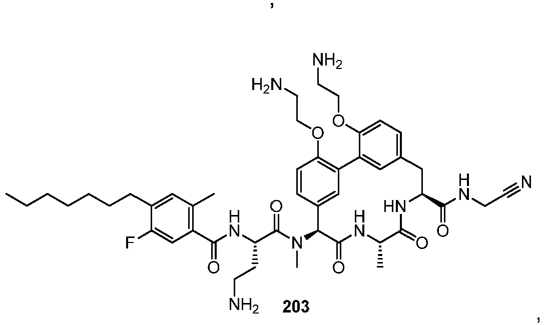 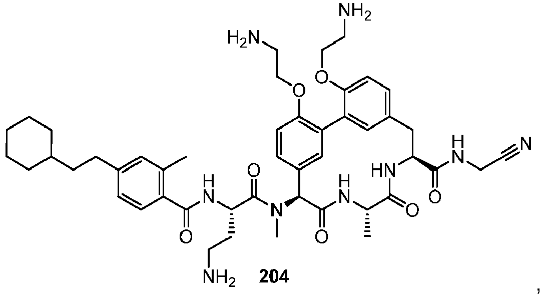 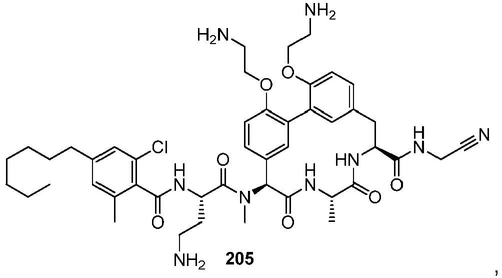 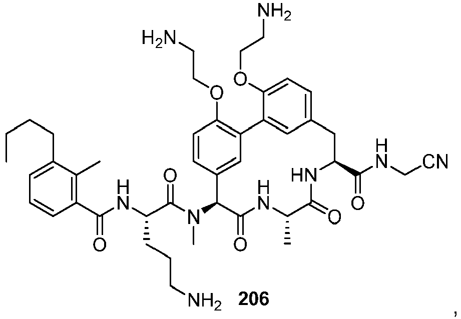 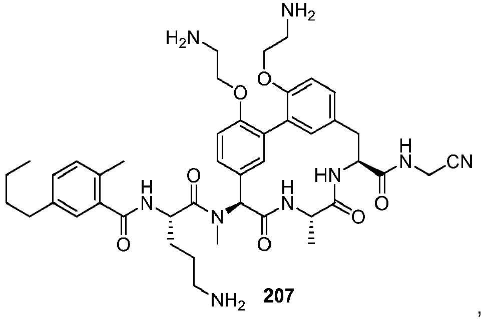 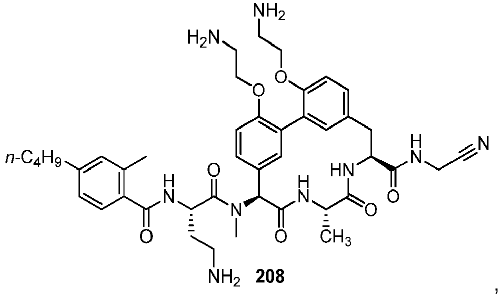 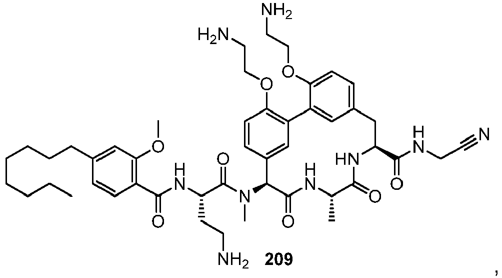 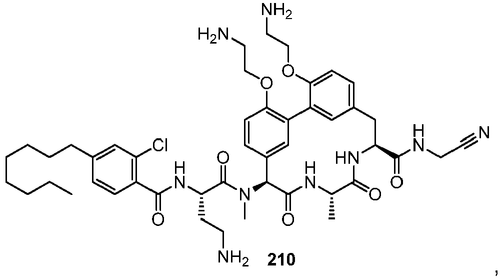 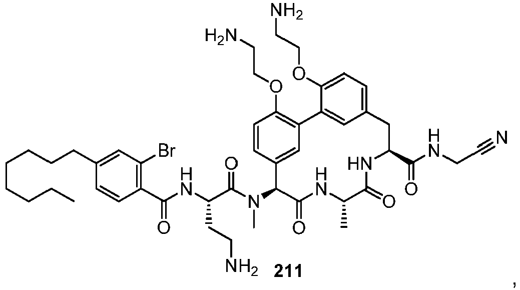 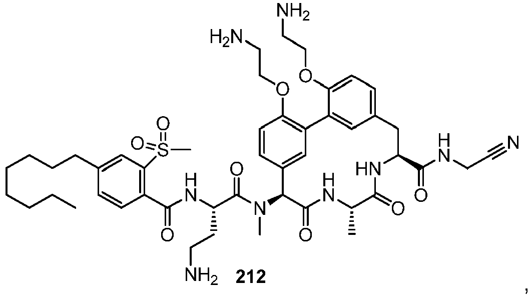 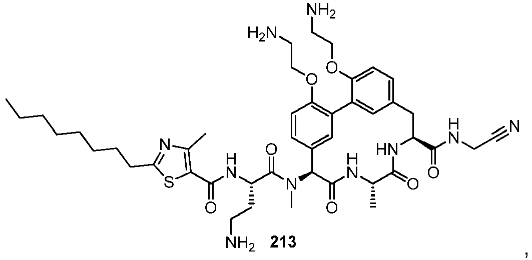 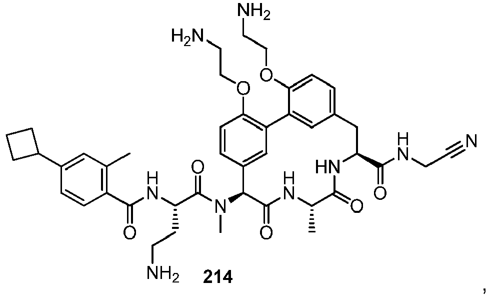 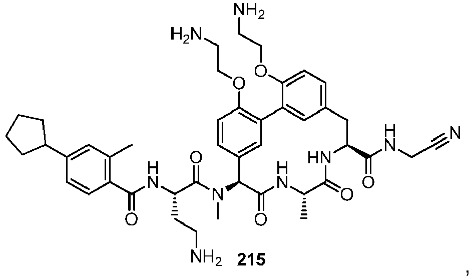 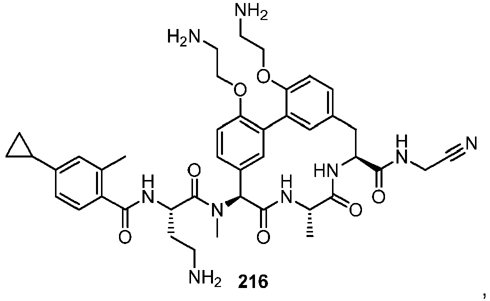 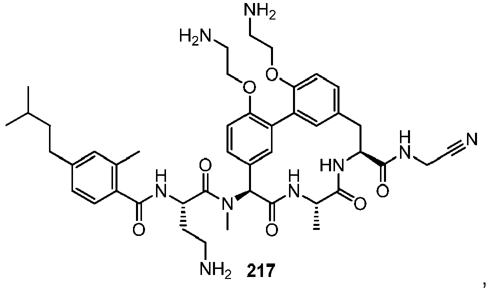 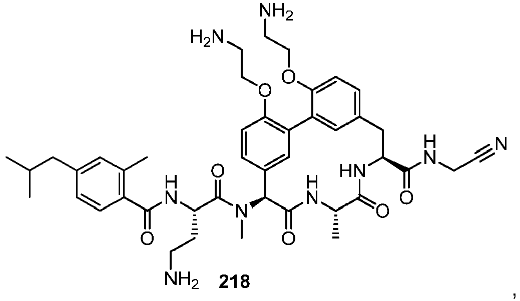 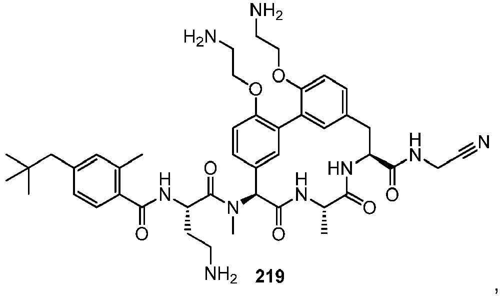 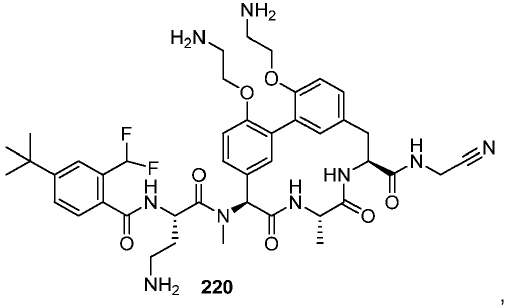 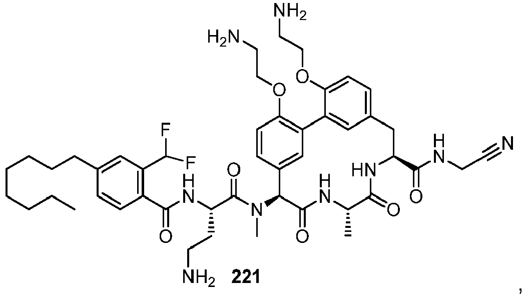 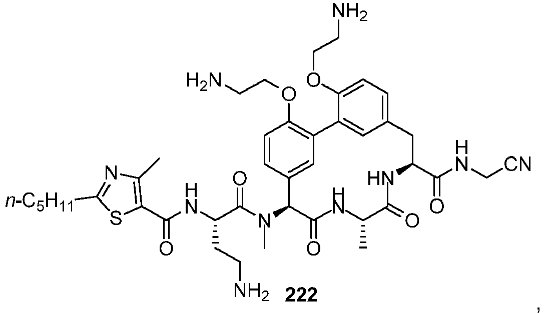 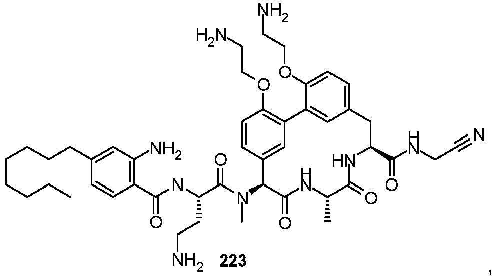 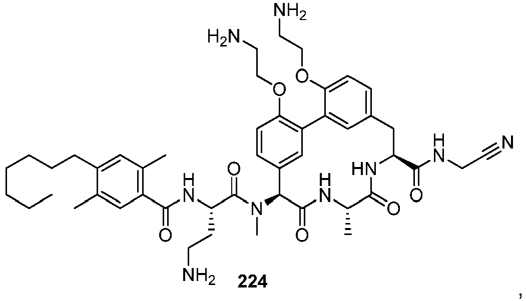 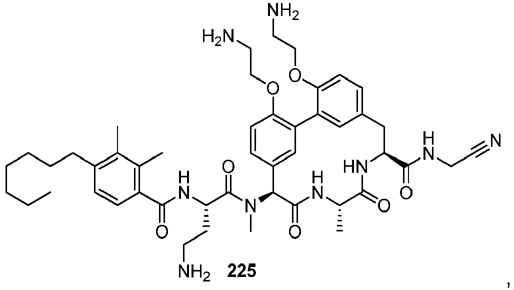 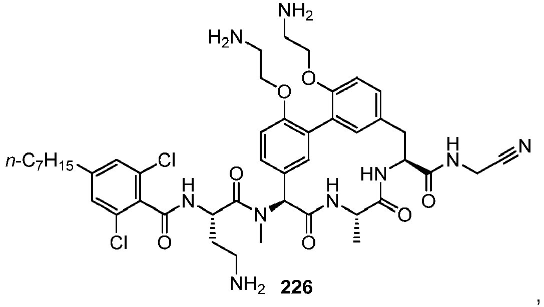 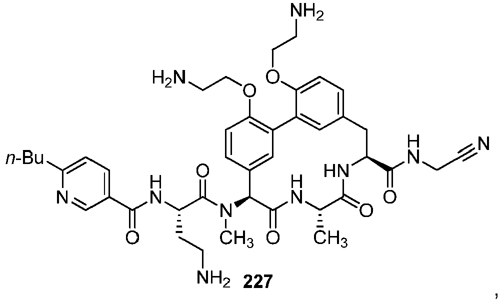 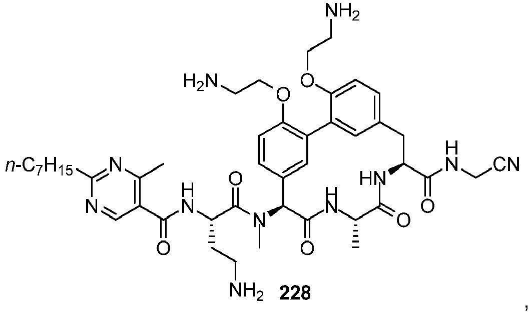 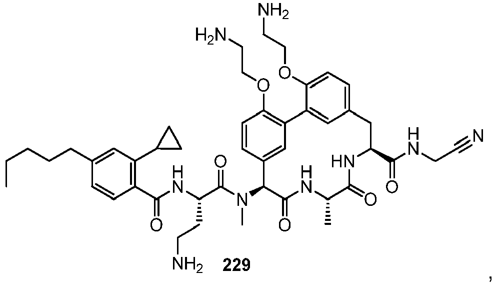 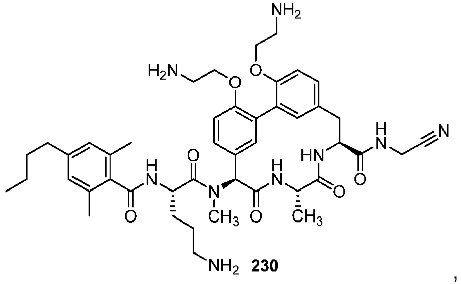 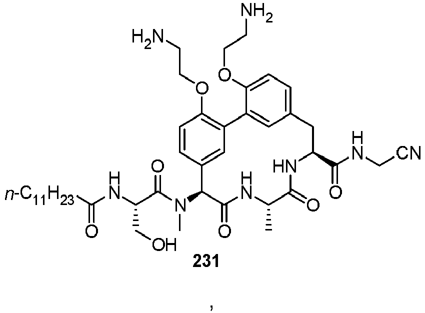 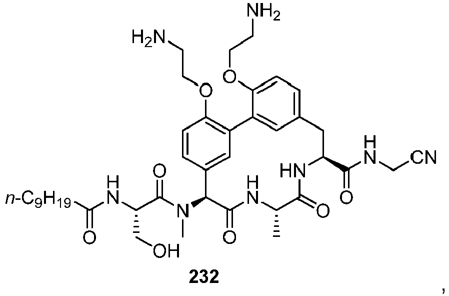 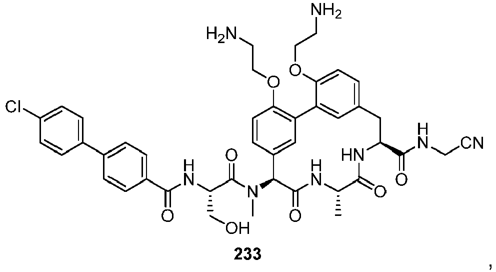 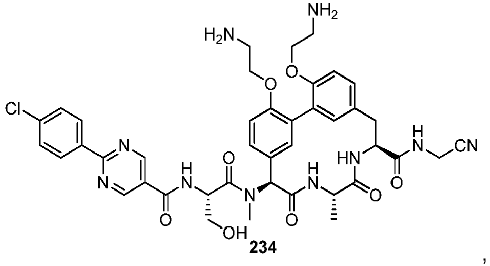 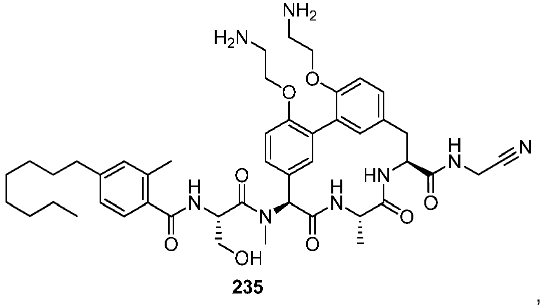 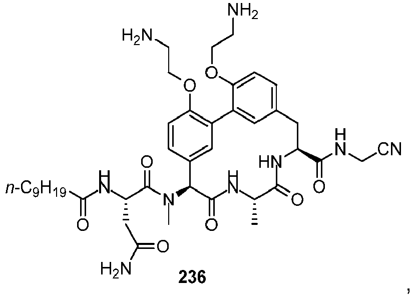 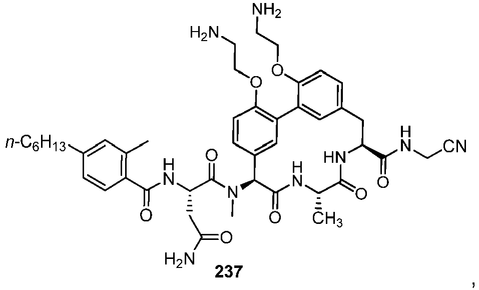 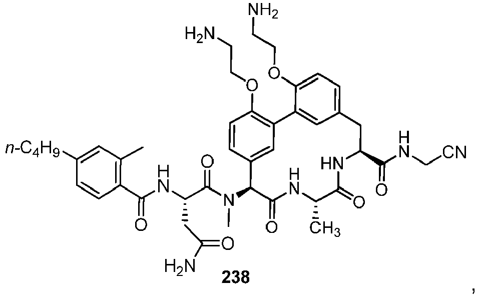 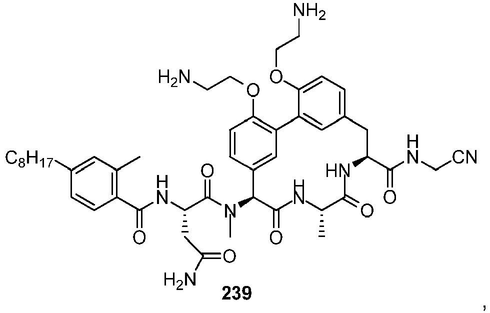 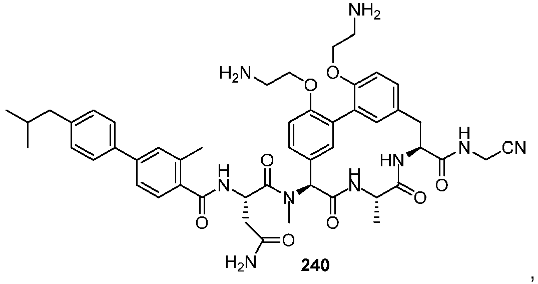 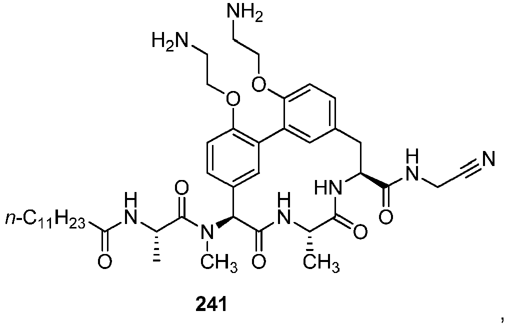 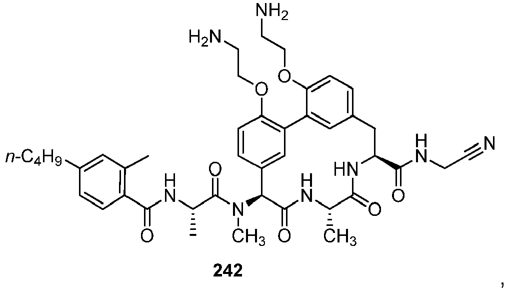 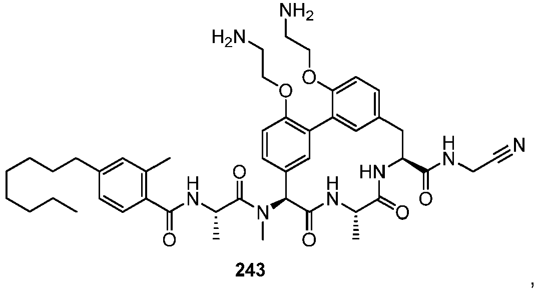 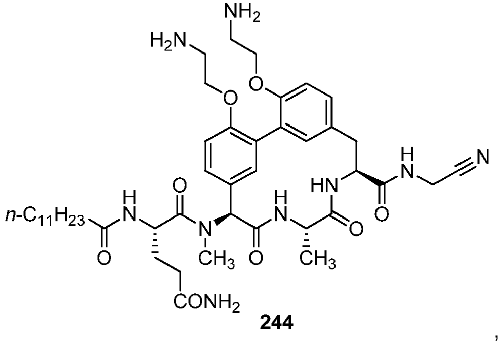 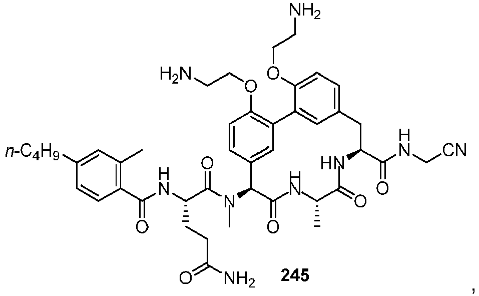 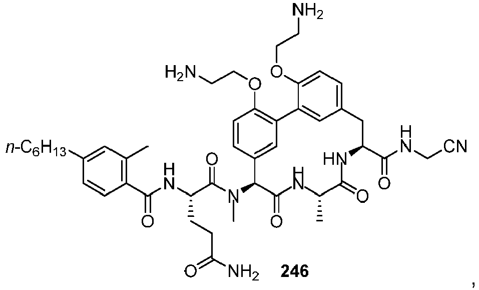 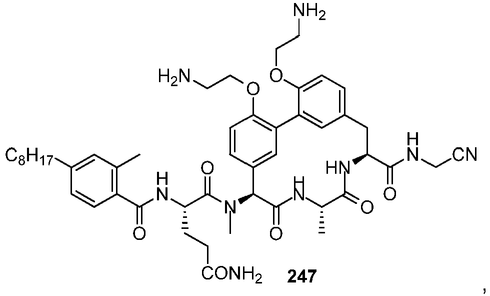 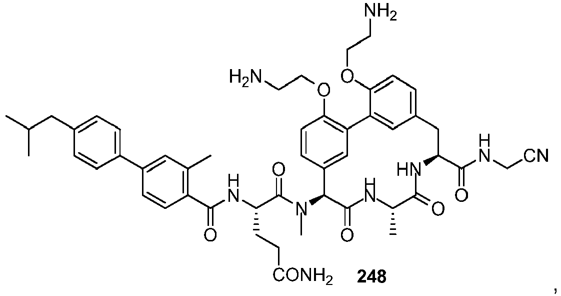 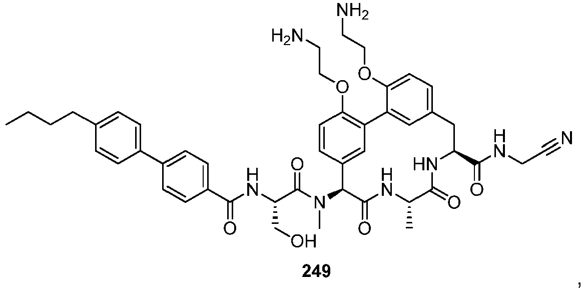 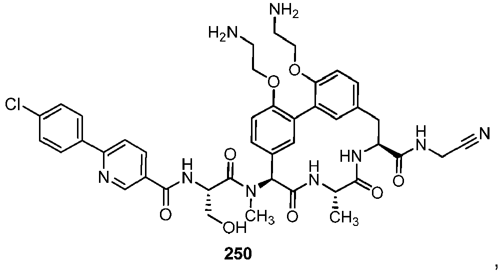 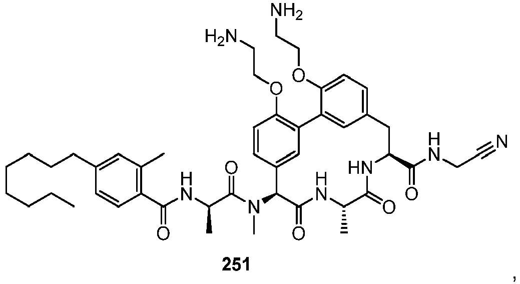 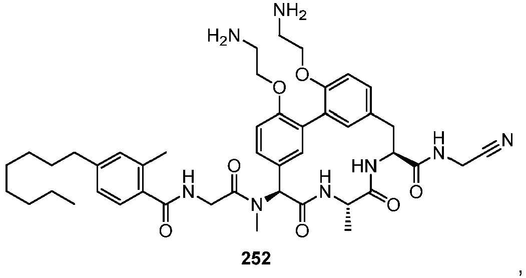 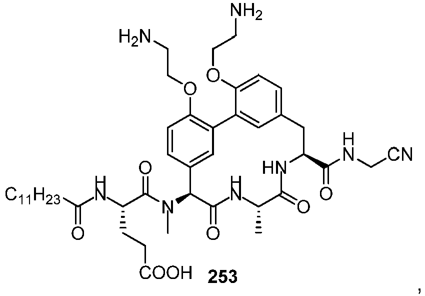 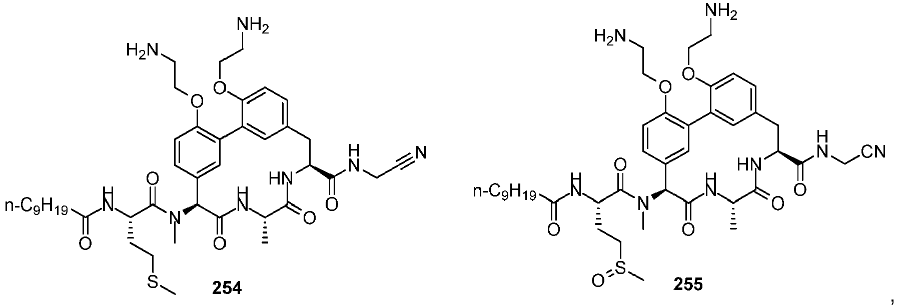 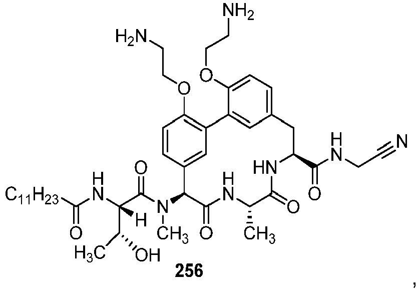 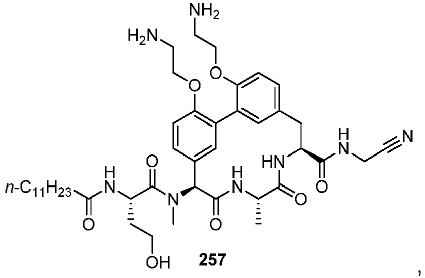 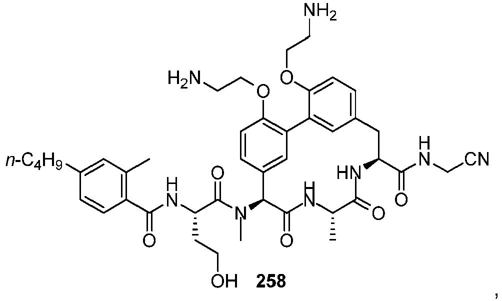 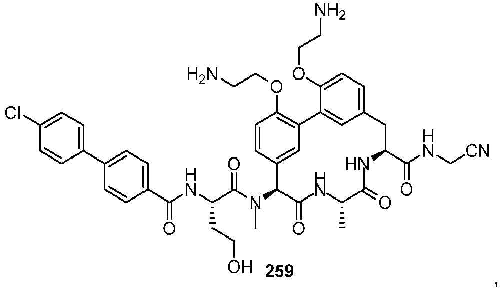 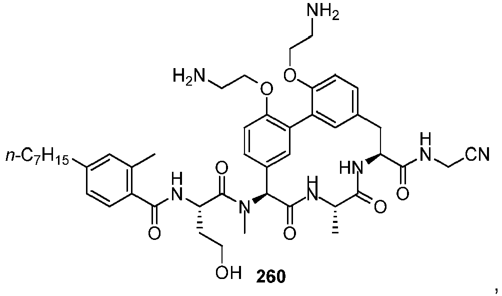 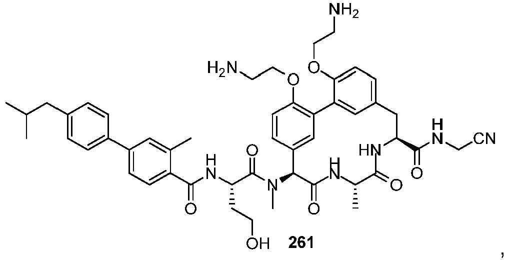 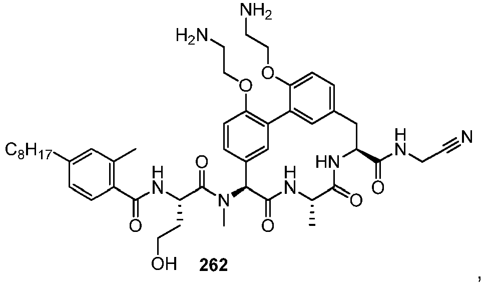 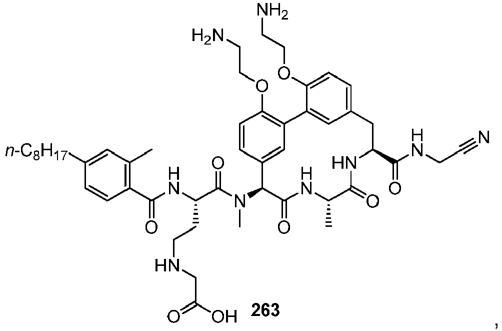 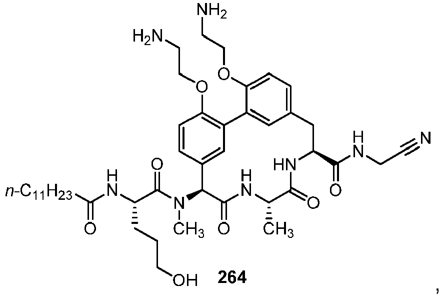 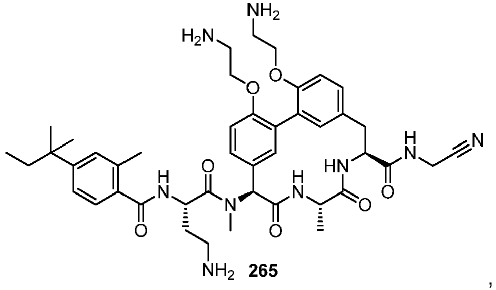 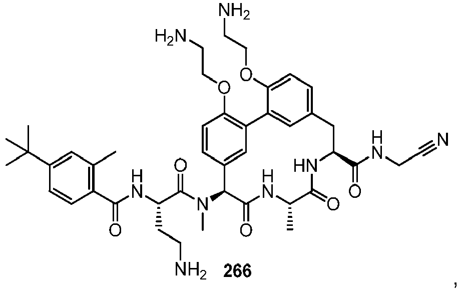 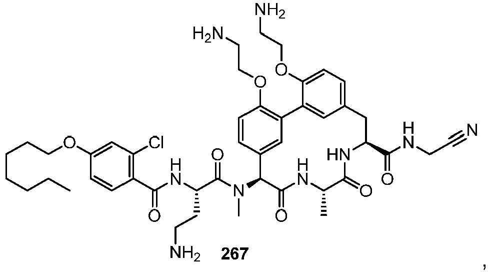 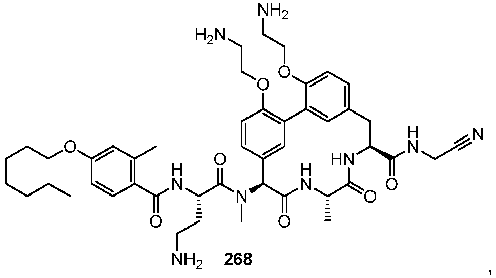 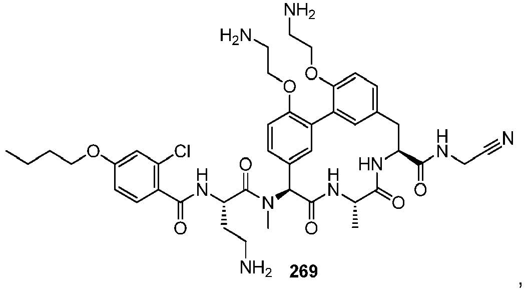 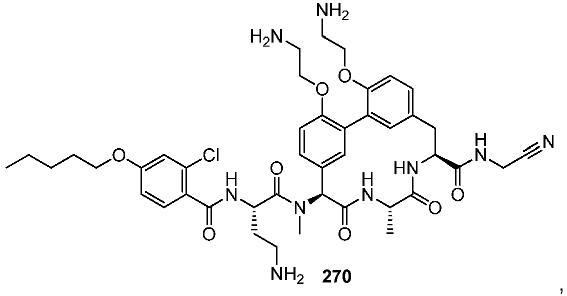 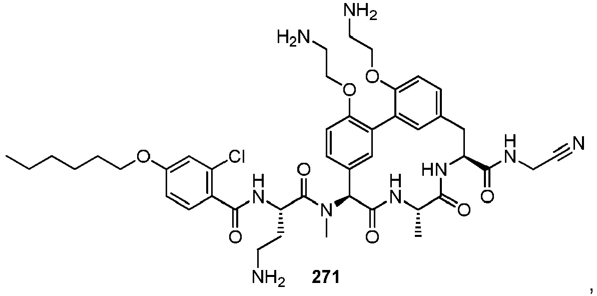 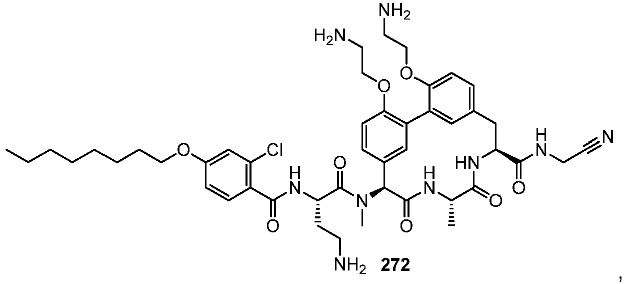 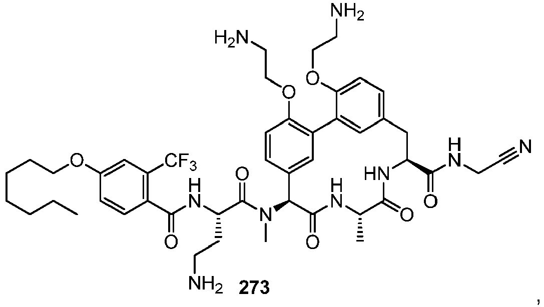 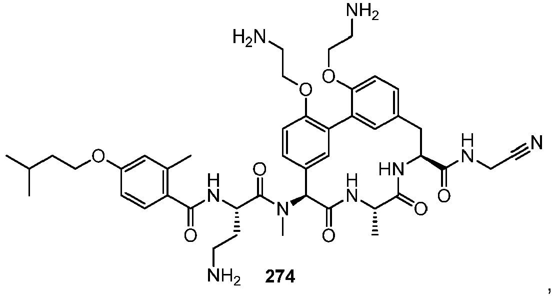 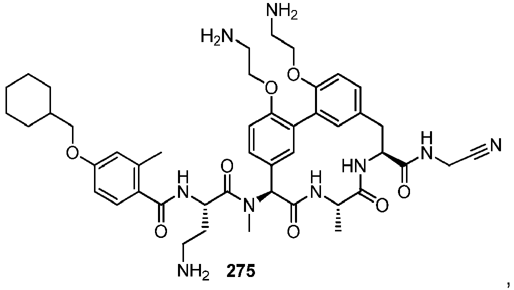 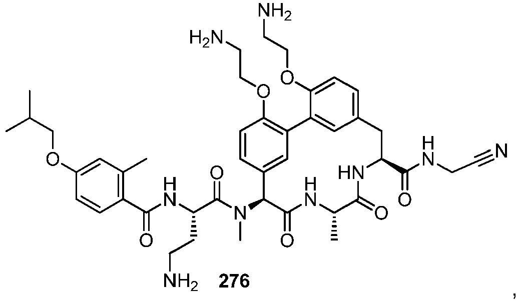 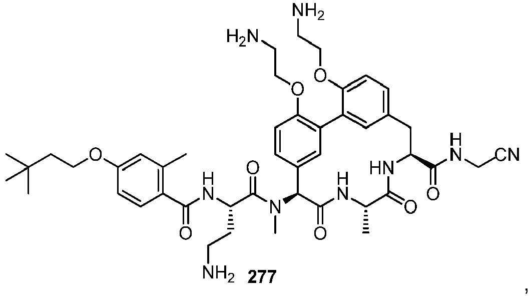 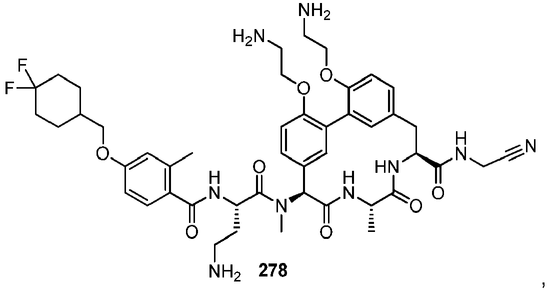 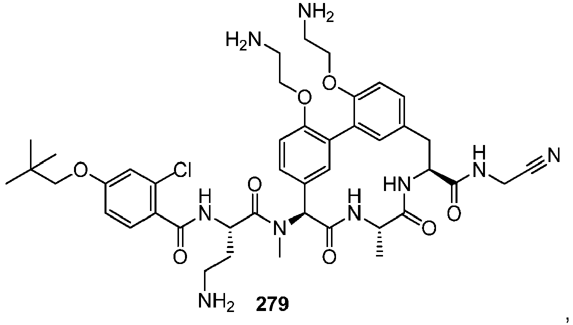 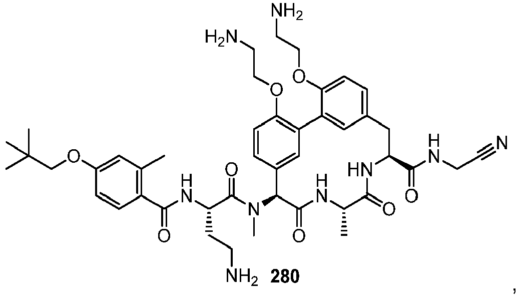 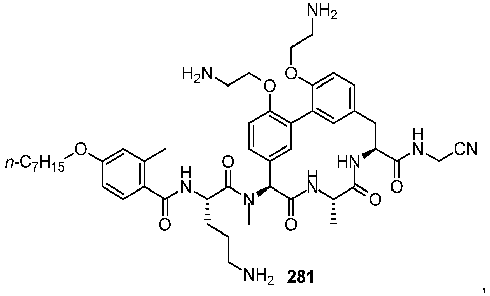 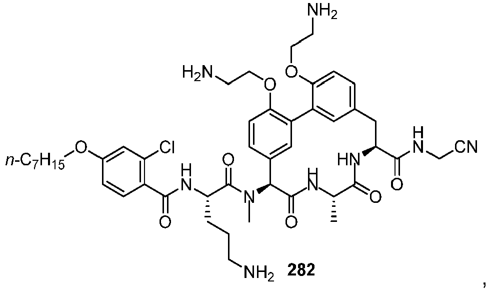 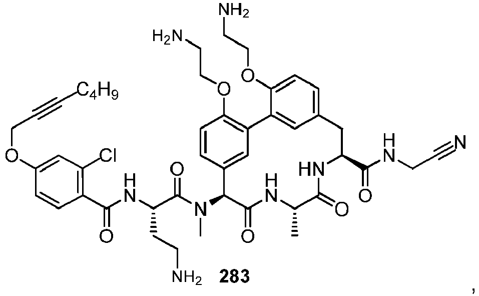 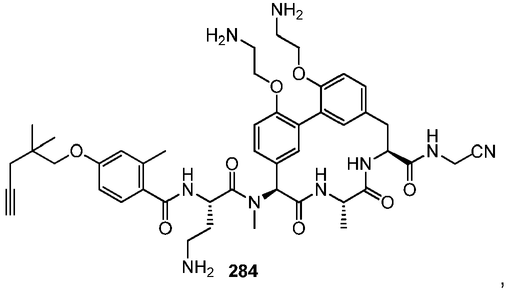 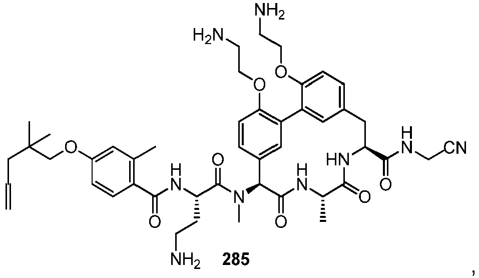 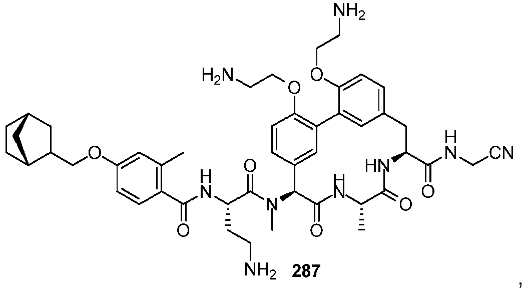 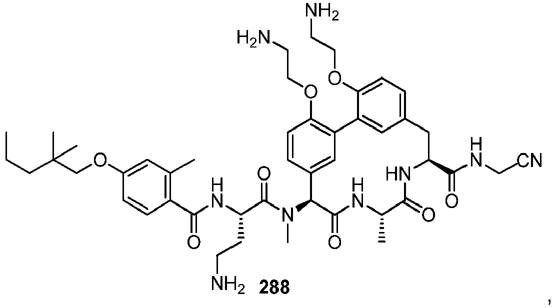 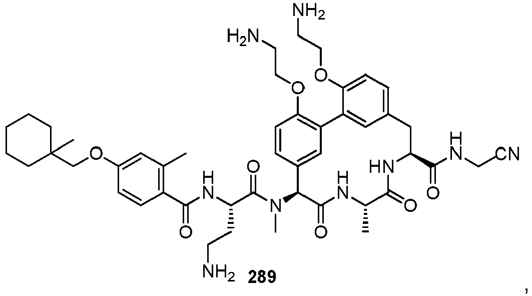 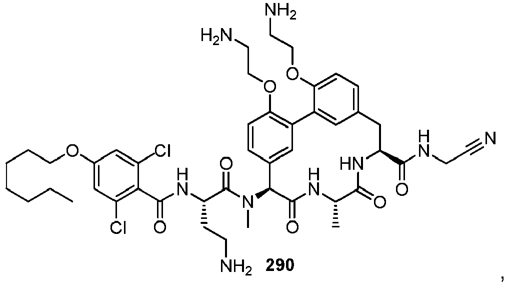 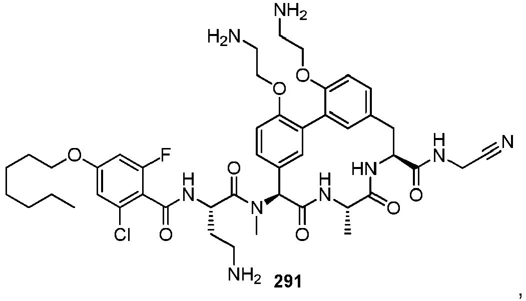 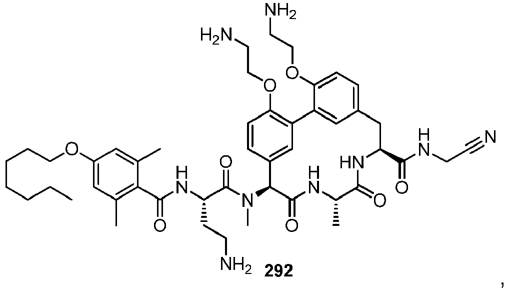 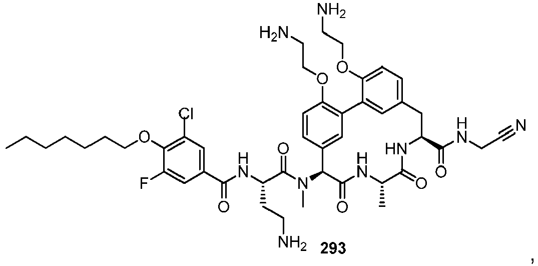 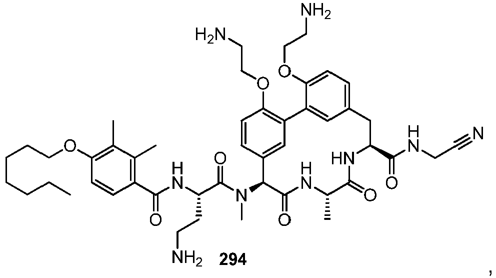 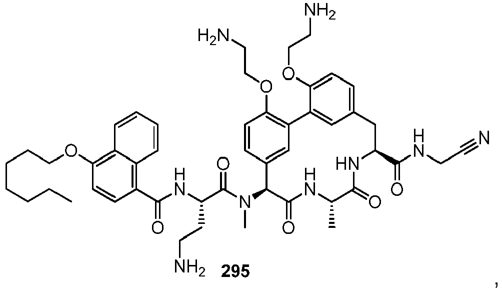 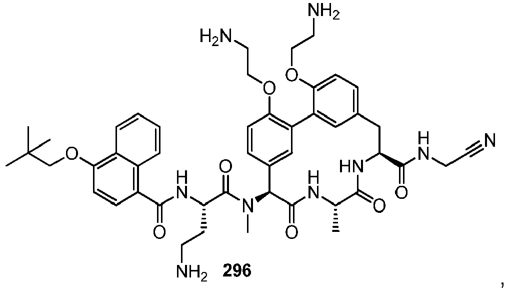 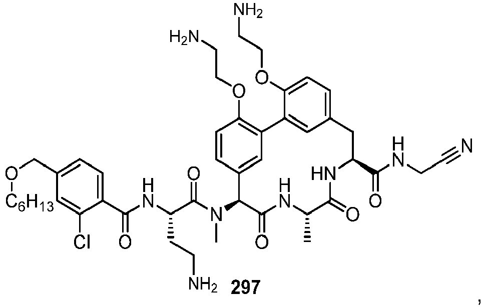 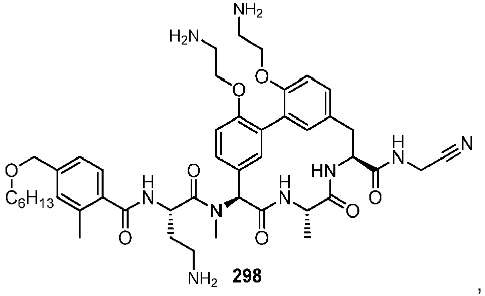 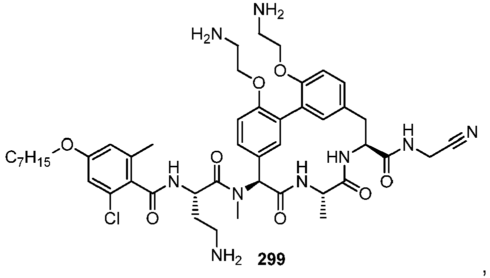 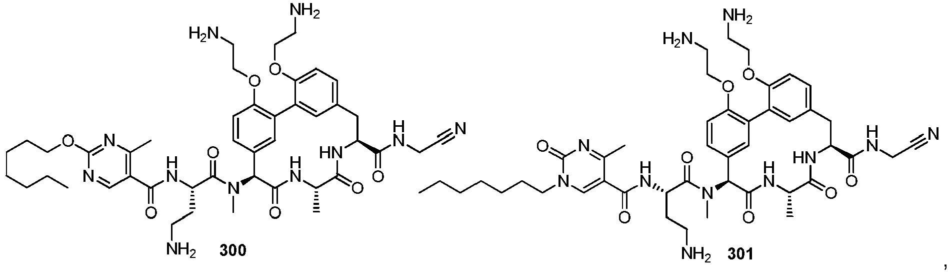 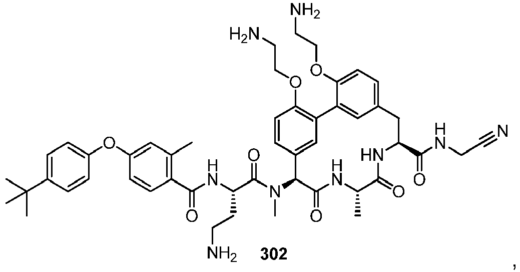 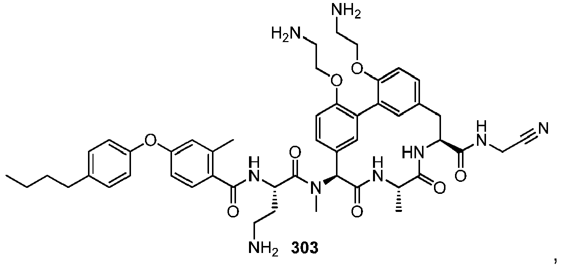 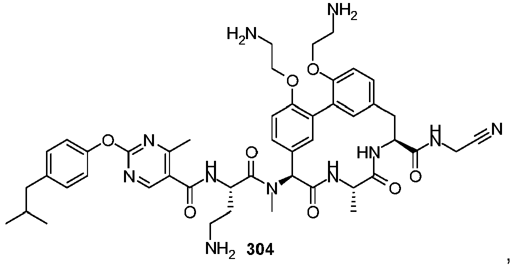 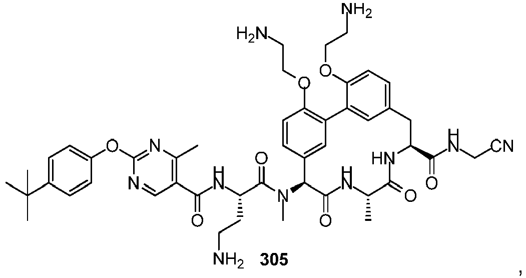 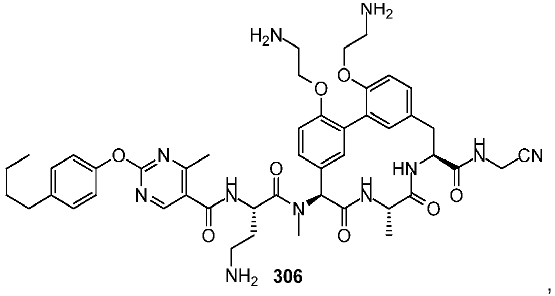 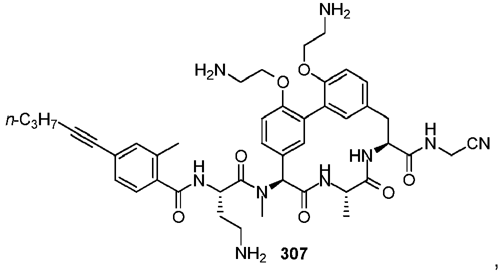 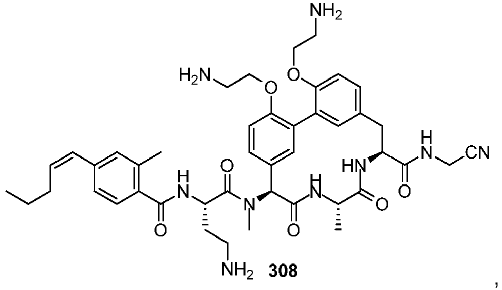 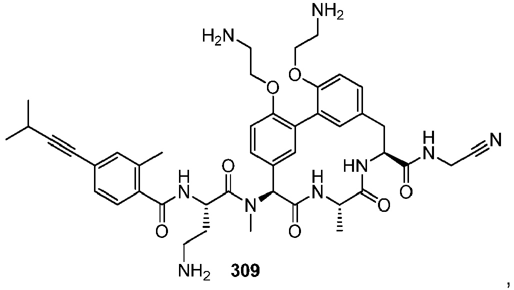 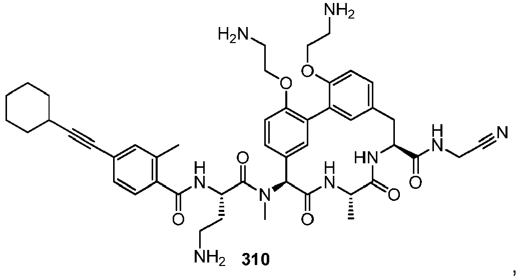 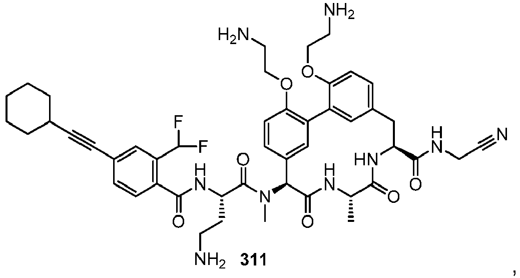 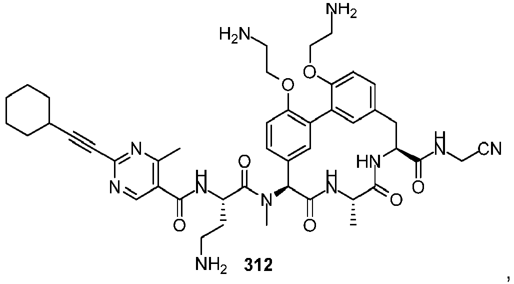 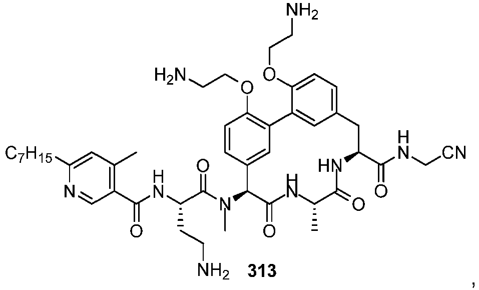 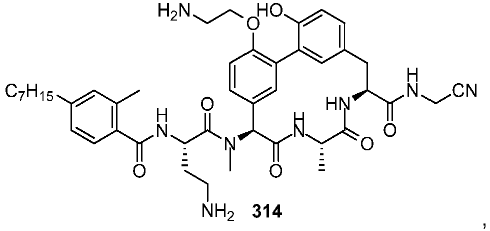 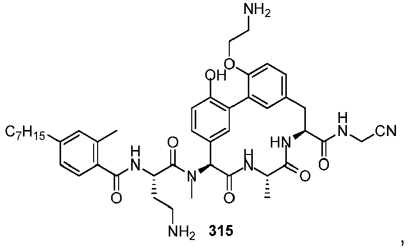 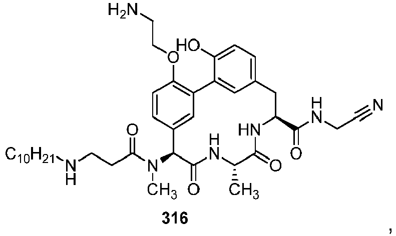 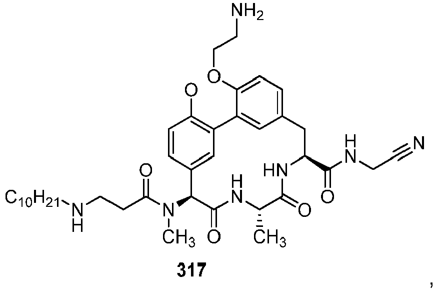 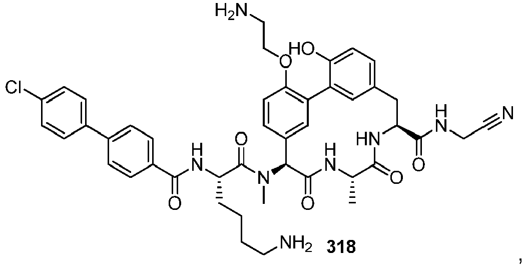 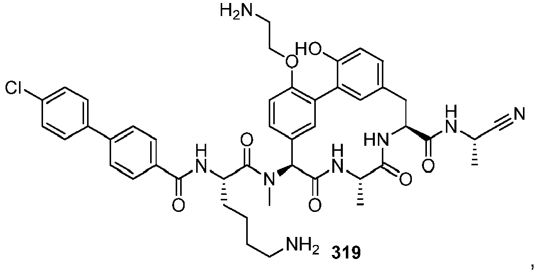 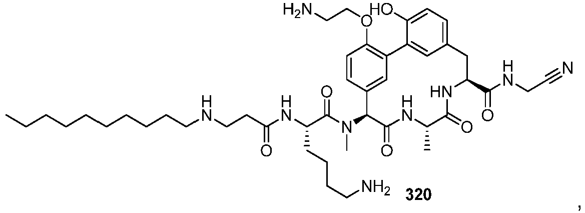 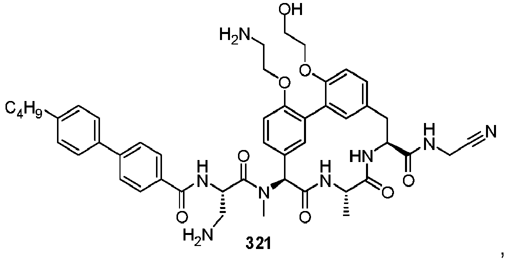 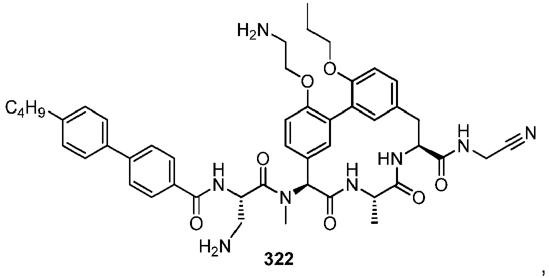 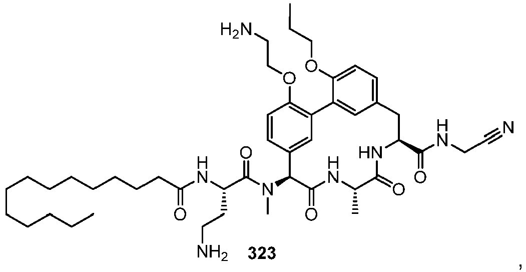 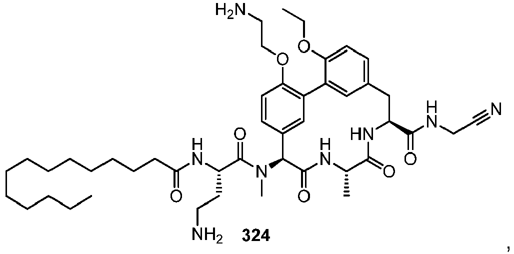 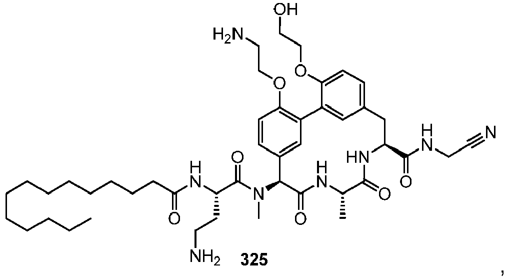 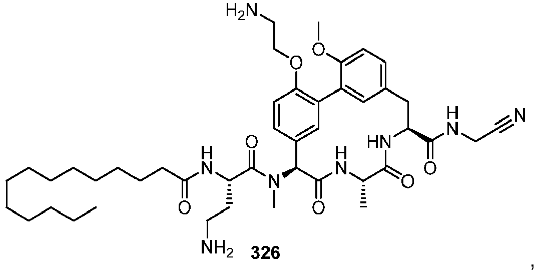 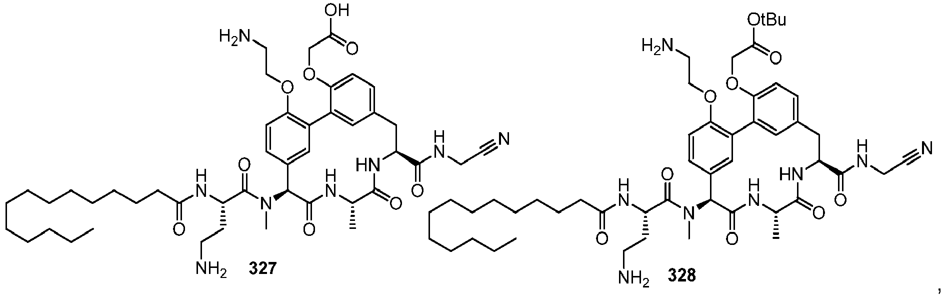 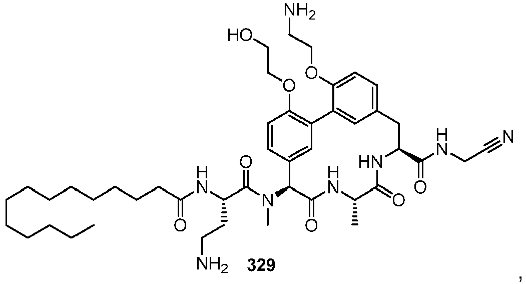 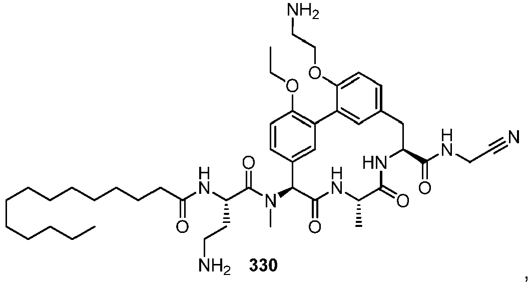 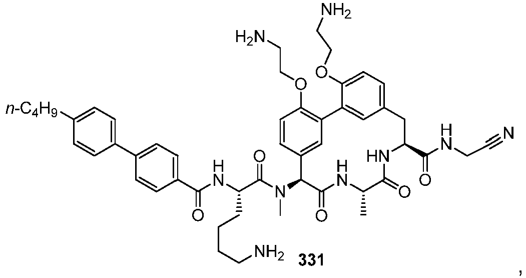 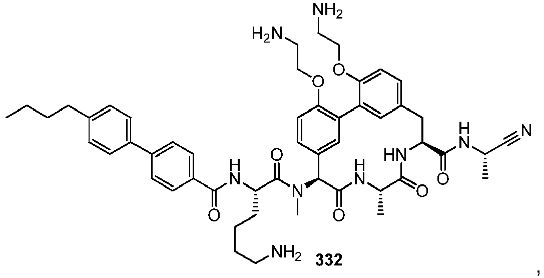 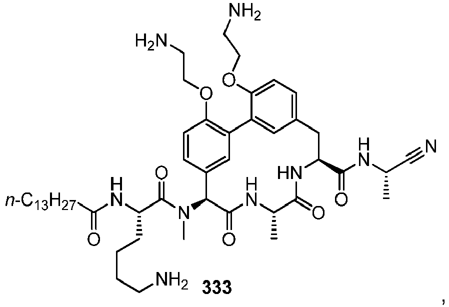 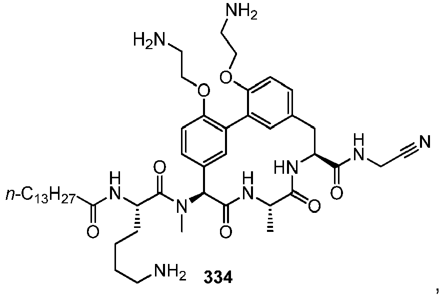 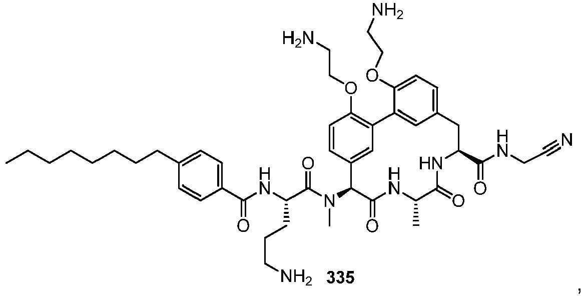 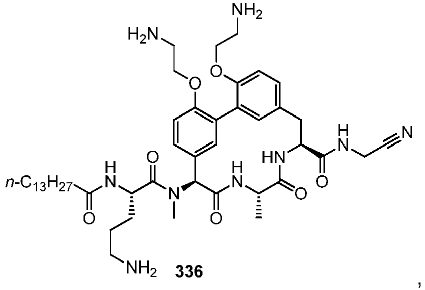 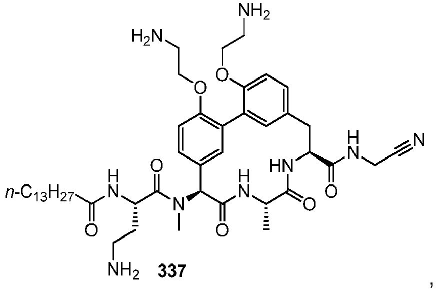 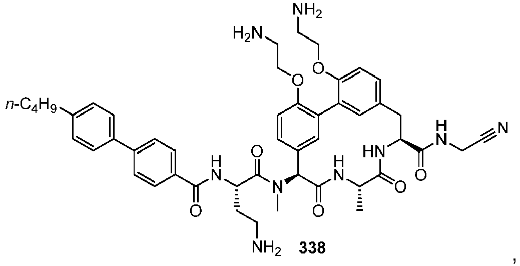 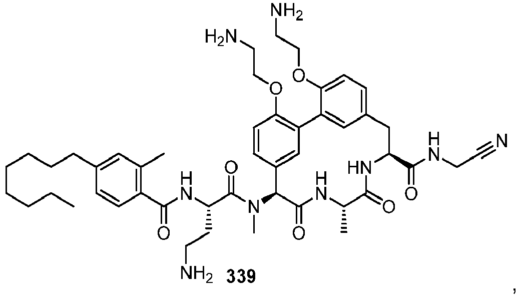 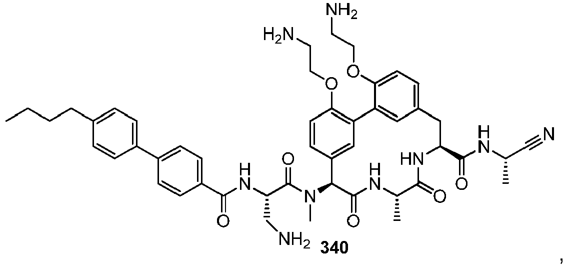 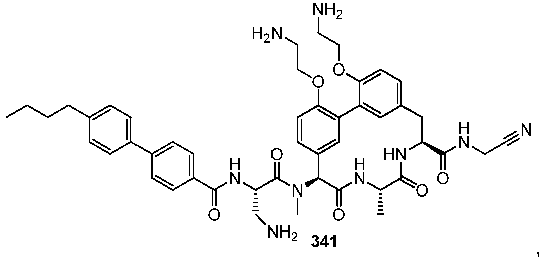 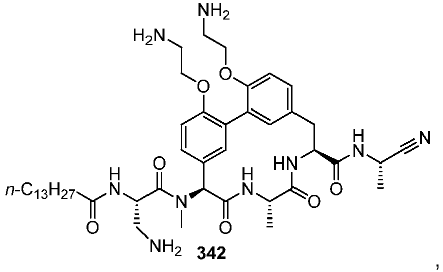 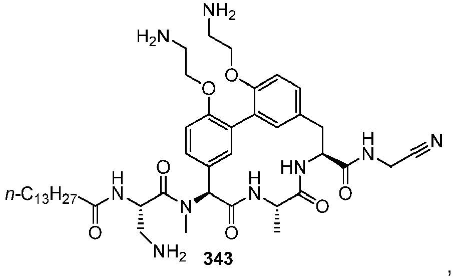 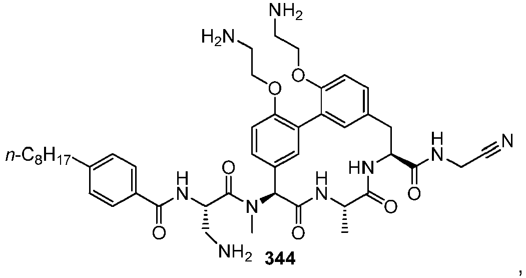 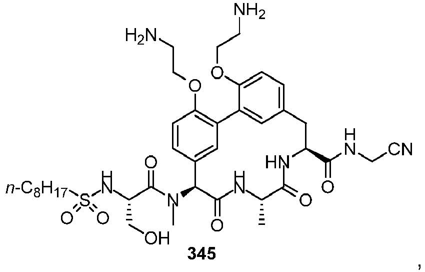 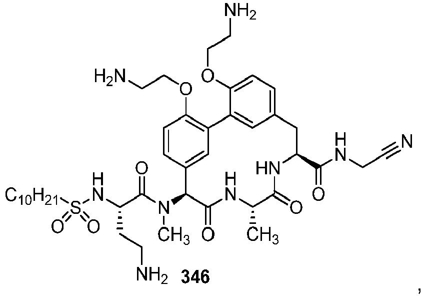 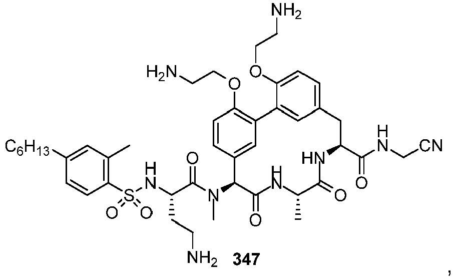 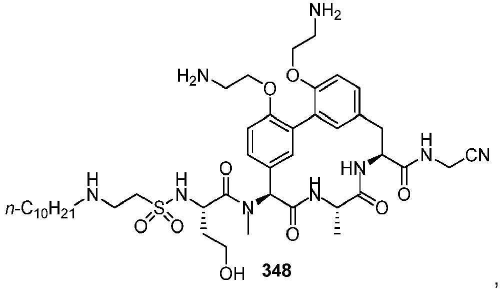 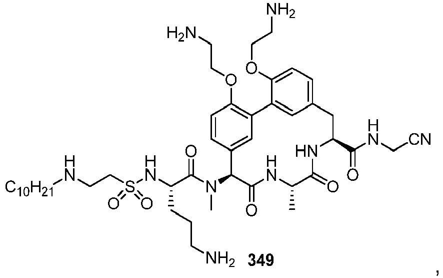 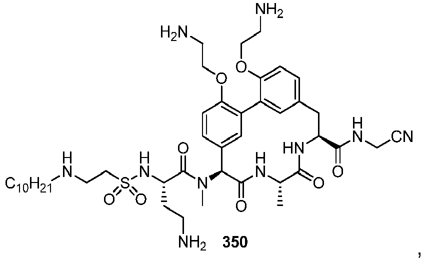 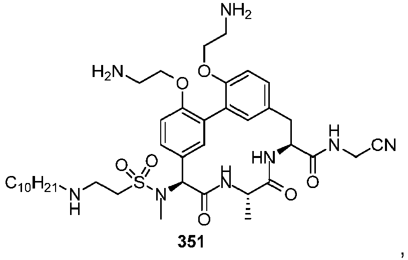 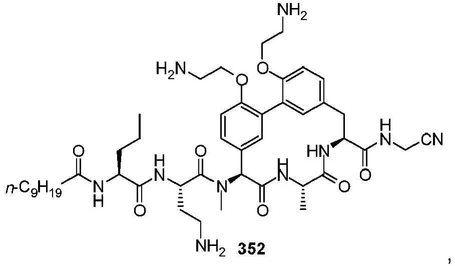 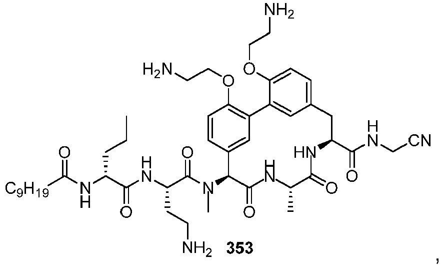 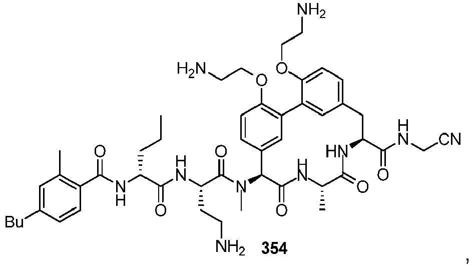 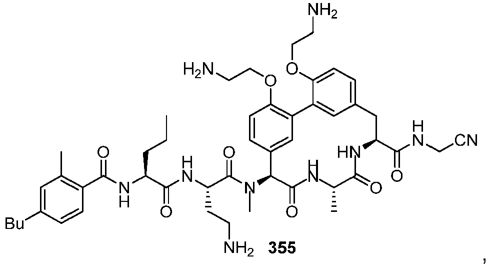 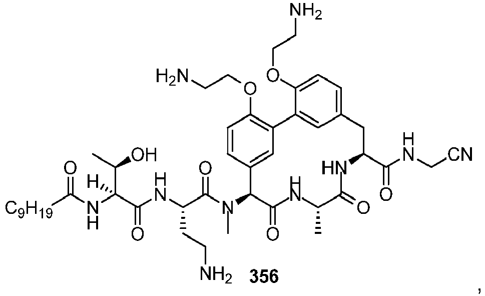 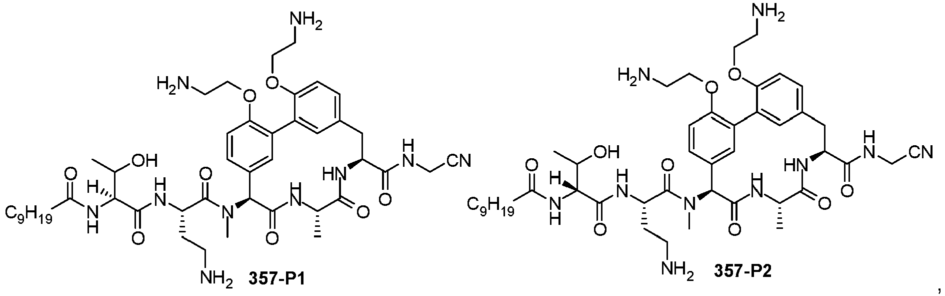 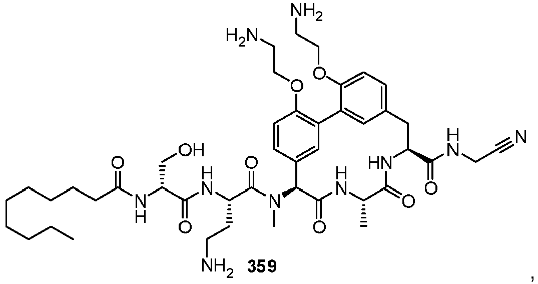 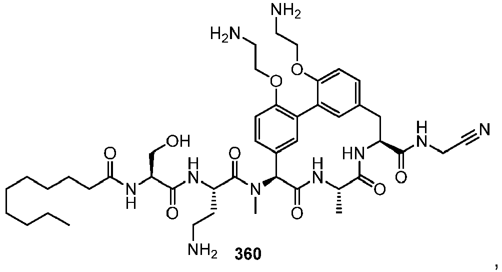 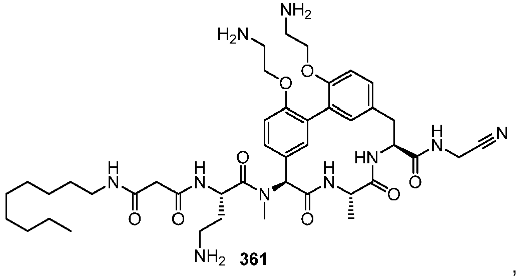 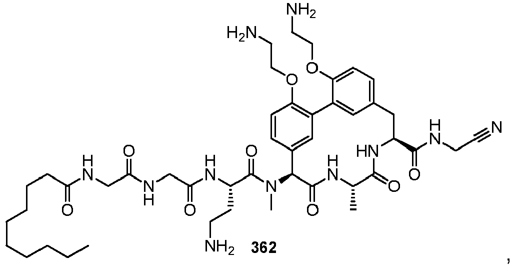 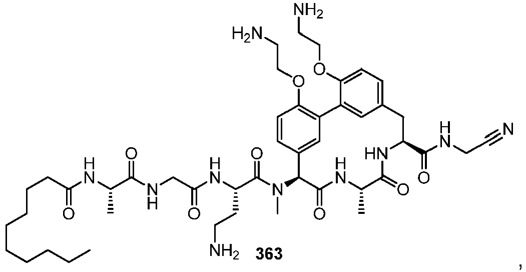 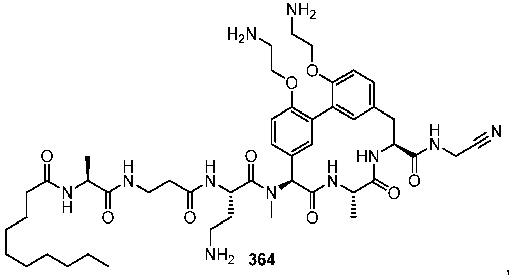 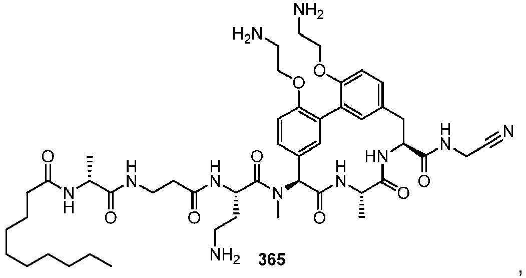 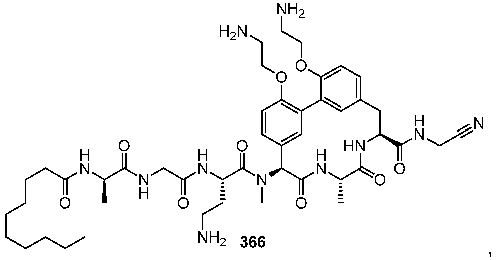 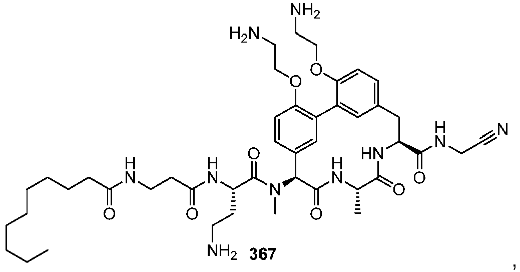 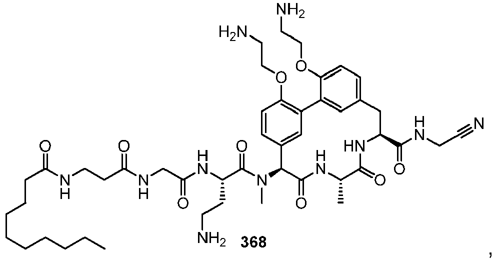 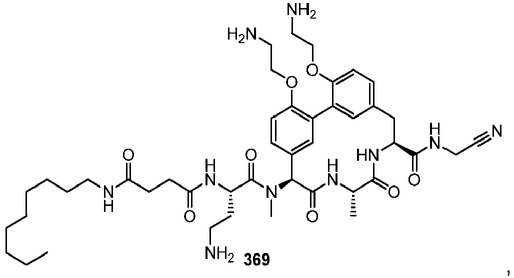 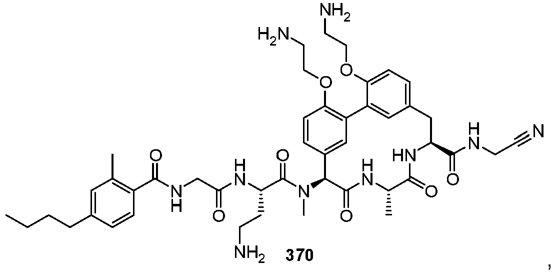 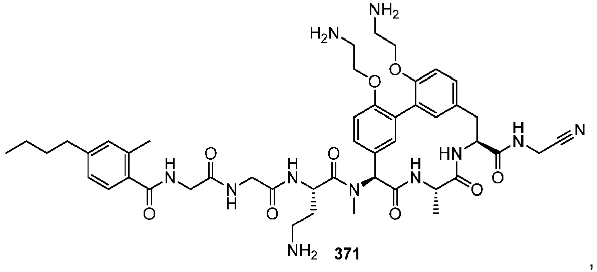 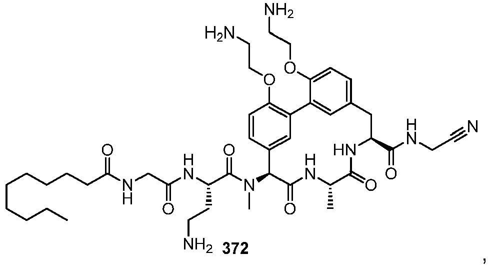 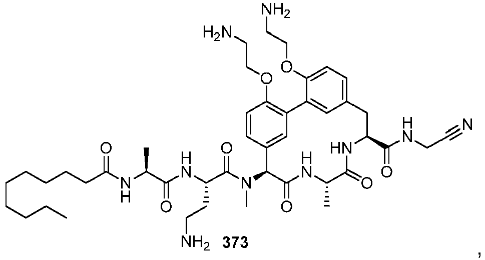 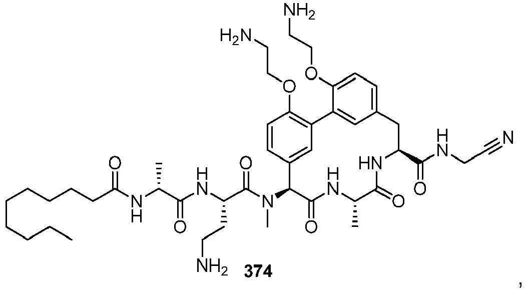 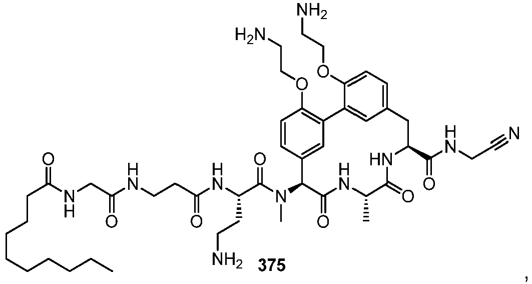 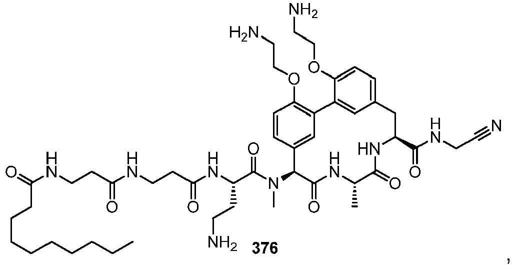 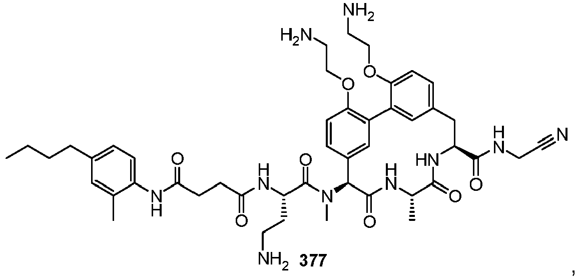 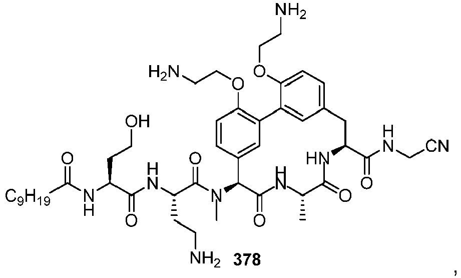 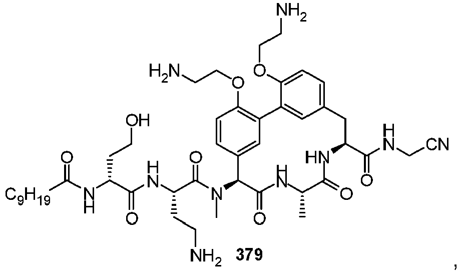 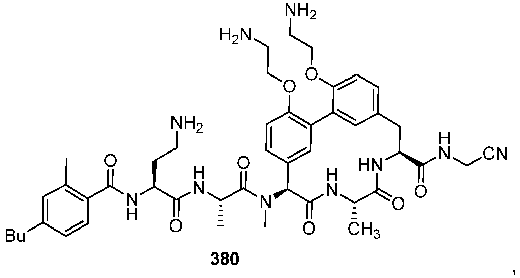 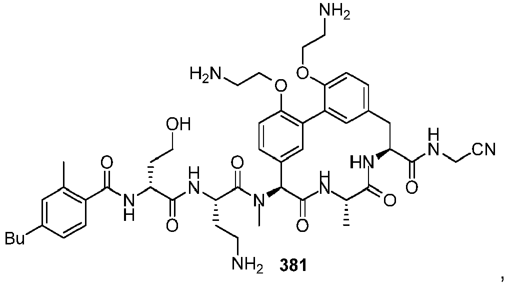 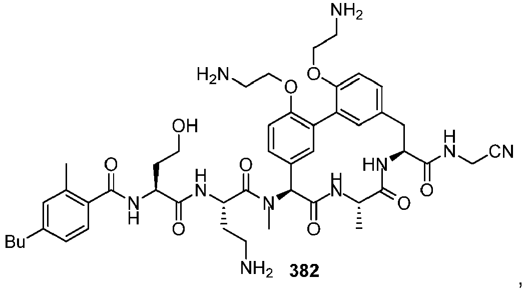 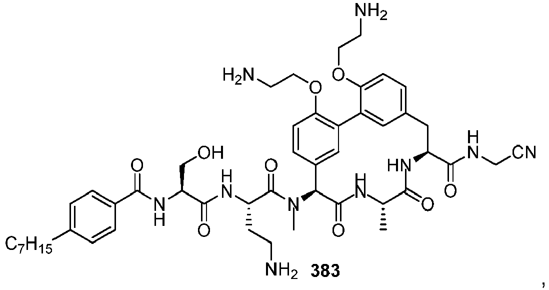 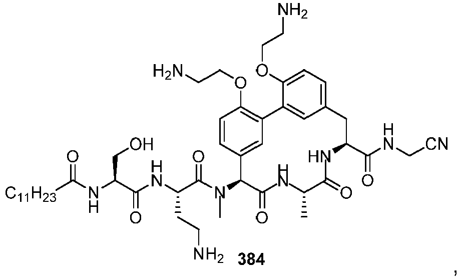 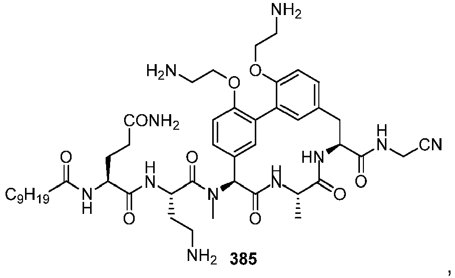 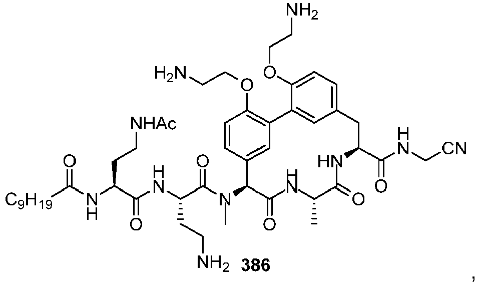 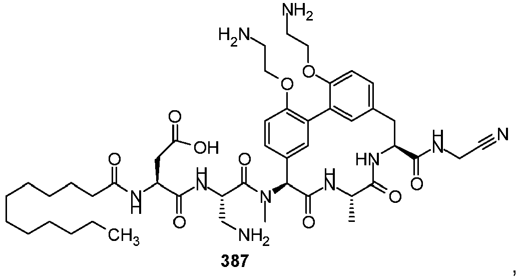 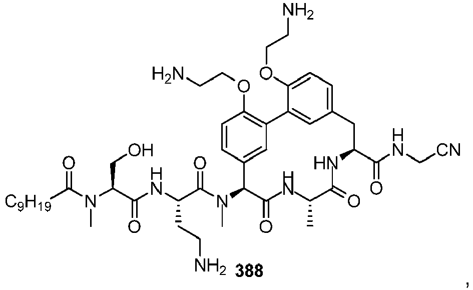 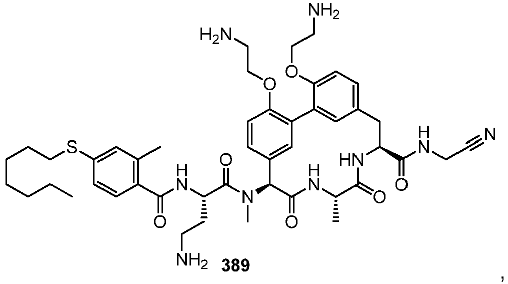 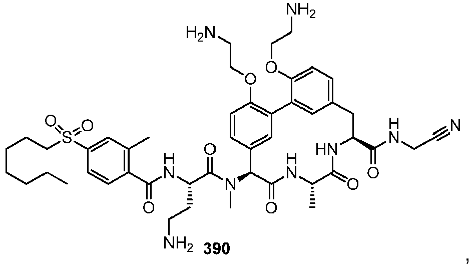 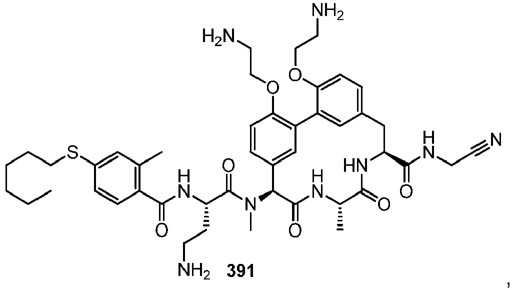 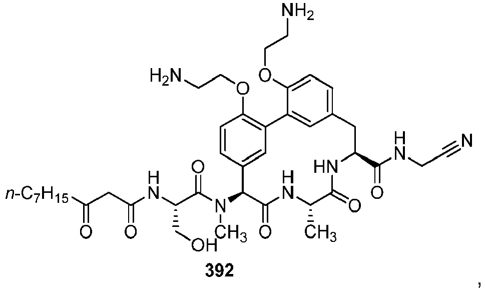 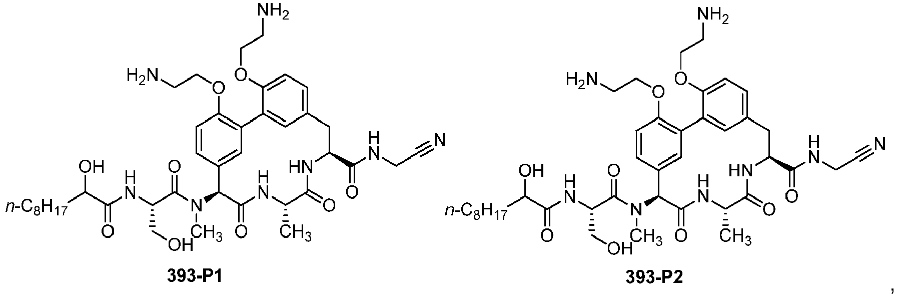 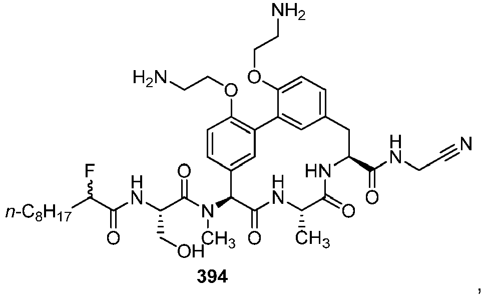 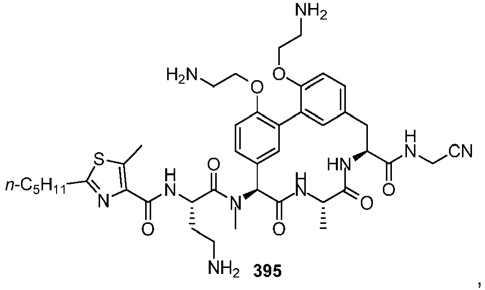 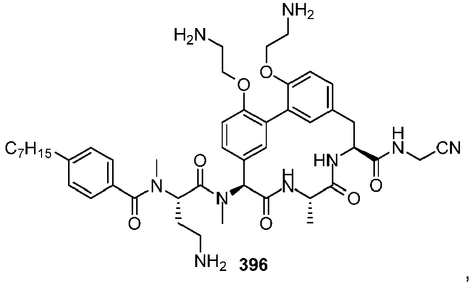 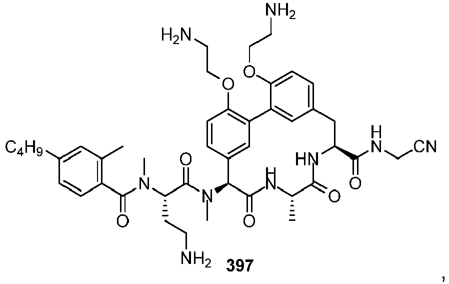 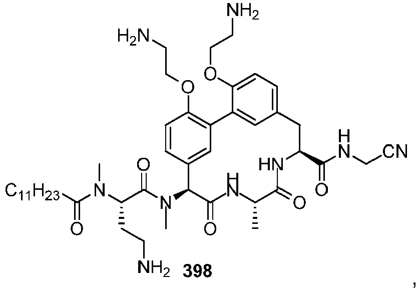 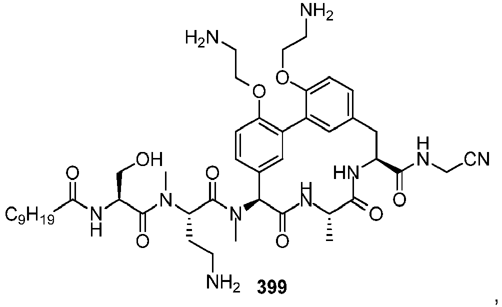 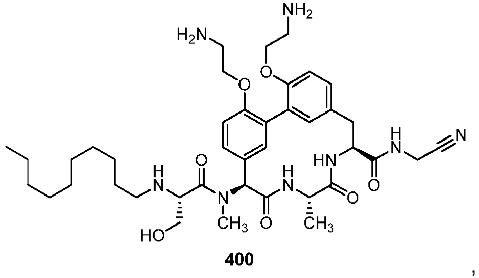 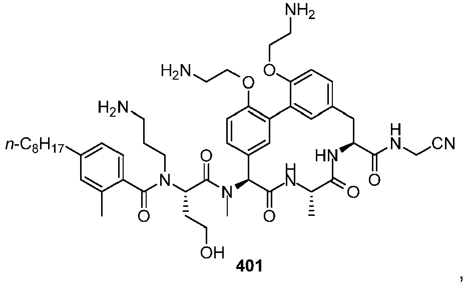 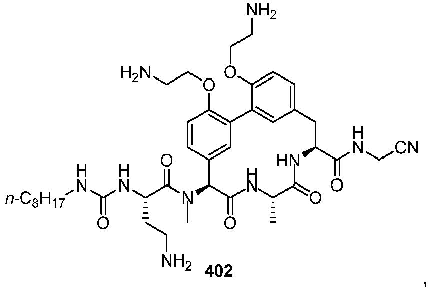 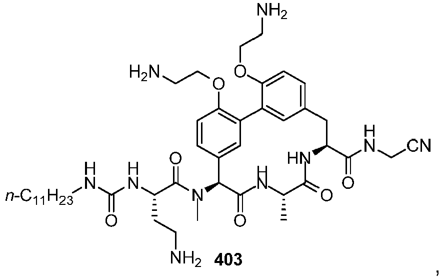 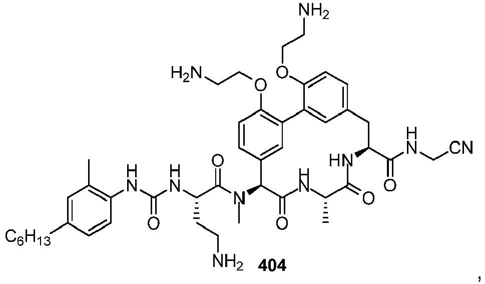 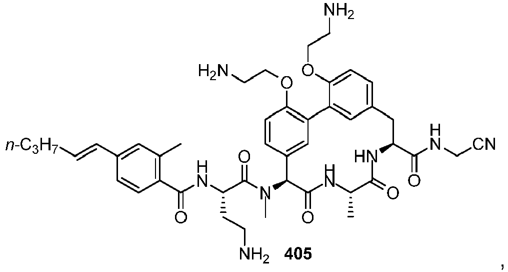 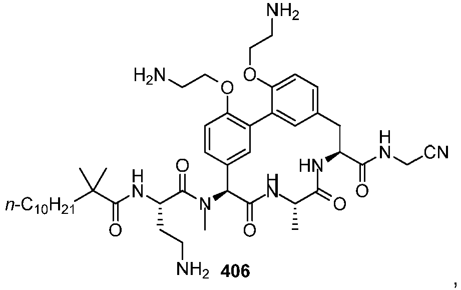 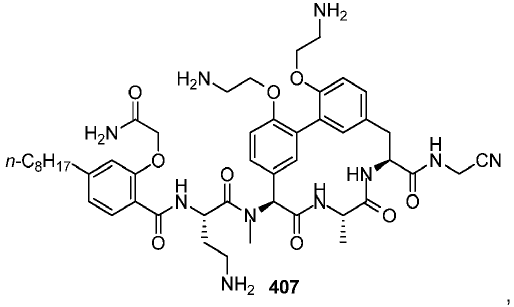 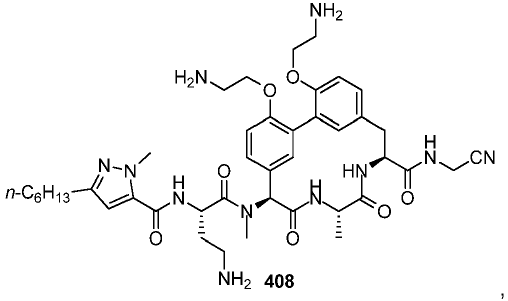 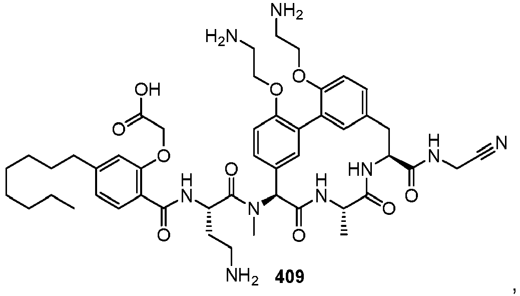 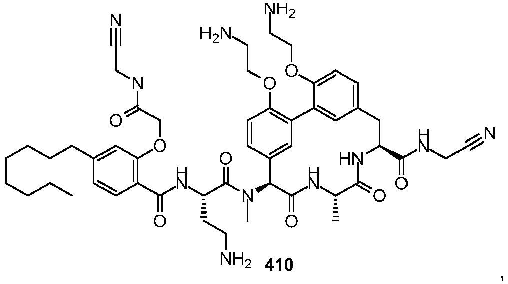 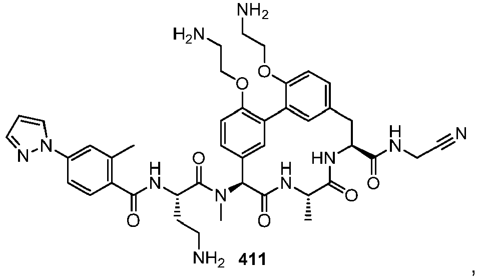 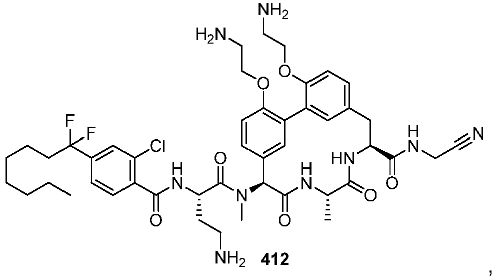 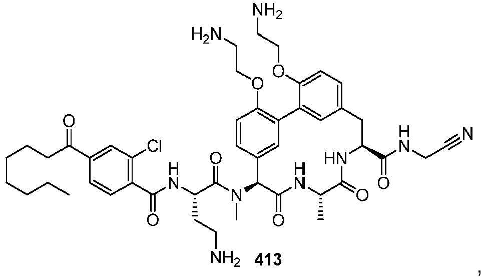 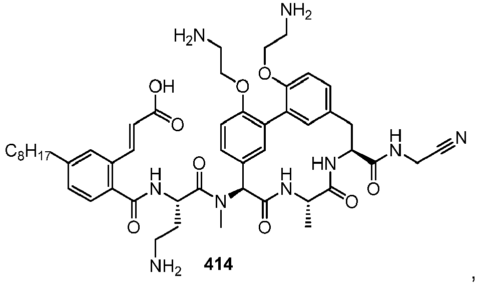 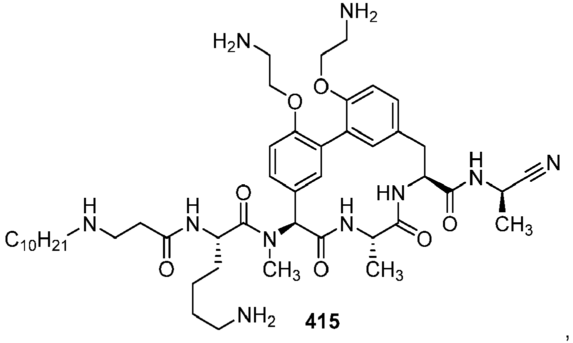 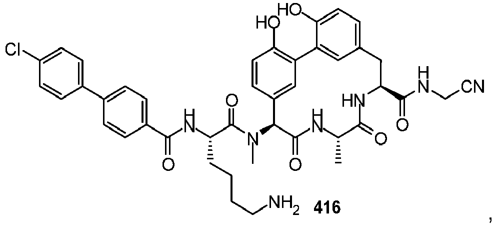 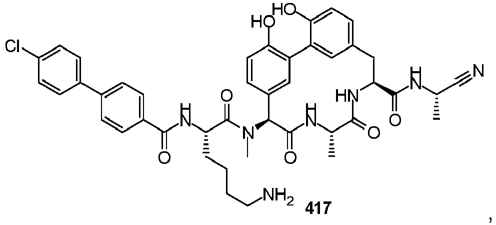 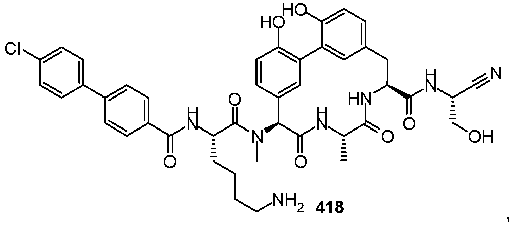 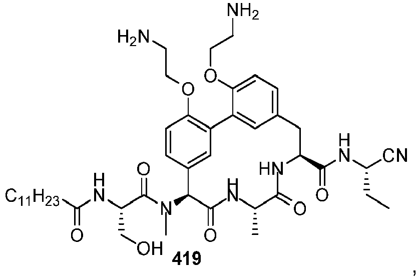 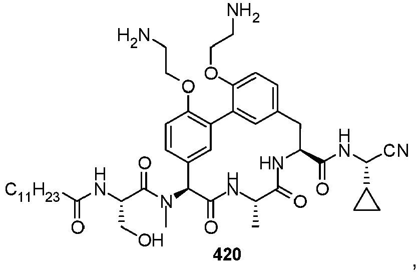 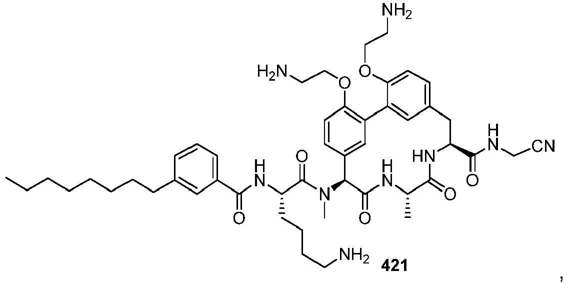 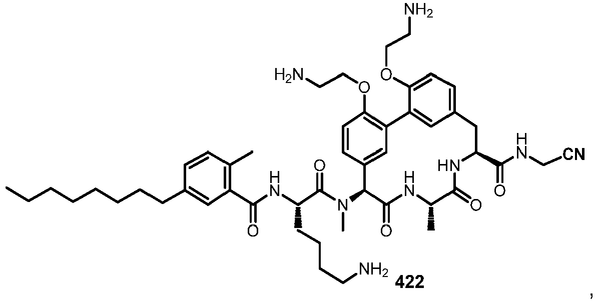 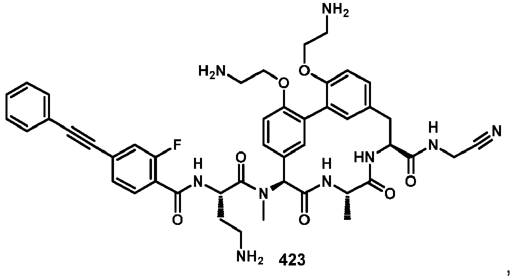 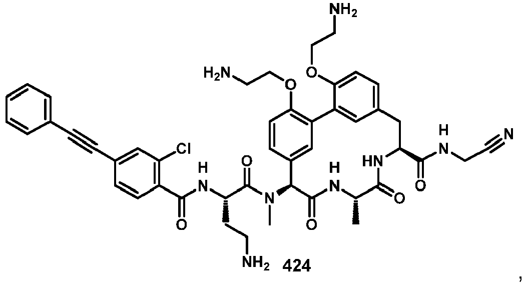 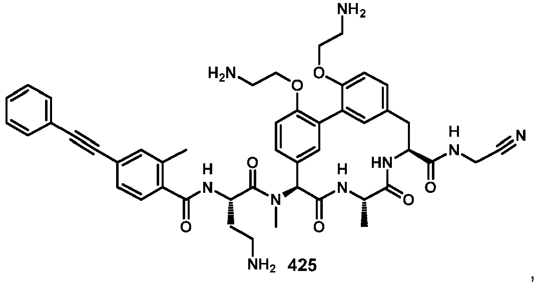 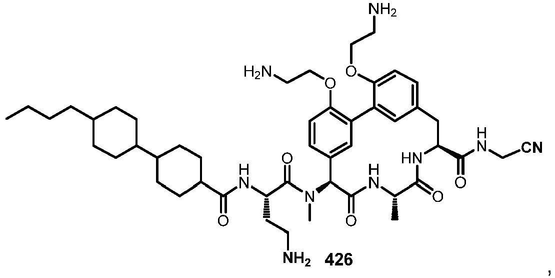 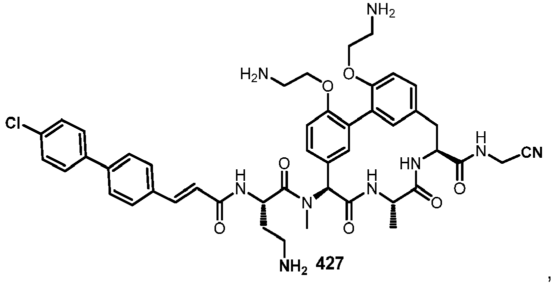 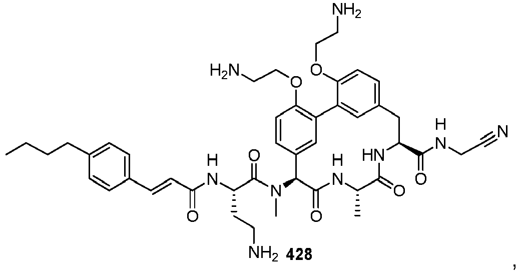 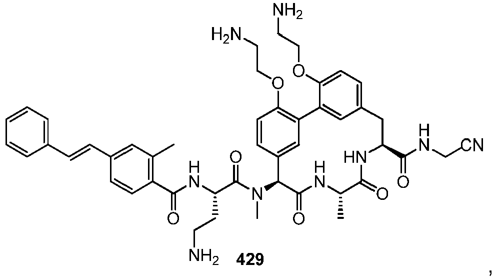 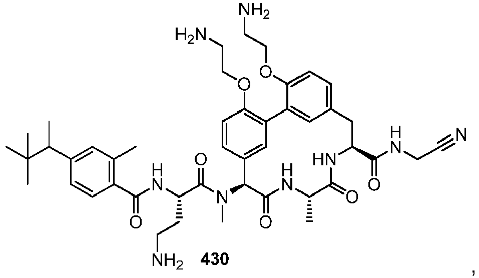 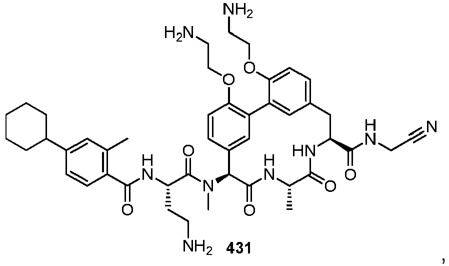 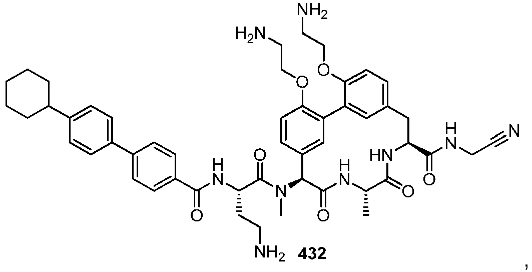 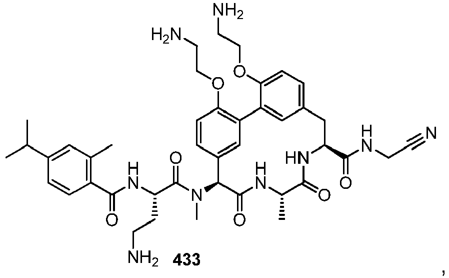 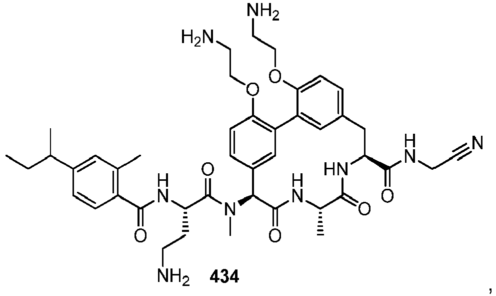 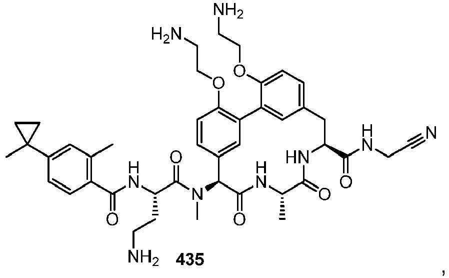 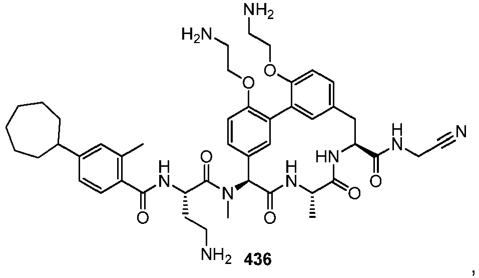 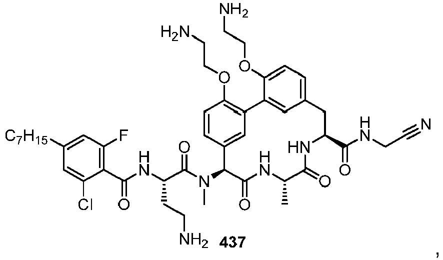 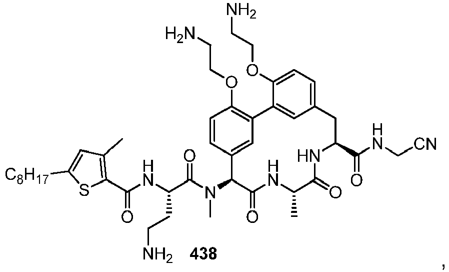 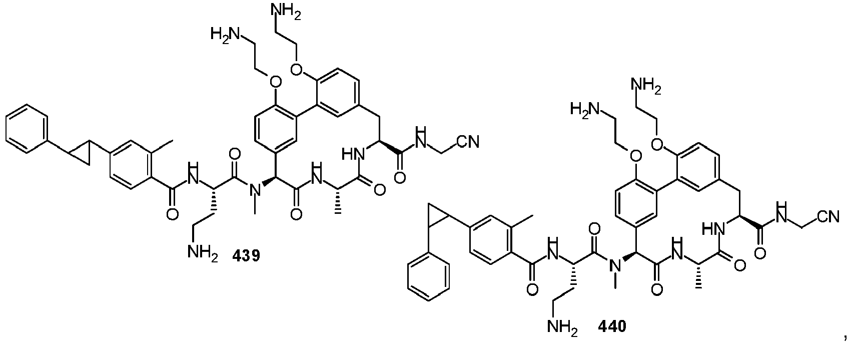 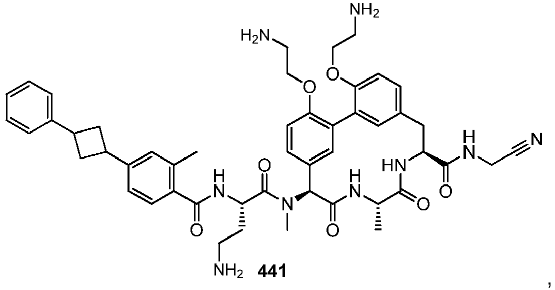 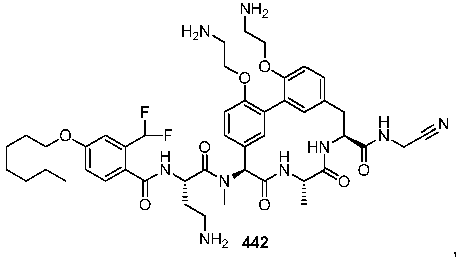 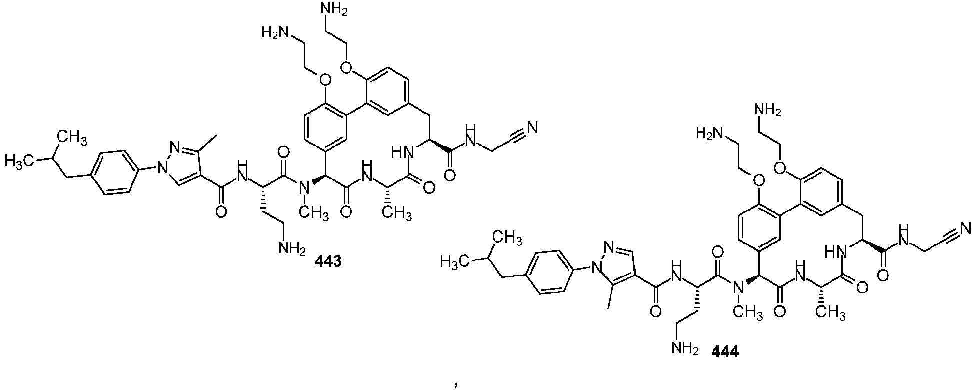 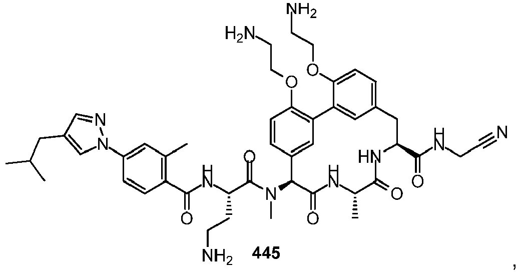 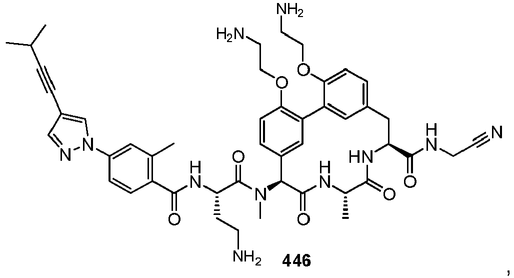 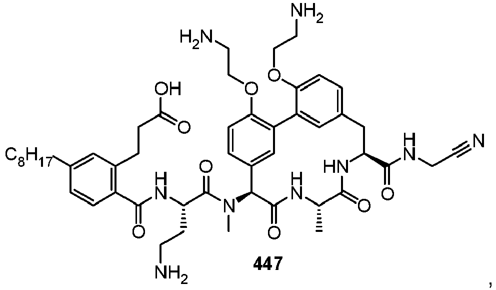 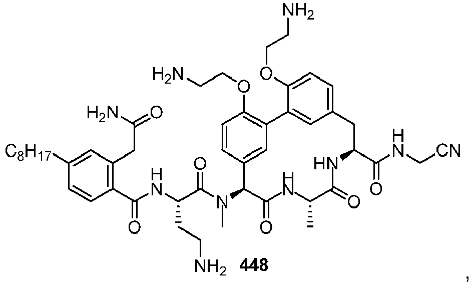 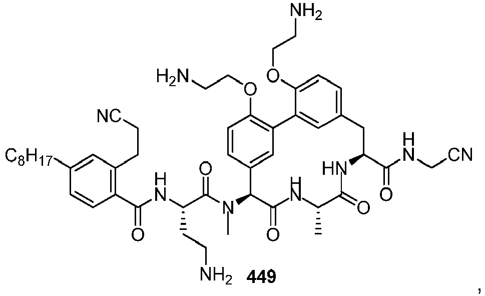 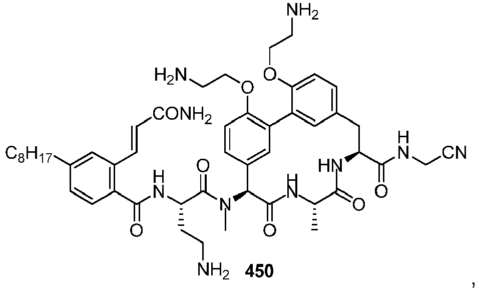 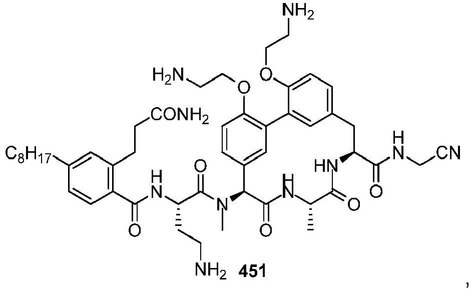 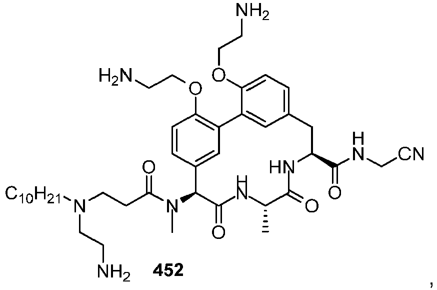 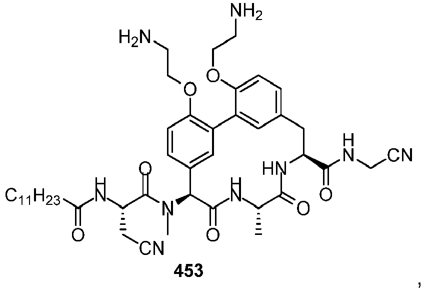 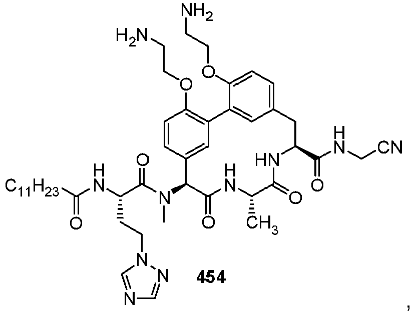 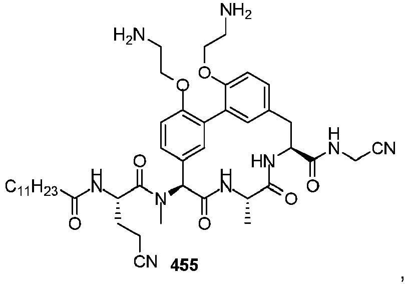 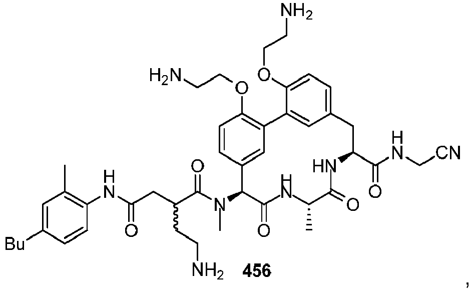 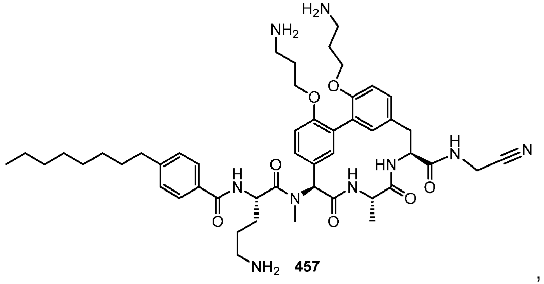 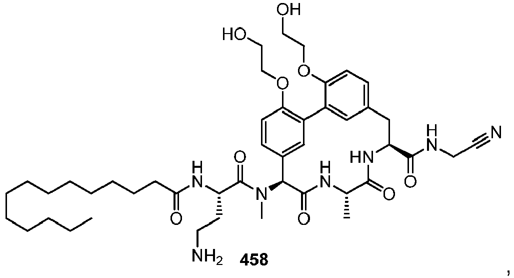 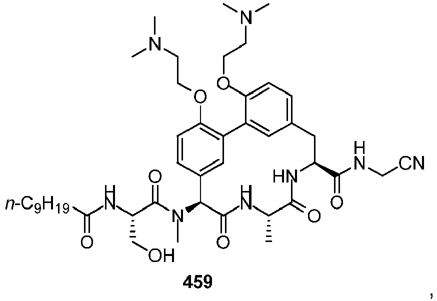 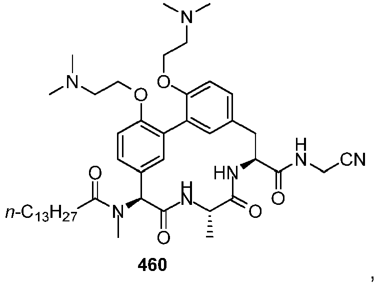 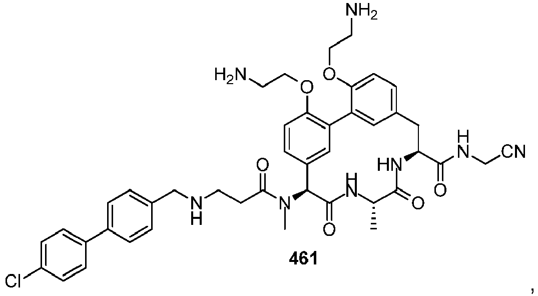 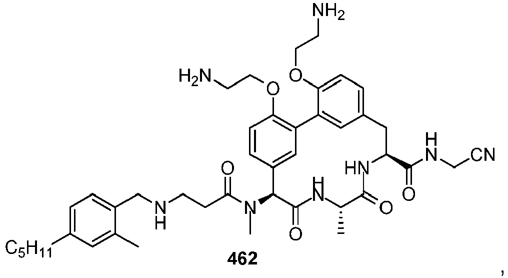 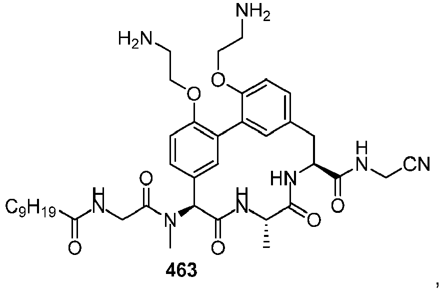 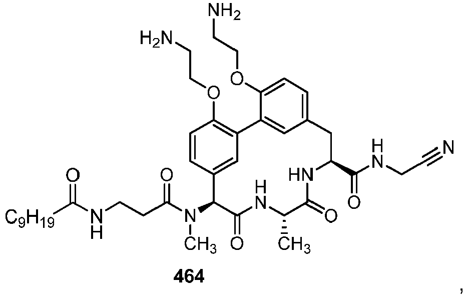 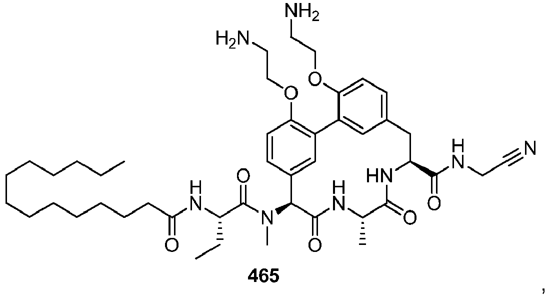 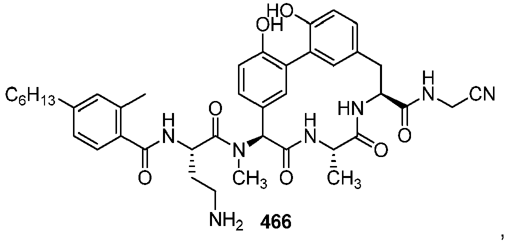 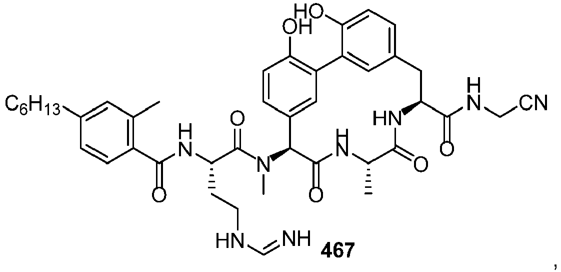 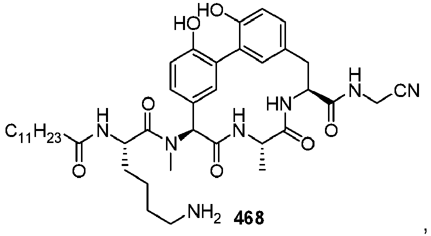 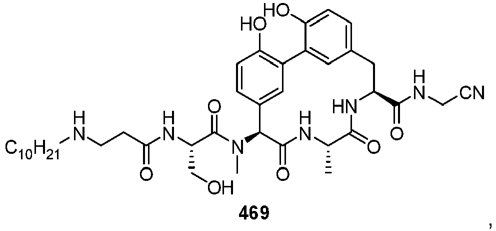 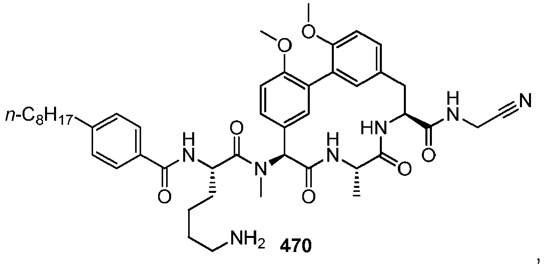 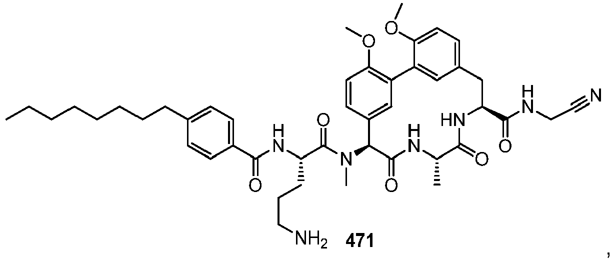 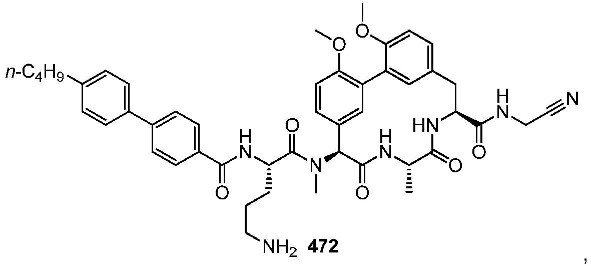 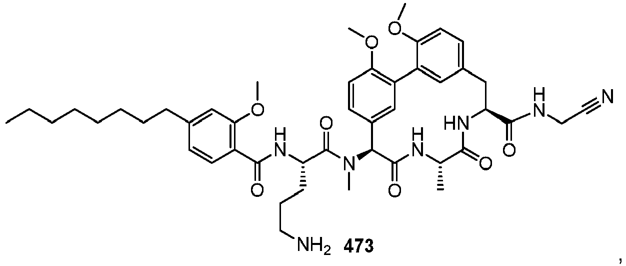 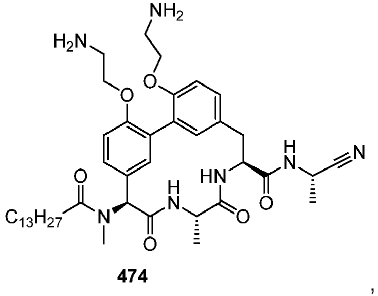 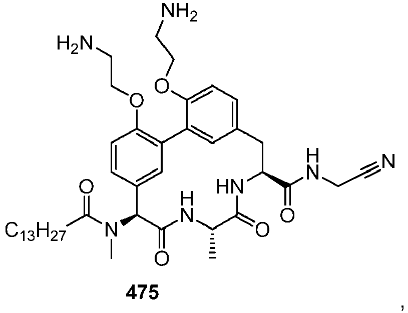 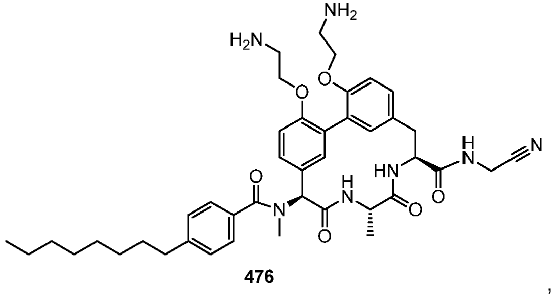 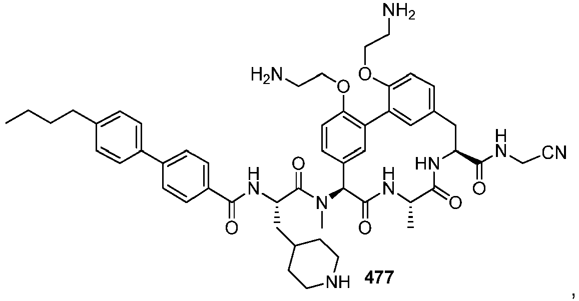 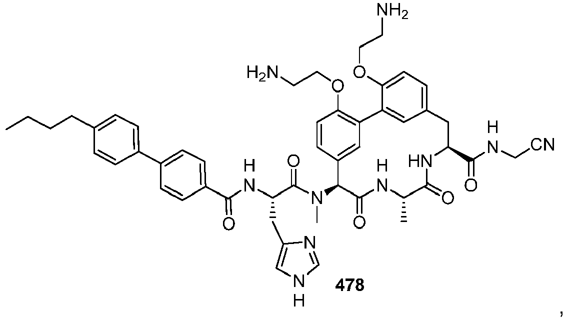 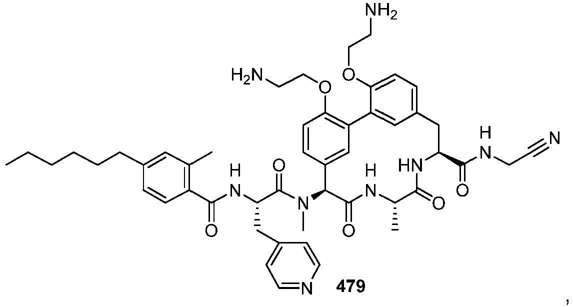 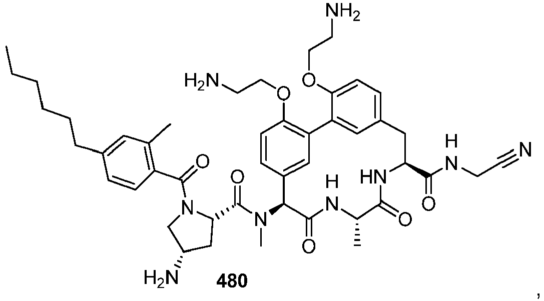 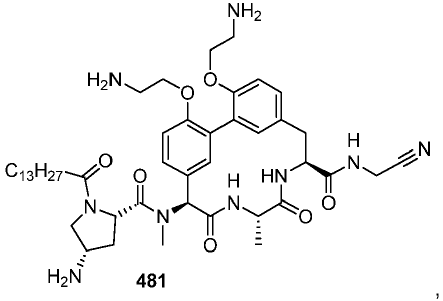 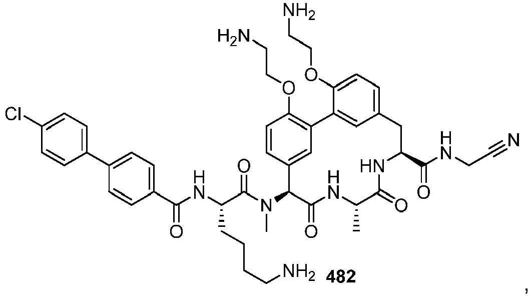 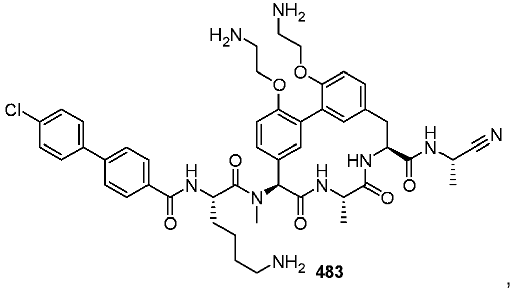 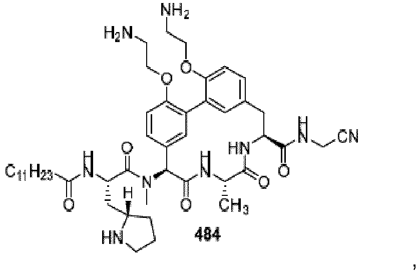 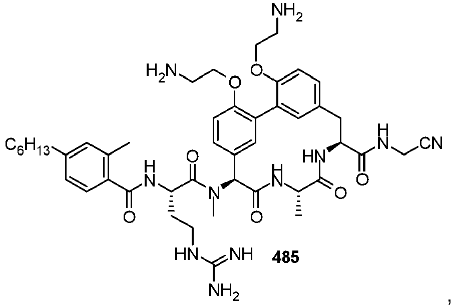 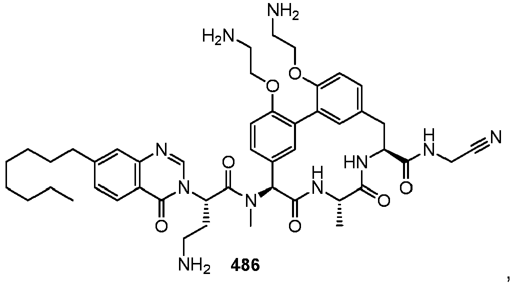 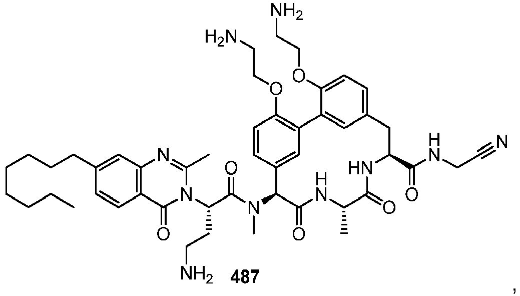 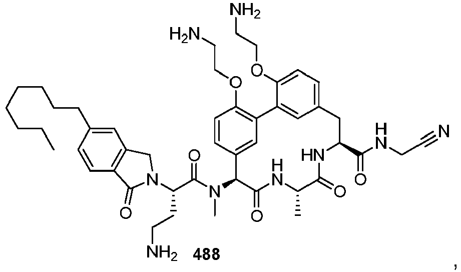 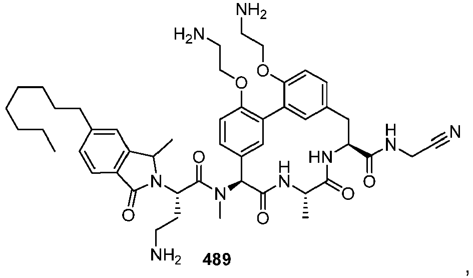 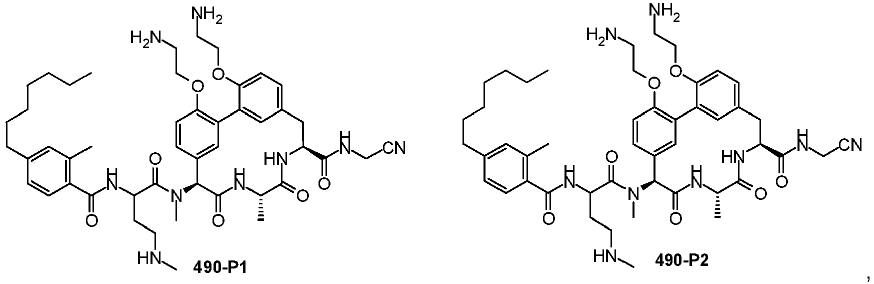 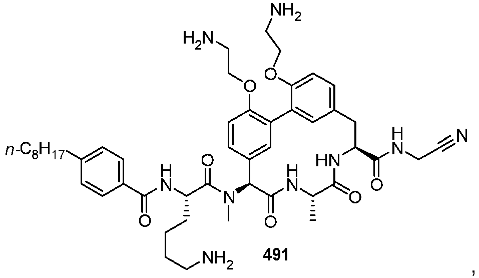 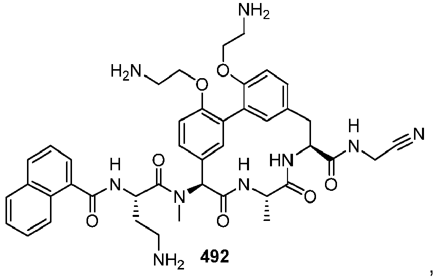 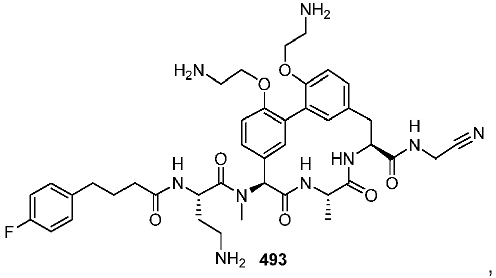 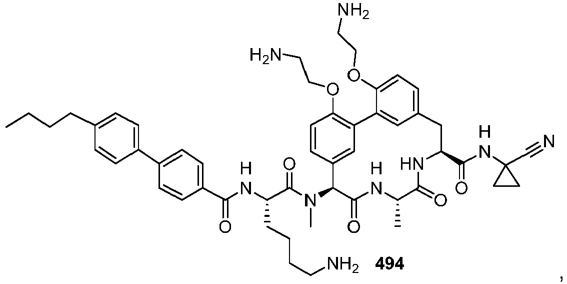 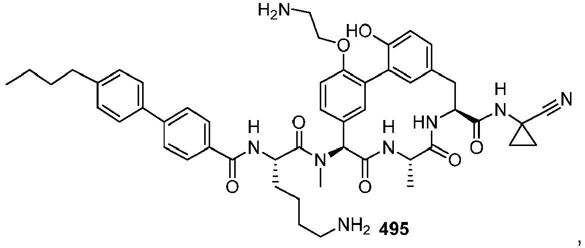 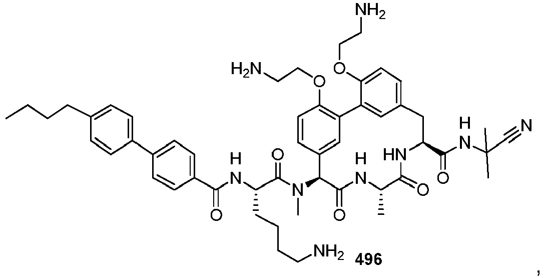 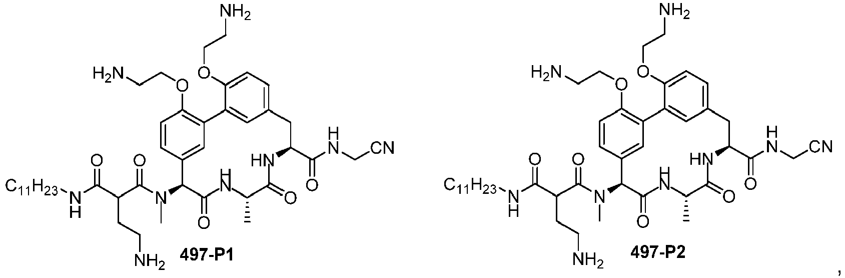 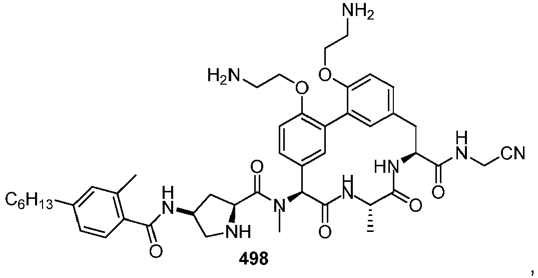 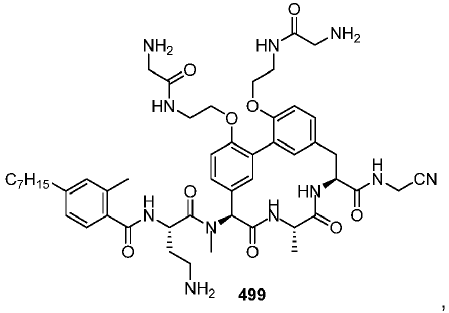 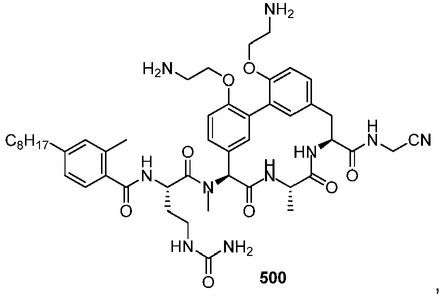 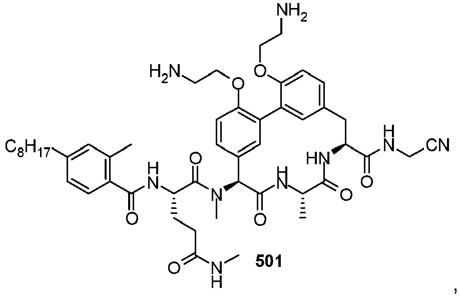 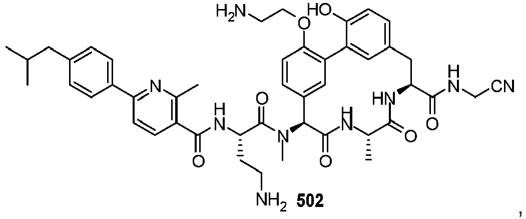 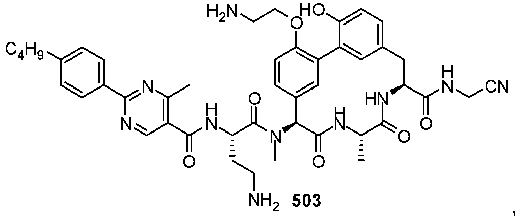 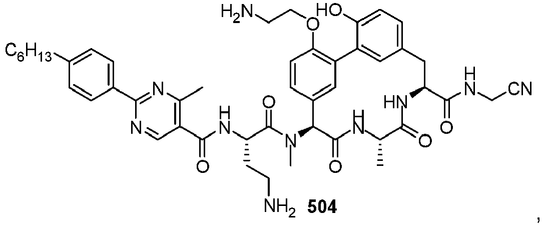 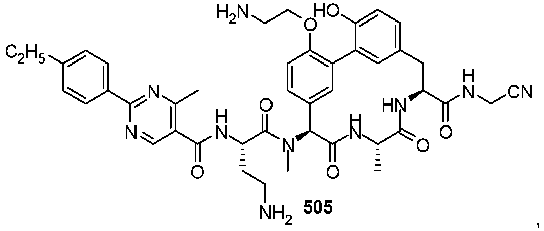 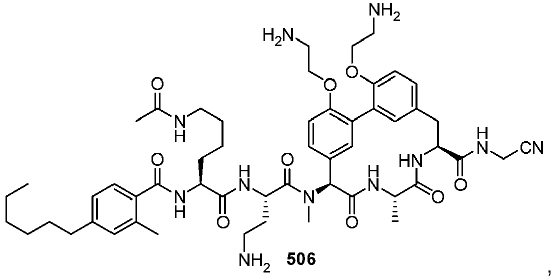 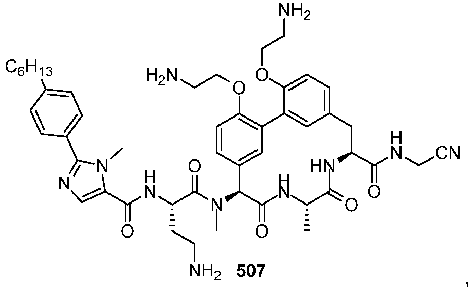 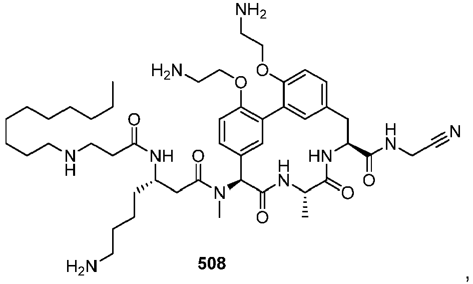 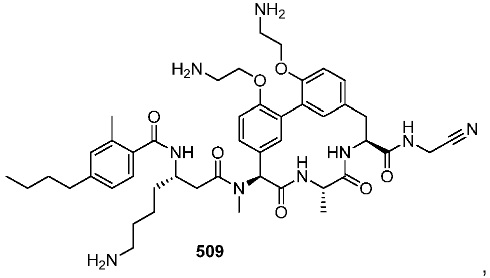 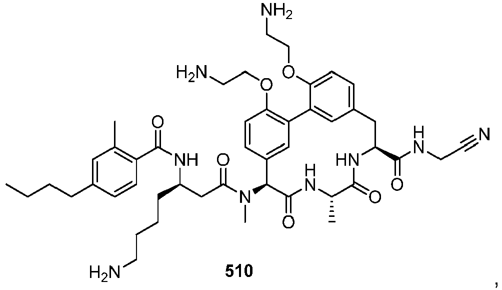 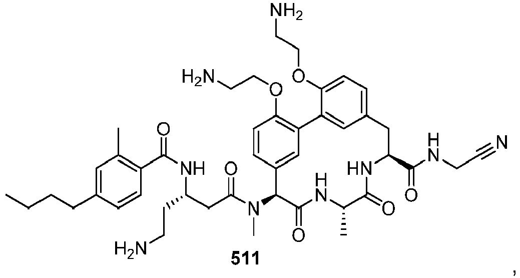 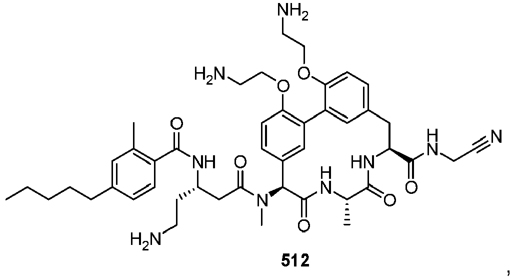 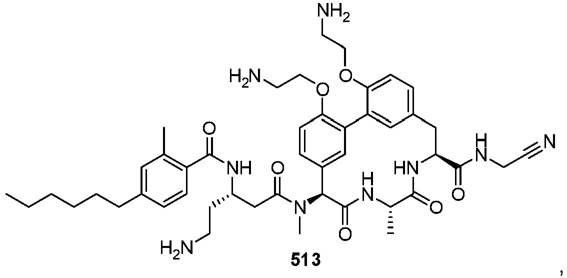 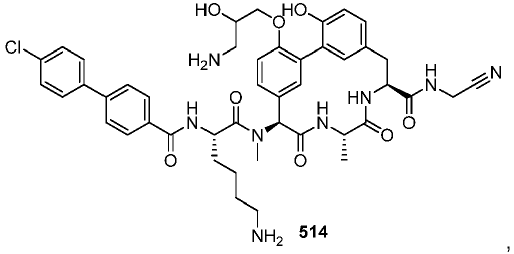 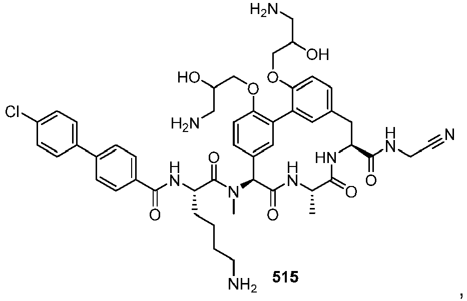 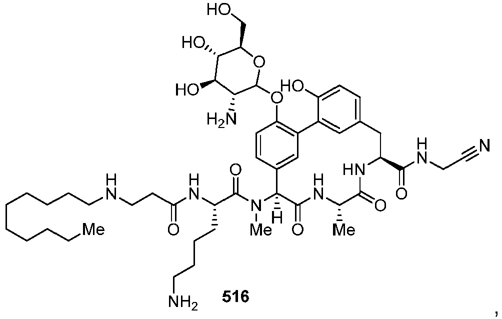 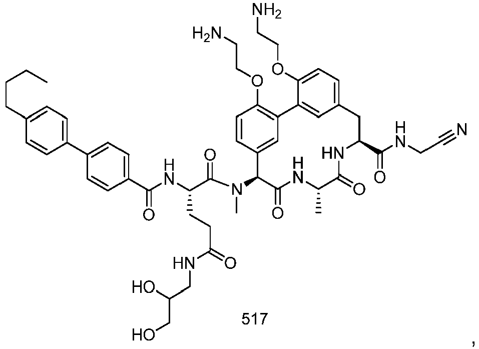 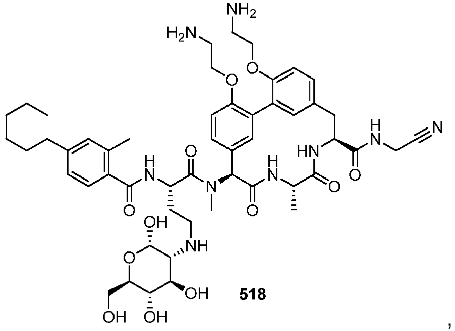 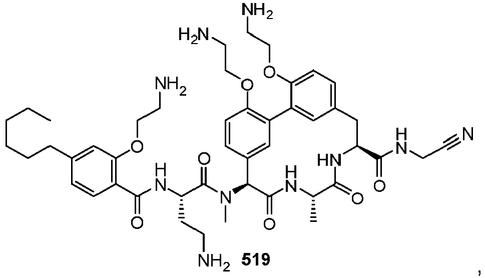 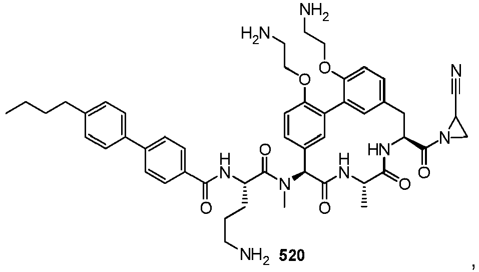 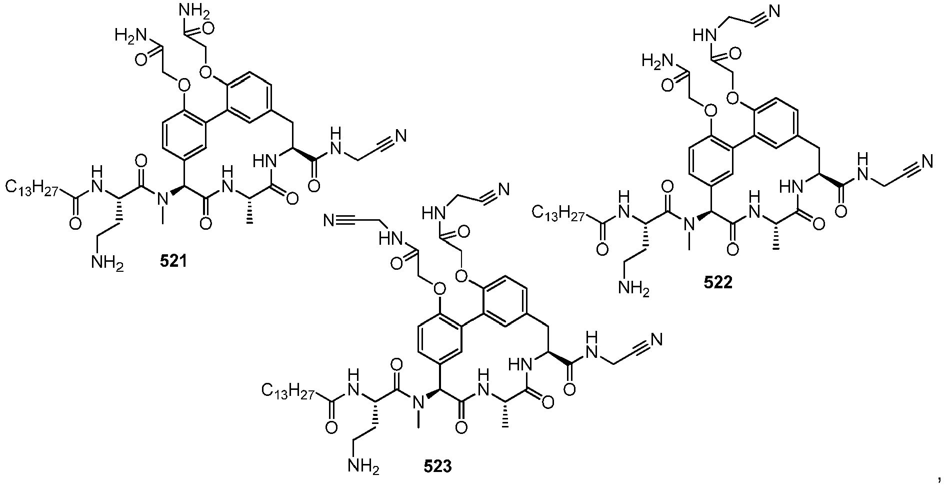 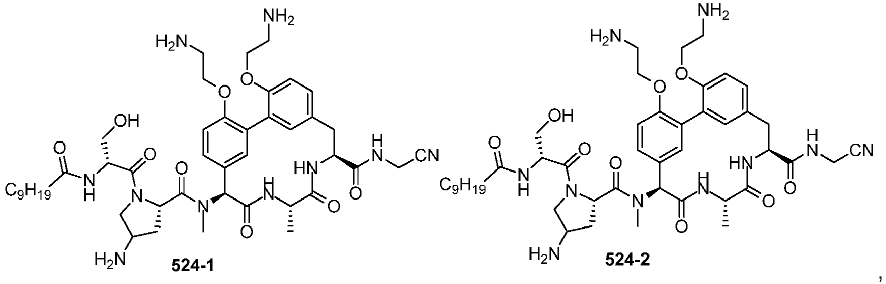 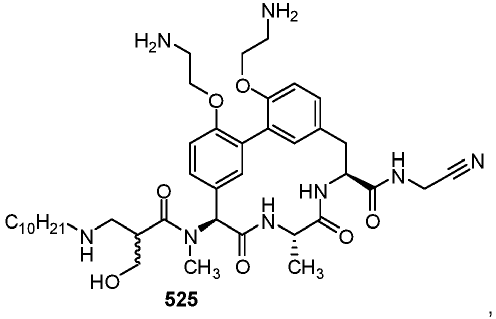 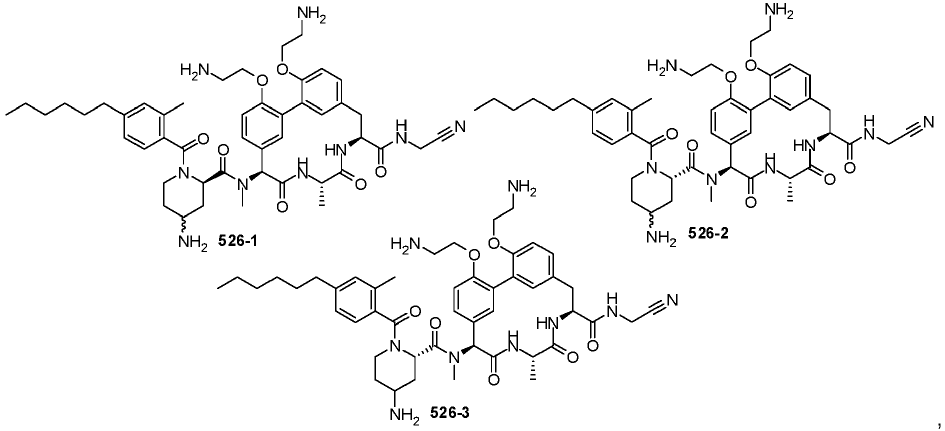 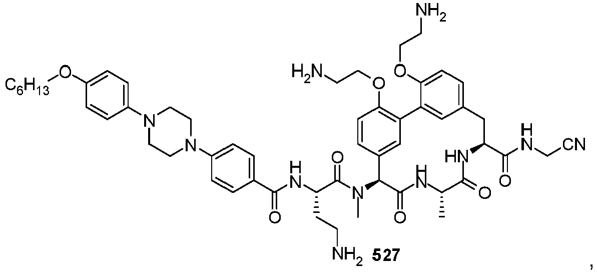 and 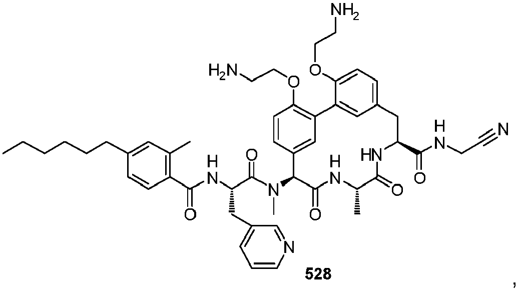 or a pharmaceutically acceptable salt or solvate thereof.

2. A pharmaceutical composition comprising a compound of claim 1, or a pharmaceutically acceptable salt or pharmaceutically acceptable solvate thereof, and a pharmaceutically acceptable excipient.

3. A compound of claim 1, or a pharmaceutically acceptable salt or pharmaceutically acceptable solvate thereof, for use in a method of treatment of a bacterial infection in a mammal.

4. The compound, salt, or solvate for use according to claim 3, wherein the bacterial infection is an infection involving:
Pseudomonas aeruginosa, Pseudomonas fluorescens, Pseudomonas acidovorans, Pseudomonas alcaligenes, Pseudomonas putida, Stenotrophomonas maltophilia, Burkholderia cepacia, Aeromonas hydrophilia, Escherichia coli, Citrobacter freundii, Salmonella typhimurium, Salmonella typhi, Salmonella paratyphi, Salmonella enteritidis, Shigella dysenteriae, Shigella flexneri, Shigella sonnei, Enterobacter cloacae, Enterobacter aerogenes, Klebsiella pneumoniae, Klebsiella oxytoca, Serratia marcescens, Francisella tularensis, Morganella morganii, Proteus mirabilis, Proteus vulgaris, Providencia alcalifaciens, Providencia rettgeri, Providencia stuartii, Acinetobacter baumannii, Acinetobacter calcoaceticus, Acinetobacter haemolyticus, Yersinia enterocolitica, Yersinia pestis, Yersinia pseudotuberculosis, Yersinia intermedia, Bordetella pertussis, Bordetella parapertussis, Bordetella bronchiseptica, Haemophilus influenzae, Haemophilus parainfluenzae, Haemophilus haemolyticus, Haemophilus parahaemolyticus, Haemophilus ducreyi, Pasteurella multocida, Pasteurella haemolytica, Branhamella catarrhalis, Helicobacter pylori, Campylobacter fetus, Campylobacter jejuni, Campylobacter coli, Borrelia burgdorferi, Vibrio cholerae, Vibrio parahaemolyticus, Legionella pneumophila, Listeria monocytogenes, Neisseria gonorrhoeae, Neisseria meningitidis, Kingella, Moraxella, Gardnerella vaginalis, Bacteroides fragilis, Bacteroides distasonis, Bacteroides 3452A homology group, Bacteroides vulgatus, Bacteroides ovalus, Bacteroides thetaiotaomicron, Bacteroides uniformis, Bacteroides eggerthii, Bacteroides splanchnicus, Clostridium difficile, Mycobacterium tuberculosis, Mycobacterium avium, Mycobacterium intracellulare, Mycobacterium leprae, Corynebacterium diphtheriae, Corynebacterium ulcerans, Streptococcus pneumoniae, Streptococcus agalactiae, Streptococcus pyogenes, Enterococcus faecalis, Enterococcus faecium, Staphylococcus aureus, Staphylococcus epidermidis, Staphylococcus saprophyticus, Staphylococcus intermedius, Staphylococcus hyicus subsp. hyicus, Staphylococcus haemolyticus, Staphylococcus hominis, or Staphylococcus saccharolyticus.

5. The compound, salt, or solvate for use according to claim 3 or 4, wherein the bacterial infection is an infection involving a Gram-negative bacteria.

6. The compound, salt, or solvate for use according to any one of claims 3 to 5, wherein the method comprises topical administration of the compound, salt, or solvate.

7. The compound, salt, or solvate for use according to any one of claims 3 to 5, wherein the method further comprises administering a second therapeutic agent.

8. The compound, salt, or solvate for use according to claim 7, wherein the second therapeutic agent is not an SpsB inhibitor.

9. The compound, salt, or solvate for use according to claim 8, wherein the second therapeutic agent is an aminoglycoside antibiotic, fluoroquinolone antibiotic, β-lactam antibiotic, macrolide antibiotic, glycopeptide antibiotic, rifampicin, chloramphenicol, fluoramphenicol, colistin, mupirocin, bacitracin, daptomycin, or linezolid.

10. The compound, salt, or solvate for use according to claim 8, wherein the second therapeutic agent is a β-lactam antibiotic.

11. The compound, salt, or solvate for use according to claim 10, wherein the β-lactam antibiotic is selected from penicillins, monobactams, cephalosporins, cephamycins, and carbapenems.

12. The compound, salt, or solvate for use according to claim 11, wherein the β-lactam antibiotic is selected from Azlocillin, Amoxicillin, Ampicillin, Doripenem, Meropenem, Biapenem, Cefamandole, Imipenem, Mezlocillin, Cefmetazole, Cefprozil, Piperacillin/tazobactam, Carbenicillin, Cefaclor, Cephalothin, Ertapenem, Cefazolin, Cefepime, Cefonicid, Cefoxitin, Ceftazidime, Oxacillin, Cefdinir, Cefixime, Cefotaxime, Cefotetan, Cefpodoxime, Ceftizoxime, Ceftriaxone, Faropenem, Mecillinam, Methicillin, Moxalactam, Ticarcillin, Tomopenem, Ceftobiprole, Ceftaroline, Flomoxef, Cefiprome, and Cefozopran.

13. The compound, salt, or solvate for use according to claim 10, wherein the method further comprises administering a β-lactamase inhibitor.

## Patentansprüche

1. Verbindung, die aus den folgenden ausgewählt ist: 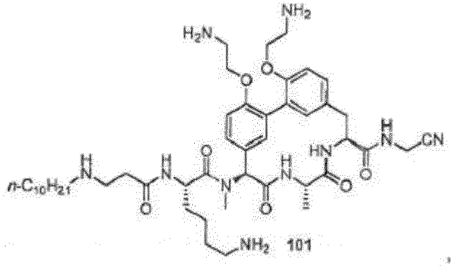 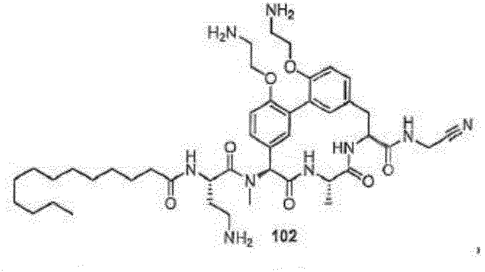 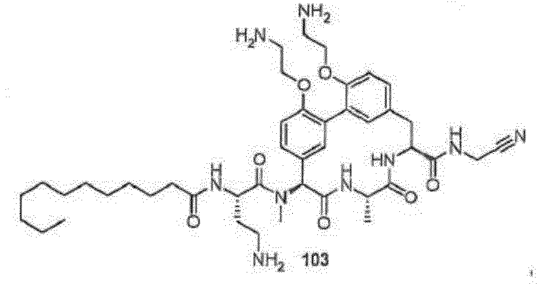 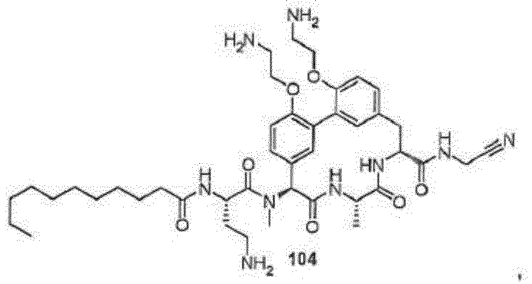 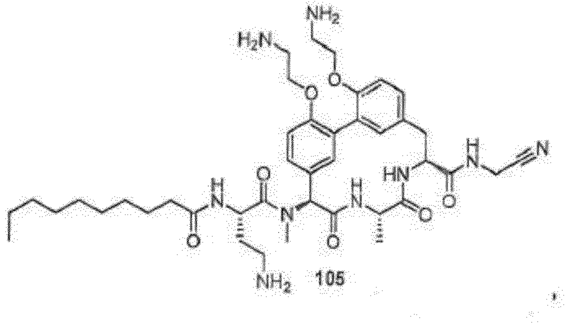 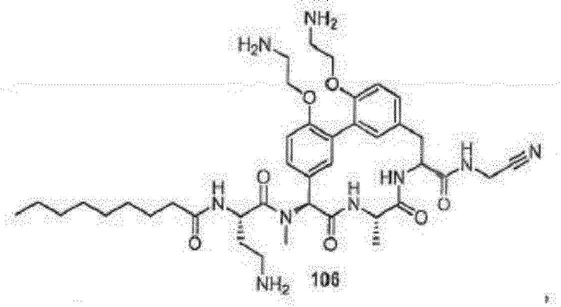 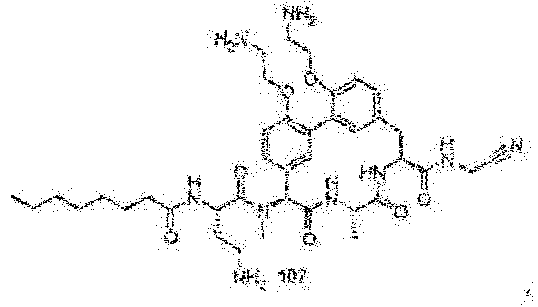 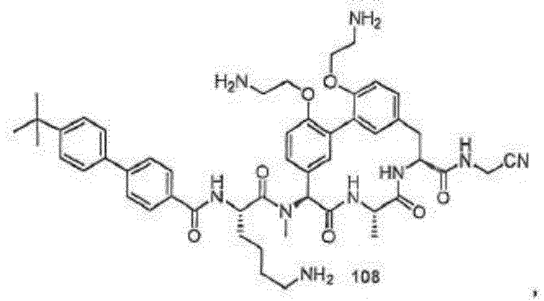 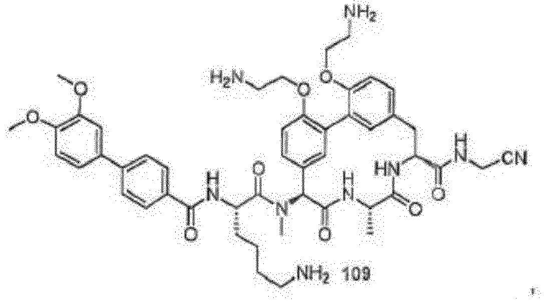 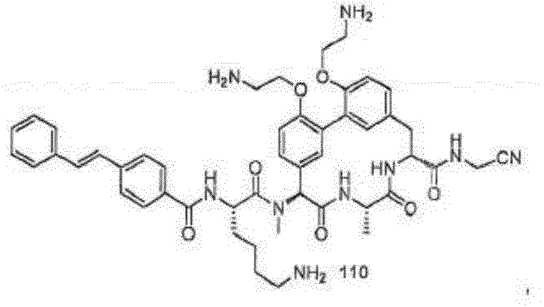 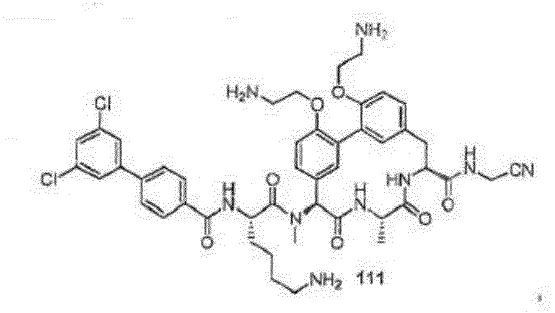 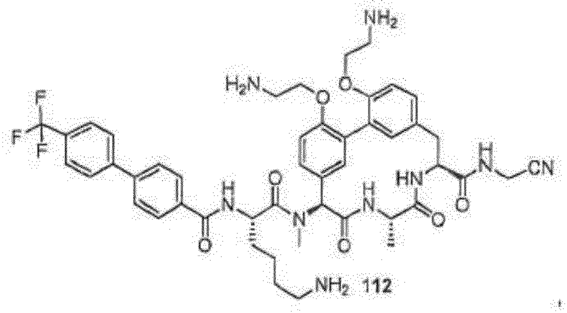 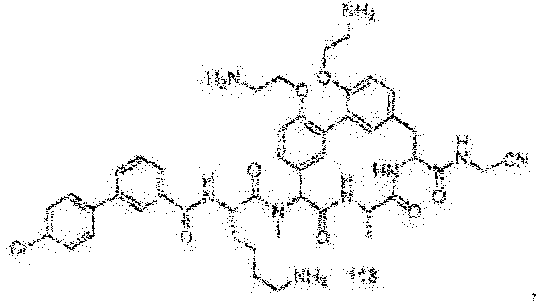 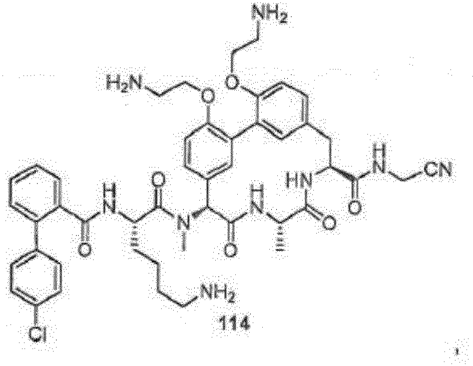 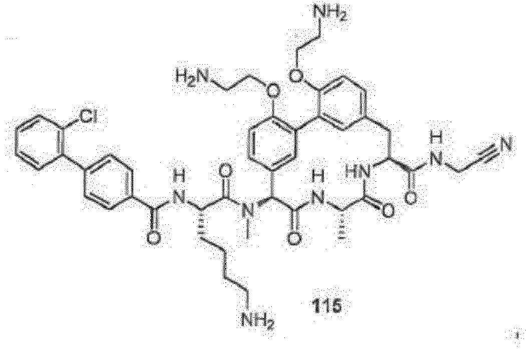 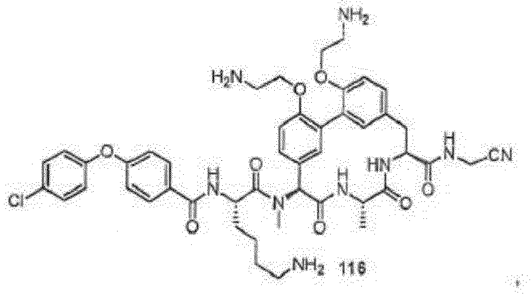 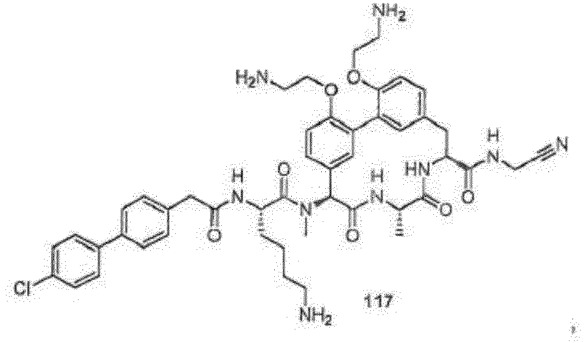 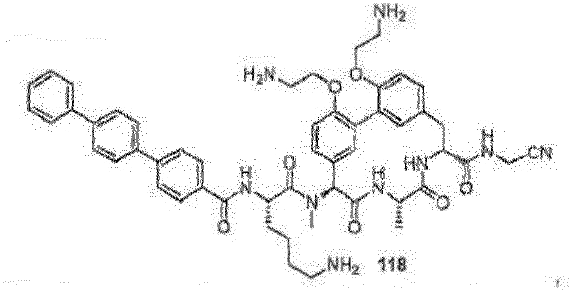 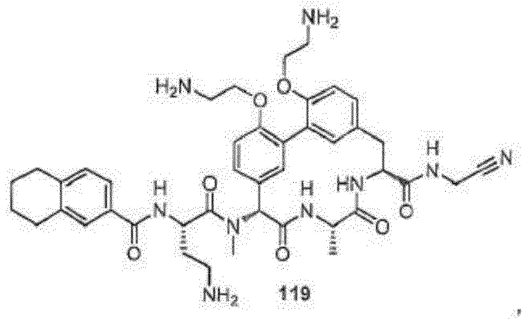 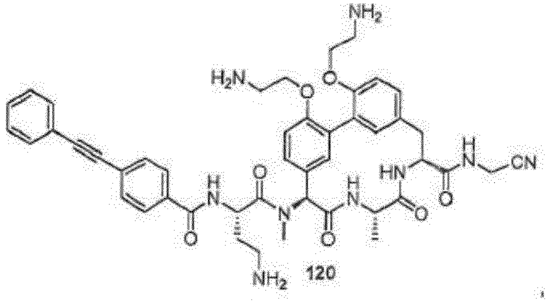 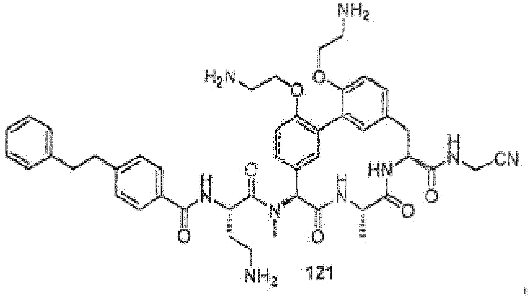 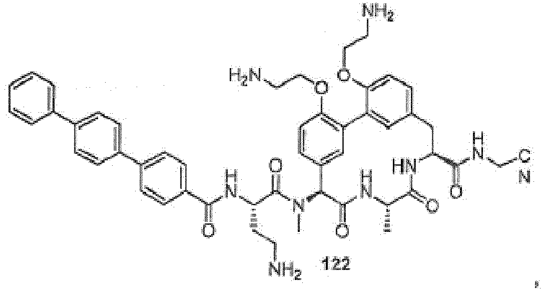 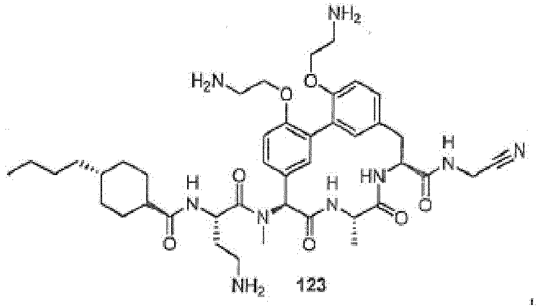 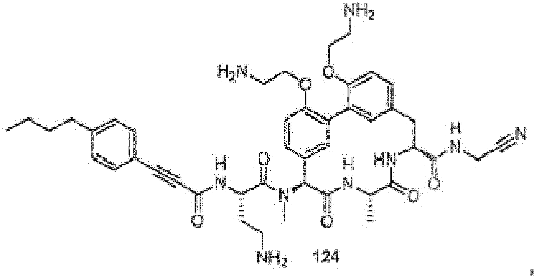 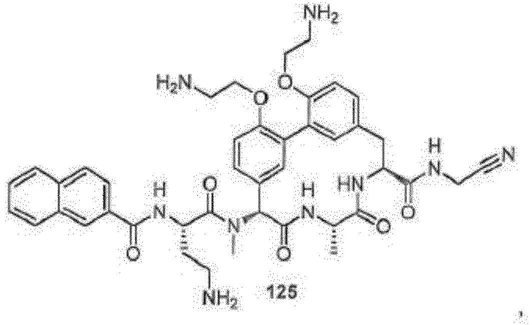 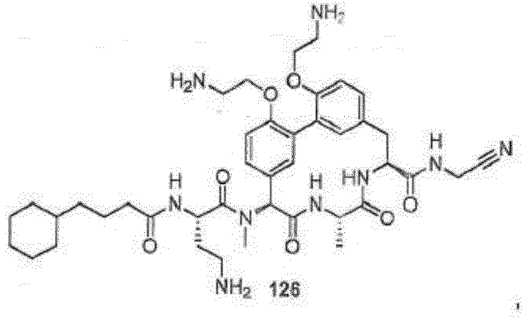 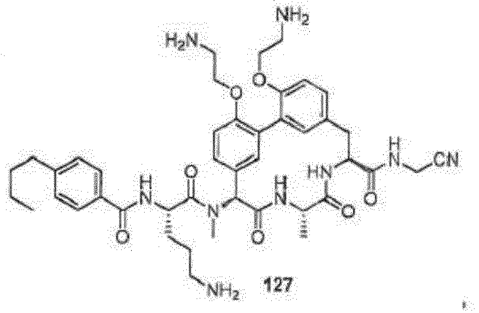 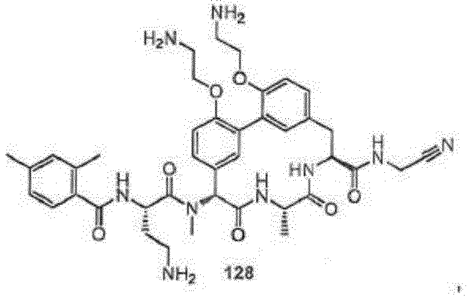 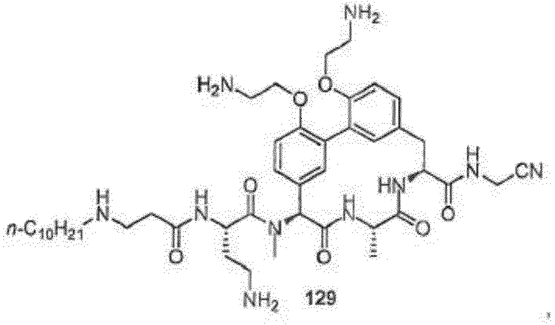 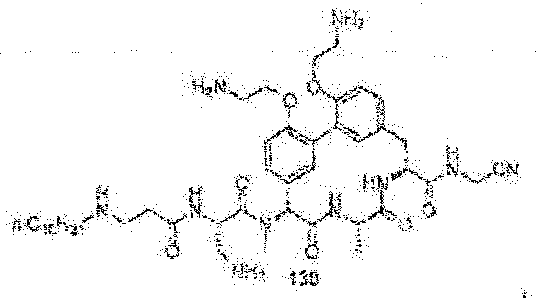 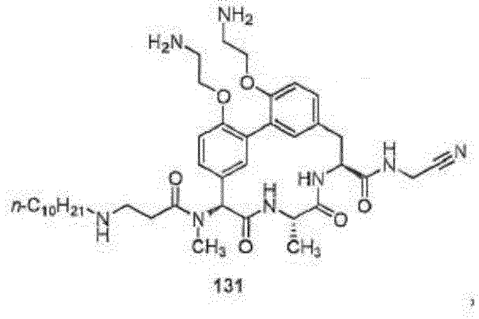 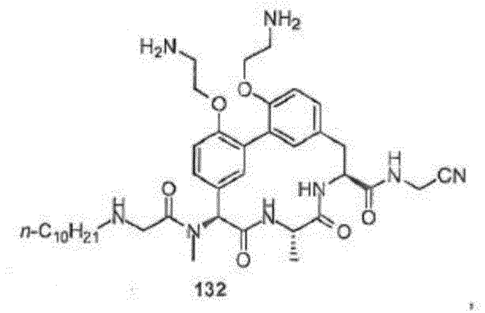 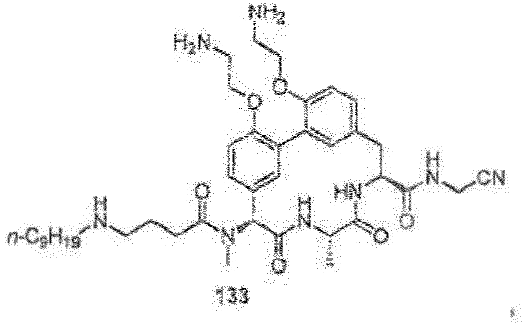 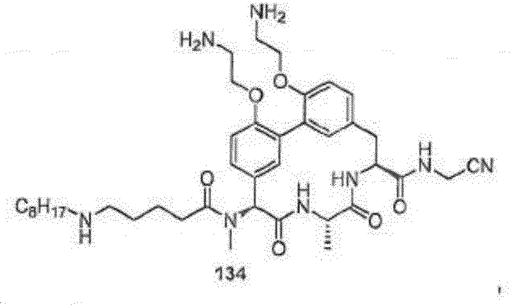 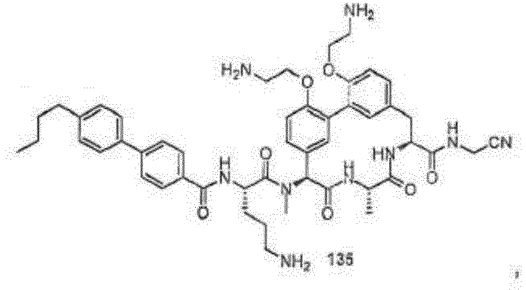 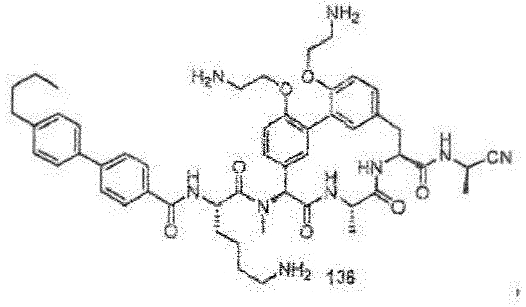 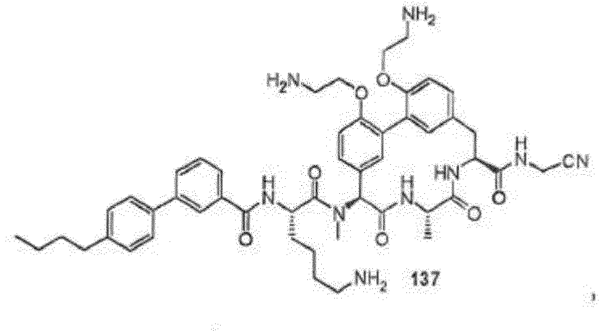 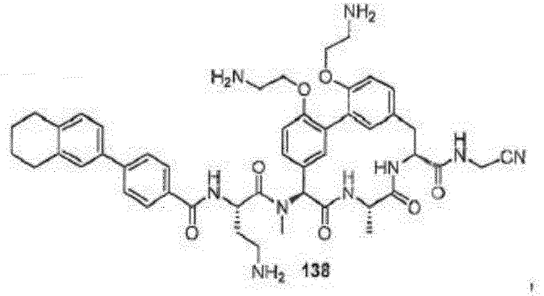 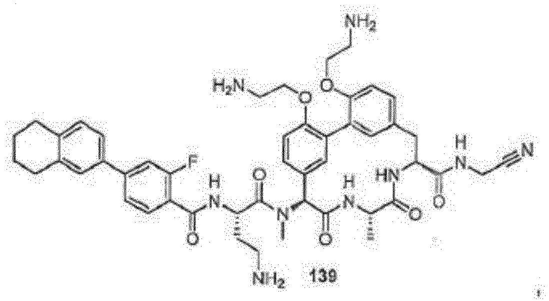 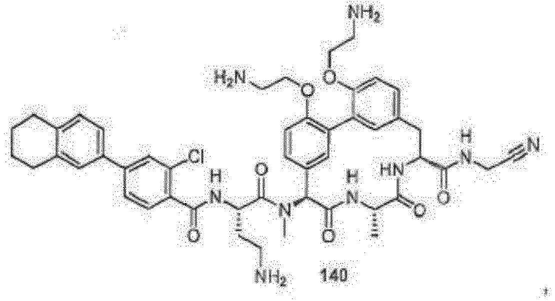 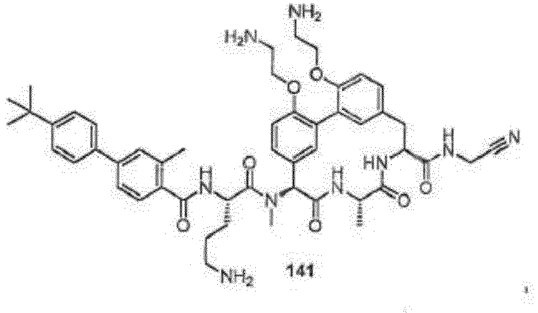 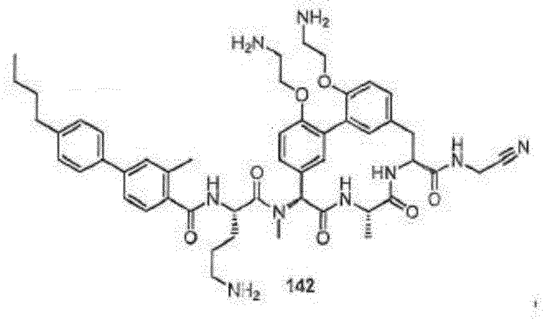 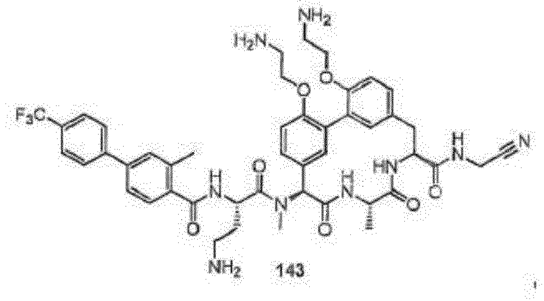 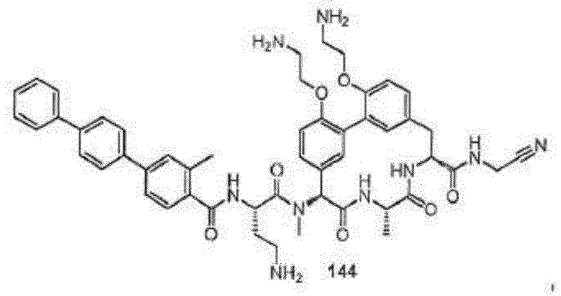 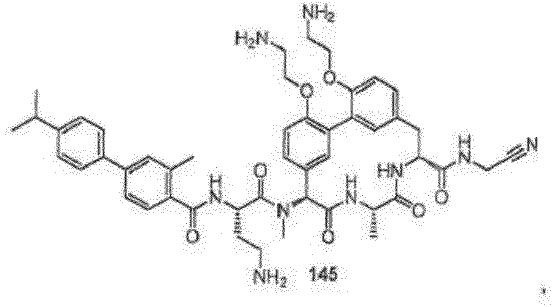 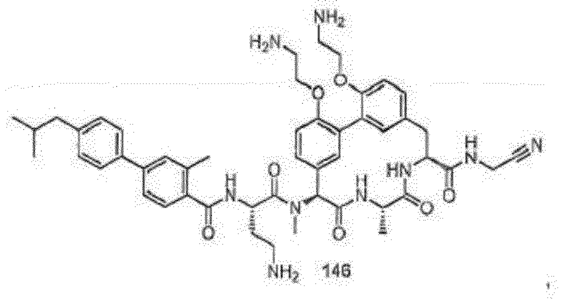 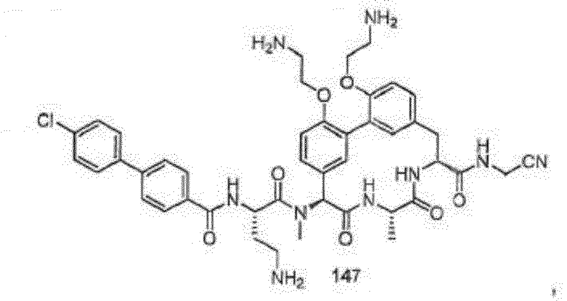 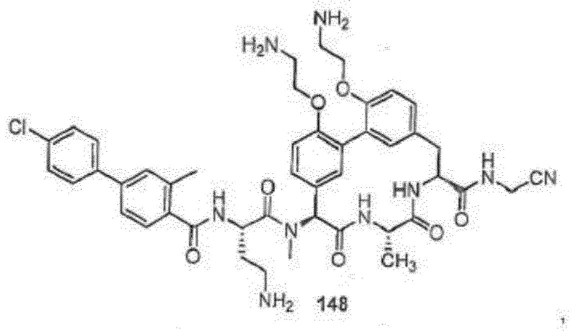 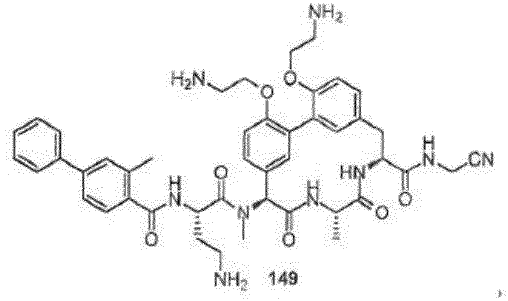 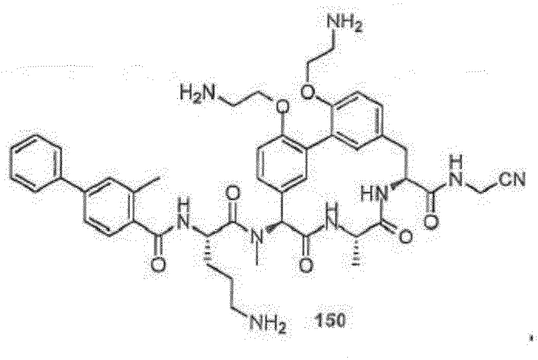 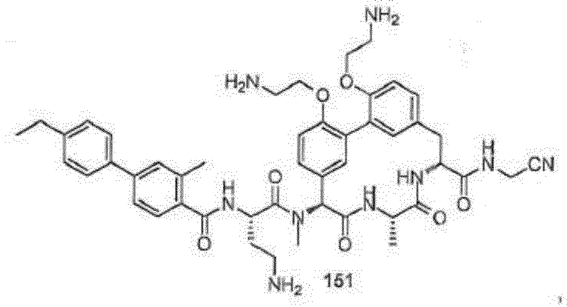 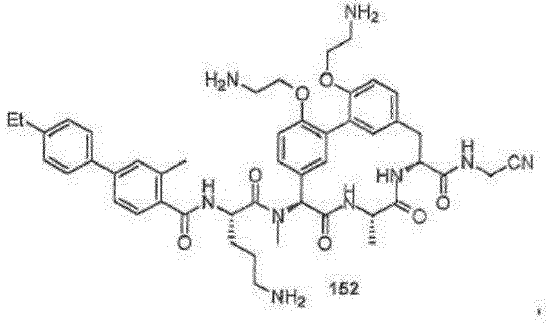 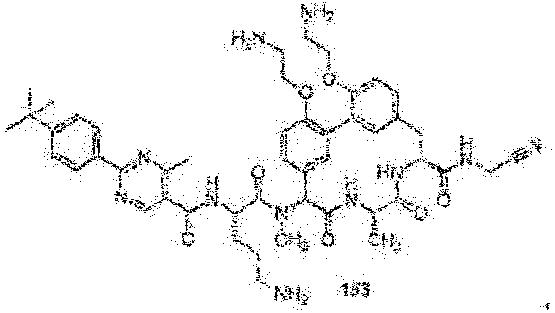 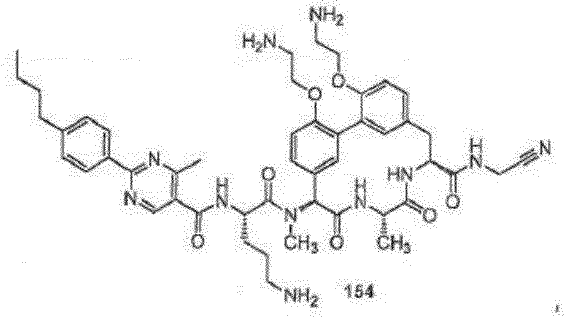 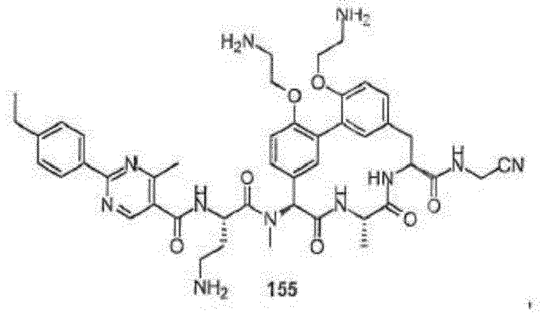 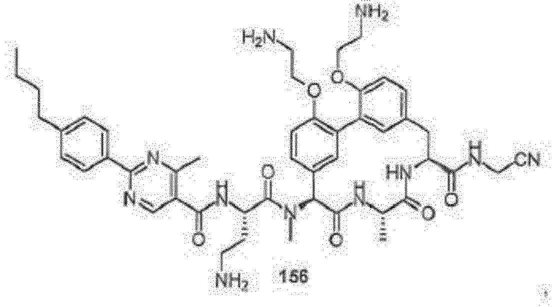 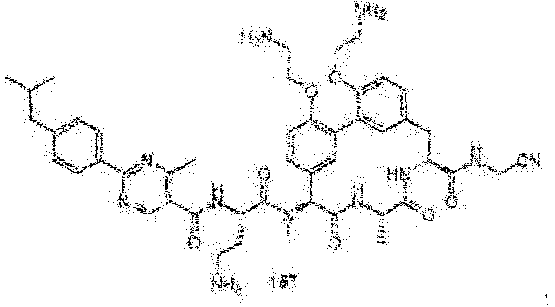 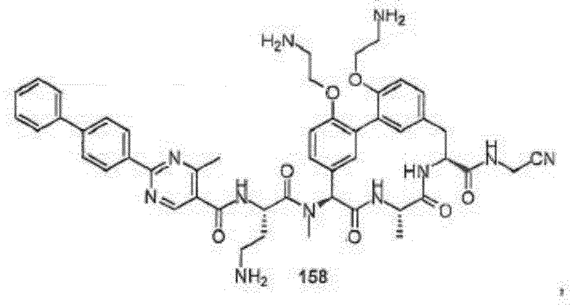 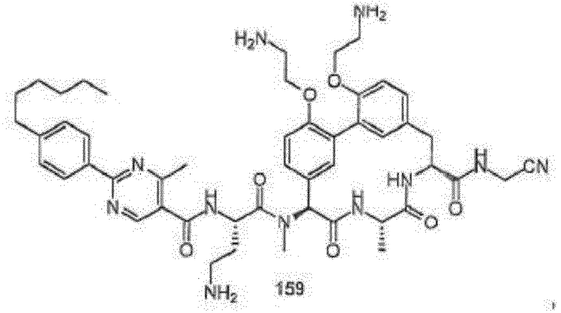 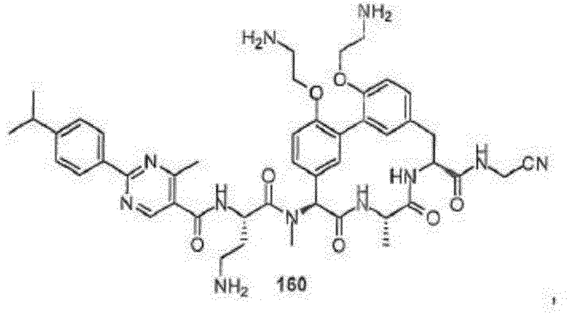 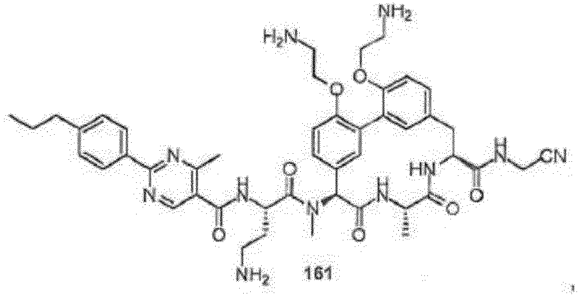 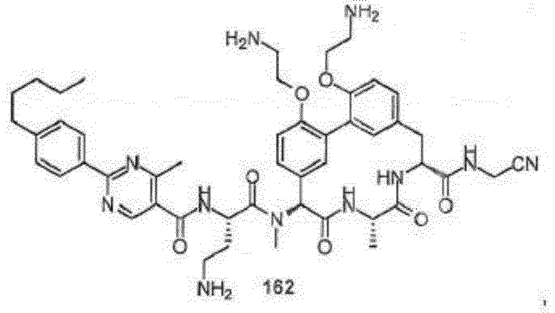 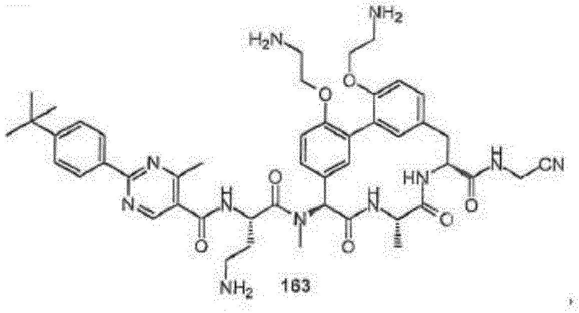 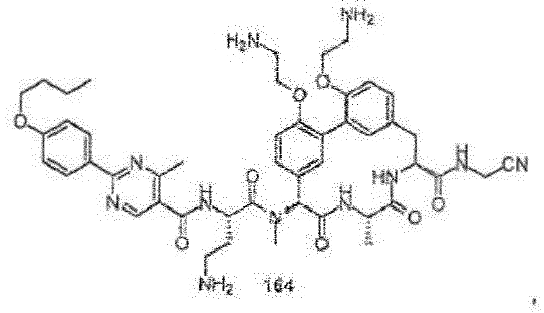 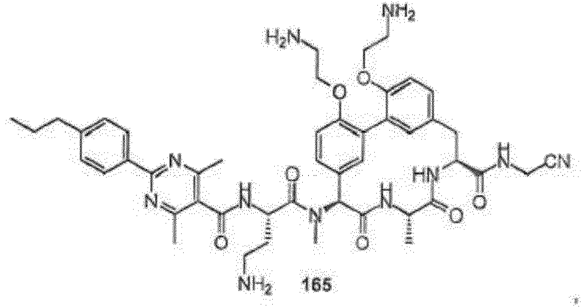 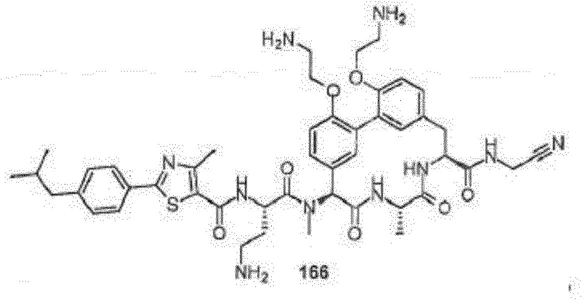 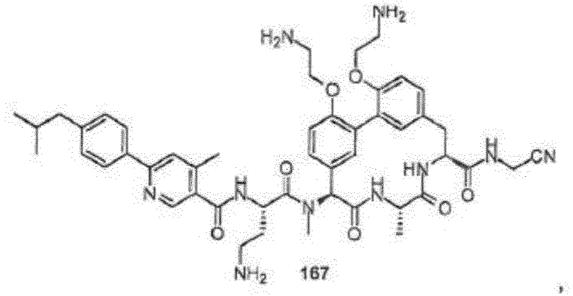 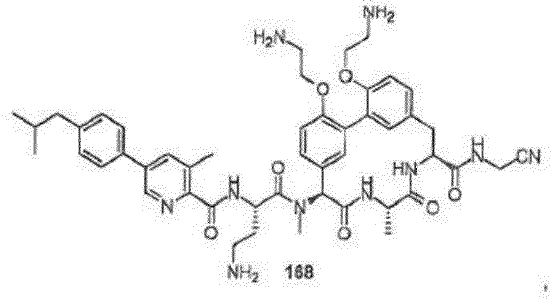 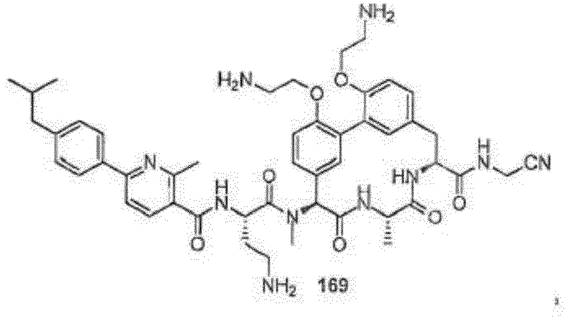 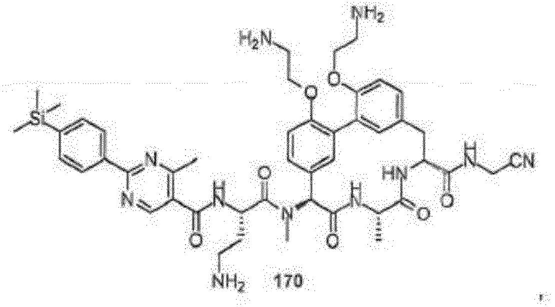 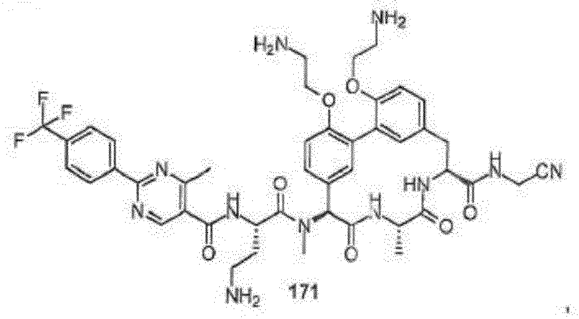 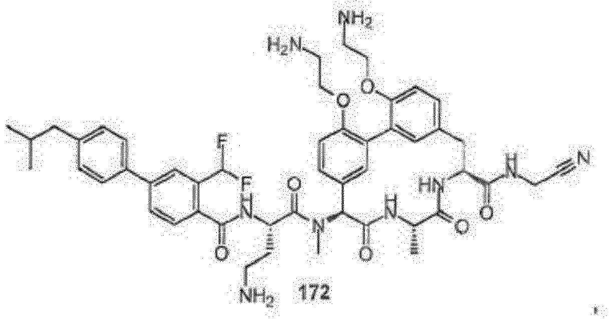 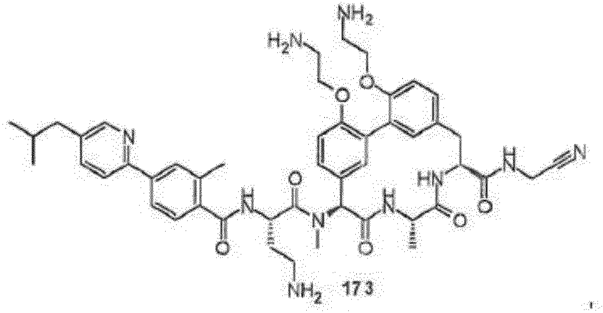 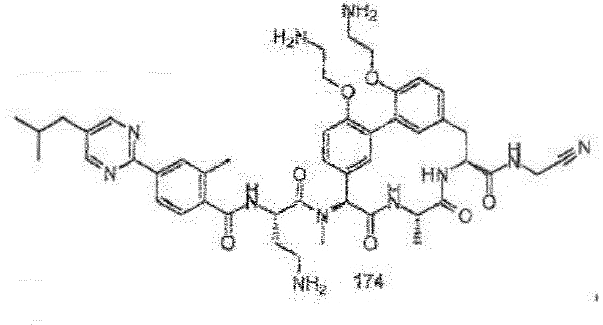 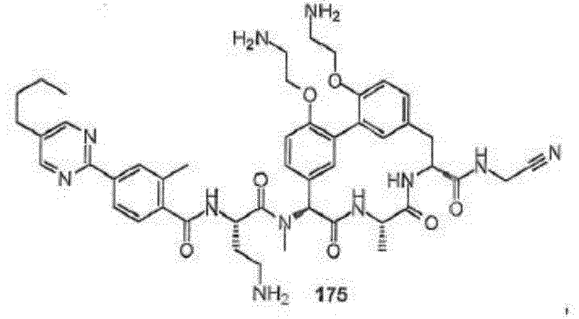 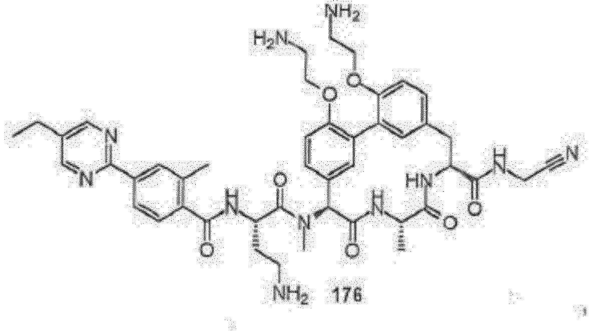 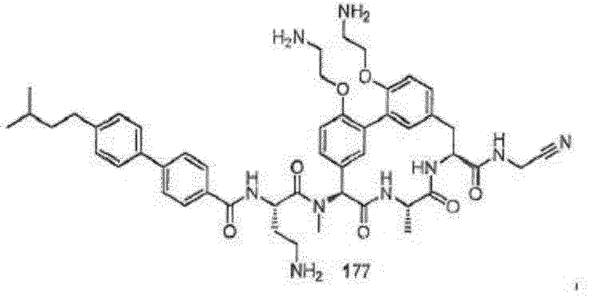 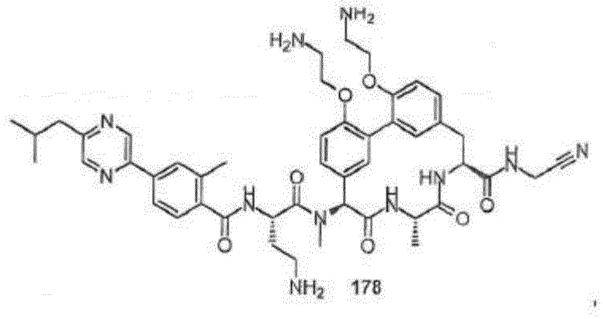 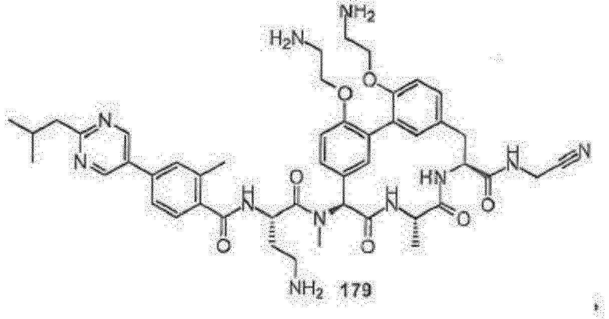 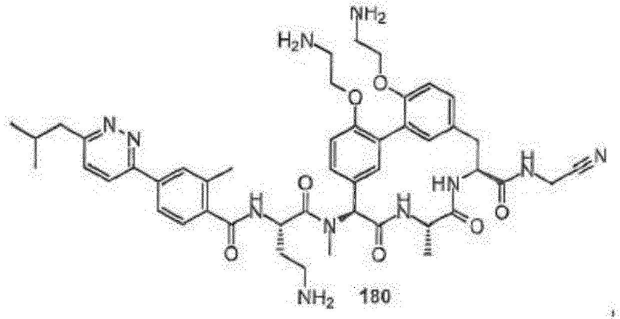 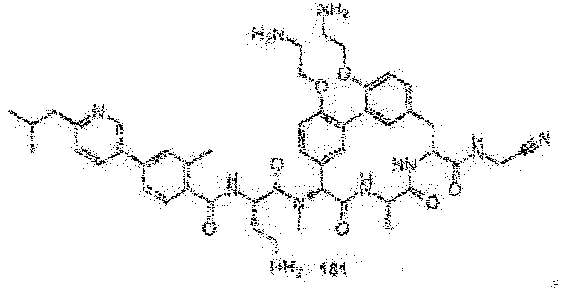 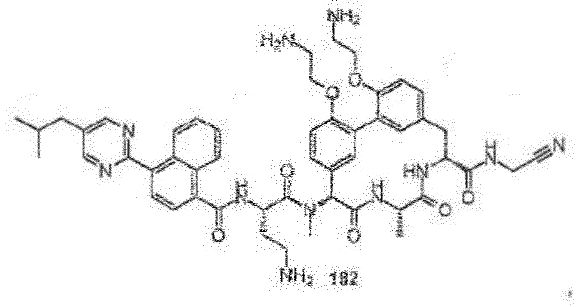 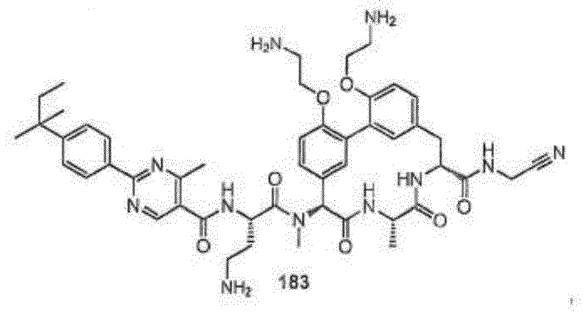 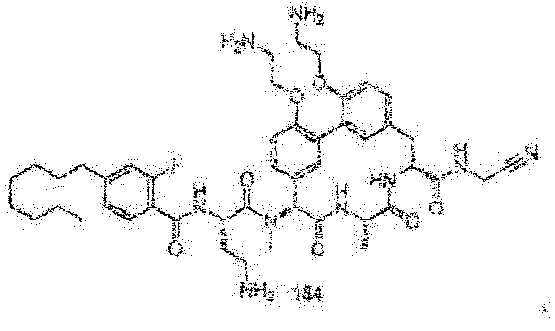 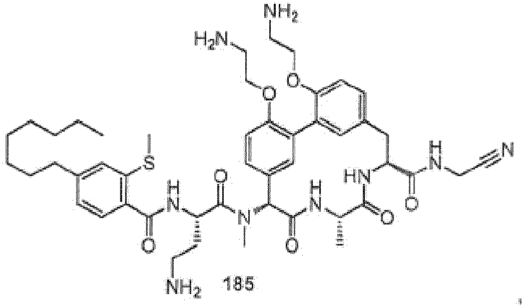 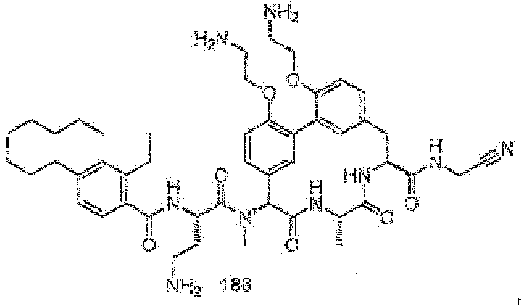 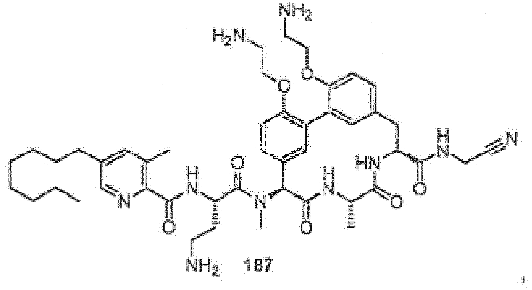 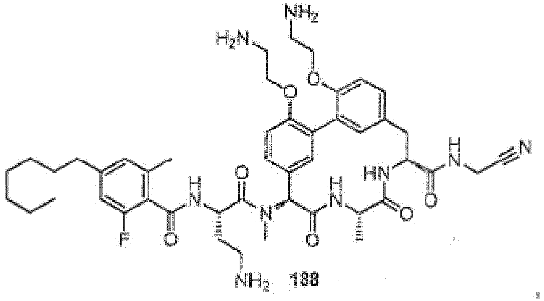 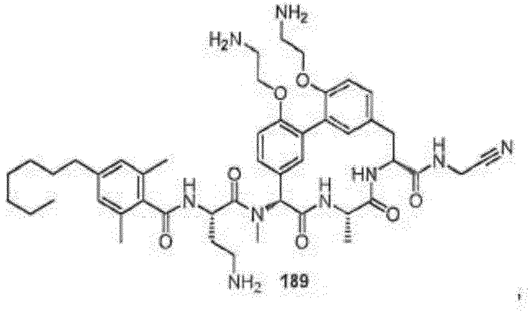 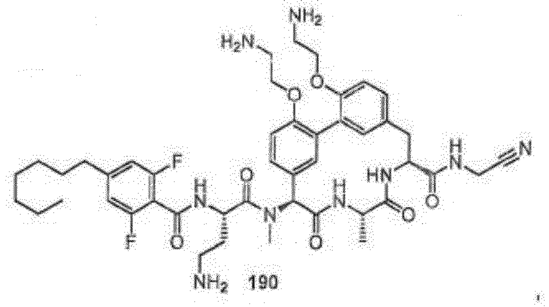 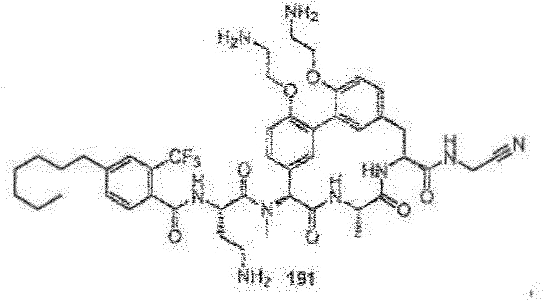 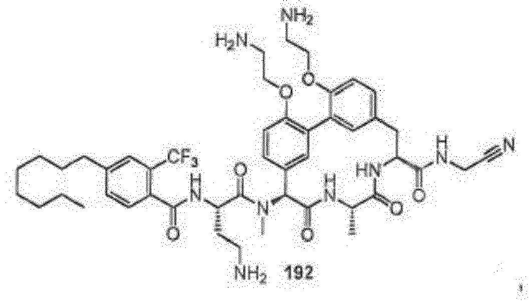 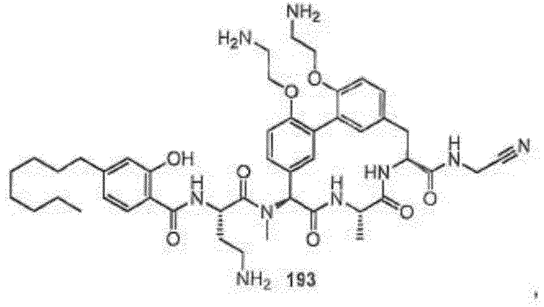 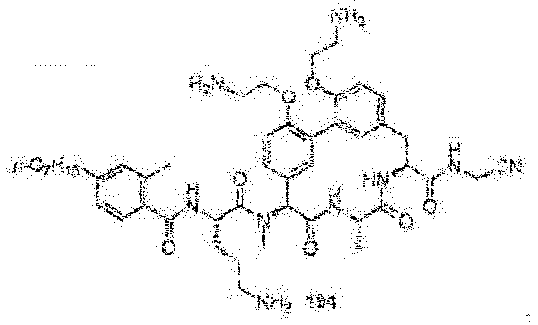 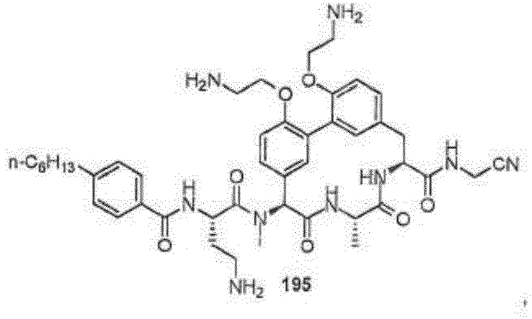 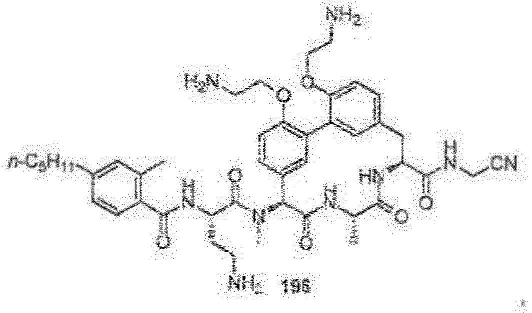 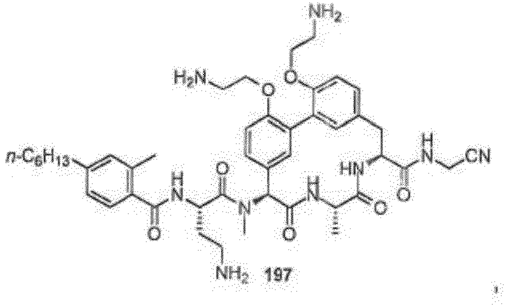 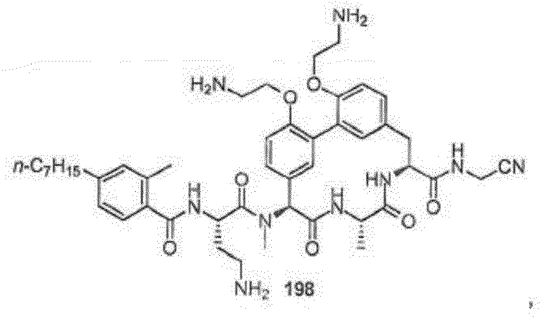 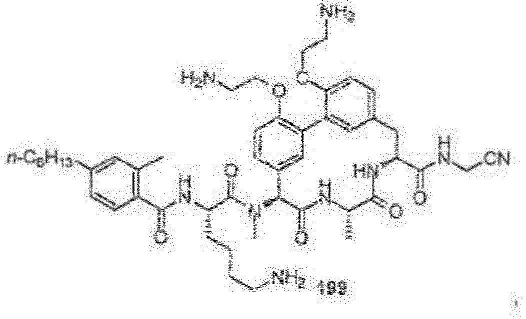 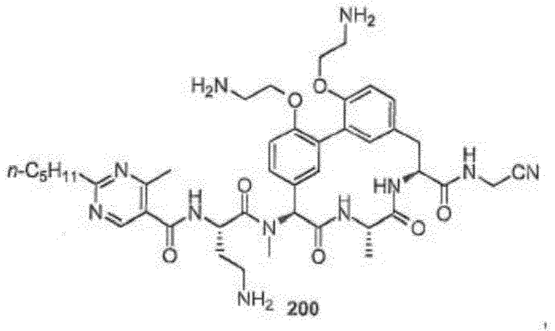 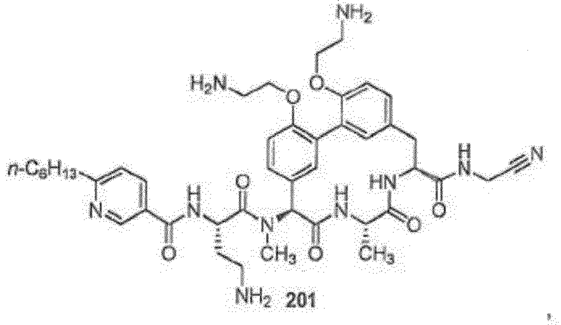 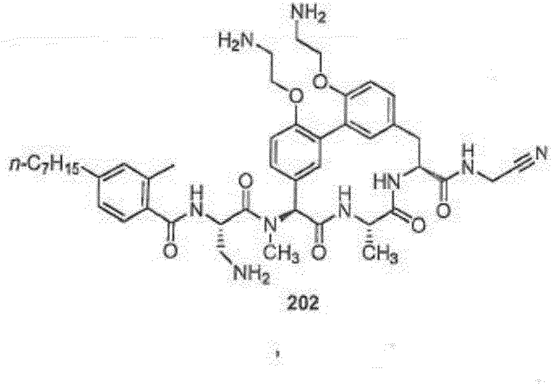 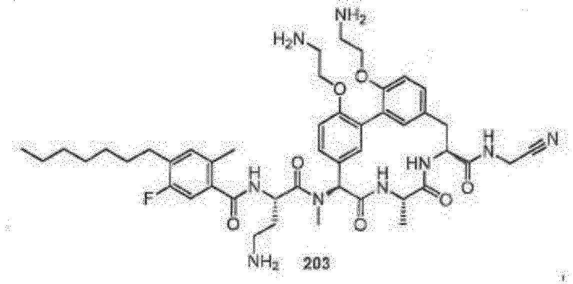 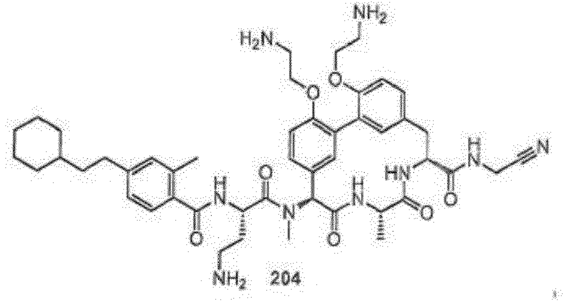 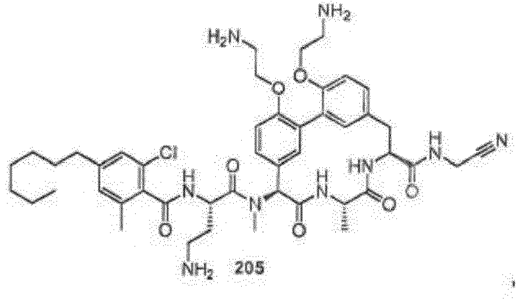 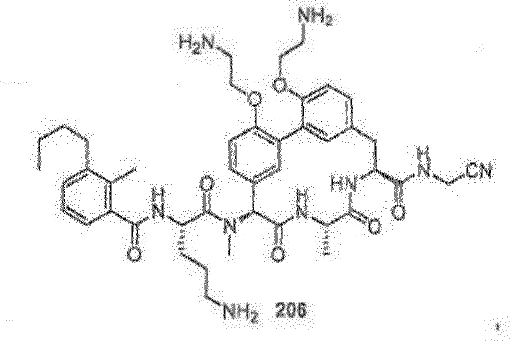 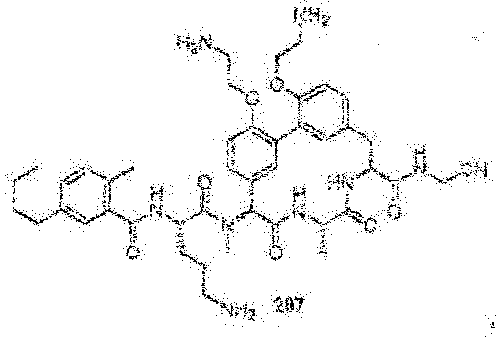 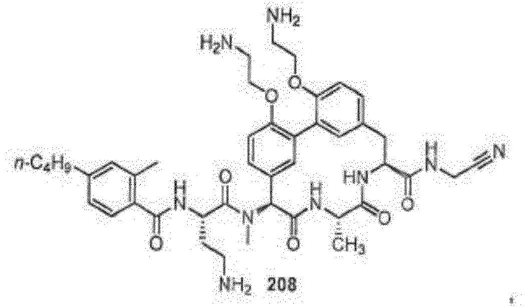 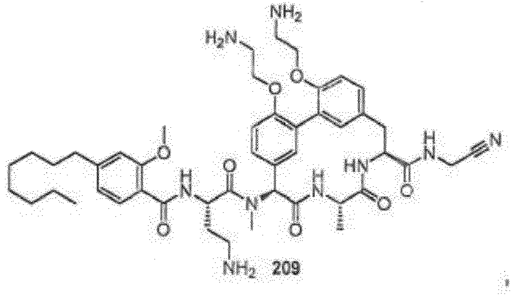 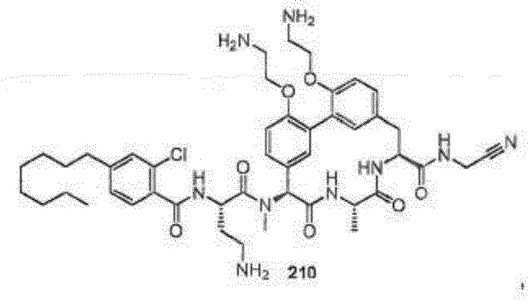 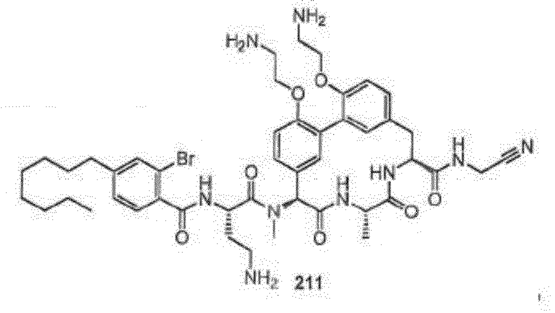 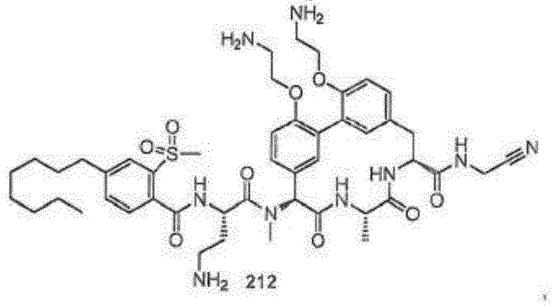 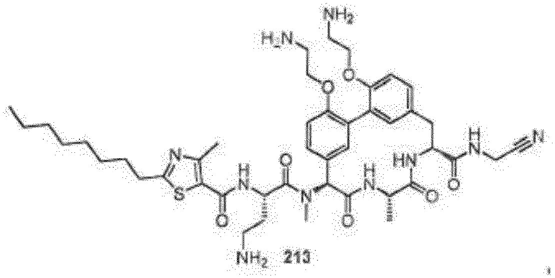 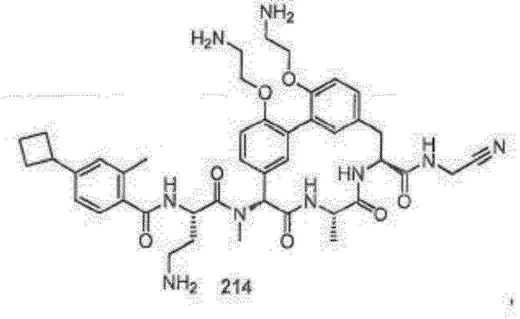 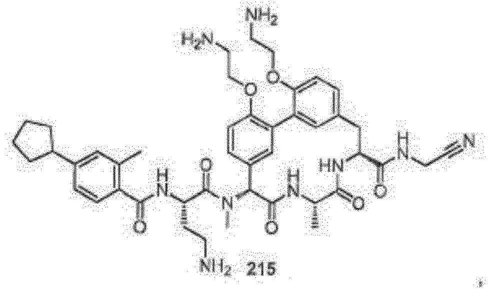 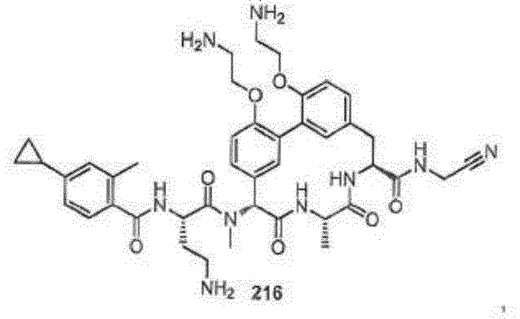 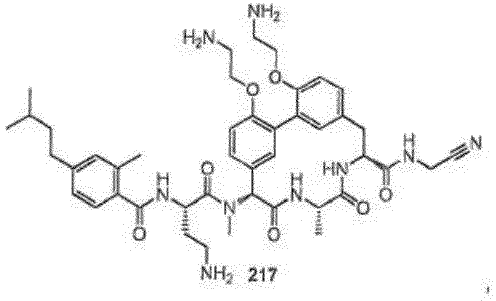 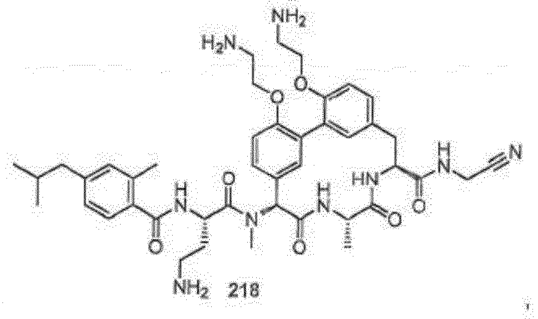 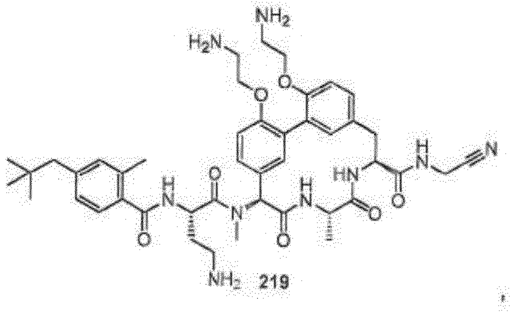 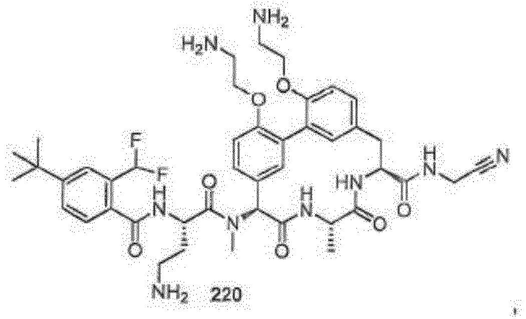 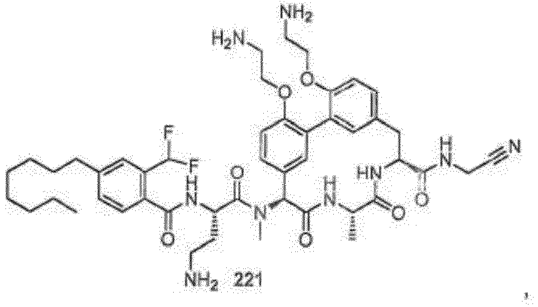 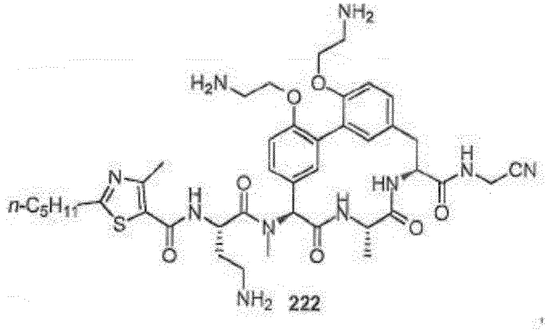 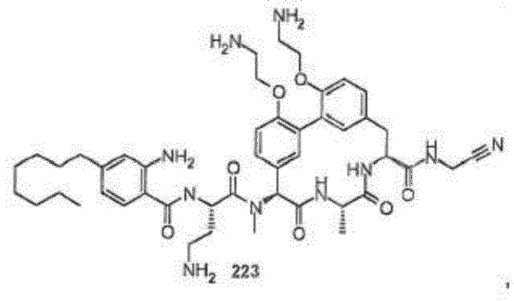 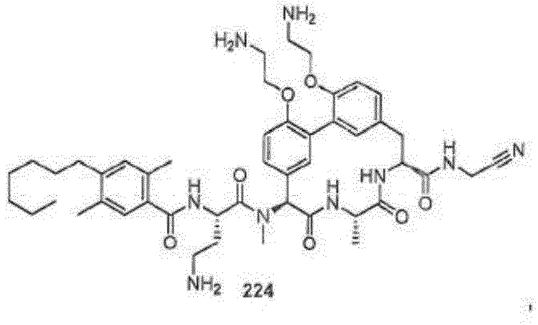 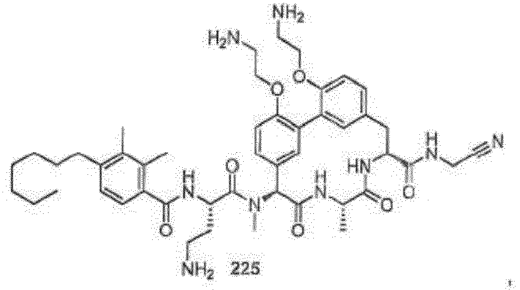 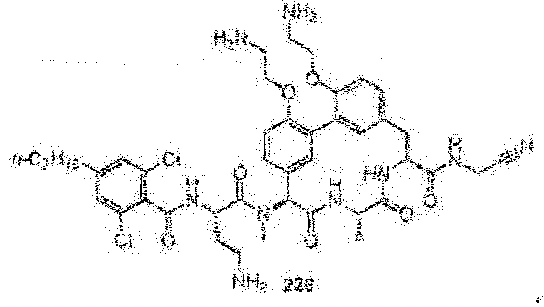 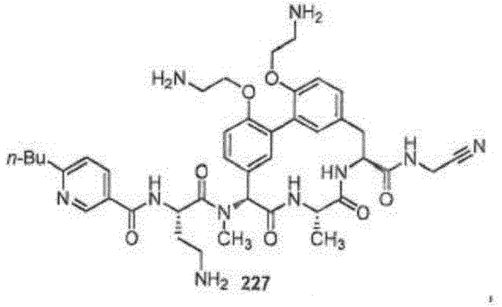 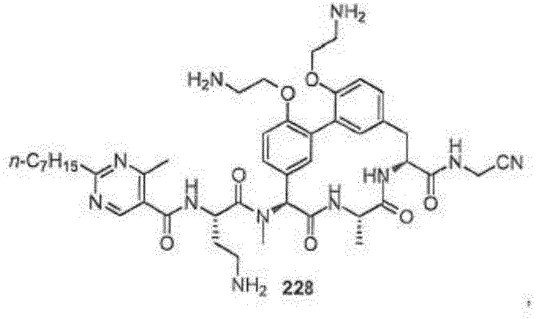 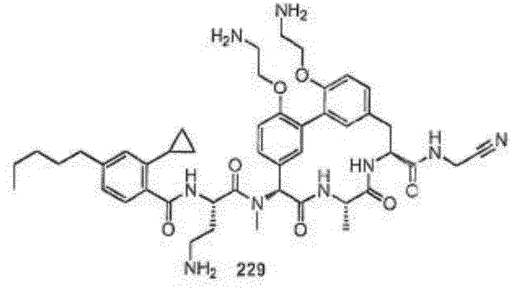 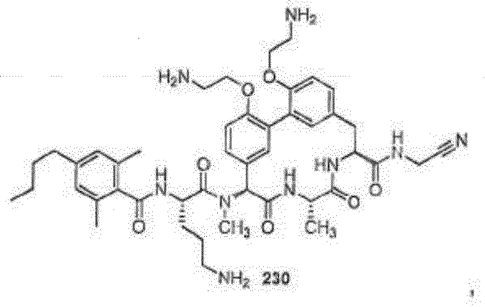 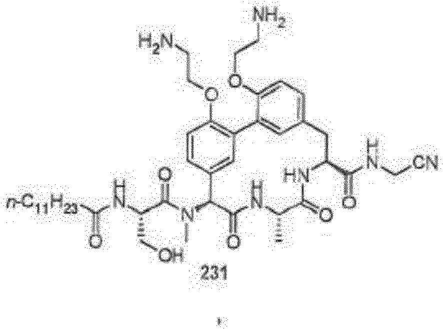 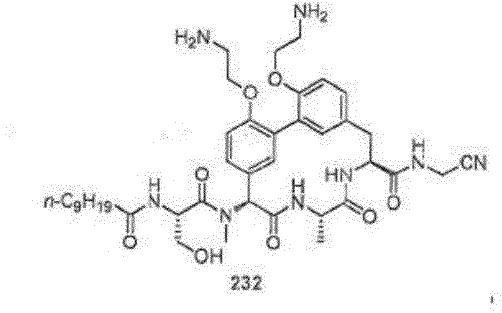 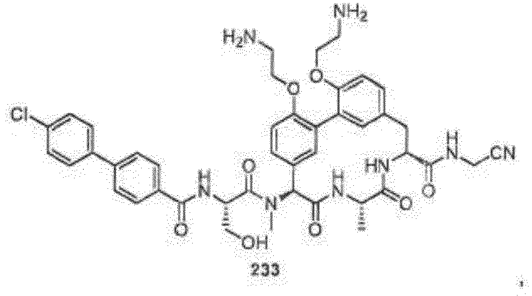 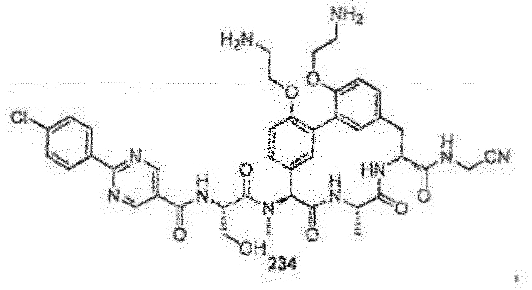 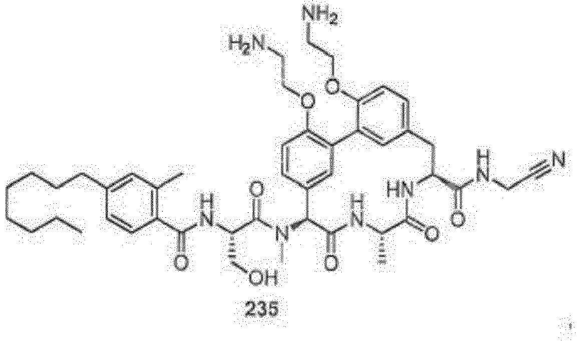 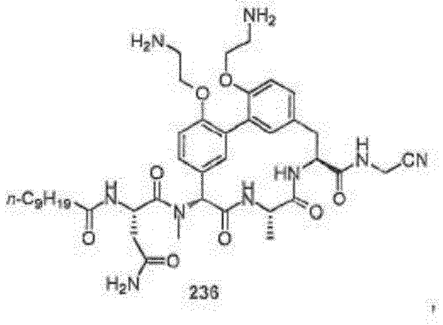 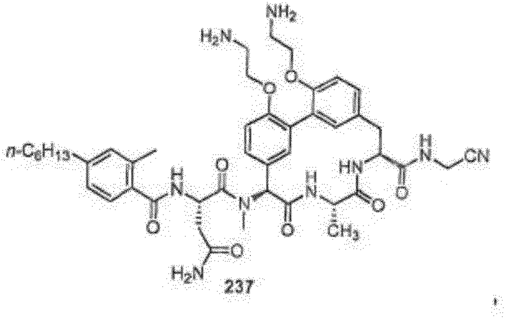 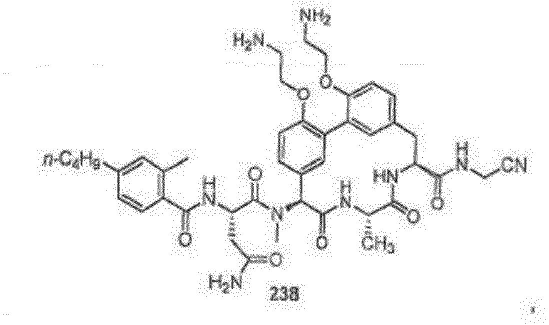 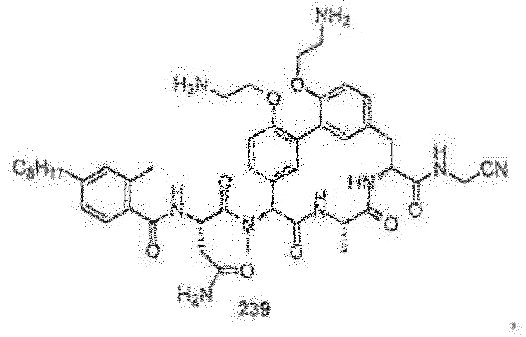 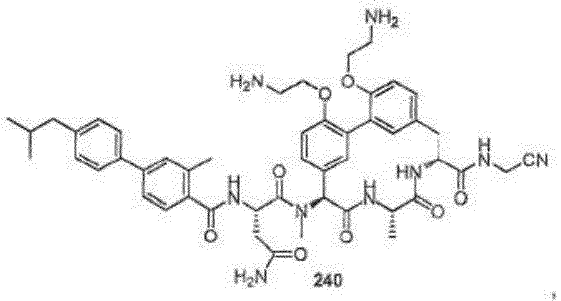 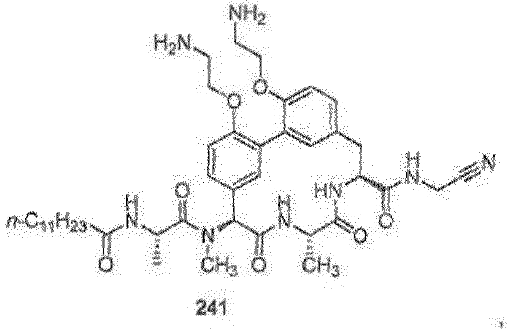 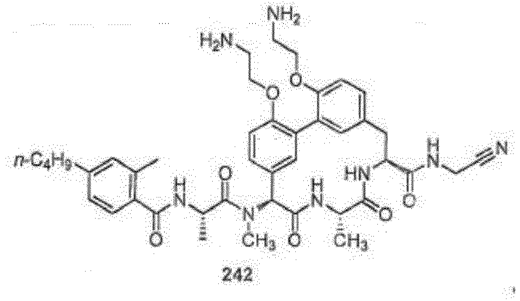 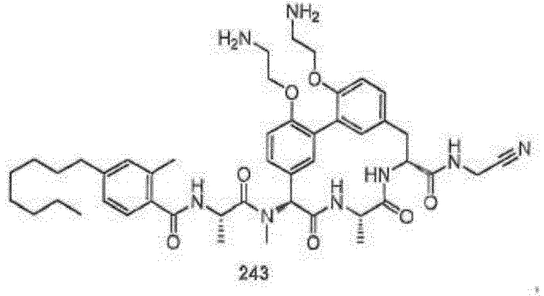 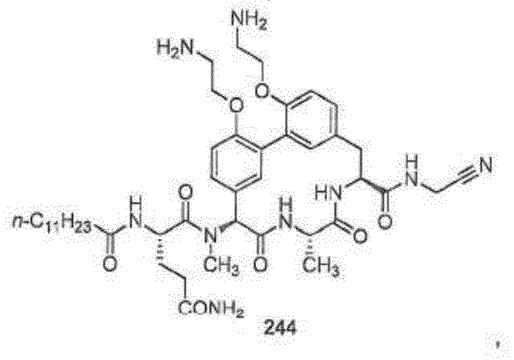 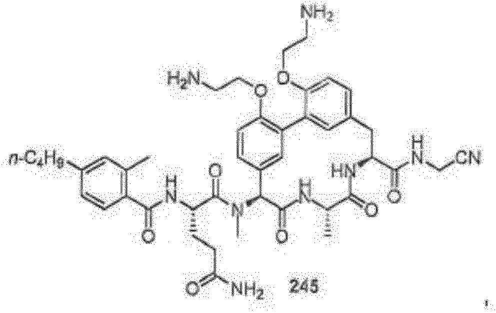 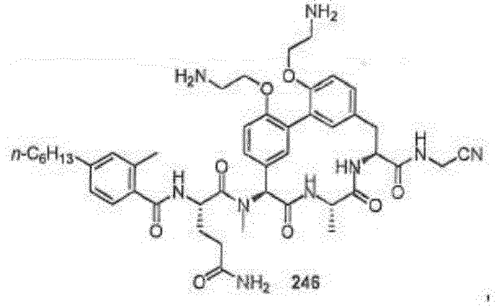 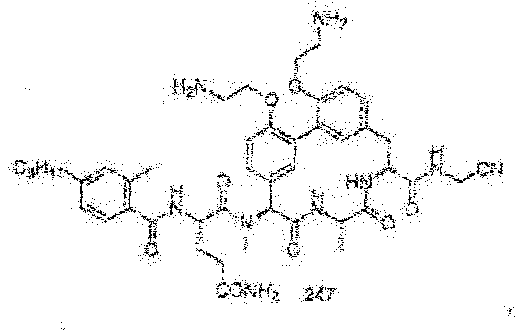 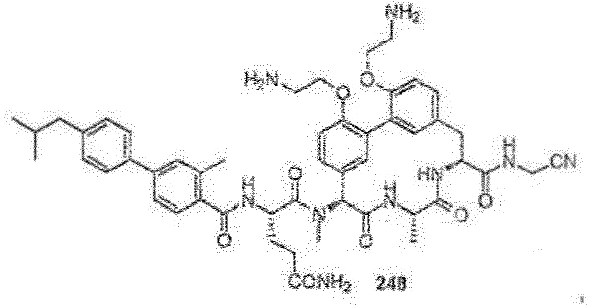 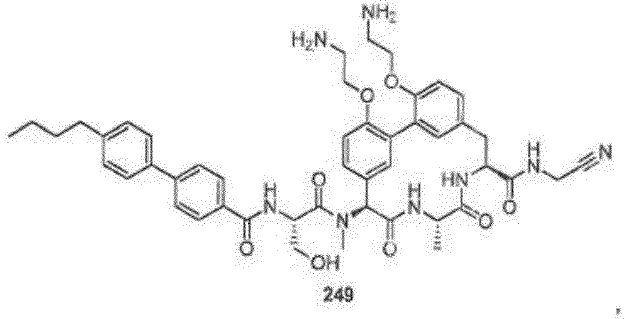 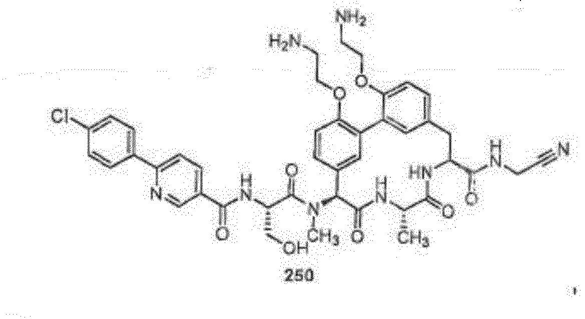 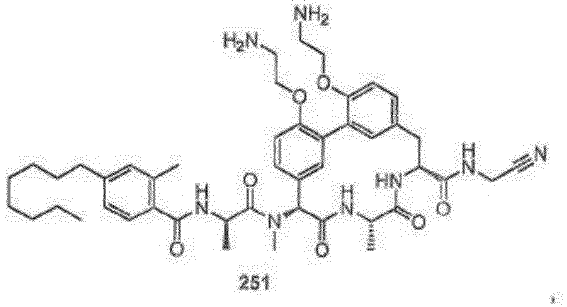 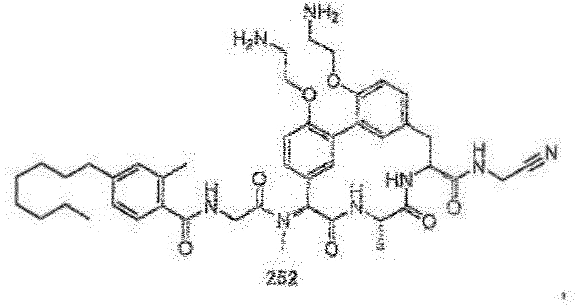 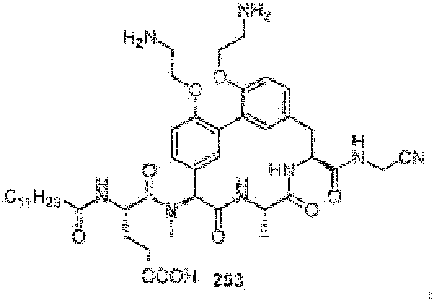 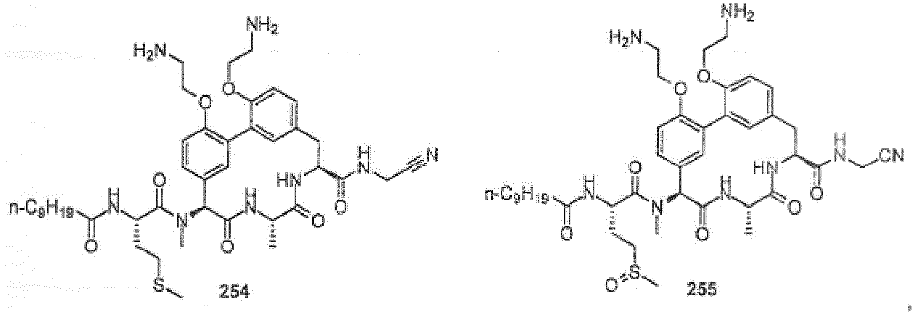 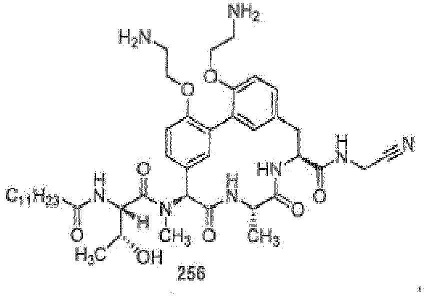 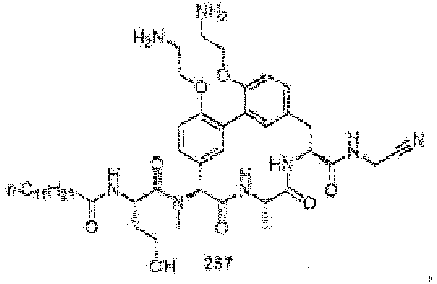 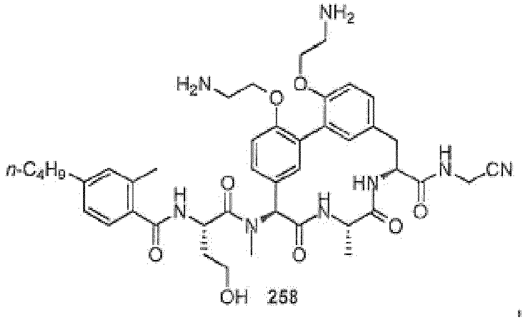 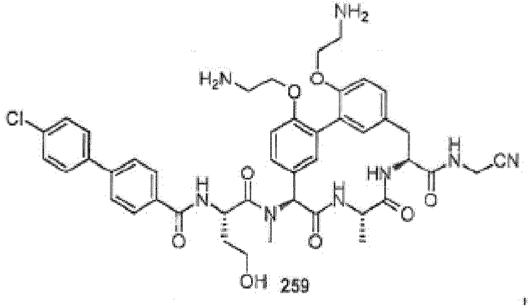 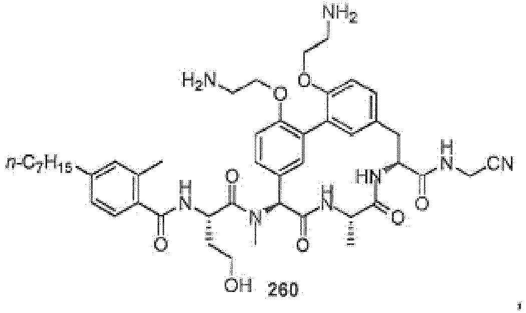 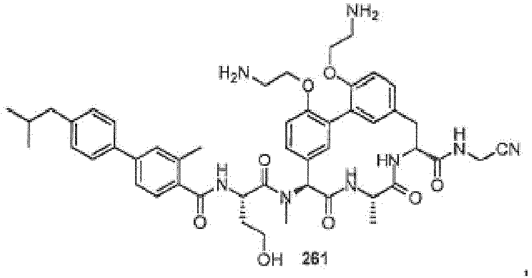 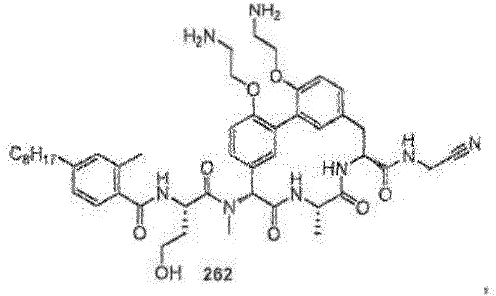 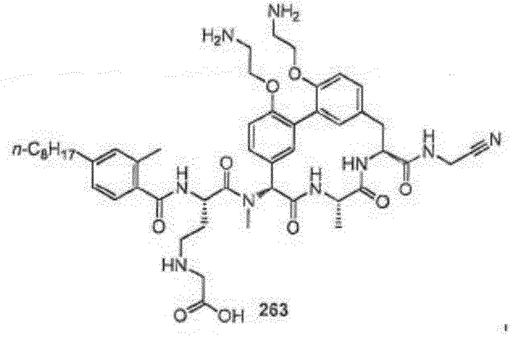 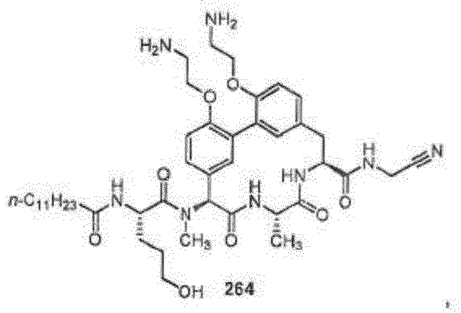 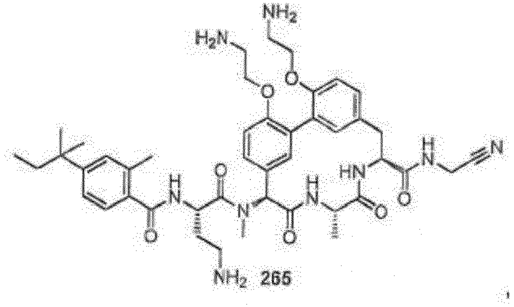 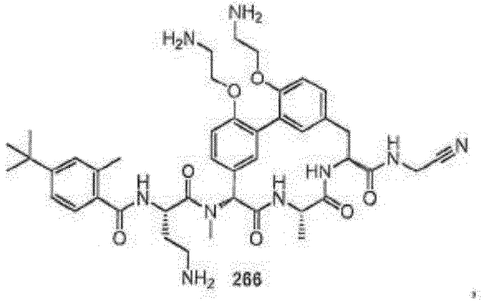 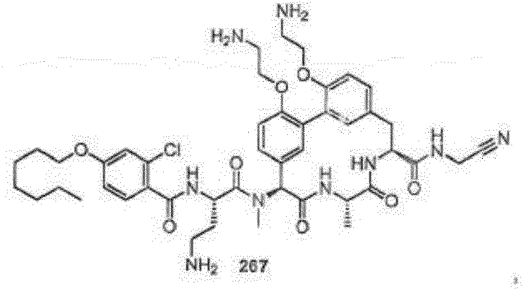 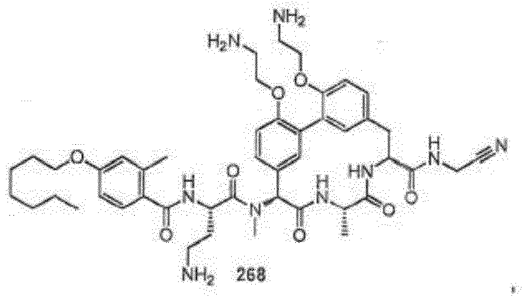 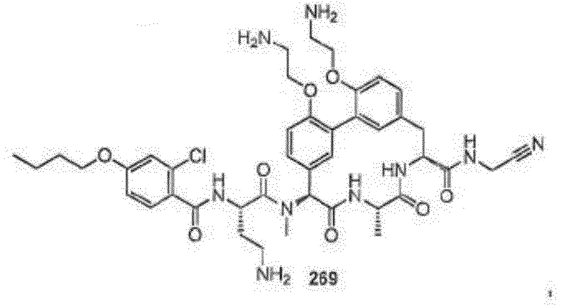 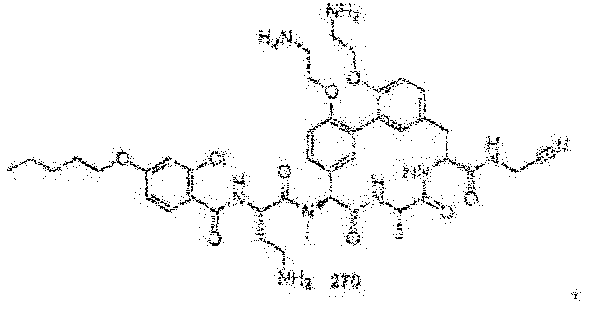 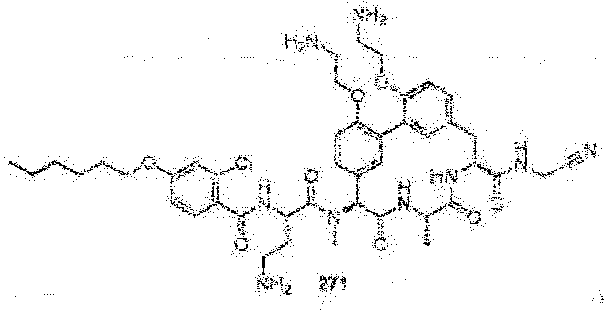 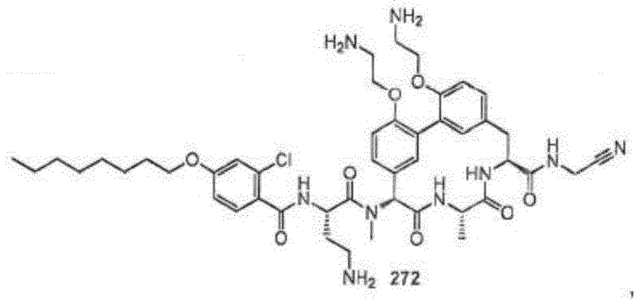 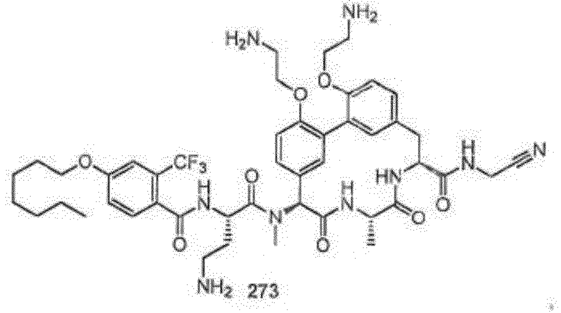 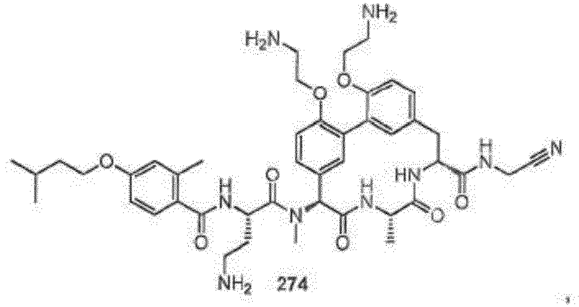 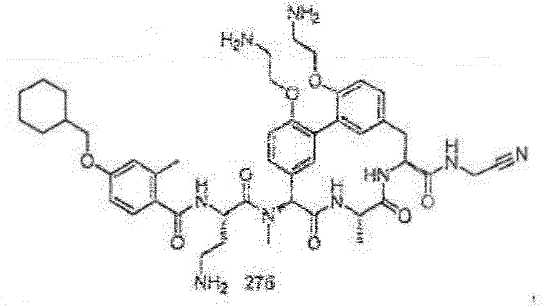 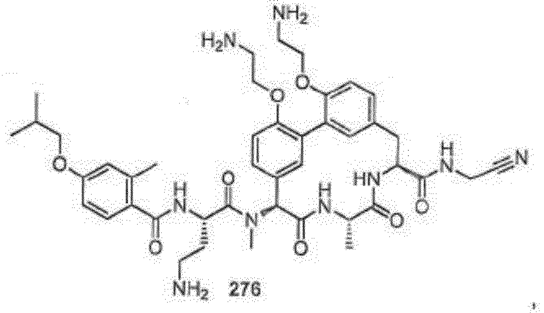 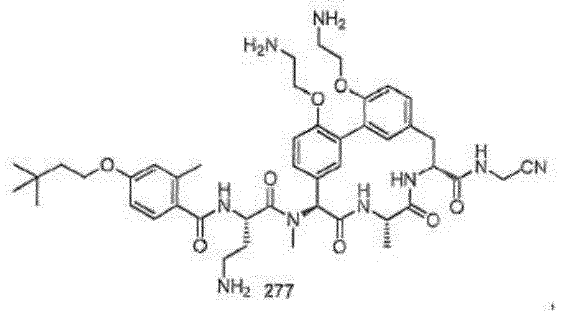 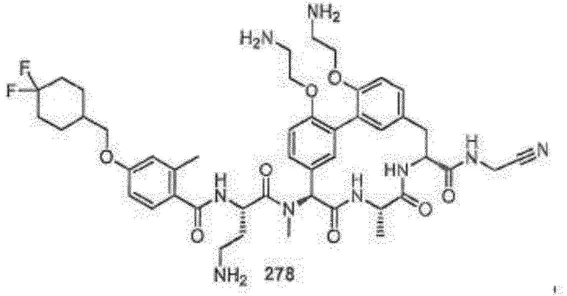 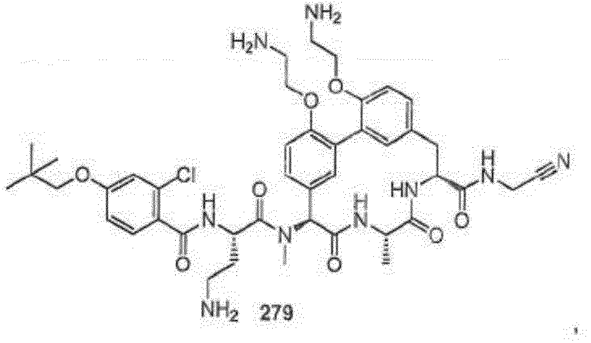 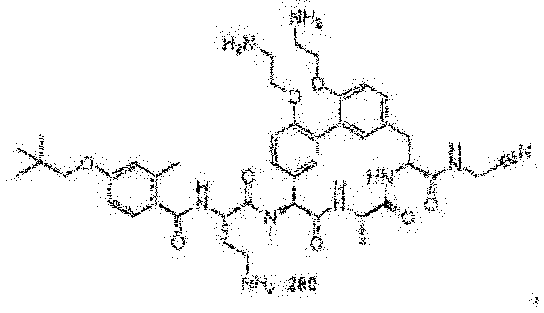 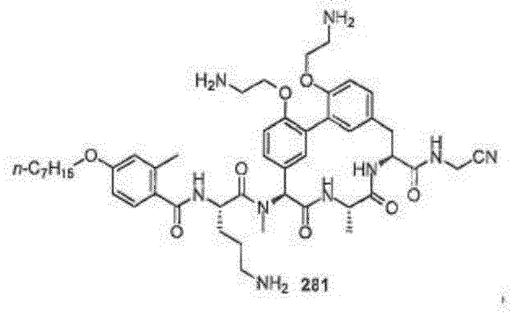 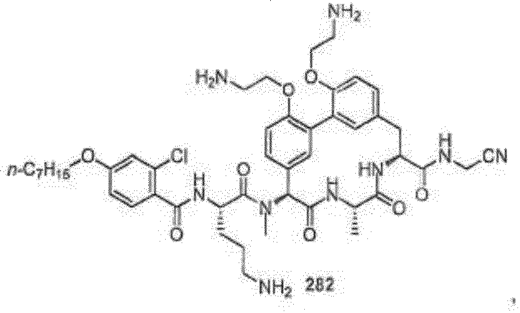 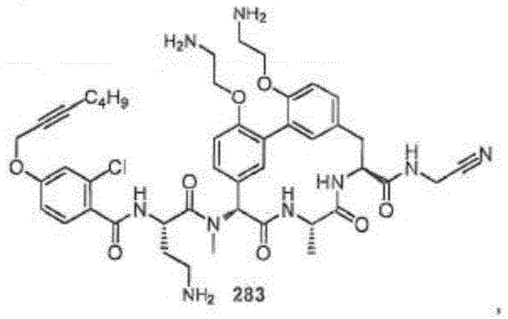 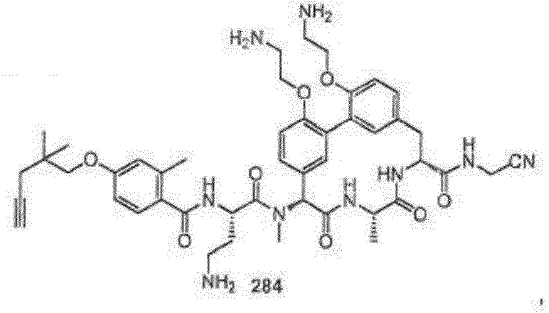 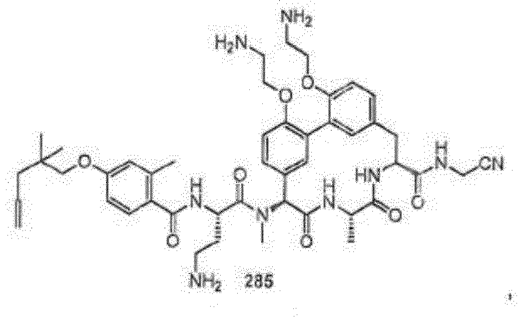 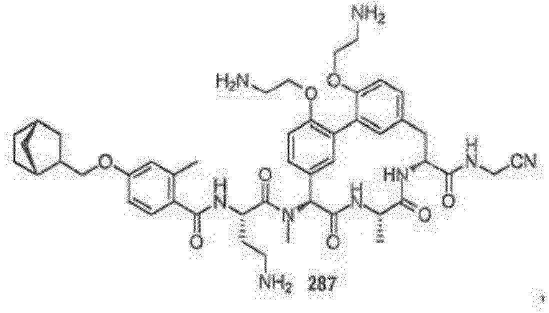 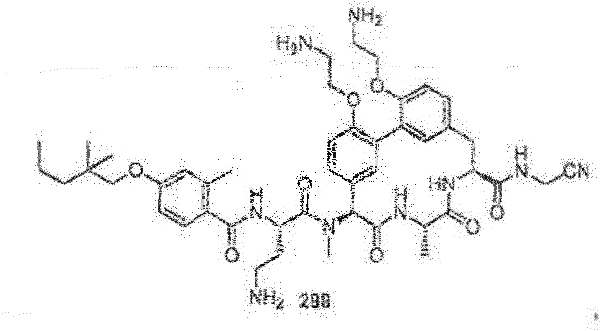 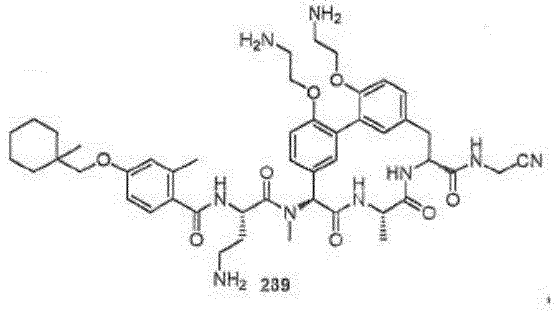 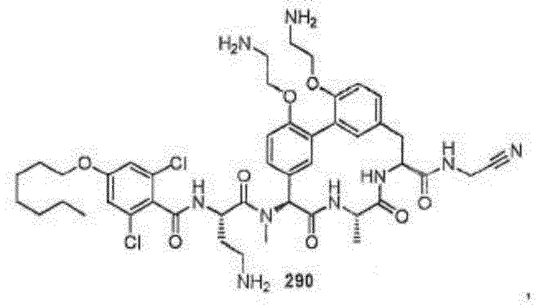 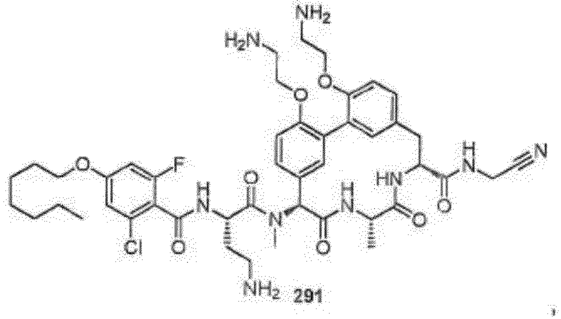 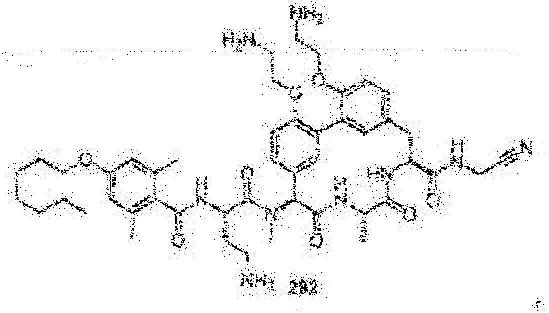 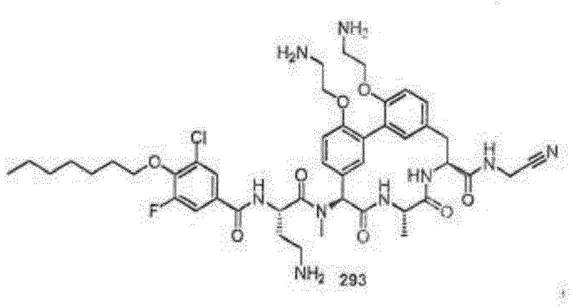 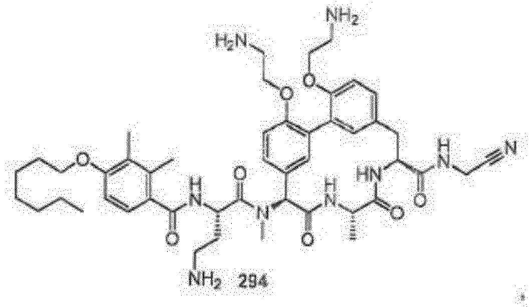 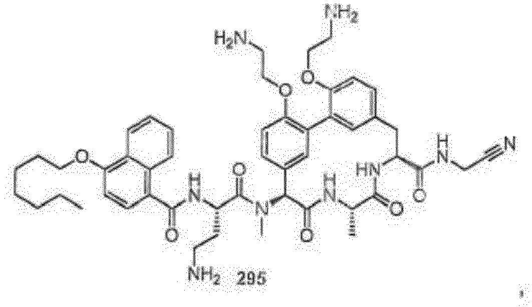 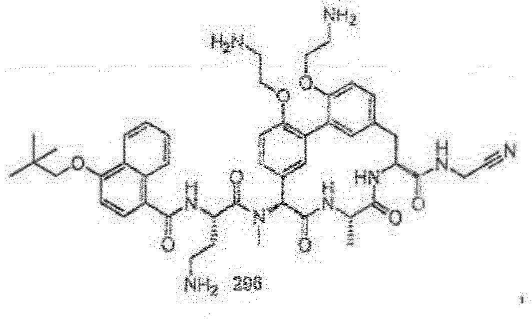 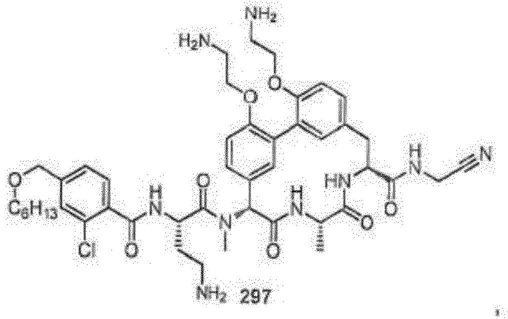 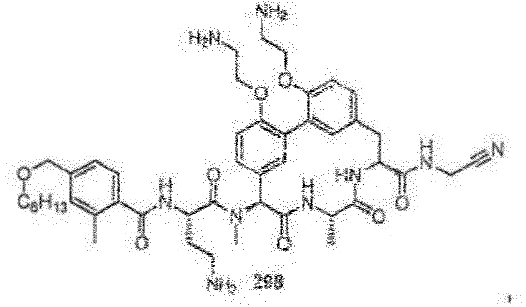 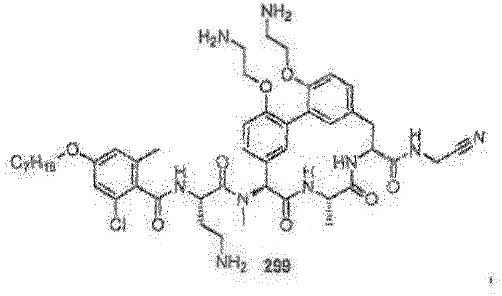 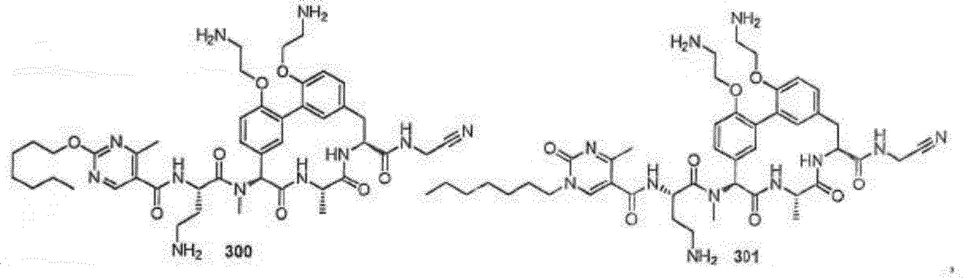 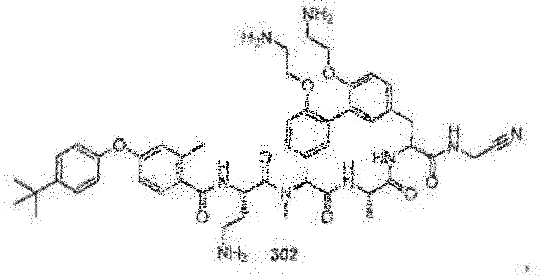 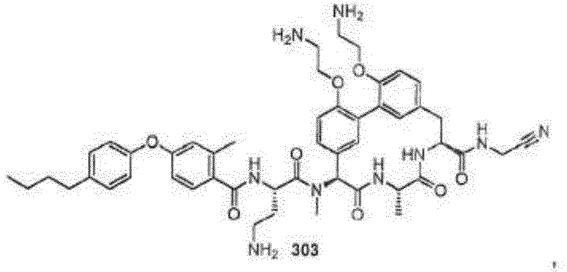 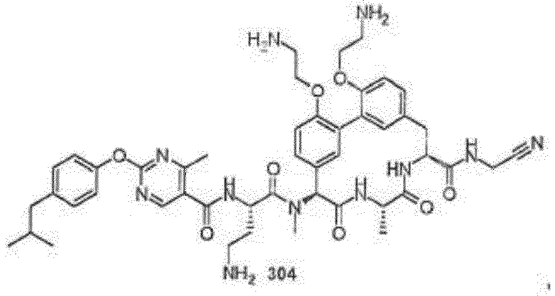 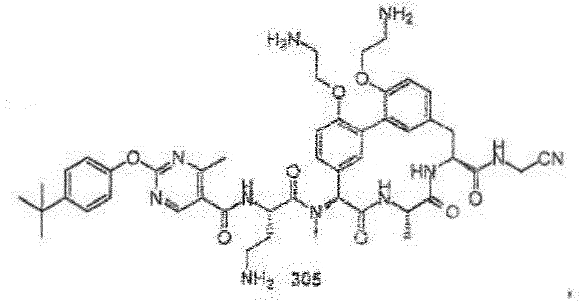 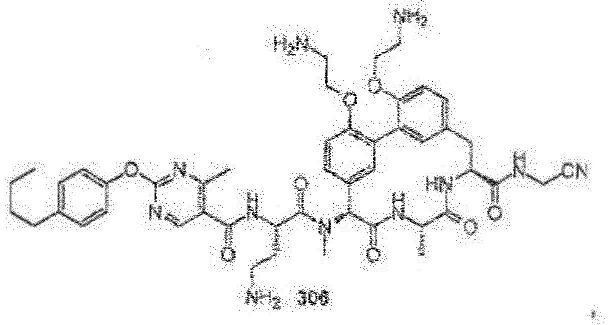 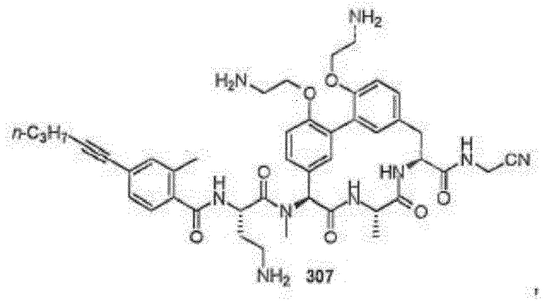 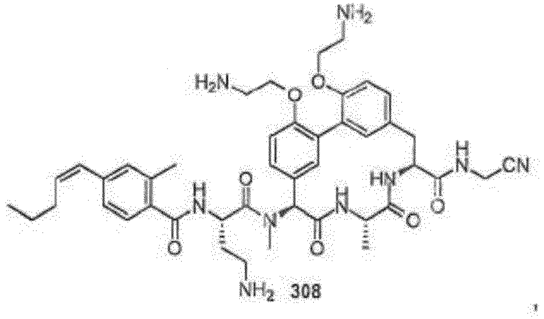 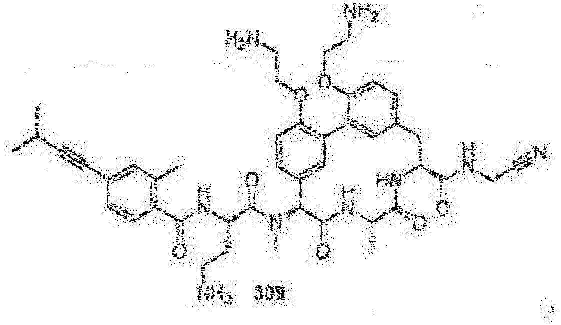 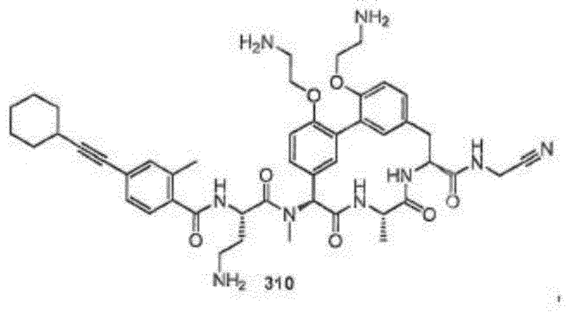 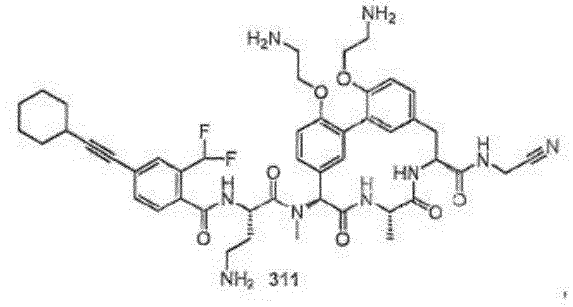 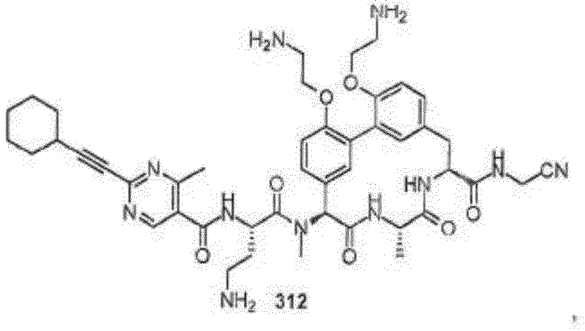 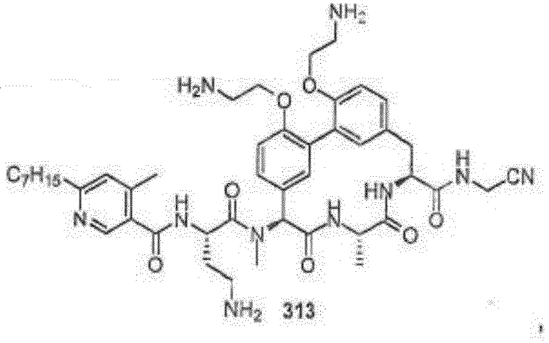 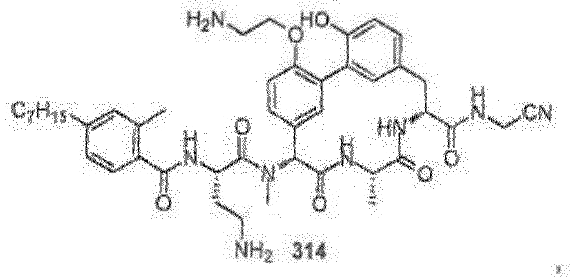 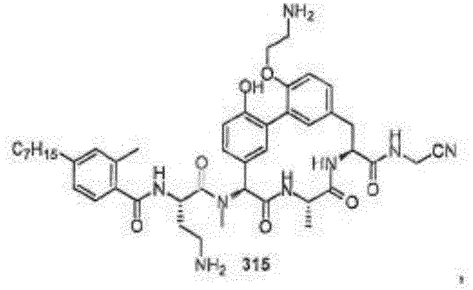 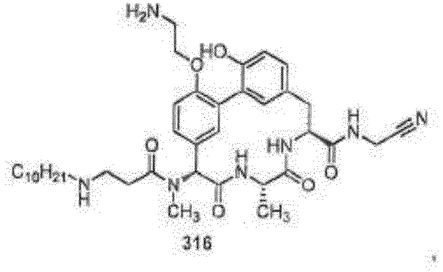 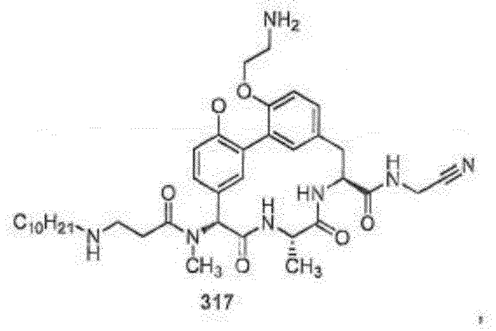 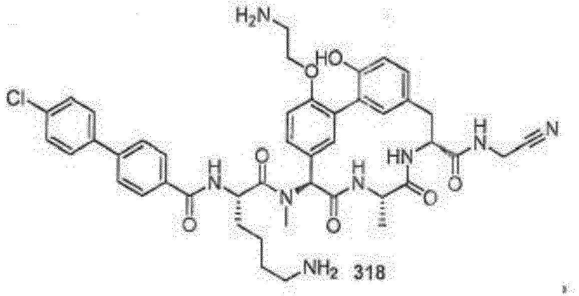 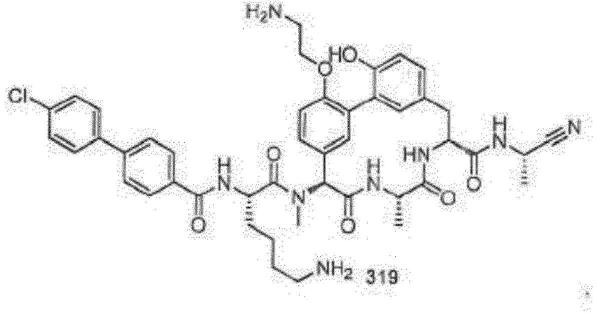 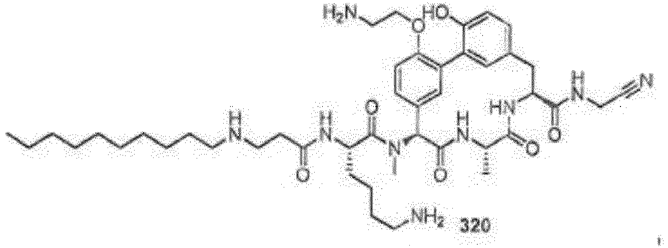 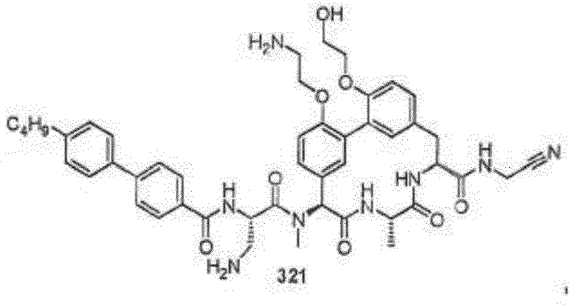 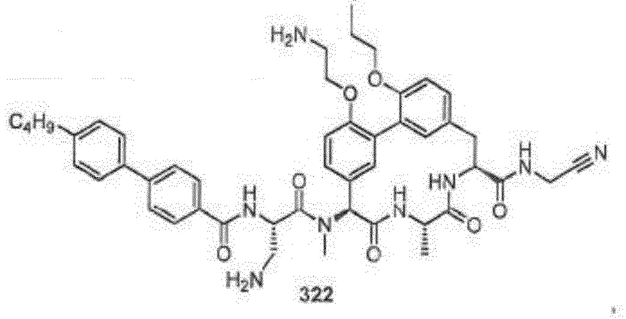 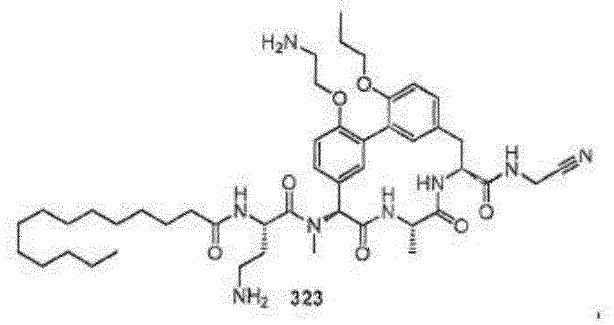 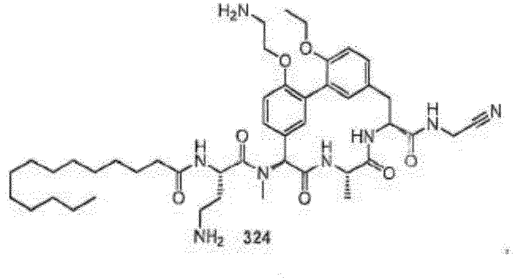 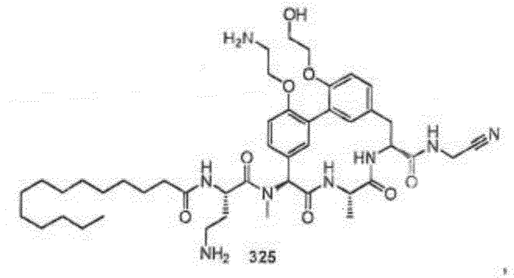 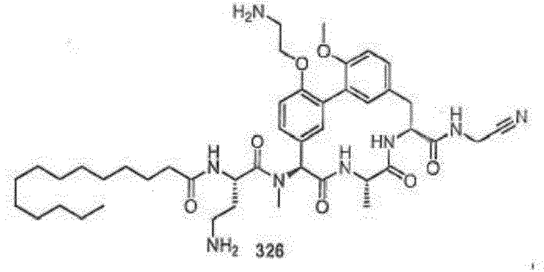 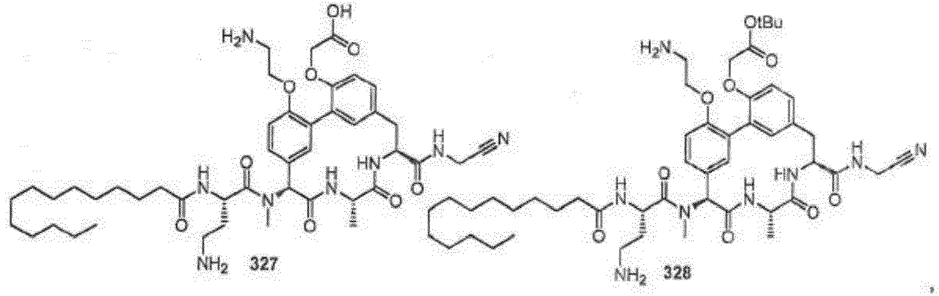 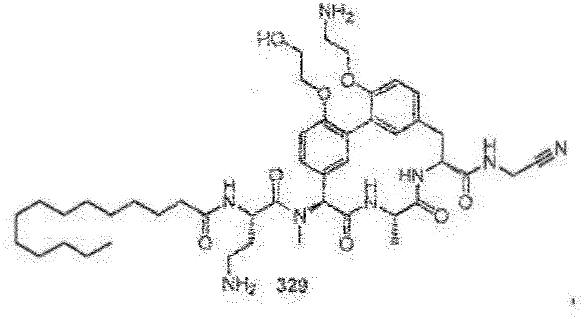 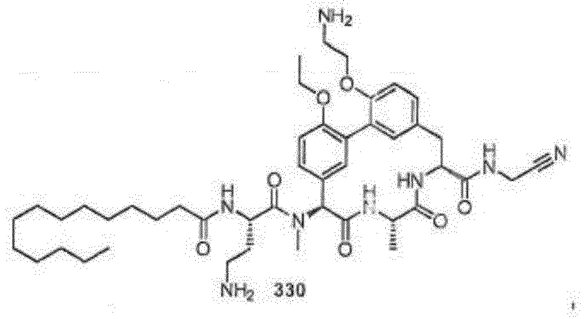 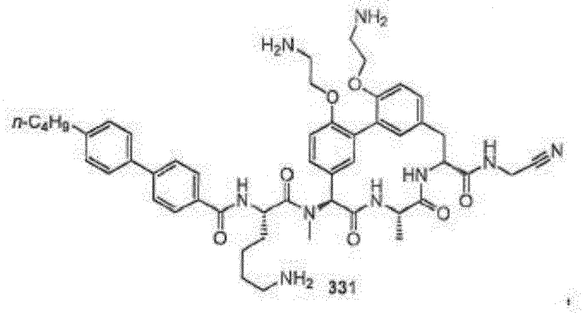 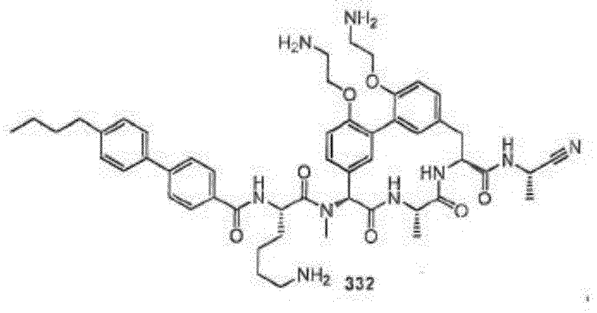 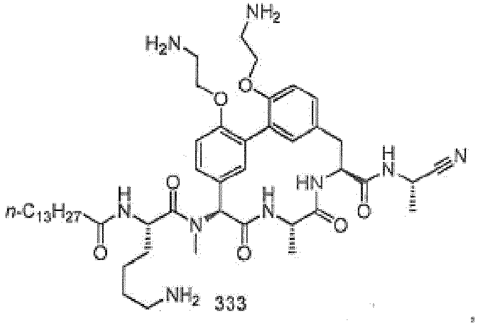 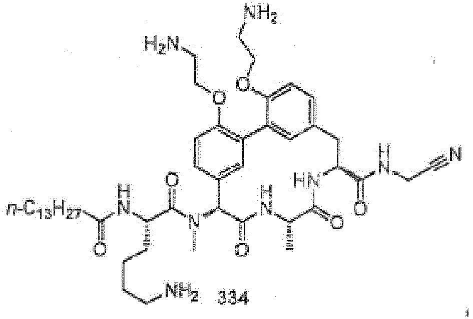 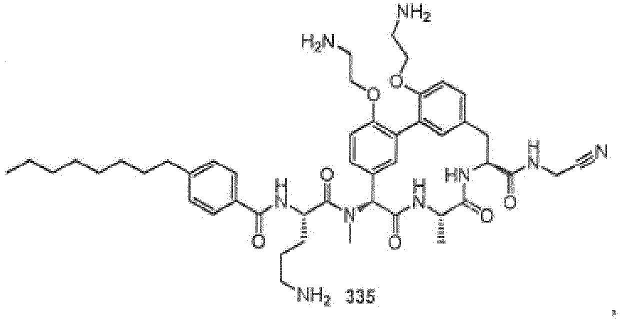 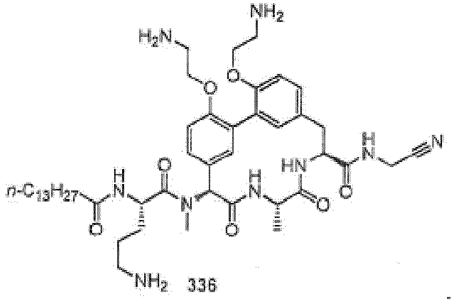 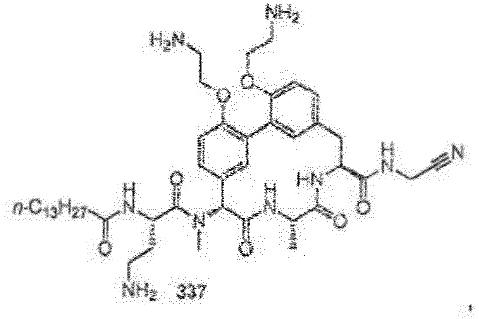 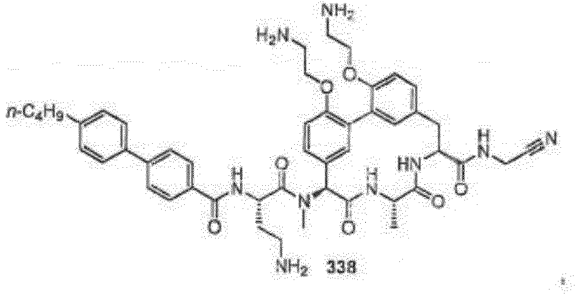 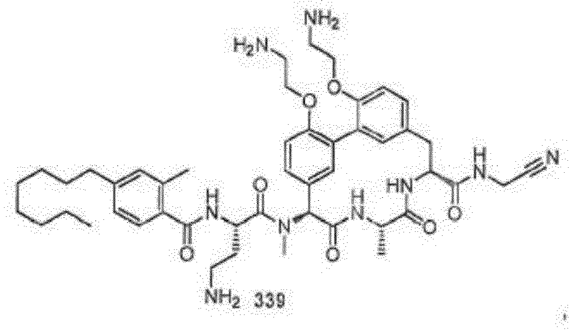 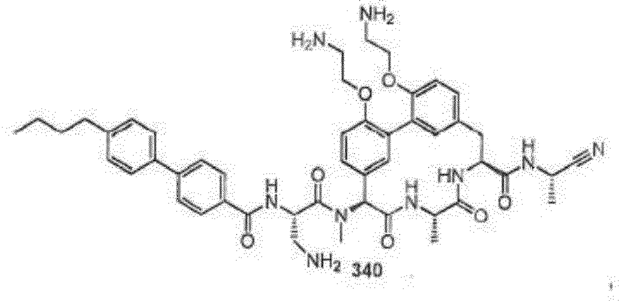 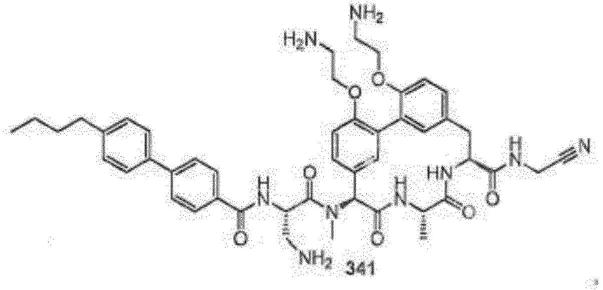 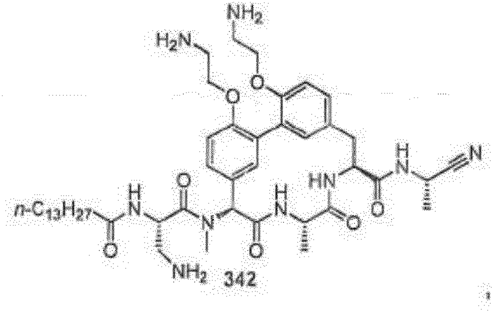 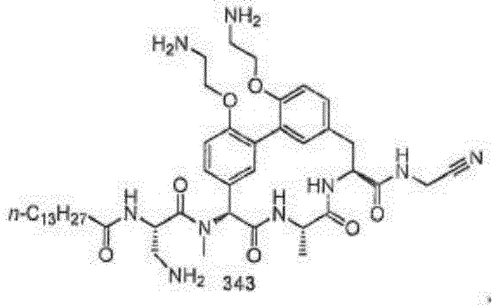 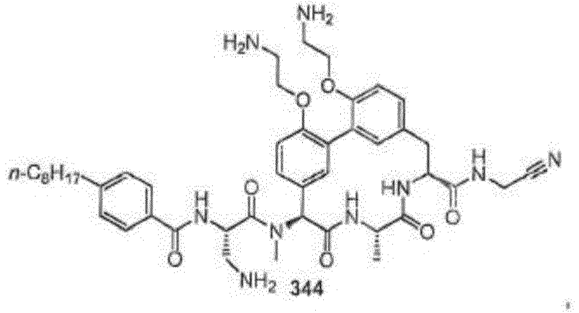 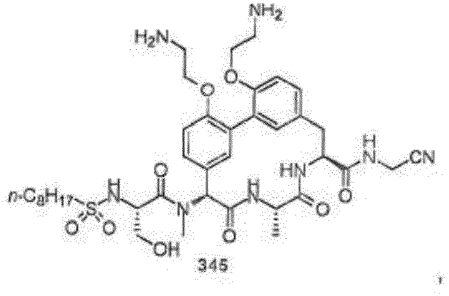 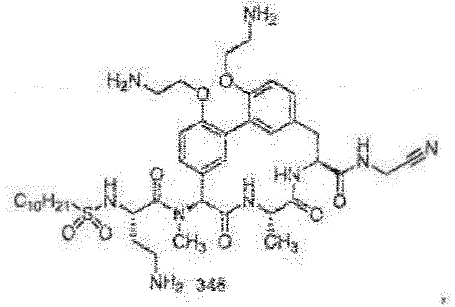 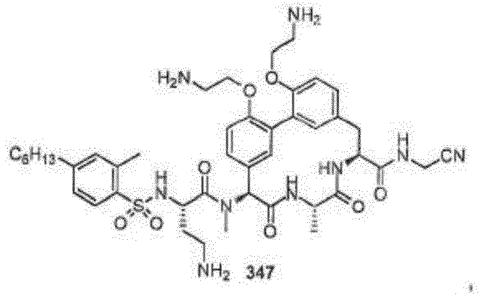 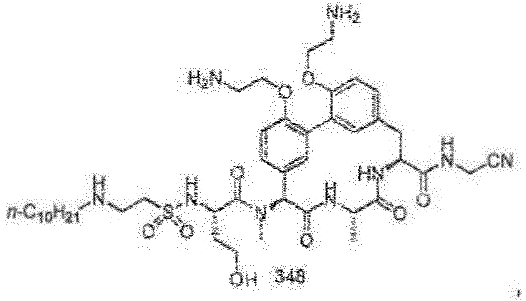 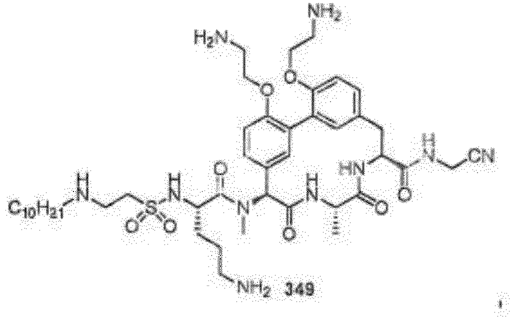 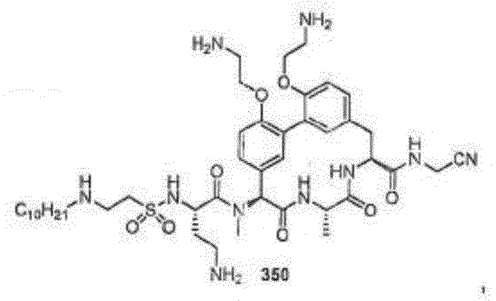 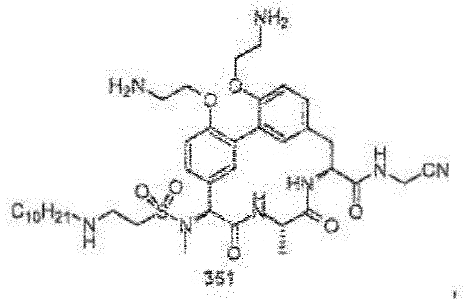 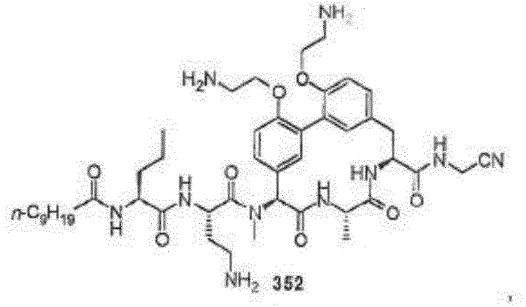 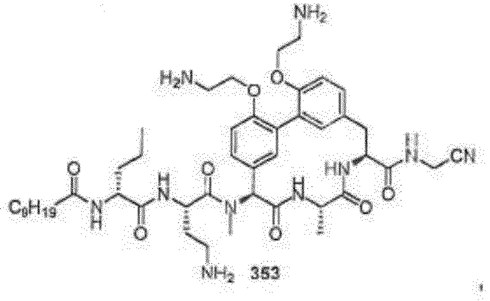 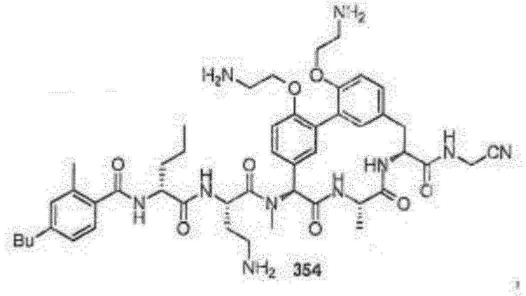 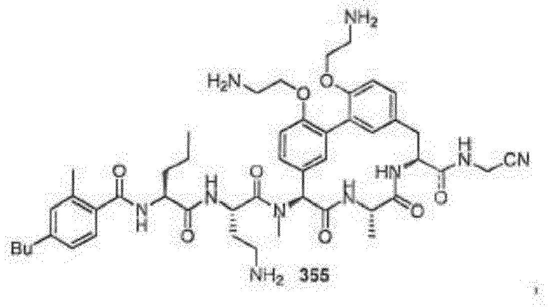 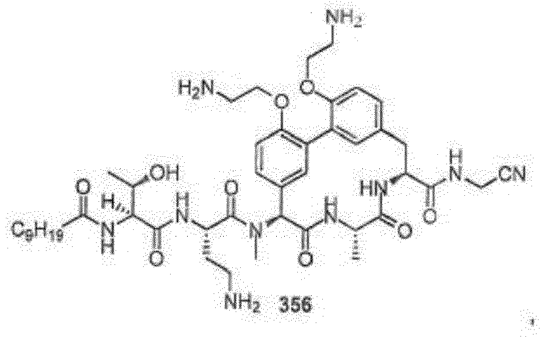 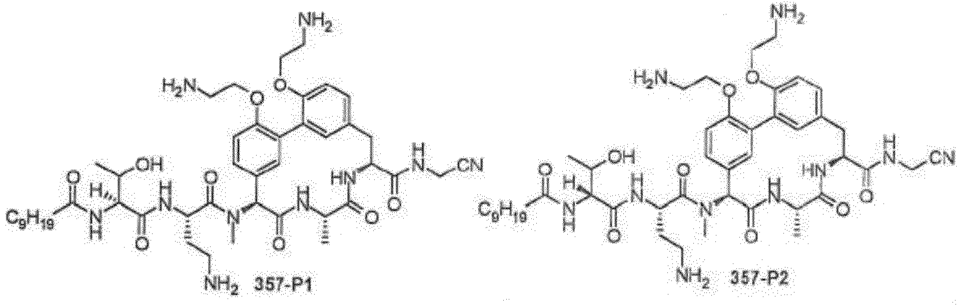 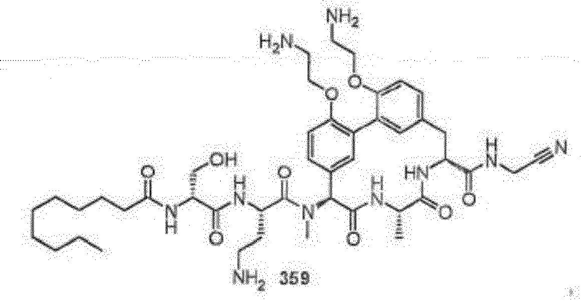 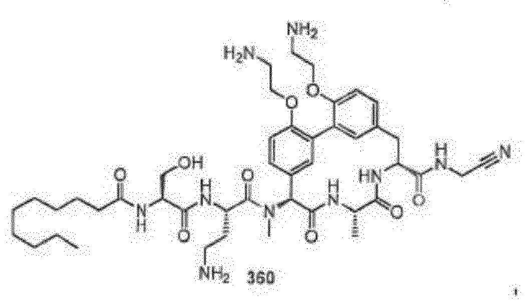 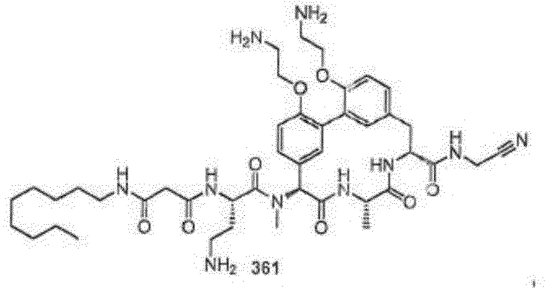 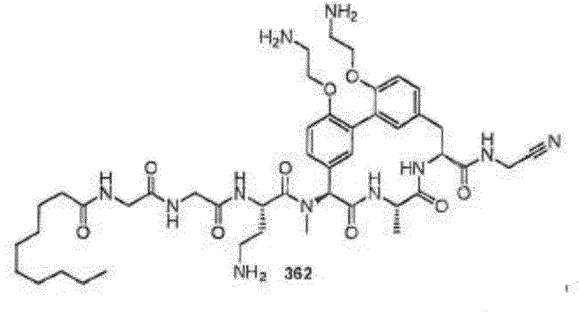 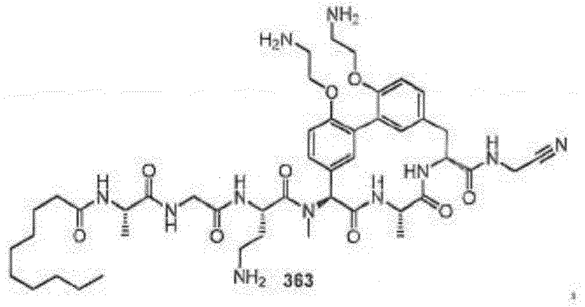 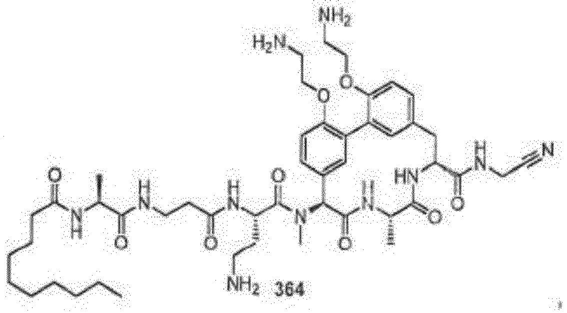 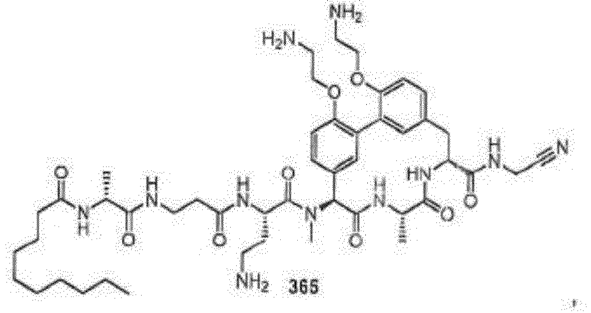 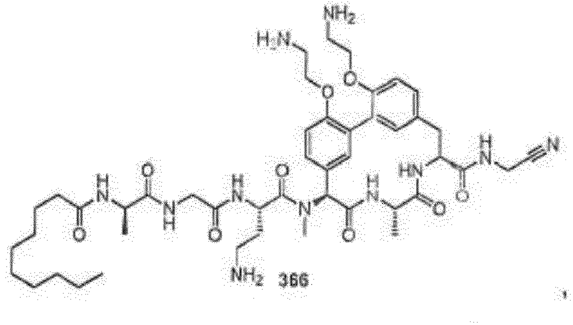 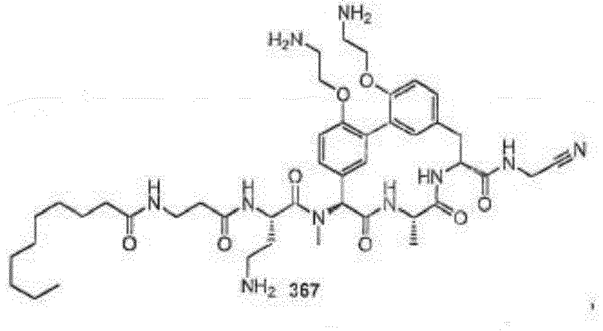 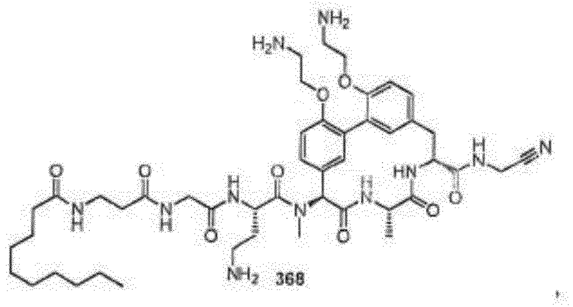 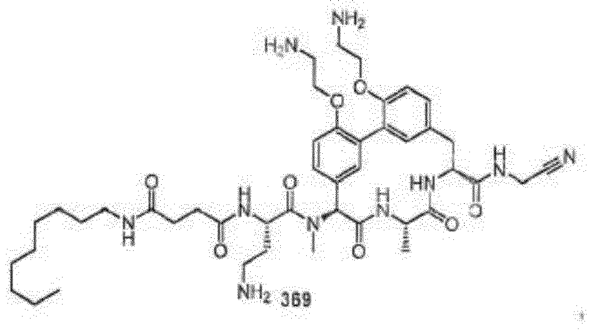 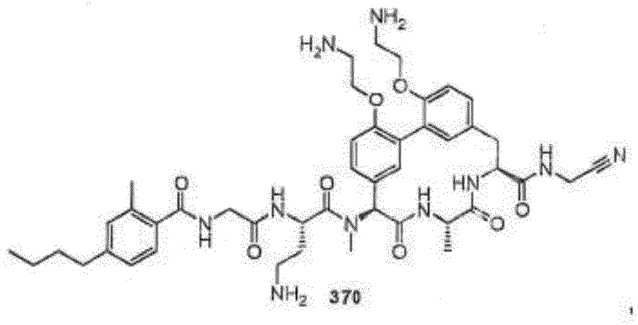 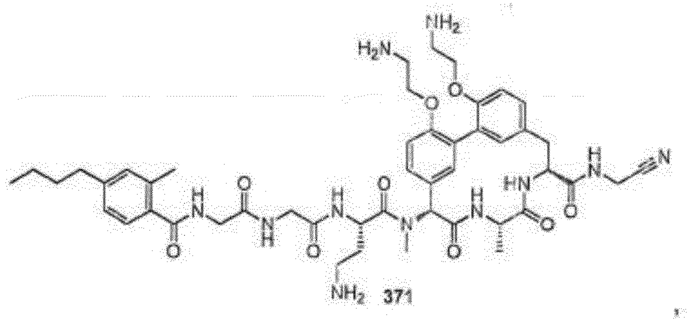 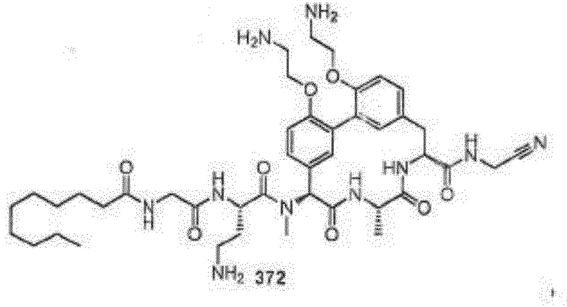 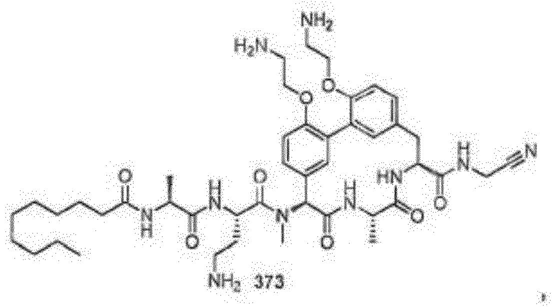 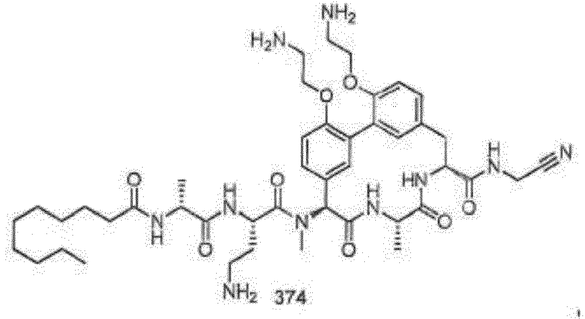 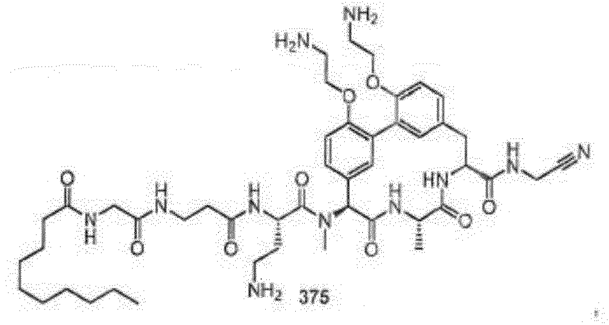 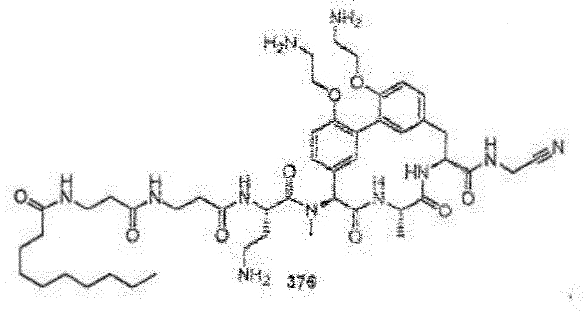 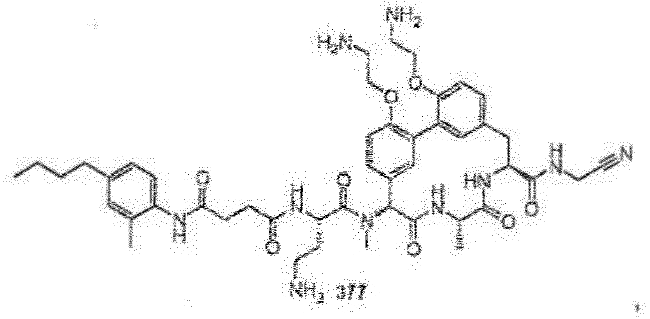 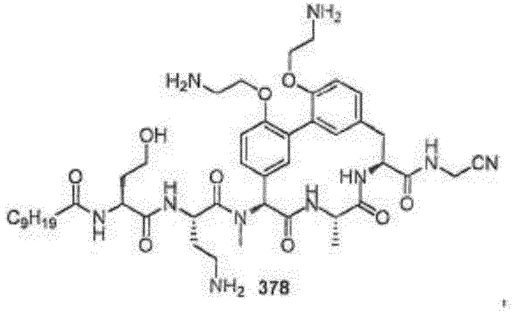 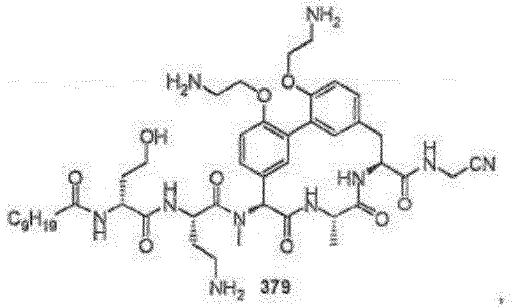 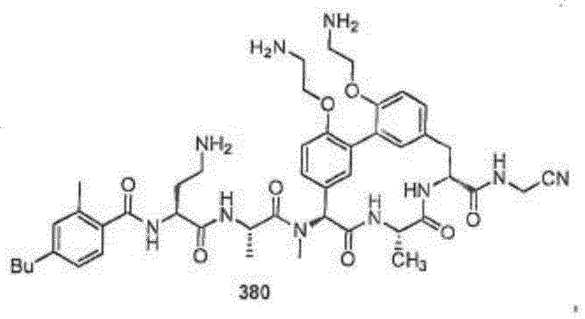 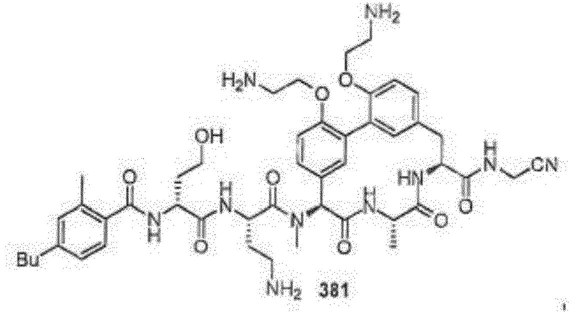 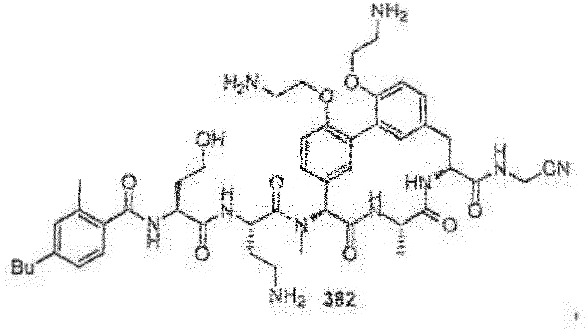 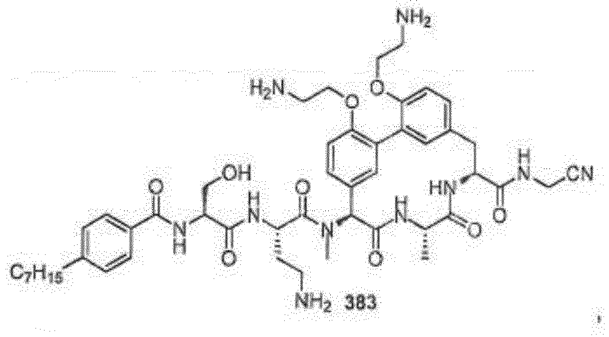 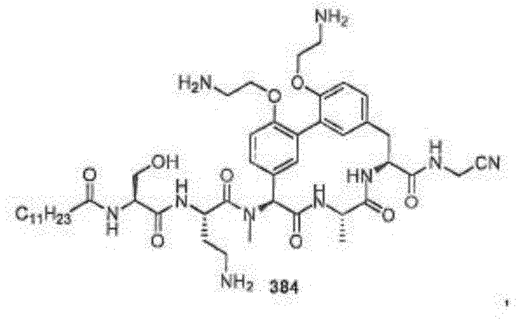 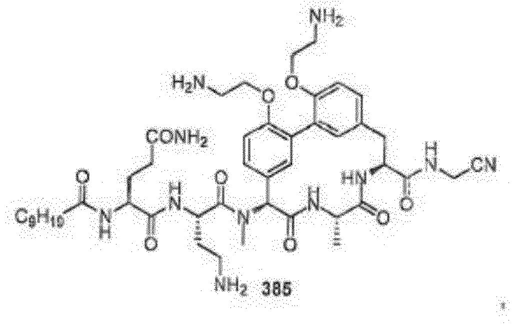 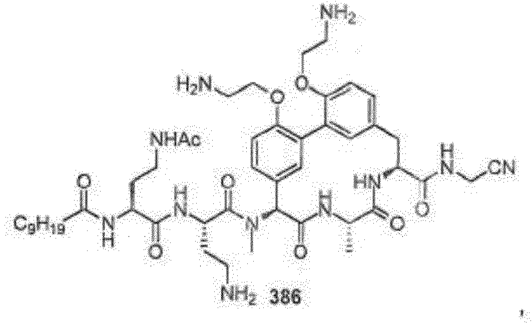 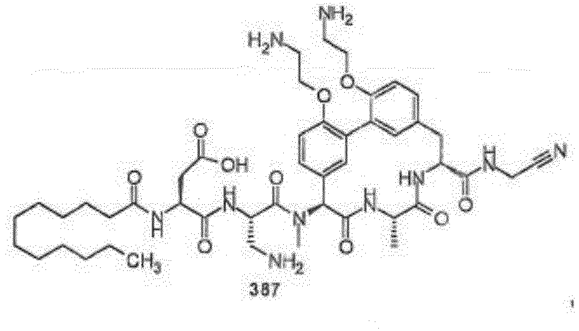 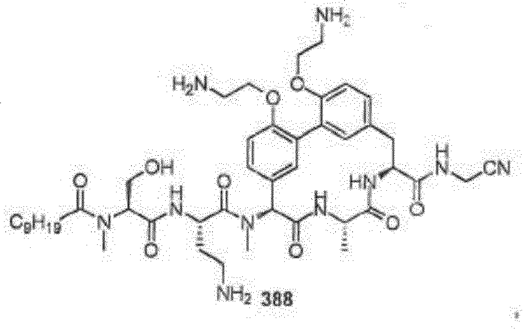 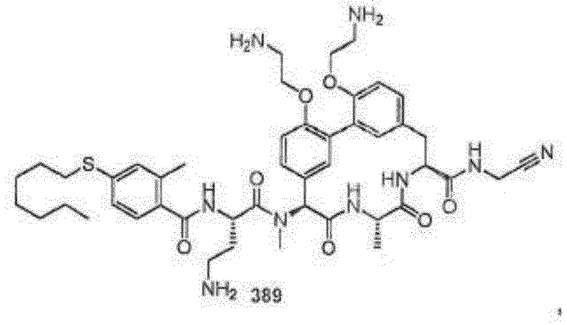 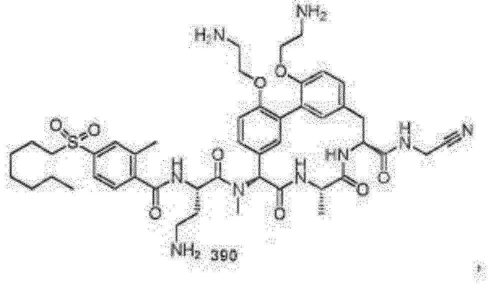 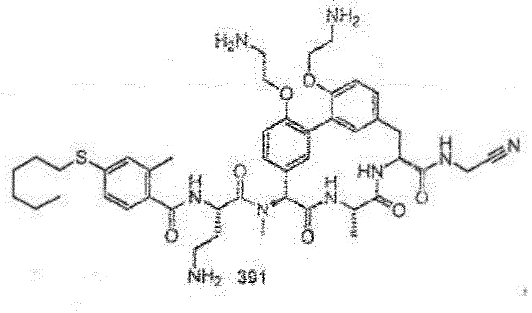 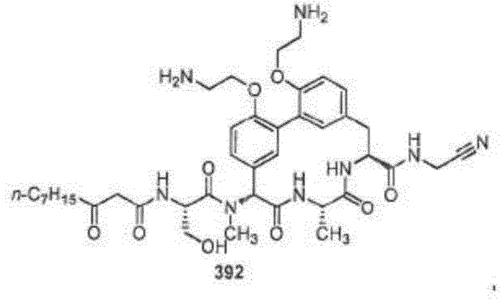 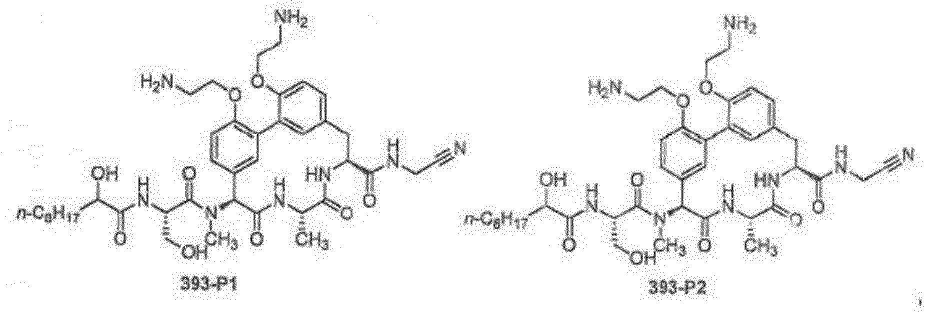 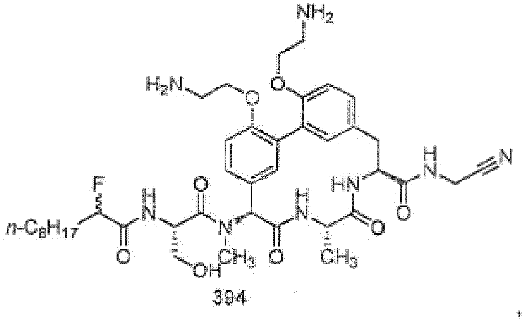 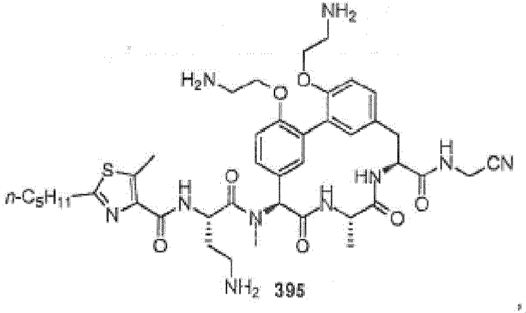 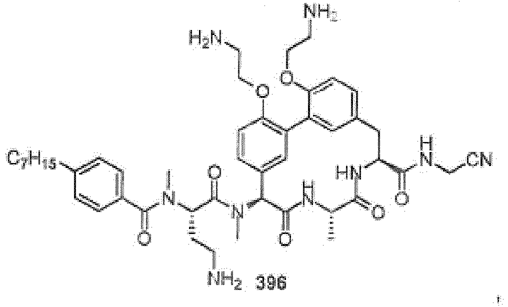 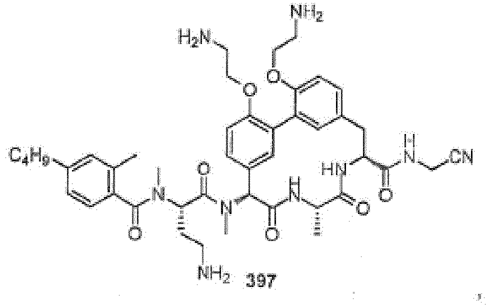 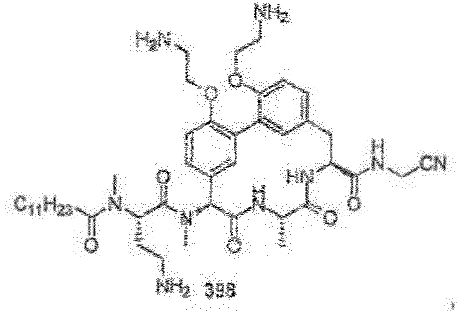 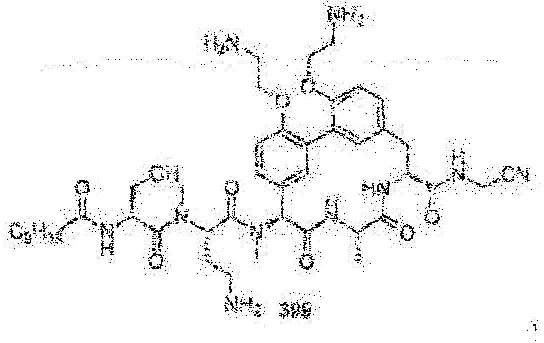 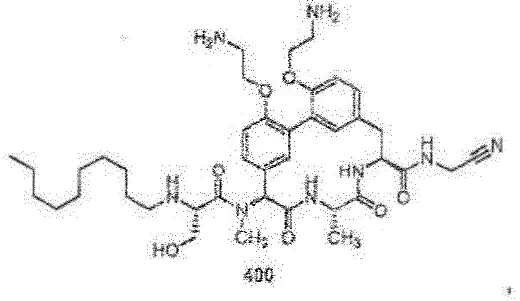 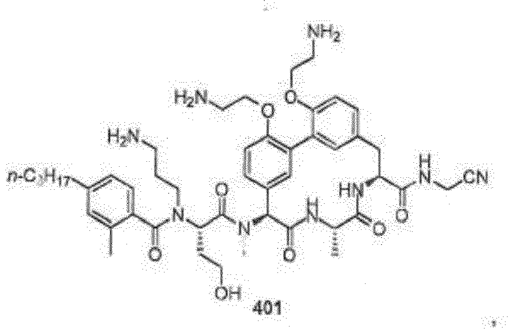 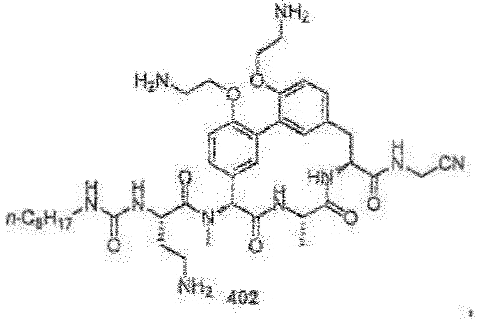 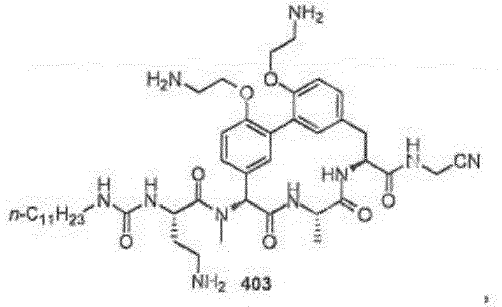 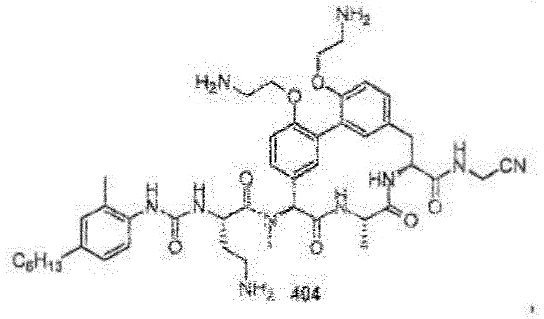 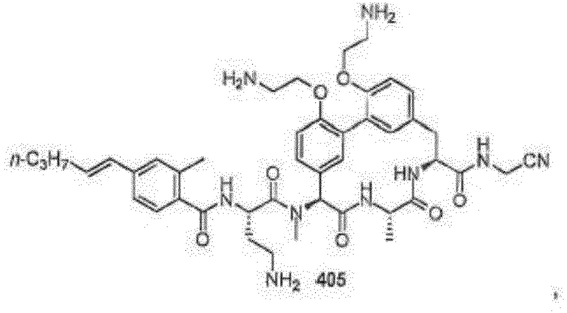 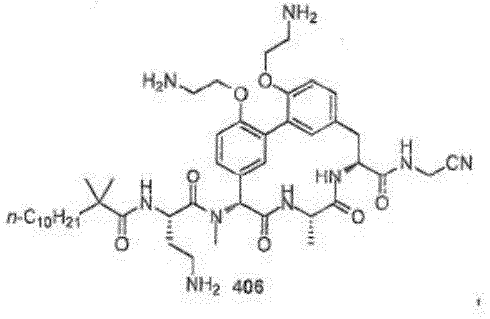 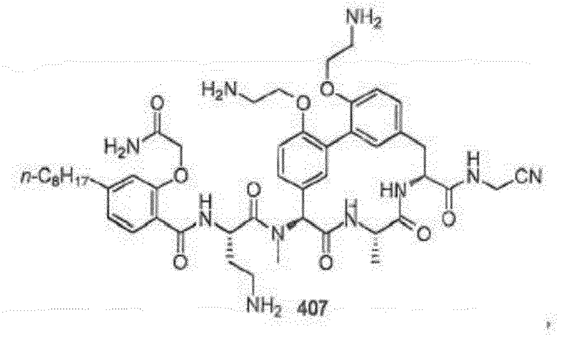 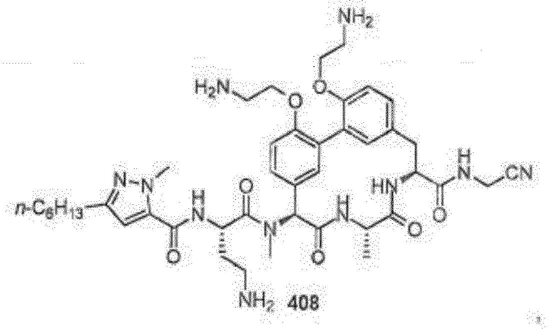 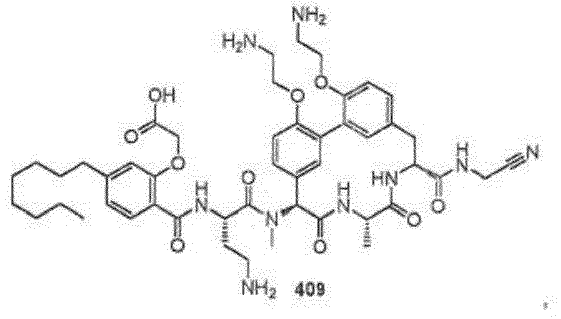 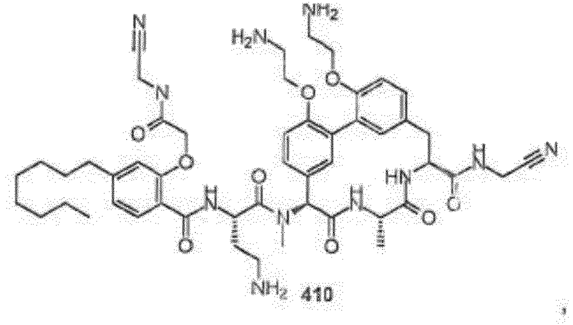 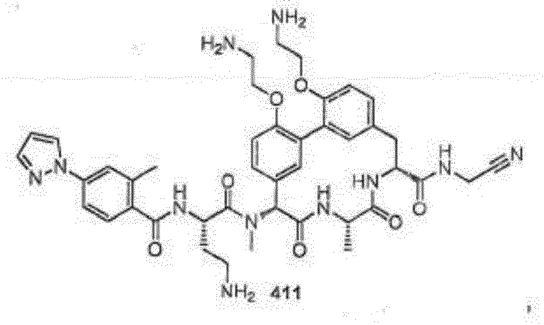 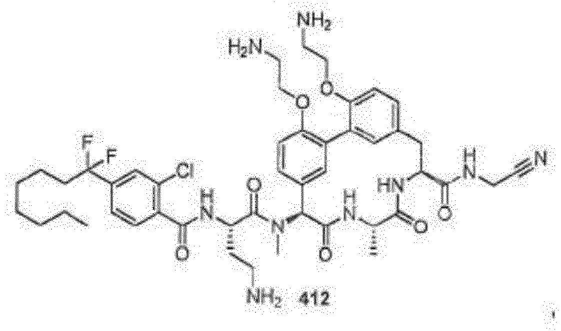 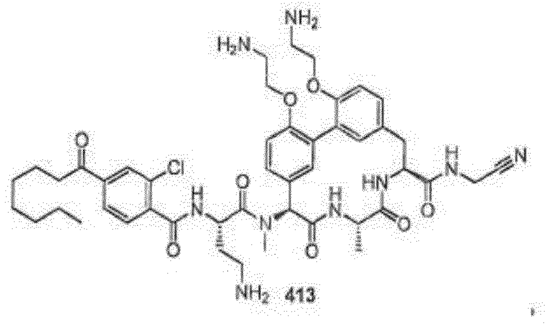 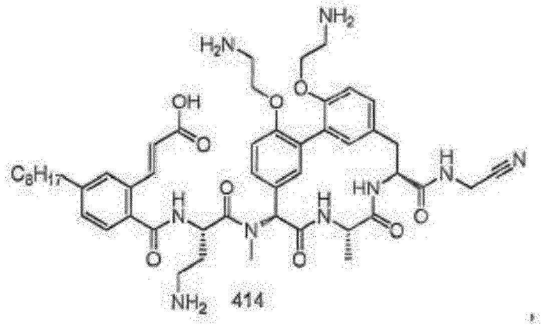 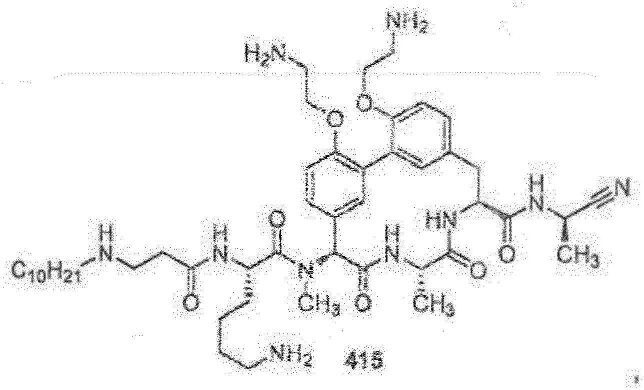 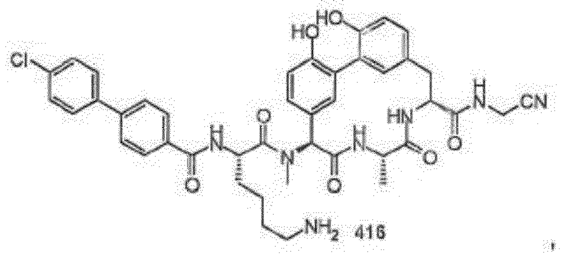 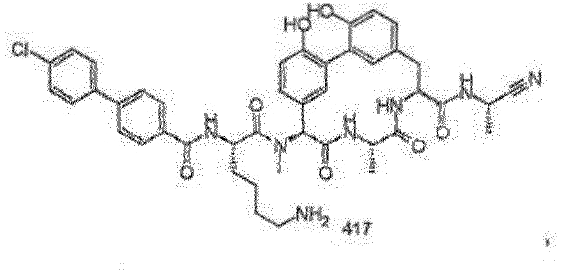 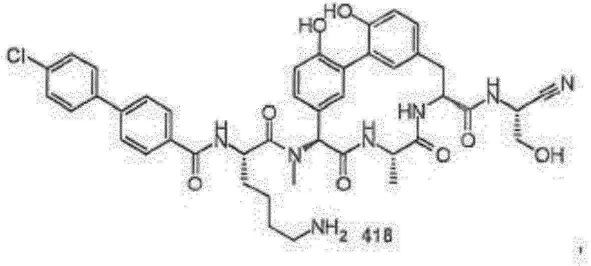 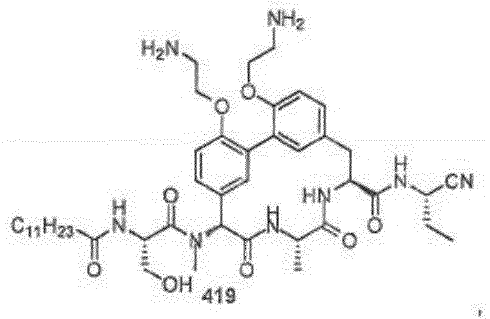 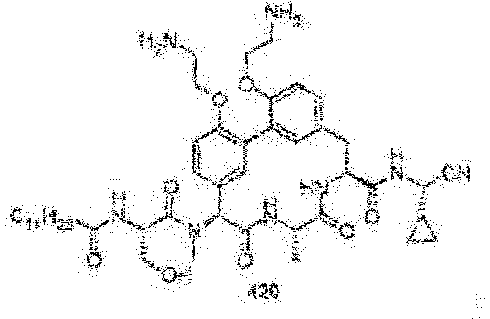 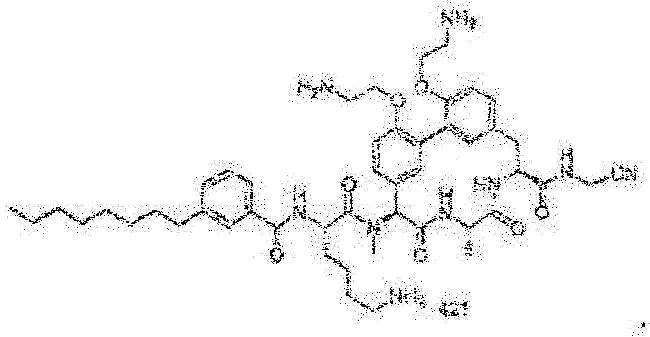 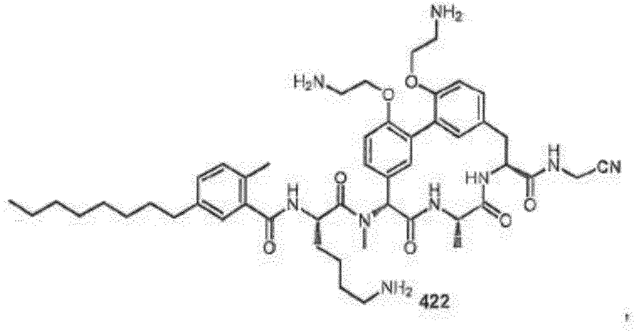 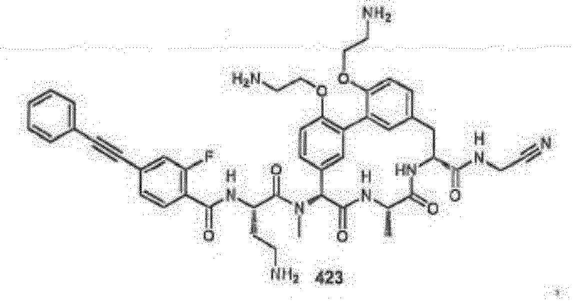 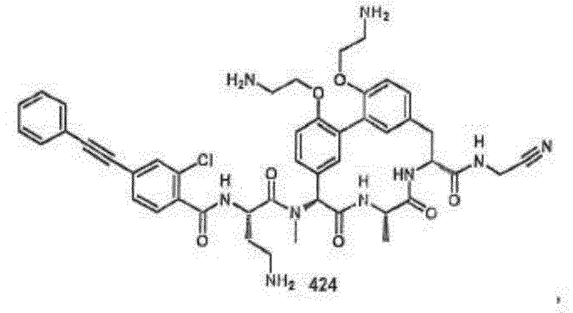 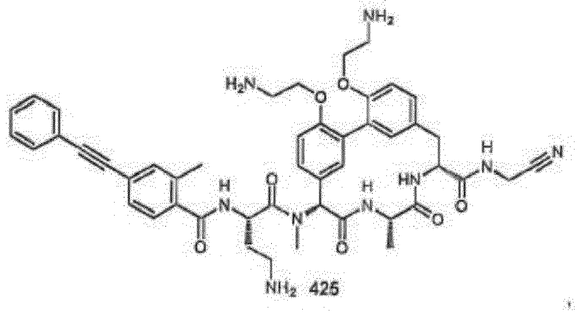 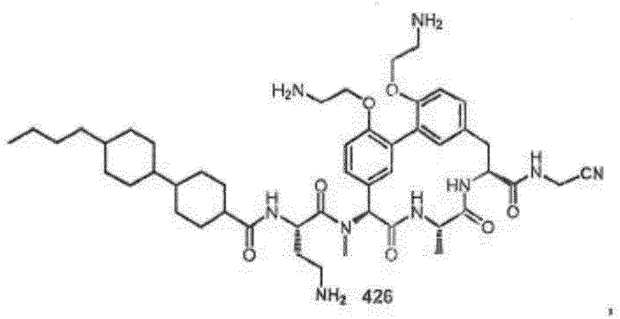 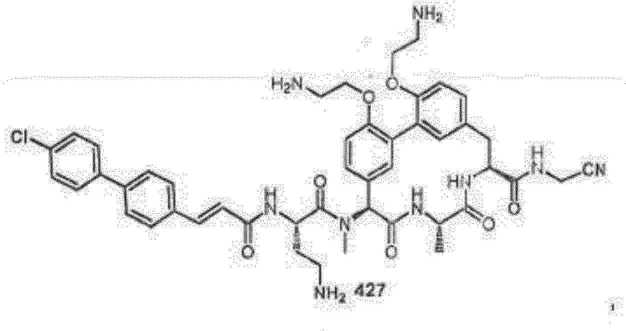 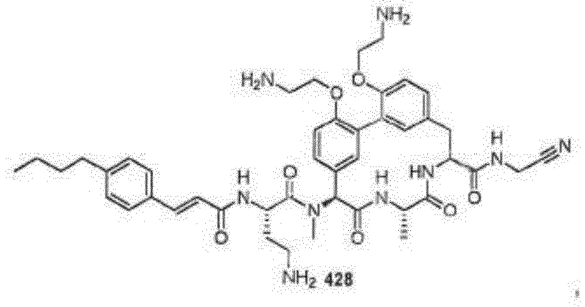 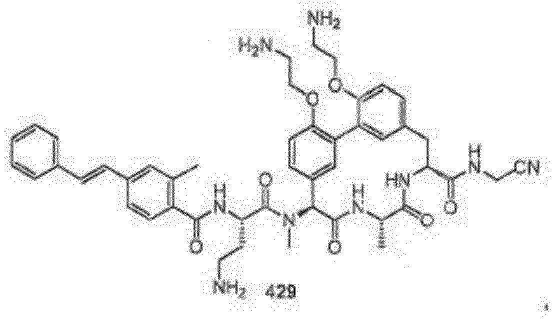 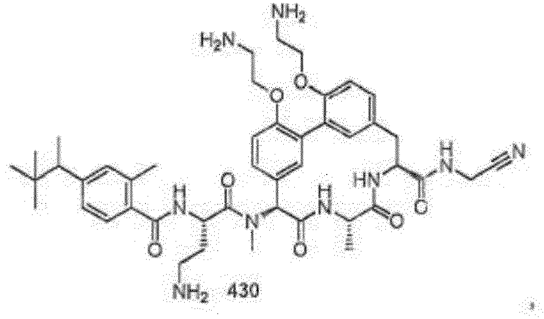 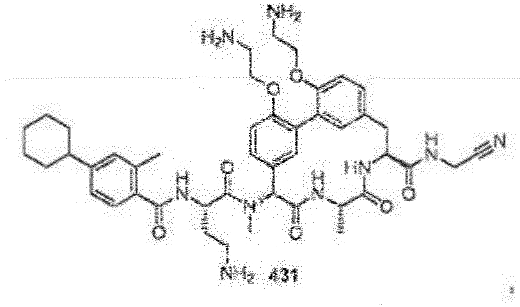 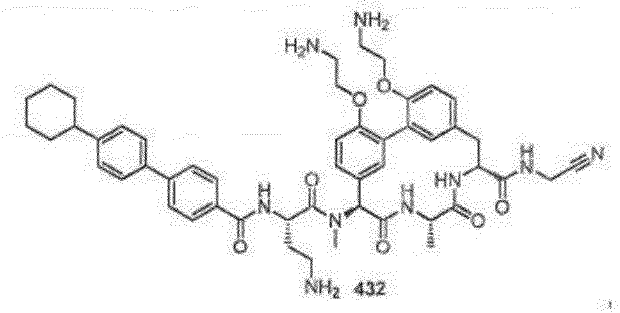 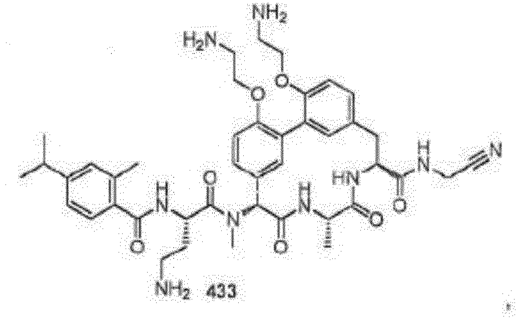 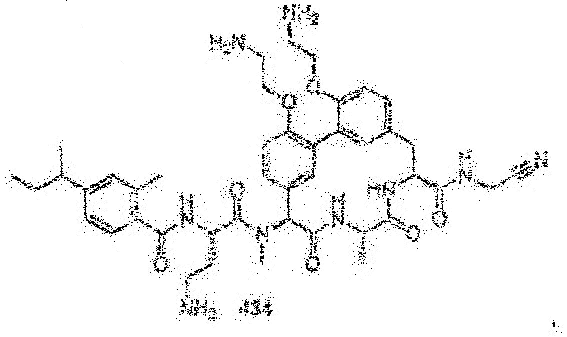 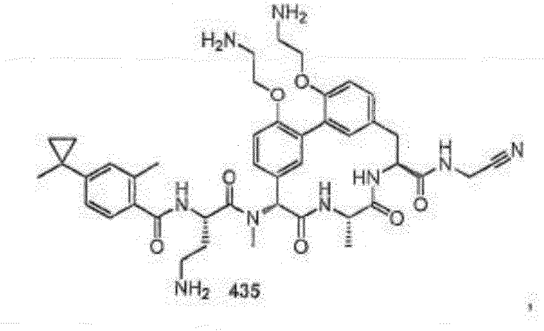 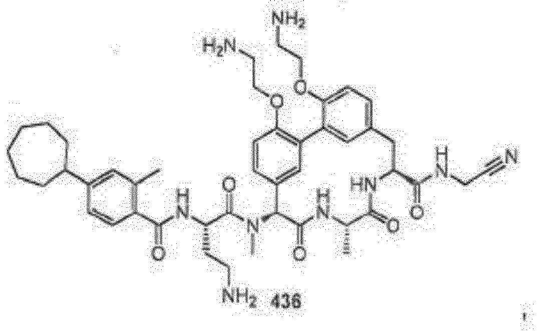 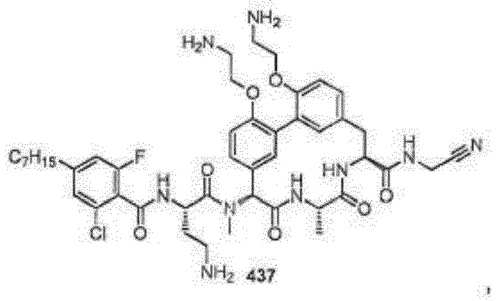 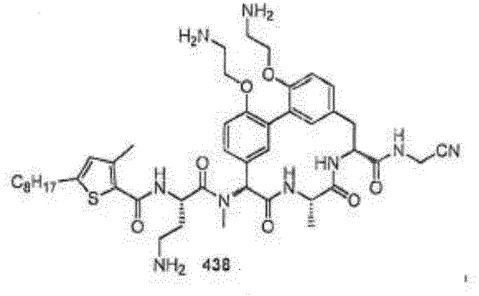 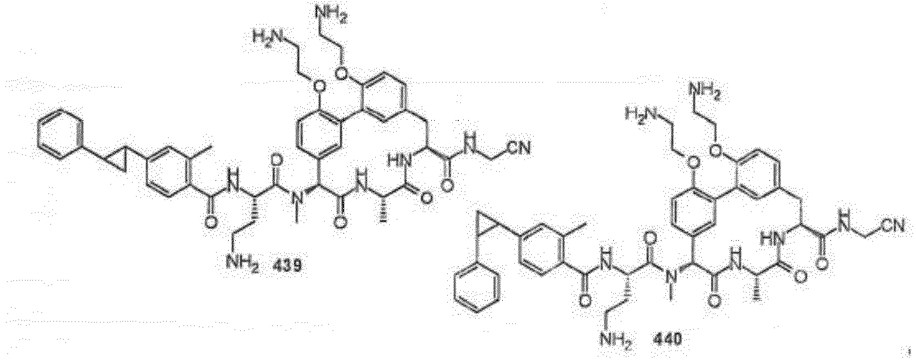 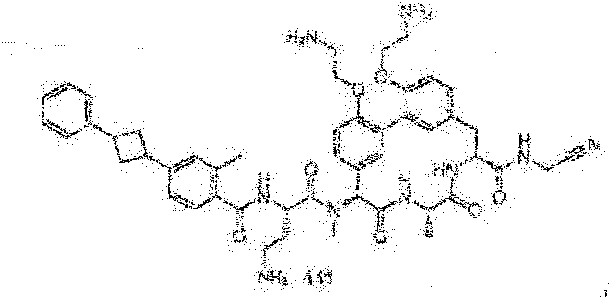 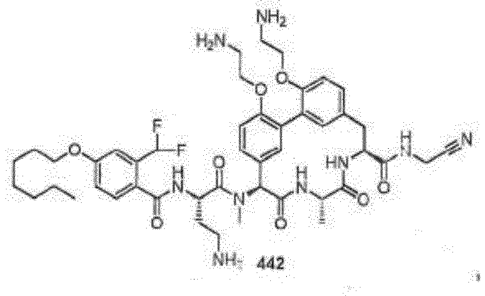 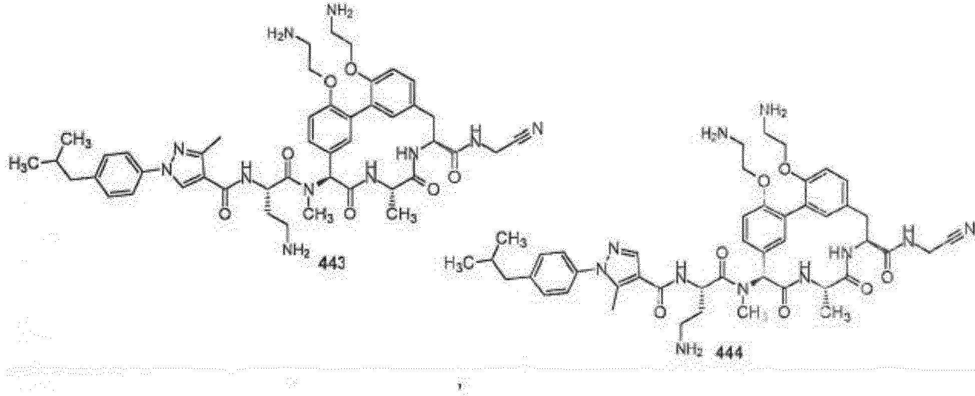 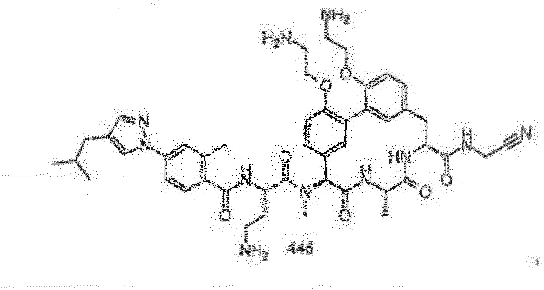 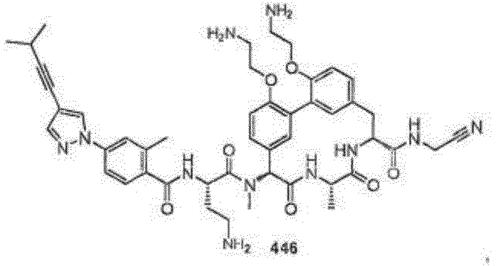 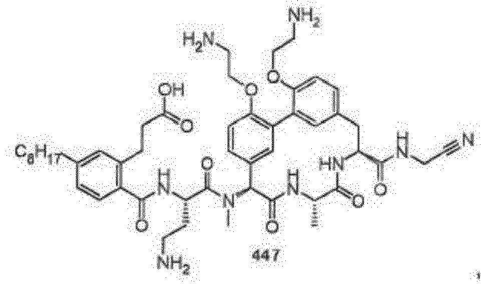 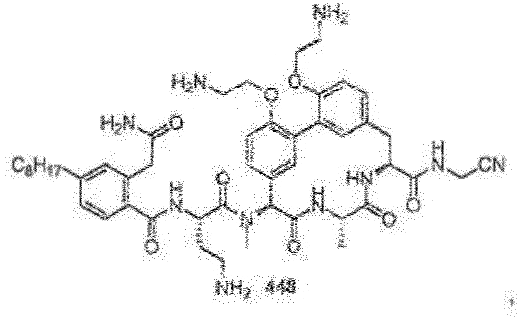 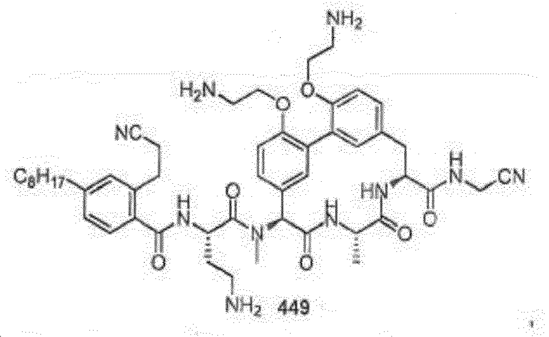 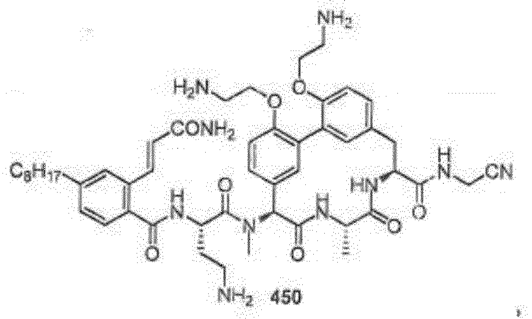 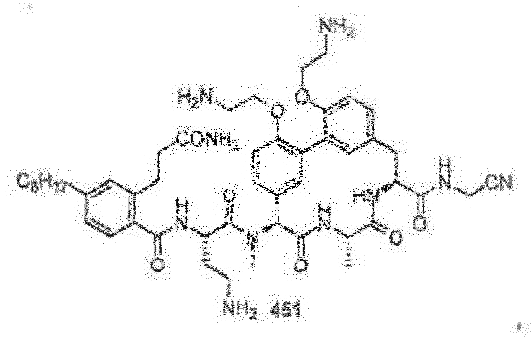 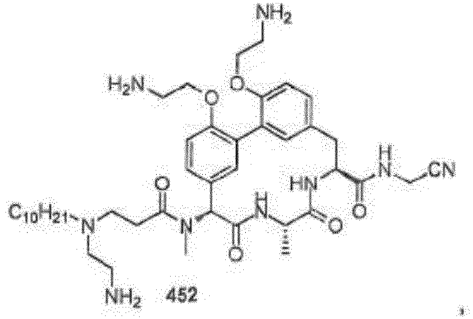 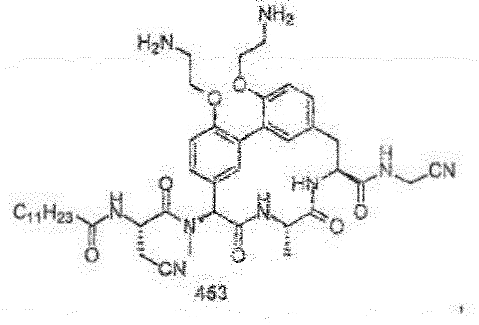 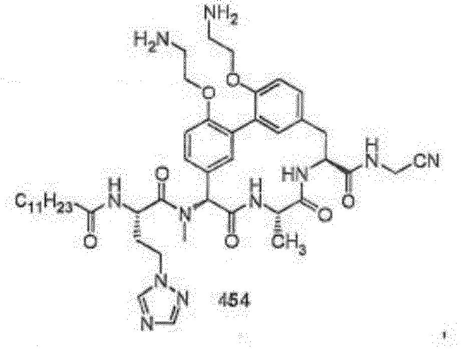 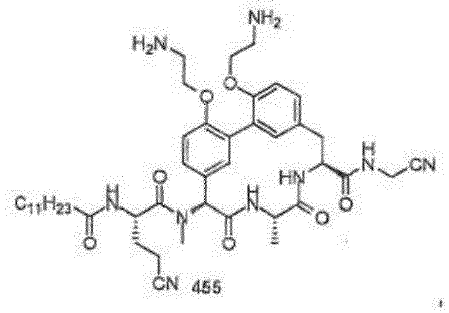 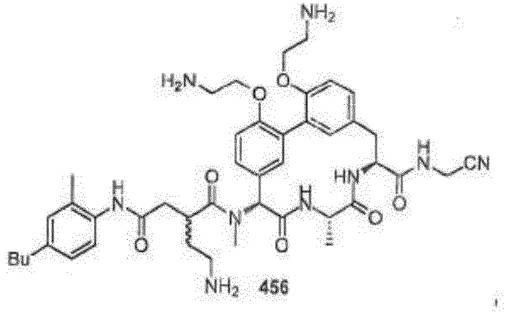 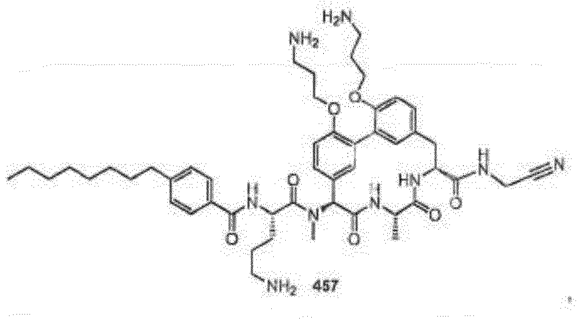 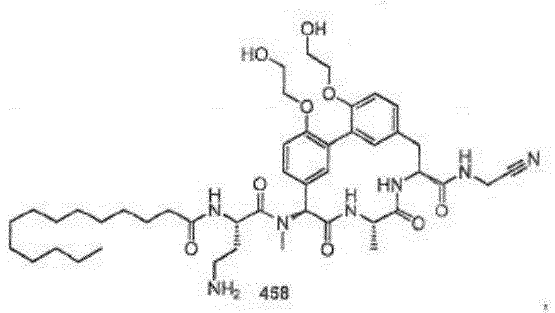 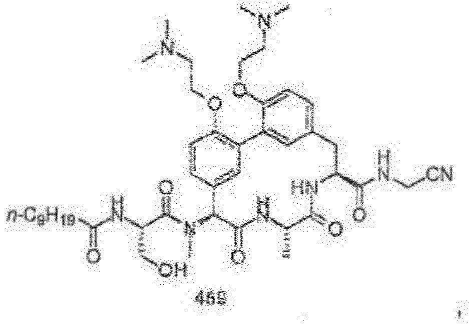 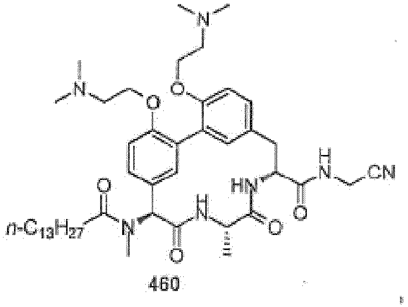 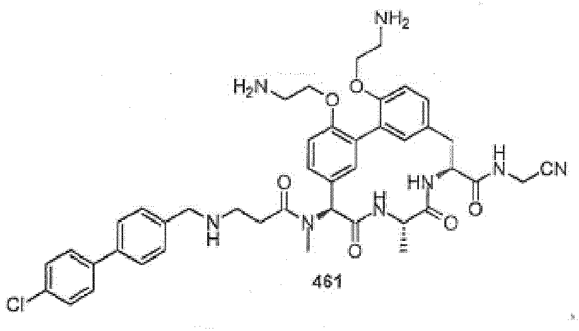 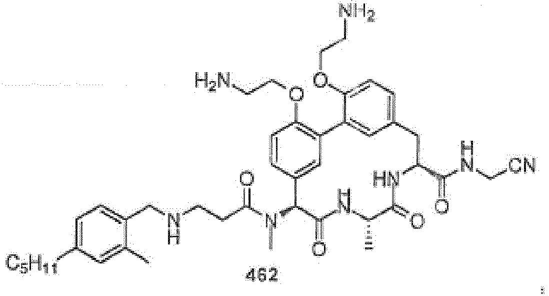 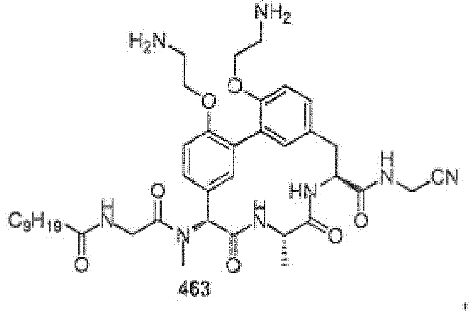 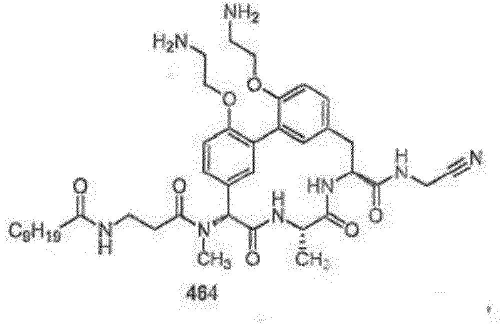 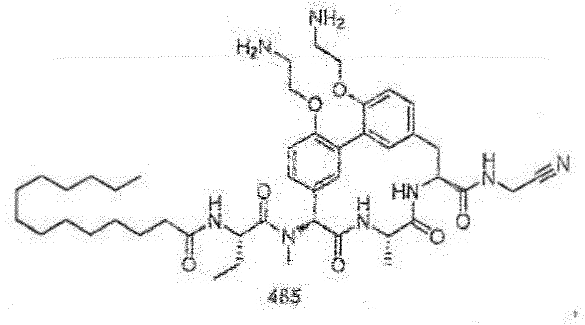 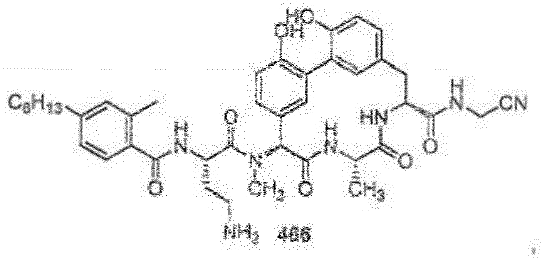 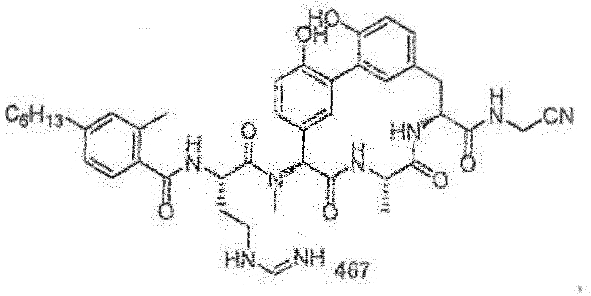 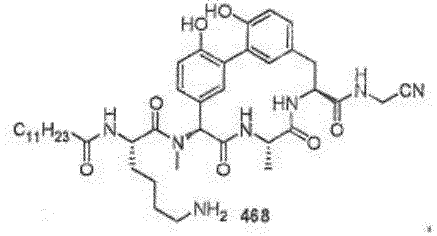 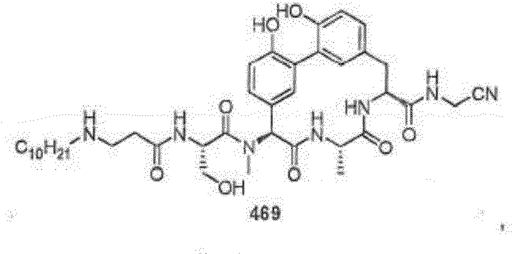 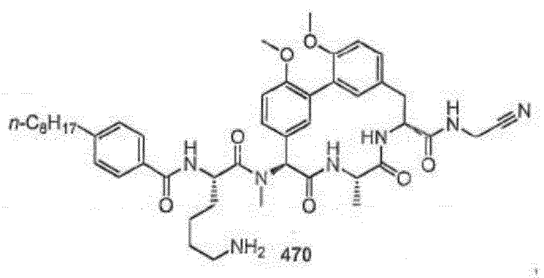 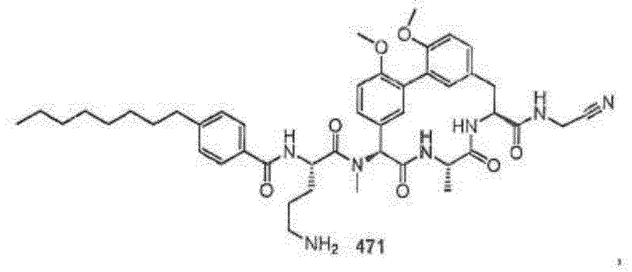 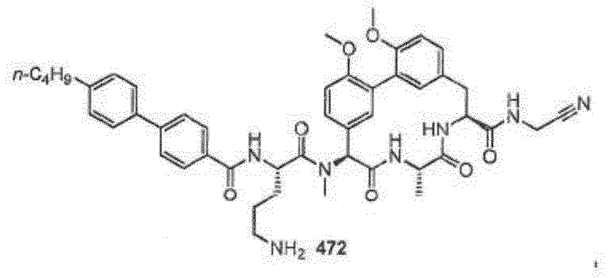 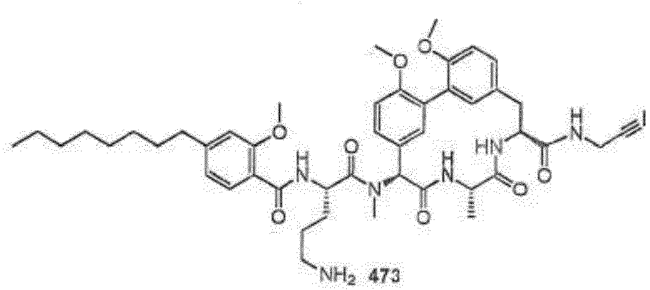 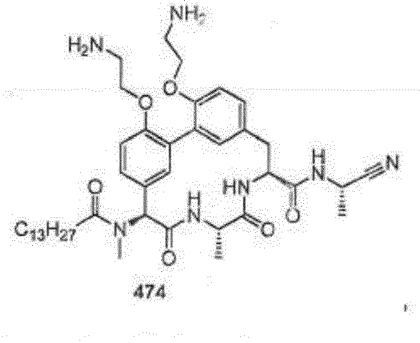 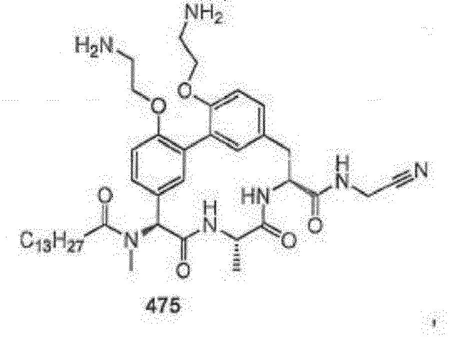 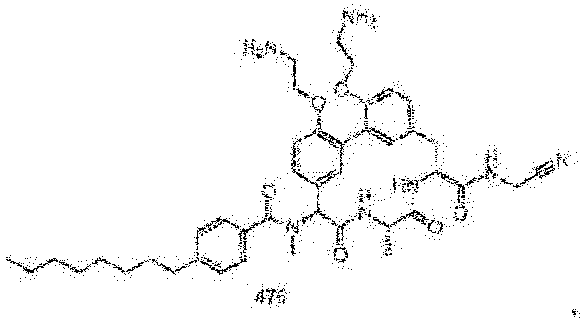 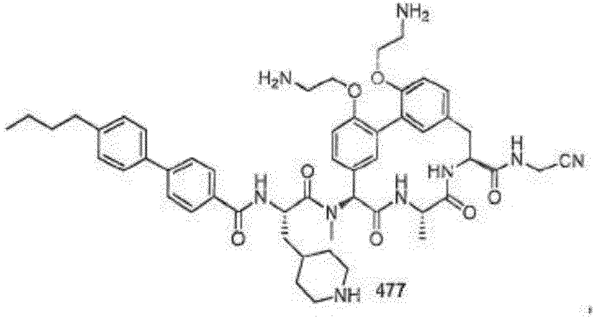 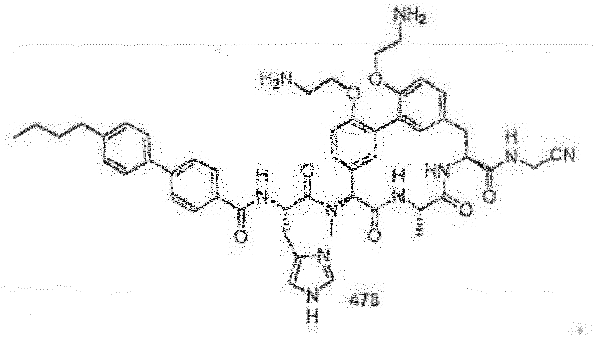 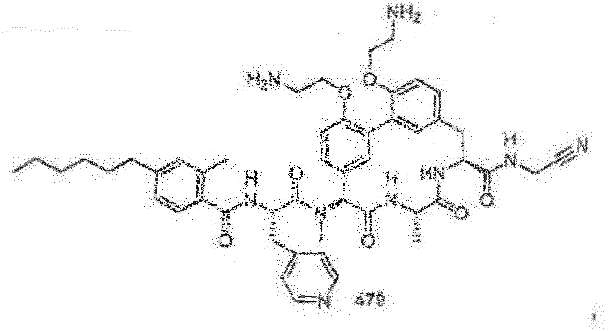 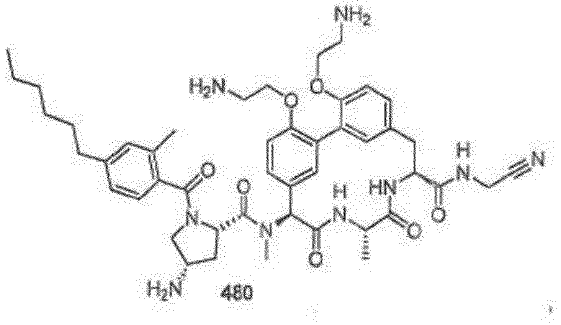 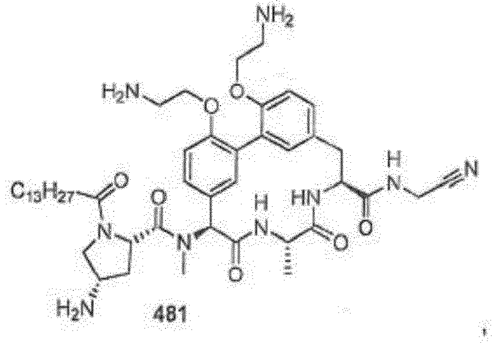 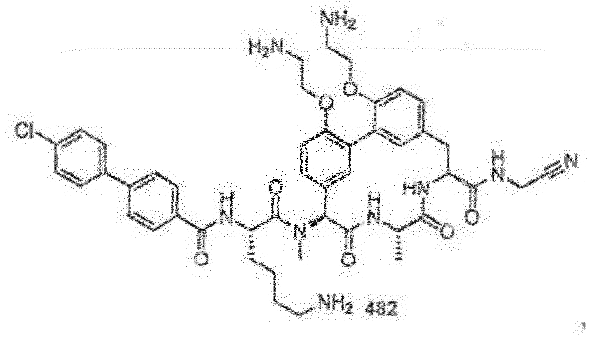 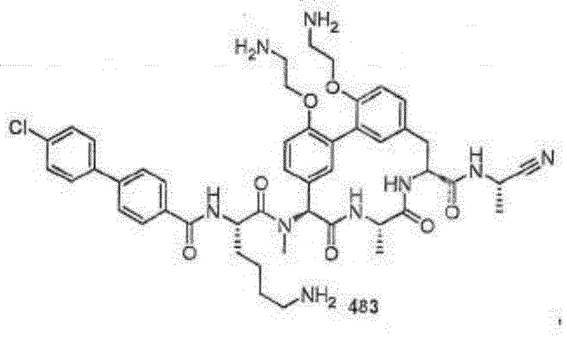 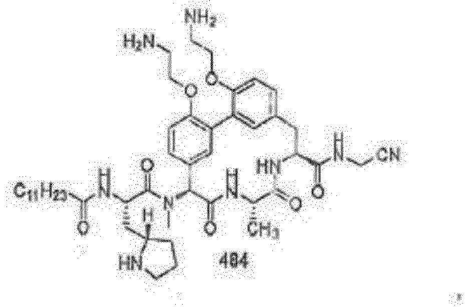 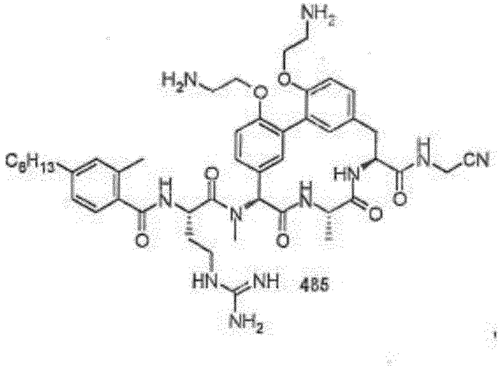 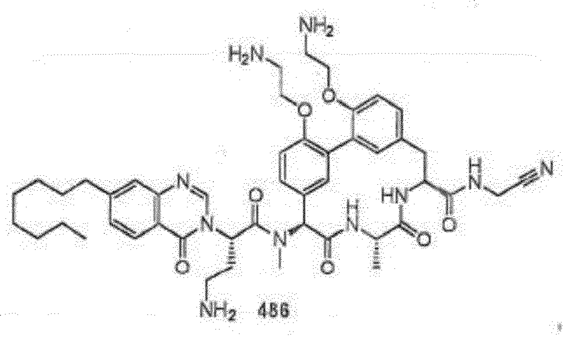 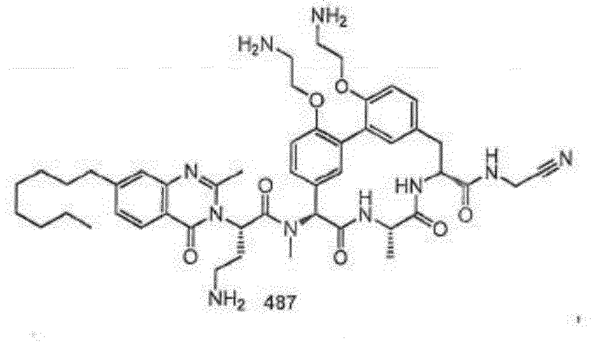 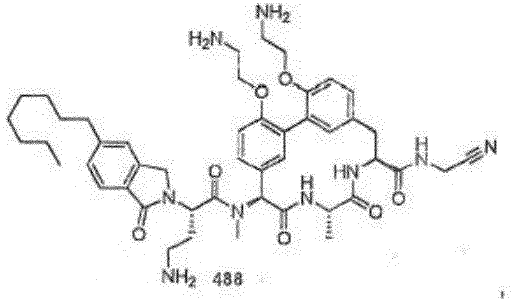 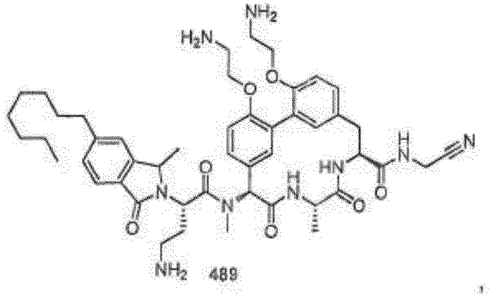 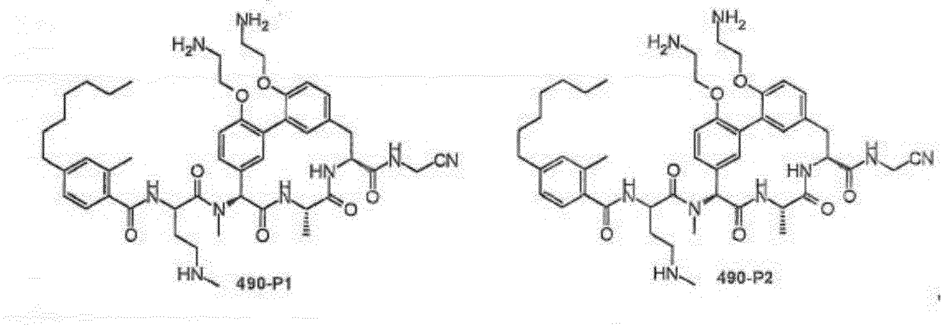 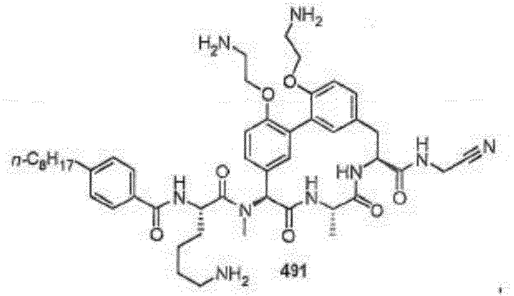 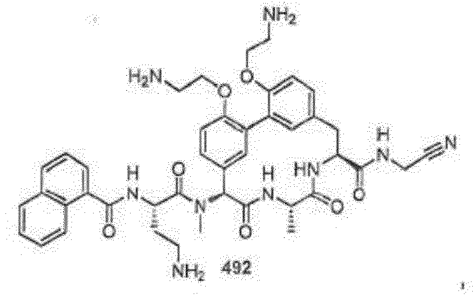 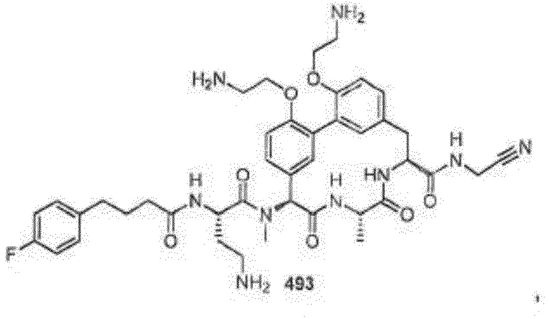 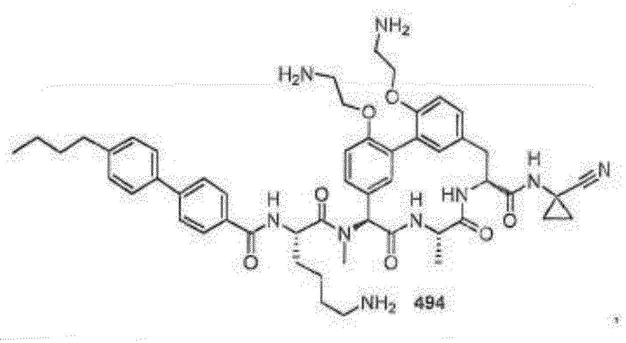 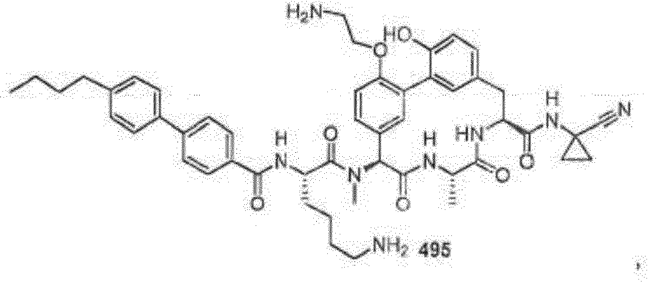 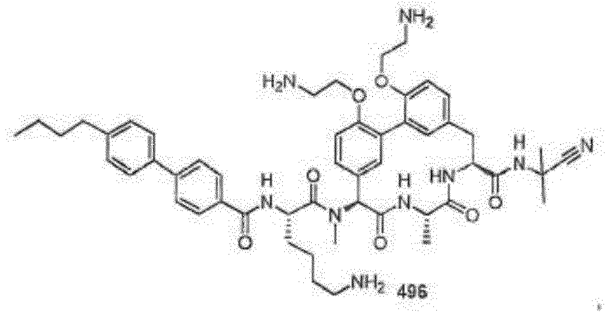 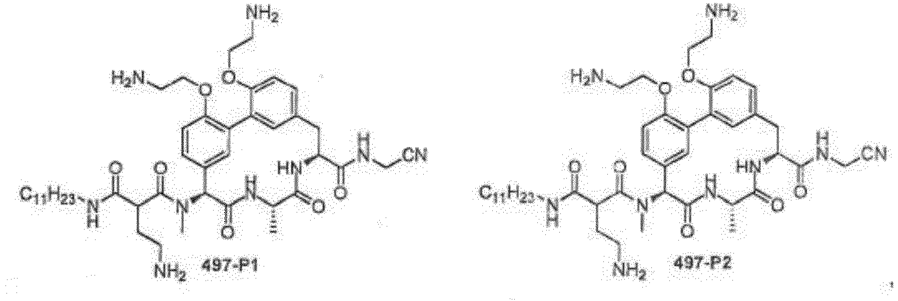 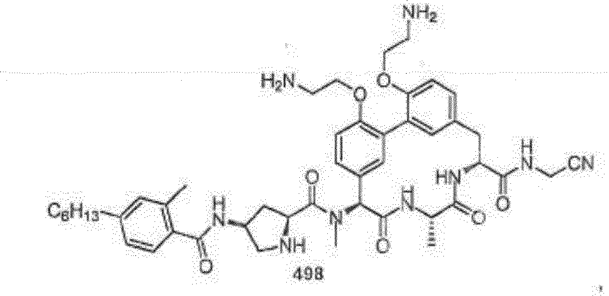 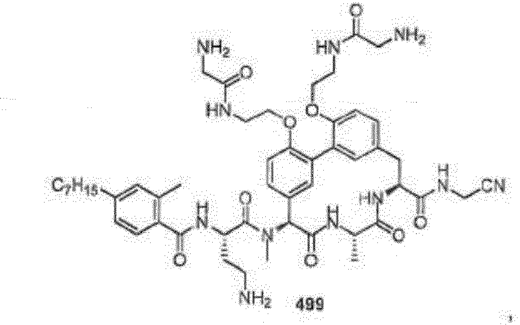 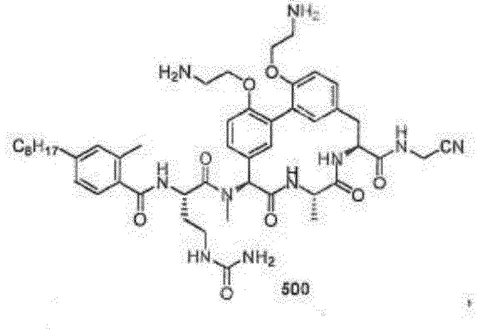 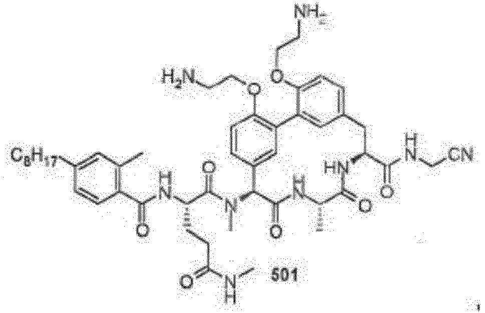 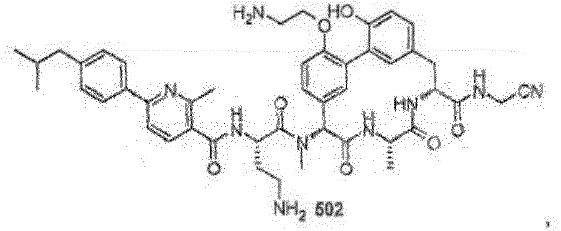 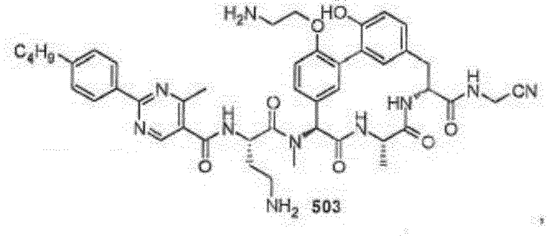 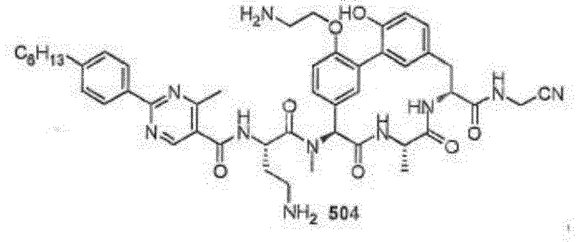 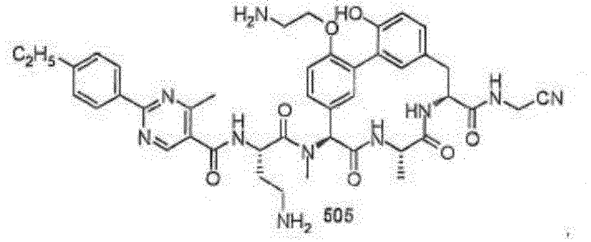 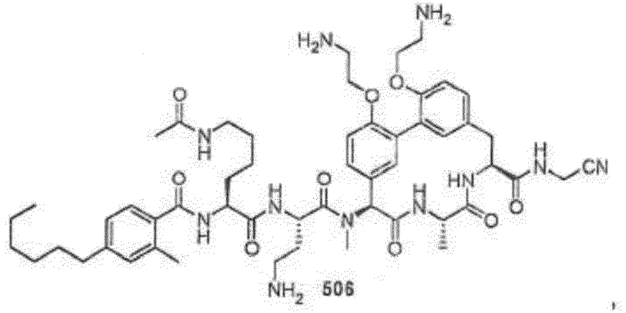 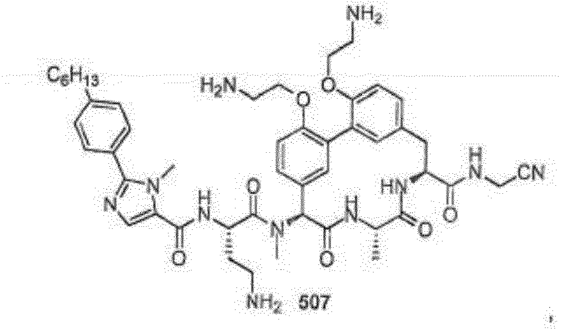 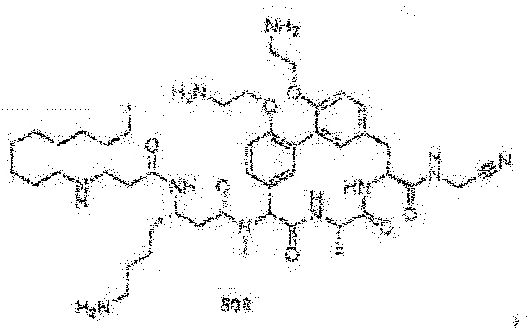 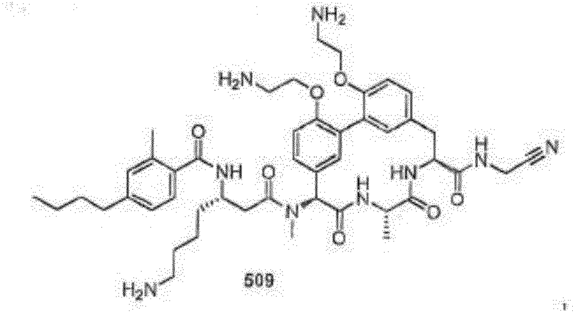 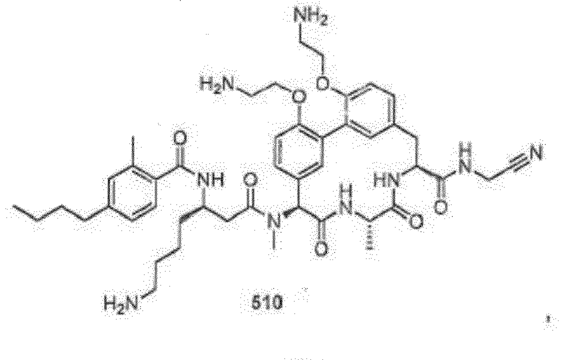 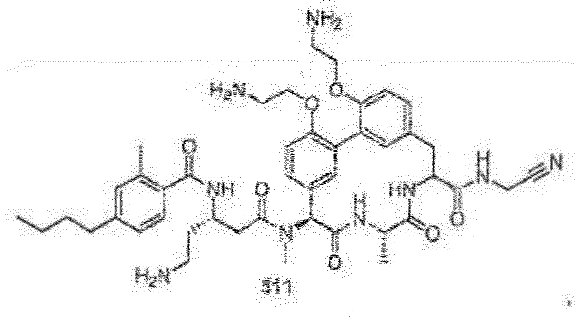 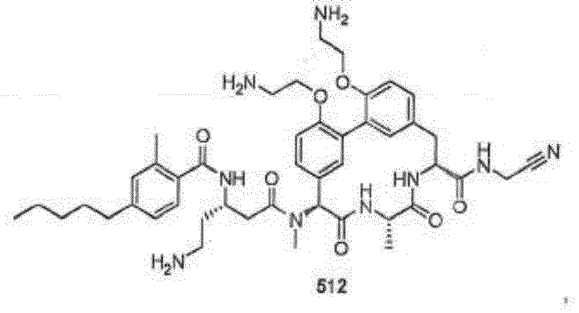 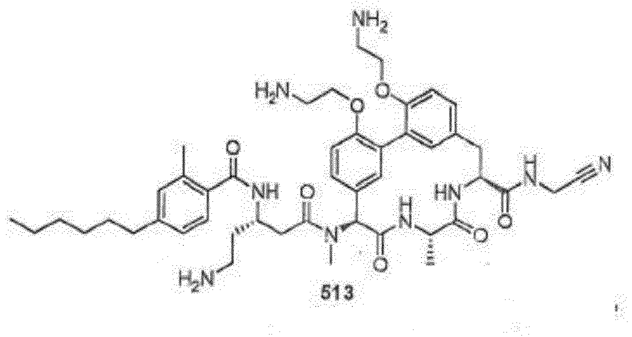 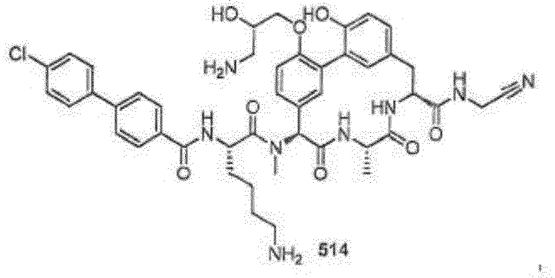 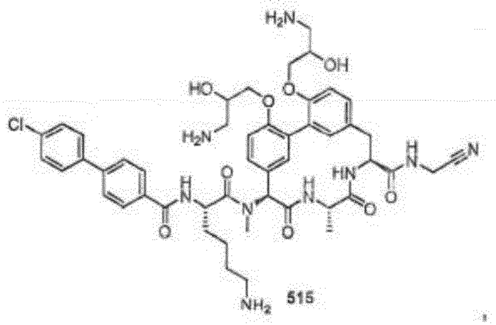 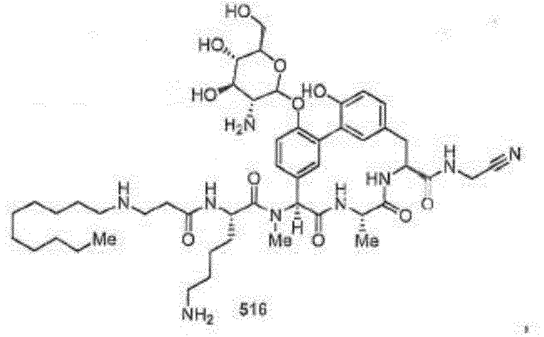 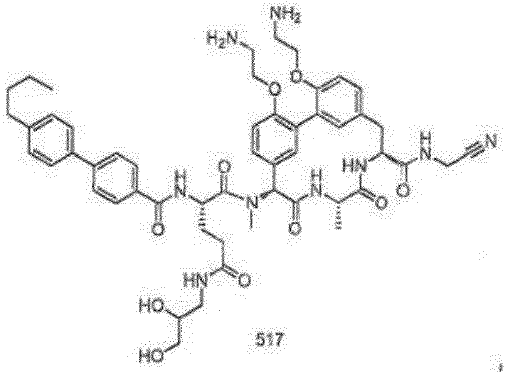 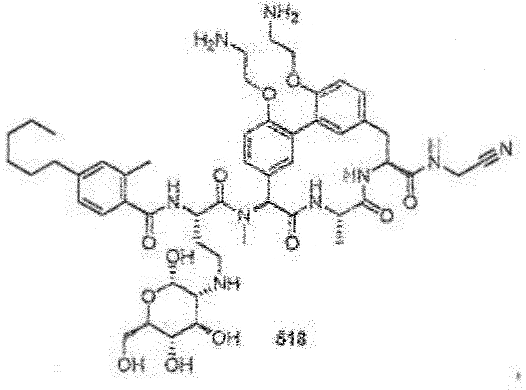 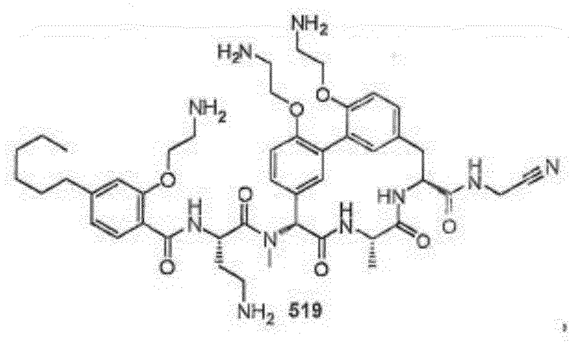 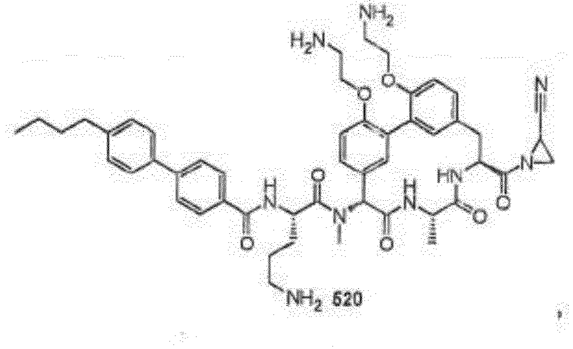 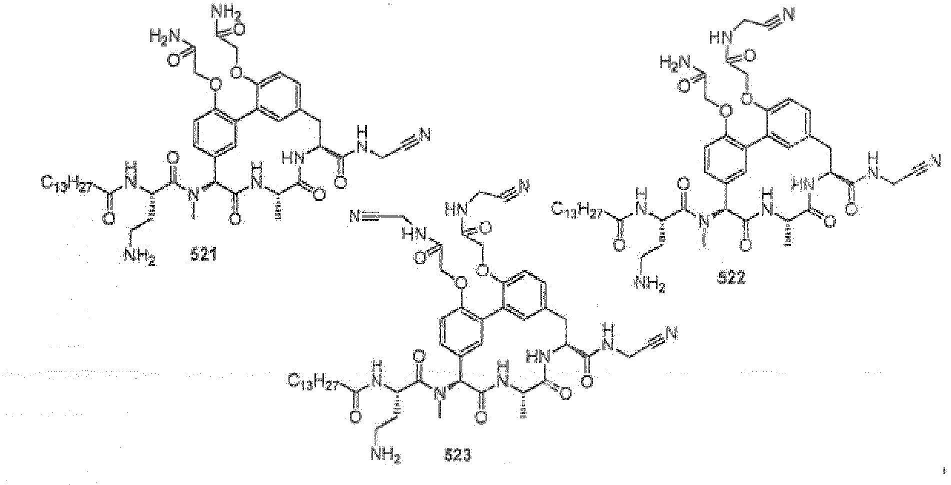 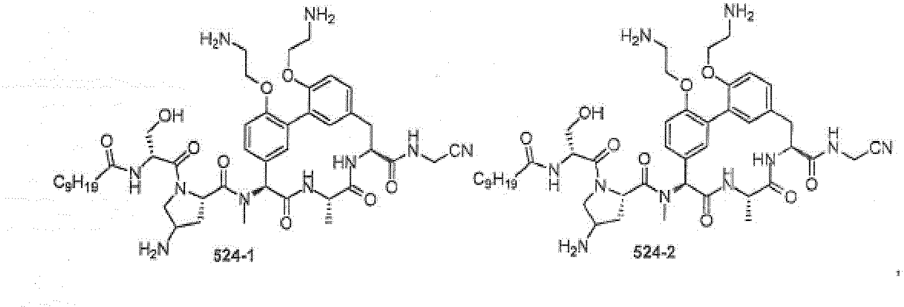 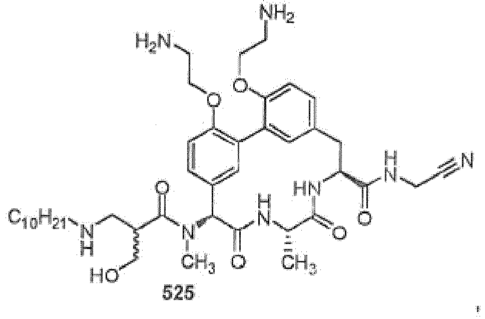 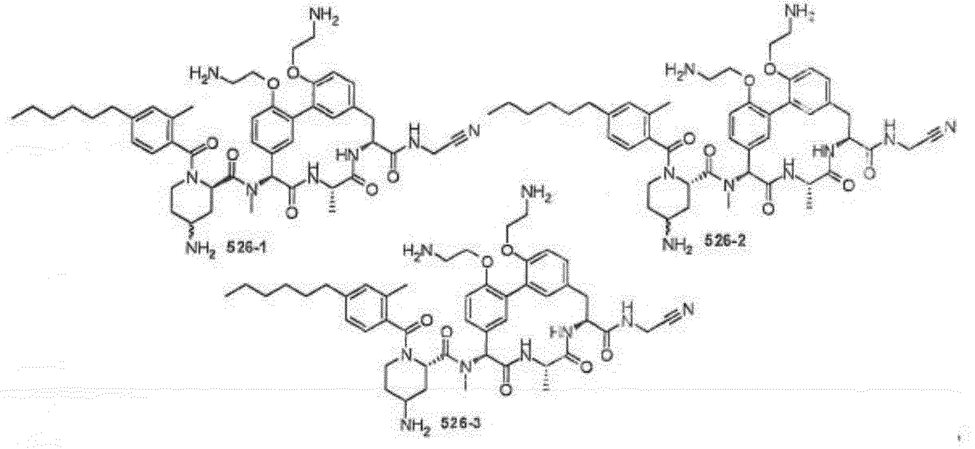 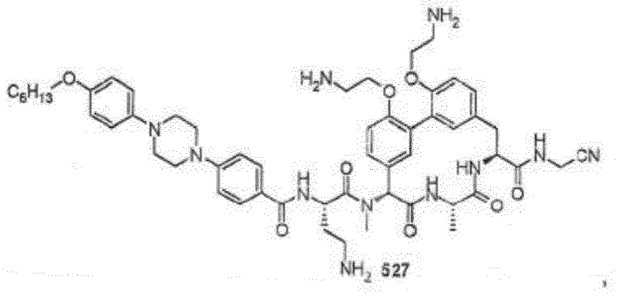 und 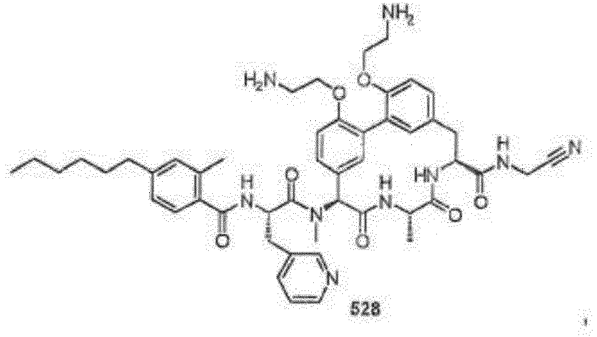 oder ein pharmazeutisch annehmbares Salz oder Solvat davon.

2. Pharmazeutische Zusammensetzung, die eine Verbindung nach Anspruch 1 oder ein pharmazeutisch annehmbares Salz oder ein pharmazeutisch annehmbares Solvat davon und einen pharmazeutisch annehmbaren Hilfsstoff umfasst.

3. Verbindung nach Anspruch 1 oder ein pharmazeutisch annehmbares Salz oder ein pharmazeutisch annehmbares Solvat davon zur Verwendung in einem Verfahren zur Behandlung einer bakteriellen Infektion bei einem Säugetier.

4. Verbindung, Salz oder Solvat zur Verwendung nach Anspruch 3, wobei die bakterielle Infektion eine Infektion ist, an der Folgendes beteiligt ist:
Pseudomonas aeruginosa, Pseudomonas fluorescens, Pseudomonas acidovorans, Pseudomonas alcaligenes, Pseudomonas putida, Stenotrophomonas maltophilia, Burkholderia cepacia, Aeromonas hydrophilia, Escherichia coli, Citrobacter freundii, Salmonella typhimurium, Salmonella typhi, Salmonella paratyphi, Salmonella enteritidis, Shigella dysenteriae, Shigella flexneri, Shigella sonnei, Enterobacter cloacae, Enterobacter aerogenes, Klebsiella pneumoniae, Klebsiella oxytoca, Serratia marcescens, Francisella tularensis, Morganella morganii, Proteus mirabilis, Proteus vulgaris, Providencia alcalifaciens, Providencia rettgeri, Providencia stuartii, Acinetobacter baumannii, Acinetobacter calcoaceticus, Acinetobacter haemolyticus, Yersinia enterocolitica, Yersinia pestis, Yersinia pseudotuberculosis, Yersinia intermedia, Bordetella pertussis, Bordetella parapertussis, Bordetella bronchiseptica, Haemophilus influenzae, Haemophilus parainfluenzae, Haemophilus haemolyticus, Haemophilus parahaemolyticus, Haemophilus ducreyi, Pasteurella multocida, Pasteurella haemolytica, Branhamella catarrhalis, Helicobacter pylori, Campylobacter fetus, Campylobacter jejuni, Campylobacter coli, Borrelia burgdorferi, Vibrio cholerae, Vibrio parahaemolyticus, Legionella pneumophila, Listeria monocytogenes, Neisseria gonorrhoeae, Neisseria meningitidis, Kingella, Moraxella, Gardnerella vaginalis, Bacteroides fragilis, Bacteroides distasonis, Bacteroides 3452A homology group, Bacteroides vulgatus, Bacteroides ovalus, Bacteroides thetaiotaomicron, Bacteroides uniformis, Bacteroides eggerthii, Bacteroides splanchnicus, Clostridium difficile, Mycobacterium tuberculosis, Mycobacterium avium, Mycobacterium intracellulare, Mycobacterium leprae, Corynebacterium diphtheriae, Corynebacterium ulcerans, Streptococcus pneumoniae, Streptococcus agalactiae, Streptococcus pyogenes, Enterococcus faecalis, Enterococcus faecium, Staphylococcus aureus, Staphylococcus epidermidis, Staphylococcus saprophyticus, Staphylococcus intermedius, Staphylococcus hyicus subsp. hyicus, Staphylococcus haemolyticus, Staphylococcus hominis oder Staphylococcus saccharolyticus.

5. Verbindung, Salz oder Solvat zur Verwendung nach Anspruch 3 oder 4, wobei die bakterielle Infektion eine Infektion ist, an der gramnegative Bakterien beteiligt sind.

6. Verbindung, Salz oder Solvat zur Verwendung nach einem der Ansprüche 3 bis 5, wobei das Verfahren eine topische Verabreichung der Verbindung, des Salzes oder des Solvats umfasst.

7. Verbindung, Salz oder Solvat zur Verwendung nach einem der Ansprüche 3 bis 5, wobei das Verfahren weiters das Verabreichen eines zweiten Therapeutikums umfasst.

8. Verbindung, Salz oder Solvat zur Verwendung nach Anspruch 7, wobei das zweite Therapeutikum kein SpsB-Inhibitor ist.

9. Verbindung, Salz oder Solvat zur Verwendung nach Anspruch 8, wobei das zweite Therapeutikum ein Aminoglykosid-Antibiotikum, Fluorchinolon-Antibiotikum, β-Lactam-Antibiotikum, Makrolid-Antibiotikum, Glykopeptid-Antibiotikum, Rifampicin, Chloramphenicol, Fluoramphenicol, Colistin, Mupirocin, Bacitracin, Daptomycin oder Linezolid ist.

10. Verbindung, Salz oder Solvat zur Verwendung nach Anspruch 8, wobei das zweite Therapeutikum ein β-Lactam-Antibiotikum ist.

11. Verbindung, Salz oder Solvat zur Verwendung nach Anspruch 10, wobei das β-Lactam-Antibiotikum aus Penicillinen, Monobactamen, Cephalosporinen, Cephamycinen und Carbapenemen ausgewählt ist.

12. Verbindung, Salz oder Solvat zur Verwendung nach Anspruch 11, wobei das β-Lactam-Antibiotikum aus Azlocillin, Amoxicillin, Ampicillin, Doripenem, Meropenem, Biapenem, Cefamandol, Imipenem, Mezlocillin, Cefmetazol, Cefprozil, Piperacillin/Tazobactam, Carbenicillin, Cefaclor, Cephalothin, Ertapenem, Cefazolin, Cefepim, Cefonicid, Cefoxitin, Ceftazidim, Oxacillin, Cefdinir, Cefixim, Cefotaxim, Cefotetan, Cefpodoxim, Ceftizoxim, Ceftriaxon, Faropenem, Mecillinam, Methicillin, Moxalactam, Ticarcillin, Tomopenem, Ceftobiprol, Ceftarolin, Flomoxef, Cefiprom und Cefozopran ausgewählt ist.

13. Verbindung, Salz oder Solvat zur Verwendung nach Anspruch 10, wobei das Verfahren weiters das Verabreichen eines β-Lactamase-Inhibitors umfasst.

## Revendications

1. Composé choisi parmi : 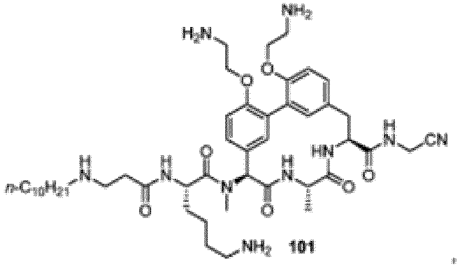 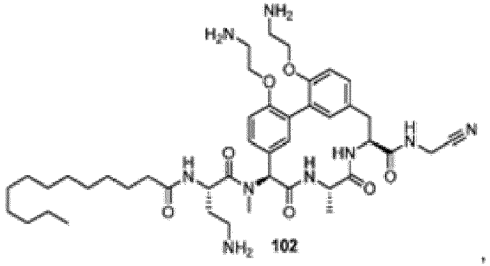 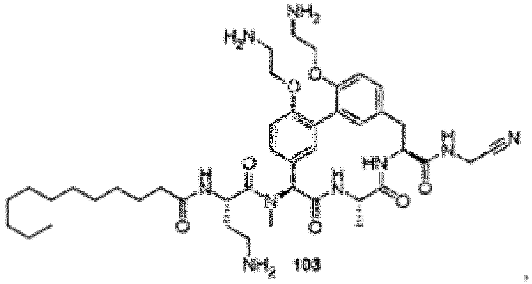 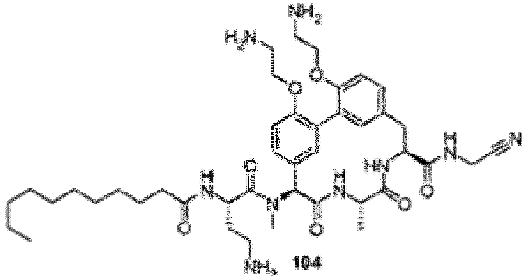 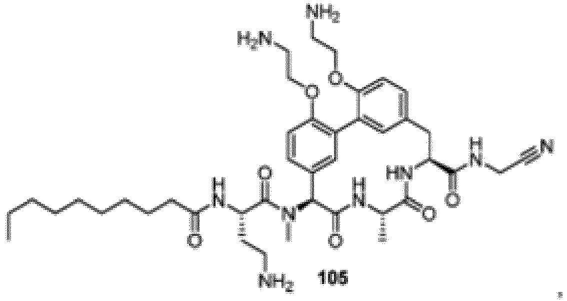 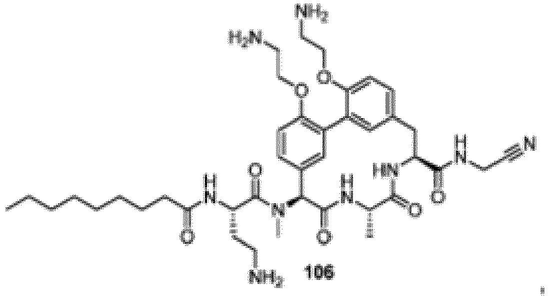 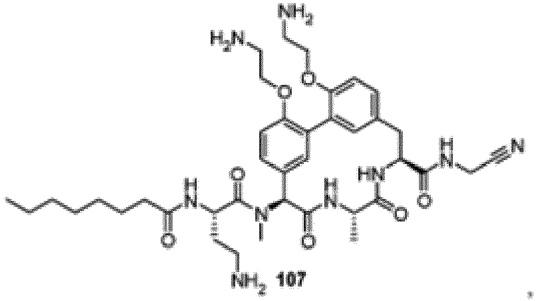 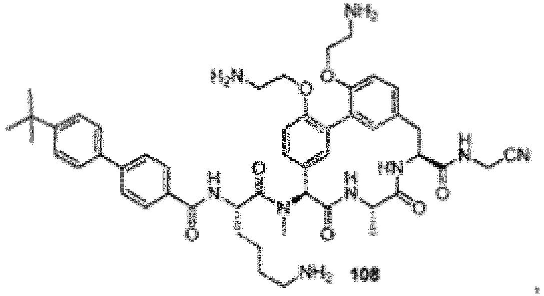 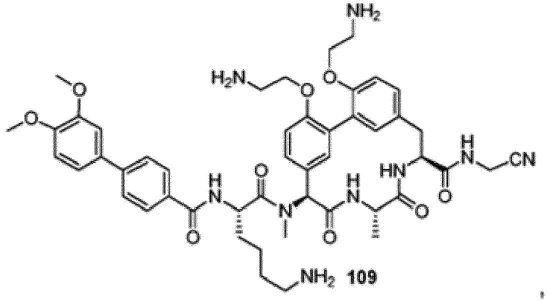 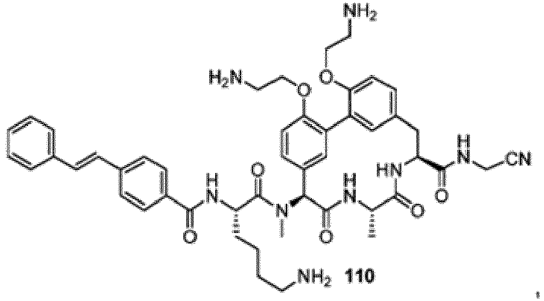 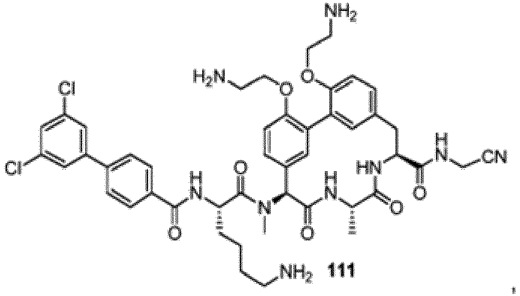 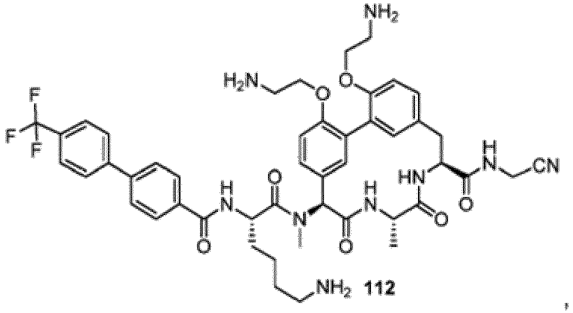 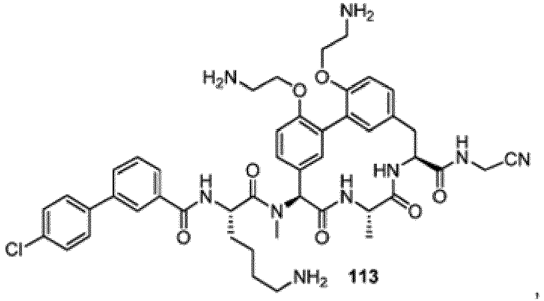 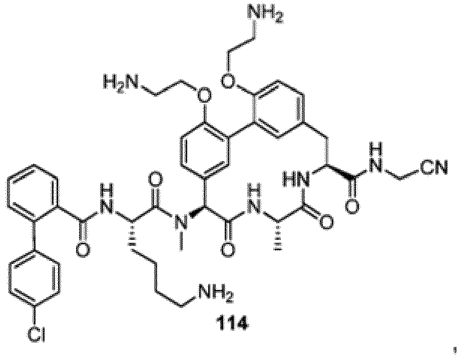 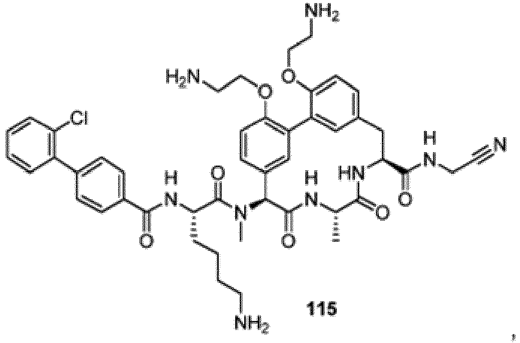 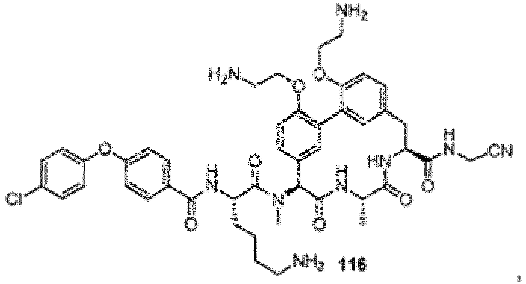 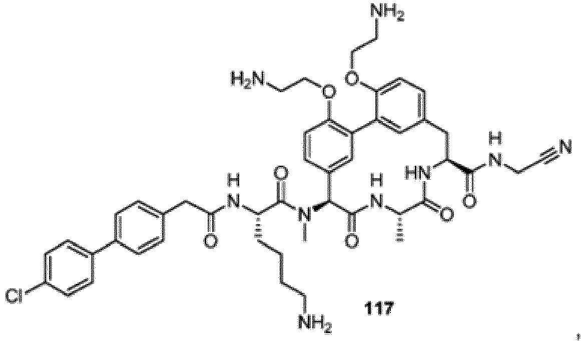 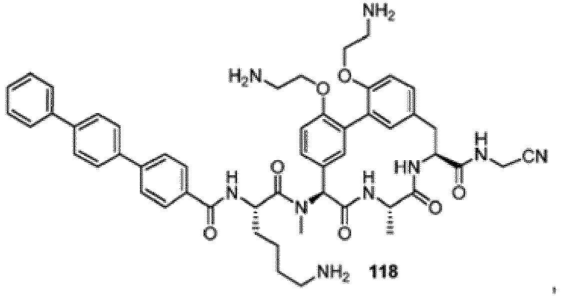 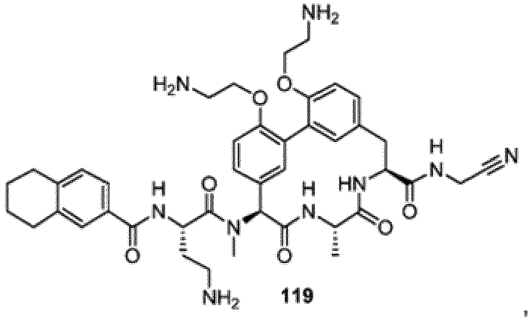 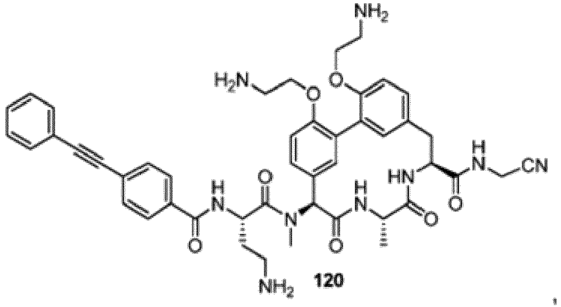 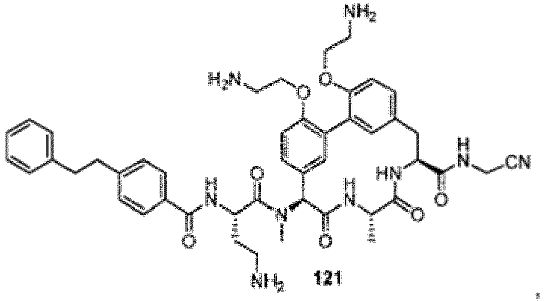 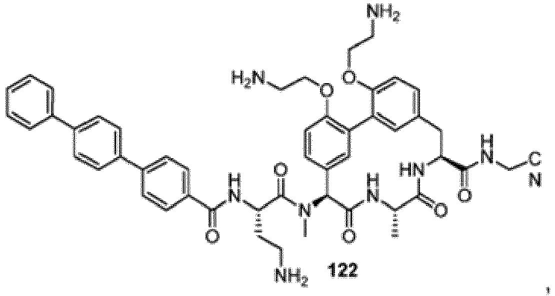 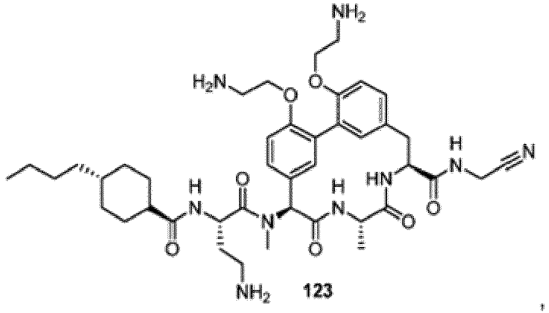 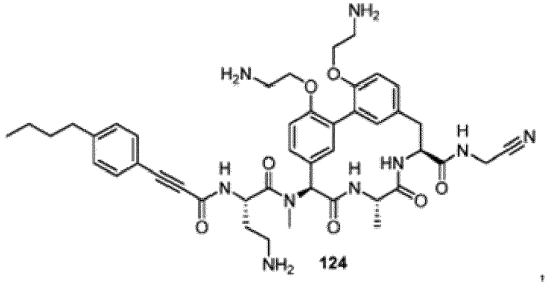 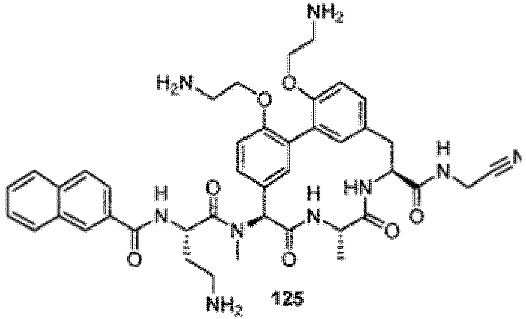 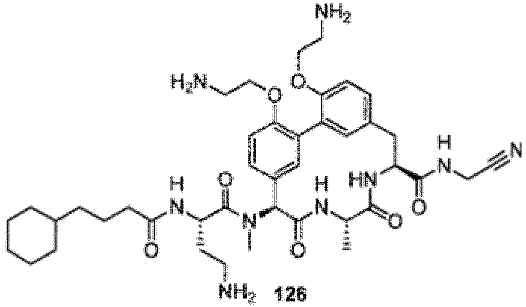 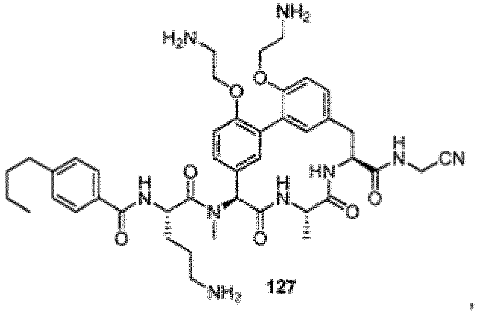 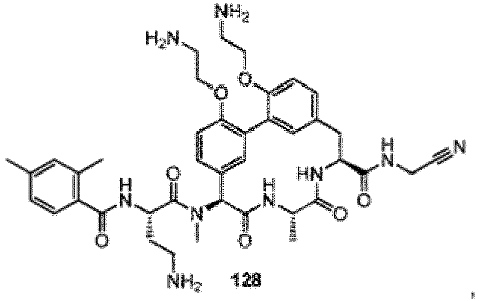 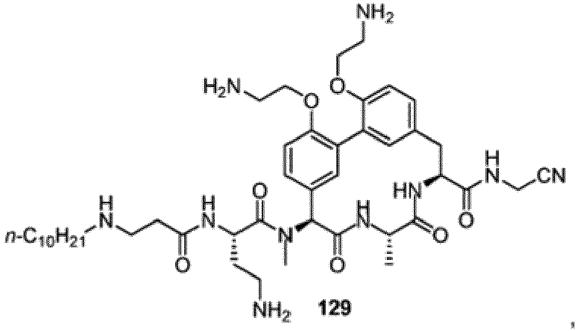 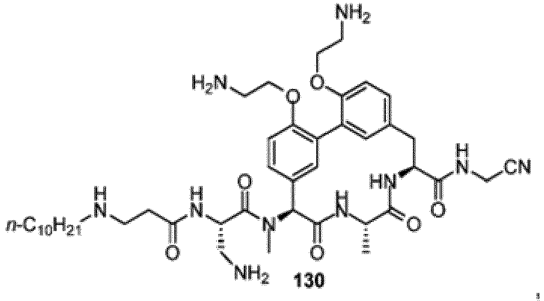 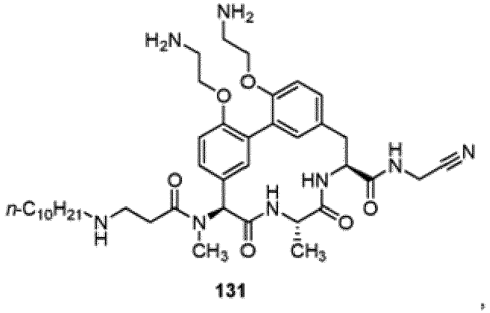 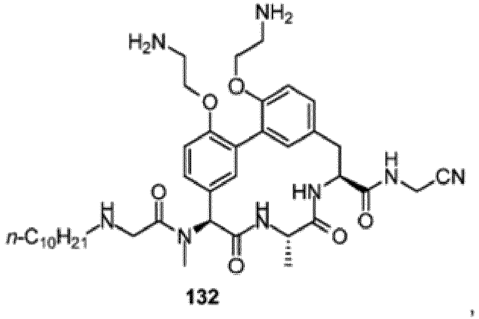 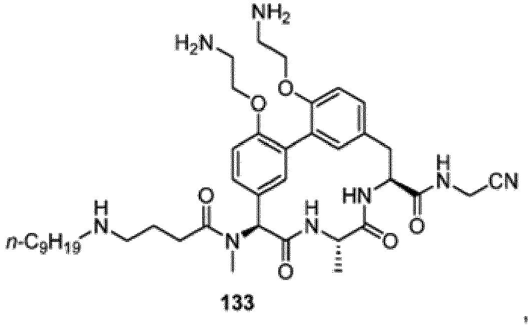 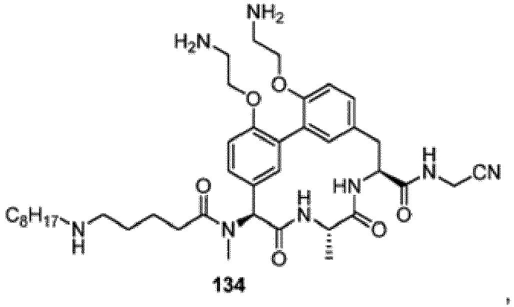 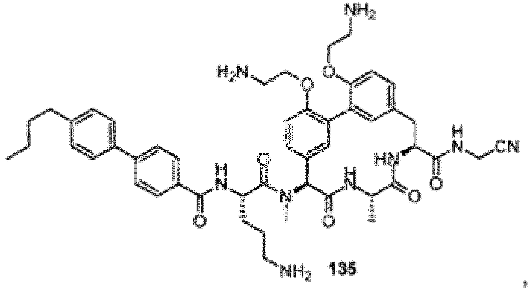 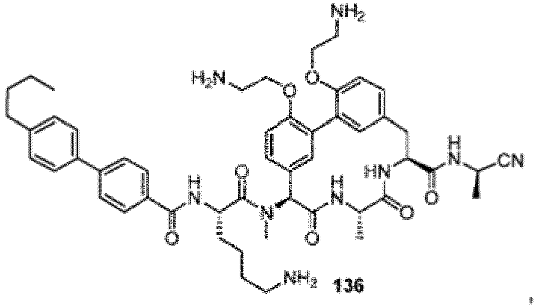 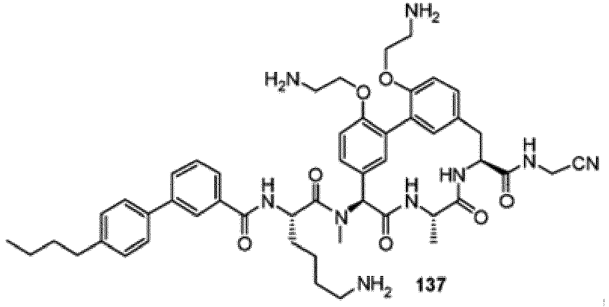 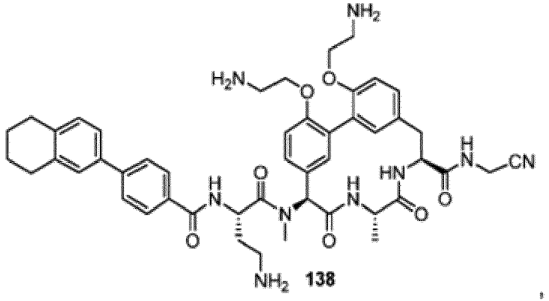 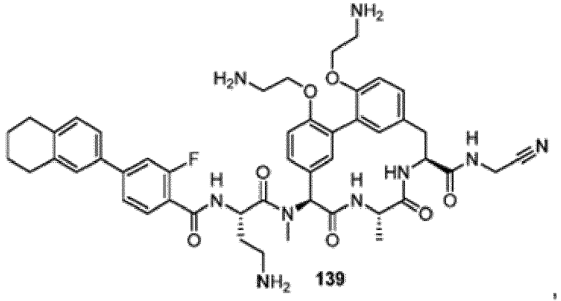 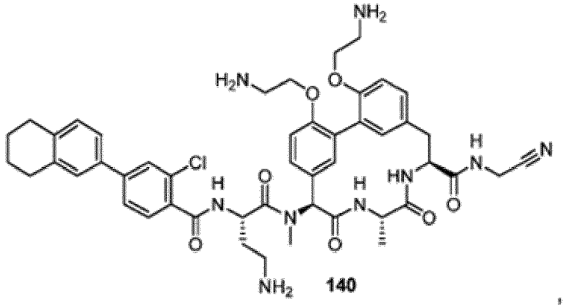 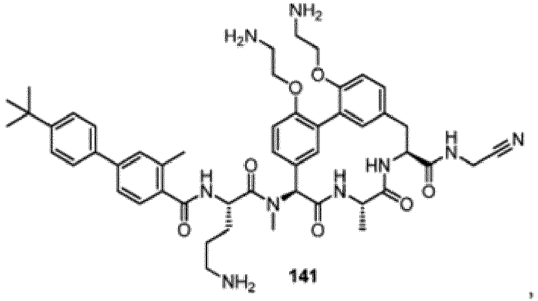 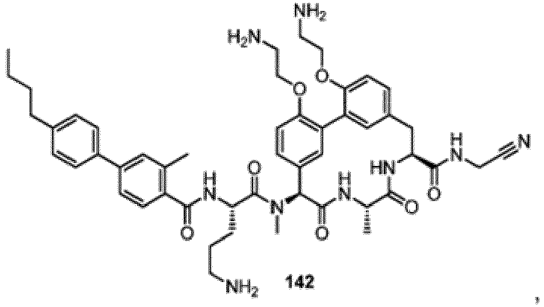 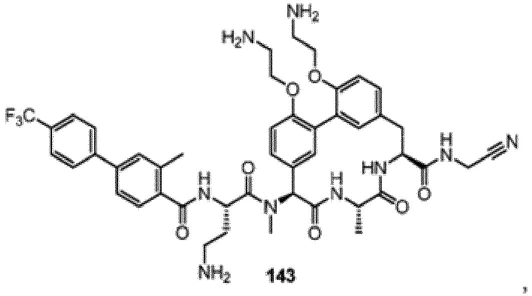 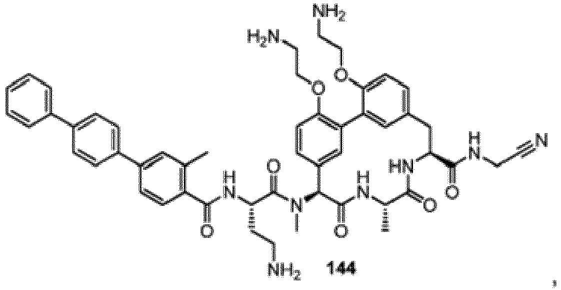 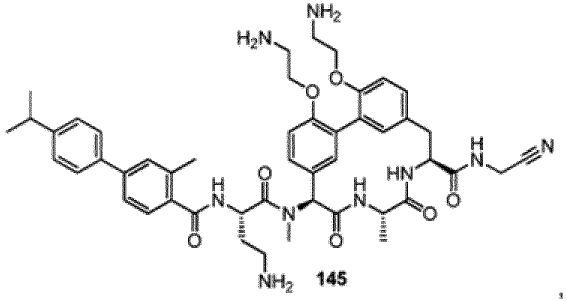 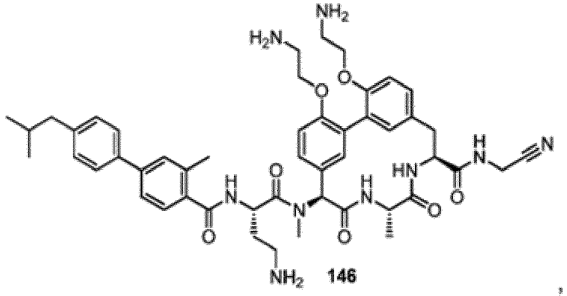 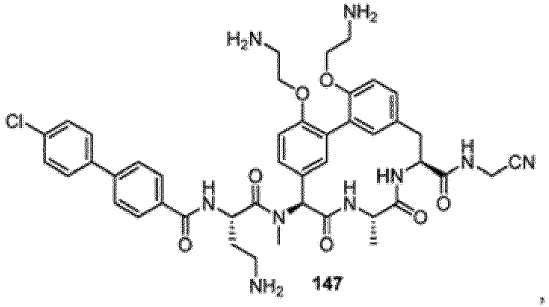 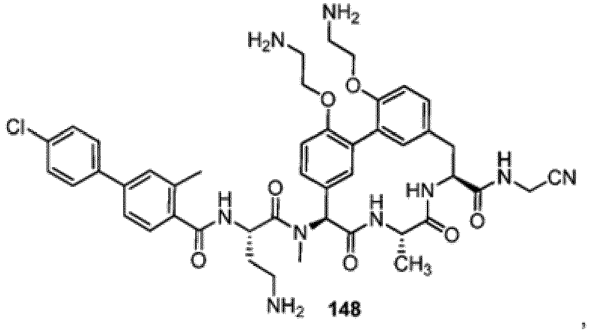 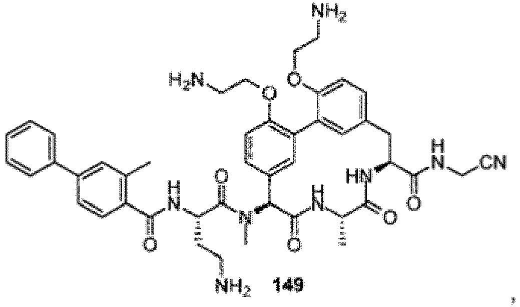 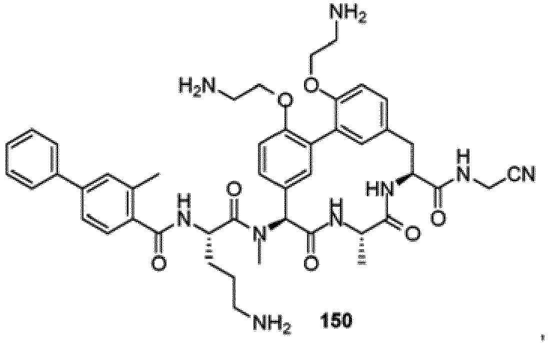 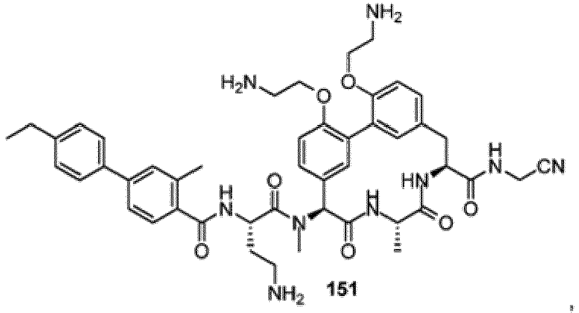 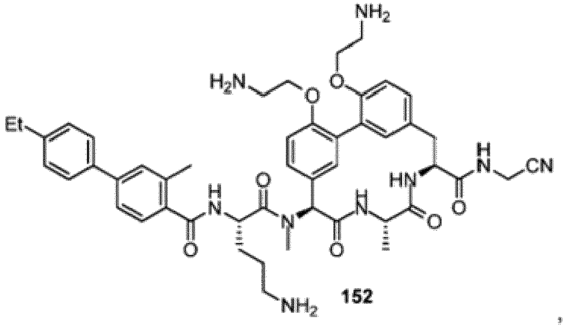 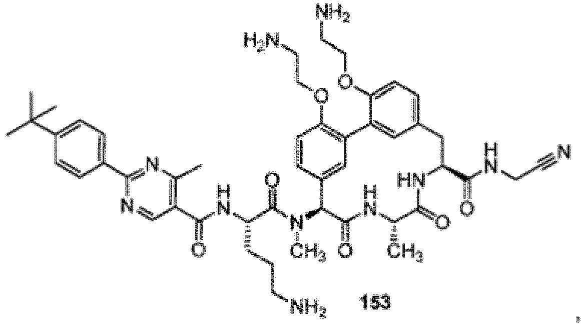 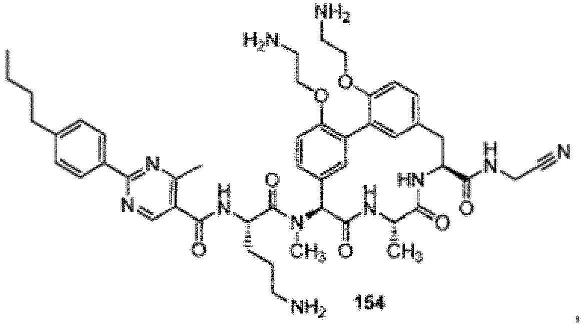 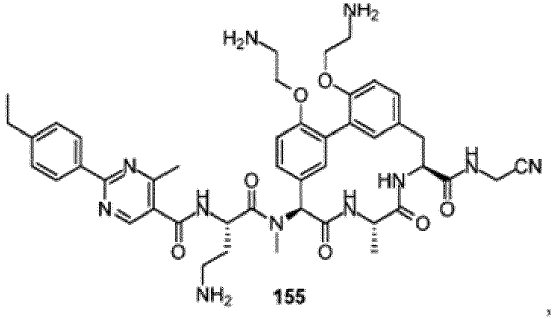 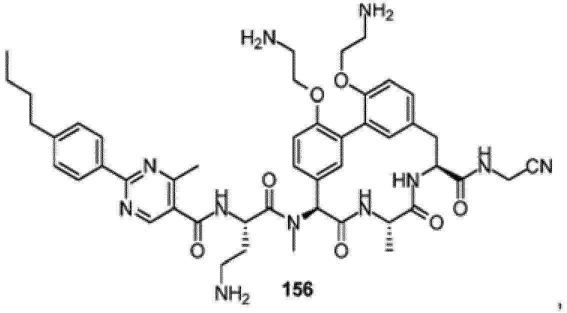 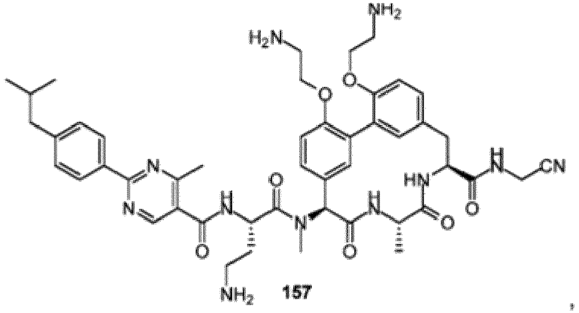 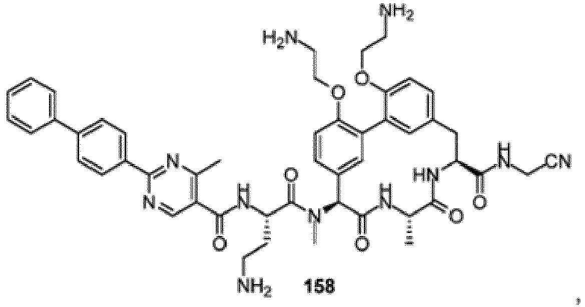 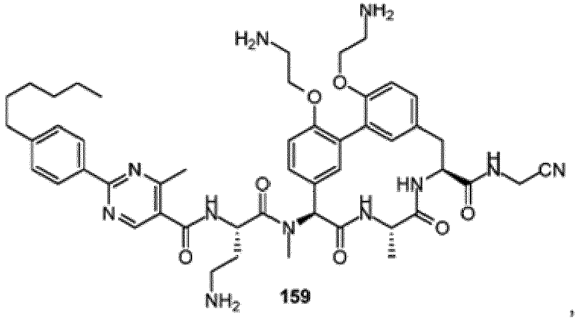 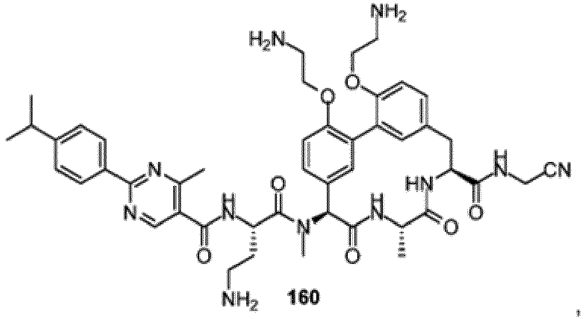 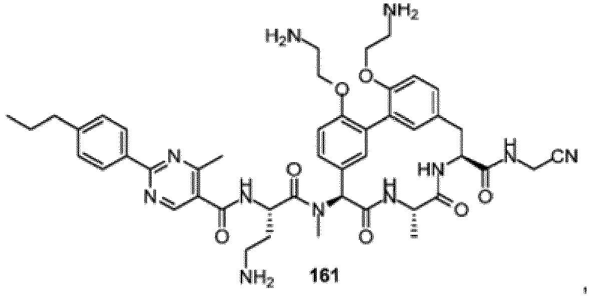 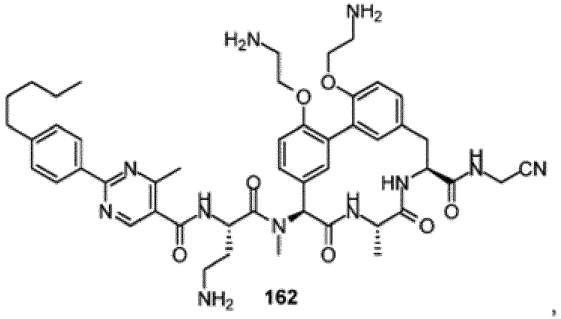 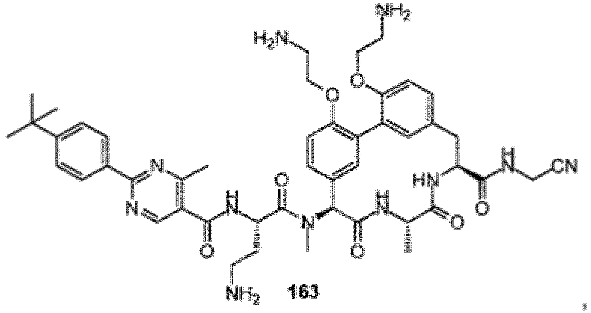 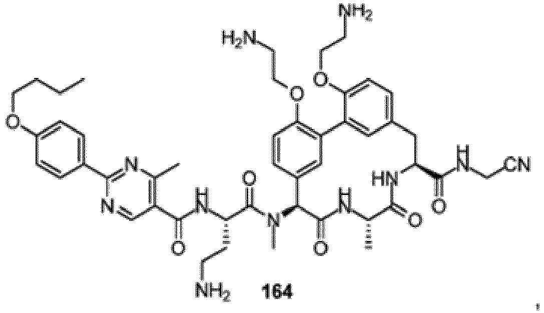 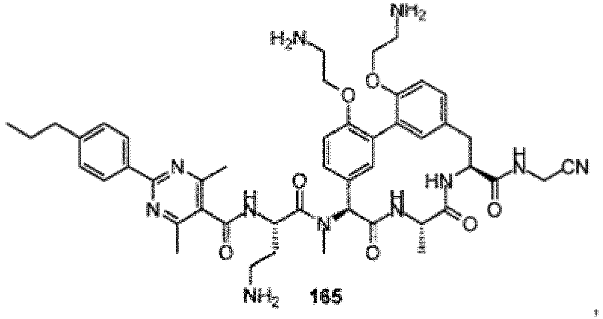 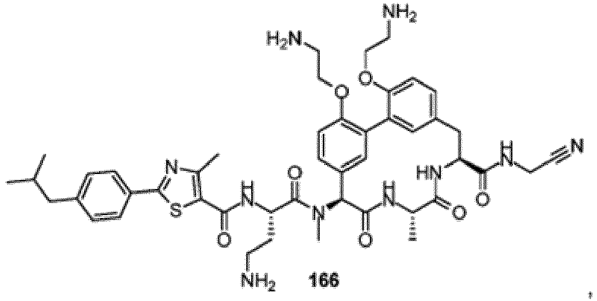 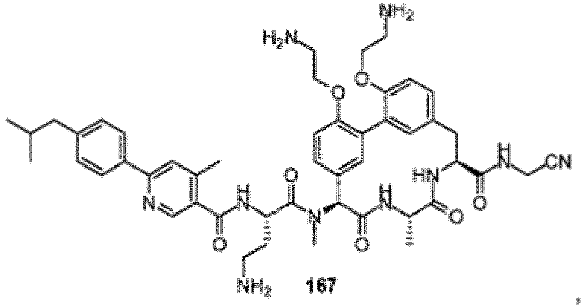 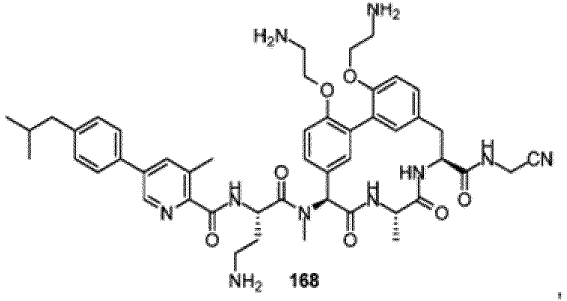 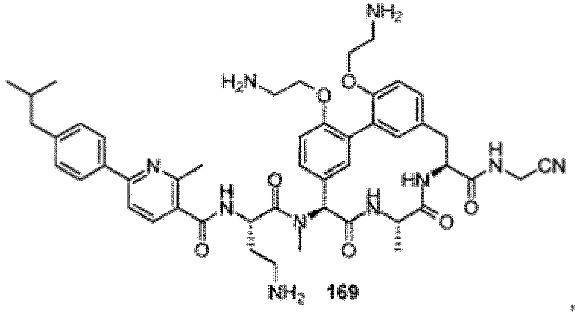 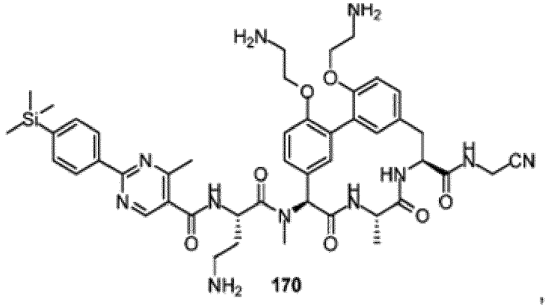 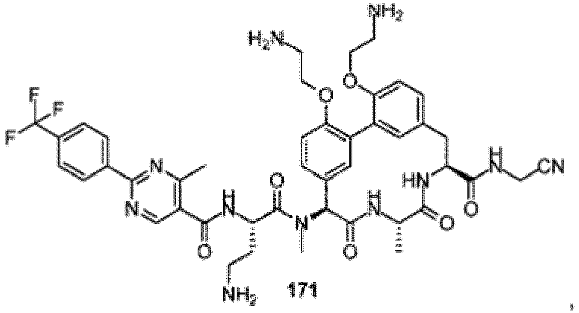 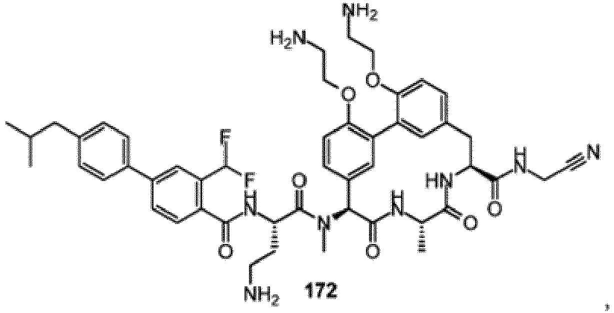 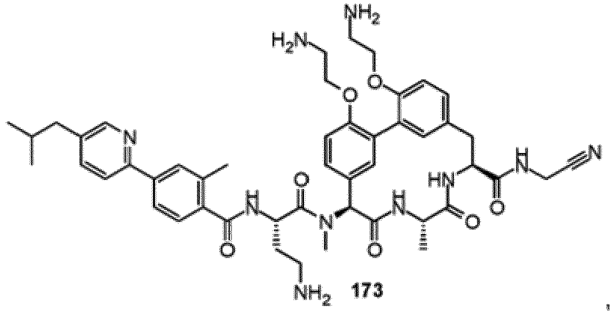 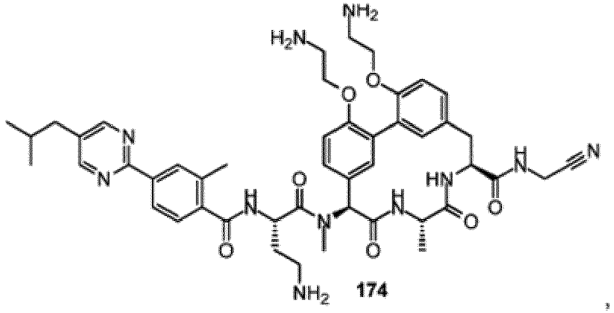 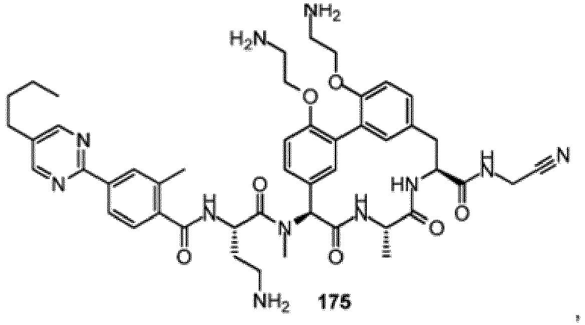 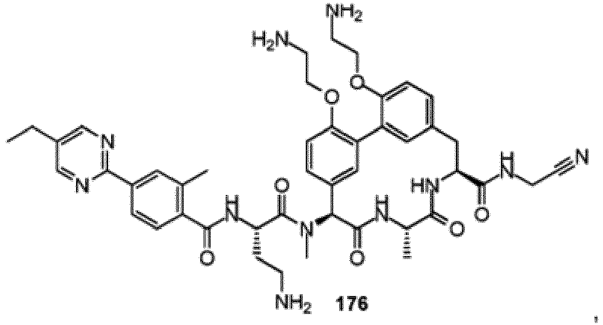 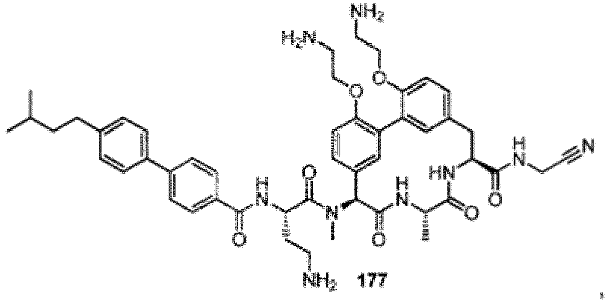 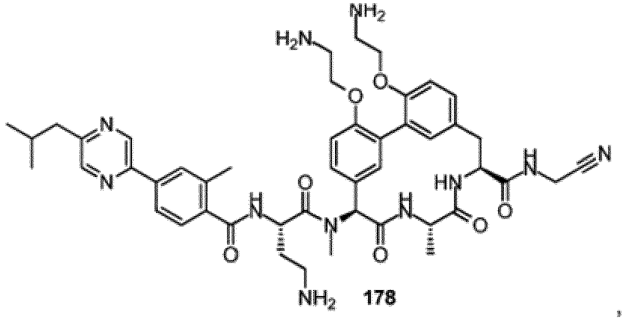 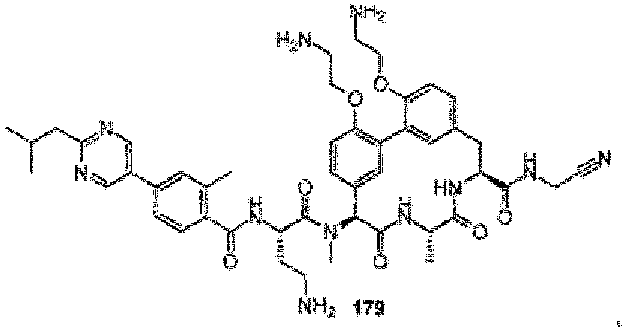 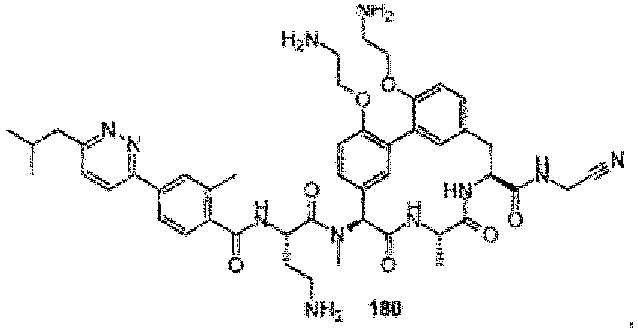 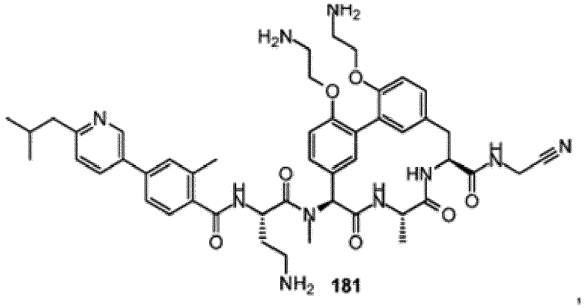 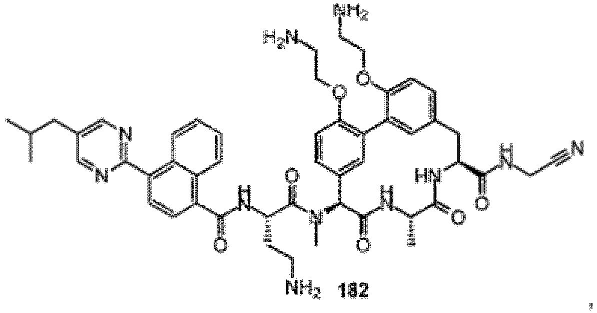 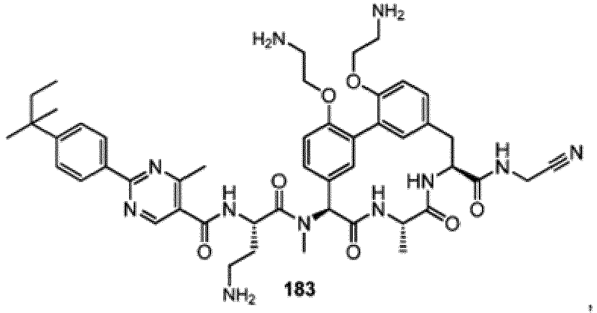 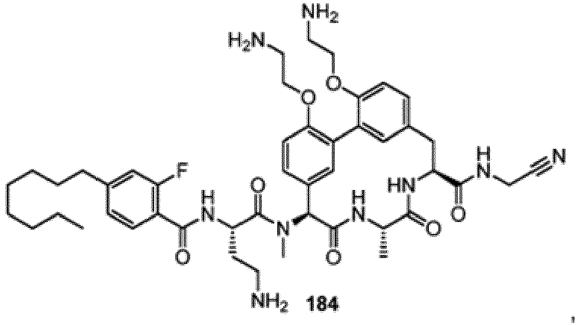 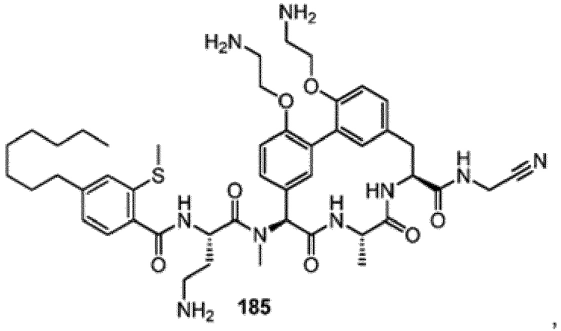 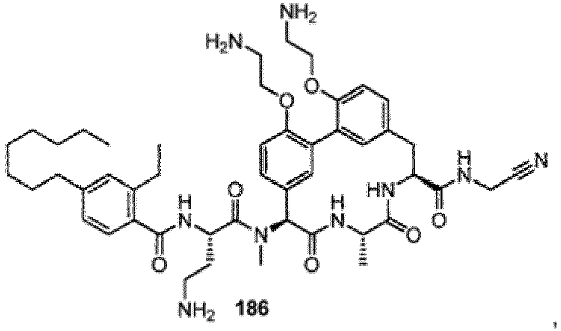 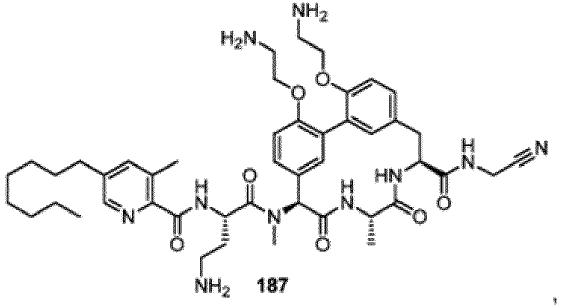 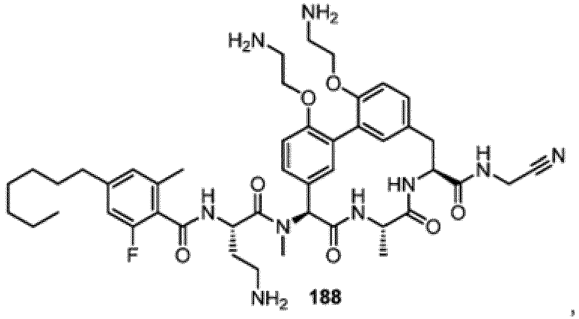 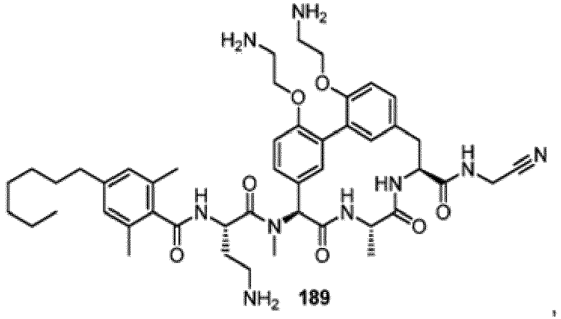 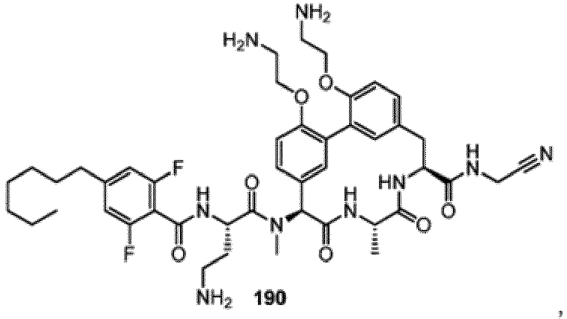 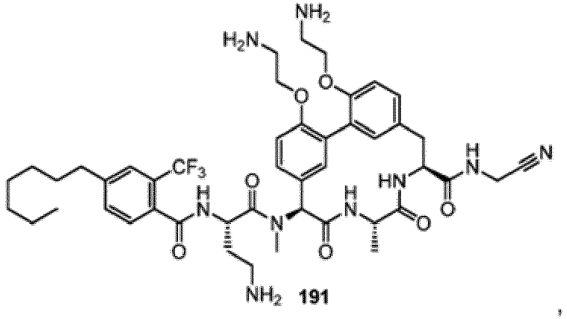 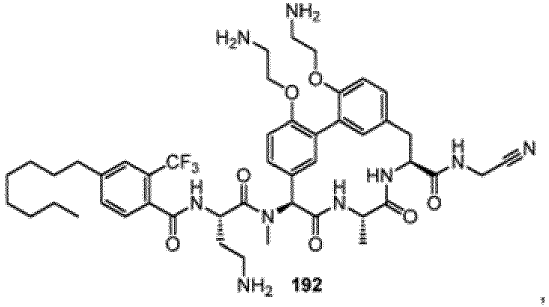 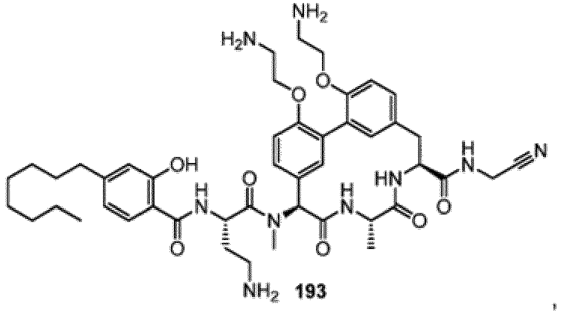 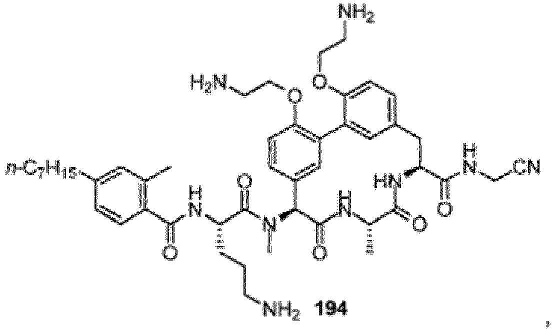 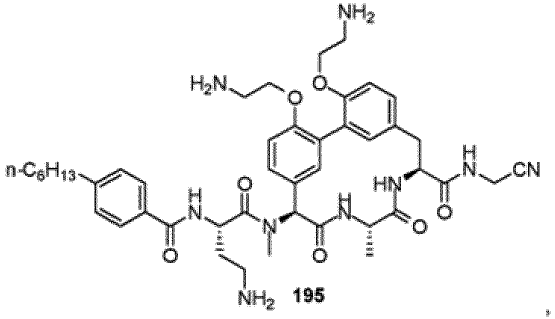 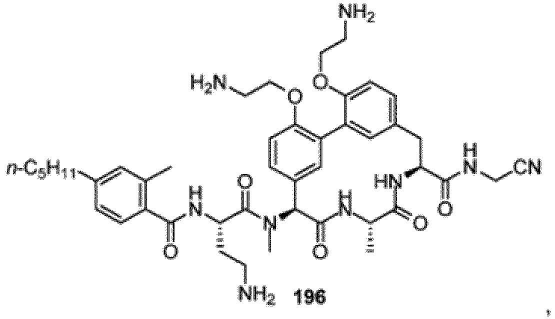 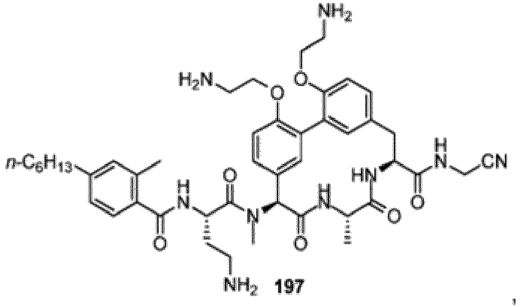 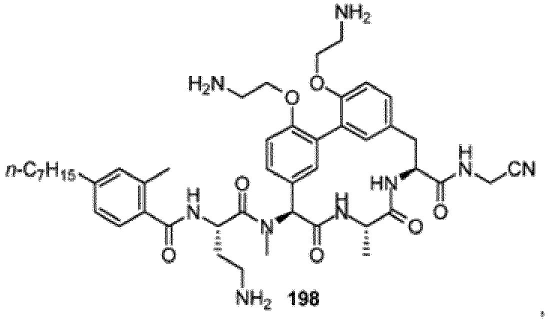 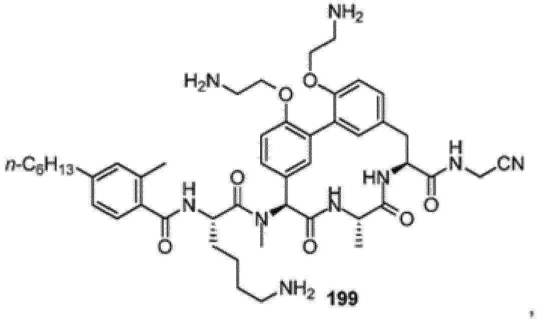 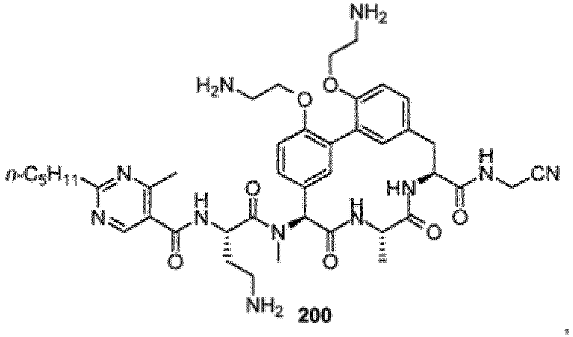 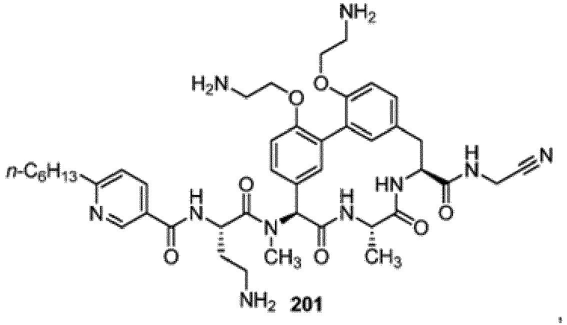 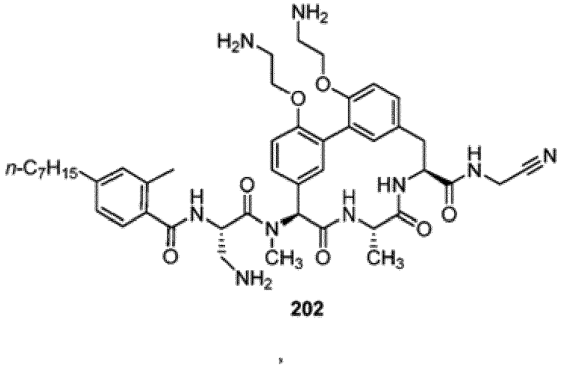 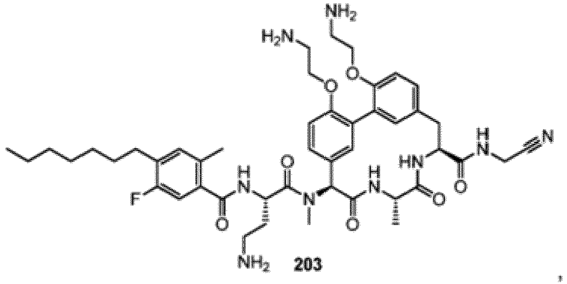 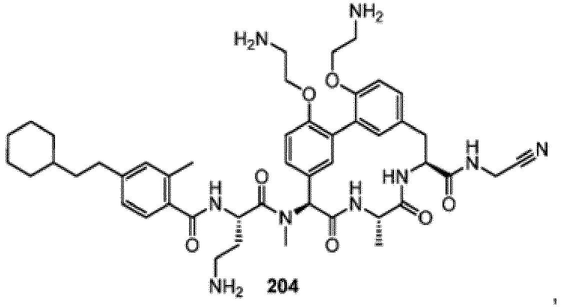 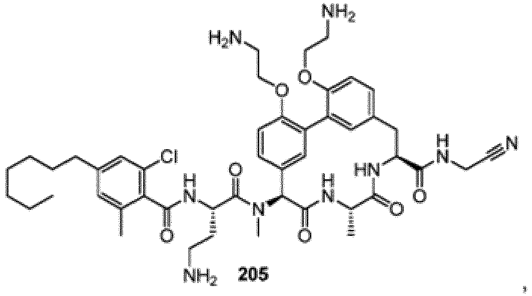 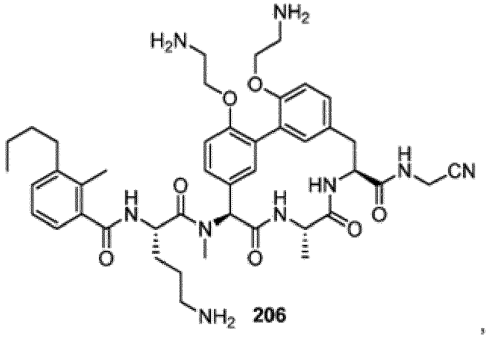 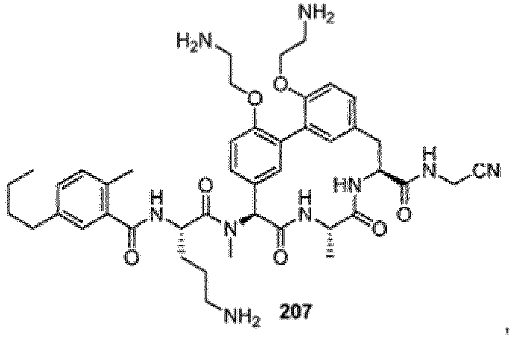 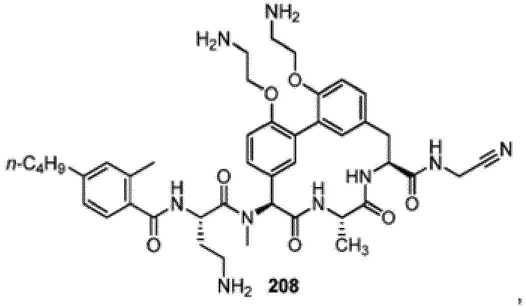 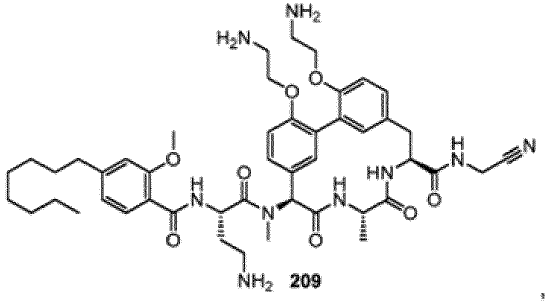 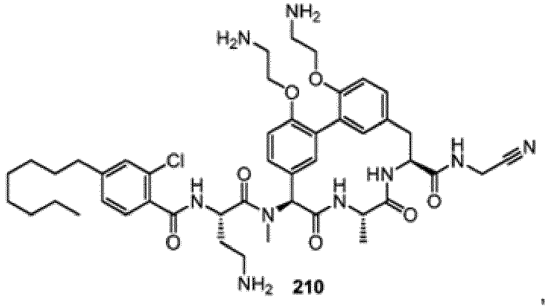 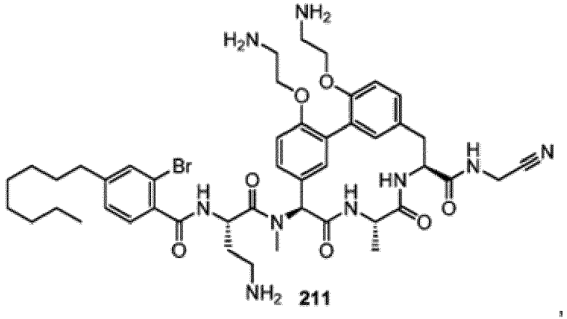 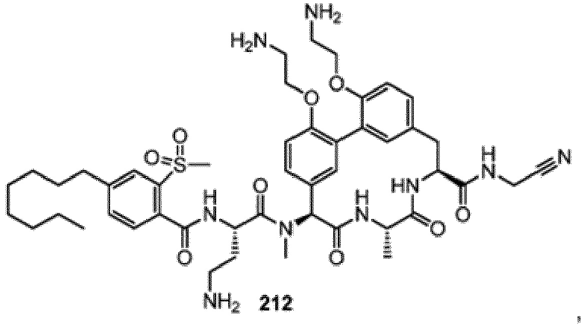 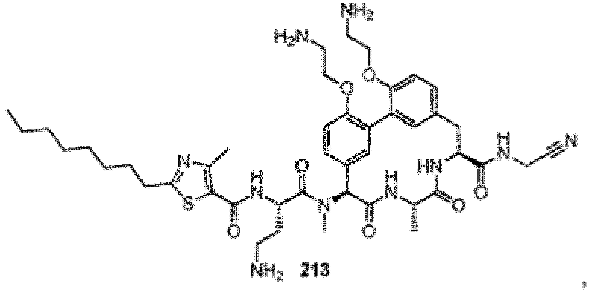 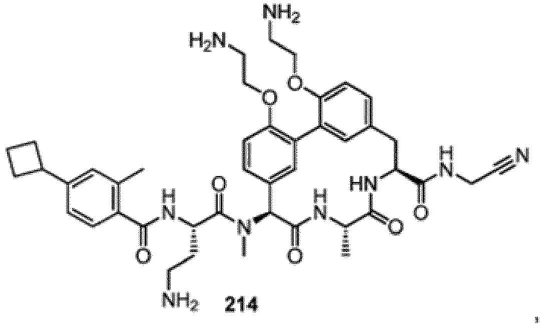 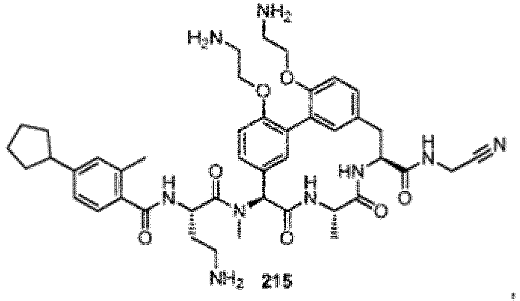 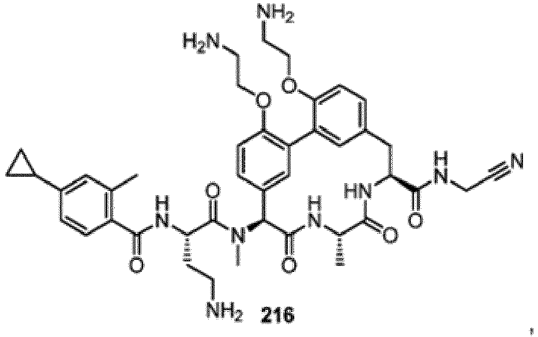 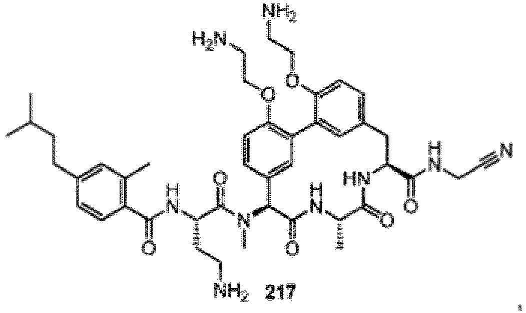 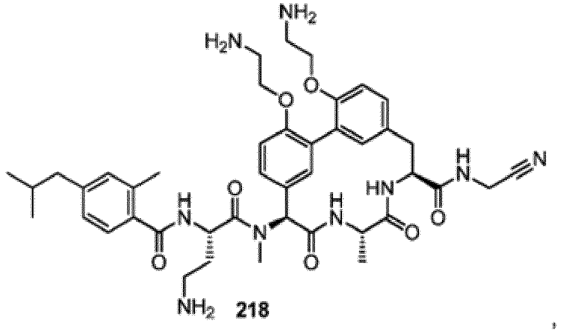 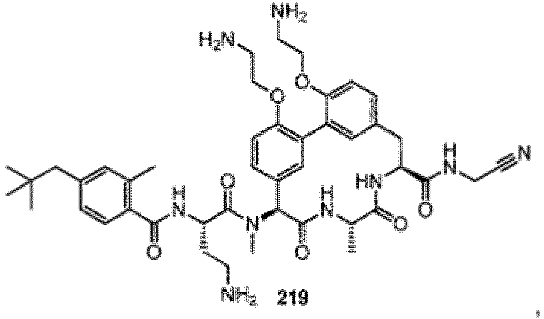 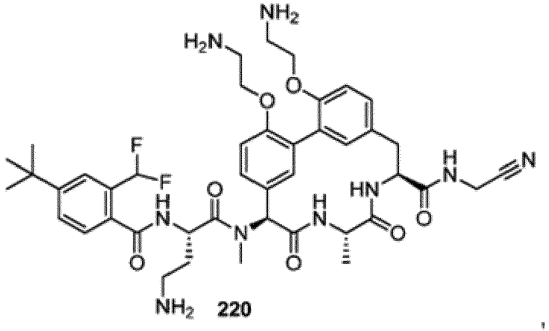 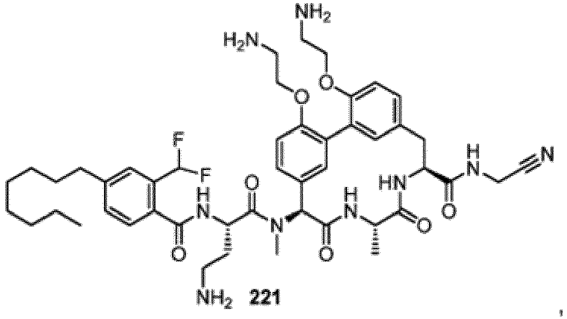 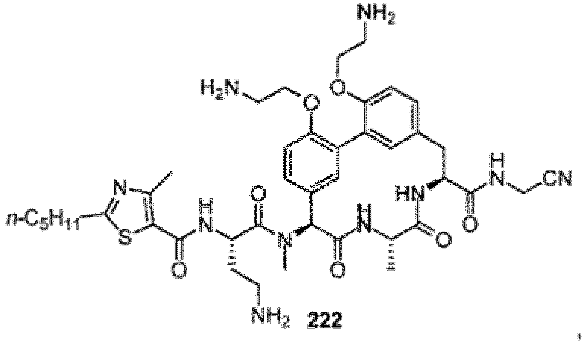 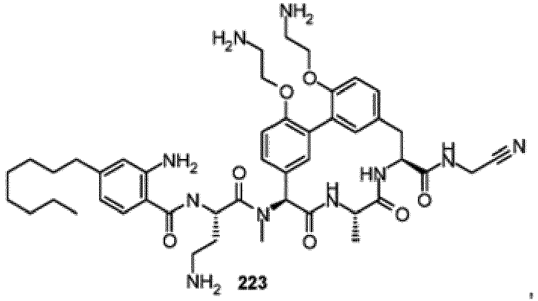 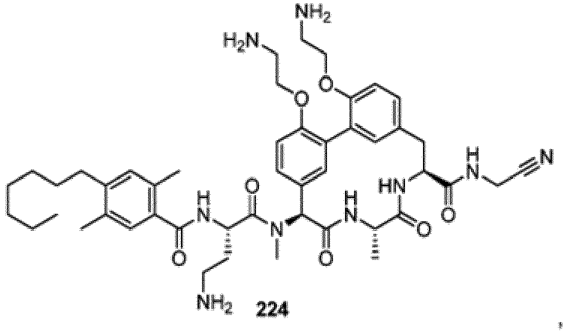 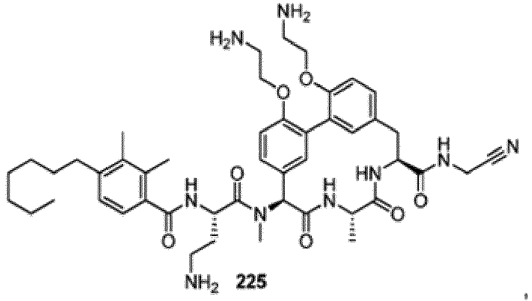 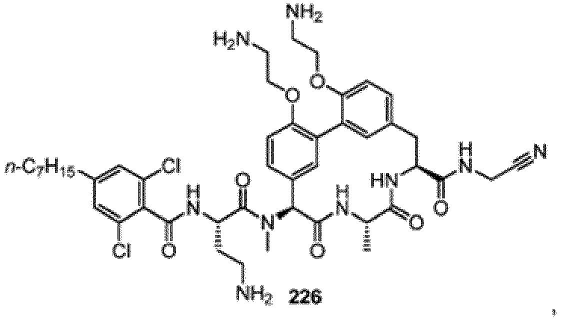 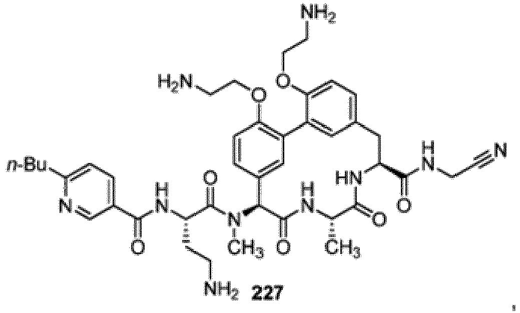 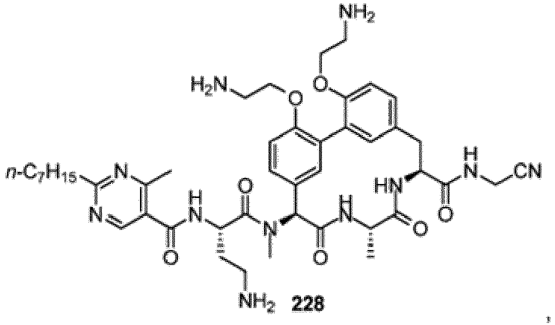 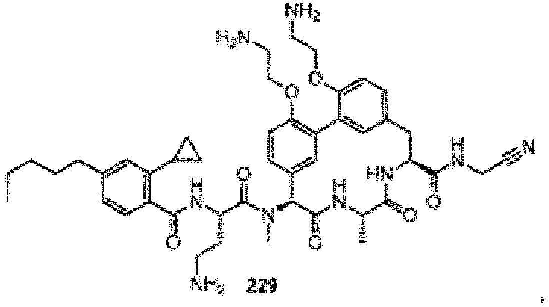 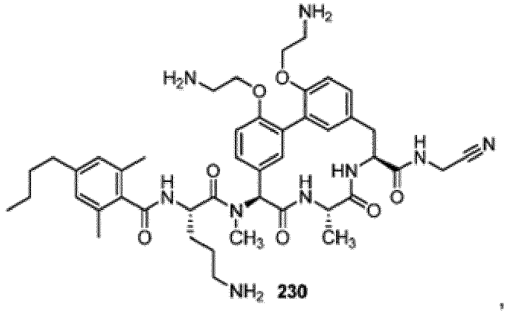 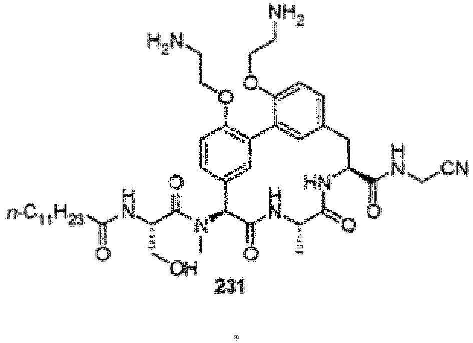 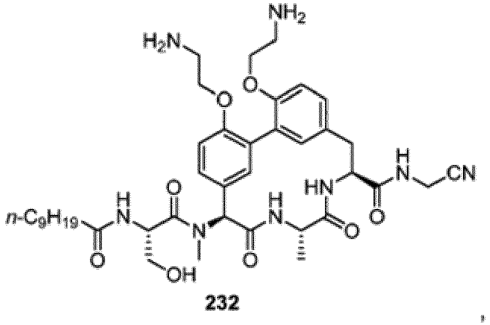 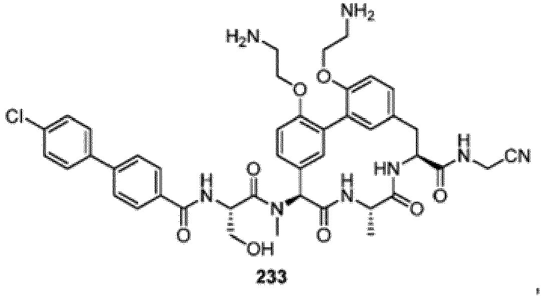 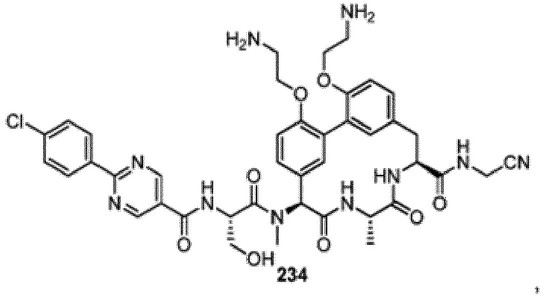 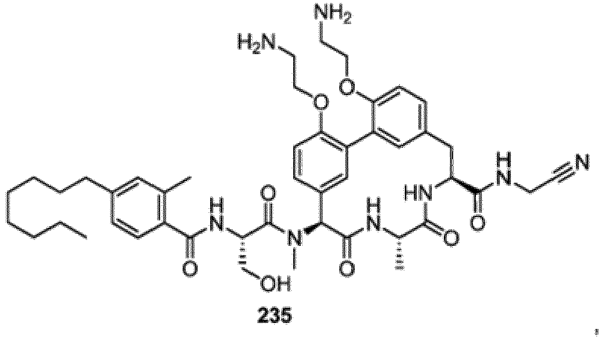 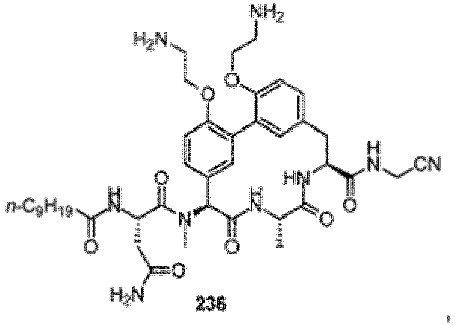 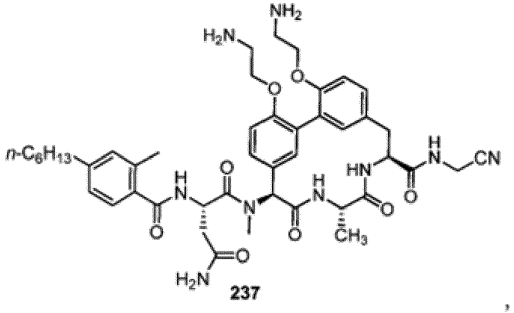 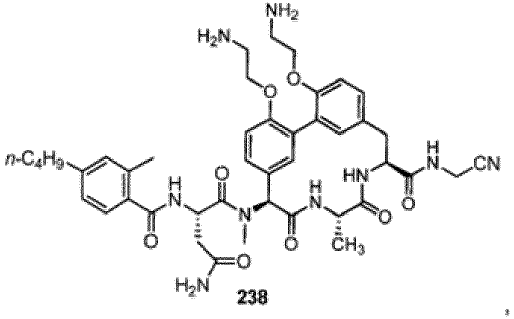 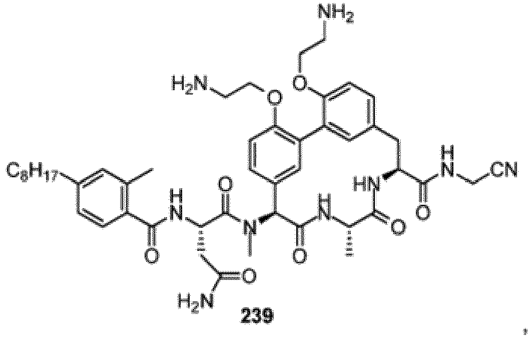 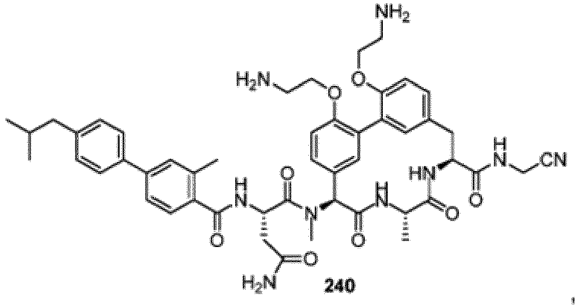 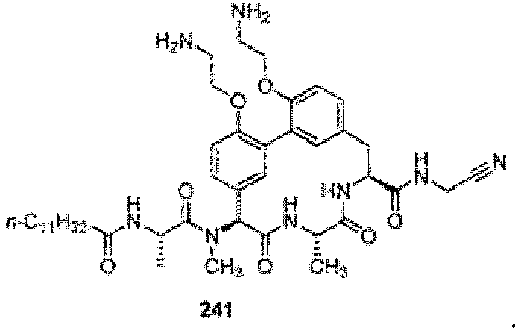 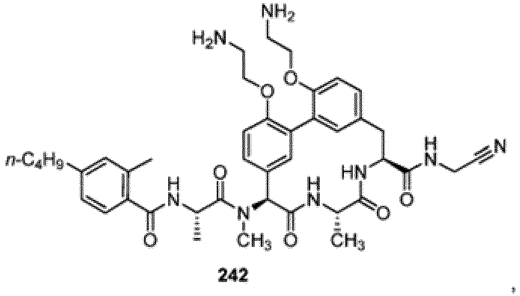 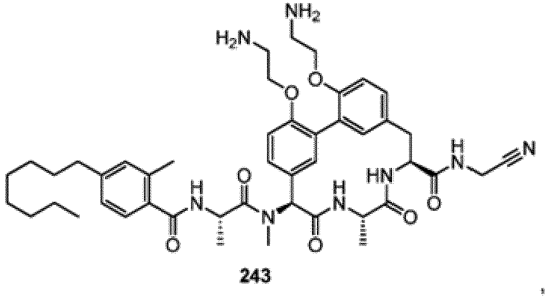 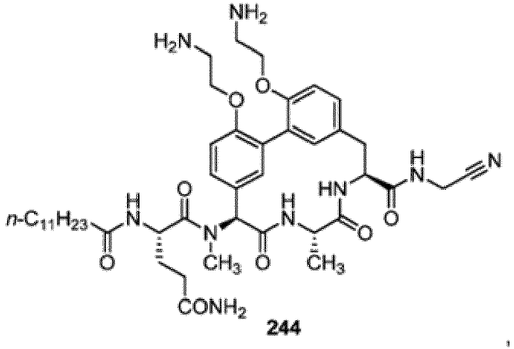 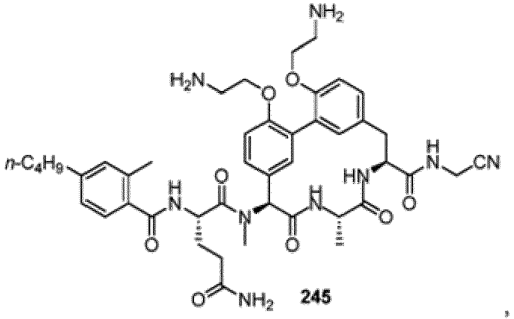 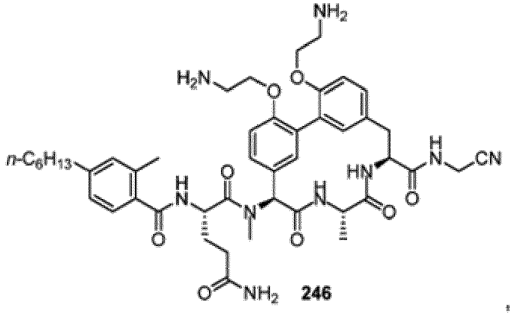 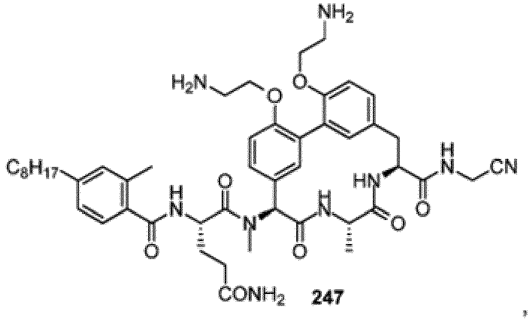 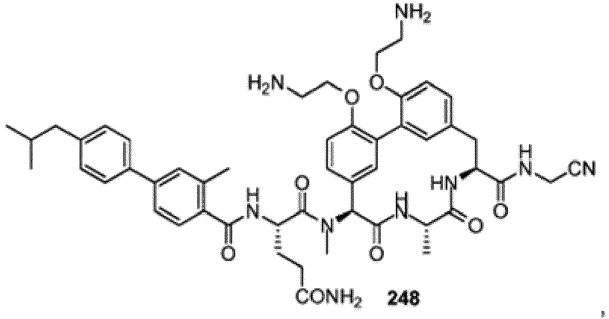 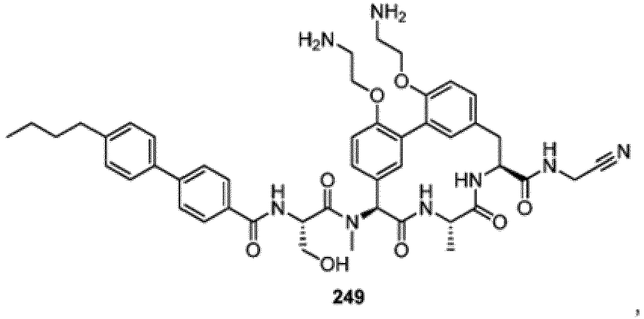 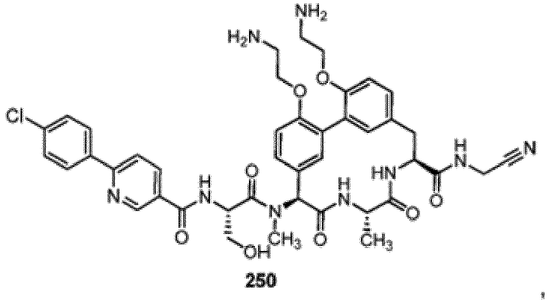 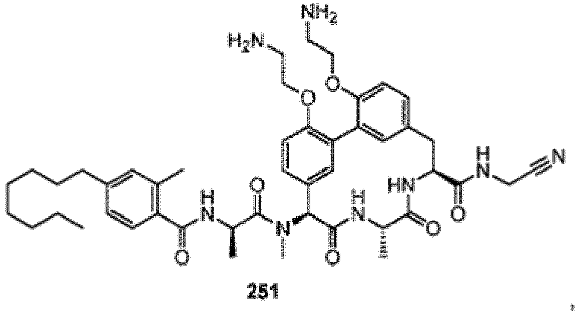 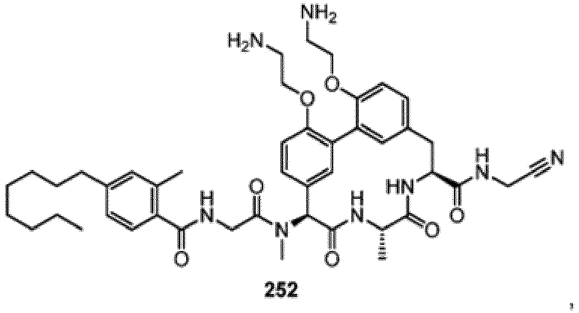 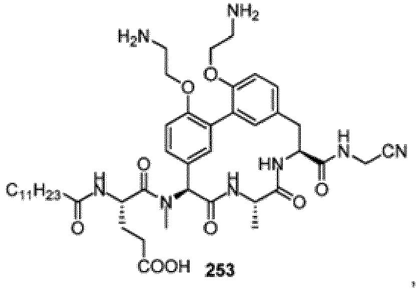 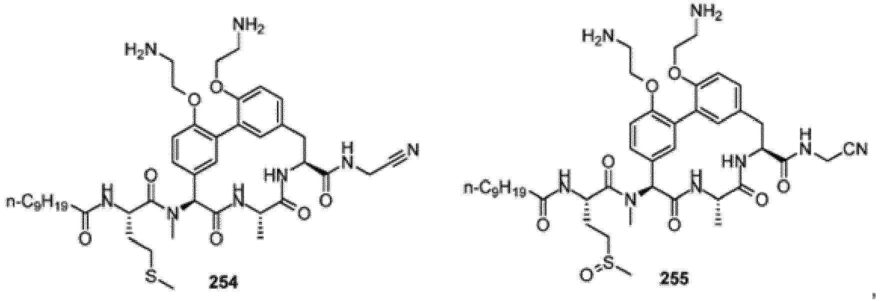 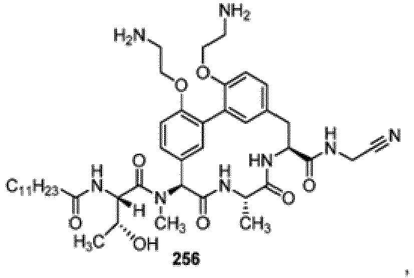 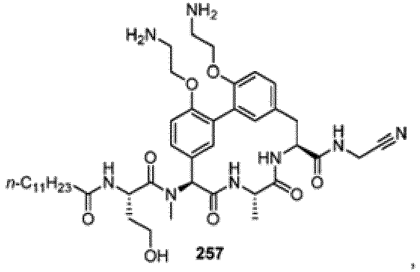 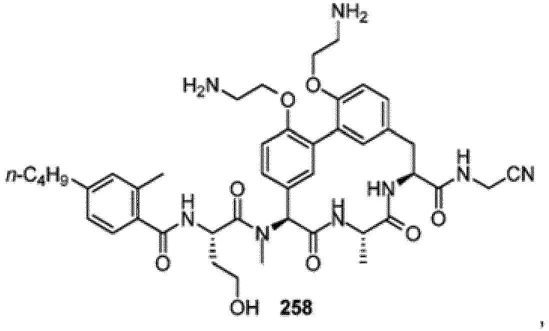 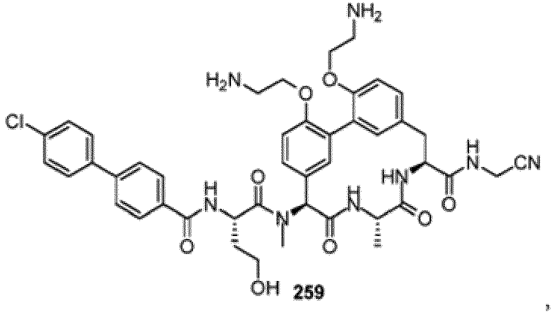 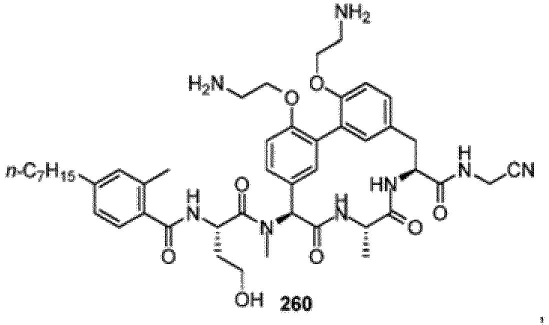 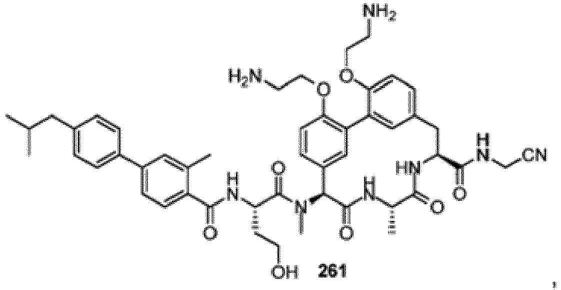 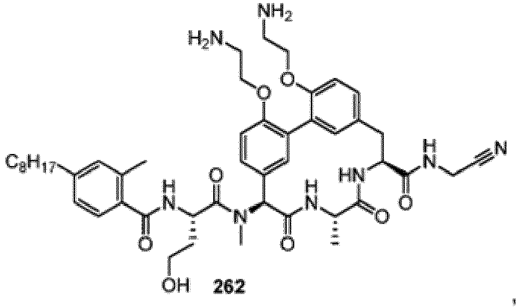 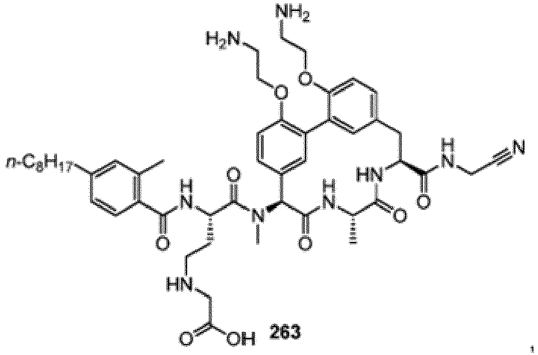 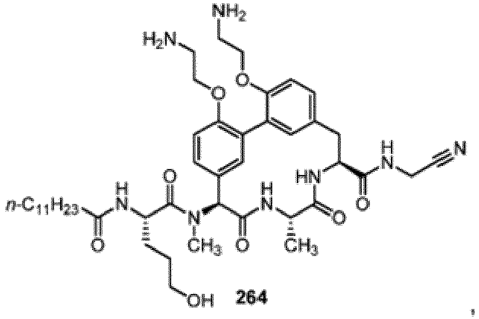 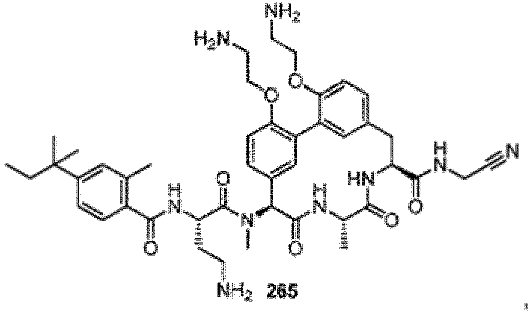 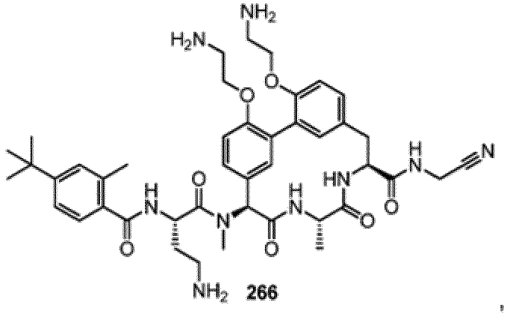 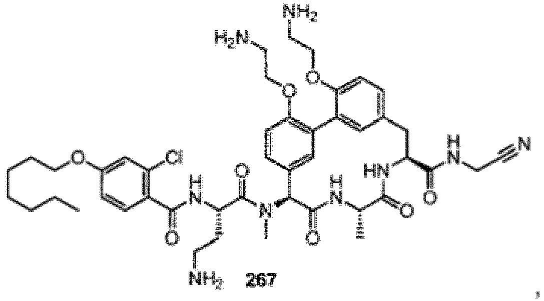 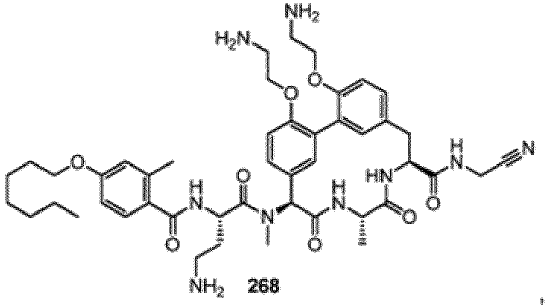 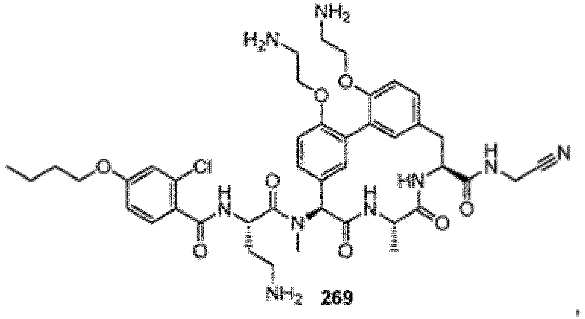 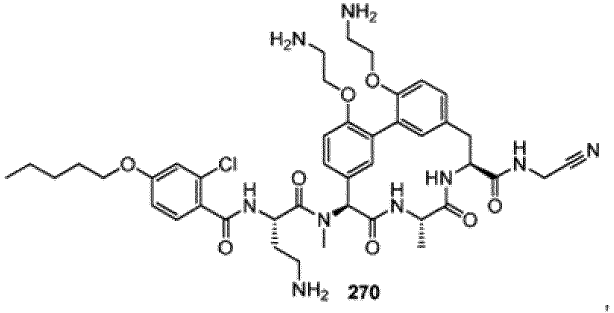 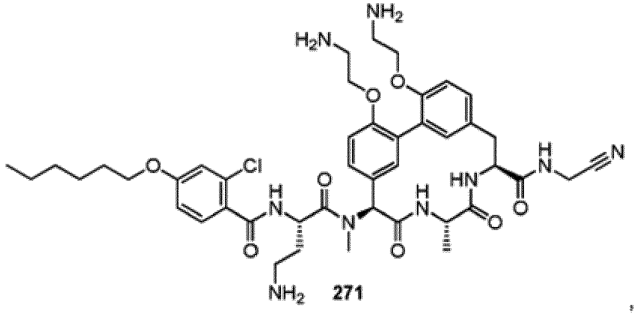 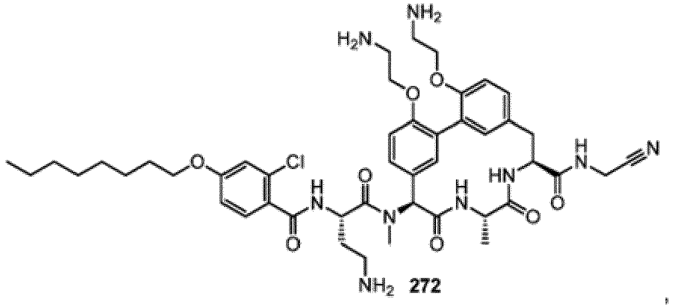 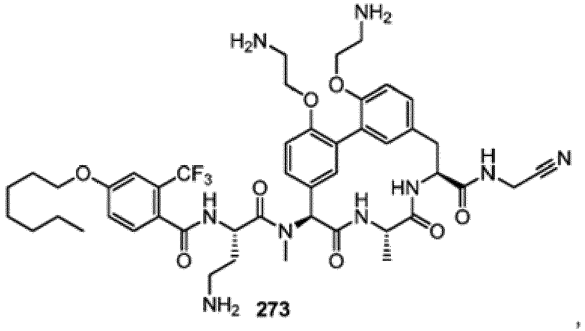 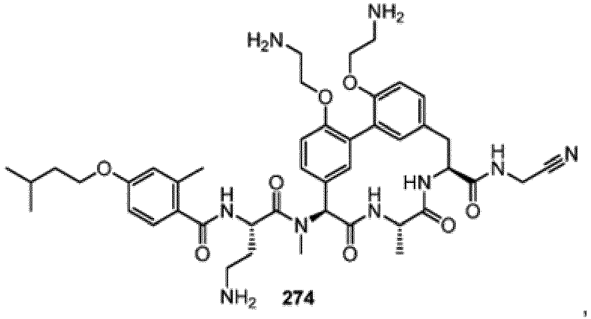 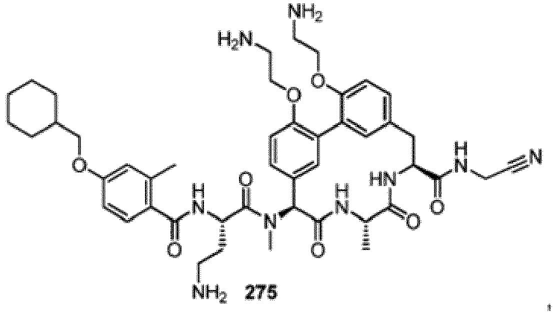 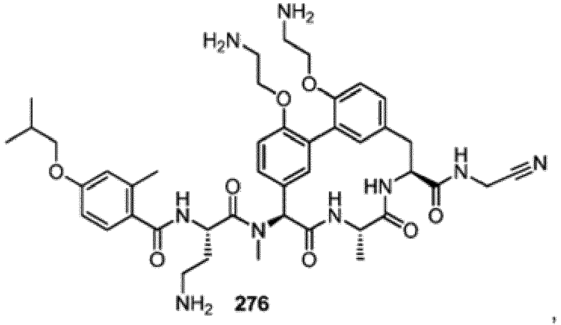 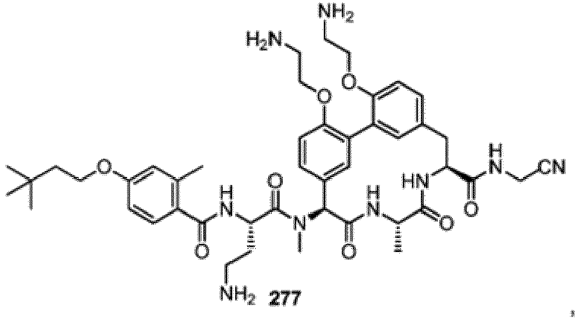 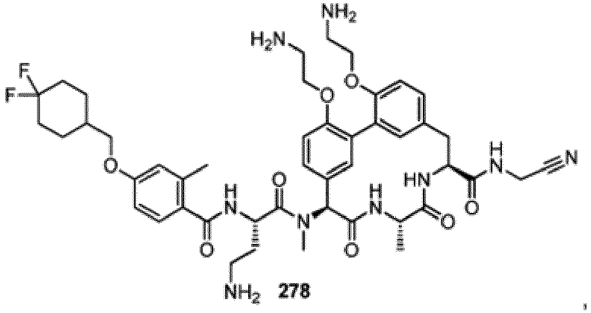 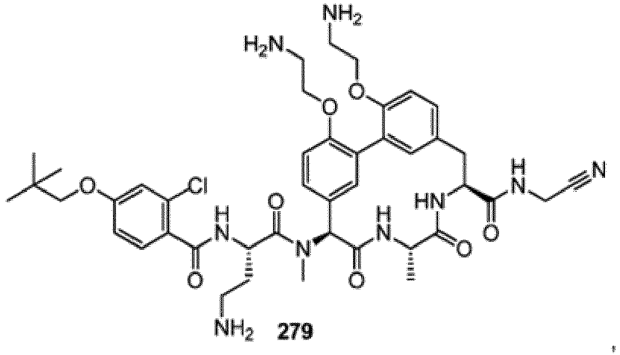 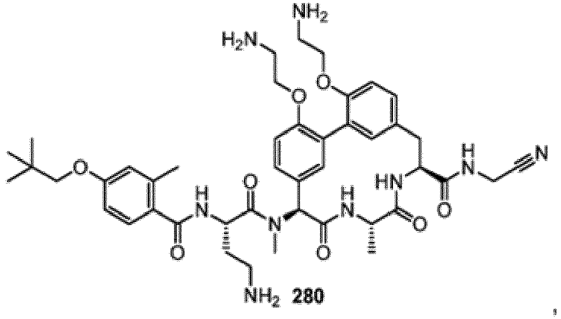 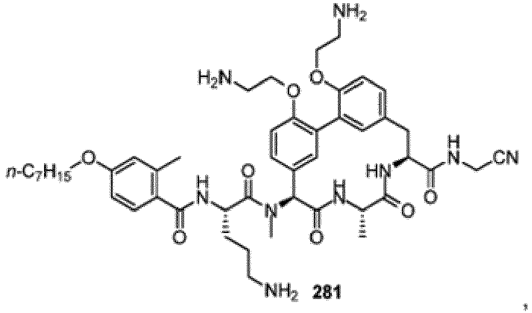 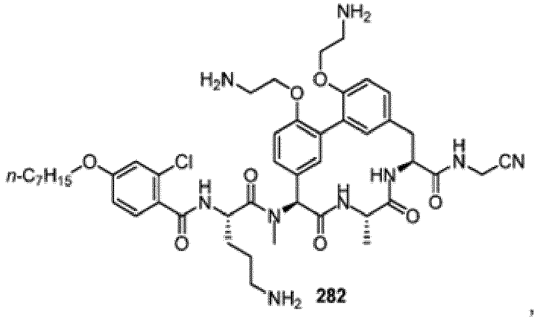 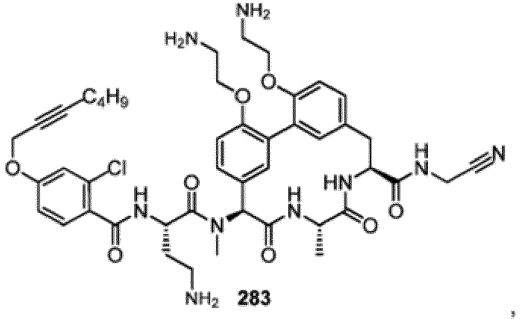 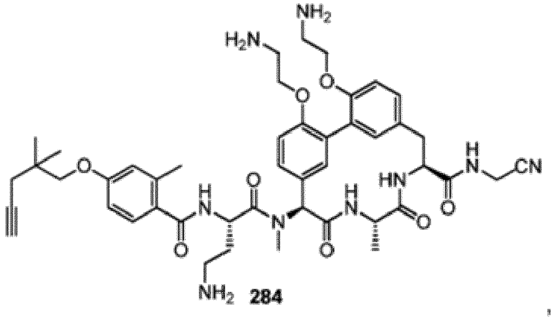 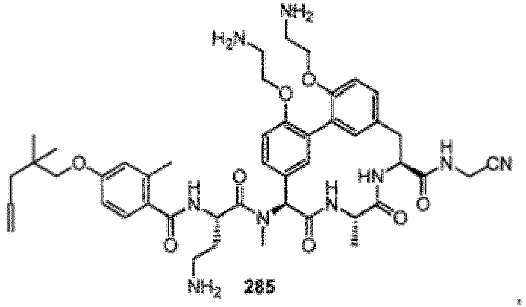 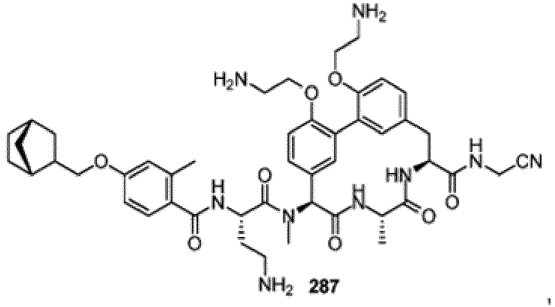 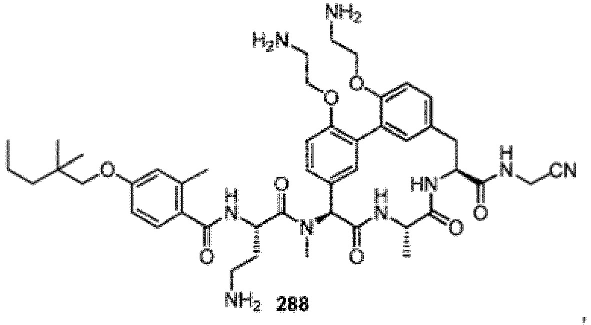 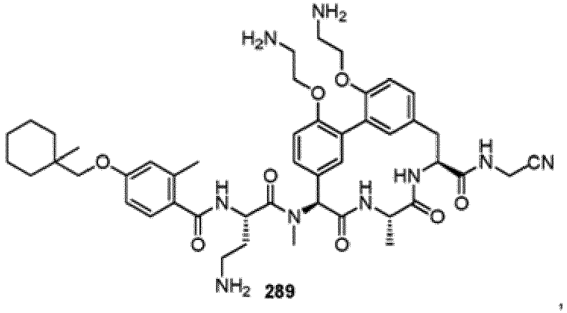 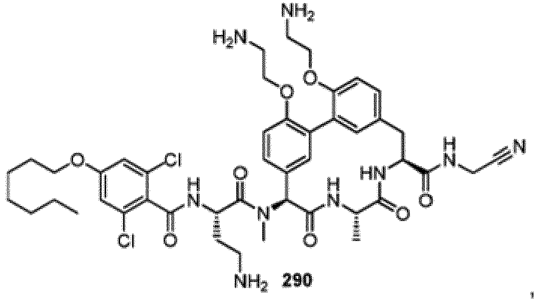 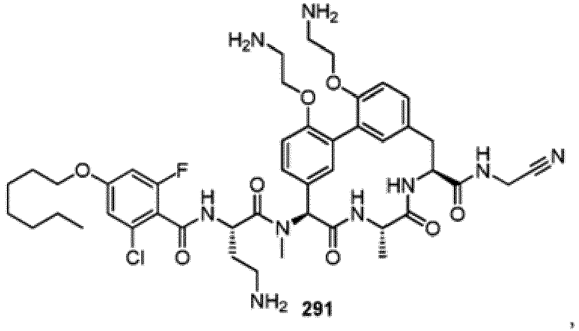 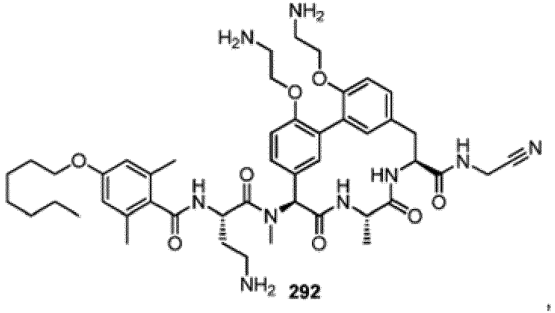 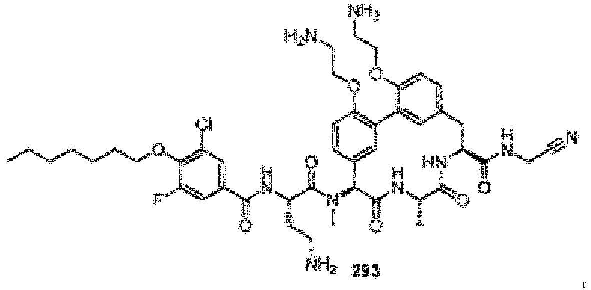 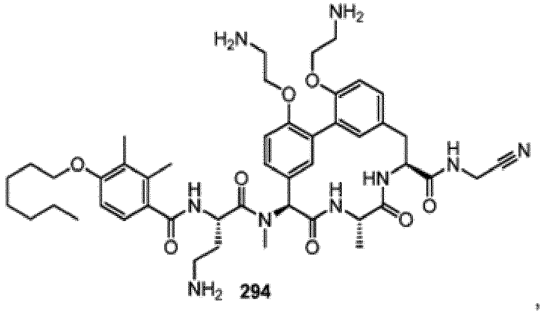 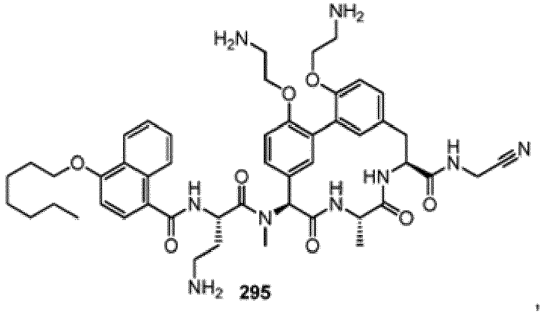 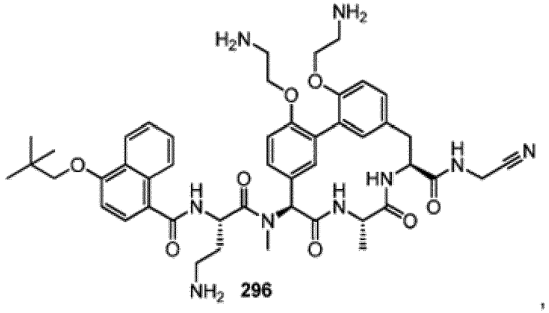 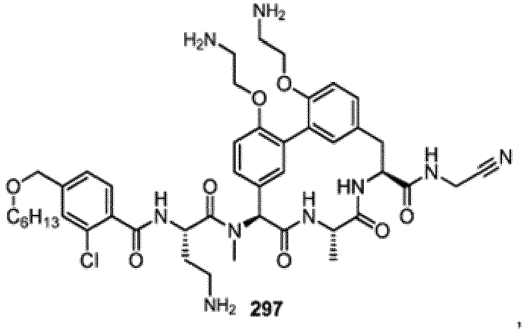 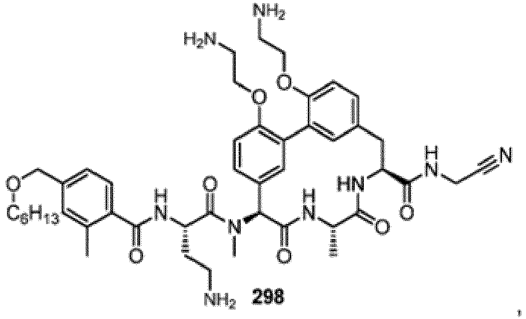 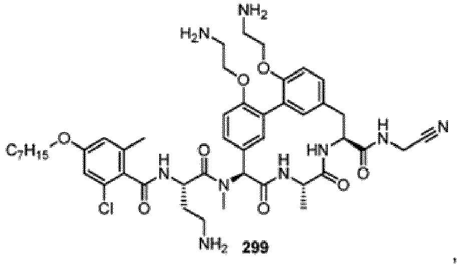 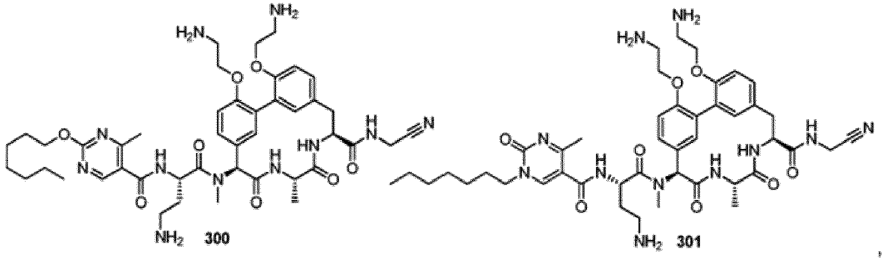 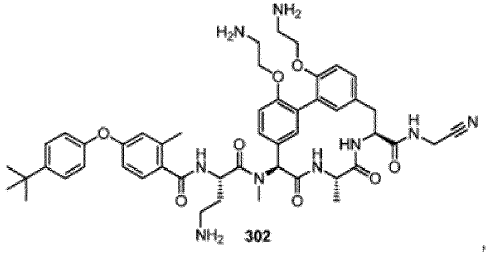 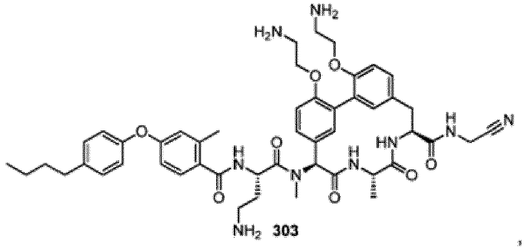 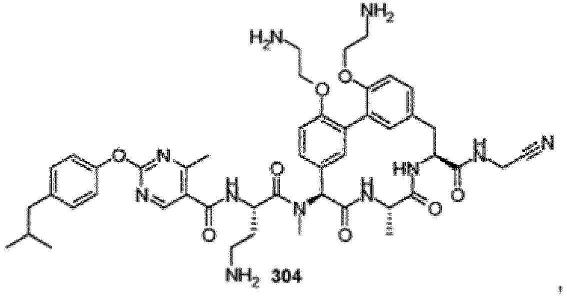 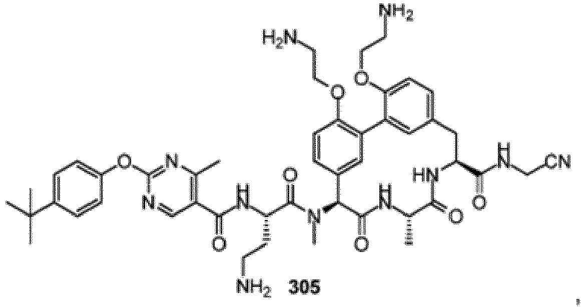 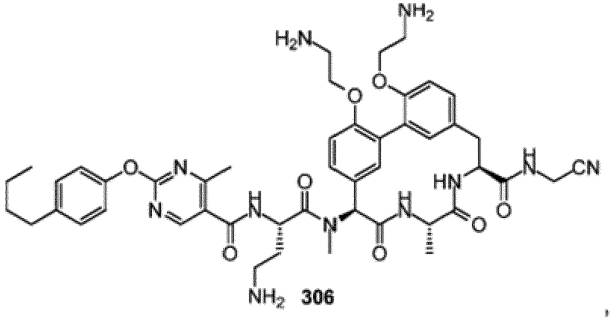 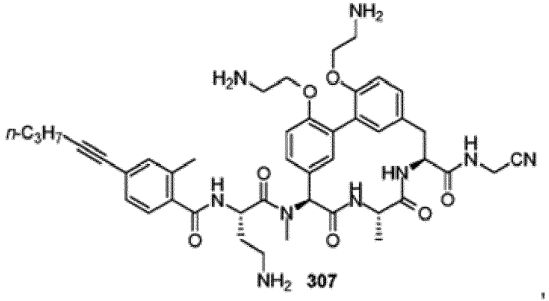 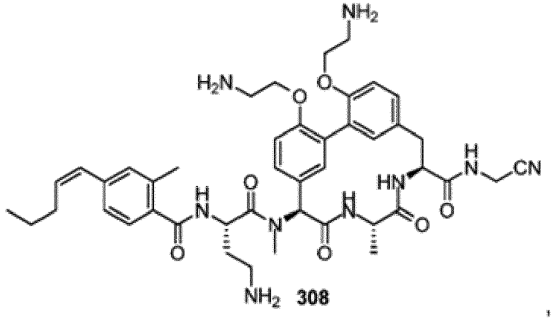 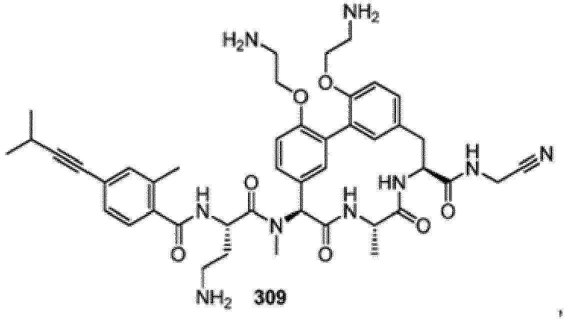 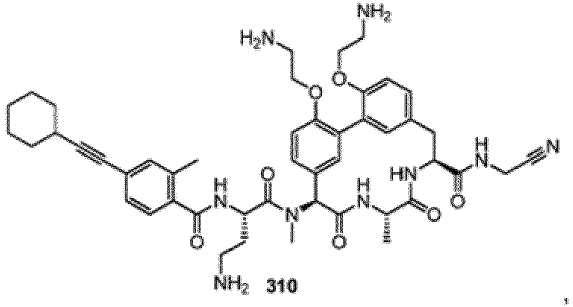 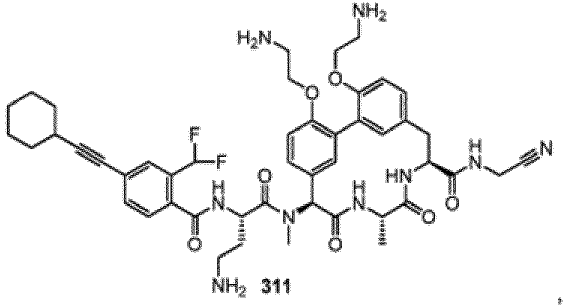 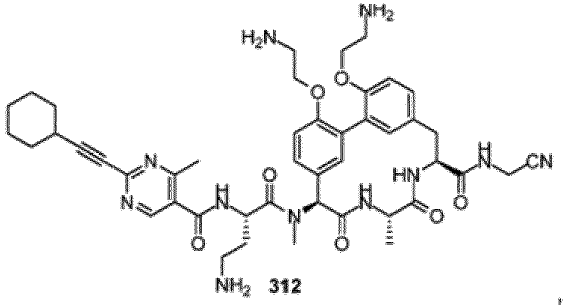 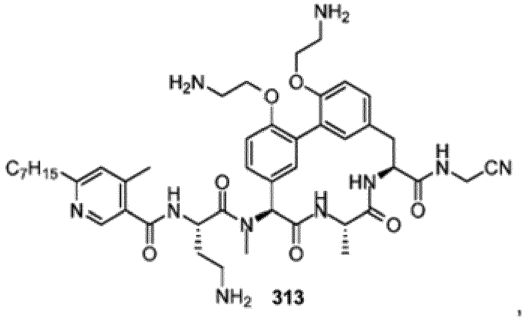 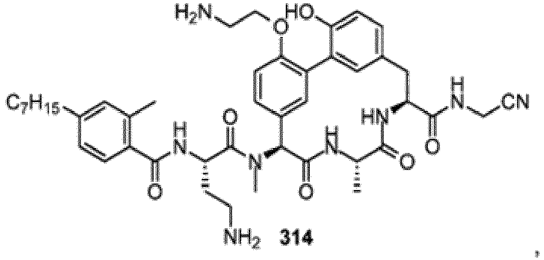 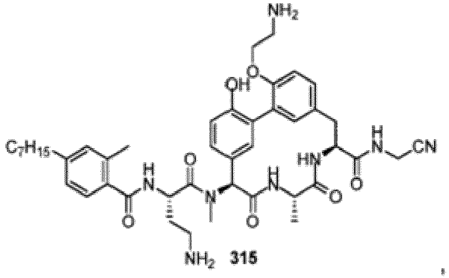 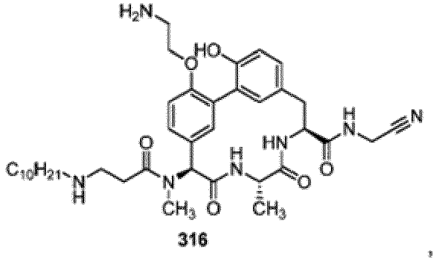 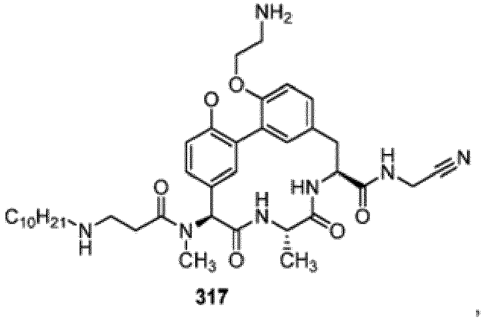 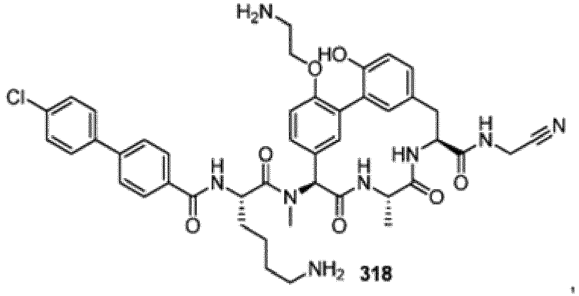 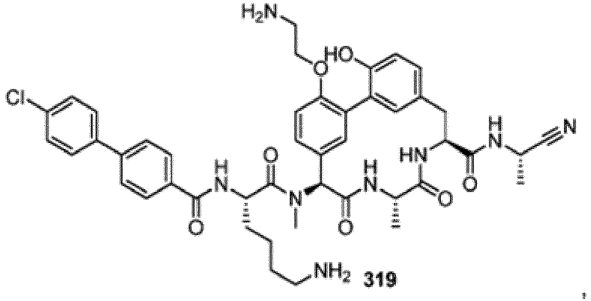 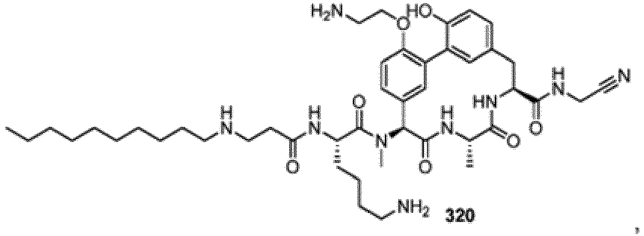 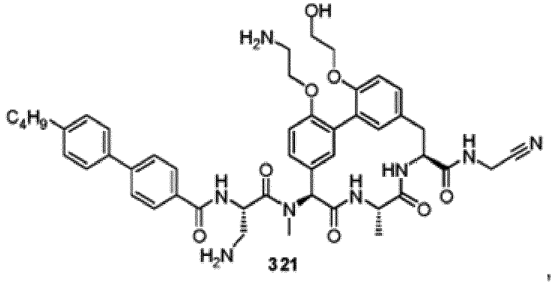 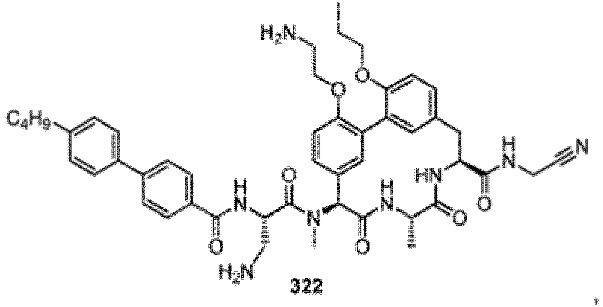 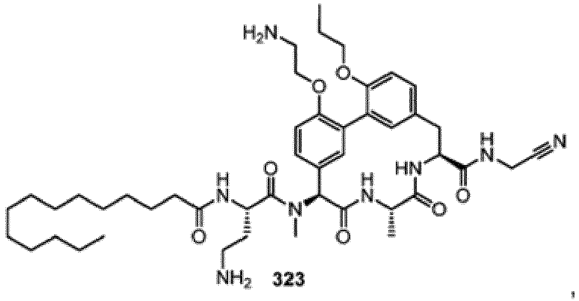 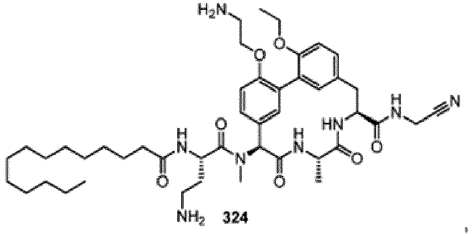 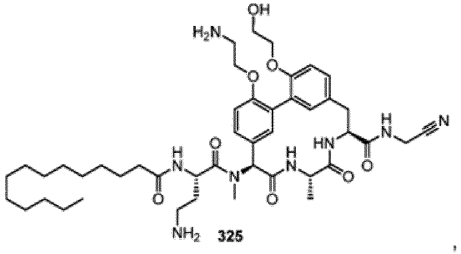 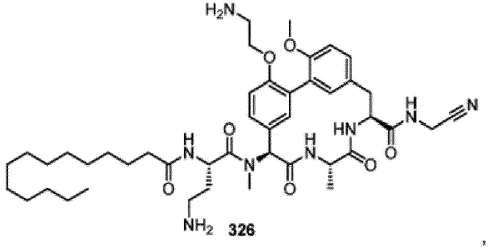 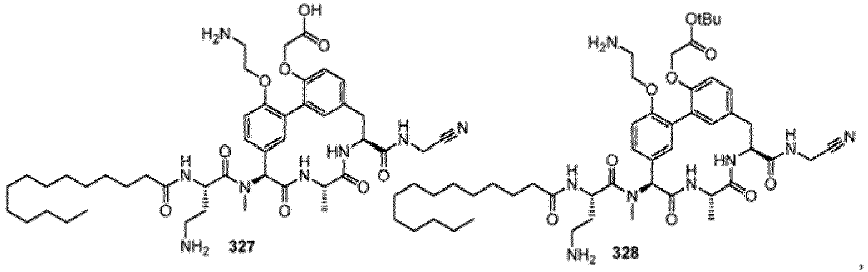 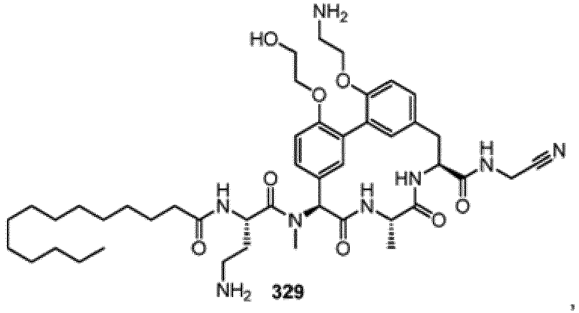 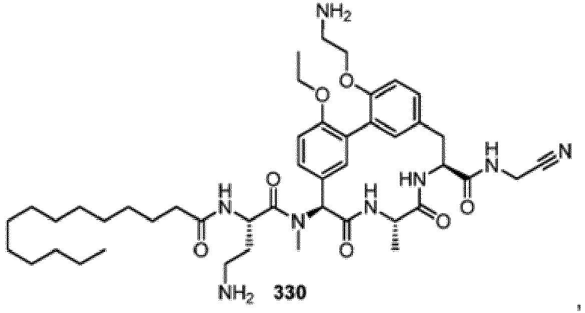 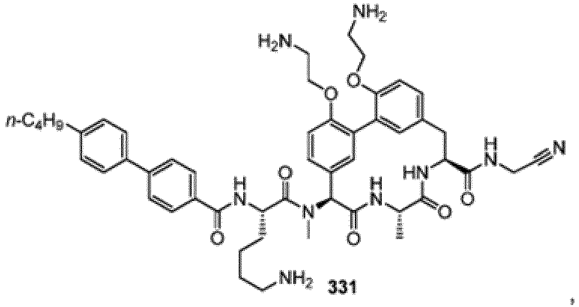 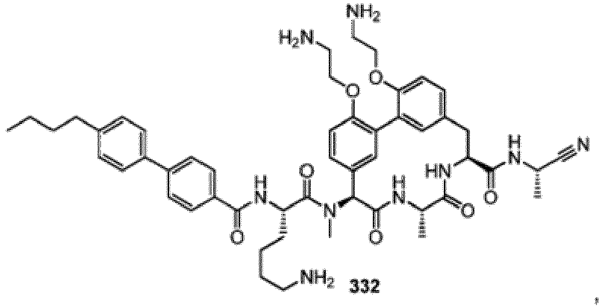 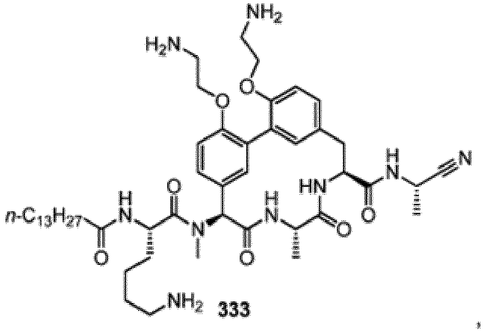 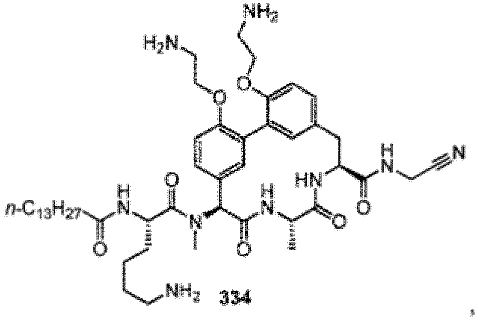 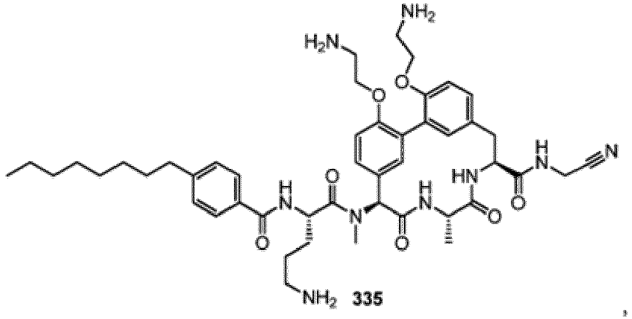 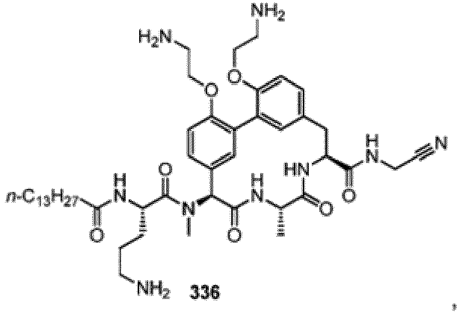 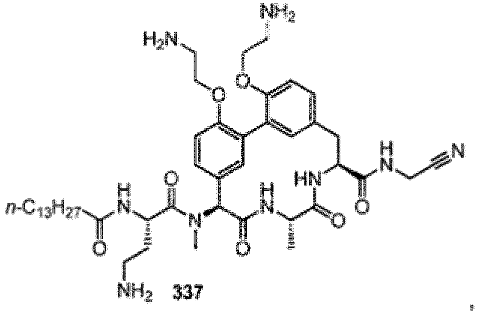 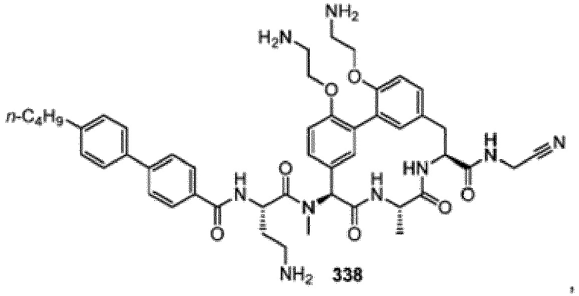 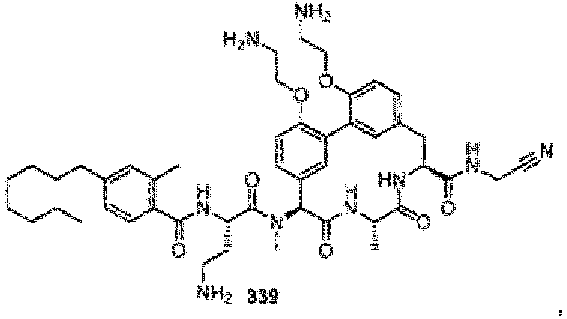 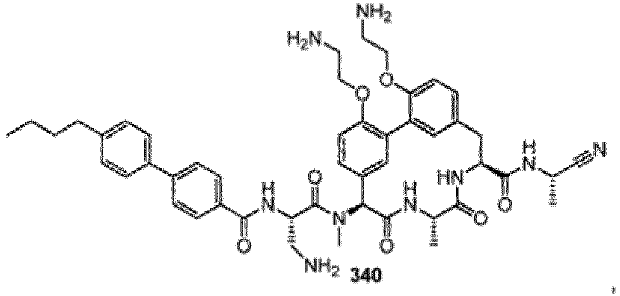 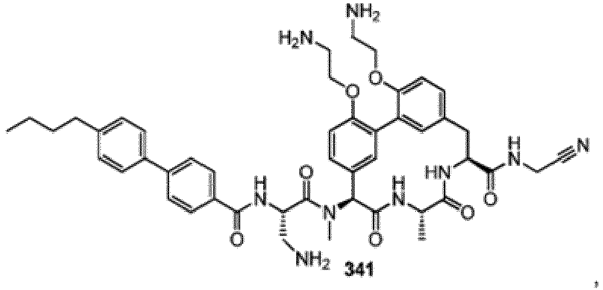 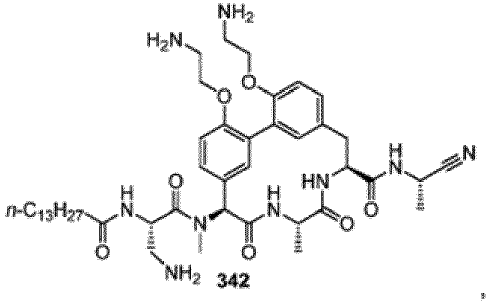 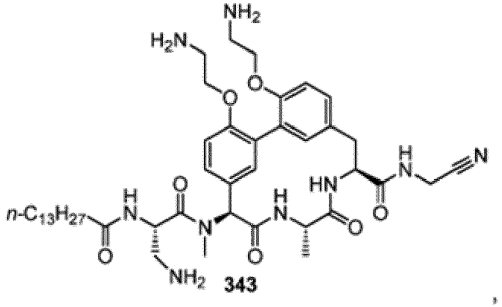 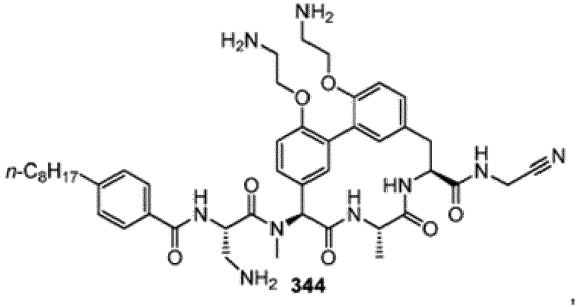 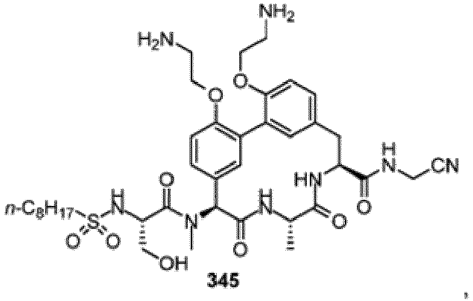 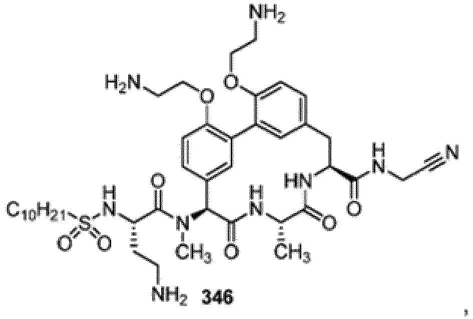 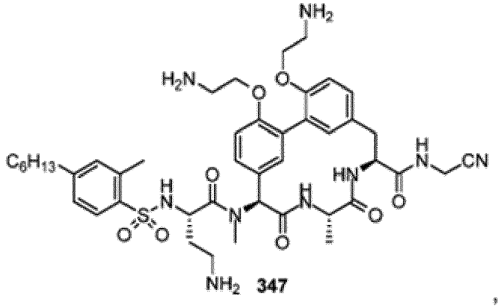 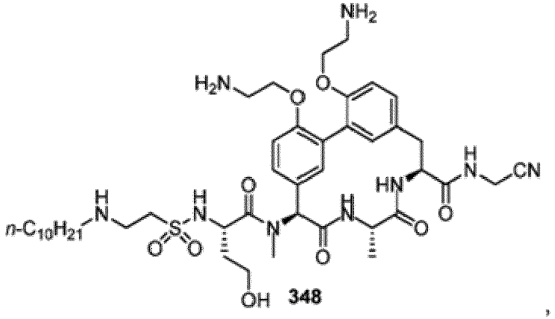 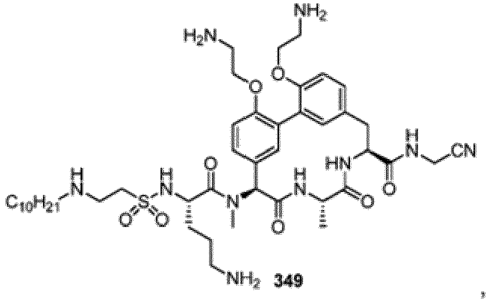 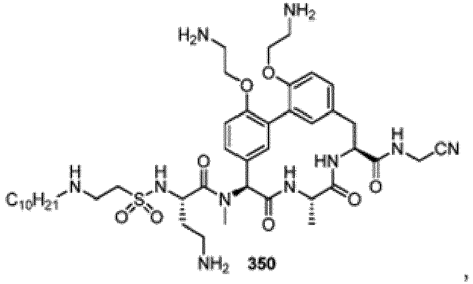 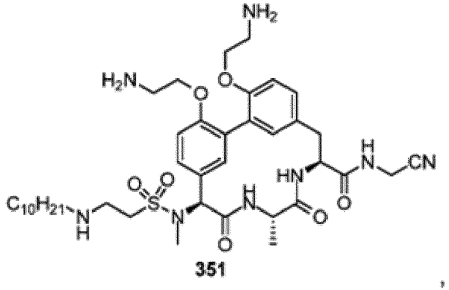 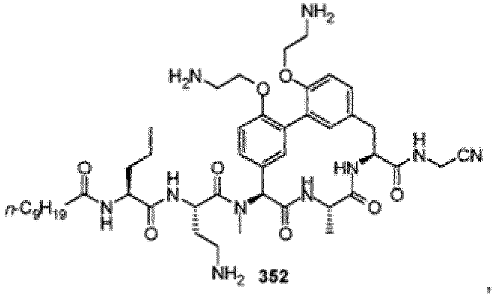 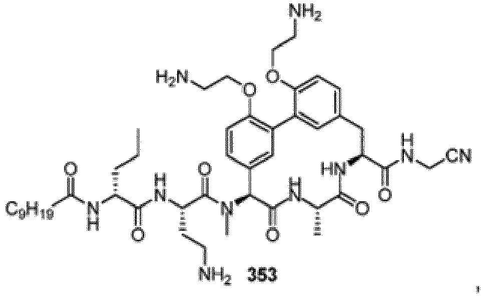 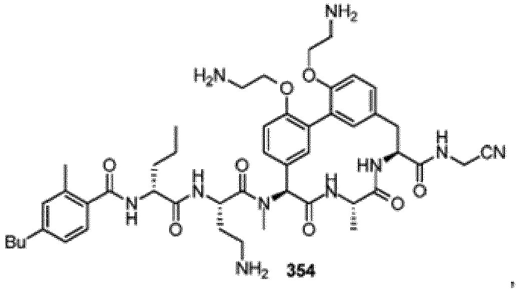 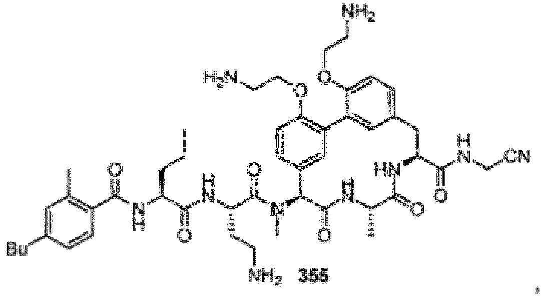 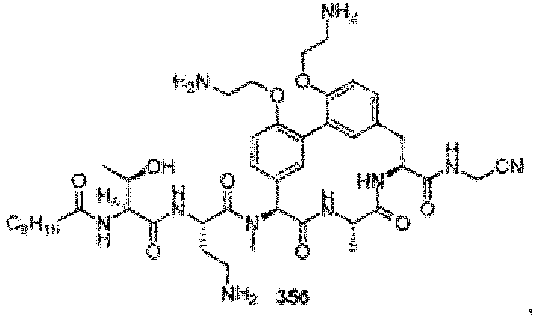 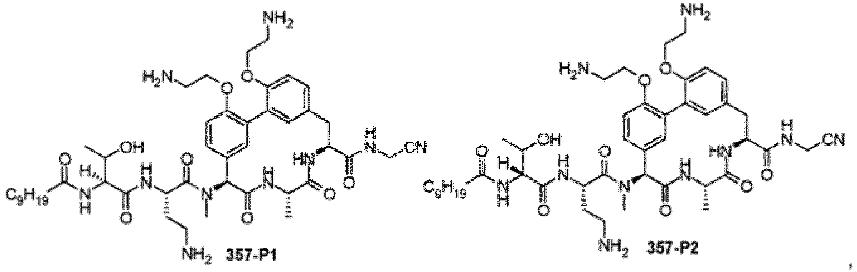 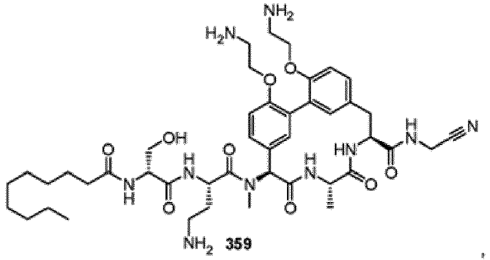 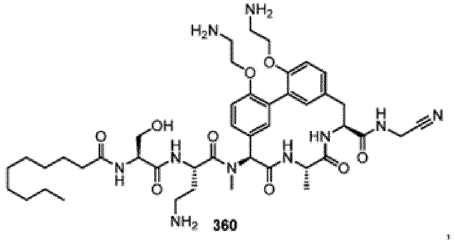 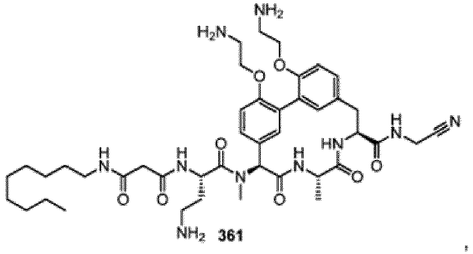 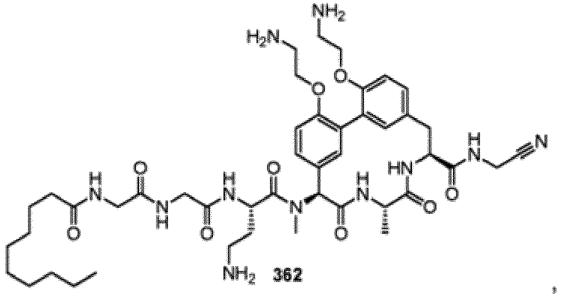 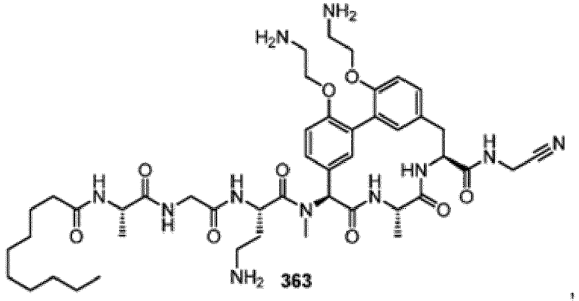 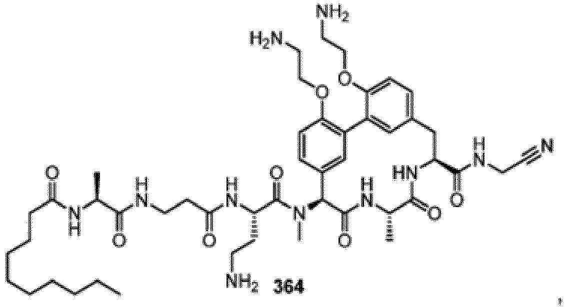 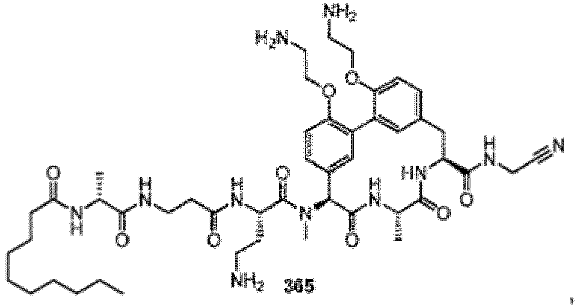 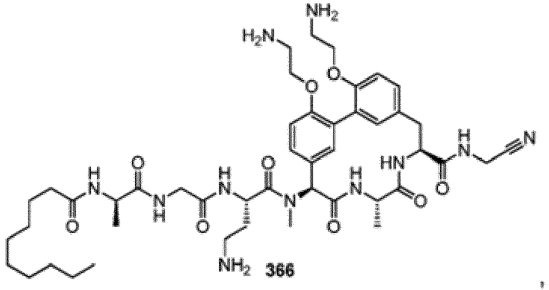 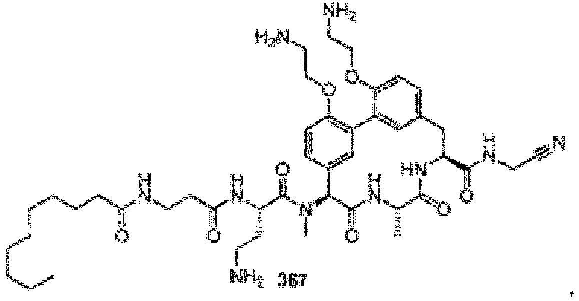 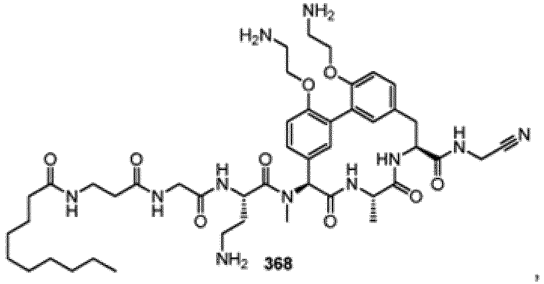 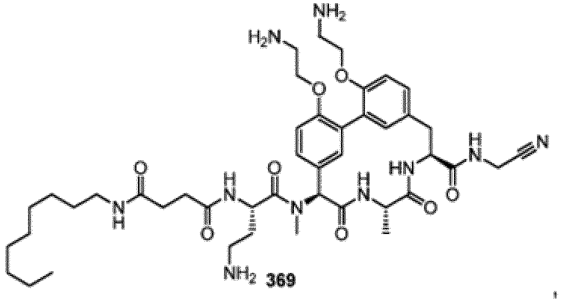 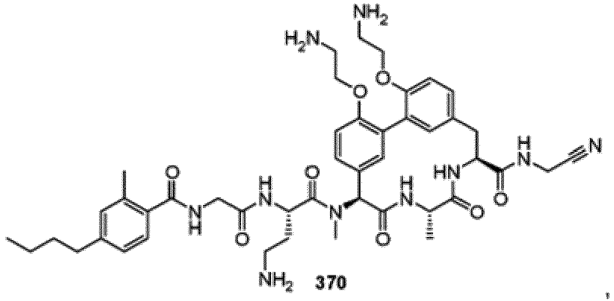 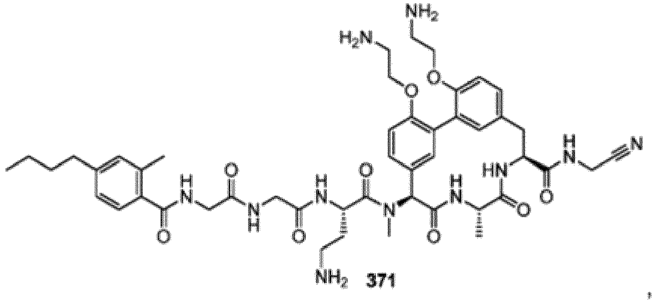 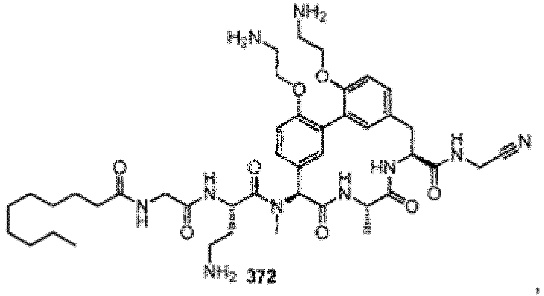 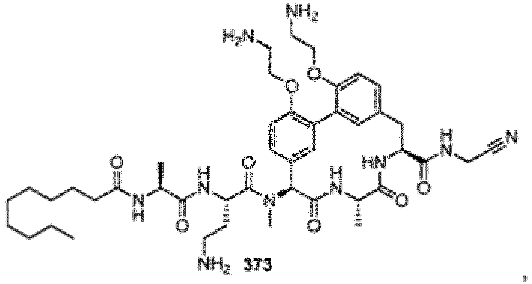 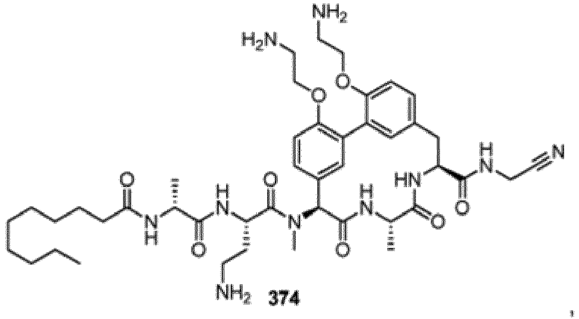 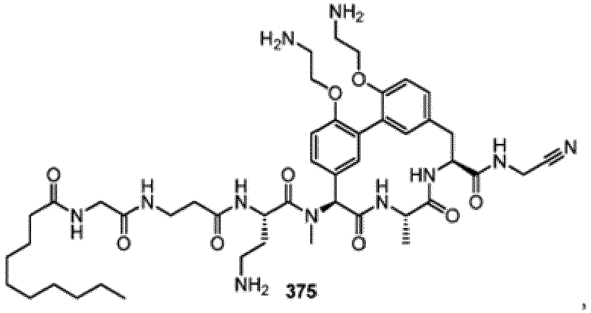 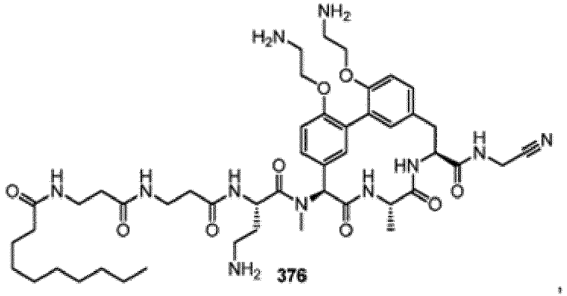 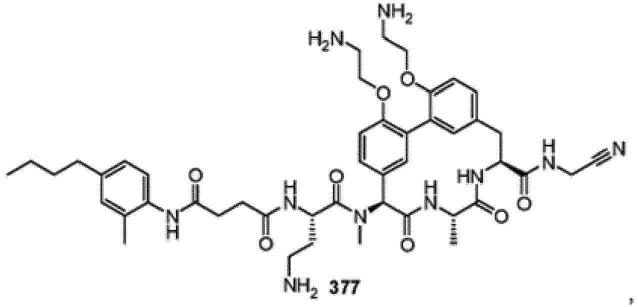 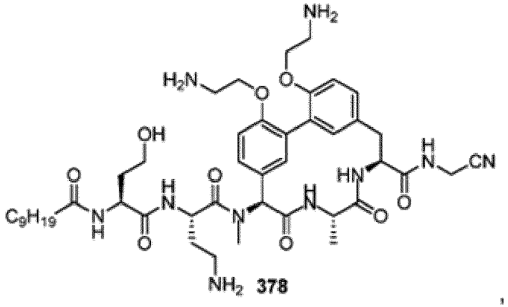 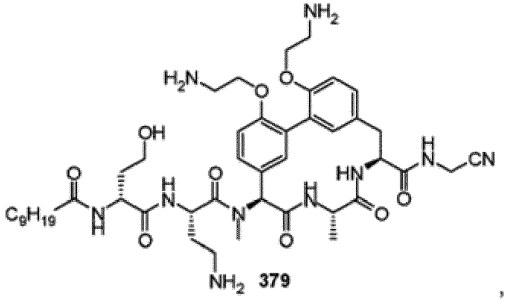 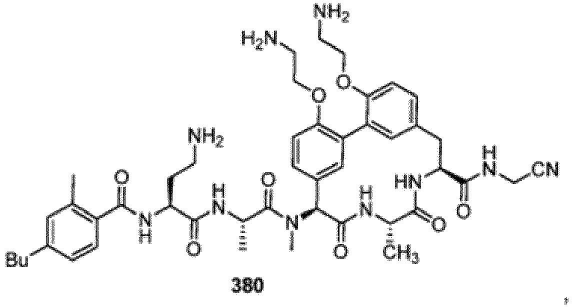 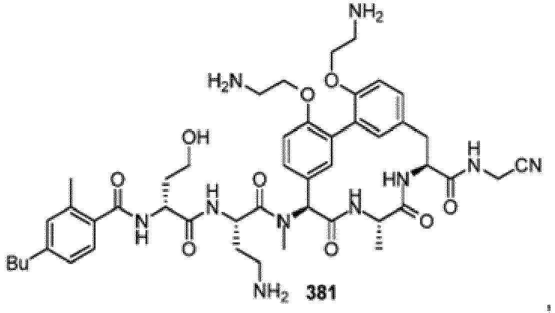 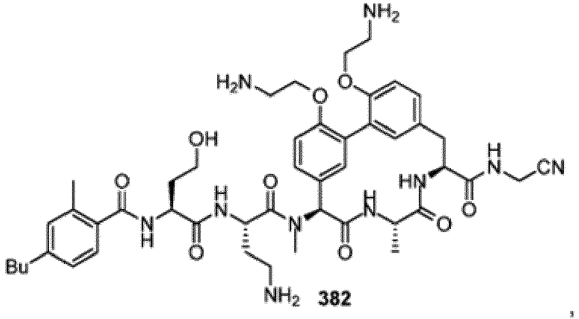 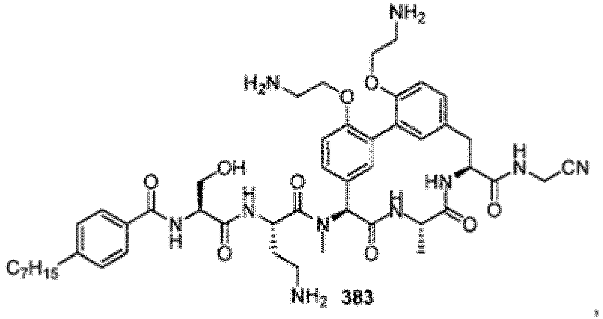 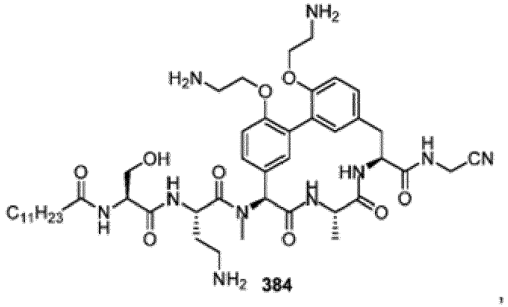 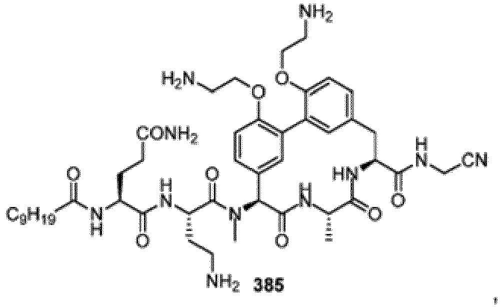 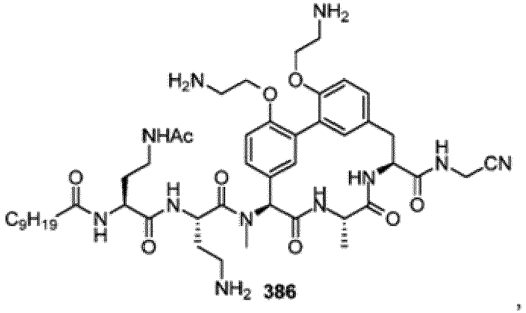 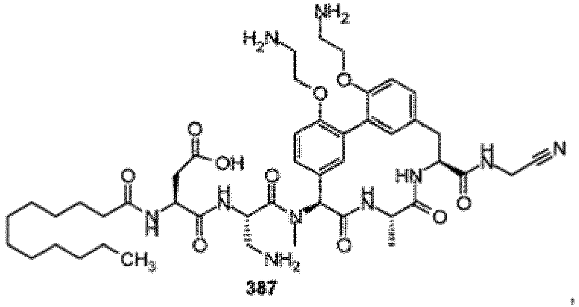 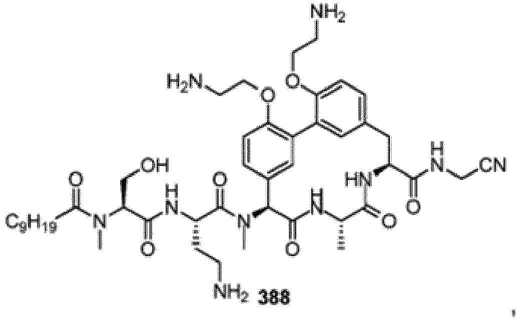 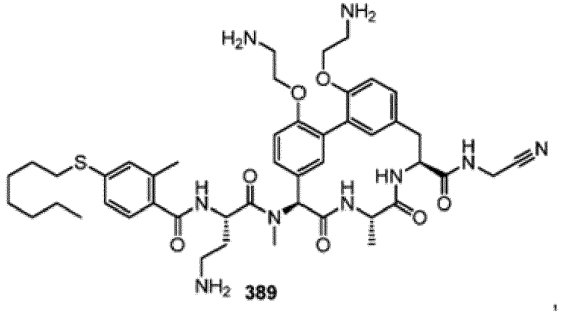 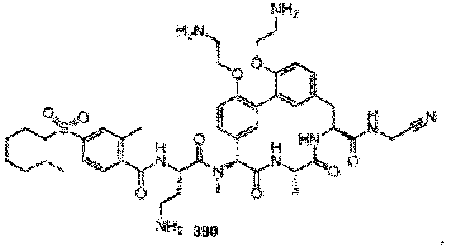 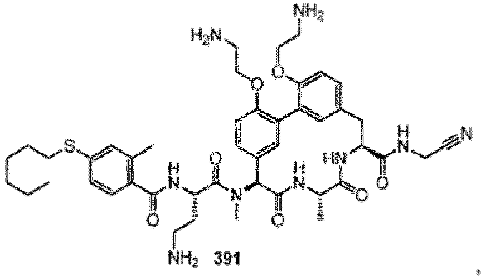 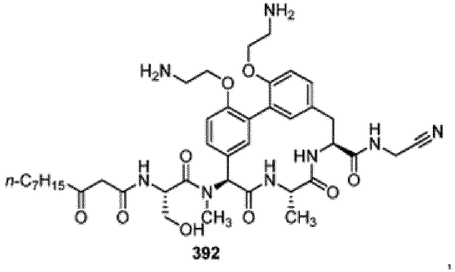 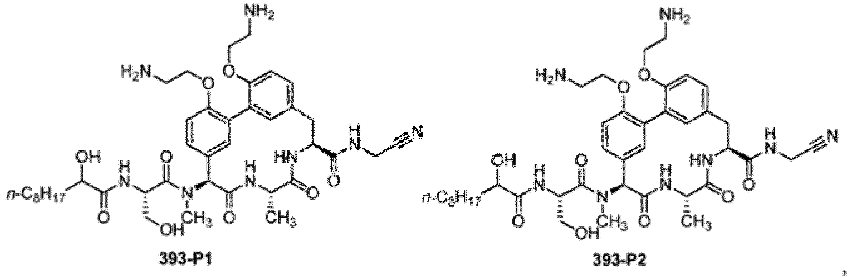 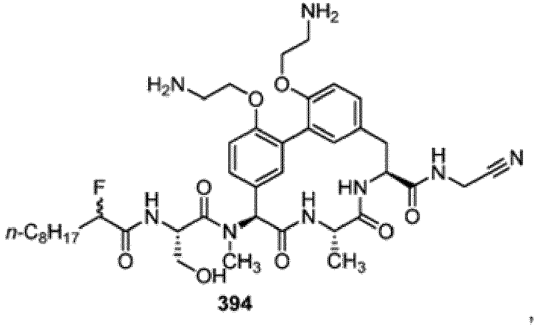 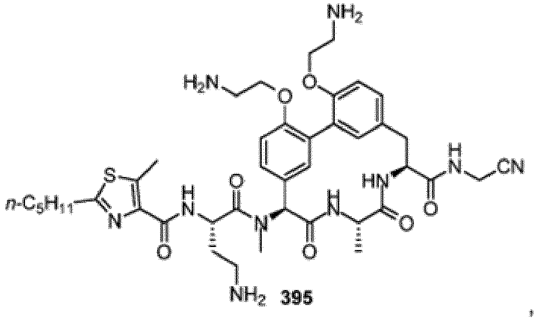 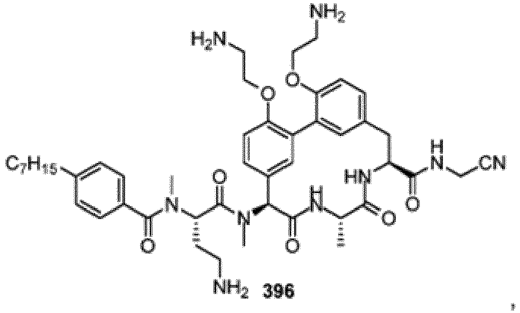 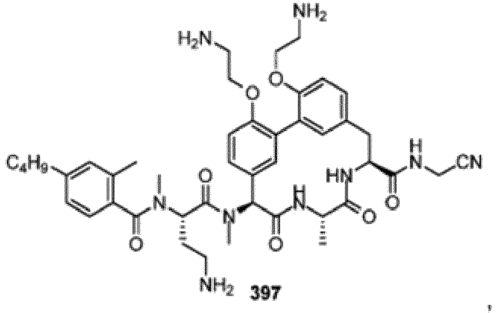 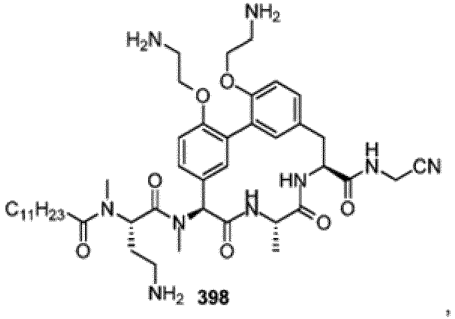 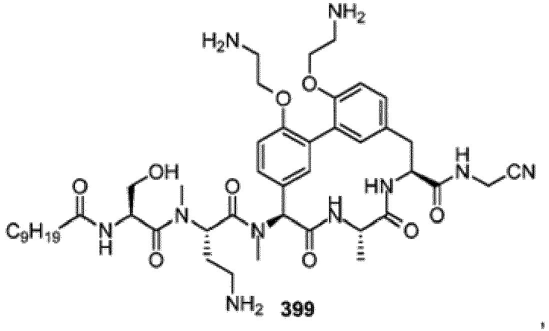 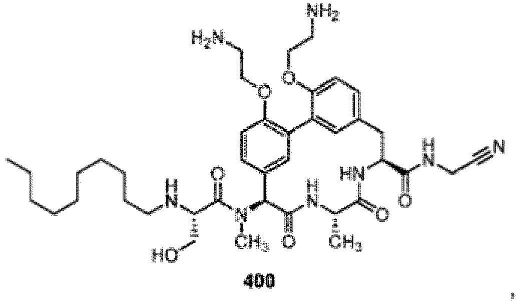 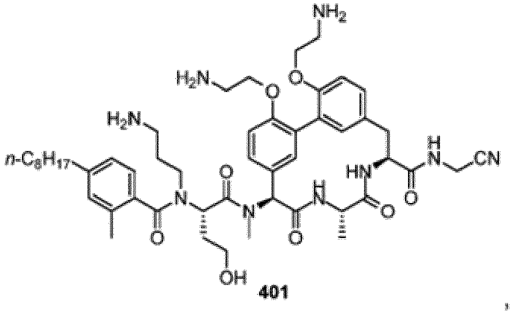 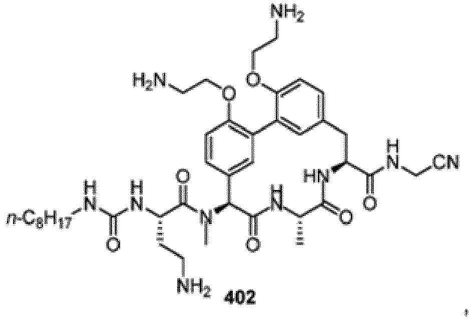 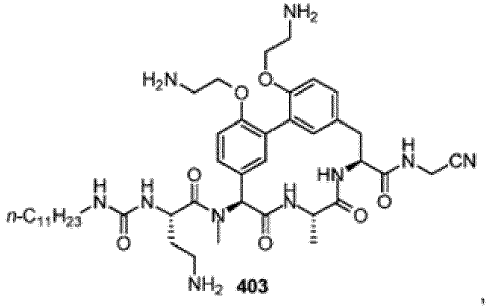 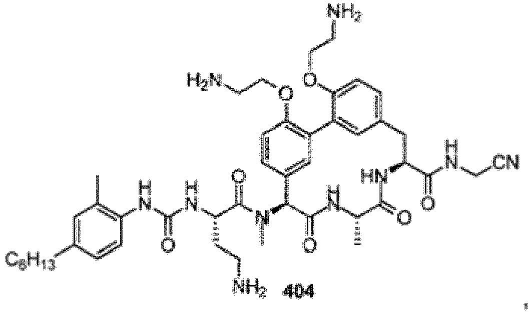 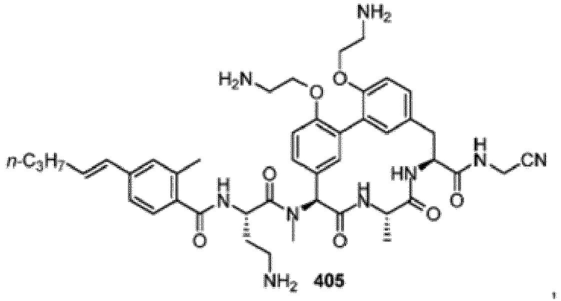 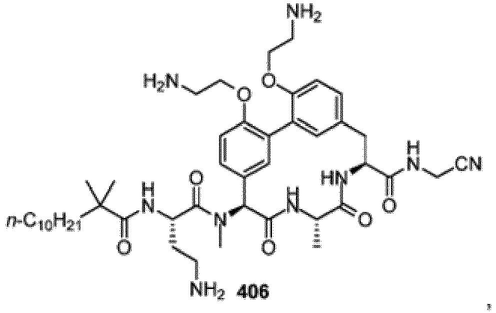 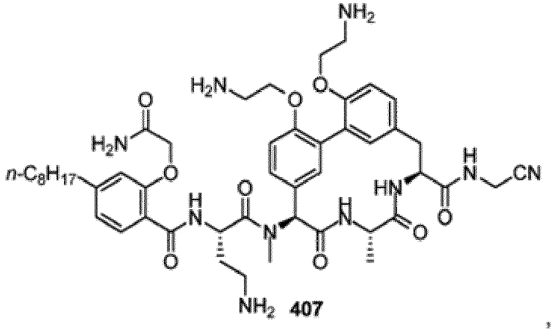 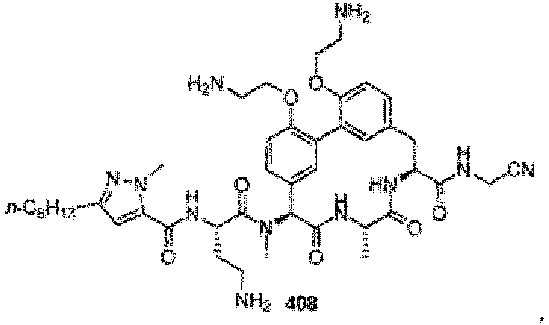 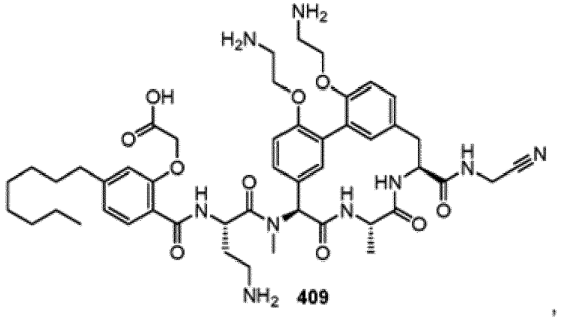 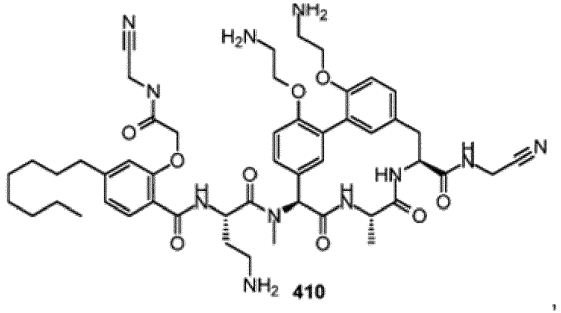 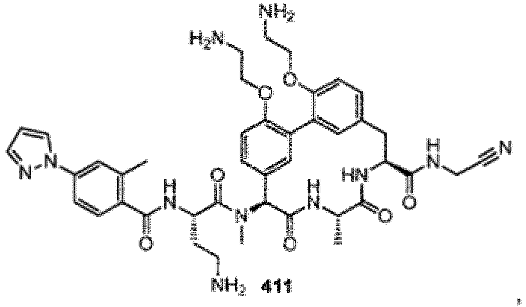 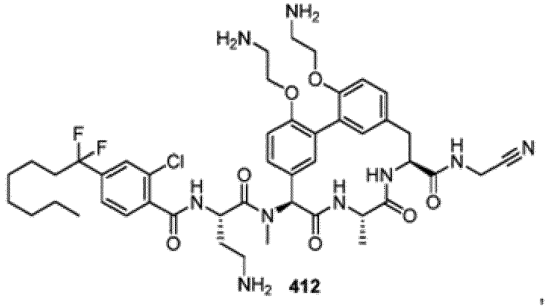 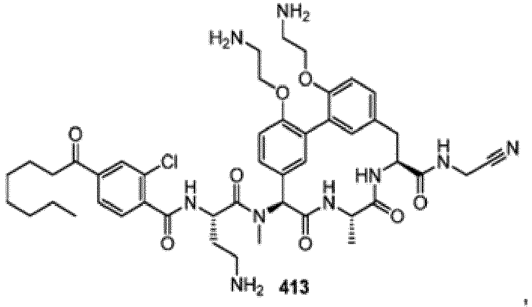 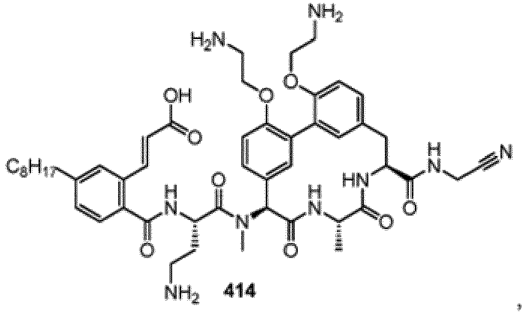 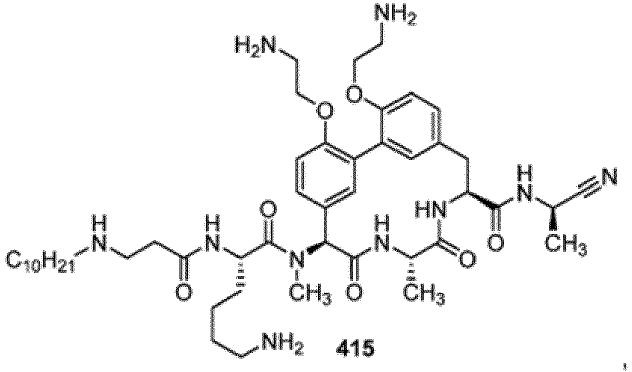 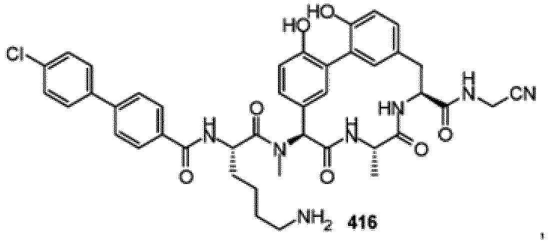 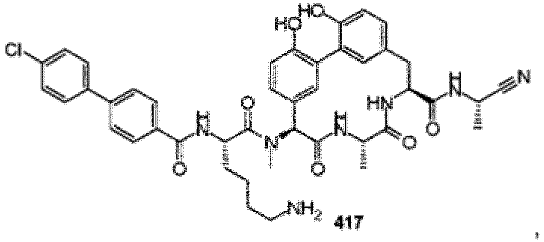 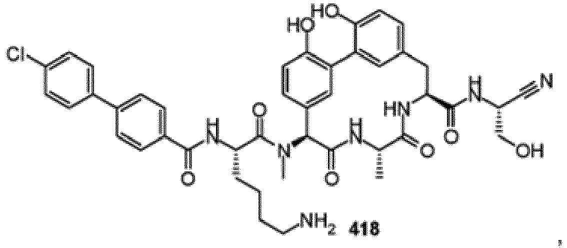 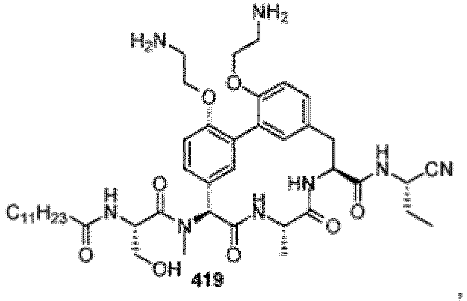 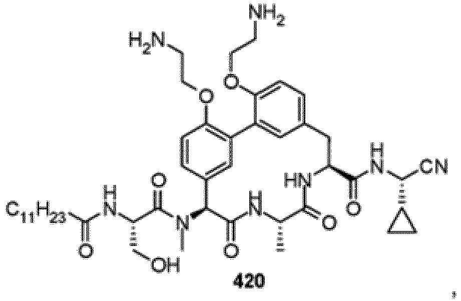 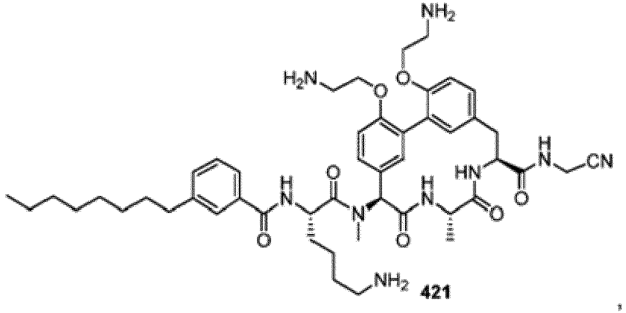 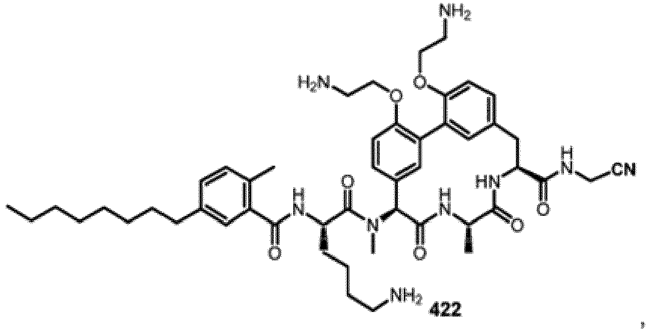 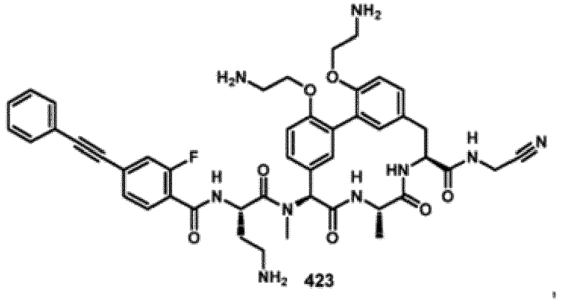 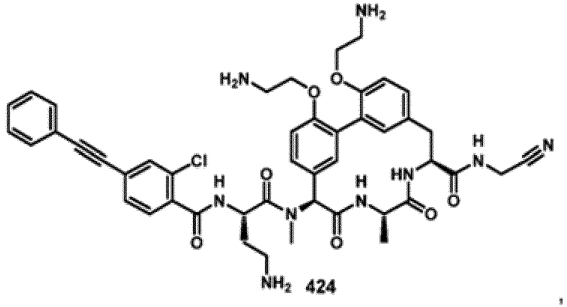 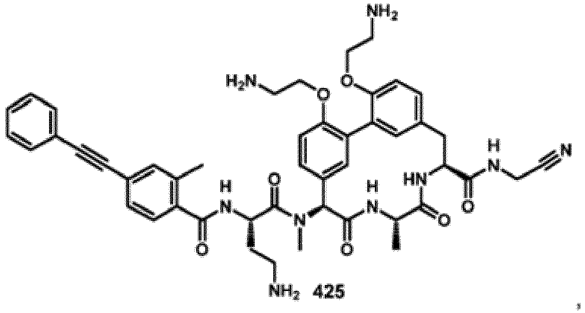 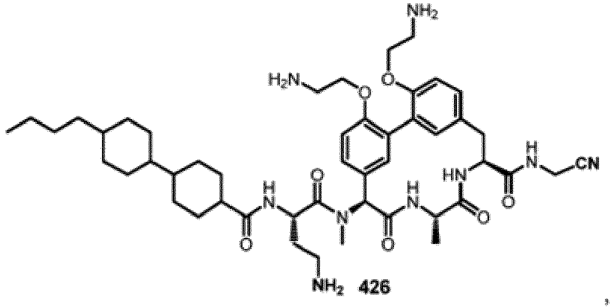 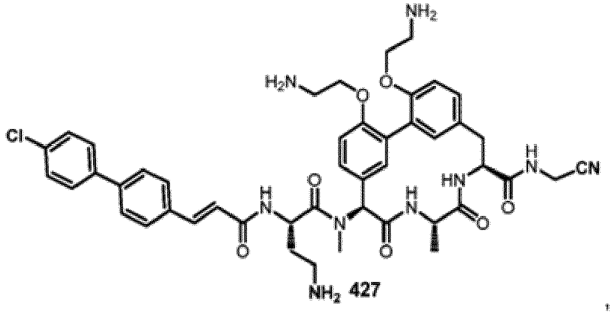 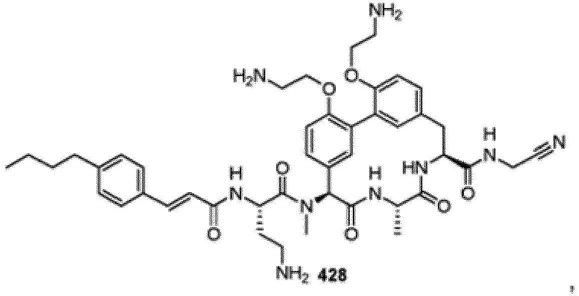 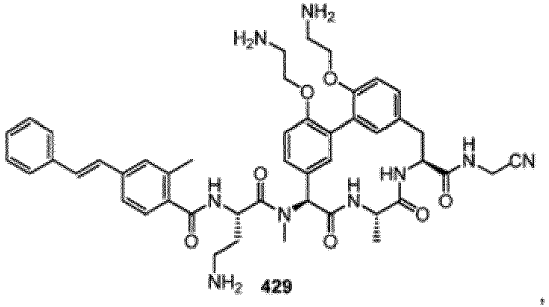 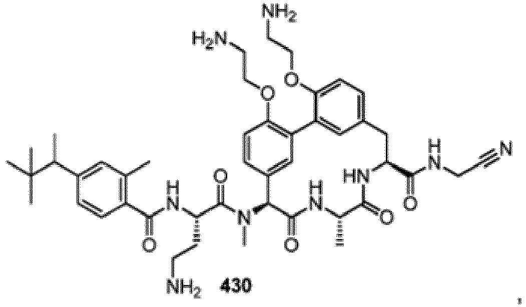 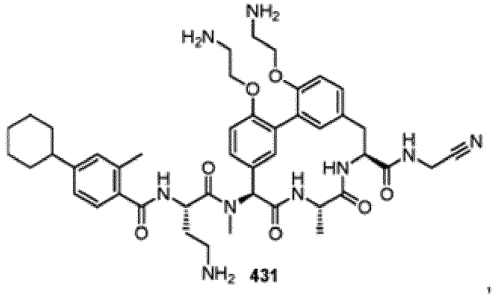 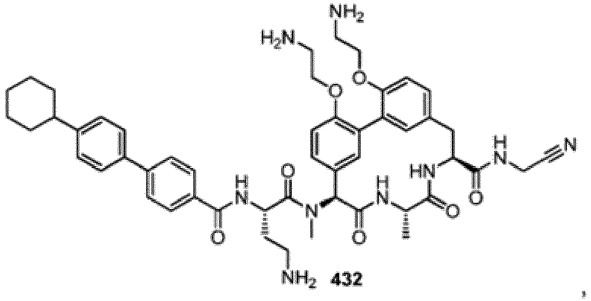 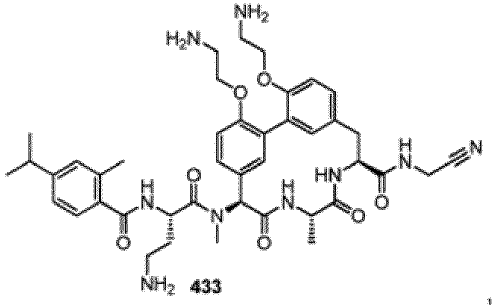 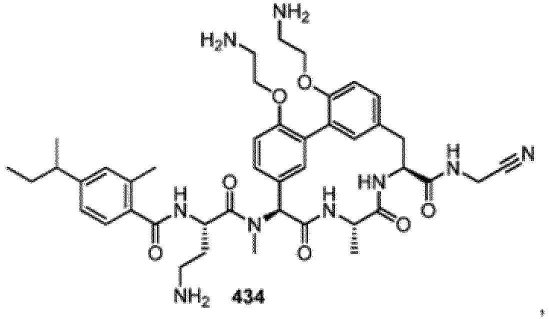 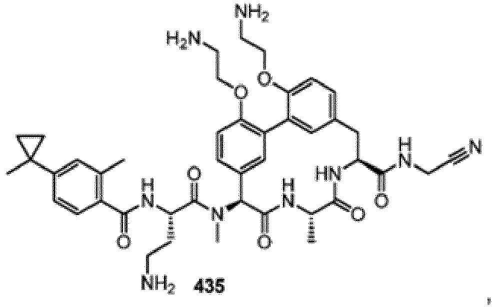 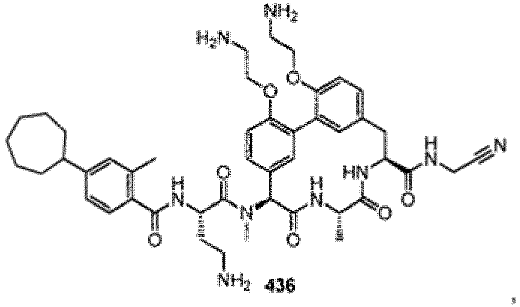 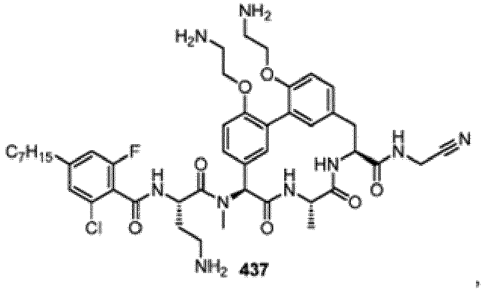 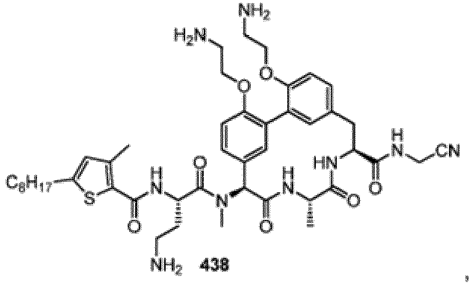 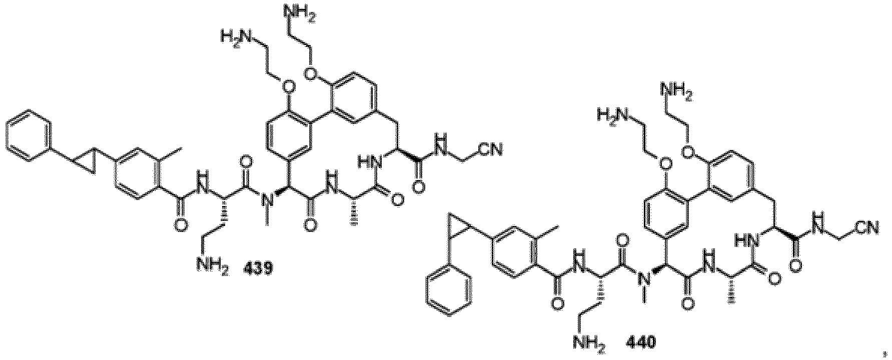 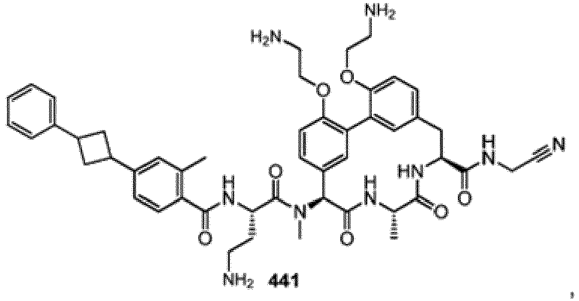 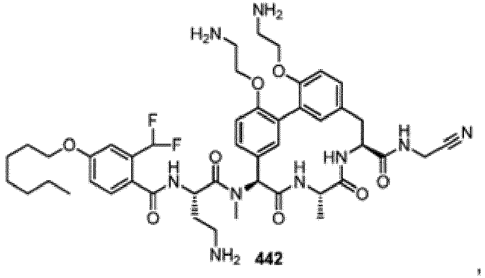 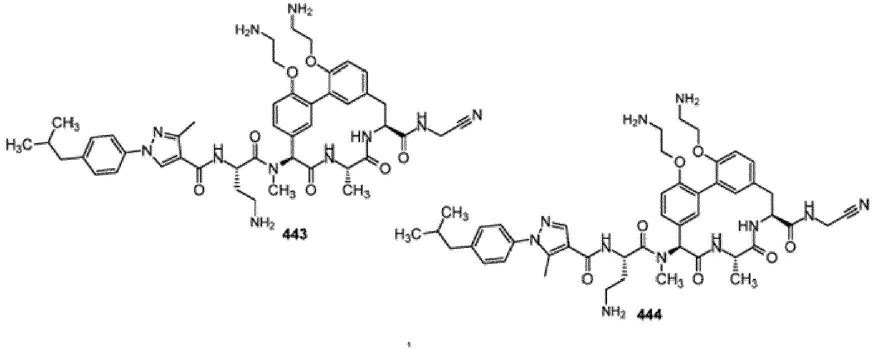 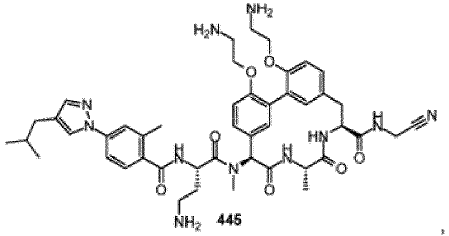 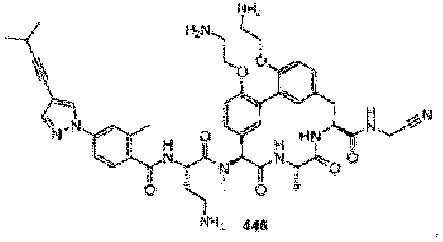 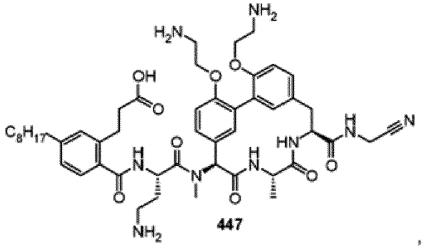 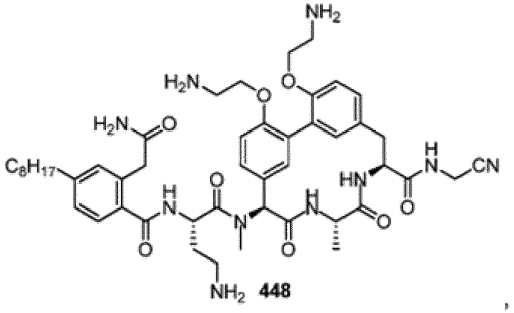 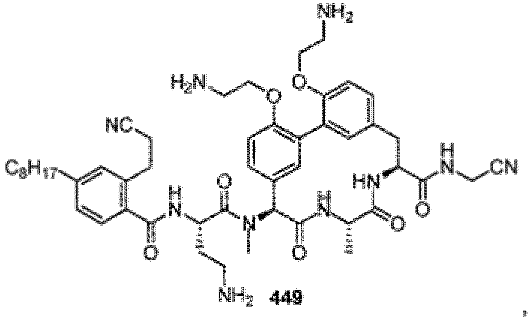 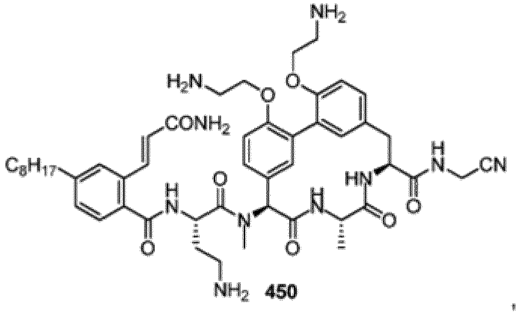 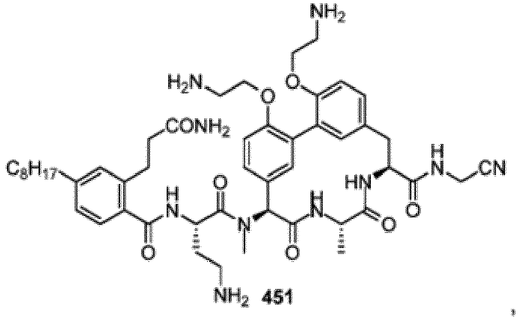 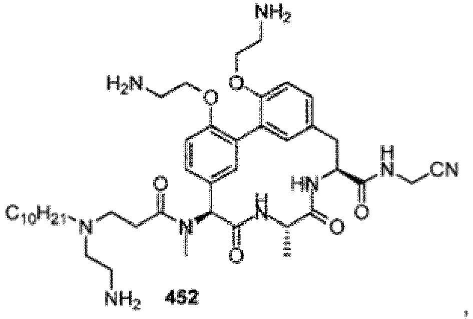 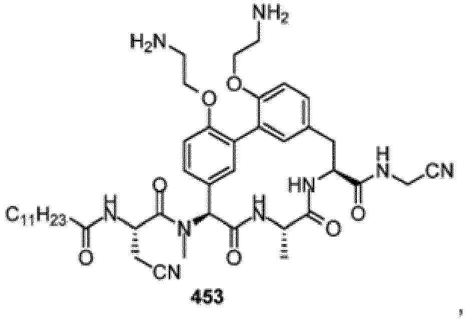 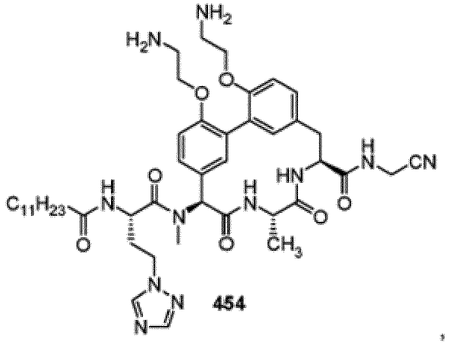 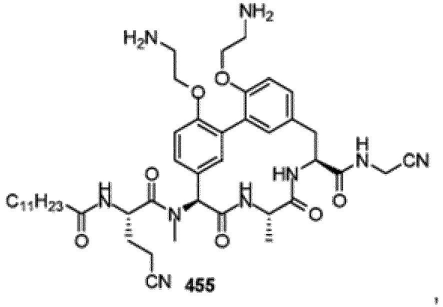 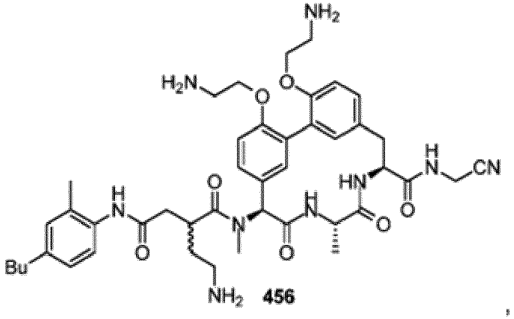 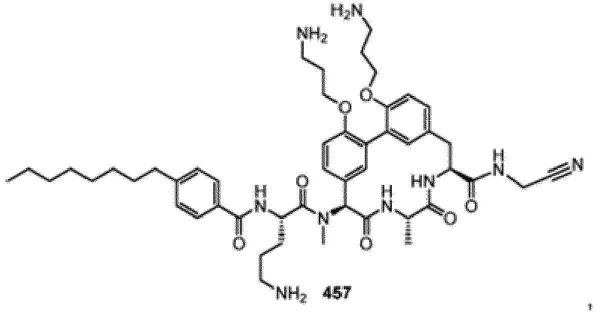 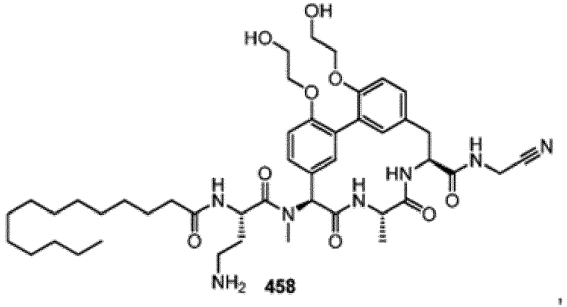 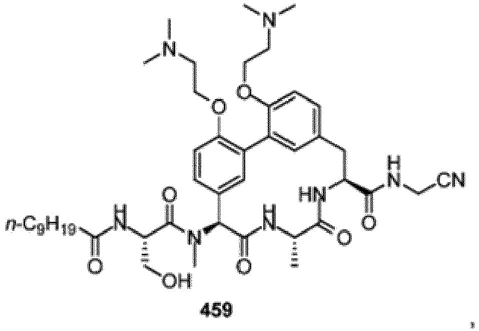 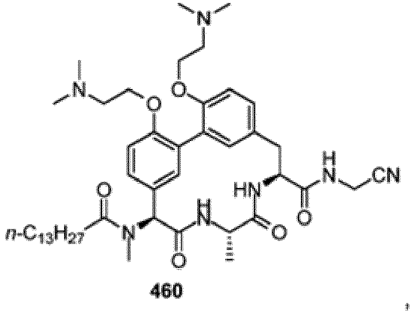 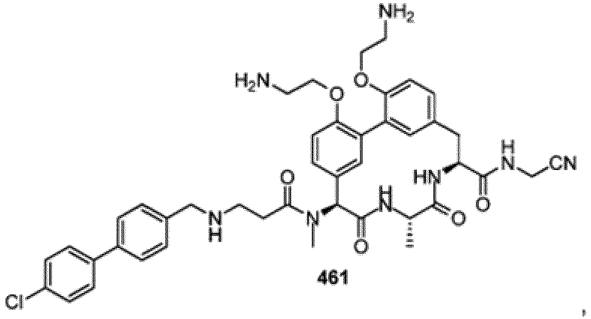 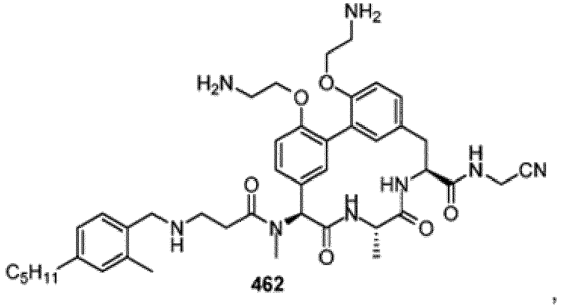 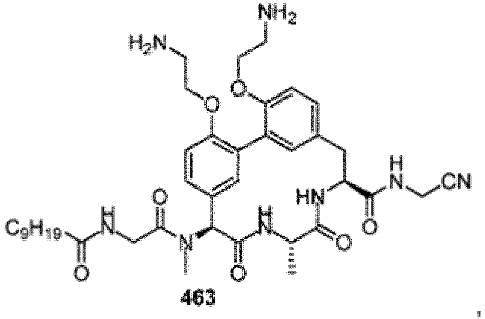 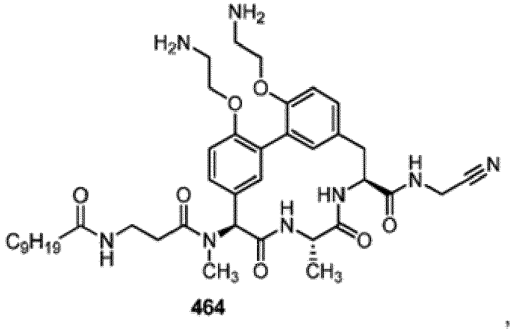 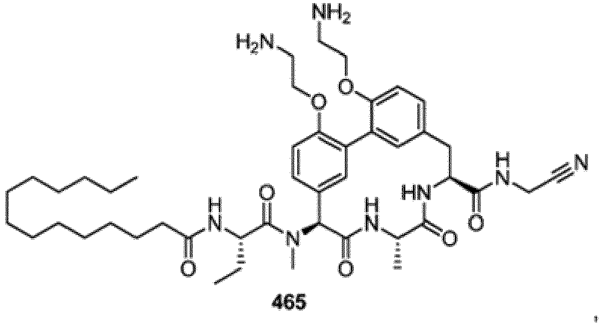 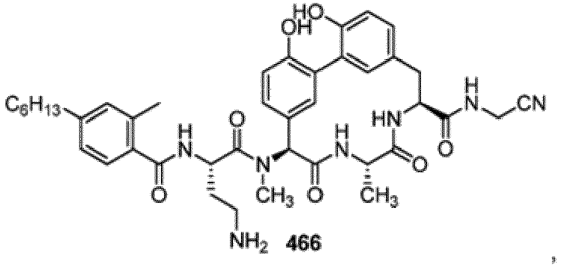 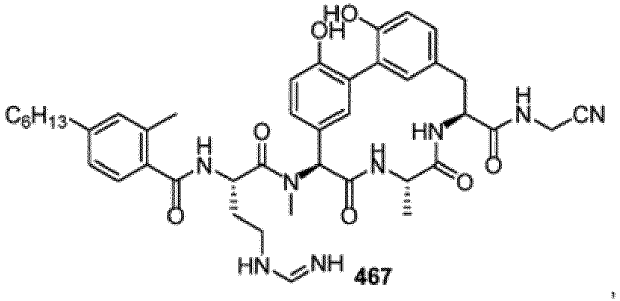 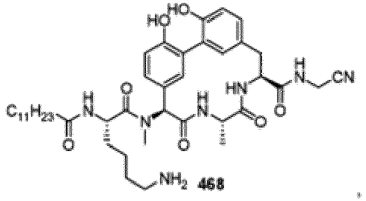 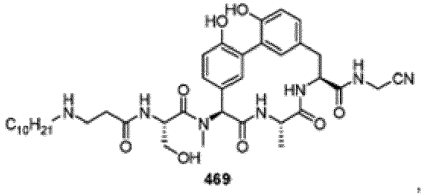 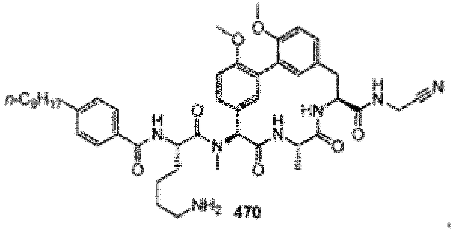 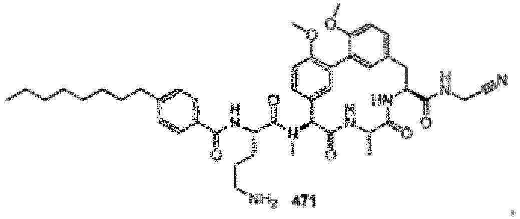 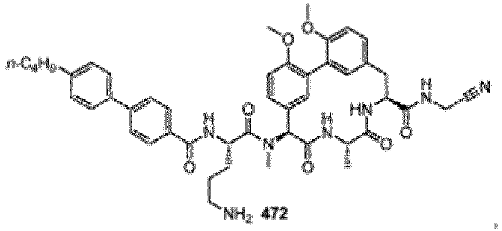 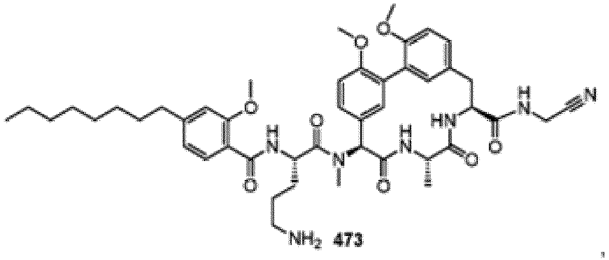 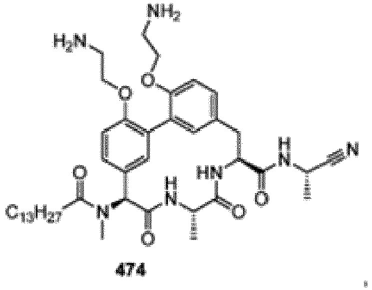 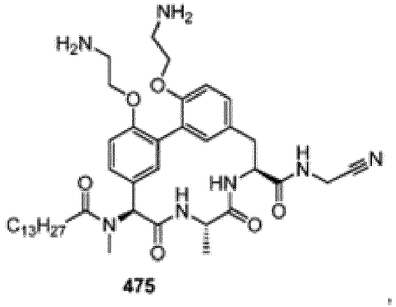 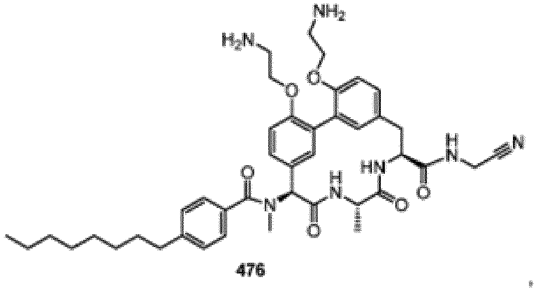 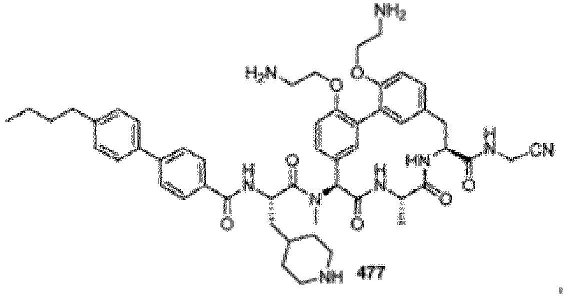 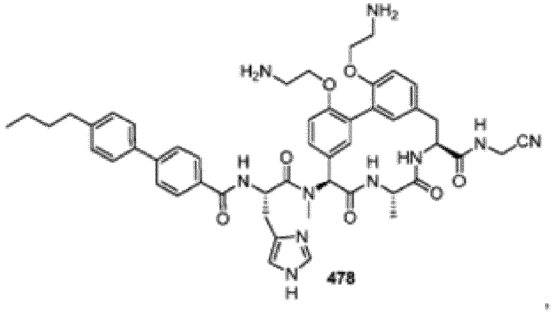 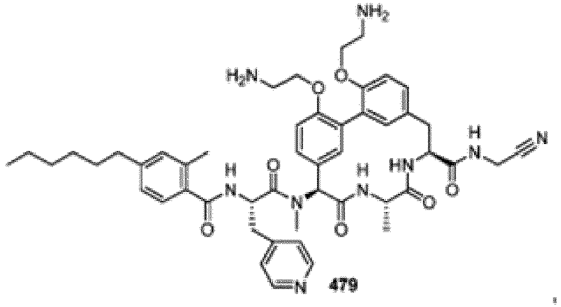 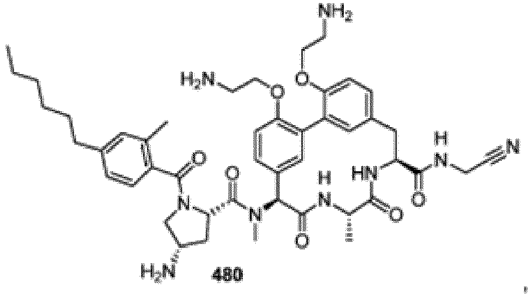 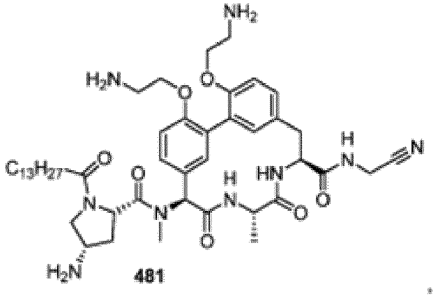 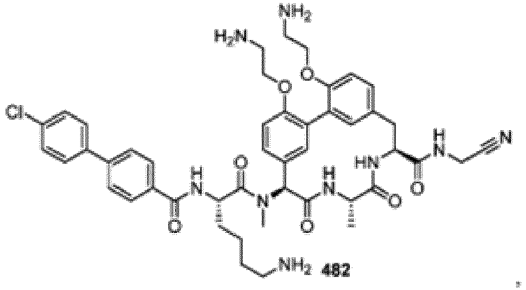 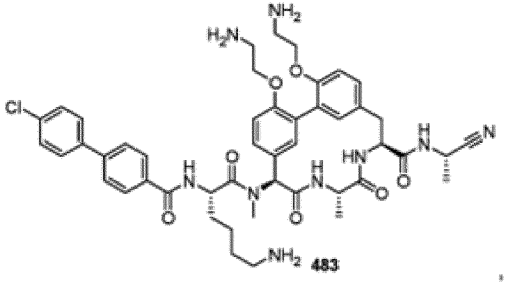 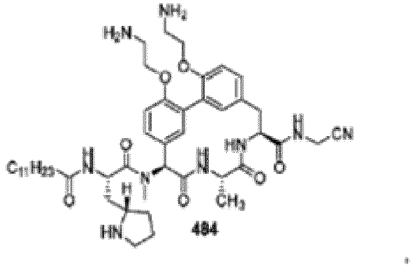 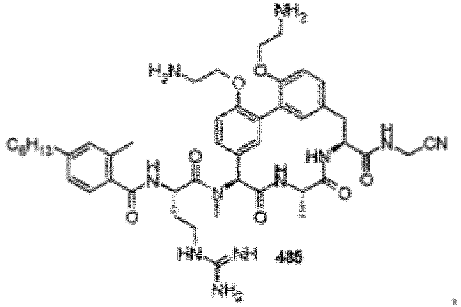 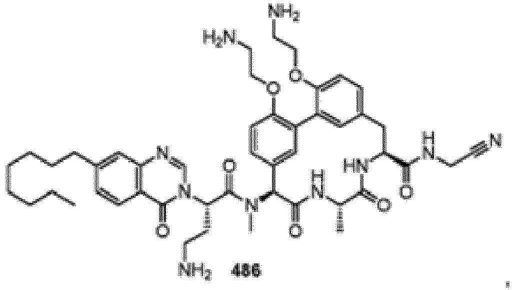 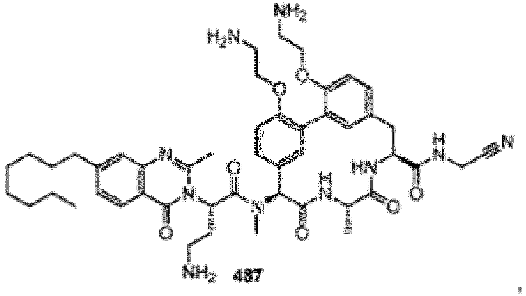 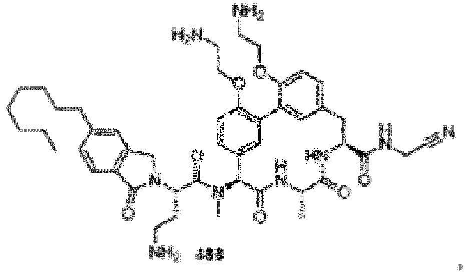 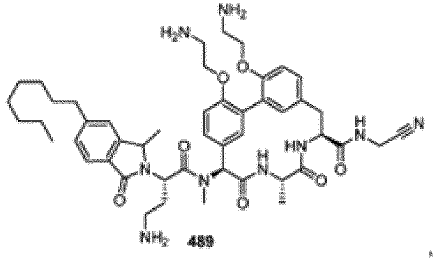 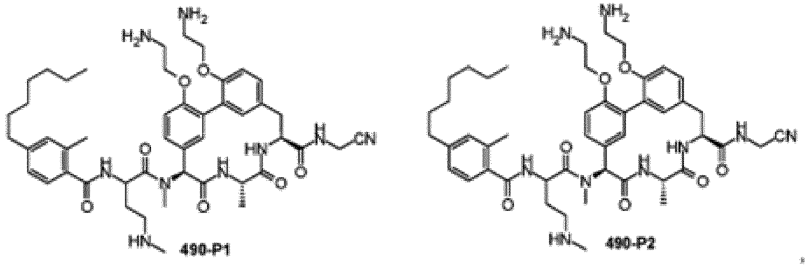 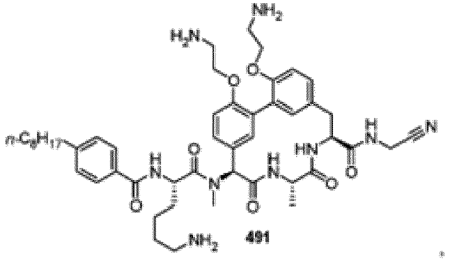 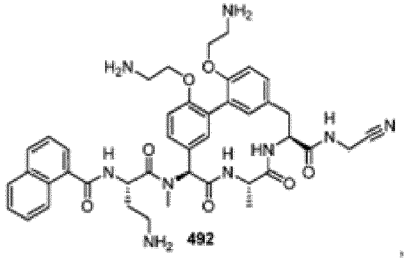 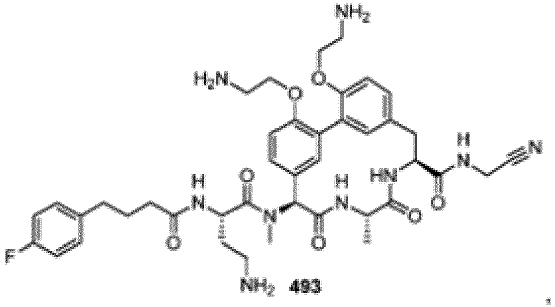 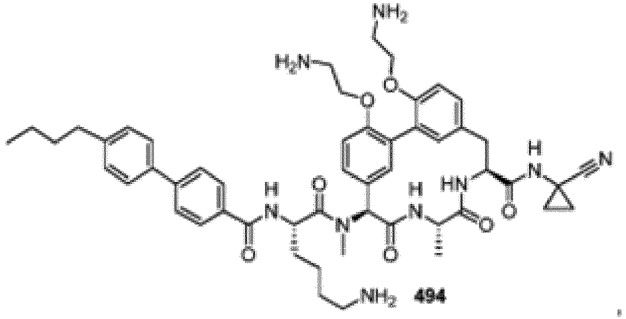 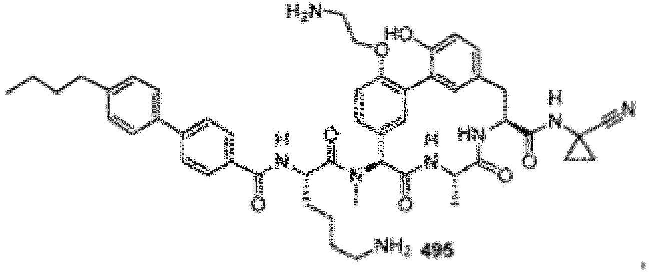 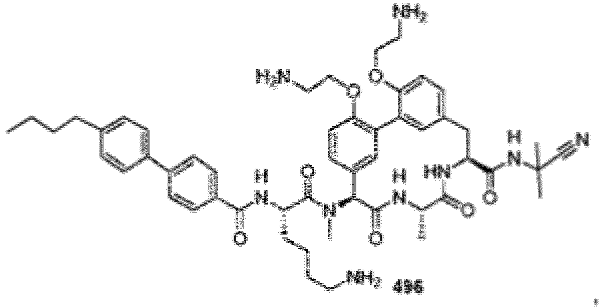 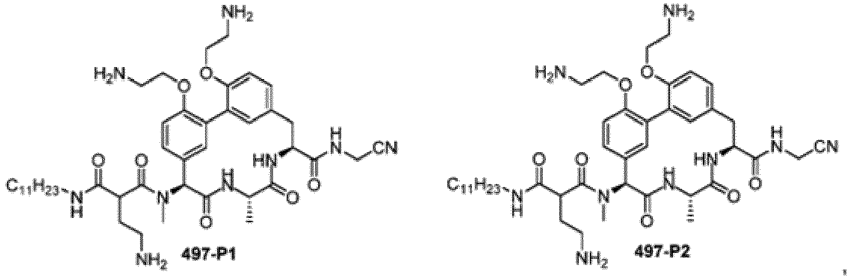 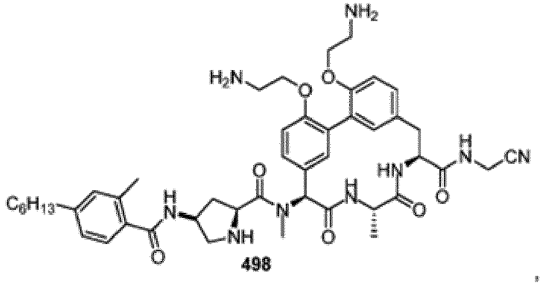 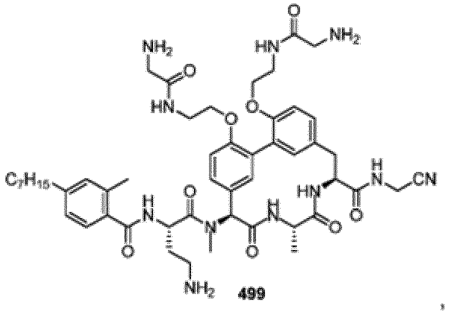 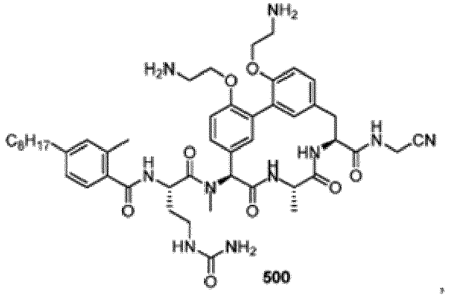 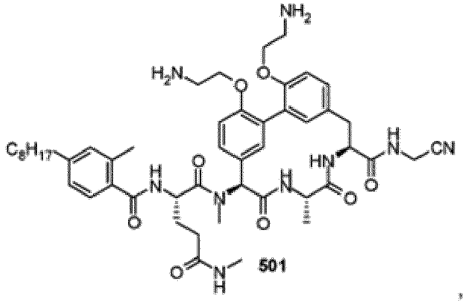 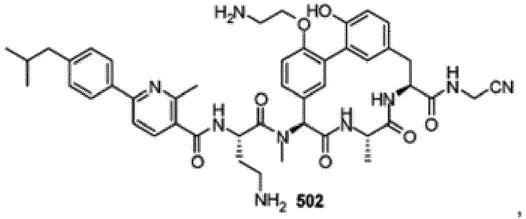 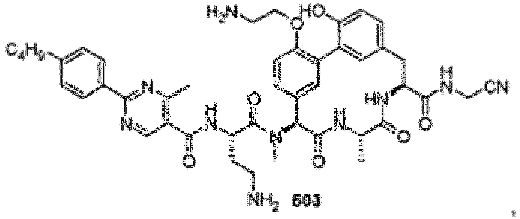 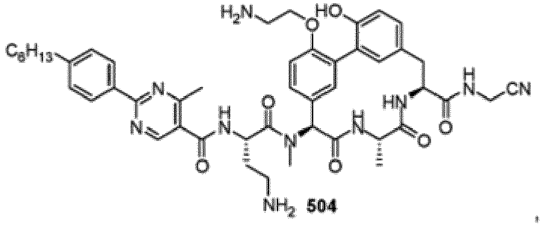 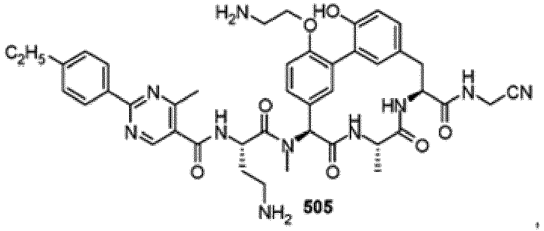 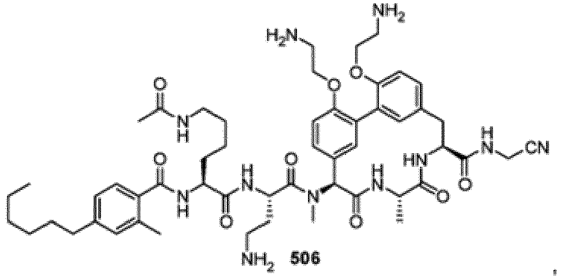 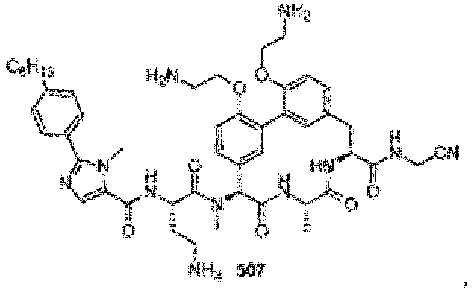 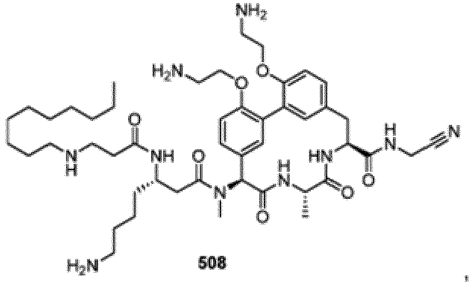 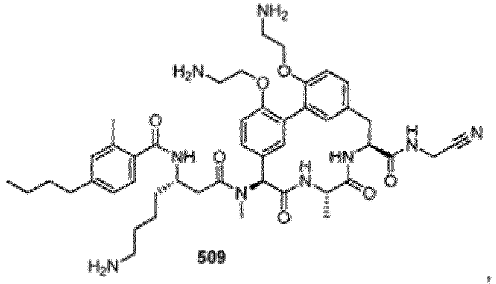 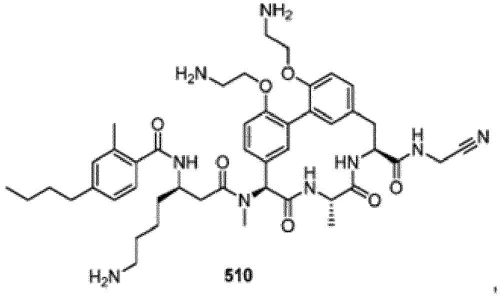 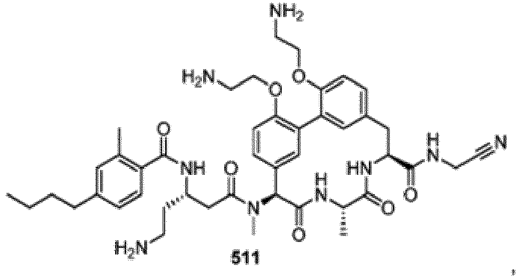 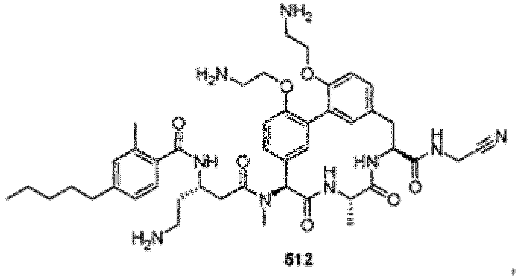 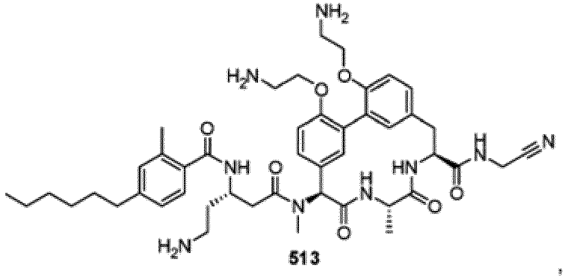 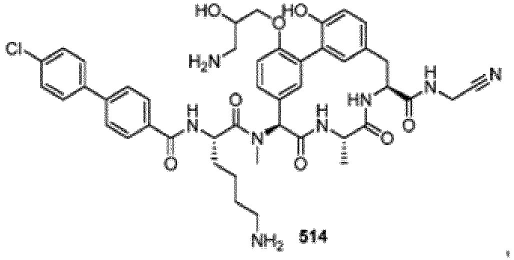 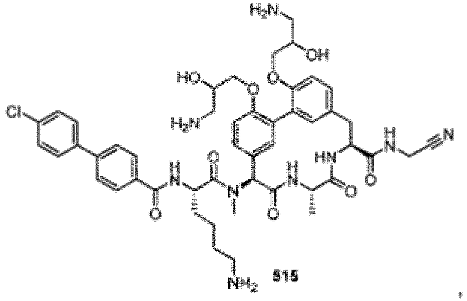 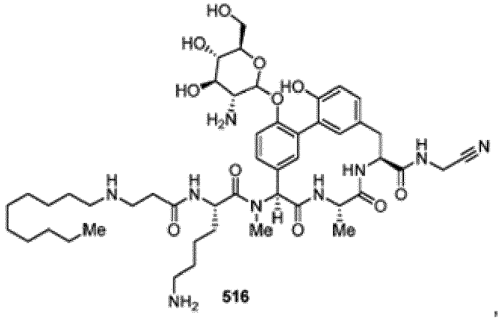 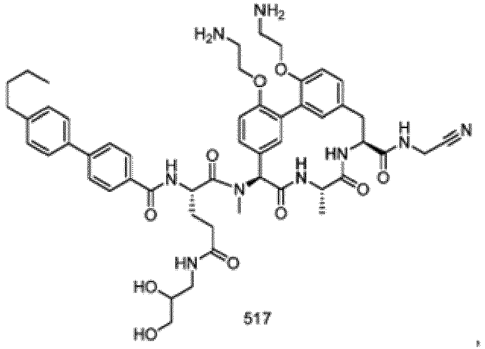 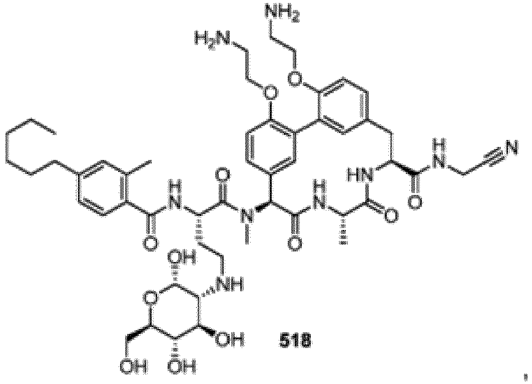 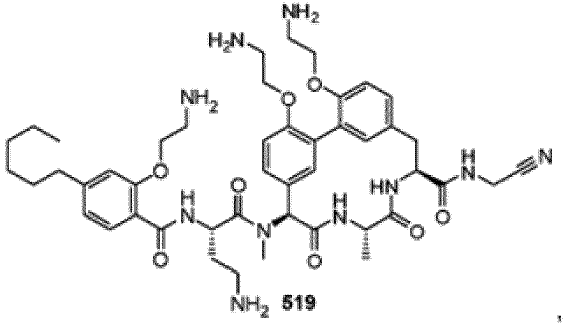 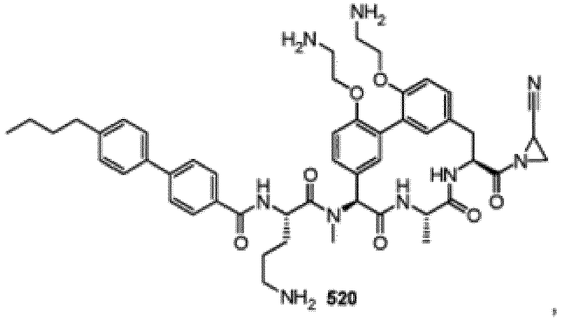 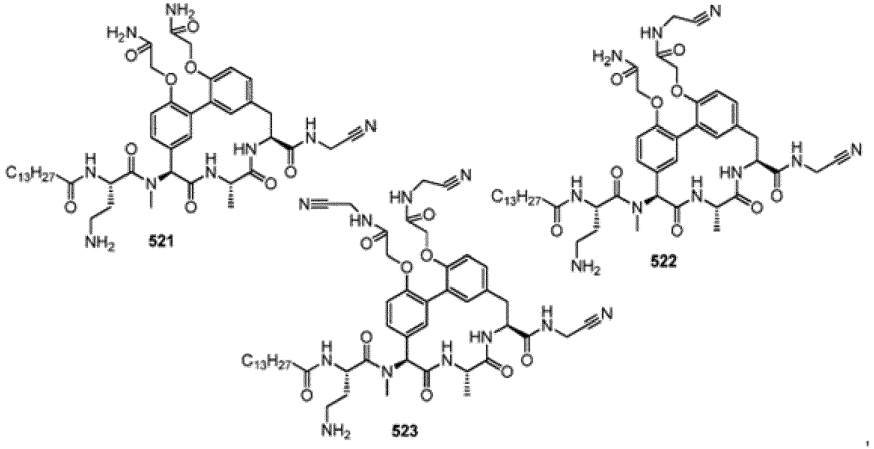 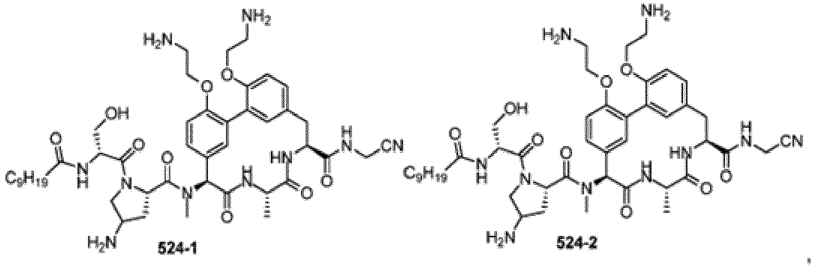 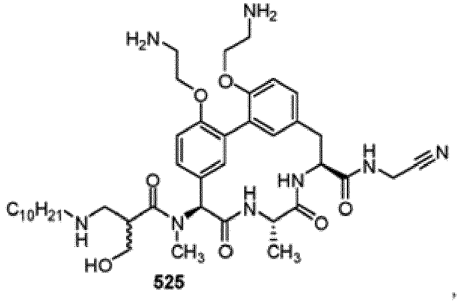 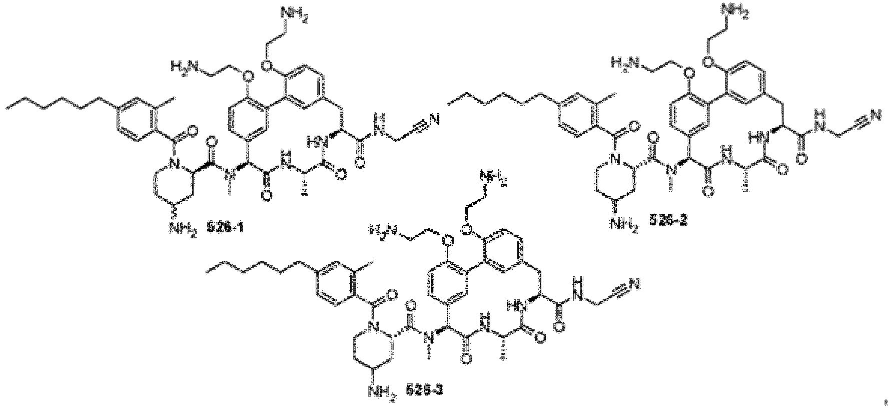 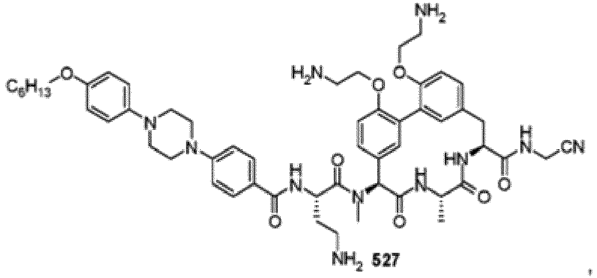 et 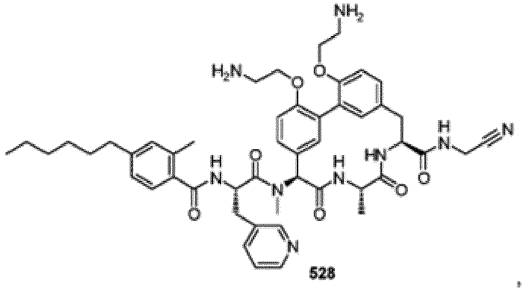 ou sel ou solvate pharmaceutiquement acceptable de celui-ci.

2. Composition pharmaceutique comprenant un composé selon la revendication 1, ou un sel pharmaceutiquement acceptable ou un solvate pharmaceutiquement acceptable de celui-ci, et un excipient pharmaceutiquement acceptable.

3. Composé selon la revendication 1, ou sel pharmaceutiquement acceptable ou solvate pharmaceutiquement acceptable de celui-ci, pour utilisation dans un procédé de traitement d'une infection bactérienne chez un mammifère.

4. Composé, sel ou solvate pour utilisation selon la revendication 3, l'infection bactérienne étant une infection impliquant :
Pseudomonas aeruginosa, Pseudomonas fluorescens, Pseudomonas acidovorans, Pseudomonas alcaligenes, Pseudomonas putida, Stenotrophomonas maltophilia, Burkholderia cepacia, Aeromonas hydrophilia, Escherichia coli, Citrobacterfreundii, Salmonella typhimurium, Salmonella typhi, Salmonella paratyphi, Salmonella enteritidis, Shigella dysenteriae, Shigella flexneri, Shigella sonnei, Enterobacter cloacae, Enterobacter aerogenes, Klebsiella pneumoniae, Klebsiella oxytoca, Serratia marcescens, Francisella tularensis, Morganella morganii, Proteus mirabilis, Proteus vulgaris, Providencia alcalifaciens, Providencia rettgeri, Providencia stuartii, Acinetobacter baumannii, Acinetobacter calcoaceticus, Acinetobacter haemolyticus, Yersinia enterocolitica, Yersinia pestis, Yersinia pseudotuberculosis, Yersinia intermedia, Bordetella pertussis, Bordetella parapertussis, Bordetella bronchiseptica, Haemophilus influenzae, Haemophilus parainfluenzae, Haemophilus haemolyticus, Haemophilus parahaemolyticus, Haemophilus ducreyi, Pasteurella multocida, Pasteurella haemolytica, Branhamella catarrhalis, Helicobacter pylori, Campylobacter fétus, Campylobacter jejuni, Campylobacter coli, Borrelia burgdorferi, Vibrio cholerae, Vibrio parahaemolyticus, Legionella pneumophila, Listeria monocytogenes, Neisseria gonorrhoeae, Neisseria meningitidis, Kingel la, Moraxella, Gardnerella vaginalis, Bacteroides fragilis, Bacteroides distasonis, Bacteroides 3452A homology group, Bacteroides vulgatus, Bacteroides ovalus, Bacteroides thetaiotaomicron, Bacteroides uniformis, Bacteroides eggerthii, Bacteroides splanchnicus, Clostridium difficile, Mycobacterium tuberculosis, Mycobacterium avium, Mycobacterium intracellulare, Mycobacterium leprae, Corynebacterium diphtheriae, Corynebacterium ulcerans, Streptococcus pneumoniae, Streptococcus agalactiae, Streptococcus pyogenes, Enterococcus faecalis, Enterococcus faecium, Staphylococcus aureus, Staphylococcus epidermidis, Staphylococcus saprophyticus, Staphylococcus intermedius, Staphylococcus hyicus subsp. hyicus, Staphylococcus haemolyticus, Staphylococcus hominis ou Staphylococcus saccharolyticus.

5. Composé, sel ou solvate pour utilisation selon la revendication 3 ou 4, l'infection bactérienne étant une infection impliquant une bactérie Gram négatif.

6. Composé, sel ou solvate pour utilisation selon l'une quelconque des revendications 3 à 5, le procédé comprenant l'administration topique du composé, sel ou solvate.

7. Composé, sel ou solvate pour utilisation selon l'une quelconque des revendications 3 à 5, le procédé comprenant en outre l'administration d'un deuxième agent thérapeutique.

8. Composé, sel ou solvate pour utilisation selon la revendication 7, le deuxième agent thérapeutique n'étant pas un inhibiteur de SpsB.

9. Composé, sel ou solvate pour utilisation selon la revendication 8, le deuxième agent thérapeutique étant un antibiotique aminoglycoside, un antibiotique fluoroquinolone, un antibiotique β-lactame, un antibiotique macrolide, un antibiotique glycopeptide, la rifampicine, le chloramphénicol, le fluoramphénicol, la colistine, la mupirocine, la bacitracine, la daptomycine ou le linézolide.

10. Composé, sel ou solvate pour utilisation selon la revendication 8, le deuxième agent thérapeutique étant un antibiotique β-lactame.

11. Composé, sel ou solvate pour utilisation selon la revendication 10, l'antibiotique β-lactame étant choisi parmi les pénicillines, les monobactames, les céphalosporines, les céphamycines et les carbapénèmes.

12. Composé, sel ou solvate pour utilisation selon la revendication 11, l'antibiotique β-lactame étant choisi parmi l'azlocilline, l'amoxicilline, l'ampicilline, le doripénème, le méropénème, le biapénème, le céfamandole, l'imipénème, la mezlocilline, le cefmétazole, le cefprozil, la pipéracilline/tazobactam, la carbénicilline, le céfaclor, la céphalothine, l'ertapénème, la céfazoline, la céfépime, le céfonicide, la céfoxitine, la ceftazidime, l'oxacilline, le cefdinir, la céfixime, la céfotaxime, le céfotétan, la cefpodoxime, la ceftizoxime, la ceftriaxone, le faropénème, le mécillinam, la méthicilline, le moxalactame, la ticarcilline, le tomopénème, le ceftobiprole, l'aroline, le flomoxef, le céfiprome et le céfozopran.

13. Composé, sel ou solvate pour utilisation selon la revendication 10, le procédé comprenant en outre l'administration d'un inhibiteur de β-lactamase.
